(19) Europäisches Patentamt
European Patent Office
Office européen des brevets

(11) EP 2 228 386 B1

# (12) EUROPEAN PATENT SPECIFICATION

(45) Date of publication and mention of the grant of the patent:
**18.03.2015 Bulletin 2015/12**

(51) Int Cl.:
***C07K 14/415*** *(2006.01)* ***C12N 15/82*** *(2006.01)*
*A01H 5/00* *(2006.01)*

(21) Application number: **09178941.2**

(22) Date of filing: **02.05.2008**

(54) **Plants having enhanced yield-related traits and a method for making the same**

Pflanzen mit verbesserten Eigenschaften bezüglich des Ertrags und Verfahren zu ihrer Herstellung

Installations dotées de caractéristiques de rendement améliorées et procédé de fabrication de celles-ci

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority:
**03.05.2007 EP 07107448**
**08.05.2007 US 916575 P**
**29.05.2007 EP 07109052**
**29.05.2007 EP 07109068**
**06.06.2007 US 942214 P**
**11.06.2007 EP 07109961**
**19.06.2007 EP 07110548**
**19.06.2007 EP 07110557**
**29.06.2007 US 937989 P**
**05.07.2007 US 948036 P**

(43) Date of publication of application:
**15.09.2010 Bulletin 2010/37**

(62) Document number(s) of the earlier application(s) in accordance with Art. 76 EPC:
**08767542.7 / 2 069 509**

(73) Proprietor: **BASF Plant Science GmbH**
**67056 Ludwigshafen (DE)**

(72) Inventors:
- **Hatzfeld, Yves**
  **59000 Lille (FR)**
- **Sanz Molinero, Ana Isabel**
  **9050 Gentbrugge (BE)**
- **Shirley, Amber**
  **Durham, NC 27703 (US)**
- **Darnielle, Lalitree**
  **Durham, NC 27707 (US)**
- **Frankard, Valerie**
  **1410 Waterloo (BE)**
- **Vandenabeele, Steven**
  **9700 Oudenaarde (BE)**
- **McKersie, Bryan**
  **Raleigh, NC 27617 (US)**

(74) Representative: **Popp, Andreas**
**BASF SE**
**Global Intellectual Property**
**GVX - C6**
**67056 Ludwigshafen (DE)**

(56) References cited:
**US-A1- 2004 123 343**

- **DATABASE EMBL [Online] 26 October 2005 (2005-10-26), Kim H. et al.: XP002593644 retrieved from EBI Database accession no. DV527573**
- **DATABASE Geneseq [Online] 21 April 2005 (2005-04-21), Liu et al.: XP002593645 retrieved from EBI Database accession no. ADX72380 -& DATABASE Geneseq [Online] 21 April 2005 (2005-04-21), "Plant full length insert polypeptide seqid 67500." XP002593651 retrieved from EBI accession no. GSP:ADY11685 Database accession no. ADY11685 -& US 2004/034888 A1 (LIU JINGDONG [US] ET AL) 19 February 2004 (2004-02-19)**
- **DATABASE UniProt [Online] 7 June 2005 (2005-06-07), "RecName: Full=Squamosa promoter-binding-like protein 11;" XP002593646 retrieved from EBI accession no. UNIPROT: Q9FZK0 Database accession no. Q9FZK0**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **CARDON G H ET AL: "Functional analysis of the Arabidopsis thaliana SBP-box gene SPL3: A novel gene involved in the floral transition" PLANT JOURNAL, BLACKWELL SCIENTIFIC PUBLICATIONS, OXFORD, GB LNKD- DOI: 10.1046/J.1365-313X.1997.12020367.X, vol. 12, no. 2, 1 January 1997 (1997-01-01), pages 367-377, XP002134823 ISSN: 0960-7412**
- **KLEIN J ET AL: "A new family of DNA binding proteins includes putative transcriptional regulators of the antirrhinum majus floral meristem identity genes SQUAMOSA" MOLECULAR AND GENERAL GENETICS, SPRINGER VERLAG, BERLIN, DE LNKD- DOI: 10.1007/S004380050046, vol. 250, no. 1, 15 January 1996 (1996-01-15), pages 7-16, XP002961672 ISSN: 0026-8925**
- **STONE JULIE M ET AL: "Arabidopsis AtSPL14, a plant-specific SBP-domain transcription factor, participates in plant development and sensitivity to fumonisin B1" PLANT JOURNAL, vol. 41, no. 5, March 2005 (2005-03), pages 744-754, XP002593647 ISSN: 0960-7412**
- **SHIKATA MASAHITO ET AL: "Arabidopsis SBP-Box Genes SPL10, SPL11 and SPL2 Control Morphological Change in Association with Shoot Maturation in the Reproductive Phase" PLANT AND CELL PHYSIOLOGY, vol. 50, no. 12, December 2009 (2009-12), pages 2133-2145, XP002593648 ISSN: 0032-0781**

## Description

The present application relates generally to the field of molecular biology and concerns a method for enhancing yield-related traits and/or improving various plant growth characteristics by modulating expression in a plant of a nucleic acid encoding a GRP (Growth Regulating Protein). The GRP is a Squamosa promoter binding protein-like 11 (SPL11) transcription factor polypeptide. The present invention also concerns plants having modulated expression of a nucleic acid encoding a GRP, which plants have improved growth characteristics relative to corresponding wild type plants or other control plants. The invention also provides novel GRP nucleic acids and GRP polypeptides as well as constructs useful in the methods of the invention.

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is increased yield. Yield is normally defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance and early vigour may also be important factors in determining yield. Optimizing the abovementioned factors may therefore contribute to increasing crop yield.

Seed yield is a particularly important trait, since the seeds of many plants are important for human and animal nutrition. Crops such as corn, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

Another important trait for many crops is early vigour. Improving early vigour is an important objective of modern rice breeding programs in both temperate and tropical rice cultivars. Long roots are important for proper soil anchorage in water-seeded rice. Where rice is sown directly into flooded fields, and where plants must emerge rapidly through water, longer shoots are associated with vigour. Where drill-seeding is practiced, longer mesocotyls and coleoptiles are important for good seedling emergence. The ability to engineer early vigour into plants would be of great importance in agriculture. For example, poor early vigor has been a limitation to the introduction of maize (Zea mays L.) hybrids based on Corn Belt germplasm in the European Atlantic.

A further important trait is that of improved abiotic stress tolerance. Abiotic stress is a primary cause of crop loss worldwide, reducing average yields for most major crop plants by more than 50% (Wang et al., Planta (2003) 218: 1-14). Abiotic stresses may be caused by drought, salinity, extremes of temperature, chemical toxicity and oxidative stress. The ability to improve plant tolerance to abiotic stress would be of great economic advantage to farmers worldwide and would allow for the cultivation of crops during adverse conditions and in territories where cultivation of crops may not otherwise be possible.

Crop yield may therefore be increased by optimising one of the above-mentioned factors.

Depending on the end use, the modification of certain yield traits may be favoured over others. For example for applications such as forage or wood production, or bio-fuel resource, an increase in the vegetative parts of a plant may be desirable, and for applications such as flour, starch or oil production, an increase in seed parameters may be particularly desirable. Even amongst the seed parameters, some may be favoured over others, depending on the application. Various mechanisms may contribute to increasing seed yield, whether that is in the form of increased seed size or increased seed number.

One approach to increasing yield (seed yield and/or biomass) in plants may be through modification of the inherent growth mechanisms of a plant, such as the cell cycle or various signalling pathways involved in plant growth or in defense mechanisms.

Surprisingly, it has now been found that modulating expression of a nucleic acid encoding a a Squamosa promoter binding protein-like 11 (SPL11) transcription factor polypeptide gives plants having enhanced yield-related traits and/or improved various plant growth characteristics relative to control plants.

According to one aspect of the application there is provided a method for improving or enhancing yield related traits and/or improving various plant growth characteristics of a plant relative to control plants, comprising modulating expression of a nucleic acid encoding a Squamosa promoter binding protein-like 11 (SPL11) transcription factor polypeptide in a plant.

## Background

### Squamosa promoter binding protein-like 11 (SPL11)

Transcription factor polypeptides are usually defined as proteins that show sequence-specific DNA binding affinity and that are capable of activating and/or repressing transcription. The Squamosa promoter binding protein-like (SPL) transcription factor polypeptides are structurally diverse proteins that share a highly conserved DNA binding domain (DBD) of about 80 amino acid residues in length (Klein et al. (1996) Mol Gen Genet 259: 7-16; Cardon et al. (1999) Gene 237: 91-104). The SPL transcription factor DNA consensus sequence binding site in the promoter of target genes is 5'-TNCGTACAA-3' where N represents any base. Within the SPL DBD are ten conserved cysteine (Cys) or histidine (His) residues (see Figure 28) of which eight are zinc coordinating residues binding two zinc ions necessary for the formation of SPL specific zinc finger tertiary structure (Yamasaki et al. (2004) J Mol Biol 337: 49-63). A second conserved feature within the SPL DBD is a bipartite nuclear localisation signal. Outside of the DBD, a micro RNA (miRNA) target motif, specifically targeted by miR156 family of miRNAs is found in most of the nucleic acid sequences encoding SPL transcription factor polypeptides (either in the coding region, or the 3' UTR) across the plant kingdom (Rhoades et al. (2002) Cell 110: 513-520). miRNAs control SPL gene expression post-transcriptionally by targeting SPL encoding mRNAs for degradation or by translational repression.

The Arabidopsis genome codes for at least 1533 transcriptional regulators, accounting for ~5.9% of its estimated total number of genes (Riechmann et al. (2000) Science 290: 2105-2109). The authors report 16 SPL transcription factor polypeptides in Arabidopsis thaliana, with little sequence similarity among themselves (apart from the abovementioned features), the size of the deduced SPL polypeptide ranging from 131 to 927 amino acids. Nevertheless, pairs of SPL transcription factor polypeptides sharing higher sequence homology were detected within the SPL family of this plant (Cardon et al. (1999)).

The SPL transcription factor polypeptides (only found in plants) characterized to date have been shown to function in plant development, in particular in flower development. Transgenic plants overexpressing an SPL3 transcription factor polypeptide were reported to flower earlier (Cardon et al. (1997) Plant J 12: 367-377). In European patent application EP1033405, the nucleic acid and deduced polypeptide sequences of the SPL11 transcription factor polypeptide are disclosed.

Surprisingly, it has now been found that modulating expression I a plant of a nucleic acid encoding a SPL11 polypeptide gives plants having enhanced yield-related traits, in particular increased yield relative to control plants.

According to one embodiment, there is provided a method for increasing seed yield in plants relative to control plants, comprising increasing expression in a plant of a nucleic acid encoding a SPL11 polypeptide, wherein said SPL11 comprises a SBP domain having in increasing order of preference at least 70%, 75%, 80%, 90%, 95%, 97% or more sequence identity to any one of SEQ ID NO: 456 to SEQ ID NO: 468 and SEQ ID NO: 478, wherein said increased seed yield preferably comprises increased total seed weight, number of filled seeds, number of seeds or florets per panicle, thousand-kernel weight, seed filling rate and/or harvest index relative to control,

According to one embodiment, there is provided novel SPL11 nucleic acids and SPL11 polypeptides as well as constructs comprising SPL11-encoding nucleic acids, useful in performing the methods of the invention

## Definitions

Polypeptide(s)/Protein(s)

The terms "polypeptide" and "protein" are used interchangeably herein and refer to amino acids in a polymeric form of any length, linked together by peptide bonds.

Polynucleotide(s)/Nucleic acid(s)/Nucleic acid sequence(s)/nucleotide sequence(s)

The terms "polynucleotide(s)", "nucleic acid sequence(s)", "nucleotide sequence(s)", "nucleic acid(s)", "nucleic acid molecule" are used interchangeably herein and refer to nucleotides, either ribonucleotides or deoxyribonucleotides or a combination of both, in a polymeric unbranched form of any length.

Control plant(s)

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. Nullizygotes are individuals missing the transgene by segregation. A "control plant" as used herein refers not only to whole plants, but also to plant parts, including seeds and seed parts.

Homologue(s)

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.

A deletion refers to removal of one or more amino acids from a protein.

An insertion refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag•100 epitope, c-myc epitope, FLAG®-epitope, lacZ, CMP (calmodulin-binding peptide), HA epitope, protein C epitope and VSV epitope.

A substitution refers to replacement of amino acids of the protein with other amino acids having similar properties (such as similar hydrophobicity, hydrophilicity, antigenicity, propensity to form or break $\alpha$-helical structures or $\beta$-sheet structures). Amino acid substitutions are typically of single residues, but may be clustered depending upon functional constraints placed upon the polypeptide; insertions will usually be of the order of about 1 to 10 amino acid residues. The amino acid substitutions are preferably conservative amino acid substitutions. Conservative substitution tables are well known in the art (see for example Creighton (1984) Proteins. W.H. Freeman and Company (Eds) and Table 1 below).

**Table 1:** Examples of conserved amino acid substitutions

| **Residue** | **Conservative Substitutions** | **Residue** | **Conservative Substitutions** |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu, Val | | |

Amino acid substitutions, deletions and/or insertions may readily be made using peptide synthetic techniques well known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols.

Derivatives

"Derivatives" include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein. Furthermore, "derivatives" also include fusions of the naturally-occurring form of the protein with tagging peptides such as FLAG, HIS6 or thioredoxin (for a review of tagging peptides, see Terpe, Appl. Microbiol. Biotechnol. 60, 523-533, 2003).

Orthologue(s)/Paralogue(s)

Orthologues and paralogues encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene.

Domain

The term "domain" refers to a set of amino acids conserved at specific positions along an alignment of sequences of evolutionarily related proteins. While amino acids at other positions can vary between homologues, amino acids that are highly conserved at specific positions indicate amino acids that are likely essential in the structure, stability or function of a protein. Identified by their high degree of conservation in aligned sequences of a family of protein homologues, they can be used as identifiers to determine if any polypeptide in question belongs to a previously identified polypeptide family.

Motif/Consensus sequence/Signature

The term "motif" or "consensus sequence" or "signature" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).

Hybridisation

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitrocellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids.

The term "stringency" refers to the conditions under which a hybridisation takes place. The stringency of hybridisation is influenced by conditions such as temperature, salt concentration, ionic strength and hybridisation buffer composition. Generally, low stringency conditions are selected to be about 30°C lower than the thermal melting point ($T_m$) for the specific sequence at a defined ionic strength and pH. Medium stringency conditions are when the temperature is 20°C below $T_m$, and high stringency conditions are when the temperature is 10°C below $T_m$. High stringency hybridisation conditions are typically used for isolating hybridising sequences that have high sequence similarity to the target nucleic acid sequence. However, nucleic acids may deviate in sequence and still encode a substantially identical polypeptide, due to the degeneracy of the genetic code. Therefore medium stringency hybridisation conditions may sometimes be needed to identify such nucleic acid molecules.

The Tm is the temperature under defined ionic strength and pH, at which 50% of the target sequence hybridises to a perfectly matched probe. The $T_m$ is dependent upon the solution conditions and the base composition and length of the probe. For example, longer sequences hybridise specifically at higher temperatures. The maximum rate of hybridisation is obtained from about 16°C up to 32°C below $T_m$. The presence of monovalent cations in the hybridisation solution reduce the electrostatic repulsion between the two nucleic acid strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the Tm decreases about 1 °C per % base mismatch. The Tm may be calculated using the following equations, depending on the types of hybrids:

1) DNA-DNA hybrids (Meinkoth and Wahl, Anal. Biochem., 138: 267-284, 1984):

$$T_m = 81.5°C + 16.6 x \log_{10}[Na^+]^a + 0.41 x \%[G/C^b] - 500 x [L^c]^{-1} - 0.61 x \% \text{ formamide}$$

2) DNA-RNA or RNA-RNA hybrids:

$$Tm = 79.8 + 18.5 (\log_{10}[Na^+]^a) + 0.58 (\%G/C^b) + 11.8 (\%G/C^b)^2 - 820/L^c$$

3) oligo-DNA or oligo-RNA[d] hybrids:

For <20 nucleotides: $T_m = 2 (l_n)$
For 20-35 nucleotides: $T_m = 22 + 1.46 (l_n)$

[a] or for other monovalent cation, but only accurate in the 0.01-0.4 M range.
[b] only accurate for %GC in the 30% to 75% range.
[c] L = length of duplex in base pairs.
[d] oligo, oligonucleotide; $l_n$, = effective length of primer = 2×(no. of G/C)+(no. of A/T).

Non-specific binding may be controlled using any one of a number of known techniques such as, for example, blocking the membrane with protein containing solutions, additions of heterologous RNA, DNA, and SDS to the hybridisation buffer, and treatment with Rnase. For non-homologous probes, a series of hybridizations may be performed by varying one of (i) progressively lowering the annealing temperature (for example from 68°C to 42°C) or (ii) progressively lowering the formamide concentration (for example from 50% to 0%). The skilled artisan is aware of various parameters which may be altered during hybridisation and which will either maintain or change the stringency conditions.

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid hybridisation assays or gene amplification detection procedures are as set forth above. More or less stringent conditions may also be selected. The skilled artisan is aware of various parameters which may be altered during washing and which will either maintain or change the stringency conditions.

For example, typical high stringency hybridisation conditions for DNA hybrids longer than 50 nucleotides encompass hybridisation at 65°C in 1x SSC or at 42°C in 1x SSC and 50% formamide, followed by washing at 65°C in 0.3x SSC. Examples of medium stringency hybridisation conditions for DNA hybrids longer than 50 nucleotides encompass hybridisation at 50°C in 4x SSC or at 40°C in 6x SSC and 50% formamide, followed by washing at 50°C in 2x SSC. The length of the hybrid is the anticipated length for the hybridising nucleic acid. When nucleic acids of known sequence are hybridised, the hybrid length may be determined by aligning the sequences and identifying the conserved regions described herein. 1×SSC is 0.15M NaCl and 15mM sodium citrate; the hybridisation solution and wash solutions may additionally include 5x Denhardt's reagent, 0.5-1.0% SDS, 100 μg/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition, Cold Spring Harbor Laboratory Press, CSH, New York or to Current Protocols in Molecular Biology, John Wiley & Sons, N.Y. (1989 and yearly updates).

Splice variant

The term "splice variant" as used herein encompasses variants of a nucleic acid sequence in which selected introns and/or exons have been excised, replaced, displaced or added, or in which introns have been shortened or lengthened. Such variants will be ones in which the biological activity of the protein is substantially retained; this may be achieved by selectively retaining functional segments of the protein. Such splice variants may be found in nature or may be manmade. Methods for predicting and isolating such splice variants are well known in the art (see for example Foissac and Schiex (2005) BMC Bioinformatics 6: 25).

Allelic variant

Alleles or allelic variants are alternative forms of a given gene, located at the same chromosomal position. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

Gene shuffling/Directed evolution

Gene shuffling or directed evolution consists of iterations of DNA shuffling followed by appropriate screening and/or selection to generate variants of nucleic acids or portions thereof encoding proteins having a modified biological activity (Castle et al., (2004) Science 304(5674): 1151-4; US patents 5,811,238 and 6,395,547).

Regulatory element/Control sequence/Promoter

The terms "regulatory element", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or enhances expression of a nucleic acid molecule in a cell, tissue or organ.

A "plant promoter" comprises regulatory elements, which mediate the expression of a coding sequence segment in plant cells. Accordingly, a plant promoter need not be of plant origin, but may originate from viruses or micro-organisms, for example from viruses which attack plant cells. The "plant promoter" can also originate from a plant cell, e.g. from the plant which is transformed with the nucleic acid sequence to be expressed in the inventive process and described herein. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid molecule must, as described above, be linked operably to or comprise a suitable promoter which expresses the gene at the right point in time and with the required spatial expression pattern.

For the identification of functionally equivalent promoters, the promoter strength and/or expression pattern of a candidate promoter may be analysed for example by operably linking the promoter to a reporter gene and assaying the expression level and pattern of the reporter gene in various tissues of the plant. Suitable well-known reporter genes include for example beta-glucuronidase or beta-galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1000 transcripts per cell. Generally, by "medium strength promoter" is intended a promoter that drives expression of a coding sequence at a lower level than a strong promoter, in particular at a level that is in all instances below that obtained when under the control of a 35S CaMV promoter.

Operably linked

The term "operably linked" as used herein refers to a functional linkage between the promoter sequence and the gene of interest, such that the promoter sequence is able to initiate transcription of the gene of interest.

Constitutive promoter

A "constitutive promoter" refers to a promoter that is transcriptionally active during most, but not necessarily all, phases of growth and development and under most environmental conditions, in at least one cell, tissue or organ. Table 2a below gives examples of constitutive promoters.

**Table 2a:** Examples of constitutive promoters

| Gene Source | Reference |
|---|---|
| Actin | McElroy et al, Plant Cell, 2: 163-171, 1990 |
| HMGP | WO 2004/070039 |
| CAMV 35S | Odell et al, Nature, 313: 810-812, 1985 |
| CaMV 19S | Nilsson et al., Physiol. Plant. 100:456-462, 1997 |
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin n 2 | An et al, Plant J. 10(1); 107-121, 1996 |
| 34S FMV | Sanger et al., Plant. Mol. Biol., 14, 1990: 433-443 |
| Rubisco small subunit | US 4,962,028 |
| OCS | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| nos | Shaw et al. (1984) Nucleic Acids Res. 12(20):7831-7846 |
| V-ATPase | WO 01/14572 |
| Super promoter | WO 95/14098 |
| G-box proteins | WO 94/12015 |

Ubiquitous promoter

A ubiquitous promoter is active in substantially all tissues or cells of an organism.

Developmentally-regulated promoter

A developmentally-regulated promoter is active during certain developmental stages or in parts of the plant that undergo developmental changes.

Inducible promoter

An inducible promoter has induced or increased transcription initiation in response to a chemical (for a review see Gatz 1997, Annu. Rev. Plant Physiol. Plant Mol. Biol., 48:89-108), environmental or physical stimulus, or may be "stress-inducible", i.e. activated when a plant is exposed to various stress conditions, or a "pathogen-inducible" i.e. activated when a plant is exposed to exposure to various pathogens.

Organ-specific/Tissue-specific promoter

An organ-specific or tissue-specific promoter is one that is capable of preferentially initiating transcription in certain organs or tissues, such as the leaves, roots, seed tissue etc. For example, a "root-specific promoter" is a promoter that is transcriptionally active predominantly in plant roots, substantially to the exclusion of any other parts of a plant, whilst still allowing for any leaky expression in these other plant parts. Promoters able to initiate transcription in certain cells only are referred to herein as "cell-specific".

Examples of root-specific promoters are listed in Table 2b below:

**Table 2b:** Examples of root-specific promoters

| Gene Source | Reference |
|---|---|
| RCc3 | Plant Mol Biol. 1995 Jan;27(2):237-48 |
| Arabidopsis PHT1 | Kovama et al., 2005; Mudge et al. (2002, Plant J. 31:341) |
| Medicago phosphate transporter | Xiao et al., 2006 |
| Arabidopsis Pyk10 | Nitz et al. (2001) Plant Sci 161(2): 337-346 |
| root-expressible genes | Tingey et al., EMBO J. 6: 1, 1987. |
| tobacco auxin-inducible gene | Van der Zaal et al., Plant Mol. Biol. 16, 983, 1991. |
| β-tubulin | Oppenheimer, et al., Gene 63: 87, 1988. |
| tobacco root-specific genes | Conkling, et al., Plant Physiol. 93: 1203, 1990. |
| B. napus G1-3b gene | United States Patent No. 5, 401, 836 |
| SbPRP1 | Suzuki et al., Plant Mol. Biol. 21: 109-119, 1993. |
| LRX1 | Baumberger et al. 2001, Genes & Dev. 15:1128 |
| BTG-26 Brassica napus | US 20050044585 |
| LeAMT1 (tomato) | Lauter et al. (1996, PNAS 3:8139) |
| The LeNRT1-1 (tomato) | Lauter et al. (1996, PNAS 3:8139) |
| class I patatin gene (potato) | Liu et al., Plant Mol. Biol. 153:386-395, 1991. |
| KDC1 (Daucus carota) | Downey et al. (2000, J. Biol. Chem. 275:39420) |
| TobRB7 gene | W Song (1997) PhD Thesis, North Carolina State University, Raleigh, NC USA |
| OsRAB5a (rice) | Wang et al. 2002, Plant Sci. 163:273 |
| ALF5 (Arabidopsis) | Diener et al. (2001, Plant Cell 13:1625) |
| NRT2;1Np (N. plumbaginifolia) | Quesada et al. (1997, Plant Mol. Biol. 34:265) |

A seed-specific promoter is transcriptionally active predominantly in seed tissue, but not necessarily exclusively in seed tissue (in cases of leaky expression). The seed-specific promoter may be active during seed development and/or during germination. The seed specific promoter may be endosperm/aleurone/embryo specific. Examples of seed-specific promoters (endosperm/aleurone/embryo specific) are shown in Table 2d, 2e, 2f. Further examples of seed-specific promoters are given in Qing Qu and Takaiwa (Plant Biotechnol. J. 2, 113-125, 2004), which disclosure is incorporated by reference herein as if fully set forth.

**Table 2c:** Examples of seed-specific promoters

| Gene source | Reference |
|---|---|
| seed-specific genes | Simon et al., Plant Mol. Biol. 5: 191, 1985; |
| | Scofield et al., J. Biol. Chem. 262: 12202, 1987.; |
| | Baszczynski et al., Plant Mol. Biol. 14: 633, 1990. |
| Brazil Nut albumin | Pearson et al., Plant Mol. Biol. 18: 235-245, 1992. |
| legumin | Ellis et al., Plant Mol. Biol. 10: 203-214, 1988. |
| glutelin (rice) | Takaiwa et al., Mol. Gen. Genet. 208: 15-22, 1986; |
| | Takaiwa et al., FEBS Letts. 221: 43-47, 1987. |
| zein | Matzke et al Plant Mol Biol, 14(3):323-32 1990 |
| napA | Stalberg et al, Planta 199: 515-519, 1996. |
| wheat LMW and HMW glutenin-1 | Mol Gen Genet 216:81-90, 1989; NAR 17:461-2, 1989 |
| wheat SPA | Albani et al, Plant Cell, 9: 171-184, 1997 |
| wheat α, β, γ-gliadins | EMBO J. 3:1409-15, 1984 |
| barley Itr1 promoter | Diaz et al. (1995) Mol Gen Genet 248(5):592-8 |
| barley B1, C, D, hordein | Theor Appl Gen 98:1253-62, 1999; Plant J 4:343-55, 1993; Mol Gen Genet 250:750-60, 1996 |
| barley DOF | Mena et al, The Plant Journal, 116(1): 53-62, 1998 |
| blz2 | EP99106056.7 |
| synthetic promoter | Vicente-Carbajosa et al., Plant J. 13: 629-640, 1998. |
| rice prolamin NRP33 | Wu et al, Plant Cell Physiology 39(8) 885-889, 1998 |
| rice α-globulin Glb-1 | Wu et al, Plant Cell Physiology 39(8) 885-889, 1998 |
| rice OSH1 | Sato et al, Proc. Natl. Acad. Sci. USA, 93: 8117-8122, 1996 |
| rice α-globulin REB/OHP-1 | Nakase et al. Plant Mol. Biol. 33: 513-522, 1997 |
| rice ADP-glucose pyrophosphorylase | Trans Res 6:157-68, 1997 |
| maize ESR gene family | Plant J 12:235-46, 1997 |
| sorghum α-kafirin | DeRose et al., Plant Mol. Biol 32:1029-35, 1996 |
| KNOX | Postma-Haarsma et al, Plant Mol. Biol. 39:257-71, 1999 |
| rice oleosin | Wu et al, J. Biochem. 123:386, 1998 |
| sunflower oleosin | Cummins et al., Plant Mol. Biol. 19: 873-876, 1992 |
| PRO0117, putative rice 40S ribosomal protein | WO 2004/070039 |
| PRO0136, rice alanine aminotransferase | Unpublished |
| PRO0147, trypsin inhibitor ITR1 (barley) | unpublished |
| PRO0151, rice WSI18 | WO 2004/070039 |
| PRO0175, rice RAB21 | WO 2004/070039 |
| PRO005 | WO 2004/070039 |
| PRO0095 | WO 2004/070039 |

(continued)

| Gene source | Reference |
|---|---|
| α-amylase (Amy32b) | Lanahan et al, Plant Cell 4:203-211, 1992; Skriver et al, Proc Natl Acad Sci USA 88:7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

**Table 2d:** examples of endosperm-specific promoters

| Gene source | Reference |
|---|---|
| glutelin (rice) | Takaiwa et al. (1986) Mol Gen Genet 208:15-22; Takaiwa et al. (1987) FEBS Letts. 221:43-47 |
| zein | Matzke et al., (1990) Plant Mol Biol 14(3): 323-32 |
| wheat LMW and HMW glutenin-1 | Colot et al. (1989) Mol Gen Genet 216:81-90, Anderson et al. (1989) NAR 17:461-2 |
| wheat SPA | Albani et al. (1997) Plant Cell 9:171-184 |
| wheat gliadins | Rafalski et al. (1984) EMBO 3:1409-15 |
| barley Itr1 promoter | Diaz et al. (1995) Mol Gen Genet 248(5):592-8 |
| barley B1, C, D, hordein | Cho et al. (1999) Theor Appl Genet 98:1253-62; Muller et al. (1993) Plant J 4:343-55; Sorenson et al. (1996) Mol Gen Genet 250:750-60 |
| barley DOF | Mena et al, (1998) Plant J 116(1): 53-62 |
| blz2 | Onate et al. (1999) J Biol Chem 274(14):9175-82 |
| synthetic promoter | Vicente-Carbajosa et al. (1998) Plant J 13:629-640 |
| rice prolamin NRP33 | Wu et al, (1998) Plant Cell Physiol 39(8) 885-889 |
| rice globulin Glb-1 | Wu et al. (1998) Plant Cell Physiol 39(8) 885-889 |
| rice globulin REB/OHP-1 | Nakase et al. (1997) Plant Molec Biol 33: 513-522 |
| rice ADP-glucose pyrophosphorylase | Russell et al. (1997) Trans Res 6:157-68 |
| maize ESR gene family | Opsahl-Ferstad et al. (1997) Plant J 12:235-46 |
| sorghum kafirin | DeRose et al. (1996) Plant Mol Biol 32:1029-35 |

**Table 2e:** Examples of embryo specific promoters:

| Gene source | Reference |
|---|---|
| rice OSH1 | Sato et al, Proc. Natl. Acad. Sci. USA, 93: 8117-8122, 1996 |
| KNOX | Postma-Haarsma et al, Plant Mol. Biol. 39:257-71, 1999 |
| PRO0151 | WO 2004/070039 |
| PRO0175 | WO 2004/070039 |
| PRO005 | WO 2004/070039 |
| PRO0095 | WO 2004/070039 |

**Table 2f:** Examples of aleurone-specific promoters:

| Gene source | Reference |
|---|---|
| α-amylase (Amy32b) | Lanahan et al, Plant Cell 4:203-211, 1992; Skriver et al, Proc Natl Acad Sci USA 88:7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

A green tissue-specific promoter as defined herein is a promoter that is transcriptionally active predominantly in green tissue, substantially to the exclusion of any other parts of a plant, whilst still allowing for any leaky expression in these other plant parts.

Examples of green tissue-specific promoters which may be used to perform the methods of the invention are shown in Table 2g.

**Table 2g:** Examples of green tissue-specific promoters

| Gene | Expression | Reference |
|---|---|---|
| Maize Orthophosphate dikinase | Leaf specific | Fukavama et al., 2001 |
| Maize Phosphoenolpyruvate carboxylase | Leaf specific | Kausch et al., 2001 |
| Rice Phosphoenolpyruvate carboxylase | Leaf specific | Liu et al., 2003 |
| Rice small subunit Rubisco | Leaf specific | Nomura et al., 2000 |
| rice beta expansin EXBP9 | Shoot specific | WO 2004/070039 |
| Pigeonpea small subunit Rubisco | Leaf specific | Panguluri et al., 2005 |
| Pea RBCS3A | Leaf specific | |

Another example of a tissue-specific promoter is a meristem-specific promoter, which is transcriptionally active predominantly in meristematic tissue, substantially to the exclusion of any other parts of a plant, whilst still allowing for any leaky expression in these other plant parts. Examples of green meristem-specific promoters which may be used to perform the methods of the invention are shown in Table 2h below.

**Table 2h:** Examples of meristem-specific promoters

| Gene source | Expression pattern | Reference |
|---|---|---|
| rice OSH1 | Shoot apical meristem, from embryo globular stage to seedling stage | Sato et al. (1996) Proc. Natl. Acad. Sci. USA, 93: 8117-8122 |
| Rice metallothionein | Meristem specific | BAD87835.1 |
| WAK1 & WAK 2 | Shoot and root apical meristems, and in expanding leaves and sepals | Wagner & Kohorn (2001) Plant Cell 13(2): 303-318 |

<u>Terminator</u>

The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene.

Modulation

The term "modulation" means in relation to expression or gene expression, a process in which the expression level is changed by said gene expression in comparison to the control plant, the expression level may be increased or decreased. The original, unmodulated expression may be of any kind of expression of a structural RNA (rRNA, tRNA) or mRNA with subsequent translation. The term "modulating the activity" shall mean any change of the expression of the inventive nucleic acid sequences or encoded proteins, which leads to increased yield and/or increased growth of the plants.

Expression

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

Increased expression/overexpression

The term "increased expression" or "overexpression" as used herein means any form of expression that is additional to the original wild-type expression level.

Methods for increasing expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of a nucleic acid encoding the polypeptide of interest. For example, endogenous promoters may be altered in vivo by mutation, deletion, and/or substitution (see, Kmiec, US 5,565,350; Zarling et al., WO9322443), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1:1183-1200). Such intron enhancement of gene expression is typically greatest when placed near the 5' end of the transcription unit. Use of the maize introns Adh1-S intron 1, 2, and 6, the Bronze-1 intron are known in the art. For general information see: The Maize Handbook, Chapter 116, Freeling and Walbot, Eds., Springer, N.Y. (1994).

Endogenous gene

Reference herein to an "endogenous" gene not only refers to the gene in question as found in a plant in its natural form (i.e., without there being any human intervention), but also refers to that same gene (or a substantially homologous nucleic acid/gene) in an isolated form subsequently (re)introduced into a plant (a transgene). For example, a transgenic plant containing such a transgene may encounter a substantial reduction of the transgene expression and/or substantial reduction of expression of the endogenous gene. The isolated gene may be isolated from an organism or may be manmade, for example by chemical synthesis.

Decreased expression

Reference herein to "decreased expression" or "reduction or substantial elimination" of expression is taken to mean a decrease in endogenous gene expression and/or polypeptide levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants.

For the reduction or substantial elimination of expression an endogenous gene in a plant, a sufficient length of substantially contiguous nucleotides of a nucleic acid sequence is required. In order to perform gene silencing, this may be as little as 20, 19, 18, 17, 16, 15, 14, 13, 12, 11, 10 or fewer nucleotides, alternatively this may be as much as the entire gene (including the 5' and/or 3' UTR, either in part or in whole). The stretch of substantially contiguous nucleotides may be derived from the nucleic acid encoding the protein of interest (target gene), or from any nucleic acid capable of encoding an orthologue, paralogue or homologue of the protein of interest. Preferably, the stretch of substantially contiguous nucleotides is capable of forming hydrogen bonds with the target gene (either sense or antisense strand), more preferably, the stretch of substantially contiguous nucleotides has, in increasing order of preference, 50%, 60%, 70%, 80%, 85%, 90%, 95%, 96%, 97%, 98%, 99%, 100% sequence identity to the target gene (either sense or antisense strand). A nucleic acid sequence encoding a (functional) polypeptide is not a requirement for the various methods discussed herein for the reduction or substantial elimination of expression of an endogenous gene.

This reduction or substantial elimination of expression may be achieved using routine tools and techniques. A preferred method for the reduction or substantial elimination of endogenous gene expression is by introducing and expressing in a plant a genetic construct into which the nucleic acid (in this case a stretch of substantially contiguous nucleotides derived from the gene of interest, or from any nucleic acid capable of encoding an orthologue, paralogue or homologue of any one of the protein of interest) is cloned as an inverted repeat (in part or completely), separated by a spacer (non-coding DNA).

In such a preferred method, expression of the endogenous gene is reduced or substantially eliminated through RNA-mediated silencing using an inverted repeat of a nucleic acid or a part thereof (in this case a stretch of substantially contiguous nucleotides derived from the gene of interest, or from any nucleic acid capable of encoding an orthologue, paralogue or homologue of the protein of interest), preferably capable of forming a hairpin structure. The inverted repeat is cloned in an expression vector comprising control sequences. A non-coding DNA nucleic acid sequence (a spacer, for example a matrix attachment region fragment (MAR), an intron, a polylinker, etc.) is located between the two inverted nucleic acids forming the inverted repeat. After transcription of the inverted repeat, a chimeric RNA with a self-complementary structure is formed (partial or complete). This double-stranded RNA structure is referred to as the hairpin RNA (hpRNA). The hpRNA is processed by the plant into siRNAs that are incorporated into an RNA-induced silencing complex (RISC). The RISC further cleaves the mRNA transcripts, thereby substantially reducing the number of mRNA transcripts to be translated into polypeptides. For further general details see for example, Grierson et al. (1998) WO 98/53083; Waterhouse et al. (1999) WO 99/53050).

Performance of the methods of the invention does not rely on introducing and expressing in a plant a genetic construct into which the nucleic acid is cloned as an inverted repeat, but any one or more of several well-known "gene silencing" methods may be used to achieve the same effects.

One such method for the reduction of endogenous gene expression is RNA-mediated silencing of gene expression (downregulation). Silencing in this case is triggered in a plant by a double stranded RNA sequence (dsRNA) that is substantially similar to the target endogenous gene. This dsRNA is further processed by the plant into about 20 to about 26 nucleotides called short interfering RNAs (siRNAs). The siRNAs are incorporated into an RNA-induced silencing complex (RISC) that cleaves the mRNA transcript of the endogenous target gene, thereby substantially reducing the number of mRNA transcripts to be translated into a polypeptide. Preferably, the double stranded RNA sequence corresponds to a target gene.

Another example of an RNA silencing method involves the introduction of nucleic acid sequences or parts thereof (in this case a stretch of substantially contiguous nucleotides derived from the gene of interest, or from any nucleic acid capable of encoding an orthologue, paralogue or homologue of the protein of interest) in a sense orientation into a plant. "Sense orientation" refers to a DNA sequence that is homologous to an mRNA transcript thereof. Introduced into a plant would therefore be at least one copy of the nucleic acid sequence. The additional nucleic acid sequence will reduce expression of the endogenous gene, giving rise to a phenomenon known as co-suppression. The reduction of gene expression will be more pronounced if several additional copies of a nucleic acid sequence are introduced into the plant, as there is a positive correlation between high transcript levels and the triggering of co-suppression.

Another example of an RNA silencing method involves the use of antisense nucleic acid sequences. An "antisense" nucleic acid sequence comprises a nucleotide sequence that is complementary to a "sense" nucleic acid sequence encoding a protein, i.e. complementary to the coding strand of a double-stranded cDNA molecule or complementary to an mRNA transcript sequence. The antisense nucleic acid sequence is preferably complementary to the endogenous gene to be silenced. The complementarity may be located in the "coding region" and/or in the "non-coding region" of a gene. The term "coding region" refers to a region of the nucleotide sequence comprising codons that are translated into amino acid residues. The term "non-coding region" refers to 5' and 3' sequences that flank the coding region that are transcribed but not translated into amino acids (also referred to as 5' and 3' untranslated regions).

Antisense nucleic acid sequences can be designed according to the rules of Watson and Crick base pairing. The antisense nucleic acid sequence may be complementary to the entire nucleic acid sequence (in this case a stretch of substantially contiguous nucleotides derived from the gene of interest, or from any nucleic acid capable of encoding an orthologue, paralogue or homologue of the protein of interest), but may also be an oligonucleotide that is antisense to only a part of the nucleic acid sequence (including the mRNA 5' and 3' UTR). For example, the antisense oligonucleotide sequence may be complementary to the region surrounding the translation start site of an mRNA transcript encoding a polypeptide. The length of a suitable antisense oligonucleotide sequence is known in the art and may start from about 50, 45, 40, 35, 30, 25, 20, 15 or 10 nucleotides in length or less. An antisense nucleic acid sequence according to the invention may be constructed using chemical synthesis and enzymatic ligation reactions using methods known in the art. For example, an antisense nucleic acid sequence (e.g., an antisense oligonucleotide sequence) may be chemically synthesized using naturally occurring nucleotides or variously modified nucleotides designed to increase the biological stability of the molecules or to increase the physical stability of the duplex formed between the antisense and sense nucleic acid sequences, e.g., phosphorothioate derivatives and acridine substituted nucleotides may be used. Examples of modified nucleotides that may be used to generate the antisense nucleic acid sequences are well known in the art. Known nucleotide modifications include methylation, cyclization and 'caps' and substitution of one or more of the naturally occurring nucleotides with an analogue such as inosine. Other modifications of nucleotides are well known in the art.

The antisense nucleic acid sequence can be produced biologically using an expression vector into which a nucleic acid sequence has been subcloned in an antisense orientation (i.e., RNA transcribed from the inserted nucleic acid will be of an antisense orientation to a target nucleic acid of interest). Preferably, production of antisense nucleic acid sequences in plants occurs by means of a stably integrated nucleic acid construct comprising a promoter, an operably linked antisense oligonucleotide, and a terminator.

The nucleic acid molecules used for silencing in the methods of the application (whether introduced into a plant or generated in situ) hybridize with or bind to mRNA transcripts and/or genomic DNA encoding a polypeptide to thereby inhibit expression of the protein, e.g., by inhibiting transcription and/or translation. The hybridization can be by conventional nucleotide complementarity to form a stable duplex, or, for example, in the case of an antisense nucleic acid sequence which binds to DNA duplexes, through specific interactions in the major groove of the double helix. Antisense nucleic acid sequences may be introduced into a plant by transformation or direct injection at a specific tissue site. Alternatively, antisense nucleic acid sequences can be modified to target selected cells and then administered systemically. For example, for systemic administration, antisense nucleic acid sequences can be modified such that they specifically bind to receptors or antigens expressed on a selected cell surface, e.g., by linking the antisense nucleic acid sequence to peptides or antibodies which bind to cell surface receptors or antigens. The antisense nucleic acid sequences can also be delivered to cells using the vectors described herein.

According to a further aspect, the antisense nucleic acid sequence is an a-anomeric nucleic acid sequence. An a-anomeric nucleic acid sequence forms specific double-stranded hybrids with complementary RNA in which, contrary to the usual b-units, the strands run parallel to each other (Gaultier et al. (1987) Nucl Ac Res 15: 6625-6641). The antisense nucleic acid sequence may also comprise a 2'-o-methylribonucleotide (Inoue et al. (1987) Nucl Ac Res 15, 6131-6148) or a chimeric RNA-DNA analogue (Inoue et al. (1987) FEBS Lett. 215, 327-330). The reduction or substantial elimination of endogenous gene expression may also be performed using ribozymes. Ribozymes are catalytic RNA molecules with ribonuclease activity that are capable of cleaving a single-stranded nucleic acid sequence, such as an mRNA, to which they have a complementary region. Thus, ribozymes (e.g., hammerhead ribozymes (described in Haselhoff and Gerlach (1988) Nature 334, 585-591) can be used to catalytically cleave mRNA transcripts encoding a polypeptide, thereby substantially reducing the number of mRNA transcripts to be translated into a polypeptide. A ribozyme having specificity for a nucleic acid sequence can be designed (see for example: Cech et al. U.S. Patent No. 4,987,071; and Cech et al. U.S. Patent No. 5,116,742). Alternatively, mRNA transcripts corresponding to a nucleic acid sequence can be used to select a catalytic RNA having a specific ribonuclease activity from a pool of RNA molecules (Bartel and Szostak (1993) Science 261, 1411-1418). The use of ribozymes for gene silencing in plants is known in the art (e.g., Atkins et al. (1994) WO 94/00012; Lenne et al. (1995) WO 95/03404; Lutziger et al. (2000) WO 00/00619; Prinsen et al. (1997) WO 97/13865 and Scott et al. (1997) WO 97/38116).

Gene silencing may also be achieved by insertion mutagenesis (for example, T-DNA insertion or transposon insertion) or by strategies as described by, among others, Angell and Baulcombe ((1999) Plant J 20(3): 357-62), (Amplicon VIGS WO 98/36083), or Baulcombe (WO 99/15682).

Gene silencing may also occur if there is a mutation on an endogenous gene and/or a mutation on an isolated gene/nucleic acid subsequently introduced into a plant. The reduction or substantial elimination may be caused by a non-functional polypeptide. For example, the polypeptide may bind to various interacting proteins; one or more mutation(s) and/or truncation(s) may therefore provide for a polypeptide that is still able to bind interacting proteins (such as receptor proteins) but that cannot exhibit its normal function (such as signalling ligand).

A further approach to gene silencing is by targeting nucleic acid sequences complementary to the regulatory region of the gene (e.g., the promoter and/or enhancers) to form triple helical structures that prevent transcription of the gene in target cells. See Helene, C., Anticancer Drug Res. 6, 569-84, 1991; Helene et al., Ann. N.Y. Acad. Sci. 660, 27-36 1992; and Maher, L.J. Bioassays 14, 807-15, 1992.

Other methods, such as the use of antibodies directed to an endogenous polypeptide for inhibiting its function in planta, or interference in the signalling pathway in which a polypeptide is involved, will be well known to the skilled man. In particular, it can be envisaged that manmade molecules may be useful for inhibiting the biological function of a target polypeptide, or for interfering with the signalling pathway in which the target polypeptide is involved.

Alternatively, a screening program may be set up to identify in a plant population natural variants of a gene, which variants encode polypeptides with reduced activity. Such natural variants may also be used for example, to perform homologous recombination.

Artificial and/or natural microRNAs (miRNAs) may be used to knock out gene expression and/or mRNA translation. Endogenous miRNAs are single stranded small RNAs of typically 19-24 nucleotides long. They function primarily to regulate gene expression and/ or mRNA translation. Most plant microRNAs (miRNAs) have perfect or near-perfect complementarity with their target sequences. However, there are natural targets with up to five mismatches. They are processed from longer non-coding RNAs with characteristic fold-back structures by double-strand specific RNases of the Dicer family. Upon processing, they are incorporated in the RNA-induced silencing complex (RISC) by binding to its main component, an Argonaute protein. MiRNAs serve as the specificity components of RISC, since they base-pair to target nucleic acids, mostly mRNAs, in the cytoplasm. Subsequent regulatory events include target mRNA cleavage and destruction and/or translational inhibition. Effects of miRNA overexpression are thus often reflected in decreased mRNA levels of target genes.

Artificial microRNAs (amiRNAs), which are typically 21 nucleotides in length, can be genetically engineered specifically to negatively regulate gene expression of single or multiple genes of interest. Determinants of plant microRNA target selection are well known in the art. Empirical parameters for target recognition have been defined and can be used to aid in the design of specific amiRNAs, (Schwab et al., Dev. Cell 8, 517-527, 2005). Convenient tools for design and generation of amiRNAs and their precursors are also available to the public (Schwab et al., Plant Cell 18, 1121-1133, 2006).

For optimal performance, the gene silencing techniques used for reducing expression in a plant of an endogenous gene requires the use of nucleic acid sequences from monocotyledonous plants for transformation of monocotyledonous plants, and from dicotyledonous plants for transformation of dicotyledonous plants. Preferably, a nucleic acid sequence from any given plant species is introduced into that same species. For example, a nucleic acid sequence from rice is transformed into a rice plant. However, it is not an absolute requirement that the nucleic acid sequence to be introduced originates from the same plant species as the plant in which it will be introduced. It is sufficient that there is substantial homology between the endogenous target gene and the nucleic acid to be introduced.

Described above are examples of various methods for the reduction or substantial elimination of expression in a plant of an endogenous gene. A person skilled in the art would readily be able to adapt the aforementioned methods for silencing so as to achieve reduction of expression of an endogenous gene in a whole plant or in parts thereof through the use of an appropriate promoter, for example.

<u>Selectable marker (gene)/Reporter gene</u>

"Selectable marker", "selectable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptII that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta®; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die).

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such a method is what is known as co-transformation. The co-transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the loxP sequences. If the marker gene is integrated between the loxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

Transgenic/Transgene/Recombinant

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either

(a) the nucleic acid sequences encoding proteins useful in the methods of the invention, or
(b) genetic control sequence(s) which is operably linked with the nucleic acid sequence according to the invention, for example a promoter, or
(c) a) and b)

are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.

A transgenic plant for the purposes of the invention is thus understood as meaning, as above, that the nucleic acids used in the method of the invention are not at their natural locus in the genome of said plant, it being possible for the nucleic acids to be expressed homologously or heterologously. However, as mentioned, transgenic also means that, while the nucleic acids according to the invention or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, preferably, heterologous expression of the nucleic acids takes place. Preferred transgenic plants are mentioned herein.

Transformation

The term "introduction" or "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid.

Alternatively, it may be integrated into the host genome. The resulting transformed plant cell may then be used to regenerate a transformed plant in a manner known to persons skilled in the art.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via Agrobacterium-mediated transformation. An advantageous transformation method is the transformation *in planta.* To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for *Agrobacterium*-mediated transformation of rice include well known methods for rice transformation, such as those described in any of the following: European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994), which disclosures are incorporated by reference herein as if fully set forth. In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth. Said methods are further described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming *Agrobacterium tumefaciens,* for example pBin19 (Bevan et al., Nucl. Acids Res. 12 (1984) 8711). Agrobacteria transformed by such a vector can then be used in known manner for the transformation of plants, such as plants used as a model, like *Arabidopsis* (*Arabidopsis thaliana* is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of *Agrobacterium tumefaciens* is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of *Arabidopsis* are treated with agrobacteria and seeds are obtained from the developing plants of which a certain proportion is transformed and thus transgenic [Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of *Arabidopsis,* intact plants under reduced pressure are treated with an agrobacterial suspension [Bechthold, N (1993). C R Acad Sci Paris Life Sci, 316: 1194-1199], while in the case of the "floral dip" method the developing floral tissue is incubated briefly with a surfactant-treated agrobacterial suspension [Clough, SJ and Bent AF (1998) The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome.

Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

T-DNA activation tagging

T-DNA activation tagging (Hayashi et al. Science (1992) 1350-1353), involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to modified expression of genes near the inserted T-DNA. The resulting transgenic plants show dominant phenotypes due to modified expression of genes close to the introduced promoter.

TILLING

The term "TILLING" is an abbreviation of "Targeted Induced Local Lesions In Genomes" and refers to a mutagenesis technology useful to generate and/or identify nucleic acids encoding proteins with modified expression and/or activity. TILLING also allows selection of plants carrying such mutant variants. These mutant variants may exhibit modified expression, either in strength or in location or in timing (if the mutations affect the promoter for example). These mutant variants may exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

Homologous recombination

Homologous recombination allows introduction in a genome of a selected nucleic acid at a defined selected position. Homologous recombination is a standard technology used routinely in biological sciences for lower organisms such as yeast or the moss *Physcomitrella.* Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; Iida and Terada (2004) Curr Opin Biotech 15(2): 132-8), and approaches exist that are generally applicable regardless of the target organism (Miller et al, Nature Biotechnol. 25, 778-785, 2007).

Yield

The term "yield" in general means a measurable produce of economic value, typically related to a specified crop, to an area, and to a period of time. Individual plant parts directly contribute to yield based on their number, size and/or weight, or the actual yield is the yield per square meter for a crop and year, which is determined by dividing total production (includes both harvested and appraised production) by planted square meters. The term "yield" of a plant may relate to vegetative biomass (root and/or shoot biomass), to reproductive organs, and/or to propagules (such as seeds) of that plant.

Early vigour

"Early vigour" refers to active healthy well-balanced growth especially during early stages of plant growth, and may result from increased plant fitness due to, for example, the plants being better adapted to their environment (i.e. optimizing the use of energy resources and partitioning between shoot and root). Plants having early vigour also show increased seedling survival and a better establishment of the crop, which often results in highly uniform fields (with the crop growing in uniform manner, i.e. with the majority of plants reaching the various stages of development at substantially the same time), and often better and higher yield. Therefore, early vigour may be determined by measuring various factors, such as thousand kernel weight, percentage germination, percentage emergence, seedling growth, seedling height, root length, root and shoot biomass and many more.

Increase/Improve/Enhance

The terms "increase", "improve" or "enhance" are interchangeable and shall mean in the sense of the application at least a 3%, 4%, 5%, 6%, 7%, 8%, 9% or 10%, preferably at least 15% or 20%, more preferably 25%, 30%, 35% or 40% more yield and/or growth in comparison to control plants as defined herein.

Seed yield

Increased seed yield may manifest itself as one or more of the following: a) an increase in seed biomass (total seed weight) which may be on an individual seed basis and/or per plant and/or per square meter; b) increased number of flowers per plant; c) increased number of (filled) seeds; d) increased seed filling rate (which is expressed as the ratio between the number of filled seeds divided by the total number of seeds); e) increased harvest index, which is expressed as a ratio of the yield of harvestable parts, such as seeds, divided by the total biomass; and f) increased thousand kernel weight (TKW), which is extrapolated from the number of filled seeds counted and their total weight. An increased TKW may result from an increased seed size and/or seed weight, and may also result from an increase in embryo and/or endosperm size.

An increase in seed yield may also be manifested as an increase in seed size and/or seed volume. Furthermore, an increase in seed yield may also manifest itself as an increase in seed area and/or seed length and/or seed width and/or seed perimeter. Increased yield may also result in modified architecture, or may occur because of modified architecture.

Greenness Index

The "greenness index" as used herein is calculated from digital images of plants. For each pixel belonging to the plant object on the image, the ratio of the green value versus the red value (in the RGB model for encoding color) is calculated. The greenness index is expressed as the percentage of pixels for which the green-to-red ratio exceeds a given threshold. Under normal growth conditions, under salt stress growth conditions, and under reduced nutrient availability growth conditions, the greenness index of plants is measured in the last imaging before flowering. In contrast, under drought stress growth conditions, the greenness index of plants is measured in the first imaging after drought.

Plant

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising *Acer spp., Actinidia* spp., *Abelmoschus* spp., *Agave sisalana, Agropyron* spp., *Agrostis stolonifera, Allium* spp., *Amaranthus* spp., *Ammophila arenaria, Ananas comosus, Annona* spp., *Apium graveolens, Arachis spp, Artocarpus* spp., *Asparagus officinalis, Avena* spp. *(e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica* spp. *(e.g. Brassica napus, Brassica rapa* ssp. [canola, oilseed rape, turnip rape]), *Cadaba farinosa, Camellia sinensis, Canna indica, Cannabis sativa, Capsicum* spp., *Carex elata, Carica papaya, Carissa macrocarpa, Carya* spp., *Carthamus tinctorius, Castanea* spp., *Ceiba pentandra, Cichorium endivia, Cinnamomum* spp., *Citrullus lanatus, Citrus* spp., *Cocos* spp., *Coffea* spp., *Colocasia esculenta, Cola* spp., *Corchorus sp., Coriandrum sativum, Corylus* spp., *Crataegus* spp., *Crocus sativus, Cucurbita* spp., *Cucumis* spp., *Cynara* spp., *Daucus carota, Desmodium* spp., *Dimocarpus longan, Dioscorea* spp., *Diospyros* spp., *Echinochloa* spp., *Elaeis (e.g. Elaeis guineensis, Elaeis oleifera), Eleusine coracana, Eragrostis tef, Erianthus sp., Eriobotrya japonica, Eucalyptus sp., Eugenia uniflora, Fagopyrum* spp., *Fagus* spp., *Festuca arundinacea, Ficus carica, Fortunella* spp., *Fragaria* spp., *Ginkgo biloba, Glycine* spp. *(e.g. Glycine max, Soja hispida or Soja max), Gossypium hirsutum, Helianthus* spp. *(e.g. Helianthus annuus), Hemerocallis fulva, Hibiscus* spp., *Hordeum* spp. *(e.g. Hordeum vulgare), Ipomoea batatas, Juglans* spp., *Lactuca sativa, Lathyrus* spp., *Lens culinaris, Linum usitatissimum, Litchi chinensis, Lotus* spp., *Luffa acutangula, Lupinus* spp., *Luzula sylvatica, Lycopersicon* spp. *(e.g. Lycopersicon esculentum, Lycopersicon lycopersicum, Lycopersicon pyriforme), Macrotyloma* spp., *Malus* spp., *Malpighia emarginata, Mammea americana, Mangifera indica, Manihot* spp., *Manilkara zapota, Medicago sativa, Melilotus* spp., *Mentha* spp., *Miscanthus sinensis, Momordica* spp., *Morus nigra, Musa* spp., *Nicotiana* spp., *Olea* spp., *Opuntia* spp., *Ornithopus* spp., *Oryza* spp. *(e.g. Oryza sativa, Oryza latifolia), Panicum miliaceum, Panicum virgatum, Passiflora edulis, Pastinaca sativa, Pennisetum sp., Persea* spp., *Petroselinum crispum, Phalaris arundinacea, Phaseolus* spp., *Phleum pratense, Phoenix* spp., *Phragmites australis, Physalis* spp., *Pinus* spp., *Pistacia vera, Pisum* spp., *Poa* spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Ricinus communis, Rubus* spp., *Saccharum* spp., *Salix sp., Sambucus* spp., *Secale cereale, Sesamum* spp., *Sinapis sp., Solanum* spp. *(e.g. Solanum tuberosum, Solanum integrifolium or Solanum lycopersicum), Sorghum bicolor, Spinacia* spp., *Syzygium* spp., *Tagetes* spp., *Tamarindus indica, Theobroma cacao*, *Trifolium* spp., *Tripsacum dactyloides, Triticosecale rimpaui, Triticum* spp. *(e.g. Triticum aestivum, Triticum durum, Triticum turgidum, Triticum hybernum, Triticum macha, Triticum sativum, Triticum monococcum or Triticum vulgare), Tropaeolum minus, Tropaeolum majus, Vaccinium* spp., *Vicia* spp., *Vigna* spp., *Viola odorata, Vitis* spp., *Zea mays, Zizania palustris, Ziziphus* spp., amongst others.

## Detailed description of the invention

### I. Squamosa promoter binding protein-like 11 (SPL11) transcription factor polypeptide

Surprisingly, it has now been found that increasing expression in a plant of a nucleic acid encoding a SPL11 polypeptide gives plants having enhanced seed yield relative to control plants. According to a first embodiment, the present invention provides a method for enhancing seed yield in plants relative to control plants, comprising increasing expression in a plant of a nucleic acid encoding a SPL11 polypeptide.

The present invention also provides hitherto unknown SPL11-encoding nucleic acids and SPL11 polypeptides. These sequences also being useful in performing the methods of the invention.

Therefore according to a further embodiment of the present invention there is provided an isolated nucleic acid molecule comprising:

(i) a nucleic acid represented by SEQ ID NO: 448;
(ii) the complement of a nucleic acid represented by SEQ ID NO: 448;
(iii) a nucleic acid encoding a SPL11 polypeptide having, in increasing order of preference, at least 70%, 75%, 80%, 85%, 90%, 95%, 96%, 97%, 98%, 99% or more sequence identity to the amino acid sequence represented by SEQ ID NO: 449, and having in increasing order of preference at least 70%, 75%, 80%, 85%, 90%, 95%, 96%, 97%, 98%, 99% or more sequence identity to SEQ ID NO: 465: SYCQVEGCRTDLSSAKDYHRKHRVCEPHSKAPKVVVAGLERRFC QQCSRFHGLAEFDQKKKSCRRRLNDHNARRRKPQPEAL (which represents the SBP domain in SEQ ID NO: 449);

Furthermore, there is also provided an isolated polypeptide comprising:

(i) an amino acid sequence represented by SEQ ID NO: 449;
(ii) an amino acid sequence having, in increasing order of preference, at least 70%, 75%, 80%, 85%, 90%, 95%, 96%, 97%, 98%, 99% or more sequence identity to the amino acid sequence represented by SEQ ID NO: 449, and having in increasing order of preference at least 70%, 75%, 80%, 85%, 90%, 95%, 96%, 97%, 98%, 99% or more sequence identity to SEQ ID NO: 465: SYCQVEGCRTDLSSAKDYHRKHRVCEPHSKAPKVVVAGLERRFCQQCSRFHG LAEFDQKKKSCRRRLNDHNARRRKPQPEAL (which represents the SBP domain in SEQ ID NO: 449).

A preferred method for increasing expression of a nucleic acid encoding a SPL11 polypeptide is by introducing and expressing in a plant a nucleic acid encoding a SPL11 polypeptide.

Any reference hereinafter to a "protein useful in the methods of the invention" is taken to mean a SPL11 polypeptide as defined herein. Any reference hereinafter to a "nucleic acid useful in the methods of the invention" is taken to mean a nucleic acid capable of encoding such a SPL11 polypeptide. The nucleic acid to be introduced into a plant (and therefore useful in performing the methods of the invention) is any nucleic acid encoding the type of protein which will now be described, herein also named "SPL11 nucleic acid" or "SPL11 gene".

A SPL11 polypeptide as defined herein refers to a polypeptide comprising a Squamosa Binding Protein (SBP) domain, such domain having in increasing order of preference at least 70%, 75%, 80%, 85%, 90%, 95%, 97% or more sequence identity to any one of the SBP domains as represented by SEQ ID NO: 456 to SEQ ID NO: 468 or SEQ ID NO: 478.

An "SPL11 polypeptide" as defined herein comprises the protein represented by SEQ ID NO: 448 which identical to SEQ ID NO: 172 and to homologues (orthologues and paralogues) thereof. Preferably, the homologues of SEQ ID NO: 172 have a DNA binding domain.

SPL11 polypeptides can be found in specialized databases such as Pfam, (Finn et al. Nucleic Acids Research (2006) Database Issue 34:D247-D251). Pfam compiles a large collection of multiple sequence alignments and hidden Markov models (HMM) covering many common protein domains and families and is available through the Sanger Institute in the United Kingdom.

The gathering cutoff threshold of the SBP domain in the Pfam HMM_fs and Pfam HUM_ls models is 25.0. Trusted matches as considered in the Pfam database are those sequences scoring higher than the gathering cut-off threshold. However potential matches, comprising true SBP domains, may still fall under the gathering cut-off. Preferably a SPL11 polypeptide is a protein having one or more domains in their sequence that exceed the gathering cutoff of the Pfam protein domain family PF03110, known as SBP domain.

Alternatively, a SBP domain in a polypeptide may be identified by performing a sequence comparison with known polypeptides comprising a SBP domain and establishing the similarity in the region of the SBP domain. The sequences may be aligned using any of the methods well known in the art such as Blast algorithms. The probability for the alignment to occur with a given sequence is taken as basis for identifying similar polypeptides. A parameter that is typically used to represent such probability is called e-value. The E-value is a measure of the reliability of the S score. The S score is a measure of the similarity of the query to the sequence shown. The e-value describes how often a given S score is expected to occur at random. The e-value cut-off may be as high as 1.0. The typical threshold for a good e-value from a BLAST search output using an SPL11 polypeptide as query sequence can is lower than $e^5(=10^{-5})$, $1.e^{-10}$, $1.e^{-15}$, $1.e^{-20}$, $1.e^{-25}$, $1.e^{-50}$, $1.e^{-75}$, $1.e^{-100}$, $1.e^{-200}$, $1.e^{-300}$, $1.e^{-400}$, $1.e^{-500}$, $1.e^{-600}$, $1.e^{-700}$ and $1.e^{-800}$. Preferably SPL11 polypeptides of the invention comprise a sequence having in increasing order of preference an e-value lower than $e^{-5}(=10^{-5})$, $1.e^{-10}$, $1.e^{-15}$, $1.e^{-20}$, $1.e^{-25}$, $1.e^{-50}$, $1.e^{-75}$, $1.e^{-100}$, $1.e^{-200}$, $1.e^{-300}$, $1.e^{-400}$, $1.e^{-500}$, $1.e^{-600}$, $1.e^{-700}$ and $1.e^{-800}$ in an alignment with a SBP domain found in a known SPL11 polypeptide.

Examples of SPL11 polypeptides useful in the methods of the invention are given in Table G1. The amino acid coordinates of the SBP domain in the representative SPL11 protein of Table G1 are given in Table G4 and the domain sequence is represented by SEQ ID NO: 456 to SEQ ID NO: 468. A consensus sequence of the SBP domains present in SPL11 polypeptides is given in SEQID NO: 478.

Preferred SPL11 polypeptides of the invention are those having in increasing order of preference at least 70%, 75%, 80%, 85%, 90%, 95%, or more sequence identity to any of the polypeptides given in Table G2.

Typically SPL11 polypeptides may comprise in addition to the SBP domain one or more of the following conserved motifs at conserved positions in the sequence relative to the SBP domain: (i) Motif 1 as represented by SEQ ID NO: 469, (ii) Motif 2 is a serine rich region typically found at the N-terminal end of the SBP domain and can be represented by SEQ ID NO: 470; (iii) Motif 3 as presented by SEQ ID: 471 which is typically encoded by nucleotides comprised within the SPL11 polynucleotide region targeted by members of the miR156 microRNA family; (iv) Motif 4 as represented by SEQ ID NO: 472. Figure 27 shows the conserved motifs and their relative position in the SPL11 polypeptide sequence represented by SEQ ID NO: 428.

Therefore, preferred SPL11 polypeptides useful in the method of the invention, comprise in addition to the SBP domain any one or more of the following conserved motifs:

(i) Motif 1 as represented by SEQ ID NO: 469 wherein any conservative amino acid substitution and/or 1 or 2 non conservative substitutions are allowed,

(ii) Motif 2 as represented by SEQ ID NO: 470 wherein any amino acid substitution is allowed, provided that at least 4 amino acids have a polar side chain, preferably serine or threonine, and provided that this motif is located at the N-terminal end of the SBP domain;

(iii) Motif 3 as represented by SEQ ID: 471, wherein 1 or 2 mismatches are allowed;

(iv) Motif 4 as represented by SEQ ID: 472, wherein 1, 2 or 3 mismatches are allowed.

Examples of conservative amino acid substitutions are given in the background section. Typically amino acids comprised in polypeptides are alpha amino acids having an amine and a carboxyl group attached to the same carbon, the alpha carbon, which amino acid molecules often comprise a side chain attached to the alpha carbon. Table 3 shows the classification of amino acids based on the physical and biochemical properties of the side chain.

**Table 3.** Classification of amino acids according to the side chain properties.

| Amino Acid | 3-Letter | 1-Letter | Side chain polarity | Side chain acidity or basicity | Hydropathy index |
|---|---|---|---|---|---|
| Arginine | Arg | R | polar | basic | -4.5 |
| Asparagine | Asn | N | polar | neutral | -3.5 |
| Aspartic acid | Asp | D | polar | acidic | -3.5 |
| Cysteine | Cys | C | polar | neutral | 2.5 |
| Glutamic acid | Glu | E | polar | acidic | -3.5 |
| Glutamine | Gln | Q | polar | neutral | -3.5 |
| Histidine | His | H | polar | basic | -3.2 |
| Lysine | Lys | K | polar | basic | -3.9 |
| Serine | Ser | S | polar | neutral | -0.8 |
| Threonine | Thr | T | polar | neutral | -0.7 |
| Tyrosine | Tyr | Y | polar | neutral | -1.3 |
| Alanine | Ala | A | nonpolar | neutral | 1.8 |
| Glycine | Gly | G | nonpolar | neutral | -0.4 |
| Isoleucine | Ile | I | nonpolar | neutral | 4.5 |
| Leucine | Leu | L | nonpolar | neutral | 3.8 |
| Methionine | Met | M | nonpolar | neutral | 1.9 |
| Phenylalanine | Phe | F | nonpolar | neutral | 2.8 |
| Proline | Pro | P | nonpolar | neutral | -1.6 |
| Tryptophan | Trp | W | nonpolar | neutral | -0.9 |
| Valine | Val | V | nonpolar | neutral | 4.2 |

Examples of SPL11 polypeptides comprising one or more of the conserved motifs Motif 1 to Motif 4 are given in Example 62. Figure 28 shows the position of the conserved motifs in those SPL11 polypeptides.

Preferably, the SPL11 polypeptide of the invention when used in the construction of a phylogenetic tree, such as the one depicted in Figure 29, clusters within the S3 group comprising the amino acid sequence represented by SEQ ID NO: 428 (named AtSPL11 in Figure 29) rather than with any other group.

The term "domain" and "motif" is defined in the "definitions" section herein. Specialist databases exist for the identification of domains, for example, SMART (Schultz et al. (1998) Proc. Natl. Acad. Sci. USA 95, 5857-5864; Letunic et al. (2002) Nucleic Acids Res 30, 242-244, InterPro (Mulder et al., (2003) Nucl. Acids. Res. 31, 315-318, Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAI Press, Menlo Park; Hulo et al., Nucl. Acids. Res. 32:D134-D137, (2004), or Pfam (Bateman et al., Nucleic Acids Research 30(1): 276-280 (2002). A set of tools for *in silico* analysis of protein sequences is available on the ExPASy proteomics server (Swiss Institute of Bioinformatics (Gasteiger et al., ExPASy: the proteomics server for in-depth protein knowledge and analysis, Nucleic Acids Res. 31:3784-3788(2003)). Domains may also be identified using routine techniques, such as by sequence alignment.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic acid or amino acid sequence or over selected domains or conserved motif(s), using the programs mentioned above using the default parameters.

Furthermore, SPL11 polypeptides (at least in their native form) may typically have DNA binding activity, in particular they may bind DNA fragments comprising the SBP domain DNA binding box as represented by SEQ ID NO: 49. Typically the SBP domain DNA binding box is found in gene promoters of plant origin such as the SQUAMOSA gene. Fragments comprising the SBP domain DNA binding box are preferably more than 10, 15, 20, 25, 100, 200, 500, 1000, 2000 base pairs long. Methods to determine DNA binding of SBP domain containing proteins are applicable to SPL11 polypeptides and are known in the art (Klein at al. Mol Gen Genet. 1996, 15;250(1):7-16; Yamasaki et al. 2004 J Mol Biol. 2004, 12;337(1):49-63).

Preferred SPL11 polypetides of the invention are those having DNA binding activity, more preferably those binding a DNA fragment comprising SEQ ID NO: 475.

The present invention is illustrated by transforming plants with the nucleic acid sequence represented by SEQ ID NO: 427, encoding the polypeptide sequence of SEQ ID NO: 428. However, performance of the invention is not restricted to these sequences; the methods of the invention may advantageously be performed using any SPL11 encoding nucleic acid or SPL11 polypeptide as defined herein.

Examples of nucleic acids encoding SPL11 polypeptides are given in Table G1 of Example 62 herein. Such nucleic acids are useful in performing the methods of the invention. The amino acid sequences given in Table G1 of Example 62 are example sequences of orthologues and paralogues of the SPL11 polypeptide represented by SEQ ID NO: 428, the terms "orthologues" and "paralogues" being as defined herein. Further orthologues and paralogues may readily be identified by performing a so-called reciprocal blast search. Typically, this involves a first BLAST involving BLASTing a query sequence (for example using any of the sequences listed in Table G1 of Example 62) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 427 or SEQ ID NO: 428, the second BLAST would therefore be against Arabidopsis sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues.

Nucleic acid variants may also be useful in practising the methods of the invention. Examples of such variants include nucleic acids encoding homologues and derivatives of any one of the amino acid sequences given in Table G1 of Example 62, the terms "homologue" and "derivative" being as defined herein. Also useful in the methods of the invention are nucleic acids encoding homologues and derivatives of orthologues or paralogues of any one of the amino acid sequences given in Table G1 of Example 62. Homologues and derivatives useful in the methods of the present invention have substantially the same biological and functional activity as the unmodified protein from which they are derived.

Further nucleic acid variants useful in practising the methods of the application include portions of nucleic acids encoding SPL11 polypeptides, nucleic acids hybridising to nucleic acids encoding SPL11 polypeptides, splice variants of nucleic acids encoding SPL11 polypeptides, allelic variants of nucleic acids encoding SPL11 polypeptides and variants of nucleic acids encoding SPL11 polypeptides obtained by gene shuffling. The terms hybridising sequence, splice variant, allelic variant and gene shuffling are as described herein.

A preferred nucleic acid variant useful in practising the methods of the application is a nucleic acid encoding a SPL11 polypeptide, which is microRNA insensitive, further preferably the nucleic acid is insensitive to microRNAs belonging to the miR156 family. MicroRNAs target nucleic acids (RNA in particular) for destruction typically causing a reduction or inhibition of the accumulation of the targeted RNA. Targeting requires hybridisation between the microRNA and the targeted nucleic acid (RNA) in a very specific region, called the miR (microRNA) target site, which comprises a sequence complementary to a portion of the mature microRNA gene. Typically microRNA insensitive nucleic acids comprise a sequence having in increasing order of preference 1, 2, 3, 4, 5 or more mistmatches in an alignment to the relevant microRNA molecule in the miR target site. MicroRNA insensitive nucleic acids may accumulated in a cell to levels in increasing order of preference 5, 10, 20, 30, 40, 50 times or higher than the corresponding nucleic acid targeted by the relevant MicroRNA, which typically comprises 100 % sequence complementary in the miR target site.

The miR156 family has been described earlier and a compilation of the microRNAs including the miR156 family members can be found at miRBase database (Griffiths-Jones et al. 2006 Nucleic Acids Research, 2006, Vol. 34, Database issue D140-D144). The miRBase database is maintained by the The Wellcome Trust Sanger Institute, in Cambridge, UK. The miR156 target site in a SPL11 nucleic acid is complementary to the mature sequence of miR156 microRNAs. An example of mature sequences of the miR156 family members from rice and their corresponding target sites on rice SPL nucleic acids is given in Figure 30A. Figure 31B shows an alignment of representative SPL11 nucleic acids (in DNA form), in which the target site of miR156 in the corresponding ribonucleic acids is indicated. An example of a miR156 insensitive SPL11 nucleic acid encoding SEQ ID NO: 428 is represented by SEQ ID NO: 431. Further examples of miR156 insensitive SPL11 nucleic acids are represented by SEQ ID 440 and SEQ ID NO: 454, the latter lacking the miR156 target site.

Preferably a SPL11 nucleic acid useful in the methods of the application has 1, 2, 3, 4 or more mismatches in the miR156 target site or lack the target site of the miR156 gene. Examples of such SPL11 nucleic acids are given in Figure 31B. Further preferably is a nucleic acid as represented by SEQ ID NO: 431, SEQ ID 440 and SEQ ID NO: 454.

Nucleic acids encoding SPL11 polypeptides need not be full-length nucleic acids, since performance of the methods of the invention does not rely on the use of full-length nucleic acid sequences. According to the present application there is provided a method for enhancing yield-related traits in plants, comprising introducing and expressing in a plant a portion of any one of the nucleic acid sequences given in Table G1 of Example 62, or a portion of a nucleic acid encoding an orthologue, paralogue or homologue of any of the amino acid sequences given in Table G1 of Example 62.

A portion of a nucleic acid may be prepared, for example, by making one or more deletions to the nucleic acid. The portions may be used in isolated form or they may be fused to other coding (or non-coding) sequences in order to, for example, produce a protein that combines several activities. When fused to other coding sequences, the resultant polypeptide produced upon translation may be bigger than that predicted for the protein portion.

Portions useful in the methods of the application encode a SPL11 polypeptide as defined herein, and have substantially the same biological activity as the amino acid sequences given in Table G1 of Example 62. Preferably, the portion is a portion of any one of the nucleic acids given in Table G1 of Example 62, or is a portion of a nucleic acid encoding an orthologue or paralogue of any one of the amino acid sequences given in Table G1 of Example 62. Preferably the portion is at least 70, 100, 200, 500, 550, 600, 650, 700, 750, 800, 850, 900, 950, 1000, 1500, 2000, 2500, 3000, 3500, 4000, 4500, 5000 consecutive nucleotides in length, the consecutive nucleotides being of any one of the nucleic acid sequences given in Table G1 of Example 62, or of a nucleic acid encoding an orthologue or paralogue of any one of the amino acid sequences given in Table G1 of Example 62. Preferably the portion encodes at least an SBP domain. Most preferably the portion is a portion of the nucleic acid of SEQ ID NO: 427. Preferably, the portion encodes an amino acid sequence which when used in the construction of a phylogenetic tree, such as the one depicted in Figure 29, clusters with the group of SPL11 polypeptides comprising the amino acid sequence represented by SEQ ID NO: 428 (AtSPL11) rather than with any other group.

Another nucleic acid variant useful in the methods of the application is a nucleic acid capable of hybridising, under reduced stringency conditions, preferably under stringent conditions, with a nucleic acid encoding a SPL11 polypeptide as defined herein, or with a portion as defined herein.

According to the present application there is provided a method for enhancing yield-related traits in plants, comprising introducing and expressing in a plant a nucleic acid capable of hybridizing to any one of the nucleic acids given in Table G1 of Example 62, or comprising introducing and expressing in a plant a nucleic acid capable of hybridising to a nucleic acid encoding an orthologue, paralogue or homologue of any of the nucleic acid sequences given in Table G1 of Example 62.

Hybridising sequences useful in the methods of the application encode a SPL11 polypeptide as defined herein, and have substantially the same biological activity as the amino acid sequences given in Table G1 of Example 62. Preferably, the hybridising sequence is capable of hybridising to any one of the nucleic acids given in Table G1 of Example 62, or to a portion of any of these sequences, a portion being as defined above, or wherein the hybridising sequence is capable of hybridising to a nucleic acid encoding an orthologue or paralogue of any one of the amino acid sequences given in Table G1 of Example 62. Most preferably, the hybridising sequence is capable of hybridising to a nucleic acid as represented by SEQ ID NO: 427 or to a portion thereof.

Preferably, the hybridising sequence encodes an amino acid sequence which when used in the construction of a phylogenetic tree, such as the one depicted in Figure 29, clusters with the group of SPL11 polypeptides comprising the amino acid sequence represented by SEQ ID NO: 428 (AtSPL11) rather than with any other group.

Another nucleic acid variant useful in the methods of the application is a splice variant encoding a SPL11 polypeptide as defined hereinabove, a splice variant being as defined herein.

According to the present application there is provided a method for enhancing yield-related traits in plants, comprising introducing and expressing in a plant a splice variant of any one of the nucleic acid sequences given in Table G1 of Example 62, or a splice variant of a nucleic acid encoding an orthologue, paralogue or homologue of any of the amino acid sequences given in Table G1 of Example 62. Examples of spliced variants of the gene encoding SEQ ID NO: 428 are represented by SEQ ID NO: 427; SEQ ID NO: 429 and SEQ ID NO: 430.

Preferred splice variants are splice variants of a nucleic acid represented by SEQ ID NO: 427, or a splice variant of a nucleic acid encoding an orthologue or paralogue of SEQ ID NO: 428. Preferably, the amino acid sequence encoded by the splice variant, when used in the construction of a phylogenetic tree, such as the one depicted in Figure 29, clusters with the group of SPL11 polypeptides comprising the amino acid sequence represented by SEQ ID NO: 428 (AtSPL11) rather than with any other group.

Another nucleic acid variant useful in performing the methods of the application is an allelic variant of a nucleic acid encoding a SPL11 polypeptide as defined hereinabove, an allelic variant being as defined herein.

According to the present application there is provided a method for enhancing yield-related traits in plants, comprising introducing and expressing in a plant an allelic variant of any one of the nucleic acids given in Table G1 of Example 62, or comprising introducing and expressing in a plant an allelic variant of a nucleic acid encoding an orthologue, paralogue or homologue of any of the amino acid sequences given in Table G1 of Example 62.

The allelic variants useful in the methods of the present application have substantially the same biological activity as the SPL11 polypeptide of SEQ ID NO: 428 and any of the amino acids depicted in Table G1 of Example 62. Allelic variants exist in nature, and encompassed within the methods of the present invention is the use of these natural alleles. Preferably, the allelic variant is an allelic variant of SEQ ID NO: 427 or an allelic variant of a nucleic acid encoding an orthologue or paralogue of SEQ ID NO: 428. Preferably, the amino acid sequence encoded by the allelic variant, when used in the construction of a phylogenetic tree, such as the one depicted in Figure 29, clusters with the SPL11 polypeptides comprising the amino acid sequence represented by SEQ ID NO: 428 (AtSPL11) rather than with any other group.

Gene shuffling or directed evolution may also be used to generate variants of nucleic acids encoding SPL11 polypeptides as defined above; the term "gene shuffling" being as defined herein.

According to the present application there is provided a method for enhancing yield-related traits in plants, comprising introducing and expressing in a plant a variant of any one of the nucleic acid sequences given in Table G1 of Example 62, or comprising introducing and expressing in a plant a variant of a nucleic acid encoding an orthologue, paralogue or homologue of any of the amino acid sequences given in Table G1 of Example 62, which variant nucleic acid is obtained by gene shuffling.

Preferably, the amino acid sequence encoded by the variant nucleic acid obtained by gene shuffling, when used in the construction of a phylogenetic tree such as the one depicted in Figure 29, clusters with the group of SPL11 polypeptides comprising the amino acid sequence represented by SEQ ID NO: 428 (AtSPL11) rather than with any other group.

Furthermore, nucleic acid variants may also be obtained by site-directed mutagenesis. Several methods are available to achieve site-directed mutagenesis, the most common being PCR based methods (Current Protocols in Molecular Biology. Wiley Eds.).

Nucleic acids encoding SPL11 polypeptides may be derived from any natural or artificial source. The nucleic acid may be modified from its native form in composition and/or genomic environment through deliberate human manipulation. Preferably the SPL11 polypeptide-encoding nucleic acid is from a plant, further preferably from a dicotyledonous plant, more preferably from the family Brassicaceae, most preferably the nucleic acid is from *Arabidopsis thaliana.*

Performance of the methods of the application gives plants having enhanced yield-related traits.

In particular performance of the methods of the invention gives plants having increased seed yield relative to control plants. The terms "yield" and "seed yield" are described in more detail in the "definitions" section herein.

Reference herein to enhanced yield-related traits is taken to mean an increase in biomass (weight) of one or more parts of a plant, which may include aboveground (harvestable) parts and/or (harvestable) parts below ground. In particular, such harvestable parts are seeds, and performance of the methods of the invention results in plants having increased yield relative to the yield of control plants.

Taking corn as an example, a yield increase may be manifested as one or more of the following: increase in the number of plants established per hectare or acre, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per hectare or acre, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

The present invention provides a method for increasing seed yield of plants, relative to control plants, which method comprises increasing expression, in a plant of a nucleic acid encoding a SPL11 polypeptide as defined herein.

Since the transgenic plants according to the present invention have increased yield, it is likely that these plants exhibit an increased growth rate (during at least part of their life cycle), relative to the growth rate of control plants at a corresponding stage in their life cycle.

The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per acre (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

According to a preferred feature of the present invention, performance of the methods of the invention gives plants having an increased growth rate relative to control plants. Therefore, according to the present invention, there is provided a method for increasing the growth rate of plants, which method comprises modulating expression, preferably increasing expression, in a plant of a nucleic acid encoding a SPL11 polypeptide as defined herein.

An increase in seed yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11 % or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi and insects.

In particular, the methods of the present invention may be performed under non-stress conditions or under conditions of mild drought to give plants having increased yield relative to control plants. As reported in Wang et al.

(Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location. Plants with optimal growth conditions, (grown under non-stress conditions) typically yield in increasing order of preference at least 90%, 87%, 85%, 83%, 80%, 77% or 75% of the average production of such plant in a given environment. Average production may be calculated on harvest and/or season basis. Persons skilled in the art are aware of average yield productions of a crop.

Performance of the methods of the invention gives plants grown under non-stress conditions or under mild drought conditions increased yield relative to control plants grown under comparable conditions. Therefore, according to the present invention, there is provided a method for increasing seed yield in plants grown under non-stress conditions or under mild drought conditions, which method comprises increasing expression in a plant of a nucleic acid encoding a SPL11 polypeptide.

Performance of the methods of the invention gives plants grown under conditions of nutrient deficiency, particularly under conditions of nitrogen deficiency, increased yield relative to control plants grown under comparable conditions. Therefore, according to the present invention, there is provided a method for increasing seed yield in plants grown under conditions of nutrient deficiency, which method comprises increasing expression in a plant of a nucleic acid encoding a SPL11 polypeptide. Nutrient deficiency may result from a lack or excess of nutrients such as nitrogen, phosphates and other phosphorous-containing compounds, potassium, calcium, cadmium, magnesium, manganese, iron and boron, amongst others.

The present invention encompasses plants or parts thereof (including seeds) obtainable by the methods according to the present invention. The plants or parts thereof comprise a nucleic acid transgene encoding a SPL11 polypeptide as defined above.

The invention also provides genetic constructs and vectors to facilitate introduction and/or expression in plants of nucleic acids encoding SPL11 polypeptides. The gene constructs may be inserted into vectors, which may be commercially available, suitable for transforming into plants and suitable for expression of the gene of interest in the transformed cells. The invention also provides use of a gene construct as defined herein in the methods of the invention.

More specifically, the present invention provides a construct comprising:

(a) a nucleic acid encoding a SPL11 polypeptide as defined above;
(b) one or more control sequences capable of driving expression of the nucleic acid sequence of (a); and optionally
(c) a transcription termination sequence.

Preferably, the nucleic acid encoding a SPL11 polypeptide is as defined above. The term "control sequence" and "termination sequence" are as defined herein.

Plants are transformed with a vector comprising any of the nucleic acids described above. The skilled artisan is well aware of the genetic elements that must be present on the vector in order to successfully transform, select and propagate host cells containing the sequence of interest. The sequence of interest is operably linked to one or more control sequences (at least to a promoter).

Advantageously, any type of promoter, whether natural or synthetic, may be used to drive expression of the nucleic acid sequence. A constitutive promoter is particularly useful in the methods. See the "Definitions" section herein for definitions of the various promoter types.

It should be clear that the applicability of the present invention is not restricted to the SPL11 polypeptide-encoding nucleic acid represented by SEQ ID NO: 427, nor is the applicability of the invention restricted to expression of a SPL11 polypeptide-encoding nucleic acid when driven by a constitutive promoter.

The constitutive promoter is preferably a GOS2 promoter, preferably a GOS2 promoter from rice. Further preferably the constitutive promoter is represented by a nucleic acid sequence substantially similar to SEQ ID NO: 476, most preferably the constitutive promoter is as represented by SEQ ID NO: 476. See the "Definitions" section herein for further examples of constitutive promoters.

In an alternative embodiment, a seed specific promoter is used. The seed specific promoter is preferably ABA (abcisic acid) inducible, it is preferably the WSI18 promoter, preferably the WSI18 from rice. Further preferably the constitutive promoter is represented by a nucleic acid sequence substantially similar to SEQ ID NO: 477. See the "Definitions" section herein for further examples of seed specific promoters.

It should be clear that the promoters useful in the methods of the invention are not limited to those specified in the abovementioned embodiments.

Optionally, one or more terminator sequences may be used in the construct introduced into a plant. Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die). The marker genes may be removed or excised from the transgenic cell once they are no longer needed. Techniques for marker gene removal are known in the art, useful techniques are described above in the definitions section.

The invention also provides a method for the production of transgenic plants having enhanced seed yield relative to control plants, comprising introduction and expression in a plant of any nucleic acid encoding a SPL11 polypeptide as defined hereinabove.

More specifically, the present invention provides a method for the production of transgenic plants having increased seed yield, which method comprises:

(i) introducing and expressing in a plant or plant cell a SPL11 polypeptide-encoding nucleic acid; and
(ii) cultivating the plant cell under conditions promoting plant growth and development.

The nucleic acid of (i) may be any of the nucleic acids capable of encoding a SPL11 polypeptide as defined herein.

The nucleic acid may be introduced directly into a plant cell or into the plant itself (including introduction into a tissue, organ or any other part of a plant). According to a preferred feature of the present invention, the nucleic acid is preferably introduced into a plant by transformation. The term "transformation" is described in more detail in the "definitions" section herein.

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

The application also includes host cells containing an isolated nucleic acid encoding a SPL11 polypeptide as defined hereinabove. Preferred host cells according to the invention are plant cells. Host plants for the nucleic acids or the vector used in the method according to the invention, the expression cassette or construct or vector are, in principle, advantageously all plants, which are capable of synthesizing the polypeptides used in the inventive method.

The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum and oats.

The invention also extends to harvestable parts of a plant such as, but not limited to seeds, leaves, fruits, flowers, stems, roots, rhizomes, tubers and bulbs. The invention furthermore relates to products derived, preferably directly derived, from a harvestable part of such a plant, such as dry pellets or powders, oil, fat and fatty acids, starch or proteins.

According to a preferred feature of the invention, the modulated expression is increased expression. Methods for increasing expression of nucleic acids or genes, or gene products, are well documented in the art and examples are provided in the definitions section.

As mentioned above, a preferred method for increasing expression of a nucleic acid encoding a SPL11 polypeptide is by introducing and expressing in a plant a nucleic acid encoding a SPL11 polypeptide; however the effects of performing the method, i.e. enhancing yield-related traits may also be achieved using other well-known techniques, including but not limited to T-DNA activation tagging, TILLING, homologous recombination. A description of these techniques is provided in the definitions section.

The present invention also encompasses use of nucleic acids encoding SPL11 polypeptides as described herein and use of these SPL11 polypeptides in enhancing any of the aforementioned seed yield-related traits in plants.

Nucleic acids encoding SPL11 polypeptides described herein, or the SPL11 polypeptides themselves, may find use in breeding programmes in which a DNA marker is identified, which may be genetically linked to a SPL11 polypeptide-encoding gene. The nucleic acids/genes, or the SPL11 polypeptides themselves may be used to define a molecular marker. This DNA or protein marker may then be used in breeding programmes to select plants having enhanced yield-related traits as defined hereinabove in the methods of the invention.

Allelic variants of a SPL11 polypeptide-encoding nucleic acid/gene may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Nucleic acids encoding SPL11 polypeptides may also be used as probes for genetically and physically mapping the genes that they are a part of, and as markers for traits linked to those genes. Such information may be useful in plant breeding in order to develop lines with desired phenotypes. Such use of SPL11 polypeptide-encoding nucleic acids requires only a nucleic acid sequence of at least 15 nucleotides in length. The SPL11 polypeptide-encoding nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the SPL11-encoding nucleic acids. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the SPL11 polypeptide-encoding nucleic acid in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

Moreover the nucleic acid probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having enhanced seed yield as described hereinbefore. These traits may also be combined with other economically advantageous traits, such as further yield-enhancing traits, tolerance to other abiotic and biotic stresses, traits modifying various architectural features and/or biochemical and/or physiological features.

The present application will now be described in reference to the following items:

1. A method for enhancing yield-related traits in plants relative to control plants, comprising increasing expression in a plant of a nucleic acid encoding a SPL11 polypeptide, wherein said SPL11 comprises a SBP domain having in increasing order of preference at least 70%, 75%, 80%, 90%, 95%, 97% or more sequence identity to any one of SEQ ID NO: 456 to SEQ ID NO: 468 and SEQ ID NO: 478, wherein said increased seed yield preferably comprises increased total seed weight, number of filled seeds, number of seeds or florets per panicle, thousand-kernel weight, seed filling rate and/or harvest index relative to control plants

2. Method according to item 1 wherein said SPL11 polypeptide in addition to the SBP domain comprises any one or more of the following conserved motifs:

   (i) Motif 1 as represented by SEQ ID NO: 469 wherein any conservative amino acid substitution and/or 1 or 2 non conservative substitution are allowed;
   (ii) Motif 2 as represented by SEQ ID NO: 470 wherein any change is allowed, provided that at least 4 amino acids have a polar side chain, preferably serine or threonine, and provided that the domain is located at the N-terminal end of the SBP domain;
   (iii) Motif 3 as represented by SEQ ID: 471 wherein 1 or 2 mismatches are allowed;
   (iv) Motif 4 as represented by SEQ ID: 472 wherein 1, 2 or 3 mismatches are allowed.

3. Method according to item 1 or 2, wherein said modulated expression is effected by introducing and expressing in a plant a nucleic acid encoding a SPL11 polypeptide as defined in item 1 or 2.

4. Method according to any of items 1 to 3, wherein said modulated expression is increased expression.

5. Method according to any of items 1 to 4, wherein said nucleic acid encoding a SPL11 polypeptide encodes any one of the proteins listed in Table G1 or is a portion of such a nucleic acid, or a nucleic acid capable of hybridising with such a nucleic acid.

6. Method according to any of items 1 to 5, wherein said nucleic acid sequence encodes an orthologue or paralogue of any of the proteins given in Table G1.

7. Method according to item 6, wherein said nucleic acid encodes SEQ ID NO: 428.

8. Method according to any of items 1 to 7, wherein said enhanced yield-related traits comprise increased yield, preferably increased total seed weight, number of filled seeds, number of seeds or florets per panicle, thousand-kernel weight, seed filling rate, and/or harvest index relative to control plants.

9. Method according to any of items 1 to 8, wherein said enhanced yield-related traits comprise plant (seedling) early vigour relative to control plants.

10. Method according to any one of items 1 to 8, wherein said enhanced yield-related traits are obtained under non-stress conditions.

11. Method according to any one of items 1 to 8, wherein said enhanced yield-related traits are obtained under mild drought stress growth conditions.

12. Method according to any one of items 1 to 8, wherein said enhanced yield-related traits are obtained under growth conditions of nitrogen deficiency.

13. Method according to any one of items 1 to 12, wherein said nucleic acid is operably linked to a constitutive promoter, preferably to a GOS2 promoter, most preferably to a GOS2 promoter from rice.

14. Method according to any one of items 1 to 12, wherein said nucleic acid is operably linked to a seed specific promoter, preferably to a WSI18 promoter, most preferably to a WSI18 promoter from rice.

15. Method according to any of items 1 to 14, wherein said nucleic acid encoding a SPL11 polypeptide is of plant origin, preferably from a dicotyledonous plant, further preferably from the family Brassicaceae, more preferably from the genus *Arabidopsis,* most preferably from *Arabidopsis thaliana.*

16. Plant or part thereof, including seeds, obtainable by a method according to any one of items 1 to 15, wherein said plant or part thereof comprises a recombinant nucleic acid encoding a SPL11 polypeptide.

17. An isolated nucleic acid molecule comprising:

(i) a nucleic acid represented by SEQ ID NO: 448;
(ii) the complement of a nucleic acid represented by SEQ ID NO: 448;
(iii) a nucleic acid encoding a SPL11 polypeptide having, in increasing order of preference, at least 70%, 75%, 80%, 85%, 90%, 95%, 96%, 97%, 98%, 99% or more sequence identity to the amino acid sequence represented by SEQ ID NO: 449, and having in increasing order of preference at least 70%, 75%, 80%, 85%, 90%, 95%, 96%, 97%, 98%, 99% or more sequence identity to SEQ ID NO: 465: SYCQVEGCRTDLSSAKDYHRKHRVCEPHSKAPKVVVAGLERRFCQQCSRFHG LAEFDQKKKSCRRRLNDHNARRRKPQPEAL;
(iv) a nucleic acid hybridising under stringent conditions to SEQ ID NO: 448.

18. An isolated polypeptide comprising:

(i) an amino acid sequence represented by SEQ ID NO: 449;
(ii) an amino acid sequence having, in increasing order of preference, at least 70%, 75%, 80%, 85%, 90%, 95%, 96%, 97%, 98%, 99% or more sequence identity to the amino acid sequence represented by SEQ ID NO: 449, and having in increasing order of preference at least 70%, 75%, 80%, 85%, 90%, 95%, 96%, 97%, 98%, 99% or more sequence identity to SEQ ID NO: 465: SYCQVEGCRTDLSSAKDYHRKHRVCEPHSKAPKVVVAGLERRFCQQCSRFHG LAEFDQKKKSCRRRLNDHNARRRKPQPEAL.
(iii) derivatives of any of the amino acid sequences given in (i) or (ii) above.

19. Construct comprising:

(i) nucleic acid encoding a SPL11 polypeptide;
(ii) one or more control sequences capable of driving expression of the nucleic acid sequence of (i); and optionally
(iii) a transcription termination sequence

20. Construct according to item 17 wherein said nucleic acid encoding a SPL11 polypeptide is a nucleic acid according to item 156.

21. Construct according to item 19 or 20 wherein one of said control sequences is a constitutive promoter, preferably a GOS2 promoter, most preferably a GOS2 promoter from rice.

22. Construct according to item 19 or 20 wherein one of said control sequences is a seed specific promoter, preferably a WSI18 promoter, most preferably a WSI18 promoter from rice.

23. Use of a construct according to items 19 to 22 in a method for making plants having increased yield related traits; particularly increased seed yield relative to control plants and/or plant or seedling early vigour.

24. Plant, plant part or plant cell transformed with a construct according to items 158 to 161.

25. Method for the production of a transgenic plant having increased yield related traits, particularly increased seed yield and/or plant or seedling early vigour relative to control plants, comprising:

(i) introducing and expressing in a plant a nucleic acid encoding a SPL11 polypeptide; and
(ii) cultivating the plant cell under conditions promoting plant growth and development.

26. Transgenic plant having increased yield, particularly increased plant seedling vigour and/or increased seed yield, relative to control plants, resulting from increased expression of a nucleic acid encoding a SPL11 polypeptide, or a transgenic plant cell derived from said transgenic plant.

27. Transgenic plant according to item 16, 24 or 26, or a transgenic plant cell derived thereof, wherein said plant is a dicot crop plant such as soybean, cotton or canola or a monocot crop plant or a cereal, such as rice, maize, wheat, barley, millet, rye, triticale, sorghum and oats.

28. Harvestable parts of a plant according to item 2, wherein said harvestable parts are preferably shoot biomass, flowers and/or seeds.

29. Products derived from a plant according to item 27 and/or from harvestable parts of a plant according to item 28.

30. Use of a nucleic acid encoding a SPL11 polypeptide in increasing yield, particularly in increasing seed yield and/or shoot biomass in plants, relative to control plants.

## Description of figures

### V. Squamosa promoter binding protein-like 11 (SPL11)

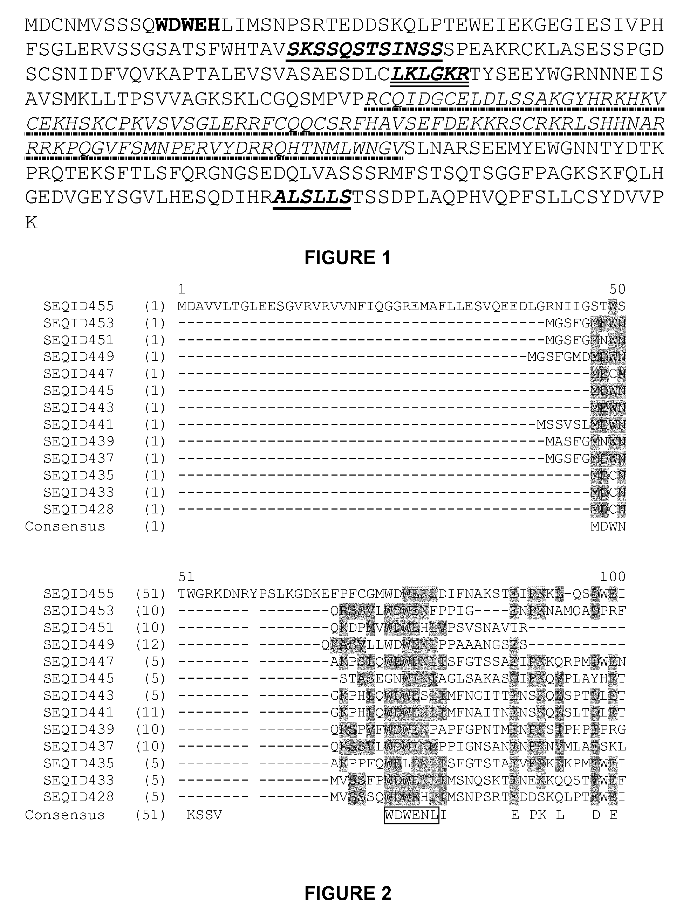

**Figure 1** represents the amino acid sequence and domain structure of SEQ ID NO: 428. Conserved motifs and domains are indicated. SBP domain is underlined by an interrupted line; Motif 1 is indicated in bold; Motif 2 is indicated in bold and underlined; Motif 3 is bold capital letters and underlined and Motif 4 is in bold and underlined with a double line.

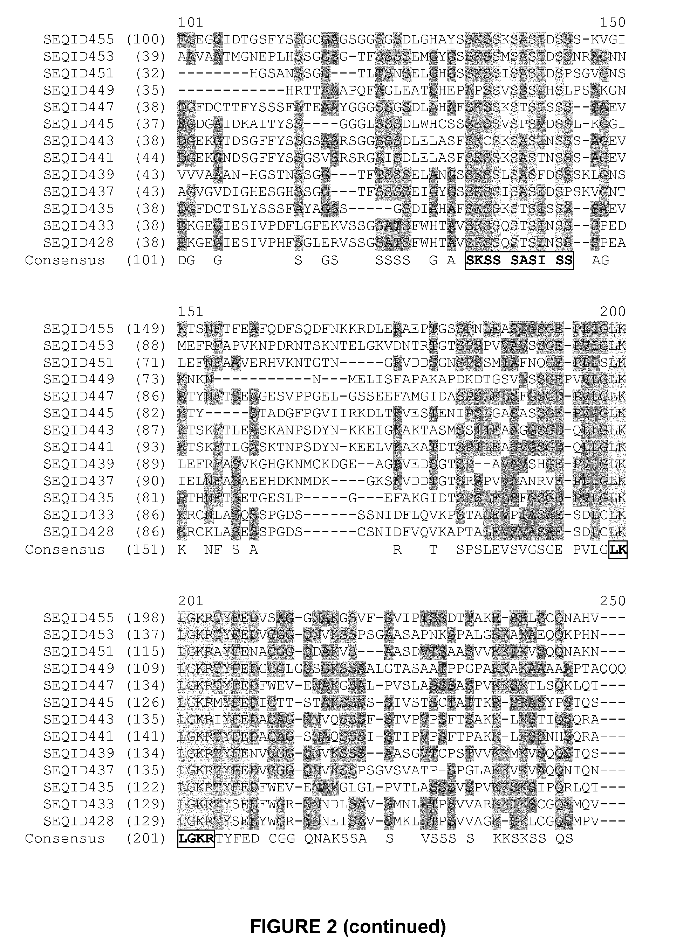

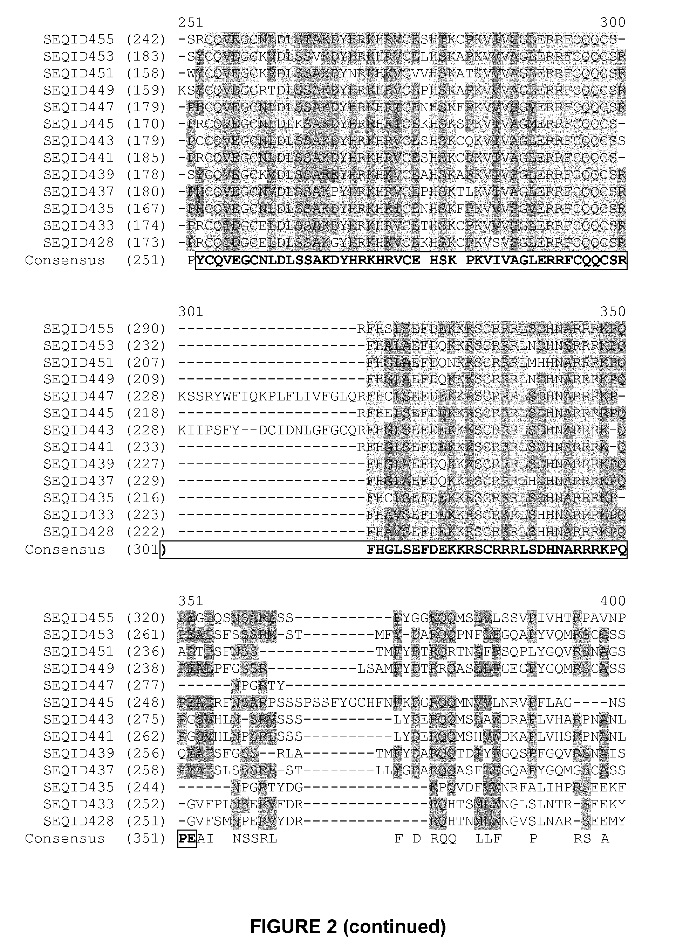

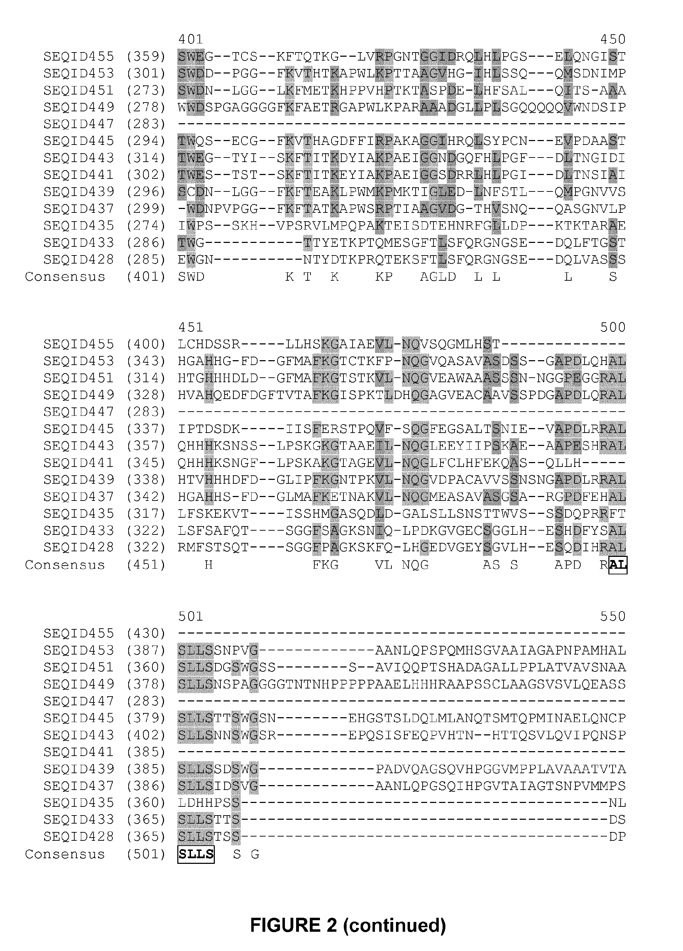

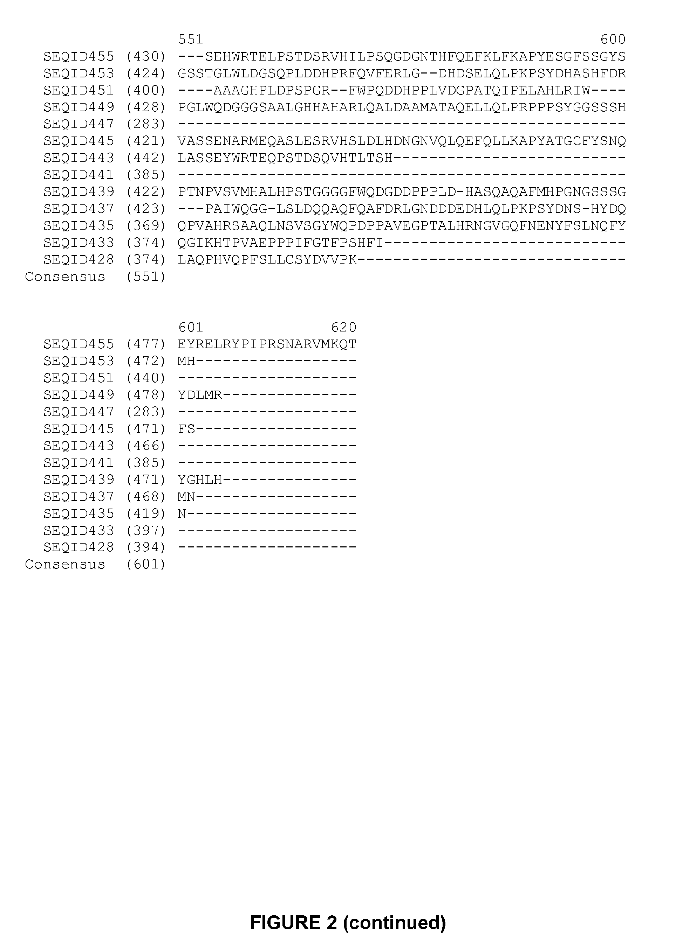

**Figure 2** represents a multiple sequence alignment of SPL11 polypeptides. Position of the conserved amino acid residues and domains corresponding to those described in Figure 27 is indicated. A consensus sequence representative of SPL11 polypeptides is given. In the consensus sequence the highly conserved amino acids are provided and empty blank spaces in between represent any amino acid.

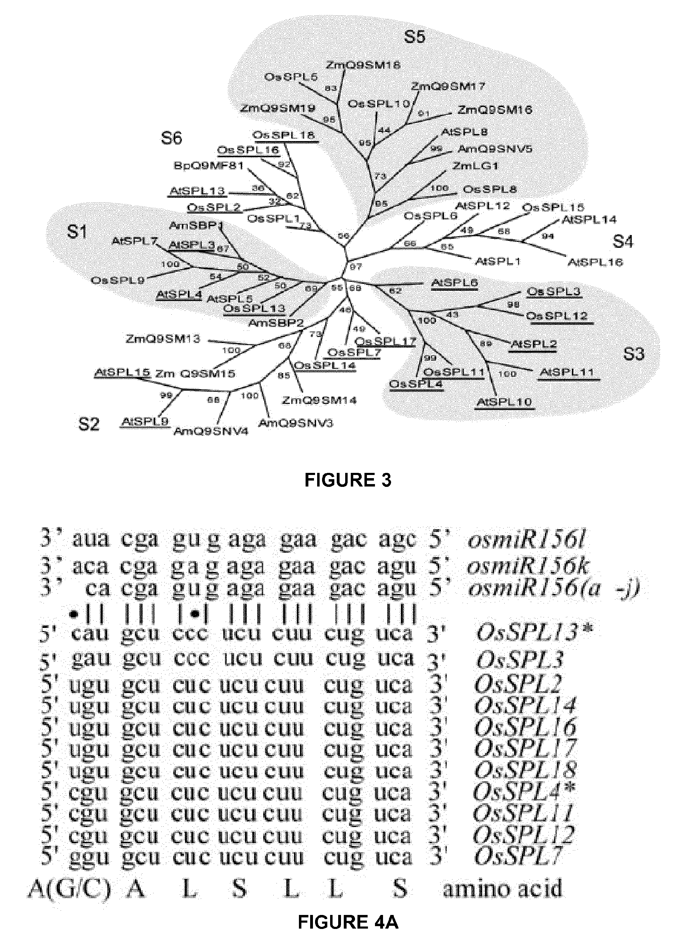

**Figure 3** shows a phylogenetic tree of SPL11 polypeptides. The phylogenetic tree is as present in Xie et al. Plant Physiology, 2006, Vol. 142, pp. 280-293, which is incorporated by reference herein as if fully set forth. SPL11 polypeptides cluster in the same group as AtSPL11 (identical to SEQ ID NO: 2) within the class named Class S3.

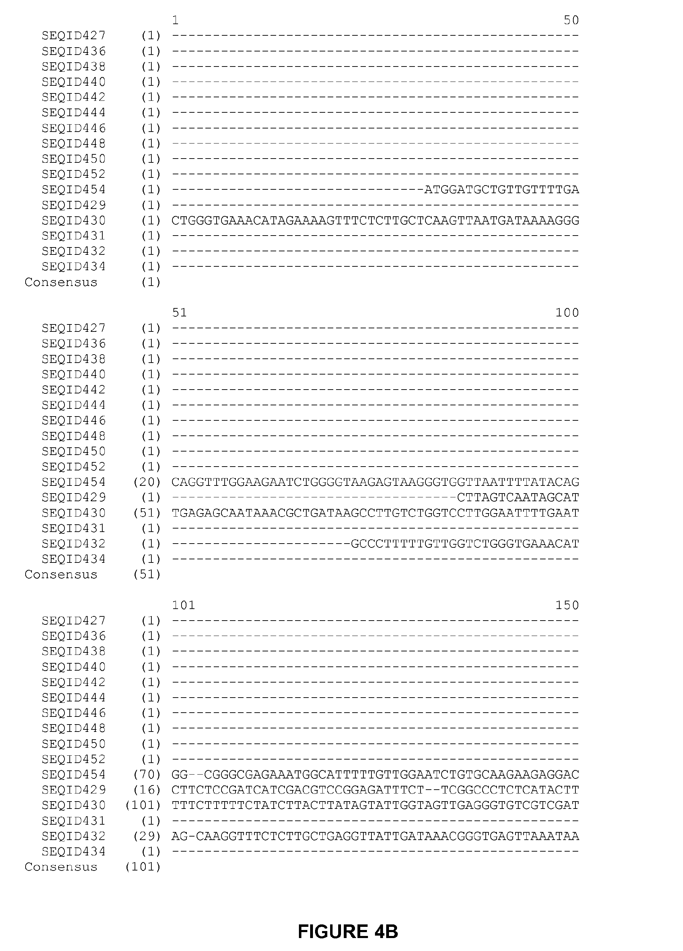

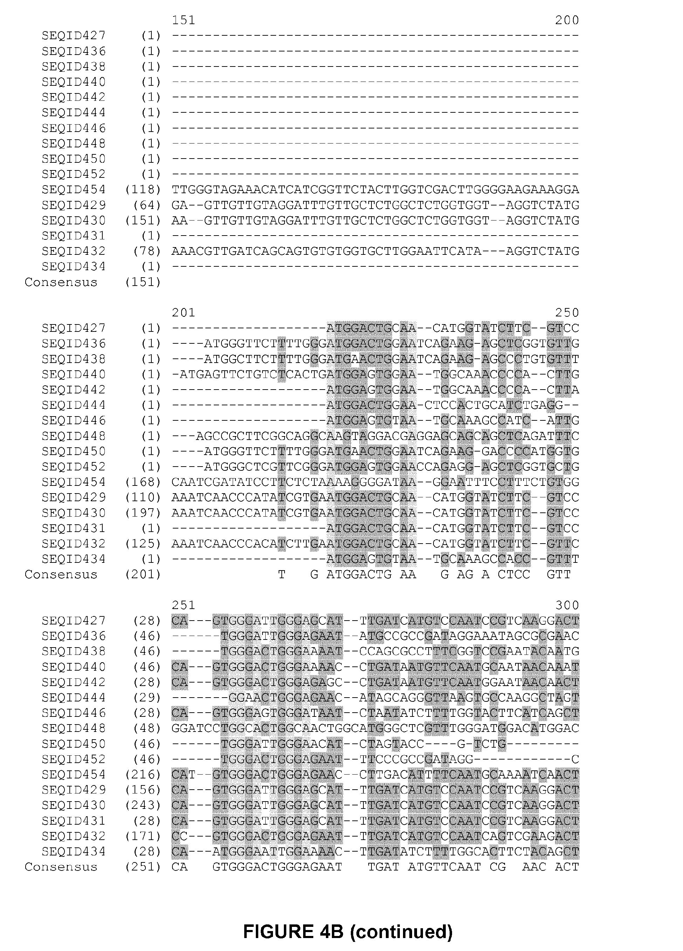

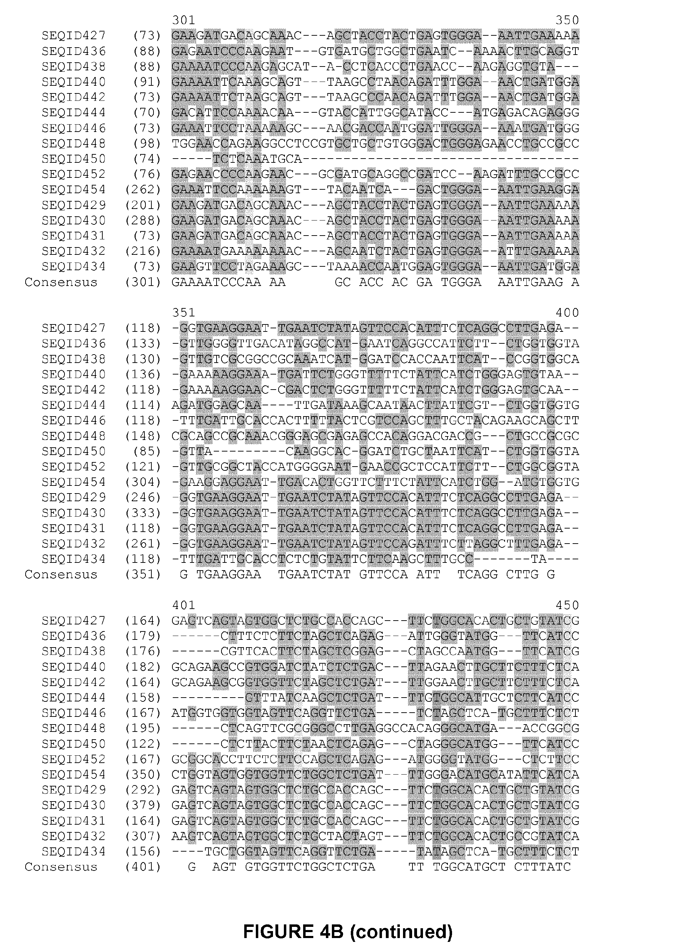

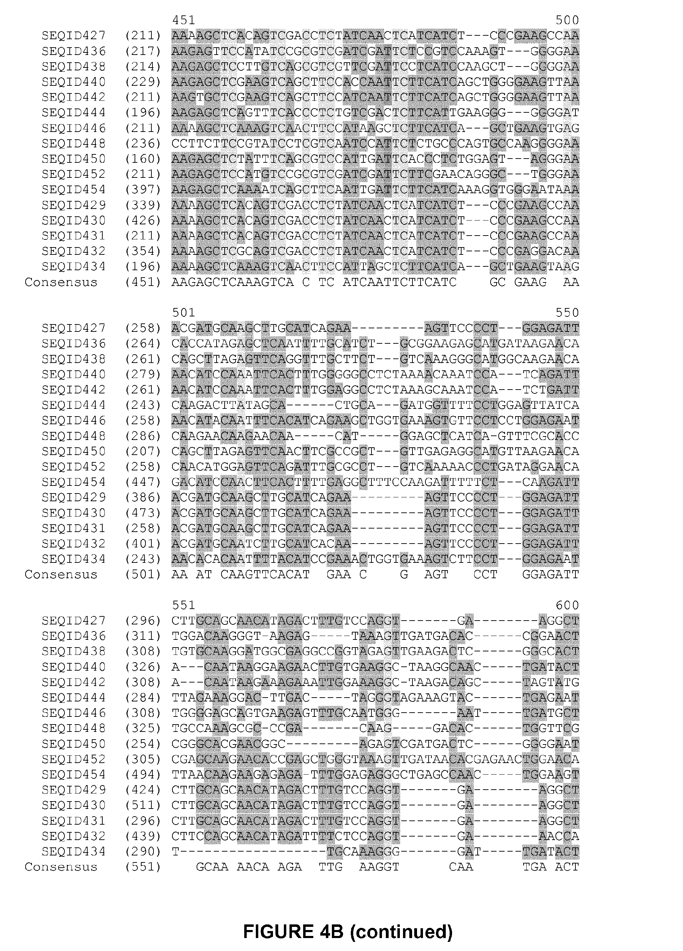

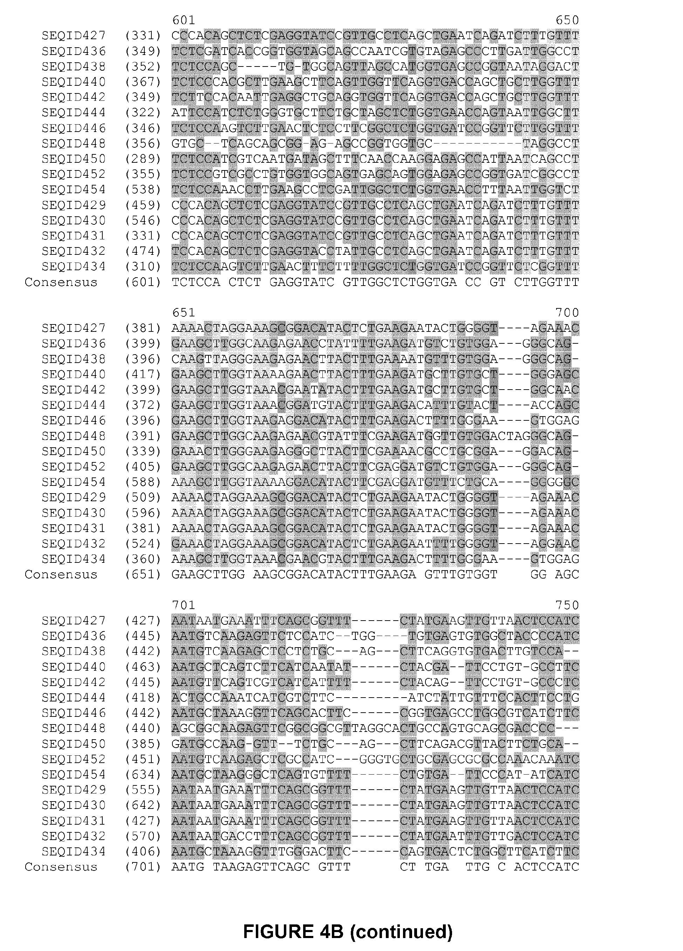

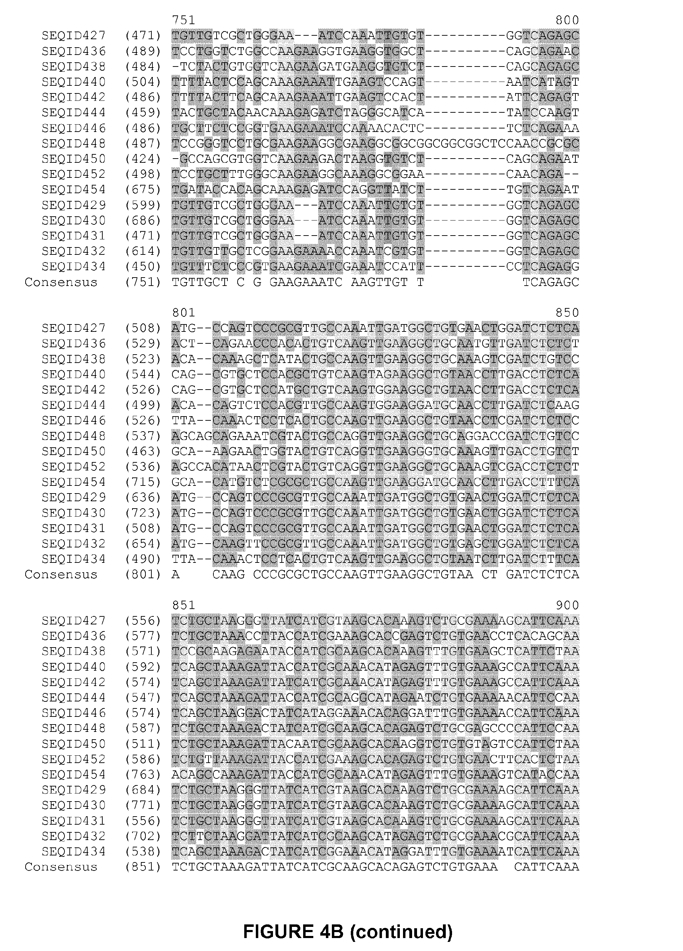

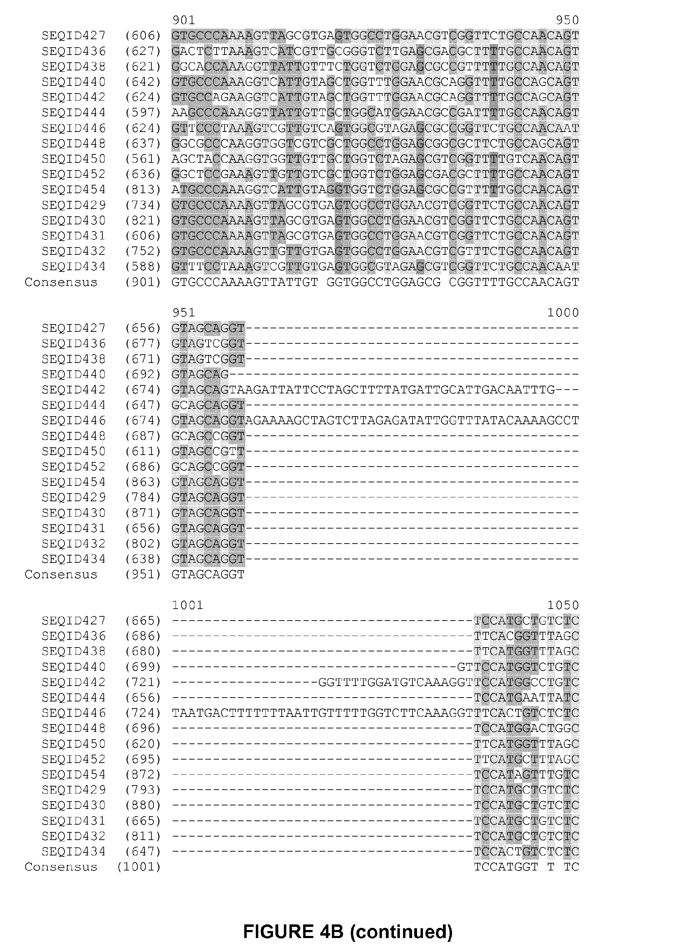

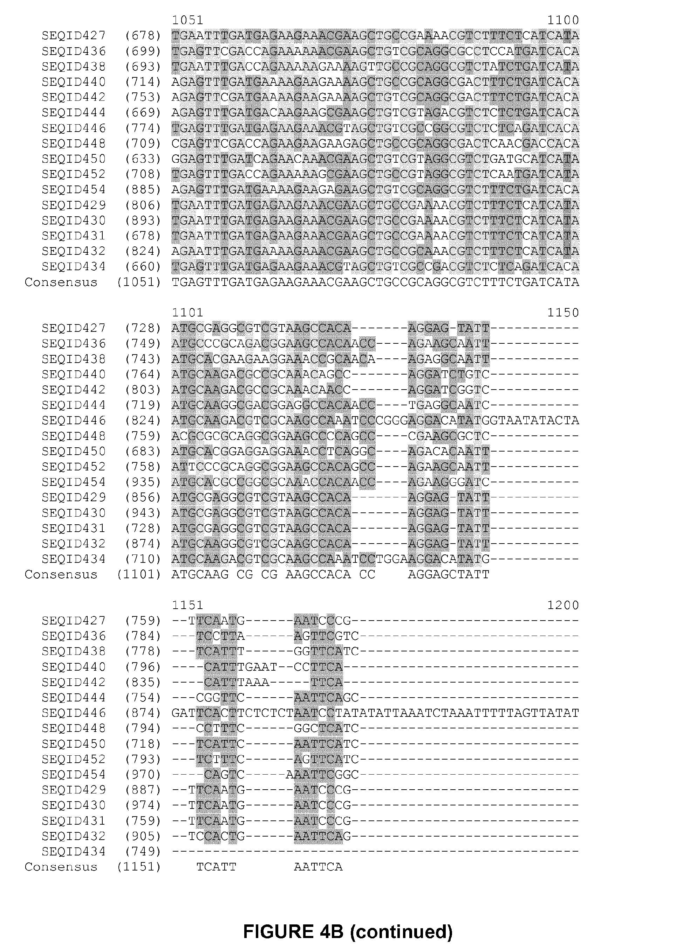

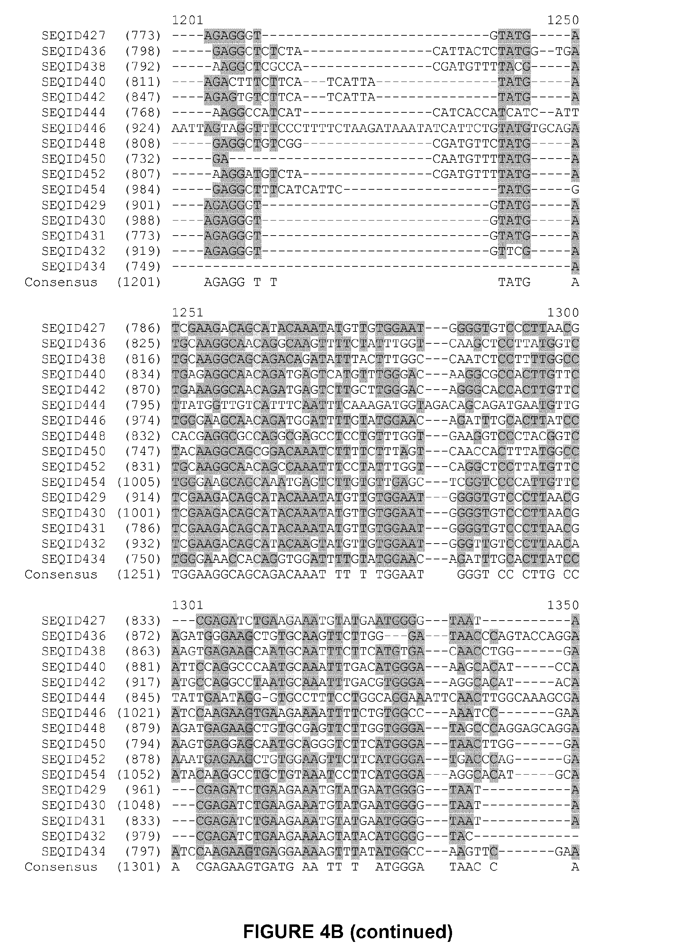

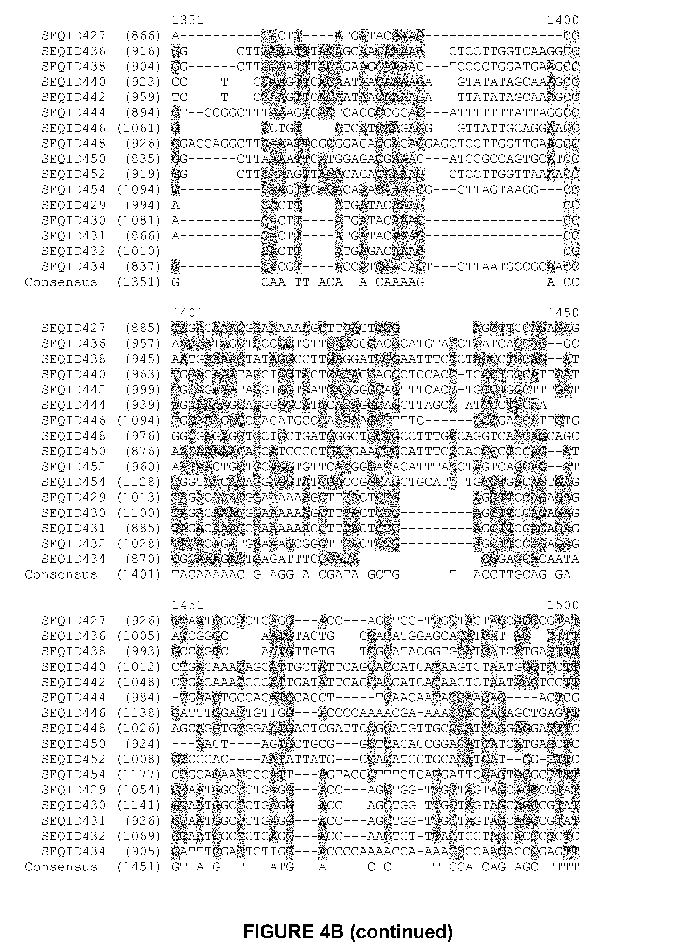

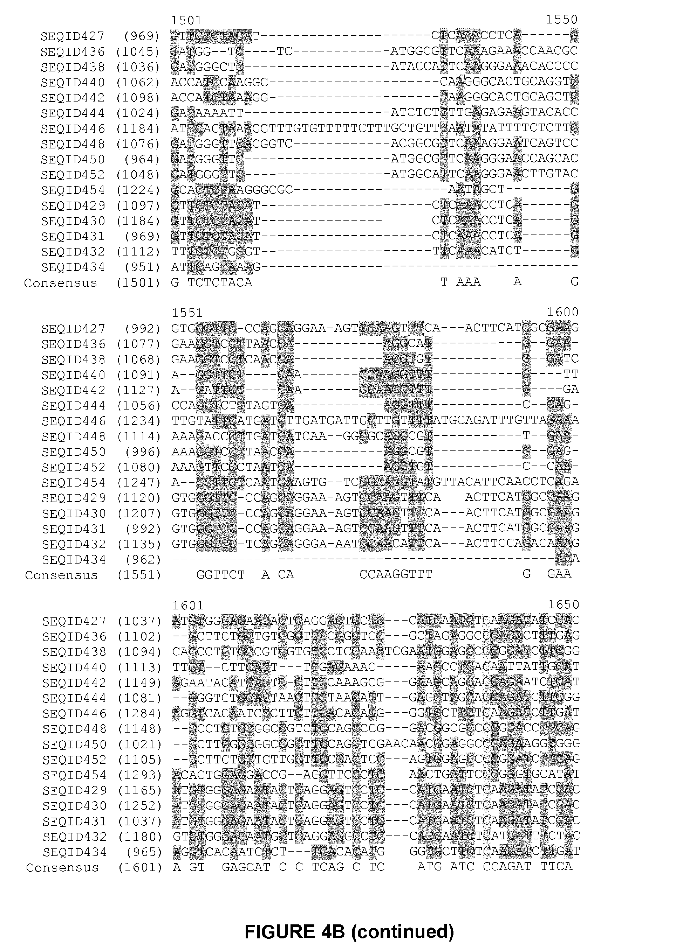

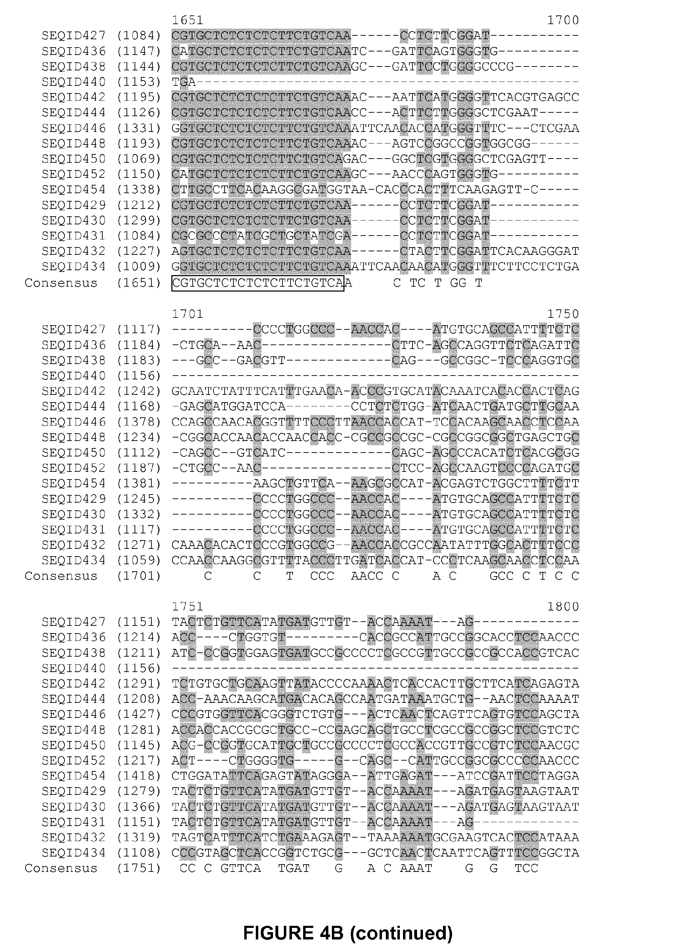

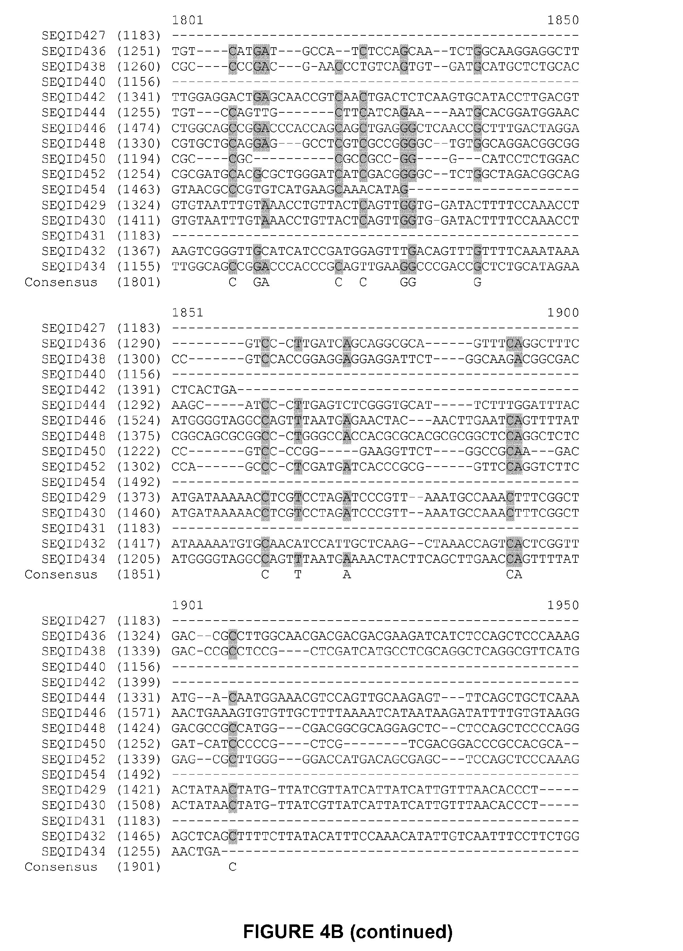

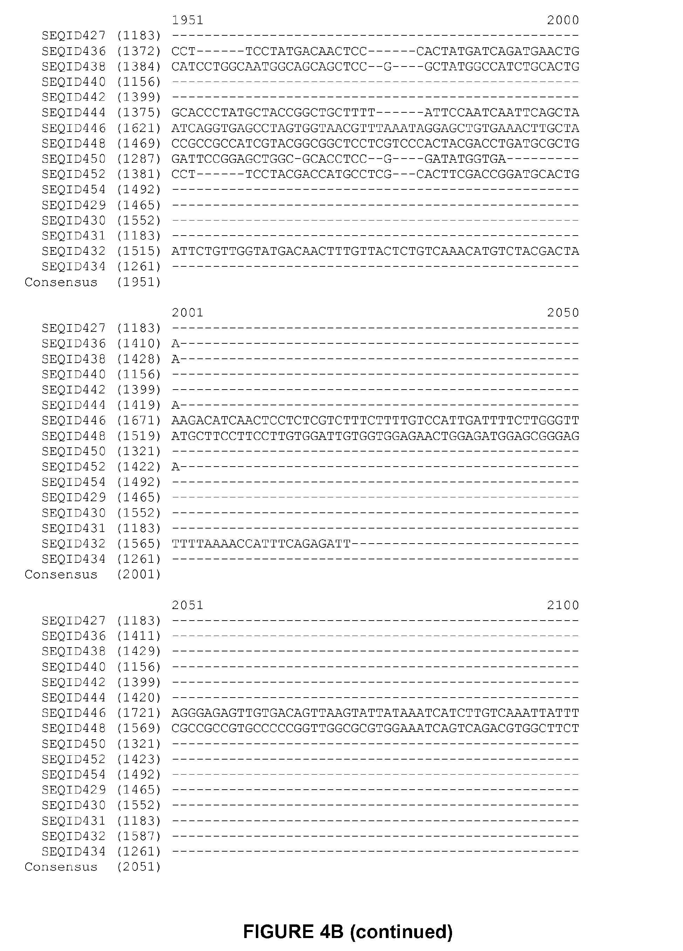

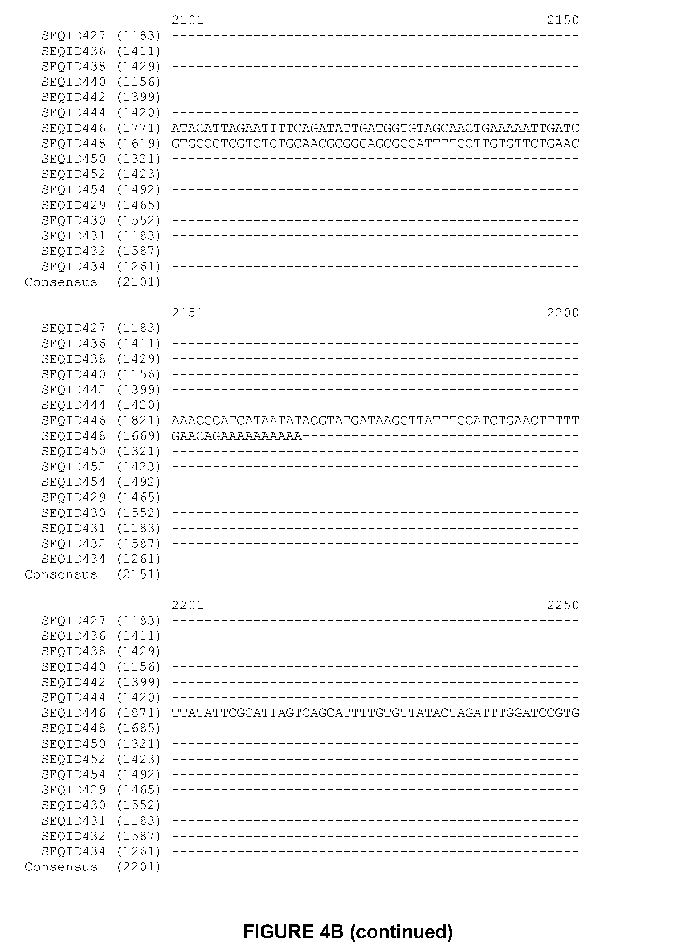

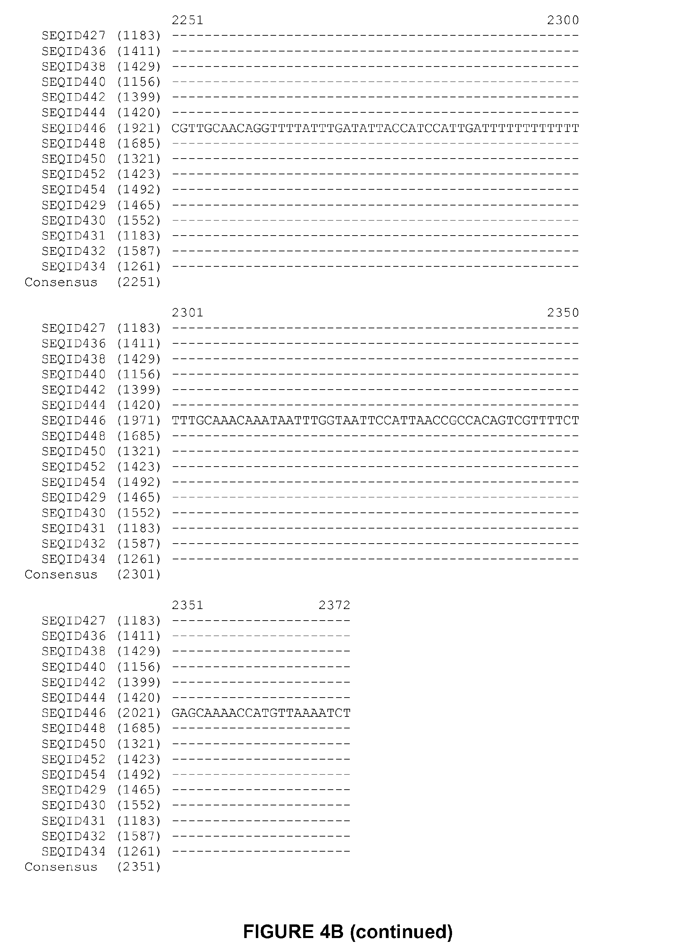

**Figure 4** provides a sequence analysis of rice miR156 genes (OsmiR156) and their targeted sequences in rice SPL polypeptides (Figure 30A) as well as a multiple alignment of SPL11 nucleic acids (Figure 30B). Fig. 30 A shows a sequence alignment of OsmiR156 mature sequences with complementary sequences of OsSPL genes. The conserved amino acid sequence encoded by the target sequences is shown at the bottom. The dots between miR156 and targeted OsSPL sequences indicate mismatches. Figure 4B shows a multiple alignment of SPL11 nucleic acids where in highly conserved nucleic acid residues are indicated in the consensus sequence. The position of the highly conserved miR156 target site is indicated in bold over the consensus sequence. SEQ ID NO: 431, SEQ ID NO: 440 and SEQ ID NO: 454 representatives of SPL11 nucleic acids which are miR156 insensitive do not have the conserved miR156 target site or show a great divergence in their sequence at that position.

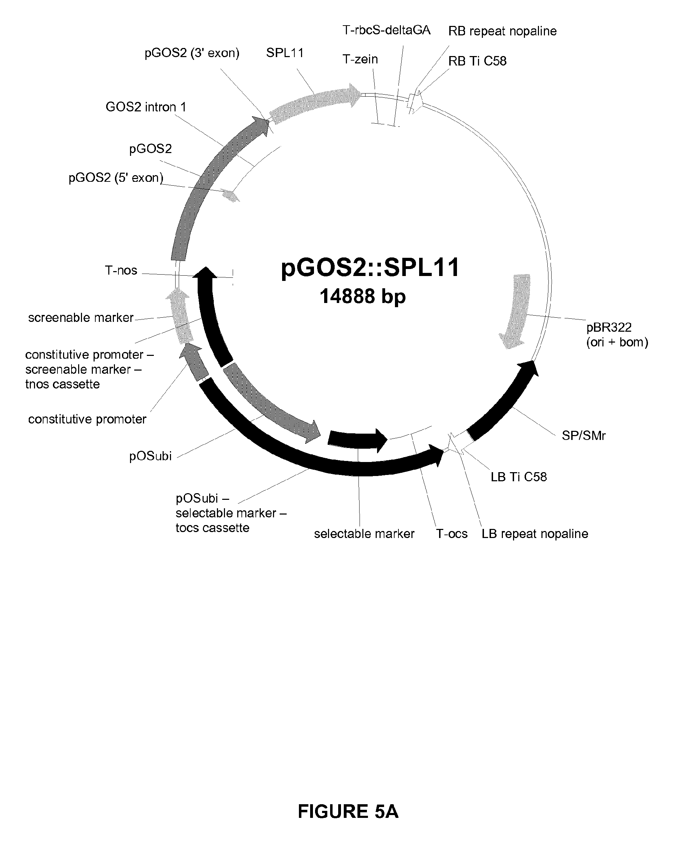

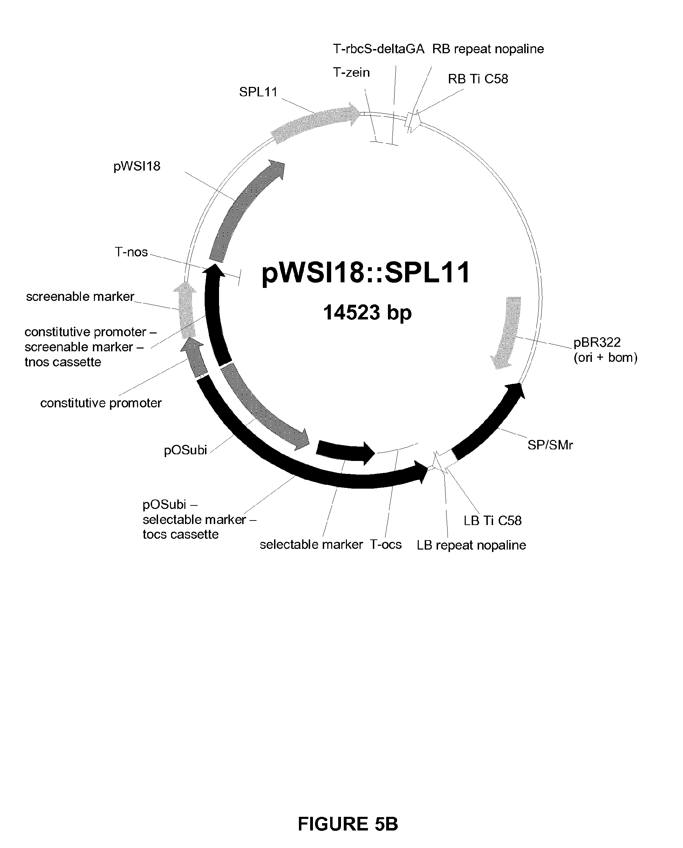

**Figure 5** represents the binary vector for increased expression in Oryza sativa of SEQ ID NO: 427 under the control of a rice GOS2 promoter (pGOS2) (Figure 31 A) or under a rice WSI 18 promoter (Figure 31B).

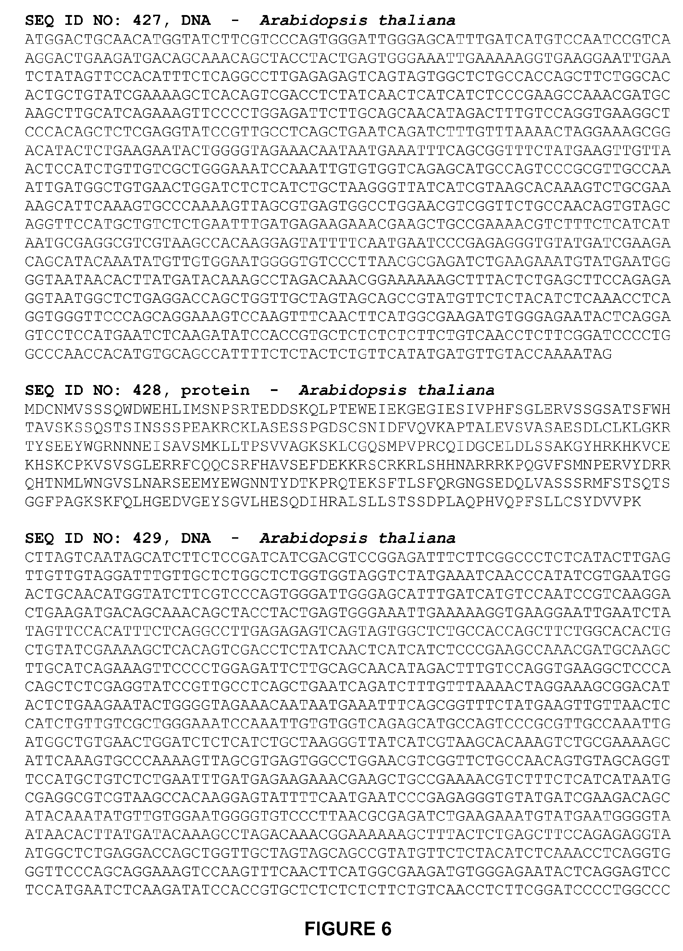

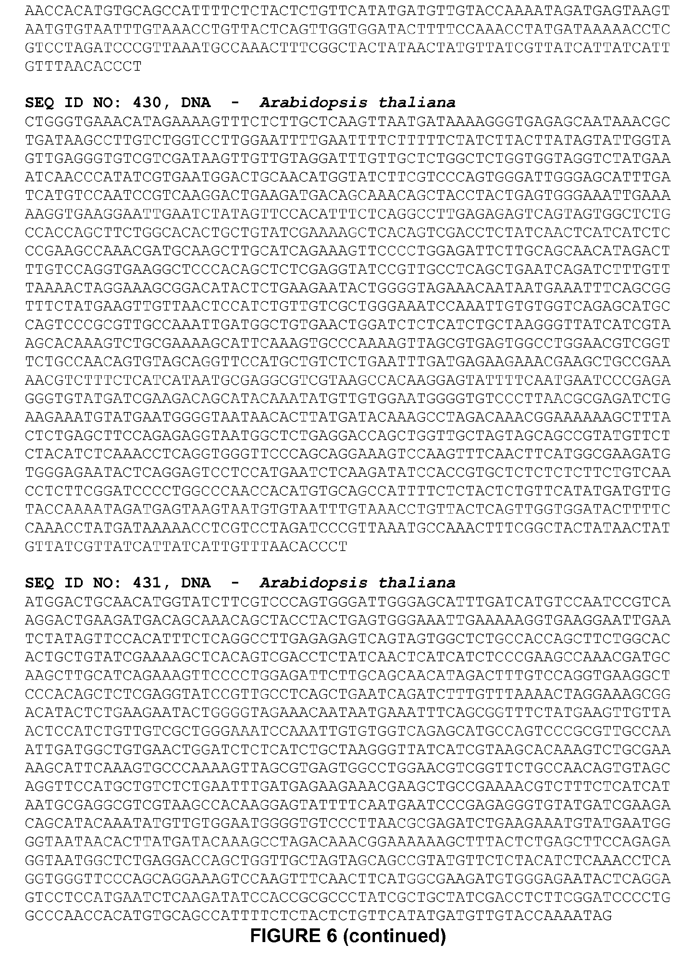

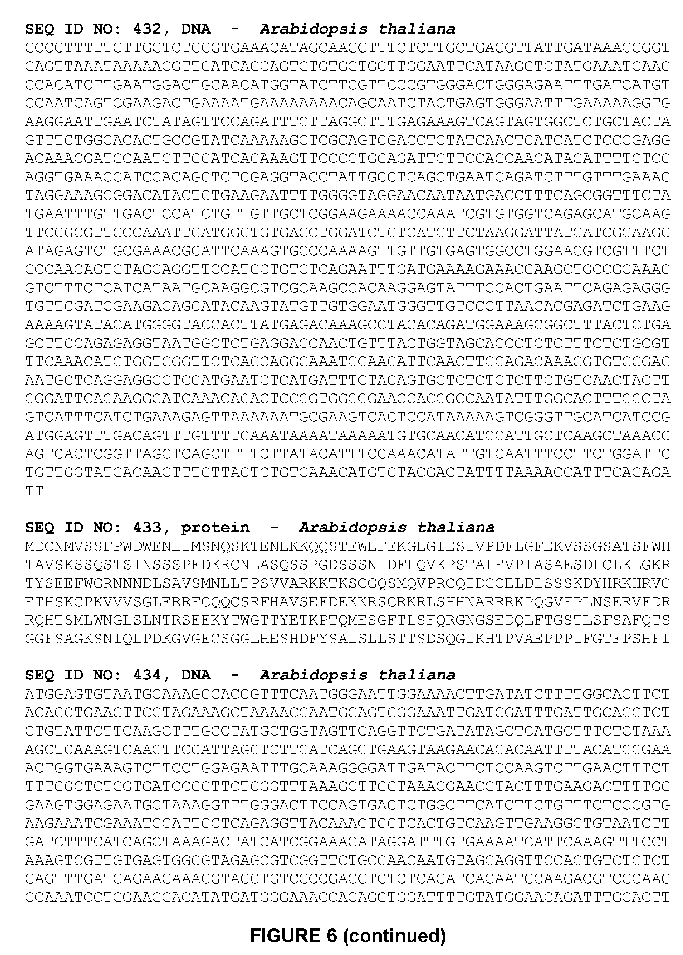

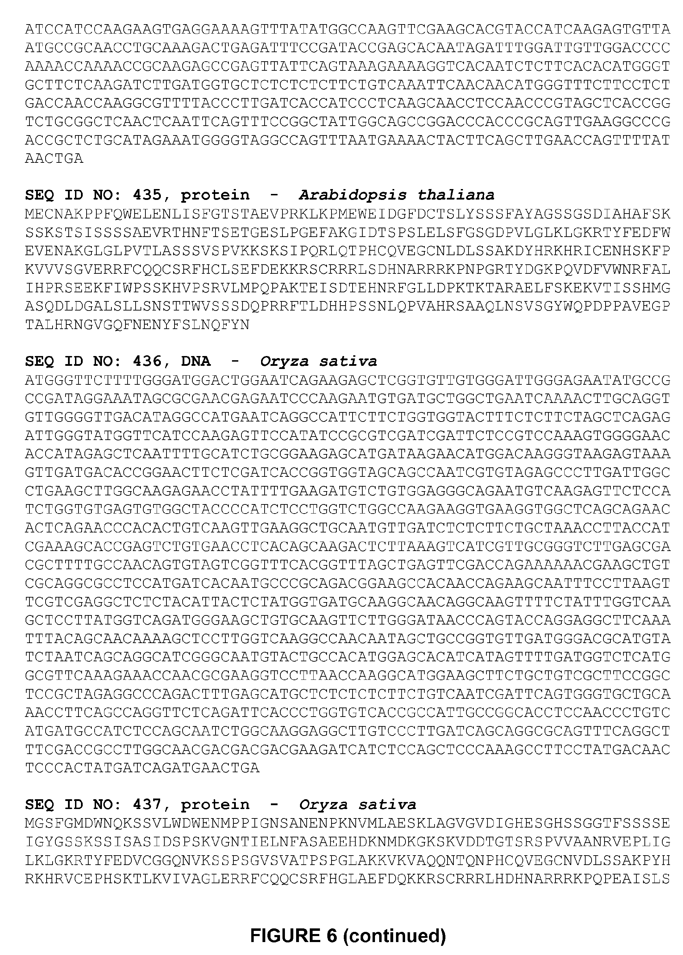

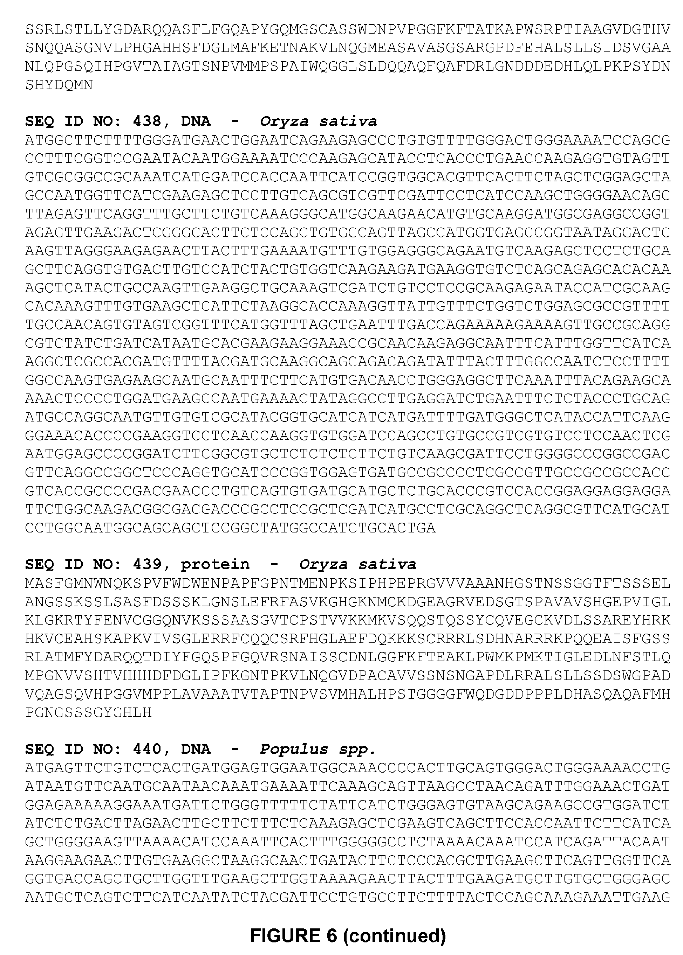

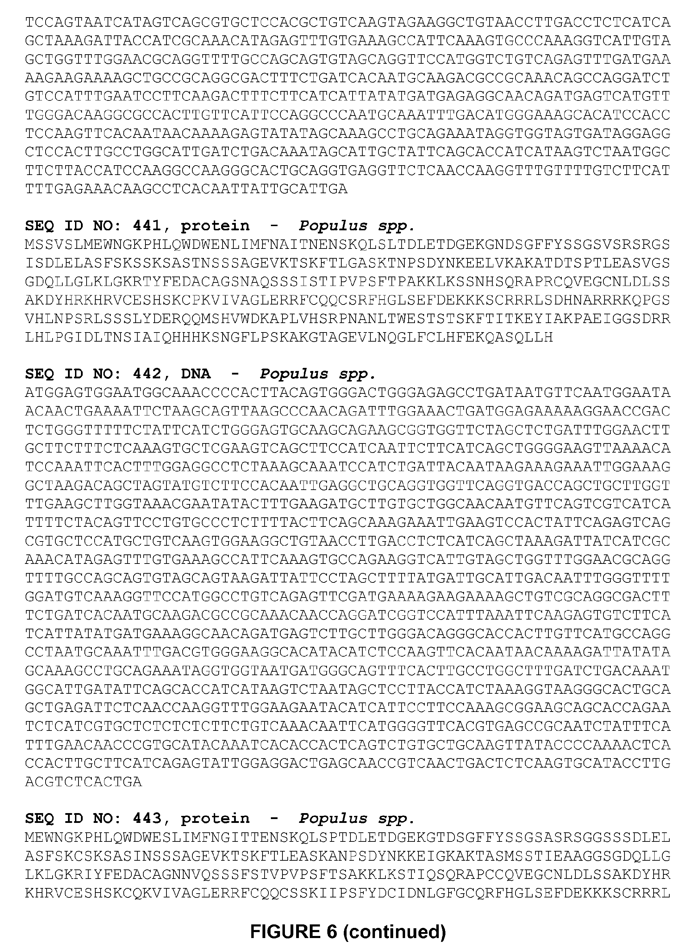

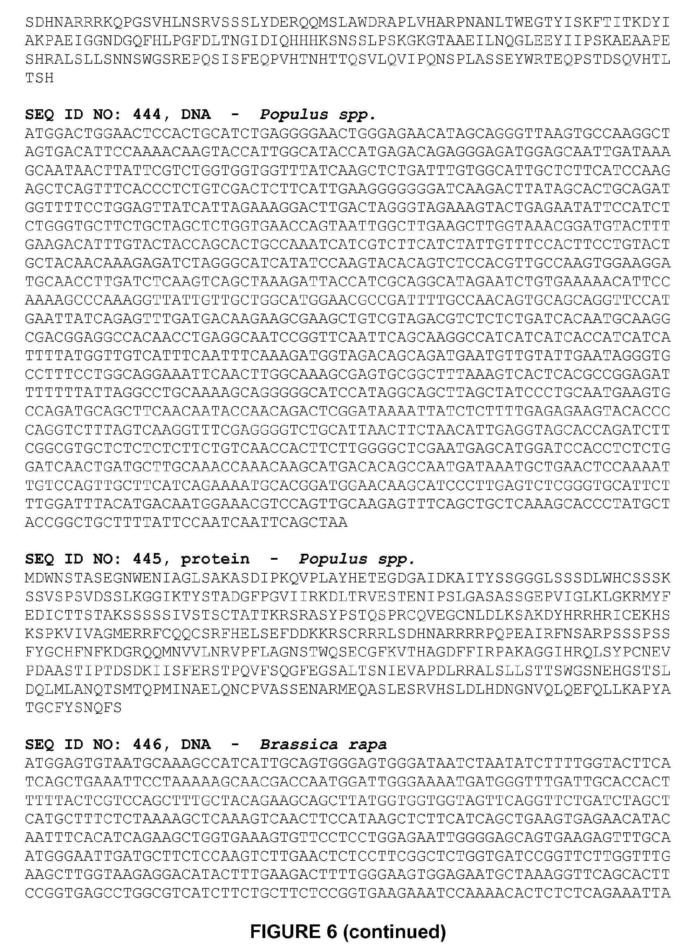

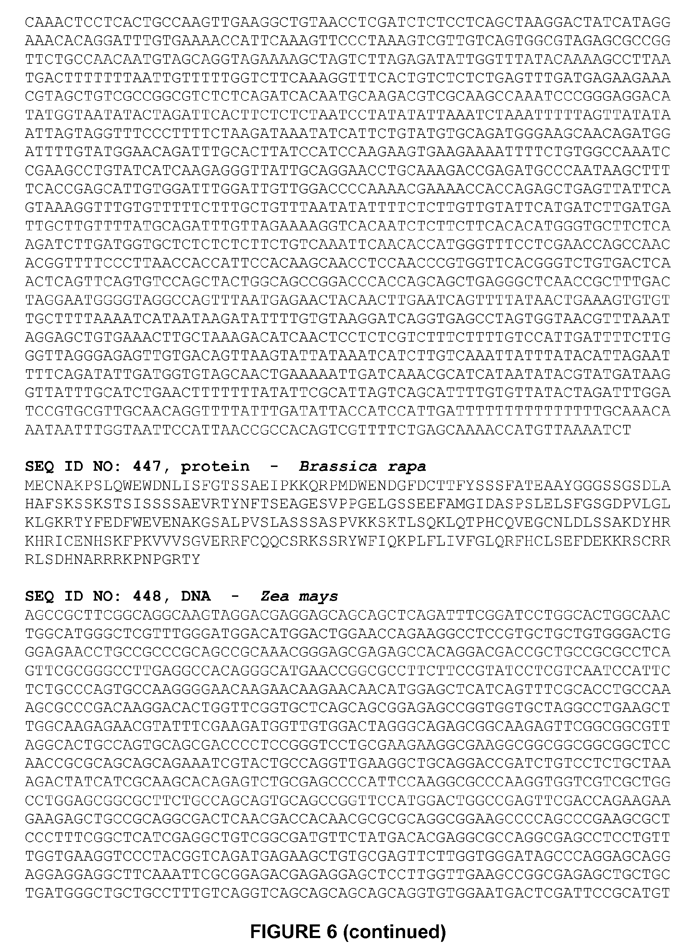

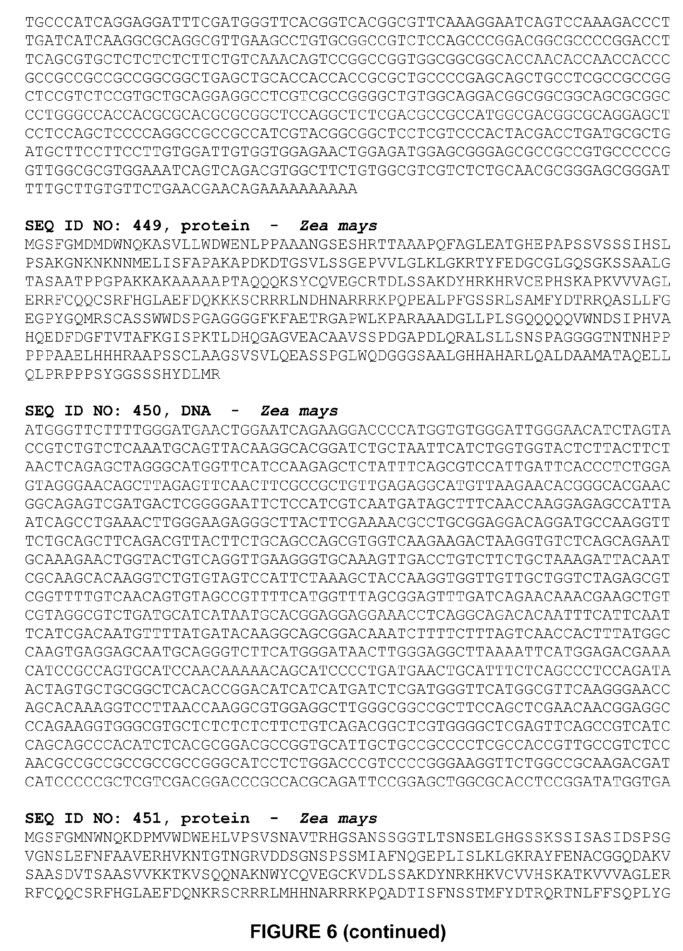

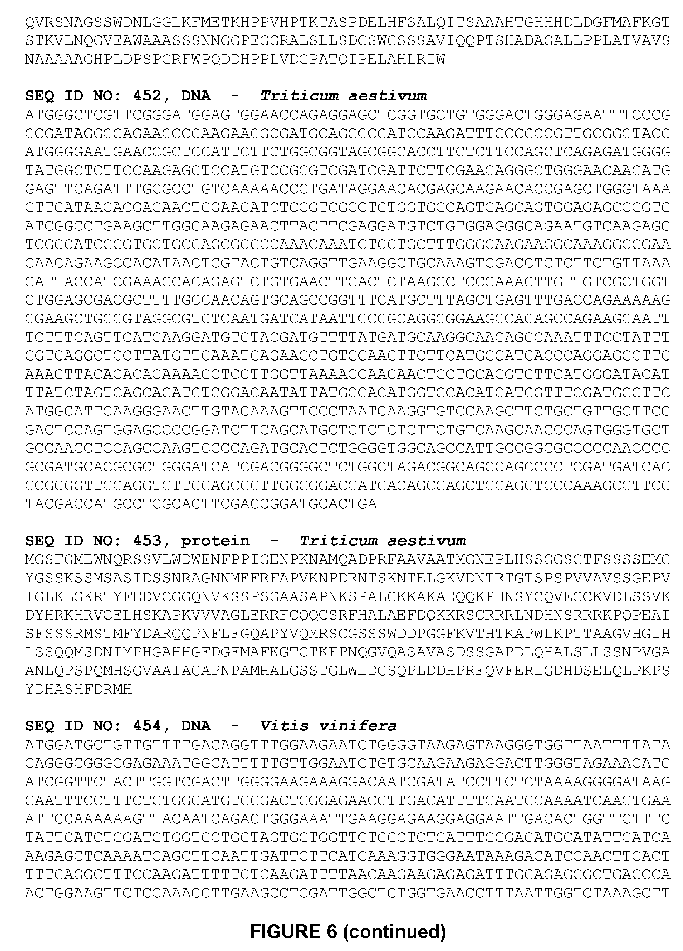

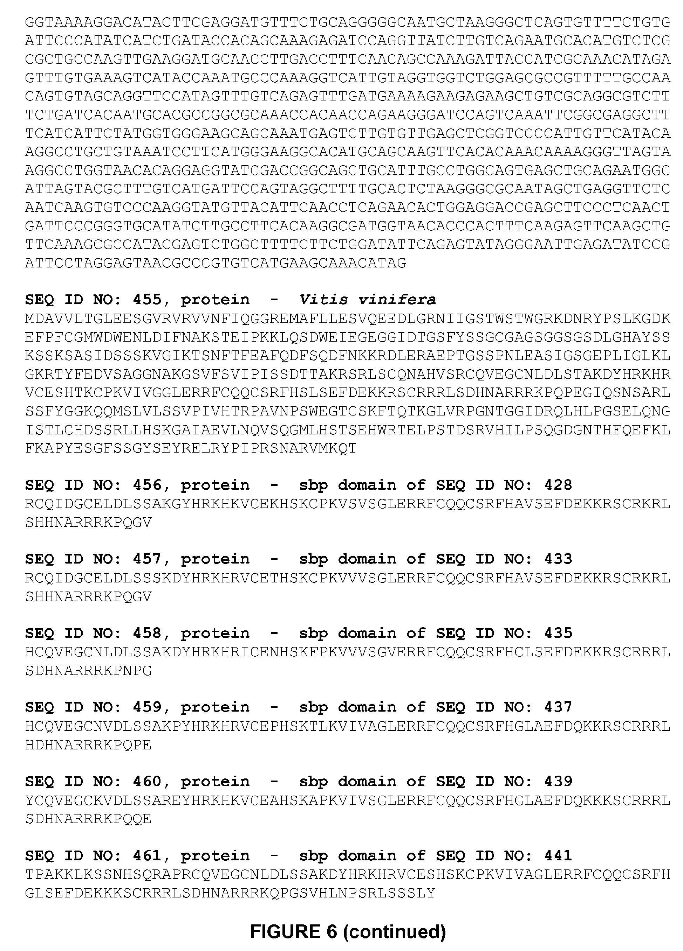

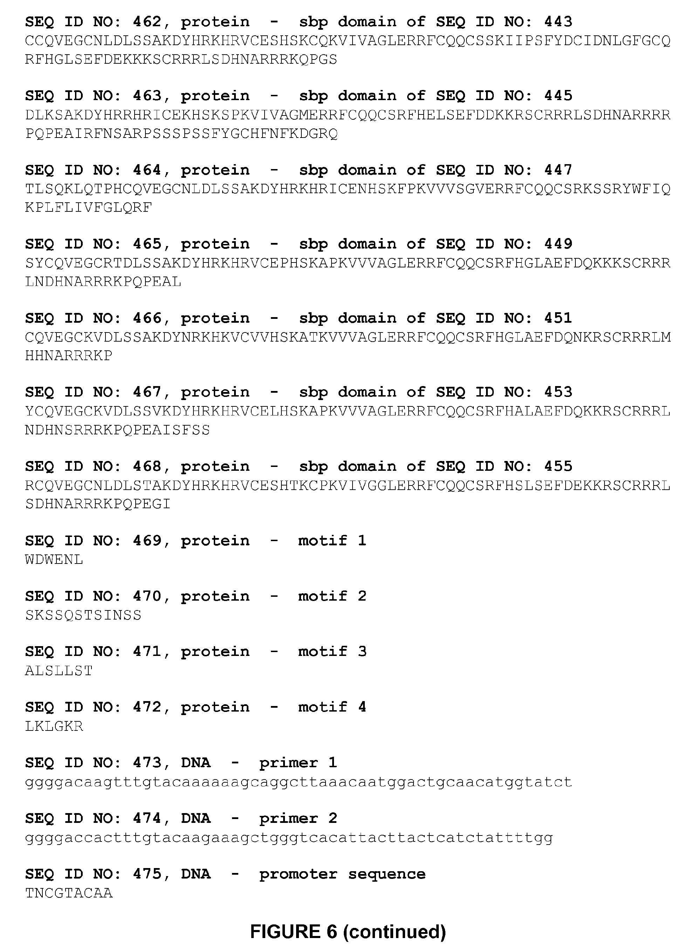

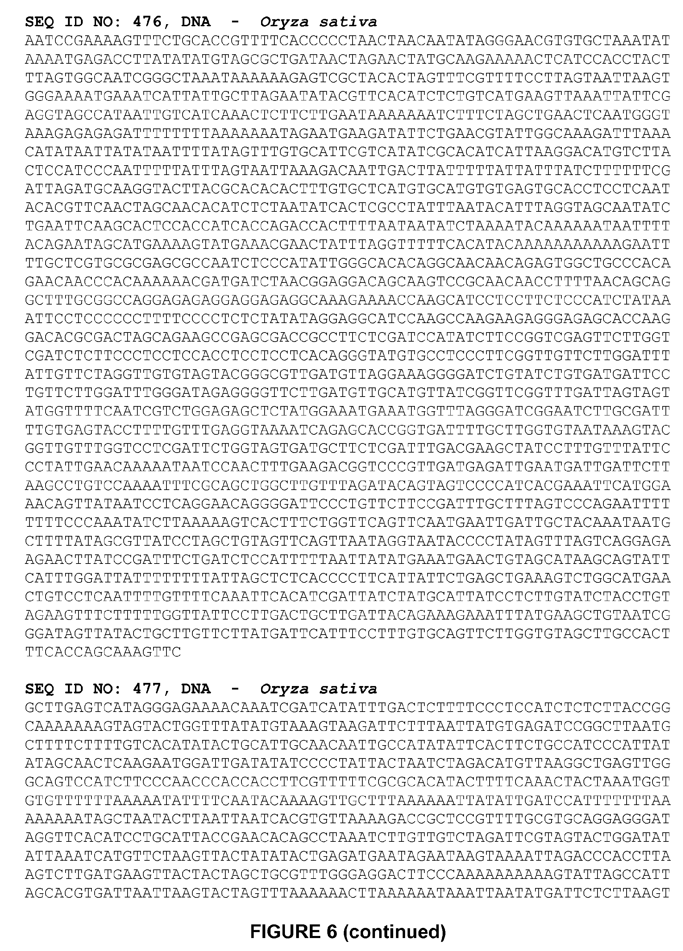

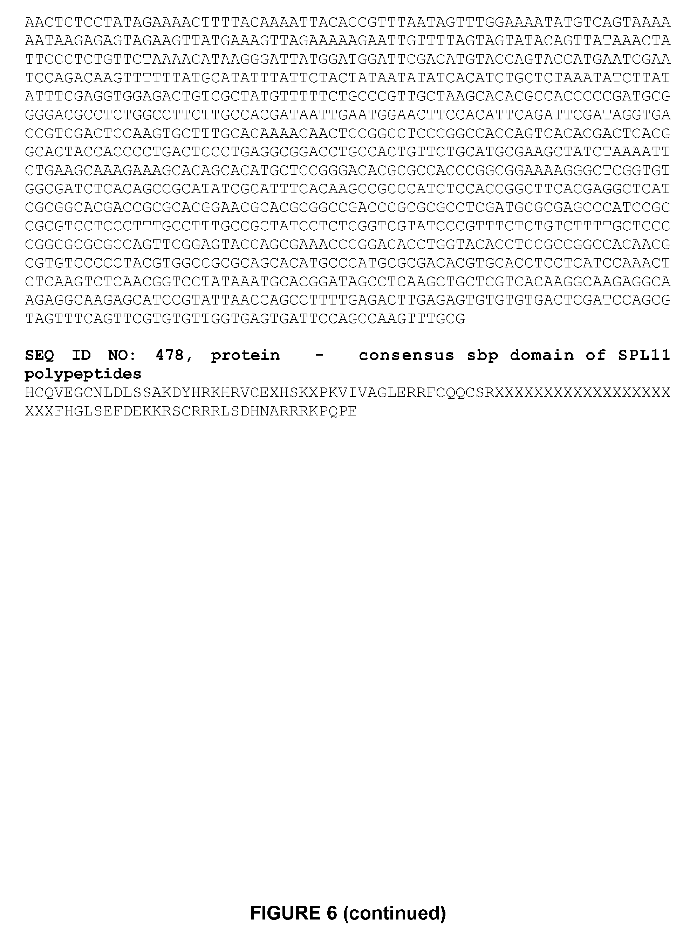

**Figure 6** details examples of SPL11 sequences useful in performing the methods according to the present invention.

## Examples

The present invention will now be described with reference to the following examples, which are by way of illustration alone. The following examples are not intended to completely define or otherwise limit the scope of the invention.

DNA manipulation: unless otherwise stated, recombinant DNA techniques are performed according to standard protocols described in Sambrook (2001) Molecular Cloning: a laboratory manual, 3rd Edition Cold Spring Harbor Laboratory Press, CSH, New York, or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

### Squamosa promoter binding protein-like 11 (SPL11)

#### *Example 1: Identification of sequences related to the SPL 11 nucleic acid sequence used in the methods of the invention*

Sequences (full length cDNA, ESTs or genomic) related to the nucleic acid sequence used in the methods of the present invention were identified amongst those maintained in the Entrez Nucleotides database at the National Center for Biotechnology Information (NCBI). The sequences search was also carried out in other public and proprietary databases. Sequence search tools were used, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid used in the present invention was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.

Table G1 provides a list of nucleic acid sequences related to the nucleic acid sequence used in the methods of the present invention. Polypeptides with an accession extended by a dot and one digit represent splice variants.

**Table G1:** Examples of SPL11 nucleic acids and polypeptides

| Name | Alias | Plant origin | Nucleic acid SEQ ID NO: | Protein SEQ ID NO: |
|---|---|---|---|---|
| Arath_SPL11a | At1g27360 | *Arabidopsis thaliana* | 427 | 428 |
| Arath_SPL11aa | NM_202191 (splicing variant) | *Arabidopsis thaliana* | 429 | 428 |
| Arath_SPL11aaa | NM_001084135 | *Arabidopsis thaliana* | 430 | 428 |
| Arath_SPL11aaaa | SEQID NO:1 Variant MiR156 insensitive | Synthetic | 431 | 428 |
| Arath_SPL11b | At1g27370 | *Arabidopsis thaliana* | 432 | 433 |
| Arath_SPL11c | At5g43270 | *Arabidopsis thaliana* | 434 | 435 |
| Orysa_SPL11a | LOC_Os02g04680 | *Oryza sativa* | 436 | 437 |
| Orysa_SPL11b | LOC_Os06g49010 | *Oryza sativa* | 438 | 439 |
| Popth_SPL11a | Populus SPL11a | *Populus Species* | 440 | 441 |
| Popth SPL11_b | Populus SPL11b | *Populus Species* | 442 | 443 |
| Popth_SPL11c | Populus SPL11c | *Populus Species* | 444 | 445 |
| Brara_SPL11a | Br_AC189413 | *Brassica rapa* | 446 | 447 |
| Zeama_SPL11a | ZM_60752693 | *Zea mays* | 448 | 449 |
| Zeama_SPL11b | ZM_SPL11b | *Zea mays* | 450 | 451 |
| Triae_SPL11a | Ta_SPL11a | *Triticum aestivum* | 452 | 453 |
| Vitvi_SPL11a | Vv_SPL11a | *Vitis vinifera* | 454 | 455 |

***Example 2: Alignment of SPL11 polypeptide sequences***

Alignment of polypeptide sequences was performed using the AlignX programme from the Vector NTI (Invitrogen) which is based on the popular Clustal W algorithm of progressive alignment (Thompson et al. (1997) Nucleic Acids Res 25:4876-4882; Chenna et al. (2003). Nucleic Acids Res 31:3497-3500). Default values are for the gap open penalty of 10, for the gap extension penalty of 0,1 and the selected weight matrix is Blosum 62 (if polypeptides are aligned). Minor manual editing was done to further optimise the alignment. A consensus sequence comprising highly conserved representative amino acids at each position is given; blanks in between given amino acids in the consensus sequence represent any amino acid. High sequence conservation among SPL11 polypeptides is observed mostly along the SBP domain of the polypeptides. The position of other conserved sequence motifs outside of the SBP domain herein represented by Motif 1 to Motif 4 (SEQ ID NO: 466 to SEQ ID NO: 472) is indicated over the consensus sequence.

Figure 2 provides an alignment of representative SPL11 polypeptides.

***Example 3: Calculation of global percentage identity between SPL11 polypeptide sequences useful in performing the methods of the invention***

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.

Parameters used in the comparison were:

| | |
|---|---|
| Scoring matrix: | Blosum62 |
| First Gap: | 12 |
| Extending gap: | 2 |

Results of the software analysis are shown in Table G2 for the global similarity and identity over the full length of the polypeptide sequences and Table G3 for the similarity on the SBP domain. Percentage identity is given above the diagonal in bold and percentage similarity is given below the diagonal (normal face).

The percentage identity between the SPL11 polypeptide sequences useful in performing the methods of the invention can be as low as 20 % amino acid identity compared to SEQ ID NO: 428. Typically, SPL11 polypeptides have a global (along the sequence of the entire polypeptide) sequence identity in the range of 40%.

The percentage identity between SBP domains comprised in SPL11 polypeptide sequences useful in performing the methods of the invention can be as low as 30 % amino acid identity compared to SEQ ID NO: 456 (SBP domain comprised in Arath_SPL11a or SEQ ID NO: 428). The SBP domains comprised in the SPL11 polypeptides given in Table G1 have a sequence identity in the range of 31 to 93.6%.

**Table G2:** MatGAT results for global similarity and identity over the full length of the polypeptide sequences.

| Name sequence | 1 | 2 | 3 | 4 | 5 | 6 | 7 | 8 | 9 | 10 | 11 | 12 | 13 |
|---|---|---|---|---|---|---|---|---|---|---|---|---|---|
| 1. Arath_SPL11a | | 75.3 | 37.2 | 31.2 | 30.9 | 38.4 | 34.3 | 29.9 | 31 | 27.5 | 30.7 | 31.4 | 31.5 |
| 2. Arath_SPL11b | 82.3 | | 36.6 | 33.2 | 32.1 | 37.9 | 36.4 | 29.7 | 31.3 | 29.5 | 30.7 | 31.2 | 33.2 |
| 3. Arath_SPL11c | 52.5 | 52 | | 33.6 | 349 | 40.4 | 38.6 | 33.7 | 48.5 | 32.9 | 31.8 | 32.4 | 34.4 |
| 4. Orysa_SPL11a | 46.5 | 44.6 | 46.7 | | 54.3 | 36.6 | 38.9 | 38.1 | 25.6 | 46.9 | 49.2 | 66.2 | 34.5 |
| 5. Orysa_SPL11 | 43.8 | 43.4 | 47.8 | 68.2 | | 35.4 | 36.7 | 38.4 | 25.2 | 44.3 | 55.1 | 55.2 | 32.6 |
| 6. Popth_SPL11a | 54.5 | 52 | 54.7 | 49 | 49.1 | | 66.5 | 35.4 | 36.4 | 30 | 32.3 | 35.4 | 44.1 |
| 7. Popth_SPL11b | 46.7 | 48.6 | 52.3 | 55.9 | 53.9 | 71.2 | | 37.7 | 32.7 | 30.7 | 34.7 | 37 | 42.5 |
| 8. Popth_SPL11c | 44.7 | 44.7 | 50.4 | 55.9 | 55.6 | 48.9 | 55.7 | | 25.8 | 33.3 | 34.2 | 35.7 | 37.6 |
| 9. Brara_SPL11a | 41.7 | 42.4 | 55.6 | 34.1 | 34.3 | 46.6 | 40 | 35.2 | | 22.2 | 24.1 | 24.8 | 29.4 |
| 10. Zeama-SPL11a | 42.1 | 42.5 | 44.6 | 61.4 | 58.3 | 42.7 | 45.9 | 51 | 31.5 | | 41.6 | 49.4 | 28.6 |
| 11. Zeama_SPL11b | 44.2 | 44.4 | 46.7 | 60.6 | 66.7 | 47.6 | 50.5 | 50.4 | 35.1 | 53.1 | | 49.5 | 32.1 |
| 12. Triae_SPL11a | 44 | 44 | 46.3 | 77 | 69.5 | 48 | 52 | 53.9 | 33.2 | 62.2 | 61.1 | | 32.8 |
| 13. Vitvi_SPL11a | 44.6 | 43.5 | 47.2 | 50.2 | 50.4 | 53.8 | 55.8 | 55.8 | 36.7 | 46.4 | 46.4 | 50.2 | |

**Table G3:** MatGAT results for similarity and identity over the SBP domain of polypeptide sequences.

| Name sequence | 1 | 2 | 3 | 4 | 5 | 6 | 7 | 8 | 9 | 10 | 11 | 12 |
|---|---|---|---|---|---|---|---|---|---|---|---|---|
| 1. Arath_SPL11a | | 93.7 | 77.2 | 73.4 | 75.9 | 58.3 | 62.2 | 49.5 | 46.6 | 73.4 | 73.4 | 68.2 |
| 2. Arath_SPL11b | 96.2 | | 79.7 | 73.4 | 73.4 | 59.3 | 63.3 | 49.5 | 48.9 | 75.9 | 77.2 | 71.8 |
| 3. Arath_SPL11c | 86.1 | 88.6 | | 81 | 77.2 | 63 | 66.3 | 54.2 | 59.1 | 81 | 78.5 | 74.1 |
| 4. Orysa_SPL11a | 84.8 | 86.1 | 89.9 | | 84.8 | 61.5 | 66.3 | 53.3 | 50 | 88.6 | 83.5 | 80 |
| 5. Orysa_SPL11b | 88.6 | 91.1 | 91.1 | 91.1 | | 59.3 | 64.3 | 50.5 | 47.7 | 87.3 | 83.5 | 78.8 |
| 6. Popth_SPL11a | 64.8 | 67.6 | 68.5 | 65.7 | 66.7 | | 60.6 | 52 | 45.4 | 62.4 | 57.4 | 60.2 |
| 7. Popth_SPL11b | 69.4 | 72.4 | 72.4 | 70.4 | 72.4 | 71.3 | | 45.2 | 41.3 | 66.3 | 62.2 | 61.5 |
| 8. Popth_SPL11c | 63.3 | 64.3 | 65.3 | 64.3 | 66.3 | 66.7 | 65.3 | | 31 | 53.3 | 50.5 | 56.1 |

(continued)

| Name sequence | 1 | 2 | 3 | 4 | 5 | 6 | 7 | 8 | 9 | 10 | 11 | 12 |
|---|---|---|---|---|---|---|---|---|---|---|---|---|
| 9. Brara_SPL11a | 62 | 64.6 | 68.4 | 63.3 | 64.6 | 56.5 | 55.1 | 43.9 | | 51.1 | 51.2 | 46.8 |
| 10. Zeama_SPL11a | 86.1 | 88.6 | 91.1 | 93.7 | 96.2 | 66.7 | 70.4 | 67.3 | 63.3 | | 88.6 | 84.7 |
| 11. Zeama_SPL11b | 88.6 | 91.1 | 89.9 | 88.6 | 93.7 | 63.9 | 69.4 | 64.3 | 64.6 | 92.4 | | 90.6 |
| 12. Triae_SPL11a | 82.4 | 84.7 | 84.7 | 84.7 | 88.2 | 67.6 | 72.4 | 71.4 | 60 | 88.2 | 90.6 | |
| 13. Vitvi_SPL11a | 90.1 | 93.8 | 90.1 | 87.7 | 90.1 | 70.4 | 75.5 | 67.3 | 64.2 | 88.9 | 87.7 | 87.1 |

***Example 4: Identification of domains comprised in polypeptide sequences useful in performing the methods of the invention***

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Pfam is a large collection of multiple sequence alignments and hidden Markov models covering many common protein domains and families. Pfam is hosted at the Sanger Institute server in the United Kingdom. Interpro is hosted at the European Bioinformatics Institute in the United Kingdom.

Table G4 shows the settings (Gathering cut off, trusted cut off and noise cut off) as described in the Pfam database that were used to build the HMMs_fs for SBP different domains.

**Table G4:** HMMs_build information in domain database Pfam Version 21.0

| **HMMER build information** | | |
|---|---|---|
| | Pfam_ls [Download HMM] | Pfam_fs [Download HMM] |
| Gathering cutoff | 25.0 25.0; | 25.0 25.0 |
| Trusted cutoff | 41.4 41.4; | 26.2 54.1 |
| Noise cutoff | 20.8 20.8; | 18.0 11.2 |
| Build method of HMM | hmmbuild -F HMM_ls SEED | hmmbuild -f -F HMM_fs SEED |
| | hmmcalibrate --seed 0 HMM_ls | hmmcalibrate --seed 0 HMM_fs |

The results of the Pfam scan for representative SPL11 polypeptides of plant origin are presented in Table G5. The amino acid coordinates for SBP domain in the sequence of reference is indicated in the corresponding column. The E-value of the alignment is also given.

**Table G5:** Pfam scan results for the SBP domain (Pfam Reference PF03110) of representative SPL11 polypeptides as perform on Pfam Version 21.0.

| **Name sequence** | **Amino acid coordinates of the SBP domain** | **e-value of the alignment** |
|---|---|---|
| Arath_SPL11a | 174-252 | 1.10E-50 |
| Arath_SPL11b | 175-253 | 1.61 E-47 |
| Arath_SPL11c | 168-246 | 1.90E-52 |
| Orysa_SPL11a | 181-259 | 5.10E-50 |
| Orysa_SPL11b | 179-257 | 3.90E-47 |
| Popth_SPL11a | 170-277 | 170-277 |
| Popth_SPL11b | 180-277 | 1.50E-41 |
| Popth_SPL11c | 171-249 | 7.80E-52 |

(continued)

| Name sequence | Amino acid coordinates of the SBP domain | e-value of the alignment |
|---|---|---|
| Brara_SPL11a | 180-279 | 5.20E-44 |
| Zeama_SPL11a | 161-239 | 2.20E-51 |
| Zeama_SPL11b | 159-237 | 1.10E-48 |
| Triae_SPL11a | 184-262 | 2.90E-52 |
| Vitvi_SPL11a | 171-249 | 7.80E-52 |

***Example 5: Cloning of the nucleic acid sequence used in the methods of the invention***

The nucleic acid sequence used in the methods of the invention was amplified by PCR using as template a custom-made *Arabidopsis thaliana* seedlings cDNA library (in pCMV Sport 6.0; Invitrogen, Paisley, UK). PCR was performed using Hifi Taq DNA polymerase in standard conditions, using 200 ng of template in a 50 pl PCR mix. An uspstream and downstream primer as represented by SEQ ID NO: 473 and SEQ ID NO: 474 respectively were used to amplify by PCR (Polymerase Chain Reaction) the coding region of a SPL11 nucleic acid as represented by SEQ ID NO: 427. The primers include the AttB sites for Gateway recombination to facilitate the cloning of the amplified PCR DNA fragment into a Gateway cloning vector.

The amplified PCR fragment was purified also using standard methods. The first step of the Gateway procedure, the BP reaction, was then performed, during which the PCR fragment recombines in vivo with the pDONR201 plasmid to produce, according to the Gateway terminology, an "entry clone", pSPL11. Plasmid pDONR201 was purchased from Invitrogen, as part of the Gateway® technology.

The entry clone comprising SEQ ID NO: 427 was then used in an LR reaction with a destination vector used for *Oryza sativa* transformation. This vector contained as functional elements within the T-DNA borders: a plant selectable marker; a screenable marker expression cassette; and a Gateway cassette intended for LR *in vivo* recombination with the nucleic acid sequence of interest already cloned in the entry clone. A rice GOS2 promoter (SEQ ID NO: 476) for constitutive expression was located upstream of this Gateway cassette in the vector pGOS2::SPL11. The SPL11 coding sequence was also cloned in an expression vector comprising a rice WSI18 promoter (SEQ ID NO: 477) for seed specific (ABA inducible) expression (pWSI18::SPL11).

After the LR recombination step, the resulting expression vectors pGOS2::SPL11 and pWSI18::SPL11 (Figure 5) were transformed into Agrobacterium strain LBA4044 according to methods well known in the art.

***Example 6: Plant transformation***

*Rice transformation*

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 minutes in 0.2% $HgCl_2$, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were subcultured on fresh medium 3 days before co-cultivation (to boost cell division activity).

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density ($OD_{600}$) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan et al. 1993, Hiei et al. 1994).

*Corn transformation*

Transformation of maize (*Zea mays*) is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with *Agrobacterium,* the embryos are grown in vitro on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Soybean transformation*

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

*Rapeseed/canola transformation*

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

*Alfalfa transformation*

A regenerating clone of alfalfa (*Medicago sativa*) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 μm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

*Cotton transformation*

Cotton (*Gossypium hirsutum* L.) transformation is performed using *Agrobacterium tumefaciens,* on hypocotyls explants. The commercial cultivars such as Coker 130 or Coker 312 (SeedCo, Lubbock, TX) are standard varieties used for transformation, but other varieties can also be used. The seeds are surface sterilized and germinated in the dark. Hypocotyl explants are cut from the germinated seedlings to lengths of about 1-1.5 centimeter. The hypotocyl explant is submersed in the *Agrobacterium tumefaciens* inoculum containing the expression vector, for 5 minutes then cocultivated for about 48 hours on MS +1.8 mg/l KNO3 + 2% glucose at 24° C, in the dark. The explants are transferred the same medium containing appropriate bacterial and plant selectable markers (renewed several times), until embryogenic calli is seen. The calli are separated and subcultured until somatic embryos appear. Plantlets derived from the somatic embryos are matured on rooting medium until roots develop. The rooted shoots are transplanted to potting soil in the greenhouse. T1 seeds are produced from plants that exhibit tolerance to the selection agent and that contain a single copy of the T-DNA insert.

***Example 7: Phenotypic evaluation procedure***

<u>68.1 Evaluation setup</u>

Approximately 35 independent T0 rice transformants were generated. The primary transformants were transferred from a tissue culture chamber to a greenhouse for growing and harvest of T1 seed. Six events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%. Plants grown under non-stress conditions are supplied with water at regular intervals to ensure that water and nutrients are not limiting to satisfy plant needs to complete growth and development.

Four T1 events were further evaluated in the T2 generation following the same evaluation procedure as for the T1 generation but with more individuals per event. From the stage of sowing until the stage of maturity the plants were passed several times through a digital imaging cabinet. At each time digital images (2048x1536 pixels, 16 million colours) were taken of each plant from at least 6 different angles.

*Drought screen*

Plants from T2 seeds were grown in potting soil under normal conditions until they approached the heading stage. They were then transferred to a "dry" section where irrigation was withheld. Humidity probes were inserted in randomly chosen pots to monitor the soil water content (SWC). When SWC went below certain thresholds, the plants were automatically re-watered continuously until a normal level was reached again. The plants were then re-transferred again to normal conditions. The rest of the cultivation (plant maturation, seed harvest) was the same as for plants not grown under abiotic stress conditions. Growth and yield parameters are recorded as detailed for growth under normal conditions.

*Nitrogen use efficiency screen*

Rice plants from T2 seeds are grown in potting soil under normal conditions except for the nutrient solution. The pots are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Growth and yield parameters are recorded as detailed for growth under normal conditions.

68.2 Statistical analysis: F test

A two factor ANOVA (analysis of variants) was used as a statistical model for the overall evaluation of plant phenotypic characteristics. An F test was carried out on all the parameters measured of all the plants of all the events transformed with the gene of the present invention. The F test was carried out to check for an effect of the gene over all the transformation events and to verify for an overall effect of the gene, also known as a global gene effect. The threshold for significance for a true global gene effect was set at a 5% probability level for the F test. A significant F test value to a gene effect, meaning that it is not only the mere presence or position of the gene that is causing the differences in phenotype.

When two experiments with overlapping events were carried out, a combined analysis was performed. This is useful to check consistency of the effects over the two experiments, and if this is the case, to accumulate evidence from both experiments in order to increase confidence in the conclusion. The method used was a mixed-model approach that takes into account the multilevel structure of the data (i.e. experiment - event - segregants). P values were obtained by comparing likelihood ratio test to chi square distributions.

68.3 Parameters measured

*Biomass-related parameter measurement*

From the stage of sowing until the stage of maturity the plants were passed several times through a digital imaging cabinet. At each time digital images (2048x1536 pixels, 16 million colours) were taken of each plant from at least 6 different angles.

The plant aboveground area (or leafy biomass) was determined by counting the total number of pixels on the digital images from aboveground plant parts discriminated from the background. This value was averaged for the pictures taken on the same time from the different angles and was converted to a physical surface value expressed in square mm by calibration. Experiments show that the aboveground plant area measured this way correlates with the biomass of plant parts above ground. The above ground area is the area measured at the time at which the plant had reached its maximal leafy biomass. The early vigour is the plant (seedling) aboveground area three weeks post-germination. Increase in root biomass is expressed as an increase in total root biomass (measured as maximum biomass of roots observed during the lifespan of a plant); or as an increase in the root/shoot index (measured as the ratio between root mass and shoot mass in the period of active growth of root and shoot).

Emergence vigour was determined by counting the total number of pixels from aboveground plant parts discriminated from the background. This value was averaged for the pictures taken on the same time from different angles and was converted to a physical surface value expressed in square mm by calibration. The results described below are for plants three weeks post-germination.

*Seed-related parameter measurements*

The mature primary panicles were harvested, counted, bagged, barcode-labelled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand Kernel Weight (TKW) is extrapolated from the number of filled seeds counted and their total weight. The Harvest Index (HI) in the present invention is defined as the ratio between the total seed yield and the above ground area ($mm^2$), multiplied by a factor $10^6$. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

***Example 8: Results of the phenotypic evaluation of the transgenic plants***

The results of the evaluation of transgenic rice plants expressing a SPL11 nucleic acid corresponding to the construct pGOS2::SPL11 and pWS118::SPL11 whether under drought stress or non-stress conditions are given herein. An increase of at least 5 % was observed for one or more of the following parameters, emergence vigour (seedling early vigour), total seed yield (seed weight), the seed fill rate (seed filling rate), the number of filled seeds, the number of flowers (seeds) per panicle and the harvest index, and of at least 3 % for thousand-kernel (1000-seed) weight in the transgenic plants of when compared to the control nullizygote plants in at least one of the growth conditions.

## Claims

1. A method for increasing seed yield in plants relative to control plants, comprising increasing expression in a plant of a nucleic acid encoding a SPL11 polypeptide, wherein said SPL11 polypeptide comprises a SBP domain having in increasing order of preference at least 70%, 75%, 80%, 85%, 90%, 95%, 97% or more sequence identity to any one of SEQ ID NO: 456 to SEQ ID NO: 468 and SEQ ID NO: 478, wherein said increased seed yield preferably comprises increased total seed weight, number of filled seeds, number of seeds or florets per panicle, thousand-kernel weight, seed filling rate, and/or harvest index relative to control plants.

2. Method according to claim 1 wherein said SPL11 polypeptide in addition to the SBP domain comprises any one or more of the following conserved motifs:

   (i) Motif 1 as represented by SEQ ID NO: 469 wherein any conservative amino acid substitution and/or 1 or 2 non conservative substitution are allowed;
   (ii) Motif 2 as represented by SEQ ID NO: 470 wherein any change is allowed, provided that at least 4 amino acids have a polar side chain, preferably serine or threonine, and provided that the domain is located at the N-terminal end of the SBP domain;
   (iii) Motif 3 as represented by SEQ ID NO: 471 wherein 1 or 2 mismatches are allowed;
   (iv) Motif 4 as represented by SEQ ID NO: 472 wherein 1, 2 or 3 mismatches are allowed.

3. Method according to claim 1 or 2, wherein said increased expression is effected by introducing and expressing in a plant a nucleic acid encoding an SPL11 polypeptide as defined in claim 1 or 2.

4. Method according to any of claims 1 to 3, wherein said nucleic acid encoding a SPL11 polypeptide encodes any one of the proteins listed in Table G1 or is a portion of such a nucleic acid, or a nucleic acid capable of hybridising with such a nucleic acid and/or wherein said nucleic acid sequence encodes an orthologue or paralogue of any of the proteins given in Table G1 and/or wherein said nucleic acid encodes SEQ ID NO: 428.

5. Method according to any of claims 1 to 4, wherein said increased seed yield is obtained under non-stress conditions and/or wherein said increased seed yield is obtained under mild drought stress growth conditions.

6. Method according to any one of claims 1 to 5, wherein said nucleic acid is operably linked to a constitutive promoter, preferably to a GOS2 promoter, most preferably to a GOS2 promoter from rice and/or wherein said nucleic acid is operably linked to a seed specific promoter, preferably to a WSI18 promoter, most preferably to a WSI18 promoter from rice.

7. An isolated nucleic acid molecule comprising:

   (i) A nucleic acid represented by SEQ ID NO: 448;
   (ii) The complement of a nucleic acid represented by SEQ ID NO: 448;
   (iii) A nucleic acid encoding an SPL11 polypeptide having, in increasing order of preference, at least 70%, 75%, 80%, 85%, 90%, 95%, 96%, 97%, 98%, 99% or more sequence identity to the amino acid sequence represented by SEQ ID NO: 449, and having in increasing order of preference at least 70%, 75%, 80%, 85%, 90%, 95%, 96%, 97%, 98%, 99% or more sequence identity to SEQ ID NO: 465:

   SYCQVEGCRTDLSSAKDYHRKHRVCEPHSKAPKVVVAGLERRFCQQCSRFHGLAEFDQKKKSCRRR
   LNDHNARRRKPQPEAL.

8. An isolated squamosa promoter binding protein-like 11 (SPL11) polypeptide comprising:

   (i) an amino acid sequence represented by SEQ ID NO: 449;
   (ii) an amino acid sequence having, in increasing order of preference, at least 70%, 75%, 80%, 85%, 90%, 95%, 96%, 97%, 98%, 99% or more sequence identity to the amino acid sequence represented by SEQ ID NO: 449, and having in increasing order of preference at least 70%, 75%, 80%, 85%, 90%, 95%, 96%, 97%, 98%, 99% or more sequence identity to SEQ ID NO: 465:

SYCQVEGCRTDLSSAKDYHRKHRVCEPHSKAPKVVVAGLERRFCQQCSRFHGLAEFDQKKKSCRRRLND
HNARRRKPQPEAL.

**9.** Construct comprising:

(i) nucleic acid encoding an SPL11 polypeptide as defined in claim 1, 2 or 7;
(ii) one or more control sequences capable of driving expression of the nucleic acid sequence of (i); and optionally
(iii) a transcription termination sequence.

**10.** Construct according to claim 9 wherein one of said control sequences is a constitutive promoter, preferably a GOS2 promoter, most preferably a GOS2 promoter from rice and/or wherein one of said control sequences is a seed specific promoter, preferably a WSI18 promoter, most preferably a WSI18 promoter from rice.

**11.** Use of a construct according to claim 9 or 10 in a method for making plants having increased seed yield relative to control plants

**12.** Plant, plant part or plant cell transformed with a construct according to claim 9 or 10.

**13.** Method for the production of a transgenic plant having increased seed yield relative to control plants, comprising:

(i) Introducing and expressing in a plant a nucleic acid encoding an SPL11 polypeptide as defined in claim 1, 2 or 7; and
(ii) Cultivating the plant cell under conditions promoting plant growth and development.

**14.** Transgenic plant having increased seed yield relative to control plants, resulting from increased expression of a nucleic acid transgene encoding an SPL11 polypeptide as defined in claim 1, 2 or 7, or a transgenic plant cell derived from said transgenic plant.

**15.** Transgenic plant according to claim12 or 14, or a transgenic plant cell derived thereof, wherein said plant is a dicot crop plant such as soybean, cotton or canola or a monocot crop plant or cereal, such as rice, maize, wheat, barley, millet, rye, triticale, sorghum and oats, and wherein said transgenic plant or plant cell comprises a recombinant nucleic acid encoding a SPL11 polypeptide as defined in claim 1, 2 or 7.

**16.** Harvestable parts of a plant according to claim 15, wherein said harvestable parts are preferably shoot biomass, flowers and/or seeds, and wherein cells of said harvestable parts comprise a recombinant nucleic acid encoding a SPL11 polypeptide as defined in claim 1, 2 or 7.

**17.** Use of a nucleic acid encoding an SPL11 polypeptide as defined in claim 1, 2 or 7 in increasing seed yield and/or shoot biomass in plants relative to control plants.

## Patentansprüche

**1.** Verfahren zur Steigerung des Samenertrags in Pflanzen im Vergleich zu Kontrollpflanzen, bei dem man in einer Pflanze die Expression einer Nukleinsäure, die ein SPL11-Polypeptid kodiert, steigert, wobei das SPL11-Polypeptid eine SBP-Domäne umfasst, die in steigender Reihenfolge der Präferenz mindestens 70%, 75%, 80%, 85%, 90%, 95%, 97% oder mehr Sequenzidentität zu einer der Sequenzen von SEQ ID NO: 456 bis SEQ ID NO: 468 und SEQ ID NO: 478 aufweist, wobei der gesteigerte Samenertrag vorzugsweise gesteigertes Gesamtsamengewicht, Anzahl der gefüllten Samen, Anzahl der Samen oder Blütchen pro Rispe, Tausendkorngewicht, Samenfüllungsrate und/oder Harvest-Index im Vergleich zu Kontrollpflanzen umfasst.

**2.** Verfahren nach Anspruch 1, wobei das SPL11-Polypeptid neben der SBP-Domäne eine oder mehrere der folgenden konservierten Motive umfasst:

(i) Motiv 1 nach SEQ ID NO: 469, wobei jede konservative Aminosäuresubstitution und/oder 1 oder 2 nichtkonservative Substitutionen erlaubt sind;

(ii) Motiv 2 nach SEQ ID NO: 470, wobei jede Änderung erlaubt ist, vorausgesetzt dass mindestens 4 Aminosäuren eine polare Seitenkette, vorzugsweise Serin oder Threonin, aufweisen, und vorausgesetzt dass sich die Domäne am N-terminalen Ende der SBP-Domäne befindet;
(iii) Motiv 3 nach SEQ ID NO: 471 wobei 1 oder 2 Mismatches erlaubt sind;
(iv) Motiv 4 nach SEQ ID NO: 472 wobei 1, 2 oder 3 Mismatches erlaubt sind.

**3.** Verfahren nach Anspruch 1 oder 2, wobei die gesteigerte Expression durch Einführen und Exprimieren einer Nukleinsäure, die ein SPL11-Polypeptid nach Anspruch 1 oder 2 kodiert, in einer Pflanze herbeigeführt wird.

**4.** Verfahren nach einem der Ansprüche 1 bis 3, wobei die Nukleinsäure, die ein SPL11-Polypeptid kodiert, eines der in Tabelle G1 aufgeführten Proteine kodiert, oder ein Abschnitt einer solchen Nukleinsäure, oder eine Nukleinsäure ist, die mit einer solchen Nukleinsäure hybridisieren kann, und/oder wobei die Nukleinsäuresequenz ein Orthologon oder Paralogon von einem der in Tabelle G1 angegebenen Proteine kodiert und/oder wobei die Nukleinsäure SEQ ID NO: 428 kodiert.

**5.** Verfahren nach einem der Ansprüche 1 bis 4, wobei der gesteigerte Samenertrag unter Nichtstressbedingungen erhalten wird und/oder wobei der gesteigerte Samenertrag unter Wachstumsbedingungen eines leichten Trockenheits-Stresses erhalten wird.

**6.** Verfahren nach einem der Ansprüche 1 bis 5, wobei die Nukleinsäure funktionsfähig mit einem konstitutiven Promotor, vorzugsweise einem GOS2-Promotor, am stärksten bevorzugt einem GOS2-Promotor aus Reis, verknüpft ist und/oder wobei die Nukleinsäure funktionsfähig mit einem samenspezifischen Promotor, vorzugsweise einem WSI18-Promotor, am stärksten bevorzugt einem WSI18-Promotor aus Reis, verknüpft ist.

**7.** Isoliertes Nukleinsäuremolekül, umfassend:

(i) eine Nukleinsäure nach SEQ ID NO: 448;
(ii) das Komplement einer Nukleinsäure nach SEQ ID NO: 448;
(iii) eine Nukleinsäure, die ein SPL11-Polypeptid kodiert, das in steigender Reihenfolge der Präferenz mindestens 70%, 75%, 80%, 85%, 90%, 95%, 96%, 97%, 98%, 99% oder mehr Sequenzidentität zu der Aminosäuresequenz nach SEQ ID NO: 449 aufweist, und das in steigender Reihenfolge der Präferenz mindestens 70%, 75%, 80%, 85%, 90%, 95%, 96%, 97%, 98%, 99% oder mehr Sequenzidentität zu SEQ ID NO: 465:

SYCQVEGCRTDLSSAKDYHRKHRVCEPHSKAPKVVVAGLERRFCQQCS
RFHGLAEFDQKKKSCRRRLNDHNARRRKPQPEAL

aufweist.

**8.** Isoliertes Squamosa-Promotor-Bindungsproteinähnliches 11 (SPL11) Polypeptid, umfassend:

(i) eine Aminosäuresequenz nach SEQ ID NO: 449;
(ii) eine Aminosäuresequenz, die in steigender Reihenfolge der Präferenz mindestens 70%, 75%, 80%, 85%, 90%, 95%, 96%, 97%, 98%, 99% oder mehr Sequenzidentität zu der Aminosäuresequenz nach SEQ ID NO: 449 aufweist, und die in steigender Reihenfolge der Präferenz mindestens 70%, 75%, 80%, 85%, 90%, 95%, 96%, 97%, 98%, 99% oder mehr Sequenzidentität zu SEQ ID NO: 465:

SYCQVEGCRTDLSSAKDYHRKHRVCEPHSKAPKVVVAGLERRFCQQCSRF

HGLAEFDQKKKSCRRRLNDHNARRRKPQPEAL

aufweist.

**9.** Konstrukt, umfassend:

(i) eine Nukleinsäure, die ein SPL11-Polypeptid nach Anspruch 1, 2 oder 7 kodiert;
(ii) eine oder mehrere Kontrollsequenzen, die die Expression der Nukleinsäuresequenz von (i) steuern können; und gegebenenfalls
(iii) eine Transkriptionsterminationssequenz.

**10.** Konstrukt nach Anspruch 9, wobei eine der Kontrollsequenzen ein konstitutiver Promotor, vorzugsweise ein GOS2-Promotor, am stärksten bevorzugt ein GOS2-Promotor aus Reis, ist und/oder wobei eine der Kontrollsequenzen ein samenspezifischer Promotor, vorzugsweise ein WSI18-Promotor, am stärksten bevorzugt ein WSI18-Promotor aus Reis, ist.

**11.** Verwendung eines Konstrukts nach Anspruch 9 oder 10 in einem Verfahren zur Herstellung von Pflanzen mit gesteigertem Samenertrag im Vergleich zu Kontrollpflanzen.

**12.** Pflanze, Pflanzenteil oder Pflanzenzelle, die mit einem Konstrukt nach Anspruch 9 oder 10 transformiert sind.

**13.** Verfahren zur Herstellung einer transgenen Pflanze mit gesteigertem Samenertrag im Vergleich zu Kontrollpflanzen, umfassend:

(i) Einführen und Exprimieren einer Nukleinsäure, die ein SPL11-Polypeptid nach Anspruch 1, 2 oder 7 kodiert, in eine(r) Pflanze; und
(ii) Züchten der Pflanzenzelle unter Bedingungen, die das Wachstum und die Entwicklung der Pflanze fördern.

**14.** Transgene Pflanze mit gesteigertem Samenertrag im Vergleich zu Kontrollpflanzen, der sich aus einer gesteigerten Expression eines Nukleinsäuretransgens, das ein SPL11-Polypeptid nach Anspruch 1, 2 oder 7 kodiert, ergibt, oder eine transgene Pflanzenzelle, die aus der transgenen Pflanze stammt.

**15.** Transgene Pflanze nach Anspruch 12 oder 14, oder eine daraus stammende transgene Pflanzenzelle, wobei die Pflanze eine dikotyle Kulturpflanze, wie Sojabohne, Baumwolle oder Canola-Raps, oder eine monokotyle Kulturpflanze oder Getreide, wie Reis, Mais, Weizen, Gerste, Hirse, Roggen, Triticale, Sorghum und Hafer, ist, und wobei die transgene Pflanze oder Pflanzenzelle eine rekombinante Nukleinsäure umfasst, die ein SPL11-Polypeptid nach Anspruch 1, 2 oder 7 kodiert.

**16.** Erntefähige Teile einer Pflanze nach Anspruch 15, wobei die erntefähigen Teile vorzugsweise Sprossbiomasse, Blüten und/oder Samen sind, und wobei Zellen der erntefähigen Teile eine rekombinante Nukleinsäure umfassen, die ein SPL11 Polypeptid nach Anspruch 1, 2 oder 7 kodiert.

**17.** Verwendung einer Nukleinsäure, die ein SPL11-Polypeptid nach Anspruch 1, 2 oder 7 kodiert, bei der Steigerung des Samenertrags, und/oder der Sprossbiomasse in Pflanzen im Vergleich zu Kontrollpflanzen.

## Revendications

**1.** Procédé pour accroître le rendement en graines dans des plantes par rapport à des plantes témoins, comprenant l'augmentation de l'expression dans une plante d'un acide nucléique codant pour un polypeptide SPL11, ledit polypeptide SPL11 comprenant un domaine SBP ayant, dans l'ordre croissant de préférence, une identité de séquence d'au moins 70 %, 75 %, 80 %, 85 %, 90 %, 95 %, 97 % ou plus avec l'une quelconque des séquences SEQ ID NO:456 à SEQ ID NO:468 et SEQ ID NO:478, ledit rendement en graines accru comprenant de préférence un nombre total de graines accru, un nombre de graines pleines accru, un nombre de graines ou de fleurs parfaites par panicule accru, un poids de mille graines accru, un taux de remplissage des graines accru et/ou un indice de récolte accru, par rapport aux plantes témoins.

**2.** Procédé selon la revendication 1, dans lequel ledit polypeptide SPL11, en plus du domaine SBP, comprend l'un ou plusieurs quelconques des motifs conservés suivants :

(i) Motif 1, tel que représenté par SEQ ID NO:469, dans lequel sont autorisées toutes substitutions conservatives d'acides aminés et/ou une ou deux substitutions non conservatives ;
(ii) Motif 2, tel que représenté par SEQ ID NO:470, dans lequel tous changements sont autorisés, à la condition qu'au moins quatre acides aminés aient une chaîne latérale polaire, de préférence la sérine ou la thréonine, et à la condition que le domaine soit situé au niveau de l'extrémité N-terminal du domaine SBP ;
(iii) Motif 3, tel que représenté par SEQ ID NO:471, dans lequel 1 ou 2 mésappariements sont autorisés ;
(iv) Motif 4, tel que représenté par SEQ ID NO:472, dans lequel 1, 2 ou 3 mésappariements sont autorisés.

**3.** Procédé selon la revendication 1 ou 2, dans lequel ladite expression accrue est réalisée par introduction et expression dans une plante d'un acide nucléique codant pour un polypeptide SPL11 tel que défini dans la revendication 1 ou 2.

**4.** Procédé selon l'une quelconque des revendications 1 à 3, dans lequel ledit acide nucléique codant pour un polypeptide SPL11 code pour l'une quelconque des protéines listées dans le Tableau G1 ou est une portion d'un tel acide nucléique ou d'un acide nucléique capable de s'hybrider à un tel acide nucléique, et/ou dans lequel ladite séquence d'acide nucléique code pour un orthologue ou un paralogue de l'une quelconque des protéines consignées dans le Tableau G1, et/ou dans lequel ledit acide nucléique code pour SEQ ID NO:428.

**5.** Procédé selon l'une quelconque des revendications 1 à 4, dans lequel ledit rendement en graines accru est obtenu dans des conditions sans stress, et/ou dans lequel ledit rendement en graines accru est obtenu dans des conditions de croissance en présence d'un stress de sécheresse ménagé.

**6.** Procédé selon l'une quelconque des revendications 1 à 5, dans lequel ledit acide nucléique est fonctionnellement lié à un promoteur constitutif, de préférence un promoteur GOS2, tout spécialement un promoteur GOS2 du riz, et/ou dans lequel ledit acide nucléique est fonctionnellement lié à un promoteur spécifique de graine, de préférence un promoteur WSI18, tout spécialement un promoteur WSI18 du riz.

**7.** Molécule d'acide nucléique isolée, comprenant :

(i) un acide nucléique représenté par SEQ ID NO:448 ;
(ii) le complément d'un acide nucléique représenté par SEQ ID NO:448 ;
(iii) un acide nucléique codant pour un polypeptide SPL11 ayant, dans l'ordre croissant de préférence, une identité de séquence d'au moins 70 %, 75 %, 80 %, 85 %, 90 %, 95 %, 96 %, 97 %, 98 %, 99 % ou plus avec la séquence d'acides aminés représentée par SEQ ID NO:449, et ayant, dans l'ordre croissant de préférence, une identité de séquence d'au moins 70 %, 75 %, 80 %, 85 %, 90 %, 95 %, 96 %, 97 %, 98 %, 99 % ou plus avec SEQ ID NO:465 :

SYCQVEGCRTDLSSAKDYHRKHRVCEPHSKAPKVVVAGLERRFCQQCSRFHGLAEFDQKKKSCRRR
LNDHNARRRKPQPEAL.

**8.** Polypeptide 11 de type protéine de liaison au promoteur squamosa (SPL11) isolé, comprenant :

(i) une séquence d'acides aminés représentée par SEQ ID NO:449 ;
(ii) une séquence d'acides aminés ayant, dans l'ordre croissant de préférence, une identité de séquence d'au moins 70 %, 75 %, 80 %, 85 %, 90 %, 95 %, 96 %, 97 %, 98 %, 99 % ou plus avec la séquence d'acides aminés représentée par SEQ ID NO:449 et ayant dans l'ordre croissant de préférence une identité de séquence d'au moins 70 %, 75 %, 80 %, 85 %, 90 %, 95 %, 96 %, 97 %, 98 %, 99 % ou plus avec SEQ ID NO:465 :

SYCQVEGCRTDLSSAKDYHRKHRVCEPHSKAPKVVVAGLERRFCQQCSRFHGLAEFDQKKKSCRRRLND
HNARRRKPQPEAL.

**9.** Construction comprenant :

(i) un acide nucléique codant pour un polypeptide SPL11 tel que défini dans la revendication 1, 2 ou 7 ;
(ii) une ou plusieurs séquences de contrôle capables de commander l'expression de la séquence d'acide nucléique de (i) ; et en option
(iii) une séquence terminatrice de transcription.

**10.** Construction selon la revendication 9, dans laquelle l'une desdites séquences de contrôle est un promoteur constitutif, de préférence un promoteur GOS2, tout spécialement un promoteur GOS2 du riz, et/ou dans lequel l'une desdites séquences de contrôle est un promoteur spécifique de graine, de préférence un promoteur WSI18, tout spécialement un promoteur WSI18 du riz.

**11.** Utilisation d'une construction selon la revendication 9 ou 10 dans un procédé de fabrication de plantes ayant un rendement en graines accru par rapport à des plantes témoins.

**12.** Plante, partie de plante ou cellule végétale transformée avec une construction selon la revendication 9 ou 10.

**13.** Procédé de production d'une plante transgénique ayant un rendement en graines accru par rapport à des plantes témoins, comprenant :

(i) l'introduction et l'expression dans une plante d'un acide nucléique codant pour un polypeptide SPL11 tel que défini dans la revendication 1, 2 ou 7 ; et
ii) la culture de la cellule végétale dans des conditions favorisant la croissance et le développement de la plante.

**14.** Plante transgénique ayant un rendement en graines accru par rapport à des plantes témoins, résultant de l'expression accrue d'un transgène d'acide nucléique codant pour un polypeptide SPL11 tel que défini dans la revendication 1, 2 ou 7, ou cellule de plante transgénique dérivée de ladite plante transgénique.

**15.** Plante transgénique selon la revendication 12 ou 14, ou cellule de plante transgénique qui en dérive, ladite plante étant une plante de culture dicotylédone telle que le soja, le coton ou le canola, ou une plante de culture monocotylédone ou une céréale, telle que le riz, le maïs, le blé, l'orge, le millet, le seigle, le triticale, le sorgho et l'avoine, et ladite plante transgénique ou ladite cellule végétale comprenant un acide nucléique recombinant codant pour un polypeptide SPL11 tel que défini dans la revendication 1, 2 ou 7.

**16.** Parties récoltables d'une plante selon la revendication 15, lesdites parties récoltables étant de préférence la biomasse des pousses, les fleurs et/ou les graines, et les cellules desdites parties récoltables comprenant un acide nucléique recombinant codant pour un polypeptide SPL11 tel que défini dans la revendication 1, 2 ou 7.

**17.** Utilisation d'un acide nucléique codant pour un polypeptide SPL11 tel que défini dans la revendication 1, 2 ou 7, pour augmenter le rendement en graines et/ou la biomasse des pousses dans des plantes, par rapport à des plantes témoins.

MDCNMVSSSQ**WDWEH**LIMSNPSRTEDDSKQLPTEWEIEKGEGIESIVPH
FSGLERVSSGSATSFWHTAV***SKSSQSTSINSS***SPEAKRCKLASESSPGD
SCSNIDFVQVKAPTALEVSVASAESDLC***LKLGKR***TYSEEYWGRNNNEIS
AVSMKLLTPSVVAGKSKLCGQSMPVP*RCQIDGCELDLSSAKGYHRKHKV*
*CEKHSKCPKVSVSGLERRFCQQCSRFHAVSEFDEKKRSCRKRLSHHNAR*
*RRKPQGVFSMNPERVYDRRQHTNMLWNGV*SLNARSEEMYEWGNNTYDTK
PRQTEKSFTLSFQRGNGSEDQLVASSSRMFSTSQTSGGFPAGKSKFQLH
GEDVGEYSGVLHESQDIHR***ALSLLS***TSSDPLAQPHVQPFSLLCSYDVVP
K

**FIGURE 1**

```
                   1                                                50
  SEQID455    (1)  MDAVVLTGLEESGVRVRVVNFIQGGREMAFLLESVQEEDLGRNIIGSTWS
  SEQID453    (1)  ----------------------------------------MGSFGMEWN
  SEQID451    (1)  ----------------------------------------MGSFGMNWN
  SEQID449    (1)  --------------------------------------MGSFGMDMDWN
  SEQID447    (1)  ---------------------------------------------MECN
  SEQID445    (1)  ---------------------------------------------MDWN
  SEQID443    (1)  ---------------------------------------------MEWN
  SEQID441    (1)  ---------------------------------------MSSVSLMEWN
  SEQID439    (1)  ----------------------------------------MASFGMNWN
  SEQID437    (1)  ----------------------------------------MGSFGMDWN
  SEQID435    (1)  ---------------------------------------------MECN
  SEQID433    (1)  ---------------------------------------------MDCN
  SEQID428    (1)  ---------------------------------------------MDCN
Consensus     (1)                                                   MDWN

                   51                                               100
  SEQID455   (51)  TWGRKDNRYPSLKGDKEFPFCGMWDWENLDIFNAKSTEIPKKL-QSDWEI
  SEQID453   (10)  -------- --------QRSSVLWDWENFPPIG----ENPKNAMQADPRF
  SEQID451   (10)  -------- --------QKDPMVWDWEHLVPSVSNAVTR-----------
  SEQID449   (12)  -------- -------QKASVLLWDWENLPPAAANGSES-----------
  SEQID447    (5)  -------- --------AKPSLQWEWDNLISFGTSSAEIPKKQRPMDWEN
  SEQID445    (5)  -------- ---------STASEGNWENIAGLSAKASDIPKQVPLAYHET
  SEQID443    (5)  -------- --------GKPHLQWDWESLIMFNGITTENSKQLSPTDLET
  SEQID441   (11)  -------- --------GKPHLQWDWENLIMFNAITNENSKQLSLTDLET
  SEQID439   (10)  -------- --------QKSPVFWDWENPAPFGPNTMENPKSIPHPEPRG
  SEQID437   (10)  -------- --------QKSSVLWDWENMPPIGNSANENPKNVMLAESKL
  SEQID435    (5)  -------- --------AKPPFQWELENLISFGTSTAEVPRKLKPMEWEI
  SEQID433    (5)  -------- --------MVSSFPWDWENLIMSNQSKTENEKKQQSTEWEF
  SEQID428    (5)  -------- --------MVSSSQWDWEHLIMSNPSRTEDDSKQLPTEWEI
Consensus    (51)   KSSV                WDWENLI        E PK L    D E
```

**FIGURE 2**

```
                  101                                                150
   SEQID455 (100) EGEGGIDTGSFYSSGCGAGSGGSGSDLGHAYSSKSSKSASIDSSS-KVGI
   SEQID453  (39) AAVAATMGNEPLHSSGGSG-TFSSSSEMGYGSSKSSMSASIDSSNRAGNN
   SEQID451  (32) --------HGSANSSGG---TLTSNSELGHGSSKSSISASIDSPSGVGNS
   SEQID449  (35) ------------HRTTAAAPQFAGLEATGHEPAPSSVSSSIHSLPSAKGN
   SEQID447  (38) DGFDCTTFYSSSFATEAAYGGGSSGSDLAHAFSKSSKSTSISSS--SAEV
   SEQID445  (37) EGDGAIDKAITYSS----GGGLSSSDLWHCSSSKSSVSPSVDSSL-KGGI
   SEQID443  (38) DGEKGTDSGFFYSSGSASRSGGSSSDLELASFSKCSKSASINSSS-AGEV
   SEQID441  (44) DGEKGNDSGFFYSSGSVSRSRGSISDLELASFSKSSKSASTNSSS-AGEV
   SEQID439  (43) VVVAAAN-HGSTNSSGG---TFTSSSELANGSSKSSLSASFDSSSKLGNS
   SEQID437  (43) AGVGVDIGHESGHSSGG---TFSSSSEIGYGSSKSSISASIDSPSKVGNT
   SEQID435  (38) DGFDCTSLYSSSFAYAGSS-----GSDIAHAFSKSSKSTSISSS--SAEV
   SEQID433  (38) EKGEGIESIVPDFLGFEKVSSGSATSFWHTAVSKSSQSTSINSS--SPED
   SEQID428  (38) EKGEGIESIVPHFSGLERVSSGSATSFWHTAVSKSSQSTSINSS--SPEA
 Consensus (101) DG  G         S  GS    SSSS  G A SKSS SASI SS   AG

                  151                                                200
   SEQID455 (149) KTSNFTFEAFQDFSQDFNKKRDLERAEPTGSSPNLEASIGSGE-PLIGLK
   SEQID453  (88) MEFRFAPVKNPDRNTSKNTELGKVDNTRTGTSPSPVVAVSSGE-PVIGLK
   SEQID451  (71) LEFNFAAVERHVKNTGTN-----GRVDDSGNSPSSMIAFNQGE-PLISLK
   SEQID449  (73) KNKN-----------N---MELISFAPAKAPDKDTGSVLSSGEPVVLGLK
   SEQID447  (86) RTYNFTSEAGESVPPGEL-GSSEEFAMGIDASPSLELSFGSGD-PVLGLK
   SEQID445  (82) KTY-----STADGFPGVIIRKDLTRVESTENIPSLGASASSGE-PVIGLK
   SEQID443  (87) KTSKFTLEASKANPSDYN-KKEIGKAKTASMSSTIEAAGGSGD-QLLGLK
   SEQID441  (93) KTSKFTLGASKTNPSDYN-KEELVKAKATDTSPTLEASVGSGD-QLLGLK
   SEQID439  (89) LEFRFASVKGHGKNMCKDGE--AGRVEDSGTSP--AVAVSHGE-PVIGLK
   SEQID437  (90) IELNFASAEEHDKNMDK----GKSKVDDTGTSRSPVVAANRVE-PLIGLK
   SEQID435  (81) RTHNFTSETGESLP-----G---EFAKGIDTSPSLELSFGSGD-PVLGLK
   SEQID433  (86) KRCNLASQSSPGDS------SSNIDFLQVKPSTALEVPIASAE-SDLCLK
   SEQID428  (86) KRCKLASESSPGDS------CSNIDFVQVKAPTALEVSVASAE-SDLCLK
 Consensus (151) K  NF S A                R   T  SPSLEVSVGSGE PVLGLK

                  201                                                250
   SEQID455 (198) LGKRTYFEDVSAG-GNAKGSVF-SVIPISSDTTAKR-SRLSCQNAHV---
   SEQID453 (137) LGKRTYFEDVCGG-QNVKSSPSGAASAPNKSPALGKKAKAEQQKPHN---
   SEQID451 (115) LGKRAYFENACGG-QDAKVS---AASDVTSAASVVKKTKVSQQNAKN---
   SEQID449 (109) LGKRTYFEDGCGLGQSGKSSAALGTASAATPPGPAKKAKAAAAAPTAQQQ
   SEQID447 (134) LGKRTYFEDFWEV-ENAKGSAL-PVSLASSSASPVKKSKTLSQKLQT---
   SEQID445 (126) LGKRMYFEDICTT-STAKSSSS-SIVSTSCTATTKR-SRASYPSTQS---
   SEQID443 (135) LGKRIYFEDACAG-NNVQSSSF-STVPVPSFTSAKK-LKSTIQSQRA---
   SEQID441 (141) LGKRTYFEDACAG-SNAQSSSI-STIPVPSFTPAKK-LKSSNHSQRA---
   SEQID439 (134) LGKRTYFENVCGG-QNVKSSS--AASGVTCPSTVVKKMKVSQQSTQS---
   SEQID437 (135) LGKRTYFEDVCGG-QNVKSSPSGVSVATP-SPGLAKKVKVAQQNTQN---
   SEQID435 (122) LGKRTYFEDFWEV-ENAKGLGL-PVTLASSSVSPVKKSKSIPQRLQT---
   SEQID433 (129) LGKRTYSEEFWGR-NNNDLSAV-SMNLLTPSVVARKKTKSCGQSMQV---
   SEQID428 (129) LGKRTYSEEYWGR-NNNEISAV-SMKLLTPSVVAGK-SKLCGQSMPV---
 Consensus (201) LGKRTYFED CGG QNAKSSA  S   VSSS S  KKSKSS QS
```

**FIGURE 2 (continued)**

```
                    251                                               300
 SEQID455 (242) -SRCQVEGCNLDLSTAKDYHRKHRVCESHTKCPKVIVGGLERRFCQQCS-
 SEQID453 (183) -SYCQVEGCKVDLSSVKDYHRKHRVCELHSKAPKVVVAGLERRFCQQCSR
 SEQID451 (158) -WYCQVEGCKVDLSSAKDYNRKHKVCVVHSKATKVVVAGLERRFCQQCSR
 SEQID449 (159) KSYCQVEGCRTDLSSAKDYHRKHRVCEPHSKAPKVVVAGLERRFCQQCSR
 SEQID447 (179) -PHCQVEGCNLDLSSAKDYHRKHRICENHSKFPKVVVSGVERRFCQQCSR
 SEQID445 (170) -PRCQVEGCNLDLKSAKDYHRRHRICEKHSKSPKVIVAGMERRFCQQCS-
 SEQID443 (179) -PCCQVEGCNLDLSSAKDYHRKHRVCESHSKCQKVIVAGLERRFCQQCSS
 SEQID441 (185) -PRCQVEGCNLDLSSAKDYHRKHRVCESHSKCPKVIVAGLERRFCQQCS-
 SEQID439 (178) -SYCQVEGCKVDLSSAREYHRKHKVCEAHSKAPKVIVSGLERRFCQQCSR
 SEQID437 (180) -PHCQVEGCNVDLSSAKPYHRKHRVCEPHSKTLKVIVAGLERRFCQQCSR
 SEQID435 (167) -PHCQVEGCNLDLSSAKDYHRKHRICENHSKFPKVVVSGVERRFCQQCSR
 SEQID433 (174) -PRCQIDGCELDLSSSKDYHRKHRVCETHSKCPKVVVSGLERRFCQQCSR
 SEQID428 (173) -PRCQIDGCELDLSSAKGYHRKHKVCEKHSKCPKVSVSGLERRFCQQCSR
Consensus (251)  PYCQVEGCNLDLSSAKDYHRKHRVCE HSK PKVIVAGLERRFCQQCSR

                    301                                               350
 SEQID455 (290) --------------------RFHSLSEFDEKKRSCRRRLSDHNARRRKPQ
 SEQID453 (232) ---------------------FHALAEFDQKKRSCRRRLNDHNSRRRKPQ
 SEQID451 (207) ---------------------FHGLAEFDQNKRSCRRRLMHHNARRRKPQ
 SEQID449 (209) ---------------------FHGLAEFDQKKKSCRRRLNDHNARRRKPQ
 SEQID447 (228) KSSRYWFIQKPLFLIVFGLQRFHCLSEFDEKKRSCRRRLSDHNARRRKP-
 SEQID445 (218) --------------------RFHELSEFDDKKRSCRRRLSDHNARRRRPQ
 SEQID443 (228) KIIPSFY--DCIDNLGFGCQRFHGLSEFDEKKKSCRRRLSDHNARRRK-Q
 SEQID441 (233) --------------------RFHGLSEFDEKKKSCRRRLSDHNARRRK-Q
 SEQID439 (227) ---------------------FHGLAEFDQKKKSCRRRLSDHNARRRKPQ
 SEQID437 (229) ---------------------FHGLAEFDQKKRSCRRRLHDHNARRRKPQ
 SEQID435 (216) ---------------------FHCLSEFDEKKRSCRRRLSDHNARRRKP-
 SEQID433 (223) ---------------------FHAVSEFDEKKRSCRKRLSHHNARRRKPQ
 SEQID428 (222) ---------------------FHAVSEFDEKKRSCRKRLSHHNARRRKPQ
Consensus (301)                      FHGLSEFDEKKRSCRRRLSDHNARRRKPQ

                    351                                               400
 SEQID455 (320) PEGIQSNSARLSS-----------FYGGKQQMSLVLSSVPIVHTRPAVNP
 SEQID453 (261) PEAISFSSSRM-ST--------MFY-DARQQPNFLFGQAPYVQMRSCGSS
 SEQID451 (236) ADTISFNSS-------------TMFYDTRQRTNLFFSQPLYGQVRSNAGS
 SEQID449 (238) PEALPFGSSR----------LSAMFYDTRRQASLLFGEGPYGQMRSCASS
 SEQID447 (277) ------NPGRTY--------------------------------------
 SEQID445 (248) PEAIRFNSARPSSSPSSFYGCHFNFKDGRQQMNVVLNRVPFLAG----NS
 SEQID443 (275) PGSVHLN-SRVSSS----------LYDERQQMSLAWDRAPLVHARPNANL
 SEQID441 (262) PGSVHLNPSRLSSS----------LYDERQQMSHVWDKAPLVHSRPNANL
 SEQID439 (256) QEAISFGSS--RLA--------TMFYDARQQTDIYFGQSPFGQVRSNAIS
 SEQID437 (258) PEAISLSSSRL-ST--------LLYGDARQQASFLFGQAPYGQMGSCASS
 SEQID435 (244) ------NPGRTYDG--------------KPQVDFVWNRFALIHPRSEEKF
 SEQID433 (252) -GVFPLNSERVFDR--------------RQHTSMLWNGLSLNTR-SEEKY
 SEQID428 (251) -GVFSMNPERVYDR--------------RQHTNMLWNGVSLNAR-SEEMY
Consensus (351) PEAI  NSSRL            F D RQQ  LLF   P    RS A
```

FIGURE 2 (continued)

```
                     401                                                450
  SEQID455  (359)  SWEG--TCS--KFTQTKG--LVRPGNTGGIDRQLHLPGS---ELQNGIST
  SEQID453  (301)  SWDD--PGG--FKVTHTKAPWLKPTTAAGVHG-IHLSSQ---QMSDNIMP
  SEQID451  (273)  SWDN--LGG--LKFMETKHPPVHPTKTASPDE-LHFSAL---QITS-AAA
  SEQID449  (278)  WWDSPGAGGGGFKFAETRGAPWLKPARAAADGLLPLSGQQQQQVWNDSIP
  SEQID447  (283)  --------------------------------------------------
  SEQID445  (294)  TWQS--ECG--FKVTHAGDFFIRPAKAGGIHRQLSYPCN---EVPDAAST
  SEQID443  (314)  TWEG--TYI--SKFTITKDYIAKPAEIGGNDGQFHLPGF---DLTNGIDI
  SEQID441  (302)  TWES--TST--SKFTITKEYIAKPAEIGGSDRRLHLPGI---DLTNSIAI
  SEQID439  (296)  SCDN--LGG--FKFTEAKLPWMKPMKTIGLED-LNFSTL---QMPGNVVS
  SEQID437  (299)  -WDNPVPGG--FKFTATKAPWSRPTIAAGVDG-THVSNQ---QASGNVLP
  SEQID435  (274)  IWPS--SKH--VPSRVLMPQPAKTEISDTEHNRFGLLDP---KTKTARAE
  SEQID433  (286)  TWG-----------TTYETKPTQMESGFTLSFQRGNGSE---DQLFTGST
  SEQID428  (285)  EWGN----------NTYDTKPRQTEKSFTLSFQRGNGSE---DQLVASSS
 Consensus  (401)  SWD         K T  K    KP   AGLD  L L        L    S

                     451                                                500
  SEQID455  (400)  LCHDSSR-----LLHSKGAIAEVL-NQVSQGMLHST--------------
  SEQID453  (343)  HGAHHG-FD--GFMAFKGTCTKFP-NQGVQASAVASDSS--GAPDLQHAL
  SEQID451  (314)  HTGHHHDLD--GFMAFKGTSTKVL-NQGVEAWAAASSSN-NGGPEGGRAL
  SEQID449  (328)  HVAHQEDFDGFTVTAFKGISPKTLDHQGAGVEACAAVSSPDGAPDLQRAL
  SEQID447  (283)  --------------------------------------------------
  SEQID445  (337)  IPTDSDK-----IISFERSTPQVF-SQGFEGSALTSNIE--VAPDLRRAL
  SEQID443  (357)  QHHHKSNSS--LPSKGKGTAAEIL-NQGLEEYIIPSKAE--AAPESHRAL
  SEQID441  (345)  QHHHKSNGF--LPSKAKGTAGEVL-NQGLFCLHFEKQAS--QLLH-----
  SEQID439  (338)  HTVHHHDFD--GLIPFKGNTPKVL-NQGVDPACAVVSSNSNGAPDLRRAL
  SEQID437  (342)  HGAHHS-FD--GLMAFKETNAKVL-NQGMEASAVASGSA--RGPDFEHAL
  SEQID435  (317)  LFSKEKVT----ISSHMGASQDLD-GALSLLSNSTTWVS--SSDQPRRFT
  SEQID433  (322)  LSFSAFQT----SGGFSAGKSNIQ-LPDKGVGECSGGLH--ESHDFYSAL
  SEQID428  (322)  RMFSTSQT----SGGFPAGKSKFQ-LHGEDVGEYSGVLH--ESQDIHRAL
 Consensus  (451)    H           FKG    VL NQG       AS S     APD  RAL

                     501                                                550
  SEQID455  (430)  --------------------------------------------------
  SEQID453  (387)  SLLSSNPVG-------------AANLQPSPQMHSGVAAIAGAPNPAMHAL
  SEQID451  (360)  SLLSDGSWGSS--------S--AVIQQPTSHADAGALLPPLATVAVSNAA
  SEQID449  (378)  SLLSNSPAGGGGTNTNHPPPPPAAELHHHRAAPSSCLAAGSVSVLQEASS
  SEQID447  (283)  --------------------------------------------------
  SEQID445  (379)  SLLSTTSWGSN--------EHGSTSLDQLMLANQTSMTQPMINAELQNCP
  SEQID443  (402)  SLLSNNSWGSR--------EPQSISFEQPVHTN--HTTQSVLQVIPQNSP
  SEQID441  (385)  --------------------------------------------------
  SEQID439  (385)  SLLSSDSWG-------------PADVQAGSQVHPGGVMPPLAVAAATVTA
  SEQID437  (386)  SLLSIDSVG-------------AANLQPGSQIHPGVTAIAGTSNPVMMPS
  SEQID435  (360)  LDHHPSS-----------------------------------------NL
  SEQID433  (365)  SLLSTTS-----------------------------------------DS
  SEQID428  (365)  SLLSTSS-----------------------------------------DP
 Consensus  (501)  SLLS  S G
```

**FIGURE 2 (continued)**

```
                    551                                               600
  SEQID455 (430) ---SEHWRTELPSTDSRVHILPSQGDGNTHFQEFKLFKAPYESGFSSGYS
  SEQID453 (424) GSSTGLWLDGSQPLDDHPRFQVFERLG--DHDSELQLPKPSYDHASHFDR
  SEQID451 (400) ----AAAGHPLDPSPGR--FWPQDDHPPLVDGPATQIPELAHLRIW----
  SEQID449 (428) PGLWQDGGGSAALGHHAHARLQALDAAMATAQELLQLPRPPPSYGGSSSH
  SEQID447 (283) --------------------------------------------------
  SEQID445 (421) VASSENARMEQASLESRVHSLDLHDNGNVQLQEFQLLKAPYATGCFYSNQ
  SEQID443 (442) LASSEYWRTEQPSTDSQVHTLTSH--------------------------
  SEQID441 (385) --------------------------------------------------
  SEQID439 (422) PTNPVSVMHALHPSTGGGGFWQDGDDPPPLD-HASQAQAFMHPGNGSSSG
  SEQID437 (423) ---PAIWQGG-LSLDQQAQFQAFDRLGNDDDEDHLQLPKPSYDNS-HYDQ
  SEQID435 (369) QPVAHRSAAQLNSVSGYWQPDPPAVEGPTALHRNGVGQFNENYFSLNQFY
  SEQID433 (374) QGIKHTPVAEPPPIFGTFPSHFI---------------------------
  SEQID428 (374) LAQPHVQPFSLLCSYDVVPK------------------------------
Consensus  (551)

                    601                620
  SEQID455 (477) EYRELRYPIPRSNARVMKQT
  SEQID453 (472) MH------------------
  SEQID451 (440) --------------------
  SEQID449 (478) YDLMR---------------
  SEQID447 (283) --------------------
  SEQID445 (471) FS------------------
  SEQID443 (466) --------------------
  SEQID441 (385) --------------------
  SEQID439 (471) YGHLH---------------
  SEQID437 (468) MN------------------
  SEQID435 (419) N-------------------
  SEQID433 (397) --------------------
  SEQID428 (394) --------------------
Consensus  (601)
```

## FIGURE 2 (continued)

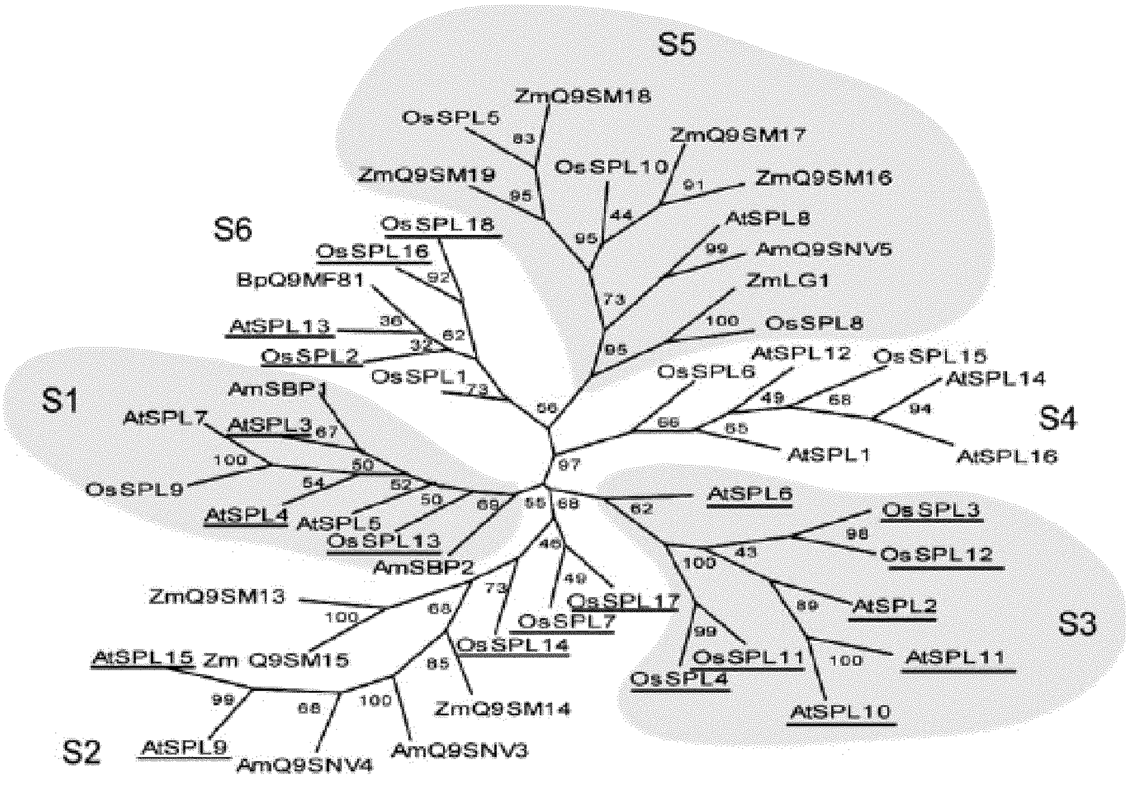


**FIGURE 3**

```
3' aua cga gu g aga gaa gac agc 5'  osmiR156l
3' aca cga ga g aga gaa gac agu 5'  osmiR156k
3'  ca cga gu g aga gaa gac agu 5'  osmiR156(a -j)
   •|| ||| |•| ||| ||| ||| |||
5' cau gcu ccc ucu cuu cug uca 3'   OsSPL13*
5' gau gcu ccc ucu cuu cug uca 3'   OsSPL3
5' ugu gcu cuc ucu cuu cug uca 3'   OsSPL2
5' ugu gcu cuc ucu cuu cug uca 3'   OsSPL14
5' ugu gcu cuc ucu cuu cug uca 3'   OsSPL16
5' ugu gcu cuc ucu cuu cug uca 3'   OsSPL17
5' ugu gcu cuc ucu cuu cug uca 3'   OsSPL18
5' cgu gcu cuc ucu cuu cug uca 3'   OsSPL4*
5' cgu gcu cuc ucu cuu cug uca 3'   OsSPL11
5' cgu gcu cuc ucu cuu cug uca 3'   OsSPL12
5' ggu gcu cuc ucu cuu cug uca 3'   OsSPL7
A(G/C) A  L  S  L  L  S   amino acid
```

**FIGURE 4A**

```
                  1                                                50
   SEQID427   (1) --------------------------------------------------
   SEQID436   (1) --------------------------------------------------
   SEQID438   (1) --------------------------------------------------
   SEQID440   (1) --------------------------------------------------
   SEQID442   (1) --------------------------------------------------
   SEQID444   (1) --------------------------------------------------
   SEQID446   (1) --------------------------------------------------
   SEQID448   (1) --------------------------------------------------
   SEQID450   (1) --------------------------------------------------
   SEQID452   (1) --------------------------------------------------
   SEQID454   (1) -------------------------------ATGGATGCTGTTGTTTTGA
   SEQID429   (1) --------------------------------------------------
   SEQID430   (1) CTGGGTGAAACATAGAAAAGTTTCTCTTGCTCAAGTTAATGATAAAAGGG
   SEQID431   (1) --------------------------------------------------
   SEQID432   (1) --------------------------------------------------
   SEQID434   (1) --------------------------------------------------
Consensus     (1)

                  51                                              100
   SEQID427   (1) --------------------------------------------------
   SEQID436   (1) --------------------------------------------------
   SEQID438   (1) --------------------------------------------------
   SEQID440   (1) --------------------------------------------------
   SEQID442   (1) --------------------------------------------------
   SEQID444   (1) --------------------------------------------------
   SEQID446   (1) --------------------------------------------------
   SEQID448   (1) --------------------------------------------------
   SEQID450   (1) --------------------------------------------------
   SEQID452   (1) --------------------------------------------------
   SEQID454  (20) CAGGTTTGGAAGAATCTGGGGTAAGAGTAAGGGTGGTTAATTTTATACAG
   SEQID429   (1) -----------------------------------CTTAGTCAATAGCAT
   SEQID430  (51) TGAGAGCAATAAACGCTGATAAGCCTTGTCTGGTCCTTGGAATTTTGAAT
   SEQID431   (1) --------------------------------------------------
   SEQID432   (1) ----------------------GCCCTTTTTGTTGGTCTGGGTGAAACAT
   SEQID434   (1) --------------------------------------------------
Consensus    (51)

                  101                                             150
   SEQID427   (1) --------------------------------------------------
   SEQID436   (1) --------------------------------------------------
   SEQID438   (1) --------------------------------------------------
   SEQID440   (1) --------------------------------------------------
   SEQID442   (1) --------------------------------------------------
   SEQID444   (1) --------------------------------------------------
   SEQID446   (1) --------------------------------------------------
   SEQID448   (1) --------------------------------------------------
   SEQID450   (1) --------------------------------------------------
   SEQID452   (1) --------------------------------------------------
   SEQID454  (70) GG--CGGGCGAGAAATGGCATTTTTGTTGGAATCTGTGCAAGAAGAGGAC
   SEQID429  (16) CTTCTCCGATCATCGACGTCCGGAGATTTCT--TCGGCCCTCTCATACTT
   SEQID430 (101) TTTCTTTTTCTATCTTACTTATAGTATTGGTAGTTGAGGGTGTCGTCGAT
   SEQID431   (1) --------------------------------------------------
   SEQID432  (29) AG-CAAGGTTTCTCTTGCTGAGGTTATTGATAAACGGGTGAGTTAAATAA
   SEQID434   (1) --------------------------------------------------
Consensus   (101)
```

# FIGURE 4B

```
                    151                                              200
  SEQID427    (1)   --------------------------------------------------
  SEQID436    (1)   --------------------------------------------------
  SEQID438    (1)   --------------------------------------------------
  SEQID440    (1)   --------------------------------------------------
  SEQID442    (1)   --------------------------------------------------
  SEQID444    (1)   --------------------------------------------------
  SEQID446    (1)   --------------------------------------------------
  SEQID448    (1)   --------------------------------------------------
  SEQID450    (1)   --------------------------------------------------
  SEQID452    (1)   --------------------------------------------------
  SEQID454  (118)   TTGGGTAGAAACATCATCGGTTCTACTTGGTCGACTTGGGGAAGAAAGGA
  SEQID429   (64)   GA--GTTGTTGTAGGATTTGTTGCTCTGGCTCTGGTGGT--AGGTCTATG
  SEQID430  (151)   AA--GTTGTTGTAGGATTTGTTGCTCTGGCTCTGGTGGT--AGGTCTATG
  SEQID431    (1)   --------------------------------------------------
  SEQID432   (78)   AAACGTTGATCAGCAGTGTGTGGTGCTTGGAATTCATA---AGGTCTATG
  SEQID434    (1)   --------------------------------------------------
Consensus   (151)

                    201                                              250
  SEQID427    (1)   -------------------ATGGACTGCAA--CATGGTATCTTC--GTCC
  SEQID436    (1)   ----ATGGGTTCTTTTGGGATGGACTGGAATCAGAAG-AGCTCGGTGTTG
  SEQID438    (1)   ----ATGGCTTCTTTTGGGATGAACTGGAATCAGAAG-AGCCCTGTGTTT
  SEQID440    (1)   -ATGAGTTCTGTCTCACTGATGGAGTGGAA--TGGCAAACCCCA--CTTG
  SEQID442    (1)   -------------------ATGGAGTGGAA--TGGCAAACCCCA--CTTA
  SEQID444    (1)   -------------------ATGGACTGGAA-CTCCACTGCATCTGAGG--
  SEQID446    (1)   -------------------ATGGAGTGTAA--TGCAAAGCCATC--ATTG
  SEQID448    (1)   ---AGCCGCTTCGGCAGGCAAGTAGGACGAGGAGCAGCAGCTCAGATTTC
  SEQID450    (1)   ----ATGGGTTCTTTTGGGATGAACTGGAATCAGAAG-GACCCCATGGTG
  SEQID452    (1)   ----ATGGGCTCGTTCGGGATGGAGTGGAACCAGAGG-AGCTCGGTGCTG
  SEQID454  (168)   CAATCGATATCCTTCTCTAAAAGGGGATAA--GGAATTTCCTTTCTGTGG
  SEQID429  (110)   AAATCAACCCATATCGTGAATGGACTGCAA--CATGGTATCTTC--GTCC
  SEQID430  (197)   AAATCAACCCATATCGTGAATGGACTGCAA--CATGGTATCTTC--GTCC
  SEQID431    (1)   -------------------ATGGACTGCAA--CATGGTATCTTC--GTCC
  SEQID432  (125)   AAATCAACCCACATCTTGAATGGACTGCAA--CATGGTATCTTC--GTTC
  SEQID434    (1)   -------------------ATGGAGTGTAA--TGCAAAGCCACC--GTTT
Consensus   (201)                T   G ATGGACTG AA   G AG A CTCC  GTT

                    251                                              300
  SEQID427   (28)   CA---GTGGGATTGGGAGCAT--TTGATCATGTCCAATCCGTCAAGGACT
  SEQID436   (46)   ------TGGGATTGGGAGAAT--ATGCCGCCGATAGGAAATAGCGCGAAC
  SEQID438   (46)   ------TGGGACTGGGAAAAT--CCAGCGCCTTTCGGTCCGAATACAATG
  SEQID440   (46)   CA---GTGGGACTGGGAAAAC--CTGATAATGTTCAATGCAATAACAAAT
  SEQID442   (28)   CA---GTGGGACTGGGAGAGC--CTGATAATGTTCAATGGAATAACAACT
  SEQID444   (29)   -------GGAACTGGGAGAAC--ATAGCAGGGTTAAGTGCCAAGGCTAGT
  SEQID446   (28)   CA---GTGGGAGTGGGATAAT--CTAATATCTTTTGGTACTTCATCAGCT
  SEQID448   (48)   GGATCCTGGCACTGGCAACTGGCATGGGCTCGTTTGGGATGGACATGGAC
  SEQID450   (46)   ------TGGGATTGGGAACAT--CTAGTACC----G-TCTG---------
  SEQID452   (46)   ------TGGGACTGGGAGAAT--TTCCCGCCGATAGG------------C
  SEQID454  (216)   CAT--GTGGGACTGGGAGAAC--CTTGACATTTCAATGCAAAATCAACT
  SEQID429  (156)   CA---GTGGGATTGGGAGCAT--TTGATCATGTCCAATCCGTCAAGGACT
  SEQID430  (243)   CA---GTGGGATTGGGAGCAT--TTGATCATGTCCAATCCGTCAAGGACT
  SEQID431   (28)   CA---GTGGGATTGGGAGCAT--TTGATCATGTCCAATCCGTCAAGGACT
  SEQID432  (171)   CC---GTGGGACTGGGAGAAT--TTGATCATGTCCAATCAGTCGAAGACT
  SEQID434   (28)   CA---ATGGGAATTGGAAAAC--TTGATATCTTTTGGCACTTCTACAGCT
Consensus   (251)   CA   GTGGGACTGGGAGAAT   TGAT ATGTTCAAT CG  AAC ACT
```

# FIGURE 4B (continued)

```
                      301                                              350
  SEQID427     (73)   GAAGATGACAGCAAAC---AGCTACCTACTGAGTGGGA--AATTGAAAAA
  SEQID436     (88)   GAGAATCCCAAGAAT---GTGATGCTGGCTGAATC--AAAACTTGCAGGT
  SEQID438     (88)   GAAAATCCCAAGAGCAT--A-CCTCACCCTGAACC--AAGAGGTGTA---
  SEQID440     (91)   GAAAATTCAAAGCAGT---TAAGCCTAACAGATTTGGA--AACTGATGGA
  SEQID442     (73)   GAAAATTCTAAGCAGT---TAAGCCCAACAGATTTGGA--AACTGATGGA
  SEQID444     (70)   GACATTCCAAAACAA---GTACCATTGGCATACC---ATGAGACAGAGGG
  SEQID446     (73)   GAAATTCCTAAAAAGC---AACGACCAATGGATTGGGA--AAATGATGGG
  SEQID448     (98)   TGGAACCAGAAGGCCTCCGTGCTGCTGTGGGACTGGGAGAACCTGCCGCC
  SEQID450     (74)   -----TCTCAAATGCA----------------------------------
  SEQID452     (76)   GAGAACCCCAAGAAC---GCGATGCAGGCCGATCC--AAGATTTGCCGCC
  SEQID454    (262)   GAAATTCCAAAAAAGT---TACAATCA---GACTGGGA--AATTGAAGGA
  SEQID429    (201)   GAAGATGACAGCAAAC---AGCTACCTACTGAGTGGGA--AATTGAAAAA
  SEQID430    (288)   GAAGATGACAGCAAAC---AGCTACCTACTGAGTGGGA--AATTGAAAAA
  SEQID431     (73)   GAAGATGACAGCAAAC---AGCTACCTACTGAGTGGGA--AATTGAAAAA
  SEQID432    (216)   GAAAATGAAAAAAAAC---AGCAATCTACTGAGTGGGA--ATTTGAAAAA
  SEQID434     (73)   GAAGTTCCTAGAAAGC---TAAAACCAATGGAGTGGGA--AATTGATGGA
Consensus     (301)   GAAAATCCCAA AA      GC ACC AC GA TGGGA  AATTGAAG A

                      351                                              400
  SEQID427    (118)   -GGTGAAGGAAT-TGAATCTATAGTTCCACATTTCTCAGGCCTTGAGA--
  SEQID436    (133)   -GTTGGGGTTGACATAGGCCAT-GAATCAGGCCATTCTT--CTGGTGGTA
  SEQID438    (130)   -GTTGTCGCGGCCGCAAATCAT-GGATCCACCAATTCAT--CCGGTGGCA
  SEQID440    (136)   -GAAAAAGGAAA-TGATTCTGGGTTTTTCTATTCATCTGGGAGTGTAA--
  SEQID442    (118)   -GAAAAAGGAAC-CGACTCTGGGTTTTTCTATTCATCTGGGAGTGCAA--
  SEQID444    (114)   AGATGGAGCAA----TTGATAAAGCAATAACTTATTCGT--CTGGTGGTG
  SEQID446    (118)   -TTTGATTGCACCACTTTTACTCGTCCAGCTTTGCTACAGAAGCAGCTT
  SEQID448    (148)   CGCAGCCGCAAACGGGAGCGAGAGCCACAGGACGACCG---CTGCCGCGC
  SEQID450     (85)   -GTTA---------CAAGGCAC-GGATCTGCTAATTCAT--CTGGTGGTA
  SEQID452    (121)   -GTTGCGGCTACCATGGGGAAT-GAACCGCTCCATTCTT--CTGGCGGTA
  SEQID454    (304)   -GAAGGAGGAAT-TGACACTGGTTCTTTCTATTCATCTGG--ATGTGGTG
  SEQID429    (246)   -GGTGAAGGAAT-TGAATCTATAGTTCCACATTTCTCAGGCCTTGAGA--
  SEQID430    (333)   -GGTGAAGGAAT-TGAATCTATAGTTCCACATTTCTCAGGCCTTGAGA--
  SEQID431    (118)   -GGTGAAGGAAT-TGAATCTATAGTTCCACATTTCTCAGGCCTTGAGA--
  SEQID432    (261)   -GGTGAAGGAAT-TGAATCTATAGTTCCAGATTTCTTAGGCTTTGAGA--
  SEQID434    (118)   -TTTGATTGCACCTCTCTGTATTCTTCAAGCTTTGCC-------TA----
Consensus     (351)    G TGAAGGAA  TGAATCTAT GTTCCA ATT  TCAGG CTTG G

                      401                                              450
  SEQID427    (164)   GAGTCAGTAGTGGCTCTGCCACCAGC---TTCTGGCACACTGCTGTATCG
  SEQID436    (179)   ------CTTTCTCTTCTAGCTCAGAG---ATTGGGTATGG---TTCATCC
  SEQID438    (176)   ------CGTTCACTTCTAGCTCGGAG---CTAGCCAATGG---TTCATCG
  SEQID440    (182)   GCAGAAGCCGTGGATCTATCTCTGAC---TTAGAACTTGCTTCTTTCTCA
  SEQID442    (164)   GCAGAAGCGGTGGTTCTAGCTCTGAT---TTGGAACTTGCTTCTTTCTCA
  SEQID444    (158)   ---------GTTTATCAAGCTCTGAT---TTGTGGCATTGCTCTTCATCC
  SEQID446    (167)   ATGGTGGTGGTAGTTCAGGTTCTGA-----TCTAGCTCA-TGCTTTCTCT
  SEQID448    (195)   ------CTCAGTTCGCGGGCCTTGAGGCCACAGGGCATGA---ACCGGCG
  SEQID450    (122)   ------CTCTTACTTCTAACTCAGAG---CTAGGGCATGG---TTCATCC
  SEQID452    (167)   GCGGCACCTTCTCTTCCAGCTCAGAG---ATGGGGTATGG---CTCTTCC
  SEQID454    (350)   CTGGTAGTGGTGGTTCTGGCTCTGAT---TTGGGACATGCATATTCATCA
  SEQID429    (292)   GAGTCAGTAGTGGCTCTGCCACCAGC---TTCTGGCACACTGCTGTATCG
  SEQID430    (379)   GAGTCAGTAGTGGCTCTGCCACCAGC---TTCTGGCACACTGCTGTATCG
  SEQID431    (164)   GAGTCAGTAGTGGCTCTGCCACCAGC---TTCTGGCACACTGCTGTATCG
  SEQID432    (307)   AAGTCAGTAGTGGCTCTGCTACTAGT---TTCTGGCACACTGCCGTATCA
  SEQID434    (156)   ----TGCTGGTAGTTCAGGTTCTGA-----TATAGCTCA-TGCTTTCTCT
Consensus     (401)     G  AGT GTGGTTCTGGCTCTGA    TT TGGCATGCT CTTTATC
```

**FIGURE 4B (continued)**

```
                    451                                               500
  SEQID427   (211)  AAAAGCTCACAGTCGACCTCTATCAACTCATCATCT---CCCGAAGCCAA
  SEQID436   (217)  AAGAGTTCCATATCCGCGTCGATCGATTCTCCGTCCAAAGT---GGGGAA
  SEQID438   (214)  AAGAGCTCCTTGTCAGCGTCGTTCGATTCCTCATCCAAGCT---GGGGAA
  SEQID440   (229)  AAGAGCTCGAAGTCAGCTTCCACCAATTCTTCATCAGCTGGGGAAGTTAA
  SEQID442   (211)  AAGTGCTCGAAGTCAGCTTCCATCAATTCTTCATCAGCTGGGGAAGTTAA
  SEQID444   (196)  AAGAGCTCAGTTTCACCCTCTGTCGACTCTTCATTGAAGGG---GGGGAT
  SEQID446   (211)  AAAAGCTCAAAGTCAACTTCCATAAGCTCTTCATCA---GCTGAAGTGAG
  SEQID448   (236)  CCTTCTTCCGTATCCTCGTCAATCCATTCTCTGCCCAGTGCCAAGGGGAA
  SEQID450   (160)  AAGAGCTCTATTTCAGCGTCCATTGATTCACCCTCTGGAGT---AGGGAA
  SEQID452   (211)  AAGAGCTCCATGTCCGCGTCGATCGATTCTTCGAACAGGGC---TGGGAA
  SEQID454   (397)  AAGAGCTCAAAATCAGCTTCAATTGATTCTTCATCAAAGGTGGGAATAAA
  SEQID429   (339)  AAAAGCTCACAGTCGACCTCTATCAACTCATCATCT---CCCGAAGCCAA
  SEQID430   (426)  AAAAGCTCACAGTCGACCTCTATCAACTCATCATCT---CCCGAAGCCAA
  SEQID431   (211)  AAAAGCTCACAGTCGACCTCTATCAACTCATCATCT---CCCGAAGCCAA
  SEQID432   (354)  AAAAGCTCGCAGTCGACCTCTATCAACTCATCATCT---CCCGAGGACAA
  SEQID434   (196)  AAAAGCTCAAAGTCAACTTCCATTAGCTCTTCATCA---GCTGAAGTAAG
Consensus    (451)  AAGAGCTCAAAGTCA C TC ATCAATTCTTCATC    GC GAAG  AA

                    501                                               550
  SEQID427   (258)  ACGATGCAAGCTTGCATCAGAA---------AGTTCCCCT---GGAGATT
  SEQID436   (264)  CACCATAGAGCTCAATTTTGCATCT---GCGGAAGAGCATGATAAGAACA
  SEQID438   (261)  CAGCTTAGAGTTCAGGTTTGCTTCT---GTCAAAGGGCATGGCAAGAACA
  SEQID440   (279)  AACATCCAAATTCACTTTGGGGGCCTCTAAAACAAATCCA---TCAGATT
  SEQID442   (261)  AACATCCAAATTCACTTTGGAGGCCTCTAAAGCAAATCCA---TCTGATT
  SEQID444   (243)  CAAGACTTATAGCA------CTGCA---GATGGTTTTCCTGGAGTTATCA
  SEQID446   (258)  AACATACAATTTCACATCAGAAGCTGGTGAAAGTGTTCCTCCTGGAGAAT
  SEQID448   (286)  CAAGAACAAGAACAA-----CAT-----GGAGCTCATCA-GTTTCGCACC
  SEQID450   (207)  CAGCTTAGAGTTCAACTTCGCCGCT---GTTGAGAGGCATGTTAAGAACA
  SEQID452   (258)  CAACATGGAGTTCAGATTTGCGCCT---GTCAAAAACCCTGATAGGAACA
  SEQID454   (447)  GACATCCAACTTCACTTTTGAGGCTTTCCAAGATTTTTCT---CAAGATT
  SEQID429   (386)  ACGATGCAAGCTTGCATCAGAA---------AGTTCCCCT---GGAGATT
  SEQID430   (473)  ACGATGCAAGCTTGCATCAGAA---------AGTTCCCCT---GGAGATT
  SEQID431   (258)  ACGATGCAAGCTTGCATCAGAA---------AGTTCCCCT---GGAGATT
  SEQID432   (401)  ACGATGCAATCTTGCATCACAA---------AGTTCCCCT---GGAGATT
  SEQID434   (243)  AACACACAATTTTACATCCGAAACTGGTGAAAGTCTTCCT---GGAGAAT
Consensus    (501)  AA AT CAAGTTCACAT  GAA C    G  AGT   CCT   GGAGATT

                    551                                               600
  SEQID427   (296)  CTTGCAGCAACATAGACTTTGTCCAGGT-------GA--------AGGCT
  SEQID436   (311)  TGGACAAGGGT-AAGAG-----TAAAGTTGATGACAC------CGGAACT
  SEQID438   (308)  TGTGCAAGGATGGCGAGGCCGGTAGAGTTGAAGACTC------GGGCACT
  SEQID440   (326)  A---CAATAAGGAAGAACTTGTGAAGGC-TAAGGCAAC-----TGATACT
  SEQID442   (308)  A---CAATAAGAAAGAAATTGGAAAGGC-TAAGACAGC-----TAGTATG
  SEQID444   (284)  TTAGAAAGGAC-TTGAC-----TAGGGTAGAAAGTAC------TGAGAAT
  SEQID446   (308)  TGGGGAGCAGTGAAGAGTTTGCAATGGG-------AAT-----TGATGCT
  SEQID448   (325)  TGCCAAAGCGC-CCGA-------CAAG-----GACAC------TGGTTCG
  SEQID450   (254)  CGGGCACGAACGGC---------AGAGTCGATGACTC------GGGGAAT
  SEQID452   (305)  CGAGCAAGAACACCGAGCTGGGTAAAGTTGATAACACGAGAACTGGAACA
  SEQID454   (494)  TTAACAAGAAGAGAGA-TTTGGAGAGGGCTGAGCCAAC-----TGGAAGT
  SEQID429   (424)  CTTGCAGCAACATAGACTTTGTCCAGGT-------GA--------AGGCT
  SEQID430   (511)  CTTGCAGCAACATAGACTTTGTCCAGGT-------GA--------AGGCT
  SEQID431   (296)  CTTGCAGCAACATAGACTTTGTCCAGGT-------GA--------AGGCT
  SEQID432   (439)  CTTCCAGCAACATAGATTTTCTCCAGGT-------GA--------AACCA
  SEQID434   (290)  T-----------------TGCAAAGGG-------GAT-----TGATACT
Consensus    (551)     GCAA AACA AGA  TTG  AAGGT      CAA      TGA ACT
```

## FIGURE 4B (continued)

```
                     601                                               650
  SEQID427   (331)   CCCACAGCTCTCGAGGTATCCGTTGCCTCAGCTGAATCAGATCTTTGTTT
  SEQID436   (349)   TCTCGATCACCGGTGGTAGCAGCCAATCGTGTAGAGCCCTTGATTGGCCT
  SEQID438   (352)   TCTCCAGC-----TG-TGGCAGTTAGCCATGGTGAGCCGGTAATAGGACT
  SEQID440   (367)   TCTCCCACGCTTGAAGCTTCAGTTGGTTCAGGTGACCAGCTGCTTGGTTT
  SEQID442   (349)   TCTTCCACAATTGAGGCTGCAGGTGGTTCAGGTGACCAGCTGCTTGGTTT
  SEQID444   (322)   ATTCCATCTCTGGGTGCTTCTGCTAGCTCTGGTGAACCAGTAATTGGCTT
  SEQID446   (346)   TCTCCAAGTCTTGAACTCTCCTTCGGCTCTGGTGATCCGGTTCTTGGTTT
  SEQID448   (356)   GTGC--TCAGCAGCGG-AG-AGCCGGTGGTGC-----------TAGGCCT
  SEQID450   (289)   TCTCCATCGTCAATGATAGCTTTCAACCAAGGAGAGCCATTAATCAGCCT
  SEQID452   (355)   TCTCCGTCGCCTGTGGTGGCAGTGAGCAGTGGAGAGCCGGTGATCGGCCT
  SEQID454   (538)   TCTCCAAACCTTGAAGCCTCGATTGGCTCTGGTGAACCTTTAATTGGTCT
  SEQID429   (459)   CCCACAGCTCTCGAGGTATCCGTTGCCTCAGCTGAATCAGATCTTTGTTT
  SEQID430   (546)   CCCACAGCTCTCGAGGTATCCGTTGCCTCAGCTGAATCAGATCTTTGTTT
  SEQID431   (331)   CCCACAGCTCTCGAGGTATCCGTTGCCTCAGCTGAATCAGATCTTTGTTT
  SEQID432   (474)   TCCACAGCTCTCGAGGTACCTATTGCCTCAGCTGAATCAGATCTTTGTTT
  SEQID434   (310)   TCTCCAAGTCTTGAACTTTCTTTTGGCTCTGGTGATCCGGTTCTCGGTTT
Consensus    (601)   TCTCCA CTCT GAGGTATC GTTGGCTCTGGTGA CC GT CTTGGTTT

                     651                                               700
  SEQID427   (381)   AAAACTAGGAAAGCGGACATACTCTGAAGAATACTGGGGT----AGAAAC
  SEQID436   (399)   GAAGCTTGGCAAGAGAACCTATTTTGAAGATGTCTGTGGA---GGGCAG-
  SEQID438   (396)   CAAGTTAGGGAAGAGAACTTACTTTGAAAATGTTTGTGGA---GGGCAG-
  SEQID440   (417)   GAAGCTTGGTAAAAGAACTTACTTTGAAGATGCTTGTGCT----GGGAGC
  SEQID442   (399)   GAAGCTTGGTAAACGAATATACTTTGAAGATGCTTGTGCT----GGCAAC
  SEQID444   (372)   GAAGCTTGGTAAACGGATGTACTTTGAAGACATTTGTACT----ACCAGC
  SEQID446   (396)   GAAGCTTGGTAAGAGGACATACTTTGAAGACTTTTGGGAA----GTGGAG
  SEQID448   (391)   GAAGCTTGGCAAGAGAACGTATTTCGAAGATGGTTGTGGACTAGGGCAG-
  SEQID450   (339)   GAAACTTGGGAAGAGGGCTTACTTCGAAAACGCCTGCGGA---GGACAG-
  SEQID452   (405)   GAAGCTTGGCAAGAGAACTTACTTCGAGGATGTCTGTGGA---GGGCAG-
  SEQID454   (588)   AAAGCTTGGTAAAAGGACATACTTCGAGGATGTTTCTGCA----GGGGGC
  SEQID429   (509)   AAAACTAGGAAAGCGGACATACTCTGAAGAATACTGGGGT----AGAAAC
  SEQID430   (596)   AAAACTAGGAAAGCGGACATACTCTGAAGAATACTGGGGT----AGAAAC
  SEQID431   (381)   AAAACTAGGAAAGCGGACATACTCTGAAGAATACTGGGGT----AGAAAC
  SEQID432   (524)   GAAACTAGGAAAGCGGACATACTCTGAAGAATTTTGGGGT----AGGAAC
  SEQID434   (360)   AAAGCTTGGTAAACGAACGTACTTTGAAGACTTTTGGGAA----GTGGAG
Consensus    (651)   GAAGCTTGG AAGCGGACATACTTTGAAGA GTTTGTGGT    GG AGC

                     701                                               750
  SEQID427   (427)   AATAATGAAATTTCAGCGGTTT------CTATGAAGTTGTTAACTCCATC
  SEQID436   (445)   AATGTCAAGAGTTCTCCATC--TGG----TGTGAGTGTGGCTACCCCATC
  SEQID438   (442)   AATGTCAAGAGCTCCTCTGC---AG---CTTCAGGTGTGACTTGTCCA--
  SEQID440   (463)   AATGCTCAGTCTTCATCAATAT------CTACGA--TTCCTGT-GCCTTC
  SEQID442   (445)   AATGTTCAGTCGTCATCATTTT------CTACAG--TTCCTGT-GCCCTC
  SEQID444   (418)   ACTGCCAAATCATCGTCTTC---------ATCTATTGTTTCCACTTCCTG
  SEQID446   (442)   AATGCTAAAGGTTCAGCACTTC------CGGTGAGCCTGGCGTCATCTTC
  SEQID448   (440)   AGCGGCAAGAGTTCGGCGGCGTTAGGCACTGCCAGTGCAGCGACCCC---
  SEQID450   (385)   GATGCCAAG-GTT--TCTGC---AG---CTTCAGACGTTACTTCTGCA--
  SEQID452   (451)   AATGTCAAGAGCTCGCCATC---GGGTGCTGCGAGCGCGCCAAACAAATC
  SEQID454   (634)   AATGCTAAGGGCTCAGTGTTTT------CTGTGA--TTCCCAT-ATCATC
  SEQID429   (555)   AATAATGAAATTTCAGCGGTTT------CTATGAAGTTGTTAACTCCATC
  SEQID430   (642)   AATAATGAAATTTCAGCGGTTT------CTATGAAGTTGTTAACTCCATC
  SEQID431   (427)   AATAATGAAATTTCAGCGGTTT------CTATGAAGTTGTTAACTCCATC
  SEQID432   (570)   AATAATGACCTTTCAGCGGTTT------CTATGAATTTGTTGACTCCATC
  SEQID434   (406)   AATGCTAAAGGTTTGGGACTTC------CAGTGACTCTGGCTTCATCTTC
Consensus    (701)   AATG TAAGAGTTCAGC GTTT       CT TGA  TTG C ACTCCATC
```

**FIGURE 4B (continued)**

```
                   751                                                800
  SEQID427   (471) TGTTGTCGCTGGGAA---ATCCAAATTGTGT----------GGTCAGAGC
  SEQID436   (489) TCCTGGTCTGGCCAAGAAGGTGAAGGTGGCT----------CAGCAGAAC
  SEQID438   (484) -TCTACTGTGGTCAAGAAGATGAAGGTGTCT----------CAGCAGAGC
  SEQID440   (504) TTTTACTCCAGCAAAGAAATTGAAGTCCAGT----------AATCATAGT
  SEQID442   (486) TTTTACTTCAGCAAAGAAATTGAAGTCCACT----------ATTCAGAGT
  SEQID444   (459) TACTGCTACAACAAAGAGATCTAGGGCATCA----------TATCCAAGT
  SEQID446   (486) TGCTTCTCCGGTGAAGAAATCCAAAACACTC----------TCTCAGAAA
  SEQID448   (487) TCCGGGTCCTGCGAAGAAGGCGAAGGCGGCGGCGGCGGCTCCAACCGCGC
  SEQID450   (424) -GCCAGCGTGGTCAAGAAGACTAAGGTGTCT----------CAGCAGAAT
  SEQID452   (498) TCCTGCTTTGGGCAAGAAGGCAAAGGCGGAA----------CAACAGA--
  SEQID454   (675) TGATACCACAGCAAAGAGATCCAGGTTATCT----------TGTCAGAAT
  SEQID429   (599) TGTTGTCGCTGGGAA---ATCCAAATTGTGT----------GGTCAGAGC
  SEQID430   (686) TGTTGTCGCTGGGAA---ATCCAAATTGTGT----------GGTCAGAGC
  SEQID431   (471) TGTTGTCGCTGGGAA---ATCCAAATTGTGT----------GGTCAGAGC
  SEQID432   (614) TGTTGTTGCTCGGAAGAAAACCAAATCGTGT----------GGTCAGAGC
  SEQID434   (450) TGTTTCTCCCGTGAAGAAATCGAAATCCATT----------CCTCAGAGG
Consensus    (751) TGTTGCT C G GAAGAAATC AAGTTGT T           TCAGAGC

                   801                                                850
  SEQID427   (508) ATG--CCAGTCCCGCGTTGCCAAATTGATGGCTGTGAACTGGATCTCTCA
  SEQID436   (529) ACT--CAGAACCCACACTGTCAAGTTGAAGGCTGCAATGTTGATCTCTCT
  SEQID438   (523) ACA--CAAAGCTCATACTGCCAAGTTGAAGGCTGCAAAGTCGATCTGTCC
  SEQID440   (544) CAG--CGTGCTCCACGCTGTCAAGTAGAAGGCTGTAACCTTGACCTCTCA
  SEQID442   (526) CAG--CGTGCTCCATGCTGTCAAGTGGAAGGCTGTAACCTTGACCTCTCA
  SEQID444   (499) ACA--CAGTCTCCACGTTGCCAAGTGGAAGGATGCAACCTTGATCTCAAG
  SEQID446   (526) TTA--CAAACTCCTCACTGCCAAGTTGAAGGCTGTAACCTCGATCTCTCC
  SEQID448   (537) AGCAGCAGAAATCGTACTGCCAGGTTGAAGGCTGCAGGACCGATCTGTCC
  SEQID450   (463) GCA--AAGAACTGGTACTGTCAGGTTGAAGGGTGCAAAGTTGACCTGTCT
  SEQID452   (536) AGCCACATAACTCGTACTGTCAGGTTGAAGGCTGCAAAGTCGACCTCTCT
  SEQID454   (715) GCA--CATGTCTCGCGCTGCCAAGTTGAAGGATGCAACCTTGACCTTTCA
  SEQID429   (636) ATG--CCAGTCCCGCGTTGCCAAATTGATGGCTGTGAACTGGATCTCTCA
  SEQID430   (723) ATG--CCAGTCCCGCGTTGCCAAATTGATGGCTGTGAACTGGATCTCTCA
  SEQID431   (508) ATG--CCAGTCCCGCGTTGCCAAATTGATGGCTGTGAACTGGATCTCTCA
  SEQID432   (654) ATG--CAAGTTCCGCGTTGCCAAATTGATGGCTGTGAGCTGGATCTCTCA
  SEQID434   (490) TTA--CAAACTCCTCACTGTCAAGTTGAAGGCTGTAATCTTGATCTTTCA
Consensus    (801) A    CAAG CCCGCGCTGCCAAGTTGAAGGCTGTAA CT GATCTCTCA

                   851                                                900
  SEQID427   (556) TCTGCTAAGGGTTATCATCGTAAGCACAAAGTCTGCGAAAAGCATTCAAA
  SEQID436   (577) TCTGCTAAACCTTACCATCGAAAGCACCGAGTCTGTGAACCTCACAGCAA
  SEQID438   (571) TCCGCAAGAGAATACCATCGCAAGCACAAAGTTTGTGAAGCTCATTCTAA
  SEQID440   (592) TCAGCTAAAGATTACCATCGCAAACATAGAGTTTGTGAAAGCCATTCAAA
  SEQID442   (574) TCAGCTAAAGATTATCATCGCAAACATAGAGTTTGTGAAAGCCATTCAAA
  SEQID444   (547) TCAGCTAAAGATTACCATCGCAGGCATAGAATCTGTGAAAAACATTCCAA
  SEQID446   (574) TCAGCTAAGGACTATCATAGGAAACACAGGATTTGTGAAAACCATTCAAA
  SEQID448   (587) TCTGCTAAAGACTATCATCGCAAGCACAGAGTCTGCGAGCCCCATTCCAA
  SEQID450   (511) TCTGCTAAAGATTACAATCGCAAGCACAAGGTCTGTGTAGTCCATTCTAA
  SEQID452   (586) TCTGTTAAAGATTACCATCGAAAGCACAGAGTCTGTGAACTTCACTCTAA
  SEQID454   (763) ACAGCCAAAGATTACCATCGCAAACATAGAGTTTGTGAAAGTCATACCAA
  SEQID429   (684) TCTGCTAAGGGTTATCATCGTAAGCACAAAGTCTGCGAAAAGCATTCAAA
  SEQID430   (771) TCTGCTAAGGGTTATCATCGTAAGCACAAAGTCTGCGAAAAGCATTCAAA
  SEQID431   (556) TCTGCTAAGGGTTATCATCGTAAGCACAAAGTCTGCGAAAAGCATTCAAA
  SEQID432   (702) TCTTCTAAGGATTATCATCGCAAGCATAGAGTCTGCGAAACGCATTCAAA
  SEQID434   (538) TCAGCTAAAGACTATCATCGGAAACATAGGATTTGTGAAAATCATTCAAA
Consensus    (851) TCTGCTAAAGATTATCATCGCAAGCACAGAGTCTGTGAAA  CATTCAAA
```

# FIGURE 4B (continued)

```
                   901                                               950
  SEQID427  (606) GTGCCCAAAAGTTAGCGTGAGTGGCCTGGAACGTCGGTTCTGCCAACAGT
  SEQID436  (627) GACTCTTAAAGTCATCGTTGCGGGTCTTGAGCGACGCTTTTGCCAACAGT
  SEQID438  (621) GGCACCAAAGGTTATTGTTTCTGGTCTGGAGCGCCGTTTTTGCCAACAGT
  SEQID440  (642) GTGCCCAAAGGTCATTGTAGCTGGTTTGGAACGCAGGTTTTGCCAGCAGT
  SEQID442  (624) GTGCCAGAAGGTCATTGTAGCTGGTTTGGAACGCAGGTTTTGCCAGCAGT
  SEQID444  (597) AAGCCCAAAGGTTATTGTTGCTGGCATGGAACGCCGATTTTGCCAACAGT
  SEQID446  (624) GTTCCCTAAAGTCGTTGTCAGTGGCGTAGAGCGCCGGTTCTGCCAACAAT
  SEQID448  (637) GGCGCCCAAGGTGGTCGTCGCTGGCCTGGAGCGGCGCTTCTGCCAGCAGT
  SEQID450  (561) AGCTACCAAGGTGGTTGTTGCTGGTCTAGAGCGTCGGTTTTGTCAACAGT
  SEQID452  (636) GGCTCCGAAAGTTGTTGTCGCTGGTCTGGAGCGACGCTTTTGCCAACAGT
  SEQID454  (813) ATGCCCAAAGGTCATTGTAGGTGGTCTGGAGCGCCGTTTTTGCCAACAGT
  SEQID429  (734) GTGCCCAAAAGTTAGCGTGAGTGGCCTGGAACGTCGGTTCTGCCAACAGT
  SEQID430  (821) GTGCCCAAAAGTTAGCGTGAGTGGCCTGGAACGTCGGTTCTGCCAACAGT
  SEQID431  (606) GTGCCCAAAAGTTAGCGTGAGTGGCCTGGAACGTCGGTTCTGCCAACAGT
  SEQID432  (752) GTGCCCAAAAGTTGTTGTGAGTGGCCTGGAACGTCGTTTCTGCCAACAGT
  SEQID434  (588) GTTTCCTAAAGTCGTTGTGAGTGGCGTAGAGCGTCGGTTCTGCCAACAAT
Consensus   (901) GTGCCCAAAAGTTATTGT GGTGGCCTGGAGCG CGGTTTTGCCAACAGT

                   951                                              1000
  SEQID427  (656) GTAGCAGGT-----------------------------------------
  SEQID436  (677) GTAGTCGGT-----------------------------------------
  SEQID438  (671) GTAGTCGGT-----------------------------------------
  SEQID440  (692) GTAGCAG-------------------------------------------
  SEQID442  (674) GTAGCAGTAAGATTATTCCTAGCTTTTATGATTGCATTGACAATTTG---
  SEQID444  (647) GCAGCAGGT-----------------------------------------
  SEQID446  (674) GTAGCAGGTAGAAAAGCTAGTCTTAGAGATATTGGTTTATACAAAAGCCT
  SEQID448  (687) GCAGCCGGT-----------------------------------------
  SEQID450  (611) GTAGCCGTT-----------------------------------------
  SEQID452  (686) GCAGCCGGT-----------------------------------------
  SEQID454  (863) GTAGCAGGT-----------------------------------------
  SEQID429  (784) GTAGCAGGT-----------------------------------------
  SEQID430  (871) GTAGCAGGT-----------------------------------------
  SEQID431  (656) GTAGCAGGT-----------------------------------------
  SEQID432  (802) GTAGCAGGT-----------------------------------------
  SEQID434  (638) GTAGCAGGT-----------------------------------------
Consensus   (951) GTAGCAGGT

                   1001                                             1050
  SEQID427  (665) -------------------------------------TCCATGCTGTCTC
  SEQID436  (686) -------------------------------------TTCACGGTTTAGC
  SEQID438  (680) -------------------------------------TTCATGGTTTAGC
  SEQID440  (699) -----------------------------------GTTCCATGGTCTGTC
  SEQID442  (721) ------------------GGTTTTGGATGTCAAAGGTTCCATGGCCTGTC
  SEQID444  (656) -------------------------------------TCCATGAATTATC
  SEQID446  (724) TAATGACTTTTTTTAATTGTTTTTGGTCTTCAAAGGTTTCACTGTCTCTC
  SEQID448  (696) -------------------------------------TCCATGGACTGGC
  SEQID450  (620) -------------------------------------TTCATGGTTTAGC
  SEQID452  (695) -------------------------------------TTCATGCTTTAGC
  SEQID454  (872) -------------------------------------TCCATAGTTTGTC
  SEQID429  (793) -------------------------------------TCCATGCTGTCTC
  SEQID430  (880) -------------------------------------TCCATGCTGTCTC
  SEQID431  (665) -------------------------------------TCCATGCTGTCTC
  SEQID432  (811) -------------------------------------TCCATGCTGTCTC
  SEQID434  (647) -------------------------------------TCCACTGTCTCTC
Consensus  (1001)                                      TCCATGGT T TC
```

**FIGURE 4B (continued)**

```
                      1051                                              1100
  SEQID427   (678)  TGAATTTGATGAGAAGAAACGAAGCTGCCGAAAACGTCTTTCTCATCATA
  SEQID436   (699)  TGAGTTCGACCAGAAAAAACGAAGCTGTCGCAGGCGCCTCCATGATCACA
  SEQID438   (693)  TGAATTTGACCAGAAAAAGAAAAGTTGCCGCAGGCGTCTATCTGATCATA
  SEQID440   (714)  AGAGTTTGATGAAAAGAAGAAAAGCTGCCGCAGGCGACTTTCTGATCACA
  SEQID442   (753)  AGAGTTCGATGAAAAGAAGAAAAGCTGTCGCAGGCGACTTTCTGATCACA
  SEQID444   (669)  AGAGTTTGATGACAAGAAGCGAAGCTGTCGTAGACGTCTCTCTGATCACA
  SEQID446   (774)  TGAGTTTGATGAGAAGAAACGTAGCTGTCGCCGGCGTCTCTCAGATCACA
  SEQID448   (709)  CGAGTTCGACCAGAAGAAGAAGAGCTGCCGCAGGCGACTCAACGACCACA
  SEQID450   (633)  GGAGTTTGATCAGAACAAACGAAGCTGTCGTAGGCGTCTGATGCATCATA
  SEQID452   (708)  TGAGTTTGACCAGAAAAAGCGAAGCTGCCGTAGGCGTCTCAATGATCATA
  SEQID454   (885)  AGAGTTTGATGAAAAGAAGAGAAGCTGTCGCAGGCGTCTTTCTGATCACA
  SEQID429   (806)  TGAATTTGATGAGAAGAAACGAAGCTGCCGAAAACGTCTTTCTCATCATA
  SEQID430   (893)  TGAATTTGATGAGAAGAAACGAAGCTGCCGAAAACGTCTTTCTCATCATA
  SEQID431   (678)  TGAATTTGATGAGAAGAAACGAAGCTGCCGAAAACGTCTTTCTCATCATA
  SEQID432   (824)  AGAATTTGATGAAAAGAAACGAAGCTGCCGCAAACGTCTTTCTCATCATA
  SEQID434   (660)  TGAGTTTGATGAGAAGAAACGTAGCTGTCGCCGACGTCTCTCAGATCACA
Consensus   (1051)  TGAGTTTGATGAGAAGAAACGAAGCTGCCGCAGGCGTCTTTCTGATCATA

                      1101                                              1150
  SEQID427   (728)  ATGCGAGGCGTCGTAAGCCACA-------AGGAG-TATT-----------
  SEQID436   (749)  ATGCCCGCAGACGGAAGCCACAACC----AGAAGCAATT-----------
  SEQID438   (743)  ATGCACGAAGAAGGAAACCGCAACA----AGAGGCAATT-----------
  SEQID440   (764)  ATGCAAGACGCCGCAAACAGCC-------AGGATCTGTC-----------
  SEQID442   (803)  ATGCAAGACGCCGCAAACAACC-------AGGATCGGTC-----------
  SEQID444   (719)  ATGCAAGGCGACGGAGGCCACAACC----TGAGGCAATC-----------
  SEQID446   (824)  ATGCAAGACGTCGCAAGCCAAATCCCGGGAGGACATATGGTAATATACTA
  SEQID448   (759)  ACGCGCGCAGGCGGAAGCCCCAGCC----CGAAGCGCTC-----------
  SEQID450   (683)  ATGCACGGAGGAGGAAACCTCAGGC----AGACACAATT-----------
  SEQID452   (758)  ATTCCCGCAGGCGGAAGCCACAGCC----AGAAGCAATT-----------
  SEQID454   (935)  ATGCACGCCGGCGCAAACCACAACC----AGAAGGGATC-----------
  SEQID429   (856)  ATGCGAGGCGTCGTAAGCCACA-------AGGAG-TATT-----------
  SEQID430   (943)  ATGCGAGGCGTCGTAAGCCACA-------AGGAG-TATT-----------
  SEQID431   (728)  ATGCGAGGCGTCGTAAGCCACA-------AGGAG-TATT-----------
  SEQID432   (874)  ATGCAAGGCGTCGCAAGCCACA-------AGGAG-TATT-----------
  SEQID434   (710)  ATGCAAGACGTCGCAAGCCAAATCCTGGAAGGACATATG-----------
Consensus   (1101)  ATGCAAG CG CG AAGCCACA CC    AGGAGCTATT

                      1151                                              1200
  SEQID427   (759)  --TTCAATG------AATCCCG----------------------------
  SEQID436   (784)  ---TCCTTA------AGTTCGTC---------------------------
  SEQID438   (778)  ---TCATTT------GGTTCATC---------------------------
  SEQID440   (796)  ----CATTTGAAT--CCTTCA-----------------------------
  SEQID442   (835)  ----CATTTAAA-----TTCA-----------------------------
  SEQID444   (754)  ---CGGTTC------AATTCAGC---------------------------
  SEQID446   (874)  GATTCACTTCTCTCTAATCCTATATATTAAATCTAAATTTTTAGTTATAT
  SEQID448   (794)  ---CCTTTC------GGCTCATC---------------------------
  SEQID450   (718)  ---TCATTC------AATTCATC---------------------------
  SEQID452   (793)  ---TCTTTC------AGTTCATC---------------------------
  SEQID454   (970)  ----CAGTC-----AAATTCGGC---------------------------
  SEQID429   (887)  --TTCAATG------AATCCCG----------------------------
  SEQID430   (974)  --TTCAATG------AATCCCG----------------------------
  SEQID431   (759)  --TTCAATG------AATCCCG----------------------------
  SEQID432   (905)  --TCCACTG------AATTCAG----------------------------
  SEQID434   (749)  --------------------------------------------------
Consensus   (1151)     TCATT       AATTCA
```

**FIGURE 4B (continued)**

```
                    1201                                              1250
  SEQID427   (773)  ----AGAGGGT----------------------------GTATG-----A
  SEQID436   (798)  -----GAGGCTCTCTA----------------CATTACTCTATGG--TGA
  SEQID438   (792)  -----AAGGCTCGCCA----------------CGATGTTTTACG-----A
  SEQID440   (811)  ----AGACTTTCTTCA---TCATTA---------------TATG-----A
  SEQID442   (847)  ----AGAGTGTCTTCA---TCATTA---------------TATG-----A
  SEQID444   (768)  -----AAGGCCATCAT----------------CATCACCATCATC--ATT
  SEQID446   (924)  AATTAGTAGGTTTCCCTTTTCTAAGATAAATATCATTCTGTATGTGCAGA
  SEQID448   (808)  -----GAGGCTGTCGG----------------CGATGTTCTATG-----A
  SEQID450   (732)  -----GA-------------------------CAATGTTTTATG-----A
  SEQID452   (807)  -----AAGGATGTCTA----------------CGATGTTTTATG-----A
  SEQID454   (984)  -----GAGGCTTTCATCATTC-------------------TATG-----G
  SEQID429   (901)  ----AGAGGGT----------------------------GTATG-----A
  SEQID430   (988)  ----AGAGGGT----------------------------GTATG-----A
  SEQID431   (773)  ----AGAGGGT----------------------------GTATG-----A
  SEQID432   (919)  ----AGAGGGT----------------------------GTTCG-----A
  SEQID434   (749)  -------------------------------------------------A
Consensus   (1201)      AGAGG T T                           TATG     A

                    1251                                              1300
  SEQID427   (786)  TCGAAGACAGCATACAAATATGTTGTGGAAT---GGGGTGTCCCTTAACG
  SEQID436   (825)  TGCAAGGCAACAGGCAAGTTTTCTATTTGGT---CAAGCTCCTTATGGTC
  SEQID438   (816)  TGCAAGGCAGCAGACAGATATTTACTTTGGC---CAATCTCCTTTTGGCC
  SEQID440   (834)  TGAGAGGCAACAGATGAGTCATGTTTGGGAC---AAGGCGCCACTTGTTC
  SEQID442   (870)  TGAAAGGCAACAGATGAGTCTTGCTTGGGAC---AGGGCACCACTTGTTC
  SEQID444   (795)  TTATGGTTGTCATTTCAATTTCAAAGATGGTAGACAGCAGATGAATGTTG
  SEQID446   (974)  TGGGAAGCAACAGATGGATTTTGTATGGAAC---AGATTTGCACTTATCC
  SEQID448   (832)  CACGAGGCGCCAGGCGAGCCTCCTGTTTGGT---GAAGGTCCCTACGGTC
  SEQID450   (747)  TACAAGGCAGCGGACAAATCTTTTCTTTAGT---CAACCACTTTATGGCC
  SEQID452   (831)  TGCAAGGCAACAGCCAAATTTCCTATTTGGT---CAGGCTCCTTATGTTC
  SEQID454  (1005)  TGGGAAGCAGCAAATGAGTCTTGTGTTGAGC---TCGGTCCCCATTGTTC
  SEQID429   (914)  TCGAAGACAGCATACAAATATGTTGTGGAAT---GGGGTGTCCCTTAACG
  SEQID430  (1001)  TCGAAGACAGCATACAAATATGTTGTGGAAT---GGGGTGTCCCTTAACG
  SEQID431   (786)  TCGAAGACAGCATACAAATATGTTGTGGAAT---GGGGTGTCCCTTAACG
  SEQID432   (932)  TCGAAGACAGCATACAAGTATGTTGTGGAAT---GGGTTGTCCCTTAACA
  SEQID434   (750)  TGGGAAACCACAGGTGGATTTTGTATGGAAC---AGATTTGCACTTATCC
Consensus   (1251)  TGGAAGGCAGCAGACAAAT TT T TGGAAT    GGGT CC CTTG CC

                    1301                                              1350
  SEQID427   (833)  ---CGAGATCTGAAGAAATGTATGAATGGGG---TAAT-----------A
  SEQID436   (872)  AGATGGGAAGCTGTGCAAGTTCTTGG---GA---TAACCCAGTACCAGGA
  SEQID438   (863)  AAGTGAGAAGCAATGCAATTTCTTCATGTGA---CAACCTGG------GA
  SEQID440   (881)  ATTCCAGGCCCAATGCAAATTTGACATGGGA---AAGCACAT-----CCA
  SEQID442   (917)  ATGCCAGGCCTAATGCAAATTTGACGTGGGA---AGGCACAT-----ACA
  SEQID444   (845)  TATTGAATAGG-GTGCCTTTCCTGGCAGGAAATTCAACTTGGCAAAGCGA
  SEQID446  (1021)  ATCCAAGAAGTGAAGAAAATTTTCTGTGGCC---AAATCC-------GAA
  SEQID448   (879)  AGATGAGAAGCTGTGCGAGTTCTTGGTGGGA---TAGCCCAGGAGCAGGA
  SEQID450   (794)  AAGTGAGGAGCAATGCAGGGTCTTCATGGGA---TAACTTGG------GA
  SEQID452   (878)  AAATGAGAAGCTGTGGAAGTTCTTCATGGGA---TGACCCAG------GA
  SEQID454  (1052)  ATACAAGGCCTGCTGTAAATCCTTCATGGGA---AGGCACAT-----GCA
  SEQID429   (961)  ---CGAGATCTGAAGAAATGTATGAATGGGG---TAAT-----------A
  SEQID430  (1048)  ---CGAGATCTGAAGAAATGTATGAATGGGG---TAAT-----------A
  SEQID431   (833)  ---CGAGATCTGAAGAAATGTATGAATGGGG---TAAT-----------A
  SEQID432   (979)  ---CGAGATCTGAAGAAAAGTATACATGGGG---TAC-------------
  SEQID434   (797)  ATCCAAGAAGTGAGGAAAAGTTTATATGGCC---AAGTTC-------GAA
Consensus   (1301)  A  CGAGAAGTGATG AA TT T  ATGGGA    TAAC C         A
```

## FIGURE 4B (continued)

```
                  1351                                              1400
   SEQID427   (866) A----------CACTT----ATGATACAAAG-----------------CC
   SEQID436   (916) GG------CTTCAAATTTACAGCAACAAAAG---CTCCTTGGTCAAGGCC
   SEQID438   (904) GG------CTTCAAATTTACAGAAGCAAAAC---TCCCCTGGATGAAGCC
   SEQID440   (923) CC----T---CCAAGTTCACAATAACAAAAGA---GTATATAGCAAAGCC
   SEQID442   (959) TC----T---CCAAGTTCACAATAACAAAAGA---TTATATAGCAAAGCC
   SEQID444   (894) GT--GCGGCTTTAAAGTCACTCACGCCGGAG---ATTTTTTATTAGGCC
   SEQID446  (1061) G----------CCTGT----ATCATCAAGAGG---GTTATTGCAGGAACC
   SEQID448   (926) GGAGGAGGCTTCAAATTCGCGGAGACGAGAGGAGCTCCTTGGTTGAAGCC
   SEQID450   (835) GG------CTTAAAATTCATGGAGACGAAAC---ATCCGCCAGTGCATCC
   SEQID452   (919) GG------CTTCAAAGTTACACACACAAAAG---CTCCTTGGTTAAAACC
   SEQID454  (1094) G----------CAAGTTCACACAAACAAAAGG---GTTAGTAAGG---CC
   SEQID429   (994) A----------CACTT----ATGATACAAAG-----------------CC
   SEQID430  (1081) A----------CACTT----ATGATACAAAG-----------------CC
   SEQID431   (866) A----------CACTT----ATGATACAAAG-----------------CC
   SEQID432  (1010) -----------CACTT----ATGAGACAAAG-----------------CC
   SEQID434   (837) G----------CACGT----ACCATCAAGAGT---GTTAATGCCGCAACC
  Consensus  (1351) G          CAA TT ACA  A CAAAAG                A CC

                  1401                                              1450
   SEQID427   (885) TAGACAAACGGAAAAAAGCTTTACTCTG---------AGCTTCCAGAGAG
   SEQID436   (957) AACAATAGCTGCCGGTGTTGATGGGACGCATGTATCTAATCAGCAG--GC
   SEQID438   (945) AATGAAAACTATAGGCCTTGAGGATCTGAATTTCTCTACCCTGCAG--AT
   SEQID440   (963) TGCAGAAATAGGTGGTAGTGATAGGAGGCTCCACT-TGCCTGGCATTGAT
   SEQID442   (999) TGCAGAAATAGGTGGTAATGATGGGCAGTTTCACT-TGCCTGGCTTTGAT
   SEQID444   (939) TGCAAAAGCAGGGGGCATCCATAGGCAGCTTAGCT-ATCCCTGCAA----
   SEQID446  (1094) TGCAAAGACCGAGATGCCCAATAAGCTTTTC------ACCGAGCATTGTG
   SEQID448   (976) GGCGAGAGCTGCTGCTGATGGGCTGCTGCCTTTGTCAGGTCAGCAGCAGC
   SEQID450   (876) AACAAAAACAGCATCCCCTGATGAACTGCATTTCTCAGCCCTCCAG--AT
   SEQID452   (960) AACAACTGCTGCAGGTGTTCATGGGATACATTTATCTAGTCAGCAG--AT
   SEQID454  (1128) TGGTAACACAGGAGGTATCGACCGGCAGCTGCATT-TGCCTGGCAGTGAG
   SEQID429  (1013) TAGACAAACGGAAAAAAGCTTTACTCTG---------AGCTTCCAGAGAG
   SEQID430  (1100) TAGACAAACGGAAAAAAGCTTTACTCTG---------AGCTTCCAGAGAG
   SEQID431   (885) TAGACAAACGGAAAAAAGCTTTACTCTG---------AGCTTCCAGAGAG
   SEQID432  (1028) TACACAGATGGAAAGCGGCTTTACTCTG---------AGCTTCCAGAGAG
   SEQID434   (870) TGCAAAGACTGAGATTTCCGATA---------------CCGAGCACAATA
  Consensus  (1401) TACAAAAAC G AGG A CGATA GCTG       T  ACCTTGCAG GA

                  1451                                              1500
   SEQID427   (926) GTAATGGCTCTGAGG---ACC---AGCTGG-TTGCTAGTAGCAGCCGTAT
   SEQID436  (1005) ATCGGGC----AATGTACTG---CCACATGGAGCACATCAT-AG--TTTT
   SEQID438   (993) GCCAGGC----AATGTTGTG---TCGCATACGGTGCATCATCATGATTTT
   SEQID440  (1012) CTGACAAATAGCATTGCTATTCAGCACCATCATAAGTCTAATGGCTTCTT
   SEQID442  (1048) CTGACAAATGGCATTGATATTCAGCACCATCATAAGTCTAATAGCTCCTT
   SEQID444   (984) -TGAAGTGCCAGATGCAGCT-----TCAACAATACCAACAG----ACTCG
   SEQID446  (1138) GATTTGGATTGTTGG---ACCCCAAAACGA-AAACCACCAGAGCTGAGTT
   SEQID448  (1026) AGCAGGTGTGGAATGACTCGATTCCGCATGTTGCCCATCAGGAGGATTTC
   SEQID450   (924) ---AACT----AGTGCTGCG---GCTCACACCGGACATCATCATGATCTC
   SEQID452  (1008) GTCGGAC----AATATTATG---CCACATGGTGCACATCAT--GG-TTTC
   SEQID454  (1177) CTGCAGAATGGCATT---AGTACGCTTTGTCATGATTCCAGTAGGCTTTT
   SEQID429  (1054) GTAATGGCTCTGAGG---ACC---AGCTGG-TTGCTAGTAGCAGCCGTAT
   SEQID430  (1141) GTAATGGCTCTGAGG---ACC---AGCTGG-TTGCTAGTAGCAGCCGTAT
   SEQID431   (926) GTAATGGCTCTGAGG---ACC---AGCTGG-TTGCTAGTAGCAGCCGTAT
   SEQID432  (1069) GTAATGGCTCTGAGG---ACC---AACTGT-TTACTGGTAGCACCCTCTC
   SEQID434   (905) GATTTGGATTGTTGG---ACCCCAAAACCA-AAACCGCAAGAGCCGAGTT
  Consensus  (1451) GT A G  T   ATG   A     C C     T CCA CAG AGC TTTT
```

## FIGURE 4B (continued)

```
                   1501                                              1550
 SEQID427   (969) GTTCTCTACAT---------------------CTCAAACCTCA------G
 SEQID436  (1045) GATGG--TC----TC------------ATGGCGTTCAAAGAAACCAACGC
 SEQID438  (1036) GATGGGCTC------------------ATACCATTCAAGGGAAACACCCC
 SEQID440  (1062) ACCATCCAAGGC---------------------CAAGGGCACTGCAGGTG
 SEQID442  (1098) ACCATCTAAAGG---------------------TAAGGGCACTGCAGCTG
 SEQID444  (1024) GATAAAATT------------------ATCTCTTTGAGAGAAGTACACC
 SEQID446  (1184) ATTCAGTAAAGGTTTGTGTTTTTCTTTGCTGTTTAATATATTTTCTCTTG
 SEQID448  (1076) GATGGGTTCACGGTC------------ACGGCGTTCAAAGGAATCAGTCC
 SEQID450   (964) GATGGGTTC------------------ATGGCGTTCAAGGGAACCAGCAC
 SEQID452  (1048) GATGGGTTC------------------ATGGCATTCAAGGGAACTTGTAC
 SEQID454  (1224) GCACTCTAAGGGCGC-------------------AATAGCT--------G
 SEQID429  (1097) GTTCTCTACAT---------------------CTCAAACCTCA------G
 SEQID430  (1184) GTTCTCTACAT---------------------CTCAAACCTCA------G
 SEQID431   (969) GTTCTCTACAT---------------------CTCAAACCTCA------G
 SEQID432  (1112) TTTCTCTGCGT---------------------TTCAAACATCT------G
 SEQID434   (951) ATTCAGTAAAG---------------------------------------
Consensus  (1501) G TCTCTACA                        T AAA    A      G

                   1551                                              1600
 SEQID427   (992) GTGGGTTC-CCAGCAGGAA-AGTCCAAGTTTCA---ACTTCATGGCGAAG
 SEQID436  (1077) GAAGGTCCTTAACCA------------AGGCAT-----------G--GAA-
 SEQID438  (1068) GAAGGTCCTCAACCA------------AGGTGT-----------G--GATC
 SEQID440  (1091) A--GGTTCT----CAA-------CCAAGGTTT-----------G----TT
 SEQID442  (1127) A--GATTCT----CAA-------CCAAGGTTT-----------G----GA
 SEQID444  (1056) CCAGGTCTTTAGTCA------------AGGTTT-----------C--GAG-
 SEQID446  (1234) TTGTATTCATGATCTTGATGATTGCTTGTTTTATGCAGATTTGTTAGAAA
 SEQID448  (1114) AAAGACCCTTGATCATCAA--GGCGCAGGCGT-----------T--GAA-
 SEQID450   (996) AAAGGTCCTTAACCA------------AGGCGT-----------G--GAG-
 SEQID452  (1080) AAAGTTCCCTAATCA------------AGGTGT-----------C--CAA-
 SEQID454  (1247) A--GGTTCTCAATCAAGTG--TCCCAAGGTATGTTACATTCAACCTCAGA
 SEQID429  (1120) GTGGGTTC-CCAGCAGGAA-AGTCCAAGTTTCA---ACTTCATGGCGAAG
 SEQID430  (1207) GTGGGTTC-CCAGCAGGAA-AGTCCAAGTTTCA---ACTTCATGGCGAAG
 SEQID431   (992) GTGGGTTC-CCAGCAGGAA-AGTCCAAGTTTCA---ACTTCATGGCGAAG
 SEQID432  (1135) GTGGGTTC-TCAGCAGGGA-AATCCAACATTCA---ACTTCCAGACAAAG
 SEQID434   (962) -----------------------------------------------AAA
Consensus  (1551)    GGTTCT  A CA         CCAAGGTTT            G  GAA

                   1601                                              1650
 SEQID427  (1037) ATGTGGGAGAATACTCAGGAGTCCTC---CATGAATCTCAAGATATCCAC
 SEQID436  (1102) --GCTTCTGCTGTCGCTTCCGGCTCC---GCTAGAGGCCCAGACTTTGAG
 SEQID438  (1094) CAGCCTGTGCCGTCGTGTCCTCCAACTCGAATGGAGCCCCGGATCTTCGG
 SEQID440  (1113) TTGT--CTTCATT---TTGAGAAAC-----AAGCCTCACAATTATTGCAT
 SEQID442  (1149) AGAATACATCATTC-CTTCCAAAGCG---GAAGCAGCACCAGAATCTCAT
 SEQID444  (1081) --GGGTCTGCATTAACTTCTAACATT---GAGGTAGCACCAGATCTTCGG
 SEQID446  (1284) AGGTCACAATCTCTTCTTCACACATG---GGTGCTTCTCAAGATCTTGAT
 SEQID448  (1148) --GCCTGTGCGGCCGTCTCCAGCCCG---GACGGCGCCCCGGACCTTCAG
 SEQID450  (1021) --GCTTGGGCGGCCGCTTCCAGCTCGAACAACGGAGGCCCAGAAGGTGGG
 SEQID452  (1105) --GCTTCTGCTGTTGCTTCCGACTCC---AGTGGAGCCCCGGATCTTCAG
 SEQID454  (1293) ACACTGGAGGACCG--AGCTTCCCTC---AACTGATTCCCGGGTGCATAT
 SEQID429  (1165) ATGTGGGAGAATACTCAGGAGTCCTC---CATGAATCTCAAGATATCCAC
 SEQID430  (1252) ATGTGGGAGAATACTCAGGAGTCCTC---CATGAATCTCAAGATATCCAC
 SEQID431  (1037) ATGTGGGAGAATACTCAGGAGTCCTC---CATGAATCTCAAGATATCCAC
 SEQID432  (1180) GTGTGGGAGAATGCTCAGGAGGCCTC---CATGAATCTCATGATTTCTAC
 SEQID434   (965) AGGTCACAATCTCT---TCACACATG---GGTGCTTCTCAAGATCTTGAT
Consensus  (1601) A GT   GAGCAT C C TCAG C TC    ATG ATC CCAGAT TTCA
```

# FIGURE 4B (continued)

```
                  1651                                              1700
  SEQID427 (1084) CGTGCTCTCTCTCTTCTGTCAA------CCTCTTCGGAT-----------
  SEQID436 (1147) CATGCTCTCTCTCTTCTGTCAATC---GATTCAGTGGGTG----------
  SEQID438 (1144) CGTGCTCTCTCTCTTCTGTCAAGC---GATTCCTGGGGCCCG--------
  SEQID440 (1153) TGA-----------------------------------------------
  SEQID442 (1195) CGTGCTCTCTCTCTTCTGTCAAAC---AATTCATGGGGTTCACGTGAGCC
  SEQID444 (1126) CGTGCTCTCTCTCTTCTGTCAACC---ACTTCTTGGGGCTCGAAT-----
  SEQID446 (1331) GGTGCTCTCTCTCTTCTGTCAAATTCAACACCATGGGTTTC---CTCGAA
  SEQID448 (1193) CGTGCTCTCTCTCTTCTGTCAAAC---AGTCCGGCCGGTGGCGG------
  SEQID450 (1069) CGTGCTCTCTCTCTTCTGTCAGAC---GGCTCGTGGGGCTCGAGTT----
  SEQID452 (1150) CATGCTCTCTCTCTTCTGTCAAGC---AACCCAGTGGGTG----------
  SEQID454 (1338) CTTGCCTTCACAAGGCGATGGTAA-CACCCACTTTCAAGAGTT-C-----
  SEQID429 (1212) CGTGCTCTCTCTCTTCTGTCAA------CCTCTTCGGAT-----------
  SEQID430 (1299) CGTGCTCTCTCTCTTCTGTCAA------CCTCTTCGGAT-----------
  SEQID431 (1084) CGCGCCCTATCGCTGCTATCGA------CCTCTTCGGAT-----------
  SEQID432 (1227) AGTGCTCTCTCTCTTCTGTCAA------CTACTTCGGATTCACAAGGGAT
  SEQID434 (1009) GGTGCTCTCTCTCTTCTGTCAAATTCAACAACATGGGTTTCTTCCTCTGA
Consensus  (1651) CGTGCTCTCTCTCTTCTGTCAA      C TC T GG T

                  1701                                              1750
  SEQID427 (1117) ----------CCCCTGGCCC--AACCAC----ATGTGCAGCCATTTTCTC
  SEQID436 (1184) -CTGCA--AAC----------------CTTC-AGCCAGGTTCTCAGATTC
  SEQID438 (1183) ---GCC--GACGTT-------------CAG---GCCGGC-TCCCAGGTGC
  SEQID440 (1156) --------------------------------------------------
  SEQID442 (1242) GCAATCTATTTCATTTGAACA-ACCCGTGCATACAAATCACACCACTCAG
  SEQID444 (1168) -GAGCATGGATCCA--------CCTCTCTGG-ATCAACTGATGCTTGCAA
  SEQID446 (1378) CCAGCCAACACGGTTTTCCCTTAACCACCAT-TCCACAAGCAACCTCCAA
  SEQID448 (1234) -CGGCACCAACACCAACCACC-CGCCGCCGC-CGCCGGCGGCTGAGCTGC
  SEQID450 (1112) -CAGCC--GTCATC-------------CAGC-AGCCCACATCTCACGCGG
  SEQID452 (1187) -CTGCC--AAC----------------CTCC-AGCCAAGTCCCCAGATGC
  SEQID454 (1381) ----------AAGCTGTTCA--AAGCGCCAT-ACGAGTCTGGCTTTTCTT
  SEQID429 (1245) ----------CCCCTGGCCC--AACCAC----ATGTGCAGCCATTTTCTC
  SEQID430 (1332) ----------CCCCTGGCCC--AACCAC----ATGTGCAGCCATTTTCTC
  SEQID431 (1117) ----------CCCCTGGCCC--AACCAC----ATGTGCAGCCATTTTCTC
  SEQID432 (1271) CAAACACACTCCCGTGGCCG--AACCACCGCCAATATTTGGCACTTTCCC
  SEQID434 (1059) CCAACCAAGGCGTTTTACCCTTGATCACCAT-CCCTCAAGCAACCTCCAA
Consensus  (1701)     C     C   T  CCC  AACC C    A C    GCC C T C C

                  1751                                              1800
  SEQID427 (1151) TACTCTGTTCATATGATGTTGT--ACCAAAAT---AG-------------
  SEQID436 (1214) ACC----CTGGTGT---------CACCGCCATTGCCGGCACCTCCAACCC
  SEQID438 (1211) ATC-CCGGTGGAGTGATGCCGCCCCTCGCCGTTGCCGCCGCCACCGTCAC
  SEQID440 (1156) --------------------------------------------------
  SEQID442 (1291) TCTGTGCTGCAAGTTATACCCCAAAACTCACCACTTGCTTCATCAGAGTA
  SEQID444 (1208) ACC-AAACAAGCATGACACAGCCAATGATAAATGCTG--AACTCCAAAAT
  SEQID446 (1427) CCCGTGGTTCACGGGTCTGTG---ACTCAACTCAGTTCAGTGTCCAGCTA
  SEQID448 (1281) ACCACCACCGCGCTGCC-CCGAGCAGCTGCCTCGCCGCCGGCTCCGTCTC
  SEQID450 (1145) ACG-CCGGTGCATTGCTGCCGCCCCTCGCCACCGTTGCCGTCTCCAACGC
  SEQID452 (1217) ACT----CTGGGGTG-----G--CAGC--CATTGCCGGCGCCCCCAACCC
  SEQID454 (1418) CTGGATATTCAGAGTATAGGGA--ATTGAGAT---ATCCGATTCCTAGGA
  SEQID429 (1279) TACTCTGTTCATATGATGTTGT--ACCAAAAT---AGATGAGTAAGTAAT
  SEQID430 (1366) TACTCTGTTCATATGATGTTGT--ACCAAAAT---AGATGAGTAAGTAAT
  SEQID431 (1151) TACTCTGTTCATATGATGTTGT--ACCAAAAT---AG-------------
  SEQID432 (1319) TAGTCATTTCATCTGAAAGAGT--TAAAAAATGCGAAGTCACTCCATAAA
  SEQID434 (1108) CCCGTAGCTCACCGGTCTGCG---GCTCAACTCAATTCAGTTTCCGGCTA
Consensus  (1751)  CC C GTTCA  TGAT   G   A C AAAT    G  G  TCC
```

# FIGURE 4B (continued)

```
                    1801                                              1850
   SEQID427 (1183) --------------------------------------------------
   SEQID436 (1251) TGT----CATGAT---GCCA--TCTCCAGCAA--TCTGGCAAGGAGGCTT
   SEQID438 (1260) CGC----CCCGAC---G-AACCCTGTCAGTGT--GATGCATGCTCTGCAC
   SEQID440 (1156) --------------------------------------------------
   SEQID442 (1341) TTGGAGGACTGAGCAACCGTCAACTGACTCTCAAGTGCATACCTTGACGT
   SEQID444 (1255) TGT---CCAGTTG-------CTTCATCAGAA---AATGCACGGATGGAAC
   SEQID446 (1474) CTGGCAGCCGGACCCACCAGCAGCTGAGGGCTCAACCGCTTTGACTAGGA
   SEQID448 (1330) CGTGCTGCAGGAG---GCCTCGTCGCCGGGGC--TGTGGCAGGACGGCGG
   SEQID450 (1194) CGC----CGC----------CGCCGCC-GG----G---CATCCTCTGGAC
   SEQID452 (1254) CGCGATGCACGCGCTGGGATCATCGACGGGGC--TCTGGCTAGACGGCAG
   SEQID454 (1463) GTAACGCCCGTGTCATGAAGCAAACATAG---------------------
   SEQID429 (1324) GTGTAATTTGTAAACCTGTTACTCAGTTGGTG-GATACTTTTCCAAACCT
   SEQID430 (1411) GTGTAATTTGTAAACCTGTTACTCAGTTGGTG-GATACTTTTCCAAACCT
   SEQID431 (1183) --------------------------------------------------
   SEQID432 (1367) AAGTCGGGTTGCATCATCCGATGGAGTTTGACAGTTTGTTTTCAAATAAA
   SEQID434 (1155) TTGGCAGCCGGACCCACCCGCAGTTGAAGGCCCGACCGCTCTGCATAGAA
Consensus   (1801)        C  GA        C  C    GG       G

                    1851                                              1900
   SEQID427 (1183) --------------------------------------------------
   SEQID436 (1290) ---------GTCC-CTTGATCAGCAGGCGCA------GTTTCAGGCTTTC
   SEQID438 (1300) CC-------GTCCACCGGAGGAGGAGGATTCT----GGCAAGACGGCGAC
   SEQID440 (1156) --------------------------------------------------
   SEQID442 (1391) CTCACTGA------------------------------------------
   SEQID444 (1292) AAGC-----ATCC-CTTGAGTCTCGGGTGCAT-----TCTTTGGATTTAC
   SEQID446 (1524) ATGGGGTAGGCCAGTTTAATGAGAACTAC---AACTTGAATCAGTTTTAT
   SEQID448 (1375) CGGCAGCGCGGCC-CTGGGCCACCACGCGCACGCGCGGCTCCAGGCTCTC
   SEQID450 (1222) CC-------GTCC-CCGG-----GAAGGTTCT----GGCCGCAA---GAC
   SEQID452 (1302) CCA------GCCC-CTCGATGATCACCCGCG------GTTCCAGGTCTTC
   SEQID454 (1492) --------------------------------------------------
   SEQID429 (1373) ATGATAAAAACCTCGTCCTAGATCCCGTT--AAATGCCAAACTTTCGGCT
   SEQID430 (1460) ATGATAAAAACCTCGTCCTAGATCCCGTT--AAATGCCAAACTTTCGGCT
   SEQID431 (1183) --------------------------------------------------
   SEQID432 (1417) ATAAAAATGTGCAACATCCATTGCTCAAG--CTAAACCAGTCACTCGGTT
   SEQID434 (1205) ATGGGGTAGGCCAGTTTAATGAAAACTACTTCAGCTTGAACCAGTTTTAT
Consensus   (1851)            C   T     A                       CA

                    1901                                              1950
   SEQID427 (1183) --------------------------------------------------
   SEQID436 (1324) GAC--CGCCTTGGCAACGACGACGACGAAGATCATCTCCAGCTCCCAAAG
   SEQID438 (1339) GAC-CCGCCTCCG----CTCGATCATGCCTCGCAGGCTCAGGCGTTCATG
   SEQID440 (1156) --------------------------------------------------
   SEQID442 (1399) --------------------------------------------------
   SEQID444 (1331) ATG--A-CAATGGAAACGTCCAGTTGCAAGAGT---TTCAGCTGCTCAAA
   SEQID446 (1571) AACTGAAAGTGTGTTGCTTTTAAAATCATAATAAGATATTTTGTGTAAGG
   SEQID448 (1424) GACGCCGCCATGG---CGACGGCGCAGGAGCTC--CTCCAGCTCCCCAGG
   SEQID450 (1252) GAT-CATCCCCCG----CTCG--------TCGACGGACCCGCCACGCA--
   SEQID452 (1339) GAG--CGCTTGGG---GGACCATGACAGCGAGC---TCCAGCTCCCAAAG
   SEQID454 (1492) --------------------------------------------------
   SEQID429 (1421) ACTATAACTATG-TTATCGTTATCATTATCATTGTTTAACACCCT-----
   SEQID430 (1508) ACTATAACTATG-TTATCGTTATCATTATCATTGTTTAACACCCT-----
   SEQID431 (1183) --------------------------------------------------
   SEQID432 (1465) AGCTCAGCTTTTCTTATACATTTCCAAACATATTGTCAATTTCCTTCTGG
   SEQID434 (1255) AACTGA--------------------------------------------
Consensus   (1901)        C
```

# FIGURE 4B (continued)

```
                     1951                                              2000
  SEQID427 (1183) --------------------------------------------------
  SEQID436 (1372) CCT------TCCTATGACAACTCC------CACTATGATCAGATGAACTG
  SEQID438 (1384) CATCCTGGCAATGGCAGCAGCTCC--G----GCTATGGCCATCTGCACTG
  SEQID440 (1156) --------------------------------------------------
  SEQID442 (1399) --------------------------------------------------
  SEQID444 (1375) GCACCCTATGCTACCGGCTGCTTTT------ATTCCAATCAATTCAGCTA
  SEQID446 (1621) ATCAGGTGAGCCTAGTGGTAACGTTTAAATAGGAGCTGTGAAACTTGCTA
  SEQID448 (1469) CCGCCGCCATCGTACGGCGGCTCCTCGTCCCACTACGACCTGATGCGCTG
  SEQID450 (1287) GATTCCGGAGCTGGC-GCACCTCC--G----GATATGGTGA---------
  SEQID452 (1381) CCT------TCCTACGACCATGCCTCG---CACTTCGACCGGATGCACTG
  SEQID454 (1492) --------------------------------------------------
  SEQID429 (1465) --------------------------------------------------
  SEQID430 (1552) --------------------------------------------------
  SEQID431 (1183) --------------------------------------------------
  SEQID432 (1515) ATTCTGTTGGTATGACAACTTTGTTACTCTGTCAAACATGTCTACGACTA
  SEQID434 (1261) --------------------------------------------------
Consensus  (1951)

                     2001                                              2050
  SEQID427 (1183) --------------------------------------------------
  SEQID436 (1410) A-------------------------------------------------
  SEQID438 (1428) A-------------------------------------------------
  SEQID440 (1156) --------------------------------------------------
  SEQID442 (1399) --------------------------------------------------
  SEQID444 (1419) A-------------------------------------------------
  SEQID446 (1671) AAGACATCAACTCCTCTCGTCTTTCTTTTGTCCATTGATTTTCTTGGGTT
  SEQID448 (1519) ATGCTTCCTTCCTTGTGGATTGTGGTGGAGAACTGGAGATGGAGCGGGAG
  SEQID450 (1321) --------------------------------------------------
  SEQID452 (1422) A-------------------------------------------------
  SEQID454 (1492) --------------------------------------------------
  SEQID429 (1465) --------------------------------------------------
  SEQID430 (1552) --------------------------------------------------
  SEQID431 (1183) --------------------------------------------------
  SEQID432 (1565) TTTTAAAACCATTTCAGAGATT----------------------------
  SEQID434 (1261) --------------------------------------------------
Consensus  (2001)

                     2051                                              2100
  SEQID427 (1183) --------------------------------------------------
  SEQID436 (1411) --------------------------------------------------
  SEQID438 (1429) --------------------------------------------------
  SEQID440 (1156) --------------------------------------------------
  SEQID442 (1399) --------------------------------------------------
  SEQID444 (1420) --------------------------------------------------
  SEQID446 (1721) AGGGAGAGTTGTGACAGTTAAGTATTATAAATCATCTTGTCAAATTATTT
  SEQID448 (1569) CGCCGCCGTGCCCCCGGTTGGCGCGTGGAAATCAGTCAGACGTGGCTTCT
  SEQID450 (1321) --------------------------------------------------
  SEQID452 (1423) --------------------------------------------------
  SEQID454 (1492) --------------------------------------------------
  SEQID429 (1465) --------------------------------------------------
  SEQID430 (1552) --------------------------------------------------
  SEQID431 (1183) --------------------------------------------------
  SEQID432 (1587) --------------------------------------------------
  SEQID434 (1261) --------------------------------------------------
Consensus  (2051)
```

# FIGURE 4B (continued)

```
                    2101                                              2150
   SEQID427 (1183) --------------------------------------------------
   SEQID436 (1411) --------------------------------------------------
   SEQID438 (1429) --------------------------------------------------
   SEQID440 (1156) --------------------------------------------------
   SEQID442 (1399) --------------------------------------------------
   SEQID444 (1420) --------------------------------------------------
   SEQID446 (1771) ATACATTAGAATTTTCAGATATTGATGGTGTAGCAACTGAAAAATTGATC
   SEQID448 (1619) GTGGCGTCGTCTCTGCAACGCGGGAGCGGGATTTTGCTTGTGTTCTGAAC
   SEQID450 (1321) --------------------------------------------------
   SEQID452 (1423) --------------------------------------------------
   SEQID454 (1492) --------------------------------------------------
   SEQID429 (1465) --------------------------------------------------
   SEQID430 (1552) --------------------------------------------------
   SEQID431 (1183) --------------------------------------------------
   SEQID432 (1587) --------------------------------------------------
   SEQID434 (1261) --------------------------------------------------
Consensus  (2101)

                    2151                                              2200
   SEQID427 (1183) --------------------------------------------------
   SEQID436 (1411) --------------------------------------------------
   SEQID438 (1429) --------------------------------------------------
   SEQID440 (1156) --------------------------------------------------
   SEQID442 (1399) --------------------------------------------------
   SEQID444 (1420) --------------------------------------------------
   SEQID446 (1821) AAACGCATCATAATATACGTATGATAAGGTTATTTGCATCTGAACTTTTT
   SEQID448 (1669) GAACAGAAAAAAAAAA----------------------------------
   SEQID450 (1321) --------------------------------------------------
   SEQID452 (1423) --------------------------------------------------
   SEQID454 (1492) --------------------------------------------------
   SEQID429 (1465) --------------------------------------------------
   SEQID430 (1552) --------------------------------------------------
   SEQID431 (1183) --------------------------------------------------
   SEQID432 (1587) --------------------------------------------------
   SEQID434 (1261) --------------------------------------------------
Consensus  (2151)

                    2201                                              2250
   SEQID427 (1183) --------------------------------------------------
   SEQID436 (1411) --------------------------------------------------
   SEQID438 (1429) --------------------------------------------------
   SEQID440 (1156) --------------------------------------------------
   SEQID442 (1399) --------------------------------------------------
   SEQID444 (1420) --------------------------------------------------
   SEQID446 (1871) TTATATTCGCATTAGTCAGCATTTTGTGTTATACTAGATTTGGATCCGTG
   SEQID448 (1685) --------------------------------------------------
   SEQID450 (1321) --------------------------------------------------
   SEQID452 (1423) --------------------------------------------------
   SEQID454 (1492) --------------------------------------------------
   SEQID429 (1465) --------------------------------------------------
   SEQID430 (1552) --------------------------------------------------
   SEQID431 (1183) --------------------------------------------------
   SEQID432 (1587) --------------------------------------------------
   SEQID434 (1261) --------------------------------------------------
Consensus  (2201)
```

## FIGURE 4B (continued)

```
                    2251                                              2300
  SEQID427 (1183) --------------------------------------------------
  SEQID436 (1411) --------------------------------------------------
  SEQID438 (1429) --------------------------------------------------
  SEQID440 (1156) --------------------------------------------------
  SEQID442 (1399) --------------------------------------------------
  SEQID444 (1420) --------------------------------------------------
  SEQID446 (1921) CGTTGCAACAGGTTTTATTTGATATTACCATCCATTGATTTTTTTTTTTT
  SEQID448 (1685) --------------------------------------------------
  SEQID450 (1321) --------------------------------------------------
  SEQID452 (1423) --------------------------------------------------
  SEQID454 (1492) --------------------------------------------------
  SEQID429 (1465) --------------------------------------------------
  SEQID430 (1552) --------------------------------------------------
  SEQID431 (1183) --------------------------------------------------
  SEQID432 (1587) --------------------------------------------------
  SEQID434 (1261) --------------------------------------------------
Consensus  (2251)

                    2301                                              2350
  SEQID427 (1183) --------------------------------------------------
  SEQID436 (1411) --------------------------------------------------
  SEQID438 (1429) --------------------------------------------------
  SEQID440 (1156) --------------------------------------------------
  SEQID442 (1399) --------------------------------------------------
  SEQID444 (1420) --------------------------------------------------
  SEQID446 (1971) TTTGCAAACAAATAATTTGGTAATTCCATTAACCGCCACAGTCGTTTTCT
  SEQID448 (1685) --------------------------------------------------
  SEQID450 (1321) --------------------------------------------------
  SEQID452 (1423) --------------------------------------------------
  SEQID454 (1492) --------------------------------------------------
  SEQID429 (1465) --------------------------------------------------
  SEQID430 (1552) --------------------------------------------------
  SEQID431 (1183) --------------------------------------------------
  SEQID432 (1587) --------------------------------------------------
  SEQID434 (1261) --------------------------------------------------
Consensus  (2301)

                    2351              2372
  SEQID427 (1183) ----------------------
  SEQID436 (1411) ----------------------
  SEQID438 (1429) ----------------------
  SEQID440 (1156) ----------------------
  SEQID442 (1399) ----------------------
  SEQID444 (1420) ----------------------
  SEQID446 (2021) GAGCAAAACCATGTTAAAATCT
  SEQID448 (1685) ----------------------
  SEQID450 (1321) ----------------------
  SEQID452 (1423) ----------------------
  SEQID454 (1492) ----------------------
  SEQID429 (1465) ----------------------
  SEQID430 (1552) ----------------------
  SEQID431 (1183) ----------------------
  SEQID432 (1587) ----------------------
  SEQID434 (1261) ----------------------
Consensus  (2351)
```

## FIGURE 4B (continued)

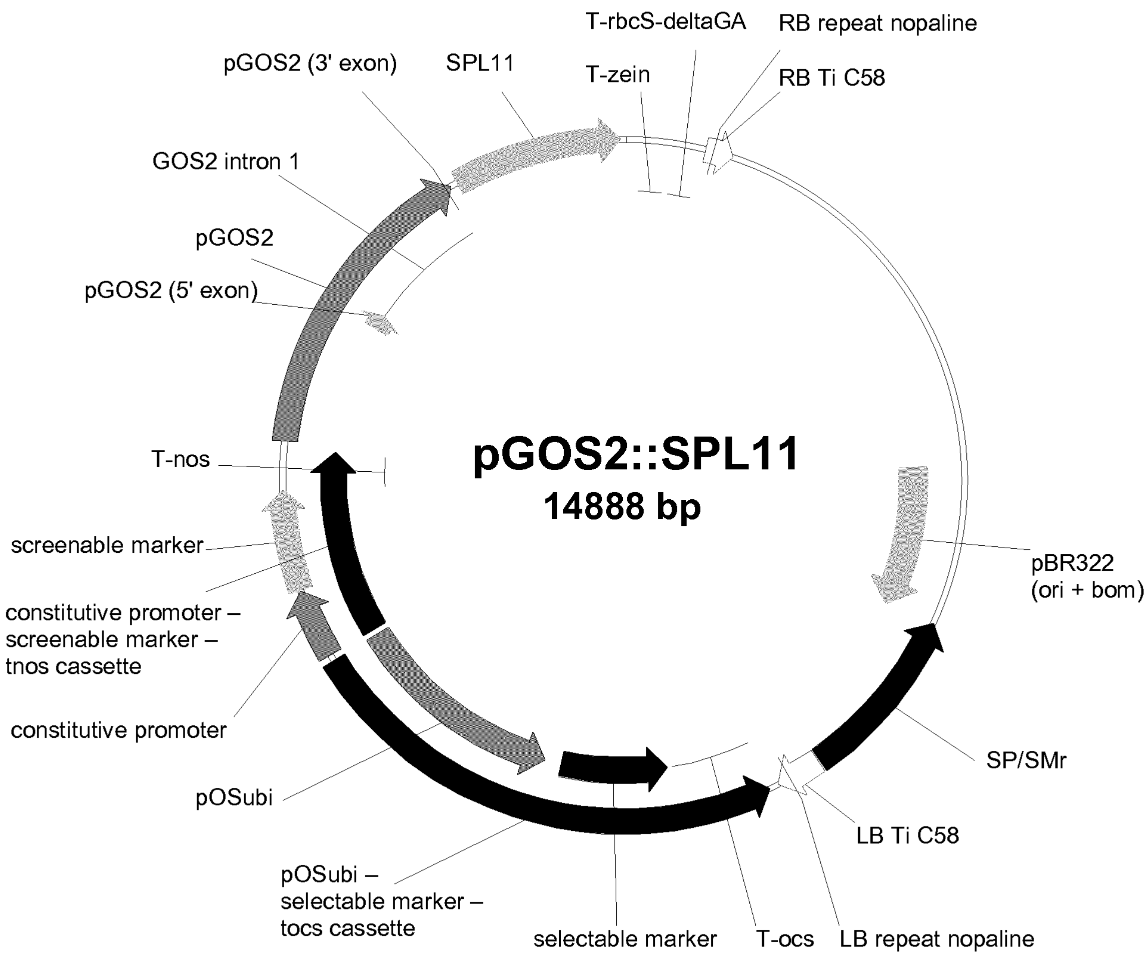
T-rbcS-deltaGA
RB repeat nopaline
pGOS2 (3' exon)
SPL11
T-zein
RB Ti C58
GOS2 intron 1
pGOS2
pGOS2 (5' exon)
pGOS2::SPL11
14888 bp
T-nos
screenable marker
constitutive promoter – screenable marker – tnos cassette
constitutive promoter
pOSubi
pOSubi – selectable marker – tocs cassette
selectable marker
T-ocs
LB repeat nopaline
LB Ti C58
SP/SMr
pBR322 (ori + bom)

FIGURE 5A

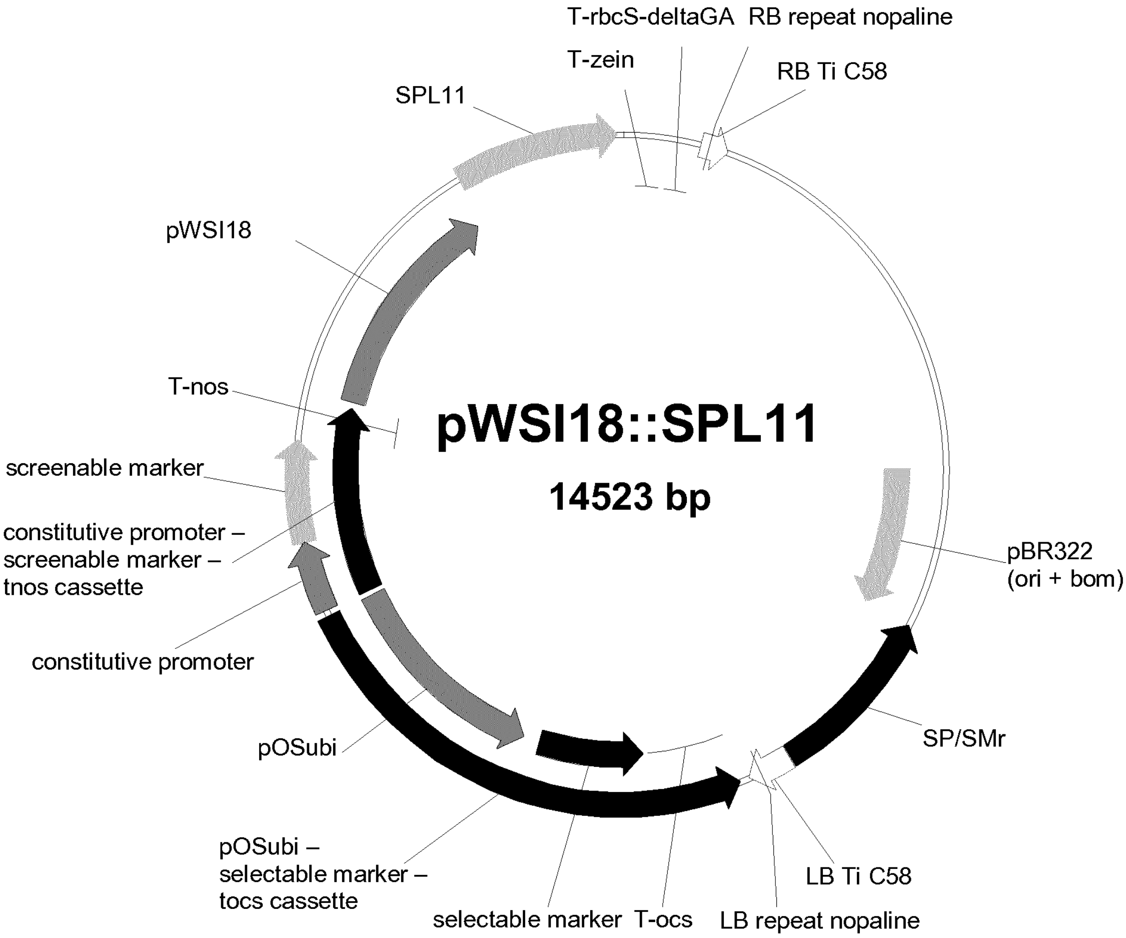
T-rbcS-deltaGA
RB repeat nopaline
T-zein
RB Ti C58
SPL11
pWSI18
T-nos
pWSI18::SPL11
14523 bp
screenable marker
constitutive promoter – screenable marker – tnos cassette
constitutive promoter
pBR322 (ori + bom)
SP/SMr
pOSubi
pOSubi – selectable marker – tocs cassette
selectable marker
T-ocs
LB Ti C58
LB repeat nopaline

FIGURE 5B

**SEQ ID NO: 427, DNA - *Arabidopsis thaliana***

ATGGACTGCAACATGGTATCTTCGTCCCAGTGGGATTGGGAGCATTTGATCATGTCCAATCCGTCA
AGGACTGAAGATGACAGCAAACAGCTACCTACTGAGTGGGAAATTGAAAAAGGTGAAGGAATTGAA
TCTATAGTTCCACATTTCTCAGGCCTTGAGAGAGTCAGTAGTGGCTCTGCCACCAGCTTCTGGCAC
ACTGCTGTATCGAAAAGCTCACAGTCGACCTCTATCAACTCATCATCTCCCGAAGCCAAACGATGC
AAGCTTGCATCAGAAAGTTCCCCTGGAGATTCTTGCAGCAACATAGACTTTGTCCAGGTGAAGGCT
CCCACAGCTCTCGAGGTATCCGTTGCCTCAGCTGAATCAGATCTTTGTTTAAAACTAGGAAAGCGG
ACATACTCTGAAGAATACTGGGGTAGAAACAATAATGAAATTTCAGCGGTTTCTATGAAGTTGTTA
ACTCCATCTGTTGTCGCTGGGAAATCCAAATTGTGTGGTCAGAGCATGCCAGTCCCGCGTTGCCAA
ATTGATGGCTGTGAACTGGATCTCTCATCTGCTAAGGGTTATCATCGTAAGCACAAAGTCTGCGAA
AAGCATTCAAAGTGCCCAAAAGTTAGCGTGAGTGGCCTGGAACGTCGGTTCTGCCAACAGTGTAGC
AGGTTCCATGCTGTCTCTGAATTTGATGAGAAGAAACGAAGCTGCCGAAAACGTCTTTCTCATCAT
AATGCGAGGCGTCGTAAGCCACAAGGAGTATTTTCAATGAATCCCGAGAGGGTGTATGATCGAAGA
CAGCATACAAATATGTTGTGGAATGGGGTGTCCCTTAACGCGAGATCTGAAGAAATGTATGAATGG
GGTAATAACACTTATGATACAAAGCCTAGACAAACGGAAAAAAAGCTTTACTCTGAGCTTCCAGAGA
GGTAATGGCTCTGAGGACCAGCTGGTTGCTAGTAGCAGCCGTATGTTCTCTACATCTCAAACCTCA
GGTGGGTTCCCAGCAGGAAAGTCCAAGTTTCAACTTCATGGCGAAGATGTGGGAGAATACTCAGGA
GTCCTCCATGAATCTCAAGATATCCACCGTGCTCTCTCTCTTCTGTCAACCTCTTCGGATCCCCTG
GCCCAACCACATGTGCAGCCATTTTCTCTACTCTGTTCATATGATGTTGTACCAAAATAG

**SEQ ID NO: 428, protein - *Arabidopsis thaliana***

MDCNMVSSSQWDWEHLIMSNPSRTEDDSKQLPTEWEIEKGEGIESIVPHFSGLERVSSGSATSFWH
TAVSKSSQSTSINSSSPEAKRCKLASESSPGDSCSNIDFVQVKAPTALEVSVASAESDLCLKLGKR
TYSEEYWGRNNNEISAVSMKLLTPSVVAGKSKLCGQSMPVPRCQIDGCELDLSSAKGYHRKHKVCE
KHSKCPKVSVSGLERRFCQQCSRFHAVSEFDEKKRSCRKRLSHHNARRRKPQGVFSMNPERVYDRR
QHTNMLWNGVSLNARSEEMYEWGNNTYDTKPRQTEKSFTLSFQRGNGSEDQLVASSSRMFSTSQTS
GGFPAGKSKFQLHGEDVGEYSGVLHESQDIHRALSLLSTSSDPLAQPHVQPFSLLCSYDVVPK

**SEQ ID NO: 429, DNA - *Arabidopsis thaliana***

CTTAGTCAATAGCATCTTCTCCGATCATCGACGTCCGGAGATTTCTTCGGCCCTCTCATACTTGAG
TTGTTGTAGGATTTGTTGCTCTGGCTCTGGTGGTAGGTCTATGAAATCAACCCATATCGTGAATGG
ACTGCAACATGGTATCTTCGTCCCAGTGGGATTGGGAGCATTTGATCATGTCCAATCCGTCAAGGA
CTGAAGATGACAGCAAACAGCTACCTACTGAGTGGGAAATTGAAAAAGGTGAAGGAATTGAATCTA
TAGTTCCACATTTCTCAGGCCTTGAGAGAGTCAGTAGTGGCTCTGCCACCAGCTTCTGGCACACTG
CTGTATCGAAAAGCTCACAGTCGACCTCTATCAACTCATCATCTCCCGAAGCCAAACGATGCAAGC
TTGCATCAGAAAGTTCCCCTGGAGATTCTTGCAGCAACATAGACTTTGTCCAGGTGAAGGCTCCCA
CAGCTCTCGAGGTATCCGTTGCCTCAGCTGAATCAGATCTTTGTTTAAAACTAGGAAAGCGGACAT
ACTCTGAAGAATACTGGGGTAGAAACAATAATGAAATTTCAGCGGTTTCTATGAAGTTGTTAACTC
CATCTGTTGTCGCTGGGAAATCCAAATTGTGTGGTCAGAGCATGCCAGTCCCGCGTTGCCAAATTG
ATGGCTGTGAACTGGATCTCTCATCTGCTAAGGGTTATCATCGTAAGCACAAAGTCTGCGAAAAGC
ATTCAAAGTGCCCAAAAGTTAGCGTGAGTGGCCTGGAACGTCGGTTCTGCCAACAGTGTAGCAGGT
TCCATGCTGTCTCTGAATTTGATGAGAAGAAACGAAGCTGCCGAAAACGTCTTTCTCATCATAATG
CGAGGCGTCGTAAGCCACAAGGAGTATTTTCAATGAATCCCGAGAGGGTGTATGATCGAAGACAGC
ATACAAATATGTTGTGGAATGGGGTGTCCCTTAACGCGAGATCTGAAGAAATGTATGAATGGGGTA
ATAACACTTATGATACAAAGCCTAGACAAACGGAAAAAAAGCTTTACTCTGAGCTTCCAGAGAGGTA
ATGGCTCTGAGGACCAGCTGGTTGCTAGTAGCAGCCGTATGTTCTCTACATCTCAAACCTCAGGTG
GGTTCCCAGCAGGAAAGTCCAAGTTTCAACTTCATGGCGAAGATGTGGGAGAATACTCAGGAGTCC
TCCATGAATCTCAAGATATCCACCGTGCTCTCTCTCTTCTGTCAACCTCTTCGGATCCCCTGGCCC

## FIGURE 6

AACCACATGTGCAGCCATTTTCTCTACTCTGTTCATATGATGTTGTACCAAAATAGATGAGTAAGT
AATGTGTAATTTGTAAACCTGTTACTCAGTTGGTGGATACTTTTCCAAACCTATGATAAAAACCTC
GTCCTAGATCCCGTTAAATGCCAAACTTTCGGCTACTATAACTATGTTATCGTTATCATTATCATT
GTTTAACACCCT

**SEQ ID NO: 430, DNA - *Arabidopsis thaliana***
CTGGGTGAAACATAGAAAAGTTTCTCTTGCTCAAGTTAATGATAAAAGGGTGAGAGCAATAAACGC
TGATAAGCCTTGTCTGGTCCTTGGAATTTTGAATTTTCTTTTTCTATCTTACTTATAGTATTGGTA
GTTGAGGGTGTCGTCGATAAGTTGTTGTAGGATTTGTTGCTCTGGCTCTGGTGGTAGGTCTATGAA
ATCAACCCATATCGTGAATGGACTGCAACATGGTATCTTCGTCCCAGTGGGATTGGGAGCATTTGA
TCATGTCCAATCCGTCAAGGACTGAAGATGACAGCAAACAGCTACCTACTGAGTGGGAAATTGAAA
AAGGTGAAGGAATTGAATCTATAGTTCCACATTTCTCAGGCCTTGAGAGAGTCAGTAGTGGCTCTG
CCACCAGCTTCTGGCACACTGCTGTATCGAAAAGCTCACAGTCGACCTCTATCAACTCATCATCTC
CCGAAGCCAAACGATGCAAGCTTGCATCAGAAAGTTCCCCTGGAGATTCTTGCAGCAACATAGACT
TTGTCCAGGTGAAGGCTCCCACAGCTCTCGAGGTATCCGTTGCCTCAGCTGAATCAGATCTTTGTT
TAAAACTAGGAAAGCGGACATACTCTGAAGAATACTGGGGTAGAAACAATAATGAAATTTCAGCGG
TTTCTATGAAGTTGTTAACTCCATCTGTTGTCGCTGGGAAATCCAAATTGTGTGGTCAGAGCATGC
CAGTCCCGCGTTGCCAAATTGATGGCTGTGAACTGGATCTCTCATCTGCTAAGGGTTATCATCGTA
AGCACAAAGTCTGCGAAAAGCATTCAAAGTGCCCAAAAGTTAGCGTGAGTGGCCTGGAACGTCGGT
TCTGCCAACAGTGTAGCAGGTTCCATGCTGTCTCTGAATTTGATGAGAAGAAACGAAGCTGCCGAA
AACGTCTTTCTCATCATAATGCGAGGCGTCGTAAGCCACAAGGAGTATTTTCAATGAATCCCGAGA
GGGTGTATGATCGAAGACAGCATACAAATATGTTGTGGAATGGGGTGTCCCTTAACGCGAGATCTG
AAGAAATGTATGAATGGGGTAATAACACTTATGATACAAAGCCTAGACAAACGGAAAAAAAGCTTTA
CTCTGAGCTTCCAGAGAGGTAATGGCTCTGAGGACCAGCTGGTTGCTAGTAGCAGCCGTATGTTCT
CTACATCTCAAACCTCAGGTGGGTTCCCAGCAGGAAAGTCCAAGTTTCAACTTCATGGCGAAGATG
TGGGAGAATACTCAGGAGTCCTCCATGAATCTCAAGATATCCACCGTGCTCTCTCTCTTCTGTCAA
CCTCTTCGGATCCCCTGGCCCAACCACATGTGCAGCCATTTTCTCTACTCTGTTCATATGATGTTG
TACCAAAATAGATGAGTAAGTAATGTGTAATTTGTAAACCTGTTACTCAGTTGGTGGATACTTTTC
CAAACCTATGATAAAAACCTCGTCCTAGATCCCGTTAAATGCCAAACTTTCGGCTACTATAACTAT
GTTATCGTTATCATTATCATTGTTTAACACCCT

**SEQ ID NO: 431, DNA - *Arabidopsis thaliana***
ATGGACTGCAACATGGTATCTTCGTCCCAGTGGGATTGGGAGCATTTGATCATGTCCAATCCGTCA
AGGACTGAAGATGACAGCAAACAGCTACCTACTGAGTGGGAAATTGAAAAAGGTGAAGGAATTGAA
TCTATAGTTCCACATTTCTCAGGCCTTGAGAGAGTCAGTAGTGGCTCTGCCACCAGCTTCTGGCAC
ACTGCTGTATCGAAAAGCTCACAGTCGACCTCTATCAACTCATCATCTCCCGAAGCCAAACGATGC
AAGCTTGCATCAGAAAGTTCCCCTGGAGATTCTTGCAGCAACATAGACTTTGTCCAGGTGAAGGCT
CCCACAGCTCTCGAGGTATCCGTTGCCTCAGCTGAATCAGATCTTTGTTTAAAACTAGGAAAGCGG
ACATACTCTGAAGAATACTGGGGTAGAAACAATAATGAAATTTCAGCGGTTTCTATGAAGTTGTTA
ACTCCATCTGTTGTCGCTGGGAAATCCAAATTGTGTGGTCAGAGCATGCCAGTCCCGCGTTGCCAA
ATTGATGGCTGTGAACTGGATCTCTCATCTGCTAAGGGTTATCATCGTAAGCACAAAGTCTGCGAA
AAGCATTCAAAGTGCCCAAAAGTTAGCGTGAGTGGCCTGGAACGTCGGTTCTGCCAACAGTGTAGC
AGGTTCCATGCTGTCTCTGAATTTGATGAGAAGAAACGAAGCTGCCGAAAACGTCTTTCTCATCAT
AATGCGAGGCGTCGTAAGCCACAAGGAGTATTTTCAATGAATCCCGAGAGGGTGTATGATCGAAGA
CAGCATACAAATATGTTGTGGAATGGGGTGTCCCTTAACGCGAGATCTGAAGAAATGTATGAATGG
GGTAATAACACTTATGATACAAAGCCTAGACAAACGGAAAAAAAGCTTTACTCTGAGCTTCCAGAGA
GGTAATGGCTCTGAGGACCAGCTGGTTGCTAGTAGCAGCCGTATGTTCTCTACATCTCAAACCTCA
GGTGGGTTCCCAGCAGGAAAGTCCAAGTTTCAACTTCATGGCGAAGATGTGGGAGAATACTCAGGA
GTCCTCCATGAATCTCAAGATATCCACCGCGCCCTATCGCTGCTATCGACCTCTTCGGATCCCCTG
GCCCAACCACATGTGCAGCCATTTTCTCTACTCTGTTCATATGATGTTGTACCAAAATAG

**FIGURE 6 (continued)**

**SEQ ID NO: 432, DNA - *Arabidopsis thaliana***
GCCCTTTTTGTTGGTCTGGGTGAAACATAGCAAGGTTTCTCTTGCTGAGGTTATTGATAAACGGGT
GAGTTAAATAAAAACGTTGATCAGCAGTGTGGTGCTTGGAATTCATAAGGTCTATGAAATCAAC
CCACATCTTGAATGGACTGCAACATGGTATCTTCGTTCCCGTGGGACTGGGAGAATTTGATCATGT
CCAATCAGTCGAAGACTGAAAATGAAAAAAAACAGCAATCTACTGAGTGGGAATTTGAAAAAGGTG
AAGGAATTGAATCTATAGTTCCAGATTTCTTAGGCTTTGAGAAAGTCAGTAGTGGCTCTGCTACTA
GTTTCTGGCACACTGCCGTATCAAAAAGCTCGCAGTCGACCTCTATCAACTCATCATCTCCCGAGG
ACAAACGATGCAATCTTGCATCACAAAGTTCCCCTGGAGATTCTTCCAGCAACATAGATTTTCTCC
AGGTGAAACCATCCACAGCTCTCGAGGTACCTATTGCCTCAGCTGAATCAGATCTTTGTTTGAAAC
TAGGAAAGCGGACATACTCTGAAGAATTTTGGGGTAGGAACAATAATGACCTTTCAGCGGTTTCTA
TGAATTTGTTGACTCCATCTGTTGTTGCTCGGAAGAAAACCAAATCGTGTGGTCAGAGCATGCAAG
TTCCGCGTTGCCAAATTGATGGCTGTGAGCTGGATCTCTCATCTTCTAAGGATTATCATCGCAAGC
ATAGAGTCTGCGAAACGCATTCAAAGTGCCCAAAAGTTGTTGTGAGTGGCCTGGAACGTCGTTTCT
GCCAACAGTGTAGCAGGTTCCATGCTGTCTCAGAATTTGATGAAAAGAAACGAAGCTGCCGCAAAC
GTCTTTCTCATCATAATGCAAGGCGTCGCAAGCCACAAGGAGTATTTCCACTGAATTCAGAGAGGG
TGTTCGATCGAAGACAGCATACAAGTATGTTGTGGAATGGGTTGTCCCTTAACACGAGATCTGAAG
AAAAGTATACATGGGGTACCACTTATGAGACAAAGCCTACACAGATGGAAAGCGGCTTTACTCTGA
GCTTCCAGAGAGGTAATGGCTCTGAGGACCAACTGTTTACTGGTAGCACCCTCTCTTTCTCTGCGT
TTCAAACATCTGGTGGGTTCTCAGCAGGGAAATCCAACATTCAACTTCCAGACAAAGGTGTGGGAG
AATGCTCAGGAGGCCTCCATGAATCTCATGATTTCTACAGTGCTCTCTCTCTTCTGTCAACTACTT
CGGATTCACAAGGGATCAAACACACTCCCGTGGCCGAACCACCGCCAATATTTGGCACTTTCCCTA
GTCATTTCATCTGAAAGAGTTAAAAAATGCGAAGTCACTCCATAAAAAGTCGGGTTGCATCATCCG
ATGGAGTTTGACAGTTTGTTTTCAAATAAAATAAAAATGTGCAACATCCATTGCTCAAGCTAAACC
AGTCACTCGGTTAGCTCAGCTTTTCTTATACATTTCCAAACATATTGTCAATTTCCTTCTGGATTC
TGTTGGTATGACAACTTTGTTACTCTGTCAAACATGTCTACGACTATTTTAAAACCATTTCAGAGA
TT

**SEQ ID NO: 433, protein - *Arabidopsis thaliana***
MDCNMVSSFPWDWENLIMSNQSKTENEKKQQSTEWEFEKGEGIESIVPDFLGFEKVSSGSATSFWH
TAVSKSSQSTSINSSSPEDKRCNLASQSSPGDSSSNIDFLQVKPSTALEVPIASAESDLCLKLGKR
TYSEEFWGRNNNDLSAVSMNLLTPSVVARKKTKSCGQSMQVPRCQIDGCELDLSSSKDYHRKHRVC
ETHSKCPKVVVSGLERRFCQQCSRFHAVSEFDEKKRSCRKRLSHHNARRRKPQGVFPLNSERVFDR
RQHTSMLWNGLSLNTRSEEKYTWGTTYETKPTQMESGFTLSFQRGNGSEDQLFTGSTLSFSAFQTS
GGFSAGKSNIQLPDKGVGECSGGLHESHDFYSALSLLSTTSDSQGIKHTPVAEPPPIFGTFPSHFI

**SEQ ID NO: 434, DNA - *Arabidopsis thaliana***
ATGGAGTGTAATGCAAAGCCACCGTTTCAATGGGAATTGGAAAACTTGATATCTTTTGGCACTTCT
ACAGCTGAAGTTCCTAGAAAGCTAAAACCAATGGAGTGGGAAATTGATGGATTTGATTGCACCTCT
CTGTATTCTTCAAGCTTTGCCTATGCTGGTAGTTCAGGTTCTGATATAGCTCATGCTTTCTCTAAA
AGCTCAAAGTCAACTTCCATTAGCTCTTCATCAGCTGAAGTAAGAACACACAATTTTACATCCGAA
ACTGGTGAAAGTCTTCCTGGAGAATTTGCAAAGGGGATTGATACTTCTCCAAGTCTTGAACTTTCT
TTTGGCTCTGGTGATCCGGTTCTCGGTTTAAAGCTTGGTAAACGAACGTACTTTGAAGACTTTTGG
GAAGTGGAGAATGCTAAAGGTTTGGGACTTCCAGTGACTCTGGCTTCATCTTCTGTTTCTCCCGTG
AAGAAATCGAAATCCATTCCTCAGAGGTTACAAACTCCTCACTGTCAAGTTGAAGGCTGTAATCTT
GATCTTTCATCAGCTAAAGACTATCATCGGAAACATAGGATTTGTGAAAATCATTCAAAGTTTCCT
AAAGTCGTTGTGAGTGGCGTAGAGCGTCGGTTCTGCCAACAATGTAGCAGGTTCCACTGTCTCTCT
GAGTTTGATGAGAAGAAACGTAGCTGTCGCCGACGTCTCTCAGATCACAATGCAAGACGTCGCAAG
CCAAATCCTGGAAGGACATATGATGGGAAACCACAGGTGGATTTTGTATGGAACAGATTTGCACTT

## FIGURE 6 (continued)

ATCCATCCAAGAAGTGAGGAAAAGTTTATATGGCCAAGTTCGAAGCACGTACCATCAAGAGTGTTA
ATGCCGCAACCTGCAAAGACTGAGATTTCCGATACCGAGCACAATAGATTTGGATTGTTGGACCCC
AAAACCAAAACCGCAAGAGCCGAGTTATTCAGTAAAGAAAAGGTCACAATCTCTTCACACATGGGT
GCTTCTCAAGATCTTGATGGTGCTCTCTCTCTTCTGTCAAATTCAACAACATGGGTTTCTTCCTCT
GACCAACCAAGGCGTTTTACCCTTGATCACCATCCCTCAAGCAACCTCCAACCCGTAGCTCACCGG
TCTGCGGCTCAACTCAATTCAGTTTCCGGCTATTGGCAGCCGGACCCACCCGCAGTTGAAGGCCCG
ACCGCTCTGCATAGAAATGGGGTAGGCCAGTTTAATGAAAACTACTTCAGCTTGAACCAGTTTTAT
AACTGA

**SEQ ID NO: 435, protein - *Arabidopsis thaliana***
MECNAKPPFQWELENLISFGTSTAEVPRKLKPMEWEIDGFDCTSLYSSSFAYAGSSGSDIAHAFSK
SSKSTSISSSSAEVRTHNFTSETGESLPGEFAKGIDTSPSLELSFGSGDPVLGLKLGKRTYFEDFW
EVENAKGLGLPVTLASSSVSPVKKSKSIPQRLQTPHCQVEGCNLDLSSAKDYHRKHRICENHSKFP
KVVVSGVERRFCQQCSRFHCLSEFDEKKRSCRRRLSDHNARRRKPNPGRTYDGKPQVDFVWNRFAL
IHPRSEEKFIWPSSKHVPSRVLMPQPAKTEISDTEHNRFGLLDPKTKTARAELFSKEKVTISSHMG
ASQDLDGALSLLSNSTTWVSSSDQPRRFTLDHHPSSNLQPVAHRSAAQLNSVSGYWQPDPPAVEGP
TALHRNGVGQFNENYFSLNQFYN

**SEQ ID NO: 436, DNA - *Oryza sativa***
ATGGGTTCTTTTGGGATGGACTGGAATCAGAAGAGCTCGGTGTTGTGGGATTGGGAGAATATGCCG
CCGATAGGAAATAGCGCGAACGAGAATCCCAAGAATGTGATGCTGGCTGAATCAAAACTTGCAGGT
GTTGGGGTTGACATAGGCCATGAATCAGGCCATTCTTCTGGTGGTACTTTCTCTTCTAGCTCAGAG
ATTGGGTATGGTTCATCCAAGAGTTCCATATCCGCGTCGATCGATTCTCCGTCCAAAGTGGGGAAC
ACCATAGAGCTCAATTTTGCATCTGCGGAAGAGCATGATAAGAACATGGACAAGGGTAAGAGTAAA
GTTGATGACACCGGAACTTCTCGATCACCGGTGGTAGCAGCCAATCGTGTAGAGCCCTTGATTGGC
CTGAAGCTTGGCAAGAGAACCTATTTTGAAGATGTCTGTGGAGGGCAGAATGTCAAGAGTTCTCCA
TCTGGTGTGAGTGTGGCTACCCCATCTCCTGGTCTGGCCAAGAAGGTGAAGGTGGCTCAGCAGAAC
ACTCAGAACCCACACTGTCAAGTTGAAGGCTGCAATGTTGATCTCTCTTCTGCTAAACCTTACCAT
CGAAAGCACCGAGTCTGTGAACCTCACAGCAAGACTCTTAAAGTCATCGTTGCGGGTCTTGAGCGA
CGCTTTTGCCAACAGTGTAGTCGGTTTCACGGTTTAGCTGAGTTCGACCAGAAAAAACGAAGCTGT
CGCAGGCGCCTCCATGATCACAATGCCCGCAGACGGAAGCCACAACCAGAAGCAATTTCCTTAAGT
TCGTCGAGGCTCTCTACATTACTCTATGGTGATGCAAGGCAACAGGCAAGTTTTCTATTTGGTCAA
GCTCCTTATGGTCAGATGGGAAGCTGTGCAAGTTCTTGGGATAACCCAGTACCAGGAGGCTTCAAA
TTTACAGCAACAAAAGCTCCTTGGTCAAGGCCAACAATAGCTGCCGGTGTTGATGGGACGCATGTA
TCTAATCAGCAGGCATCGGGCAATGTACTGCCACATGGAGCACATCATAGTTTTGATGGTCTCATG
GCGTTCAAAGAAACCAACGCGAAGGTCCTTAACCAAGGCATGGAAGCTTCTGCTGTCGCTTCCGGC
TCCGCTAGAGGCCCAGACTTTGAGCATGCTCTCTCTCTTCTGTCAATCGATTCAGTGGGTGCTGCA
AACCTTCAGCCAGGTTCTCAGATTCACCCTGGTGTCACCGCCATTGCCGGCACCTCCAACCCTGTC
ATGATGCCATCTCCAGCAATCTGGCAAGGAGGCTTGTCCCTTGATCAGCAGGCGCAGTTTCAGGCT
TTCGACCGCCTTGGCAACGACGACGACGAAGATCATCTCCAGCTCCCAAAGCCTTCCTATGACAAC
TCCCACTATGATCAGATGAACTGA

**SEQ ID NO: 437, protein - *Oryza sativa***
MGSFGMDWNQKSSVLWDWENMPPIGNSANENPKNVMLAESKLAGVGVDIGHESGHSSGGTFSSSSE
IGYGSSKSSISASIDSPSKVGNTIELNFASAEEHDKNMDKGKSKVDDTGTSRSPVVAANRVEPLIG
LKLGKRTYFEDVCGGQNVKSSPSGVSVATPSPGLAKKVKVAQQNTQNPHCQVEGCNVDLSSAKPYH
RKHRVCEPHSKTLKVIVAGLERRFCQQCSRFHGLAEFDQKKRSCRRRLHDHNARRRKPQPEAISLS

## FIGURE 6 (continued)

SSRLSTLLYGDARQQASFLFGQAPYGQMGSCASSWDNPVPGGFKFTATKAPWSRPTIAAGVDGTHV
SNQQASGNVLPHGAHHSFDGLMAFKETNAKVLNQGMEASAVASGSARGPDFEHALSLLSIDSVGAA
NLQPGSQIHPGVTAIAGTSNPVMMPSPAIWQGGLSLDQQAQFQAFDRLGNDDDEDHLQLPKPSYDN
SHYDQMN

**SEQ ID NO: 438, DNA - *Oryza sativa***
ATGGCTTCTTTTGGGATGAACTGGAATCAGAAGAGCCCTGTGTTTTGGGACTGGGAAAATCCAGCG
CCTTTCGGTCCGAATACAATGGAAAATCCCAAGAGCATACCTCACCCTGAACCAAGAGGTGTAGTT
GTCGCGGCCGCAAATCATGGATCCACCAATTCATCCGGTGGCACGTTCACTTCTAGCTCGGAGCTA
GCCAATGGTTCATCGAAGAGCTCCTTGTCAGCGTCGTTCGATTCCTCATCCAAGCTGGGGAACAGC
TTAGAGTTCAGGTTTGCTTCTGTCAAAGGGCATGGCAAGAACATGTGCAAGGATGGCGAGGCCGGT
AGAGTTGAAGACTCGGGCACTTCTCCAGCTGTGGCAGTTAGCCATGGTGAGCCGGTAATAGGACTC
AAGTTAGGGAAGAGAACTTACTTTGAAAATGTTTGTGGAGGGCAGAATGTCAAGAGCTCCTCTGCA
GCTTCAGGTGTGACTTGTCCATCTACTGTGGTCAAGAAGATGAAGGTGTCTCAGCAGAGCACACAA
AGCTCATACTGCCAAGTTGAAGGCTGCAAAGTCGATCTGTCCTCCGCAAGAGAATACCATCGCAAG
CACAAAGTTTGTGAAGCTCATTCTAAGGCACCAAAGGTTATTGTTTCTGGTCTGGAGCGCCGTTTT
TGCCAACAGTGTAGTCGGTTTCATGGTTTAGCTGAATTTGACCAGAAAAAGAAAAGTTGCCGCAGG
CGTCTATCTGATCATAATGCACGAAGAAGGAAACCGCAACAAGAGGCAATTTCATTTGGTTCATCA
AGGCTCGCCACGATGTTTTACGATGCAAGGCAGCAGACAGATATTTACTTTGGCCAATCTCCTTTT
GGCCAAGTGAGAAGCAATGCAATTTCTTCATGTGACAACCTGGGAGGCTTCAAATTTACAGAAGCA
AAACTCCCCTGGATGAAGCCAATGAAAACTATAGGCCTTGAGGATCTGAATTTCTCTACCCTGCAG
ATGCCAGGCAATGTTGTGTCGCATACGGTGCATCATCATGATTTTGATGGGCTCATACCATTCAAG
GGAAACACCCCGAAGGTCCTCAACCAAGGTGTGGATCCAGCCTGTGCCGTCGTGTCCTCCAACTCG
AATGGAGCCCCGGATCTTCGGCGTGCTCTCTCTCTTCTGTCAAGCGATTCCTGGGGCCCGGCCGAC
GTTCAGGCCGGCTCCCAGGTGCATCCCGGTGGAGTGATGCCGCCCCTCGCCGTTGCCGCCGCCACC
GTCACCGCCCCGACGAACCCTGTCAGTGTGATGCATGCTCTGCACCCGTCCACCGGAGGAGGAGGA
TTCTGGCAAGACGGCGACGACCCGCCTCCGCTCGATCATGCCTCGCAGGCTCAGGCGTTCATGCAT
CCTGGCAATGGCAGCAGCTCCGGCTATGGCCATCTGCACTGA

**SEQ ID NO: 439, protein - *Oryza sativa***
MASFGMNWNQKSPVFWDWENPAPFGPNTMENPKSIPHPEPRGVVVAAANHGSTNSSGGTFTSSSEL
ANGSSKSSLSASFDSSSKLGNSLEFRFASVKGHGKNMCKDGEAGRVEDSGTSPAVAVSHGEPVIGL
KLGKRTYFENVCGGQNVKSSSAASGVTCPSTVVKKMKVSQQSTQSSYCQVEGCKVDLSSAREYHRK
HKVCEAHSKAPKVIVSGLERRFCQQCSRFHGLAEFDQKKKSCRRRLSDHNARRRKPQQEAISFGSS
RLATMFYDARQQTDIYFGQSPFGQVRSNAISSCDNLGGFKFTEAKLPWMKPMKTIGLEDLNFSTLQ
MPGNVVSHTVHHHDFDGLIPFKGNTPKVLNQGVDPACAVVSSNSNGAPDLRRALSLLSSDSWGPAD
VQAGSQVHPGGVMPPLAVAAATVTAPTNPVSVMHALHPSTGGGGFWQDGDDPPPLDHASQAQAFMH
PGNGSSSGYGHLH

**SEQ ID NO: 440, DNA - *Populus spp.***
ATGAGTTCTGTCTCACTGATGGAGTGGAATGGCAAACCCCACTTGCAGTGGGACTGGGAAAACCTG
ATAATGTTCAATGCAATAACAAATGAAAATTCAAAGCAGTTAAGCCTAACAGATTTGGAAACTGAT
GGAGAAAAAGGAAATGATTCTGGGTTTTTCTATTCATCTGGGAGTGTAAGCAGAAGCCGTGGATCT
ATCTCTGACTTAGAACTTGCTTCTTTCTCAAAGAGCTCGAAGTCAGCTTCCACCAATTCTTCATCA
GCTGGGGAAGTTAAAACATCCAAATTCACTTTGGGGGCCTCTAAAACAAATCCATCAGATTACAAT
AAGGAAGAACTTGTGAAGGCTAAGGCAACTGATACTTCTCCCACGCTTGAAGCTTCAGTTGGTTCA
GGTGACCAGCTGCTTGGTTTGAAGCTTGGTAAAAGAACTTACTTTGAAGATGCTTGTGCTGGGAGC
AATGCTCAGTCTTCATCAATATCTACGATTCCTGTGCCTTCTTTTACTCCAGCAAAGAAATTGAAG

## FIGURE 6 (continued)

TCCAGTAATCATAGTCAGCGTGCTCCACGCTGTCAAGTAGAAGGCTGTAACCTTGACCTCTCATCA
GCTAAAGATTACCATCGCAAACATAGAGTTTGTGAAAGCCATTCAAAGTGCCCAAAGGTCATTGTA
GCTGGTTTGGAACGCAGGTTTTGCCAGCAGTGTAGCAGGTTCCATGGTCTGTCAGAGTTTGATGAA
AAGAAGAAAAGCTGCCGCAGGCGACTTTCTGATCACAATGCAAGACGCCGCAAACAGCCAGGATCT
GTCCATTTGAATCCTTCAAGACTTTCTTCATCATTATATGATGAGAGGCAACAGATGAGTCATGTT
TGGGACAAGGCGCCACTTGTTCATTCCAGGCCCAATGCAAATTTGACATGGGAAAGCACATCCACC
TCCAAGTTCACAATAACAAAAGAGTATATAGCAAAGCCTGCAGAAATAGGTGGTAGTGATAGGAGG
CTCCACTTGCCTGGCATTGATCTGACAAATAGCATTGCTATTCAGCACCATCATAAGTCTAATGGC
TTCTTACCATCCAAGGCCAAGGGCACTGCAGGTGAGGTTCTCAACCAAGGTTTGTTTTGTCTTCAT
TTTGAGAAACAAGCCTCACAATTATTGCATTGA

**SEQ ID NO: 441, protein - *Populus spp.***
MSSVSLMEWNGKPHLQWDWENLIMFNAITNENSKQLSLTDLETDGEKGNDSGFFYSSGSVSRSRGS
ISDLELASFSKSSKSASTNSSSAGEVKTSKFTLGASKTNPSDYNKEELVKAKATDTSPTLEASVGS
GDQLLGLKLGKRTYFEDACAGSNAQSSSISTIPVPSFTPAKKLKSSNHSQRAPRCQVEGCNLDLSS
AKDYHRKHRVCESHSKCPKVIVAGLERRFCQQCSRFHGLSEFDEKKKSCRRRLSDHNARRRKQPGS
VHLNPSRLSSSLYDERQQMSHVWDKAPLVHSRPNANLTWESTSTSKFTITKEYIAKPAEIGGSDRR
LHLPGIDLTNSIAIQHHHKSNGFLPSKAKGTAGEVLNQGLFCLHFEKQASQLLH

**SEQ ID NO: 442, DNA - *Populus spp.***
ATGGAGTGGAATGGCAAACCCCACTTACAGTGGGACTGGGAGAGCCTGATAATGTTCAATGGAATA
ACAACTGAAAATTCTAAGCAGTTAAGCCCAACAGATTTGGAAACTGATGGAGAAAAAGGAACCGAC
TCTGGGTTTTTCTATTCATCTGGGAGTGCAAGCAGAAGCGGTGGTTCTAGCTCTGATTTGGAACTT
GCTTCTTTCTCAAAGTGCTCGAAGTCAGCTTCCATCAATTCTTCATCAGCTGGGGAAGTTAAAACA
TCCAAATTCACTTTGGAGGCCTCTAAAGCAAATCCATCTGATTACAATAAGAAAGAAATTGGAAAG
GCTAAGACAGCTAGTATGTCTTCCACAATTGAGGCTGCAGGTGGTTCAGGTGACCAGCTGCTTGGT
TTGAAGCTTGGTAAACGAATATACTTTGAAGATGCTTGTGCTGGCAACAATGTTCAGTCGTCATCA
TTTTCTACAGTTCCTGTGCCCTCTTTTACTTCAGCAAAGAAATTGAAGTCCACTATTCAGAGTCAG
CGTGCTCCATGCTGTCAAGTGGAAGGCTGTAACCTTGACCTCTCATCAGCTAAAGATTATCATCGC
AAACATAGAGTTTGTGAAAGCCATTCAAAGTGCCAGAAGGTCATTGTAGCTGGTTTGGAACGCAGG
TTTTGCCAGCAGTGTAGCAGTAAGATTATTCCTAGCTTTTATGATTGCATTGACAATTTGGGTTTT
GGATGTCAAAGGTTCCATGGCCTGTCAGAGTTCGATGAAAAGAAGAAAAGCTGTCGCAGGCGACTT
TCTGATCACAATGCAAGACGCCGCAAACAACCAGGATCGGTCCATTTAAATTCAAGAGTGTCTTCA
TCATTATATGATGAAAGGCAACAGATGAGTCTTGCTTGGGACAGGGCACCACTTGTTCATGCCAGG
CCTAATGCAAATTTGACGTGGGAAGGCACATACATCTCCAAGTTCACAATAACAAAAGATTATATA
GCAAAGCCTGCAGAAATAGGTGGTAATGATGGGCAGTTTCACTTGCCTGGCTTTGATCTGACAAAT
GGCATTGATATTCAGCACCATCATAAGTCTAATAGCTCCTTACCATCTAAAGGTAAGGGCACTGCA
GCTGAGATTCTCAACCAAGGTTTGGAAGAATACATCATTCCTTCCAAAGCGGAAGCAGCACCAGAA
TCTCATCGTGCTCTCTCTCTTCTGTCAAACAATTCATGGGGTTCACGTGAGCCGCAATCTATTTCA
TTTGAACAACCCGTGCATACAAATCACACCACTCAGTCTGTGCTGCAAGTTATACCCCAAAACTCA
CCACTTGCTTCATCAGAGTATTGGAGGACTGAGCAACCGTCAACTGACTCTCAAGTGCATACCTTG
ACGTCTCACTGA

**SEQ ID NO: 443, protein - *Populus spp.***
MEWNGKPHLQWDWESLIMFNGITTENSKQLSPTDLETDGEKGTDSGFFYSSGSASRSGGSSSDLEL
ASFSKCSKSASINSSSAGEVKTSKFTLEASKANPSDYNKKEIGKAKTASMSSTIEAAGGSGDQLLG
LKLGKRIYFEDACAGNNVQSSSFSTVPVPSFTSAKKLKSTIQSQRAPCCQVEGCNLDLSSAKDYHR
KHRVCESHSKCQKVIVAGLERRFCQQCSSKIIPSFYDCIDNLGFGCQRFHGLSEFDEKKKSCRRRL

## FIGURE 6 (continued)

SDHNARRRKQPGSVHLNSRVSSSLYDERQQMSLAWDRAPLVHARPNANLTWEGTYISKFTITKDYI
AKPAEIGGNDGQFHLPGFDLTNGIDIQHHHKSNSSLPSKGKGTAAEILNQGLEEYIIPSKAEAAPE
SHRALSLLSNNSWGSREPQSISFEQPVHTNHTTQSVLQVIPQNSPLASSEYWRTEQPSTDSQVHTL
TSH

**SEQ ID NO: 444, DNA - *Populus spp.***
ATGGACTGGAACTCCACTGCATCTGAGGGGAACTGGGAGAACATAGCAGGGTTAAGTGCCAAGGCT
AGTGACATTCCAAAACAAGTACCATTGGCATACCATGAGACAGAGGGAGATGGAGCAATTGATAAA
GCAATAACTTATTCGTCTGGTGGTGGTTTATCAAGCTCTGATTTGTGGCATTGCTCTTCATCCAAG
AGCTCAGTTTCACCCTCTGTCGACTCTTCATTGAAGGGGGGGATCAAGACTTATAGCACTGCAGAT
GGTTTTCCTGGAGTTATCATTAGAAAGGACTTGACTAGGGTAGAAAGTACTGAGAATATTCCATCT
CTGGGTGCTTCTGCTAGCTCTGGTGAACCAGTAATTGGCTTGAAGCTTGGTAAACGGATGTACTTT
GAAGACATTTGTACTACCAGCACTGCCAAATCATCGTCTTCATCTATTGTTTCCACTTCCTGTACT
GCTACAACAAAGAGATCTAGGGCATCATATCCAAGTACACAGTCTCCACGTTGCCAAGTGGAAGGA
TGCAACCTTGATCTCAAGTCAGCTAAAGATTACCATCGCAGGCATAGAATCTGTGAAAAACATTCC
AAAAGCCCAAAGGTTATTGTTGCTGGCATGGAACGCCGATTTTGCCAACAGTGCAGCAGGTTCCAT
GAATTATCAGAGTTTGATGACAAGAAGCGAAGCTGTCGTAGACGTCTCTCTGATCACAATGCAAGG
CGACGGAGGCCACAACCTGAGGCAATCCGGTTCAATTCAGCAAGGCCATCATCATCACCATCATCA
TTTTATGGTTGTCATTTCAATTTCAAAGATGGTAGACAGCAGATGAATGTTGTATTGAATAGGGTG
CCTTTCCTGGCAGGAAATTCAACTTGGCAAAGCGAGTGCGGCTTTAAAGTCACTCACGCCGGAGAT
TTTTTTATTAGGCCTGCAAAAGCAGGGGGCATCCATAGGCAGCTTAGCTATCCCTGCAATGAAGTG
CCAGATGCAGCTTCAACAATACCAACAGACTCGGATAAAATTATCTCTTTTGAGAGAAGTACACCC
CAGGTCTTTAGTCAAGGTTTCGAGGGGTCTGCATTAACTTCTAACATTGAGGTAGCACCAGATCTT
CGGCGTGCTCTCTCTCTTCTGTCAACCACTTCTTGGGGCTCGAATGAGCATGGATCCACCTCTCTG
GATCAACTGATGCTTGCAAACCAAACAAGCATGACACAGCCAATGATAAATGCTGAACTCCAAAAT
TGTCCAGTTGCTTCATCAGAAAATGCACGGATGGAACAAGCATCCCTTGAGTCTCGGGTGCATTCT
TTGGATTTACATGACAATGGAAACGTCCAGTTGCAAGAGTTTCAGCTGCTCAAAGCACCCTATGCT
ACCGGCTGCTTTTATTCCAATCAATTCAGCTAA

**SEQ ID NO: 445, protein - *Populus spp.***
MDWNSTASEGNWENIAGLSAKASDIPKQVPLAYHETEGDGAIDKAITYSSGGGLSSSDLWHCSSSK
SSVSPSVDSSLKGGIKTYSTADGFPGVIIRKDLTRVESTENIPSLGASASSGEPVIGLKLGKRMYF
EDICTTSTAKSSSSSIVSTSCTATTKRSRASYPSTQSPRCQVEGCNLDLKSAKDYHRRHRICEKHS
KSPKVIVAGMERRFCQQCSRFHELSEFDDKKRSCRRRLSDHNARRRRPQPEAIRFNSARPSSSPSS
FYGCHFNFKDGRQQMNVVLNRVPFLAGNSTWQSECGFKVTHAGDFFIRPAKAGGIHRQLSYPCNEV
PDAASTIPTDSDKIISFERSTPQVFSQGFEGSALTSNIEVAPDLRRALSLLSTTSWGSNEHGSTSL
DQLMLANQTSMTQPMINAELQNCPVASSENARMEQASLESRVHSLDLHDNGNVQLQEFQLLKAPYA
TGCFYSNQFS

**SEQ ID NO: 446, DNA - *Brassica rapa***
ATGGAGTGTAATGCAAAGCCATCATTGCAGTGGGAGTGGGATAATCTAATATCTTTTGGTACTTCA
TCAGCTGAAATTCCTAAAAAGCAACGACCAATGGATTGGGAAAATGATGGGTTTGATTGCACCACT
TTTTACTCGTCCAGCTTTGCTACAGAAGCAGCTTATGGTGGTGGTAGTTCAGGTTCTGATCTAGCT
CATGCTTTCTCTAAAAGCTCAAAGTCAACTTCCATAAGCTCTTCATCAGCTGAAGTGAGAACATAC
AATTTCACATCAGAAGCTGGTGAAAGTGTTCCTCCTGGAGAATTGGGGAGCAGTGAAGAGTTTGCA
ATGGGAATTGATGCTTCTCCAAGTCTTGAACTCTCCTTCGGCTCTGGTGATCCGGTTCTTGGTTTG
AAGCTTGGTAAGAGGACATACTTTGAAGACTTTTGGGAAGTGGAGAATGCTAAAGGTTCAGCACTT
CCGGTGAGCCTGGCGTCATCTTCTGCTTCTCCGGTGAAGAAATCCAAAACACTCTCTCAGAAATTA

## FIGURE 6 (continued)

CAAACTCCTCACTGCCAAGTTGAAGGCTGTAACCTCGATCTCTCCTCAGCTAAGGACTATCATAGG
AAACACAGGATTTGTGAAAACCATTCAAAGTTCCCTAAAGTCGTTGTCAGTGGCGTAGAGCGCCGG
TTCTGCCAACAATGTAGCAGGTAGAAAAGCTAGTCTTAGAGATATTGGTTTATACAAAAGCCTTAA
TGACTTTTTTTAATTGTTTTTGGTCTTCAAAGGTTTCACTGTCTCTCTGAGTTTGATGAGAAGAAA
CGTAGCTGTCGCCGGCGTCTCTCAGATCACAATGCAAGACGTCGCAAGCCAAATCCCGGGAGGACA
TATGGTAATATACTAGATTCACTTCTCTCTAATCCTATATATTAAATCTAAATTTTTAGTTATATA
ATTAGTAGGTTTCCCTTTTCTAAGATAAATATCATTCTGTATGTGCAGATGGGAAGCAACAGATGG
ATTTTGTATGGAACAGATTTGCACTTATCCATCCAAGAAGTGAAGAAAATTTTCTGTGGCCAAATC
CGAAGCCTGTATCATCAAGAGGGTTATTGCAGGAACCTGCAAAGACCGAGATGCCCAATAAGCTTT
TCACCGAGCATTGTGGATTTGGATTGTTGGACCCCAAAACGAAAACCACCAGAGCTGAGTTATTCA
GTAAAGGTTTGTGTTTTTCTTTGCTGTTTAATATATTTTCTCTTGTTGTATTCATGATCTTGATGA
TTGCTTGTTTTATGCAGATTTGTTAGAAAAGGTCACAATCTCTTCTTCACACATGGGTGCTTCTCA
AGATCTTGATGGTGCTCTCTCTCTTCTGTCAAATTCAACACCATGGGTTTCCTCGAACCAGCCAAC
ACGGTTTTCCCTTAACCACCATTCCACAAGCAACCTCCAACCCGTGGTTCACGGGTCTGTGACTCA
ACTCAGTTCAGTGTCCAGCTACTGGCAGCCGGACCCACCAGCAGCTGAGGGCTCAACCGCTTTGAC
TAGGAATGGGGTAGGCCAGTTTAATGAGAACTACAACTTGAATCAGTTTTATAACTGAAAGTGTGT
TGCTTTTAAAATCATAATAAGATATTTTGTGTAAGGATCAGGTGAGCCTAGTGGTAACGTTTAAAT
AGGAGCTGTGAAACTTGCTAAAGACATCAACTCCTCTCGTCTTTCTTTTGTCCATTGATTTTCTTG
GGTTAGGGAGAGTTGTGACAGTTAAGTATTATAAATCATCTTGTCAAATTATTTATACATTAGAAT
TTTCAGATATTGATGGTGTAGCAACTGAAAAATTGATCAAACGCATCATAATATACGTATGATAAG
GTTATTTGCATCTGAACTTTTTTTATATTCGCATTAGTCAGCATTTTGTGTTATACTAGATTTGGA
TCCGTGCGTTGCAACAGGTTTTATTTGATATTACCATCCATTGATTTTTTTTTTTTTTTGCAAACA
AATAATTTGGTAATTCCATTAACCGCCACAGTCGTTTTCTGAGCAAAACCATGTTAAAATCT

**SEQ ID NO: 447, protein - *Brassica rapa***
MECNAKPSLQWEWDNLISFGTSSAEIPKKQRPMDWENDGFDCTTFYSSSFATEAAYGGGSSGSDLA
HAFSKSSKSTSISSSSAEVRTYNFTSEAGESVPPGELGSSEEFAMGIDASPSLELSFGSGDPVLGL
KLGKRTYFEDFWEVENAKGSALPVSLASSSASPVKKSKTLSQKLQTPHCQVEGCNLDLSSAKDYHR
KHRICENHSKFPKVVVSGVERRFCQQCSRKSSRYWFIQKPLFLIVFGLQRFHCLSEFDEKKRSCRR
RLSDHNARRRKPNPGRTY

**SEQ ID NO: 448, DNA - *Zea mays***
AGCCGCTTCGGCAGGCAAGTAGGACGAGGAGCAGCAGCTCAGATTTCGGATCCTGGCACTGGCAAC
TGGCATGGGCTCGTTTGGGATGGACATGGACTGGAACCAGAAGGCCTCCGTGCTGCTGTGGGACTG
GGAGAACCTGCCGCCCGCAGCCGCAAACGGGAGCGAGAGCCACAGGACGACCGCTGCCGCGCCTCA
GTTCGCGGGCCTTGAGGCCACAGGGCATGAACCGGCGCCTTCTTCCGTATCCTCGTCAATCCATTC
TCTGCCCAGTGCCAAGGGGAACAAGAACAAGAACAACATGGAGCTCATCAGTTTCGCACCTGCCAA
AGCGCCCGACAAGGACACTGGTTCGGTGCTCAGCAGCGGAGAGCCGGTGGTGCTAGGCCTGAAGCT
TGGCAAGAGAACGTATTTCGAAGATGGTTGTGGACTAGGGCAGAGCGGCAAGAGTTCGGCGGCGTT
AGGCACTGCCAGTGCAGCGACCCCTCCGGGTCCTGCGAAGAAGGCGAAGGCGGCGGCGGCGGCTCC
AACCGCGCAGCAGCAGAAATCGTACTGCCAGGTTGAAGGCTGCAGGACCGATCTGTCCTCTGCTAA
AGACTATCATCGCAAGCACAGAGTCTGCGAGCCCCATTCCAAGGCGCCCAAGGTGGTCGTCGCTGG
CCTGGAGCGGCGCTTCTGCCAGCAGTGCAGCCGGTTCCATGGACTGGCCGAGTTCGACCAGAAGAA
GAAGAGCTGCCGCAGGCGACTCAACGACCACAACGCGCGCAGGCGGAAGCCCCAGCCCGAAGCGCT
CCCTTTCGGCTCATCGAGGCTGTCGGCGATGTTCTATGACACGAGGCGCCAGGCGAGCCTCCTGTT
TGGTGAAGGTCCCTACGGTCAGATGAGAAGCTGTGCGAGTTCTTGGTGGGATAGCCCAGGAGCAGG
AGGAGGAGGCTTCAAATTCGCGGAGACGAGAGGAGCTCCTTGGTTGAAGCCGGCGAGAGCTGCTGC
TGATGGGCTGCTGCCTTTGTCAGGTCAGCAGCAGCAGCAGGTGTGGAATGACTCGATTCCGCATGT

## FIGURE 6 (continued)

TGCCCATCAGGAGGATTTCGATGGGTTCACGGTCACGGCGTTCAAAGGAATCAGTCCAAAGACCCT
TGATCATCAAGGCGCAGGCGTTGAAGCCTGTGCGGCCGTCTCCAGCCCGGACGGCGCCCCGGACCT
TCAGCGTGCTCTCTCTCTTCTGTCAAACAGTCCGGCCGGTGGCGGCGGCACCAACACCAACCACCC
GCCGCCGCCGCCGGCGGCTGAGCTGCACCACCACCGCGCTGCCCCGAGCAGCTGCCTCGCCGCCGG
CTCCGTCTCCGTGCTGCAGGAGGCCTCGTCGCCGGGGCTGTGGCAGGACGGCGGCGGCAGCGCGGC
CCTGGGCCACCACGCGCACGCGCGGCTCCAGGCTCTCGACGCCGCCATGGCGACGGCGCAGGAGCT
CCTCCAGCTCCCCAGGCCGCCGCCATCGTACGGCGGCTCCTCGTCCCACTACGACCTGATGCGCTG
ATGCTTCCTTCCTTGTGGATTGTGGTGGAGAACTGGAGATGGAGCGGGAGCGCCGCCGTGCCCCCG
GTTGGCGCGTGGAAATCAGTCAGACGTGGCTTCTGTGGCGTCGTCTCTGCAACGCGGGAGCGGGAT
TTTGCTTGTGTTCTGAACGAACAGAAAAAAAAAA

**SEQ ID NO: 449, protein - *Zea mays***
MGSFGMDMDWNQKASVLLWDWENLPPAAANGSESHRTTAAAPQFAGLEATGHEPAPSSVSSSIHSL
PSAKGNKNKNNMELISFAPAKAPDKDTGSVLSSGEPVVLGLKLGKRTYFEDGCGLGQSGKSSAALG
TASAATPPGPAKKAKAAAAAPTAQQQKSYCQVEGCRTDLSSAKDYHRKHRVCEPHSKAPKVVVAGL
ERRFCQQCSRFHGLAEFDQKKKSCRRRLNDHNARRRKPQPEALPFGSSRLSAMFYDTRRQASLLFG
EGPYGQMRSCASSWWDSPGAGGGGFKFAETRGAPWLKPARAAADGLLPLSGQQQQQVWNDSIPHVA
HQEDFDGFTVTAFKGISPKTLDHQGAGVEACAAVSSPDGAPDLQRALSLLSNSPAGGGGTNTNHPP
PPPAAELHHHRAAPSSCLAAGSVSVLQEASSPGLWQDGGGSAALGHHAHARLQALDAAMATAQELL
QLPRPPPSYGGSSSHYDLMR

**SEQ ID NO: 450, DNA - *Zea mays***
ATGGGTTCTTTTGGGATGAACTGGAATCAGAAGGACCCCATGGTGTGGGATTGGGAACATCTAGTA
CCGTCTGTCTCAAATGCAGTTACAAGGCACGGATCTGCTAATTCATCTGGTGGTACTCTTACTTCT
AACTCAGAGCTAGGGCATGGTTCATCCAAGAGCTCTATTTCAGCGTCCATTGATTCACCCTCTGGA
GTAGGGAACAGCTTAGAGTTCAACTTCGCCGCTGTTGAGAGGCATGTTAAGAACACGGGCACGAAC
GGCAGAGTCGATGACTCGGGGAATTCTCCATCGTCAATGATAGCTTTCAACCAAGGAGAGCCATTA
ATCAGCCTGAAACTTGGGAAGAGGGCTTACTTCGAAAACGCCTGCGGAGGACAGGATGCCAAGGTT
TCTGCAGCTTCAGACGTTACTTCTGCAGCCAGCGTGGTCAAGAAGACTAAGGTGTCTCAGCAGAAT
GCAAAGAACTGGTACTGTCAGGTTGAAGGGTGCAAAGTTGACCTGTCTTCTGCTAAAGATTACAAT
CGCAAGCACAAGGTCTGTGTAGTCCATTCTAAAGCTACCAAGGTGGTTGTTGCTGGTCTAGAGCGT
CGGTTTTGTCAACAGTGTAGCCGTTTTCATGGTTTAGCGGAGTTTGATCAGAACAAACGAAGCTGT
CGTAGGCGTCTGATGCATCATAATGCACGGAGGAGGAAACCTCAGGCAGACACAATTTCATTCAAT
TCATCGACAATGTTTTATGATACAAGGCAGCGGACAAATCTTTTCTTTAGTCAACCACTTTATGGC
CAAGTGAGGAGCAATGCAGGGTCTTCATGGGATAACTTGGGAGGCTTAAAATTCATGGAGACGAAA
CATCCGCCAGTGCATCCAACAAAAACAGCATCCCCTGATGAACTGCATTTCTCAGCCCTCCAGATA
ACTAGTGCTGCGGCTCACACCGGACATCATCATGATCTCGATGGGTTCATGGCGTTCAAGGGAACC
AGCACAAAGGTCCTTAACCAAGGCGTGGAGGCTTGGGCGGCCGCTTCCAGCTCGAACAACGGAGGC
CCAGAAGGTGGGCGTGCTCTCTCTCTTCTGTCAGACGGCTCGTGGGGCTCGAGTTCAGCCGTCATC
CAGCAGCCCACATCTCACGCGGACGCCGGTGCATTGCTGCCGCCCCTCGCCACCGTTGCCGTCTCC
AACGCCGCCGCCGCCGCCGGGCATCCTCTGGACCCGTCCCCGGGAAGGTTCTGGCCGCAAGACGAT
CATCCCCCGCTCGTCGACGGACCCGCCACGCAGATTCCGGAGCTGGCGCACCTCCGGATATGGTGA

**SEQ ID NO: 451, protein - *Zea mays***
MGSFGMNWNQKDPMVWDWEHLVPSVSNAVTRHGSANSSGGTLTSNSELGHGSSKSSISASIDSPSG
VGNSLEFNFAAVERHVKNTGTNGRVDDSGNSPSSMIAFNQGEPLISLKLGKRAYFENACGGQDAKV
SAASDVTSAASVVKKTKVSQQNAKNWYCQVEGCKVDLSSAKDYNRKHKVCVVHSKATKVVVAGLER
RFCQQCSRFHGLAEFDQNKRSCRRRLMHHNARRRKPQADTISFNSSTMFYDTRQRTNLFFSQPLYG

## FIGURE 6 (continued)

QVRSNAGSSWDNLGGLKFMETKHPPVHPTKTASPDELHFSALQITSAAAHTGHHHDLDGFMAFKGT
STKVLNQGVEAWAAASSSNNGGPEGGRALSLLSDGSWGSSSAVIQQPTSHADAGALLPPLATVAVS
NAAAAAGHPLDPSPGRFWPQDDHPPLVDGPATQIPELAHLRIW

**SEQ ID NO: 452, DNA - *Triticum aestivum***
ATGGGCTCGTTCGGGATGGAGTGGAACCAGAGGAGCTCGGTGCTGTGGGACTGGGAGAATTTCCCG
CCGATAGGCGAGAACCCCAAGAACGCGATGCAGGCCGATCCAAGATTTGCCGCCGTTGCGGCTACC
ATGGGGAATGAACCGCTCCATTCTTCTGGCGGTAGCGGCACCTTCTCTTCCAGCTCAGAGATGGGG
TATGGCTCTTCCAAGAGCTCCATGTCCGCGTCGATCGATTCTTCGAACAGGGCTGGGAACAACATG
GAGTTCAGATTTGCGCCTGTCAAAAACCCTGATAGGAACACGAGCAAGAACACCGAGCTGGGTAAA
GTTGATAACACGAGAACTGGAACATCTCCGTCGCCTGTGGTGGCAGTGAGCAGTGGAGAGCCGGTG
ATCGGCCTGAAGCTTGGCAAGAGAACTTACTTCGAGGATGTCTGTGGAGGGCAGAATGTCAAGAGC
TCGCCATCGGGTGCTGCGAGCGCGCCAAACAAATCTCCTGCTTTGGGCAAGAAGGCAAAGGCGGAA
CAACAGAAGCCACATAACTCGTACTGTCAGGTTGAAGGCTGCAAAGTCGACCTCTCTTCTGTTAAA
GATTACCATCGAAAGCACAGAGTCTGTGAACTTCACTCTAAGGCTCCGAAAGTTGTTGTCGCTGGT
CTGGAGCGACGCTTTTGCCAACAGTGCAGCCGGTTTCATGCTTTAGCTGAGTTTGACCAGAAAAAG
CGAAGCTGCCGTAGGCGTCTCAATGATCATAATTCCCGCAGGCGGAAGCCACAGCCAGAAGCAATT
TCTTTCAGTTCATCAAGGATGTCTACGATGTTTTATGATGCAAGGCAACAGCCAAATTTCCTATTT
GGTCAGGCTCCTTATGTTCAAATGAGAAGCTGTGGAAGTTCTTCATGGGATGACCCAGGAGGCTTC
AAAGTTACACACACAAAAGCTCCTTGGTTAAAACCAACAACTGCTGCAGGTGTTCATGGGATACAT
TTATCTAGTCAGCAGATGTCGGACAATATTATGCCACATGGTGCACATCATGGTTTCGATGGGTTC
ATGGCATTCAAGGGAACTTGTACAAAGTTCCCTAATCAAGGTGTCCAAGCTTCTGCTGTTGCTTCC
GACTCCAGTGGAGCCCCGGATCTTCAGCATGCTCTCTCTCTTCTGTCAAGCAACCCAGTGGGTGCT
GCCAACCTCCAGCCAAGTCCCCAGATGCACTCTGGGGTGGCAGCCATTGCCGGCGCCCCCAACCCC
GCGATGCACGCGCTGGGATCATCGACGGGGCTCTGGCTAGACGGCAGCCAGCCCCTCGATGATCAC
CCGCGGTTCCAGGTCTTCGAGCGCTTGGGGGACCATGACAGCGAGCTCCAGCTCCCAAAGCCTTCC
TACGACCATGCCTCGCACTTCGACCGGATGCACTGA

**SEQ ID NO: 453, protein - *Triticum aestivum***
MGSFGMEWNQRSSVLWDWENFPPIGENPKNAMQADPRFAAVAATMGNEPLHSSGGSGTFSSSSEMG
YGSSKSSMSASIDSSNRAGNNMEFRFAPVKNPDRNTSKNTELGKVDNTRTGTSPSPVVAVSSGEPV
IGLKLGKRTYFEDVCGGQNVKSSPSGAASAPNKSPALGKKAKAEQQKPHNSYCQVEGCKVDLSSVK
DYHRKHRVCELHSKAPKVVVAGLERRFCQQCSRFHALAEFDQKKRSCRRRLNDHNSRRRKPQPEAI
SFSSSRMSTMFYDARQQPNFLFGQAPYVQMRSCGSSSWDDPGGFKVTHTKAPWLKPTTAAGVHGIH
LSSQQMSDNIMPHGAHHGFDGFMAFKGTCTKFPNQGVQASAVASDSSGAPDLQHALSLLSSNPVGA
ANLQPSPQMHSGVAAIAGAPNPAMHALGSSTGLWLDGSQPLDDHPRFQVFERLGDHDSELQLPKPS
YDHASHFDRMH

**SEQ ID NO: 454, DNA - *Vitis vinifera***
ATGGATGCTGTTGTTTTGACAGGTTTGGAAGAATCTGGGGTAAGAGTAAGGGTGGTTAATTTTATA
CAGGGCGGGCGAGAAATGGCATTTTTGTTGGAATCTGTGCAAGAAGAGGACTTGGGTAGAAACATC
ATCGGTTCTACTTGGTCGACTTGGGGAAGAAAGGACAATCGATATCCTTCTCTAAAAGGGGATAAG
GAATTTCCTTTCTGTGGCATGTGGGACTGGGAGAACCTTGACATTTTCAATGCAAAATCAACTGAA
ATTCCAAAAAAGTTACAATCAGACTGGGAAATTGAAGGAGAAGGAGGAATTGACACTGGTTCTTTC
TATTCATCTGGATGTGGTGCTGGTAGTGGTGGTTCTGGCTCTGATTTGGGACATGCATATTCATCA
AAGAGCTCAAAATCAGCTTCAATTGATTCTTCATCAAAGGTGGGAATAAAGACATCCAACTTCACT
TTTGAGGCTTTCCAAGATTTTTCTCAAGATTTTAACAAGAAGAGAGATTTGGAGAGGGCTGAGCCA
ACTGGAAGTTCTCCAAACCTTGAAGCCTCGATTGGCTCTGGTGAACCTTTAATTGGTCTAAAGCTT

## FIGURE 6 (continued)

GGTAAAAGGACATACTTCGAGGATGTTTCTGCAGGGGGCAATGCTAAGGGCTCAGTGTTTTCTGTG
ATTCCCATATCATCTGATACCACAGCAAAGAGATCCAGGTTATCTTGTCAGAATGCACATGTCTCG
CGCTGCCAAGTTGAAGGATGCAACCTTGACCTTTCAACAGCCAAAGATTACCATCGCAAACATAGA
GTTTGTGAAAGTCATACCAAATGCCCAAAGGTCATTGTAGGTGGTCTGGAGCGCCGTTTTTGCCAA
CAGTGTAGCAGGTTCCATAGTTTGTCAGAGTTTGATGAAAAGAAGAGAAGCTGTCGCAGGCGTCTT
TCTGATCACAATGCACGCCGGCGCAAACCACAACCAGAAGGGATCCAGTCAAATTCGGCGAGGCTT
TCATCATTCTATGGTGGGAAGCAGCAAATGAGTCTTGTGTTGAGCTCGGTCCCCATTGTTCATACA
AGGCCTGCTGTAAATCCTTCATGGGAAGGCACATGCAGCAAGTTCACACAAACAAAAGGGTTAGTA
AGGCCTGGTAACACAGGAGGTATCGACCGGCAGCTGCATTTGCCTGGCAGTGAGCTGCAGAATGGC
ATTAGTACGCTTTGTCATGATTCCAGTAGGCTTTTGCACTCTAAGGGCGCAATAGCTGAGGTTCTC
AATCAAGTGTCCCAAGGTATGTTACATTCAACCTCAGAACACTGGAGGACCGAGCTTCCCTCAACT
GATTCCCGGGTGCATATCTTGCCTTCACAAGGCGATGGTAACACCCACTTTCAAGAGTTCAAGCTG
TTCAAAGCGCCATACGAGTCTGGCTTTTCTTCTGGATATTCAGAGTATAGGGAATTGAGATATCCG
ATTCCTAGGAGTAACGCCCGTGTCATGAAGCAAACATAG

**SEQ ID NO: 455, protein - *Vitis vinifera***
MDAVVLTGLEESGVRVRVVNFIQGGREMAFLLESVQEEDLGRNIIGSTWSTWGRKDNRYPSLKGDK
EFPFCGMWDWENLDIFNAKSTEIPKKLQSDWEIEGEGGIDTGSFYSSGCGAGSGGSGSDLGHAYSS
KSSKSASIDSSSKVGIKTSNFTFEAFQDFSQDFNKKRDLERAEPTGSSPNLEASIGSGEPLIGLKL
GKRTYFEDVSAGGNAKGSVFSVIPISSDTTAKRSRLSCQNAHVSRCQVEGCNLDLSTAKDYHRKHR
VCESHTKCPKVIVGGLERRFCQQCSRFHSLSEFDEKKRSCRRRLSDHNARRRKPQPEGIQSNSARL
SSFYGGKQQMSLVLSSVPIVHTRPAVNPSWEGTCSKFTQTKGLVRPGNTGGIDRQLHLPGSELQNG
ISTLCHDSSRLLHSKGAIAEVLNQVSQGMLHSTSEHWRTELPSTDSRVHILPSQGDGNTHFQEFKL
FKAPYESGFSSGYSEYRELRYPIPRSNARVMKQT

**SEQ ID NO: 456, protein - sbp domain of SEQ ID NO: 428**
RCQIDGCELDLSSAKGYHRKHKVCEKHSKCPKVSVSGLERRFCQQCSRFHAVSEFDEKKRSCRKRL
SHHNARRRKPQGV

**SEQ ID NO: 457, protein - sbp domain of SEQ ID NO: 433**
RCQIDGCELDLSSSKDYHRKHRVCETHSKCPKVVVSGLERRFCQQCSRFHAVSEFDEKKRSCRKRL
SHHNARRRKPQGV

**SEQ ID NO: 458, protein - sbp domain of SEQ ID NO: 435**
HCQVEGCNLDLSSAKDYHRKHRICENHSKFPKVVVSGVERRFCQQCSRFHCLSEFDEKKRSCRRRL
SDHNARRRKPNPG

**SEQ ID NO: 459, protein - sbp domain of SEQ ID NO: 437**
HCQVEGCNVDLSSAKPYHRKHRVCEPHSKTLKVIVAGLERRFCQQCSRFHGLAEFDQKKRSCRRRL
HDHNARRRKPQPE

**SEQ ID NO: 460, protein - sbp domain of SEQ ID NO: 439**
YCQVEGCKVDLSSAREYHRKHKVCEAHSKAPKVIVSGLERRFCQQCSRFHGLAEFDQKKKSCRRRL
SDHNARRRKPQQE

**SEQ ID NO: 461, protein - sbp domain of SEQ ID NO: 441**
TPAKKLKSSNHSQRAPRCQVEGCNLDLSSAKDYHRKHRVCESHSKCPKVIVAGLERRFCQQCSRFH
GLSEFDEKKKSCRRRLSDHNARRRKQPGSVHLNPSRLSSSLY

## FIGURE 6 (continued)

**SEQ ID NO: 462, protein - sbp domain of SEQ ID NO: 443**
CCQVEGCNLDLSSAKDYHRKHRVCESHSKCQKVIVAGLERRFCQQCSSKIIPSFYDCIDNLGFGCQ
RFHGLSEFDEKKKSCRRRLSDHNARRRKQPGS

**SEQ ID NO: 463, protein - sbp domain of SEQ ID NO: 445**
DLKSAKDYHRRHRICEKHSKSPKVIVAGMERRFCQQCSRFHELSEFDDKKRSCRRRLSDHNARRRR
PQPEAIRFNSARPSSSPSSFYGCHFNFKDGRQ

**SEQ ID NO: 464, protein - sbp domain of SEQ ID NO: 447**
TLSQKLQTPHCQVEGCNLDLSSAKDYHRKHRICENHSKFPKVVVSGVERRFCQQCSRKSSRYWFIQ
KPLFLIVFGLQRF

**SEQ ID NO: 465, protein - sbp domain of SEQ ID NO: 449**
SYCQVEGCRTDLSSAKDYHRKHRVCEPHSKAPKVVVAGLERRFCQQCSRFHGLAEFDQKKKSCRRR
LNDHNARRRKPQPEAL

**SEQ ID NO: 466, protein - sbp domain of SEQ ID NO: 451**
CQVEGCKVDLSSAKDYNRKHKVCVVHSKATKVVVAGLERRFCQQCSRFHGLAEFDQNKRSCRRRLM
HHNARRRKP

**SEQ ID NO: 467, protein - sbp domain of SEQ ID NO: 453**
YCQVEGCKVDLSSVKDYHRKHRVCELHSKAPKVVVAGLERRFCQQCSRFHALAEFDQKKRSCRRRL
NDHNSRRRKPQPEAISFSS

**SEQ ID NO: 468, protein - sbp domain of SEQ ID NO: 455**
RCQVEGCNLDLSTAKDYHRKHRVCESHTKCPKVIVGGLERRFCQQCSRFHSLSEFDEKKRSCRRRL
SDHNARRRKPQPEGI

**SEQ ID NO: 469, protein - motif 1**
WDWENL

**SEQ ID NO: 470, protein - motif 2**
SKSSQSTSINSS

**SEQ ID NO: 471, protein - motif 3**
ALSLLST

**SEQ ID NO: 472, protein - motif 4**
LKLGKR

**SEQ ID NO: 473, DNA - primer 1**
ggggacaagtttgtacaaaaaagcaggcttaaacaatggactgcaacatggtatct

**SEQ ID NO: 474, DNA - primer 2**
ggggaccactttgtacaagaaagctgggtcacattacttactcatctattttgg

**SEQ ID NO: 475, DNA - promoter sequence**
TNCGTACAA

**FIGURE 6 (continued)**

**SEQ ID NO: 476, DNA - *Oryza sativa***

AATCCGAAAAGTTTCTGCACCGTTTTCACCCCCTAACTAACAATATAGGGAACGTGTGCTAAATAT
AAAATGAGACCTTATATATGTAGCGCTGATAACTAGAACTATGCAAGAAAAACTCATCCACCTACT
TTAGTGGCAATCGGGCTAAATAAAAAAGAGTCGCTACACTAGTTTCGTTTTCCTTAGTAATTAAGT
GGGAAAATGAAATCATTATTGCTTAGAATATACGTTCACATCTCTGTCATGAAGTTAAATTATTCG
AGGTAGCCATAATTGTCATCAAACTCTTCTTGAATAAAAAAATCTTTCTAGCTGAACTCAATGGGT
AAAGAGAGAGATTTTTTTTAAAAAAATAGAATGAAGATATTCTGAACGTATTGGCAAAGATTTAAA
CATATAATTATATAATTTTATAGTTTGTGCATTCGTCATATCGCACATCATTAAGGACATGTCTTA
CTCCATCCCAATTTTTATTTAGTAATTAAAGACAATTGACTTATTTTTATTATTTATCTTTTTTCG
ATTAGATGCAAGGTACTTACGCACACACTTTGTGCTCATGTGCATGTGTGAGTGCACCTCCTCAAT
ACACGTTCAACTAGCAACACATCTCTAATATCACTCGCCTATTTAATACATTTAGGTAGCAATATC
TGAATTCAAGCACTCCACCATCACCAGACCACTTTTAATAATATCTAAAATACAAAAAATAATTTT
ACAGAATAGCATGAAAAGTATGAAACGAACTATTTAGGTTTTTCACATACAAAAAAAAAAAGAATT
TTGCTCGTGCGCGAGCGCCAATCTCCCATATTGGGCACACAGGCAACAACAGAGTGGCTGCCCACA
GAACAACCCACAAAAAACGATGATCTAACGGAGGACAGCAAGTCCGCAACAACCTTTTAACAGCAG
GCTTTGCGGCCAGGAGAGAGGAGGAGAGGCAAAGAAAACCAAGCATCCTCCTTCTCCCATCTATAA
ATTCCTCCCCCCTTTTCCCCTCTCTATATAGGAGGCATCCAAGCCAAGAAGAGGGAGAGCACCAAG
GACACGCGACTAGCAGAAGCCGAGCGACCGCCTTCTCGATCCATATCTTCCGGTCGAGTTCTTGGT
CGATCTCTTCCCTCCTCCACCTCCTCCTCACAGGGTATGTGCCTCCCTTCGGTTGTTCTTGGATTT
ATTGTTCTAGGTTGTGTAGTACGGGCGTTGATGTTAGGAAAGGGGATCTGTATCTGTGATGATTCC
TGTTCTTGGATTTGGGATAGAGGGGTTCTTGATGTTGCATGTTATCGGTTCGGTTTGATTAGTAGT
ATGGTTTTCAATCGTCTGGAGAGCTCTATGGAAATGAAATGGTTTAGGGATCGGAATCTTGCGATT
TTGTGAGTACCTTTTGTTTGAGGTAAAATCAGAGCACCGGTGATTTTGCTTGGTGTAATAAAGTAC
GGTTGTTTGGTCCTCGATTCTGGTAGTGATGCTTCTCGATTTGACGAAGCTATCCTTTGTTTATTC
CCTATTGAACAAAAATAATCCAACTTTGAAGACGGTCCCGTTGATGAGATTGAATGATTGATTCTT
AAGCCTGTCCAAAATTTCGCAGCTGGCTTGTTTAGATACAGTAGTCCCCATCACGAAATTCATGGA
AACAGTTATAATCCTCAGGAACAGGGGATTCCCTGTTCTTCCGATTTGCTTTAGTCCCAGAATTTT
TTTTCCCAAATATCTTAAAAAGTCACTTTCTGGTTCAGTTCAATGAATTGATTGCTACAAATAATG
CTTTTATAGCGTTATCCTAGCTGTAGTTCAGTTAATAGGTAATACCCCTATAGTTTAGTCAGGAGA
AGAACTTATCCGATTTCTGATCTCCATTTTTAATTATATGAAATGAACTGTAGCATAAGCAGTATT
CATTTGGATTATTTTTTTTATTAGCTCTCACCCCTTCATTATTCTGAGCTGAAAGTCTGGCATGAA
CTGTCCTCAATTTTGTTTTCAAATTCACATCGATTATCTATGCATTATCCTCTTGTATCTACCTGT
AGAAGTTTCTTTTTGGTTATTCCTTGACTGCTTGATTACAGAAAGAAATTTATGAAGCTGTAATCG
GGATAGTTATACTGCTTGTTCTTATGATTCATTTCCTTTGTGCAGTTCTTGGTGTAGCTTGCCACT
TTCACCAGCAAAGTTC

**SEQ ID NO: 477, DNA - *Oryza sativa***

GCTTGAGTCATAGGGAGAAAACAAATCGATCATATTTGACTCTTTTCCCTCCATCTCTCTTACCGG
CAAAAAAAGTAGTACTGGTTTATATGTAAAGTAAGATTCTTTAATTATGTGAGATCCGGCTTAATG
CTTTTCTTTTGTCACATATACTGCATTGCAACAATTGCCATATATTCACTTCTGCCATCCCATTAT
ATAGCAACTCAAGAATGGATTGATATATCCCCTATTACTAATCTAGACATGTTAAGGCTGAGTTGG
GCAGTCCATCTTCCCAACCCACCACCTTCGTTTTTCGCGCACATACTTTTCAAACTACTAAATGGT
GTGTTTTTTAAAAATATTTTCAATACAAAAGTTGCTTTAAAAAATTATATTGATCCATTTTTTTAA
AAAAAATAGCTAATACTTAATTAATCACGTGTTAAAAGACCGCTCCGTTTTGCGTGCAGGAGGGAT
AGGTTCACATCCTGCATTACCGAACACAGCCTAAATCTTGTTGTCTAGATTCGTAGTACTGGATAT
ATTAAATCATGTTCTAAGTTACTATATACTGAGATGAATAGAATAAGTAAAATTAGACCCACCTTA
AGTCTTGATGAAGTTACTACTAGCTGCGTTTGGGAGGACTTCCCAAAAAAAAAAGTATTAGCCATT
AGCACGTGATTAATTAAGTACTAGTTTAAAAAACTTAAAAAATAAATTAATATGATTCTCTTAAGT

**FIGURE 6 (continued)**

AACTCTCCTATAGAAAACTTTTACAAAATTACACCGTTTAATAGTTTGGAAAATATGTCAGTAAAA
AATAAGAGAGTAGAAGTTATGAAAGTTAGAAAAAGAATTGTTTTAGTAGTATACAGTTATAAACTA
TTCCCTCTGTTCTAAAACATAAGGGATTATGGATGGATTCGACATGTACCAGTACCATGAATCGAA
TCCAGACAAGTTTTTTATGCATATTTATTCTACTATAATATATCACATCTGCTCTAAATATCTTAT
ATTTCGAGGTGGAGACTGTCGCTATGTTTTTCTGCCCGTTGCTAAGCACACGCCACCCCCGATGCG
GGGACGCCTCTGGCCTTCTTGCCACGATAATTGAATGGAACTTCCACATTCAGATTCGATAGGTGA
CCGTCGACTCCAAGTGCTTTGCACAAAACAACTCCGGCCTCCCGGCCACCAGTCACACGACTCACG
GCACTACCACCCCTGACTCCCTGAGGCGGACCTGCCACTGTTCTGCATGCGAAGCTATCTAAAATT
CTGAAGCAAAGAAAGCACAGCACATGCTCCGGGACACGCGCCACCCGGCGGAAAAGGGCTCGGTGT
GGCGATCTCACAGCCGCATATCGCATTTCACAAGCCGCCCATCTCCACCGGCTTCACGAGGCTCAT
CGCGGCACGACCGCGCACGGAACGCACGCGGCCGACCCGCGCGCCTCGATGCGCGAGCCCATCCGC
CGCGTCCTCCCTTTGCCTTTGCCGCTATCCTCTCGGTCGTATCCCGTTTCTCTGTCTTTTGCTCCC
CGGCGCGCGCCAGTTCGGAGTACCAGCGAAACCCGGACACCTGGTACACCTCCGCCGGCCACAACG
CGTGTCCCCCTACGTGGCCGCGCAGCACATGCCCATGCGCGACACGTGCACCTCCTCATCCAAACT
CTCAAGTCTCAACGGTCCTATAAATGCACGGATAGCCTCAAGCTGCTCGTCACAAGGCAAGAGGCA
AGAGGCAAGAGCATCCGTATTAACCAGCCTTTTGAGACTTGAGAGTGTGTGTGACTCGATCCAGCG
TAGTTTCAGTTCGTGTGTTGGTGAGTGATTCCAGCCAAGTTTGCG

**SEQ ID NO: 478, protein - consensus sbp domain of SPL11 polypeptides**

HCQVEGCNLDLSSAKDYHRKHRVCEXHSKXPKVIVAGLERRFCQQCSRXXXXXXXXXXXXXXXXX
XXXFHGLSEFDEKKRSCRRRLSDHNARRRKPQPE

FIGURE 6 (continued)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1033405 A
- US 5811238 A
- US 6395547 A
- WO 2004070039 A
- WO 2004065596 A
- US 4962028 A
- WO 0114572 A
- WO 9514098 A
- WO 9412015 A
- US 5401836 A
- US 20050044585 A
- EP 99106056 A
- US 5565350 A, Kmiec
- WO 9322443 A, Zarling
- WO 9853083 A, Grierson
- WO 9953050 A, Waterhouse
- US 4987071 A, Cech
- US 5116742 A, Cech
- WO 9400012 A, Atkins
- WO 9503404 A, Lenne
- WO 0000619 A, Lutziger
- WO 9713865 A, Prinsen
- WO 9738116 A, Scott
- WO 9836083 A
- WO 9915682 A
- WO 0015815 A
- EP 1198985 A1
- US 5164310 A
- EP 07107448 A
- US 60916575 B
- EP 07109052 A
- EP 07109068 A
- US 60942214 B
- EP 07109961 A
- US 60937989 B
- EP 07110548 A
- EP 07110557 A
- US 60948036 B


### Non-patent literature cited in the description


- **WANG et al.** *Planta,* 2003, vol. 218, 1-14
- **KLEIN et al.** *Mol Gen Genet,* 1996, vol. 259, 7-16
- **CARDON et al.** *Gene,* 1999, vol. 237, 91-104
- **YAMASAKI et al.** *J Mol Biol,* 2004, vol. 337, 49-63
- **RHOADES et al.** *Cell,* 2002, vol. 110, 513-520
- **RIECHMANN et al.** *Science,* 2000, vol. 290, 2105-2109
- **CARDON et al.** *Plant J,* 1997, vol. 12, 367-377
- **CREIGHTON.** Proteins. W.H. Freeman and Company, 1984
- **TERPE.** *Appl. Microbiol. Biotechnol.,* 2003, vol. 60, 523-533
- **MEINKOTH ; WAHL.** *Anal. Biochem.,* 1984, vol. 138, 267-284
- **SAMBROOK et al.** Molecular Cloning: a laboratory manual. Cold Spring Harbor Laboratory Press, 2001
- Current Protocols in Molecular Biology. John Wiley & Sons, 1989
- **FOISSAC ; SCHIEX.** *BMC Bioinformatics,* 2005, vol. 6, 25
- **CASTLE et al.** *Science,* 2004, vol. 304 (5674), 1151-4
- **HEID et al.** *Genome Methods,* 1996, vol. 6, 986-994
- **MCELROY et al.** *Plant Cell,* 1990, vol. 2, 163-171
- **ODELL et al.** *Nature,* 1985, vol. 313, 810-812
- **NILSSON et al.** *Physiol. Plant.,* 1997, vol. 100, 456-462
- **DE PATER et al.** *Plant J,* November 1992, vol. 2 (6), 837-44
- **CHRISTENSEN et al.** *Plant Mol. Biol.,* 1992, vol. 18, 675-689
- **BUCHHOLZ et al.** *Plant Mol Biol.,* 1994, vol. 25 (5), 837-43
- **LEPETIT et al.** *Mol. Gen. Genet.,* 1992, vol. 231, 276-285
- **WU et al.** *Plant Mol. Biol.,* 1988, vol. 11, 641-649
- **AN et al.** *Plant J.,* 1996, vol. 10 (1), 107-121
- **SANGER et al.** *Plant. Mol. Biol.,* 1990, vol. 14, 433-443
- **LEISNER.** *Proc Natl Acad Sci USA,* 1988, vol. 85 (5), 2553
- **JAIN et al.** *Crop Science,* 1999, vol. 39 (6), 1696
- **SHAW et al.** *Nucleic Acids Res.,* 1984, vol. 12 (20), 7831-7846

- **GATZ.** *Annu. Rev. Plant Physiol. Plant Mol. Biol.,* 1997, vol. 48, 89-108
- *Plant Mol Biol.,* January 1995, vol. 27 (2), 237-48
- **MUDGE et al.** *Plant J.,* 2002, vol. 31, 341
- **NITZ et al.** *Plant Sci,* 2001, vol. 161 (2), 337-346
- **TINGEY et al.** *EMBO J.,* 1987, vol. 6, 1
- **VAN DER ZAAL et al.** *Plant Mol. Biol.,* 1991, vol. 16, 983
- **OPPENHEIMER et al.** *Gene,* 1988, vol. 63, 87
- **CONKLING et al.** *Plant Physiol.,* 1990, vol. 93, 1203
- **SUZUKI et al.** *Plant Mol. Biol.,* 1993, vol. 21, 109-119
- **BAUMBERGER et al.** *Genes & Dev.,* 2001, vol. 15, 1128
- **LAUTER et al.** *PNAS,* 1996, vol. 3, 8139
- **LIU et al.** *Plant Mol. Biol.,* 1991, vol. 153, 386-395
- **DOWNEY et al.** *J. Biol. Chem.,* 2000, vol. 275, 39420
- **W SONG.** *PhD Thesis,* 1997
- **WANG et al.** *Plant Sci.,* 2002, vol. 163, 273
- **DIENER et al.** *Plant Cell,* 2001, vol. 13, 1625
- **QING QU ; TAKAIWA.** *Plant Biotechnol. J.,* 2004, vol. 2, 113-125
- **SIMON et al.** *Plant Mol. Biol.,* 1985, vol. 5, 191
- **SCOFIELD et al.** *J. Biol. Chem.,* 1987, vol. 262, 12202
- **BASZCZYNSKI et al.** *Plant Mol. Biol.,* 1990, vol. 14, 633
- **PEARSON et al.** *Plant Mol. Biol.,* 1992, vol. 18, 235-245
- **ELLIS et al.** *Plant Mol. Biol.,* 1988, vol. 10, 203-214
- **TAKAIWA et al.** *Mol. Gen. Genet.,* 1986, vol. 208, 15-22
- **TAKAIWA et al.** *FEBS Letts,* 1987, vol. 221, 43-47
- **MATZKE et al.** *Plant Mol Biol,* 1990, vol. 14 (3), 323-32
- **STALBERG et al.** *Planta,* 1996, vol. 199, 515-519
- *Mol Gen Genet,* 1989, vol. 216, 81-90
- *NAR,* 1989, vol. 17, 461-2
- **ALBANI et al.** *Plant Cell,* 1997, vol. 9, 171-184
- *EMBO J.,* 1984, vol. 3, 1409-15
- **DIAZ et al.** *Mol Gen Genet,* 1995, vol. 248 (5), 592-8
- *Theor Appl Gen,* 1999, vol. 98, 1253-62
- *Plant J,* 1993, vol. 4, 343-55
- *Mol Gen Genet,* 1996, vol. 250, 750-60
- **MENA et al.** *The Plant Journal,* 1998, vol. 116 (1), 53-62
- **VICENTE-CARBAJOSA et al.** *Plant J.,* 1998, vol. 13, 629-640
- **WU et al.** *Plant Cell Physiology,* 1998, vol. 39 (8), 885-889
- **SATO et al.** *Proc. Natl. Acad. Sci. USA,* 1996, vol. 93, 8117-8122
- **NAKASE et al.** *Plant Mol. Biol.,* 1997, vol. 33, 513-522
- *Trans Res,* 1997, vol. 6, 157-68
- *Plant J,* 1997, vol. 12, 235-46
- **DEROSE et al.** *Plant Mol. Biol,* 1996, vol. 32, 1029-35
- **POSTMA-HAARSMA et al.** *Plant Mol. Biol.,* 1999, vol. 39, 257-71
- **WU et al.** *J. Biochem.,* 1998, vol. 123, 386
- **CUMMINS et al.** *Plant Mol. Biol.,* 1992, vol. 19, 873-876
- **LANAHAN et al.** *Plant Cell,* 1992, vol. 4, 203-211
- **SKRIVER et al.** *Proc Natl Acad Sci USA,* 1991, vol. 88, 7266-7270
- **CEJUDO et al.** *Plant Mol Biol,* 1992, vol. 20, 849-856
- **KALLA et al.** *Plant J.,* 1994, vol. 6, 849-60
- **LEAH et al.** *Plant J.,* 1994, vol. 4, 579-89
- **SELINGER et al.** *Genetics,* 1998, vol. 149, 1125-38
- **TAKAIWA et al.** *Mol Gen Genet,* 1986, vol. 208, 15-22
- **COLOT et al.** *Mol Gen Genet,* 1989, vol. 216, 81-90
- **ANDERSON et al.** *NAR,* 1989, vol. 17, 461-2
- **RAFALSKI et al.** *EMBO,* 1984, vol. 3, 1409-15
- **CHO et al.** *Theor Appl Genet,* 1999, vol. 98, 1253-62
- **MULLER et al.** *Plant J,* 1993, vol. 4, 343-55
- **SORENSON et al.** *Mol Gen Genet,* 1996, vol. 250, 750-60
- **MENA et al.** *Plant J,* 1998, vol. 116 (1), 53-62
- **ONATE et al.** *J Biol Chem,* 1999, vol. 274 (14), 9175-82
- **VICENTE-CARBAJOSA et al.** *Plant J,* 1998, vol. 13, 629-640
- **WU et al.** *Plant Cell Physiol,* 1998, vol. 39 (8), 885-889
- **NAKASE et al.** *Plant Molec Biol,* 1997, vol. 33, 513-522
- **RUSSELL et al.** *Trans Res,* 1997, vol. 6, 157-68
- **OPSAHL-FERSTAD et al.** *Plant J,* 1997, vol. 12, 235-46
- **DEROSE et al.** *Plant Mol Biol,* 1996, vol. 32, 1029-35
- **WAGNER ; KOHORN.** *Plant Cell,* 2001, vol. 13 (2), 303-318
- **BUCHMAN ; BERG.** *Mol. Cell biol.,* 1988, vol. 8, 4395-4405
- **CALLIS et al.** *Genes Dev,* 1987, vol. 1, 1183-1200
- The Maize Handbook. Springer, 1994

- **GAULTIER et al.** *Nucl Ac Res,* 1987, vol. 15, 6625-6641
- **INOUE et al.** *Nucl Ac Res,* 1987, vol. 15, 6131-6148
- **INOUE et al.** *FEBS Lett.,* 1987, vol. 215, 327-330
- **HASELHOFF ; GERLACH.** *Nature,* 1988, vol. 334, 585-591
- **BARTEL ; SZOSTAK.** *Science,* 1993, vol. 261, 1411-1418
- **ANGELL ; BAULCOMBE.** *Plant J,* 1999, vol. 20 (3), 357-62
- **HELENE, C.** *Anticancer Drug Res.,* 1991, vol. 6, 569-84
- **HELENE et al.** *Ann. N.Y. Acad. Sci.,* 1992, vol. 660, 27-36
- **MAHER, L.J.** *Bioassays,* 1992, vol. 14, 807-15
- **SCHWAB et al.** *Dev. Cell,* 2005, vol. 8, 517-527
- **SCHWAB et al.** *Plant Cell,* 2006, vol. 18, 1121-1133
- **TRIBBLE et al.** *J. Biol. Chem.,* 2000, vol. 275, 22255-22267
- **VELMURUGAN et al.** *J. Cell Biol.,* 2000, vol. 149, 553-566
- **KRENS, F.A. et al.** *Nature,* 1982, vol. 296, 72-74
- **NEGRUTIU I et al.** *Plant Mol Biol,* 1987, vol. 8, 363-373
- **SHILLITO R.D. et al.** *Bio/Technol,* 1985, vol. 3, 1099-1102
- **CROSSWAY A et al.** *Mol. Gen Genet,* 1986, vol. 202, 179-185
- **KLEIN TM et al.** *Nature,* 1987, vol. 327, 70
- **CLOUGH ; BENT.** *Plant J.,* 1998, vol. 16, 735-743
- **ALDEMITA ; HODGES.** *Planta,* 1996, vol. 199, 612-617
- **CHAN et al.** *Plant Mol Biol,* 1993, vol. 22 (3), 491-506
- **HIEI et al.** *Plant J,* 1994, vol. 6 (2), 271-282
- **ISHIDA et al.** *Nat. Biotechnol,* 1996, vol. 14 (6), 745-50
- **FRAME et al.** *Plant Physiol,* 2002, vol. 129 (1), 13-22
- Techniques for Gene Transfer. **B. JENES et al.** Transgenic Plants, Vol. 1, Engineering and Utilization. Academic Press, 1993, vol. 1, 128-143
- **POTRYKUS.** *Annu. Rev. Plant Physiol. Plant Molec. Biol.,* 1991, vol. 42, 205-225
- **BEVAN et al.** *Nucl. Acids Res.,* 1984, vol. 12, 8711
- **HÖFGEN ; WILLMITZER.** *Nucl. Acid Res.,* 1988, vol. 16, 9877
- Vectors for Gene Transfer in Higher Plants. **F.F. WHITE.** Transgenic Plants, Vol. 1, Engineering and Utilization. Academic Press, 1993, 15-38
- **FELDMAN, KA ; MARKS MD.** *Mol Gen Genet,* 1987, vol. 208, 274-289
- **FELDMANN K.** Methods in Arabidopsis Research. Word Scientific, 1992, 274-289
- **CHANG.** *Plant J.,* 1994, vol. 5, 551-558
- **KATAVIC.** *Mol Gen Genet,* 1994, vol. 245, 363-370
- **BECHTHOLD, N.** *C R Acad Sci Paris Life Sci,* 1993, vol. 316, 1194-1199
- **CLOUGH, SJ ; BENT AF.** *The Plant J.,* 1998, vol. 16, 735-743
- **KLAUS et al.** *Nature Biotechnology,* 2004, vol. 22 (2), 225-229
- **BOCK.** Transgenic plastids in basic research and plant biotechnology. *J Mol Biol.,* 21 September 2001, vol. 312 (3), 425-38
- **MALIGA, P.** Progress towards commercialization of plastid transformation technology. *Trends Biotechnol.,* 2003, vol. 21, 20-28
- **REDEI GP ; KONCZ C.** Methods in Arabidopsis Research. World Scientific Publishing Co, 1992, 16-82
- **FELDMANN et al.** Arabidopsis. Cold Spring Harbor Laboratory Press, 1994, 137-172
- **LIGHTNER J ; CASPAR T.** Methods on Molecular Biology. Humana Press, 1998, vol. 82, 91-104
- **MCCALLUM et al.** *Nat Biotechnol,* 2000, vol. 18, 455-457
- **STEMPLE.** *Nat Rev Genet,* 2004, vol. 5 (2), 145-50
- **OFFRINGA et al.** *EMBO J,* 1990, vol. 9 (10), 3077-84
- **TERADA et al.** *Nat Biotech,* 2002, vol. 20 (10), 1030-4
- **LIDA ; TERADA.** *Curr Opin Biotech,* 2004, vol. 15 (2), 132-8
- **MILLER et al.** *Nature Biotechnol.,* 2007, vol. 25, 778-785
- **FINN et al.** *Nucleic Acids Research,* 2006, vol. 34, D247-D251
- **SCHULTZ et al.** *Proc. Natl. Acad. Sci. USA,* 1998, vol. 95, 5857-5864
- **LETUNIC et al.** *Nucleic Acids Res,* 2002, vol. 30, 242-244
- **MULDER et al.** *Nucl. Acids. Res.,* 2003, vol. 31, 315-318
- A generalized profile syntax for biomolecular sequences motifs and its function in automatic sequence interpretation. **BUCHER ; BAIROCH.** Proceedings 2nd International Conference on Intelligent Systems for Molecular Biology. AAAI Press, 1994, 53-61
- **HULO et al.** *Nucl. Acids. Res.,* 2004, vol. 32, D134-D137
- **BATEMAN et al.** *Nucleic Acids Research,* 2002, vol. 30 (1), 276-280

- **GASTEIGER et al.** ExPASy: the proteomics server for in-depth protein knowledge and analysis. *Nucleic Acids Res.,* 2003, vol. 31, 3784-3788
- **NEEDLEMAN ; WUNSCH.** *J Mol Biol,* 1970, vol. 48, 443-453
- **ALTSCHUL et al.** *J Mol Biol,* 1990, vol. 215, 403-10
- **CAMPANELLA et al.** MatGAT: an application that generates similarity/identity matrices using protein or DNA sequences. *BMC Bioinformatics,* 10 July 2003, vol. 4, 29
- **KLEIN.** *Mol Gen Genet.,* 1996, vol. 250 (1), 7-16
- **YAMASAKI et al.** *J Mol Biol.,* 2004, vol. 337 (1), 49-63
- **GRIFFITHS-JONES et al.** *Nucleic Acids Research,* 2006, vol. 34, D140-D144
- Current Protocols in Molecular Biology
- **RABBANI et al.** *Plant Physiol,* 2003, vol. 133, 1755-1767
- **SAMBROOK J ; FRITSCH EF ; MANIATIS T.** Molecular Cloning, A Laboratory Manual. 1989
- **LANDER et al.** *Genomics,* 1987, vol. 1, 174-181
- **BOTSTEIN et al.** *Am. J. Hum. Genet.,* 1980, vol. 32, 314-331
- **BERNATZKY ; TANKSLEY.** *Plant Mol. Biol. Reporter,* 1986, vol. 4, 37-41
- **HOHEISEL et al.** Non-mammalian Genomic Analysis: A Practical Guide. Academic press, 1996, 319-346
- **TRASK.** *Trends Genet.,* 1991, vol. 7, 149-154
- **LAAN et al.** *Genome Res.,* 1995, vol. 5, 13-20
- **KAZAZIAN.** *J. Lab. Clin. Med,* 1989, vol. 11, 95-96
- **SHEFFIELD et al.** *Genomics,* 1993, vol. 16, 325-332
- **LANDEGREN et al.** *Science,* 1988, vol. 241, 1077-1080
- **SOKOLOV.** *Nucleic Acid Res.,* 1990, vol. 18, 3671
- **WALTER et al.** *Nat. Genet.,* 1997, vol. 7, 22-28
- **DEAR ; COOK.** *Nucleic Acid Res.,* 1989, vol. 17, 6795-6807
- **XIE et al.** *Plant Physiology,* 2006, vol. 142, 280-293
- **SAMBROOK.** Molecular Cloning: a laboratory manual. Cold Spring Harbor Laboratory Press, 2001, vol. 1
- **AUSUBEL et al.** Current Protocols in Molecular Biology, Current Protocols. 1994, vol. 2
- **R.D.D. CROY.** Plant Molecular Biology Labfax. BIOS Scientific Publications Ltd (UK), 1993
- **ALTSCHUL et al.** *J. Mol. Biol.,* 1990, vol. 215, 403-410
- **ALTSCHUL et al.** *Nucleic Acids Res.,* 1997, vol. 25, 3389-3402
- **THOMPSON et al.** *Nucleic Acids Res,* 1997, vol. 25, 4876-4882
- **CHENNA et al.** *Nucleic Acids Res,* 2003, vol. 31, 3497-3500
- **CAMPANELLA JJ ; BITINCKA L ; SMALLEY J.** MatGAT: an application that generates similarity/identity matrices using protein or DNA sequences. *BMC Bioinformatics,* 2003, vol. 4, 29
- **ISHIDA et al.** *Nature Biotech,* 1996, vol. 14 (6), 745-50
- **BABIC et al.** *Plant Cell Rep,* 1998, vol. 17, 183-188
- **MCKERSIE et al.** *Plant Physiol,* 1999, vol. 119, 839-847
- **BROWN DCW ; A ATANASSOV.** *Plant Cell Tissue Organ Culture,* 1985, vol. 4, 111-112
- **WALKER et al.** *Am J Bot,* 1978, vol. 65, 654-659

SEQUENCE LISTING

<110> BASF Plant Science GmbH

<120> Plants having enhanced yield-related traits and a method for making the same

<130> PF59065

<150> EP 07107448.8
<151> 2007-05-03

<150> US 60/916575
<151> 2007-05-08

<150> EP 07109052.6
<151> 2007-05-29

<150> EP 07109068.2
<151> 2007-05-29

<150> US 60/942214
<151> 2007-06-06

<150> EP 07109961.8
<151> 2007-06-11

<150> US 60/937989
<151> 2007-06-29

<150> EP 07110548.0
<151> 2007-06-19

<150> EP 07110557.1
<151> 2007-06-19

<150> US 60/948036
<151> 2007-07-05

<160> 478

<170> PatentIn version 3.3

<210> 1
<211> 753
<212> DNA
<213> Arabidopsis thaliana

<400> 1

```
atgagctgca atggttgccg tgttctccgg aaaggttgca gcgagaattg tatcctccgg     60
ccatgtattc aatggattga aaccgccgat gctcaaggcc acgccaccgt cttcgtcgct    120
aaattcttcg gccgtgctgg tctcatgtcc tttatctccg ctgttccgga ttctcaacgt    180
cctgctttgt ttcagtcgtt gctctacgaa gcttgtggaa gaactgtcaa tccagttaac    240
ggagcaatcg gaatgttatg gactggaaac tggaatatct gtcaagcggc tgttgaaaca    300
gtgcttcgcg gcggttcttt aagaccgatc ccggagcttc tcactcacgg cggcggtttc    360
gctggctttc cttcgcctac atctgaagaa gcatctgaga tctgcaccga aatgttgaat    420
ctccagcaaa atgattccac cgatcgtaac atctatcatc attcacgatt ctcaagctct    480
agatctagat ctactatgga ttcttcttct ccgacgaaac gtaagagatt atcatcggaa    540
gaccaaccat cttcggagct tgatctatct ctcatcccta attttcccat taagcaagca    600
acaccttctt ctacacggcg gcgatcagta acaccgtcga tgaactcaga ggactccggg    660
acgacgacga ctacgacggc gttttgtgac aagggtgatg tgtacggtaa cggaggagga    720

gaaacgacca agttgcttaa ccttttgtt taa                                  753
```

<210> 2
<211> 250
<212> PRT
<213> Arabidopsis thaliana

<400> 2

```
Met Ser Cys Asn Gly Cys Arg Val Leu Arg Lys Gly Cys Ser Glu Asn
1               5                   10                  15
Cys Ile Leu Arg Pro Cys Ile Gln Trp Ile Glu Thr Ala Asp Ala Gln
            20                  25                  30
Gly His Ala Thr Val Phe Val Ala Lys Phe Phe Gly Arg Ala Gly Leu
        35                  40                  45
Met Ser Phe Ile Ser Ala Val Pro Asp Ser Gln Arg Pro Ala Leu Phe
    50                  55                  60
Gln Ser Leu Leu Tyr Glu Ala Cys Gly Arg Thr Val Asn Pro Val Asn
65                  70                  75                  80
Gly Ala Ile Gly Met Leu Trp Thr Gly Asn Trp Asn Ile Cys Gln Ala
                85                  90                  95
Ala Val Glu Thr Val Leu Arg Gly Gly Ser Leu Arg Pro Ile Pro Glu
            100                 105                 110
Leu Leu Thr His Gly Gly Gly Phe Ala Gly Phe Pro Ser Pro Thr Ser
        115                 120                 125
Glu Glu Ala Ser Glu Ile Cys Thr Glu Met Leu Asn Leu Gln Gln Asn
    130                 135                 140
Asp Ser Thr Asp Arg Asn Ile Tyr His His Ser Arg Phe Ser Ser Ser
145                 150                 155                 160
Arg Ser Arg Ser Thr Met Asp Ser Ser Ser Pro Thr Lys Arg Lys Arg
                165                 170                 175
Leu Ser Ser Glu Asp Gln Pro Ser Ser Glu Leu Asp Leu Ser Leu Ile
            180                 185                 190
Pro Asn Phe Pro Ile Lys Gln Ala Thr Pro Ser Ser Thr Arg Arg Arg
        195                 200                 205
Ser Val Thr Pro Ser Met Asn Ser Glu Asp Ser Gly Thr Thr Thr Thr
    210                 215                 220
Thr Thr Ala Phe Cys Asp Lys Gly Asp Val Tyr Gly Asn Gly Gly Gly
225                 230                 235                 240
Glu Thr Thr Lys Leu Leu Asn Leu Phe Val
                245                 250
```

<210> 3
<211> 53
<212> DNA
<213> Artificial sequence

<220>
<223> primer: prm009067

<400> 3
ggggacaagt ttgtacaaaa aagcaggctt aaacaatgag ctgcaatggt tgc 53

<210> 4
<211> 50
<212> DNA
<213> Artificial sequence

<220>
<223> primer: prm009068

<400> 4
ggggaccact ttgtacaaga aagctgggta ctaactctga gaaaaccgcc 50

<210> 5
<211> 14
<212> PRT
<213> Artificial sequence

<220>
<223> conserved motif 1

<220>
<221> UNSURE
<222> (8)..(8)
<223> Xaa can be any naturally occurring amino acid

<220>
<221> UNSURE
<222> (14)..(14)
<223> Xaa can be any naturally occurring amino acid

<400> 5

```
Met Ser Cys Asn Gly Cys Arg Xaa Leu Arg Lys Gly Cys Xaa
1               5                   10
```

<210> 6
<211> 14
<212> PRT
<213> Artificial sequence

<220>
<223> conserved motif 2

<220>
<221> UNSURE
<222> (2)..(3)
<223> Xaa can be any naturally occurring amino acid

<220>
<221> UNSURE
<222> (6) .. (6)
<223> Xaa can be any naturally occurring amino acid

<220>
<221> UNSURE
<222> (8)..(8)
<223> Xaa can be any naturally occurring amino acid

<220>
<221> UNSURE
<222> (12)..(12)
<223> Xaa can be any naturally occurring amino acid

<400> 6

```
Gln Xaa Xaa Ala Thr Xaa Phe Xaa Ala Lys Phe Xaa Gly Arg
1               5                   10
```

<210> 7
<211> 10
<212> PRT
<213> Artificial sequence

<220>
<223> conserved motif 3

<220>
<221> UNSURE
<222> (2)..(2)
<223> Xaa can be any naturally occurring amino acid

<220>
<221> UNSURE
<222> (6)..(6)
<223> Xaa can be any naturally occurring amino acid

<220>
<221> UNSURE
<222> (9)..(9)
<223> Xaa can be any naturally occurring amino acid

<400> 7

```
Phe Xaa Ser Leu Leu Xaa Glu Ala Xaa Gly
1               5                   10
```

<210> 8
<211> 5
<212> PRT
<213> Artificial sequence

<220>
<223> DP(V/I)YG signature

<220>
<221> VARIANT
<222> (3)..(3)
<223> /replace = "Ile"

<400> 8

```
Asp Pro Val Tyr Gly
1               5
```

<210> 9
<211> 15
<212> PRT
<213> Artificial sequence

<220>
<223> conserved Cys motif

<220>
<221> UNSURE
<222> (2)..(3)
<223> Xaa can any naturally occurring amino acid

<220>
<221> UNSURE
<222> (5)..(10)
<223> Xaa can any naturally occurring amino acid

<220>
<221> UNSURE

<222> (12)..(14)
<223> Xaa can any naturally occurring amino acid

<400> 9

```
Cys Xaa Xaa Cys Xaa Xaa Xaa Xaa Xaa Xaa Cys Xaa Xaa Xaa Cys
1               5                   10                  15
```

<210> 10
<211> 2194
<212> DNA
<213> Oryza sativa

<400> 10

```
aatccgaaaa gtttctgcac cgttttcacc ccctaactaa caatataggg aacgtgtgct     60
aaatataaaa tgagacctta tatatgtagc gctgataact agaactatgc aagaaaaact    120
catccaccta ctttagtggc aatcgggcta aataaaaaag agtcgctaca ctagtttcgt    180
tttccttagt aattaagtgg gaaaatgaaa tcattattgc ttagaatata cgttcacatc    240
tctgtcatga agttaaatta ttcgaggtag ccataattgt catcaaactc ttcttgaata    300
aaaaaatctt tctagctgaa ctcaatgggt aaagagagag atttttttta aaaaaataga    360
atgaagatat tctgaacgta ttggcaaaga tttaaacata taattatata attttatagt    420
ttgtgcattc gtcatatcgc acatcattaa ggacatgtct tactccatcc caatttttat    480
ttagtaatta aagacaattg acttattttt attatttatc tttttcgat tagatgcaag    540

gtacttacgc acacactttg tgctcatgtg catgtgtgag tgcacctcct caatacacgt    600
tcaactagca acacatctct aatatcactc gcctatttaa tacatttagg tagcaatatc    660
tgaattcaag cactccacca tcaccagacc acttttaata atatctaaaa tacaaaaaat    720
aattttacag aatagcatga aaagtatgaa acgaactatt taggtttttc acatacaaaa    780
aaaaaaagaa ttttgctcgt gcgcgagcgc caatctccca tattgggcac acaggcaaca    840
acagagtggc tgcccacaga acaacccaca aaaaacgatg atctaacgga ggacagcaag    900
tccgcaacaa ccttttaaca gcaggctttg cggccaggag agaggaggag aggcaaagaa    960
aaccaagcat cctccttctc ccatctataa attcctcccc ccttttcccc tctctatata   1020
ggaggcatcc aagccaagaa gagggagagc accaaggaca cgcgactagc agaagccgag   1080
cgaccgcctt ctcgatccat atcttccggt cgagttcttg gtcgatctct tccctcctcc   1140
acctcctcct cacagggtat gtgcctccct tcggttgttc ttggatttat tgttctaggt   1200
tgtgtagtac gggcgttgat gttaggaaag gggatctgta tctgtgatga ttcctgttct   1260
tggatttggg atagaggggt tcttgatgtt gcatgttatc ggttcggttt gattagtagt   1320
atggttttca atcgtctgga gagctctatg gaaatgaaat ggtttaggga tcggaatctt   1380
gcgattttgt gagtaccttt tgtttgaggt aaaatcagag caccggtgat tttgcttggt   1440
gtaataaagt acggttgttt ggtcctcgat tctggtagtg atgcttctcg atttgacgaa   1500
gctatccttt gtttattccc tattgaacaa aaataatcca actttgaaga cggtcccgtt   1560
gatgagattg aatgattgat tcttaagcct gtccaaaatt tcgcagctgg cttgtttaga   1620
tacagtagtc cccatcacga aattcatgga aacagttata atcctcagga acaggggatt   1680
ccctgttctt ccgatttgct ttagtcccag aattttttttt cccaaatatc ttaaaaagtc   1740
actttctggt tcagttcaat gaattgattg ctacaaataa tgcttttata gcgttatcct   1800
agctgtagtt cagttaatag gtaatacccc tatagtttag tcaggagaag aacttatccg   1860
atttctgatc tccattttta attatatgaa atgaactgta gcataagcag tattcatttg   1920
gattattttt tttattagct ctcacccctt cattattctg agctgaaagt ctggcatgaa   1980
ctgtcctcaa ttttgtttc aaattcacat cgattatcta tgcattatcc tcttgtatct   2040
acctgtagaa gtttcttttt ggttattcct tgactgcttg attacagaaa gaaatttatg   2100
aagctgtaat cgggatagtt atactgcttg ttcttatgat tcatttcctt tgtgcagttc   2160
ttggtgtagc ttgccacttt caccagcaaa gttc                                2194
```

<210> 11
<211> 307
<212> PRT
<213> Oryza sativa

<400> 11

```
Met Arg Met Ser Cys Asn Gly Cys Arg Val Leu Arg Lys Gly Cys Ser
1               5                   10                  15
Asp Asn Cys Ala Ile Arg Pro Cys Leu Gln Trp Ile Arg Ser Pro Asp
            20                  25                  30

Ala Gln Ala Asn Ala Thr Val Phe Leu Ala Lys Phe Tyr Gly Arg Ala
        35                  40                  45
Gly Leu Ile Asn Leu Ile Thr Ala Gly Pro Glu His Val Arg Pro Ala
    50                  55                  60
Ile Phe Arg Ser Leu Leu Tyr Glu Ala Cys Gly Arg Met Leu Asn Pro
65                  70                  75                  80
Val Tyr Gly Ser Val Gly Leu Leu Trp Ser Gly Asn Trp Gln Leu Cys
                85                  90                  95
Gln Ser Ala Val Glu Ser Val Leu Arg Gly Met Pro Ile Ala Gln Pro
            100                 105                 110
Pro Pro Ser Ala Thr Ala Val Pro Pro Leu Pro Thr Cys Asp Ile Arg
        115                 120                 125
His Val Gly Ala Arg Arg Gly Asp Val His Gly Ala Ala Ala Gly Pro
    130                 135                 140
Val Ala Asp Leu His Arg Leu Asp Ile Ser Ser Arg Ala Lys Phe Lys
145                 150                 155                 160
Arg Pro Gly Gly Gly Ala Ala Ala Ala His Arg Ser Asp His Ala Ala
                165                 170                 175
Phe Glu Leu Val Phe Ser Lys Pro Ala Ala Ala Met Ala Val Asp Val
            180                 185                 190
Ile Arg Gln Ala Gln Pro Leu Asn Trp Ala Pro Gly Ala Leu Ser His
        195                 200                 205
Glu Ser Ala Ser His Asp Ala Ala Pro Pro Glu Ser Glu Gly His Ser
    210                 215                 220
Asn Asp Thr Ala Asp Thr Val Asp Gly Ser His Val Ser Gln Ser Glu
225                 230                 235                 240
Pro Glu Pro Arg Ala Thr Ser Ala Ala Thr Glu Val His Asp Ala Gly
                245                 250                 255
Leu Asp Leu Thr Leu Gly Leu Pro Pro Pro Pro Pro Pro Val Gln Lys
            260                 265                 270
Thr Glu Pro Ala Asp Ser Asp Gly Gly Ser Gln Gln Gln His Asp His
        275                 280                 285
Arg Lys Glu Lys Pro Val Glu Leu Gly Leu Ala Ile Ser Thr Lys Val
    290                 295                 300
Ala Ala Gln
305
```

<210> 12
<211> 330
<212> PRT
<213> Oryza sativa

<400> 12

```
Met Arg Met Ser Cys Asn Gly Cys Arg Val Leu Arg Lys Gly Cys Ser
1               5                   10                  15
Glu Gly Cys Thr Ile Arg Pro Cys Leu Gln Trp Ile Lys Thr Pro Glu
            20                  25                  30
Ala Gln Ala Asn Ala Thr Val Phe Leu Ala Lys Phe Tyr Gly Arg Ala
        35                  40                  45
Gly Leu Leu Asn Leu Leu Ala Ala Gly Pro Asp His Leu Arg Pro Ala
    50                  55                  60
Val Phe Arg Ser Leu Leu Tyr Glu Ala Cys Gly Arg Ile Val Asn Pro
65                  70                  75                  80
Ile Tyr Gly Ser Val Gly Leu Leu Trp Ser Gly Gln Trp Gln Ala Cys
                85                  90                  95
Gln Ala Ala Val Glu Ala Val Leu Lys Gly Asp Pro Val Val Gln Val
            100                 105                 110
Ser Ser Glu Ala Ala Ala Ala Ala Gln Ala Thr Pro Pro Leu Arg Ala
        115                 120                 125
Tyr Asp Ile Arg His Val Ser Lys Asp Ala Glu Ala Asp Ala Ala Ala
    130                 135                 140

Asn Leu Leu Arg Val Ala Arg Gly Gly Arg Thr Arg Phe Lys Arg Ala
145                 150                 155                 160
Ser Ser Ser Ser Asn Ser Lys His Gly Ala Lys Leu Ala Gly Ala Ala
                165                 170                 175
Ala Ala Lys Arg Ala Ala Ser Pro Ser Ser Ser Ser Pro Thr His Glu
            180                 185                 190
Thr Glu Pro Glu Ala Val Val Val Val Gly Asp His Asp Asp Asp His

        195                 200                 205
His His Pro Ala Leu Ser His Glu Val His Glu Glu Ser Ala Gly Ser
    210                 215                 220
His Asp His Asp Asp Asp Asp His Val Asp Asp Gly Asp Asn Asn Asp
225                 230                 235                 240
Met Ala Ile Ala Asp Val Thr Pro Pro Arg Ala Gly Ser Glu Asp Thr
                245                 250                 255
Glu Val Glu Thr Gly Ser His Val Ser Gln Ala Glu Gln Ser Pro Val
            260                 265                 270
Pro Val Glu His Glu Glu Gly Glu Glu Glu Glu Val Gly Leu Glu Leu
        275                 280                 285
Thr Leu Gly Phe Gln Pro Leu Val Val Arg Ala Ser Arg Arg Pro Ser
    290                 295                 300
Ser Ala Glu Ala Arg Cys Asp Leu Ser Gly Leu Ser Ala Glu Ser Ser
305                 310                 315                 320
Arg Ile Gly Leu Arg Leu Glu Leu Pro Ala
                325                 330
```

<210> 13
<211> 204
<212> PRT
<213> Oryza sativa

<400> 13

```
Met Ser Cys Asn Gly Cys Arg Val Leu Arg Lys Gly Cys Ser Asp Gly
1               5                   10                  15
Cys Val Leu Arg Pro Cys Leu Gln Trp Ile Asp Ala Ala Asp Ala Gln
            20                  25                  30
Gly His Ala Thr Val Phe Val Ala Lys Phe Phe Gly Arg Ala Gly Leu
        35                  40                  45
Leu Ser Phe Ile Ser Ala Val Pro Glu Ala Gln Arg Pro Ala Leu Phe
    50                  55                  60
Gln Ser Leu Leu Tyr Glu Ala Ala Gly Arg Thr Ile Asn Pro Val His
65                  70                  75                  80
Gly Ala Val Gly Leu Leu Trp Thr Gly Asn Trp Pro Leu Cys Gln Ala
                85                  90                  95
Ala Val Glu Thr Val Leu Arg Gly Gly Ala Ile Gly Pro Leu Pro Glu
            100                 105                 110
Leu Gly Gly Ala Cys Gly Gly Ala Gly Gly Asp Leu Tyr Gly Ala Ala
        115                 120                 125
Lys Arg Asn Gly Gly Trp Ser Thr Phe Ser Thr Ala Lys Arg Val Arg
    130                 135                 140
Lys Ala Glu Val Pro Glu Ala Pro Ser Cys Asp Leu Gly Leu Cys Leu
145                 150                 155                 160
Ser Pro Gly Ser Pro Pro Ala Val Gly Glu Arg Lys Pro Ala Leu Arg
                165                 170                 175
Pro Gly Thr Pro Ser Met Ser Ser Asp Glu Ser Gly Thr Thr Thr Gly
            180                 185                 190
Gly Glu Arg Asp Pro Val Leu Leu Asn Leu Phe Val
        195                 200
```

<210> 14
<211> 232
<212> PRT
<213> Oryza sativa

<400> 14

```
Met Ser Cys Asn Gly Cys Arg Val Leu Arg Lys Gly Cys Ser Asp Ala
1               5                   10                  15
Cys Val Leu Arg Pro Ser Ile Glu Trp Ile Asp Gly Ala Gln Pro Gln
            20                  25                  30
Ala Asn Ala Thr Val Phe Val Ala Lys Phe Phe Gly Arg Ala Gly Leu
        35                  40                  45
Val Ala Ser Leu Ala Ala Val Pro Leu His His Arg Pro Ala Leu Phe
    50                  55                  60
Arg Ser Leu Leu Tyr Glu Ala Cys Gly Arg Thr Ile Asn Pro Val Ser
65                  70                  75                  80
Gly Ala Ile Gly Leu Met Trp Thr Gly Asn Trp Asp Leu Cys Gln Ala
                85                  90                  95
Ala Ala Asp Ala Val Leu Arg Gly Asp Ser Leu Arg Ala Leu Ser Ala
            100                 105                 110
Val Pro Ala Ala Phe Thr Asp Arg Asp Met Ala Gly Leu Tyr Gly Asn
        115                 120                 125
Val Gly Ala Ala Ala Gly Thr Ser Ser Ser Ser Pro Asp Asn Asp Asn
    130                 135                 140
Ser Ser Ala Ser Ala Ala Pro Arg Arg Lys Arg Pro Arg Asn Asn Gly
145                 150                 155                 160
Ala Gly Ala Gly Val Gly Gln Gln Gln Leu Pro His Ala Val Ala Ala
                165                 170                 175

Val Leu Gln Ser Cys Glu Leu Asp Leu Cys Leu Thr Pro Val Ser Pro
            180                 185                 190
Pro Ala Val Gln Leu Val Gly Gly Gly Gly Gly Ala Ser Asp Glu His
        195                 200                 205
Ser Thr Thr Thr Cys Glu Glu Ala Ser Asp Gly Asp Gly Ala Gly Ala
    210                 215                 220
Pro Thr Leu Leu Asn Leu Phe Ser
225                 230
```

<210> 15
<211> 226
<212> PRT
<213> Oryza sativa

<400> 15

```
Met Ser Cys Asn Gly Cys Arg Val Leu Arg Lys Gly Cys Ser Glu Gly
1               5                   10                  15
Cys Val Leu Arg Pro Cys Leu Gln Trp Ile Asp Gly Ala Glu Ala Gln
            20                  25                  30
Gly His Ala Thr Val Phe Val Ala Lys Phe Phe Gly Arg Ala Gly Leu
        35                  40                  45
Met Ser Phe Leu Thr Ala Val Pro Glu Pro Gln Arg Ala Ala Val Phe
    50                  55                  60
Gln Ser Leu Leu Tyr Glu Ala Ala Gly Arg Thr Ile Asn Pro Val Gly
65                  70                  75                  80
Gly Ala Val Gly Leu Leu Ser Gly Gly Ser Trp His Leu Cys Gln Ala
                85                  90                  95
Ala Val Asp Thr Val Leu Arg Gly Gly Gly Ile Gln Pro Leu Pro Asp
            100                 105                 110
Gln Val Asp Ala Ala Ala Ala Gly Gly Arg Asp Val Phe Ala Ser Thr
        115                 120                 125
Ala Arg Arg Ala Met Gly Gly Cys Ser Thr Phe Ser Thr Ala Lys Arg
    130                 135                 140
Ser Thr Thr Thr Thr Thr Thr Lys Asn Pro Gly Thr Pro His Asp Ala
```

```
145                 150                 155                 160
Ala Ala Ala Ala Pro Gln Pro Glu Pro Ser Cys Asp Leu Gly Leu Trp
                165                 170                 175
Leu Ser Pro Gly Ser Pro Pro Ala Pro Gly Asp Arg Arg Ser Gly Gly
            180                 185                 190
Arg Arg Ala Asp Thr Pro Ser Met Asn Ser Glu Gly Ser Val Thr Thr
        195                 200                 205
Cys Gly Val Val Gly Asp Gly Glu Arg Glu Pro Glu Leu Leu Asn Leu
    210                 215                 220
Phe Val
225
```

<210> 16
<211> 224
<212> PRT
<213> Nicotiana benthamiana

<400> 16

```
Met Ser Cys Asn Gly Cys Arg Val Leu Arg Lys Gly Cys Ser Glu Asn
1               5                   10                  15
Cys Ile Leu Arg Pro Cys Leu Gln Trp Ile Glu Thr Ala Glu Ser Gln
            20                  25                  30
Gly His Ala Thr Val Phe Val Ala Lys Phe Phe Gly Arg Ala Gly Leu
        35                  40                  45
Thr Ser Phe Ile Ser Ala Val Pro Glu Asn Gln Arg Pro Ser Leu Phe
    50                  55                  60
Gln Ser Leu Leu Tyr Glu Ala Ala Gly Arg Thr Val Asn Pro Val Asn
65                  70                  75                  80
Gly Ala Val Gly Leu Leu Trp Thr Gly Asn Trp His Val Cys Gln Ala
                85                  90                  95
Ala Val Glu Thr Val Leu Arg Gly Gly Ala Leu Arg Pro Ile Pro Asp
            100                 105                 110
Phe Leu Gly Ala Ser Leu Asp Ile Asp Glu Ser Ser Glu Cys Thr Asp
        115                 120                 125
Ile Phe Lys Leu Gln His Pro Ser Gln Asn Asn Ile Gln Pro Thr Met
    130                 135                 140
Gln Lys Arg Arg Arg Phe Pro Glu Glu Ala Ser Asn Val Leu Gln Leu
145                 150                 155                 160
Ala Asp Leu Asp Leu Ser Leu Thr Pro Gly Phe Gln Ser Gln Lys Val
                165                 170                 175
Phe Asn His Ala Leu Pro Glu Asn Arg Arg Pro Ala Thr Pro Ser Met
            180                 185                 190
Asn Ser Glu Glu Ser Gly Thr Thr Thr Cys Phe Glu Ser Gly Thr Val
        195                 200                 205
Ile Gly Asn His Gln Gly Asn Glu Pro Lys Leu Leu Ser Leu Phe Asn
    210                 215                 220
```

<210> 17
<211> 230
<212> PRT
<213> Glycine max

<400> 17

```
Met Ser Cys Asn Gly Cys Arg Val Leu Arg Lys Gly Cys Ser Glu Ser
1               5                   10                  15
Cys Ile Leu Arg Pro Cys Leu Gln Trp Ile Glu Thr Ala Glu Ala Gln
            20                  25                  30
Gly His Ala Thr Val Phe Val Ala Lys Phe Phe Gly Arg Ala Gly Leu
        35                  40                  45
Met Ser Phe Ile Ser Asn Val Pro Glu Asn Gln Arg Pro Ala Leu Phe
    50                  55                  60

Gln Ser Leu Leu Phe Glu Ala Cys Gly Arg Thr Val Asn Pro Val Asn
65                  70                  75                  80
Gly Ala Val Gly Leu Leu Trp Thr Gly Asn Trp His Val Cys Gln Ala
                85                  90                  95
Ala Val Glu Thr Val Leu Arg Gly Gly Thr Leu Arg Pro Met Pro Glu
            100                 105                 110
Leu Met Gly Leu Asp Ala Ala Ala Ala Ala Ala Ala Ala Ala Asp Glu
        115                 120                 125
Thr Ser Glu Ala Glu Val Gly Cys Thr Asp Thr Trp Arg Ile Arg Asn
    130                 135                 140
Pro Asn Thr Asn Cys Arg Phe Thr Ser Ser Arg Ser Ser Ser Gly Gly
145                 150                 155                 160
Gly Gly Gly Lys Arg Lys Arg Ser Glu Glu Leu Ala Lys Leu Gln Glu
                165                 170                 175
Gln Gln Pro Asn Leu Asp Leu Arg Leu Thr Pro Val Phe Leu Gln Lys
            180                 185                 190
Glu Glu Ser Arg Arg Pro Glu Ser Pro Ser Met Thr Ser Glu Glu Ser
        195                 200                 205
Gly Thr Thr Thr Glu Asn Arg Phe Gly Asp Gln Trp Ser His Trp Lys
    210                 215                 220
Val Leu Asn Leu Phe Ile
225                 230
```

<210> 18
<211> 240
<212> PRT
<213> Glycine max

<400> 18

```
Met Ser Cys Asn Gly Cys Arg Val Leu Arg Lys Gly Cys Ser Glu Ser
1               5                   10                  15
Cys Ile Leu Arg Pro Cys Leu Gln Trp Ile Asp Thr Pro Glu Ala Gln
            20                  25                  30
Gly His Ala Thr Val Phe Val Ala Lys Phe Phe Gly Arg Ala Asp Leu
        35                  40                  45
Met Ser Phe Ile Ser Asn Val Pro Glu Pro Gln Arg Pro Ala Leu Phe
    50                  55                  60
Gln Ser Leu Leu Phe Glu Ala Cys Gly Arg Thr Val Asn Pro Val Asn
65                  70                  75                  80
Gly Ala Val Gly Leu Leu Trp Thr Gly Asn Trp His Val Cys Gln Ala
                85                  90                  95
Ala Val Glu Thr Val Leu Arg Gly Gly Thr Val Arg Pro Met Pro Glu
            100                 105                 110
Leu Phe Gly Leu Asp Ala Pro Thr Pro Thr Thr Asp Asp Ala Ser Glu
        115                 120                 125
Gly Glu Val Thr Cys Thr Asp Lys Trp Arg Val Arg Asp Pro Asn Pro
    130                 135                 140
Asn Phe Arg Phe Pro Gly Ser Arg Ser Asp Lys Gly Ser Ser Gly Gly
145                 150                 155                 160
Lys Arg Lys Arg Phe Glu Glu Leu Ala Lys Leu Gln Thr Ala Thr Thr
                165                 170                 175
Asp Leu Asn Leu Arg Leu Thr Pro Ser Phe Leu Gln Asn Ala Ala Asn
            180                 185                 190
Phe Gly Cys Arg Gln Glu Ile Arg Trp Pro Gly Ser Pro Ser Met Asn
        195                 200                 205
Ser Glu Glu Ser Gly Thr Thr Thr Ala Cys Leu Glu Ser Gly Ile Gly
    210                 215                 220
Asp His Tyr Ala Pro Asp Gly Asp Arg Lys Val Leu Asn Leu Phe Ile
225                 230                 235                 240
```

<210> 19
<211> 222
<212> PRT
<213> Hordeum vulgare

<400> 19

```
Met Ser Cys Asn Gly Cys Arg Val Leu Arg Lys Gly Cys Asn Asp Ala
1               5                   10                  15
Cys Met Leu Arg Pro Cys Leu Leu Trp Ile Glu Gly Ala Asp Ala Gln
            20                  25                  30
Gly His Ala Thr Ile Phe Ala Ala Lys Phe Phe Gly Arg Ala Gly Leu
        35                  40                  45
Met Ser Phe Leu Thr Ala Val Pro Glu Ser Gln Arg Pro Ala Val Phe
    50                  55                  60
Gln Ser Leu Leu Tyr Glu Ala Ala Gly Arg Thr Ile Asn Pro Val Gly
65                  70                  75                  80
Gly Ala Val Gly Leu Leu Trp Ala Gly Ser Trp His Leu Cys Glu Ala
                85                  90                  95
Ala Val Gln Thr Val Leu Arg Gly Gly Ala Ile Arg Pro Leu Pro Glu
            100                 105                 110
Leu Ala Gly Gly Val Pro Glu Gly Gly Val Gly Gly Ser Asp Leu Phe
        115                 120                 125
Ala Ser Ser Ser Arg Arg Ala Val Val Gly Cys Ser Thr Tyr Ser Met
    130                 135                 140
Ala Lys Arg Val Thr Pro Arg Lys Thr Trp Ala Pro Glu Ala Ala Ser
145                 150                 155                 160
His His Gln Glu Pro Ser Cys Asp Leu Gly Leu Phe Leu Thr Pro Gly
                165                 170                 175
Ser Ala Ala Ala Ala Glu Gly Glu Arg Arg Ala Arg Arg Ala Gly Thr
            180                 185                 190
Pro Ser Met Ser Ser Asp Gly Ser Val Thr Thr Thr Ala Gly Ala Gly
        195                 200                 205
Ala Asp Gly Asp Lys Glu Pro Glu Leu Leu Asn Leu Phe Val
    210                 215                 220
```

<210> 20
<211> 235
<212> PRT
<213> Sorghum bicolor

<400> 20

```
Met Ser Cys Asn Gly Cys Arg Val Leu Arg Lys Gly Cys Ser Glu Ala
1               5                   10                  15
Cys Val Leu Arg Pro Cys Leu Gln Trp Ile Asp Ala Ala Asp Ala Gln
            20                  25                  30
Gly His Ala Thr Val Phe Val Ala Lys Phe Phe Gly Arg Ala Gly Leu
        35                  40                  45
Leu Ser Phe Ile Ser Ala Val Pro Asp Ala Gln Arg Pro Ala Leu Phe
    50                  55                  60
Gln Ser Leu Leu Tyr Glu Ala Ala Gly Arg Thr Ile Asn Pro Val His
65                  70                  75                  80
Gly Ala Val Gly Leu Leu Gly Thr Gly Asn Trp His Leu Cys Gln Ala
                85                  90                  95
Ala Val Asp Thr Val Leu Arg Gly Gly Ala Ile Gly Pro Leu Pro Glu
            100                 105                 110
Leu Gly Ile Thr Gly Ser Ala Gly Asp Leu Tyr Gly Pro Pro Gly Ala
        115                 120                 125
Gly Thr Gly His Gly Gly Gly Gly Gly Lys Arg Ala Gly Gly Trp Ser
    130                 135                 140
Thr Phe Ser Thr Ala Lys Arg Val Arg Lys Ala Ala Ala Thr Gly Ala
145                 150                 155                 160
Pro Pro Pro Pro Pro Ala Ala Glu Pro Glu Ala Ser Cys Asp Leu Arg
```

```
                    165                     170                     175
Leu Cys Leu Leu Ser Pro Gly Ser Pro Pro Pro Pro Ala Gly Asp Arg
                180                     185                     190
Arg Ala Pro Phe Met Arg Pro Gly Thr Pro Ser Ile Thr Ser Asp Asp
        195                     200                     205
Asp Ser Val Thr Thr Thr Thr Thr Thr Thr Thr Thr Gly Gly Gly Gly
    210                     215                     220
Asp Arg Glu Pro Val Leu Leu Asn Leu Phe Ala
225                     230                     235
```

<210> 21
<211> 231
<212> PRT
<213> Aquilegia sp.

<400> 21

```
Met Ser Cys Asn Gly Cys Arg Val Leu Arg Lys Gly Cys Ser Glu Ser
1               5                   10                  15
Cys Ile Leu Arg Pro Cys Leu Gln Trp Ile Glu Ser Pro Glu Ala Gln
            20                  25                  30
Gly His Ala Thr Val Phe Ile Ala Lys Phe Phe Gly Arg Ala Gly Leu
        35                  40                  45
Met Ser Phe Ile Ser Ser Val Pro Glu Ser Gln Arg Pro Ala Leu Phe
    50                  55                  60
Gln Ser Leu Leu Phe Glu Ala Cys Gly Arg Thr Val Asn Pro Val Asn
65                  70                  75                  80
Gly Ala Val Gly Leu Leu Trp Thr Gly Asn Trp His Ile Cys Gln Ala
                85                  90                  95
Ala Val Glu Thr Val Leu Arg Gly Gly Thr Leu Arg Pro Ile Asn Glu
            100                 105                 110
Val Leu Gly Ala Ser Leu Met Met Met Pro Glu Ser Ala Asp Glu Met
        115                 120                 125
Ser Glu Val Ile Thr Thr Thr Gln Asp Pro Thr Tyr Ser Arg Pro Lys
    130                 135                 140
Val Met His Ile Lys Lys Lys Glu Ile Ile His Glu Asn Val Asn Lys
145                 150                 155                 160
Ile Met Ile His Asp Leu Asp Leu Ser Leu Thr Ser Gly Ser Ser Ser
                165                 170                 175
Pro Arg Lys Thr Thr Lys Arg Ser Thr Thr Pro Ser Thr Glu Glu Ser
            180                 185                 190
Val Thr Thr Ser Leu Asp Ser Gly Ala Gly Val Trp Arg Asp Asp His
        195                 200                 205
Tyr Leu Arg Arg Gly Ser Gly Glu Asp Asn Asn Asn Asn Gly Asp Gln
    210                 215                 220
Lys Leu Leu Asn Leu Phe Val
225                 230
```

<210> 22
<211> 228
<212> PRT
<213> Vitis vinifera

<400> 22

```
Met Ser Cys Asn Gly Cys Arg Val Leu Arg Lys Gly Cys Ser Glu Ser
1               5                   10                  15
Cys Ile Leu Arg Pro Cys Leu Gln Trp Ile Glu Ser Ala Glu Ser Gln
            20                  25                  30
Gly His Ala Thr Val Phe Val Ala Lys Phe Phe Gly Arg Ala Gly Leu
        35                  40                  45
Met Ser Phe Ile Ser Ala Val Pro Glu Asn Gln Arg Pro Ala Leu Phe
    50                  55                  60

Arg Ser Leu Leu Tyr Glu Ala Cys Gly Arg Thr Val Asn Pro Val Asn
65                  70                  75                  80
Gly Ala Val Gly Leu Leu Gly Thr Gly Asn Trp His Val Cys Gln Ala
                85                  90                  95
Ala Met Glu Thr Val Leu Arg Gly Gly Thr Leu Arg Ala Met Pro Glu
            100                 105                 110
Leu Leu Asn Gly Val Ser Thr Pro Glu Cys Asp Glu Ala Ser Glu Ala
        115                 120                 125
Glu Val Thr Cys Thr Asp Met Phe Lys Leu Arg Asp Pro Asn Ser Ser
    130                 135                 140
Ser Arg Ser Lys Val Pro Lys Arg Lys Arg Leu Glu Glu Ala Ser Lys
145                 150                 155                 160
Val Gln Cys Thr Asp Leu Asp Leu Arg Leu Thr Pro Gly Leu Thr Gly
                165                 170                 175
Lys Ile Ser Ser Arg Gly Leu Gly Ala Met Ser Tyr Leu Pro Glu Thr
            180                 185                 190
Arg Arg Pro Gly Thr Pro Ser Met Asn Ser Glu Glu Ser Val Thr Thr
        195                 200                 205
Thr Cys Phe Glu Thr Pro His Ser Pro Glu Gly Glu Arg Lys Leu Leu
    210                 215                 220
Asn Leu Phe Leu
225
```

<210> 23
<211> 237
<212> PRT
<213> Lotus japonicus

<400> 23

```
Met Ser Cys Asn Gly Cys Arg Val Leu Arg Lys Gly Cys Ser Glu Ser
1               5                   10                  15
Cys Ile Leu Arg Pro Cys Leu Gln Trp Ile Asp Thr Ala Glu Ala Gln
            20                  25                  30
Gly His Ala Thr Val Phe Val Ala Lys Phe Phe Gly Arg Ala Asp Leu
        35                  40                  45
Met Ser Phe Ile Ser Asn Val Pro Gln Pro Gln Arg Pro Ser Leu Phe
    50                  55                  60
Gln Ser Leu Leu Phe Glu Ala Cys Gly Arg Thr Val Asn Pro Val Asn
65                  70                  75                  80
Gly Ala Val Gly Leu Leu Trp Thr Gly Asn Trp His Val Cys Gln Ala
                85                  90                  95
Ala Val Glu Thr Val Leu Arg Gly Gly Thr Leu Lys Pro Leu Pro Glu
            100                 105                 110
Leu Leu Gly Leu Asp Ala Pro Ser His Val Pro Thr Ala Ala Asp Asp
        115                 120                 125
Ala Ser Glu Gly Glu Val Thr Tyr Asp Thr Trp Arg Ile Arg Asp Pro
    130                 135                 140
Asn Pro Asn Ala Arg Leu Thr Asn Ser Arg Ser Ser Ala Gly Lys Arg
145                 150                 155                 160
Gln Arg Pro Glu Glu Leu Ala Lys Leu Gln Thr Thr Thr Asp Leu Asn
                165                 170                 175
Leu Arg Leu Thr Pro Gly Phe Leu Gln Asn Val Ser Ser Tyr Gly Cys
            180                 185                 190
Lys Arg Glu Ala Arg Arg Pro Gly Ser Pro Ser Met Asn Ser Glu Glu
        195                 200                 205
Ser Val Thr Thr Thr Ala Cys Leu Gly Ser Gly Ile Gly Asp His Cys
    210                 215                 220
Ser Arg Asp Glu Asp Arg Lys Val Leu Lys Leu Phe Val
225                 230                 235
```

<210> 24

<211> 232
<212> PRT
<213> Gossypium hirsutum

<400> 24

```
Met Ser Cys Asn Gly Cys Arg Val Leu Arg Lys Gly Cys Ser Glu Ser
1               5                   10                  15
Cys Ile Leu Arg Pro Cys Leu Gln Trp Ile Glu Ser Pro Glu Ala Gln
            20                  25                  30
Gly His Ala Thr Val Phe Val Ala Lys Phe Phe Gly Arg Ala Gly Leu
        35                  40                  45
Met Ser Phe Ile Ser Ala Val Pro Glu Ser Gln Arg Pro Ala Leu Phe
    50                  55                  60
Gln Ser Leu Leu Phe Glu Ala Cys Gly Arg Thr Val Asn Pro Val Asn
65                  70                  75                  80
Gly Ala Val Gly Leu Leu Trp Asn Gly Asn Trp His Val Cys Gln Ala
                85                  90                  95
Ala Val Glu Thr Val Leu Arg Gly Gly Ser Leu Arg Ser Met Pro Glu
            100                 105                 110
Leu Met Ala Pro Thr Pro Ala Ser Asp Glu Ala Ser Glu Ala Thr Cys
        115                 120                 125
Thr Asp Met Trp Lys Leu Arg Glu Thr Ser Thr Asn Leu Asn Ser Asn
    130                 135                 140
Cys Arg Phe Ser Asn Ser Arg Ser Arg Val Ser Pro Lys Arg Lys Arg
145                 150                 155                 160
Val Glu Glu Glu Phe Lys Lys Leu Gln Pro Ser Asp Leu Asp Leu Gly
                165                 170                 175
Leu Thr Pro Ser Phe Thr Gly Lys Arg Val Thr Asp Asn Arg Arg Pro
            180                 185                 190
Gly Thr Pro Ser Met Asn Ser Glu Glu Ser Val Thr Thr Thr Cys Phe
        195                 200                 205
Glu Ser Val Phe Ala Asp Gln Gln Gly Gln Gly Ser Arg Gly Thr Asp
    210                 215                 220
Lys Lys Leu Leu Lys Leu Phe Val
225                 230
```

<210> 25
<211> 287
<212> PRT
<213> Triticum aestivum

<400> 25

```
Met Arg Leu Ser Cys Asn Gly Cys Arg Val Leu Arg Lys Gly Cys Ser
1               5                   10                  15
Glu Asp Cys Ser Ile Arg Pro Cys Leu Gln Trp Ile Lys Ser Pro Glu
            20                  25                  30
Ala Gln Ala Asn Ala Thr Val Phe Leu Ala Lys Phe Tyr Gly Arg Ala
        35                  40                  45
Gly Leu Met Asn Leu Ile Asn Ala Gly Thr Asp Asp Ser Leu Arg Pro
    50                  55                  60
Gly Ile Phe Arg Ser Leu Leu Tyr Glu Ala Cys Gly Arg Ile Val Asn
65                  70                  75                  80
Pro Ile Tyr Gly Ser Val Gly Leu Leu Trp Ser Asn Asn Trp Gln Met
                85                  90                  95
Cys Gln Ala Ala Val Glu Ala Val Leu Ser Gly Lys Pro Ile Val Gln
            100                 105                 110
Val Ser Ser Glu Asp Ala Ala Ala Asp Arg Thr Pro Pro Leu Lys Ala
        115                 120                 125
Tyr Asp Ile Arg His Val Ser Thr Ser Pro Ala Ala Asp Gly Arg Leu
    130                 135                 140
His Lys Val Ala Lys Pro Gly Arg Thr Arg Phe Lys Arg Ala Ser Ser
```

145 150 155 160
Ala Ser Ser His His Asn Pro Ser Ser Asp Ser Asn Asn Lys Pro Lys
165 170 175
Pro Lys Pro Gln Pro Gln Thr Arg Ala Pro Thr Ala Glu Glu Glu Arg
180 185 190
Asp Arg Gln His Arg Lys Glu Met Glu Glu Gly Ala Phe Gln Arg Ala
195 200 205
Pro Ser His Glu Ser Ser Asp Ser Arg His Glu Asp Pro Val Glu Pro
210 215 220
His Cys Gln Gln Glu Ala Ser Ala Asp Thr Glu Ala Glu Ala Gly Ser
225 230 235 240
His Val Ser Gln Ala Glu Gln Glu Gln Ser Thr Glu Pro Ala Ala Glu
245 250 255
His Ala Glu Glu Val Glu Lys Glu Asp Glu Glu Leu Gly Leu Glu Leu
260 265 270
Thr Leu Gly Phe Ala Pro Val Ala Ala Arg Pro Ala Glu Phe Thr
275 280 285

<210> 26
<211> 274
<212> PRT
<213> Solanum tuberosum

<400> 26

Met Arg Met Ser Cys Asn Gly Cys Arg Val Leu Arg Lys Gly Cys Ser
1 5 10 15
Lys Ser Cys Ser Ile Arg Pro Cys Leu Gln Trp Ile Lys Thr Pro Asp
20 25 30
Ser Gln Ser Asn Ala Thr Val Phe Leu Ala Lys Phe Tyr Gly Arg Ala
35 40 45
Gly Leu Met Asn Leu Ile Asn Ala Gly Pro Asp His Leu Arg Pro Ala
50 55 60
Ile Phe Arg Ser Leu Leu Tyr Glu Ala Cys Gly Arg Ile Val Asn Pro
65 70 75 80
Ile Tyr Gly Ser Val Gly Leu Leu Trp Ser Gly Asn Trp Gln Leu Cys
85 90 95
Gln Asn Ala Val Glu Ala Val Leu Lys Gly Thr Pro Ile Thr Pro Ile
100 105 110
Ala Ser Glu Ile Ala Val Asn Asn Asn Gly Pro Pro Leu Lys Leu Pro
115 120 125
Tyr Asp Ile Arg His Ile Asn Lys Asp Glu Asn Ser Thr Lys Ser Ser
130 135 140
Glu Leu His Arg Val Arg Thr Arg Cys Arg Phe Lys Arg Ser Gly Ala
145 150 155 160
Asn Thr Lys Ala Thr Lys Ser Asn Pro Ala Cys Ser Gly Ser Gly Asp
165 170 175
Glu Phe Ala His Glu Lys Val Asn Gly Ser Thr Ser His Glu Ser Ser
180 185 190
Leu Ser His Gln Ser Glu Glu Glu Ala Ala Ala Ala Ala Ala Val Ala
195 200 205
Leu Asn Val Glu Cys Glu Ser Pro Glu Met Ala Glu Val Glu Asp Ser
210 215 220
Ala Lys Ala Asp Gly Glu Val Glu Leu Glu Leu Thr Leu Gly Phe Ser
225 230 235 240
Ser Leu Gly Thr Val Glu Gly Lys Pro Lys Glu Thr Lys Arg Asn Lys
245 250 255
Gly Val Gln Leu Val Asp Gly Ala Gly Glu Cys Lys Ile Glu Leu Gly
260 265 270
Leu His

<210> 27

<211> 301
<212> PRT
<213> Medicago truncatula

<400> 27

```
Met Arg Met Ser Cys Asn Gly Cys Arg Val Leu Arg Lys Gly Cys Ser
1               5                   10                  15
Glu Asn Cys Ser Ile Arg Pro Cys Leu Gln Trp Ile Lys Ser Pro Glu
            20                  25                  30
Ser Gln Ala Asn Ala Thr Val Phe Leu Ala Lys Phe Tyr Gly Arg Ala
        35                  40                  45
Gly Leu Met Asn Leu Val Asn Ala Gly Pro Glu His Leu Arg Pro Ala
    50                  55                  60
Ile Phe Arg Ser Leu Leu Tyr Glu Ala Cys Gly Arg Ile Val Asn Pro
65                  70                  75                  80
Ile Tyr Gly Ser Val Gly Leu Leu Trp Ser Gly Ser Trp Gln Leu Cys
                85                  90                  95
Gln Ala Ala Val Glu Ala Val Leu Lys Gly Ala Pro Ile Thr Pro Ile
            100                 105                 110
Thr Ser Glu Ala Ala Ala Asn Gly Arg Gly Pro Pro Leu Lys Ala Tyr
        115                 120                 125
Asp Ile Arg His Val Ser Lys Glu Glu Asn Ser Ala Ala Ser Thr Glu
    130                 135                 140
Leu Thr Gln Gln Arg Val Lys Pro Arg Ser Arg Lys Arg Ser Ser Leu
145                 150                 155                 160
Ala Lys Leu Lys Ile Gln Glu Glu Glu Lys Ser Ser Asn Asp Asn Lys
                165                 170                 175
Gly Val Glu Ser Gly Ser Thr Glu Pro Thr Gly Ser Val Glu Pro Val
            180                 185                 190
Glu Glu Val Met Asn Arg Ser Val Ser His Glu Ser Ser Ile Ser His
        195                 200                 205
Gln Ser Glu Ala Val Ala Val Val Asp Gly Glu Ser Lys Asp Ser Glu
    210                 215                 220
Ser Glu Thr Ser Met Ile Leu Phe Arg Asp Glu Pro Glu Leu Asp Arg
225                 230                 235                 240
Lys Met Lys Pro Ala Asp Arg Thr Gly Glu Asn Gly Glu Glu Lys Val
                245                 250                 255
Gly Leu Glu Leu Thr Leu Gly Leu Glu Pro Val Ser Arg Val Tyr His
            260                 265                 270
Val Val Pro Val Lys Lys Arg Arg Val Val Leu Lys Asp Cys Gly Val
        275                 280                 285
Gly Ser Trp Asn Val Glu Leu Gly Leu Gln Tyr Pro Ala
    290                 295                 300
```

<210> 28
<211> 302
<212> PRT
<213> Hordeum vulgare

<400> 28

```
Met Arg Leu Ser Cys Asn Gly Cys Arg Val Leu Arg Lys Gly Cys Ser
1               5                   10                  15
Glu Asp Cys Ser Ile Arg Pro Cys Leu Gln Trp Ile Lys Ser Pro Glu
            20                  25                  30
Ala Gln Ala Asn Ala Thr Val Phe Leu Ala Lys Phe Tyr Gly Arg Ala
        35                  40                  45
Gly Leu Met Asn Leu Ile Asn Ala Gly Thr Asp Asp Ser Leu Arg Pro
    50                  55                  60
Gly Ile Phe Arg Ser Leu Leu Tyr Glu Ala Cys Gly Arg Ile Val Asn
65                  70                  75                  80

Pro Ile Tyr Gly Ser Val Gly Leu Leu Trp Ser Asn Asn Trp Gln Met
                85                  90                  95
Cys Gln Ala Ala Val Glu Ala Val Leu Ser Gly Lys Pro Ile Val Gln
            100                 105                 110
Val Ser Ser Glu Asp Ala Ala Ala Asp Arg Thr Pro Pro Leu Lys Ala
        115                 120                 125
Tyr Asp Ile Arg His Val Ser Thr Ser Pro Ala Ala Asp Gly Arg Leu
    130                 135                 140
His Lys Val Ala Lys Pro Gly Arg Thr Arg Phe Lys Arg Ala Ser Ser
145                 150                 155                 160
Ala Ser Ser His His Asn Pro Ser Ser Asp Ser Asn Asn Lys Pro Lys
                165                 170                 175
Pro Gln Pro Arg Pro Pro Thr Ala Glu Glu Glu Leu Asp Arg Gln His
            180                 185                 190
Arg Lys Glu Met Glu Glu Gly Ala Phe Gln Arg Ala Pro Ser His Glu
        195                 200                 205
Ser Ser Asp Ser Arg His Glu Asp Pro Val Glu Pro His Ser Gln Gln
    210                 215                 220
Glu Ala Ser Ala Asp Thr Glu Ala Glu Ala Gly Ser His Val Ser Gln
225                 230                 235                 240
Ala Glu Gln Glu Gln Glu Gln Ser Thr Glu Pro Ala Ala Asp His Ala
                245                 250                 255
Glu Glu Val Glu Lys Asp Asp Glu Glu Leu Gly Leu Glu Leu Thr Leu
            260                 265                 270
Gly Phe Ala Pro Val Ala Ala Arg Pro Ala Gly Cys His Leu Ser Val
        275                 280                 285
Arg Thr Ala Ala Glu Pro Ala Phe Val Gly Leu Arg Phe Leu
    290                 295                 300
```

<210> 29
<211> 253
<212> PRT
<213> Vitis vinifera

<400> 29

```
Met Arg Met Ser Cys Asn Gly Cys Arg Val Leu Arg Lys Gly Cys Ser
1               5                   10                  15
Asp Asp Cys Ser Leu Arg Pro Cys Leu Glu Trp Ile Lys Thr Pro Glu
            20                  25                  30
Ser Gln Ala Asn Ala Thr Val Phe Leu Ala Lys Phe Tyr Gly Arg Ala
        35                  40                  45
Gly Leu Met Asn Leu Leu Asn Ala Gly Pro Glu His Leu Arg Pro Ala
    50                  55                  60
Ile Phe Arg Ser Leu Leu Tyr Glu Ala Cys Gly Arg Ile Val Asn Pro
65                  70                  75                  80
Ile Tyr Gly Ser Val Gly Leu Leu Trp Ser Gly Asn Trp Asn His Cys
                85                  90                  95
Gln Asn Ala Val Asp Ala Val Leu Gly Gly Ser Gln Ile Met Glu Val
            100                 105                 110
Pro Cys Asp Thr Ala Ala Ile Arg Pro Ile Leu Pro Leu Lys Ala Tyr
        115                 120                 125
Asp Ile Arg His Leu Ser Lys Asp Ala Asn Ser Pro His Asp Leu His
    130                 135                 140
Lys Ala Lys Thr Arg Ser Arg Phe Lys Arg Cys Gly Ala Arg Ser Lys
145                 150                 155                 160
Pro His Leu Asp Ser Ala Glu Phe Cys Asn Pro Gly Trp Asn Gln Phe
                165                 170                 175
Ser Pro Val Asp Tyr Asn Arg Ala Pro Ser His Asp Ser Ser Leu Ile
            180                 185                 190
Gln Gln Glu Thr Asp Ser Met Phe Ser Val Glu Thr Val Glu Ala Ser
        195                 200                 205

Leu Ala Thr Pro Pro Lys Pro Asn Gln Leu Leu Lys Ser Asp Gly Gln
    210                 215                 220
Thr Asp Lys Thr Glu Ala Gly Leu Glu Leu Thr Leu Gly Phe Tyr Pro
225                 230                 235                 240
Ile Ser Arg Pro His Ala Cys Lys Leu Glu Leu Gly Pro
                245                 250
```

<210> 30
<211> 234
<212> PRT
<213> Solanum tuberosum

<400> 30

```
Met Arg Met Ser Cys Asn Gly Cys Arg Val Leu Arg Lys Gly Cys Ser
1               5                   10                  15

Glu Asn Cys Ser Leu Arg Pro Ser Leu Gln Trp Ile Lys Ser Pro Asp
            20                  25                  30
Ser Gln Ala Asn Ala Thr Val Phe Leu Ala Lys Phe Tyr Gly Arg Ala
        35                  40                  45
Gly Leu Ile Asn Leu Ile Asn Ala Gly Pro Asp His Leu Arg Pro Ala
    50                  55                  60
Ile Phe Arg Ser Leu Leu Tyr Glu Ala Cys Gly Arg Ile Ile Asn Pro
65                  70                  75                  80
Val Asn Gly Ser Val Gly Leu Met Cys Ser Gly Asn Trp Gln Arg Cys
                85                  90                  95
Gln Glu Ala Val Glu Ser Val Leu Lys Gly Ser Thr Ile Met Gln Val
            100                 105                 110
Pro Ile Asn Asn Asp Asp Asp Asn Asn Asn Asn Asn Asn Asn Asn Thr
        115                 120                 125
Asp Gln Ile Val Pro Leu Lys Gly Cys Asp Ile Arg His Val Ser Lys
    130                 135                 140
Asn Asn Ser Thr Asn Ser Asp Asn Gln Gln Val Ile Lys Thr Arg Lys
145                 150                 155                 160
Gly Arg Leu Lys Arg Lys Gly Thr Phe Asp Ser Val Ala Ser Ala Ser
                165                 170                 175
Ala Glu Ala Glu Ala Ala Glu Glu Tyr Leu Leu Lys Asn Gln Pro Pro
            180                 185                 190
Leu Lys Phe Ser Ile Ala Gly Trp Asp Gln Phe Glu Glu Ile Asp Asp
        195                 200                 205
Glu Leu Ile Lys Arg Ala Ala Ser His Asp Ser Phe Ser Val Glu Thr
    210                 215                 220
Val Glu Leu Thr Leu Leu Gly Glu Pro Gly
225                 230
```

<210> 31
<211> 334
<212> PRT
<213> Pinus taeda

<400> 31

```
Met Arg Val Ser Cys Asn Gly Cys Arg Val Leu Arg Lys Gly Cys Ser
1               5                   10                  15
Asp Ser Cys Glu Leu Arg Pro Cys Leu Gln Trp Ile Lys Thr Ser Glu
            20                  25                  30
Ser Gln Ala Gln Ala Thr Val Phe Leu Ala Lys Phe Tyr Gly Arg Ser
        35                  40                  45
Gly Leu Met Asn Leu Ile Lys Ser Gly Pro Glu Phe Leu Arg Pro Thr
    50                  55                  60
Leu Phe Arg Ser Leu Leu Tyr Glu Ala Cys Gly Arg Ile Val Asn Pro
65                  70                  75                  80
```

```
Ile Phe Gly Ser Val Gly Leu Ile Trp Ser Gly Asn Trp His Ile Cys
                85                  90                  95
Gln Ala Ala Val Glu Ser Val Leu Lys Gly Tyr Pro Leu Gln Pro Leu
            100                 105                 110
His Pro Leu Asn Ser Leu Glu Ile Ala Ser Pro Pro Pro Ala Ala Ala
        115                 120                 125
Ala Pro Gln Ser Met Lys Ala Val Ala Asp Lys Leu Arg Asn Val Arg
    130                 135                 140
Ser Gln Gly Arg Phe Lys Arg Pro Leu Tyr Arg Lys Pro Ser Arg Asp
145                 150                 155                 160
Val Lys Asn Leu Asn Leu His Ala Leu Lys Asp Glu Glu Ala Ala Gly
                165                 170                 175
Ile Leu Thr Gly Ser Gln Ala Tyr Asn Leu Arg Trp Arg Arg Cys Tyr
            180                 185                 190
Pro Lys Glu Phe Gln Leu Ile Asn Ala Gln Pro Val Asn Asn Phe Leu
        195                 200                 205
Ser Ile Asp Ser Ser Ser Asp Ser Asp Asp Leu Ile Arg Leu Lys Thr
    210                 215                 220
Gly Phe Glu Ser Val Lys Ser Asp Cys Asn Pro Ile Glu Asn Arg Glu
225                 230                 235                 240
Thr Arg Ala Leu Ser Glu Gly Ile Ser Glu Gly Pro Ala Leu Ser Glu
                245                 250                 255
Gly Ile Glu Asn Gln Asn Glu Cys Pro Leu Gln Asn Leu Phe Gln Gln
            260                 265                 270
Asn Asp Asn Asn Trp Val Glu Asn Asn Ile Met Arg Thr Glu Leu Thr
        275                 280                 285
Asp Glu Arg Asn Leu Gly Leu Glu Leu Thr Leu Gly Ser Ser Met Gly
    290                 295                 300
Glu Thr Thr Gln Ser His Arg Lys Gln Leu Gly Ser Asn Cys Met Asp
305                 310                 315                 320
Arg Pro Pro Leu Tyr Arg Ser Leu Asp Leu Ser Leu Ser Gln
                325                 330
```

<210> 32
<211> 336
<212> PRT
<213> Pinus taeda

<400> 32

```
Met Arg Met Ser Cys Asn Gly Cys Arg Val Leu Arg Lys Gly Cys Ser
1               5                   10                  15
Glu Thr Cys Ile Ile Arg Pro Cys Leu Gln Trp Ile Lys Asn Glu Glu
            20                  25                  30
Ser Gln Ala Asn Ala Thr Val Phe Leu Ala Lys Phe Tyr Gly Arg Ala
        35                  40                  45
Gly Leu Met Asn Leu Ile Ser Ala Gly Pro Glu His Leu Arg Pro Ala
    50                  55                  60
Ile Phe Lys Ser Leu Leu Tyr Glu Ala Cys Gly Arg Met Val Asn Pro
65                  70                  75                  80
Ile Phe Gly Ser Val Gly Leu Leu Cys Ser Gly Asn Trp Gln Leu Cys
                85                  90                  95
Gln Ala Ala Val Glu Ser Ile Leu Lys Gly Cys Pro Ile His Pro Ile
            100                 105                 110
Leu Val Asp Ser Ser Gly Thr Gln Met Pro Thr Glu Arg Ser Asn Thr
        115                 120                 125
Asn Pro Ser Val Arg Ser Thr Leu Met Glu Thr Ile Thr Asn Glu Leu
    130                 135                 140
His Lys Val Lys Thr Lys Gly Arg Phe Lys Arg Arg Lys Ser Thr Val
145                 150                 155                 160
Lys Asp Val Ser Leu Asn Asn Ser Asp Ala Glu Asp Leu Gly Ala Ser
                165                 170                 175

Gln Val Phe Asn Leu Arg Trp Arg Arg Cys Tyr Pro Gly Glu Ser Gln
            180                 185                 190
Thr Pro Ala Leu Glu Gly Ile Pro Asn His Leu Val Ser Ser Asp Ser
        195                 200                 205
Ser Cys Glu Ser Leu Glu His Phe Asp Asp Leu Arg Asn His Lys Glu
    210                 215                 220
Ala Asp Met Glu Gly His Met Ser Lys Ala Met Asp Met Gly Pro Val
225                 230                 235                 240
Asn Ile Lys Cys Cys Ser Asp Gln Asn Asp His Lys Ala Thr Met Glu
                245                 250                 255
Ser Val Pro Glu Val Ser Tyr Thr Gln Ala Pro Asp Leu Glu Asp Met
            260                 265                 270
Gln Ala Ile Lys His Glu Tyr Glu Ser Ile Ser Ser Asp Val Thr Asp
        275                 280                 285
Val Lys Leu Glu Leu Thr Leu Ser Ser Gln Gly Ser Ala Thr His Ala
    290                 295                 300
Ala Glu Lys His His Thr Asn Leu His Trp Asn Ser Cys Met Glu His
305                 310                 315                 320
Ser Asp Glu Pro Thr Ile Thr Tyr Leu Gly Leu Gly Leu Thr Gln Ala
                325                 330                 335
```

<210> 33
<211> 184
<212> PRT
<213> Glycine max

<400> 33

Met Arg Met Ser Cys Asn Gly Cys Arg Val Leu Arg Lys Gly Cys Ser
1               5                   10                  15
Glu Asp Cys Ser Ile Arg Pro Cys Leu Gln Trp Ile Lys Asn Pro Glu
            20                  25                  30
Ser Gln Ala Asn Ala Thr Val Phe Leu Ala Lys Phe Tyr Gly Arg Ala
        35                  40                  45
Gly Leu Met Asn Leu Ile Asn Ala Gly Pro Glu Asn Leu Arg Pro Ala
    50                  55                  60
Ile Phe Arg Ser Leu Leu Tyr Glu Ala Cys Gly Arg Ile Val Asn Pro
65                  70                  75                  80
Ile Tyr Gly Ser Val Gly Leu Leu Trp Ser Gly Ser Trp Gln Leu Cys
                85                  90                  95
Gln Ala Ala Val Glu Ala Val Leu Lys Gly Glu Pro Ile Thr Pro Ile
            100                 105                 110
Thr Ser Glu Ala Ala Ala Asn Gly Arg Ala Pro Pro Leu Lys Ala Tyr
        115                 120                 125
Asp Ile Arg His Val Ser Lys Asp Gln Asn Ser Ala Asn Glu Thr Pro
    130                 135                 140
Lys Thr Lys Thr Arg Ser Arg Phe Lys Arg Thr Ser Gly Thr Leu Ile
145                 150                 155                 160
Lys Pro Lys Ala Ser Lys Gly Thr Gly Phe Val Pro Val Glu Pro Glu
                165                 170                 175
Met Ala Asn Pro Asp Cys Glu Ser
            180

<210> 34
<211> 230
<212> PRT
<213> Triticum aestivum

<400> 34

Met Ser Cys Asn Gly Cys Arg Val Leu Arg Lys Gly Cys Ser Asp Ala
1               5                   10                  15
Cys Val Leu Arg Pro Ser Ile Glu Trp Ile Asp Gly Ala Gln Pro Gln

```
                20                  25                  30
Ala Asn Ala Thr Val Phe Val Ala Lys Phe Phe Gly Arg Ala Gly Leu
            35                  40                  45
Val Ala Ser Leu Ala Ala Val Pro Leu His His Arg Pro Ala Leu Phe
    50                  55                  60
Arg Ser Leu Leu Tyr Glu Ala Cys Gly Leu Thr Ile Asn Pro Val Ser

65                  70                  75                  80
Gly Ala Ile Gly Leu Met Trp Thr Ser Asn Trp Asp Leu Cys Gln Ala
                85                  90                  95
Ala Ala Glu Ala Val Leu Arg Gly Asp Ser Leu Arg Ser Leu Ser Ala
            100                 105                 110
Val Pro Ala Ala Phe Thr Glu Arg Asp Met Ala Gly Leu Tyr Gly Asn
        115                 120                 125
Val Gly Thr Asn Thr Gly Ser Ser Ser Ser Leu His Ser Ser Pro Glu
    130                 135                 140
Asn Ser Thr Ser Ala Pro Ala Arg Lys Arg Ser Lys Asn Asn Cys Gly
145                 150                 155                 160
Ala Ala Val Gly Gln Gln Val Lys Leu Pro Gly Pro Gly Pro Val Leu
                165                 170                 175
Gln Ser Cys Glu Leu Asp Leu Cys Leu Thr Pro Leu Ser Ser Pro Leu
            180                 185                 190
Ala Gly Gly Arg Arg Gly Gly Ala Ser Asp Glu Tyr Ser Thr Thr Thr
        195                 200                 205
Cys Cys Glu Glu Ala Ser Gly Asp Ala Ala Glu Ala Gly Ala Pro Pro
    210                 215                 220
Leu Leu Asn Leu Phe Asn

225                 230
```

<210> 35
<211> 290
<212> PRT
<213> Aquilegia sp.

<400> 35

```
Met Arg Met Ser Cys Asn Gly Cys Arg Val Leu Arg Lys Gly Cys Ser
1               5                   10                  15
Glu Asn Cys Ser Ile Arg Pro Cys Leu Gln Trp Ile Lys Thr Pro Glu
            20                  25                  30
Ser Gln Ala Asn Ala Thr Val Phe Leu Ala Lys Phe Tyr Gly Arg Ala
        35                  40                  45
Gly Leu Met Asn Leu Ile Asn Ser Gly Pro Glu His Leu Arg Pro Ala
    50                  55                  60
Ile Phe Arg Ser Leu Leu Tyr Glu Ala Cys Gly Arg Ile Val Asn Pro
65                  70                  75                  80
Ile Tyr Gly Ser Val Gly Leu Leu Trp Ser Gly Ser Trp Gln Leu Cys
                85                  90                  95
Gln Ala Ala Val Glu Ala Val Leu Lys Gly Thr Pro Ile Met Gln Ile
            100                 105                 110
Ser Ser Glu Val Ala Ala Ile Ser Pro Ser Pro Pro Leu Lys Ala Tyr
        115                 120                 125
Asp Ile Arg His Val Ser Lys Asp Glu Asn Ser Ser Ala Ala Ser Gln
    130                 135                 140
Glu Leu His Lys Val Lys Thr Arg Ser Arg Phe Lys Arg Ser Gly Ala
145                 150                 155                 160
Gly Lys Ala Ala Asn Asn Lys Gln Val Asn Glu Ser Asn Lys Val His
                165                 170                 175
Thr Tyr Leu Asp Arg Ala Pro Ser His Glu Ser Ser Val Ser His Gln
            180                 185                 190
Thr Glu Leu Asn Asn Gly Glu Gly Cys Ser Arg Glu Thr Glu Ser Met
```

```
                195                 200                 205
Phe Ser Val Glu Thr Val Glu Ala Ser Leu Val Asn Arg Ala Asp Pro
        210                 215                 220
Glu Pro Val Pro Lys Phe Asp Asn Phe Asn Gly Ser Asp Ala Asp Asp
225                 230                 235                 240
Asn Asn Asn Asp Asp Val Glu Leu Glu Leu Thr Leu Gly Phe Glu Pro
                245                 250                 255
Ser Arg Arg Glu Lys Ser Met Ser Pro Lys Lys Trp Arg Asp Glu Ile
            260                 265                 270
Asn Asn Ser Tyr Thr Asp Thr Cys Lys Ile Glu Val Gly Ile Asp Tyr
        275                 280                 285
Ala Val
    290
```

<210> 36
<211> 217
<212> PRT
<213> Solanum tuberosum

<400> 36

```
Met Ser Cys Asn Gly Cys Arg Val Leu Arg Lys Gly Cys Ser Asp Asn
1               5                   10                  15
Cys Ile Leu Arg Pro Cys Leu Gln Trp Ile Glu Thr Pro Glu Ala Gln
            20                  25                  30
Gly His Ala Thr Val Phe Val Ala Lys Phe Phe Gly Arg Ala Gly Leu
        35                  40                  45
Met Ser Phe Ile Ser Ala Val Pro Glu Asn Gln Arg Pro Ala Leu Phe
    50                  55                  60
Gln Ser Leu Leu Tyr Glu Ala Ala Gly Arg Thr Val Asn Pro Val Asn
65                  70                  75                  80
Gly Ala Val Gly Leu Leu Trp Thr Gly Asn Trp His Val Cys Gln Ala
                85                  90                  95
Ala Val Glu Thr Val Leu Arg Gly Gly Ala Leu Arg Pro Ile Ser Glu
            100                 105                 110
Phe Leu Gly Ala Ser Val Glu Ile Asp Glu Val Ser Asp Cys Thr Asp
        115                 120                 125
Val Phe Lys Leu Gln Asp Pro Ser Leu Asn Met Arg Pro Lys Met Gln
    130                 135                 140
Lys Arg Arg Arg Phe Pro Glu Glu Thr Ser Met Leu Ala Asp Leu Asp
145                 150                 155                 160
Leu Ser Leu Thr Pro Gly Phe Asn Gln Lys Val Tyr Asn Ser His Pro
                165                 170                 175
Leu Ala Glu Asn His Arg Arg Pro Gly Thr Pro Ser Met Asn Ser Glu
            180                 185                 190
Glu Ser Gly Thr Thr Thr Cys Phe Glu Ser Thr Ala Val Ile Gly Glu
        195                 200                 205
Glu Pro Lys Leu Leu Ser Leu Phe Asn
    210                 215
```

<210> 37
<211> 212
<212> PRT
<213> Solanum tuberosum

<400> 37

```
Met Ser Cys Asn Gly Cys Arg Val Leu Arg Lys Gly Cys Ser Asp Asn
1               5                   10                  15
Cys Ile Leu Arg Pro Cys Leu Gln Trp Ile Glu Thr Pro Glu Ala Gln
            20                  25                  30
Gly His Ala Thr Val Phe Val Ala Lys Phe Phe Gly Arg Ala Gly Leu
        35                  40                  45


Met Ser Phe Ile Ser Ala Val Pro Glu Asn Gln Arg Pro Ala Leu Phe
    50                  55                  60
Gln Ser Leu Leu Tyr Glu Ala Ala Gly Arg Thr Val Asn Pro Val Asn
65                  70                  75                  80
Gly Ala Val Gly Leu Leu Trp Thr Gly Asn Trp His Val Cys Gln Ala
                85                  90                  95
Ala Val Glu Thr Val Leu Arg Gly Gly Ala Leu Arg Pro Ile Ser Glu
            100                 105                 110
Phe Leu Gly Ala Ser Val Glu Ile Asp Glu Val Ser Asp Cys Thr Asp
        115                 120                 125
Val Phe Lys Leu Gln Asp Pro Arg Lys Met Gln Lys Arg Arg Arg Phe
    130                 135                 140
Pro Glu Glu Thr Ser Met Leu Ala Asp Leu Asp Leu Ser Leu Thr Pro
145                 150                 155                 160
Gly Phe Asn Gln Lys Val Tyr Asn Ser His Pro Leu Pro Glu Asn His
                165                 170                 175
Arg Arg Pro Gly Thr Pro Ser Met Asn Ser Glu Glu Ser Gly Thr Thr
            180                 185                 190
Thr Cys Phe Glu Ser Thr Ala Val Ile Gly Glu Glu Pro Lys Leu Leu
        195                 200                 205
Ser Leu Phe Asn
    210
```

<210> 38
<211> 217
<212> PRT
<213> Solanum tuberosum

<400> 38

```
Met Ser Cys Asn Gly Cys Arg Ile Leu Arg Lys Gly Cys Asn Glu Asn
1               5                   10                  15
Cys Ile Leu Arg Gln Ser Leu Gln Gly Ile Glu Ser Pro His Ala Gln
            20                  25                  30
Ala Asn Ala Thr Val Phe Val Ala Lys Phe Phe Gly Arg Ala Gly Leu
        35                  40                  45
Met Ser Phe Leu Thr Ser Val Ser Glu Ser Gln Arg Pro Ala Leu Phe
    50                  55                  60
Gln Ser Leu Leu Phe Glu Ala Cys Gly Arg Thr Val Asn Pro Val His
65                  70                  75                  80
Gly Ala Val Gly Leu Leu Trp Thr Gly Asn Trp His Val Cys Gln Ser
                85                  90                  95
Ala Val Glu Thr Val Leu Lys Gly Gly Val Leu Arg Pro Leu Pro Glu
            100                 105                 110
Phe Ser Gly Val Thr Ala Ser Pro Glu Phe Asp Ser Glu Ala Asn Asp
        115                 120                 125
Val Asp Leu Phe Arg Ser Gln Ser Ser Asn Phe Gln Ser Lys Arg Lys
    130                 135                 140
Ile Ile Asp Glu Val Ser Glu Asp Leu Asp Leu Gly Leu Thr Ser Gly
145                 150                 155                 160
Leu Ser Thr Met Val Thr Gly Gly Lys Leu Asn Arg Arg Thr Glu Lys
                165                 170                 175
Arg Arg Ala Ala Thr Pro Ser Leu Asn Ser Asp Glu Ser Asp Thr Thr
            180                 185                 190
Thr Leu Glu Ser Gly Val Val Tyr His Gln Asn Pro Gln Gln Gly Asn
        195                 200                 205
Glu Thr Lys Leu Leu Arg Leu Phe Phe
    210                 215
```

<210> 39
<211> 277
<212> PRT
<213> Picea alba

<400> 39

Met Ser Ala Thr Asp Lys Asn Ile Thr Glu Arg Lys Glu Gln Gln Arg
1 5 10 15
Lys Met Ser Cys Asn Gly Cys Arg Val Leu Arg Lys Gly Cys Ser Asp
20 25 30
Asn Cys Ile Leu Arg Gln Ser Leu Gln Trp Ile Glu Ser Pro Gln Ser
35 40 45
Gln Ala His Ala Thr Val Phe Val Ala Lys Phe Phe Gly Arg Ala Gly
50 55 60
Leu Met Thr Phe Ile Ala Thr Val Ser Glu Pro Glu Arg Pro Asp Leu
65 70 75 80
Phe Lys Ser Leu Leu Tyr Glu Ala Cys Gly Arg Thr Val Asn Pro Val
85 90 95
His Gly Ala Val Gly Leu Met Trp Thr Gly Lys Trp Gln Ile Cys Gln
100 105 110
Ala Ala Val Gln Thr Val Leu Ser Gly Gly Ser Leu Asp Arg Thr Pro
115 120 125
Ala Ser Pro Trp Phe Pro Leu Thr Asp Ala Asn Gly Asp Arg Asn Arg
130 135 140
Met Ser Ala Gly Asp Gln Trp Pro Asn Glu Thr Asp Val Gly Asn Tyr
145 150 155 160
Pro Gly Asp Asp Asp Val Cys Lys Ile Tyr Glu Leu Asp Ala Cys Val
165 170 175
Asp Ser Arg Arg Val Arg Gln Lys Ile Asp Gly Ser Asn Asn Asn Asn
180 185 190
Asn Glu Ile Ala Ser Thr Asp Ala Asp Lys Leu Gly Gln Arg Gly Ser
195 200 205
Gly Ile Asp Gly Gln Lys Asn Asp Ile Lys Leu Asp Leu Ser Leu Asn
210 215 220
Cys Ser Ser Lys Ser Lys Ser Ser Asn Leu Lys Gln Gln Arg Val Ser
225 230 235 240
Ser Pro Ser Thr Asn Ser Val His Ser Glu Gly Ser Val Asn Arg Leu
245 250 255
Lys Ile Ser Asn Pro Leu Pro Ser Ser Thr Met Ala Ser Gln Gln Lys
260 265 270
Phe Leu Asp Leu Leu
275

<210> 40
<211> 363
<212> PRT
<213> Pinus taeda

<400> 40

Met Thr Ala Thr Thr Gly Ala Gly Asn Ser Gly Arg Asp Leu Ser Val
1 5 10 15
Phe Ile Gln Arg Lys Gln Glu Ala Asp Gly Arg Lys Arg Met Ser Cys
20 25 30
Asn Gly Cys Arg Val Leu Arg Lys Gly Cys Ser Asp Thr Cys Ile Leu
35 40 45
Arg Pro Cys Leu Gln Trp Ile Glu Ser Pro Glu Ala Gln Gly His Ala
50 55 60
Thr Val Phe Val Ala Lys Phe Phe Gly Arg Ala Gly Leu Met Ser Phe
65 70 75 80
Ile Ser Ala Val Pro Asp His Gln Arg Pro Ala Leu Phe Gln Ser Leu
85 90 95
Leu Tyr Glu Ala Cys Gly Arg Thr Val Asn Pro Val Asn Gly Ala Val
100 105 110
Gly Leu Leu Trp Asn Gly Asn Trp Gln Leu Cys Gln Ala Ala Val Glu

```
        115                 120                 125
Thr Val Leu Lys Gly Gly Thr Leu Arg Pro Pro Gln Ser Ser Ser Val
    130                 135                 140
Ile Pro Thr Cys Ser Thr Arg Pro Ser Leu Met Asn Ser Phe Ser Ala
145                 150                 155                 160
Asn Phe Arg Val Asn Ser Asn Val Asn Asn Ile Asn Ser Ser Gly Ser
                165                 170                 175
Arg Leu Ser Leu Glu Val Gly Lys Ala Arg Pro Val Asn Glu Met Arg
            180                 185                 190
Glu Met Tyr Thr Val Lys Pro Glu Val Asp Phe Pro Leu Asn Arg Pro
        195                 200                 205
Gln Glu Ser Cys Asn Pro His Gln Pro Trp Ala Ala Lys Val Lys Ser
    210                 215                 220
Glu Pro His Asp Leu Thr Leu Cys Glu Ala Phe Glu Ala Val Arg His
225                 230                 235                 240
Ser Gln Thr Ile Ile His Ser Pro Pro Ser Glu Ile Ala Ala Ala Pro
                245                 250                 255
Arg Arg Val Arg Gln Arg Ile Glu Gly Ile Asn Glu Val Asn Tyr Arg
            260                 265                 270
Asp Leu Ser Asn Ser Asn Ser Asp Asp Val Lys Cys Leu Gly Val Phe
        275                 280                 285
Asp Ile His Pro Asn Asn Thr Met Glu Met Lys Ala Asn Leu Asp Leu
    290                 295                 300
Thr Leu Asn Phe Glu Ala Ser Ser Ser Ser His Ile Lys Arg Arg Val
305                 310                 315                 320
Ser Ser Pro Ser Ile Val Ser Val Asn Ser Glu Gly Ser Val Thr Ser
                325                 330                 335
Leu Glu Ser Gly Ser Leu Arg Val Pro Asn Gln Lys Asp Ser Ser Ala
            340                 345                 350
Ala Lys Glu His Lys Val Leu Asp Leu Phe Pro
        355                 360
```

<210> 41
<211> 276
<212> PRT
<213> Pinus taeda

<400> 41

```
Met Ser Ala Thr Glu Asn His Ile Thr Glu Arg Lys Glu Gln Glu Arg
1               5                   10                  15
Lys Met Ser Cys Asn Gly Cys Arg Val Leu Arg Lys Gly Cys Ser Glu
            20                  25                  30
Asn Cys Ile Leu Arg Gln Ser Leu Gln Trp Ile Glu Ser Pro Gln Ser
        35                  40                  45
Gln Ala His Ala Thr Val Phe Val Ala Lys Phe Phe Gly Arg Ala Gly
    50                  55                  60
Leu Met Thr Phe Ile Ala Ala Val Ser Glu Pro Glu Arg Pro Ala Leu
65                  70                  75                  80
Phe Lys Ser Leu Leu Tyr Glu Ala Cys Gly Arg Thr Val Asn Pro Val
                85                  90                  95
His Gly Ala Val Gly Leu Leu Trp Thr Gly Lys Trp Gln Ile Cys Gln
            100                 105                 110
Ala Ala Val Gln Thr Val Leu Ser Gly Gly Ser Leu Asp Arg Thr Pro
        115                 120                 125
Ala Gly Thr Cys Phe Pro Ser Thr Asp Ala Asn Gly Asp Arg Asn Gly
    130                 135                 140
Met Ser Asp Gly Asp Gln Trp Pro Asn Glu Met Asp Gly Asn Phe Pro
145                 150                 155                 160
Gly Asp Asp Asp Val Cys Lys Ile Tyr Glu Leu Gly Ala Cys Asp Asp
                165                 170                 175
Ser Arg Arg Val Arg Gln Lys Ile Asp Gly Asn Asn Asn Asn Ile Asn


            180                 185                 190
Glu Leu Met Ser Thr Asp Gly Glu Lys Leu Gly Gln Arg Gly Ser Gly
        195                 200                 205
Ile Asp Gly Gln Lys Asn Asp Leu Lys Leu Asp Leu Ser Leu Asn Cys
    210                 215                 220
Pro Ser Lys Ser Lys Ser Ser Asn Leu Lys Gln Gln Arg Val Ser Ser
225                 230                 235                 240
Pro Ser Thr Asn Ser Val His Ser Glu Gly Ser Val Asn Arg Leu Lys
                245                 250                 255
Ser Ser Ser Pro Pro Pro Ser Ser Thr Met Ala Ala Glu Gln Lys Leu
            260                 265                 270
Leu Asp Leu Leu
        275
```

<210> 42
<211> 218
<212> PRT
<213> Lycopersicon esculentum

<400> 42

```
Met Ser Cys Asn Gly Cys Arg Val Leu Arg Lys Gly Cys Ser Asp Asn
1               5                   10                  15
Cys Ile Leu Arg Pro Cys Leu Gln Trp Ile Glu Thr Pro Glu Ala Gln
            20                  25                  30
Gly His Ala Thr Val Phe Val Ala Lys Phe Phe Gly Arg Ala Gly Leu
        35                  40                  45
Met Ser Phe Ile Ser Ala Val Pro Glu Asn Gln Arg Pro Ala Leu Phe
    50                  55                  60
Gln Ser Leu Leu Tyr Glu Ala Ala Gly Arg Thr Val Asn Pro Val Asn
65                  70                  75                  80
Gly Ala Val Gly Leu Leu Trp Thr Gly Asn Trp His Val Cys Gln Ala
                85                  90                  95
Ala Val Glu Thr Val Leu Arg Gly Gly Ala Leu Arg Pro Ile Ser Glu
            100                 105                 110
Phe Leu Gly Ala Ser Val Glu Ile Asp Glu Val Ser Asp Cys Thr Asp
        115                 120                 125
Val Phe Lys Leu Gln Asp Pro Ser Leu Asn Met Arg Pro Lys Met Gln
    130                 135                 140
Lys Arg Arg Arg Ser Pro Glu Glu Thr Ser Met Leu Asp Leu Ser Leu
145                 150                 155                 160
Thr Pro Gly Phe Asn Gln Lys Val Tyr Asn Ser His Pro Leu Pro Glu
                165                 170                 175
Asn His Arg Arg Pro Gly Thr Pro Ser Met Asn Ser Glu Glu Ser Gly
            180                 185                 190
Thr Thr Thr Cys Phe Glu Ser Ser Ala Val Ile Gly Asp His Gln Gly
        195                 200                 205
Lys Glu Pro Lys Leu Leu Ser Leu Phe Asn
    210                 215
```

<210> 43
<211> 238
<212> PRT
<213> Populus tremuloides

<400> 43

```
Met Ser Cys Asn Gly Cys Arg Val Leu Arg Lys Gly Cys Ser Glu Asn
1               5                   10                  15
Cys Ile Leu Arg Pro Cys Leu Gln Trp Ile Glu Ser Pro Glu Ala Gln
            20                  25                  30
Gly His Ala Thr Val Phe Val Ala Lys Phe Phe Gly Arg Ala Gly Leu
        35                  40                  45
```

```
Met Ser Phe Ile Ser Ala Val Pro Glu Asn Gln Arg Pro Ser Leu Phe
    50                  55                  60
Gln Ser Leu Leu Phe Glu Ala Cys Gly Arg Thr Val Asn Pro Val Asn
65                  70                  75                  80
Gly Ala Ala Gly Leu Leu Trp Thr Gly Asn Trp His Val Cys Gln Ala
                85                  90                  95
Ala Val Glu Thr Val Leu Arg Gly Gly Thr Leu Gln Pro Met Pro Glu
            100                 105                 110
Leu Leu Thr Gly Gly Gly Gly Ser Pro Ser Pro Ser Ser Asp Glu Ala
        115                 120                 125
Ser Glu Val Glu Val Ala Cys Thr Asp Ile Trp Lys Leu Gln Asp Pro
    130                 135                 140
Asn Pro Asn His His Pro Arg Phe Ser Ile Ser Arg Ser Arg Leu Ser
145                 150                 155                 160
Pro Lys Arg Lys Arg Thr Glu Glu Pro Val Ala Val Lys Val Gln His
                165                 170                 175
Asn Asp Leu Asp Leu Gly Leu Thr Pro Ser Phe Ser Leu Lys Gly Phe
            180                 185                 190
Pro Tyr Lys Gln Gln Ile Arg Arg Pro Gly Thr Pro Ser Met Asn Ser
        195                 200                 205
Ala Glu Ser Val Thr Thr Asn Thr Ala Cys Phe Asp Ser Ala Gly Leu
    210                 215                 220
Gly Asp Glu Gly Gly Glu Thr Lys Leu Leu Asn Leu Phe Leu
225                 230                 235
```

<210> 44
<211> 242
<212> PRT
<213> Populus tremuloides

<400> 44

```
Met Ser Cys Asn Gly Cys Arg Val Leu Arg Lys Gly Cys Ser Glu Asn
1               5                   10                  15
Cys Ile Leu Arg Pro Cys Leu Gln Trp Ile Glu Ser Pro Glu Ala Gln
            20                  25                  30
Gly His Ala Thr Val Phe Val Ala Lys Phe Phe Gly Arg Ala Gly Leu
        35                  40                  45
Met Ser Phe Ile Ser Ser Val Pro Glu Asp Gln Arg Pro Ser Leu Phe
    50                  55                  60
Gln Ser Leu Leu Phe Glu Ala Cys Gly Arg Thr Val Asn Pro Val Asn
65                  70                  75                  80
Gly Ala Val Gly Leu Leu Trp Thr Gly Asn Trp His Val Cys Gln Ala
                85                  90                  95
Ala Val Glu Thr Val Leu Arg Gly Gly Thr Leu Arg Pro Met Pro Asp
            100                 105                 110
Leu Leu Thr Gly Gly Gly Ser Pro Ser Ser Ser Pro Pro Ser Asp Glu
        115                 120                 125
Ala Ser Glu Phe Glu Val Ala Cys Thr Asp Ile Trp Lys Leu Gln Asp
    130                 135                 140
Pro Asn Pro Asn Pro Ile His His Ser Arg Phe Ser Asn Ser Arg Ser
145                 150                 155                 160
Arg Val Ser Ser Lys Arg Lys Gly Thr Glu Glu Pro Met Val Val Asn
                165                 170                 175
Met Arg His Asp Asp Leu Asp Leu Leu Leu Thr Pro Ser Thr Ser Gln
            180                 185                 190
Lys Gly Phe Ala Glu Ile Cys Arg Pro Gly Thr Pro Ser Met Asn Ser
        195                 200                 205
Glu Glu Ser Val Thr Thr Asn Ala Thr Cys Phe Asp Ser Ala Gly Phe
    210                 215                 220
Gly Asp Gln Tyr Gly Asn Gly Gly Gly Glu Thr Lys Leu Leu Asn Leu
225                 230                 235                 240
```

Phe Leu

<210> 45
<211> 208
<212> PRT
<213> Populus tremuloides

<400> 45

```
Met Ser Cys Asn Gly Cys Arg Val Leu Arg Lys Gly Cys Ser Glu Thr
1               5                   10                  15
Cys Val Leu Arg Ser Cys Leu His Trp Ile Pro Thr Pro Glu Ala Gln
            20                  25                  30
Gly Asn Ala Thr Leu Phe Leu Ala Lys Phe Phe Gly Arg Ser Asp Leu
        35                  40                  45
Ile Ser Leu Ile Ser Ala Val Pro Glu Ser Gln Arg Pro Val Leu Phe
    50                  55                  60
Gln Ser Leu Leu Phe Glu Ala Cys Gly Arg Thr Val Asn Pro Val Asn
65                  70                  75                  80
Gly Ala Val Gly Leu Leu Trp Ser Gly Asn Trp His Val Cys Gln Ala
                85                  90                  95
Ala Val Glu Ser Val Leu Ala Gly Glu Thr Leu Arg Pro Leu Pro Gly
            100                 105                 110
Ile Leu Thr Gly Val Leu Ala Pro Asn Cys Asp Glu Ser Ser Asp Ser
        115                 120                 125
Phe Ser Ala Ala Ala Tyr Ala Val His Ser Met Thr Trp Asn Gln Ser
    130                 135                 140
Lys Pro Phe Asp Lys Glu Asn Asn Asn Gln Val Val Ser Asp His His
145                 150                 155                 160
Val Asn Leu Cys Leu Ala Arg Gly Arg Gly Gly Arg Asp Lys Arg Gly
                165                 170                 175
Arg Asp Ala Val Ser Phe Tyr Thr Gly Gly Glu Ser Glu Thr Thr Ser
            180                 185                 190
Phe Glu Ser Ser Gly Ala Asp Lys Asn Lys Leu Leu Asn Leu Phe Val
        195                 200                 205
```

<210> 46
<211> 208
<212> PRT
<213> Populus tremuloides

<400> 46

```
Met Ser Cys Asn Gly Cys Arg Val Leu Arg Lys Gly Cys Ser Glu Thr
1               5                   10                  15
Cys Val Leu Arg Ser Cys Leu His Trp Ile Thr Asn Pro Glu Ala Gln
            20                  25                  30
Gly Asn Ala Thr Leu Phe Leu Ala Lys Phe Phe Gly Arg Ser Asp Leu
        35                  40                  45
Met Ser Leu Ile Ser Ala Val Pro Glu Ala Gln Arg Pro Ala Leu Phe
    50                  55                  60
Gln Ser Leu Leu Phe Glu Ala Cys Gly Arg Thr Val Asn Pro Val Asn
65                  70                  75                  80
Gly Val Val Gly Leu Leu Trp Ser Gly Asn Trp His Met Cys Gln Glu
                85                  90                  95
Ala Val Glu Thr Val Leu Ser Gly Gly Ala Leu Gln Pro Leu Pro Gly
            100                 105                 110
Ile Leu Thr Gly Val Leu Ala Pro Asn Cys Asp Glu Ser Ser Asp Arg
        115                 120                 125
Phe Ser Ala Ala Ala Tyr Ala Val His Asn Met Ala Arg Asn Gln Ser
    130                 135                 140
Arg Ser Phe Ala Lys Glu Asn Asn Lys Glu Val Val Ser Asp His Arg


145                 150                 155                 160
Ile Asn Leu Cys Leu Ala Arg Gly Lys Gly Gly Arg Asp Lys Arg Gly
                165                 170                 175
Arg Asp Ala Val Ser Phe Tyr Thr Gly Gly Glu Ser Glu Thr Ile Ser
            180                 185                 190
Phe Glu Ser Ser Gly Gly Asp Lys Lys Arg Leu Leu Asn Leu Phe Val
        195                 200                 205
```

<210> 47
<211> 296
<212> PRT
<213> Populus tremuloides

<400> 47

```
Met Arg Met Ser Cys Asn Gly Cys Arg Val Leu Arg Lys Gly Cys Ser
1               5                   10                  15
Glu Asn Cys Ser Ile Arg Pro Cys Leu Gln Trp Ile Lys Ser Ser Glu
            20                  25                  30
Ser Gln Ala Asn Ala Thr Val Phe Leu Ala Lys Phe Tyr Gly Arg Ala
        35                  40                  45
Gly Leu Met Asn Leu Ile Asn Ala Gly Pro Glu His Leu Arg Pro Ala
    50                  55                  60
Ile Phe Arg Ser Leu Leu Tyr Glu Ala Cys Gly Arg Ile Val Asn Pro
65                  70                  75                  80
Ile Tyr Gly Ser Val Gly Leu Met Trp Ser Gly Ser Trp Gln Leu Cys
                85                  90                  95
Gln Ala Ala Val Glu Ala Val Leu Lys Gly Ala Pro Ile Thr Pro Ile
            100                 105                 110
Asn Ser Glu Ala Ala Val Asn Gly His Gly Pro Pro Leu Lys Val Tyr
        115                 120                 125
Asp Ile Arg His Val Ser Lys Glu Glu Asn Ser Ala Ala Ser Asn Asp
    130                 135                 140
Ala Asn Arg Ala Arg Thr Arg Cys Arg Val Arg Arg Val Val Lys Pro
145                 150                 155                 160
Lys Ala Ser Lys Arg Ala Cys Phe Gly Asn Gly Leu Ala Ser Ile Ala
                165                 170                 175
Val Asp Thr Leu Ser Ala Arg Asp Glu Leu Thr Arg Cys Thr Ser His
            180                 185                 190
Glu Ser Ser Val Ser His Gln Ser Glu Leu Ala Val Leu Asp Gly Asp
        195                 200                 205
Ser Lys Glu Ser Asp Glu Ser Met Val Ser Val Glu Thr Ala Glu Ala
    210                 215                 220
Ser Leu Leu Phe Pro Pro Asn Pro Glu Ser Lys Asn Asp Cys Lys Ala
225                 230                 235                 240
His Asp Val Ala Ser Asn Gly Ile Ser Gly Leu Glu Leu Gly Leu Asp
                245                 250                 255
Leu Thr Leu Gly Leu Glu Pro Val Ser Arg Ala His His Val Val Pro
            260                 265                 270
Val Lys Lys Arg Arg Val Glu Ala Tyr Gly Ser Gly Asp Val Asp Thr
        275                 280                 285
Cys Lys Met Glu Leu Arg Leu Glu
    290                 295
```

<210> 48
<211> 241
<212> PRT
<213> Populus tremuloides

<400> 48

```
Met Ser Cys Asn Gly Cys Arg Val Leu Arg Lys Gly Cys Asn Asp Asn
1               5                   10                  15
```

```
Cys Thr Ile Arg Pro Cys Leu Gln Trp Ile Lys Ser Thr Asp Ser Gln
            20                  25                  30
Ala Asn Ala Thr Leu Phe Leu Ala Lys Phe Tyr Gly Arg Ala Gly Leu
        35                  40                  45
Ile Asn Leu Ile Glu Ala Ala Pro Gln Arg Leu Arg Pro Ala Ile Phe
    50                  55                  60
Ser Ser Leu Leu Tyr Glu Ala Cys Gly Arg Ile Val Asn Pro Val Tyr
65                  70                  75                  80
Gly Ser Val Gly Met Leu Trp Ser Gly Asn Trp Ala Gln Cys Gln Ala
                85                  90                  95
Ala Val Asp Ala Val Leu Lys Gly Leu Pro Ile Ile Ser Ile Pro Ser
            100                 105                 110
Ser Asp Val Pro Thr Pro His Leu Ile Ser Ser Leu Asn Thr Tyr Asp
        115                 120                 125
Ile Arg His Val Ser Arg Asp Gln Asn Ser Pro Glu Leu Asn Lys Val
    130                 135                 140
Lys Ser Arg Thr Arg Cys Lys Arg Ser Ile Gly Thr Arg Pro Ser Ser
145                 150                 155                 160
Leu Ala Glu Pro Thr Gly Arg Leu Asn Gln Gly Glu Leu Gly Phe Glu
                165                 170                 175
Pro Val Arg Glu Ser Trp Leu Gly His Leu Gly Asn Gly Asp Ser Arg
            180                 185                 190
Ile Lys Asp Glu Asp Ser Ile Leu Ala Ala Glu Asn Val Glu Asp Ser
        195                 200                 205
Leu Trp Ser Arg Val Glu Pro Asp Gln Val Phe Lys Phe Asn Gly Gln
    210                 215                 220
Ser Asp Asp Ser Asp Leu Ser Leu Glu Leu Thr Leu Ala Leu Val Ser
225                 230                 235                 240
Glu
```

<210> 49
<211> 273
<212> PRT
<213> Populus tremuloides

<400> 49

```
Met Pro Phe Ser Ala Leu Ser Phe Ser Ser Leu Pro His Arg Ile Ile
1               5                   10                  15
Leu Leu Leu Ile Gly Phe Leu Asn Ser Ser Pro Ser Leu Ser Arg His
            20                  25                  30
Phe Ser Ile Ser Phe Arg Thr Glu Tyr Thr Met Arg Met Ser Cys Asn
        35                  40                  45
Gly Cys Arg Ile Leu Arg Lys Gly Cys Gly Asp Asn Cys Ser Ile Lys
    50                  55                  60
Pro Cys Leu Gln Trp Ile Glu Thr Pro Asp Ser Gln Ala Asn Ala Thr
65                  70                  75                  80
Leu Phe Leu Ala Lys Phe Tyr Gly Arg Ala Gly Leu Met Asn Leu Ile
                85                  90                  95
Asn Ala Cys Pro Gln His Leu Arg Pro Asp Thr Phe Lys Ser Leu Leu
            100                 105                 110
Tyr Glu Ala Cys Gly Arg Ile Val Asn Pro Val Ser Gly Ser Val Gly
        115                 120                 125
Leu Leu Ser Thr Gly Ser Trp Gln Gln Cys Gln Ala Ala Val Glu Ala
    130                 135                 140
Val Leu Lys Gly Glu Pro Ile Thr Gln Ile Ala Ser Thr Asp Gln Leu
145                 150                 155                 160
Thr Leu Gly Gly Ser Asp Thr Arg His Val Ser Arg Glu Glu Asp Trp
                165                 170                 175
Ser Ala Ser Asp Gln Pro Arg Lys Ile Lys Ser Lys Arg Gln Phe Asn
            180                 185                 190
```

Arg Ser Thr Ser Lys Arg Lys Pro Ser Arg Thr Glu Ala Glu His Thr
195 200 205
Cys Phe Glu Phe Met Ile Gly Phe Asp Gly Leu Arg Ser Val Ser Pro
210 215 220
Asp Ser Val Leu Ser Trp Arg Pro Ser Leu Gly Ser Asp Asn Glu Glu
225 230 235 240
Thr Asp Ser Ile Gly Ser Val Glu Thr Val Glu Ala Ser Lys Leu Asp
245 250 255
Glu Pro Ala Glu Gly Ser Asp Leu Asp Leu Asp Leu Thr Leu Gly His
260 265 270
Tyr

<210> 50
<211> 231
<212> PRT
<213> Caragana korshinskii

<400> 50

Met Ser Cys Asn Gly Cys Arg Val Leu Arg Lys Gly Cys Ser Glu Ser
1 5 10 15
Cys Ile Leu Arg Pro Cys Leu Gln Trp Leu Glu Asn Pro Glu Ala Gln
20 25 30
Gly His Ala Thr Val Phe Val Ala Lys Phe Phe Gly Arg Ala Gly Leu
35 40 45
Met Ser Phe Leu Ser Asn Val Pro Glu Thr Gln Arg Pro Ala Leu Phe
50 55 60
Gln Ser Leu Leu Phe Glu Ala Cys Gly Arg Thr Val Asn Pro Val Asn
65 70 75 80
Gly Ala Val Gly Leu Leu Trp Thr Gly Asn Trp His Val Cys Gln Ala
85 90 95
Ala Val Glu Thr Val Leu Gln Gly Gly Thr Leu Arg Pro Leu Pro Glu
100 105 110
His Thr Leu Leu Asp Ala Pro Pro Ala Ala Thr Asp Glu Ala Ser Glu
115 120 125
Ala Glu Asp Ala Cys Thr Gly Ile Trp Arg Ile Arg Asp Pro Asn Pro
130 135 140
Asn Cys Arg Pro Asn Gly Ser Arg Ser Asn Asn Lys Val Ser Thr Gly
145 150 155 160
Gly Lys Arg Lys Arg Ser Glu Glu Leu Val Lys Ile Pro Ala Ala Thr
165 170 175
Asn Leu Asp Leu Arg Leu Thr Pro Ile Phe Leu Gln Lys Glu Ser Glu
180 185 190
Ser Arg Arg Pro Gly Ser Pro Ser Met Asn Thr Glu Glu Ser Val Thr
195 200 205
Thr Thr Ala Thr Gly Leu Gly Asp Ser Trp Gly His Gly Gly Glu Arg
210 215 220
Lys Val Leu Asn Leu Phe Ile

225 230

<210> 51
<211> 264
<212> PRT
<213> Brassica napus

<400> 51

```
Met Arg Met Ser Cys Asn Gly Cys Arg Val Leu Arg Lys Gly Cys Ser
1               5                   10                  15
Asp Asp Cys Ser Ile Arg Pro Cys Leu Gly Trp Ile Lys Ser Pro Glu
            20                  25                  30
Ala Gln Ala Asn Ala Thr Val Phe Leu Ala Lys Phe Tyr Gly Arg Ala
        35                  40                  45
Gly Leu Met Asn Leu Ile Asn Ala Gly Pro Asp His Leu Arg Pro Gly
    50                  55                  60
Ile Phe Arg Ser Leu Leu His Glu Ala Cys Gly Arg Ile Val Asn Pro
65                  70                  75                  80
Ile Tyr Gly Ser Val Gly Leu Leu Trp Ser Gly Asn Trp Gln Leu Cys
                85                  90                  95
Gln Ser Ala Val Glu Ala Val Met Lys Gly Glu Pro Ile Thr Glu Met
            100                 105                 110
Ala Thr Asp Ala Ala Thr Ser Gly Gln Gly Pro Pro Leu Lys Met Tyr
        115                 120                 125
Asp Ile Arg His Ile Ala Lys Asp Glu Asn Ser Pro Ala Ala Ala Ala
    130                 135                 140
Ala Ala Gly Ser Thr Asp Arg Lys Arg Gly Lys Thr Arg Arg Ala Lys
145                 150                 155                 160
Arg Val Ala Ala Val Ala Lys Pro Ala Glu Ser Gly Gly Gly Glu Ala
                165                 170                 175
Ser His His Ser Ser Leu Ser His Gln Ser Glu Val Val Val Ala Pro
            180                 185                 190
His Glu Gly Glu Ser Lys Glu Ser Glu Ser Asn Asn Ser Glu Val Met
        195                 200                 205
Thr Phe Ser Pro Pro Ala Val Gln Gly Ser Gly Glu Ile Lys Leu Asp
    210                 215                 220
Leu Thr Leu Gly Leu Glu Pro Val Ser Arg Ala Asp His Val Val Pro
225                 230                 235                 240
Val Lys Lys Arg Lys Met Gly Val Phe Ser Thr Trp Gln Glu Glu Ser
                245                 250                 255
Ser Cys Lys Thr Asp Pro Val Leu
            260
```

<210> 52
<211> 233
<212> PRT
<213> Medicago truncatula

<400> 52

```
Met Ser Cys Asn Gly Cys Arg Val Leu Arg Lys Gly Cys Ser Glu Ser
1               5                   10                  15
Cys Ile Leu Arg Pro Cys Leu Gln Trp Ile Glu Thr Pro Glu Ala Gln
            20                  25                  30
Gly His Ala Thr Val Phe Val Ala Lys Phe Phe Gly Arg Ala Gly Leu
        35                  40                  45
Met Ser Phe Ile Ser Asn Val Pro Glu Pro Gln Arg Pro Ala Leu Phe
    50                  55                  60
Gln Ser Leu Leu Phe Glu Ala Cys Gly Arg Thr Val Asn Pro Val Asn
65                  70                  75                  80
Gly Ala Val Gly Leu Leu Trp Thr Gly Asn Trp His Val Cys Gln Ala
                85                  90                  95
Ala Val Glu Thr Val Leu Arg Gly Gly Thr Leu Arg Pro Leu Pro Glu
            100                 105                 110
Leu Thr Leu Leu Asp Ala Pro Leu Thr Ala Thr Asp Glu Ala Ser Glu
        115                 120                 125
Ala Glu Asp Gly Gly Ala Asp Ala Met Trp Lys Ile Arg Glu Pro Asn
    130                 135                 140
Phe Gly Leu Ala Ser Ser Arg Ser Ser Asn Lys Val Cys Ser Gly Ser
145                 150                 155                 160
Lys Arg Arg Arg Ser Glu Glu Phe Val Lys Val Pro Ala Ala Ile Asn
                165                 170                 175
Leu Asp Leu Arg Leu Thr Pro Ile Phe Gln Gln Lys Ala Val Glu Glu
            180                 185                 190

Arg Arg His Gly Ser Pro Ser Met Thr Ser Glu Glu Ser Val Thr Thr
        195                 200                 205
Thr Ala Cys Leu Glu Thr Gly Ile Gly Asp Arg Trp Ser His Gly Gly
    210                 215                 220
Asp Arg Lys Val Leu Asn Leu Phe Ile
225                 230
```

<210> 53
<211> 278
<212> PRT
<213> Medicago truncatula

<400> 53

```
Met Arg Met Ser Cys Asn Gly Cys Arg Val Leu Arg Lys Gly Cys Ser
1               5                   10                  15
Glu Asp Cys Ser Ile Arg Pro Cys Leu Glu Trp Ile Lys Cys Pro Gln
            20                  25                  30
Ser Gln Ala Asn Ala Thr Leu Phe Leu Ala Lys Phe Tyr Gly Arg Ala
        35                  40                  45
Gly Leu Ile Asn Leu Ile Asn Ser Gly Ser Glu Asn Leu Arg Pro Ala
    50                  55                  60
Ile Phe Arg Ser Leu Leu Tyr Glu Ala Cys Gly Arg Ile Val Asn Pro
65                  70                  75                  80
Ile Tyr Gly Ser Val Gly Leu Leu Trp Ser Gly Ser Trp His Leu Cys
                85                  90                  95
Gln Ala Ala Val Glu Ala Val Leu Lys Gly Glu Pro Ile Thr Pro Ile
            100                 105                 110
Asp Ser Glu Ala Asp Glu Asn Glu Arg Gly Pro Pro Phe Lys Ala Tyr
        115                 120                 125
Asp Ile Arg His Val Ser Lys Asp Glu Asn Leu Glu Glu Thr Lys Gln
    130                 135                 140
Val Arg Thr Arg Ser Arg Phe Arg Arg Ala Met Lys Pro Lys Pro Ser
145                 150                 155                 160
Lys Glu Val Gly Ser Gly Ser Gly Ser Ser Ser Ala Lys Glu Val Asp
                165                 170                 175
Arg Ser Lys Ser Gln Glu Ser Ser Leu Ser Gln Pro Gly Lys Asp Ser
            180                 185                 190
Glu Ser Gly Val Ser Val Glu Thr Ser Ile Leu Phe His Asp Glu Ser
        195                 200                 205
Glu Phe Glu Pro Glu Ser Gln Ala Lys Val Lys Gly Arg Ala Glu Gly
    210                 215                 220
Arg Ala Glu Glu Gly Leu Ser Asp Gly Val Gly Leu Glu Leu Arg Leu
225                 230                 235                 240
Gly Ile Glu Pro Val Ser Arg Glu Glu Phe Met Val Pro Ile Lys Lys
                245                 250                 255
Arg Lys Ile Val Leu Lys Asp Cys Ser Ala Ser Ser Lys Val Glu Leu
            260                 265                 270
Gly Leu Glu Leu Ser Ala
        275
```

<210> 54
<211> 247
<212> PRT
<213> Arabidopsis thaliana

<400> 54

```
Met Ser Cys Asn Gly Cys Arg Val Leu Arg Lys Gly Cys Ser Glu Asn
1               5                   10                  15
Cys Ile Leu Arg Pro Cys Ile Gln Trp Ile Glu Ser Pro Glu Ala Gln
            20                  25                  30
```

```
Gly His Ala Thr Val Phe Val Ala Lys Phe Phe Gly Arg Ala Gly Leu
        35                  40                  45
Met Ser Phe Ile Ser Ala Val Pro Glu Ser Gln Cys Pro Ala Leu Phe
    50                  55                  60
Gln Ser Leu Leu Tyr Glu Ala Cys Gly Arg Thr Val Asn Pro Val Asn
65                  70                  75                  80
Gly Ala Val Gly Leu Leu Trp Thr Gly Asn Trp Asn Val Cys Gln Ala
                85                  90                  95
Ala Val Glu Thr Val Leu Arg Gly Gly Ser Leu Lys Pro Ile Pro Glu
            100                 105                 110
Leu Leu Asn Gly Gly Gly Phe Ala Gly Phe Pro Ser Pro Thr Ser Asp
        115                 120                 125
Glu Ala Ser Glu Ile Cys Thr Glu Met Leu Asn Leu Arg Lys Ala Asp
    130                 135                 140
Asp Ser Gly Asp Arg Asn Ile Tyr His His Cys Arg Phe Ser Ser Ser
145                 150                 155                 160
Arg Ser Arg Ser Arg Ser Thr Ala Ser Pro Pro Lys Arg Lys Arg Leu
                165                 170                 175
Ser Ser Glu Gln Gln Pro Ser Ser Glu Leu Asp Leu Ser Leu Ile Pro
            180                 185                 190
Ile Tyr Pro Ile Lys Thr Leu Pro Phe Lys Glu Asp Thr Pro Ser Met
        195                 200                 205
Tyr Ser Glu Glu Ser Val Thr Thr Val Ser Phe Gln Asn Asn Asn Ala
    210                 215                 220
Gly Asp Arg Tyr Val Arg Cys Gly Gly Gly Gly Gly Gly Ala Thr Thr
225                 230                 235                 240
Lys Leu Leu Asn Leu Phe Ala
                245
```

<210> 55
<211> 240
<212> PRT
<213> Arabidopsis thaliana

<400> 55

```
Met Ser Cys Asn Gly Cys Arg Val Leu Arg Lys Gly Cys Ser Glu Thr
1               5                   10                  15
Cys Ile Leu Arg Pro Cys Leu Gln Trp Ile Glu Ser Ala Glu Ser Gln
            20                  25                  30
Gly His Ala Thr Val Phe Val Ala Lys Phe Phe Gly Arg Ala Gly Leu
        35                  40                  45
Met Ser Phe Ile Ser Ser Val Pro Glu Leu Gln Arg Pro Ala Leu Phe
    50                  55                  60
Gln Ser Leu Leu Phe Glu Ala Cys Gly Arg Thr Val Asn Pro Val Asn
65                  70                  75                  80
Gly Ala Val Gly Met Leu Trp Thr Arg Asn Trp His Val Cys Gln Ala
                85                  90                  95
Ala Val Glu Thr Val Leu Arg Gly Gly Thr Leu Arg Pro Ile Ser Asp
            100                 105                 110
Leu Leu Glu Ser Pro Ser Leu Met Ile Ser Cys Asp Glu Ser Ser Glu
        115                 120                 125
Ile Trp His Gln Asp Val Ser Arg Asn Gln Thr His His Cys Arg Phe
    130                 135                 140
Ser Thr Ser Arg Ser Thr Thr Glu Met Lys Asp Ser Leu Val Asn Arg
145                 150                 155                 160
Lys Arg Leu Lys Ser Asp Ser Asp Leu Asp Leu Gln Val Asn His Gly
                165                 170                 175
Leu Thr Leu Thr Ala Pro Ala Val Pro Val Pro Phe Leu Pro Pro Ser
            180                 185                 190
Ser Phe Cys Lys Val Val Lys Gly Asp Arg Pro Gly Ser Pro Ser Glu
        195                 200                 205
```

```
Glu Ser Val Thr Thr Ser Cys Trp Glu Asn Gly Met Arg Gly Asp Asn
    210                 215                 220
Lys Gln Lys Arg Asn Lys Gly Glu Lys Lys Leu Leu Asn Leu Phe Val
225                 230                 235                 240
```

<210> 56
<211> 233
<212> PRT
<213> Arabidopsis thaliana

<400> 56

```
Met Arg Met Ser Cys Asn Gly Cys Arg Val Leu Arg Lys Gly Cys Ser
1               5                   10                  15
Glu Asn Cys Ser Ile Arg Pro Cys Leu Gln Trp Ile Lys Ser Ala Glu
            20                  25                  30
Ser Gln Ala Asn Ala Thr Val Phe Leu Ala Lys Phe Tyr Gly Arg Ala
        35                  40                  45
Gly Leu Met Asn Leu Leu Asn Thr Gly Pro Asp His Leu Arg Pro Ala
    50                  55                  60
Ile Phe Arg Ser Leu Leu Tyr Glu Ala Cys Gly Arg Ile Val Asn Pro
65                  70                  75                  80
Ile Tyr Gly Ser Val Gly Leu Leu Trp Ser Gly Asn Trp His Leu Cys
                85                  90                  95
Gln Ala Ala Val Glu Ala Val Met Arg Gly Ser Pro Val Thr Pro Ile
            100                 105                 110
Ala Cys Asp Ala Ala Val Thr Gly Gln Ala Pro Pro Phe Asn Asn Lys
        115                 120                 125
Leu Cys Asp Ile Arg His Val Ser Ser Arg Asp Glu Asn Val Lys Arg
    130                 135                 140
Arg Ser Arg Gly Ala Cys Lys Glu Glu Arg Asn Val Arg Ser Leu Ser
145                 150                 155                 160
His Glu Ser Ser Leu Ser His Glu Ser Pro Val Ser Ser Glu Glu Thr
                165                 170                 175
Thr Thr Glu Glu Pro Lys Thr Trp Ile Gly Leu Glu Leu Thr Leu Gly
            180                 185                 190
Leu Glu Pro Leu Ala Arg Gly Asn His Val Val Val Pro Met Lys Lys
        195                 200                 205
Arg Lys Leu Glu Arg Cys Gly Thr Ser Glu Asp Glu Asp Thr Cys Lys
    210                 215                 220
Ile Glu Leu Gly Leu Val Cys Ser Glu
225                 230
```

<210> 57
<211> 263
<212> PRT
<213> Arabidopsis thaliana

<400> 57

```
Met Arg Met Ser Cys Asn Gly Cys Arg Val Leu Arg Lys Gly Cys Ser
1               5                   10                  15
Glu Asp Cys Ser Ile Arg Pro Cys Leu Ala Trp Ile Lys Ser Pro Glu
            20                  25                  30
Ala Gln Ala Asn Ala Thr Val Phe Leu Ala Lys Phe Tyr Gly Arg Ala
        35                  40                  45
Gly Leu Met Asn Leu Ile Asn Ala Gly Pro Asn His Leu Arg Pro Gly
    50                  55                  60
Ile Phe Arg Ser Leu Leu His Glu Ala Cys Gly Arg Ile Val Asn Pro
65                  70                  75                  80
Ile Tyr Gly Ser Val Gly Leu Leu Trp Ser Gly Asn Trp Gln Leu Cys
                85                  90                  95
Gln Asp Ala Val Glu Ala Val Met Lys Gly Glu Pro Val Lys Glu Ile
            100                 105                 110
Ala Thr Asp Ala Ala Thr Ile Gly Gln Gly Pro Pro Leu Lys Ile Tyr
        115                 120                 125
Asp Ile Arg His Ile Ser Lys Asp Asp Asn Ser Ala Ala Ala Ala Thr
    130                 135                 140
Gly Ser Thr Asp Leu Lys Leu Ala Lys Thr Arg Arg Ala Lys Arg Val
145                 150                 155                 160
Ser Thr Val Ala Ile Gln Ala Glu Ser Glu Gly Lys Ser Asp Glu Ala
                165                 170                 175
Ser His Asp Ser Ser Leu Ser His Gln Ser Glu Ile Val Ala Ala His
            180                 185                 190
Glu Gly Glu Ser Lys Glu Ser Glu Ser Asn Val Ser Glu Val Leu Ala
        195                 200                 205
Phe Ser Pro Pro Ala Val Lys Gly Ser Gly Glu Ile Lys Leu Asp Leu
    210                 215                 220
Thr Leu Arg Leu Glu Pro Val Ser Arg Ala Tyr His Val Val Pro Val
225                 230                 235                 240
Lys Lys Arg Arg Ile Gly Val Phe Gly Thr Cys Gln Lys Glu Ser Thr
                245                 250                 255
Cys Lys Thr Glu Leu Met Leu
            260
```

<210> 58
<211> 233
<212> PRT
<213> Arabidopsis thaliana

<400> 58

```
Met Arg Ile Ser Cys Asn Gly Cys Arg Val Leu Arg Lys Gly Cys Asn
1               5                   10                  15
Gln Asp Cys Thr Ile Arg Pro Cys Leu Gln Trp Ile Lys Ser Ala Asp
            20                  25                  30
Ser Gln Ala Asn Ala Thr Leu Phe Leu Ala Lys Phe Tyr Gly Arg Ala
        35                  40                  45
Gly Leu Leu Asn Leu Ile Glu Ser Gly Pro Asp His Leu Arg Pro Ala
    50                  55                  60
Ile Phe Arg Ser Leu Leu Tyr Glu Ala Cys Gly Arg Ile Val Asn Pro
65                  70                  75                  80
Val Asp Gly Ser Val Gly Leu Met Trp Ser Gly Asn Trp Ala Gln Cys
                85                  90                  95
Gln Ala Ala Val Asp Ala Val Leu Asn Gly Leu Pro Ile Thr His Thr
            100                 105                 110
Pro Leu Pro Ser Ala Ser Ala Ser His Gln Ile Ile Pro Pro His Arg
        115                 120                 125
Thr Tyr Asp Ile Arg His Val Ala Lys Asp Pro Thr Thr Gly Gly Asp
    130                 135                 140
Ser Ser Glu Asn Leu Ala Thr Arg Val Asn Ala Asn Lys Ser Lys Thr
145                 150                 155                 160
Gln Thr Gly Arg Phe Lys Arg Glu Ser Val Asn Gln Leu Gly Glu Cys
                165                 170                 175
Ser His Asp Met Trp Gln Leu Pro Ser Ser Ser Ala Thr His Gly Tyr
            180                 185                 190
Gly His Phe Thr Leu Glu Asn Val Glu Ser Arg Arg Glu Ala Pro Phe
        195                 200                 205
Asn Gln Ser Ser Pro Asn Leu Gly Phe Asp Asp Gln Val Asp Ile Asn
    210                 215                 220
Glu Val Gly Leu Glu Leu Arg Leu Gly
225                 230
```

<210> 59
<211> 924
<212> DNA
<213> Oryza sativa

<400> 59

```
atgcggatga gctgcaacgg gtgccgcgtg ctgcgcaagg ggtgcagcga caactgcgcc     60
atccgcccct gcctgcagtg gattcgcagc cccgatgcgc aggccaacgc caccgtcttc    120
ctcgccaagt tctacggccg cgccggcctc atcaacctca tcaccgccgg ccccgagcac    180
gtccgtcctg ccatattccg gtcgctgctg tacgaggctt gcgggcggat gctgaacccg    240
gtgtacggat ccgtcggcct cctctggtcg ggaactggc agctttgcca gtccgccgtc    300
gagtccgtcc tccgcggcat gcccatcgcg cagccgccgc cgtccgccac cgccgtgccc    360
ccgctcccca cctgcgacat ccgccacgtc ggcgccagga gaggcgacgt ccatggcgcc    420
gcggccggcc cggttgccga cctccaccgg cttgacatca gctcgcgcgc caagttcaag    480
aggcccggcg gcggcgccgc cgccgcccac aggtccgacc atgccgcctt cgagctcgtc    540
ttctccaagc ccgccgccgc catggccgtc gatgtgataa ggcaggcgca gccgctcaac    600
tgggcgcccg gggcgctcag ccacgagtcg gcgagccacg acgccgcgcc gccggagagc    660
gagggccaca gcaacgacac ggccgacacc gtggacggct cccacgtcag ccagtcggag    720
ccggagccga gagcgacgtc ggcggcgacc gaggtgcacg acgccggcct agacctcaca    780
ttaggtctac cgccgccgcc gccgccggtg caaaagaccg agccggcgga cagcgacggc    840
ggcagccagc agcaacacga tcatcggaag gagaagccgg tggagctcgg cttggcgatc    900
tcaactaaag tagcagctca atga                                           924
```

<210> 60
<211> 993
<212> DNA
<213> Oryza sativa

<400> 60

```
atgcggatga gttgcaacgg ttgccgggtg ctgcgcaagg gatgcagcga agggtgcacc     60
atccgtccgt gcctgcagtg gatcaagacc cccgaggcgc aggccaacgc caccgtcttc    120
ctcgccaagt tctacggccg cgccgggctt ctcaacctgc tcgctgccgg gcctgaccac    180
ctccgccccg ccgtcttccg ctcgctcctc tacgaggcct gcggccgcat cgtcaacccg    240
atctacggct ccgtcggcct gctctggtcg ggccagtggc aggcgtgcca ggccgccgtc    300
gaggccgtcc tcaagggcga ccccgtcgtg caggtctcct ctgaggccgc cgccgccgcc    360
caggccaccc cgccgctcag ggcctacgac atccgccacg tctccaagga cgccgaggcc    420
gacgccgccg ccaacctgct ccgcgtcgcg cgcggcggcc gcacccgctt caagcgcgcc    480
tcctccagct ccaactccaa gcacggcgcc aagcttgccg gagcagccgc cgccaagcgc    540
gccgcgtccc ccagcagcag cagcccgacg cacgagacgg agccggaggc ggtggtcgtc    600
gtcggtgacc atgacgacga ccaccaccac cctgcgctga gccacgaggt ccacgaggag    660
tcggctggca gccatgacca cgacgacgac gaccatgtgg acgacggtga caacaacgac    720
atggccatcg ccgacgtgac gccgccgcgg gcgggatcgg aggataccga ggtcgagacc    780
ggctcacacg tgagccaggc cgagcagagc cccgtgccgg tggagcacga agaggggag     840

gaggaggagg tcgggctgga gctcacgctc gggttccagc cgctcgtcgt gcgcgcctcg    900
aggaggcctt cgtcggcgga ggcgcgctgc gaccttagcg gcttgagcgc ggagtcgagt    960
cgcatcggcc tgcggctcga gctgccagcg tga                                 993
```

<210> 61
<211> 615
<212> DNA
<213> Oryza sativa

<400> 61

```
atgagctgca acggttgccg ggtgctgcgg aaggggtgca gtgacggctg cgtgctgcgg     60
ccatgcctgc agtggatcga cgccgccgac gcgcagggcc acgccaccgt cttcgtcgcc    120
aagttcttcg gccgcgccgg actcctctcc ttcatctccg ctgtccccga ggcgcagcga    180
cctgcgttgt tccagtcgct gctgtacgaa gcagcggggc gaaccatcaa tccggtgcac    240
ggcgcggtgg gcctcctctg gacggggaac tggcccctct gccaggccgc tgtcgagacc    300
gtgctgcgtg gaggcgccat cggccctctg ccggagctcg gcggggcctg cggcggcgct    360
ggcggggacc tctacggcgc cgccaagcgc aacggcggct ggtctacctt ctccactgcg    420

aagcgggtga ggaaggccga ggtacctgaa gccccgtcgt gcgacctagg cctgtgcctc    480
agccccggct ctccgccggc ggtgggggag aggaaaccag cactgcggcc ggggacgccg    540
tcaatgagct ccgatgagtc cggcaccaca accggaggcg aaagggatcc tgtgctgctc    600
aacctttttg tctga                                                     615
```

<210> 62
<211> 699
<212> DNA
<213> Oryza sativa

<400> 62

```
atgagctgca acgggtgccg cgtgctgcgg aaggggtgca gcgacgcgtg cgtgctgcgg     60
ccgagcatcg agtggatcga cggcgcgcag ccgcaggcca acgccaccgt cttcgtcgcc    120
aagttcttcg gccgcgccgg cctcgtcgcc tccctcgccg ccgtccctct ccaccatcgt    180
ccagcgctgt tccggtcgct tctgtacgag gcgtgcgggc ggacgataaa cccggtgagc    240
ggcgccatcg ggctcatgtg gacgggcaac tgggacctct gccaggccgc cgccgacgcc    300
gtcctccgcg gcgactccct ccgcgccctc tcggccgtgc cagccgcctt caccgaccgc    360
gacatggccg gcctctacgg caacgtcggc gccgccgccg ggacctcctc ctcctcgccc    420
gacaacgaca actcctccgc ctccgccgcg ccccggagga agcggcccag gaataacggc    480
gccggcgccg gcgtcggtca gcagcagctg ccgcacgcgg tggctgcggt gctccagtcc    540
tgcgagctgg acctctgcct cacgcccgtc tcaccgccgg ccgtgcagct ggtgggtggg    600
ggcggcggcg cgtcggacga gcactcgacg acgacgtgcg aggaagccag cgacggggat    660
ggcgccgggg cgcccacgct gctgaacctc ttcagctga                           699
```

<210> 63
<211> 681
<212> DNA
<213> Oryza sativa

<400> 63

```
atgagctgca acggctgccg cgtgctgcgg aaggggtgca gcgaggggtg cgtgttgcgg     60
ccgtgcctgc agtggatcga cggcgccgag gcgcagggcc acgccaccgt gttcgtcgcc    120
aagttctttg gccgcgccgg cctcatgtcc ttcctcaccg ccgtccccga gccgcagcgc    180
gcagcggtgt tccagtcgct gctgtacgag gcggcgggga ggacgatcaa cccggtgggc    240
ggcgcggtcg ggctgctctc cggcgggagc tggcacctct gccaggcggc ggtcgacacc    300
gtgctgcgcg gcggaggcat ccaaccgctg ccggaccagg tcgacgccgc cgccgccggt    360
ggccgcgacg tgttcgcctc cacggcgagg cgcgccatgg gcgggtgctc cacgttctcg    420
acggcgaagc ggtcgacgac gacgacgacg accaagaacc ccggtactcc gcatgacgcc    480
gccgccgcgg cgccccagcc ggaaccgtcg tgcgacctcg gcctgtggct cagccccggc    540
tccccgccgg cgcccgggga ccggcgatcc ggcggcaggc gggccgacac gccgtcgatg    600
aactccgagg gatccgtcac gacgtgcggc gtcgtcggcg acggcgagag ggagcccgag    660
ctgctgaatc tttttgtcta g                                               681
```

<210> 64
<211> 1036
<212> DNA
<213> Oryza sativa

<400> 64

```
aaacattgaa acactttgtc ccactctctc tctttctctt tcttgtacca aaagcttttt     60
gaatctccaa gattatagca aaaccaaaga taaaatacta acttaaaaag atttctgaaa    120
ataatgagtt gcaatggttg tcgagttcta cgaaaaggtt gcagtgagaa ttgcatcctc    180
cgtccatgta ttcaatggat cgaatcacct gaagctcaag gccacgccac cgtcttcgtc    240
gctaagttct tcggccgtgc cggtttaatg tctttcatct ccgccgtacc ggaatctcaa    300
tgccctgctt tgtttcagtc tttgctatac gaagcttgtg ggagaactgt gaatccggtg    360
aacggagccg tcggattgtt gtggacgggg aattggaatg tttgtcaagc ggcggttgag    420
acggtgcttc gtggtggttc tttaaaacca ataccggagc ttcttaacgg cggtggattc    480
gccgggtttc cgtctcctac ttccgacgaa gcttcggaga tctgtacgga aatgttgaat    540
ctacgaaaag ctgatgattc cggtgatcgg aacatttatc atcactgccg attctcaagc    600
tctagatcta gatcaagatc aacagcttct ccgccgaaac ggaaacgatt atcgtcggaa    660
caacaacctt cgtcggagct tgatctctct cttattccta tttatccgat taaaccttg     720

ccgtttaagg aagatacacc gtcgatgtac tcggaggagt ctgttaccac ggtttcgttt    780
caaaacaaca acgccggtga tcggtacgta cgctgcggcg gaggaggagg aggagcaacg    840
acaaagttgc tcaatctctt cgcttgaacg gcgtcgtttt tgttatgagg gttaaaaatg    900
taatttcgag taactttttg ttgtggcctt ttttggatta atagccctta atattttgtg    960
aaatgtagca aattacgtga tttatttctc tctttaatta tgagaaatta tccaaagtaa   1020
tagagaataa tgatat                                                   1036
```

<210> 65
<211> 996
<212> DNA
<213> Oryza sativa

<400> 65

```
gtttttctt tcccttcttc aatcaaaacc tatttgcatg ctctcaaacc cgaattaaat      60
cgacactttt cagtttttgt tttaacaagt agagtttccc aaaatattgg atatatttct    120
ttttcaaatt tcggaaaaga aatgagttgc aatggatgta gagttcttcg aaaaggttgc    180
agtgaaacat gcatccttcg tccttgcctt caatggatcg aatccgccga gtcacaaggc    240
cacgccaccg tcttcgtcgc taaattcttt ggtcgtgctg gtctcatgtc tttcatctcc    300
tccgtacctg aactccaacg tcctgctttg tttcagtcgt tgttgtttga agcgtgtggg    360
agaacggtga atccggttaa cggagcggtt ggtatgttgt ggaccaggaa ctggcacgta    420
tgccaagcgg cggttgagac tgttcttcgc ggcggaactt tacgaccgat atcagatctt    480
cttgaatctc cgtcgttgat gatctcctgt gatgagtctt cagagatttg gcatcaagac    540
gtttcaagaa accaaaccca ccattgtcgc ttctccacct ccagatccac gacggagatg    600
aaagactctc tggttaaccg aaaacgattg aagtccgatt cggatcttga tctccaagtg    660
aaccacggtt taaccctaac cgctccggct gtaccggttc cttttcttcc tccgtcgtcg    720
ttttgtaagg tggttaaggg tgatcgtccg ggaagtccat cggaggaatc tgtaacgacg    780
tcgtgttggg aaaatgggat gagaggagat aataaacaaa aaagaaacaa aggagagaaa    840
aagttattga accttttgt ttaaaaccga cgacgcaaaa cactcaaaga ttttgaggct     900
ctcttttta gggttttgag tgggaatgga tatttagtta atgattttc tctatcgaga      960
aatatgataa aattttgggg aaaaaaaaaa aaaaaa                              996
```

<210> 66
<211> 804
<212> DNA
<213> Oryza sativa

<400> 66

```
ctcaaaccca aaacacattt tgtttttgt ttcccatcta aaatgcgtat gagttgtaac      60
ggatgtcgag ttctacgaaa aggctgcagt gaaaactgta gcataagacc ttgtcttcaa    120
tggatcaaat ccgctgaatc tcaagccaac gccaccgtct tcctcgccaa gttttatggc    180
cgtgcggggc tcatgaacct cctcaacacc ggtcctgacc acctccgtcc tgcgatattt    240
agatcattgt tgtacgaagc atgcgggagg attgtgaatc cgatatacgg atccgtgggg    300
ttattgtggt caggaaactg gcatctttgt caagcggccg ttgaggcagt aatgagaggc    360
tcacccgtga ctccgatcgc atgcgacgca gcggttactg gtcaagcacc tccttttaac    420
aacaagcttt gcgacataag acacgtgtca agcagggacg agaacgttaa gagacggagc    480
cgtggtgcat gcaaggaaga gagaaacgtg aggtctttga gtcatgagtc gtcactgagt    540
cacgagtcac cggtgtcttc tgaggagacg acgacggagg aaccaaagac ttggatcggg    600
cttgagctga ctttggggtt ggagccttta gcacgtggaa atcacgtggt ggtaccgatg    660
aagaaaagaa agttagagag gtgtggcacg tctgaggatg aggacacgtg taagattgag    720
cttggactgg tgtgcagtga gtgaatggtt cttttttgt ggctggtctt aattacaagt     780
tttggtgttg agttttaggt gtac                                           804
```

<210> 67
<211> 1217
<212> DNA
<213> Oryza sativa

<400> 67

```
ctctctccgc ttcccccaaa aatccagggg cttcttacac gcacacaatc ctttaaagct     60
atcgtctttc ttatataatc agtgaatctt cattaacacc tcccacaaaa tctcagaaat    120
aactttcaca acagagtcaa agagttccaa atcgtttgtt tgtattttgt acgatcaaag    180
```

```
ttgttggtac ttgtaagata atcgaaacca aagatgcgga tgagctgtaa tggatgcaga     240
gttcttcgga aagggtgtag tgaggattgt agtataaggc cgtgtttggc ttggatcaaa     300
tcgcctgaag cgcaagctaa cgcaactgtc tttctcgcca agttctatgg ccgtgctgga     360
ctcatgaacc tcatcaacgc cggtcccaat caccttcgtc ctgggatttt ccgatcgttg     420
ttgcacgaag cttgtgggag gattgtgaat ccgatctatg gttcggtggg tttgttgtgg     480
tcggggaatt ggcagctttg tcaagacgcc gtggaggctg tgatgaaagg agaaccggtc     540
aaagagatcg ccacagacgc tgcgacgatc ggccaaggtc cgcctcttaa gatctacgac     600
atccgacata tctccaagga tgataactct gccgccgcgg ctactggctc aaccgatttg     660
aaacttgcga aaactcgccg tgctaagcgg gtctccaccg tcgcgataca ggcggaatcg     720
gagggaaagt ctgacgaggc tagtcacgat tcgtcgttga gtcatcagtc tgaaatagtg     780
gctgctcatg aaggagagag caaggaatcc gagagcaatg tctctgaggt tttggcattc     840
tcgcctccgg ctgtgaaggg ctccggcgag ataaagcttg acctaacttt aaggctcgaa     900
ccggtgtcac gtgcgtatca tgtggtacct gttaagaaga gaaggatcgg cgtgtttggc     960
acgtgtcaga aggagagcac gtgtaagact gagcttatgc tctaagctcc gttttagcta    1020
tactcagaag ttttgccccc atcagctcta ttcggctcgg ctctgatgat cttcaagtcg    1080
agccggtaac tttttttaa tatatagttc tctatttccc cctttaatgt tcactttcca    1140
tttgtgacaa aagaatagag tatatgtaca aaattattta cagagattaa ttcaagaatt    1200
ggctgagata ttatcta                                                   1217
```

<210> 68
<211> 983
<212> DNA
<213> Oryza sativa

<400> 68

```
caaaagttga aaaataccag ctcattcact tatgactcat gagtaacttc ttccttataa      60
attcaaattg ttcacaactt ccttgagttc catttccgaa cacaaacaca catcatacgt     120
atatttgtca ctaataaaca tataaaggaa tatgagaatc agctgcaacg ggtgtagggt     180
tctacgcaaa ggttgcaacc aagattgcac cattcgacca tgccttcaat ggatcaaatc     240
cgcagactct caagccaatg ctacactctt ccttgctaag ttctacggtc gagccggtct     300
tcttaacctc atcgaatccg gtcctgatca ccttcgtccc gcaatattta ggtcacttct     360
gtacgaggct tgtggtcgga tcgtgaaccc tgtagatggt tcggttggtt tgatgtggtc     420
agggaactgg gctcagtgcc aagcagctgt tgacgccgtg ctcaacggct tacccataac     480
tcacacacct cttccaagtg catccgcgtc gcaccagatc attcctcccc acaggactta     540
cgatatacgc cacgtggcta aagatccaac aaccggcggc gacagttcgg agaatctggc     600
cacacgtgta aacgctaaca agagcaagac gcaaactggc cgattcaaac gtgaatccgt     660
gaaccaacta ggtgaatgta gtcatgacat gtggcaactc ccaagttcta gtgctacgca     720
tggttatggc cacttcacgt tggagaatgt ggagagccga agggaagctc catttaatca     780
aagttctcca aatcttgggt ttgatgatca agtcgatatc aacgaggttg gcttagagct     840
cagacttggt tagaaccaga agattgcatt attattatca taattctgtc tttttaatta     900
ataatttcac tttctatata aaaggagaaa aaaaaactaa agcaaatcaa atttagtatt     960
attgtcaatc attaggttat cat                                            983
```

<210> 69
<211> 925
<212> DNA
<213> Linum usitatissimum

<400> 69

```
gtctccaacc cccaaacact caaaaaatta tagaaaatta ccagccgccg gaagcagagg     60
tgatcggagg atgagctgca acggttgcag agtactgagg aaaggatgtg gagagaattg    120
cgtgctgagg tcatccctcc gatggattcc cactcctcaa gcacaaggca acccactttg    180
ttcctcgcca aattcttcgg ccgcagcgac ctcctctcct ttatctcctc cgtccccgat    240
cctcaacgcc ccgctctgtt ccagtcgctg ctatttgaag cgtgtgggag gacggtgaac    300
ccggttaacg gggcggtggg gctgctgtgg agccgcaatt ggcacgtgtg tcaggctgcg    360
gtcgagaccg ttctcgccgg aggatcactg catccgcttc cgggaattgc ggcaggggtt    420
gcgccgccga atttcgacga gtcggatgac agctgctccg ccgccggatt cgctgctaat    480
cagctccgcc cggcgatttt ggcgattact ggcgggaatc ggaatctatc atcgtcctcc    540
tccacctcgg agtatcgacg tcgtcataat gctgctctgg tcggtggtgg gaatgagagg    600
aggattaata acagagatgc gagggcgtcg tttttgcgg aggaatcgga aacgacgaca     660
ttcgagagca ccggcggcga tcggaagaag ctgctgaatc tttttgtttg agtagagagg    720

aggaaagaaa aaagagaagc cgagtttttg tcggcaaagg aaagaacaag acttgaatgc    780
ggttactgga atgtgaactg tactttgggt ttctgtgttt tgtttcttat gaactttata    840
tgataatata tatacataag agagagaaag aaagaacgcc ggaatgacag tccggcgagt    900

tttgggggaa aaaaaaaaaa aaaaa                                           925
```

<210> 70
<211> 201
<212> PRT
<213> Linum usitatissimum

<400> 70

```
Met Trp Arg Glu Leu Arg Ala Glu Val Ile Pro Pro Met Asp Ser His
1               5                   10                  15
Ser Ser Ser Thr Arg Gln Pro Thr Leu Phe Leu Ala Lys Phe Phe Gly
            20                  25                  30
Arg Ser Asp Leu Leu Ser Phe Ile Ser Ser Val Pro Asp Pro Gln Arg
        35                  40                  45
Pro Ala Leu Phe Gln Ser Leu Leu Phe Glu Ala Cys Gly Arg Thr Val
    50                  55                  60
Asn Pro Val Asn Gly Ala Val Gly Leu Leu Trp Ser Arg Asn Trp His
65                  70                  75                  80
Val Cys Gln Ala Ala Val Glu Thr Val Leu Ala Gly Gly Ser Leu His
                85                  90                  95
Pro Leu Pro Gly Ile Ala Ala Gly Val Ala Pro Pro Asn Phe Asp Glu
            100                 105                 110
Ser Asp Asp Ser Cys Ser Ala Ala Gly Phe Ala Ala Asn Gln Leu Arg
        115                 120                 125
Pro Ala Ile Leu Ala Ile Thr Gly Gly Asn Arg Asn Leu Ser Ser Ser
    130                 135                 140
Ser Ser Thr Ser Glu Tyr Arg Arg Arg His Asn Ala Ala Leu Val Gly
145                 150                 155                 160
Gly Gly Asn Glu Arg Arg Ile Asn Asn Arg Asp Ala Arg Ala Ser Phe
                165                 170                 175
Phe Ala Glu Glu Ser Glu Thr Thr Thr Phe Glu Ser Thr Gly Gly Asp
            180                 185                 190
Arg Lys Lys Leu Leu Asn Leu Phe Val
        195                 200
```

<210> 71
<211> 894
<212> DNA
<213> Triticum aestivum

<400> 71

```
atgcggctta gctgcaacgg ctgccgcgtg ctgcggaagg gctgcagcga ggactgcagc     60
atccgcccct gcctgcagtg gatcaagagc cccgaggcgc aggccaacgc caccgtcttc    120
ctcgccaagt tctacggccg cgcggggctc atgaacctca tcaacgccgg cacggacgac    180
agcctccgcc ccggcatctt ccgctcgctc ctctacgagg cctgcggccg gatcgtcaac    240
cccatctacg gctccgtcgg cctgctctgg tccaacaact ggcagatgtg ccaggccgcc    300
gtcgaggccg tcctcagcgg caagcccatc gtccaggtct cctccgagga tgccgccgcc    360
gaccggacac cgccgctcaa ggcgtacgac atccgccacg tctccacctc gccagccgcc    420
gacgggaggc tccacaaggt cgccaagccg ggtcgcaccc gcttcaagcg cgcgtcctcc    480
gcctcgtccc accacaaccc gtccagcgac tccaacaaca agcccaagcc caagccgcag    540
cctcagacgc gggcgcccac ggcggaggag gagcgggatc gtcaacaccg caaggagatg    600
gaggagggcg cgttccagcg tgctccgagc cacgaatcct ccgacagccg acacgaggac    660
cccgtggagc cacactgcca gcaagaggcg tccgcggaca cggaggccga ggcggggtcg    720
cacgtcagcc aggcagagca ggagcagagc acagagccgg ccgcagagca cgcggaggag    780
gtcgaaaagg aggacgaaga actagggctc gagctcacgc ttggattcgc tcccgtcgca    840
gcacggcctg ccgaattcac ctgaccgtcc gaaccggggg gagcggcttt cgcg          894
```

<210> 72
<211> 1068
<212> DNA
<213> Medicago truncatula

<400> 72

```
atgagttgca atgggtgccg agttcttcga aaaggttgca gtgagtcgtg tattttacga     60
ccttgtttgc aatggatcga aactccagaa gctcaaggcc acgctactgt tttcgtagca    120
aaattctttg gtcgggctgg tctcatgtcc ttcatttcca acgtccctga accacagaga    180
ccagctctgt ttcagtctct actgtttgaa gcgtgtggga gaacggttaa ccctgtgaac    240
ggtgctgttg gacttctttg gactggaaac tggcacgtgt gtcaagcagc agttgagacg    300
gttcttcgcg gtggcacgct aaggccttta ccggagctga cacttctgga cgcgcctctc    360
actgccaccg atgaagcttc tgaggcggaa gacggtggtg ctgatgctat gtggaagatc    420
cgagaaccga atttcgggtt ggcgagctca cgatctagca ataaggtgtg ttctggaagc    480
aaacggagga gatcggagga gtttgtcaag gttccggcgg cgattaatct tgatttacga    540
ttgacgccga tttttcaaca gaaggcggtg gaggaacgcc ggcatggttc gccgtcgatg    600
acgtcggagg aatcggtgac gacgacggcg tgtttggaga ctgggattgg agatcggtgg    660
agtcacggtg gtgatcggaa agttctcaac cttttcatat gaggattttc tgtatttgtg    720
ttgagcgtga ctctcacgcg ccgtaaagtt tttctgtttt gctcacaata ctcattgtgg    780
tgaaaagagt aaaacggttt tgtagttcgg tgaattttcg tgagttaccg gttcaggatt    840
tctctagaga gaaagatgat ggttcgtcgg tgattttttt ctttttgctg ctaaatatga    900
gatgtgtaaa cctgtaaccg tggaatatca aatattaaat caaattaaat aaatagtttt    960
ttttttttta atcatcgctg tttgaaattt agtagtagtt ggataaacat tcagttatta   1020
tagttcaatt tgaacttcat atattttaat catataaact caatatta                1068
```

<210> 73
<211> 1257
<212> DNA
<213> Arabidopsis thaliana

<400> 73

```
atgtcaggag ctaccaccgc ttcctccgga gaccacaaca acctccgtct cccaactccg     60
acgcttgacg ccgaatcgca aactctgctg caatcgattt ctgctgaagg gggttacgct    120
tatgctcgca tggcggtgtt agcggtggcc ggtgaccaaa gcgcggcgga ggcggcgcgt    180
gatatggctt gggagcaact tcactctggt ccgtggcact cggttctccc cgtttggcgc    240
gacgcttact caatggcttg tctccacgta gctaaaatcc atttcgccgc cggcgagttt    300
ggtgaggcgc ttggtgcgct tgatatgggt cttatcatgg gaggtatgct tctccgcaag    360
gatcttcatg attctgtttt gttggtctcc tctgaagctc gtaagatgac gaagagtctt    420
gaagaagctt ctggagattt caaaggtgag agattggtcc cggaagttcc cgtcgacgtc    480
aatgaggtcc ttaagattct accttgcagg tctttaactt gtaaaagagt agagaagagg    540
tctggtttat ctttggaggg gtttctgcgt gattattatc tgccaggtac accagttgtt    600
ataaccaatt ctatggctca ttggccagcc aggaccaagt ggaatcactt ggactacctc    660
aatgctgttg ctggtaaccg caccgttccc gtggaggtgg ggaaaaacta tttgtgttct    720
gattggaagc aagagcttgt gacattttct aagttccttg aacggatgcg gaccaataaa    780
tcgtctccaa tggagcctac ttatcttgcc cagcatcctt tgtttgatca gataaatgaa    840
ctgagagatg atatatgtat tcctgattac tgttttgtcg gtggtgggga actccaatca    900
cttaatgcat ggtttggccc ggctgggaca gttactccgt tacaccatga tccacatcat    960
aatatacttg ctcaggttgt tggcaagaag tatataaggc tttacccatc cttcctgcaa   1020
gacgaacttt acccttactc tgagacaatg ctctgcaact ctagtcaggt tgatctagac   1080
aatatcgacg aaaccgagtt cccaaaggcc atggagttgg agtttatgga ttgtattcta   1140
gaagaaggtg aaatgttgta catccctccc aaatggtggc actatgtcag gtccttaaca   1200
atgagtctct cggttagctt ctggtggagc aatgaagcag aatcttctag ctcgtag      1257
```

<210> 74
<211> 418
<212> PRT
<213> Arabidopsis thaliana

<400> 74

```
Met Ser Gly Ala Thr Thr Ala Ser Ser Gly Asp His Asn Asn Leu Arg
1               5                   10                  15
```

```
Leu Pro Thr Pro Thr Leu Asp Ala Glu Ser Gln Thr Leu Leu Gln Ser
            20                  25                  30
Ile Ser Ala Glu Gly Gly Tyr Ala Tyr Ala Arg Met Ala Val Leu Ala
        35                  40                  45
Val Ala Gly Asp Gln Ser Ala Ala Glu Ala Ala Arg Asp Met Ala Trp
    50                  55                  60
Glu Gln Leu His Ser Gly Pro Trp His Ser Val Leu Pro Val Trp Arg
65                  70                  75                  80
Asp Ala Tyr Ser Met Ala Cys Leu His Val Ala Lys Ile His Phe Ala
                85                  90                  95
Ala Gly Glu Phe Gly Glu Ala Leu Gly Ala Leu Asp Met Gly Leu Ile
            100                 105                 110
Met Gly Gly Met Leu Leu Arg Lys Asp Leu His Asp Ser Val Leu Leu
        115                 120                 125
Val Ser Ser Glu Ala Arg Lys Met Thr Lys Ser Leu Glu Glu Ala Ser
    130                 135                 140
Gly Asp Phe Lys Gly Glu Arg Leu Val Pro Glu Val Pro Val Asp Val
145                 150                 155                 160
Asn Glu Val Leu Lys Ile Leu Pro Cys Arg Ser Leu Thr Cys Lys Arg
                165                 170                 175
Val Glu Lys Arg Ser Gly Leu Ser Leu Glu Gly Phe Leu Arg Asp Tyr
            180                 185                 190
Tyr Leu Pro Gly Thr Pro Val Val Ile Thr Asn Ser Met Ala His Trp
        195                 200                 205
Pro Ala Arg Thr Lys Trp Asn His Leu Asp Tyr Leu Asn Ala Val Ala
    210                 215                 220
Gly Asn Arg Thr Val Pro Val Glu Val Gly Lys Asn Tyr Leu Cys Ser
225                 230                 235                 240
Asp Trp Lys Gln Glu Leu Val Thr Phe Ser Lys Phe Leu Glu Arg Met
                245                 250                 255
Arg Thr Asn Lys Ser Ser Pro Met Glu Pro Thr Tyr Leu Ala Gln His
            260                 265                 270
Pro Leu Phe Asp Gln Ile Asn Glu Leu Arg Asp Asp Ile Cys Ile Pro
        275                 280                 285
Asp Tyr Cys Phe Val Gly Gly Gly Glu Leu Gln Ser Leu Asn Ala Trp
    290                 295                 300
Phe Gly Pro Ala Gly Thr Val Thr Pro Leu His His Asp Pro His His
305                 310                 315                 320
Asn Ile Leu Ala Gln Val Val Gly Lys Lys Tyr Ile Arg Leu Tyr Pro
                325                 330                 335
Ser Phe Leu Gln Asp Glu Leu Tyr Pro Tyr Ser Glu Thr Met Leu Cys

            340                 345                 350
Asn Ser Ser Gln Val Asp Leu Asp Asn Ile Asp Glu Thr Glu Phe Pro
        355                 360                 365
Lys Ala Met Glu Leu Glu Phe Met Asp Cys Ile Leu Glu Glu Gly Glu
    370                 375                 380
Met Leu Tyr Ile Pro Pro Lys Trp Trp His Tyr Val Arg Ser Leu Thr
385                 390                 395                 400
Met Ser Leu Ser Val Ser Phe Trp Trp Ser Asn Glu Ala Glu Ser Ser
                405                 410                 415
Ser Ser
```

<210> 75
<211> 2194
<212> DNA
<213> Oryza sativa

<400> 75

```
aatccgaaaa gtttctgcac cgttttcacc ccctaactaa caatataggg aacgtgtgct      60
```

```
aaatataaaa tgagacctta tatatgtagc gctgataact agaactatgc aagaaaaact     120
catccaccta ctttagtggc aatcgggcta aataaaaaag agtcgctaca ctagtttcgt     180
tttccttagt aattaagtgg gaaaatgaaa tcattattgc ttagaatata cgttcacatc     240
tctgtcatga agttaaatta ttcgaggtag ccataattgt catcaaactc ttcttgaata     300
aaaaaatctt tctagctgaa ctcaatgggt aaagagagag atttttttta aaaaaataga     360
atgaagatat tctgaacgta ttggcaaaga tttaaacata taattatata attttatagt     420
ttgtgcattc gtcatatcgc acatcattaa ggacatgtct tactccatcc caatttttat     480
ttagtaatta aagacaattg acttattttt attatttatc tttttcgat tagatgcaag      540
gtacttacgc acacactttg tgctcatgtg catgtgtgag tgcacctcct caatacacgt     600
tcaactagca acacatctct aatatcactc gcctatttaa tacatttagg tagcaatatc     660
tgaattcaag cactccacca tcaccagacc acttttaata atatctaaaa tacaaaaaat     720
aattttacag aatagcatga aaagtatgaa acgaactatt taggtttttc acatacaaaa     780
aaaaaaagaa ttttgctcgt gcgcgagcgc caatctccca tattgggcac acaggcaaca     840
acagagtggc tgcccacaga acaacccaca aaaaacgatg atctaacgga ggacagcaag     900
tccgcaacaa ccttttaaca gcaggctttg cggccaggag agaggaggag aggcaaagaa     960
aaccaagcat cctccttctc ccatctataa attcctcccc cctttcccc tctctatata     1020
ggaggcatcc aagccaagaa gagggagagc accaaggaca cgcgactagc agaagccgag    1080
cgaccgcctt ctcgatccat atcttccggt cgagttcttg gtcgatctct tccctcctcc    1140
acctcctcct cacagggtat gtgcctccct tcggttgttc ttggatttat tgttctaggt    1200
tgtgtagtac gggcgttgat gttaggaaag gggatctgta tctgtgatga ttcctgttct    1260
tggatttggg atagaggggt tcttgatgtt gcatgttatc ggttcggttt gattagtagt    1320
atggttttca atcgtctgga gagctctatg gaaatgaaat ggtttaggga tcggaatctt    1380
gcgattttgt gagtaccttt tgtttgaggt aaaatcagag caccggtgat tttgcttggt    1440
gtaataaagt acggttgttt ggtcctcgat tctggtagtg atgcttctcg atttgacgaa    1500
gctatccttt gtttattccc tattgaacaa aaataatcca actttgaaga cggtcccgtt    1560
gatgagattg aatgattgat tcttaagcct gtccaaaatt tcgcagctgg cttgtttaga    1620
tacagtagtc cccatcacga aattcatgga aacagttata atcctcagga acaggggatt    1680
ccctgttctt ccgatttgct ttagtcccag aatttttttt cccaaatatc ttaaaaagtc    1740
actttctggt tcagttcaat gaattgattg ctacaaataa tgcttttata gcgttatcct    1800
agctgtagtt cagttaatag gtaatacccc tatagtttag tcaggagaag aacttatccg    1860
atttctgatc tccattttta attatatgaa atgaactgta gcataagcag tattcatttg    1920
gattattttt tttattagct ctcacccctt cattattctg agctgaaagt ctggcatgaa    1980
ctgtcctcaa ttttgtttc aaattcacat cgattatcta tgcattatcc tcttgtatct    2040
acctgtagaa gtttcttttt ggttattcct tgactgcttg attacagaaa gaaatttatg    2100
aagctgtaat cgggatagtt atactgcttg ttcttatgat tcatttcctt tgtgcagttc    2160
ttggtgtagc ttgccacttt caccagcaaa gttc                                 2194
```

<210> 76
<211> 54
<212> DNA
<213> Artificial sequence

<220>
<223> primer 1

<400> 76
ggggacaagt ttgtacaaaa aagcaggctt aaacaatgtc aggagctacc accg 54

<210> 77
<211> 50
<212> DNA
<213> Artificial sequence

<220>
<223> primer 2

<400> 77
ggggaccact ttgtacaaga aagctgggta aaataaatcc attcgctacg 50

<210> 78
<211> 152

<212> PRT
<213> Artificial sequence

<220>
<223> JMJ-C domain of SEQ ID NO: 02

<400> 78

```
Thr Tyr Leu Ala Gln His Pro Leu Phe Asp Gln Ile Asn Glu Leu Arg
1               5                   10                  15
Asp Asp Ile Cys Ile Pro Asp Tyr Cys Phe Val Gly Gly Gly Glu Leu
            20                  25                  30
Gln Ser Leu Asn Ala Trp Phe Gly Pro Ala Gly Thr Val Thr Pro Leu
        35                  40                  45
His His Asp Pro His His Asn Ile Leu Ala Gln Val Val Gly Lys Lys
    50                  55                  60
Tyr Ile Arg Leu Tyr Pro Ser Phe Leu Gln Asp Glu Leu Tyr Pro Tyr
65                  70                  75                  80
Ser Glu Thr Met Leu Cys Asn Ser Ser Gln Val Asp Leu Asp Asn Ile
                85                  90                  95
Asp Glu Thr Glu Phe Pro Lys Ala Met Glu Leu Glu Phe Met Asp Cys
            100                 105                 110
Ile Leu Glu Glu Gly Glu Met Leu Tyr Ile Pro Pro Lys Trp Trp His
        115                 120                 125
Tyr Val Arg Ser Leu Thr Met Ser Leu Ser Val Ser Phe Trp Trp Ser
    130                 135                 140
Asn Glu Ala Glu Ser Ser Ser Ser
145                 150
```

<210> 79
<211> 32
<212> PRT
<213> Artificial sequence

<220>
<223> conserved box in JMJ-C domain of SEQ ID NO: 02

<400> 79

```
Cys Ile Leu Glu Glu Gly Glu Met Leu Tyr Ile Pro Pro Lys Trp Trp
1               5                   10                  15
His Tyr Val Arg Ser Leu Thr Met Ser Leu Ser Val Ser Phe Trp Trp
            20                  25                  30
```

<210> 80
<211> 23
<212> PRT
<213> Artificial sequence

<220>
<223> DUF335 domain

<400> 80

```
Arg Lys Met Thr Lys Ser Leu Glu Glu Ala Ser Gly Asp Phe Lys Gly
1               5                   10                  15
Glu Arg Leu Val Pro Glu Val
            20
```

<210> 81
<211> 17
<212> PRT
<213> Artificial sequence

<220>
<223> conserved motif

<220>
<221> UNSURE
<222> (11)..(11)
<223> Xaa can be any naturally occurring amino acid

<400> 81

```
Ala Leu Asp Met Gly Leu Ile Met Gly Gly Xaa Leu Leu Arg Lys Asp
1               5                   10                  15
Leu
```

<210> 82
<211> 3
<212> PRT
<213> Artificial sequence

<220>
<223> FE(II) binding consensus seq

<220>
<221> UNSURE
<222> (2)..(2)
<223> Xaa can be any naturally occurring amino acid

<220>
<221> VARIANT
<222> (3)..(3)
<223> /replace = "Val"

<400> 82

```
His Xaa Asp
1
```

<210> 83
<211> 1290
<212> DNA
<213> Arabidopsis thaliana

<400> 83

```
atgtcaggag ctaccaccgc ttcctccgga gaccacaaca acctccgtct cccaactccg     60
acgcttgacg ccgaatcgca aactctgctg caatcgattt ctgctgaagg gggttacgct    120
tatgctcgca tggcggtgtt agcggtggcc ggtgaccaaa gcgcggcgga ggcggcgcgt    180
gatatggctt gggagcaact tcactctggt ccgtggcact cggttctccc cgtttggcgc    240
gacgcttact caatggcttg tctccacgta gctaaaatcc atttcgccgc cggcgagttt    300
ggtgaggcgc ttggtgcgct tgatatgggt cttatcatgg gaggtatgct tctccgcaag    360
gatcttcatg attctgtttt gttggtctcc tctgaagctc gtaagatgac gaagagtctt    420
gaagaagctt ctggagattt caaaggtgag agattggtcc cggaagttcc cgtcgacgtc    480
aatgaggtga gacatgtttt ggctaatcta caattacttg tccttaagat tctaccttgc    540
aggtctttaa cttgtaaaag agtagagaag aggtctggtt tatctttgga ggggtttctg    600
cgtgattatt atctgccagg tacaccagtt gttataacca attctatggc tcattggcca    660
gccaggacca agtggaatca cttggactac ctcaatgctg ttgctggtaa ccgcaccgtt    720
cccgtggagg tggggaaaaa ctatttgtgt tctgattgga agcaagagct tgtgacattt    780
tctaagttcc ttgaacggat gcggaccaat aaatcgtctc caatggagcc tacttatctt    840
gcccagcatc ctttgtttga tcagataaat gaactgagag atgatatatg tattcctgat    900
tactgttttg tcggtggtgg ggaactccaa tcacttaatg catggtttgg cccggctggg    960
acagttactc cgttacacca tgatccacat cataatatac ttgctcaggt tgttggcaag   1020
aagtatataa ggctttaccc atccttcctg caagacgaac tttaccctta ctctgagaca   1080

atgctctgca actctagtca ggttgatcta gacaatatcg acgaaaccga gttcccaaag   1140
gccatggagt tggagtttat ggattgtatt ctagaagaag gtgaaatgtt gtacatccct   1200
cccaaatggt ggcactatgt caggtcctta acaatgagtc tctcggttag cttctggtgg   1260
agcaatgaag cagaatcttc tagctcgtag                                    1290
```

<210> 84
<211> 429
<212> PRT
<213> Arabidopsis thaliana

<400> 84

```
Met Ser Gly Ala Thr Thr Ala Ser Ser Gly Asp His Asn Asn Leu Arg
1               5                   10                  15
Leu Pro Thr Pro Thr Leu Asp Ala Glu Ser Gln Thr Leu Leu Gln Ser
            20                  25                  30
Ile Ser Ala Glu Gly Gly Tyr Ala Tyr Ala Arg Met Ala Val Leu Ala
        35                  40                  45
Val Ala Gly Asp Gln Ser Ala Ala Glu Ala Ala Arg Asp Met Ala Trp
    50                  55                  60
Glu Gln Leu His Ser Gly Pro Trp His Ser Val Leu Pro Val Trp Arg
65                  70                  75                  80
Asp Ala Tyr Ser Met Ala Cys Leu His Val Ala Lys Ile His Phe Ala
                85                  90                  95
Ala Gly Glu Phe Gly Glu Ala Leu Gly Ala Leu Asp Met Gly Leu Ile
            100                 105                 110
Met Gly Gly Met Leu Leu Arg Lys Asp Leu His Asp Ser Val Leu Leu
        115                 120                 125
Val Ser Ser Glu Ala Arg Lys Met Thr Lys Ser Leu Glu Glu Ala Ser
    130                 135                 140
Gly Asp Phe Lys Gly Glu Arg Leu Val Pro Glu Val Pro Val Asp Val
145                 150                 155                 160
Asn Glu Val Arg His Val Leu Ala Asn Leu Gln Leu Leu Val Leu Lys
                165                 170                 175
Ile Leu Pro Cys Arg Ser Leu Thr Cys Lys Arg Val Glu Lys Arg Ser
            180                 185                 190
Gly Leu Ser Leu Glu Gly Phe Leu Arg Asp Tyr Tyr Leu Pro Gly Thr
        195                 200                 205
Pro Val Val Ile Thr Asn Ser Met Ala His Trp Pro Ala Arg Thr Lys
    210                 215                 220
Trp Asn His Leu Asp Tyr Leu Asn Ala Val Ala Gly Asn Arg Thr Val
225                 230                 235                 240
Pro Val Glu Val Gly Lys Asn Tyr Leu Cys Ser Asp Trp Lys Gln Glu
                245                 250                 255
Leu Val Thr Phe Ser Lys Phe Leu Glu Arg Met Arg Thr Asn Lys Ser
            260                 265                 270
Ser Pro Met Glu Pro Thr Tyr Leu Ala Gln His Pro Leu Phe Asp Gln
        275                 280                 285
Ile Asn Glu Leu Arg Asp Asp Ile Cys Ile Pro Asp Tyr Cys Phe Val
    290                 295                 300
Gly Gly Gly Glu Leu Gln Ser Leu Asn Ala Trp Phe Gly Pro Ala Gly
305                 310                 315                 320
Thr Val Thr Pro Leu His His Asp Pro His His Asn Ile Leu Ala Gln
                325                 330                 335
Val Val Gly Lys Lys Tyr Ile Arg Leu Tyr Pro Ser Phe Leu Gln Asp
            340                 345                 350
Glu Leu Tyr Pro Tyr Ser Glu Thr Met Leu Cys Asn Ser Ser Gln Val
        355                 360                 365
Asp Leu Asp Asn Ile Asp Glu Thr Glu Phe Pro Lys Ala Met Glu Leu
    370                 375                 380
Glu Phe Met Asp Cys Ile Leu Glu Glu Gly Glu Met Leu Tyr Ile Pro
385                 390                 395                 400

Pro Lys Trp Trp His Tyr Val Arg Ser Leu Thr Met Ser Leu Ser Val
                405                 410                 415
Ser Phe Trp Trp Ser Asn Glu Ala Glu Ser Ser Ser Ser
            420                 425
```

<210> 85
<211> 1518
<212> DNA
<213> Arabidopsis thaliana

<400> 85

```
atgccgacgg gatgcgagtt tccgatgatc aaaaccttcg agaatgctct tactgccgcc     60
gacttcgagt ctaccgtcga gctcaccaac ttcccagcag tttttcgggg atgtgctagt    120
gtctgggatg cgtattctaa gtggaatcca ttcaacagtg gcctcgatta tctggaggaa    180
cgtgcaggtt cagttgaagt ggaggcaatg ctttcgagga cggctccagt tttcaatggt    240
gatatcagaa gccatgagag ggtgtcattg cctttctcgg attttatcag attctgcaag    300
cagcatatga gaggcaaagg gaatggttct ggtgttgatg ctaagtctgc agatttaaat    360
cctatgtgtg aggactatag acctggacaa atatacctag cacagtttcc aattctgaat    420
gatgagaaag aagagaaggt tctacttaag attctgagac aagatatcca gacgcccaca    480
tttttagacg caaagtcact ttcttctatc aacttttgga tgaattctgc agaagctcga    540
tcaagtaccc actatgatcc acatcataac cttctatgcg tagtctcagg gcgtaaaaaa    600
gttgtattgt ggcccccttc cgccagtccc tcgctctacc cgatgcctat atacggagaa    660
gcatcaaacc atagttcggt tggtctagaa aatccaaatt tatcagatta tccaagagca    720
gagcattcac tgaagcagtc acaggagatt actcttaacg ctggtgatgc agtattcatt    780
cctgaaggtt ggttccatca ggtggatagt gatgaactga ctgttgctgt caacttttgg    840
tggcaatcaa actatatgtc taacatgcct gaacacatgg attcgtatta tctgcgtcga    900
atcacaagaa gattgataga cagagaaatg agcctgttag tctcgaagcc ttcttcaact    960
gatttaaggc acctgtctga gcatattgac caatctcgca ttgagatggc agaaggtggg   1020
aatgataata taggcaatga aagcattaaa aagggcctca gcacattgca tgaaaaagcg   1080
tcgctgcacg atctagatcc ttctgcttcc caggcgcttc atgatcttat ttctttagtc   1140
catgaccacg ttaatgctgt tgatacaaaa gatgatcgcg ttgcccattt gctttggaat   1200
cttgaagcct ctagacttcg ggatgttctc cttgcaatgg cacgttattt cccaagaact   1260
ttagaagcat taatactcca catgctctca cccattgctg cggaagttct aacacagaag   1320
tttgatgaga ttgaccaaca gactggtgaa gaagatagga cccagttctt tcgggaattt   1380
tatagtgcat tcgatgatga agcagctgcg atggatataa ttttaagtag gaaagaggct   1440
tttgcatttc aggcatttaa gaatgtacta gacaagtttt tgggggtcaa cattgcttca   1500
ccaacaacaa acatttga                                                 1518
```

<210> 86

<211> 505

<212> PRT

<213> Arabidopsis thaliana

<400> 86

```
Met Pro Thr Gly Cys Glu Phe Pro Met Ile Lys Thr Phe Glu Asn Ala
1               5                   10                  15
Leu Thr Ala Ala Asp Phe Glu Ser Thr Val Glu Leu Thr Asn Phe Pro
            20                  25                  30
Ala Val Phe Arg Gly Cys Ala Ser Val Trp Asp Ala Tyr Ser Lys Trp
        35                  40                  45
Asn Pro Phe Asn Ser Gly Leu Asp Tyr Leu Glu Glu Arg Ala Gly Ser
    50                  55                  60
Val Glu Val Glu Ala Met Leu Ser Arg Thr Ala Pro Val Phe Asn Gly
65                  70                  75                  80
Asp Ile Arg Ser His Glu Arg Val Ser Leu Pro Phe Ser Asp Phe Ile
                85                  90                  95
Arg Phe Cys Lys Gln His Met Arg Gly Lys Gly Asn Gly Ser Gly Val
            100                 105                 110
Asp Ala Lys Ser Ala Asp Leu Asn Pro Met Cys Glu Asp Tyr Arg Pro
        115                 120                 125
Gly Gln Ile Tyr Leu Ala Gln Phe Pro Ile Leu Asn Asp Glu Lys Glu
```

```
        130                 135                 140
Glu Lys Val Leu Leu Lys Ile Leu Arg Gln Asp Ile Gln Thr Pro Thr
145                 150                 155                 160
Phe Leu Asp Ala Lys Ser Leu Ser Ser Ile Asn Phe Trp Met Asn Ser
                165                 170                 175
Ala Glu Ala Arg Ser Ser Thr His Tyr Asp Pro His His Asn Leu Leu
            180                 185                 190
Cys Val Val Ser Gly Arg Lys Lys Val Val Leu Trp Pro Pro Ser Ala
        195                 200                 205
Ser Pro Ser Leu Tyr Pro Met Pro Ile Tyr Gly Glu Ala Ser Asn His
    210                 215                 220
Ser Ser Val Gly Leu Glu Asn Pro Asn Leu Ser Asp Tyr Pro Arg Ala
225                 230                 235                 240
Glu His Ser Leu Lys Gln Ser Gln Glu Ile Thr Leu Asn Ala Gly Asp
                245                 250                 255
Ala Val Phe Ile Pro Glu Gly Trp Phe His Gln Val Asp Ser Asp Glu
            260                 265                 270
Leu Thr Val Ala Val Asn Phe Trp Trp Gln Ser Asn Tyr Met Ser Asn
        275                 280                 285
Met Pro Glu His Met Asp Ser Tyr Tyr Leu Arg Arg Ile Thr Arg Arg
    290                 295                 300
Leu Ile Asp Arg Glu Met Ser Leu Leu Val Ser Lys Pro Ser Ser Thr
305                 310                 315                 320
Asp Leu Arg His Leu Ser Glu His Ile Asp Gln Ser Arg Ile Glu Met
                325                 330                 335
Ala Glu Gly Gly Asn Asp Asn Ile Gly Asn Glu Ser Ile Lys Lys Gly
            340                 345                 350
Leu Ser Thr Leu His Glu Lys Ala Ser Leu His Asp Leu Asp Pro Ser
        355                 360                 365
Ala Ser Gln Ala Leu His Asp Leu Ile Ser Leu Val His Asp His Val
    370                 375                 380
Asn Ala Val Asp Thr Lys Asp Asp Arg Val Ala His Leu Leu Trp Asn
385                 390                 395                 400
Leu Glu Ala Ser Arg Leu Arg Asp Val Leu Leu Ala Met Ala Arg Tyr
                405                 410                 415
Phe Pro Arg Thr Leu Glu Ala Leu Ile Leu His Met Leu Ser Pro Ile
            420                 425                 430
Ala Ala Glu Val Leu Thr Gln Lys Phe Asp Glu Ile Asp Gln Gln Thr
        435                 440                 445
Gly Glu Glu Asp Arg Thr Gln Phe Phe Arg Glu Phe Tyr Ser Ala Phe
    450                 455                 460
Asp Asp Glu Ala Ala Ala Met Asp Ile Ile Leu Ser Arg Lys Glu Ala
465                 470                 475                 480
Phe Ala Phe Gln Ala Phe Lys Asn Val Leu Asp Lys Phe Leu Gly Val
                485                 490                 495
Asn Ile Ala Ser Pro Thr Thr Asn Ile
            500                 505
```

<210> 87
<211> 1038
<212> DNA
<213> Arabidopsis thaliana

<400> 87

```
atggctaaag agatagagaa tttatggaga gaagtgagag aattgagctt aggaactaaa      60
atcgatcgtt ttgattctca accgtctccg gtgaagtttc tccgaaacta cgtgtctcag     120
agcaagcctt gtgtaatctc caaagctatt actcattggc cggcgttaaa actctggtca     180
gatccggctt acctcaccgg agctttgtca gatgacgttg tctcgctaca tctcactcct     240
aacggctgcg cagacgccgt caccggagat agtgatcttt gtttcgcttc ggctcatgtt     300
gagaaagttc tgttcccgga ggctcttaag gttgttcaat cgtcgtgtaa aggactgaag     360
gttgggtatt tgcaacagca aaacgattgt tttagaacgg agtattcgac ggttgcgtta     420
```

```
gactgcgacg gtgatattga gtgggctacg gaggcatttg gttgttcgcc ggaagctgtg    480
aacctttgga ttggtactga tgactccgtg acttcatttc ataaagatca ctatgagaat    540
ctttacgctg ttgtttcagg ggagaaacat ttccttcttc ttcctccgac tgatgttcac    600
cgtctctata tcgaacagta tccggcagct aattactctt accaccgaga tactgatgcg    660
ttcaagcttg aggttgagga gccggttagg catgtacctt ggtctagtgt tgatccatac    720
ccttcgccag agaaggaagc aagcgagaga ttgaaatttc cgttgttctt tgatggcccg    780
aagccgtttc attgtactgt caaggctggc gaggttcttt acctgccaag catgtggttt    840
caccatgtta gtcaaacccc aggagatgga ggttatacca ttgcagtgaa ctattggtat    900
gacatgcagt ttgacatcaa gtatgcttat tttaacttct tgcaatcttt attatacaag    960
tcatcttcac tcaatccagt tctttcttgg agagaagacg aagattcaga atccagtgat   1020
gcagagattg ctccctga                                                 1038
```

<210> 88

<211> 431

<212> PRT

<213> Arabidopsis thaliana

<400> 88

```
Met Ala Lys Glu Ile Glu Asn Leu Trp Arg Glu Val Arg Glu Leu Ser
1               5                   10                  15
Leu Gly Thr Lys Ile Asp Arg Phe Asp Ser Gln Pro Ser Pro Val Lys
            20                  25                  30
Phe Leu Arg Asn Tyr Val Ser Gln Ser Lys Pro Cys Val Ile Ser Lys
        35                  40                  45
Ala Ile Thr His Trp Pro Ala Leu Lys Leu Trp Ser Asp Pro Ala Tyr
    50                  55                  60
Leu Thr Gly Ala Leu Ser Asp Asp Val Val Ser Leu His Leu Thr Pro
65                  70                  75                  80
Asn Gly Cys Ala Asp Ala Val Thr Gly Asp Ser Asp Leu Cys Phe Ala
                85                  90                  95
Ser Ala His Val Glu Lys Val Leu Phe Pro Glu Ala Leu Lys Val Val
            100                 105                 110
Gln Ser Ser Cys Lys Gly Leu Lys Val Gly Tyr Leu Gln Gln Gln Asn
        115                 120                 125
Asp Cys Phe Arg Thr Glu Tyr Ser Thr Val Ala Leu Asp Cys Asp Gly
    130                 135                 140
Asp Ile Glu Trp Ala Thr Glu Ala Phe Gly Cys Ser Pro Glu Ala Val
145                 150                 155                 160
Asn Leu Trp Ile Gly Thr Asp Asp Ser Val Thr Ser Phe His Lys Asp
                165                 170                 175
His Tyr Glu Asn Leu Tyr Ala Val Val Ser Gly Glu Lys His Phe Leu
            180                 185                 190
Leu Leu Pro Pro Thr Asp Val His Arg Leu Tyr Ile Glu Gln Tyr Pro
        195                 200                 205
Ala Ala Asn Tyr Ser Tyr His Arg Asp Thr Asp Ala Phe Lys Leu Glu
    210                 215                 220
Val Glu Glu Pro Val Arg His Val Pro Trp Ser Ser Val Asp Pro Tyr
225                 230                 235                 240
Pro Ser Pro Glu Lys Glu Ala Ser Glu Arg Leu Lys Phe Pro Leu Phe
                245                 250                 255
Phe Asp Gly Pro Lys Pro Phe His Cys Thr Val Lys Ala Gly Glu Val
            260                 265                 270
Leu Tyr Leu Pro Ser Met Trp Phe His His Val Ser Gln Thr Pro Gly
        275                 280                 285
Asp Gly Gly Tyr Thr Ile Ala Val Asn Tyr Trp Tyr Asp Met Gln Phe
    290                 295                 300
Asp Ile Lys Tyr Ala Tyr Phe Asn Phe Leu Gln Ser Leu Leu Tyr Lys
305                 310                 315                 320
Ser Ser Ser Leu Asn Pro Val Leu Ser Trp Arg Glu Asp Glu Asp Ser
                325                 330                 335
Glu Ser Ser Asp Ala Glu Gly Ser Lys Phe Thr Pro Ser Ala Thr Asn


            340                 345                 350
Leu Tyr Leu Ser Ser Thr Tyr Val Ser Asp Gly Ala Arg Phe Trp Lys
        355                 360                 365
Arg Val His Val Asn Ser Ile Lys Leu Gly Phe Glu Ser Asn Gly Leu
    370                 375                 380
Ile Gln Ser Gly Leu Ile Asp Met Tyr Arg Lys Tyr Arg Leu Val Ile
385                 390                 395                 400
Asn Ala Glu Lys Val Phe Arg Ala Met Glu Gly Gln Thr Val Leu Thr
                405                 410                 415
Val Gly Gly Tyr Gly Phe Glu Tyr Tyr Ala Glu Trp Phe Ser His
            420                 425                 430
```

<210> 89
<211> 1142
<212> DNA
<213> Medicago truncatula

<400> 89

```
atgaatgaga agatagaaga actatggaga gaagtacgag aattaagtct aggaagcaaa      60
agaagcatag aacgtttaga atcagcacca acaccactcc aattccatag aaacttcatc     120
actccaaaca aaccttgcat catctccaac tccatctccc attggccctc cctctctctc     180
tggtcccacc cttcttacct cactcaatcc ctctcttcca ccaccgtctc cctccatctc     240
acccccaccg gctccgccga ctctttaacc cctctcccct cttccccctc ctccctctgc     300
ttcgcctctg cccatgtcca aaacctccct ttccccgaag ccctccgtct catcaactcc     360
tctaaccctt cccaatgtgt cgcctacgcg cagcaacaga acgattgttt tcgttcagag     420
tatgattcca ttgttaaaga ttgtgatcaa catattgctt gggctactga agcatttggt     480
ttagagcctg aagctgttaa tctttggatt ggaaacaaac attcatctac ttggtttcat     540
aaggatcatt atgagaatct ttatgctgtt gttactggtc aaaaacactt tcttttgttt     600
cctcctactg atgttcatcg cttttatatt cggaattacc ctgctgctac ttataaatat     660
tacatggaaa ctggagagtt tgatttggaa cttgacaagc caactaggta tgtgccttgg     720
tgtagtgtga acccttttcc ttctccggag aacttggagg acgagatttc aaagtttcct     780
ttatacttca atggccctcc accgtttgag tgtactgtca aggctgggga gattctttac     840
ttgtatgatt ctcttctttt gcattgcatc atgccaagca tgtggtttca tcatgttaga     900
caaagtgggg atgatggaga attaactatt gcagtaaatt attggtatga tatgcaattt     960
gacattaagt atgcttattt caacttttta caatctattg attaccgatc tcctacgagt    1020
ccaatgatgc ctgagaaatt gtgtgaggag atagattttg gtcctgatga cgatgagact    1080
agatgaatta tcatttctca gtcggtgata ttcggcaatg tggtccttcg atcgaatgct    1140
ga                                                                   1142
```

<210> 90
<211> 361
<212> PRT
<213> Medicago truncatula

<400> 90

```
Met Asn Glu Lys Ile Glu Glu Leu Trp Arg Glu Val Arg Glu Leu Ser
1               5                   10                  15
Leu Gly Ser Lys Arg Ser Ile Glu Arg Leu Glu Ser Ala Pro Thr Pro
            20                  25                  30
Leu Gln Phe His Arg Asn Phe Ile Thr Pro Asn Lys Pro Cys Ile Ile
        35                  40                  45
Ser Asn Ser Ile Ser His Trp Pro Ser Leu Ser Leu Trp Ser His Pro
    50                  55                  60
Ser Tyr Leu Thr Gln Ser Leu Ser Ser Thr Thr Val Ser Leu His Leu
65                  70                  75                  80
Thr Pro Thr Gly Ser Ala Asp Ser Leu Thr Pro Leu Pro Ser Ser Pro
                85                  90                  95
Ser Ser Leu Cys Phe Ala Ser Ala His Val Gln Asn Leu Pro Phe Pro
            100                 105                 110
Glu Ala Leu Arg Leu Ile Asn Ser Ser Asn Pro Ser Gln Cys Val Ala
        115                 120                 125
```

```
Tyr Ala Gln Gln Gln Asn Asp Cys Phe Arg Ser Glu Tyr Asp Ser Ile
    130                 135                 140
Val Lys Asp Cys Asp Gln His Ile Ala Trp Ala Thr Glu Ala Phe Gly
145                 150                 155                 160
Leu Glu Pro Glu Ala Val Asn Leu Trp Ile Gly Asn Lys His Ser Ser
                165                 170                 175
Thr Trp Phe His Lys Asp His Tyr Glu Asn Leu Tyr Ala Val Val Thr
            180                 185                 190
Gly Gln Lys His Phe Leu Leu Phe Pro Pro Thr Asp Val His Arg Phe
        195                 200                 205
Tyr Ile Arg Asn Tyr Pro Ala Ala Thr Tyr Lys Tyr Tyr Met Glu Thr
    210                 215                 220
Gly Glu Phe Asp Leu Glu Leu Asp Lys Pro Thr Arg Tyr Val Pro Trp
225                 230                 235                 240
Cys Ser Val Asn Pro Phe Pro Ser Pro Glu Asn Leu Glu Asp Glu Ile
                245                 250                 255
Ser Lys Phe Pro Leu Tyr Phe Asn Gly Pro Pro Pro Phe Glu Cys Thr
            260                 265                 270
Val Lys Ala Gly Glu Ile Leu Tyr Leu Tyr Asp Ser Leu Leu Leu His
        275                 280                 285
Cys Ile Met Pro Ser Met Trp Phe His His Val Arg Gln Ser Gly Asp
    290                 295                 300
Asp Gly Glu Leu Thr Ile Ala Val Asn Tyr Trp Tyr Asp Met Gln Phe
305                 310                 315                 320
Asp Ile Lys Tyr Ala Tyr Phe Asn Phe Leu Gln Ser Ile Asp Tyr Arg
                325                 330                 335
Ser Pro Thr Ser Pro Met Met Pro Glu Lys Leu Cys Glu Glu Ile Asp
            340                 345                 350
Phe Gly Pro Asp Asp Asp Glu Thr Arg
        355                 360
```

<210> 91
<211> 1071
<212> DNA
<213> Brachypodium sylvaticum

<400> 91

```
tcaatcgttc ttctcctcaa gatcaccttc caaggcagca tctttgttgt ccagcggaga     60
attactgatc tccagtgacc tcaggaagtt aaagtacgca tacttgatgt caaactgcat    120
gtcataccag taattcacag caatggtgag cccgttaggc ccggggctct ggctgacatg    180
gtgaaaccac atgctcggca agtagagcac ctcgccagca cggacggtgc accgtatcgg    240
cctgggcccc tcgaagtaga gcgggaagga tgagacctgc gccgccatct cctccggcga    300
cgacgggttc gggtccacgc tgctccacgg cacgatcctc tcgggctctt ccatctccag    360
cttaagcccc gtcagctcct cctctccttc gttctccgtg acgtagcggg cggcggggta    420
gtcgcggacg tagaggcggt ggtgctcggt gggggcaag aggaggaaat gcttctcgcc    480
ggagaggacg gcgtagacgt tgtcgtagtg gtccttgtgg aaggaggtga cggagcagga    540
gttgccgatc cagaggttga cggcctccgg gaggcagccg agcgcctcgc tggcccaggg    600
cacgtgcgcg tccacgtcgc cggccaccgc cgcgtactcc ccgcgcaggc agtcgtcctg    660
ctgctgcgcg tacgccacca ggccgccagc ggccgcccgg tcggagcccc tgatgagccg    720
cacggcggta gggaagtcga cccggcggac gtgcgcggac gcgaagcacc tggcgccggg    780
gaggcacggg tgagggcgga gggcgtcggc gcggccgtcg gggtgaggt ggacggagac    840
gtcggtggag cggagcgcgt cggtgaggta ggattcggtg ggccagaggg aggcggcggg    900
ccagtggcgg gtggcggcgg cggagacgag gagcgggcgg cccggggaga cgtggtcgcg    960
gaggaaggcg agcggggtcg gggcaggtc ggcgcgcggc acggcctccg gagagtggag   1020
acccagcagg tcccgtgatt ccgcccacag ctcccgcacc gcgcgctcca t            1071
```

<210> 92
<211> 356
<212> PRT
<213> Brachypodium sylvaticum

<400> 92

```
Met Glu Arg Ala Val Arg Glu Leu Trp Ala Glu Ser Arg Asp Leu Leu
1               5                   10                  15
Gly Leu His Ser Pro Glu Ala Val Pro Arg Ala Asp Leu Pro Pro Thr
            20                  25                  30
Pro Leu Ala Phe Leu Arg Asp His Val Ser Pro Gly Arg Pro Leu Leu
        35                  40                  45
Val Ser Ala Ala Ala Thr Arg His Trp Pro Ala Ala Ser Leu Trp Pro
    50                  55                  60
Thr Glu Ser Tyr Leu Thr Asp Ala Leu Arg Ser Thr Asp Val Ser Val
65                  70                  75                  80
His Leu Thr Pro Asp Gly Arg Ala Asp Ala Leu Ala Pro His Pro Cys
                85                  90                  95
Leu Pro Gly Ala Arg Cys Phe Ala Ser Ala His Val Arg Arg Val Asp
            100                 105                 110
Phe Pro Thr Ala Val Arg Leu Ile Arg Gly Ser Asp Arg Ala Ala Ala
        115                 120                 125
Gly Gly Leu Val Ala Tyr Ala Gln Gln Gln Asp Asp Cys Leu Arg Gly
    130                 135                 140
Glu Tyr Ala Ala Val Ala Gly Asp Val Asp Ala His Val Pro Trp Ala
145                 150                 155                 160
Ser Glu Ala Leu Gly Cys Leu Pro Glu Ala Val Asn Leu Trp Ile Gly
                165                 170                 175
Asn Ser Cys Ser Val Thr Ser Phe His Lys Asp His Tyr Asp Asn Val
            180                 185                 190
Tyr Ala Val Leu Ser Gly Glu Lys His Phe Leu Leu Leu Pro Pro Thr
        195                 200                 205
Glu His His Arg Leu Tyr Val Arg Asp Tyr Pro Ala Ala Arg Tyr Val
    210                 215                 220
Thr Glu Asn Glu Gly Glu Glu Glu Leu Thr Gly Leu Lys Leu Glu Met
225                 230                 235                 240
Glu Glu Pro Glu Arg Ile Val Pro Trp Ser Ser Val Asp Pro Asn Pro
                245                 250                 255
Ser Ser Pro Glu Glu Met Ala Ala Gln Val Ser Ser Phe Pro Leu Tyr
            260                 265                 270
Phe Glu Gly Pro Arg Pro Ile Arg Cys Thr Val Arg Ala Gly Glu Val
        275                 280                 285
Leu Tyr Leu Pro Ser Met Trp Phe His His Val Ser Gln Ser Pro Gly
    290                 295                 300
Pro Asn Gly Leu Thr Ile Ala Val Asn Tyr Trp Tyr Asp Met Gln Phe
305                 310                 315                 320
Asp Ile Lys Tyr Ala Tyr Phe Asn Phe Leu Arg Ser Leu Glu Ile Ser
                325                 330                 335
Asn Ser Pro Leu Asp Asn Lys Asp Ala Ala Leu Glu Gly Asp Leu Glu
            340                 345                 350
Glu Lys Asn Asp
        355
```

<210> 93
<211> 1080
<212> DNA
<213> Oryza sativa

<400> 93

```
atggagcgcg cagtgcggga gctgtgggcg gagtcgcggg acctgctggg cctccactcc      60
ccggacgacg ccgccgccgc cgacgccgcg atgccccgcg ccgagatgcc cccgacgccg     120
ctcgcgtttc tccgcgacca cgtctcgccg gggcgccctc tcctcgtgtc ctccgccgcc     180
accagccact ggccggccgc ctcgctgtgg ccgaccgact cctacctcac cgacgcgctc     240
cgctccaccg ccgtctcgct ccacctcacc cccgacggcc gcgccgacgc cctcgccccg     300
cacccgcgcc cgagccaccc cggcgccaag tgcttcgcct ccgcgcacgt ccgccaggtc     360
```

```
gacttcccca ccgccgtgcg cctcatccgg agctccgatc cggcctccgg cctggtggcc    420
tacgcgcagc agcaggacga ctgcctgcgc ggggagtacg ccgccgtcgc cggcgacgtg    480
gacgcgcacg tgccctgggc cagcgacgcg ctcggctgcc tccccgaggc cgtcaacctc    540
tggatcggca gcgcctgctc ccagacctcc ttccacaagg accactacga caacatctac    600
gtcgtcgtct ccggcgagaa gcacttcctc ctcttgcccc ccaccgagca ccaccgcctc    660
tacgtccgcg actaccccgc cgcccactac gccgcggaag atgaagcgga gctgaggctt    720
aagctggagc tggaggagcc cgagaggatc gtgccatgga gcagcgttga cccctacccg    780
ccgtcgccgg aggaggcggc cgcgcaggcg tcatccttcc cgctctactt cgaggggccg    840
aggccgatcc gctgcacggt gcgcgccggc gagatgctct acttgccgag catgtggttt    900
caccatgtga gccagagccc cgggccgaac gggctcacca ttgcagtgaa ctactggtat    960
gacatgcagt ttgacattaa gtatgcctac ttcaacttct tgaggtcatt ggagatcgat   1020

ggtagttcgt cgaaaaagac ggatgctttg gaagacgatc tcgaggagac gaatgattga   1080
```

<210> 94
<211> 354
<212> PRT
<213> Oryza sativa

<400> 94

```
Met Glu Arg Ala Val Arg Glu Leu Trp Ala Glu Ser Arg Asp Leu Leu
1               5                   10                  15
Gly Leu His Ser Pro Asp Asp Ala Ala Ala Ala Asp Ala Ala Met Pro
            20                  25                  30
Arg Ala Glu Met Pro Pro Thr Pro Leu Ala Phe Leu Arg Asp His Val
        35                  40                  45
Ser Pro Gly Arg Pro Leu Leu Val Ser Ser Ala Ala Thr Ser His Trp
    50                  55                  60
Pro Ala Ala Ser Leu Trp Pro Thr Asp Ser Tyr Leu Thr Asp Ala Leu
65                  70                  75                  80
Arg Ser Thr Ala Val Ser Leu His Leu Thr Pro Asp Gly Arg Ala Asp
                85                  90                  95
Ala Leu Ala Pro His Pro Arg Pro Ser His Pro Gly Ala Lys Cys Phe
            100                 105                 110
Ala Ser Ala His Val Arg Gln Val Asp Phe Pro Thr Ala Val Arg Leu
        115                 120                 125
Ile Arg Ser Ser Asp Pro Ala Ser Gly Leu Val Ala Tyr Ala Gln Gln
    130                 135                 140
Gln Asp Asp Cys Leu Arg Gly Glu Tyr Ala Ala Val Ala Gly Asp Val
145                 150                 155                 160
Asp Ala His Val Pro Trp Ala Ser Asp Ala Leu Gly Cys Leu Pro Glu
                165                 170                 175
Ala Val Asn Leu Trp Ile Gly Ser Ala Cys Ser Gln Thr Ser Phe His
            180                 185                 190
Lys Asp His Tyr Asp Asn Ile Tyr Val Val Val Ser Gly Glu Lys His
        195                 200                 205
Phe Leu Leu Leu Pro Pro Thr Glu His His Arg Leu Tyr Val Arg Asp
    210                 215                 220
Tyr Pro Ala Ala His Tyr Ala Ala Glu Asp Glu Ala Glu Leu Arg Leu
225                 230                 235                 240
Lys Leu Glu Leu Glu Glu Pro Glu Arg Ile Val Pro Trp Ser Ser Val
                245                 250                 255
Asp Pro Tyr Pro Pro Ser Pro Glu Glu Ala Ala Ala Gln Ala Ser Ser
            260                 265                 270
Phe Pro Leu Tyr Phe Glu Gly Pro Arg Pro Ile Arg Cys Thr Val Arg
        275                 280                 285
Ala Gly Glu Met Leu Tyr Leu Pro Ser Met Trp Phe His His Val Ser
    290                 295                 300
Gln Ser Pro Gly Pro Asn Gly Leu Thr Ile Ala Val Asn Tyr Trp Tyr
305                 310                 315                 320
Asp Met Gln Phe Asp Ile Lys Tyr Ala Tyr Phe Asn Phe Leu Arg Ser

                325                 330                 335
Leu Glu Ile Asp Gly Ser Ser Ser Lys Lys Thr Asp Ala Leu Glu Asp
            340                 345                 350
Asp Leu
```

<210> 95

<211> 3552

<212> DNA

<213> Arabidopsis thaliana

<400> 95

```
atggggacag agctaatgag aatctgtgtg aaagaagata gtgatgattt gccatctgtt     60
cctcctggtt ttgaatcata tgcaactttt accttaaaaa gagtagtccc tgctactact    120
tctgataaag ccaagactcc tgcaattgaa agtgtttctg caaccgaaca agctaagatg    180
gaagttgaat ctgatgaagc caaagctgct cgggctctac gccgtagacc ttggattaat    240
catagcggat gtgatgatga tggtgattgt gctgcaaaca atgacaatgc tgcatctcaa    300
aatcctgatc aaaattgtga tgtgaagcca gctcttccaa agggtgtggt caggggatgt    360
gaagaatgta aagattgtca gaaggagttt gaagacactt taaattatat cgctaaaata    420
agaccggagg ctgaaaagta tggaatctgt cgcattgttc ctcctccttc ctggaaaccg    480
ccgtgcccac tgaaagaaaa gcaagtatgg gagggttcta aatttacaac acgtgtccaa    540
agagttgata aacttcagaa tcggagttca atgaagaaga tttcaaagct tcctaatcaa    600
atgagaaaga agaaaagaaa gtgcatgaaa atgggaatgg attctgtcac taatggtatg    660
ggcgatccct gttctgcaag cactggaatg aatgaacttg agacgtttgg gtttgaacct    720
ggtccagggt tcacactaaa agacttccaa aaatatgctg atgagttcaa ggctcagtac    780
tttaaaaaga gtgaaacttc tacagacgat aaatgtaaag ttgataattc aatagactgc    840
tgggagccag cacttgagga tgttgaaggt gaatattggc ggatagtaga taaagcaact    900
gaagagatag aggtgctata tggtgctgac cttgaaactg ggtatttgg tagcggattc    960
cccaagatat catcaagtca caatgcttct tcttcagaag ataaatatgc aaaatcaggc   1020
tggaacttaa ataactttcc aaggcttccg ggatccctcc ttaagtacga gggcagtgat   1080
atttccggtg tccttgtgcc gtggctgtat attgggatgt gcttttcttc tttctgctgg   1140
catgttgaag atcaccactt gtattcgttg aattatatgc actggggtgc accgaaattg   1200
tggtatggtg ttggggggaa ggatgctgtt aaactcgagg aggcaatgag gaagcatttg   1260
cctgaccttt ttgaagaaca gcctgactta cttcataagc tagttacaca actctccccg   1320
tcaaaactga aaaccgcagg agtacctgtg caccgttgcg tccagcatgc tggagagttt   1380
gtcttgactt ttcctcgggc atatcatgct ggatttaatt ctggtttcaa ctgtgctgaa   1440
gctgtgaatg tagcacccgt tgactggtta cctcatgggc agattgccat agagttatac   1500
tgtcaacagg gtagaaaaac gtccatctcg catgataaat tgttgcttgg ggcagcaagg   1560
gaagtagtga aagccgactg ggagctgaac ttactaagga aaaatactgt agataactta   1620
aggtggaaag cattcagtgc gaaggatgga attttggcaa aaacattaaa ggcacgcatt   1680
gacatggaac gtacgagaag agaatttctg tgcaactctt cacttgcatt gaaaatgcac   1740
agtaatttcg atgctaccaa cgagagagag tgctgtatat gcttctttga tttgcacctg   1800
tctgctgctg gctgtcgttg ttccccggag aagtattcat gtttgactca tgtgaaggag   1860
ttgtgttcat gtccatgggt tactaaatat tttctatttc gctatgatat cgatgaactg   1920
aatgttcttg ttgaggcagt ggaaggaaaa ctgagttctg tatatagatg ggcgcgtcaa   1980
gatttaggat tggcattaag tacagacgtc tcaggaagca agatggagat agatgaagaa   2040
ggaaaagtcc acaaggaccc gactccacaa acaactgcac tttcagggaa ggatttgcag   2100
ttgaaagtaa catcgaaaga agtaagtaaa gagttagaaa agactagtaa attatctcat   2160
gttaatttac ttctgaaaga aaaggaggag caaataacgt caagtcactg tatgaagcct   2220
gtcaaagaag aaactgtttg tgattcttct gatccaaacg tttcagcttg ccaaccatct   2280
gaaggaggca taatctgtat gacagctgtt aagtccgcaa gtggtaaaaa gaattcacag   2340
agtctaccca atgatgtgat actcctcagc gatgatgagt atgacatacc taggaaacga   2400
ggttctgtga gaagagatgc aatttcttct ggcaagaaat tagaaatacg agagagaccg   2460
acccacgttt tggcgttaga agcttctgcc aaaattgctg ctccaatttg ccaaagggaa   2520
ggagattcgc tgcgtgatac acgaaacact atatcattgc ctactaatga ccaaaaaaca   2580
atgagaaggg atgtaccgag ttctacttcg cacgcggaag ttaatgcgga ggctactggg   2640
cttactcaag atatatgtaa cagaatggca actaacagcc acggtggtgg aaaacctact   2700
agttgtaaat ccaaaaactc tggaggtttg gctatagtgg atgttgtgga tgggacaaga   2760
agtagttcag gtacaccgtc ttgttcgcaa aataatagtc cggataggtt catccgccaa   2820
aagggtcccc gcattgcaaa ggtcgtgagg agaatcaatt gcaatgtaga gcctttaagt   2880
tatggatgtg tgctgtctgg aaaatcatgg tgcagccggc gagcaatttt tcctaaagga   2940

tttcgtagcc gggttaagta cataaacatt ttggatccaa caaatatgtg cttctacatt   3000
tcagaaattc tggatgctgg acgcaacagt ccattgttca tggtttactt ggagagtaat   3060
cccagtgagg tgttcgttca tatgtcgcct acaagatgct gggagatggt aagagagaga   3120
gtaaatcaag agataactaa gcagcacaaa gctggaaaat cagatcttcc tcctttgcaa   3180
ccctctggga gtcccgacgg ttttgaaatg tttggatatt cgtcacccgc aatcgtacag   3240
gctatcgaag cattagacgt gaatcgagtt tgcacagact attgggattc caggccttat   3300
tcccggccac aagttcagtt ccctgcaaat cctcttctca gagaagccaa cacaagtggc   3360
agatcgaatg tgggaaatct ccagctaaac cctggacacc atatatcgcc tactggaata   3420
aattctattc ttaaagttct gttcaagaaa gctagcatgg aggaactaag ctcacttcaa   3480
gaggttttaa gtgagactaa ctcagatatg gtgaccgaac ttgtgaagga agagatccag   3540
aaccgtcgct ga                                                       3552
```

<210> 96
<211> 1183
<212> PRT
<213> Arabidopsis thaliana

<400> 96

```
Met Gly Thr Glu Leu Met Arg Ile Cys Val Lys Glu Asp Ser Asp Asp
1               5                   10                  15
Leu Pro Ser Val Pro Pro Gly Phe Glu Ser Tyr Ala Thr Phe Thr Leu
            20                  25                  30
Lys Arg Val Val Pro Ala Thr Thr Ser Asp Lys Ala Lys Thr Pro Ala
        35                  40                  45
Ile Glu Ser Val Ser Ala Thr Glu Gln Ala Lys Met Glu Val Glu Ser
    50                  55                  60
Asp Glu Ala Lys Ala Ala Arg Ala Leu Arg Arg Arg Pro Trp Ile Asn
65                  70                  75                  80
His Ser Gly Cys Asp Asp Asp Gly Asp Cys Ala Ala Asn Asn Asp Asn
                85                  90                  95
Ala Ala Ser Gln Asn Pro Asp Gln Asn Cys Asp Val Lys Pro Ala Leu
            100                 105                 110
Pro Lys Gly Val Val Arg Gly Cys Glu Glu Cys Lys Asp Cys Gln Lys
        115                 120                 125
Glu Phe Glu Asp Thr Leu Asn Tyr Ile Ala Lys Ile Arg Pro Glu Ala
    130                 135                 140
Glu Lys Tyr Gly Ile Cys Arg Ile Val Pro Pro Pro Ser Trp Lys Pro
145                 150                 155                 160
Pro Cys Pro Leu Lys Glu Lys Gln Val Trp Glu Gly Ser Lys Phe Thr
                165                 170                 175
Thr Arg Val Gln Arg Val Asp Lys Leu Gln Asn Arg Ser Ser Met Lys
            180                 185                 190
Lys Ile Ser Lys Leu Pro Asn Gln Met Arg Lys Lys Lys Arg Lys Cys
        195                 200                 205
Met Lys Met Gly Met Asp Ser Val Thr Asn Gly Met Gly Asp Pro Cys
    210                 215                 220
Ser Ala Ser Thr Gly Met Asn Glu Leu Glu Thr Phe Gly Phe Glu Pro
225                 230                 235                 240
Gly Pro Gly Phe Thr Leu Lys Asp Phe Gln Lys Tyr Ala Asp Glu Phe
                245                 250                 255
Lys Ala Gln Tyr Phe Lys Lys Ser Glu Thr Ser Thr Asp Asp Lys Cys
            260                 265                 270
Lys Val Asp Asn Ser Ile Asp Cys Trp Glu Pro Ala Leu Glu Asp Val
        275                 280                 285
Glu Gly Glu Tyr Trp Arg Ile Val Asp Lys Ala Thr Glu Glu Ile Glu
    290                 295                 300
Val Leu Tyr Gly Ala Asp Leu Glu Thr Gly Val Phe Gly Ser Gly Phe
305                 310                 315                 320
Pro Lys Ile Ser Ser Ser His Asn Ala Ser Ser Ser Glu Asp Lys Tyr
                325                 330                 335
Ala Lys Ser Gly Trp Asn Leu Asn Asn Phe Pro Arg Leu Pro Gly Ser
```

```
                    340                 345                 350
Leu Leu Lys Tyr Glu Gly Ser Asp Ile Ser Gly Val Leu Val Pro Trp
            355                 360                 365
Leu Tyr Ile Gly Met Cys Phe Ser Ser Phe Cys Trp His Val Glu Asp
        370                 375                 380
His His Leu Tyr Ser Leu Asn Tyr Met His Trp Gly Ala Pro Lys Leu
385                 390                 395                 400
Trp Tyr Gly Val Gly Gly Lys Asp Ala Val Lys Leu Glu Glu Ala Met
                405                 410                 415
Arg Lys His Leu Pro Asp Leu Phe Glu Glu Gln Pro Asp Leu Leu His
            420                 425                 430
Lys Leu Val Thr Gln Leu Ser Pro Ser Lys Leu Lys Thr Ala Gly Val
        435                 440                 445
Pro Val His Arg Cys Val Gln His Ala Gly Glu Phe Val Leu Thr Phe
    450                 455                 460
Pro Arg Ala Tyr His Ala Gly Phe Asn Ser Gly Phe Asn Cys Ala Glu
465                 470                 475                 480
Ala Val Asn Val Ala Pro Val Asp Trp Leu Pro His Gly Gln Ile Ala
                485                 490                 495
Ile Glu Leu Tyr Cys Gln Gln Gly Arg Lys Thr Ser Ile Ser His Asp
            500                 505                 510
Lys Leu Leu Leu Gly Ala Ala Arg Glu Val Val Lys Ala Asp Trp Glu
        515                 520                 525
Leu Asn Leu Leu Arg Lys Asn Thr Val Asp Asn Leu Arg Trp Lys Ala
    530                 535                 540
Phe Ser Ala Lys Asp Gly Ile Leu Ala Lys Thr Leu Lys Ala Arg Ile
545                 550                 555                 560
Asp Met Glu Arg Thr Arg Arg Glu Phe Leu Cys Asn Ser Ser Leu Ala
                565                 570                 575
Leu Lys Met His Ser Asn Phe Asp Ala Thr Asn Glu Arg Glu Cys Cys
            580                 585                 590
Ile Cys Phe Phe Asp Leu His Leu Ser Ala Ala Gly Cys Arg Cys Ser
        595                 600                 605
Pro Glu Lys Tyr Ser Cys Leu Thr His Val Lys Glu Leu Cys Ser Cys
    610                 615                 620
Pro Trp Val Thr Lys Tyr Phe Leu Phe Arg Tyr Asp Ile Asp Glu Leu
625                 630                 635                 640
Asn Val Leu Val Glu Ala Val Glu Gly Lys Leu Ser Ser Val Tyr Arg
                645                 650                 655
Trp Ala Arg Gln Asp Leu Gly Leu Ala Leu Ser Thr Asp Val Ser Gly
            660                 665                 670
Ser Lys Met Glu Ile Asp Glu Glu Gly Lys Val His Lys Asp Pro Thr
        675                 680                 685
Pro Gln Thr Thr Ala Leu Ser Gly Lys Asp Leu Gln Leu Lys Val Thr
    690                 695                 700
Ser Lys Glu Val Ser Lys Glu Leu Glu Lys Thr Ser Lys Leu Ser His
705                 710                 715                 720
Val Asn Leu Leu Leu Lys Glu Lys Glu Glu Gln Ile Thr Ser Ser His
                725                 730                 735
Cys Met Lys Pro Val Lys Glu Glu Thr Val Cys Asp Ser Ser Asp Pro
            740                 745                 750
Asn Val Ser Ala Cys Gln Pro Ser Glu Gly Gly Ile Ile Cys Met Thr
        755                 760                 765
Ala Val Lys Ser Ala Ser Gly Lys Lys Asn Ser Gln Ser Leu Pro Asn
    770                 775                 780
Asp Val Ile Leu Leu Ser Asp Asp Glu Tyr Asp Ile Pro Arg Lys Arg
785                 790                 795                 800
Gly Ser Val Arg Arg Asp Ala Ile Ser Ser Gly Lys Lys Leu Glu Ile
                805                 810                 815
Arg Glu Arg Pro Thr His Val Leu Ala Leu Glu Ala Ser Ala Lys Ile
            820                 825                 830
```

```
Ala Ala Pro Ile Cys Gln Arg Glu Gly Asp Ser Leu Arg Asp Thr Arg
        835                 840                 845
Asn Thr Ile Ser Leu Pro Thr Asn Asp Gln Lys Thr Met Arg Arg Asp
    850                 855                 860
Val Pro Ser Ser Thr Ser His Ala Glu Val Asn Ala Glu Ala Thr Gly
865                 870                 875                 880
Leu Thr Gln Asp Ile Cys Asn Arg Met Ala Thr Asn Ser His Gly Gly
                885                 890                 895
Gly Lys Pro Thr Ser Cys Lys Ser Lys Asn Ser Gly Gly Leu Ala Ile
            900                 905                 910
Val Asp Val Val Asp Gly Thr Arg Ser Ser Ser Gly Thr Pro Ser Cys
        915                 920                 925
Ser Gln Asn Asn Ser Pro Asp Arg Phe Ile Arg Gln Lys Gly Pro Arg
    930                 935                 940
Ile Ala Lys Val Val Arg Arg Ile Asn Cys Asn Val Glu Pro Leu Ser
945                 950                 955                 960
Tyr Gly Cys Val Leu Ser Gly Lys Ser Trp Cys Ser Arg Arg Ala Ile
                965                 970                 975
Phe Pro Lys Gly Phe Arg Ser Arg Val Lys Tyr Ile Asn Ile Leu Asp
            980                 985                 990
Pro Thr Asn Met Cys Phe Tyr Ile  Ser Glu Ile Leu Asp  Ala Gly Arg
        995                 1000                1005
Asn Ser  Pro Leu Phe Met Val  Tyr Leu Glu Ser Asn  Pro Ser Glu
    1010                1015                1020
Val Phe  Val His Met Ser Pro  Thr Arg Cys Trp Glu  Met Val Arg
    1025                1030                1035
Glu Arg  Val Asn Gln Glu Ile  Thr Lys Gln His Lys  Ala Gly Lys
    1040                1045                1050
Ser Asp  Leu Pro Pro Leu Gln  Pro Ser Gly Ser Pro  Asp Gly Phe
    1055                1060                1065
Glu Met  Phe Gly Tyr Ser Ser  Pro Ala Ile Val Gln  Ala Ile Glu
    1070                1075                1080
Ala Leu  Asp Val Asn Arg Val  Cys Thr Asp Tyr Trp  Asp Ser Arg
    1085                1090                1095
Pro Tyr  Ser Arg Pro Gln Val  Gln Phe Pro Ala Asn  Pro Leu Leu
    1100                1105                1110
Arg Glu  Ala Asn Thr Ser Gly  Arg Ser Asn Val Gly  Asn Leu Gln
    1115                1120                1125
Leu Asn  Pro Gly His His Ile  Ser Pro Thr Gly Ile  Asn Ser Ile
    1130                1135                1140
Leu Lys  Val Leu Phe Lys Lys  Ala Ser Met Glu Glu  Leu Ser Ser
    1145                1150                1155
Leu Gln  Glu Val Leu Ser Glu  Thr Asn Ser Asp Met  Val Thr Glu
    1160                1165                1170
Leu Val  Lys Glu Glu Ile Gln  Asn Arg Arg
    1175                1180
```

<210> 97
<211> 2793
<212> DNA
<213> Arabidopsis thaliana

<400> 97

```
atgaatgcta atgagcaaac tcgatccgcc aatggcattg gcaatggcaa tggtgagtct     60
attcccggga ttccagatga cttacggtgc aagagatcgg atggtaaaca gtggagatgc    120
actgcaatgt ccatggctga taagactgtt tgtgagaagc actacatcca agcaaagaag    180
cgggcggcta attctgcttt cagggcgaac cagaagaaag cgaaaaggcg atcatcgtta    240
ggcgaaacag atacgtattc ggaagggaag atggatgatt tcgagttacc agtcaccagc    300
attgaccact ataataacgg tcttgcctct gcttccaaga gtaatggtag actagagaag    360
agacataata aaagcctgat gcggtactcg cccgagacac cgatgatgag gagtttctct    420
ccacgtgttg cagtggattt gaatgatgac ttgggtagag atgttgtaat gtttgaagag    480
```

```
ggctacagat cttataggac accaccatct gttgctgtta tggatccgac acgaaacaga     540
tcacaccaaa gcaccagtcc tatggaatac tcagcagcaa gcacagatgt gtctgcagag     600
tctttggggg aaatctgcca tcaatgccag agaaaagata gagagagaat catttcttgc     660
ctcaaatgca atcaaagagc cttctgccac aattgtctat cggcaaggta ctcggagata     720
tcacttgaag aagtcgagaa agtttgccct gcatgtcgtg gcttgtgtga ttgcaaatct     780
tgcctgcgtt cagataatac aataaaggtt cggatccggg aaatacccgt tttggacaag     840
ttgcagtatc tttatcgtct attatcagct gtcctaccag tcataaagca gatccatctt     900
gaacaatgta tggaagttga actagagaag aggcttcgtg aagttgagat tgatcttgtc     960
agggcaagat tgaaagcaga tgagcagatg tgctgcaacg tgtgtcggat accagttgtt    1020
gactactacc gtcactgtcc gaactgctca tatgaccttt gcctgagatg ctgtcaagat    1080
ctacgggaag agtcttcagt gacgattagt gggactaacc aaaacgtaca agatagaaaa    1140
ggagctccca aactaaaact aaacttttca tacaagtttc ctgagtggga agccaacggt    1200
gatgggagca tcccttgccc tcctaaggag tatggaggct gcggttcaca ttctttgaat    1260
cttgcccgca ttttcaagat gaattgggtt gcaaagcttg tgaaaaatgc tgaggagatt    1320
gttagtggct gcaaattatc tgatcttctg aaccctgata tgtgtgattc aagattctgc    1380
aaatttgctg agagagaaga gagcggtgac aactacgtgt acagcccgtc gcttgaaacg    1440
attaaaactg atggagtagc taagtttgag caacaatggg cagagggtcg gcttgttact    1500
gtgaaaatgg tacttgatga ctcatcttgc tctagatggg atcctgagac tatttggagg    1560
gatatagacg agctttcgga cgagaaactg agagaacatg atccattctt gaaggccatt    1620
aattgcttgg atggtttaga ggttgatgta agacttgggg agtttacaag agcatataaa    1680
gatggaaaga accaagagac aggtcttccg ctattgtgga agttaaagga ctggccgagc    1740
ccaagtgctt ccgaggagtt cattttctac caaagacctg agtttatcag aagttttccg    1800
tttctcgagt acattcatcc ccggttaggc cttctgaatg ttgcagccaa gttacctcat    1860
tactcgctcc aaaacgattc aggtccaaag atttatgtgt cttgtgggac gtaccaagaa    1920
atcagtgctg gcgattcatt gactggtatt cactacaaca tgcgtgacat ggtataccta    1980
ttggtgcaca cgtctgaaga aacaacattc gaaagggtga gaaaaacaaa acctgttcca    2040
gaggaacctg accagaagat gagcgaaaat gagtcacttc ttagccctga gcagaaatta    2100
agggacggag agttacatga tctatcactt ggtgaagcca gtatggagaa gaatgaacct    2160
gagttggcgt tgactgtgaa tccagagaac ttaacggaaa acggtgacaa catggaatct    2220
tcttgcacat cttcatgtgc aggaggagcc cagtgggatg tctttcgacg ccaagacgtc    2280
ccaaagttgt ccgggtattt gcagagaaca ttccagaagc ctgataatat ccagactgat    2340
tttgtgtcac gcccgttgta tgaaggattg ttcttaaatg aacaccacaa gagacaacta    2400
agagacgagt ttggagttga gccatggaca tttgagcaac atcgtggtga ggctatcttc    2460
attccggctg gatgtccgtt ccaaatcact aatcttcagt cgaatattca ggtggcactt    2520
gacttcttgt gccctgaaag cgttggagag tcagcaagac tagctgaaga aatccggtgt    2580
ttaccaaacg accacgaggc aaaacttcag attctagaga ttggaaagat atcattatac    2640
gcagctagct cagccattaa agaggttcag aaactggtct tggatccaaa gtttggagca    2700
gagcttggat ttgaagactc taacttaacc aaagcagtct ctcacaactt agacgaggca    2760
accaagcggc cgcagcaaaa cagctgcact taa                                 2793
```

<210> 98
<211> 930
<212> PRT
<213> Arabidopsis thaliana

<400> 98

```
Met Asn Ala Asn Glu Gln Thr Arg Ser Ala Asn Gly Ile Gly Asn Gly
1               5                   10                  15
Asn Gly Glu Ser Ile Pro Gly Ile Pro Asp Asp Leu Arg Cys Lys Arg
            20                  25                  30
Ser Asp Gly Lys Gln Trp Arg Cys Thr Ala Met Ser Met Ala Asp Lys
        35                  40                  45
Thr Val Cys Glu Lys His Tyr Ile Gln Ala Lys Lys Arg Ala Ala Asn
    50                  55                  60
Ser Ala Phe Arg Ala Asn Gln Lys Lys Ala Lys Arg Arg Ser Ser Leu
65                  70                  75                  80
Gly Glu Thr Asp Thr Tyr Ser Glu Gly Lys Met Asp Asp Phe Glu Leu
                85                  90                  95
Pro Val Thr Ser Ile Asp His Tyr Asn Asn Gly Leu Ala Ser Ala Ser
            100                 105                 110
Lys Ser Asn Gly Arg Leu Glu Lys Arg His Asn Lys Ser Leu Met Arg
```

```
        115                 120                 125
Tyr Ser Pro Glu Thr Pro Met Met Arg Ser Phe Ser Pro Arg Val Ala
    130                 135                 140
Val Asp Leu Asn Asp Asp Leu Gly Arg Asp Val Val Met Phe Glu Glu
145                 150                 155                 160
Gly Tyr Arg Ser Tyr Arg Thr Pro Pro Ser Val Ala Val Met Asp Pro
                165                 170                 175
Thr Arg Asn Arg Ser His Gln Ser Thr Ser Pro Met Glu Tyr Ser Ala
            180                 185                 190
Ala Ser Thr Asp Val Ser Ala Glu Ser Leu Gly Glu Ile Cys His Gln
        195                 200                 205
Cys Gln Arg Lys Asp Arg Glu Arg Ile Ile Ser Cys Leu Lys Cys Asn
    210                 215                 220
Gln Arg Ala Phe Cys His Asn Cys Leu Ser Ala Arg Tyr Ser Glu Ile
225                 230                 235                 240
Ser Leu Glu Glu Val Glu Lys Val Cys Pro Ala Cys Arg Gly Leu Cys
                245                 250                 255
Asp Cys Lys Ser Cys Leu Arg Ser Asp Asn Thr Ile Lys Val Arg Ile
            260                 265                 270
Arg Glu Ile Pro Val Leu Asp Lys Leu Gln Tyr Leu Tyr Arg Leu Leu
        275                 280                 285
Ser Ala Val Leu Pro Val Ile Lys Gln Ile His Leu Glu Gln Cys Met
    290                 295                 300
Glu Val Glu Leu Glu Lys Arg Leu Arg Glu Val Glu Ile Asp Leu Val
305                 310                 315                 320
Arg Ala Arg Leu Lys Ala Asp Glu Gln Met Cys Cys Asn Val Cys Arg
                325                 330                 335
Ile Pro Val Val Asp Tyr Tyr Arg His Cys Pro Asn Cys Ser Tyr Asp
            340                 345                 350
Leu Cys Leu Arg Cys Cys Gln Asp Leu Arg Glu Glu Ser Ser Val Thr
        355                 360                 365
Ile Ser Gly Thr Asn Gln Asn Val Gln Asp Arg Lys Gly Ala Pro Lys
    370                 375                 380
Leu Lys Leu Asn Phe Ser Tyr Lys Phe Pro Glu Trp Glu Ala Asn Gly
385                 390                 395                 400
Asp Gly Ser Ile Pro Cys Pro Pro Lys Glu Tyr Gly Gly Cys Gly Ser
                405                 410                 415

His Ser Leu Asn Leu Ala Arg Ile Phe Lys Met Asn Trp Val Ala Lys
            420                 425                 430
Leu Val Lys Asn Ala Glu Glu Ile Val Ser Gly Cys Lys Leu Ser Asp
        435                 440                 445
Leu Leu Asn Pro Asp Met Cys Asp Ser Arg Phe Cys Lys Phe Ala Glu
    450                 455                 460
Arg Glu Glu Ser Gly Asp Asn Tyr Val Tyr Ser Pro Ser Leu Glu Thr
465                 470                 475                 480
Ile Lys Thr Asp Gly Val Ala Lys Phe Glu Gln Gln Trp Ala Glu Gly
                485                 490                 495
Arg Leu Val Thr Val Lys Met Val Leu Asp Asp Ser Ser Cys Ser Arg
            500                 505                 510
Trp Asp Pro Glu Thr Ile Trp Arg Asp Ile Asp Glu Leu Ser Asp Glu
        515                 520                 525
Lys Leu Arg Glu His Asp Pro Phe Leu Lys Ala Ile Asn Cys Leu Asp
    530                 535                 540
Gly Leu Glu Val Asp Val Arg Leu Gly Glu Phe Thr Arg Ala Tyr Lys
545                 550                 555                 560
Asp Gly Lys Asn Gln Glu Thr Gly Leu Pro Leu Leu Trp Lys Leu Lys
                565                 570                 575
Asp Trp Pro Ser Pro Ser Ala Ser Glu Glu Phe Ile Phe Tyr Gln Arg
            580                 585                 590
Pro Glu Phe Ile Arg Ser Phe Pro Phe Leu Glu Tyr Ile His Pro Arg
```

```
        595                 600                 605
Leu Gly Leu Leu Asn Val Ala Ala Lys Leu Pro His Tyr Ser Leu Gln
    610                 615                 620
Asn Asp Ser Gly Pro Lys Ile Tyr Val Ser Cys Gly Thr Tyr Gln Glu
625                 630                 635                 640
Ile Ser Ala Gly Asp Ser Leu Thr Gly Ile His Tyr Asn Met Arg Asp
                645                 650                 655
Met Val Tyr Leu Leu Val His Thr Ser Glu Glu Thr Thr Phe Glu Arg
            660                 665                 670
Val Arg Lys Thr Lys Pro Val Pro Glu Glu Pro Asp Gln Lys Met Ser
        675                 680                 685
Glu Asn Glu Ser Leu Leu Ser Pro Glu Gln Lys Leu Arg Asp Gly Glu
    690                 695                 700
Leu His Asp Leu Ser Leu Gly Glu Ala Ser Met Glu Lys Asn Glu Pro
705                 710                 715                 720
Glu Leu Ala Leu Thr Val Asn Pro Glu Asn Leu Thr Glu Asn Gly Asp
                725                 730                 735
Asn Met Glu Ser Ser Cys Thr Ser Ser Cys Ala Gly Gly Ala Gln Trp
            740                 745                 750
Asp Val Phe Arg Arg Gln Asp Val Pro Lys Leu Ser Gly Tyr Leu Gln
        755                 760                 765
Arg Thr Phe Gln Lys Pro Asp Asn Ile Gln Thr Asp Phe Val Ser Arg
    770                 775                 780
Pro Leu Tyr Glu Gly Leu Phe Leu Asn Glu His His Lys Arg Gln Leu
785                 790                 795                 800
Arg Asp Glu Phe Gly Val Glu Pro Trp Thr Phe Glu Gln His Arg Gly
                805                 810                 815
Glu Ala Ile Phe Ile Pro Ala Gly Cys Pro Phe Gln Ile Thr Asn Leu
            820                 825                 830
Gln Ser Asn Ile Gln Val Ala Leu Asp Phe Leu Cys Pro Glu Ser Val
        835                 840                 845
Gly Glu Ser Ala Arg Leu Ala Glu Glu Ile Arg Cys Leu Pro Asn Asp
    850                 855                 860
His Glu Ala Lys Leu Gln Ile Leu Glu Ile Gly Lys Ile Ser Leu Tyr
865                 870                 875                 880
Ala Ala Ser Ser Ala Ile Lys Glu Val Gln Lys Leu Val Leu Asp Pro
                885                 890                 895
Lys Phe Gly Ala Glu Leu Gly Phe Glu Asp Ser Asn Leu Thr Lys Ala
            900                 905                 910
Val Ser His Asn Leu Asp Glu Ala Thr Lys Arg Pro Gln Gln Asn Ser
        915                 920                 925
Cys Thr
    930
```

<210> 99
<211> 2793
<212> DNA
<213> Arabidopsis thaliana

<400> 99

```
atgaatgcta atgagcaaac tcgatccgcc aatggcattg gcaatggcaa tggtgagtct     60
attcccggga ttccagatga cttacggtgc aagagatcgg atggtaaaca gtggagatgc    120
actgcaatgt ccatggctga taagactgtt tgtgagaagc actacatcca agcaaagaag    180
cgggcggcta attctgcttt cagggcgaac cagaagaaag cgaaaaggcg atcatcgtta    240
ggcgaaacag atacgtattc ggaagggaag atggatgatt tcgagttacc agtcaccagc    300
attgaccact ataataacgg tcttgcctct gcttccaaga gtaatggtag actagagaag    360
agacataata aaagcctgat gcggtactcg cccgagacac cgatgatgag gagtttctct    420
ccacgtgttg cagtggattt gaatgatgac ttgggtagag atgttgtaat gtttgaagag    480
ggctacagat cttataggac accaccatct gttgctgtta tggatccgac acgaaacaga    540
tcacaccaaa gcaccagtcc tatggaatac tcagcagcaa gcacagatgt gtctgcagag    600
tctttggggg aaatctgcca tcaatgccag agaaaagata gagagagaat catttcttgc    660

ctcaaatgca atcaaagagc cttctgccac aattgtctat cggcaaggta ctcggagata    720
tcacttgaag aagtcgagaa agtttgccct gcatgtcgtg gcttgtgtga ttgcaaatct    780
tgcctgcgtt cagataatac aataaaggtt cggatccggg aaatacccgt tttggacaag    840
ttgcagtatc tttatcgtct attatcagct gtcctaccag tcataaagca gatccatctt    900
gaacaatgta tggaagttga actagagaag aggcttcgtg aagttgagat tgatcttgtc    960
agggcaagat tgaaagcaga tgagcagatg tgctgcaacg tgtgtcggat accagttgtt   1020
gactactacc gtcactgtcc gaactgctca tatgaccttt gcctgagatg ctgtcaagat   1080
ctacgggaag agtcttcagt gacgattagt gggactaacc aaaacgtaca agatagaaaa   1140
ggagctccca aactaaaact aaacttttca tacaagtttc ctgagtggga agccaacggt   1200
gatgggagca tcccttgccc tcctaaggag tatggaggct gcggttcaca ttctttgaat   1260
cttgcccgca ttttcaagat gaattgggtt gcaaagcttg tgaaaaatgc tgaggagatt   1320
gttagtggct gcaaattatc tgatcttctg aaccctgata tgtgtgattc aagattctgc   1380
aaatttgctg agagagaaga gagcggtgac aactacgtgt acagcccgtc gcttgaaacg   1440
attaaaactg atggagtagc taagtttgag caacaatggg cagagggtcg gcttgttact   1500
gtgaaaatgg tacttgatga ctcatcttgc tctagatggg atcctgagac tatttggagg   1560
gatatagacg agctttcgga cgagaaactg agagaacatg atccattctt gaaggccatt   1620
aattgcttgg atggtttaga ggttgatgta agacttgggg agtttacaag agcatataaa   1680
gatggaaaga accaagagac aggtcttccg ctattgtgga agttaaagga ctggccgagc   1740
ccaagtgctt ccgaggagtt cattttctac caaagacctg agtttatcag aagttttccg   1800
tttctcgagt acattcatcc ccggttaggc cttctgaatg ttgcagccaa gttacctcat   1860
tactcgctcc aaaacgattc aggtccaaag atttatgtgt cttgtgggac gtaccaagaa   1920
atcagtgctg gcgattcatt gactggtatt cactacaaca tgcgtgacat ggtataccta   1980
ttggtgcaca cgtctgaaga aacaacattc gaaagggtga gaaaaacaaa acctgttcca   2040
gaggaacctg accagaagat gagcgaaaat gagtcacttc ttagccctga gcagaaatta   2100
agggacggag agttacatga tctatcactt ggtgaagcca gtatggagaa gaatgaacct   2160
gagttggcgt tgactgtgaa tccagagaac ttaacggaaa acggtgacaa catggaatct   2220
tcttgcacat cttcatgtgc aggaggagcc cagtgggatg tctttcgacg ccaagacgtc   2280
ccaaagttgt ccgggtattt gcagagaaca ttccagaagc ctgataatat ccagactgat   2340
tttgtgtcac gcccgttgta tgaaggattg ttcttaaatg aacaccacaa gagacaacta   2400
agagacgagt ttggagttga gccatggaca tttgagcaac atcgtggtga ggctatcttc   2460
attccggctg gatgtccgtt ccaaatcact aatcttcagt cgaatattca ggtggcactt   2520
gacttcttgt gccctgaaag cgttggagag tcagcaagac tagctgaaga aatccggtgt   2580
ttaccaaacg accacgaggc aaaacttcag attctagaga ttggaaagat atcattatac   2640
gcagctagct cagccattaa agaggttcag aaactggtct tggatccaaa gtttggagca   2700
gagcttggat ttgaagactc taacttaacc aaagcagtct ctcacaactt agacgaggca   2760
accaagcggc cgcagcaaaa cagctgcact taa                                2793
```

<210> 100
<211> 930
<212> PRT
<213> Arabidopsis thaliana

<400> 100

```
Met Asn Ala Asn Glu Gln Thr Arg Ser Ala Asn Gly Ile Gly Asn Gly
1               5                   10                  15
Asn Gly Glu Ser Ile Pro Gly Ile Pro Asp Asp Leu Arg Cys Lys Arg
            20                  25                  30
Ser Asp Gly Lys Gln Trp Arg Cys Thr Ala Met Ser Met Ala Asp Lys
        35                  40                  45
Thr Val Cys Glu Lys His Tyr Ile Gln Ala Lys Lys Arg Ala Ala Asn
    50                  55                  60
Ser Ala Phe Arg Ala Asn Gln Lys Lys Ala Lys Arg Arg Ser Ser Leu
65                  70                  75                  80
Gly Glu Thr Asp Thr Tyr Ser Glu Gly Lys Met Asp Asp Phe Glu Leu
                85                  90                  95
Pro Val Thr Ser Ile Asp His Tyr Asn Asn Gly Leu Ala Ser Ala Ser
            100                 105                 110
Lys Ser Asn Gly Arg Leu Glu Lys Arg His Asn Lys Ser Leu Met Arg
        115                 120                 125
Tyr Ser Pro Glu Thr Pro Met Met Arg Ser Phe Ser Pro Arg Val Ala
    130                 135                 140
```

```
Val Asp Leu Asn Asp Asp Leu Gly Arg Asp Val Val Met Phe Glu Glu
145                 150                 155                 160
Gly Tyr Arg Ser Tyr Arg Thr Pro Pro Ser Val Ala Val Met Asp Pro
                165                 170                 175
Thr Arg Asn Arg Ser His Gln Ser Thr Ser Pro Met Glu Tyr Ser Ala
            180                 185                 190
Ala Ser Thr Asp Val Ser Ala Glu Ser Leu Gly Glu Ile Cys His Gln
        195                 200                 205
Cys Gln Arg Lys Asp Arg Glu Arg Ile Ile Ser Cys Leu Lys Cys Asn
    210                 215                 220
Gln Arg Ala Phe Cys His Asn Cys Leu Ser Ala Arg Tyr Ser Glu Ile
225                 230                 235                 240
Ser Leu Glu Glu Val Glu Lys Val Cys Pro Ala Cys Arg Gly Leu Cys
                245                 250                 255
Asp Cys Lys Ser Cys Leu Arg Ser Asp Asn Thr Ile Lys Val Arg Ile
            260                 265                 270
Arg Glu Ile Pro Val Leu Asp Lys Leu Gln Tyr Leu Tyr Arg Leu Leu
        275                 280                 285
Ser Ala Val Leu Pro Val Ile Lys Gln Ile His Leu Glu Gln Cys Met
    290                 295                 300
Glu Val Glu Leu Glu Lys Arg Leu Arg Glu Val Glu Ile Asp Leu Val
305                 310                 315                 320
Arg Ala Arg Leu Lys Ala Asp Glu Gln Met Cys Cys Asn Val Cys Arg
                325                 330                 335
Ile Pro Val Val Asp Tyr Tyr Arg His Cys Pro Asn Cys Ser Tyr Asp
            340                 345                 350
Leu Cys Leu Arg Cys Cys Gln Asp Leu Arg Glu Glu Ser Ser Val Thr
        355                 360                 365
Ile Ser Gly Thr Asn Gln Asn Val Gln Asp Arg Lys Gly Ala Pro Lys
    370                 375                 380
Leu Lys Leu Asn Phe Ser Tyr Lys Phe Pro Glu Trp Glu Ala Asn Gly
385                 390                 395                 400
Asp Gly Ser Ile Pro Cys Pro Pro Lys Glu Tyr Gly Gly Cys Gly Ser
                405                 410                 415
His Ser Leu Asn Leu Ala Arg Ile Phe Lys Met Asn Trp Val Ala Lys
            420                 425                 430
Leu Val Lys Asn Ala Glu Glu Ile Val Ser Gly Cys Lys Leu Ser Asp
        435                 440                 445
Leu Leu Asn Pro Asp Met Cys Asp Ser Arg Phe Cys Lys Phe Ala Glu
    450                 455                 460
Arg Glu Glu Ser Gly Asp Asn Tyr Val Tyr Ser Pro Ser Leu Glu Thr
465                 470                 475                 480
Ile Lys Thr Asp Gly Val Ala Lys Phe Glu Gln Gln Trp Ala Glu Gly
                485                 490                 495
Arg Leu Val Thr Val Lys Met Val Leu Asp Asp Ser Ser Cys Ser Arg
            500                 505                 510
Trp Asp Pro Glu Thr Ile Trp Arg Asp Ile Asp Glu Leu Ser Asp Glu
        515                 520                 525
Lys Leu Arg Glu His Asp Pro Phe Leu Lys Ala Ile Asn Cys Leu Asp
    530                 535                 540
Gly Leu Glu Val Asp Val Arg Leu Gly Glu Phe Thr Arg Ala Tyr Lys
545                 550                 555                 560
Asp Gly Lys Asn Gln Glu Thr Gly Leu Pro Leu Leu Trp Lys Leu Lys
                565                 570                 575
Asp Trp Pro Ser Pro Ser Ala Ser Glu Glu Phe Ile Phe Tyr Gln Arg
            580                 585                 590
Pro Glu Phe Ile Arg Ser Phe Pro Phe Leu Glu Tyr Ile His Pro Arg
        595                 600                 605
Leu Gly Leu Leu Asn Val Ala Ala Lys Leu Pro His Tyr Ser Leu Gln
    610                 615                 620
Asn Asp Ser Gly Pro Lys Ile Tyr Val Ser Cys Gly Thr Tyr Gln Glu
```

```
625                 630                 635                 640
Ile Ser Ala Gly Asp Ser Leu Thr Gly Ile His Tyr Asn Met Arg Asp
                645                 650                 655
Met Val Tyr Leu Leu Val His Thr Ser Glu Glu Thr Thr Phe Glu Arg
            660                 665                 670
Val Arg Lys Thr Lys Pro Val Pro Glu Glu Pro Asp Gln Lys Met Ser
        675                 680                 685
Glu Asn Glu Ser Leu Leu Ser Pro Glu Gln Lys Leu Arg Asp Gly Glu
    690                 695                 700
Leu His Asp Leu Ser Leu Gly Glu Ala Ser Met Glu Lys Asn Glu Pro
705                 710                 715                 720
Glu Leu Ala Leu Thr Val Asn Pro Glu Asn Leu Thr Glu Asn Gly Asp
                725                 730                 735
Asn Met Glu Ser Ser Cys Thr Ser Ser Cys Ala Gly Gly Ala Gln Trp
            740                 745                 750
Asp Val Phe Arg Arg Gln Asp Val Pro Lys Leu Ser Gly Tyr Leu Gln
        755                 760                 765
Arg Thr Phe Gln Lys Pro Asp Asn Ile Gln Thr Asp Phe Val Ser Arg
    770                 775                 780
Pro Leu Tyr Glu Gly Leu Phe Leu Asn Glu His His Lys Arg Gln Leu
785                 790                 795                 800
Arg Asp Glu Phe Gly Val Glu Pro Trp Thr Phe Glu Gln His Arg Gly
                805                 810                 815
Glu Ala Ile Phe Ile Pro Ala Gly Cys Pro Phe Gln Ile Thr Asn Leu
            820                 825                 830
Gln Ser Asn Ile Gln Val Ala Leu Asp Phe Leu Cys Pro Glu Ser Val
        835                 840                 845
Gly Glu Ser Ala Arg Leu Ala Glu Glu Ile Arg Cys Leu Pro Asn Asp
    850                 855                 860
His Glu Ala Lys Leu Gln Ile Leu Glu Ile Gly Lys Ile Ser Leu Tyr
865                 870                 875                 880
Ala Ala Ser Ser Ala Ile Lys Glu Val Gln Lys Leu Val Leu Asp Pro
                885                 890                 895
Lys Phe Gly Ala Glu Leu Gly Phe Glu Asp Ser Asn Leu Thr Lys Ala
            900                 905                 910
Val Ser His Asn Leu Asp Glu Ala Thr Lys Arg Pro Gln Gln Asn Ser
        915                 920                 925
Cys Thr
    930
```

<210> 101
<211> 2835
<212> DNA
<213> Arabidopsis thaliana

<400> 101

```
atgcaggtca atttgatga aacttgtgat tcagtgatta gaatgaatgc taatgagcaa      60
actcgatccg ccaatggcat tggcaatggc aatggtgagt ctattcccgg gattccagat    120
gacttacggt gcaagagatc ggatggtaaa cagtggagat gcactgcaat gtccatggct    180
gataagactg tttgtgagaa gcactacatc caagcaaaga agcgggcggc taattctgct    240
ttcagggcga accagaagaa agcgaaaagg cgatcatcgt taggcgaaac agatacgtat    300
tcggaaggga agatggatga tttcgagtta ccagtcacca gcattgacca ctataataac    360
ggtcttgcct ctgcttccaa gagtaatggt agactagaga agagacataa taaaagcctg    420
atgcggtact cgcccgagac accgatgatg aggagtttct ctccacgtgt tgcagtggat    480
ttgaatgatg acttgggtag agatgttgta atgtttgaag agggctacag atcttatagg    540
acaccaccat ctgttgctgt tatggatccg acacgaaaca gatcacacca aagcaccagt    600
cctatggaat actcagcagc aagcacagat gtgtctgcag agtctttggg ggaaatctgc    660
catcaatgcc agagaaaaga tagagagaga atcatttctt gcctcaaatg caatcaaaga    720
gccttctgcc acaattgtct atcggcaagg tactcggaga tatcacttga agaagtcgag    780
aaagtttgcc ctgcatgtcg tggcttgtgt gattgcaaat cttgcctgcg ttcagataat    840
acaataaagg ttcggatccg ggaaataccc gttttggaca agttgcagta tctttatcgt    900
```

```
ctattatcag ctgtcctacc agtcataaag cagatccatc ttgaacaatg tatggaagtt     960
gaactagaga agaggcttcg tgaagttgag attgatcttg tcagggcaag attgaaagca    1020
gatgagcaga tgtgctgcaa cgtgtgtcgg ataccagttg ttgactacta ccgtcactgt    1080
ccgaactgct catatgacct ttgcctgaga tgctgtcaag atctacggga agagtcttca    1140
gtgacgatta gtgggactaa ccaaaacgta caagatagaa aaggagctcc caaactaaaa    1200
ctaaactttt catacaagtt tcctgagtgg gaagccaacg gtgatgggag catcccttgc    1260
cctcctaagg agtatggagg ctgcggttca cattctttga atcttgcccg cattttcaag    1320
atgaattggg ttgcaaagct tgtgaaaaat gctgaggaga ttgttagtgg ctgcaaatta    1380
tctgatcttc tgaaccctga tatgtgtgat tcaagattct gcaaatttgc tgagagagaa    1440
gagagcggtg acaactacgt gtacagcccg tcgcttgaaa cgattaaaac tgatggagta    1500
gctaagtttg agcaacaatg ggcagagggt cggcttgtta ctgtgaaaat ggtacttgat    1560
gactcatctt gctctagatg ggatcctgag actatttgga gggatataga cgagctttcg    1620
gacgagaaac tgagagaaca tgatccattc ttgaaggcca ttaattgctt ggatggttta    1680
gaggttgatg taagacttgg ggagtttaca agagcatata aagatggaaa gaaccaagag    1740
acaggtcttc cgctattgtg gaagttaaag gactggccga gcccaagtgc ttccgaggag    1800
ttcattttct accaaagacc tgagtttatc agaagttttc cgtttctcga gtacattcat    1860
ccccggttag gccttctgaa tgttgcagcc aagttacctc attactcgct ccaaaacgat    1920
tcaggtccaa agatttatgt gtcttgtggg acgtaccaag aaatcagtgc tggcgattca    1980
ttgactggta ttcactacaa catgcgtgac atggtatacc tattggtgca cacgtctgaa    2040
gaaacaacat tcgaaagggt gagaaaaaca aaacctgttc cagaggaacc tgaccagaag    2100
atgagcgaaa atgagtcact tcttagccct gagcagaaat taagggacgg agagttacat    2160
gatctatcac ttggtgaagc cagtatggag aagaatgaac ctgagttggc gttgactgtg    2220
aatccagaga acttaacgga aaacggtgac aacatggaat cttcttgcac atcttcatgt    2280
gcaggaggag cccagtggga tgtctttcga cgccaagacg tcccaaagtt gtccgggtat    2340
ttgcagagaa cattccagaa gcctgataat atccagactg attttgtgtc acgcccgttg    2400
tatgaaggat tgttcttaaa tgaacaccac aagagacaac taagagacga gtttggagtt    2460
gagccatgga catttgagca acatcgtggt gaggctatct tcattccggc tggatgtccg    2520
ttccaaatca ctaatcttca gtcgaatatt caggtggcac ttgacttctt gtgccctgaa    2580
agcgttggag agtcagcaag actagctgaa gaaatccggt gtttaccaaa cgaccacgag    2640
gcaaaacttc agattctaga gattggaaag atatcattat acgcagctag ctcagccatt    2700
aaagaggttc agaaactggt cttggatcca aagtttggag cagagcttgg atttgaagac    2760
tctaacttaa ccaaagcagt ctctcacaac ttagacgagg caaccaagcg gccgcagcaa    2820
aacagctgca cttaa                                                    2835
```

<210> 102
<211> 944
<212> PRT
<213> Arabidopsis thaliana

<400> 102

```
Met Gln Val Asn Phe Asp Glu Thr Cys Asp Ser Val Ile Arg Met Asn
1               5                   10                  15
Ala Asn Glu Gln Thr Arg Ser Ala Asn Gly Ile Gly Asn Gly Asn Gly
            20                  25                  30
Glu Ser Ile Pro Gly Ile Pro Asp Asp Leu Arg Cys Lys Arg Ser Asp
        35                  40                  45
Gly Lys Gln Trp Arg Cys Thr Ala Met Ser Met Ala Asp Lys Thr Val
    50                  55                  60
Cys Glu Lys His Tyr Ile Gln Ala Lys Lys Arg Ala Ala Asn Ser Ala
65                  70                  75                  80
Phe Arg Ala Asn Gln Lys Lys Ala Lys Arg Arg Ser Ser Leu Gly Glu
                85                  90                  95
Thr Asp Thr Tyr Ser Glu Gly Lys Met Asp Asp Phe Glu Leu Pro Val
            100                 105                 110
Thr Ser Ile Asp His Tyr Asn Asn Gly Leu Ala Ser Ala Ser Lys Ser
        115                 120                 125
Asn Gly Arg Leu Glu Lys Arg His Asn Lys Ser Leu Met Arg Tyr Ser
    130                 135                 140
Pro Glu Thr Pro Met Met Arg Ser Phe Ser Pro Arg Val Ala Val Asp
145                 150                 155                 160
Leu Asn Asp Asp Leu Gly Arg Asp Val Val Met Phe Glu Glu Gly Tyr
```

```
                165                 170                 175
Arg Ser Tyr Arg Thr Pro Pro Ser Val Ala Val Met Asp Pro Thr Arg
            180                 185                 190
Asn Arg Ser His Gln Ser Thr Ser Pro Met Glu Tyr Ser Ala Ala Ser
        195                 200                 205
Thr Asp Val Ser Ala Glu Ser Leu Gly Glu Ile Cys His Gln Cys Gln
    210                 215                 220
Arg Lys Asp Arg Glu Arg Ile Ile Ser Cys Leu Lys Cys Asn Gln Arg
225                 230                 235                 240
Ala Phe Cys His Asn Cys Leu Ser Ala Arg Tyr Ser Glu Ile Ser Leu
                245                 250                 255
Glu Glu Val Glu Lys Val Cys Pro Ala Cys Arg Gly Leu Cys Asp Cys
            260                 265                 270
Lys Ser Cys Leu Arg Ser Asp Asn Thr Ile Lys Val Arg Ile Arg Glu
        275                 280                 285
Ile Pro Val Leu Asp Lys Leu Gln Tyr Leu Tyr Arg Leu Leu Ser Ala
    290                 295                 300
Val Leu Pro Val Ile Lys Gln Ile His Leu Glu Gln Cys Met Glu Val
305                 310                 315                 320
Glu Leu Glu Lys Arg Leu Arg Glu Val Glu Ile Asp Leu Val Arg Ala
                325                 330                 335
Arg Leu Lys Ala Asp Glu Gln Met Cys Cys Asn Val Cys Arg Ile Pro
            340                 345                 350
Val Val Asp Tyr Tyr Arg His Cys Pro Asn Cys Ser Tyr Asp Leu Cys
        355                 360                 365
Leu Arg Cys Cys Gln Asp Leu Arg Glu Glu Ser Ser Val Thr Ile Ser
    370                 375                 380
Gly Thr Asn Gln Asn Val Gln Asp Arg Lys Gly Ala Pro Lys Leu Lys
385                 390                 395                 400
Leu Asn Phe Ser Tyr Lys Phe Pro Glu Trp Glu Ala Asn Gly Asp Gly
                405                 410                 415
Ser Ile Pro Cys Pro Pro Lys Glu Tyr Gly Gly Cys Gly Ser His Ser
            420                 425                 430
Leu Asn Leu Ala Arg Ile Phe Lys Met Asn Trp Val Ala Lys Leu Val
        435                 440                 445
Lys Asn Ala Glu Glu Ile Val Ser Gly Cys Lys Leu Ser Asp Leu Leu
    450                 455                 460
Asn Pro Asp Met Cys Asp Ser Arg Phe Cys Lys Phe Ala Glu Arg Glu
465                 470                 475                 480
Glu Ser Gly Asp Asn Tyr Val Tyr Ser Pro Ser Leu Glu Thr Ile Lys
                485                 490                 495
Thr Asp Gly Val Ala Lys Phe Glu Gln Gln Trp Ala Glu Gly Arg Leu
            500                 505                 510
Val Thr Val Lys Met Val Leu Asp Asp Ser Ser Cys Ser Arg Trp Asp
        515                 520                 525
Pro Glu Thr Ile Trp Arg Asp Ile Asp Glu Leu Ser Asp Glu Lys Leu
    530                 535                 540
Arg Glu His Asp Pro Phe Leu Lys Ala Ile Asn Cys Leu Asp Gly Leu
545                 550                 555                 560
Glu Val Asp Val Arg Leu Gly Glu Phe Thr Arg Ala Tyr Lys Asp Gly
                565                 570                 575
Lys Asn Gln Glu Thr Gly Leu Pro Leu Leu Trp Lys Leu Lys Asp Trp
            580                 585                 590
Pro Ser Pro Ser Ala Ser Glu Glu Phe Ile Phe Tyr Gln Arg Pro Glu
        595                 600                 605
Phe Ile Arg Ser Phe Pro Phe Leu Glu Tyr Ile His Pro Arg Leu Gly
    610                 615                 620
Leu Leu Asn Val Ala Ala Lys Leu Pro His Tyr Ser Leu Gln Asn Asp
625                 630                 635                 640
Ser Gly Pro Lys Ile Tyr Val Ser Cys Gly Thr Tyr Gln Glu Ile Ser
                645                 650                 655
```

```
Ala Gly Asp Ser Leu Thr Gly Ile His Tyr Asn Met Arg Asp Met Val
            660                 665                 670
Tyr Leu Leu Val His Thr Ser Glu Glu Thr Thr Phe Glu Arg Val Arg
        675                 680                 685
Lys Thr Lys Pro Val Pro Glu Glu Pro Asp Gln Lys Met Ser Glu Asn
    690                 695                 700
Glu Ser Leu Leu Ser Pro Glu Gln Lys Leu Arg Asp Gly Glu Leu His
705                 710                 715                 720
Asp Leu Ser Leu Gly Glu Ala Ser Met Glu Lys Asn Glu Pro Glu Leu
                725                 730                 735
Ala Leu Thr Val Asn Pro Glu Asn Leu Thr Glu Asn Gly Asp Asn Met
            740                 745                 750
Glu Ser Ser Cys Thr Ser Ser Cys Ala Gly Gly Ala Gln Trp Asp Val
        755                 760                 765
Phe Arg Arg Gln Asp Val Pro Lys Leu Ser Gly Tyr Leu Gln Arg Thr
    770                 775                 780
Phe Gln Lys Pro Asp Asn Ile Gln Thr Asp Phe Val Ser Arg Pro Leu
785                 790                 795                 800
Tyr Glu Gly Leu Phe Leu Asn Glu His His Lys Arg Gln Leu Arg Asp
                805                 810                 815
Glu Phe Gly Val Glu Pro Trp Thr Phe Glu Gln His Arg Gly Glu Ala
            820                 825                 830
Ile Phe Ile Pro Ala Gly Cys Pro Phe Gln Ile Thr Asn Leu Gln Ser
        835                 840                 845
Asn Ile Gln Val Ala Leu Asp Phe Leu Cys Pro Glu Ser Val Gly Glu
    850                 855                 860
Ser Ala Arg Leu Ala Glu Glu Ile Arg Cys Leu Pro Asn Asp His Glu
865                 870                 875                 880
Ala Lys Leu Gln Ile Leu Glu Ile Gly Lys Ile Ser Leu Tyr Ala Ala
                885                 890                 895
Ser Ser Ala Ile Lys Glu Val Gln Lys Leu Val Leu Asp Pro Lys Phe
            900                 905                 910
Gly Ala Glu Leu Gly Phe Glu Asp Ser Asn Leu Thr Lys Ala Val Ser
        915                 920                 925
His Asn Leu Asp Glu Ala Thr Lys Arg Pro Gln Gln Asn Ser Cys Thr
    930                 935                 940
```

<210> 103
<211> 2643
<212> DNA
<213> Arabidopsis thaliana

<400> 103

```
atggagggtg aagttgctac taacggagtg attctcaagc ataatggtgt aaaggatatt     60
tctttagaaa cttgttggcc agagaagaag aaaccggttg aggcaacgag tcttagcagt    120
ggttcatcag acatagaaga agagattagt gtggaatgtc ctaagcgagt ggctaatcaa    180
agacggaaac ggtctaaagc tgatgagatt aaaacgaaat cttcaagaaa gaggaaatgt    240
gatgatgaga acaagtgtga agagaatgag aagaagcaga ggagctctgt taaaaagaga    300
gcaactacat ggaaagaaga agaagttgtt gttgatgatg aaaagaaatg cgaacaacaa    360
ctccaactag ttccttccag caaggcaaca tctcgaagca ggagcaagaa atcagtcagt    420
gtggatactt ggttggttaa caacgagatt gatgttagcg ctttgagttc gcgttctgag    480
tctgaacttt cggattctta tctgaaaacc gagtacttta atgactgcag aagcatgaca    540
aggagtttaa aggcaaattt gggagagctt gcaatttgcc atcaatgctc taaaggggaa    600
agaagatatc ttttcatttg caccttttgt gaagtgaggt tgtattgttt tccatgcata    660
aagaaatggt atccccattt gtccacggat gatatccttg aaaaatgtcc cttttgccga    720
ggaacttgca actgctgcac atgtttgcac tctagtggtt taatcgagac atctaagaga    780
aagctcgata aatacgaaag attttatcat cttcgcttct tgattgtggc gatgcttcct    840
ttcctgaaaa agttatgcaa agcacaagat caagaaatcg aaaccgaggc taaggttcaa    900
gattcaatgg cttctcaagt tgatatatcc gagagtctat gctcaaatga agagcgtgtc    960
ttctgtaacc actgtgcaac atcaattgtt gacttgcatc gaagctgtcc aaagtgttca   1020
tatgaattgt gtctgaactg ttgccaagag atccgtggag gttggctttc tgaccgcccg   1080
```

```
gaatgtcagc tacaattcga gtacagaggg acccggtata tacatggcga agctgcagaa    1140
ccgagctctt cttctgtttc cgaggatgaa actaaaactc catccatcaa gtggaatgct    1200
gatgaaaatg gaagcatacg gtgtgcgcca aaagagctag gaggttgcgg ggactctgtg    1260
ctggagctta agcgaatctt accagtgact tggatgtcag atttggagca aaaggcagaa    1320
acattcttag cctcttatag tatcaaaccg ccaatgtcat attgtagatg ttcttctgat    1380
atgagtagta tgaagaggaa ggcagcttcg agggacggat ctagtgacaa ttacctttat    1440
tctcctgatt ctttggatgt cctgaagcaa gaagaacttc tgcatttcca agagcattgg    1500
tccaaaggtg aaccagtgat tgttcgaaat gctcttaaca acacagctgg tttaagctgg    1560
gagccaatgg tgatgtggcg ggctttatgt gagaatgttg attcagcaat tagctctaat    1620
atgtctgatg tcaaagctat cgattgttta gctaattgtg aggtgaagat aaatactcta    1680
tgtttctttg aaggatatag caaaggaaga acatatgaaa acttctggcc tgagatgctg    1740
aagctgaagg attggcctcc ttcagacaag tttgaaaacc ttttacctcg tcactgcgat    1800
gagttcattt ccgcattacc atttcaagaa tacagcgatc ctagatcagg aattctcaac    1860
attgctacaa aacttcctga aggacttctt aaaccagatc ttggtccaaa aacttatgtt    1920
gcatatggga cttcagatga gctcggtaga ggagattcag tcactaagct tcactgtgat    1980
atgtcagatg ctgtaagttt cagaattgtt aatattctga tgcatactgc tgaagtaaca    2040
ctaagtgagg agcaaaggtc tgcaattgca gatttgaaac agaaacataa gcaacagaat    2100
gagaaagagc ttcaggagca aaatggttta gaggaagaag aggttgtgag tgatgagatt    2160
gtggtttatg atgaaacaag tggtgcactt tgggacatct ttaaaagaga agatgttcct    2220
aagcttgaag agtatctaag gaagcattgc atagaattca gacatactta ttgctctcgt    2280
gttacaaagg tgtatcatcc aatacatgat cagtcatatt tcttaaccgt ggagcataaa    2340
agaaaactca aggcagaatt tgggattgag ccatggacat ttgtgcaaaa gctaggagaa    2400
gcagtgttta ttccagcagg ttgtcctcat caagttcgga atctcaagtc gtgcacaaag    2460
gtagctgttg attttgtttc ccctgaaaac atcgatgagt gtctccgttt gactgatgag    2520
tttcgacaac tccctaagaa ccataaagcc agagaagaca aacttgagat aaaaaagatg    2580
gtgatctatg cagttgaaca agctctgaaa gaagtggaga cattattgct agatcgcagc    2640
taa                                                                  2643
```

<210> 104
<211> 880
<212> PRT
<213> Arabidopsis thaliana

<400> 104

```
Met Glu Gly Glu Val Ala Thr Asn Gly Val Ile Leu Lys His Asn Gly
1               5                   10                  15
Val Lys Asp Ile Ser Leu Glu Thr Cys Trp Pro Glu Lys Lys Lys Pro
            20                  25                  30
Val Glu Ala Thr Ser Leu Ser Ser Gly Ser Ser Asp Ile Glu Glu Glu
        35                  40                  45
Ile Ser Val Glu Cys Pro Lys Arg Val Ala Asn Gln Arg Arg Lys Arg
    50                  55                  60
Ser Lys Ala Asp Glu Ile Lys Thr Lys Ser Ser Arg Lys Arg Lys Cys
65                  70                  75                  80
Asp Asp Glu Asn Lys Cys Glu Glu Asn Glu Lys Lys Gln Arg Ser Ser
                85                  90                  95
Val Lys Lys Arg Ala Thr Thr Trp Lys Glu Glu Glu Val Val Val Asp
            100                 105                 110
Asp Glu Lys Lys Cys Glu Gln Gln Leu Gln Leu Val Pro Ser Ser Lys
        115                 120                 125
Ala Thr Ser Arg Ser Arg Ser Lys Lys Ser Val Ser Val Asp Thr Trp
    130                 135                 140
Leu Val Asn Asn Glu Ile Asp Val Ser Ala Leu Ser Ser Arg Ser Glu
145                 150                 155                 160
Ser Glu Leu Ser Asp Ser Tyr Leu Lys Thr Glu Tyr Phe Asn Asp Cys
                165                 170                 175
Arg Ser Met Thr Arg Ser Leu Lys Ala Asn Leu Gly Glu Leu Ala Ile
            180                 185                 190
Cys His Gln Cys Ser Lys Gly Glu Arg Arg Tyr Leu Phe Ile Cys Thr
        195                 200                 205
Phe Cys Glu Val Arg Leu Tyr Cys Phe Pro Cys Ile Lys Lys Trp Tyr
```

```
    210                 215                 220
Pro His Leu Ser Thr Asp Asp Ile Leu Glu Lys Cys Pro Phe Cys Arg
225                 230                 235                 240
Gly Thr Cys Asn Cys Cys Thr Cys Leu His Ser Ser Gly Leu Ile Glu
                245                 250                 255
Thr Ser Lys Arg Lys Leu Asp Lys Tyr Glu Arg Phe Tyr His Leu Arg
            260                 265                 270
Phe Leu Ile Val Ala Met Leu Pro Phe Leu Lys Lys Leu Cys Lys Ala
        275                 280                 285
Gln Asp Gln Glu Ile Glu Thr Glu Ala Lys Val Gln Asp Ser Met Ala
    290                 295                 300
Ser Gln Val Asp Ile Ser Glu Ser Leu Cys Ser Asn Glu Glu Arg Val
305                 310                 315                 320
Phe Cys Asn His Cys Ala Thr Ser Ile Val Asp Leu His Arg Ser Cys
                325                 330                 335
Pro Lys Cys Ser Tyr Glu Leu Cys Leu Asn Cys Cys Gln Glu Ile Arg
            340                 345                 350
Gly Gly Trp Leu Ser Asp Arg Pro Glu Cys Gln Leu Gln Phe Glu Tyr
        355                 360                 365
Arg Gly Thr Arg Tyr Ile His Gly Glu Ala Ala Glu Pro Ser Ser Ser
    370                 375                 380
Ser Val Ser Glu Asp Glu Thr Lys Thr Pro Ser Ile Lys Trp Asn Ala
385                 390                 395                 400
Asp Glu Asn Gly Ser Ile Arg Cys Ala Pro Lys Glu Leu Gly Gly Cys
                405                 410                 415
Gly Asp Ser Val Leu Glu Leu Lys Arg Ile Leu Pro Val Thr Trp Met
            420                 425                 430
Ser Asp Leu Glu Gln Lys Ala Glu Thr Phe Leu Ala Ser Tyr Ser Ile
        435                 440                 445
Lys Pro Pro Met Ser Tyr Cys Arg Cys Ser Ser Asp Met Ser Ser Met
    450                 455                 460
Lys Arg Lys Ala Ala Ser Arg Asp Gly Ser Ser Asp Asn Tyr Leu Tyr
465                 470                 475                 480
Ser Pro Asp Ser Leu Asp Val Leu Lys Gln Glu Glu Leu Leu His Phe
                485                 490                 495
Gln Glu His Trp Ser Lys Gly Glu Pro Val Ile Val Arg Asn Ala Leu
            500                 505                 510
Asn Asn Thr Ala Gly Leu Ser Trp Glu Pro Met Val Met Trp Arg Ala
        515                 520                 525
Leu Cys Glu Asn Val Asp Ser Ala Ile Ser Ser Asn Met Ser Asp Val
    530                 535                 540
Lys Ala Ile Asp Cys Leu Ala Asn Cys Glu Val Lys Ile Asn Thr Leu
545                 550                 555                 560
Cys Phe Phe Glu Gly Tyr Ser Lys Gly Arg Thr Tyr Glu Asn Phe Trp
                565                 570                 575
Pro Glu Met Leu Lys Leu Lys Asp Trp Pro Pro Ser Asp Lys Phe Glu
            580                 585                 590
Asn Leu Leu Pro Arg His Cys Asp Glu Phe Ile Ser Ala Leu Pro Phe
        595                 600                 605
Gln Glu Tyr Ser Asp Pro Arg Ser Gly Ile Leu Asn Ile Ala Thr Lys
    610                 615                 620
Leu Pro Glu Gly Leu Leu Lys Pro Asp Leu Gly Pro Lys Thr Tyr Val
625                 630                 635                 640
Ala Tyr Gly Thr Ser Asp Glu Leu Gly Arg Gly Asp Ser Val Thr Lys
                645                 650                 655
Leu His Cys Asp Met Ser Asp Ala Val Ser Phe Arg Ile Val Asn Ile
            660                 665                 670
Leu Met His Thr Ala Glu Val Thr Leu Ser Glu Glu Gln Arg Ser Ala
        675                 680                 685
Ile Ala Asp Leu Lys Gln Lys His Lys Gln Gln Asn Glu Lys Glu Leu
    690                 695                 700
```

```
Gln Glu Gln Asn Gly Leu Glu Glu Glu Glu Val Val Ser Asp Glu Ile
705                 710                 715                 720
Val Val Tyr Asp Glu Thr Ser Gly Ala Leu Trp Asp Ile Phe Lys Arg
                725                 730                 735
Glu Asp Val Pro Lys Leu Glu Glu Tyr Leu Arg Lys His Cys Ile Glu
            740                 745                 750
Phe Arg His Thr Tyr Cys Ser Arg Val Thr Lys Val Tyr His Pro Ile
        755                 760                 765
His Asp Gln Ser Tyr Phe Leu Thr Val Glu His Lys Arg Lys Leu Lys
    770                 775                 780
Ala Glu Phe Gly Ile Glu Pro Trp Thr Phe Val Gln Lys Leu Gly Glu
785                 790                 795                 800
Ala Val Phe Ile Pro Ala Gly Cys Pro His Gln Val Arg Asn Leu Lys
                805                 810                 815
Ser Cys Thr Lys Val Ala Val Asp Phe Val Ser Pro Glu Asn Ile Asp
            820                 825                 830
Glu Cys Leu Arg Leu Thr Asp Glu Phe Arg Gln Leu Pro Lys Asn His
        835                 840                 845
Lys Ala Arg Glu Asp Lys Leu Glu Ile Lys Lys Met Val Ile Tyr Ala
    850                 855                 860
Val Glu Gln Ala Leu Lys Glu Val Glu Thr Leu Leu Leu Asp Arg Ser
865                 870                 875                 880
```

<210> 105
<211> 2364
<212> DNA
<213> Arabidopsis thaliana

<400> 105

```
atggaaaatc ctccattaga atctgagatc aaagaggata tgtctttgaa gaatcatcct     60
ccagacaagg ataaggacaa agacactatc atggaacaac ctagtagtcc acgtcatcgg    120
aaggttgttg ctagatggtt acctgatgaa gcacagaggc caattatcaa tgacgctccc    180
gtattcactc catcactaga ggagtttgta gatccacttg cgtatatcga gaagatacgt    240
ccactagcag agccgtatgg tatctgtcga atcatcccac catcgacatg gaagcctcct    300
tgccggctta aggagaagag tatatgggag cagacaaagt ttcctactcg gattcaaacc    360
gtagacctgc ttcagaaccg tgagccgatg aagaagaaac cgaagagtag aaagcggaaa    420
cggagaagaa actcgagaat gggttcgtct aagagacgat ctggttcttc tcccgctgaa    480
tcgacttcat cacccgaggc tgaggagaag tttggtttca attctggttc agacttcact    540
cttgatgagt ttgagaaata tgctctgcat tttaaagact cgtactttga aaagaaagac    600
tctggtggag atatagtaaa atggacaccg tctgtggatg atatagaagg ggaatactgg    660
cggatagttg agcaaccaac agatgaagtg gaggtttact atggagctga cttggagaat    720
ggggtgcttg gaagcgggtt ttataaaaga gccgagaagt ttaccggtag cgatatggag    780
cagtacacat tatctggctg gaacttgaat aacttaccac ggcttcctgg ctctgtactc    840
tcttttgaag attgtgacat atccggagtt ctagttccat ggctatatgt gggaatgtgt    900
ttttcatcat tttgttggca tgtggaggac catcatttat attcacttaa ctatcatcac    960
tttggggagc caaaagtatg gtatggtgtt ccaggaagca atgcaaccgc tctcgagaag   1020
gcgatgagga aacatttacc tgacttgttc gaagacagcc tgatctacta catggcctgg   1080
gcgtatcatg ctggattcaa ctgcggtttc aactgcgcag aagcggttaa tgtggctccg   1140
gttgattggt tggctcatgg acagaacgct gtggaactat atagtaaaga gacaaggaag   1200
acttctctgt ctcacgacaa gcttcttctt ggagcagctt atgaagcggt gaaggctctt   1260
tgggaactct cagcttctga gggaaaggaa aatacaacaa atttgagatg gaagagtttc   1320
tgtgggaaga acgggacact taccaacgcg atacaagctc ggttacaaat ggaagagggg   1380
agaattacag ctcttggtag ggattcttca agcttgaaga agatggagaa ggactttgat   1440
tcaaactgtg aaagggaatg tttctcatgc ttttatgatt tgcatctctc ggcttctggc   1500
tgcaagtgct ctcccgaaga atacgcgtgc cttaaacatg cagatgatct ttgttcatgt   1560
gatgtgaaag atggatttat tcttctccgg tacacaatgg atgagttaag ctcgttggtc   1620
agagcattgg aaggggaatc agatgattta aagatatggg cttccaaggt tttaggcatt   1680
gaacatagtg atgaagatca gacaaagact agttcagtta tcagtgagga gaagaagtta   1740
aaggaaggtt cttttgatct gaacattgac ttagagatgg attatcaaga agacgttaaa   1800
gaagaagcca gcactagtgg cggcgagtta accgcctcag agaaccttgg tgtatcggtc   1860
gagcctataa acctcgggtt cttgattttt ggaaagcttt ggtgcaataa gtatgctata   1920

ttcccaaaag gattcaggag tcgtgtcaag ttctacaatg ttcttgatcc aacaagaatg   1980
agcaattaca tctctgaggt tttggatgca ggactcatgg gtccattgtt cagggttact   2040
ctggaagaat ctccagacga gagcttcttc aatgtctctg cacaacaatg ctgggaaatg   2100
gtgatgcgga gagttaaaga tacatcaaca agtcttggtc ttcctatttt accacagttt   2160
gagagtatca acgggcttca aatgtttggt ttcctctcac cctctatagt tcaggccatt   2220
gaagctcttg acccaaacca tcgactagtt gagtactgga accacaagaa ccaaacttca   2280
tcagactcaa aagatcactt catatcatca aactgttccg caagtttaac caaaggaaaa   2340
cttttcggag tagatttgat gtaa                                           2364
```

<210> 106
<211> 787
<212> PRT
<213> Arabidopsis thaliana

<400> 106

```
Met Glu Asn Pro Pro Leu Glu Ser Glu Ile Lys Glu Asp Met Ser Leu
1               5                   10                  15
Lys Asn His Pro Pro Asp Lys Asp Lys Asp Lys Asp Thr Ile Met Glu
            20                  25                  30
Gln Pro Ser Ser Pro Arg His Arg Lys Val Val Ala Arg Trp Leu Pro
        35                  40                  45
Asp Glu Ala Gln Arg Pro Ile Ile Asn Asp Ala Pro Val Phe Thr Pro
    50                  55                  60
Ser Leu Glu Glu Phe Val Asp Pro Leu Ala Tyr Ile Glu Lys Ile Arg
65                  70                  75                  80
Pro Leu Ala Glu Pro Tyr Gly Ile Cys Arg Ile Ile Pro Pro Ser Thr
                85                  90                  95
Trp Lys Pro Pro Cys Arg Leu Lys Glu Lys Ser Ile Trp Glu Gln Thr
            100                 105                 110
Lys Phe Pro Thr Arg Ile Gln Thr Val Asp Leu Leu Gln Asn Arg Glu
        115                 120                 125
Pro Met Lys Lys Lys Pro Lys Ser Arg Lys Arg Lys Arg Arg Arg Asn
    130                 135                 140
Ser Arg Met Gly Ser Ser Lys Arg Arg Ser Gly Ser Ser Pro Ala Glu
145                 150                 155                 160
Ser Thr Ser Ser Pro Glu Ala Glu Glu Lys Phe Gly Phe Asn Ser Gly
                165                 170                 175
Ser Asp Phe Thr Leu Asp Glu Phe Glu Lys Tyr Ala Leu His Phe Lys
            180                 185                 190
Asp Ser Tyr Phe Glu Lys Lys Asp Ser Gly Gly Asp Ile Val Lys Trp
        195                 200                 205
Thr Pro Ser Val Asp Asp Ile Glu Gly Glu Tyr Trp Arg Ile Val Glu
    210                 215                 220
Gln Pro Thr Asp Glu Val Glu Val Tyr Tyr Gly Ala Asp Leu Glu Asn
225                 230                 235                 240
Gly Val Leu Gly Ser Gly Phe Tyr Lys Arg Ala Glu Lys Phe Thr Gly
                245                 250                 255
Ser Asp Met Glu Gln Tyr Thr Leu Ser Gly Trp Asn Leu Asn Asn Leu
            260                 265                 270
Pro Arg Leu Pro Gly Ser Val Leu Ser Phe Glu Asp Cys Asp Ile Ser
        275                 280                 285
Gly Val Leu Val Pro Trp Leu Tyr Val Gly Met Cys Phe Ser Ser Phe
    290                 295                 300
Cys Trp His Val Glu Asp His His Leu Tyr Ser Leu Asn Tyr His His
305                 310                 315                 320
Phe Gly Glu Pro Lys Val Trp Tyr Gly Val Pro Gly Ser Asn Ala Thr
                325                 330                 335
Ala Leu Glu Lys Ala Met Arg Lys His Leu Pro Asp Leu Phe Glu Asp

            340                 345                 350
Ser Leu Ile Tyr Tyr Met Ala Trp Ala Tyr His Ala Gly Phe Asn Cys
```

355 360 365
Gly Phe Asn Cys Ala Glu Ala Val Asn Val Ala Pro Val Asp Trp Leu
370 375 380
Ala His Gly Gln Asn Ala Val Glu Leu Tyr Ser Lys Glu Thr Arg Lys
385 390 395 400
Thr Ser Leu Ser His Asp Lys Leu Leu Leu Gly Ala Ala Tyr Glu Ala
405 410 415
Val Lys Ala Leu Trp Glu Leu Ser Ala Ser Glu Gly Lys Glu Asn Thr
420 425 430
Thr Asn Leu Arg Trp Lys Ser Phe Cys Gly Lys Asn Gly Thr Leu Thr
435 440 445
Asn Ala Ile Gln Ala Arg Leu Gln Met Glu Glu Gly Arg Ile Thr Ala
450 455 460
Leu Gly Arg Asp Ser Ser Ser Leu Lys Lys Met Glu Lys Asp Phe Asp
465 470 475 480
Ser Asn Cys Glu Arg Glu Cys Phe Ser Cys Phe Tyr Asp Leu His Leu
485 490 495
Ser Ala Ser Gly Cys Lys Cys Ser Pro Glu Glu Tyr Ala Cys Leu Lys
500 505 510
His Ala Asp Asp Leu Cys Ser Cys Asp Val Lys Asp Gly Phe Ile Leu
515 520 525
Leu Arg Tyr Thr Met Asp Glu Leu Ser Ser Leu Val Arg Ala Leu Glu
530 535 540
Gly Glu Ser Asp Asp Leu Lys Ile Trp Ala Ser Lys Val Leu Gly Ile
545 550 555 560
Glu His Ser Asp Glu Asp Gln Thr Lys Thr Ser Ser Val Ile Ser Glu
565 570 575
Glu Lys Lys Leu Lys Glu Gly Ser Phe Asp Leu Asn Ile Asp Leu Glu
580 585 590
Met Asp Tyr Gln Glu Asp Val Lys Glu Glu Ala Ser Thr Ser Gly Gly
595 600 605
Glu Leu Thr Ala Ser Glu Asn Leu Gly Val Ser Val Glu Pro Ile Asn
610 615 620
Leu Gly Phe Leu Ile Phe Gly Lys Leu Trp Cys Asn Lys Tyr Ala Ile
625 630 635 640
Phe Pro Lys Gly Phe Arg Ser Arg Val Lys Phe Tyr Asn Val Leu Asp
645 650 655
Pro Thr Arg Met Ser Asn Tyr Ile Ser Glu Val Leu Asp Ala Gly Leu
660 665 670
Met Gly Pro Leu Phe Arg Val Thr Leu Glu Glu Ser Pro Asp Glu Ser
675 680 685
Phe Phe Asn Val Ser Ala Gln Gln Cys Trp Glu Met Val Met Arg Arg
690 695 700
Val Lys Asp Thr Ser Thr Ser Leu Gly Leu Pro Ile Leu Pro Gln Phe
705 710 715 720
Glu Ser Ile Asn Gly Leu Gln Met Phe Gly Phe Leu Ser Pro Ser Ile
725 730 735
Val Gln Ala Ile Glu Ala Leu Asp Pro Asn His Arg Leu Val Glu Tyr
740 745 750
Trp Asn His Lys Asn Gln Thr Ser Ser Asp Ser Lys Asp His Phe Ile
755 760 765
Ser Ser Asn Cys Ser Ala Ser Leu Thr Lys Gly Lys Leu Phe Gly Val
770 775 780
Asp Leu Met
785

<210> 107
<211> 2652
<212> DNA
<213> Arabidopsis thaliana

<400> 107
atggattctg gagttaaatt ggagcatatg aattgtttcc aattatctta tcaatattcg 60
tggacgacta ggaagaagag aactctgaaa ccattcatgt caaaaggttc tagccctagt 120
agtagtagtg atagtaggaa acggaagctt tcgagagctg aagatagcga tgattcagcg 180
gttaaacgga atgcgaaacg aaggagaaag atatgcaaag tagaagaata ttatgaagat 240
gatgattgca tattaagtga ttgggttcag aggaatactg cgaaaaggat tgataagcgc 300
aacgaagaag ttgaagttat ggttaagata gagtcaggtg atgattgcac aataggtaag 360
tggttttctg atgttagtag taaaaggaaa gataaacgac aagtagaagt tgatgaagat 420
gaggaatggg aagaagaagt tacactgtgt tctaagatca aggcaacgtc gtcgcgaagc 480
agaacccata gtttaagtgc taacagtcca gaaaatgtta cagatgttat tagtccttgt 540
cgttcacggt ctccagcttc taatgtatca gattccattc agaaaaatga ctgcaccagt 600
agtagaaaac agagtggccc aatttgtcat cagtgcttga aaggggaaag gataacactt 660
ctcatttgta gtgaatgtga gaagacaatg ttttgtcttc aatgtataag gaaatggtat 720
ccaaatctat ctgaggatga tgttgttgag aaatgtcctt tatgccgtca aaattgtaat 780
tgtagcaaat gcttgcattt gaatggttta attgagacat cgaagaggga actcgcgaaa 840
tctgaaagac gtcatcatct ccagtatttg attacgttga tgcttccttt tctgaataag 900
ttatccatat tccagaagct agagattgaa tttgaggcaa ctgttcaagg aaaactgcct 960
tctgaagttg aaataactgc ggctataagc tacactgatg aacgtgtata ctgtgatcat 1020
tgtgcaactt caattgttga cttgcatcga agctgtccaa agtgctctta tgaactttgt 1080
ttgaagtgct gtcaagagat tcgtgaagga tcgctttctg aacgccctga aatgaagttc 1140
cattatgttg atagaggaca tcgatatatg catggtttag atgctgcgga accgagcttg 1200
tcttccactt ttgaagacga agaagctaac ccatctgacg ctaagtggag ccttggtgaa 1260
aatggaagca tcacttgtgc accagaaaag ctaggtggtt gtggtgaacg gatgctagag 1320
cttaggcgga tcctaccact cacgtggatg tcagatttag agcacaaagc agagactttc 1380
ttatcatcat acaatataag ccctagaatg ttgaactgta ggtgttcttc tttggagaca 1440
gagctgacaa ggaaatcagc ttcgaggaca acatcaagtg acaactacct tttctgtcct 1500
gaatctctcg gtgtcttgaa ggaagaagag cttcttcatt ttcaagagca ttgggcaaaa 1560
ggcgaaccag taatcgttag gaacgctctt gacaacacac ctggtttaag ctgggagccg 1620
atggttatgt ggcgggcttt atgcgaaaat gtgaattcaa catcaagctc tgagatgtcc 1680
caagtcaaag caattgattg cttagctaat tgtgaggtgg agatcaatac tcgtcagttc 1740
tttgaaggtt atagcaaagg aagaacatat gaaaatttct ggcctgagat gttgaagctt 1800
aaggactggc ctccttctga taagtttgaa gatcttctac ctcgtcactg tgatgagttc 1860
atatccgcgt tacctttcca ggaatatagt gatcctagaa ctggaattct taacattgca 1920
acaaagcttc ctgaaggatt tatcaaacca gatttaggtc caaaaactta catcgcctat 1980
gggattccag atgagcttgg tagaggcgat tccgtgacta agcttcactg cgatatgtca 2040
gacgcggtga atattctaac gcatacggcc gaagtgaccc taagtcaaga acaaatatcc 2100
tcagtcaaag cactgaaaca gaaacacaag ttacagaata aggtcgataa acagagtact 2160
gaagactgta acgaaaagga agaagaagaa gaagaagaat tgaacatgcc agagatttcg 2220
agcaatgaaa atgaagaaac gggcagtgct ctttgggaca tatttaggag agaagacgtt 2280
cccaagttag aagagtatct aagaaagcat tgcaaagaat ttagacacac ttattgtagt 2340
ccagttacaa aggtttatca tccgatacat gaccaatcat gctatttaac cttagagcac 2400
aaaagaaaac tcaaggcgga atatgggatc gagccatgga catttgtgca aaagctaggt 2460
gaagcggtgt ttattcccgc gggttgtcct catcaagttc ggaatctaaa gtcgtgcaca 2520
aaagtcgcag ttgactttgt gtcaccagag aacatccatg agtgtctgcg tttgaccgaa 2580
gagtttcgcc aacttcccaa aaaccacaag gccagagagg acaaactcga ggcaagtctt 2640
ttatctcttt ga 2652

<210> 108
<211> 883
<212> PRT
<213> Arabidopsis thaliana

<400> 108

```
Met Asp Ser Gly Val Lys Leu Glu His Met Asn Cys Phe Gln Leu Ser
1               5                   10                  15
Tyr Gln Tyr Ser Trp Thr Thr Arg Lys Lys Arg Thr Leu Lys Pro Phe
            20                  25                  30
Met Ser Lys Gly Ser Ser Pro Ser Ser Ser Ser Asp Ser Arg Lys Arg
        35                  40                  45
Lys Leu Ser Arg Ala Glu Asp Ser Asp Asp Ser Ala Val Lys Arg Asn
    50                  55                  60
```

```
Ala Lys Arg Arg Arg Lys Ile Cys Lys Val Glu Glu Tyr Tyr Glu Asp
65                  70                  75                  80
Asp Asp Cys Ile Leu Ser Asp Trp Val Gln Arg Asn Thr Ala Lys Arg
                85                  90                  95
Ile Asp Lys Arg Asn Glu Glu Val Glu Val Met Val Lys Ile Glu Ser
            100                 105                 110
Gly Asp Asp Cys Thr Ile Gly Lys Trp Phe Ser Asp Val Ser Ser Lys
        115                 120                 125
Arg Lys Asp Lys Arg Gln Val Glu Val Asp Glu Asp Glu Glu Trp Glu
    130                 135                 140
Glu Glu Val Thr Leu Cys Ser Lys Ile Lys Ala Thr Ser Ser Arg Ser
145                 150                 155                 160
Arg Thr His Ser Leu Ser Ala Asn Ser Pro Glu Asn Val Thr Asp Val
                165                 170                 175
Ile Ser Pro Cys Arg Ser Arg Ser Pro Ala Ser Asn Val Ser Asp Ser
            180                 185                 190
Ile Gln Lys Asn Asp Cys Thr Ser Ser Arg Lys Gln Ser Gly Pro Ile
        195                 200                 205
Cys His Gln Cys Leu Lys Gly Glu Arg Ile Thr Leu Leu Ile Cys Ser
    210                 215                 220
Glu Cys Glu Lys Thr Met Phe Cys Leu Gln Cys Ile Arg Lys Trp Tyr
225                 230                 235                 240
Pro Asn Leu Ser Glu Asp Asp Val Val Glu Lys Cys Pro Leu Cys Arg
                245                 250                 255
Gln Asn Cys Asn Cys Ser Lys Cys Leu His Leu Asn Gly Leu Ile Glu
            260                 265                 270
Thr Ser Lys Arg Glu Leu Ala Lys Ser Glu Arg Arg His His Leu Gln
        275                 280                 285
Tyr Leu Ile Thr Leu Met Leu Pro Phe Leu Asn Lys Leu Ser Ile Phe
    290                 295                 300
Gln Lys Leu Glu Ile Glu Phe Glu Ala Thr Val Gln Gly Lys Leu Pro
305                 310                 315                 320
Ser Glu Val Glu Ile Thr Ala Ala Ile Ser Tyr Thr Asp Glu Arg Val
                325                 330                 335
Tyr Cys Asp His Cys Ala Thr Ser Ile Val Asp Leu His Arg Ser Cys
            340                 345                 350
Pro Lys Cys Ser Tyr Glu Leu Cys Leu Lys Cys Cys Gln Glu Ile Arg
        355                 360                 365
Glu Gly Ser Leu Ser Glu Arg Pro Glu Met Lys Phe His Tyr Val Asp
    370                 375                 380
Arg Gly His Arg Tyr Met His Gly Leu Asp Ala Ala Glu Pro Ser Leu
385                 390                 395                 400
Ser Ser Thr Phe Glu Asp Glu Glu Ala Asn Pro Ser Asp Ala Lys Trp
                405                 410                 415
Ser Leu Gly Glu Asn Gly Ser Ile Thr Cys Ala Pro Glu Lys Leu Gly
            420                 425                 430
Gly Cys Gly Glu Arg Met Leu Glu Leu Arg Arg Ile Leu Pro Leu Thr
        435                 440                 445
Trp Met Ser Asp Leu Glu His Lys Ala Glu Thr Phe Leu Ser Ser Tyr
    450                 455                 460
Asn Ile Ser Pro Arg Met Leu Asn Cys Arg Cys Ser Ser Leu Glu Thr
465                 470                 475                 480
Glu Leu Thr Arg Lys Ser Ala Ser Arg Thr Thr Ser Ser Asp Asn Tyr
                485                 490                 495
Leu Phe Cys Pro Glu Ser Leu Gly Val Leu Lys Glu Glu Glu Leu Leu
            500                 505                 510
His Phe Gln Glu His Trp Ala Lys Gly Glu Pro Val Ile Val Arg Asn
        515                 520                 525
Ala Leu Asp Asn Thr Pro Gly Leu Ser Trp Glu Pro Met Val Met Trp
    530                 535                 540
Arg Ala Leu Cys Glu Asn Val Asn Ser Thr Ser Ser Ser Glu Met Ser
```

```
545                 550                 555                 560
Gln Val Lys Ala Ile Asp Cys Leu Ala Asn Cys Glu Val Glu Ile Asn
                565                 570                 575
Thr Arg Gln Phe Phe Glu Gly Tyr Ser Lys Gly Arg Thr Tyr Glu Asn
            580                 585                 590
Phe Trp Pro Glu Met Leu Lys Leu Lys Asp Trp Pro Pro Ser Asp Lys
        595                 600                 605
Phe Glu Asp Leu Leu Pro Arg His Cys Asp Glu Phe Ile Ser Ala Leu
    610                 615                 620
Pro Phe Gln Glu Tyr Ser Asp Pro Arg Thr Gly Ile Leu Asn Ile Ala
625                 630                 635                 640
Thr Lys Leu Pro Glu Gly Phe Ile Lys Pro Asp Leu Gly Pro Lys Thr
                645                 650                 655
Tyr Ile Ala Tyr Gly Ile Pro Asp Glu Leu Gly Arg Gly Asp Ser Val
            660                 665                 670
Thr Lys Leu His Cys Asp Met Ser Asp Ala Val Asn Ile Leu Thr His
        675                 680                 685
Thr Ala Glu Val Thr Leu Ser Gln Glu Gln Ile Ser Ser Val Lys Ala
    690                 695                 700
Leu Lys Gln Lys His Lys Leu Gln Asn Lys Val Asp Lys Gln Ser Thr
705                 710                 715                 720
Glu Asp Cys Asn Glu Lys Glu Glu Glu Glu Glu Glu Glu Leu Asn Met
                725                 730                 735
Pro Glu Ile Ser Ser Asn Glu Asn Glu Glu Thr Gly Ser Ala Leu Trp
            740                 745                 750
Asp Ile Phe Arg Arg Glu Asp Val Pro Lys Leu Glu Glu Tyr Leu Arg
        755                 760                 765
Lys His Cys Lys Glu Phe Arg His Thr Tyr Cys Ser Pro Val Thr Lys
    770                 775                 780
Val Tyr His Pro Ile His Asp Gln Ser Cys Tyr Leu Thr Leu Glu His
785                 790                 795                 800
Lys Arg Lys Leu Lys Ala Glu Tyr Gly Ile Glu Pro Trp Thr Phe Val
                805                 810                 815
Gln Lys Leu Gly Glu Ala Val Phe Ile Pro Ala Gly Cys Pro His Gln
            820                 825                 830
Val Arg Asn Leu Lys Ser Cys Thr Lys Val Ala Val Asp Phe Val Ser
        835                 840                 845
Pro Glu Asn Ile His Glu Cys Leu Arg Leu Thr Glu Glu Phe Arg Gln
    850                 855                 860
Leu Pro Lys Asn His Lys Ala Arg Glu Asp Lys Leu Glu Ala Ser Leu
865                 870                 875                 880
Leu Ser Leu
```

<210> 109
<211> 3351
<212> DNA
<213> Arabidopsis thaliana

<400> 109

```
atgtatggta atgacttgga tacttctgtt tacgggagtg gttttcctcg aataggcgac     60
caaagaccag aatcagttga ggcagatata tgggatgagt attgtggtag cccctggaat    120
ctcaataaca tgcctaagtt gaaaggatct atgcttcagg ccattcgaca taacattaat    180
ggtgttacag tgccttggct atatcttgga atgctcttct cttcttttg ttggcatttt    240
gaggaccatt gtttttactc tgtcaattat ctacactggg gagaggcaaa atgttggtat    300
ggtattccag gcagtgctgc tagtgctttt gaaaaggtca tgcgaaaaac cctacccgat    360
ctctttgatg ctcagccaga tttgctcttt caactagtta ccatgttgag tccgactgtt    420
ttacaagaaa ataaagtccc ggtctacaca gtattacagg agccaggaaa cttcgtgatc    480
acatttccaa aatcctttca tgctggattc aattttggtc taaattgtgc agaggccgtc    540
aactttgcta ctgctgattg gctaccttat ggtggttctg ggcggagct gtatcggctg    600
taccggaaac cttcagtcat atctcatgaa gagcttctct gcgtggtagc taagggaaac    660
```

```
tgctgcaata acgaagggtc aatacatttg aagaaagaat tgctcagaat atacagcaag     720
gaaaaaacct ggagagagca gctctggaaa agtggtattt tgagatcttc tcctatgttt     780
gtgcctgaat gcgctgattc tgtgggcatc gaagaggatc caacatgcat catatgccag     840
cagtttcttc atctttctgc tatcgtctgc aattgcaggc catctgtttt tgcatgctta     900
gagcactgga agcatctttg tgaatgtgaa cctacgaagt tgcgacttga atatcgttat     960
acccttgccg agttagatat gatggtacaa gaagttgaaa agtttggtgg gtgcaaaaca    1020
caagaaacca aaatctcaca acggccgagt tcaggcacca aacgatctat tgctttaaat    1080
aaaaagcagg agggaatgca agtgagtcag gcacggccag cagacaaatg gcttcttaga    1140
gcatcaaagg ttcttgacgc tgcgttttct agtgttgaat atgccaccct tttgaaggaa    1200
tcagaacagt ttctttgggc tggatcagaa atggaccgtg tacgagatgt tacaaaaagt    1260
ttgaacaaag caaagatatg ggctgaagct gttagtgact gtctttcaaa agtcgaaggt    1320
gaagtcaacg atgattcaat gaaagttcac ttggaattta ttgatgagtt gctgagagtt    1380
aatcctgttc cttgctttaa ttctggttat ctgaaactaa aggactatgc tgaagaagct    1440
agaaaattgt ccgagaaaat cgattcagct ctctcgagta gcccaactat cacccagctg    1500
gagctattgc attctgaagt ctccagatcg ccaatctccc taaaaaaaca cgagatcttg    1560
tcaaagaaaa tctcttccgc aaagatgtta gctaaaaggg cgaaacgcta tctcacagac    1620
gctaaacctc caggaattga aatggatgca cttttcaagc taaattcaga gatgttagag    1680
cttcatgttc aacttccaga aacagaaggg attctggatt tggtaaagaa atcagaatca    1740
gcccgtgata aaagtaacaa agttttgact ggttctttat ctctcgagaa tgtggaagag    1800
ttgcttcatg aattcgatag tttcagcatt aacgttcctg agttgaatat cctgaggcag    1860
tatcatgttg acactttgtc ttggatttca cgctttaatg atgtaatggt tgatgttcgt    1920
gaaggcaagg accaacgaaa gctaattagt gacctcagtt cccttctacg ggatggagca    1980
tccttaggca ttcaagttga aggactccct cttgttgaag ttgaattgaa gaaggcatct    2040
tgccgggaaa aagctcgaac ggtttatact gcaagaaaat ctctggattt cattgagcaa    2100
ctgctctcgg aagctgttat actacacatc gaagaggagg agatatttgt tgagatctcg    2160
ggaattttgt caacagcacg gtgttgggag gaaagagcaa gcactatcct tgaaaatgaa    2220
actcagatgt acgagcttaa agatctcgta aggatgtcag tcaacattga tgcggtttta    2280
cctactctgc aaggcataga gaacacaatc tcgtcggctg aaacttggct tcagaaatct    2340
gagccttttc tatcagccac ttcctctatg gcatcatctc catgttctat gcttgaactt    2400
cctgtattaa aggatctagt tactcaggct aaattgctca atgttcagct tcaagagcca    2460
cgaattcttg aaacattgtt gcttaactgc gagaggtggc agtgtgataa tcatcagctc    2520
ttgcaagaaa ctgaagattt gttggacaat gcgaaaatag atgatggcac gcatagcaat    2580
atccttccga agataatgga tttgataacc agagtggact ccgctaggag atctggtctg    2640
gcccttggtc tcaatttcga tgaacttccc aaacttcgaa ctgcaagtct aaaactagga    2700
tggtgttgta agaccatcac gttaagctct agctcaccta cctctgagtt gctagaagat    2760
gttggaaagc cctcgttaca gcatattcag cagcacttaa aagagggaca aacacttgaa    2820
atattacctg aagagtatta cttaggcaag agactcatgg agttaaaaga cactggactg    2880
gagtgggcaa aacgagctag aaaggtggta acagactcag gtgctcttgc cttggaagat    2940
gttttcgagc ttatttctga gggtgaaaac ttacctgttc atgcagagca ggaacttcag    3000
tctttacgag ctcggagtat gttacactgc atttgtctaa agccatacaa ctcaagatcc    3060
atggtttctt gtagtcaatg tggcgaatgg tatcacacct attgtttaaa gcttcattgg    3120
cggcctaagg cttatgtctg ctccgcttgc tgtccactgg cagaaaccac tccacagatc    3180
gatcccgcca gagcaacaga gccagagaga ccgtctctga accaaagacg gacaagaatg    3240
gtcgcgactg atgcagcagt taatgactta aagtggaaaa cccgaaaaca catcaaacgg    3300
acaactaaac ggagtcctca ggttcatatt cttccctggt tttcactta a              3351
```

<210> 110
<211> 1116
<212> PRT
<213> Arabidopsis thaliana

<400> 110

```
Met Tyr Gly Asn Asp Leu Asp Thr Ser Val Tyr Gly Ser Gly Phe Pro
1               5                   10                  15
Arg Ile Gly Asp Gln Arg Pro Glu Ser Val Glu Ala Asp Ile Trp Asp
            20                  25                  30
Glu Tyr Cys Gly Ser Pro Trp Asn Leu Asn Asn Met Pro Lys Leu Lys
        35                  40                  45
Gly Ser Met Leu Gln Ala Ile Arg His Asn Ile Asn Gly Val Thr Val
    50                  55                  60
Pro Trp Leu Tyr Leu Gly Met Leu Phe Ser Ser Phe Cys Trp His Phe
```

```
65                  70                  75                  80
Glu Asp His Cys Phe Tyr Ser Val Asn Tyr Leu His Trp Gly Glu Ala
                85                  90                  95
Lys Cys Trp Tyr Gly Ile Pro Gly Ser Ala Ala Ser Ala Phe Glu Lys
            100                 105                 110
Val Met Arg Lys Thr Leu Pro Asp Leu Phe Asp Ala Gln Pro Asp Leu
        115                 120                 125
Leu Phe Gln Leu Val Thr Met Leu Ser Pro Thr Val Leu Gln Glu Asn
    130                 135                 140
Lys Val Pro Val Tyr Thr Val Leu Gln Glu Pro Gly Asn Phe Val Ile
145                 150                 155                 160
Thr Phe Pro Lys Ser Phe His Ala Gly Phe Asn Phe Gly Leu Asn Cys
                165                 170                 175
Ala Glu Ala Val Asn Phe Ala Thr Ala Asp Trp Leu Pro Tyr Gly Gly
            180                 185                 190
Ser Gly Ala Glu Leu Tyr Arg Leu Tyr Arg Lys Pro Ser Val Ile Ser
        195                 200                 205
His Glu Glu Leu Leu Cys Val Val Ala Lys Gly Asn Cys Cys Asn Asn
    210                 215                 220
Glu Gly Ser Ile His Leu Lys Lys Glu Leu Leu Arg Ile Tyr Ser Lys
225                 230                 235                 240
Glu Lys Thr Trp Arg Glu Gln Leu Trp Lys Ser Gly Ile Leu Arg Ser
                245                 250                 255
Ser Pro Met Phe Val Pro Glu Cys Ala Asp Ser Val Gly Ile Glu Glu
            260                 265                 270
Asp Pro Thr Cys Ile Ile Cys Gln Gln Phe Leu His Leu Ser Ala Ile
        275                 280                 285
Val Cys Asn Cys Arg Pro Ser Val Phe Ala Cys Leu Glu His Trp Lys
    290                 295                 300
His Leu Cys Glu Cys Glu Pro Thr Lys Leu Arg Leu Glu Tyr Arg Tyr
305                 310                 315                 320
Thr Leu Ala Glu Leu Asp Met Met Val Gln Glu Val Glu Lys Phe Gly
                325                 330                 335
Gly Cys Lys Thr Gln Glu Thr Lys Ile Ser Gln Arg Pro Ser Ser Gly
            340                 345                 350
Thr Lys Arg Ser Ile Ala Leu Asn Lys Lys Gln Glu Gly Met Gln Val
        355                 360                 365
Ser Gln Ala Arg Pro Ala Asp Lys Trp Leu Leu Arg Ala Ser Lys Val
    370                 375                 380
Leu Asp Ala Ala Phe Ser Ser Val Glu Tyr Ala Thr Leu Leu Lys Glu
385                 390                 395                 400
Ser Glu Gln Phe Leu Trp Ala Gly Ser Glu Met Asp Arg Val Arg Asp
                405                 410                 415
Val Thr Lys Ser Leu Asn Lys Ala Lys Ile Trp Ala Glu Ala Val Ser
            420                 425                 430
Asp Cys Leu Ser Lys Val Glu Gly Glu Val Asn Asp Asp Ser Met Lys
        435                 440                 445
Val His Leu Glu Phe Ile Asp Glu Leu Leu Arg Val Asn Pro Val Pro
    450                 455                 460
Cys Phe Asn Ser Gly Tyr Leu Lys Leu Lys Asp Tyr Ala Glu Glu Ala
465                 470                 475                 480
Arg Lys Leu Ser Glu Lys Ile Asp Ser Ala Leu Ser Ser Ser Pro Thr
                485                 490                 495
Ile Thr Gln Leu Glu Leu Leu His Ser Glu Val Ser Arg Ser Pro Ile
            500                 505                 510
Ser Leu Lys Lys His Glu Ile Leu Ser Lys Lys Ile Ser Ser Ala Lys
        515                 520                 525
Met Leu Ala Lys Arg Ala Lys Arg Tyr Leu Thr Asp Ala Lys Pro Pro
    530                 535                 540
Gly Ile Glu Met Asp Ala Leu Phe Lys Leu Asn Ser Glu Met Leu Glu
545                 550                 555                 560
```

Leu His Val Gln Leu Pro Glu Thr Glu Gly Ile Leu Asp Leu Val Lys
565 570 575
Lys Ser Glu Ser Ala Arg Asp Lys Ser Asn Lys Val Leu Thr Gly Ser
580 585 590
Leu Ser Leu Glu Asn Val Glu Glu Leu Leu His Glu Phe Asp Ser Phe
595 600 605
Ser Ile Asn Val Pro Glu Leu Asn Ile Leu Arg Gln Tyr His Val Asp
610 615 620
Thr Leu Ser Trp Ile Ser Arg Phe Asn Asp Val Met Val Asp Val Arg
625 630 635 640
Glu Gly Lys Asp Gln Arg Lys Leu Ile Ser Asp Leu Ser Ser Leu Leu
645 650 655
Arg Asp Gly Ala Ser Leu Gly Ile Gln Val Glu Gly Leu Pro Leu Val
660 665 670
Glu Val Glu Leu Lys Lys Ala Ser Cys Arg Glu Lys Ala Arg Thr Val
675 680 685
Tyr Thr Ala Arg Lys Ser Leu Asp Phe Ile Glu Gln Leu Leu Ser Glu
690 695 700
Ala Val Ile Leu His Ile Glu Glu Glu Glu Ile Phe Val Glu Ile Ser
705 710 715 720
Gly Ile Leu Ser Thr Ala Arg Cys Trp Glu Glu Arg Ala Ser Thr Ile
725 730 735
Leu Glu Asn Glu Thr Gln Met Tyr Glu Leu Lys Asp Leu Val Arg Met
740 745 750
Ser Val Asn Ile Asp Ala Val Leu Pro Thr Leu Gln Gly Ile Glu Asn
755 760 765
Thr Ile Ser Ser Ala Glu Thr Trp Leu Gln Lys Ser Glu Pro Phe Leu
770 775 780
Ser Ala Thr Ser Ser Met Ala Ser Ser Pro Cys Ser Met Leu Glu Leu
785 790 795 800
Pro Val Leu Lys Asp Leu Val Thr Gln Ala Lys Leu Leu Asn Val Gln
805 810 815
Leu Gln Glu Pro Arg Ile Leu Glu Thr Leu Leu Leu Asn Cys Glu Arg
820 825 830
Trp Gln Cys Asp Asn His Gln Leu Leu Gln Glu Thr Glu Asp Leu Leu
835 840 845
Asp Asn Ala Lys Ile Asp Asp Gly Thr His Ser Asn Ile Leu Pro Lys
850 855 860
Ile Met Asp Leu Ile Thr Arg Val Asp Ser Ala Arg Arg Ser Gly Leu
865 870 875 880
Ala Leu Gly Leu Asn Phe Asp Glu Leu Pro Lys Leu Arg Thr Ala Ser
885 890 895
Leu Lys Leu Gly Trp Cys Cys Lys Thr Ile Thr Leu Ser Ser Ser Ser
900 905 910
Pro Thr Ser Glu Leu Leu Glu Asp Val Gly Lys Pro Ser Leu Gln His
915 920 925
Ile Gln Gln His Leu Lys Glu Gly Gln Thr Leu Glu Ile Leu Pro Glu
930 935 940
Glu Tyr Tyr Leu Gly Lys Arg Leu Met Glu Leu Lys Asp Thr Gly Leu
945 950 955 960
Glu Trp Ala Lys Arg Ala Arg Lys Val Val Thr Asp Ser Gly Ala Leu
965 970 975
Ala Leu Glu Asp Val Phe Glu Leu Ile Ser Glu Gly Glu Asn Leu Pro
980 985 990
Val His Ala Glu Gln Glu Leu Gln Ser Leu Arg Ala Arg Ser Met Leu
995 1000 1005
His Cys Ile Cys Leu Lys Pro Tyr Asn Ser Arg Ser Met Val Ser
1010 1015 1020
Cys Ser Gln Cys Gly Glu Trp Tyr His Thr Tyr Cys Leu Lys Leu
1025 1030 1035

```
His Trp Arg Pro Lys Ala Tyr Val Cys Ser Ala Cys Cys Pro Leu
    1040                1045                1050
Ala Glu Thr Thr Pro Gln Ile Asp Pro Ala Arg Ala Thr Glu Pro
    1055                1060                1065
Glu Arg Pro Ser Leu Asn Gln Arg Arg Thr Arg Met Val Ala Thr
    1070                1075                1080
Asp Ala Ala Val Asn Asp Leu Lys Trp Lys Thr Arg Lys His Ile
    1085                1090                1095
Lys Arg Thr Thr Lys Arg Ser Pro Gln Val His Ile Leu Pro Trp
    1100                1105                1110
Phe Phe Thr
    1115
```

<210> 111
<211> 2751
<212> DNA
<213> Arabidopsis thaliana

<400> 111

```
atgacgacgt taggacaaag agatcgccga ccagatgctc tcggtagtct cagtgttcta     60
ccagatgaga ccatctgtgt tcttctcgaa taccttgctc cccgagatat tgctcacctc    120
gcttgtgtca gcagtgtgat gtatattcta tgtaatgaag aaccattgtg gatgagtttg    180
tgtctcagaa gagcaaaagg tcctcttgaa tacaaaggtt cttggaaaaa aacaacattg    240
catctagaag gagttactca agaaaatgac gcatacagaa aatgttttca ttttgatgga    300
ttcatgtcgt tgtacttgta taaacgattc tataggtgta atacatctct tgacgggttc    360
tcttttgata atgggaatgt ggaacgtagg agaaatatct ccttggatga gttttctaag    420
gaatacgacg ccaagaaacc tgttttgctt tctggccttg ctgattcttg gccagccagt    480
aatacgtgga ctatcgacca actctcagag aaatatggtg aagtcccgtt tagaatatct    540
cagaggagcc ccaacaaaat ttccatgaag ttcaaggatt acatcgcata tatgaaaact    600
cagcgggatg aagatcctct atacgttttc gatgacaagt ttggagaagc tgctccagaa    660
ttattgaaag actatagtgt gccccatttg tttcaagaag attggtttga gatcttagac    720
aaggaaagtc gtcctccata cagatggctt atagttggtc cggagaggtc tggtgcatct    780
tggcatgttg acccagctct taccagcgcc tggaacaccc tgctttgcgg tcggaaaagg    840
tgggcattgt atccccctgg aaaagtgcct ctaggtgtta cagtccatgt caatgaagat    900
gagggtgatg tcagcattga tacaccctca tctctgcagt ggtggctaga ctattatccc    960
cttcttgcgg acgaagacaa accgattgag tgcacactac tacctggcga aacgatttat   1020
gttccaagtg gttggtggca ctgtatcctt aatcttgaac caacagtggc tgttacccag   1080
aattttgtga acaaagaaaa ctttgggttc gtgtgcttgg atatggcgcc tggttaccat   1140
cacaaaggag tttgccgtgc tgggcttctt gctcttgatg atgaaaattc tgaagatttg   1200
gaagaagaaa cacacgatga agaagataat actttgagct attcagacct taccaggaaa   1260
gagaagagga cacggatgaa tggaggtgga gagactgaaa accgcgaaga ggatgtgaat   1320
ggagtatcga agagatataa tatgtggaag aatggatttt catatgatat cgatttcctc   1380
gcttcatttc ttgataaaga aagagatcat tacaatttcc catggtcaat ggggaactct   1440
gtaggccaac gagaaatgag agcctggcta tccaagcttt gggttctgaa gcctgagatg   1500
agagaactaa tatggaaggg agcatgcatt gctttaaatg ctgagaaatg gttgcgatgc   1560
ctagaggaag tatgtacttt ccacaacttg ccgttggtta ccgaagatga aaagcttcca   1620
gttggaactg gcagcaaccc tcttgagttt tatgacattc ttggccgggc tgattctcct   1680
ttgaaaacac atattcctga agttctagca agcgggattc ttttcttcga aaaaggatct   1740
tacaaagttg tcccttggga tggcaagaga atcccagata ttatctctag ctccagtttc   1800
gattttgatg catccatgtt aaacagcgaa ttcccatttg gtatttggaa taaaacgcta   1860
cgtgaacata aaaaccaggg gaagccagca cccgattctt ttggttcatt gagttcacat   1920
gtttggccat atattataac caaaagatgc aaagggaaga tttttgccca actaagagat   1980
gatctaacct ggaacgacgc tcagaacctg gctttctttc tcggacaaca actacgcaac   2040
cttcatctac ttccatatcc accggtcaca cgtccggagt tgttgaatgt gaatgctgtt   2100
catgaggagt tgaacattcc agcagaatgg aaagtttttg tcgatgctct gtgccaaaag   2160
aagaaggacg tcactagtcg tctggaaaac tggggaaatc cgatacctcg ggctctgatg   2220
accaagatag acgaatacat tcccgatgac ttttttgtag atttactcca cgtcttcaag   2280
gagacaaatg gtggagatga aatcaagccc tgcacctgga tacactcaga cgtaatggat   2340
gacaacattc acatggaacc atacgcagat gattcagttg atggtcaaca caactcatgg   2400
cgtcccagtc atattcttga cttcagcgat ttaaccatag gagatcccat ctgcgacttg   2460
```

```
ataccaatat acttggacgt ctttagagga gacgctgatc ttcttaagaa gcttctagaa    2520
aattatggac ttccattaat cagaagtaga tcttcagaga acggaacaac aaaaacagca    2580
gacagtacga ggaagaaagt attgtctcca tcctaccgta caatgtgtta ctgtatatta    2640
catgaagaaa atgtgttggg ttcaatattc agtatttggg acgaacttcg gactgctgaa    2700
tcatgggaac aagttgagca aactgtttgg agtctactta acacctacta a             2751
```

<210> 112
<211> 916
<212> PRT
<213> Arabidopsis thaliana

<400> 112

```
Met Thr Thr Leu Gly Gln Arg Asp Arg Arg Pro Asp Ala Leu Gly Ser
1               5                   10                  15
Leu Ser Val Leu Pro Asp Glu Thr Ile Cys Val Leu Leu Glu Tyr Leu
            20                  25                  30
Ala Pro Arg Asp Ile Ala His Leu Ala Cys Val Ser Ser Val Met Tyr
        35                  40                  45
Ile Leu Cys Asn Glu Glu Pro Leu Trp Met Ser Leu Cys Leu Arg Arg
    50                  55                  60
Ala Lys Gly Pro Leu Glu Tyr Lys Gly Ser Trp Lys Lys Thr Thr Leu
65                  70                  75                  80
His Leu Glu Gly Val Thr Gln Glu Asn Asp Ala Tyr Arg Lys Cys Phe
                85                  90                  95
His Phe Asp Gly Phe Met Ser Leu Tyr Leu Tyr Lys Arg Phe Tyr Arg
            100                 105                 110
Cys Asn Thr Ser Leu Asp Gly Phe Ser Phe Asp Asn Gly Asn Val Glu
        115                 120                 125
Arg Arg Arg Asn Ile Ser Leu Asp Glu Phe Ser Lys Glu Tyr Asp Ala
    130                 135                 140
Lys Lys Pro Val Leu Leu Ser Gly Leu Ala Asp Ser Trp Pro Ala Ser
145                 150                 155                 160
Asn Thr Trp Thr Ile Asp Gln Leu Ser Glu Lys Tyr Gly Glu Val Pro
                165                 170                 175
Phe Arg Ile Ser Gln Arg Ser Pro Asn Lys Ile Ser Met Lys Phe Lys
            180                 185                 190
Asp Tyr Ile Ala Tyr Met Lys Thr Gln Arg Asp Glu Asp Pro Leu Tyr
        195                 200                 205
Val Phe Asp Asp Lys Phe Gly Glu Ala Ala Pro Glu Leu Leu Lys Asp
    210                 215                 220
Tyr Ser Val Pro His Leu Phe Gln Glu Asp Trp Phe Glu Ile Leu Asp
225                 230                 235                 240
Lys Glu Ser Arg Pro Pro Tyr Arg Trp Leu Ile Val Gly Pro Glu Arg
                245                 250                 255
Ser Gly Ala Ser Trp His Val Asp Pro Ala Leu Thr Ser Ala Trp Asn
            260                 265                 270
Thr Leu Leu Cys Gly Arg Lys Arg Trp Ala Leu Tyr Pro Pro Gly Lys
        275                 280                 285
Val Pro Leu Gly Val Thr Val His Val Asn Glu Asp Glu Gly Asp Val
    290                 295                 300
Ser Ile Asp Thr Pro Ser Ser Leu Gln Trp Trp Leu Asp Tyr Tyr Pro
305                 310                 315                 320
Leu Leu Ala Asp Glu Asp Lys Pro Ile Glu Cys Thr Leu Leu Pro Gly
                325                 330                 335
Glu Thr Ile Tyr Val Pro Ser Gly Trp Trp His Cys Ile Leu Asn Leu
            340                 345                 350
Glu Pro Thr Val Ala Val Thr Gln Asn Phe Val Asn Lys Glu Asn Phe
        355                 360                 365
Gly Phe Val Cys Leu Asp Met Ala Pro Gly Tyr His His Lys Gly Val
    370                 375                 380
Cys Arg Ala Gly Leu Leu Ala Leu Asp Asp Glu Asn Ser Glu Asp Leu
```

```
385                 390                 395                 400
Glu Glu Glu Thr His Asp Glu Glu Asp Asn Thr Leu Ser Tyr Ser Asp
                405                 410                 415
Leu Thr Arg Lys Glu Lys Arg Thr Arg Met Asn Gly Gly Gly Glu Thr

            420                 425                 430
Glu Asn Arg Glu Glu Asp Val Asn Gly Val Ser Lys Arg Tyr Asn Met
        435                 440                 445
Trp Lys Asn Gly Phe Ser Tyr Asp Ile Asp Phe Leu Ala Ser Phe Leu
    450                 455                 460
Asp Lys Glu Arg Asp His Tyr Asn Phe Pro Trp Ser Met Gly Asn Ser
465                 470                 475                 480
Val Gly Gln Arg Glu Met Arg Ala Trp Leu Ser Lys Leu Trp Val Leu
                485                 490                 495
Lys Pro Glu Met Arg Glu Leu Ile Trp Lys Gly Ala Cys Ile Ala Leu
            500                 505                 510
Asn Ala Glu Lys Trp Leu Arg Cys Leu Glu Glu Val Cys Thr Phe His
        515                 520                 525
Asn Leu Pro Leu Val Thr Glu Asp Glu Lys Leu Pro Val Gly Thr Gly
    530                 535                 540
Ser Asn Pro Leu Glu Phe Tyr Asp Ile Leu Gly Arg Ala Asp Ser Pro
545                 550                 555                 560
Leu Lys Thr His Ile Pro Glu Val Leu Ala Ser Gly Ile Leu Phe Phe
                565                 570                 575
Glu Lys Gly Ser Tyr Lys Val Val Pro Trp Asp Gly Lys Arg Ile Pro
            580                 585                 590
Asp Ile Ile Ser Ser Ser Ser Phe Asp Phe Asp Ala Ser Met Leu Asn
        595                 600                 605
Ser Glu Phe Pro Phe Gly Ile Trp Asn Lys Thr Leu Arg Glu His Lys
    610                 615                 620
Asn Gln Gly Lys Pro Ala Pro Asp Ser Phe Gly Ser Leu Ser Ser His
625                 630                 635                 640
Val Trp Pro Tyr Ile Ile Thr Lys Arg Cys Lys Gly Lys Ile Phe Ala
                645                 650                 655
Gln Leu Arg Asp Asp Leu Thr Trp Asn Asp Ala Gln Asn Leu Ala Phe
            660                 665                 670
Phe Leu Gly Gln Gln Leu Arg Asn Leu His Leu Leu Pro Tyr Pro Pro
        675                 680                 685
Val Thr Arg Pro Glu Leu Leu Asn Val Asn Ala Val His Glu Glu Leu
    690                 695                 700
Asn Ile Pro Ala Glu Trp Lys Val Phe Val Asp Ala Leu Cys Gln Lys
705                 710                 715                 720
Lys Lys Asp Val Thr Ser Arg Leu Glu Asn Trp Gly Asn Pro Ile Pro
                725                 730                 735
Arg Ala Leu Met Thr Lys Ile Asp Glu Tyr Ile Pro Asp Asp Phe Phe
            740                 745                 750
Val Asp Leu Leu His Val Phe Lys Glu Thr Asn Gly Gly Asp Glu Ile
        755                 760                 765
Lys Pro Cys Thr Trp Ile His Ser Asp Val Met Asp Asp Asn Ile His
    770                 775                 780
Met Glu Pro Tyr Ala Asp Asp Ser Val Asp Gly Gln His Asn Ser Trp
785                 790                 795                 800
Arg Pro Ser His Ile Leu Asp Phe Ser Asp Leu Thr Ile Gly Asp Pro
                805                 810                 815
Ile Cys Asp Leu Ile Pro Ile Tyr Leu Asp Val Phe Arg Gly Asp Ala
            820                 825                 830
Asp Leu Leu Lys Lys Leu Leu Glu Asn Tyr Gly Leu Pro Leu Ile Arg
        835                 840                 845
Ser Arg Ser Ser Glu Asn Gly Thr Thr Lys Thr Ala Asp Ser Thr Arg
    850                 855                 860
Lys Lys Val Leu Ser Pro Ser Tyr Arg Thr Met Cys Tyr Cys Ile Leu
```

```
865                 870                 875                 880
His Glu Glu Asn Val Leu Gly Ser Ile Phe Ser Ile Trp Asp Glu Leu
                885                 890                 895
Arg Thr Ala Glu Ser Trp Glu Gln Val Glu Gln Thr Val Trp Ser Leu
            900                 905                 910
Leu Asn Thr Tyr
        915
```

<210> 113
<211> 2421
<212> DNA
<213> Arabidopsis thaliana

<400> 113

```
atggagcctt tcagtgctgc tcagaacaaa gaggacaagg acactagcgt agaacctcct      60
cgtaggcgtt gtcatcgaaa gaacaagggc actaatgtag aacctcctag tagtccgtat     120
catccgaagg ttcttgctag atgggatccg gctaatgaaa agaggccaga cattggtgaa     180
gctccagtat tccaccctac atcagaggag tttgaagata cgcttgctta catagaaaaa     240
atacgtccct tagctgaatc atttggtatt tgtcgaatcg tgccaccgtc gaattggtct     300
cctccttgcc ggcttaaagg ggatagtata tggaagaata aaaatttccc aacccgtgtt     360
cagtttgttg acctgcttca gaacagagga cccgtgaaga agaagacacc taaaggaagg     420
aaacggaaac gaggaaaata ctcaagaacc gtcgccccca agaaacgaaa tggctctgtt     480
tctaaatcag tttctacccc caaggcgacc gaagaagaga attttggttt cgaatctggt     540
ccagaattta ctcttgagaa gtttgagaaa tacgctcaag atttcaaaga ctcctacttt     600
gaaagaaaag acaatgttgg agatccatct gttgaggaga tcgaaggaga gtactggaga     660
atcattgaga aagaaacaaa tgaagttaag gtattgtatg ggacggattt ggagaatccc     720
atacttggaa gcggtttctc caaaggggta aaaattccaa ccagaagaaa tgatatggat     780
aaatacatat cttctggttg gaatttgaac aacctcgccc gtctgcaggg atctctacta     840
tcctttgagg attgtgagat ctcaggggtt caggtgccat ggctctatgt gggcatgtgc     900
ttttcaacgt tttgttggca tgttgaggac aatcatctat attcactgaa ctatcatcac     960
tttggcgagc caaaagtatg gtacggagtt ccgggaagcc atgcaactgg gctagagaag    1020
gcaatgagaa aacatctacc agatttgttc gatgaacaac ctgatctact tcatgaactg    1080
gttactcagt tttctccaac gattttgaaa aatgagggag tcccggttta tcgagctgtt    1140
cagaatgcag gggagtatgt tctaacattc cctagagcat atcattcggg tttcaactgc    1200
ggattcaact gtgcagaagc ggtgaatgtg gctccggttg attggctggc tcatggacag    1260
aatgctgtgg aaatatatag ccaagagact cggaaaacgt ctctatctca tgacaaaatt    1320
ctccttggag cagcttttga agctgttaag tccctctcag cacatgggga agataataca    1380
aaaaggttca gctggaagag attctgtggg aaggacggta tcataactaa ggccatcgag    1440
gcacggttac ggatggaaga aaaaagaatt gaggctcttg gaaatggttt cagcttggta    1500
aagatggata aagactttga ttcaaactgt gaaagggaat gcatctcttg ctttagtgac    1560
ttgcatctct ctgctactgg ctgcaagaat tgctcatctc tcgaagaata tggttgcacg    1620
aaacacgata tttgttcatg tgaagggaaa gaccgtttca tttttcttcg ttacaccata    1680
gatgagttaa gctcattggt aagagcattg gaaggtgaat cagatgatct aaaagcttgg    1740
ctttccaaag tcatggaagg ttgttcagag actcagaagg gagaatctag tgggattatc    1800
gttaaggaaa agcaggtaca agaagaatgt tttgatctga atggtgagtg taataaatct    1860
tctgagatat gtgaggatgc atccatcatg gacttagctg cttatcatgt tgaacctata    1920
aatcttgggt tcttggtcgt gggaaagctt tggtgtaaca agcatgctat atttccaaaa    1980
gggtttaaga gtcgtgttaa gttctataac gtgcaagatc caatgagaat aagctattac    2040
gtctctgaga ttgtagatgc aggacttctg ggtccactct tcaaggttac tttggaagaa    2100
tctcaagacg agagcttctc ttatgcctca cctcagaagt gctgggaaat ggtgttgctt    2160
agagttaagg aagagatcat gagacgcagc aaccaaaaac aagatgtcca tatgttggaa    2220
agcatcgatg ggttaaaaat gtttggcttt cgctctccgt ttattgttca ggccactgag    2280
gctcttgacc cgaaccatgg ccaagtggag tattggaacc ataagaatga gaaggattcg    2340
ctggaaatga aagattgctt catgtcaaat tcttcccaga gcttaagcaa agcaagactt    2400
tttggggttg atttgaattg a                                              2421
```

<210> 114
<211> 806
<212> PRT
<213> Arabidopsis thaliana

<400> 114

```
Met Glu Pro Phe Ser Ala Ala Gln Asn Lys Glu Asp Lys Asp Thr Ser
1               5                   10                  15
Val Glu Pro Pro Arg Arg Arg Cys His Arg Lys Asn Lys Gly Thr Asn
            20                  25                  30
Val Glu Pro Pro Ser Ser Pro Tyr His Pro Lys Val Leu Ala Arg Trp
        35                  40                  45
Asp Pro Ala Asn Glu Lys Arg Pro Asp Ile Gly Glu Ala Pro Val Phe
    50                  55                  60
His Pro Thr Ser Glu Glu Phe Glu Asp Thr Leu Ala Tyr Ile Glu Lys
65                  70                  75                  80
Ile Arg Pro Leu Ala Glu Ser Phe Gly Ile Cys Arg Ile Val Pro Pro
                85                  90                  95
Ser Asn Trp Ser Pro Pro Cys Arg Leu Lys Gly Asp Ser Ile Trp Lys
            100                 105                 110
Asn Lys Asn Phe Pro Thr Arg Val Gln Phe Val Asp Leu Leu Gln Asn
        115                 120                 125
Arg Gly Pro Val Lys Lys Lys Thr Pro Lys Gly Arg Lys Arg Lys Arg
    130                 135                 140
Gly Lys Tyr Ser Arg Thr Val Ala Pro Lys Lys Arg Asn Gly Ser Val
145                 150                 155                 160
Ser Lys Ser Val Ser Thr Pro Lys Ala Thr Glu Glu Glu Asn Phe Gly
                165                 170                 175
Phe Glu Ser Gly Pro Glu Phe Thr Leu Glu Lys Phe Glu Lys Tyr Ala
            180                 185                 190
Gln Asp Phe Lys Asp Ser Tyr Phe Glu Arg Lys Asp Asn Val Gly Asp
        195                 200                 205
Pro Ser Val Glu Glu Ile Glu Gly Glu Tyr Trp Arg Ile Ile Glu Lys
    210                 215                 220
Glu Thr Asn Glu Val Lys Val Leu Tyr Gly Thr Asp Leu Glu Asn Pro
225                 230                 235                 240
Ile Leu Gly Ser Gly Phe Ser Lys Gly Val Lys Ile Pro Thr Arg Arg
                245                 250                 255
Asn Asp Met Asp Lys Tyr Ile Ser Ser Gly Trp Asn Leu Asn Asn Leu
            260                 265                 270
Ala Arg Leu Gln Gly Ser Leu Leu Ser Phe Glu Asp Cys Glu Ile Ser
        275                 280                 285
Gly Val Gln Val Pro Trp Leu Tyr Val Gly Met Cys Phe Ser Thr Phe
    290                 295                 300
Cys Trp His Val Glu Asp Asn His Leu Tyr Ser Leu Asn Tyr His His
305                 310                 315                 320
Phe Gly Glu Pro Lys Val Trp Tyr Gly Val Pro Gly Ser His Ala Thr
                325                 330                 335
Gly Leu Glu Lys Ala Met Arg Lys His Leu Pro Asp Leu Phe Asp Glu
            340                 345                 350
Gln Pro Asp Leu Leu His Glu Leu Val Thr Gln Phe Ser Pro Thr Ile
        355                 360                 365
Leu Lys Asn Glu Gly Val Pro Val Tyr Arg Ala Val Gln Asn Ala Gly
    370                 375                 380
Glu Tyr Val Leu Thr Phe Pro Arg Ala Tyr His Ser Gly Phe Asn Cys
385                 390                 395                 400
Gly Phe Asn Cys Ala Glu Ala Val Asn Val Ala Pro Val Asp Trp Leu
                405                 410                 415
Ala His Gly Gln Asn Ala Val Glu Ile Tyr Ser Gln Glu Thr Arg Lys
            420                 425                 430
Thr Ser Leu Ser His Asp Lys Ile Leu Leu Gly Ala Ala Phe Glu Ala
        435                 440                 445
Val Lys Ser Leu Ser Ala His Gly Glu Asp Asn Thr Lys Arg Phe Ser
    450                 455                 460
Trp Lys Arg Phe Cys Gly Lys Asp Gly Ile Ile Thr Lys Ala Ile Glu
465                 470                 475                 480
```

```
Ala Arg Leu Arg Met Glu Glu Lys Arg Ile Glu Ala Leu Gly Asn Gly
                485                 490                 495
Phe Ser Leu Val Lys Met Asp Lys Asp Phe Asp Ser Asn Cys Glu Arg
            500                 505                 510
Glu Cys Ile Ser Cys Phe Ser Asp Leu His Leu Ser Ala Thr Gly Cys
        515                 520                 525
Lys Asn Cys Ser Ser Leu Glu Glu Tyr Gly Cys Thr Lys His Asp Ile
    530                 535                 540
Cys Ser Cys Glu Gly Lys Asp Arg Phe Ile Phe Leu Arg Tyr Thr Ile
545                 550                 555                 560
Asp Glu Leu Ser Ser Leu Val Arg Ala Leu Glu Gly Glu Ser Asp Asp
                565                 570                 575
Leu Lys Ala Trp Leu Ser Lys Val Met Glu Gly Cys Ser Glu Thr Gln
            580                 585                 590
Lys Gly Glu Ser Ser Gly Ile Ile Val Lys Glu Lys Gln Val Gln Glu
        595                 600                 605
Glu Cys Phe Asp Leu Asn Gly Glu Cys Asn Lys Ser Ser Glu Ile Cys
    610                 615                 620
Glu Asp Ala Ser Ile Met Asp Leu Ala Ala Tyr His Val Glu Pro Ile
625                 630                 635                 640
Asn Leu Gly Phe Leu Val Val Gly Lys Leu Trp Cys Asn Lys His Ala
                645                 650                 655
Ile Phe Pro Lys Gly Phe Lys Ser Arg Val Lys Phe Tyr Asn Val Gln
            660                 665                 670
Asp Pro Met Arg Ile Ser Tyr Tyr Val Ser Glu Ile Val Asp Ala Gly
        675                 680                 685
Leu Leu Gly Pro Leu Phe Lys Val Thr Leu Glu Glu Ser Gln Asp Glu
    690                 695                 700
Ser Phe Ser Tyr Ala Ser Pro Gln Lys Cys Trp Glu Met Val Leu Leu
705                 710                 715                 720
Arg Val Lys Glu Glu Ile Met Arg Arg Ser Asn Gln Lys Gln Asp Val
                725                 730                 735
His Met Leu Glu Ser Ile Asp Gly Leu Lys Met Phe Gly Phe Arg Ser
            740                 745                 750
Pro Phe Ile Val Gln Ala Thr Glu Ala Leu Asp Pro Asn His Gly Gln
        755                 760                 765
Val Glu Tyr Trp Asn His Lys Asn Glu Lys Asp Ser Leu Glu Met Lys
    770                 775                 780
Asp Cys Phe Met Ser Asn Ser Ser Gln Ser Leu Ser Lys Ala Arg Leu
785                 790                 795                 800
Phe Gly Val Asp Leu Asn
                805
```

<210> 115
<211> 2127
<212> DNA
<213> Arabidopsis thaliana

<400> 115

```
atgggcattg aaggtgtatc aacgtatttg aaatctggaa atatggacac aatttctgct     60
ccgccaggtt ttgtatctca aacatcgttt gtgctgagga atgtaccccg agacaaagaa    120
agtcccaggt ccgtgtcgag acaagagcag acaactggat tcggcacaga tgataaagat    180
agttgcaata tgtttcttaa aagccgaccg tggatagtgc atggtcacac aattcccagc    240
tcagaggcct taaggccgaa aaaaactgag gtacggagaa gacggcccct caaagtgtct    300
gagaccaaag tccttgagga agctcctgtg ttcaacccaa ccgaagagga attcagggac    360
acactctcat atatatcaag cttgcgtgac agagctgagc cgtatggaat ctgttgtgtt    420
gttcctccac cttcatggaa acctccatgt cttctcaagg agaaacaaat atgggaggct    480
tctacatttt tccctcaagt tcagctcttt ggaattcaga ccgagaaccg aaagattaaa    540
aaggaggttg atgcagatag taatgatgct gcatctgaag gggttcagtt atgtagagta    600
gaacgtggtc ctgggtatac tttgaagtcc tttaaaaact ttgcagatac atacaagaag    660
agtcattttg gcatgaagga tgaggttttg ggttcagaga actcatctcc atcacttaaa    720

ccaaatgagc taattgtggc agacattgaa aaagagtaca gacaaattgt tgagagtcca    780
cttattgaaa ttggggtgct ttatggcaat gatttagaca ctgcaacgtt tggaagtgga    840
tttcccttat ctgcaccatc tgaatctagc aaatattcat caggttggaa tctgaacagt    900
acggctaagc ttcctggttc tcttctatca ttagaagact gcgaatcagt ttgtgttcct    960
cgtctaagtg taggaatgtg cttgtcttcc cagttctgga aatctgagaa ggagagactc   1020
tactctctct gctatttgca tgtgggcgct cctagagtgt ggtattccgt agcaggatgt   1080
catcgatcaa agtttaaggc tgccatgaaa agcttcatcc ttgagatgtc aggagaacag   1140
ccaaagaaga gtcataatcc tgtgatgatg atgtctccat atcaattgag cgtggagggt   1200
ataccagtga cccgctgtgt ccagcaccct ggccagtatg ttattatatt tccagggtcg   1260
tattactctg cctttgactg tggcttcaac tgtttggaga aagcaaactt tgctcccctt   1320
gattggttgc cccatgggga tattgctgtt caggtgaatc aagagatgag taaaacgtcg   1380
ctgatatcgt atgataagct attatttagt gctgcaaggg aagctgtaaa atgtcttaag   1440
gaatatgggt tgtcgaagaa aaacacagca tgttacacga gatggaatga ttcttgtggg   1500
acggatgggt tgttctccaa cataatcaag tcacggatca aactggagaa aaatagacgt   1560
gaatttctta tcagttcact agaatcgcaa aggatggaca agagttatga tgctgtgaac   1620
aaaagggaat gctgtgtatg tcttggggat ttgtatcttt ccgcggttaa ctgttcatgc   1680
tctgctaatc gttactcgtg tctgaaccac atgaggaagc tctgtgcatg tccgtgtgac   1740
agaaagagtt ttctctatag gtacactatg gatgagttga atcttctcgt tgaagccctg   1800
gaaggtaaaa agctcagctc tatgttcaga tgggcaggca tagatcaaaa attctgtgct   1860
tctccagcca ccacaagttc aaaacccgaa gaagacaaag gcaaggaaac agatgaggtt   1920
acaccctgta atatcacgag gaaagatgtc gcagctggaa ctaaggacca gacaagagtg   1980
aaggcgagat ctttggctga tatactgaat gttaaagatg gaaacaatga tgcaaaggag   2040
actttagaat cttgttctaa gaaatcaaac aggccatgcg ataacgattc ctctgaggcc   2100
aacgcgccaa agaaacagaa gcagtga                                        2127
```

<210> 116
<211> 708
<212> PRT
<213> Arabidopsis thaliana

<400> 116

```
Met Gly Ile Glu Gly Val Ser Thr Tyr Leu Lys Ser Gly Asn Met Asp
1               5                   10                  15
Thr Ile Ser Ala Pro Pro Gly Phe Val Ser Gln Thr Ser Phe Val Leu
            20                  25                  30
Arg Asn Val Pro Arg Asp Lys Glu Ser Pro Arg Ser Val Ser Arg Gln
        35                  40                  45
Glu Gln Thr Thr Gly Phe Gly Thr Asp Asp Lys Asp Ser Cys Asn Met
    50                  55                  60
Phe Leu Lys Ser Arg Pro Trp Ile Val His Gly His Thr Ile Pro Ser
65                  70                  75                  80
Ser Glu Ala Leu Arg Pro Lys Lys Thr Glu Val Arg Arg Arg Arg Pro
                85                  90                  95
Leu Lys Val Ser Glu Thr Lys Val Leu Glu Glu Ala Pro Val Phe Asn
            100                 105                 110
Pro Thr Glu Glu Glu Phe Arg Asp Thr Leu Ser Tyr Ile Ser Ser Leu
        115                 120                 125
Arg Asp Arg Ala Glu Pro Tyr Gly Ile Cys Cys Val Val Pro Pro Pro
    130                 135                 140
Ser Trp Lys Pro Pro Cys Leu Leu Lys Glu Lys Gln Ile Trp Glu Ala
145                 150                 155                 160
Ser Thr Phe Phe Pro Gln Val Gln Leu Phe Gly Ile Gln Thr Glu Asn
                165                 170                 175
Arg Lys Ile Lys Lys Glu Val Asp Ala Asp Ser Asn Asp Ala Ala Ser
            180                 185                 190
Glu Gly Val Gln Leu Cys Arg Val Glu Arg Gly Pro Gly Tyr Thr Leu
        195                 200                 205
Lys Ser Phe Lys Asn Phe Ala Asp Thr Tyr Lys Lys Ser His Phe Gly
    210                 215                 220
Met Lys Asp Glu Val Leu Gly Ser Glu Asn Ser Ser Pro Ser Leu Lys
225                 230                 235                 240
```

```
Pro Asn Glu Leu Ile Val Ala Asp Ile Glu Lys Glu Tyr Arg Gln Ile
                245                 250                 255
Val Glu Ser Pro Leu Ile Glu Ile Gly Val Leu Tyr Gly Asn Asp Leu
            260                 265                 270
Asp Thr Ala Thr Phe Gly Ser Gly Phe Pro Leu Ser Ala Pro Ser Glu
        275                 280                 285
Ser Ser Lys Tyr Ser Ser Gly Trp Asn Leu Asn Ser Thr Ala Lys Leu
    290                 295                 300
Pro Gly Ser Leu Leu Ser Leu Glu Asp Cys Glu Ser Val Cys Val Pro
305                 310                 315                 320
Arg Leu Ser Val Gly Met Cys Leu Ser Ser Gln Phe Trp Lys Ser Glu
                325                 330                 335
Lys Glu Arg Leu Tyr Ser Leu Cys Tyr Leu His Val Gly Ala Pro Arg
            340                 345                 350
Val Trp Tyr Ser Val Ala Gly Cys His Arg Ser Lys Phe Lys Ala Ala
        355                 360                 365
Met Lys Ser Phe Ile Leu Glu Met Ser Gly Glu Gln Pro Lys Lys Ser
    370                 375                 380
His Asn Pro Val Met Met Met Ser Pro Tyr Gln Leu Ser Val Glu Gly
385                 390                 395                 400
Ile Pro Val Thr Arg Cys Val Gln His Pro Gly Gln Tyr Val Ile Ile
                405                 410                 415
Phe Pro Gly Ser Tyr Tyr Ser Ala Phe Asp Cys Gly Phe Asn Cys Leu
            420                 425                 430
Glu Lys Ala Asn Phe Ala Pro Leu Asp Trp Leu Pro His Gly Asp Ile
        435                 440                 445
Ala Val Gln Val Asn Gln Glu Met Ser Lys Thr Ser Leu Ile Ser Tyr
    450                 455                 460
Asp Lys Leu Leu Phe Ser Ala Ala Arg Glu Ala Val Lys Cys Leu Lys
465                 470                 475                 480
Glu Tyr Gly Leu Ser Lys Lys Asn Thr Ala Cys Tyr Thr Arg Trp Asn
                485                 490                 495
Asp Ser Cys Gly Thr Asp Gly Leu Phe Ser Asn Ile Ile Lys Ser Arg
            500                 505                 510
Ile Lys Leu Glu Lys Asn Arg Arg Glu Phe Leu Ile Ser Ser Leu Glu
        515                 520                 525
Ser Gln Arg Met Asp Lys Ser Tyr Asp Ala Val Asn Lys Arg Glu Cys
    530                 535                 540
Cys Val Cys Leu Gly Asp Leu Tyr Leu Ser Ala Val Asn Cys Ser Cys
545                 550                 555                 560
Ser Ala Asn Arg Tyr Ser Cys Leu Asn His Met Arg Lys Leu Cys Ala
                565                 570                 575
Cys Pro Cys Asp Arg Lys Ser Phe Leu Tyr Arg Tyr Thr Met Asp Glu
            580                 585                 590
Leu Asn Leu Leu Val Glu Ala Leu Glu Gly Lys Lys Leu Ser Ser Met
        595                 600                 605
Phe Arg Trp Ala Gly Ile Asp Gln Lys Phe Cys Ala Ser Pro Ala Thr
    610                 615                 620
Thr Ser Ser Lys Pro Glu Glu Asp Lys Gly Lys Glu Thr Asp Glu Val
625                 630                 635                 640
Thr Pro Cys Asn Ile Thr Arg Lys Asp Val Ala Ala Gly Thr Lys Asp
                645                 650                 655
Gln Thr Arg Val Lys Ala Arg Ser Leu Ala Asp Ile Leu Asn Val Lys
            660                 665                 670
Asp Gly Asn Asn Asp Ala Lys Glu Thr Leu Glu Ser Cys Ser Lys Lys
        675                 680                 685
Ser Asn Arg Pro Cys Asp Asn Asp Ser Ser Glu Ala Asn Ala Pro Lys
    690                 695                 700
Lys Gln Lys Gln
705
```

<210> 117

<211> 3084

<212> DNA
<213> Arabidopsis thaliana

<400> 117

```
atggattctg tggaggaaga aggtgttgtt agggttgaag aagagaacgg acgcggtggt     60
ctccgtaggc atcggagggt ttctacgaag ctggcgaatt acgtagatcc tccgactgac    120
gacgaagaag atggaggacc caagcgaaag ggcaaaaggg gtggaaatag ggctccgaaa    180
aagacaccga agaaagacga ggagatgcaa aagaacgaaa ttgatgaagc aaaccgagtc    240
actggtctgg tgaaggaaaa aagagccgcg actaagattc tgaatcgcaa agactccatt    300
attgaagtag gagaagcttc tggaagcatg cccaaggaag taaagggaat taggattggg    360
aaaaggaagg gagaaatcga tggtgagatt ccaactaagc cgggcaagaa gccaaagact    420
acagtagacc cgagaatcat tggttacaga ccggacaata tgtgccatca atgtcaaaag    480
agcgatagga ttgttgaacg gtgtcagacc tgcaatagta agcgttattg tcatccatgc    540
ttggacactt ggtacccccct tatagcaaaa gaagatgttg ccaagaaatg catgttctgc    600
tctagtactt gcaattgcag agcatgcttg cgtctagata ctaaattgaa agggataaat    660
tcgaacctca tagtcagcga ggaagaaaag gtccaagcct ctaagtttat tctgcagagc    720

cttctcccac atctgaaagg aataaatgat gaacaagttg ctgagaagga agttgaggcc    780
aaaatatacg gactgaagtt tgaagaggtg aggccccaag acgctaaagc tttccctgat    840
gaaagactat actgtgatat ctgcaagact tctatctatg atctccatag gaattgcaag    900
tcctgtagtt ttgatatctg ccttagctgt tgccttgaga tccgcaatgg aaaggctctg    960
gcatgtaagg aggatgtgtc ttggaattat attaaccgag gtttagagta tgaacatgga   1020
caagaaggaa aagtgattga aaagccggcg aataagctag atgataagtt gaaagataag   1080
ctggatggta agccggatga taagccgaag ggtaagccaa agggtaggcc aaagggtaag   1140
ccggatgata agccgaaggg taaactgaag ggtaagcagg atgataaacc ggatgataag   1200
ccagatgaga agccggttaa tacagaccat atgaagtacc cttctctgtg gaaagcaaat   1260
gaagctggga tcattacttg ttgttgtggt gctggggagt tagtgctgaa acgactactt   1320
ccggatggtt ggatatctga gttggtcaac agagttgaaa aaactgctga agctggcgag   1380
cttttgaatt tacctgaaac ggttttggag cgatgccctt gttctaactc tgacagacat   1440
attgacatag acagctgtaa tttgttaaaa gctgcttgtc gagaaggttc agaagacaat   1500
tatctttact ctccaagtgt atgggatgtt caacaagatg atttgaagca ttttcagcac   1560
cattgggtaa aaggcgagcc tgtgattgtg agaaatgtgc ttgaagctac atctggttta   1620
agctgggaac caatggtaat gcatcgtgcc tgccgccaga taagccatgt ccagcatgga   1680
tcacttaagg atgttgttgc tgttgattgt ttggacttct gtgaggtaaa agttaatctt   1740
catgaatttt ttactggata cacggatggc cgatatgatc gaatgggttg gccactagtt   1800
ctgaaactga aagattggcc tccagctaaa gtcttcaaag ataacttgcc acgtcatgct   1860
gaggagttct tatgtagttt gcctttgaag cattacactc atccggttaa tggacctttg   1920
aatctcgctg tcaagcttcc ccaaaattgc ttgaagccag acatgggacc aaagacgtac   1980
gttgcttctg gatttgctca agaattaggc cgtggagatt ctgttactaa gctccactgc   2040
gacatgtctg atgcggtgaa tattttgacg cacatatctg aagtgcccaa tatgcaacct   2100
ggaattggaa atctgaaaaa gaagcatgcc gaacaagatc tcaaggagct gtatagctca   2160
gtagctaaca aggaggaaat gatggagatt cttgaaaatt ccagacaaca agtccaaaat   2220
gttgaaactg atgatggagc tctttgggac attttccgta gagaagatat tcctaaatta   2280
gaaagttaca ttgagaaaca tcacaaggag ttcagacatc tctactgctg tcctgtgtct   2340
caggttgttc atcctataca tgaccagaac ttctatctga cgcggtatca tataatgaag   2400
ctgaaagaag aatatggcat tgaaccttgg accttcaatc agaagcttgg tgatgctgtt   2460
ttgatacctg taggttgccc tcatcaagtt agaaatttga agtcctgcaa taaggtagcg   2520
cttgacttcg tctcacctga aaatgtcagc gagtgtttac gcttgacaaa acagtatcgt   2580
ctacttccac caaatcattt tgcaaaagaa gacaagttag ggttaagaa gatgatagtc    2640
catgcagttg ataaagctct cagagactta agtggagaga agtctccaga acctgaagag   2700
aagaaacaaa atatgagagg gccgaagaag ggggcagcca aggcagttgc caaggctctc   2760
aaagacttat ctccaagtga aaagaagtct tcggaagcag ctgaagagga gatatcaaat   2820
gggatagtca atgcaatcga taagggtctc aaagacttac ctccaagtga agagaagtct   2880
tccgaagcca aagtggagat atcaaatggg atagtcagtg caatggataa ggatctcgaa   2940
cacatatctt caagcgaaaa gaagtctacg gaagaggaag gggtgaaaag accaaatatt   3000
gtgaggacat atgagcggag aaaaaagctg ggaagtgaag taaccaacgc atacattgac   3060
agattagaaa tggagaagat gtag                                           3084
```

<210> 118
<211> 1027
<212> PRT

<213> Arabidopsis thaliana

<400> 118

```
Met Asp Ser Val Glu Glu Glu Gly Val Val Arg Val Glu Glu Glu Asn
1               5                   10                  15
Gly Arg Gly Gly Leu Arg Arg His Arg Arg Val Ser Thr Lys Leu Ala
            20                  25                  30
Asn Tyr Val Asp Pro Pro Thr Asp Asp Glu Glu Asp Gly Gly Pro Lys
        35                  40                  45
Arg Lys Gly Lys Arg Gly Gly Asn Arg Ala Pro Lys Lys Thr Pro Lys
    50                  55                  60
Lys Asp Glu Glu Met Gln Lys Asn Glu Ile Asp Glu Ala Asn Arg Val
65                  70                  75                  80
Thr Gly Leu Val Lys Glu Lys Arg Ala Ala Thr Lys Ile Leu Asn Arg
                85                  90                  95
Lys Asp Ser Ile Ile Glu Val Gly Glu Ala Ser Gly Ser Met Pro Lys
            100                 105                 110
Glu Val Lys Gly Ile Arg Ile Gly Lys Arg Lys Gly Glu Ile Asp Gly
        115                 120                 125
Glu Ile Pro Thr Lys Pro Gly Lys Lys Pro Lys Thr Thr Val Asp Pro
    130                 135                 140
Arg Ile Ile Gly Tyr Arg Pro Asp Asn Met Cys His Gln Cys Gln Lys
145                 150                 155                 160
Ser Asp Arg Ile Val Glu Arg Cys Gln Thr Cys Asn Ser Lys Arg Tyr
                165                 170                 175
Cys His Pro Cys Leu Asp Thr Trp Tyr Pro Leu Ile Ala Lys Glu Asp
            180                 185                 190
Val Ala Lys Lys Cys Met Phe Cys Ser Ser Thr Cys Asn Cys Arg Ala
        195                 200                 205
Cys Leu Arg Leu Asp Thr Lys Leu Lys Gly Ile Asn Ser Asn Leu Ile
    210                 215                 220
Val Ser Glu Glu Glu Lys Val Gln Ala Ser Lys Phe Ile Leu Gln Ser
225                 230                 235                 240
Leu Leu Pro His Leu Lys Gly Ile Asn Asp Glu Gln Val Ala Glu Lys
                245                 250                 255
Glu Val Glu Ala Lys Ile Tyr Gly Leu Lys Phe Glu Glu Val Arg Pro
            260                 265                 270
Gln Asp Ala Lys Ala Phe Pro Asp Glu Arg Leu Tyr Cys Asp Ile Cys
        275                 280                 285
Lys Thr Ser Ile Tyr Asp Leu His Arg Asn Cys Lys Ser Cys Ser Phe
    290                 295                 300
Asp Ile Cys Leu Ser Cys Cys Leu Glu Ile Arg Asn Gly Lys Ala Leu
305                 310                 315                 320
Ala Cys Lys Glu Asp Val Ser Trp Asn Tyr Ile Asn Arg Gly Leu Glu
                325                 330                 335
Tyr Glu His Gly Gln Glu Gly Lys Val Ile Glu Lys Pro Ala Asn Lys
            340                 345                 350
Leu Asp Asp Lys Leu Lys Asp Lys Leu Asp Gly Lys Pro Asp Asp Lys
        355                 360                 365
Pro Lys Gly Lys Pro Lys Gly Arg Pro Lys Gly Lys Pro Asp Asp Lys

    370                 375                 380
Pro Lys Gly Lys Leu Lys Gly Lys Gln Asp Asp Lys Pro Asp Asp Lys
385                 390                 395                 400
Pro Asp Glu Lys Pro Val Asn Thr Asp His Met Lys Tyr Pro Ser Leu
                405                 410                 415
Trp Lys Ala Asn Glu Ala Gly Ile Ile Thr Cys Cys Cys Gly Ala Gly
            420                 425                 430
Glu Leu Val Leu Lys Arg Leu Leu Pro Asp Gly Trp Ile Ser Glu Leu
```

```
        435                     440                     445
Val Asn Arg Val Glu Lys Thr Ala Glu Ala Gly Glu Leu Leu Asn Leu
    450                     455                     460
Pro Glu Thr Val Leu Glu Arg Cys Pro Cys Ser Asn Ser Asp Arg His
465                     470                     475                 480
Ile Asp Ile Asp Ser Cys Asn Leu Leu Lys Ala Ala Cys Arg Glu Gly
                    485                     490                     495
Ser Glu Asp Asn Tyr Leu Tyr Ser Pro Ser Val Trp Asp Val Gln Gln
            500                     505                     510
Asp Asp Leu Lys His Phe Gln His His Trp Val Lys Gly Glu Pro Val
        515                     520                     525
Ile Val Arg Asn Val Leu Glu Ala Thr Ser Gly Leu Ser Trp Glu Pro
    530                     535                     540
Met Val Met His Arg Ala Cys Arg Gln Ile Ser His Val Gln His Gly
545                     550                     555                 560
Ser Leu Lys Asp Val Val Ala Val Asp Cys Leu Asp Phe Cys Glu Val
                    565                     570                     575
Lys Val Asn Leu His Glu Phe Phe Thr Gly Tyr Thr Asp Gly Arg Tyr
            580                     585                     590
Asp Arg Met Gly Trp Pro Leu Val Leu Lys Leu Lys Asp Trp Pro Pro
        595                     600                     605
Ala Lys Val Phe Lys Asp Asn Leu Pro Arg His Ala Glu Glu Phe Leu
    610                     615                     620
Cys Ser Leu Pro Leu Lys His Tyr Thr His Pro Val Asn Gly Pro Leu
625                     630                     635                 640
Asn Leu Ala Val Lys Leu Pro Gln Asn Cys Leu Lys Pro Asp Met Gly
                    645                     650                     655
Pro Lys Thr Tyr Val Ala Ser Gly Phe Ala Gln Glu Leu Gly Arg Gly
            660                     665                     670
Asp Ser Val Thr Lys Leu His Cys Asp Met Ser Asp Ala Val Asn Ile
        675                     680                     685
Leu Thr His Ile Ser Glu Val Pro Asn Met Gln Pro Gly Ile Gly Asn
    690                     695                     700
Leu Lys Lys Lys His Ala Glu Gln Asp Leu Lys Glu Leu Tyr Ser Ser
705                     710                     715                 720
Val Ala Asn Lys Glu Glu Met Met Glu Ile Leu Glu Asn Ser Arg Gln
                    725                     730                     735
Gln Val Gln Asn Val Glu Thr Asp Asp Gly Ala Leu Trp Asp Ile Phe
            740                     745                     750
Arg Arg Glu Asp Ile Pro Lys Leu Glu Ser Tyr Ile Glu Lys His His
        755                     760                     765
Lys Glu Phe Arg His Leu Tyr Cys Cys Pro Val Ser Gln Val Val His
    770                     775                     780
Pro Ile His Asp Gln Asn Phe Tyr Leu Thr Arg Tyr His Ile Met Lys
785                     790                     795                 800
Leu Lys Glu Glu Tyr Gly Ile Glu Pro Trp Thr Phe Asn Gln Lys Leu
                    805                     810                     815
Gly Asp Ala Val Leu Ile Pro Val Gly Cys Pro His Gln Val Arg Asn
            820                     825                     830
Leu Lys Ser Cys Asn Lys Val Ala Leu Asp Phe Val Ser Pro Glu Asn
        835                     840                     845
Val Ser Glu Cys Leu Arg Leu Thr Lys Gln Tyr Arg Leu Leu Pro Pro
    850                     855                     860
Asn His Phe Ala Lys Glu Asp Lys Leu Gly Val Lys Lys Met Ile Val
865                     870                     875                 880
His Ala Val Asp Lys Ala Leu Arg Asp Leu Ser Gly Glu Lys Ser Pro
                    885                     890                     895
Glu Pro Glu Glu Lys Lys Gln Asn Met Arg Gly Pro Lys Lys Gly Ala
            900                     905                     910
Ala Lys Ala Val Ala Lys Ala Leu Lys Asp Leu Ser Pro Ser Glu Lys
        915                     920                     925
```

```
Lys Ser Ser Glu Ala Ala Glu Glu Glu Ile Ser Asn Gly Ile Val Asn
    930                 935                 940
Ala Ile Asp Lys Gly Leu Lys Asp Leu Pro Pro Ser Glu Glu Lys Ser
945                 950                 955                 960
Ser Glu Ala Lys Val Glu Ile Ser Asn Gly Ile Val Ser Ala Met Asp
                965                 970                 975
Lys Asp Leu Glu His Ile Ser Ser Ser Glu Lys Lys Ser Thr Glu Glu
            980                 985                 990
Glu Gly Val Lys Arg Pro Asn Ile  Val Arg Thr Tyr Glu  Arg Arg Lys
        995                 1000                1005
Lys Leu  Gly Ser Glu Val Thr  Asn Ala Tyr Ile Asp  Arg Leu Glu
    1010                1015                1020
Met Glu  Lys Met
    1025
```

<210> 119
<211> 4083
<212> DNA
<213> Arabidopsis thaliana

<400> 119

```
atggcggttt cagagcagag tcaagatgtg tttccatggc ttaaatcgtt accggttgct     60
ccagagttca gacctactct agcagagttt caagatccga tcgcttacat tttgaagatt    120
gaagaagaag cttctagata tggaatctgt aaaattctgc ctccactgcc tcctccttcc    180
aaaaaaacct cgatttctaa tctcaaccgt tctctggcgg caagagcggc ggcgagggtt    240
cgtgacggcg gctttggcgc gtgtgattat gacggtggtc ccacattcgc cacgcgccag    300
cagcagatcg ggttttgccc taggaaacag cgtccagtgc agagacctgt gtggcagagt    360
ggagaggagt actcttttgg tgagttcgag tttaaagcta agaactttga gaagaattat    420
ctcaagaaat gtggtaaaaa gagtcaactc tctgcccttg aaattgaaac actttactgg    480
agagccactg tggataaacc cttctctgtt gagtatgcca atgacatgcc tggctcggct    540
tttatccctc tgagtctggc tgctgcgagg aggagagagt ctggtggtga aggaggaaca    600
gttggtgaga ccgcttggaa catgagggca atgtctagag ccgaaggatc attgcttaag    660
ttcatgaagg aagagatccc tggagttaca tcaccaatgg tgtatgttgc tatgatgttt    720
agttggtttg cttggcatgt ggaggaccat gaccttcata gtctcaatta cttgcatatg    780
ggtgctggta agacttggta cggtgtgcca aaggatgctg ctctggcttt tgaggaggtt    840
gttagggttc atggttacgg tgaagagctc aatcctcttg tgacatttc tactcttggt     900
gagaagacaa ctgtgatgtc tcctgaagta tttgttaaag ccggaatacc gtgttgcagg    960
ttagtgcaaa atcctggaga gtttgtcgtc acctttccgg gagcttatca ttcgggattt   1020
agtcatggat ttaattttgg agaagcatct aacattgcca ctcccgaatg gttgagaatg   1080
gctaaagatg ctgctatccg gcgagctgct ataaattacc ctccaatggt ttctcatctc   1140
cagctacttt atgactttgt attggctcta ggttctagag tgccaacaag catcaatccc   1200
aaaccacgga gttctagatt aaaagataag gcaagaagcg aaggagaaag attgaccaaa   1260
aagctatttg tgcaaaacat tatccacaac aacgaattgc tttcttctct cggaaaagga   1320
tccccagtgg cccttctccc acagagttcc tcagatatat cagtttgttc tgacctgcga   1380
attggatccc atttgataac caaccaggaa aacccaatcc agttaaagtg tgaggactta   1440
agttctgata gtgttgtggt tgatctcagt aacggtttaa aggatacagt ttcagtgaaa   1500
gaaaaattta catctttatg tgaaaggagc agaaatcacc tagcaagcac ggagaaggac   1560
actcaagaaa ctctgtctga tgctgaaagg aggaagaatg atgcagctgt tgcgctttcg   1620
gatcaaaggc ttttctcttg tgttacatgt ggagtcttaa gctttgattg tgtagctatc   1680
gttcaaccta aagaagcagc tgctagatat ctcatgtctg cagattgtag cttcttcaat   1740
gattggacag ctgcttctgg atctgcaaat cttggtcagg ctgcaagatc acttcatcct   1800
caaagcaaag agaagcatga tgtaaattac ttctacaatg ttcctgttca aactatggat   1860
cattcagtga agactggcga tcaaaaaact tcaacaactt ccccgacaat agcgcataaa   1920
gataatgatg ttcttgggat gttagcttca gcatatggag actcttctga ttccgaggaa   1980
gaagatcaaa aaggcttagt taccccctagt tccaaagggg aaacaaaaac gtatgatcaa  2040
gaaggttcag atggccatga ggaggctaga gatggtagaa cttctgattt taactgccag   2100
agactaacca gcgaacagaa tgggttaagc aaaggcggaa aatcatcact tctggaaata   2160
gctttaccat ttattccaag atctgatgac gattcatgtc ggttgcacgt gttttgtctt   2220
gagcatgctg cggaagtgga acagcaactt cgtcctttg gggggattaa cttaatgtta    2280
ctgtgccatc cagagtaccc caggatagag gctgaagcaa agatagttgc cgaagagctc   2340
gtcatcaatc acgaatggaa tgatactgaa ttcaggaatg tgacccgaga ggatgaggaa   2400
```

```
acgattcagg cagcgttgga taatgttgaa gctaagggtg ggaacagtga ttggaccgta   2460
aaattgggtg ttaacctttc ttacagcgct attctcagtc gctctcctct gtacagtaag   2520
cagatgccgt ataactccat catatacaag gcgttcggtc gcagctctcc agtagcgagc   2580
tcaccctcga aacccaaagt ctctggtaaa agatcgtcca gacagaggaa atatgttgtt   2640
ggaaaatggt gtggtaaggt ttggatgtca catcaggtgc atcccttttt gctggagcag   2700
gacttagagg gggaagaatc tgaaagaagt tgtcatcttc gagttgctat ggatgaggat   2760
gccactggaa agagatcgtt tcctaataat gtttccaggg attcgacaac aatgtttgga   2820
agaaagtatt gtaggaagag aaagataaga gcaaaggcgg tgccacgcaa gaagcttact   2880
tcttttaaga gggaagatgg agtttctgat gacacatcag aagatcattc ttataagcag   2940
caatggaggg cttccgggaa tgaggaagag tcttattttg agacagggaa cacagcttct   3000
ggtgattcat caaatcaaat gtctgatccg cacaagggaa ttatcagaca taaaggttat   3060
aaagaatttg agtcagatga tgaggtttca gaccgttcac ttggggaaga gtatactgta   3120
cgggcatgtg cagcttcaga gagctcaatg gagaatggct ctcagcattc aatgtatgac   3180
catgatgatg atgatgatga tatcgacagg cagcctaggg ggattccaag gagccaacag   3240
acaagagttt ttaggaatcc agtttcatat gagtcagaag ataatggcgt ttatcagcaa   3300
agcggaagaa tatccataag taataggcaa gctaatcgaa tggttggtga atatgattca   3360
gcagagaatt ctttggagga acgaggcttt tgcagtacag ggaaaaggca aaccaggtca   3420
acagccaaac gaatagcaaa aaccaagaca gttcagagtt cgagagacac aaaaggtcgc   3480
tttttgcaag aatttgcatc tggaaagaag aatgaagaat tggattcata catggaggga   3540
cctagcacac ggcttagggt gagacatcag aagccgtcga gagggtcttt agaaacaaaa   3600
ccaaagaaga ttggtaagaa gagaagtggt aatgcttcct tctccagagt tgcaactgaa   3660
aaagatgtgg aggaaaaaga agaagaagaa gaagaagaag agaatgagga agaggaatgt   3720
gcagcatacc aatgtaacat ggagggttgc acgatgagtt tcagttcgga aaaacagttg   3780
atgttacaca aaagaaacat atgcccaatt aaaggctgtg gtaaaaactt cttctcacac   3840
aagtatttgg ttcaacacca gcgtgttcac tcagacgacc gtcctctgaa atgtccatgg   3900
aagggatgta agatgacgtt caagtgggct tggtctagaa ccgagcacat aagggttcac   3960
acaggcgcta ggccttatgt ttgcgctgaa ccggattgtg gtcaaacatt caggtttgtc   4020
tctgacttca gccggcataa aaggaagacc ggtcattcgg ttaagaagac caacaaaagg   4080
tga                                                                 4083
```

<210> 120
<211> 1360
<212> PRT
<213> Arabidopsis thaliana

<400> 120

```
Met Ala Val Ser Glu Gln Ser Gln Asp Val Phe Pro Trp Leu Lys Ser
1               5                   10                  15
Leu Pro Val Ala Pro Glu Phe Arg Pro Thr Leu Ala Glu Phe Gln Asp
            20                  25                  30
Pro Ile Ala Tyr Ile Leu Lys Ile Glu Glu Glu Ala Ser Arg Tyr Gly
        35                  40                  45
Ile Cys Lys Ile Leu Pro Pro Leu Pro Pro Pro Ser Lys Lys Thr Ser
    50                  55                  60
Ile Ser Asn Leu Asn Arg Ser Leu Ala Ala Arg Ala Ala Ala Arg Val
65                  70                  75                  80
Arg Asp Gly Gly Phe Gly Ala Cys Asp Tyr Asp Gly Gly Pro Thr Phe
                85                  90                  95
Ala Thr Arg Gln Gln Gln Ile Gly Phe Cys Pro Arg Lys Gln Arg Pro
            100                 105                 110
Val Gln Arg Pro Val Trp Gln Ser Gly Glu Glu Tyr Ser Phe Gly Glu
        115                 120                 125
Phe Glu Phe Lys Ala Lys Asn Phe Glu Lys Asn Tyr Leu Lys Lys Cys
    130                 135                 140
Gly Lys Lys Ser Gln Leu Ser Ala Leu Glu Ile Glu Thr Leu Tyr Trp
145                 150                 155                 160
Arg Ala Thr Val Asp Lys Pro Phe Ser Val Glu Tyr Ala Asn Asp Met
                165                 170                 175
Pro Gly Ser Ala Phe Ile Pro Leu Ser Leu Ala Ala Ala Arg Arg Arg
            180                 185                 190
Glu Ser Gly Gly Glu Gly Gly Thr Val Gly Glu Thr Ala Trp Asn Met
```

```
    195                 200                 205
Arg Ala Met Ser Arg Ala Glu Gly Ser Leu Leu Lys Phe Met Lys Glu
    210                 215                 220
Glu Ile Pro Gly Val Thr Ser Pro Met Val Tyr Val Ala Met Met Phe
225                 230                 235                 240
Ser Trp Phe Ala Trp His Val Glu Asp His Asp Leu His Ser Leu Asn
                245                 250                 255
Tyr Leu His Met Gly Ala Gly Lys Thr Trp Tyr Gly Val Pro Lys Asp
            260                 265                 270
Ala Ala Leu Ala Phe Glu Glu Val Val Arg Val His Gly Tyr Gly Glu
        275                 280                 285
Glu Leu Asn Pro Leu Val Thr Phe Ser Thr Leu Gly Glu Lys Thr Thr
    290                 295                 300
Val Met Ser Pro Glu Val Phe Val Lys Ala Gly Ile Pro Cys Cys Arg
305                 310                 315                 320
Leu Val Gln Asn Pro Gly Glu Phe Val Val Thr Phe Pro Gly Ala Tyr
                325                 330                 335
His Ser Gly Phe Ser His Gly Phe Asn Phe Gly Glu Ala Ser Asn Ile
            340                 345                 350
Ala Thr Pro Glu Trp Leu Arg Met Ala Lys Asp Ala Ala Ile Arg Arg
        355                 360                 365
Ala Ala Ile Asn Tyr Pro Pro Met Val Ser His Leu Gln Leu Leu Tyr
    370                 375                 380

Asp Phe Val Leu Ala Leu Gly Ser Arg Val Pro Thr Ser Ile Asn Pro
385                 390                 395                 400
Lys Pro Arg Ser Ser Arg Leu Lys Asp Lys Ala Arg Ser Glu Gly Glu
                405                 410                 415
Arg Leu Thr Lys Lys Leu Phe Val Gln Asn Ile Ile His Asn Asn Glu
            420                 425                 430
Leu Leu Ser Ser Leu Gly Lys Gly Ser Pro Val Ala Leu Leu Pro Gln
        435                 440                 445
Ser Ser Ser Asp Ile Ser Val Cys Ser Asp Leu Arg Ile Gly Ser His
    450                 455                 460
Leu Ile Thr Asn Gln Glu Asn Pro Ile Gln Leu Lys Cys Glu Asp Leu
465                 470                 475                 480
Ser Ser Asp Ser Val Val Val Asp Leu Ser Asn Gly Leu Lys Asp Thr
                485                 490                 495
Val Ser Val Lys Glu Lys Phe Thr Ser Leu Cys Glu Arg Ser Arg Asn
            500                 505                 510
His Leu Ala Ser Thr Glu Lys Asp Thr Gln Glu Thr Leu Ser Asp Ala
        515                 520                 525
Glu Arg Arg Lys Asn Asp Ala Ala Val Ala Leu Ser Asp Gln Arg Leu
    530                 535                 540
Phe Ser Cys Val Thr Cys Gly Val Leu Ser Phe Asp Cys Val Ala Ile
545                 550                 555                 560
Val Gln Pro Lys Glu Ala Ala Ala Arg Tyr Leu Met Ser Ala Asp Cys
                565                 570                 575
Ser Phe Phe Asn Asp Trp Thr Ala Ala Ser Gly Ser Ala Asn Leu Gly
            580                 585                 590
Gln Ala Ala Arg Ser Leu His Pro Gln Ser Lys Glu Lys His Asp Val
        595                 600                 605
Asn Tyr Phe Tyr Asn Val Pro Val Gln Thr Met Asp His Ser Val Lys
    610                 615                 620
Thr Gly Asp Gln Lys Thr Ser Thr Thr Ser Pro Thr Ile Ala His Lys
625                 630                 635                 640
Asp Asn Asp Val Leu Gly Met Leu Ala Ser Ala Tyr Gly Asp Ser Ser
                645                 650                 655
Asp Ser Glu Glu Glu Asp Gln Lys Gly Leu Val Thr Pro Ser Ser Lys
            660                 665                 670
Gly Glu Thr Lys Thr Tyr Asp Gln Glu Gly Ser Asp Gly His Glu Glu
```

```
        675                 680                 685
Ala Arg Asp Gly Arg Thr Ser Asp Phe Asn Cys Gln Arg Leu Thr Ser
    690                 695                 700
Glu Gln Asn Gly Leu Ser Lys Gly Gly Lys Ser Ser Leu Leu Glu Ile
705                 710                 715                 720
Ala Leu Pro Phe Ile Pro Arg Ser Asp Asp Asp Ser Cys Arg Leu His
                725                 730                 735
Val Phe Cys Leu Glu His Ala Ala Glu Val Glu Gln Gln Leu Arg Pro
            740                 745                 750
Phe Gly Gly Ile Asn Leu Met Leu Leu Cys His Pro Glu Tyr Pro Arg
        755                 760                 765
Ile Glu Ala Glu Ala Lys Ile Val Ala Glu Glu Leu Val Ile Asn His
    770                 775                 780
Glu Trp Asn Asp Thr Glu Phe Arg Asn Val Thr Arg Glu Asp Glu Glu
785                 790                 795                 800
Thr Ile Gln Ala Ala Leu Asp Asn Val Glu Ala Lys Gly Gly Asn Ser
                805                 810                 815
Asp Trp Thr Val Lys Leu Gly Val Asn Leu Ser Tyr Ser Ala Ile Leu
            820                 825                 830
Ser Arg Ser Pro Leu Tyr Ser Lys Gln Met Pro Tyr Asn Ser Ile Ile
        835                 840                 845
Tyr Lys Ala Phe Gly Arg Ser Ser Pro Val Ala Ser Ser Pro Ser Lys
    850                 855                 860
Pro Lys Val Ser Gly Lys Arg Ser Ser Arg Gln Arg Lys Tyr Val Val
865                 870                 875                 880
Gly Lys Trp Cys Gly Lys Val Trp Met Ser His Gln Val His Pro Phe
                885                 890                 895
Leu Leu Glu Gln Asp Leu Glu Gly Glu Glu Ser Glu Arg Ser Cys His
            900                 905                 910
Leu Arg Val Ala Met Asp Glu Asp Ala Thr Gly Lys Arg Ser Phe Pro
        915                 920                 925
Asn Asn Val Ser Arg Asp Ser Thr Thr Met Phe Gly Arg Lys Tyr Cys
    930                 935                 940
Arg Lys Arg Lys Ile Arg Ala Lys Ala Val Pro Arg Lys Lys Leu Thr
945                 950                 955                 960
Ser Phe Lys Arg Glu Asp Gly Val Ser Asp Asp Thr Ser Glu Asp His
                965                 970                 975
Ser Tyr Lys Gln Gln Trp Arg Ala Ser Gly Asn Glu Glu Glu Ser Tyr
            980                 985                 990
Phe Glu Thr Gly Asn Thr Ala Ser  Gly Asp Ser Ser Asn  Gln Met Ser
        995                 1000                1005
Asp Pro  His Lys Gly Ile Ile  Arg His Lys Gly Tyr  Lys Glu Phe
    1010                1015                1020
Glu Ser  Asp Asp Glu Val Ser  Asp Arg Ser Leu Gly  Glu Glu Tyr
    1025                1030                1035
Thr Val  Arg Ala Cys Ala Ala  Ser Glu Ser Ser Met  Glu Asn Gly
    1040                1045                1050
Ser Gln  His Ser Met Tyr Asp  His Asp Asp Asp Asp  Asp Asp Ile
    1055                1060                1065
Asp Arg  Gln Pro Arg Gly Ile  Pro Arg Ser Gln Gln  Thr Arg Val
    1070                1075                1080
Phe Arg  Asn Pro Val Ser Tyr  Glu Ser Glu Asp Asn  Gly Val Tyr
    1085                1090                1095
Gln Gln  Ser Gly Arg Ile Ser  Ile Ser Asn Arg Gln  Ala Asn Arg
    1100                1105                1110
Met Val  Gly Glu Tyr Asp Ser  Ala Glu Asn Ser Leu  Glu Glu Arg
    1115                1120                1125
Gly Phe  Cys Ser Thr Gly Lys  Arg Gln Thr Arg Ser  Thr Ala Lys
    1130                1135                1140
Arg Ile  Ala Lys Thr Lys Thr  Val Gln Ser Ser Arg  Asp Thr Lys
    1145                1150                1155
```

```
Gly Arg  Phe Leu Gln Glu Phe  Ala Ser Gly Lys Lys  Asn Glu Glu
    1160                 1165                 1170
Leu Asp  Ser Tyr Met Glu Gly  Pro Ser Thr Arg Leu  Arg Val Arg
    1175                 1180                 1185
His Gln  Lys Pro Ser Arg Gly  Ser Leu Glu Thr Lys  Pro Lys Lys
    1190                 1195                 1200
Ile Gly  Lys Lys Arg Ser Gly  Asn Ala Ser Phe Ser  Arg Val Ala
    1205                 1210                 1215
Thr Glu  Lys Asp Val Glu Glu  Lys Glu Glu Glu Glu  Glu Glu Glu
    1220                 1225                 1230
Glu Asn  Glu Glu Glu Glu Cys  Ala Ala Tyr Gln Cys  Asn Met Glu
    1235                 1240                 1245
Gly Cys  Thr Met Ser Phe Ser  Ser Glu Lys Gln Leu  Met Leu His
    1250                 1255                 1260
Lys Arg  Asn Ile Cys Pro Ile  Lys Gly Cys Gly Lys  Asn Phe Phe
    1265                 1270                 1275
Ser His  Lys Tyr Leu Val Gln  His Gln Arg Val His  Ser Asp Asp
    1280                 1285                 1290
Arg Pro  Leu Lys Cys Pro Trp  Lys Gly Cys Lys Met  Thr Phe Lys
    1295                 1300                 1305
Trp Ala  Trp Ser Arg Thr Glu  His Ile Arg Val His  Thr Gly Ala
    1310                 1315                 1320
Arg Pro  Tyr Val Cys Ala Glu  Pro Asp Cys Gly Gln  Thr Phe Arg
    1325                 1330                 1335
Phe Val  Ser Asp Phe Ser Arg  His Lys Arg Lys Thr  Gly His Ser
    1340                 1345                 1350
Val Lys  Lys Thr Asn Lys Arg
    1355                 1360
```

<210> 121
<211> 2523
<212> DNA
<213> Arabidopsis thaliana

<400> 121

```
atggagaaaa tgagagggaa gcgaatcaga ccaagagatt ccggcgagtt agtcgaagat     60
gggagatctg aaagcgagcg taagacgagg aagaaagaga atgatgttgt gagcaaagga    120
agaatcggaa gaggaagagg aagaggagag gtttcgaagc gatctattga aatagacatt    180
tctaatcccg agaaggatat taagcctgac ggaagcagaa agtgtttggg ttcgacgtgt    240
catcattgta agatcttgac gagtgagagt gatcttatct tctgttcaaa gtgtaacaag    300
aagtgttatt gctttgactg catcaagaga tcgtattcag aaagaacaca tgaggaagtt    360
agagctgctt gtccattctg tatgatgact tgcatttgca gagcttgttt gcggttgcct    420
ttggttatca agcctccaag tgaaaaagat acagatgtca agcttaagca gctgcagtat    480
ctgttagtca aagttctccc agttcttaag gatatttata cggagcagaa ccgtgaacta    540
gagattgaat caacaattag aggacaccct gtgacagaag ctaatatcaa gaggtgcaaa    600
cttgacccaa gcgaacgcat atactgtgat ctctgccgca catccattgc caattttcac    660
agaagctgtc cgaacaaaaa ttgttcagtc gatatatgtc tttcttgctg caaggaacta    720
agtgagggct tccatcaaga gagagatggg aagaaaaatg cagaagggaa aggatatgaa    780
tgtcgaatcc cagcaggtca aggaaaagac tcggacgcat atgttccact gcatttctcg    840
acttggaagc ttaactcaga cagtagcatt ccatgccctc caaaagagtg tggtggttgt    900
ggtacttcaa cactggagtt gagacgctta tggaaaagag attgggttga aaaattaata    960

acgaatgccg agaaatgtac tctgaatttt aggccaacag atgtggatat tgttcatgaa   1020
tgttcctcat gctctaccaa ttctgattcc attagaaggc aggcagcttt taggaagaat   1080
gctcatgata atttcttata ctccccaaat gctgttgatc tggctgaaga cgatattgct   1140
cattttcagt ttcattggat gaaggcagaa cctgtgatag tcagaaatgt tctcgagaag   1200
acatctggac taagttggga accaatggtt atgtggaggg cttgtaggga aatggatcca   1260
aagcgtaaag gtacagaaga agagacaaca aaagtgaaag ctttggattg cttggactgg   1320
tgtgaggttg aaataaatct gcatcagttt tttgagggct acttagaagg ccgcatgcat   1380
aaaaacggtt ggccagaaat gttgaaattg aaggactggc ctccctcaga tttatttgaa   1440
aagcgccttc caaggcataa tgctgagttt attgccgcat tgcctttctt tgattacact   1500
```

```
gacccaaagt ctggtatcct aaatctcgca acaagatttc cggaaggatc cttgaagcca 1560
gatcttggac ccaagacata cattgcgtac ggtttccatg aagagcttaa tagaggagat 1620
tcggtgacaa aacttcattg tgacatttct gacgcggtca atgtgttgac acacacagct 1680
aaagtggaaa tccctcccgt caaataccaa aacataaaag tacatcagaa aaaatatgca 1740
gaagccatgt tgcagaaaca acaatacagt ggtcaagtga aagaagccag cgaacttgaa 1800
aacaagtcaa tgaaagaagt ggatgaaagt aaaaaggatt taaaagacaa ggcagctaac 1860
gaagaacaat caaataacag ttcaagaccg tcgggctcag gagaagctga gaaagtaatt 1920
atttcaaaag aggataatcc cacacaacct gctgtgagta caagtgtgga atccatacaa 1980
gaacagaagc tggatgctcc aaaagaaact gatggcaata ccaatgagag gtcaaaggct 2040
gtgcatggtg gtgctgtctg ggatatcttt cgaagagagg atgttccaaa acttattcag 2100
tttttgaaaa ggcacgagca tgagtttcgt cacttcaata acgaacccct ggaatccgtt 2160
attcacccaa ttcatgacca gactatgttc ttgagtgata gccagaagaa acagctgaaa 2220
gaagaatttg atatagaacc atggaccttt gagcaacacc tcggcgaagc agtcttcata 2280
cctgcaggtt gtcctcacca agtgaggaat agacagtctt gcataaaggt ggcactagac 2340
tttgtggctc ctgaaagcgt cgaagaatgc cttaggctaa cacaggagtt ccggagattg 2400
cctaaagacc acagttctag cgaggataaa ttggagctta agaaaattgc actgtatgct 2460
gcgagttcag ccataagaga ggtaaaaggg ctaatgcaaa gctcccgacg cagtgatacc 2520
taa                                                               2523
```

<210> 122
<211> 840
<212> PRT
<213> Arabidopsis thaliana

<400> 122

```
Met Glu Lys Met Arg Gly Lys Arg Ile Arg Pro Arg Asp Ser Gly Glu
1               5                   10                  15
Leu Val Glu Asp Gly Arg Ser Glu Ser Glu Arg Lys Thr Arg Lys Lys
            20                  25                  30
Glu Asn Asp Val Val Ser Lys Gly Arg Ile Gly Arg Gly Arg Gly Arg
        35                  40                  45
Gly Glu Val Ser Lys Arg Ser Ile Glu Ile Asp Ile Ser Asn Pro Glu
    50                  55                  60
Lys Asp Ile Lys Pro Asp Gly Ser Arg Lys Cys Leu Gly Ser Thr Cys
65                  70                  75                  80
His His Cys Lys Ile Leu Thr Ser Glu Ser Asp Leu Ile Phe Cys Ser
                85                  90                  95
Lys Cys Asn Lys Lys Cys Tyr Cys Phe Asp Cys Ile Lys Arg Ser Tyr
            100                 105                 110
Ser Glu Arg Thr His Glu Glu Val Arg Ala Ala Cys Pro Phe Cys Met
        115                 120                 125
Met Thr Cys Ile Cys Arg Ala Cys Leu Arg Leu Pro Leu Val Ile Lys
    130                 135                 140
Pro Pro Ser Glu Lys Asp Thr Asp Val Lys Leu Lys Gln Leu Gln Tyr
145                 150                 155                 160
Leu Leu Val Lys Val Leu Pro Val Leu Lys Asp Ile Tyr Thr Glu Gln
                165                 170                 175
Asn Arg Glu Leu Glu Ile Glu Ser Thr Ile Arg Gly His Pro Val Thr
            180                 185                 190
Glu Ala Asn Ile Lys Arg Cys Lys Leu Asp Pro Ser Glu Arg Ile Tyr
        195                 200                 205
Cys Asp Leu Cys Arg Thr Ser Ile Ala Asn Phe His Arg Ser Cys Pro
    210                 215                 220
Asn Lys Asn Cys Ser Val Asp Ile Cys Leu Ser Cys Cys Lys Glu Leu
225                 230                 235                 240
Ser Glu Gly Phe His Gln Glu Arg Asp Gly Lys Lys Asn Ala Glu Gly
                245                 250                 255
Lys Gly Tyr Glu Cys Arg Ile Pro Ala Gly Gln Gly Lys Asp Ser Asp
            260                 265                 270
Ala Tyr Val Pro Leu His Phe Ser Thr Trp Lys Leu Asn Ser Asp Ser
        275                 280                 285
```

```
Ser Ile Pro Cys Pro Pro Lys Glu Cys Gly Gly Cys Gly Thr Ser Thr
    290                 295                 300
Leu Glu Leu Arg Arg Leu Trp Lys Arg Asp Trp Val Glu Lys Leu Ile
305                 310                 315                 320
Thr Asn Ala Glu Lys Cys Thr Leu Asn Phe Arg Pro Thr Asp Val Asp
                325                 330                 335
Ile Val His Glu Cys Ser Ser Cys Ser Thr Asn Ser Asp Ser Ile Arg
            340                 345                 350
Arg Gln Ala Ala Phe Arg Lys Asn Ala His Asp Asn Phe Leu Tyr Ser
        355                 360                 365
Pro Asn Ala Val Asp Leu Ala Glu Asp Asp Ile Ala His Phe Gln Phe
    370                 375                 380
His Trp Met Lys Ala Glu Pro Val Ile Val Arg Asn Val Leu Glu Lys
385                 390                 395                 400
Thr Ser Gly Leu Ser Trp Glu Pro Met Val Met Trp Arg Ala Cys Arg
                405                 410                 415
Glu Met Asp Pro Lys Arg Lys Gly Thr Glu Glu Glu Thr Thr Lys Val
            420                 425                 430
Lys Ala Leu Asp Cys Leu Asp Trp Cys Glu Val Glu Ile Asn Leu His
        435                 440                 445
Gln Phe Phe Glu Gly Tyr Leu Glu Gly Arg Met His Lys Asn Gly Trp
    450                 455                 460
Pro Glu Met Leu Lys Leu Lys Asp Trp Pro Pro Ser Asp Leu Phe Glu
465                 470                 475                 480
Lys Arg Leu Pro Arg His Asn Ala Glu Phe Ile Ala Ala Leu Pro Phe
                485                 490                 495
Phe Asp Tyr Thr Asp Pro Lys Ser Gly Ile Leu Asn Leu Ala Thr Arg
            500                 505                 510
Phe Pro Glu Gly Ser Leu Lys Pro Asp Leu Gly Pro Lys Thr Tyr Ile
        515                 520                 525
Ala Tyr Gly Phe His Glu Glu Leu Asn Arg Gly Asp Ser Val Thr Lys
    530                 535                 540
Leu His Cys Asp Ile Ser Asp Ala Val Asn Val Leu Thr His Thr Ala
545                 550                 555                 560
Lys Val Glu Ile Pro Pro Val Lys Tyr Gln Asn Ile Lys Val His Gln
                565                 570                 575
Lys Lys Tyr Ala Glu Ala Met Leu Gln Lys Gln Gln Tyr Ser Gly Gln
            580                 585                 590
Val Lys Glu Ala Ser Glu Leu Glu Asn Lys Ser Met Lys Glu Val Asp
        595                 600                 605
Glu Ser Lys Lys Asp Leu Lys Asp Lys Ala Ala Asn Glu Glu Gln Ser
    610                 615                 620
Asn Asn Ser Ser Arg Pro Ser Gly Ser Gly Glu Ala Glu Lys Val Ile
625                 630                 635                 640
Ile Ser Lys Glu Asp Asn Pro Thr Gln Pro Ala Val Ser Thr Ser Val
                645                 650                 655
Glu Ser Ile Gln Glu Gln Lys Leu Asp Ala Pro Lys Glu Thr Asp Gly
            660                 665                 670
Asn Thr Asn Glu Arg Ser Lys Ala Val His Gly Gly Ala Val Trp Asp
        675                 680                 685
Ile Phe Arg Arg Glu Asp Val Pro Lys Leu Ile Gln Phe Leu Lys Arg
    690                 695                 700
His Glu His Glu Phe Arg His Phe Asn Asn Glu Pro Leu Glu Ser Val
705                 710                 715                 720
Ile His Pro Ile His Asp Gln Thr Met Phe Leu Ser Asp Ser Gln Lys
                725                 730                 735
Lys Gln Leu Lys Glu Glu Phe Asp Ile Glu Pro Trp Thr Phe Glu Gln
            740                 745                 750
His Leu Gly Glu Ala Val Phe Ile Pro Ala Gly Cys Pro His Gln Val
        755                 760                 765
Arg Asn Arg Gln Ser Cys Ile Lys Val Ala Leu Asp Phe Val Ala Pro
```

```
        770                 775                 780
    Glu Ser Val Glu Glu Cys Leu Arg Leu Thr Gln Glu Phe Arg Arg Leu
    785                 790                 795                 800
    Pro Lys Asp His Ser Ser Ser Glu Asp Lys Leu Glu Leu Lys Lys Ile
                    805                 810                 815
    Ala Leu Tyr Ala Ala Ser Ser Ala Ile Arg Glu Val Lys Gly Leu Met
                820                 825                 830
    Gln Ser Ser Arg Arg Ser Asp Thr
            835                 840
```

<210> 123
<211> 2865
<212> DNA
<213> Arabidopsis thaliana

<400> 123

```
atggatcagc ttgcatctct agcagagtct gtggctatgg aggaagattc tgagaaacaa     60
tcgattaaag gagagagtag tcttgaacct gactctactc cttctagtcc taaaataact    120
gctaggtgga atccatcaga agcttgcagg cctttggttg atgacgctcc catcttttat    180
cccactaatg aggattttga tgatccactt ggttacatag agaagttgcg ctctaaagca    240
gaatcatatg gcatatgtcg aattgtgccg cctgttgcat ggaggccccc ttgccctctg    300
aaggagaaaa aaatatggga gaattctaaa tttcccaccc ggattcagtt cattgacttg    360
cttcaaaacc gagaaccgat taaaaaatct actaaaacca agaagagaaa acggagaaga    420
atctctaaaa ttggatacac aagaagaaaa agagattcag gctgtgatac ggcttcctct    480
ggctctagtg atagtgaagg aaaatttggt tttcagactg ggccagattt taccctggaa    540
gaatttcaga agtatgatga atactttaag gaatgctatt ttcagtcaga ggatcatcct    600
ggttccaaag cgtctgaaaa taagaaattt aaacctaagg ttaaggacct tgaaggtgaa    660
tattggcgga tagtggagca ggcaactgat gaagtagagg tgtactatgg agctgacttg    720
gaaacaaaga aatttggaag tggtttccca aagtataaac cggggtatcc tataagtgaa    780
gcagatcaat actctcaatg tggttggaac ctaaataatt tgtcccgtct gcccggatca    840
gttctagctt ttgagagctg cgatatctca ggagtcattg tgccatggct ttatgttgga    900
atgtgctttt caactttttg ttggcatgtt gaagatcatc acctttattc catgaactac    960
ttacacacag gtgatccaaa agtttggtat gggatccctg gaaaccatgc tgagtctttt   1020
gaaaatgtaa tgaaaaagcg tcttccagat ttgtttgaag aacagcctga cttgctacat   1080
caactggtca ctcagttatc tccaagaatc ttaaaagagg agggagtacc tgtctatcga   1140
gctgtccagc gcagtggaga attcattcta accttcccta aggcctatca ttctgggttt   1200
aattgtggtt tcaactgtgc ggaagcagta aatgttgcac cagttgattg gctggttcat   1260
ggacagaatg cagtggaagg ctatagcaag cagcggcgaa agagttcatt gtcacatgac   1320
aagctgcttc ttggagctgc tatggaagct acttattgcc tctgggagct ttcactttca   1380
aagaagaaga ctcctgtgat tgcgagatgg aaaagggttt gtagtgagga tggattgctt   1440
acgaaggcag tcaagaagcg tgtgcagatg gaagaagaaa gactaaatca ccttcaagat   1500
ggttttagct tgcgaaagat ggagggtgat ttcgacaata agagagaacg ggagtgcttc   1560
ttgtgcttct atgatttgca tatgtctgct tcaagctgca agtgttcccc caaccggttt   1620
gcatgtctta tccacgctaa ggatctgtgt tcatgtgaaa gtaaggatag atatatccta   1680
attcgccaca ccttggatga gttatgggca ctggtcagag ctctagaagg ggatcttgat   1740
gccatagacc tatgggcaag taaatgtcgt gaccagtatc cttcacagca tccaagagca   1800
agagaatatg cttacctcaa gtctgcccca tgtataaaga gccgtggttc atcaaaagta   1860
cagcagcgag aacaaaacaa tcttcagtta gtgtctgaac gcttacaaag tgatctaacc   1920
tcaaacaagg aagttcagtt gaaacaagat ggtgattcag atgtaaatcg ccatggtcat   1980
gaaagcgaaa gaaatcatgt acatgggatc actgataagt cagctgtcac ggatgtaaaa   2040
cttggtgtgg gaggtaagtt tgatgagaag aagatttcgg ttgaatctca aaacccacat   2100
tcagtttcag atgtagggtg tagcgagctg gcgaagaaag tggatggttg tttaggaggg   2160
aaggaccaaa atgctgcaac caataggtta agtctctctg ttgagctttt gagttctgga   2220
tctctcgttg ttaaaaagct gtggtgcagt aaacaggcca tatacccaaa agggttcaag   2280
agccgtgtta agttcttaag tgtgcttgac ccaacaaacc taaccaacta catctcagag   2340
gttctggatg cagggcttct tggtccattg ttcagggtct cagtagaaga ttaccccact   2400
gagaatttct cgaatgtatc tgctgaaaag tgctggcaaa tggtgacaca aaggctcaaa   2460
ctcgaaatca tcaagaaatg tgatcaacca gttagctctt tgacctcttt acaacctttg   2520
gagagtataa atgggcttga aatgtttgga tttctctccc cgcacgtaat taaggtggtt   2580
gaggctcttg atccaaagca tcaattggag gagtattgga accaaaaagc agtgaaactg   2640
tttggtgctg aaccaataaa ggaaggagaa aaggatgata cagagaaagg aggggcttca   2700

gatccctctt tggaccggga cacaaggctt ctgcgtggac tgttgaagaa ggcgacacct   2760
gaagaattag taatgatgca tggacttctg tgtggtgaga ctcgcaacac cgagctcaag   2820
gaagagctct ctactttggt tgataagatg gagataagtc cttaa                   2865
```

<210> 124
<211> 954
<212> PRT
<213> Arabidopsis thaliana

<400> 124

```
Met Asp Gln Leu Ala Ser Leu Ala Glu Ser Val Ala Met Glu Glu Asp
1               5                   10                  15
Ser Glu Lys Gln Ser Ile Lys Gly Glu Ser Ser Leu Glu Pro Asp Ser
            20                  25                  30
Thr Pro Ser Ser Pro Lys Ile Thr Ala Arg Trp Asn Pro Ser Glu Ala

        35                  40                  45
Cys Arg Pro Leu Val Asp Asp Ala Pro Ile Phe Tyr Pro Thr Asn Glu
    50                  55                  60
Asp Phe Asp Asp Pro Leu Gly Tyr Ile Glu Lys Leu Arg Ser Lys Ala
65                  70                  75                  80
Glu Ser Tyr Gly Ile Cys Arg Ile Val Pro Pro Val Ala Trp Arg Pro
                85                  90                  95
Pro Cys Pro Leu Lys Glu Lys Lys Ile Trp Glu Asn Ser Lys Phe Pro
            100                 105                 110
Thr Arg Ile Gln Phe Ile Asp Leu Leu Gln Asn Arg Glu Pro Ile Lys
        115                 120                 125
Lys Ser Thr Lys Thr Lys Lys Arg Lys Arg Arg Arg Ile Ser Lys Ile
    130                 135                 140
Gly Tyr Thr Arg Arg Lys Arg Asp Ser Gly Cys Asp Thr Ala Ser Ser
145                 150                 155                 160
Gly Ser Ser Asp Ser Glu Gly Lys Phe Gly Phe Gln Thr Gly Pro Asp
                165                 170                 175
Phe Thr Leu Glu Glu Phe Gln Lys Tyr Asp Glu Tyr Phe Lys Glu Cys
            180                 185                 190
Tyr Phe Gln Ser Glu Asp His Pro Gly Ser Lys Ala Ser Glu Asn Lys
        195                 200                 205
Lys Phe Lys Pro Lys Val Lys Asp Leu Glu Gly Glu Tyr Trp Arg Ile
    210                 215                 220
Val Glu Gln Ala Thr Asp Glu Val Glu Val Tyr Tyr Gly Ala Asp Leu
225                 230                 235                 240
Glu Thr Lys Lys Phe Gly Ser Gly Phe Pro Lys Tyr Lys Pro Gly Tyr
                245                 250                 255
Pro Ile Ser Glu Ala Asp Gln Tyr Ser Gln Cys Gly Trp Asn Leu Asn
            260                 265                 270
Asn Leu Ser Arg Leu Pro Gly Ser Val Leu Ala Phe Glu Ser Cys Asp
        275                 280                 285
Ile Ser Gly Val Ile Val Pro Trp Leu Tyr Val Gly Met Cys Phe Ser
    290                 295                 300
Thr Phe Cys Trp His Val Glu Asp His His Leu Tyr Ser Met Asn Tyr
305                 310                 315                 320
Leu His Thr Gly Asp Pro Lys Val Trp Tyr Gly Ile Pro Gly Asn His
                325                 330                 335
Ala Glu Ser Phe Glu Asn Val Met Lys Lys Arg Leu Pro Asp Leu Phe
            340                 345                 350
Glu Glu Gln Pro Asp Leu Leu His Gln Leu Val Thr Gln Leu Ser Pro
        355                 360                 365
Arg Ile Leu Lys Glu Glu Gly Val Pro Val Tyr Arg Ala Val Gln Arg
    370                 375                 380
Ser Gly Glu Phe Ile Leu Thr Phe Pro Lys Ala Tyr His Ser Gly Phe
385                 390                 395                 400
```

Asn Cys Gly Phe Asn Cys Ala Glu Ala Val Asn Val Ala Pro Val Asp
405 410 415
Trp Leu Val His Gly Gln Asn Ala Val Glu Gly Tyr Ser Lys Gln Arg
420 425 430
Arg Lys Ser Ser Leu Ser His Asp Lys Leu Leu Leu Gly Ala Ala Met
435 440 445
Glu Ala Thr Tyr Cys Leu Trp Glu Leu Ser Leu Ser Lys Lys Lys Thr
450 455 460
Pro Val Ile Ala Arg Trp Lys Arg Val Cys Ser Glu Asp Gly Leu Leu
465 470 475 480
Thr Lys Ala Val Lys Lys Arg Val Gln Met Glu Glu Glu Arg Leu Asn
485 490 495
His Leu Gln Asp Gly Phe Ser Leu Arg Lys Met Glu Gly Asp Phe Asp
500 505 510
Asn Lys Arg Glu Arg Glu Cys Phe Leu Cys Phe Tyr Asp Leu His Met
515 520 525
Ser Ala Ser Ser Cys Lys Cys Ser Pro Asn Arg Phe Ala Cys Leu Ile
530 535 540
His Ala Lys Asp Leu Cys Ser Cys Glu Ser Lys Asp Arg Tyr Ile Leu
545 550 555 560
Ile Arg His Thr Leu Asp Glu Leu Trp Ala Leu Val Arg Ala Leu Glu
565 570 575
Gly Asp Leu Asp Ala Ile Asp Leu Trp Ala Ser Lys Cys Arg Asp Gln
580 585 590
Tyr Pro Ser Gln His Pro Arg Ala Arg Glu Tyr Ala Tyr Leu Lys Ser
595 600 605
Ala Pro Cys Ile Lys Ser Arg Gly Ser Ser Lys Val Gln Gln Arg Glu
610 615 620
Gln Asn Asn Leu Gln Leu Val Ser Glu Arg Leu Gln Ser Asp Leu Thr
625 630 635 640
Ser Asn Lys Glu Val Gln Leu Lys Gln Asp Gly Asp Ser Asp Val Asn
645 650 655
Arg His Gly His Glu Ser Glu Arg Asn His Val His Gly Ile Thr Asp
660 665 670
Lys Ser Ala Val Thr Asp Val Lys Leu Gly Val Gly Gly Lys Phe Asp
675 680 685
Glu Lys Lys Ile Ser Val Glu Ser Gln Asn Pro His Ser Val Ser Asp
690 695 700
Val Gly Cys Ser Glu Leu Ala Lys Lys Val Asp Gly Cys Leu Gly Gly
705 710 715 720
Lys Asp Gln Asn Ala Ala Thr Asn Arg Leu Ser Leu Ser Val Glu Leu
725 730 735
Leu Ser Ser Gly Ser Leu Val Val Lys Lys Leu Trp Cys Ser Lys Gln
740 745 750
Ala Ile Tyr Pro Lys Gly Phe Lys Ser Arg Val Lys Phe Leu Ser Val
755 760 765
Leu Asp Pro Thr Asn Leu Thr Asn Tyr Ile Ser Glu Val Leu Asp Ala
770 775 780
Gly Leu Leu Gly Pro Leu Phe Arg Val Ser Val Glu Asp Tyr Pro Thr
785 790 795 800
Glu Asn Phe Ser Asn Val Ser Ala Glu Lys Cys Trp Gln Met Val Thr
805 810 815
Gln Arg Leu Lys Leu Glu Ile Ile Lys Lys Cys Asp Gln Pro Val Ser
820 825 830
Ser Leu Thr Ser Leu Gln Pro Leu Glu Ser Ile Asn Gly Leu Glu Met
835 840 845
Phe Gly Phe Leu Ser Pro His Val Ile Lys Val Val Glu Ala Leu Asp
850 855 860
Pro Lys His Gln Leu Glu Glu Tyr Trp Asn Gln Lys Ala Val Lys Leu
865 870 875 880
Phe Gly Ala Glu Pro Ile Lys Glu Gly Glu Lys Asp Asp Thr Glu Lys

```
                    885                     890                     895
Gly Gly Ala Ser Asp Pro Ser Leu Asp Arg Asp Thr Arg Leu Leu Arg
                900                     905                     910
Gly Leu Leu Lys Lys Ala Thr Pro Glu Glu Leu Val Met Met His Gly
            915                     920                     925
Leu Leu Cys Gly Glu Thr Arg Asn Thr Glu Leu Lys Glu Glu Leu Ser
    930                     935                     940
Thr Leu Val Asp Lys Met Glu Ile Ser Pro
945                     950
```

<210> 125
<211> 2694
<212> DNA
<213> Arabidopsis thaliana

<400> 125

```
atgatactgc ttcatggtca ggattttgat gatccacttg gttacataga gaagttgcgc     60
tctaaagcag aatcatatgg catatgtcga attgtgccgc ctgttgcatg gaggcccccct   120
tgccctctga aggagaaaaa aatatgggag aattctaaat ttcccacccg gattcagttc    180
attgacttgc ttcaaaaccg agaaccgatt aaaaaatcta ctaaaaccaa gaagagaaaa    240
cggagaagaa tctctaaaat tggatacaca agaagaaaaa gagattcagg ctgtgatacg    300
gcttcctctg gctctagtga tagtgaagga aaatttggtt ttcagactgg gccagatttt    360
accctggaag aatttcagaa gtatgatgaa tactttaagg aatgctattt tcagtcagag    420
gatcatcctg gttccaaagc gtctgaaaat aagaaattta aacctaaggt taaggacctt    480
gaaggtgaat attggcggat agtggagcag gcaactgatg aagtagaggt gtactatgga    540
gctgacttgg aaacaaagaa atttggaagt ggtttcccaa agtataaacc ggggtatcct    600
ataagtgaag cagatcaata ctctcaatgt ggttggaacc taaataattt gtcccgtctg    660
cccggatcag ttctagcttt tgagagctgc gatatctcag gagtcattgt gccatggctt    720
tatgttggaa tgtgcttttc aactttttgt tggcatgttg aagatcatca cctttattcc    780
atgaactact tacacacagg tgatccaaaa gtttggtatg ggatccctgg aaaccatgct    840
gagtcttttg aaaatgtaat gaaaaagcgt cttccagatt tgtttgaaga acagcctgac    900
ttgctacatc aactggtcac tcagttatct ccaagaatct taaaagagga gggagtacct    960
gtctatcgag ctgtccagcg cagtggagaa ttcattctaa ccttccctaa ggcctatcat   1020
tctgggttta attgtggttt caactgtgcg gaagcagtaa atgttgcacc agttgattgg   1080
ctggttcatg gacagaatgc agtggaaggc tatagcaagc agcggcgaaa gagttcattg   1140
tcacatgaca agctgcttct tggagctgct atggaagcta cttattgcct ctgggagctt   1200
tcactttcaa agaagaagac tcctgtgatt gcgagatgga aaagggtttg tagtgaggat   1260
ggattgctta cgaaggcagt caagaagcgt gtgcagatgg aagaagaaag actaaatcac   1320
cttcaagatg gttttagctt gcgaaagatg gagggtgatt tcgacaataa gagagaacgg   1380
gagtgcttct tgtgcttcta tgatttgcat atgtctgctt caagctgcaa gtgttccccc   1440
aaccggtttg catgtcttat ccacgctaag gatctgtgtt catgtgaaag taaggataga   1500
tatatcctaa ttcgccacac cttggatgag ttatgggcac tggtcagagc tctagaaggg   1560
gatcttgatg ccatagacct atgggcaagt aaatgtcgtg accagtatcc ttcacagcat   1620
ccaagagcaa gagaatatgc ttacctcaag tctgccccat gtataaagag ccgtggttca   1680
tcaaaagtac agcagcgaga acaaaacaat cttcagttag tgtctgaacg cttacaaagt   1740
gatctaacct caaacaagga agttcagttg aaacaagatg gtgattcaga tgtaaatcgc   1800
catggtcatg aaagcgaaag aaatcatgta catgggatca ctgataagtc agctgtcacg   1860
gatgtaaaac ttggtgtggg aggtaagttt gatgagaaga agatttcggt tgaatctcaa   1920
aacccacatt cagtttcaga tgtagggtgt agcgagctgg cgaagaaagt ggatggttgt   1980
ttaggaggga aggaccaaaa tgctgcaacc aataggttaa gtctctctgt tgagcttttg   2040
agttctggat ctctcgttgt taaaaagctg tggtgcagta aacaggccat atacccaaaa   2100
gggttcaaga gccgtgttaa gttcttaagt gtgcttgacc caacaaacct aaccaactac   2160
atctcagagg ttctggatgc agggcttctt ggtccattgt tcagggtctc agtagaagat   2220
taccccactg agaatttctc gaatgtatct gctgaaaagt gctggcaaat ggtgacacaa   2280
aggctcaaac tcgaaatcat caagaaatgt gatcaaccag ttagctcttt gacctcttta   2340
caacctttgg agagtataaa tgggcttgaa atgtttggat ttctctcccc gcacgtaatt   2400
aaggtggttg aggctcttga tccaaagcat caattggagg agtattggaa ccaaaaagca   2460
gtgaaactgt ttggtgctga accaataaag gaaggagaaa aggatgatac agagaaagga   2520
ggggcttcag atccctcttt ggaccgggac acaaggcttc tgcgtggact gttgaagaag   2580
gcgacacctg aagaattagt aatgatgcat ggacttctgt gtggtgagac tcgcaacacc   2640
gagctcaagg aagagctctc tactttggtt gataagatgg agataagtcc ttaa         2694
```

<210> 126
<211> 897
<212> PRT
<213> Arabidopsis thaliana

<400> 126

```
Met Ile Leu Leu His Gly Gln Asp Phe Asp Asp Pro Leu Gly Tyr Ile
1               5                   10                  15
Glu Lys Leu Arg Ser Lys Ala Glu Ser Tyr Gly Ile Cys Arg Ile Val
            20                  25                  30
Pro Pro Val Ala Trp Arg Pro Pro Cys Pro Leu Lys Glu Lys Lys Ile
        35                  40                  45
Trp Glu Asn Ser Lys Phe Pro Thr Arg Ile Gln Phe Ile Asp Leu Leu
    50                  55                  60
Gln Asn Arg Glu Pro Ile Lys Lys Ser Thr Lys Thr Lys Lys Arg Lys
65                  70                  75                  80
Arg Arg Arg Ile Ser Lys Ile Gly Tyr Thr Arg Arg Lys Arg Asp Ser
                85                  90                  95
Gly Cys Asp Thr Ala Ser Ser Gly Ser Ser Asp Ser Glu Gly Lys Phe
            100                 105                 110
Gly Phe Gln Thr Gly Pro Asp Phe Thr Leu Glu Glu Phe Gln Lys Tyr
        115                 120                 125
Asp Glu Tyr Phe Lys Glu Cys Tyr Phe Gln Ser Glu Asp His Pro Gly
    130                 135                 140
Ser Lys Ala Ser Glu Asn Lys Lys Phe Lys Pro Lys Val Lys Asp Leu
145                 150                 155                 160
Glu Gly Glu Tyr Trp Arg Ile Val Glu Gln Ala Thr Asp Glu Val Glu
                165                 170                 175
Val Tyr Tyr Gly Ala Asp Leu Glu Thr Lys Lys Phe Gly Ser Gly Phe
            180                 185                 190
Pro Lys Tyr Lys Pro Gly Tyr Pro Ile Ser Glu Ala Asp Gln Tyr Ser
        195                 200                 205
Gln Cys Gly Trp Asn Leu Asn Asn Leu Ser Arg Leu Pro Gly Ser Val
    210                 215                 220
Leu Ala Phe Glu Ser Cys Asp Ile Ser Gly Val Ile Val Pro Trp Leu
225                 230                 235                 240
Tyr Val Gly Met Cys Phe Ser Thr Phe Cys Trp His Val Glu Asp His
                245                 250                 255
His Leu Tyr Ser Met Asn Tyr Leu His Thr Gly Asp Pro Lys Val Trp
            260                 265                 270
Tyr Gly Ile Pro Gly Asn His Ala Glu Ser Phe Glu Asn Val Met Lys
        275                 280                 285
Lys Arg Leu Pro Asp Leu Phe Glu Glu Gln Pro Asp Leu Leu His Gln
    290                 295                 300
Leu Val Thr Gln Leu Ser Pro Arg Ile Leu Lys Glu Glu Gly Val Pro
305                 310                 315                 320
Val Tyr Arg Ala Val Gln Arg Ser Gly Glu Phe Ile Leu Thr Phe Pro
                325                 330                 335
Lys Ala Tyr His Ser Gly Phe Asn Cys Gly Phe Asn Cys Ala Glu Ala
            340                 345                 350
Val Asn Val Ala Pro Val Asp Trp Leu Val His Gly Gln Asn Ala Val
        355                 360                 365
Glu Gly Tyr Ser Lys Gln Arg Arg Lys Ser Ser Leu Ser His Asp Lys
    370                 375                 380
Leu Leu Leu Gly Ala Ala Met Glu Ala Thr Tyr Cys Leu Trp Glu Leu
385                 390                 395                 400
Ser Leu Ser Lys Lys Lys Thr Pro Val Ile Ala Arg Trp Lys Arg Val
                405                 410                 415
Cys Ser Glu Asp Gly Leu Leu Thr Lys Ala Val Lys Lys Arg Val Gln
            420                 425                 430
```

```
Met Glu Glu Glu Arg Leu Asn His Leu Gln Asp Gly Phe Ser Leu Arg
        435                 440                 445
Lys Met Glu Gly Asp Phe Asp Asn Lys Arg Glu Arg Glu Cys Phe Leu
    450                 455                 460
Cys Phe Tyr Asp Leu His Met Ser Ala Ser Ser Cys Lys Cys Ser Pro
465                 470                 475                 480
Asn Arg Phe Ala Cys Leu Ile His Ala Lys Asp Leu Cys Ser Cys Glu
                485                 490                 495
Ser Lys Asp Arg Tyr Ile Leu Ile Arg His Thr Leu Asp Glu Leu Trp
            500                 505                 510
Ala Leu Val Arg Ala Leu Glu Gly Asp Leu Asp Ala Ile Asp Leu Trp
        515                 520                 525
Ala Ser Lys Cys Arg Asp Gln Tyr Pro Ser Gln His Pro Arg Ala Arg
    530                 535                 540
Glu Tyr Ala Tyr Leu Lys Ser Ala Pro Cys Ile Lys Ser Arg Gly Ser
545                 550                 555                 560
Ser Lys Val Gln Gln Arg Glu Gln Asn Asn Leu Gln Leu Val Ser Glu
                565                 570                 575
Arg Leu Gln Ser Asp Leu Thr Ser Asn Lys Glu Val Gln Leu Lys Gln
            580                 585                 590
Asp Gly Asp Ser Asp Val Asn Arg His Gly His Glu Ser Glu Arg Asn
        595                 600                 605
His Val His Gly Ile Thr Asp Lys Ser Ala Val Thr Asp Val Lys Leu
    610                 615                 620
Gly Val Gly Gly Lys Phe Asp Glu Lys Lys Ile Ser Val Glu Ser Gln
625                 630                 635                 640
Asn Pro His Ser Val Ser Asp Val Gly Cys Ser Glu Leu Ala Lys Lys
                645                 650                 655
Val Asp Gly Cys Leu Gly Gly Lys Asp Gln Asn Ala Ala Thr Asn Arg
            660                 665                 670
Leu Ser Leu Ser Val Glu Leu Leu Ser Ser Gly Ser Leu Val Val Lys
        675                 680                 685
Lys Leu Trp Cys Ser Lys Gln Ala Ile Tyr Pro Lys Gly Phe Lys Ser
    690                 695                 700
Arg Val Lys Phe Leu Ser Val Leu Asp Pro Thr Asn Leu Thr Asn Tyr
705                 710                 715                 720
Ile Ser Glu Val Leu Asp Ala Gly Leu Leu Gly Pro Leu Phe Arg Val
                725                 730                 735
Ser Val Glu Asp Tyr Pro Thr Glu Asn Phe Ser Asn Val Ser Ala Glu
            740                 745                 750
Lys Cys Trp Gln Met Val Thr Gln Arg Leu Lys Leu Glu Ile Ile Lys
        755                 760                 765
Lys Cys Asp Gln Pro Val Ser Ser Leu Thr Ser Leu Gln Pro Leu Glu
    770                 775                 780
Ser Ile Asn Gly Leu Glu Met Phe Gly Phe Leu Ser Pro His Val Ile
785                 790                 795                 800
Lys Val Val Glu Ala Leu Asp Pro Lys His Gln Leu Glu Glu Tyr Trp
                805                 810                 815
Asn Gln Lys Ala Val Lys Leu Phe Gly Ala Glu Pro Ile Lys Glu Gly
            820                 825                 830
Glu Lys Asp Asp Thr Glu Lys Gly Gly Ala Ser Asp Pro Ser Leu Asp
        835                 840                 845
Arg Asp Thr Arg Leu Leu Arg Gly Leu Leu Lys Lys Ala Thr Pro Glu
    850                 855                 860
Glu Leu Val Met Met His Gly Leu Leu Cys Gly Glu Thr Arg Asn Thr
865                 870                 875                 880
Glu Leu Lys Glu Glu Leu Ser Thr Leu Val Asp Lys Met Glu Ile Ser
                885                 890                 895
Pro
```

<210> 127
<211> 4023

<212> DNA
<213> Arabidopsis thaliana

<400> 127

```
atgggtaatg ttgaaattcc gaattggctt aaagctttgc cattggcacc tgtgtttaga      60
cctacagaca ctgaatttgc agatcccatt gcttacatat cgaaaatcga aaaggaagct     120
agtgcctttg ggatttgcaa gatcattcct cctttaccca agccatcgaa aaagtatgtt     180
ttctacaact tgaacaagtc tcttttgaag tgtcccgaat tggtttcaga tgttgacatt     240
tcgaaggtgt gtaaagagga tagagctgta ttcaccacta ggcagcaaga gttaggccaa     300
actgtgaaaa aaaacaaagg agagaagggt aagagtaatt ctcaaaggag tggtgttaag     360
caggtttggc aaagtggagg cgtttataca ttggatcagt ttgaagctaa gtcgaaagct     420
ttctacaaaa cccaattagg aacggttaag gaactggcac cagttgtcat tgaggcattg     480
ttctggaaag cggctttaga gaagcctata tatatagagt atgctaatga cgtgcctggc     540
tctgctttcg gtgagccaga ggatcatttc aggcatttcc ggcagaggaa gaggaggggc     600
aggggatttt atcagaggaa gacagaaaat aatgatccat caggtaagaa cggtgaaaag     660
tcatcgcctg aggtagaaaa ggcacccctt gcttctacaa gtttatcatc tcaggattcg     720
tccaagcaga agaacatgga tattgttgat gaaatggaag gtactgcagg ctggaagctc     780
tccaacagtt cgtggaacct tcagatgatt gcacgttcac ctggttctgt tacacgtttc     840
atgccagatg acattcctgg tgttacatct cccatggttt atatcggtat gttgttcagt     900
tggtttgcct ggcacgttga ggatcatgag cttcacagta tgaattatct tcacactggc     960
tcaccaaaga cttggtacgc tgtcccttgt gattatgcgc ttgactttga agaggttatc    1020
cgcaaaaatt cttatggcag gaacattgac caactggctg ctctcaccca actaggcgaa    1080
aagacaactc ttgtatcacc tgagatgata gttgcatctg gcattccctg ctgtaggttg    1140
gtacaaaatc ctggtgaatt tgttgtgact tttccgaggt cttatcatgt aggattcagc    1200
cacggtttta actgtgggga agccgctaat tttggaaccc cacagtggct caacgtagca    1260
aaggaagctg ctgtgcgtcg ggcagccatg aattatctac ccatgctgtc ccatcagcag    1320
ctgctatatc tcttgactat gtcctttgtt tcaagagtgc cacgatcatt gctaccaggt    1380
ggtcgtagct cccgactaag agatcggcag agagaagaaa gagagttcct tgtgaaaaga    1440
gcttttgtag aagatatatt gaacgaaaac aagaatttat ctgttcttct tcgagaacca    1500
ggctctcgtt tggtgatgtg ggaccctgat ttacttccgc gtcatagcgc actggctctt    1560
gcagctgctg gagttgctgg tgcttctgct gtttcacctc cagcagtggc aaaaaaagaa    1620
cttgaggagg gtcattctga attgcagaat aaagaaaaga cttctctttt agaggaattg    1680
agcttgttca tggagaagct gaatgatgta tactacgacg atgatgatgg tctgcttaac    1740
gatttccaag ttgatactgg aaccttgcca tgtgtggctt gtggcgttct tggcttcccc    1800
tttatgtctg tggtacagcc ttctgaaaaa gcattaaaag atctctcaga gagacaagga    1860
gagacagatg ctcaggaaat tatgacactg tcatcagaaa agtctgactg cgaatggaaa    1920
acgtcttcta gatatataag acctcgcatt ttctgcctcg aacacactat tgaacttcag    1980
agactgctgc aatcaagagg tggattgaaa ttccttgtta tttgccataa agactttcaa    2040
aaattcaagg cacatgcggc tatagtggca gaggaggtca aagtcccttt cagctatgat    2100
gatgtcctgt tagagagtgc atctcaggaa gagttgagtc taattgatct tgcaattgaa    2160
gatgaagaaa aatacgaaca cagtgtagac tggacctcag aacttggcat caatttacgg    2220
tactgtgtga aagtgaggaa aaattcccct actaagaaaa ttcagcatgc actgtcacta    2280
ggtggcttgt tctccgatac aagccagatg ctagatttta caactatcag atggctgcag    2340
agaaaatcac gctcaaaagc taaacccagt tctacctcaa gtttcacacc ttgtgaacat    2400
cttgaagtaa aagcagatgg aaaattaagg gataatttgg attctcagac cggaaaaaag    2460
gaagaaaaga tcatccagta ctcgagaaag aaaaagttga atccaaagcc ttcagcagaa    2520
caagttcagg agctagctac cctagctaaa tcaaaagatt ttgataagac atgtaaaaat    2580
tttagcagta ggtcacatct ggatagtgca attcgttctg agatgaacag tgagattgga    2640
gactctggaa gggtcattgg ggtatcattt tccataaatc cttgtagctc atctttcact    2700
gtgggacatg ggcaagaaca tcccgagatc actgtcaagt ttggttcaga tttagatggg    2760
aatgtcacaa atagtttgag tatggtgaat ggagactctg ctgacctaac cttaacctcc    2820
atatccagag agcagcacca aggacactct atgaccagca ataataatgg ctccaactca    2880
ggtagtcatg ttgtggcaag ccagaccata cttgtttcta caggtgataa tcatgacgga    2940
ccaagaaaat tgtctggtga ttatgtctgc agtgatgtgt ctgtacgtgg tattcaggaa    3000
gcggttgaaa tgagtgacca agagtttgga gaacctaggt ctactgtcac taatattgag    3060
gatgaacagc aatcacagat tgtgaaacca acccaaagag aagctgtatt tggtgatcac    3120
gaacaggtgg agggagcaga agctgtgtct accagagaaa acttgtgctc tgaaataatt    3180
ctgcacactg agcattcttc agctcatgtg ggtatggaaa ttcctgacat aaacacggcc    3240
agtgagaact tagttgttga catgacccat gatggtgaac ctttggaaag tagcgatata    3300
```

```
ttaagttcga gcaatggtga tgaagcttct tcaaatggat tgcaagttct aaatgatgag   3360
cttagcatgg agagtgaagt ttcaagctca gaaaacaccg aagttattga ggctcccaat   3420
tctatgggag aagcaaagaa gaagcggaaa atagaatcag agtctgagac aaatgataat   3480
ccagagagta gcattggttt cataaggagt ccttgtgaag ggttgaggtc aaggggcaag   3540
aggaaagcaa catgtgaaac ttcattaaag cacactgaaa cgagtgatga agagaagaaa   3600
cccattgcga aaaggctgaa gaaaacacca aaggcttgct cagggagtcg tcagcaagaa   3660
gtccccacga caactcaccc caaccgttgt tacctagagg gatgcaagat gacttttgag   3720
agtaaagcga aattacaaac tcacaagaga aaccgttgca ctcacgaagg gtgtggaaag   3780
aaattcaggg ctcacaaata tctggtgctt catcaacgtg tgcataagga tgaaagacct   3840
ttcgagtgct cttggaaagg atgttcaatg actttcaaat ggcaatgggc gaggacagaa   3900
catttgcgtc tgcacacggg agagcgacca tacatatgca aggtcgatgg atgtggattg   3960
tcgtttaggt ttgtatctga ctatagtcgc cacagacgta aaaccatgca ctatgtcaca   4020
tag                                                                 4023
```

<210> 128
<211> 1340
<212> PRT
<213> Arabidopsis thaliana

<400> 128

Met Gly Asn Val Glu Ile Pro Asn Trp Leu Lys Ala Leu Pro Leu Ala
1               5                   10                  15
Pro Val Phe Arg Pro Thr Asp Thr Glu Phe Ala Asp Pro Ile Ala Tyr
            20                  25                  30
Ile Ser Lys Ile Glu Lys Glu Ala Ser Ala Phe Gly Ile Cys Lys Ile
        35                  40                  45
Ile Pro Pro Leu Pro Lys Pro Ser Lys Lys Tyr Val Phe Tyr Asn Leu
    50                  55                  60
Asn Lys Ser Leu Leu Lys Cys Pro Glu Leu Val Ser Asp Val Asp Ile
65                  70                  75                  80
Ser Lys Val Cys Lys Glu Asp Arg Ala Val Phe Thr Thr Arg Gln Gln
                85                  90                  95
Glu Leu Gly Gln Thr Val Lys Lys Asn Lys Gly Glu Lys Gly Lys Ser
            100                 105                 110
Asn Ser Gln Arg Ser Gly Val Lys Gln Val Trp Gln Ser Gly Gly Val
        115                 120                 125
Tyr Thr Leu Asp Gln Phe Glu Ala Lys Ser Lys Ala Phe Tyr Lys Thr
    130                 135                 140
Gln Leu Gly Thr Val Lys Glu Leu Ala Pro Val Val Ile Glu Ala Leu
145                 150                 155                 160
Phe Trp Lys Ala Ala Leu Glu Lys Pro Ile Tyr Ile Glu Tyr Ala Asn
                165                 170                 175
Asp Val Pro Gly Ser Ala Phe Gly Glu Pro Glu Asp His Phe Arg His
            180                 185                 190
Phe Arg Gln Arg Lys Arg Arg Gly Arg Gly Phe Tyr Gln Arg Lys Thr
        195                 200                 205
Glu Asn Asn Asp Pro Ser Gly Lys Asn Gly Glu Lys Ser Ser Pro Glu
    210                 215                 220
Val Glu Lys Ala Pro Leu Ala Ser Thr Ser Leu Ser Ser Gln Asp Ser
225                 230                 235                 240
Ser Lys Gln Lys Asn Met Asp Ile Val Asp Glu Met Glu Gly Thr Ala
                245                 250                 255
Gly Trp Lys Leu Ser Asn Ser Ser Trp Asn Leu Gln Met Ile Ala Arg
            260                 265                 270
Ser Pro Gly Ser Val Thr Arg Phe Met Pro Asp Asp Ile Pro Gly Val
        275                 280                 285
Thr Ser Pro Met Val Tyr Ile Gly Met Leu Phe Ser Trp Phe Ala Trp
    290                 295                 300
His Val Glu Asp His Glu Leu His Ser Met Asn Tyr Leu His Thr Gly
305                 310                 315                 320
Ser Pro Lys Thr Trp Tyr Ala Val Pro Cys Asp Tyr Ala Leu Asp Phe

```
                    325                 330                 335
Glu Glu Val Ile Arg Lys Asn Ser Tyr Gly Arg Asn Ile Asp Gln Leu
                340                 345                 350
Ala Ala Leu Thr Gln Leu Gly Glu Lys Thr Thr Leu Val Ser Pro Glu
            355                 360                 365
Met Ile Val Ala Ser Gly Ile Pro Cys Cys Arg Leu Val Gln Asn Pro
        370                 375                 380
Gly Glu Phe Val Val Thr Phe Pro Arg Ser Tyr His Val Gly Phe Ser
385                 390                 395                 400
His Gly Phe Asn Cys Gly Glu Ala Ala Asn Phe Gly Thr Pro Gln Trp
                405                 410                 415
Leu Asn Val Ala Lys Glu Ala Ala Val Arg Arg Ala Ala Met Asn Tyr
            420                 425                 430
Leu Pro Met Leu Ser His Gln Gln Leu Leu Tyr Leu Leu Thr Met Ser
        435                 440                 445
Phe Val Ser Arg Val Pro Arg Ser Leu Leu Pro Gly Gly Arg Ser Ser
    450                 455                 460
Arg Leu Arg Asp Arg Gln Arg Glu Glu Arg Glu Phe Leu Val Lys Arg
465                 470                 475                 480
Ala Phe Val Glu Asp Ile Leu Asn Glu Asn Lys Asn Leu Ser Val Leu
                485                 490                 495
Leu Arg Glu Pro Gly Ser Arg Leu Val Met Trp Asp Pro Asp Leu Leu
            500                 505                 510
Pro Arg His Ser Ala Leu Ala Leu Ala Ala Ala Gly Val Ala Gly Ala
        515                 520                 525
Ser Ala Val Ser Pro Pro Ala Val Ala Lys Lys Glu Leu Glu Glu Gly
    530                 535                 540
His Ser Glu Leu Gln Asn Lys Glu Lys Thr Ser Leu Leu Glu Glu Leu
545                 550                 555                 560
Ser Leu Phe Met Glu Lys Leu Asn Asp Val Tyr Tyr Asp Asp Asp Asp
                565                 570                 575
Gly Leu Leu Asn Asp Phe Gln Val Asp Thr Gly Thr Leu Pro Cys Val
            580                 585                 590
Ala Cys Gly Val Leu Gly Phe Pro Phe Met Ser Val Val Gln Pro Ser
        595                 600                 605
Glu Lys Ala Leu Lys Asp Leu Ser Glu Arg Gln Gly Glu Thr Asp Ala
    610                 615                 620
Gln Glu Ile Met Thr Leu Ser Ser Glu Lys Ser Asp Cys Glu Trp Lys
625                 630                 635                 640
Thr Ser Ser Arg Tyr Ile Arg Pro Arg Ile Phe Cys Leu Glu His Thr
                645                 650                 655
Ile Glu Leu Gln Arg Leu Leu Gln Ser Arg Gly Gly Leu Lys Phe Leu
            660                 665                 670
Val Ile Cys His Lys Asp Phe Gln Lys Phe Lys Ala His Ala Ala Ile
        675                 680                 685
Val Ala Glu Glu Val Lys Val Pro Phe Ser Tyr Asp Asp Val Leu Leu
    690                 695                 700
Glu Ser Ala Ser Gln Glu Glu Leu Ser Leu Ile Asp Leu Ala Ile Glu
705                 710                 715                 720
Asp Glu Glu Lys Tyr Glu His Ser Val Asp Trp Thr Ser Glu Leu Gly
                725                 730                 735
Ile Asn Leu Arg Tyr Cys Val Lys Val Arg Lys Asn Ser Pro Thr Lys
            740                 745                 750
Lys Ile Gln His Ala Leu Ser Leu Gly Gly Leu Phe Ser Asp Thr Ser
        755                 760                 765
Gln Met Leu Asp Phe Thr Thr Ile Arg Trp Leu Gln Arg Lys Ser Arg
    770                 775                 780
Ser Lys Ala Lys Pro Ser Ser Thr Ser Ser Phe Thr Pro Cys Glu His
785                 790                 795                 800
Leu Glu Val Lys Ala Asp Gly Lys Leu Arg Asp Asn Leu Asp Ser Gln
                805                 810                 815
```

```
Thr Gly Lys Lys Glu Glu Lys Ile Ile Gln Tyr Ser Arg Lys Lys Lys
            820                 825                 830
Leu Asn Pro Lys Pro Ser Ala Glu Gln Val Gln Glu Leu Ala Thr Leu
        835                 840                 845
Ala Lys Ser Lys Asp Phe Asp Lys Thr Cys Lys Asn Phe Ser Ser Arg
    850                 855                 860
Ser His Leu Asp Ser Ala Ile Arg Ser Glu Met Asn Ser Glu Ile Gly
865                 870                 875                 880
Asp Ser Gly Arg Val Ile Gly Val Ser Phe Ser Ile Asn Pro Cys Ser
                885                 890                 895
Ser Ser Phe Thr Val Gly His Gly Gln Glu His Pro Glu Ile Thr Val
            900                 905                 910
Lys Phe Gly Ser Asp Leu Asp Gly Asn Val Thr Asn Ser Leu Ser Met
        915                 920                 925
Val Asn Gly Asp Ser Ala Asp Leu Thr Leu Thr Ser Ile Ser Arg Glu
    930                 935                 940
Gln His Gln Gly His Ser Met Thr Ser Asn Asn Asn Gly Ser Asn Ser
945                 950                 955                 960
Gly Ser His Val Val Ala Ser Gln Thr Ile Leu Val Ser Thr Gly Asp
                965                 970                 975
Asn His Asp Gly Pro Arg Lys Leu Ser Gly Asp Tyr Val Cys Ser Asp
            980                 985                 990
Val Ser Val Arg Gly Ile Gln Glu  Ala Val Glu Met Ser  Asp Gln Glu
        995                 1000                1005
Phe Gly  Glu Pro Arg Ser Thr  Val Thr Asn Ile Glu  Asp Glu Gln
    1010                1015                1020
Gln Ser  Gln Ile Val Lys Pro  Thr Gln Arg Glu Ala  Val Phe Gly
    1025                1030                1035
Asp His  Glu Gln Val Glu Gly  Ala Glu Ala Val Ser  Thr Arg Glu
    1040                1045                1050
Asn Leu  Cys Ser Glu Ile Ile  Leu His Thr Glu His  Ser Ser Ala
    1055                1060                1065
His Val  Gly Met Glu Ile Pro  Asp Ile Asn Thr Ala  Ser Glu Asn
    1070                1075                1080
Leu Val  Val Asp Met Thr His  Asp Gly Glu Pro Leu  Glu Ser Ser
    1085                1090                1095
Asp Ile  Leu Ser Ser Ser Asn  Gly Asp Glu Ala Ser  Ser Asn Gly
    1100                1105                1110
Leu Gln  Val Leu Asn Asp Glu  Leu Ser Met Glu Ser  Glu Val Ser
    1115                1120                1125
Ser Ser  Glu Asn Thr Glu Val  Ile Glu Ala Pro Asn  Ser Met Gly
    1130                1135                1140
Glu Ala  Lys Lys Lys Arg Lys  Ile Glu Ser Glu Ser  Glu Thr Asn
    1145                1150                1155
Asp Asn  Pro Glu Ser Ser Ile  Gly Phe Ile Arg Ser  Pro Cys Glu
    1160                1165                1170
Gly Leu  Arg Ser Arg Gly Lys  Arg Lys Ala Thr Cys  Glu Thr Ser
    1175                1180                1185
Leu Lys  His Thr Glu Thr Ser  Asp Glu Glu Lys Lys  Pro Ile Ala
    1190                1195                1200
Lys Arg  Leu Lys Lys Thr Pro  Lys Ala Cys Ser Gly  Ser Arg Gln
    1205                1210                1215
Gln Glu  Val Pro Thr Thr Thr  His Pro Asn Arg Cys  Tyr Leu Glu
    1220                1225                1230
Gly Cys  Lys Met Thr Phe Glu  Ser Lys Ala Lys Leu  Gln Thr His
    1235                1240                1245
Lys Arg  Asn Arg Cys Thr His  Glu Gly Cys Gly Lys  Lys Phe Arg
    1250                1255                1260
Ala His  Lys Tyr Leu Val Leu  His Gln Arg Val His  Lys Asp Glu
    1265                1270                1275
Arg Pro  Phe Glu Cys Ser Trp  Lys Gly Cys Ser Met  Thr Phe Lys
```

```
    1280                1285                1290
Trp Gln Trp Ala Arg Thr Glu His Leu Arg Leu His Thr Gly Glu
    1295                1300                1305
Arg Pro Tyr Ile Cys Lys Val Asp Gly Cys Gly Leu Ser Phe Arg
    1310                1315                1320
Phe Val Ser Asp Tyr Ser Arg His Arg Arg Lys Thr Met His Tyr
    1325                1330                1335
Val Thr
    1340
```

<210> 129
<211> 1509
<212> DNA
<213> Arabidopsis thaliana

<400> 129

```
atgccaaagt gcaagaatct gttgctaacc tcgaagcgac gtaaatccaa atccaaaaga     60
ctaaagctcc atcagcatga acctgagtct ctgtttccag agaaggaggt agaagaggaa    120
gatgaagatg aaggaggctt caagctgaaa attgcagctc cttctcaaga gcatggagtt    180
caacctctgg gaaacctata tttcaatcct ggtgcggtca atgtgcgaaa cactggatta    240
ggtaatctcc agatactctc agatgagctc gttttggata ttctaggtct tcttggcgcc    300
aatcatttgg gtgttttggc tactgtaact aagtcattct atatatttgc taatcatgag    360
cctctctgga gaaatcttgt gttagaggag ttaaaaggag attttttgtt taatgggtcg    420
tggagatcta cctatgtagc tgcttaccat ccgaaattca agtttgctgg agatggtgaa    480
tcgaatttga agattataga tttctattct gattatctgt ttcagagctg gttatgtgcg    540
aatcttgaaa tgaaaccgaa atggcttaga agagataata tcacacgggt gagaggcatt    600
tctgttgaag acttcattac aaagtttgag gagccgaata agccagtttt gttggaggga    660
tgtttggatg gttggcctgc tattgagaaa tggtcaagag attatctgac taaggtggtt    720
ggtgatgtcg aattcgcggt aggtccagtg gaaatgaagc tcgagaagta ttttaggtat    780
tctgatggag ctagggaaga aaggcctttg tacttgtttg atccaaagtt tgcagagaaa    840
gttccggttt tggattcgga gtatgatgtt cctgtctatt tcagagagga cttgtttggt    900
gttttaggaa acgagcgacc tgattataga tggatcataa ttggtccagc cggatcagga    960
tcgtctttcc atattgatcc taactcaaca tcagcttgga atgcggtgat cacaggtcg    1020
aagaaatggg ttttgttccc acctgatgtg gttcctccgg gtgtgcatcc aagtcctgat   1080
ggagcagaag tggcctgtcc tgtttccata atagaatggt tcatgaactt ttatgatgat   1140
actaaggatt gggagaagaa acctattgag tgtatatgca aagccgggga ggtgatgttt   1200
gtacctaatg gatggtggca tctggtgatt aacctggagg aatcgattgc tattactcag   1260
aattatgcta gcaggagtaa tttgttgaat gtgttagaat ttctgaagaa accaaatgct   1320
aaagaacttg tctccgggac aactgacaga gagaatttgc atgacaaatt caagaaagcc   1380
attgaagaag cttacccggg gactatccaa gagttagaaa agaaagcaga agaagcaaaa   1440
agagccgaag aacaaagagt ttctttctgg gactctgcca aaactgatac tttcaagttt   1500
tctttctaa                                                           1509
```

<210> 130
<211> 502
<212> PRT
<213> Arabidopsis thaliana

<400> 130

```
Met Pro Lys Cys Lys Asn Leu Leu Leu Thr Ser Lys Arg Arg Lys Ser
1               5                   10                  15
Lys Ser Lys Arg Leu Lys Leu His Gln His Glu Pro Glu Ser Leu Phe
            20                  25                  30
Pro Glu Lys Glu Val Glu Glu Glu Asp Glu Asp Glu Gly Gly Phe Lys
        35                  40                  45
Leu Lys Ile Ala Ala Pro Ser Gln Glu His Gly Val Gln Pro Leu Gly
    50                  55                  60
Asn Leu Tyr Phe Asn Pro Gly Ala Val Asn Val Arg Asn Thr Gly Leu
65                  70                  75                  80
Gly Asn Leu Gln Ile Leu Ser Asp Glu Leu Val Leu Asp Ile Leu Gly
                85                  90                  95
```

```
Leu Leu Gly Ala Asn His Leu Gly Val Leu Ala Thr Val Thr Lys Ser
            100                 105                 110
Phe Tyr Ile Phe Ala Asn His Glu Pro Leu Trp Arg Asn Leu Val Leu
        115                 120                 125
Glu Glu Leu Lys Gly Asp Phe Leu Phe Asn Gly Ser Trp Arg Ser Thr
    130                 135                 140
Tyr Val Ala Ala Tyr His Pro Lys Phe Lys Phe Ala Gly Asp Gly Glu
145                 150                 155                 160
Ser Asn Leu Lys Ile Ile Asp Phe Tyr Ser Asp Tyr Leu Phe Gln Ser
                165                 170                 175
Trp Leu Cys Ala Asn Leu Glu Met Lys Pro Lys Trp Leu Arg Arg Asp
            180                 185                 190
Asn Ile Thr Arg Val Arg Gly Ile Ser Val Glu Asp Phe Ile Thr Lys
        195                 200                 205
Phe Glu Glu Pro Asn Lys Pro Val Leu Leu Glu Gly Cys Leu Asp Gly
    210                 215                 220
Trp Pro Ala Ile Glu Lys Trp Ser Arg Asp Tyr Leu Thr Lys Val Val
225                 230                 235                 240
Gly Asp Val Glu Phe Ala Val Gly Pro Val Glu Met Lys Leu Glu Lys
                245                 250                 255
Tyr Phe Arg Tyr Ser Asp Gly Ala Arg Glu Glu Arg Pro Leu Tyr Leu
            260                 265                 270

Phe Asp Pro Lys Phe Ala Glu Lys Val Pro Val Leu Asp Ser Glu Tyr
        275                 280                 285
Asp Val Pro Val Tyr Phe Arg Glu Asp Leu Phe Gly Val Leu Gly Asn
    290                 295                 300
Glu Arg Pro Asp Tyr Arg Trp Ile Ile Ile Gly Pro Ala Gly Ser Gly
305                 310                 315                 320
Ser Ser Phe His Ile Asp Pro Asn Ser Thr Ser Ala Trp Asn Ala Val
                325                 330                 335
Ile Thr Gly Ser Lys Lys Trp Val Leu Phe Pro Pro Asp Val Val Pro
            340                 345                 350
Pro Gly Val His Pro Ser Pro Asp Gly Ala Glu Val Ala Cys Pro Val
        355                 360                 365
Ser Ile Ile Glu Trp Phe Met Asn Phe Tyr Asp Asp Thr Lys Asp Trp
    370                 375                 380
Glu Lys Lys Pro Ile Glu Cys Ile Cys Lys Ala Gly Glu Val Met Phe
385                 390                 395                 400
Val Pro Asn Gly Trp Trp His Leu Val Ile Asn Leu Glu Glu Ser Ile
                405                 410                 415
Ala Ile Thr Gln Asn Tyr Ala Ser Arg Ser Asn Leu Leu Asn Val Leu
            420                 425                 430
Glu Phe Leu Lys Lys Pro Asn Ala Lys Glu Leu Val Ser Gly Thr Thr
        435                 440                 445
Asp Arg Glu Asn Leu His Asp Lys Phe Lys Lys Ala Ile Glu Glu Ala
    450                 455                 460
Tyr Pro Gly Thr Ile Gln Glu Leu Glu Lys Lys Ala Glu Glu Ala Lys
465                 470                 475                 480
Arg Ala Glu Glu Gln Arg Val Ser Phe Trp Asp Ser Ala Lys Thr Asp
                485                 490                 495
Thr Phe Lys Phe Ser Phe
            500
```

<210> 131
<211> 2124
<212> DNA
<213> Arabidopsis thaliana

<400> 131

```
atgaaggaaa aagtggagaa gcttgagaca gatgatctaa aatggaccga gaggcttcca      60

gaatgtcctg tctacagacc aacaaaggag gagtttgagg atcctctgac ttatttgcag     120
aagattttcc cagaagcttc aaaatatggt atttgcaaga ttgtatctcc tttgacagca     180
acagttcctg ctggcgctgt gttgatgaaa gagaaatcaa actttaagtt cactaccaga     240
gtacaacccc ttcgacttgc tgagtgggat tctgatgata aagttacatt cttcatgagt     300
gggaggacct atacatttcg cgactatgag aaaatggcga acaaggtatt tgcacgcaga     360
tattgcagtg gtggctcttt gcccgactca ttcttggaga aagaattctg gaaggaaatt     420
gcgtgtggca agactgaaac agttgagtat gcgtgtgatg tagatggtag cgcattttca     480
tctgcaccag gtgacccact aggaagcagc aaatggaact tgaataaagt ttcaaggctt     540
cctaagtcta ccctgcgcct tcttgaaaca tccattccgg gagtcacaga acccatgctt     600
tacatcggaa tgcttttcag tatgtttgcc tggcatgttg aggaccatta tttgtacagc     660
attaattacc aacattgtgg agcatcaaaa acttggtatg ggattccagg ttccgcagct     720
ctaaaatttg aaaaggtggt taaagagtgt gtgtacaatg atgatattct atcaactaat     780
ggagaagatg gtgcctttga cgtgcttctg ggaagacaa ctatattccc cccaaagact      840
ctgttggatc ataatgtgcc agtgtataaa gctgtgcaaa aacctggaga atttgttgtc     900
acattcccca gggcttatca tgctggtttc agccacggtt ttaattgtgg tgaggcagtg     960
aactttgcga tgggtgattg gtttccattt ggagctattg ccagttgccg gtatgctcat    1020
cttaaccgag taccgttgct tcctcatgaa gaattgattt gtaaagaggc aatgctcttg    1080
aactcaagtt ccaaatccga gaatctggat cttacaccta cagaattgtc aggacaacga    1140
agcatcaaga ctgcatttgt acacctgatt cgttttcttc atcttgctcg atggtctcta    1200
atgaagtcag gattatgcac aggccttgtt tcgaatacat atggaaccat agtctgcagt    1260
ttgtgtaaac gggattgcta tttggcattt atcaactgcg agtgttactc acaccccgtt    1320
tgtctacgtc atgatgttaa aaagcttgac cttccatgtg ggacaacgca tactctttac    1380
ttgagagaca acattgaaga catggaagct gctgcaatga agtttgagaa ggaagatgga    1440
gtttcagatt tgattacaac tgatgaagat ttatataagt atccatcatc gattacactt    1500
ccagctgcga aagaagacgg atatacaccg tattccacta tatactttga tttctatact    1560
gaggtagaga tgacatctca tgaccagttg caatcgggaa atcccgtcat gagttatgag    1620
gcaaacgctt catgtatctc ctcagttgct gatgattatg aatgctctga ttaccaggta    1680
aatagacgag ctaactgtag cagcagtagt gattcaaagc tctcggaaga agtagcatgc    1740
agcagtagca aaaaaactcg gttcttccct gtggttcaag atgaacaact agttgcggat    1800
caggaaagtg atggatctga ttccgagtgt tttagagtca aacgccgttc ttcattgaag    1860
tttgagaacc gaacagttgt tctagacaca agagagtctg accatcatca ggaacttaag    1920
agactgaaga aatcgcatca tcacgaagga agatatagta gtagcagtag tgttagtagg    1980
caagaagaag aagaagatga gttagtaatt tcaaacagga aagaaactca gcaacaaagt    2040
gacgtgaaga tgcagaagaa gaggatcgag aatcattttg gtggctttaa acgtctgaaa    2100
gtgaaagggc taataaagcc gtaa                                           2124
```

<210> 132
<211> 707
<212> PRT
<213> Arabidopsis thaliana

<400> 132

```
Met Lys Glu Lys Val Glu Lys Leu Glu Thr Asp Asp Leu Lys Trp Thr
1               5                   10                  15
Glu Arg Leu Pro Glu Cys Pro Val Tyr Arg Pro Thr Lys Glu Glu Phe
            20                  25                  30
Glu Asp Pro Leu Thr Tyr Leu Gln Lys Ile Phe Pro Glu Ala Ser Lys
        35                  40                  45
Tyr Gly Ile Cys Lys Ile Val Ser Pro Leu Thr Ala Thr Val Pro Ala
    50                  55                  60
Gly Ala Val Leu Met Lys Glu Lys Ser Asn Phe Lys Phe Thr Thr Arg
65                  70                  75                  80
Val Gln Pro Leu Arg Leu Ala Glu Trp Asp Ser Asp Asp Lys Val Thr
                85                  90                  95
Phe Phe Met Ser Gly Arg Thr Tyr Thr Phe Arg Asp Tyr Glu Lys Met
            100                 105                 110
Ala Asn Lys Val Phe Ala Arg Arg Tyr Cys Ser Gly Gly Ser Leu Pro
        115                 120                 125
Asp Ser Phe Leu Glu Lys Glu Phe Trp Lys Glu Ile Ala Cys Gly Lys
    130                 135                 140
Thr Glu Thr Val Glu Tyr Ala Cys Asp Val Asp Gly Ser Ala Phe Ser
```

```
145                 150                 155                 160
Ser Ala Pro Gly Asp Pro Leu Gly Ser Ser Lys Trp Asn Leu Asn Lys
                165                 170                 175
Val Ser Arg Leu Pro Lys Ser Thr Leu Arg Leu Leu Glu Thr Ser Ile
            180                 185                 190
Pro Gly Val Thr Glu Pro Met Leu Tyr Ile Gly Met Leu Phe Ser Met
        195                 200                 205
Phe Ala Trp His Val Glu Asp His Tyr Leu Tyr Ser Ile Asn Tyr Gln
    210                 215                 220
His Cys Gly Ala Ser Lys Thr Trp Tyr Gly Ile Pro Gly Ser Ala Ala
225                 230                 235                 240
Leu Lys Phe Glu Lys Val Val Lys Glu Cys Val Tyr Asn Asp Asp Ile
                245                 250                 255
Leu Ser Thr Asn Gly Glu Asp Gly Ala Phe Asp Val Leu Leu Gly Lys
            260                 265                 270
Thr Thr Ile Phe Pro Pro Lys Thr Leu Leu Asp His Asn Val Pro Val
        275                 280                 285
Tyr Lys Ala Val Gln Lys Pro Gly Glu Phe Val Val Thr Phe Pro Arg
    290                 295                 300
Ala Tyr His Ala Gly Phe Ser His Gly Phe Asn Cys Gly Glu Ala Val
305                 310                 315                 320
Asn Phe Ala Met Gly Asp Trp Phe Pro Phe Gly Ala Ile Ala Ser Cys
                325                 330                 335
Arg Tyr Ala His Leu Asn Arg Val Pro Leu Leu Pro His Glu Glu Leu
            340                 345                 350
Ile Cys Lys Glu Ala Met Leu Leu Asn Ser Ser Ser Lys Ser Glu Asn
        355                 360                 365
Leu Asp Leu Thr Pro Thr Glu Leu Ser Gly Gln Arg Ser Ile Lys Thr
    370                 375                 380
Ala Phe Val His Leu Ile Arg Phe Leu His Leu Ala Arg Trp Ser Leu
385                 390                 395                 400
Met Lys Ser Gly Leu Cys Thr Gly Leu Val Ser Asn Thr Tyr Gly Thr
                405                 410                 415
Ile Val Cys Ser Leu Cys Lys Arg Asp Cys Tyr Leu Ala Phe Ile Asn
            420                 425                 430
Cys Glu Cys Tyr Ser His Pro Val Cys Leu Arg His Asp Val Lys Lys
        435                 440                 445
Leu Asp Leu Pro Cys Gly Thr Thr His Thr Leu Tyr Leu Arg Asp Asn
    450                 455                 460
Ile Glu Asp Met Glu Ala Ala Ala Met Lys Phe Glu Lys Glu Asp Gly
465                 470                 475                 480
Val Ser Asp Leu Ile Thr Thr Asp Glu Asp Leu Tyr Lys Tyr Pro Ser
                485                 490                 495
Ser Ile Thr Leu Pro Ala Ala Lys Glu Asp Gly Tyr Thr Pro Tyr Ser
            500                 505                 510
Thr Ile Tyr Phe Asp Phe Tyr Thr Glu Val Glu Met Thr Ser His Asp
        515                 520                 525
Gln Leu Gln Ser Gly Asn Pro Val Met Ser Tyr Glu Ala Asn Ala Ser
    530                 535                 540
Cys Ile Ser Ser Val Ala Asp Asp Tyr Glu Cys Ser Asp Tyr Gln Val
545                 550                 555                 560
Asn Arg Arg Ala Asn Cys Ser Ser Ser Ser Asp Ser Lys Leu Ser Glu
                565                 570                 575
Glu Val Ala Cys Ser Ser Ser Lys Lys Thr Arg Phe Phe Pro Val Val
            580                 585                 590
Gln Asp Glu Gln Leu Val Ala Asp Gln Glu Ser Asp Gly Ser Asp Ser
        595                 600                 605
Glu Cys Phe Arg Val Lys Arg Arg Ser Ser Leu Lys Phe Glu Asn Arg
    610                 615                 620
Thr Val Val Leu Asp Thr Arg Glu Ser Asp His His Gln Glu Leu Lys
625                 630                 635                 640
```

```
Arg Leu Lys Lys Ser His His His Glu Gly Arg Tyr Ser Ser Ser Ser
                645                 650                 655
Ser Val Ser Arg Gln Glu Glu Glu Glu Asp Glu Leu Val Ile Ser Asn
            660                 665                 670
Arg Lys Glu Thr Gln Gln Gln Ser Asp Val Lys Met Gln Lys Lys Arg
        675                 680                 685
Ile Glu Asn His Phe Gly Gly Phe Lys Arg Leu Lys Val Lys Gly Leu
    690                 695                 700
Ile Lys Pro
705
```

<210> 133
<211> 1389
<212> DNA
<213> Arabidopsis thaliana

<400> 133

```
atgggaatac agattatagg tcaaattgag agaatcaatg gtaaagagct gagttacggc      60
gattttgcag agagatactt agctaagaac cagccagtgg taatatccga tctcactgaa     120
gattggcgag ctcgggaaga ttgggtcagt gagaatggaa accctaatct ccacgtattt     180
gccactcact ttggaaaatc cagagttcag gttgcagatt gtgatacgag agaatttaca     240
gatcagaaaa gattagaaat gtctgttact gaatttgtgg agcaatggac taataaggat     300
tctattgagg aatctgtttt gtatctgaaa gattggcact ttgtgaagga gtatccagac     360
tatacagctt accaaacacc gccgttgttt tctgatgatt ggcttaacgt gtatctagac     420
aattaccaaa tgcatgagga tagagatagt ttccagaagt atgatcaaat aagctgctct     480
gattatcggt ttgtttatat gggtgggaaa ggatcttgga cacctctaca tgctgatgta     540
tttagatctt atagttggtc agctaatgtc tgtggtaaaa aaagatggct tttccttcct     600
cctcctcaaa gtcatcttgt ttatgacagg tacatgaaaa attgcgttta tgacattttt     660
gaagaggtga atgaaactaa attccctggt tttaagaaga ccacctggct tgagtgcatt     720
caagaacccg gtgaaattat ctttgttccc agtggatggc atcatcaagt atataacttg     780
gaagacacaa tatctattaa ccacaattgg ctcaacgcat acaacctatc ttgggtgtgg     840
gatctactgt ggaaggacta caaggacact gaagagtcaa tagaagacat aagagacata     900
tgtgatgatt ttgaagctat ctgccaacgt aatcttgctg ccaacacagg aatgaattta     960
aatgatttct tcctcttcat gtcacggttc tctttgggga acatggttct actgcaatct    1020
tactctgaca aacacaaaaa cctgaattcg tgttcattag caatggccca aaacctgtta    1080
atgaatctct caactatact gaaagtcatg atgaagatga tatctgcagg cggtgtaact    1140
gcagaggaag tgtatctgga cttgcgagaa acactggagg atccacagtt tctcagattt    1200
gtcagagaca tgggaagaac ttatgcaagg attcacatgg aggaagagga tcagtttctt    1260
tcttcgaaag aattattaca aaagctcagt ggtcttgcag gtccaaatat gcaaatatgt    1320
tctccaaagg atcttgttga aatgataaac catcacaata ctttttcctc ccaaatctat    1380
tttatttag                                                            1389
```

<210> 134
<211> 462
<212> PRT
<213> Arabidopsis thaliana

<400> 134

```
Met Gly Ile Gln Ile Ile Gly Gln Ile Glu Arg Ile Asn Gly Lys Glu
1               5                   10                  15
Leu Ser Tyr Gly Asp Phe Ala Glu Arg Tyr Leu Ala Lys Asn Gln Pro
            20                  25                  30
Val Val Ile Ser Asp Leu Thr Glu Asp Trp Arg Ala Arg Glu Asp Trp
        35                  40                  45
Val Ser Glu Asn Gly Asn Pro Asn Leu His Val Phe Ala Thr His Phe
    50                  55                  60
Gly Lys Ser Arg Val Gln Val Ala Asp Cys Asp Thr Arg Glu Phe Thr
65                  70                  75                  80
Asp Gln Lys Arg Leu Glu Met Ser Val Thr Glu Phe Val Glu Gln Trp
                85                  90                  95
Thr Asn Lys Asp Ser Ile Glu Glu Ser Val Leu Tyr Leu Lys Asp Trp


            100                 105                 110
His Phe Val Lys Glu Tyr Pro Asp Tyr Thr Ala Tyr Gln Thr Pro Pro
        115                 120                 125
Leu Phe Ser Asp Asp Trp Leu Asn Val Tyr Leu Asp Asn Tyr Gln Met
    130                 135                 140
His Glu Asp Arg Asp Ser Phe Gln Lys Tyr Asp Gln Ile Ser Cys Ser
145                 150                 155                 160
Asp Tyr Arg Phe Val Tyr Met Gly Gly Lys Gly Ser Trp Thr Pro Leu
                165                 170                 175
His Ala Asp Val Phe Arg Ser Tyr Ser Trp Ser Ala Asn Val Cys Gly
            180                 185                 190
Lys Lys Arg Trp Leu Phe Leu Pro Pro Pro Gln Ser His Leu Val Tyr
        195                 200                 205
Asp Arg Tyr Met Lys Asn Cys Val Tyr Asp Ile Phe Glu Glu Val Asn
    210                 215                 220
Glu Thr Lys Phe Pro Gly Phe Lys Lys Thr Thr Trp Leu Glu Cys Ile
225                 230                 235                 240
Gln Glu Pro Gly Glu Ile Ile Phe Val Pro Ser Gly Trp His His Gln
                245                 250                 255
Val Tyr Asn Leu Glu Asp Thr Ile Ser Ile Asn His Asn Trp Leu Asn
            260                 265                 270
Ala Tyr Asn Leu Ser Trp Val Trp Asp Leu Leu Trp Lys Asp Tyr Lys
        275                 280                 285
Asp Thr Glu Glu Ser Ile Glu Asp Ile Arg Asp Ile Cys Asp Asp Phe
    290                 295                 300
Glu Ala Ile Cys Gln Arg Asn Leu Ala Ala Asn Thr Gly Met Asn Leu
305                 310                 315                 320
Asn Asp Phe Phe Leu Phe Met Ser Arg Phe Ser Leu Gly Asn Met Val
                325                 330                 335
Leu Leu Gln Ser Tyr Ser Asp Lys His Lys Asn Leu Asn Ser Cys Ser
            340                 345                 350
Leu Ala Met Ala Gln Asn Leu Leu Met Asn Leu Ser Thr Ile Leu Lys
        355                 360                 365
Val Met Met Lys Met Ile Ser Ala Gly Gly Val Thr Ala Glu Glu Val
    370                 375                 380
Tyr Leu Asp Leu Arg Glu Thr Leu Glu Asp Pro Gln Phe Leu Arg Phe
385                 390                 395                 400
Val Arg Asp Met Gly Arg Thr Tyr Ala Arg Ile His Met Glu Glu Glu
                405                 410                 415
Asp Gln Phe Leu Ser Ser Lys Glu Leu Leu Gln Lys Leu Ser Gly Leu
            420                 425                 430
Ala Gly Pro Asn Met Gln Ile Cys Ser Pro Lys Asp Leu Val Glu Met
        435                 440                 445
Ile Asn His His Asn Thr Phe Ser Ser Gln Ile Tyr Phe Ile
    450                 455                 460
```

<210> 135
<211> 1188

<212> DNA
<213> Oryza sativa

<400> 135

```
atgtcgatgc gggagtttgt cgaccactgg gctgcgagtt cgagcaatgg ggattctgat     60
ggttccctgc tgtacctgaa agattggcat tttgtgaagg agtaccctgg ttatgttgca    120
tacaccacac caacattctt tgccgatgat tggctcaaca tgtatcttga tagtcatcct    180
atacatcgag attctgacat tgccaaccat acaaatgaaa taaactgtgc tgactatcgg    240
tttgtttaca tgggaccaaa aggaacttgg actcctcttc atgctgatgt ttttaggtca    300
tacagctggt cggcaaatgt atgcggcaga aaactatggc tctttctacc accatcacaa    360
agtcattttg tatttgatag gaacctacga tcttcggtct ataacataaa tgatgatgtt    420
tctgaaaagc agtttcctga attcaataat actaaatggc tagagtgcac tcaggagcag    480
aatgaaatca tttttgtgcc tagtgggtgg tatcaccaag tccataacct ggaggatact    540

atatcaataa accataattg gtttaatggc tacaaccttc attgggtgtg gaacttgctt    600
catgaagatt acaaggtagc aaaagattat attgaagaca ttcgggacat atgtgacgat    660
tttgagggac tatgtcaacg caacttagca gcaaatacag gcatgaactt ctatgacttc    720
ttcgtcttca taacacgttt tgctttagcc aatatagttg agctttacca tattcagaat    780
ccaaaagata ctgacttcat ttcagccgaa actgctaacc actttgtcta taatctcatg    840
tcgatccgtg atgttgcatc aaaaatggta tctacagaag ctttcaatac cgagaatatt    900
tgtaatatct cagaacaaaa ccggagtgct ttctctgata tcataaaaat attggaagaa    960
gagagtttca gaaggttatt ggtggcgttg tcaaaggcat ataattacat agacagagga   1020
caaaaagatt gtctcaaaat gaaggattca agtcagaagg gttgtttgtc agtaacctgc   1080
ttaaaacctg actgtaatgt tgttggtgac attatttctt tcatgcgtga gattcatgga   1140
cctatggatt tggtaacact aattgatagt gctctctctg atagatag                 1188
```

<210> 136
<211> 395
<212> PRT
<213> Oryza sativa

<400> 136

```
Met Ser Met Arg Glu Phe Val Asp His Trp Ala Ala Ser Ser Ser Asn
1               5                   10                  15
Gly Asp Ser Asp Gly Ser Leu Leu Tyr Leu Lys Asp Trp His Phe Val
            20                  25                  30
Lys Glu Tyr Pro Gly Tyr Val Ala Tyr Thr Thr Pro Thr Phe Phe Ala
        35                  40                  45
Asp Asp Trp Leu Asn Met Tyr Leu Asp Ser His Pro Ile His Arg Asp
    50                  55                  60
Ser Asp Ile Ala Asn His Thr Asn Glu Ile Asn Cys Ala Asp Tyr Arg
65                  70                  75                  80
Phe Val Tyr Met Gly Pro Lys Gly Thr Trp Thr Pro Leu His Ala Asp
                85                  90                  95
Val Phe Arg Ser Tyr Ser Trp Ser Ala Asn Val Cys Gly Arg Lys Leu
            100                 105                 110
Trp Leu Phe Leu Pro Pro Ser Gln Ser His Phe Val Phe Asp Arg Asn
        115                 120                 125
Leu Arg Ser Ser Val Tyr Asn Ile Asn Asp Asp Val Ser Glu Lys Gln
    130                 135                 140
Phe Pro Glu Phe Asn Asn Thr Lys Trp Leu Glu Cys Thr Gln Glu Gln
145                 150                 155                 160
Asn Glu Ile Ile Phe Val Pro Ser Gly Trp Tyr His Gln Val His Asn
                165                 170                 175
Leu Glu Asp Thr Ile Ser Ile Asn His Asn Trp Phe Asn Gly Tyr Asn
            180                 185                 190
Leu His Trp Val Trp Asn Leu Leu His Glu Asp Tyr Lys Val Ala Lys
        195                 200                 205
Asp Tyr Ile Glu Asp Ile Arg Asp Ile Cys Asp Asp Phe Glu Gly Leu
    210                 215                 220
Cys Gln Arg Asn Leu Ala Ala Asn Thr Gly Met Asn Phe Tyr Asp Phe
225                 230                 235                 240
Phe Val Phe Ile Thr Arg Phe Ala Leu Ala Asn Ile Val Glu Leu Tyr
                245                 250                 255
His Ile Gln Asn Pro Lys Asp Thr Asp Phe Ile Ser Ala Glu Thr Ala
            260                 265                 270
Asn His Phe Val Tyr Asn Leu Met Ser Ile Arg Asp Val Ala Ser Lys
        275                 280                 285
Met Val Ser Thr Glu Ala Phe Asn Thr Glu Asn Ile Cys Asn Ile Ser
    290                 295                 300
Glu Gln Asn Arg Ser Ala Phe Ser Asp Ile Ile Lys Ile Leu Glu Glu
305                 310                 315                 320
Glu Ser Phe Arg Arg Leu Leu Val Ala Leu Ser Lys Ala Tyr Asn Tyr
                325                 330                 335
Ile Asp Arg Gly Gln Lys Asp Cys Leu Lys Met Lys Asp Ser Ser Gln


            340                 345                 350
Lys Gly Cys Leu Ser Val Thr Cys Leu Lys Pro Asp Cys Asn Val Val
        355                 360                 365
Gly Asp Ile Ile Ser Phe Met Arg Glu Ile His Gly Pro Met Asp Leu
    370                 375                 380
Val Thr Leu Ile Asp Ser Ala Leu Ser Asp Arg
385                 390                 395
```

<210> 137
<211> 3861
<212> DNA
<213> Oryza sativa

<400> 137

```
atgaggccct cgccgcctcc cgccgccccg gcggcggaac cggtgccccc gtggctgaga     60
tcgctgcccg tcgcgcccga gttccgcccc accgccgccg agttcgccga tcccgtctcc    120
tacatcctca agatcgagcc cgccgcggcg ccgtacggca tctgcaaggt cgtgccgccg    180
ttgccccccgc cgccgaagaa ggccaccttc tccaacctct cccgctcctt cgccgcgctc    240
cacccggacg accgctcgcc gtccttcccc acccgccacc agcaggtcgg cctctgcccg    300
cgccgcacgc gcccggggct caagcccgtg tggcggtcct cccaccgcta cacgctcccg    360
cagttcgagt ccaaggccgg cgccacccgc aagtcgctcc tcgccggcct caacttccct    420
gcctccaggc aactcacccc gctcgaccac gaggtgctct tctggcgcgc atcggccgac    480
cgccccatcg tggtcgagta tggcagcgac atgtcgggct cgggattctc cccttgcgcc    540
gcgcagccgc agccgccgcc gcagcagcaa ccgacggcgc gggcggcggc gcaccttggg    600
gagacggcgt ggaacatgcg cggcgtggct aggagccccg gatcgctgct gcggttcatg    660
ccggaggatg tccccggggt caccacgccg atgctctacg tcgggatgat gttcagttgg    720
ttcgcgtggc acgtcgagga ccacgacctg cacagcctca actacatgca cctcggggcc    780
gccaagacgt ggtacggcgt gccgcgcgac gccgcgctcg cgttcgagga cgtggtgcgc    840
gaacatggct acggcgggga ggtgaacccc ctagaaacct tcgctacatt ggggcagaaa    900
acaacagtga tgtcccctga agtgcttgtg gagtcgggta tcccctgctg cagattggtg    960
cagaatgcag ggaatttgt ggtcactttt ccaggatctt atcactgcgg tttcagtcat   1020
ggattcaact gtggggaagc atcaaatata gctactcctg aatggttgag aatagcaaaa   1080
gaggcagcaa tccgaagagc ttcaatcaat cgtcctccca tggtttcgca ctatcaatta   1140
ctttacgatc tcgcactgtc tatgcgtttt agggaaccat ccaatggtga aatggagaca   1200
cggagctctc gaataaagga gaagaagaaa tgcgaagggg aacaactggt caagaagatg   1260
ttcatccaaa atgttattga agataatgaa ctgcttagtc atcttctaaa tgacggatca   1320
tcttgtatta ttcttccagc aaatgcccat gatggtcctg gtctttctac tttgcgctca   1380
acagatcaat caaatatgaa ttccagaata tcacataacc tatgcagtag ggaagaagct   1440
ccagaagcct caggatgctt atcgccgaac aggaatggtg ataccagaaa ttgtatttca   1500
tcagatacgc ataacatgga aggtgataag ggagatataa tgagcgctac tggattgtta   1560
gatcagggtc tattatcatg tgtcacttgt ggaattttaa gtttttcatg tgtggctgta   1620
ctcaaaccaa gagattctac agcacgatac ttgatgtctg ctgattctaa ttcaattaat   1680
aaccagcttt ctatttctgg agggagtatt ctggcagatg cgccaacaaa tgaaaggaat   1740
ggtgtcattt ctcgtccata ttctgaacac tgttgcaacg aaataatggc cgatgatgca   1800
gaaatcgata agaattctgc tcttgatctc ttggcatttg ctcatggggg tcaaccagat   1860
cctgaagaag atccactgga gaaaatactg aagattgcac atggcattaa caaatcacaa   1920
cctaatagtt caaataatgt tggttgtgtt ggaacaaagc tgtcctcaag tagcacagag   1980
cgccaagaaa gaccatcttc ccagaacgct cattgtaatg gtagctcagt tatttcaaat   2040
ggaccgaaag gtgttcgcac gagaaataag tatcaactta agatggtact ttctgaaggt   2100
tttcaggcaa aggatatcta ttctgcgaag gaaaagaagg ttcaatcaga accatcaagc   2160
tcaaaagggg atgttaagga gacaattgat gttagtggca cagagaacga tgtcggatgt   2220
aaaagcacta caatctctgt cagtgaacac aggggttcta caaagaatat gtattcagtg   2280
aaggaaaaga aggttcaatc gaaaccatca agcttaaagg ggactgttaa agagacagtt   2340
gatgtcagtg gcacagagaa cgatgctaga tgtaaaagca ttacaatctc tgtcagtgaa   2400
cacaggggtt ctacacctat gaccaatagt ttagctgcat caatcgtgaa acctgacaag   2460
gattcatcaa gaatgcatgt attttgtctt gagcatgcta ttgaggttga gaagcaactg   2520
catgctatag gtggttctaa tattatgctt atatgtcgtc cagaatatcc caaaatagaa   2580
gcggaagcaa gattgttggg tgaagaaatg ggactggtat atgactggaa gggcattcat   2640
ttcaaggagg ccaacatgga ggacaggcag aagatacagg aagttctgcg ggatgaggaa   2700
gcaataccta caagcagtga ttgggctgta aaattgggta ttaaccttta ttatagtgca   2760
aatctggcca aatctccttt atataacaaa caaatgccat acaacagagt catttatagg   2820
```

```
gcatttggct gtgattctcc taatgactcg ccagtaatgt tcaatacttg cgaaaggaaa   2880
caaagccacc agaagaaaat agtggttgct ggccggtggt gtgggaaagt atggatgtca   2940
aaacaggtcc atccatactt ggctcacaga gttgaaagcc aggaagcgga agaagcagat   3000
agaatctgtt cttatcattt tgatgagaag cacaaagctg agccagttgg gaattctagt   3060
agagtggaag cctccaaaag aaagagtagc agtttgacag acgtaacaga atcaagcaat   3120
agaagggggg aaatacctgg tgaggaaaca aacaccaaga ggcccaaaca ttctcaagag   3180
aacaatctca gagctttgga aaccgctgct gaagttgtgg tgccctcacc agctggaaca   3240
ggtctccgcg ttagctccag gattgccaac agggcaaaca agctaaaatc aaagatggaa   3300
aaggaggatg tcccttctag ccgtccaaag tctaacataa aggagaaaag cagtcatgct   3360
agcggtcaga aatcaaatgt tcaagaggcg aatgctaatt ctgctagcca tttgagagca   3420
atgccaccaa aacagaaggc tgaagcagaa gcaaagaagc aaataagaac tccaaaacct   3480
ccaaagcaag cagtggagta ctcgtgcgat attgagggtt gttctatgag ttttcgtaca   3540
aagcgggact tgtctctgca caagagtgat atctgcccag tgaaaggctg cgggaagaag   3600
ttcttctcgc acaagtatct gctgcagcat cgcaaggttc acacagatga ccgtccgcta   3660
acatgcccat ggaagggctg caacatggca ttcaagtggc catgggcaag gactgaacat   3720
ctcagggttc atacaggcga caggccctat gtttgccatg agccaggttg cgcacagaca   3780
ttcaggtttg tctcagattt cagccgtcac aagcgcaaga caggtcattc tgtcaagaag   3840
aagaagaaag caaagagttg a                                               3861
```

<210> 138
<211> 1286
<212> PRT
<213> Oryza sativa

<400> 138

```
Met Arg Pro Ser Pro Pro Pro Ala Ala Pro Ala Ala Glu Pro Val Pro
1               5                   10                  15
Pro Trp Leu Arg Ser Leu Pro Val Ala Pro Glu Phe Arg Pro Thr Ala
            20                  25                  30
Ala Glu Phe Ala Asp Pro Val Ser Tyr Ile Leu Lys Ile Glu Pro Ala
        35                  40                  45
Ala Ala Pro Tyr Gly Ile Cys Lys Val Val Pro Pro Leu Pro Pro Pro
    50                  55                  60
Pro Lys Lys Ala Thr Phe Ser Asn Leu Ser Arg Ser Phe Ala Ala Leu
65                  70                  75                  80
His Pro Asp Asp Arg Ser Pro Ser Phe Pro Thr Arg His Gln Gln Val
                85                  90                  95
Gly Leu Cys Pro Arg Arg Thr Arg Pro Gly Leu Lys Pro Val Trp Arg
            100                 105                 110
Ser Ser His Arg Tyr Thr Leu Pro Gln Phe Glu Ser Lys Ala Gly Ala
        115                 120                 125
Thr Arg Lys Ser Leu Leu Ala Gly Leu Asn Phe Pro Ala Ser Arg Gln
    130                 135                 140
Leu Thr Pro Leu Asp His Glu Val Leu Phe Trp Arg Ala Ser Ala Asp
145                 150                 155                 160
Arg Pro Ile Val Val Glu Tyr Gly Ser Asp Met Ser Gly Ser Gly Phe
                165                 170                 175
Ser Pro Cys Ala Ala Gln Pro Gln Pro Pro Pro Gln Gln Gln Pro Thr
            180                 185                 190
Ala Arg Ala Ala Ala His Leu Gly Glu Thr Ala Trp Asn Met Arg Gly
        195                 200                 205
Val Ala Arg Ser Pro Gly Ser Leu Leu Arg Phe Met Pro Glu Asp Val
    210                 215                 220
Pro Gly Val Thr Thr Pro Met Leu Tyr Val Gly Met Met Phe Ser Trp
225                 230                 235                 240
Phe Ala Trp His Val Glu Asp His Asp Leu His Ser Leu Asn Tyr Met
                245                 250                 255
His Leu Gly Ala Ala Lys Thr Trp Tyr Gly Val Pro Arg Asp Ala Ala
            260                 265                 270
Leu Ala Phe Glu Asp Val Val Arg Glu His Gly Tyr Gly Gly Glu Val
```

```
        275                 280                 285
Asn Pro Leu Glu Thr Phe Ala Thr Leu Gly Gln Lys Thr Thr Val Met
    290                 295                 300
Ser Pro Glu Val Leu Val Glu Ser Gly Ile Pro Cys Cys Arg Leu Val
305                 310                 315                 320
Gln Asn Ala Gly Glu Phe Val Val Thr Phe Pro Gly Ser Tyr His Cys
                325                 330                 335
Gly Phe Ser His Gly Phe Asn Cys Gly Glu Ala Ser Asn Ile Ala Thr
            340                 345                 350
Pro Glu Trp Leu Arg Ile Ala Lys Glu Ala Ala Ile Arg Arg Ala Ser
        355                 360                 365
Ile Asn Arg Pro Pro Met Val Ser His Tyr Gln Leu Leu Tyr Asp Leu
    370                 375                 380
Ala Leu Ser Met Arg Phe Arg Glu Pro Ser Asn Gly Glu Met Glu Thr
385                 390                 395                 400
Arg Ser Ser Arg Ile Lys Glu Lys Lys Lys Cys Glu Gly Glu Gln Leu
                405                 410                 415
Val Lys Lys Met Phe Ile Gln Asn Val Ile Glu Asp Asn Glu Leu Leu
            420                 425                 430
Ser His Leu Leu Asn Asp Gly Ser Ser Cys Ile Ile Leu Pro Ala Asn
        435                 440                 445
Ala His Asp Gly Pro Gly Leu Ser Thr Leu Arg Ser Thr Asp Gln Ser
    450                 455                 460
Asn Met Asn Ser Arg Ile Ser His Asn Leu Cys Ser Arg Glu Glu Ala
465                 470                 475                 480
Pro Glu Ala Ser Gly Cys Leu Ser Pro Asn Arg Asn Gly Asp Thr Arg
                485                 490                 495
Asn Cys Ile Ser Ser Asp Thr His Asn Met Glu Gly Asp Lys Gly Asp
            500                 505                 510
Ile Met Ser Ala Thr Gly Leu Leu Asp Gln Gly Leu Leu Ser Cys Val
        515                 520                 525
Thr Cys Gly Ile Leu Ser Phe Ser Cys Val Ala Val Leu Lys Pro Arg
    530                 535                 540
Asp Ser Thr Ala Arg Tyr Leu Met Ser Ala Asp Ser Asn Ser Ile Asn
545                 550                 555                 560
Asn Gln Leu Ser Ile Ser Gly Gly Ser Ile Leu Ala Asp Ala Pro Thr
                565                 570                 575
Asn Glu Arg Asn Gly Val Ile Ser Arg Pro Tyr Ser Glu His Cys Cys
            580                 585                 590
Asn Glu Ile Met Ala Asp Asp Ala Glu Ile Asp Lys Asn Ser Ala Leu
        595                 600                 605
Asp Leu Leu Ala Phe Ala His Gly Gly Gln Pro Asp Pro Glu Glu Asp
    610                 615                 620
Pro Leu Glu Lys Ile Leu Lys Ile Ala His Gly Ile Asn Lys Ser Gln
625                 630                 635                 640
Pro Asn Ser Ser Asn Asn Val Gly Cys Val Gly Thr Lys Leu Ser Ser
                645                 650                 655
Ser Ser Thr Glu Arg Gln Glu Arg Pro Ser Ser Gln Asn Ala His Cys
            660                 665                 670
Asn Gly Ser Ser Val Ile Ser Asn Gly Pro Lys Gly Val Arg Thr Arg
        675                 680                 685
Asn Lys Tyr Gln Leu Lys Met Val Leu Ser Glu Gly Phe Gln Ala Lys
    690                 695                 700
Asp Ile Tyr Ser Ala Lys Glu Lys Lys Val Gln Ser Glu Pro Ser Ser
705                 710                 715                 720
Ser Lys Gly Asp Val Lys Glu Thr Ile Asp Val Ser Gly Thr Glu Asn
                725                 730                 735
Asp Val Gly Cys Lys Ser Thr Thr Ile Ser Val Ser Glu His Arg Gly
            740                 745                 750
Ser Thr Lys Asn Met Tyr Ser Val Lys Glu Lys Lys Val Gln Ser Lys
        755                 760                 765
```

Pro Ser Ser Leu Lys Gly Thr Val Lys Glu Thr Val Asp Val Ser Gly
770 775 780
Thr Glu Asn Asp Ala Arg Cys Lys Ser Ile Thr Ile Ser Val Ser Glu
785 790 795 800
His Arg Gly Ser Thr Pro Met Thr Asn Ser Leu Ala Ala Ser Ile Val
805 810 815
Lys Pro Asp Lys Asp Ser Ser Arg Met His Val Phe Cys Leu Glu His
820 825 830
Ala Ile Glu Val Glu Lys Gln Leu His Ala Ile Gly Gly Ser Asn Ile
835 840 845
Met Leu Ile Cys Arg Pro Glu Tyr Pro Lys Ile Glu Ala Glu Ala Arg
850 855 860
Leu Leu Gly Glu Glu Met Gly Leu Val Tyr Asp Trp Lys Gly Ile His
865 870 875 880
Phe Lys Glu Ala Asn Met Glu Asp Arg Gln Lys Ile Gln Glu Val Leu
885 890 895
Arg Asp Glu Glu Ala Ile Pro Thr Ser Ser Asp Trp Ala Val Lys Leu
900 905 910
Gly Ile Asn Leu Tyr Tyr Ser Ala Asn Leu Ala Lys Ser Pro Leu Tyr
915 920 925
Asn Lys Gln Met Pro Tyr Asn Arg Val Ile Tyr Arg Ala Phe Gly Cys
930 935 940
Asp Ser Pro Asn Asp Ser Pro Val Met Phe Asn Thr Cys Glu Arg Lys
945 950 955 960
Gln Ser His Gln Lys Lys Ile Val Val Ala Gly Arg Trp Cys Gly Lys
965 970 975
Val Trp Met Ser Lys Gln Val His Pro Tyr Leu Ala His Arg Val Glu
980 985 990
Ser Gln Glu Ala Glu Glu Ala Asp Arg Ile Cys Ser Tyr His Phe Asp
995 1000 1005
Glu Lys His Lys Ala Glu Pro Val Gly Asn Ser Ser Arg Val Glu
1010 1015 1020
Ala Ser Lys Arg Lys Ser Ser Ser Leu Thr Asp Val Thr Glu Ser
1025 1030 1035
Ser Asn Arg Arg Gly Glu Ile Pro Gly Glu Glu Thr Asn Thr Lys
1040 1045 1050
Arg Pro Lys His Ser Gln Glu Asn Asn Leu Arg Ala Leu Glu Thr
1055 1060 1065
Ala Ala Glu Val Val Val Pro Ser Pro Ala Gly Thr Gly Leu Arg
1070 1075 1080
Val Ser Ser Arg Ile Ala Asn Arg Ala Asn Lys Leu Lys Ser Lys
1085 1090 1095
Met Glu Lys Glu Asp Val Pro Ser Ser Arg Pro Lys Ser Asn Ile
1100 1105 1110
Lys Glu Lys Ser Ser His Ala Ser Gly Gln Lys Ser Asn Val Gln
1115 1120 1125
Glu Ala Asn Ala Asn Ser Ala Ser His Leu Arg Ala Met Pro Pro
1130 1135 1140
Lys Gln Lys Ala Glu Ala Glu Ala Lys Lys Gln Ile Arg Thr Pro
1145 1150 1155
Lys Pro Pro Lys Gln Ala Val Glu Tyr Ser Cys Asp Ile Glu Gly
1160 1165 1170
Cys Ser Met Ser Phe Arg Thr Lys Arg Asp Leu Ser Leu His Lys
1175 1180 1185
Ser Asp Ile Cys Pro Val Lys Gly Cys Gly Lys Lys Phe Phe Ser
1190 1195 1200
His Lys Tyr Leu Leu Gln His Arg Lys Val His Thr Asp Asp Arg
1205 1210 1215
Pro Leu Thr Cys Pro Trp Lys Gly Cys Asn Met Ala Phe Lys Trp
1220 1225 1230

```
Pro Trp  Ala Arg Thr Glu His  Leu Arg Val His Thr  Gly Asp Arg
    1235                 1240                 1245
Pro Tyr  Val Cys His Glu Pro  Gly Cys Ala Gln Thr  Phe Arg Phe
    1250                 1255                 1260
Val Ser  Asp Phe Ser Arg His  Lys Arg Lys Thr Gly  His Ser Val
    1265                 1270                 1275
Lys Lys  Lys Lys Lys Ala Lys  Ser
    1280                 1285
```

<210> 139
<211> 2994
<212> DNA
<213> Oryza sativa

<400> 139

```
atgccgccca agaggaagcg cggtggcggg aggccgcgca aggccccgga ggacgccgcc     60
gccaaggaaa acggtgagaa gacaaacaag gaggaggaaa cgcaggcttc tcctgaggag    120
aatggcgcag ggcagactca ggcctcgaga accgcaagaa agcgcaggaa gggtcctgtt    180
gctgatccct cttcgactga acttccaccc aggaagctca gggacaggcg gaacgttcca    240
gcggtagact acaaggagaa taagcacaca aaaaagatgg atggaacttc aaccatgtgc    300
catcagtgcc aaaggaagga cagtggaaga gttgtgaggt gccggaatgg tgctgaaaaa    360
aacaggaggc acagatattg tgtcaagtgc atcaaacgct ggtacccgca tctaacagag    420
gacgattttg agaactgctg tccagtttgt cacaataact gcaattgtaa gacttgtctg    480
cggacaaatg taataaataa gggtgacaag gagtttgccg atggtaagaa caaaattaaa    540
tactctctgc gtattgcacg ctttttgctc ccttggttga aacaacttca ccaagagcag    600
atgctagaga aaagtgttga ggccacaatt aaagggatcg atgtaactga cttggaggtt    660
cctcaagctc agtttaataa tgatgagcgg atttactgtg ataactgcag aacatccatt    720
gttgacttcc acagaagttg taaaagtggt cactatgatc tctgcctcag ctgctgccag    780
gagttgcgtc aaggtcttac tactggtact gttgtcactt gtgacacagc tgttgatgta    840
cctgaaatag aaggcaagga aggtttgcaa gagggaagta gtcatagcag tgctgtaggt    900
caaggggctt ctgatcaaca gaatgacagg ttgataggta gtgcagctcc ttcagaggat    960
tgcactccta gtttgatatg gagggcaaaa agcaacggaa gcataccatg cccacctaat   1020
gcaggtggtt gcggggattg tcttctcgaa cttaggtgtt tgtttaagga gaactttatt   1080
tctgatttac tggataaagt caattcagtg gtcaataagg aaacagagca ggagttggga   1140
ggctctagat gttcctgctt cactgaatcc ggtgaagtga acaatgaaac atcacggaaa   1200
tcagcttgca gagaggactc caatgataac tacatatatt gtccaactgc tagagaagtt   1260
caaagtggag ctttagatca ttttcagcag cattggttga atggtcagcc agtcattgtt   1320
cgtgacgtgc ttgagctaac ttctggactg agctgggagc caatggttat gtggcgggcc   1380
ttacgggaaa agagagacaa gaaagagcat gaacggctct cagttatagc tcttgattgt   1440
ctgacatggt ttgagtttat gtaccaccag gatgtgttgg tggtgccagt ttcatatctg   1500
gggttcaaca gcaccattga aactacttta tatttcaaac tggttgatat aaatattcac   1560
atgtttttttg aggggtattc ccgtggagct gttggttcgg aggatttgcc tgtgctactt   1620
aagcttaaag attggccaca acatagttcc tttgaggagc gcctgccgcg gcatggtgct   1680
gagttcatgt ctgcactacc atttcgtgag tatacagatc caaaatctgg tccccttaat   1740
ctggcagtga agcttccaaa acatgttaaa aagccagatc ttggtccaaa gacatacatt   1800
gcttatggtg tcgcccaaga gttgggaata ggtgattctg ttacgaagat ccattgcgac   1860
atgtctgatg cggttaatat tctcatgcat actgatgagg tagaactcaa agctgaaagg   1920
attacagcaa tagagaaaaa gaaagagagc ttacgtaaag atggcaagaa tcttcatgtt   1980
ctgcgaccag accatgacga tgatacatca atagctctca gtgaatcaac tgaagtgcca   2040
agatctcgag gacttgaaaa tggctcaagc attaagcagc cagcaccaaa tgttgctgta   2100
atggaccagg gaggtgttca tacagatatg gtagctgatg aagctgaggg aaacttgagt   2160
ctgtctaatg ggcaatcacc caatcaaagt gatgcacata atatggatat tactttctcc   2220
aaggggggaaa cagaccattc tatctgcaca attaatgggg gagaggagat gggcaatggc   2280
tttgggaggg aagataaatg taaatcttct catggtgtag gatcatctga atcttctgat   2340
tgtcaaagac gcagtaggcg acgtgatgct tgctcttcca gtgcaacagg ggaaataaac   2400
gaaaccagca tggagactaa caaatttacc atatctattg agcccaaaga tgatcaccca   2460
tttgttgagg gaaatcagac agagggtggt gcattgtggg atatcttccg acgtgaagat   2520
gtcagcaaat tgcatgatta tctgatgaag catgcagaag agtttagaca ttataattat   2580
gaaacagtca aacaggtttc tcatcctatt cacgatcaat gcttttatct aacaaatgag   2640
cacaagagaa agctcaagga agaacatgga attgagcctt ggacatttga acagaaactt   2700
ggtgaggctg tgtttatccc agcaggatgt ccccaccaag tcagaaattt gaagtcatgt   2760

ataaaggttg cacttgactt tgtttctccg gaaaatgtgc aagagtgcat caggctgaca   2820
gaagaatttc gcttgcttcc gaagggtcat agggtgaatg aagataaact agaggttaag   2880
aagatagctc tatatgcact tgatcaagcc attgacgata tcacaggcaa aagttgcaat   2940
gaaaggacaa aggatgaagg ggaagaggag gcgtctgccc caagtgttag ttaa         2994
```

<210> 140
<211> 997
<212> PRT
<213> Oryza sativa

<400> 140

Met Pro Pro Lys Arg Lys Arg Gly Gly Gly Arg Pro Arg Lys Ala Pro
1 5 10 15
Glu Asp Ala Ala Ala Lys Glu Asn Gly Glu Lys Thr Asn Lys Glu Glu
20 25 30
Glu Thr Gln Ala Ser Pro Glu Glu Asn Gly Ala Gly Gln Thr Gln Ala
35 40 45
Ser Arg Thr Ala Arg Lys Arg Arg Lys Gly Pro Val Ala Asp Pro Ser
50 55 60
Ser Thr Glu Leu Pro Pro Arg Lys Leu Arg Asp Arg Arg Asn Val Pro
65 70 75 80
Ala Val Asp Tyr Lys Glu Asn Lys His Thr Lys Lys Met Asp Gly Thr
85 90 95
Ser Thr Met Cys His Gln Cys Gln Arg Lys Asp Ser Gly Arg Val Val
100 105 110
Arg Cys Arg Asn Gly Ala Glu Lys Asn Arg Arg His Arg Tyr Cys Val
115 120 125
Lys Cys Ile Lys Arg Trp Tyr Pro His Leu Thr Glu Asp Asp Phe Glu
130 135 140
Asn Cys Cys Pro Val Cys His Asn Asn Cys Asn Cys Lys Thr Cys Leu
145 150 155 160
Arg Thr Asn Val Ile Asn Lys Gly Asp Lys Glu Phe Ala Asp Gly Lys
165 170 175
Asn Lys Ile Lys Tyr Ser Leu Arg Ile Ala Arg Phe Leu Leu Pro Trp
180 185 190
Leu Lys Gln Leu His Gln Glu Gln Met Leu Glu Lys Ser Val Glu Ala
195 200 205
Thr Ile Lys Gly Ile Asp Val Thr Asp Leu Glu Val Pro Gln Ala Gln
210 215 220
Phe Asn Asn Asp Glu Arg Ile Tyr Cys Asp Asn Cys Arg Thr Ser Ile
225 230 235 240
Val Asp Phe His Arg Ser Cys Lys Ser Gly His Tyr Asp Leu Cys Leu
245 250 255
Ser Cys Cys Gln Glu Leu Arg Gln Gly Leu Thr Thr Gly Thr Val Val
260 265 270
Thr Cys Asp Thr Ala Val Asp Val Pro Glu Ile Glu Gly Lys Glu Gly
275 280 285
Leu Gln Glu Gly Ser Ser His Ser Ser Ala Val Gly Gln Gly Ala Ser
290 295 300
Asp Gln Gln Asn Asp Arg Leu Ile Gly Ser Ala Ala Pro Ser Glu Asp
305 310 315 320
Cys Thr Pro Ser Leu Ile Trp Arg Ala Lys Ser Asn Gly Ser Ile Pro
325 330 335
Cys Pro Pro Asn Ala Gly Gly Cys Gly Asp Cys Leu Leu Glu Leu Arg
340 345 350
Cys Leu Phe Lys Glu Asn Phe Ile Ser Asp Leu Leu Asp Lys Val Asn
355 360 365
Ser Val Val Asn Lys Glu Thr Glu Gln Glu Leu Gly Gly Ser Arg Cys
370 375 380
Ser Cys Phe Thr Glu Ser Gly Glu Val Asn Asn Glu Thr Ser Arg Lys

```
385                 390                 395                 400
Ser Ala Cys Arg Glu Asp Ser Asn Asp Asn Tyr Ile Tyr Cys Pro Thr
                405                 410                 415
Ala Arg Glu Val Gln Ser Gly Ala Leu Asp His Phe Gln Gln His Trp
            420                 425                 430
Leu Asn Gly Gln Pro Val Ile Val Arg Asp Val Leu Glu Leu Thr Ser
        435                 440                 445
Gly Leu Ser Trp Glu Pro Met Val Met Trp Arg Ala Leu Arg Glu Lys
    450                 455                 460
Arg Asp Lys Lys Glu His Glu Arg Leu Ser Val Ile Ala Leu Asp Cys
465                 470                 475                 480
Leu Thr Trp Phe Glu Phe Met Tyr His Gln Asp Val Leu Val Val Pro
                485                 490                 495
Val Ser Tyr Leu Gly Phe Asn Ser Thr Ile Glu Thr Thr Leu Tyr Phe
            500                 505                 510
Lys Leu Val Asp Ile Asn Ile His Met Phe Phe Glu Gly Tyr Ser Arg
        515                 520                 525
Gly Ala Val Gly Ser Glu Asp Leu Pro Val Leu Leu Lys Leu Lys Asp
    530                 535                 540
Trp Pro Gln His Ser Ser Phe Glu Glu Arg Leu Pro Arg His Gly Ala
545                 550                 555                 560
Glu Phe Met Ser Ala Leu Pro Phe Arg Glu Tyr Thr Asp Pro Lys Ser
                565                 570                 575
Gly Pro Leu Asn Leu Ala Val Lys Leu Pro Lys His Val Lys Lys Pro
            580                 585                 590
Asp Leu Gly Pro Lys Thr Tyr Ile Ala Tyr Gly Val Ala Gln Glu Leu
        595                 600                 605
Gly Ile Gly Asp Ser Val Thr Lys Ile His Cys Asp Met Ser Asp Ala
    610                 615                 620
Val Asn Ile Leu Met His Thr Asp Glu Val Glu Leu Lys Ala Glu Arg
625                 630                 635                 640
Ile Thr Ala Ile Glu Lys Lys Lys Glu Ser Leu Arg Lys Asp Gly Lys
                645                 650                 655
Asn Leu His Val Leu Arg Pro Asp His Asp Asp Asp Thr Ser Ile Ala
            660                 665                 670
Leu Ser Glu Ser Thr Glu Val Pro Arg Ser Arg Gly Leu Glu Asn Gly
        675                 680                 685
Ser Ser Ile Lys Gln Pro Ala Pro Asn Val Ala Val Met Asp Gln Gly
    690                 695                 700
Gly Val His Thr Asp Met Val Ala Asp Glu Ala Glu Gly Asn Leu Ser
705                 710                 715                 720
Leu Ser Asn Gly Gln Ser Pro Asn Gln Ser Asp Ala His Asn Met Asp
                725                 730                 735
Ile Thr Phe Ser Lys Gly Glu Thr Asp His Ser Ile Cys Thr Ile Asn
            740                 745                 750
Gly Gly Glu Glu Met Gly Asn Gly Phe Gly Arg Glu Asp Lys Cys Lys
        755                 760                 765
Ser Ser His Gly Val Gly Ser Ser Glu Ser Ser Asp Cys Gln Arg Arg
    770                 775                 780
Ser Arg Arg Arg Asp Ala Cys Ser Ser Ser Ala Thr Gly Glu Ile Asn
785                 790                 795                 800
Glu Thr Ser Met Glu Thr Asn Lys Phe Thr Ile Ser Ile Glu Pro Lys
                805                 810                 815
Asp Asp His Pro Phe Val Glu Gly Asn Gln Thr Glu Gly Gly Ala Leu
            820                 825                 830
Trp Asp Ile Phe Arg Arg Glu Asp Val Ser Lys Leu His Asp Tyr Leu
        835                 840                 845
Met Lys His Ala Glu Glu Phe Arg His Tyr Asn Tyr Glu Thr Val Lys
    850                 855                 860
Gln Val Ser His Pro Ile His Asp Gln Cys Phe Tyr Leu Thr Asn Glu
865                 870                 875                 880
```

```
His Lys Arg Lys Leu Lys Glu Glu His Gly Ile Glu Pro Trp Thr Phe
                885                 890                 895
Glu Gln Lys Leu Gly Glu Ala Val Phe Ile Pro Ala Gly Cys Pro His
            900                 905                 910
Gln Val Arg Asn Leu Lys Ser Cys Ile Lys Val Ala Leu Asp Phe Val
        915                 920                 925
Ser Pro Glu Asn Val Gln Glu Cys Ile Arg Leu Thr Glu Glu Phe Arg
    930                 935                 940
Leu Leu Pro Lys Gly His Arg Val Asn Glu Asp Lys Leu Glu Val Lys
945                 950                 955                 960
Lys Ile Ala Leu Tyr Ala Leu Asp Gln Ala Ile Asp Asp Ile Thr Gly
                965                 970                 975
Lys Ser Cys Asn Glu Arg Thr Lys Asp Glu Gly Glu Glu Glu Ala Ser
            980                 985                 990
Ala Pro Ser Val Ser
        995
```

<210> 141
<211> 2988
<212> DNA
<213> Oryza sativa

<400> 141

```
atggcggcgg aggaggtgga ggcggcggcg atcgacggat ccggcgagga ggcgaagcgg      60
aagagtggta agcagagggg atcgggcgcc aaggggaggc gccggaacgg cgaccgggct     120
ttccgcccgc cggcgatgag gccggaggaa gaagggcgcg gtgttgcgac gaggcccggg     180
gcgctcaggg aaaggaagcc gccgcccaac gccttcaacg cgccagacga tgatgaggat     240
gtcgagaaaa ctgatcaact tctcgagcct ctcaacaagc ccaaaagacg tgatgcaggg     300
aagaagagag gacccagaaa aaagaaagtg gatcaagaga acatcaaaac ccacaggcat     360
aacgcaaatg cagtaaaagg caagatgtta gtcaatgata aggtttcgaa gactgaaaag     420
aagcgtaaga gaggagacac aggagcggca gaaaataatg ggaaagggaa gaaaatgttg     480
acaggtgaaa acgccttaat gtgtcaccaa tgccagagaa atgacaaagg gagagtggtc     540
tggtgcaaga cttgcaacaa taagcgcttt tgtgtgccat gcattaatca atggtatcct     600
gatttgcctg aaaatgaatt tgctgcaaaa tgcccttatt gtcgcaagaa ttgtaattgc     660
aaggcatgcc tgcgaatgag aggtgtcgaa gagcctccaa gaaaggaaat ttctaaagaa     720
aatcagattc gttatgcctg tcatgtgtta cgcttgttgc gtccttggct gatagaactg     780
cgacaggagc agatggcaga gaaggaatta gaggctaaga tacaaggtgt ttcagttgat     840
caaataaagg tggaacaagc tgtgtgtgat ctagatgagc gtgtctattg caatagatgc     900
agcacttcta tagttgattt tcatagaagc tgcaagcact gtttctatga cttgtgcttg     960
acctgctgcc aggagcttcg caaaggtgag atccctggag gagaagaggt ggaaattttg    1020
gatcctgaag aaagagacaa ggattatgct tttggtaaga ttctgtcaga tggtgagaac    1080
caaagggact ctttgaagtg tcgcagcgac acccaaaaca gtgagtcaaa caaaggcatg    1140
gcttctgacg aaaaccaaaa gaaggctctg ttactttgga aagcaaacag taatggtagc    1200
ataccttgcc cacgaaagga aaaggaagac tgcagttttt catctctaga tctgaaatgc    1260
ttgtttccag agaagttgct ccctgaatta gaagatagat ctgagaaggt cttctggagt    1320
gaaacatttg caaaagaact aggtagaaca agtgagctgt gcccttgttt tgatcactca    1380
ggcaagataa gaagtgacag caagaaatta cgtcaggctg caaataggga ggactcgagt    1440
gataactact tatactgtcc ggtggccact gatatccagg acgctgacct gttgcacttt    1500
cagatgcatt gggcaaaggg cgaaccagtt gttgtttcag acactcttaa gctaacttct    1560
ggtcttagct gggagccgat ggttatgtgg cgggcagtac gagaacgaac caaagggaag    1620
gcagaagatg agcagtttgc tgtgagggca gtagattgcc tcgactggtg tgaggtggaa    1680
attaacatac acatgttttt tatgggatat acaaggggca gaactcatcc caggacctac    1740
tggcctgaga tgcttaagct aaaggactgg ccccccttcta gctcatttga tcagcgattg    1800
cctcgccatg gtgctgaatt tatcagtgca ttgccatttc cggagtatac tgatccacga    1860
tatggtccgc taaatcttgc agtgaagctt cctggtggtg tcctgaagcc agatcttggg    1920
ccaaaaactt acattgccta tggattttct gaagagttgg gcaggggtga ctctgtaacc    1980
aaacttcact gtgacatgtc tgatgcagta aacatcttaa cacacacagc ggaagtgccc    2040
tgtgagactt atgatgctgt tcagattaaa aacacgcaga aaaagatgaa aatgcaagat    2100
gacatggaaa tatatgggat gatagaatca ggcagtgaac tcaagccatc agcatgccca    2160
gttgaattgg ggaacaaagc tgttggtgaa gcaccaaagg cctcatgcag caaagaaaat    2220
gttcatacct tgaaagacaa gtctaatggt ttggatataa acgcttcacc acctgatgat    2280
```

```
gctggaggtg atgccaggga tgaagcattg tcctatgaat ctgtggtaca tagtgatgta   2340
gctcagtgtc caaaccataa ccatgagacc aataattctg atgatgcacg tattggagct   2400
cagagatgtc aaaagaaagc taaaggtcgt cctccgaaga caggttcagg ggtatcagag   2460
caccaggaaa gtggtggtgc tttgtgggat attttccgaa gagaggactc tgagaaatta   2520
caagacttcc ttaggaagca tgcgccagaa tttcggcaca tccactgcaa tccagtgaaa   2580
caggttattc atccaattca tgatcaagct ttctatttaa ctgcggagca taaaagaaag   2640
ctgaaggaag aatatggtgt tgaaccttgg acctttgaac aaaagcttgg cgaagcagtt   2700
ttgattcctg ccggatgtcc tcaccaagtc aggaatttga agtcctgcat caaggttgca   2760
ctggactttg tttcccccga gaatgttggt gagtgtgtta ggctgaccaa ggaatttaga   2820
cgacttccat cttcccacag ggctaaagaa gataagctag agatcaagaa gatggctttc   2880
catgctctga atgaagttct aaactttctg gatcctcctt cctcagaagg gtcgaaagaa   2940
gcggcggaga agcctcgaag ggggcgtggt cgcccaagaa aacactag                 2988
```

<210> 142
<211> 995
<212> PRT
<213> Oryza sativa

<400> 142

```
Met Ala Ala Glu Glu Val Glu Ala Ala Ala Ile Asp Gly Ser Gly Glu
1               5                   10                  15
Glu Ala Lys Arg Lys Ser Gly Lys Gln Arg Gly Ser Gly Ala Lys Gly
            20                  25                  30
Arg Arg Arg Asn Gly Asp Arg Ala Phe Arg Pro Pro Ala Met Arg Pro
        35                  40                  45
Glu Glu Glu Gly Arg Gly Val Ala Thr Arg Pro Gly Ala Leu Arg Glu
    50                  55                  60
Arg Lys Pro Pro Pro Asn Ala Phe Asn Ala Pro Asp Asp Asp Glu Asp
65                  70                  75                  80
Val Glu Lys Thr Asp Gln Leu Leu Glu Pro Leu Asn Lys Pro Lys Arg
                85                  90                  95
Arg Asp Ala Gly Lys Lys Arg Gly Pro Arg Lys Lys Lys Val Asp Gln
            100                 105                 110
Glu Asn Ile Lys Thr His Arg His Asn Ala Asn Ala Val Lys Gly Lys
        115                 120                 125
Met Leu Val Asn Asp Lys Val Ser Lys Thr Glu Lys Lys Arg Lys Arg
    130                 135                 140
Gly Asp Thr Gly Ala Ala Glu Asn Asn Gly Lys Gly Lys Lys Met Leu
145                 150                 155                 160
Thr Gly Glu Asn Ala Leu Met Cys His Gln Cys Gln Arg Asn Asp Lys
                165                 170                 175
Gly Arg Val Val Trp Cys Lys Thr Cys Asn Asn Lys Arg Phe Cys Val
            180                 185                 190
Pro Cys Ile Asn Gln Trp Tyr Pro Asp Leu Pro Glu Asn Glu Phe Ala
        195                 200                 205
Ala Lys Cys Pro Tyr Cys Arg Lys Asn Cys Asn Cys Lys Ala Cys Leu
    210                 215                 220
Arg Met Arg Gly Val Glu Glu Pro Pro Arg Lys Glu Ile Ser Lys Glu
225                 230                 235                 240
Asn Gln Ile Arg Tyr Ala Cys His Val Leu Arg Leu Leu Arg Pro Trp
                245                 250                 255
Leu Ile Glu Leu Arg Gln Glu Gln Met Ala Glu Lys Glu Leu Glu Ala
            260                 265                 270
Lys Ile Gln Gly Val Ser Val Asp Gln Ile Lys Val Glu Gln Ala Val
        275                 280                 285
Cys Asp Leu Asp Glu Arg Val Tyr Cys Asn Arg Cys Ser Thr Ser Ile
    290                 295                 300
Val Asp Phe His Arg Ser Cys Lys His Cys Phe Tyr Asp Leu Cys Leu
305                 310                 315                 320
Thr Cys Cys Gln Glu Leu Arg Lys Gly Glu Ile Pro Gly Gly Glu Glu
                325                 330                 335
```

Val Glu Ile Leu Asp Pro Glu Glu Arg Asp Lys Asp Tyr Ala Phe Gly
340 345 350
Lys Ile Leu Ser Asp Gly Glu Asn Gln Arg Asp Ser Leu Lys Cys Arg
355 360 365
Ser Asp Thr Gln Asn Ser Glu Ser Asn Lys Gly Met Ala Ser Asp Glu
370 375 380
Asn Gln Lys Lys Ala Leu Leu Leu Trp Lys Ala Asn Ser Asn Gly Ser
385 390 395 400
Ile Pro Cys Pro Arg Lys Glu Lys Glu Asp Cys Ser Phe Ser Ser Leu
405 410 415
Asp Leu Lys Cys Leu Phe Pro Glu Lys Leu Leu Pro Glu Leu Glu Asp
420 425 430
Arg Ser Glu Lys Val Phe Trp Ser Glu Thr Phe Ala Lys Glu Leu Gly
435 440 445
Arg Thr Ser Glu Leu Cys Pro Cys Phe Asp His Ser Gly Lys Ile Arg
450 455 460
Ser Asp Ser Lys Lys Leu Arg Gln Ala Ala Asn Arg Glu Asp Ser Ser
465 470 475 480
Asp Asn Tyr Leu Tyr Cys Pro Val Ala Thr Asp Ile Gln Asp Ala Asp
485 490 495
Leu Leu His Phe Gln Met His Trp Ala Lys Gly Glu Pro Val Val Val
500 505 510
Ser Asp Thr Leu Lys Leu Thr Ser Gly Leu Ser Trp Glu Pro Met Val
515 520 525
Met Trp Arg Ala Val Arg Glu Arg Thr Lys Gly Lys Ala Glu Asp Glu
530 535 540
Gln Phe Ala Val Arg Ala Val Asp Cys Leu Asp Trp Cys Glu Val Glu
545 550 555 560
Ile Asn Ile His Met Phe Phe Met Gly Tyr Thr Arg Gly Arg Thr His
565 570 575
Pro Arg Thr Tyr Trp Pro Glu Met Leu Lys Leu Lys Asp Trp Pro Pro
580 585 590
Ser Ser Ser Phe Asp Gln Arg Leu Pro Arg His Gly Ala Glu Phe Ile
595 600 605
Ser Ala Leu Pro Phe Pro Glu Tyr Thr Asp Pro Arg Tyr Gly Pro Leu
610 615 620
Asn Leu Ala Val Lys Leu Pro Gly Gly Val Leu Lys Pro Asp Leu Gly
625 630 635 640
Pro Lys Thr Tyr Ile Ala Tyr Gly Phe Ser Glu Glu Leu Gly Arg Gly
645 650 655
Asp Ser Val Thr Lys Leu His Cys Asp Met Ser Asp Ala Val Asn Ile
660 665 670
Leu Thr His Thr Ala Glu Val Pro Cys Glu Thr Tyr Asp Ala Val Gln
675 680 685
Ile Lys Asn Thr Gln Lys Lys Met Lys Met Gln Asp Asp Met Glu Ile
690 695 700
Tyr Gly Met Ile Glu Ser Gly Ser Glu Leu Lys Pro Ser Ala Cys Pro
705 710 715 720
Val Glu Leu Gly Asn Lys Ala Val Gly Glu Ala Pro Lys Ala Ser Cys
725 730 735
Ser Lys Glu Asn Val His Thr Leu Lys Asp Lys Ser Asn Gly Leu Asp
740 745 750
Ile Asn Ala Ser Pro Pro Asp Asp Ala Gly Gly Asp Ala Arg Asp Glu
755 760 765
Ala Leu Ser Tyr Glu Ser Val Val His Ser Asp Val Ala Gln Cys Pro
770 775 780
Asn His Asn His Glu Thr Asn Asn Ser Asp Asp Ala Arg Ile Gly Ala
785 790 795 800
Gln Arg Cys Gln Lys Lys Ala Lys Gly Arg Pro Pro Lys Thr Gly Ser
805 810 815
Gly Val Ser Glu His Gln Glu Ser Gly Gly Ala Leu Trp Asp Ile Phe

```
                    820                 825                 830
Arg Arg Glu Asp Ser Glu Lys Leu Gln Asp Phe Leu Arg Lys His Ala
        835                 840                 845
Pro Glu Phe Arg His Ile His Cys Asn Pro Val Lys Gln Val Ile His
    850                 855                 860
Pro Ile His Asp Gln Ala Phe Tyr Leu Thr Ala Glu His Lys Arg Lys
865                 870                 875                 880
Leu Lys Glu Glu Tyr Gly Val Glu Pro Trp Thr Phe Glu Gln Lys Leu
                885                 890                 895
Gly Glu Ala Val Leu Ile Pro Ala Gly Cys Pro His Gln Val Arg Asn
            900                 905                 910
Leu Lys Ser Cys Ile Lys Val Ala Leu Asp Phe Val Ser Pro Glu Asn
        915                 920                 925
Val Gly Glu Cys Val Arg Leu Thr Lys Glu Phe Arg Arg Leu Pro Ser
    930                 935                 940
Ser His Arg Ala Lys Glu Asp Lys Leu Glu Ile Lys Lys Met Ala Phe
945                 950                 955                 960
His Ala Leu Asn Glu Val Leu Asn Phe Leu Asp Pro Pro Ser Ser Glu
                965                 970                 975
Gly Ser Lys Glu Ala Ala Glu Lys Pro Arg Arg Gly Arg Gly Arg Pro
            980                 985                 990
Arg Lys His
        995
```

<210> 143
<211> 2607
<212> DNA
<213> Oryza sativa

<400> 143

```
atgttagtca atgataaggt ttcgaagact gaaaagaagc gtaagagagg agacacagga     60
gcggcagaaa ataatgggaa agggaagaaa atgttgacag gtgaaaacgc cttaatgtgt    120
caccaatgcc agagaaatga caaagggaga gtggtctggt gcaagacttg caacaataag    180
cgcttttgtg tgccatgcat taatcaatgg tatcctgatt tgcctgaaaa tgaatttgct    240
gcaaaatgcc cttattgtcg caagaattgt aattgcaagg catgcctgcg aatgagaggt    300
gtcgaagagc agcctccaag aaaggaaatt tctaaagaaa atcagattcg ttatgcctgt    360
catgtgttac gcttgttgcg tccttggctg atagaactgc gacaggagca gatggcagag    420
aaggaattag aggctaagat acaaggtgtt tcagttgatc aaataaaggt ggaacaagct    480
gtgtgtgatc tagatgagcg tgtctattgc aatagatgca gcacttctat agttgatttt    540
catagaagct gcaagcactg tttctatgac ttgtgcttga cctgctgcca ggagcttcgc    600
aaaggtgaga tccctggagg agaagaggtg gaaattttgg atcctgaaga aagagacaag    660
gattatgctt ttggtaagat tctgtcagat ggtgagaacc aaagggactc tttgaagtgt    720
cgcagcgaca cccaaaacag tgagtcaaac aaaggcatgg cttctgacga aaaccaaaag    780
aaggctctgt tactttggaa agcaaacagt aatggtagca taccttgccc acgaaaggaa    840
aaggaagact gcagtttttc atctctagat ctgaaatgct tgtttccaga gaagttgctc    900
cctgaattag aagatagatc tgagaaggtc ttctggagtg aaacatttgc aaaagaacta    960
ggtagaacaa gtgagctgtg cccttgtttt gatcactcag gcaagataag aagtgacagc   1020
aagaaattac gtcaggctgc aaatagggag gactcgagtg ataactactt atactgtccg   1080
gtggccactg atatccagga cgctgacctg ttgcactttc agatgcattg ggcaaagggc   1140
gaaccagttg ttgtttcaga cactcttaag ctaacttctg gtcttagctg ggagccgatg   1200
gttatgtggc gggcagtacg agaacgaacc aaagggaagg cagaagatga gcagtttgct   1260
gtgagggcag tagattgcct cgactggtgt gaggtggaaa ttaacataca catgtttttt   1320
atgggatata caagggggcag aactcatccc aggacctact ggcctgagat gcttaagcta   1380
aaggactggc ccccttctag ctcatttgat cagcgattgc ctcgccatgg tgctgaattt   1440
atcagtgcat tgccatttcc ggagtatact gatccacgat atggtccgct aaatcttgca   1500
gtgaagcttc ctggtggtgt cctgaagcca gatcttgggc caaaaactta cattgcctat   1560
ggattttctg aagagttggg caggggtgac tctgtaacca aacttcactg tgacatgtct   1620
gatgcagtaa acatcttaac acacacagcg gaagtgccct gtgagactta tgatgctgtt   1680
cagattaaaa acacgcagaa aaagatgaaa atgcaagatg acatggaaat atatgggatg   1740
atagaatcag gcagtgaact caagccatca gcatgcccag ttgaattggg gaacaaagct   1800
gttggtgaag caccaaaggc ctcatgcagc aaagaaaatg ttcatacctt gaaagacaag   1860

tctaatggtt tggatataaa cgcttcacca cctgatgatg ctggaggtga tgccagggat   1920
gaagcattgt cctatgaatc tgtggtacat agtgatgtag ctcagtgtcc aaaccataac   1980
catgagacca ataattctga tgatgcacgt attggagctc agagatgtca aaagaaagct   2040
aaaggtcgtc ctccgaagac aggttcaggg gtatcagagc accaggaaag tggtggtgct   2100
ttgtgggata ttttccgaag agaggactct gagaaattac aagacttcct taggaagcat   2160
gcgccagaat ttcggcacat ccactgcaat ccagtgaaac aggttattca tccaattcat   2220
gatcaagctt tctatttaac tgcggagcat aaaagaaagc tgaaggaaga atatggtgtt   2280
gaaccttgga cctttgaaca aaagcttggc gaagcagttt tgattcctgc cggatgtcct   2340
caccaagtca ggaatttgaa gtcctgcatc aaggttgcac tggactttgt ttcccccgag   2400
aatgttggtg agtgtgttag gctgaccaag gaatttagac gacttccatc ttcccacagg   2460
gctaaagaag ataagctaga gatcaagaag atggctttcc atgctctgaa tgaagttcta   2520
aactttctgg atcctccttc ctcagaaggg tcgaaagaag cggcggagaa gcctcgaagg   2580
gggcgtggtc gcccaagaaa acactag                                        2607
```

<210> 144
<211> 868
<212> PRT
<213> Oryza sativa

<400> 144

```
Met Leu Val Asn Asp Lys Val Ser Lys Thr Glu Lys Lys Arg Lys Arg
1               5                   10                  15
Gly Asp Thr Gly Ala Ala Glu Asn Asn Gly Lys Gly Lys Lys Met Leu
            20                  25                  30
Thr Gly Glu Asn Ala Leu Met Cys His Gln Cys Gln Arg Asn Asp Lys
        35                  40                  45
Gly Arg Val Val Trp Cys Lys Thr Cys Asn Asn Lys Arg Phe Cys Val
    50                  55                  60
Pro Cys Ile Asn Gln Trp Tyr Pro Asp Leu Pro Glu Asn Glu Phe Ala
65                  70                  75                  80
Ala Lys Cys Pro Tyr Cys Arg Lys Asn Cys Asn Cys Lys Ala Cys Leu
                85                  90                  95
Arg Met Arg Gly Val Glu Glu Gln Pro Pro Arg Lys Glu Ile Ser Lys
            100                 105                 110
Glu Asn Gln Ile Arg Tyr Ala Cys His Val Leu Arg Leu Leu Arg Pro
        115                 120                 125
Trp Leu Ile Glu Leu Arg Gln Glu Gln Met Ala Glu Lys Glu Leu Glu
    130                 135                 140
Ala Lys Ile Gln Gly Val Ser Val Asp Gln Ile Lys Val Glu Gln Ala
145                 150                 155                 160
Val Cys Asp Leu Asp Glu Arg Val Tyr Cys Asn Arg Cys Ser Thr Ser
                165                 170                 175
Ile Val Asp Phe His Arg Ser Cys Lys His Cys Phe Tyr Asp Leu Cys
            180                 185                 190
Leu Thr Cys Cys Gln Glu Leu Arg Lys Gly Glu Ile Pro Gly Gly Glu
        195                 200                 205
Glu Val Glu Ile Leu Asp Pro Glu Glu Arg Asp Lys Asp Tyr Ala Phe
    210                 215                 220
Gly Lys Ile Leu Ser Asp Gly Glu Asn Gln Arg Asp Ser Leu Lys Cys
225                 230                 235                 240
Arg Ser Asp Thr Gln Asn Ser Glu Ser Asn Lys Gly Met Ala Ser Asp
                245                 250                 255
Glu Asn Gln Lys Lys Ala Leu Leu Leu Trp Lys Ala Asn Ser Asn Gly
            260                 265                 270
Ser Ile Pro Cys Pro Arg Lys Glu Lys Glu Asp Cys Ser Phe Ser Ser
        275                 280                 285
Leu Asp Leu Lys Cys Leu Phe Pro Glu Lys Leu Leu Pro Glu Leu Glu
    290                 295                 300
Asp Arg Ser Glu Lys Val Phe Trp Ser Glu Thr Phe Ala Lys Glu Leu
305                 310                 315                 320
Gly Arg Thr Ser Glu Leu Cys Pro Cys Phe Asp His Ser Gly Lys Ile
```

```
                325                 330                 335
Arg Ser Asp Ser Lys Lys Leu Arg Gln Ala Ala Asn Arg Glu Asp Ser
            340                 345                 350
Ser Asp Asn Tyr Leu Tyr Cys Pro Val Ala Thr Asp Ile Gln Asp Ala
        355                 360                 365
Asp Leu Leu His Phe Gln Met His Trp Ala Lys Gly Glu Pro Val Val
    370                 375                 380
Val Ser Asp Thr Leu Lys Leu Thr Ser Gly Leu Ser Trp Glu Pro Met
385                 390                 395                 400
Val Met Trp Arg Ala Val Arg Glu Arg Thr Lys Gly Lys Ala Glu Asp
                405                 410                 415
Glu Gln Phe Ala Val Arg Ala Val Asp Cys Leu Asp Trp Cys Glu Val
            420                 425                 430
Glu Ile Asn Ile His Met Phe Phe Met Gly Tyr Thr Arg Gly Arg Thr
        435                 440                 445
His Pro Arg Thr Tyr Trp Pro Glu Met Leu Lys Leu Lys Asp Trp Pro
    450                 455                 460
Pro Ser Ser Ser Phe Asp Gln Arg Leu Pro Arg His Gly Ala Glu Phe
465                 470                 475                 480
Ile Ser Ala Leu Pro Phe Pro Glu Tyr Thr Asp Pro Arg Tyr Gly Pro
                485                 490                 495
Leu Asn Leu Ala Val Lys Leu Pro Gly Gly Val Leu Lys Pro Asp Leu
            500                 505                 510
Gly Pro Lys Thr Tyr Ile Ala Tyr Gly Phe Ser Glu Glu Leu Gly Arg
        515                 520                 525
Gly Asp Ser Val Thr Lys Leu His Cys Asp Met Ser Asp Ala Val Asn
    530                 535                 540
Ile Leu Thr His Thr Ala Glu Val Pro Cys Glu Thr Tyr Asp Ala Val
545                 550                 555                 560
Gln Ile Lys Asn Thr Gln Lys Lys Met Lys Met Gln Asp Asp Met Glu
                565                 570                 575
Ile Tyr Gly Met Ile Glu Ser Gly Ser Glu Leu Lys Pro Ser Ala Cys
            580                 585                 590
Pro Val Glu Leu Gly Asn Lys Ala Val Gly Glu Ala Pro Lys Ala Ser
        595                 600                 605
Cys Ser Lys Glu Asn Val His Thr Leu Lys Asp Lys Ser Asn Gly Leu
    610                 615                 620
Asp Ile Asn Ala Ser Pro Pro Asp Asp Ala Gly Gly Asp Ala Arg Asp
625                 630                 635                 640
Glu Ala Leu Ser Tyr Glu Ser Val Val His Ser Asp Val Ala Gln Cys
                645                 650                 655
Pro Asn His Asn His Glu Thr Asn Asn Ser Asp Asp Ala Arg Ile Gly
            660                 665                 670
Ala Gln Arg Cys Gln Lys Lys Ala Lys Gly Arg Pro Pro Lys Thr Gly
        675                 680                 685
Ser Gly Val Ser Glu His Gln Glu Ser Gly Gly Ala Leu Trp Asp Ile
    690                 695                 700
Phe Arg Arg Glu Asp Ser Glu Lys Leu Gln Asp Phe Leu Arg Lys His
705                 710                 715                 720
Ala Pro Glu Phe Arg His Ile His Cys Asn Pro Val Lys Gln Val Ile
                725                 730                 735
His Pro Ile His Asp Gln Ala Phe Tyr Leu Thr Ala Glu His Lys Arg
            740                 745                 750
Lys Leu Lys Glu Glu Tyr Gly Val Glu Pro Trp Thr Phe Glu Gln Lys
        755                 760                 765
Leu Gly Glu Ala Val Leu Ile Pro Ala Gly Cys Pro His Gln Val Arg
    770                 775                 780
Asn Leu Lys Ser Cys Ile Lys Val Ala Leu Asp Phe Val Ser Pro Glu
785                 790                 795                 800
Asn Val Gly Glu Cys Val Arg Leu Thr Lys Glu Phe Arg Arg Leu Pro
                805                 810                 815
```

```
Ser Ser His Arg Ala Lys Glu Asp Lys Leu Glu Ile Lys Lys Met Ala
            820                 825                 830
Phe His Ala Leu Asn Glu Val Leu Asn Phe Leu Asp Pro Pro Ser Ser
        835                 840                 845

Glu Gly Ser Lys Glu Ala Ala Glu Lys Pro Arg Arg Gly Arg Gly Arg
    850                 855                 860
Pro Arg Lys His
865
```

<210> 145
<211> 1062
<212> DNA
<213> Oryza sativa

<400> 145

```
atgccgcccc agcctccgcc ggcggcgtcg gcgtcggcca gtgccccgga tcccgccgtc     60
ccggcttggc tcaggggggct cccccgcgcg cccgagtacc gcccgacgga gtccgagttc    120
gccgacccca tcgccttcct ctcccgcgtg gagcgcgagg ccgccgccta cggcatctgc    180
aaggtcatcc cgccccaccc gcgcccttcc cgccgcttcg tcttcgccca cctcaaccgc    240
tccctcgtct cctcctgcga cgcccccgcg ccctctcccg ccgccgcctc cgactcgtcc    300
attcctccat cctcctcctc gccgccgccg gtctccgccg cggtgttcac gacacggcac    360
caggagctcg gcaacccgcg gcgtggacgc ccgacgccgc aggtgcttaa gcaggtgtgg    420
cagagtgggg agcggtacac gctcgaccag tttgagtcca agtcccgcgc cttctcaaag    480
acgcacctcg ctggactcca cgaaccgact gcgctcgcgg tggaatcact cttctggaag    540
gcgtcggcgg accgtcctat ctatattgag tacgccaatg atgtccctgg ctctgggttt    600
gctgcacccg tgcagttgca acgcaagaag aagcagaaaa gagagactgc cccgatggac    660
gaatgggaga agagttcagg ctggaggctg tcaaatagcc catggaacct gcaagcaatt    720
gcgcgggctc ctggttcgct cacacggttt atgcctgacg atgttcctgg ggtgacttct    780
ccaatggttt acattggcat gctgttcagc tggtttgcgt ggcatgtgga ggatcatgat    840
ctgcatagtc tcaacttcct ccacactggc gcacctaaga cgtggtatgc ggttcctggt    900
gatagggctg ttgagcttga ggaagttatc cgtgtacatg gctatggagg caacactgat    960
cggatcgcgt cactagcagt gcttggtgag aaaacaacac taatgtcccc agaggtcctt   1020
atagacaatg attggtgcaa tatcctggtg agtttgtggt ga                      1062
```

<210> 146
<211> 353
<212> PRT
<213> Oryza sativa

<400> 146

```
Met Pro Pro Gln Pro Pro Pro Ala Ala Ser Ala Ser Ala Ser Ala Pro
1               5                   10                  15
Asp Pro Ala Val Pro Ala Trp Leu Arg Gly Leu Pro Arg Ala Pro Glu
            20                  25                  30
Tyr Arg Pro Thr Glu Ser Glu Phe Ala Asp Pro Ile Ala Phe Leu Ser
        35                  40                  45
Arg Val Glu Arg Glu Ala Ala Ala Tyr Gly Ile Cys Lys Val Ile Pro
    50                  55                  60
Pro His Pro Arg Pro Ser Arg Arg Phe Val Phe Ala His Leu Asn Arg
65                  70                  75                  80
Ser Leu Val Ser Ser Cys Asp Ala Pro Ala Pro Ser Pro Ala Ala Ala
                85                  90                  95
Ser Asp Ser Ser Ile Pro Pro Ser Ser Ser Ser Pro Pro Pro Val Ser
            100                 105                 110
Ala Ala Val Phe Thr Thr Arg His Gln Glu Leu Gly Asn Pro Arg Arg
        115                 120                 125
Gly Arg Pro Thr Pro Gln Val Leu Lys Gln Val Trp Gln Ser Gly Glu
    130                 135                 140
Arg Tyr Thr Leu Asp Gln Phe Glu Ser Lys Ser Arg Ala Phe Ser Lys
145                 150                 155                 160

Thr His Leu Ala Gly Leu His Glu Pro Thr Ala Leu Ala Val Glu Ser
                165                 170                 175
Leu Phe Trp Lys Ala Ser Ala Asp Arg Pro Ile Tyr Ile Glu Tyr Ala
            180                 185                 190
Asn Asp Val Pro Gly Ser Gly Phe Ala Ala Pro Val Gln Leu Gln Arg
        195                 200                 205
Lys Lys Lys Gln Lys Arg Glu Thr Ala Pro Met Asp Glu Trp Glu Lys
    210                 215                 220
Ser Ser Gly Trp Arg Leu Ser Asn Ser Pro Trp Asn Leu Gln Ala Ile
225                 230                 235                 240
Ala Arg Ala Pro Gly Ser Leu Thr Arg Phe Met Pro Asp Asp Val Pro
                245                 250                 255
Gly Val Thr Ser Pro Met Val Tyr Ile Gly Met Leu Phe Ser Trp Phe
            260                 265                 270
Ala Trp His Val Glu Asp His Asp Leu His Ser Leu Asn Phe Leu His
        275                 280                 285
Thr Gly Ala Pro Lys Thr Trp Tyr Ala Val Pro Gly Asp Arg Ala Val
    290                 295                 300
Glu Leu Glu Glu Val Ile Arg Val His Gly Tyr Gly Gly Asn Thr Asp
305                 310                 315                 320
Arg Ile Ala Ser Leu Ala Val Leu Gly Glu Lys Thr Thr Leu Met Ser
                325                 330                 335
Pro Glu Val Leu Ile Asp Asn Asp Trp Cys Asn Ile Leu Val Ser Leu
            340                 345                 350
Trp
```

<210> 147
<211> 2784
<212> DNA
<213> Oryza sativa

<400> 147

```
atggaggcgg cggcggcggc ggccgccgtg ccggaggagc tgcggtgcaa gcgctccgac   60
ggcaagcagt ggcgatgcag cgcgccctcc atgcccgaca agaccgtctg cgagaagcac  120
tacgtccagg ccaagaagcg cgccgcctcc tccgcgctcc gggcctccct ccgcaggtcc  180
tccgcctccg cctccgccgc gcggggcacg accccgcctg cgcggatggc ggtcgcgagg  240
cccatctacg gcagggtcgc gggggagccg gtgtacgtgg cggagccggc gctgccgccg  300
ccgccgccgc cgccacggag gaggcagccg gtccacgggc tgcccatggg caatgctgcc  360
ggcgcgcgca ccgcggcgga gctggtcggg aggggctcgg cggggctggt tgcctgcagc  420
tcggcggcgg gggcggcagc ggccgcaacc tgccatcagt gccggagggt cgctaatacc  480
atttgttgta caagctgcga cagaaggggg tactgcacca actgcatctc gagatggtac  540
tctgatatcc caattgatga tgttcgaaag gtttgtccag catgtcgtgg catttgtaac  600
tgcagagttt gcttactagg agataatgta ataaaggcaa gggttcagga gatatctgct  660
gtagataagt tggaatatct tcatagcatt ctggcatctg tccttccagt attaaagcag  720
atttattctg atcaatgttt cgaaattggt gttgatacaa aagcttatgg acttaggaca  780
gatataatca gggcgaaggt caatcctgat gagcaaatgt gctgtgactt ctgcaaagtg  840
ccagtatttg attatcaccg acactgccca aggtgtttgt atgacttatg tcttgactgt  900
tgtcgagaca taaggcgatc taggaccagt gttgcaagag gagaatatgc tgaaggtcgt  960
gtggtagata gaagtaaaga tacttcgaat aaaagagcaa gaatggaacc atctgcagaa 1020
agtgcaaatg ataagtcagt cccacaacga agggacataa aaaatattga cattagatct 1080
ttattcccta catggagagt caataatgat ggaagcatta cttgtgggcc tcatgaggct 1140
ggtggttgtg gctcctcgaa gttagtgtta aggcgaatat tcaaaataaa ctggattagt 1200
aagcttgtaa aaaattctga ggaaatggtc aatggttgta aagtacatgt tcttgagaat 1260
ggatgctcat cctgcaatga tggcagaaca ttagagttaa ctggtcatcg taactttggt 1320
gtctcaacat gttcaaataa tggtggtatt gatcggttct gtgtgttctc tcctgtatta 1380
gaggacttga aatctgaagg tattattcat tttcgtaaac actggataaa aggagaacct 1440
gtagttatca ggaatgcgtt tgagccttct ctgtcatcaa gctgggaccc actaaatatt 1500
tggcgaggaa tccaggaaat catggatgaa gaagtggatg acgatgtcat tgtcaaagct 1560
gtggattgct caaaccaagc agaggtggat attgagctga aacagtttat caaaggttat 1620
tcagatggtc acaaggggga agatggggaa ttgatgatgc tgaaattgaa agagtggccc 1680

ccacccagtg tattggagga gtttctgcta tgccaaagac cagaatttat tgtcaatttt 1740
ccattagttg attttataca ttccaggtgg gggctcctaa atctttctgc taaattgccc 1800
ccagacaccc tacaacctga agttggcttg aagctgttaa ttgcatatgg aagacatcaa 1860
gaagctggta aaggtgattc agtgacaaat ctaatgatta acatggctga tgtggtgcat 1920
atgctaatgc atacagccaa agggcatgac gtatgtccaa agaggctaca acctgagcga 1980
tctgaaaaga ttgccaatgg aatgacaatg catgtaaatg ctcatgcacc tgttcaaaat 2040
ttgaatgtgg atatggggga acaatcacct gaccatgtaa gctcaaagtt tgatgaaaga 2100
gcgcatgcgt ctgccttgcg attacaagag aagtcttcag atgccaaact taattgtggt 2160
ttcgaaggct cttcaactga gttatcttgc tcgtcacatt cagaggaacc aaaagttaat 2220
ggttcagaaa gaagtcaggc tggttctgtt tgggatgtgt tccgcaggca ggatatttca 2280
aagctgaatg aatatttaac tgctaactgg gaagaactgg cagctagtag tcaggttaag 2340
aatcctattt atgaacaatc tatatatctc aacaagtatc ataaaaggat actgaaggat 2400
caatatggaa ttgaaccctg gacattccaa caacatatcg gtgaggctgt atttgttcct 2460
gctggctgcc cattccaagt gaaaaatctc cagtctacgg ttcaattggc tcttgatttc 2520
ctgtcaccgg aaagtttggg ggagtcggcc cgaatggccc aggagattcg ctgcctacca 2580
aatgatcatg atgcaaaact gaagatgctc gagattggaa aaatctcttt atatgcagct 2640
agttctgctg tcagagaaat tcagagaata accctcgatc ccaagtttaa tctagacctt 2700
aaattcgagg atcagaatct aactcaggcg gtttctgaga acttggctag agtcaccaaa 2760
caacggaatg taccatgcag ctga                                        2784
```

<210> 148
<211> 927
<212> PRT
<213> Oryza sativa

<400> 148

```
Met Glu Ala Ala Ala Ala Ala Ala Ala Val Pro Glu Glu Leu Arg Cys
1               5                   10                  15
Lys Arg Ser Asp Gly Lys Gln Trp Arg Cys Ser Ala Pro Ser Met Pro
            20                  25                  30
Asp Lys Thr Val Cys Glu Lys His Tyr Val Gln Ala Lys Lys Arg Ala
        35                  40                  45
Ala Ser Ser Ala Leu Arg Ala Ser Leu Arg Arg Ser Ser Ala Ser Ala
    50                  55                  60
Ser Ala Ala Arg Gly Thr Thr Pro Pro Ala Arg Met Ala Val Ala Arg
65                  70                  75                  80
Pro Ile Tyr Gly Arg Val Ala Gly Glu Pro Val Tyr Val Ala Glu Pro
                85                  90                  95
Ala Leu Pro Pro Pro Pro Pro Pro Pro Arg Arg Arg Gln Pro Val His
            100                 105                 110
Gly Leu Pro Met Gly Asn Ala Ala Gly Ala Arg Thr Ala Ala Glu Leu
        115                 120                 125
Val Gly Arg Gly Ser Ala Gly Leu Val Ala Cys Ser Ser Ala Ala Gly
    130                 135                 140
Ala Ala Ala Ala Ala Thr Cys His Gln Cys Arg Arg Val Ala Asn Thr
145                 150                 155                 160
Ile Cys Cys Thr Ser Cys Asp Arg Arg Gly Tyr Cys Thr Asn Cys Ile
                165                 170                 175
Ser Arg Trp Tyr Ser Asp Ile Pro Ile Asp Asp Val Arg Lys Val Cys
            180                 185                 190
Pro Ala Cys Arg Gly Ile Cys Asn Cys Arg Val Cys Leu Leu Gly Asp
        195                 200                 205
Asn Val Ile Lys Ala Arg Val Gln Glu Ile Ser Ala Val Asp Lys Leu
    210                 215                 220
Glu Tyr Leu His Ser Ile Leu Ala Ser Val Leu Pro Val Leu Lys Gln
225                 230                 235                 240
Ile Tyr Ser Asp Gln Cys Phe Glu Ile Gly Val Asp Thr Lys Ala Tyr
                245                 250                 255
Gly Leu Arg Thr Asp Ile Ile Arg Ala Lys Val Asn Pro Asp Glu Gln
            260                 265                 270
Met Cys Cys Asp Phe Cys Lys Val Pro Val Phe Asp Tyr His Arg His
```

```
            275                 280                 285
Cys Pro Arg Cys Leu Tyr Asp Leu Cys Leu Asp Cys Cys Arg Asp Ile
    290                 295                 300
Arg Arg Ser Arg Thr Ser Val Ala Arg Gly Glu Tyr Ala Glu Gly Arg
305                 310                 315                 320
Val Val Asp Arg Ser Lys Asp Thr Ser Asn Lys Arg Ala Arg Met Glu
                325                 330                 335
Pro Ser Ala Glu Ser Ala Asn Asp Lys Ser Val Pro Gln Arg Arg Asp
            340                 345                 350
Ile Lys Asn Ile Asp Ile Arg Ser Leu Phe Pro Thr Trp Arg Val Asn
        355                 360                 365
Asn Asp Gly Ser Ile Thr Cys Gly Pro His Glu Ala Gly Gly Cys Gly
    370                 375                 380
Ser Ser Lys Leu Val Leu Arg Arg Ile Phe Lys Ile Asn Trp Ile Ser
385                 390                 395                 400
Lys Leu Val Lys Asn Ser Glu Glu Met Val Asn Gly Cys Lys Val His
                405                 410                 415
Val Leu Glu Asn Gly Cys Ser Ser Cys Asn Asp Gly Arg Thr Leu Glu
            420                 425                 430
Leu Thr Gly His Arg Asn Phe Gly Val Ser Thr Cys Ser Asn Asn Gly
        435                 440                 445
Gly Ile Asp Arg Phe Cys Val Phe Ser Pro Val Leu Glu Asp Leu Lys
    450                 455                 460
Ser Glu Gly Ile Ile His Phe Arg Lys His Trp Ile Lys Gly Glu Pro
465                 470                 475                 480
Val Val Ile Arg Asn Ala Phe Glu Pro Ser Leu Ser Ser Ser Trp Asp
                485                 490                 495
Pro Leu Asn Ile Trp Arg Gly Ile Gln Glu Ile Met Asp Glu Glu Val
            500                 505                 510
Asp Asp Asp Val Ile Val Lys Ala Val Asp Cys Ser Asn Gln Ala Glu
        515                 520                 525

Val Asp Ile Glu Leu Lys Gln Phe Ile Lys Gly Tyr Ser Asp Gly His
    530                 535                 540
Lys Gly Glu Asp Gly Glu Leu Met Met Leu Lys Leu Lys Glu Trp Pro
545                 550                 555                 560
Pro Pro Ser Val Leu Glu Glu Phe Leu Leu Cys Gln Arg Pro Glu Phe
                565                 570                 575
Ile Val Asn Phe Pro Leu Val Asp Phe Ile His Ser Arg Trp Gly Leu
            580                 585                 590
Leu Asn Leu Ser Ala Lys Leu Pro Pro Asp Thr Leu Gln Pro Glu Val
        595                 600                 605
Gly Leu Lys Leu Leu Ile Ala Tyr Gly Arg His Gln Glu Ala Gly Lys
    610                 615                 620
Gly Asp Ser Val Thr Asn Leu Met Ile Asn Met Ala Asp Val Val His
625                 630                 635                 640
Met Leu Met His Thr Ala Lys Gly His Asp Val Cys Pro Lys Arg Leu
                645                 650                 655
Gln Pro Glu Arg Ser Glu Lys Ile Ala Asn Gly Met Thr Met His Val
            660                 665                 670
Asn Ala His Ala Pro Val Gln Asn Leu Asn Val Asp Met Gly Glu Gln
        675                 680                 685
Ser Pro Asp His Val Ser Ser Lys Phe Asp Glu Arg Ala His Ala Ser
    690                 695                 700
Ala Leu Arg Leu Gln Glu Lys Ser Ser Asp Ala Lys Leu Asn Cys Gly
705                 710                 715                 720
Phe Glu Gly Ser Ser Thr Glu Leu Ser Cys Ser Ser His Ser Glu Glu
                725                 730                 735
Pro Lys Val Asn Gly Ser Glu Arg Ser Gln Ala Gly Ser Val Trp Asp
            740                 745                 750
Val Phe Arg Arg Gln Asp Ile Ser Lys Leu Asn Glu Tyr Leu Thr Ala
```

```
        755                 760                 765
Asn Trp Glu Glu Leu Ala Ala Ser Ser Gln Val Lys Asn Pro Ile Tyr
    770                 775                 780
Glu Gln Ser Ile Tyr Leu Asn Lys Tyr His Lys Arg Ile Leu Lys Asp
785                 790                 795                 800
Gln Tyr Gly Ile Glu Pro Trp Thr Phe Gln Gln His Ile Gly Glu Ala
                805                 810                 815
Val Phe Val Pro Ala Gly Cys Pro Phe Gln Val Lys Asn Leu Gln Ser
            820                 825                 830
Thr Val Gln Leu Ala Leu Asp Phe Leu Ser Pro Glu Ser Leu Gly Glu
        835                 840                 845
Ser Ala Arg Met Ala Gln Glu Ile Arg Cys Leu Pro Asn Asp His Asp
    850                 855                 860
Ala Lys Leu Lys Met Leu Glu Ile Gly Lys Ile Ser Leu Tyr Ala Ala
865                 870                 875                 880
Ser Ser Ala Val Arg Glu Ile Gln Arg Ile Thr Leu Asp Pro Lys Phe
                885                 890                 895
Asn Leu Asp Leu Lys Phe Glu Asp Gln Asn Leu Thr Gln Ala Val Ser
            900                 905                 910
Glu Asn Leu Ala Arg Val Thr Lys Gln Arg Asn Val Pro Cys Ser
        915                 920                 925
```

<210> 149
<211> 2862
<212> DNA
<213> Oryza sativa

<400> 149

```
atggcggcgg ggaacggccg gatggaggcg gcgctgggct gcctcgcggc gctccccgac     60
gaggtgctct gcgccgtcgt cgacctcctc ccgcccaccg acgtcggccg cctcgcctgc    120
gtcagcagtg tcatgtacat actttgcaat gaggagcctc tctggatgag caagtgtctt    180
tcagttgggg gtcttcttgt gtatagaggt tcttggaaga aaacagcatt gtctagactt    240
aatctttgtt cagaaaatga tgagatttac cagaagcctc gccattttga tgggttcaat    300
tccatgcact tatacaggag atggtacaga tgttttacta atttgagtag cttttccttt    360
gataatgggc acgttgaaag gaaagatgac ctttctctag accaatttcg cgctcagtat    420
gatggaaaat gtccagtttt gcttactaaa ctggctgaaa cctggccagc aaggactaaa    480
tggacagcgc agcaactgac acatgattat ggtgaagttc cctttaggat atctcagaga    540
agccctcaaa agataaaaat gaaactaaaa gattatgttt tttacatgga actccagcat    600
gacgaagatc cactttacat atttgatgat aagtttggag aatcagcacc tacactattg    660
gaagattaca gtgtccctca tctatttcaa gaagatttct ttgaaatcat ggattacgac    720
caacgaccag ctttcagatg gcttattatt ggaccagaga gatcaggtgc ttcttggcat    780
gttgatccag ggttgaccag tgcctggaat actcttcttt gtggccgaaa aaggtgggca    840
atgtaccctc ctggaagagt accaggtggt gtcacagtac atgtcagtga tgaagatggt    900
gatgttgaca ttgaaactcc tacatctttg cagtggtggc tagatatcta cccaaatctt    960
gctgagcatg agaaaccact ggaatgcaca caattaccag gagagaccat atttgttcct   1020
agtgggtggt ggcattgtgt tttgaacctt gacatgacaa ttgctgtcac gcaaaatttt   1080
gtcaaccaat caaattttaa gcatgtatgt ttggacatgg cacctggtta ctgtcacaaa   1140
ggagtttgcc gtgctggctt acttgctgct ccagacaaat ctattagaga tattgaaaat   1200
cttcctagta taacgagtag attgaaccac tctgacatgg cctgtaagga aaaaagactg   1260
aaaagttcag agcctataag aacttcaaat aatgcaaatc agtgttctgc atttgagttc   1320
tcagatgttc atgaaaactt gggggaccaa gtttttcgt atgatataga tttcttatcc   1380
caattccttg agaaagaaaa ggatcactat tcttctgtct ggagccctac taattcaatt   1440
ggccagagag aagcaagaga atggctacgt aggctatggg ttcttaaacc tgaattgaga   1500
gaactaatat ggaagggtgc atgtctagca attaatgtag acaagtggta ttcatgctta   1560
gaggaaataa gtgcatgcca tagtttacca ccaccttctg aagatgagaa gcttcctgtt   1620
ggcacaggta gcaacccagt cttcattgtt tctggcaatg tgatcaaaat ttatgctgaa   1680
ggagggttgg gttattctat acatggtttg ggcacagagc ttgagttcta tgatcttctg   1740
caaaaacttg gctcgccatt gatcaaccat gtccctgaga tcattgcaag tggctttctt   1800
gtgtacctgg atggtgtcta caagacagtt ccatgggatg gaaacggaat accagatgtt   1860
ctagctaaat actactcttt ggaggtgtct tatgcaaacg gctcttttcc tcttggatta   1920
tggagcaagc aactgtttgg attgagtaat tcaactgatg ctccagacag accaatttgt   1980

ccttacatgg ttaccagaaa atgcaaaggg gatatttttg ctcgcatacg tgataaattg   2040
accaagactg atgttttgaa tcttgcatca tccttgggag ttcaaatgcg aaatattcat   2100
caattacccc ttccacatgt ggaacacata tccaaatctg ggaacgaaga tatcaaagca   2160
aaggaaaatt caatttctga tgtcactcat gttccgcctg aatggaaaca agtagtttct   2220
actctagaca ggagaaagaa aagtataaag aagcatctaa gtaactgggg tggttcaatt   2280
ccacaggttc taattgagaa ggctgaagaa tatctccctg acgacatccg ctttcttatc   2340
aagtttgtta aggacgatga tggtgattca gtctatgtgg taccttcttg gatacattca   2400
gatataatgg atgataacat tctcattgag gggaccacag aaccaggaac ttccactgat   2460

tgcattgccg ttgaagatct gaacaaaatg gatgcaattc atatcattga tttcagtgat   2520
ctgtccattg gggatcctct atgtgactta attccactgc acttggatgt attccgtggt   2580
gatattgatc ttctcaggca gtttttacga agctatcagc ttccttttct gagagcagaa   2640
tcaaataaag atatatacaa gtcaatacaa aattctaaat tcagcagggc atcgtatcgt   2700
gcgatgtgct actgcatact tcacgaggac aacgtcctgg gagccatatt tagcctgtgg   2760
aaggatctgg gcaccgcgac gtcatgggaa gatgttgaac acttggtttg gggagagctg   2820
aatcaatacc agcagtcatg cagcgtgggc gaaattaact ga                       2862
```

<210> 150
<211> 953
<212> PRT
<213> Oryza sativa

<400> 150

```
Met Ala Ala Gly Asn Gly Arg Met Glu Ala Ala Leu Gly Cys Leu Ala
1               5                   10                  15
Ala Leu Pro Asp Glu Val Leu Cys Ala Val Val Asp Leu Leu Pro Pro
            20                  25                  30
Thr Asp Val Gly Arg Leu Ala Cys Val Ser Ser Val Met Tyr Ile Leu
        35                  40                  45
Cys Asn Glu Glu Pro Leu Trp Met Ser Lys Cys Leu Ser Val Gly Gly
    50                  55                  60
Leu Leu Val Tyr Arg Gly Ser Trp Lys Lys Thr Ala Leu Ser Arg Leu
65                  70                  75                  80
Asn Leu Cys Ser Glu Asn Asp Glu Ile Tyr Gln Lys Pro Arg His Phe
                85                  90                  95
Asp Gly Phe Asn Ser Met His Leu Tyr Arg Arg Trp Tyr Arg Cys Phe
            100                 105                 110
Thr Asn Leu Ser Ser Phe Ser Phe Asp Asn Gly His Val Glu Arg Lys
        115                 120                 125
Asp Asp Leu Ser Leu Asp Gln Phe Arg Ala Gln Tyr Asp Gly Lys Cys
    130                 135                 140
Pro Val Leu Leu Thr Lys Leu Ala Glu Thr Trp Pro Ala Arg Thr Lys
145                 150                 155                 160
Trp Thr Ala Gln Gln Leu Thr His Asp Tyr Gly Glu Val Pro Phe Arg
                165                 170                 175
Ile Ser Gln Arg Ser Pro Gln Lys Ile Lys Met Lys Leu Lys Asp Tyr
            180                 185                 190
Val Phe Tyr Met Glu Leu Gln His Asp Glu Asp Pro Leu Tyr Ile Phe
        195                 200                 205
Asp Asp Lys Phe Gly Glu Ser Ala Pro Thr Leu Leu Glu Asp Tyr Ser
    210                 215                 220
Val Pro His Leu Phe Gln Glu Asp Phe Phe Glu Ile Met Asp Tyr Asp
225                 230                 235                 240
Gln Arg Pro Ala Phe Arg Trp Leu Ile Ile Gly Pro Glu Arg Ser Gly
                245                 250                 255
Ala Ser Trp His Val Asp Pro Gly Leu Thr Ser Ala Trp Asn Thr Leu
            260                 265                 270
Leu Cys Gly Arg Lys Arg Trp Ala Met Tyr Pro Pro Gly Arg Val Pro
        275                 280                 285
Gly Gly Val Thr Val His Val Ser Asp Glu Asp Gly Asp Val Asp Ile
    290                 295                 300
```

```
Glu Thr Pro Thr Ser Leu Gln Trp Trp Leu Asp Ile Tyr Pro Asn Leu
305                 310                 315                 320
Ala Glu His Glu Lys Pro Leu Glu Cys Thr Gln Leu Pro Gly Glu Thr
                325                 330                 335
Ile Phe Val Pro Ser Gly Trp Trp His Cys Val Leu Asn Leu Asp Met
            340                 345                 350
Thr Ile Ala Val Thr Gln Asn Phe Val Asn Gln Ser Asn Phe Lys His
        355                 360                 365
Val Cys Leu Asp Met Ala Pro Gly Tyr Cys His Lys Gly Val Cys Arg
    370                 375                 380
Ala Gly Leu Leu Ala Ala Pro Asp Lys Ser Ile Arg Asp Ile Glu Asn
385                 390                 395                 400
Leu Pro Ser Ile Thr Ser Arg Leu Asn His Ser Asp Met Ala Cys Lys
                405                 410                 415
Glu Lys Arg Leu Lys Ser Ser Glu Pro Ile Arg Thr Ser Asn Asn Ala
            420                 425                 430
Asn Gln Cys Ser Ala Phe Glu Phe Ser Asp Val His Glu Asn Leu Gly
        435                 440                 445
Asp Gln Val Phe Ser Tyr Asp Ile Asp Phe Leu Ser Gln Phe Leu Glu
    450                 455                 460
Lys Glu Lys Asp His Tyr Ser Ser Val Trp Ser Pro Thr Asn Ser Ile
465                 470                 475                 480
Gly Gln Arg Glu Ala Arg Glu Trp Leu Arg Arg Leu Trp Val Leu Lys
                485                 490                 495
Pro Glu Leu Arg Glu Leu Ile Trp Lys Gly Ala Cys Leu Ala Ile Asn
            500                 505                 510
Val Asp Lys Trp Tyr Ser Cys Leu Glu Glu Ile Ser Ala Cys His Ser
        515                 520                 525
Leu Pro Pro Pro Ser Glu Asp Glu Lys Leu Pro Val Gly Thr Gly Ser
    530                 535                 540
Asn Pro Val Phe Ile Val Ser Gly Asn Val Ile Lys Ile Tyr Ala Glu
545                 550                 555                 560
Gly Gly Leu Gly Tyr Ser Ile His Gly Leu Gly Thr Glu Leu Glu Phe
                565                 570                 575
Tyr Asp Leu Leu Gln Lys Leu Gly Ser Pro Leu Ile Asn His Val Pro
            580                 585                 590
Glu Ile Ile Ala Ser Gly Phe Leu Val Tyr Leu Asp Gly Val Tyr Lys
        595                 600                 605
Thr Val Pro Trp Asp Gly Asn Gly Ile Pro Asp Val Leu Ala Lys Tyr
    610                 615                 620
Tyr Ser Leu Glu Val Ser Tyr Ala Asn Gly Ser Phe Pro Leu Gly Leu
625                 630                 635                 640
Trp Ser Lys Gln Leu Phe Gly Leu Ser Asn Ser Thr Asp Ala Pro Asp
                645                 650                 655
Arg Pro Ile Cys Pro Tyr Met Val Thr Arg Lys Cys Lys Gly Asp Ile
            660                 665                 670
Phe Ala Arg Ile Arg Asp Lys Leu Thr Lys Thr Asp Val Leu Asn Leu
        675                 680                 685
Ala Ser Ser Leu Gly Val Gln Met Arg Asn Ile His Gln Leu Pro Leu
    690                 695                 700
Pro His Val Glu His Ile Ser Lys Ser Gly Asn Glu Asp Ile Lys Ala
705                 710                 715                 720
Lys Glu Asn Ser Ile Ser Asp Val Thr His Val Pro Pro Glu Trp Lys
                725                 730                 735
Gln Val Val Ser Thr Leu Asp Arg Arg Lys Lys Ser Ile Lys Lys His
            740                 745                 750
Leu Ser Asn Trp Gly Gly Ser Ile Pro Gln Val Leu Ile Glu Lys Ala
        755                 760                 765
Glu Glu Tyr Leu Pro Asp Asp Ile Arg Phe Leu Ile Lys Phe Val Lys
    770                 775                 780
Asp Asp Asp Gly Asp Ser Val Tyr Val Val Pro Ser Trp Ile His Ser
```

```
785                 790                 795                 800
Asp Ile Met Asp Asp Asn Ile Leu Ile Glu Gly Thr Thr Glu Pro Gly
                805                 810                 815
Thr Ser Thr Asp Cys Ile Ala Val Glu Asp Leu Asn Lys Met Asp Ala
            820                 825                 830
Ile His Ile Ile Asp Phe Ser Asp Leu Ser Ile Gly Asp Pro Leu Cys
        835                 840                 845
Asp Leu Ile Pro Leu His Leu Asp Val Phe Arg Gly Asp Ile Asp Leu
    850                 855                 860
Leu Arg Gln Phe Leu Arg Ser Tyr Gln Leu Pro Phe Leu Arg Ala Glu
865                 870                 875                 880
Ser Asn Lys Asp Ile Tyr Lys Ser Ile Gln Asn Ser Lys Phe Ser Arg
                885                 890                 895
Ala Ser Tyr Arg Ala Met Cys Tyr Cys Ile Leu His Glu Asp Asn Val
            900                 905                 910
Leu Gly Ala Ile Phe Ser Leu Trp Lys Asp Leu Gly Thr Ala Thr Ser
        915                 920                 925
Trp Glu Asp Val Glu His Leu Val Trp Gly Glu Leu Asn Gln Tyr Gln
    930                 935                 940
Gln Ser Cys Ser Val Gly Glu Ile Asn
945                 950
```

<210> 151
<211> 3171
<212> DNA
<213> Oryza sativa

<400> 151

```
atggagatgg aggaggcggt agacggcaag cacccgaagc gtgggagggg caggccgagg      60
ggacgacggg gcaggggcag gggcaggggg aggggcggcc gctccctcgc gtcgccggcg     120
gcggggcccg gagaccaagg gccgcggcgg cggcgcgggg tcgtcccggc ggcggcggcg     180
gcggcggggg gaagggcgct gagggagagg aggccggcgc ctggtgccta ccgcgagagc     240
ggagctgata atgacgatga tggcggcggc gacgatgagc acgacgagca gaatgatgat     300
ggtgctgaaa aatcggataa tcaagttgtg gattctctta atgaacccaa cagaagtaat     360
acagggaaga agcggggaag accaaagaaa gtgaaagcag agcaggagga cagtaaccaa     420
ctctccaacg ggaaacacct tggcgaaaac aatgggaatg atgaagcgat aatgatgaag     480
ccttcaaaag aatcgaagaa aagaggtgca ggaaagaaac aagaggaaga agagaataat     540
accatatcca ttgaggatga aatgtgtgat gcgaacaata agaaggggaa gaagatgctc     600
actggggaaa atgctttgat gtgccaccag tgtcaaagaa atgacaaagg gagggtgatt     660
tggtgtaaat catgcaacaa taagcgcttc tgtgagccat gcatgaaacg atggtaccca     720
ggtttgtcag aagttgattt tgctgcaaaa tgcccatatt gtagaaagaa ctgtaattgc     780
aaggcatgcc taagaatgat aggagttgaa aagcccctg aaaagaagat ttctgaggaa     840
aatcaacgac gttatgcttt tcgtattgta gacttgttgc ttccttggtt gaaagaactt     900
caacaggagc agatgaagga gaaggaatta gagggtagat tacaaggtgt ttctatggat     960
gaagtaaagt tggaacaagc tgattgtgat atggatgagc gtgtttattg tgataggtgc    1020
aaaacttcga tagttgattt tcatagaagc tgcaaggctt gttcgtatga cttgtgcctt    1080
gcatgctgct gggagcttcg caaaggtgag attccaggag gagaagaggc aaagagtgtg    1140
cagtgggaag agagaggtca gaagtatgtt tttggtaata tttccaagga tgagaagaaa    1200
agggtatctt cgaagaggca catggagacc cccagtactg aaacttgcaa tgacatggct    1260
gttgctgggg acccaaataa ccctttgtta ctgtggaaag ctaatagtga tggcagtata    1320
ccttgtccac caaaggaaat agggggctgc ggtgcttcat ctttggtact cagatgctta    1380
ttaccagaaa ttatgctttc tgagttagaa cacagggcta acaaagttat taagagagaa    1440
gcatttgata aagcaataaa cgaaacaagt gatcagtgcc cttgctttta tcacacaagc    1500
aagataagaa caaatgctac tcgagaggca gcaaacagaa agggctcaag tgataactac    1560
ttgtactgtc cagatgccaa taatatccag gaggatgacc tgtcgcactt tcagatgcat    1620
tggtcaaaag gtgaaccggt tattgtttct gatgctcttc ggttaacatc tggtctgagc    1680
tgggagccat tggttatgtg gcgggcattg cgagagaaga aaaccaatgg tgatgttgaa    1740
gatgagcact ttgctgttaa ggcagtggat tgccttgatt ggaatgaggt ggaaattaac    1800
atacacatgt tctttatggg gtatatgaga ggtagaagac atccgatgac cttttggcct    1860
gagatgctta agctaaaaga ttggccacca tctagtatgt ttgaccagag gttacctcgc    1920
catggtgctg agtttataac tgcattgcca tttcccgagt atactgatcc acgatatggc    1980

ccgctaaatc ttgctgtcag gcttcctgct ggtgtactga agcctgatct tgggccaaaa    2040
acttatattg cttatggatg ttatgaagag ttaggcaggg gtgactctgt gaccaagctt    2100
cattgcgaca tgtctgatgc ggtaaatatc ttgatgcaca cagctgaagt gtcctatgac    2160
actgaacagc ttgacaagat agcaaaaatt aaaatgaaaa tgagagaaca agatcttcat    2220
gaactgtttg ggtttcagaa atcaggcgcc aagggtaaag ctgatgatga agcatcgaaa    2280
atctcatgta acatggaaaa caaacacacc tctaaccaaa gtactaaggg tttggacatt    2340
aatgctttac cacctgatga ttctggaagt gatattgggg ataaaccatc attttgtcaa    2400
tctgaggtag aaagtgaatt aacacaatgt tcaaaacaca atcacgaggt taatagttct    2460
gtcaagatgc atgctggagc tcattgtact tcagacaacc aaggatacat tgacaggagt    2520
ggattcaaac gcaaagattc agactgttca gaccaacaga aaactggtgg cgctttgtgg    2580
gatattttcc gaagagaaga ttctgagaag ttacaagatt atcttcggaa gcatgcctca    2640
gaatttcggc acatacactg caatccagtg aaaaatgttt ctcacccaat tcatgaccag    2700
actttctatt taactgtgga gcataagaga aagctcaagg aagaacacgg tgttgaacca    2760
tggacattcg agcagaagct aggcgatgca gttttcattc ctgctggatg tccacaccaa    2820
gtgagaaatt taaagtcttg catcaaggtt gctctagact ttgtttcccc tgaaaatgtt    2880
ggtgagtgtg ttaagctgac cggagagttt agacgtcttc catctgatca cagggctaaa    2940
gaagataaac tagagattaa gaagattgct ctcaatgctc ttaaagaagt cgtaaatttc    3000
ttagatcctt taccaaaagg gtcaaagaac agggatgaag tggtagaagt gaccaaacca    3060
aaaaggaaat acggtaaccg aagaggtgac ctgaagagtg ggaagacca acccattgat     3120
gaatccatag aagaaaggaa gcctaaaaag cgagggagat ctaaacggtg a             3171
```

<210> 152

<211> 1056

<212> PRT

<213> Oryza sativa

<400> 152

```
Met Glu Met Glu Glu Ala Val Asp Gly Lys His Pro Lys Arg Gly Arg
1               5                   10                  15
Gly Arg Pro Arg Gly Arg Arg Gly Arg Gly Arg Gly Arg Gly Arg Gly
            20                  25                  30
Gly Arg Ser Leu Ala Ser Pro Ala Ala Gly Pro Gly Asp Gln Gly Pro
        35                  40                  45
Arg Arg Arg Arg Gly Val Val Pro Ala Ala Ala Ala Ala Ala Gly Gly
    50                  55                  60
Arg Ala Leu Arg Glu Arg Arg Pro Ala Pro Gly Ala Tyr Arg Glu Ser
65                  70                  75                  80
Gly Ala Asp Asn Asp Asp Asp Gly Gly Gly Asp Asp Glu His Asp Glu
                85                  90                  95
Gln Asn Asp Asp Gly Ala Glu Lys Ser Asp Asn Gln Val Val Asp Ser
            100                 105                 110
Leu Asn Glu Pro Asn Arg Ser Asn Thr Gly Lys Lys Arg Gly Arg Pro
        115                 120                 125
Lys Lys Val Lys Ala Glu Gln Glu Asp Ser Asn Gln Leu Ser Asn Gly
    130                 135                 140
Lys His Leu Gly Glu Asn Asn Gly Asn Asp Glu Ala Ile Met Met Lys
145                 150                 155                 160
Pro Ser Lys Glu Ser Lys Lys Arg Gly Ala Gly Lys Lys Gln Glu Glu
                165                 170                 175
Glu Glu Asn Asn Thr Ile Ser Ile Glu Asp Glu Met Cys Asp Ala Asn
            180                 185                 190
Asn Lys Lys Gly Lys Lys Met Leu Thr Gly Glu Asn Ala Leu Met Cys
        195                 200                 205
His Gln Cys Gln Arg Asn Asp Lys Gly Arg Val Ile Trp Cys Lys Ser
    210                 215                 220
Cys Asn Asn Lys Arg Phe Cys Glu Pro Cys Met Lys Arg Trp Tyr Pro
225                 230                 235                 240
Gly Leu Ser Glu Val Asp Phe Ala Ala Lys Cys Pro Tyr Cys Arg Lys
                245                 250                 255
Asn Cys Asn Cys Lys Ala Cys Leu Arg Met Ile Gly Val Glu Lys Pro
            260                 265                 270
```

Pro Glu Lys Lys Ile Ser Glu Glu Asn Gln Arg Arg Tyr Ala Phe Arg
275 280 285
Ile Val Asp Leu Leu Leu Pro Trp Leu Lys Glu Leu Gln Gln Glu Gln
290 295 300
Met Lys Glu Lys Glu Leu Glu Gly Arg Leu Gln Gly Val Ser Met Asp
305 310 315 320
Glu Val Lys Leu Glu Gln Ala Asp Cys Asp Met Asp Glu Arg Val Tyr
325 330 335
Cys Asp Arg Cys Lys Thr Ser Ile Val Asp Phe His Arg Ser Cys Lys
340 345 350
Ala Cys Ser Tyr Asp Leu Cys Leu Ala Cys Cys Trp Glu Leu Arg Lys
355 360 365
Gly Glu Ile Pro Gly Gly Glu Glu Ala Lys Ser Val Gln Trp Glu Glu
370 375 380
Arg Gly Gln Lys Tyr Val Phe Gly Asn Ile Ser Lys Asp Glu Lys Lys
385 390 395 400
Arg Val Ser Ser Lys Arg His Met Glu Thr Pro Ser Thr Glu Thr Cys
405 410 415
Asn Asp Met Ala Val Ala Gly Asp Pro Asn Asn Pro Leu Leu Leu Trp
420 425 430
Lys Ala Asn Ser Asp Gly Ser Ile Pro Cys Pro Pro Lys Glu Ile Gly
435 440 445
Gly Cys Gly Ala Ser Ser Leu Val Leu Arg Cys Leu Leu Pro Glu Ile
450 455 460
Met Leu Ser Glu Leu Glu His Arg Ala Asn Lys Val Ile Lys Arg Glu
465 470 475 480
Ala Phe Asp Lys Ala Ile Asn Glu Thr Ser Asp Gln Cys Pro Cys Phe
485 490 495
Tyr His Thr Ser Lys Ile Arg Thr Asn Ala Thr Arg Glu Ala Ala Asn
500 505 510
Arg Lys Gly Ser Ser Asp Asn Tyr Leu Tyr Cys Pro Asp Ala Asn Asn
515 520 525
Ile Gln Glu Asp Asp Leu Ser His Phe Gln Met His Trp Ser Lys Gly
530 535 540
Glu Pro Val Ile Val Ser Asp Ala Leu Arg Leu Thr Ser Gly Leu Ser
545 550 555 560
Trp Glu Pro Leu Val Met Trp Arg Ala Leu Arg Glu Lys Lys Thr Asn
565 570 575
Gly Asp Val Glu Asp Glu His Phe Ala Val Lys Ala Val Asp Cys Leu
580 585 590
Asp Trp Asn Glu Val Glu Ile Asn Ile His Met Phe Phe Met Gly Tyr
595 600 605
Met Arg Gly Arg Arg His Pro Met Thr Phe Trp Pro Glu Met Leu Lys
610 615 620
Leu Lys Asp Trp Pro Pro Ser Ser Met Phe Asp Gln Arg Leu Pro Arg
625 630 635 640
His Gly Ala Glu Phe Ile Thr Ala Leu Pro Phe Pro Glu Tyr Thr Asp
645 650 655
Pro Arg Tyr Gly Pro Leu Asn Leu Ala Val Arg Leu Pro Ala Gly Val
660 665 670
Leu Lys Pro Asp Leu Gly Pro Lys Thr Tyr Ile Ala Tyr Gly Cys Tyr
675 680 685
Glu Glu Leu Gly Arg Gly Asp Ser Val Thr Lys Leu His Cys Asp Met
690 695 700
Ser Asp Ala Val Asn Ile Leu Met His Thr Ala Glu Val Ser Tyr Asp
705 710 715 720
Thr Glu Gln Leu Asp Lys Ile Ala Lys Ile Lys Met Lys Met Arg Glu
725 730 735
Gln Asp Leu His Glu Leu Phe Gly Val Ser Glu Ser Gly Ala Lys Gly
740 745 750
Lys Ala Asp Asp Glu Ala Ser Lys Ile Ser Cys Asn Met Glu Asn Lys

```
        755                 760                 765
His Thr Ser Asn Gln Ser Thr Lys Gly Leu Asp Ile Asn Ala Leu Pro
    770                 775                 780
Pro Asp Asp Ser Gly Ser Asp Ile Gly Asp Lys Pro Ser Phe Cys Gln
785                 790                 795                 800
Ser Glu Val Glu Ser Glu Leu Thr Gln Cys Ser Lys His Asn His Glu
                805                 810                 815
Val Asn Ser Ser Val Lys Met His Ala Gly Ala His Cys Thr Ser Asp
            820                 825                 830
Asn Gln Gly Tyr Ile Asp Arg Ser Gly Phe Lys Arg Lys Asp Ser Asp
        835                 840                 845
Cys Ser Asp Gln Gln Lys Thr Gly Gly Ala Leu Trp Asp Ile Phe Arg
    850                 855                 860
Arg Glu Asp Ser Glu Lys Leu Gln Asp Tyr Leu Arg Lys His Ala Ser
865                 870                 875                 880
Glu Phe Arg His Ile His Cys Asn Pro Val Lys Asn Val Ser His Pro
                885                 890                 895
Ile His Asp Gln Thr Phe Tyr Leu Thr Val Glu His Lys Arg Lys Leu
            900                 905                 910
Lys Glu Glu His Gly Val Glu Pro Trp Thr Phe Glu Gln Lys Leu Gly
        915                 920                 925
Asp Ala Val Phe Ile Pro Ala Gly Cys Pro His Gln Val Arg Asn Leu
    930                 935                 940
Lys Ser Cys Ile Lys Val Ala Leu Asp Phe Val Ser Pro Glu Asn Val
945                 950                 955                 960
Gly Glu Cys Val Lys Leu Thr Gly Glu Phe Arg Arg Leu Pro Ser Asp
                965                 970                 975
His Arg Ala Lys Glu Asp Lys Leu Glu Ile Lys Lys Ile Ala Leu Asn
            980                 985                 990
Ala Leu Lys Glu Val Val Asn Phe  Leu Asp Pro Leu Pro  Lys Gly Ser
        995                 1000                1005
Lys Asn  Arg Asp Glu Val Val  Glu Val Thr Lys Pro  Lys Arg Lys
    1010                1015                1020
Tyr Gly  Asn Arg Arg Gly Asp  Leu Lys Ser Gly Glu  Asp Gln Pro
    1025                1030                1035
Ile Asp  Glu Ser Ile Glu Glu  Arg Lys Pro Lys Lys  Arg Gly Arg
    1040                1045                1050
Ser Lys  Arg
    1055
```

<210> 153
<211> 3159
<212> DNA
<213> Oryza sativa

<400> 153

```
atggagatgg aggaggcggt agacggcaag cacccgaagc gtgggagggg caggccgagg      60
ggacgacggg gcagggggcag gggcaggggg aggggcggcc gctccctcgc gtcgccggcg    120
gcggggcccg gagaccaagg gccgcggcgg cggcgcgggg tcgtcccggc ggcggcggcg    180
gcggcggggg gaagggcgct gagggagagg aggccggcgc ctggtgccta ccgcgagagc    240
ggagctgata atgacgatga tggcggcggc gacgatgagc acgacgagca gaatgatgat    300
ggtgctgaaa aatcggataa tcaagttgtg gattctctta atgaacccaa cagaagtaat    360
acagggaaga agcggggaag accaaagaaa gtgaaagcag agcaggagga cagtaaccaa    420
ctctccaacg ggaaacacct tggcgaaaac aatgggaatg atgaagcgat aatgatgaag    480
ccttcaaaag aatcgaagaa aagaggtgca ggaaagaaac aagaggaaga agagaataat    540
accatatcca ttgaggatga aatgtgtgat gcgaacaata agaaggggaa gaagatgctc    600
actggggaaa atgctttgat gtgccaccag tgtcaaagaa atgacaaagg gagggtgatt    660
tggtgtaaat catgcaacaa taagcgcttc tgtgagccat gcatgaaacg atggtaccca    720
ggtttgtcag aagttgattt tgctgcaaaa tgcccatatt gtagaaagaa ctgtaattgc    780
aaggcatgcc taagaatgat aggagttgaa aagcccccctg aaaagaagat ttctgaggaa    840
aatcaacgac gttatgcttt tcgtattgta gacttgttgc ttccttggtt gaaagaactt    900
```

```
caacaggagc agatgaagga gaaggaatta gagggtagat tacaaggtgt ttctatggat     960
gaagtaaagt tggaacaagc tgattgtgat atggatgagc gtgtttattg tgataggtgc    1020
aaaacttcga tagttgattt tcatagaagc tgcaaggctt gttcgtatga cttgtgcctt    1080
gcatgctgct gggagcttcg caaaggtgag attccaggag gagaagaggc aaagagtgtg    1140
cagtgggaag agagaggtca gaagtatgtt tttggtaata tttccaagga tgagaagaaa    1200
agggtatctt cgaagaggca catggagacc cccagtactg aaacttgcaa tgacatggct    1260
gttgctgggg acccaaataa ccctttgtta ctgtggaaag ctaatagtga tggcagtata    1320
ccttgtccac caaaggaaat aggggggctgc ggtgcttcat ctttggtact cagatgctta    1380
ttaccagaaa ttatgctttc tgagttagaa cacagggcta acaaagttat taagagagaa    1440
gcatttgata aagcaataaa cgaaacaagt gatcagtgcc cttgctttta tcacacaagc    1500
aagataagaa caaatgctac tcgagaggca gcaaacagaa agggctcaag tgataactac    1560
ttgtactgtc cagatgccaa taatatccag gaggatgacc tgtcgcactt tcagatgcat    1620
tggtcaaaag gtgaaccggt tattgtttct gatgctcttc ggttaacatc tggtctgagc    1680
tgggagccat tggttatgtg gcgggcattg cgagagaaga aaaccaatgg tgatgttgaa    1740
gatgagcact ttgctgttaa ggcagtggat tgccttgatt ggaatgaggt ggaaattaac    1800
atacacatgt tctttatggg gtatatgaga ggtagaagac atccgatgac cttttggcct    1860
gagatgctta agctaaaaga ttggccacca tctagtatgt ttgaccagag gttacctcgc    1920
catggtgctg agtttataac tgcattgcca tttcccgagt atactgatcc acgatatggc    1980
ccgctaaatc ttgctgtcag gcttcctgct ggtgtactga agcctgatct tgggccaaaa    2040
acttatattg cttatggatg ttatgaagag ttaggcaggg gtgactctgt gaccaagctt    2100
cattgcgaca tgtctgatgc ggtaaatatc ttgatgcaca cagctgaagt gtcctatgac    2160
actgaacagc ttgacaagat agcaaaaatt aaaatgaaaa tgagagaaca agatcttcat    2220
gaactgtttg ggtttcaga atcaggcgcc aagggtaaag ctgatgatga agcatcgaaa    2280
atctcatgta acatggaaaa caaacacacc tctaaccaaa gtactaaggg tttggacatt    2340
aatgctttac cacctgatga ttctggaagt gatattgggg ataaaccatc attttgtcaa    2400
tctgaggtag aaagtgaatt aacacaatgt tcaaaacaca atcacgaggt taatagttct    2460
gtcaagatgc atgctggagc tcattgtact tcagacaacc aaggatacat tgacaggagt    2520
ggattcaaac gcaaagattc agactgttca gaccaacaga aaactggtgg cgctttgtgg    2580
gatattttcc gaagagaaga ttctgagaag ttacaagatt atcttcggaa gcatgcctca    2640
gaatttcggc acatacactg caatccagtg aaaaatgttt ctcacccaat tcatgaccag    2700
actttctatt taactgtgga gcataagaga aagctcaagg aagaacacgg tgttgaacca    2760
tggacattcg agcagaagct aggcgatgca gttttcattc ctgctggatg tccacaccaa    2820
gtgagaaatt taaagtcttg catcaaggtt gctctagact ttgtttcccc tgaaaatgtt    2880
ggtgagtgtg ttaagctgac cggagagttt agacgtcttc catctgatca cagggctaaa    2940
gaagataaac tagagattaa gaagattgct ctcaatgctc ttaaagaagt cgtaaatttc    3000
ttagatcctt taccaaaagg ggatgaagtg gtagaagtga ccaaaccaaa aaggaaatac    3060
ggtaaccgaa gaggtgacct gaagagtggg gaagaccaac ccattgatga atccatagaa    3120
gaaaggaagc ctaaaaagcg agggagatct aaacggtga                           3159
```

<210> 154
<211> 1052
<212> PRT
<213> Oryza sativa

<400> 154

```
Met Glu Met Glu Glu Ala Val Asp Gly Lys His Pro Lys Arg Gly Arg
1               5                   10                  15
Gly Arg Pro Arg Gly Arg Arg Gly Arg Gly Arg Gly Arg Gly Arg Gly
            20                  25                  30
Gly Arg Ser Leu Ala Ser Pro Ala Ala Gly Pro Gly Asp Gln Gly Pro
        35                  40                  45
Arg Arg Arg Arg Gly Val Val Pro Ala Ala Ala Ala Ala Ala Gly Gly
    50                  55                  60
Arg Ala Leu Arg Glu Arg Arg Pro Ala Pro Gly Ala Tyr Arg Glu Ser
65                  70                  75                  80
Gly Ala Asp Asn Asp Asp Asp Gly Gly Gly Asp Asp Glu His Asp Glu
                85                  90                  95
Gln Asn Asp Asp Gly Ala Glu Lys Ser Asp Asn Gln Val Val Asp Ser
            100                 105                 110
Leu Asn Glu Pro Asn Arg Ser Asn Thr Gly Lys Lys Arg Gly Arg Pro
        115                 120                 125
```

```
Lys Lys Val Lys Ala Glu Gln Glu Asp Ser Asn Gln Leu Ser Asn Gly
    130                 135                 140
Lys His Leu Gly Glu Asn Asn Gly Asn Asp Glu Ala Ile Met Met Lys
145                 150                 155                     160
Pro Ser Lys Glu Ser Lys Lys Arg Gly Ala Gly Lys Lys Gln Glu Glu
                165                 170                 175
Glu Glu Asn Asn Thr Ile Ser Ile Glu Asp Glu Met Cys Asp Ala Asn
            180                 185                 190
Asn Lys Lys Gly Lys Lys Met Leu Thr Gly Glu Asn Ala Leu Met Cys
        195                 200                 205
His Gln Cys Gln Arg Asn Asp Lys Gly Arg Val Ile Trp Cys Lys Ser
    210                 215                 220
Cys Asn Asn Lys Arg Phe Cys Glu Pro Cys Met Lys Arg Trp Tyr Pro
225                 230                 235                     240
Gly Leu Ser Glu Val Asp Phe Ala Ala Lys Cys Pro Tyr Cys Arg Lys
                245                 250                 255
Asn Cys Asn Cys Lys Ala Cys Leu Arg Met Ile Gly Val Glu Lys Pro
            260                 265                 270
Pro Glu Lys Lys Ile Ser Glu Glu Asn Gln Arg Arg Tyr Ala Phe Arg
        275                 280                 285
Ile Val Asp Leu Leu Leu Pro Trp Leu Lys Glu Leu Gln Gln Glu Gln
    290                 295                 300
Met Lys Glu Lys Glu Leu Glu Gly Arg Leu Gln Gly Val Ser Met Asp
305                 310                 315                     320
Glu Val Lys Leu Glu Gln Ala Asp Cys Asp Met Asp Glu Arg Val Tyr
                325                 330                 335
Cys Asp Arg Cys Lys Thr Ser Ile Val Asp Phe His Arg Ser Cys Lys
            340                 345                 350
Ala Cys Ser Tyr Asp Leu Cys Leu Ala Cys Cys Trp Glu Leu Arg Lys
        355                 360                 365
Gly Glu Ile Pro Gly Gly Glu Glu Ala Lys Ser Val Gln Trp Glu Glu
    370                 375                 380
Arg Gly Gln Lys Tyr Val Phe Gly Asn Ile Ser Lys Asp Glu Lys Lys
385                 390                 395                     400
Arg Val Ser Ser Lys Arg His Met Glu Thr Pro Ser Thr Glu Thr Cys
                405                 410                 415
Asn Asp Met Ala Val Ala Gly Asp Pro Asn Asn Pro Leu Leu Leu Trp
            420                 425                 430
Lys Ala Asn Ser Asp Gly Ser Ile Pro Cys Pro Pro Lys Glu Ile Gly
        435                 440                 445
Gly Cys Gly Ala Ser Ser Leu Val Leu Arg Cys Leu Leu Pro Glu Ile
    450                 455                 460
Met Leu Ser Glu Leu Glu His Arg Ala Asn Lys Val Ile Lys Arg Glu
465                 470                 475                     480
Ala Phe Asp Lys Ala Ile Asn Glu Thr Ser Asp Gln Cys Pro Cys Phe
                485                 490                 495
Tyr His Thr Ser Lys Ile Arg Thr Asn Ala Thr Arg Glu Ala Ala Asn
            500                 505                 510
Arg Lys Gly Ser Ser Asp Asn Tyr Leu Tyr Cys Pro Asp Ala Asn Asn
        515                 520                 525
Ile Gln Glu Asp Asp Leu Ser His Phe Gln Met His Trp Ser Lys Gly
    530                 535                 540
Glu Pro Val Ile Val Ser Asp Ala Leu Arg Leu Thr Ser Gly Leu Ser
545                 550                 555                     560
Trp Glu Pro Leu Val Met Trp Arg Ala Leu Arg Glu Lys Lys Thr Asn
                565                 570                 575
Gly Asp Val Glu Asp Glu His Phe Ala Val Lys Ala Val Asp Cys Leu
            580                 585                 590
Asp Trp Asn Glu Val Glu Ile Asn Ile His Met Phe Phe Met Gly Tyr
        595                 600                 605
Met Arg Gly Arg Arg His Pro Met Thr Phe Trp Pro Glu Met Leu Lys
```

```
    610                 615                 620
Leu Lys Asp Trp Pro Pro Ser Ser Met Phe Asp Gln Arg Leu Pro Arg
625                 630                 635                 640
His Gly Ala Glu Phe Ile Thr Ala Leu Pro Phe Pro Glu Tyr Thr Asp
                645                 650                 655
Pro Arg Tyr Gly Pro Leu Asn Leu Ala Val Arg Leu Pro Ala Gly Val
            660                 665                 670
Leu Lys Pro Asp Leu Gly Pro Lys Thr Tyr Ile Ala Tyr Gly Cys Tyr
        675                 680                 685
Glu Glu Leu Gly Arg Gly Asp Ser Val Thr Lys Leu His Cys Asp Met
    690                 695                 700
Ser Asp Ala Val Asn Ile Leu Met His Thr Ala Glu Val Ser Tyr Asp
705                 710                 715                 720
Thr Glu Gln Leu Asp Lys Ile Ala Lys Ile Lys Met Lys Met Arg Glu
                725                 730                 735
Gln Asp Leu His Glu Leu Phe Gly Val Ser Glu Ser Gly Ala Lys Gly
            740                 745                 750
Lys Ala Asp Asp Glu Ala Ser Lys Ile Ser Cys Asn Met Glu Asn Lys
        755                 760                 765
His Thr Ser Asn Gln Ser Thr Lys Gly Leu Asp Ile Asn Ala Leu Pro
    770                 775                 780
Pro Asp Asp Ser Gly Ser Asp Ile Gly Asp Lys Pro Ser Phe Cys Gln
785                 790                 795                 800
Ser Glu Val Glu Ser Glu Leu Thr Gln Cys Ser Lys His Asn His Glu
                805                 810                 815
Val Asn Ser Ser Val Lys Met His Ala Gly Ala His Cys Thr Ser Asp
            820                 825                 830
Asn Gln Gly Tyr Ile Asp Arg Ser Gly Phe Lys Arg Lys Asp Ser Asp
        835                 840                 845
Cys Ser Asp Gln Gln Lys Thr Gly Gly Ala Leu Trp Asp Ile Phe Arg
    850                 855                 860
Arg Glu Asp Ser Glu Lys Leu Gln Asp Tyr Leu Arg Lys His Ala Ser
865                 870                 875                 880
Glu Phe Arg His Ile His Cys Asn Pro Val Lys Asn Val Ser His Pro
                885                 890                 895
Ile His Asp Gln Thr Phe Tyr Leu Thr Val Glu His Lys Arg Lys Leu
            900                 905                 910
Lys Glu Glu His Gly Val Glu Pro Trp Thr Phe Glu Gln Lys Leu Gly
        915                 920                 925
Asp Ala Val Phe Ile Pro Ala Gly Cys Pro His Gln Val Arg Asn Leu
    930                 935                 940
Lys Ser Cys Ile Lys Val Ala Leu Asp Phe Val Ser Pro Glu Asn Val
945                 950                 955                 960
Gly Glu Cys Val Lys Leu Thr Gly Glu Phe Arg Arg Leu Pro Ser Asp
                965                 970                 975
His Arg Ala Lys Glu Asp Lys Leu Glu Ile Lys Lys Ile Ala Leu Asn
            980                 985                 990
Ala Leu Lys Glu Val Val Asn Phe  Leu Asp Pro Leu Pro  Lys Gly Asp
        995                 1000                1005
Glu Val  Val Glu Val Thr Lys  Pro Lys Arg Lys Tyr  Gly Asn Arg
    1010                1015                1020
Arg Gly  Asp Leu Lys Ser Gly  Glu Asp Gln Pro Ile  Asp Glu Ser
    1025                1030                1035
Ile Glu  Glu Arg Lys Pro Lys  Lys Arg Gly Arg Ser  Lys Arg
    1040                1045                1050
```

<210> 155
<211> 3717
<212> DNA
<213> Oryza sativa

<400> 155

```
atgatggggg ttaccaccac gctcaacgag gacactgaac cctctattcc acctggattt      60
ggacctttttg ctacccttcc gttatgggga atccacaatg atgccaaacc tgctgttact     120
cattctactc ctgttcaagc attgcaaagc attagaaaag acagcgaaga atgccaaccc     180
agtgcggctg tgtctcggag tgatacacct tgcagcactt ccggaaccca gacatgcaga     240
aaatcactgc gtaacagacc cccaatagac tatagccgct ttgaacatat atcggatgaa     300
gattctgatg tcgaaatagt ggaaaaggat gtaagttcaa cgagacgcag acaacagcta     360
ccgaaaggag tacttcgagg atgtgcagaa tgcagtgact gtcaaaaggt tatcgcaaaa     420
tggaatccag ctggtgcacg caggcctgtt cttgatgagg ctcctgtttt ctatccaaca     480
gaggaggaat ttgaagacac tctaaaatac attgagagta tacggccaat ggcggaacca     540
tatggtattt gccgtattgt cccaccatct tcttggaagc ctccatgcct tcttaaagat     600
aaaagcatat gggaaggatc aaaattctct actcgggtac aaaaggttga caagctccaa     660
aaccgtaaat catcaaaaaa gggcagaaga ggtggaatga tgaagaggag aaagcttgca     720
gagtcagagg agaacagtgc cactgctcac actcagacag ggatgcagca aagtccagag     780
agatttggat ttgaacctgg gccagagttc acgttacaga catttcagaa atatgcagat     840
gatttcagta agcagtactt taggaaagat acatcgatgg attcagtacc atcagtggaa     900
gatattgaag gtgagtactg gcgcatcgtt gaggttccca cagaagagat agaggtgata     960
tatggtgctg atctggagac tggaactttc ggcagtggtt ttccaaaatt atctcctgag    1020
acaaaatctg atgctgagga taaatatgca caatctggtt ggaatctaaa taacttgcct    1080
agactacaag gttcagttct ttctttcgag ggcggtgaca tttctggtgt tctagtgcct    1140
tgggtgtatg ttggcatgtg tttttcatca ttctgctggc atgttgaaga ccatcattta    1200
tactcactaa actacatgca ttggggtgcc ccaaagttgt ggtatggagt tccaggaaag    1260
gatgctgtga atttggaatc tgcaatgagg aaacatctac ctgaattatt tgaggagcaa    1320
cctgatttgc tacacaacct tgttacccag ttttcaccat cgctgctgaa atctgaagga    1380
gtacatgtat accgttgtgt tcagcatgag ggcgagtttg tcttgacatt cccaagggcg    1440
taccatgctg gtttcaattg tggcttcaat tgtgccgaag ctgttaatgt ggctcctatt    1500
gattggttac cgattggaca taatgctgta gagctttatc gtgagcaagc taggaaaata    1560
accatttctc atgataagtt gttgttgggg gctgcaagag aagcaataag agctcagtgg    1620
gatatcctat tcctcaagag gaatactgct gataatatga ggtggaagag tatatgcgga    1680
gctgatagca ctatattcaa ggctcttaag gcacgaattg agacagagtt ggtgcaaagg    1740
aaaactctag gtgttccagc tcaatcaagg aaaatggatg ctgaattcga ttccattgat    1800
agggaatgtg ccttgtgcta ctatgattta catctttctg cttctggctg tccatgctgc    1860
ccagagaaat atgcttgcct tgtacatgca aagcaacttt gctcatgtga ctgggacaaa    1920
aggtttttcc tattccgcta tgatgtcaat gagctaaata tcttagctga tgctttaggg    1980
gggaaattaa gtgccattca tagatggggc gtctctgatc ttggattaag tttgagttca    2040
tgtgtcaaac gagaaaaggt ccaagattcc aagactgttc gcagattaac tgatggtcca    2100
agaaggtctt acatgtcaca ggcatcagca gtatccttgg tttcttcttc tacttccaat    2160
gaacagaaag atgaaggaaa taagatcatg aagatagcta gcccacagac aaataatgtg    2220
tgcccttctg tcgagcaaag gaaatcagag aatatttcac cattgaagga gccatgtgta    2280
aggaatgagt tgtcatgtac aacaaattct gatagtaacg gattgcaata taatggagga    2340
cttggaggcc ataaaggatc tgcaccaggc ttgccagttt cttctagccc atcattttct    2400
tccaacgttg caacaaggcc cattagtact tcaagtgtat ccatgaaaat tgtgcaaggc    2460
ttggtggcat ctaaaagttg tatacaagct tcctctcgaa ctggagacag tagatcattg    2520
cttggtgagc atcataacag atcaccggca atgattcatg atggaaccaa catgaagtcc    2580
agtttggaaa gctcaaacaa ttcttgcagg ttgattgcat ctgactataa tgcaactccg    2640
tgtcattcat ccaaggatca ggtattagta acaccaggga ctaatgcctc agtagtgact    2700
ctgaaagata gcagccaggt ccatagtgcg tcaagtcagc agtttgtcag aactggccca    2760
tggacacaaa gtgcttctca tgaagcatca tcacctagta cctctgcttt gaagccttct    2820
ttagatcccc ctgccatgaa aaatctgtat gggggtttta ctcaaggcag tgcccatcct    2880
ggacctccaa gtttcagtaa tcagcaacca aatgatgggc gtcttcaaag aacatctgaa    2940
tctctaccag gtgtggaagc tagagctagg ggacatccaa ctgtcacggc acagcctgca    3000
ctagaaattc acagcaggaa tggaggtgca cagaagggtc ctcgcatagc caatgttgtg    3060
catcgtttca agtgctctgt tgaacctctc gaaattggtg ttgtgctatc agggaggctg    3120
tggtcttcaa gccaagcaat cttcccgaaa gggtttagaa gcagagtgaa atacttcagc    3180
attgtggatc caatccaaat ggcatactac atatcggaaa tactggatgc tgggatgcag    3240
gggcctctgt ttatggtaaa attagagaac tgtccaggtg aagttttcat taacttatct    3300
ccaaccaagt gttggaacat ggtccgtgaa aggctgaaca tggaaataag gaggcaactt    3360
aatatgggaa aatcaaatct tcctacattg cagcctccag gatcagttga tggtcttgaa    3420
atgtttggtt tattatcacc accaatagtt caggcaattt gggcgcggga cagagatcac    3480
atctgtacag agtactggag atcaaggccc catgttctca ttgaggatcc aaacaatcgg    3540
catatgttat ctcagggtcc acctctcctt gccctgaggg gtctcatcca aagggctaac    3600
```

```
cgggatgaat tgcaagtcct gcggagtttg atgacgaaca gcaacaattt ggatgatagc   3660
tccaggcaac aggccgcgca cattatcgaa gaggagattg cgaagcaatt gtgctga      3717
```

<210> 156
<211> 1238
<212> PRT
<213> Oryza sativa

<400> 156

```
Met Met Gly Val Thr Thr Thr Leu Asn Glu Asp Thr Glu Pro Ser Ile
1               5                   10                  15
Pro Pro Gly Phe Gly Pro Phe Ala Thr Leu Pro Leu Trp Gly Ile His
            20                  25                  30
Asn Asp Ala Lys Pro Ala Val Thr His Ser Thr Pro Val Gln Ala Leu
        35                  40                  45
Gln Ser Ile Arg Lys Asp Ser Glu Glu Cys Gln Pro Ser Ala Ala Val
    50                  55                  60
Ser Arg Ser Asp Thr Pro Cys Ser Thr Ser Gly Thr Gln Thr Cys Arg
65                  70                  75                  80
Lys Ser Leu Arg Asn Arg Pro Pro Ile Asp Tyr Ser Arg Phe Glu His
                85                  90                  95
Ile Ser Asp Glu Asp Ser Asp Val Glu Ile Val Glu Lys Asp Val Ser
            100                 105                 110
Ser Thr Arg Arg Arg Gln Gln Leu Pro Lys Gly Val Leu Arg Gly Cys
        115                 120                 125
Ala Glu Cys Ser Asp Cys Gln Lys Val Ile Ala Lys Trp Asn Pro Ala
    130                 135                 140
Gly Ala Arg Arg Pro Val Leu Asp Glu Ala Pro Val Phe Tyr Pro Thr
145                 150                 155                 160
Glu Glu Glu Phe Glu Asp Thr Leu Lys Tyr Ile Glu Ser Ile Arg Pro
                165                 170                 175
Met Ala Glu Pro Tyr Gly Ile Cys Arg Ile Val Pro Pro Ser Ser Trp
            180                 185                 190
Lys Pro Pro Cys Leu Leu Lys Asp Lys Ser Ile Trp Glu Gly Ser Lys
        195                 200                 205
Phe Ser Thr Arg Val Gln Lys Val Asp Lys Leu Gln Asn Arg Lys Ser
    210                 215                 220
Ser Lys Lys Gly Arg Arg Gly Gly Met Met Lys Arg Arg Lys Leu Ala
225                 230                 235                 240
Glu Ser Glu Glu Asn Ser Ala Thr Ala His Thr Gln Thr Gly Met Gln
                245                 250                 255
Gln Ser Pro Glu Arg Phe Gly Phe Glu Pro Gly Pro Glu Phe Thr Leu
            260                 265                 270
Gln Thr Phe Gln Lys Tyr Ala Asp Asp Phe Ser Lys Gln Tyr Phe Arg
        275                 280                 285
Lys Asp Thr Ser Met Asp Ser Val Pro Ser Val Glu Asp Ile Glu Gly
    290                 295                 300
Glu Tyr Trp Arg Ile Val Glu Val Pro Thr Glu Glu Ile Glu Val Ile
305                 310                 315                 320
Tyr Gly Ala Asp Leu Glu Thr Gly Thr Phe Gly Ser Gly Phe Pro Lys
                325                 330                 335
Leu Ser Pro Glu Thr Lys Ser Asp Ala Glu Asp Lys Tyr Ala Gln Ser
            340                 345                 350
Gly Trp Asn Leu Asn Asn Leu Pro Arg Leu Gln Gly Ser Val Leu Ser
        355                 360                 365
Phe Glu Gly Gly Asp Ile Ser Gly Val Leu Val Pro Trp Val Tyr Val
    370                 375                 380
Gly Met Cys Phe Ser Ser Phe Cys Trp His Val Glu Asp His His Leu
385                 390                 395                 400
Tyr Ser Leu Asn Tyr Met His Trp Gly Ala Pro Lys Leu Trp Tyr Gly
                405                 410                 415
```

```
Val Pro Gly Lys Asp Ala Val Asn Leu Glu Ser Ala Met Arg Lys His
            420                 425                 430
Leu Pro Glu Leu Phe Glu Glu Gln Pro Asp Leu Leu His Asn Leu Val
        435                 440                 445
Thr Gln Phe Ser Pro Ser Leu Leu Lys Ser Glu Gly Val His Val Tyr
    450                 455                 460
Arg Cys Val Gln His Glu Gly Glu Phe Val Leu Thr Phe Pro Arg Ala
465                 470                 475                 480
Tyr His Ala Gly Phe Asn Cys Gly Phe Asn Cys Ala Glu Ala Val Asn
                485                 490                 495
Val Ala Pro Ile Asp Trp Leu Pro Ile Gly His Asn Ala Val Glu Leu
            500                 505                 510
Tyr Arg Glu Gln Ala Arg Lys Ile Thr Ile Ser His Asp Lys Leu Leu
        515                 520                 525
Leu Gly Ala Ala Arg Glu Ala Ile Arg Ala Gln Trp Asp Ile Leu Phe
    530                 535                 540
Leu Lys Arg Asn Thr Ala Asp Asn Met Arg Trp Lys Ser Ile Cys Gly
545                 550                 555                 560
Ala Asp Ser Thr Ile Phe Lys Ala Leu Lys Ala Arg Ile Glu Thr Glu
                565                 570                 575
Leu Val Gln Arg Lys Thr Leu Gly Val Pro Ala Gln Ser Arg Lys Met
            580                 585                 590
Asp Ala Glu Phe Asp Ser Ile Asp Arg Glu Cys Ala Leu Cys Tyr Tyr
        595                 600                 605
Asp Leu His Leu Ser Ala Ser Gly Cys Pro Cys Cys Pro Glu Lys Tyr
    610                 615                 620
Ala Cys Leu Val His Ala Lys Gln Leu Cys Ser Cys Asp Trp Asp Lys
625                 630                 635                 640
Arg Phe Phe Leu Phe Arg Tyr Asp Val Asn Glu Leu Asn Ile Leu Ala
                645                 650                 655
Asp Ala Leu Gly Gly Lys Leu Ser Ala Ile His Arg Trp Gly Val Ser
            660                 665                 670
Asp Leu Gly Leu Ser Leu Ser Ser Cys Val Lys Arg Glu Lys Val Gln
        675                 680                 685
Asp Ser Lys Thr Val Arg Arg Leu Thr Asp Gly Pro Arg Arg Ser Tyr
    690                 695                 700
Met Ser Gln Ala Ser Ala Val Ser Leu Val Ser Ser Ser Thr Ser Asn
705                 710                 715                 720
Glu Gln Lys Asp Glu Gly Asn Lys Ile Met Lys Ile Ala Ser Pro Gln
                725                 730                 735
Thr Asn Asn Val Cys Pro Ser Val Glu Gln Arg Lys Ser Glu Asn Ile
            740                 745                 750
Ser Pro Leu Lys Glu Pro Cys Val Arg Asn Glu Leu Ser Cys Thr Thr
        755                 760                 765
Asn Ser Asp Ser Asn Gly Leu Gln Tyr Asn Gly Gly Leu Gly Gly His
    770                 775                 780
Lys Gly Ser Ala Pro Gly Leu Pro Val Ser Ser Ser Pro Ser Phe Ser
785                 790                 795                 800
Ser Asn Val Ala Thr Arg Pro Ile Ser Thr Ser Ser Val Ser Met Lys
                805                 810                 815
Ile Val Gln Gly Leu Val Ala Ser Lys Ser Cys Ile Gln Ala Ser Ser
            820                 825                 830
Arg Thr Gly Asp Ser Arg Ser Leu Leu Gly Glu His His Asn Arg Ser
        835                 840                 845
Pro Ala Met Ile His Asp Gly Thr Asn Met Lys Ser Ser Leu Glu Ser
    850                 855                 860
Ser Asn Asn Ser Cys Arg Leu Ile Ala Ser Asp Tyr Asn Ala Thr Pro
865                 870                 875                 880
Cys His Ser Ser Lys Asp Gln Val Leu Val Thr Pro Gly Thr Asn Ala
                885                 890                 895
Ser Val Val Thr Leu Lys Asp Ser Ser Gln Val His Ser Ala Ser Ser
```

```
                900                     905                     910
Gln Gln Phe Val Arg Thr Gly Pro Trp Thr Gln Ser Ala Ser His Glu
            915                     920                     925
Ala Ser Ser Pro Ser Thr Ser Ala Leu Lys Pro Ser Leu Asp Pro Pro
    930                     935                     940
Ala Met Lys Asn Leu Tyr Gly Gly Phe Thr Gln Gly Ser Ala His Pro
945                     950                     955                     960
Gly Pro Pro Ser Phe Ser Asn Gln Gln Pro Asn Asp Gly Arg Leu Gln
                965                     970                     975
Arg Thr Ser Glu Ser Leu Pro Gly Val Glu Ala Arg Ala Arg Gly His
            980                     985                     990
Pro Thr Val Thr Ala Gln Pro Ala  Leu Glu Ile His Ser  Arg Asn Gly
        995                     1000                    1005
Gly Ala  Gln Lys Gly Pro Arg  Ile Ala Asn Val Val  His Arg Phe
    1010                    1015                    1020
Lys Cys  Ser Val Glu Pro Leu  Glu Ile Gly Val Val  Leu Ser Gly
    1025                    1030                    1035
Arg Leu  Trp Ser Ser Ser Gln  Ala Ile Phe Pro Lys  Gly Phe Arg
    1040                    1045                    1050
Ser Arg  Val Lys Tyr Phe Ser  Ile Val Asp Pro Ile  Gln Met Ala
    1055                    1060                    1065
Tyr Tyr  Ile Ser Glu Ile Leu  Asp Ala Gly Met Gln  Gly Pro Leu
    1070                    1075                    1080
Phe Met  Val Lys Leu Glu Asn  Cys Pro Gly Glu Val  Phe Ile Asn
    1085                    1090                    1095
Leu Ser  Pro Thr Lys Cys Trp  Asn Met Val Arg Glu  Arg Leu Asn
    1100                    1105                    1110
Met Glu  Ile Arg Arg Gln Leu  Asn Met Gly Lys Ser  Asn Leu Pro
    1115                    1120                    1125
Thr Leu  Gln Pro Pro Gly Ser  Val Asp Gly Leu Glu  Met Phe Gly
    1130                    1135                    1140
Leu Leu  Ser Pro Pro Ile Val  Gln Ala Ile Trp Ala  Arg Asp Arg
    1145                    1150                    1155
Asp His  Ile Cys Thr Glu Tyr  Trp Arg Ser Arg Pro  His Val Leu
    1160                    1165                    1170
Ile Glu  Asp Pro Asn Asn Arg  His Met Leu Ser Gln  Gly Pro Pro
    1175                    1180                    1185
Leu Leu  Ala Leu Arg Gly Leu  Ile Gln Arg Ala Asn  Arg Asp Glu
    1190                    1195                    1200
Leu Gln  Val Leu Arg Ser Leu  Met Thr Asn Ser Asn  Asn Leu Asp
    1205                    1210                    1215
Asp Ser  Ser Arg Gln Gln Ala  Ala His Ile Ile Glu  Glu Glu Ile
    1220                    1225                    1230
Ala Lys  Gln Leu Cys
    1235
```

<210> 157
<211> 2916
<212> DNA
<213> Oryza sativa

<400> 157

```
atggtttcct cccgcgaccc cggcgaggag gccagcgcgc cgccgccccc gcccccgcgc      60
cgcggcgaga agcggcgaat gcgcggccgc accccgtcgc cggagccggc ctccgcgccg     120
caggatctct gcccatcagg agcttgcggg gacaatgttg ctggagctac aactacaaat     180
ggaaagtggc atccacatga atcgtacaga cctgaaattg atgatgcccc tgttttcact     240
ccaacggaag aggagtttaa agatccaatt agatatatta cgagcattcg tccccaagca     300
gaaaagtatg gaatttgtcg tattgttcca ccatcttctt ggcgaccgcc ttgttctctg     360
aaggagaaga acttctggga atgtacagag ttcaataccc gtgttcaaca agttgacaag     420
cttcaaaacc gggaacccac aaagaaaaaa tcacaacctc gagttcagaa gaagaggaag     480
aggagaaaga gactgagatt tgggatgact cacaggcgtc ctagtgcaaa tacatcagaa     540
```

```
gactgcgcag atgcagacga gaagtttggc tttcaatctg gctcagattt cacactagat    600
gagtttcaga aatatgcaga tgagtttaag cagcagtatt ttggaataaa gggaagtgac    660
gaaatccctc tttctgaaat taaaaagaag aaaaaaaatt ggcaaccatc ggtcgatgaa    720
atagagggag aatattggcg gatagttgta tgccccactg acgaagttga ggtggattat    780
ggtgctgatt tggacacttc aatgttcagt agtggattct ctaaattatc ttcagattca    840
aatagacgag atccatatgg tttatcttgt tggaatttga acaatcttcc acgtattcct    900
gggtctgtac tgtcatttga aactgaggat atatctggcg tcgtagtccc ttggctttat    960
gtagggatgt gcttctcatc attctgttgg cacgtggaag atcatttcct ttattctatg   1020
aattacatgc attttggtga accaaaagta tggtatggtg ttcctggtgc tgatgcagtg   1080
aagctggaag aagctatgag aaagaactta ccaagattgt ttgaagaaca gcctgatctc   1140
ctacatgagc tggttacgca attatctcct tctgttctta aatcagaagg agttcctgtt   1200
tatcgtgttg ttcagaatcc aggcgagttt gttctaacgc taccgcgagc ttaccattct   1260
gggttcaact gtggcttcaa ctgtgcggag gcagtaaatg tcgcacctgt ggattggctg   1320
cctcacggac aatgtgctgt tgagctctac agggagcagc ggcgcaagac atccatatca   1380
catgacaaat tattactaaa aactgcaaat gaagctgtca gacagctttg gatgaacctt   1440
agcgactgca aaagtgaaca aggagtatac agatggcagg atacttgcgg aaaggacgga   1500
atgctgacaa gtgcaattaa gacaagggtt aaaatggaga aggcagcacg ggagggaat   1560
atggcactgc gatataagaa aatggatggg gattatgatt cagctgaccg ggaatgcttt   1620
tcatgttttt atgatctcca tttgtcagct gtcagctgcc aatgctcccc aaatcgtttt   1680
gcttgcttaa accatgcaaa cattctatgt tcatgtgaaa tggacagaaa aaccgcgttg   1740
ttgcggtata ccatagagga gctccatact cttgttgcag ctctagaggg tgatccaact   1800
gcggtctacc agtggggaca gaatgattta ggtttagtct gcccatctgg ttctactcag   1860
tacaagaaga tggacttggg tgaaaacacg gaatttccgg attcagcaac caacgtcaat   1920
catggctgca gcttaggaag tcaagatcaa tatcactatg accccgcaaa gccagcagga   1980
taccagcaag agaagggaat ccagattgct tcagaaaaac atgataagaa caagatggtt   2040
gtcaatcttg agtctccagc aacagctagt aatccaagca ggtcaaagtc tgactgcagt   2100
ggctcactgt ccttgaatca ttcatctgag ttaccatctt caagaattca aacaggaaat   2160
tctacgctag cttccattac cacagagaaa ctgtttggtg ttgacattaa atccaattta   2220
gcacagtctt ctgatggcca agttagtcaa ttggccaagc cttcctcgag ccaaactgat   2280
gaagtctcta agccagcaat agctaagtat acggttgagc tgctagacag tggaacaatg   2340
atgattggta aaaagtggtg caatcagcaa gctatattcc ccaaaggatt taagagtcga   2400
gttacatttc atagtgtact agatccaaca aggacatgct gctacatctc cgaagttctt   2460
gatgctgggc ttcttggacc attgtttagg gtgactgtcg aaggtcttcc agaagtttcg   2520
tttactcaca catcaccaat gcaatgttgg gacagtgtaa gagacagagt aaatgaagaa   2580
atagcaaaac aaataagttt tggaaaatct ggccttcctg atttctatc ctgcaattct    2640
ttgaatggac ttgaaatgtt tgggttctta tcctccccta taattaagga aatcgaggct   2700
ctagatccct gtcaccaatg cttggactat tggttgtcaa gggtttcttc tgttggaact   2760
gaactcccct cggaatctgt gatggcagca atggttaatg actccactaa ccccccaata   2820
aagttgctcg ggattgagat taaccggagg gaatcagaac aatcaagtag cttcaataat   2880
tcctgtgtga ggaggtcaca cttggcaggt tgctga                             2916
```

<210> 158
<211> 971
<212> PRT
<213> Oryza sativa

<400> 158

```
Met Val Ser Ser Arg Asp Pro Gly Glu Glu Ala Ser Ala Pro Pro Pro
1               5                   10                  15
Pro Pro Pro Arg Arg Gly Glu Lys Arg Arg Met Arg Gly Arg Thr Pro
            20                  25                  30
Ser Pro Glu Pro Ala Ser Ala Pro Gln Asp Leu Cys Pro Ser Gly Ala
        35                  40                  45
Cys Gly Asp Asn Val Ala Gly Ala Thr Thr Thr Asn Gly Lys Trp His
    50                  55                  60
Pro His Glu Ser Tyr Arg Pro Glu Ile Asp Asp Ala Pro Val Phe Thr
65                  70                  75                  80
Pro Thr Glu Glu Glu Phe Lys Asp Pro Ile Arg Tyr Ile Thr Ser Ile
                85                  90                  95
Arg Pro Gln Ala Glu Lys Tyr Gly Ile Cys Arg Ile Val Pro Pro Ser
            100                 105                 110
```

```
Ser Trp Arg Pro Pro Cys Ser Leu Lys Glu Lys Asn Phe Trp Glu Cys
        115                 120                 125
Thr Glu Phe Asn Thr Arg Val Gln Gln Val Asp Lys Leu Gln Asn Arg
    130                 135                 140
Glu Pro Thr Lys Lys Lys Ser Gln Pro Arg Val Gln Lys Lys Arg Lys
145                 150                 155                 160
Arg Arg Lys Arg Leu Arg Phe Gly Met Thr His Arg Arg Pro Ser Ala
                165                 170                 175
Asn Thr Ser Glu Asp Cys Ala Asp Ala Asp Glu Lys Phe Gly Phe Gln
            180                 185                 190
Ser Gly Ser Asp Phe Thr Leu Asp Glu Phe Gln Lys Tyr Ala Asp Glu
        195                 200                 205
Phe Lys Gln Gln Tyr Phe Gly Ile Lys Gly Ser Asp Glu Ile Pro Leu
    210                 215                 220
Ser Glu Ile Lys Lys Lys Lys Lys Asn Trp Gln Pro Ser Val Asp Glu
225                 230                 235                 240
Ile Glu Gly Glu Tyr Trp Arg Ile Val Val Cys Pro Thr Asp Glu Val
                245                 250                 255
Glu Val Asp Tyr Gly Ala Asp Leu Asp Thr Ser Met Phe Ser Ser Gly

            260                 265                 270
Phe Ser Lys Leu Ser Ser Asp Ser Asn Arg Arg Asp Pro Tyr Gly Leu
        275                 280                 285
Ser Cys Trp Asn Leu Asn Asn Leu Pro Arg Ile Pro Gly Ser Val Leu
    290                 295                 300
Ser Phe Glu Thr Glu Asp Ile Ser Gly Val Val Pro Trp Leu Tyr
305                 310                 315                 320
Val Gly Met Cys Phe Ser Ser Phe Cys Trp His Val Glu Asp His Phe
                325                 330                 335
Leu Tyr Ser Met Asn Tyr Met His Phe Gly Glu Pro Lys Val Trp Tyr
            340                 345                 350
Gly Val Pro Gly Ala Asp Ala Val Lys Leu Glu Glu Ala Met Arg Lys
        355                 360                 365
Asn Leu Pro Arg Leu Phe Glu Glu Gln Pro Asp Leu Leu His Glu Leu
    370                 375                 380
Val Thr Gln Leu Ser Pro Ser Val Leu Lys Ser Glu Gly Val Pro Val
385                 390                 395                 400
Tyr Arg Val Val Gln Asn Pro Gly Glu Phe Val Leu Thr Leu Pro Arg
                405                 410                 415
Ala Tyr His Ser Gly Phe Asn Cys Gly Phe Asn Cys Ala Glu Ala Val
            420                 425                 430
Asn Val Ala Pro Val Asp Trp Leu Pro His Gly Gln Cys Ala Val Glu
        435                 440                 445
Leu Tyr Arg Glu Gln Arg Arg Lys Thr Ser Ile Ser His Asp Lys Leu
    450                 455                 460
Leu Leu Lys Thr Ala Asn Glu Ala Val Arg Gln Leu Trp Met Asn Leu
465                 470                 475                 480
Ser Asp Cys Lys Ser Glu Gln Gly Val Tyr Arg Trp Gln Asp Thr Cys
                485                 490                 495
Gly Lys Asp Gly Met Leu Thr Ser Ala Ile Lys Thr Arg Val Lys Met
            500                 505                 510
Glu Lys Ala Ala Arg Gly Gly Asn Met Ala Leu Arg Tyr Lys Lys Met
        515                 520                 525
Asp Gly Asp Tyr Asp Ser Ala Asp Arg Glu Cys Phe Ser Cys Phe Tyr
    530                 535                 540
Asp Leu His Leu Ser Ala Val Ser Cys Gln Cys Ser Pro Asn Arg Phe
545                 550                 555                 560
Ala Cys Leu Asn His Ala Asn Ile Leu Cys Ser Cys Glu Met Asp Arg
                565                 570                 575
Lys Thr Ala Leu Leu Arg Tyr Thr Ile Glu Glu Leu His Thr Leu Val
            580                 585                 590
```

```
Ala Ala Leu Glu Gly Asp Pro Thr Ala Val Tyr Gln Trp Gly Gln Asn
        595                 600                 605
Asp Leu Gly Leu Val Cys Pro Ser Gly Ser Thr Gln Tyr Lys Lys Met
    610                 615                 620
Asp Leu Gly Glu Asn Thr Glu Phe Pro Asp Ser Ala Thr Asn Val Asn
625                 630                 635                 640
His Gly Cys Ser Leu Gly Ser Gln Asp Gln Tyr His Tyr Asp Pro Ala
                645                 650                 655
Lys Pro Ala Gly Tyr Gln Gln Glu Lys Gly Ile Gln Ile Ala Ser Glu
            660                 665                 670
Lys His Asp Lys Asn Lys Met Val Val Asn Leu Glu Ser Pro Ala Thr
        675                 680                 685
Ala Ser Asn Pro Ser Arg Ser Lys Ser Asp Cys Ser Gly Ser Leu Ser
    690                 695                 700
Leu Asn His Ser Ser Glu Leu Pro Ser Ser Arg Ile Gln Thr Gly Asn
705                 710                 715                 720
Ser Thr Leu Ala Ser Ile Thr Thr Glu Lys Leu Phe Gly Val Asp Ile
                725                 730                 735
Lys Ser Asn Leu Ala Gln Ser Ser Asp Gly Gln Val Ser Gln Leu Ala
            740                 745                 750
Lys Pro Ser Ser Ser Gln Thr Asp Glu Val Ser Lys Pro Ala Ile Ala
        755                 760                 765
Lys Tyr Thr Val Glu Leu Leu Asp Ser Gly Thr Met Met Ile Gly Lys
    770                 775                 780
Lys Trp Cys Asn Gln Gln Ala Ile Phe Pro Lys Gly Phe Lys Ser Arg
785                 790                 795                 800
Val Thr Phe His Ser Val Leu Asp Pro Thr Arg Thr Cys Cys Tyr Ile
                805                 810                 815
Ser Glu Val Leu Asp Ala Gly Leu Leu Gly Pro Leu Phe Arg Val Thr
            820                 825                 830
Val Glu Gly Leu Pro Glu Val Ser Phe Thr His Thr Ser Pro Met Gln
        835                 840                 845
Cys Trp Asp Ser Val Arg Asp Arg Val Asn Glu Glu Ile Ala Lys Gln
    850                 855                 860
Ile Ser Phe Gly Lys Ser Gly Leu Pro Asp Phe Leu Ser Cys Asn Ser
865                 870                 875                 880
Leu Asn Gly Leu Glu Met Phe Gly Phe Leu Ser Ser Pro Ile Ile Lys
                885                 890                 895
Glu Ile Glu Ala Leu Asp Pro Cys His Gln Cys Leu Asp Tyr Trp Leu
            900                 905                 910
Ser Arg Val Ser Ser Val Gly Thr Glu Leu Pro Ser Glu Ser Val Met
        915                 920                 925
Ala Ala Met Val Asn Asp Ser Thr Asn Pro Pro Ile Lys Leu Leu Gly
    930                 935                 940
Ile Glu Ile Asn Arg Arg Glu Ser Glu Gln Ser Ser Ser Phe Asn Asn
945                 950                 955                 960
Ser Cys Val Arg Arg Ser His Leu Ala Gly Cys
                965                 970
```

<210> 159
<211> 1140
<212> DNA
<213> Oryza sativa

<400> 159

```
atgtcatgtt tgagttggga gccaccagat atgtggtcta aagtacatgg caccggcact      60
agtcctgaga tgaaaaacgt gaaggctatt gattgtttat cttgctgtga ggtcgagata     120
tgcactcaag acttcttcaa tgggtattat gaaggtcgga tgtatcaaaa tctatggcct     180
gaaatgctta aattgaagga ctggcctaca tcaaatcatt ttgaagagct tttgccttcc     240
catggagtta aatacatgaa ttctttacct tttcaaccat acacaaactt gaagtctggt     300
ttgctgaatg tctcaacctt gcttcctgat gacatcttaa agcttgacat gggcccaaaa     360
```

```
tcatatatag cttatggcta tgcacaggaa cttggtagag gagattctgt tacaaagctt    420
cactgcgatt tgtctgatgc agttaacgtt ttgatgcata ctgctgaagt tgacccttcc    480
gaggaacaaa tagatgcaat aaaaagtttg aaaagaagac atacagcgca aaatgaaaag    540
gagtgttctg ggaatgcaga cggaaattat acatctccta aaatctgtgg ggatgcaaat    600
gagttgtcct gtcctataaa cagtgagacc aacaagggag gtgctttatg ggatattttt    660
aggagggaag atgttccaaa actgaaattg tatctcgaca agcattctaa ggaatttcgc    720
catatatact gttctgcagt tcaaaaggta tgtaaccctg tacatgatga aacattttat    780
ctaacagaag aacacaagag aaaactcaag gaggagcatg gaattgagcc ttggacattt    840
gtacaaaaac ttggggaggc agtattcatt cctgctgggt gtcctcatca agtacggaat    900
cttaagtcct gcaccaagat tgccttggat tttgtatcac ccgagaatgt taaggagtgt    960
ctcagcttaa ccgaggactt ccgaagactt cctaagaacc acagggccaa agaagacaaa   1020
ttagagctag gtgtggttca aaatggaccc aagcccacat accctcgaga aactattcta   1080
aactctcaat gggacatctt gttgtttgca tatcatccaa atagttatga caaagtttaa   1140
```

<210> 160
<211> 379
<212> PRT
<213> Oryza sativa

<400> 160

```
Met Ser Cys Leu Ser Trp Glu Pro Pro Asp Met Trp Ser Lys Val His
1               5                   10                  15
Gly Thr Gly Thr Ser Pro Glu Met Lys Asn Val Lys Ala Ile Asp Cys
            20                  25                  30
Leu Ser Cys Cys Glu Val Glu Ile Cys Thr Gln Asp Phe Phe Asn Gly
        35                  40                  45
Tyr Tyr Glu Gly Arg Met Tyr Gln Asn Leu Trp Pro Glu Met Leu Lys
    50                  55                  60
Leu Lys Asp Trp Pro Thr Ser Asn His Phe Glu Glu Leu Leu Pro Ser
65                  70                  75                  80
His Gly Val Lys Tyr Met Asn Ser Leu Pro Phe Gln Pro Tyr Thr Asn
                85                  90                  95
Leu Lys Ser Gly Leu Leu Asn Val Ser Thr Leu Leu Pro Asp Asp Ile
            100                 105                 110
Leu Lys Leu Asp Met Gly Pro Lys Ser Tyr Ile Ala Tyr Gly Tyr Ala
        115                 120                 125
Gln Glu Leu Gly Arg Gly Asp Ser Val Thr Lys Leu His Cys Asp Leu
    130                 135                 140
Ser Asp Ala Val Asn Val Leu Met His Thr Ala Glu Val Asp Pro Ser
145                 150                 155                 160
Glu Glu Gln Ile Asp Ala Ile Lys Ser Leu Lys Arg Arg His Thr Ala
                165                 170                 175
Gln Asn Glu Lys Glu Cys Ser Gly Asn Ala Asp Gly Asn Tyr Thr Ser
            180                 185                 190
Pro Lys Ile Cys Gly Asp Ala Asn Glu Leu Ser Cys Pro Ile Asn Ser
        195                 200                 205
Glu Thr Asn Lys Gly Gly Ala Leu Trp Asp Ile Phe Arg Arg Glu Asp
    210                 215                 220
Val Pro Lys Leu Lys Leu Tyr Leu Asp Lys His Ser Lys Glu Phe Arg
225                 230                 235                 240
His Ile Tyr Cys Ser Ala Val Gln Lys Val Cys Asn Pro Val His Asp
                245                 250                 255
Glu Thr Phe Tyr Leu Thr Glu Glu His Lys Arg Lys Leu Lys Glu Glu
            260                 265                 270
His Gly Ile Glu Pro Trp Thr Phe Val Gln Lys Leu Gly Glu Ala Val
        275                 280                 285
Phe Ile Pro Ala Gly Cys Pro His Gln Val Arg Asn Leu Lys Ser Cys
    290                 295                 300
Thr Lys Ile Ala Leu Asp Phe Val Ser Pro Glu Asn Val Lys Glu Cys
305                 310                 315                 320
Leu Ser Leu Thr Glu Asp Phe Arg Arg Leu Pro Lys Asn His Arg Ala


                325                 330                 335
Lys Glu Asp Lys Leu Glu Leu Gly Val Val Gln Asn Gly Pro Lys Pro
            340                 345                 350
Thr Tyr Pro Arg Glu Thr Ile Leu Asn Ser Gln Trp Asp Ile Leu Leu
        355                 360                 365
Phe Ala Tyr His Pro Asn Ser Tyr Asp Lys Val
    370                 375
```

<210> 161
<211> 2577
<212> DNA
<213> Oryza sativa

<400> 161

```
atgcaacagg tggagggcag gaactgtctt cctgcggagg tcaggattgg cctcgagacg     60
ctcaagaggc gccggcttga gaggatgcgt ttgactgctc agaacaatgc cggcgacggt    120
cctccggtgc ccgcaaggag cggtggggat gcgctaagga ctcccgcaaa ctgcggggtc    180
aggttgcatg ctaacaatgg cacagctcta cctagcagaa ccacccagaa caaggaccct    240
tttgcaaagc gcagggtgga caagtttgat atgtctagcc tagaatggat tgacaagatc    300
gaagaatgcc ctgtgtacta tcctaccaag gaggagttcg aggatcccat tggttatata    360
cagaagattg cacctgtggc ttcgaaatac ggaatttgca aaatcgtatc tccagtaagc    420
gcttctgttc ctgctggtgt cgtgttgatg aaggaacagc ctggtttcaa gttcatgacc    480
agggttcagc cgcttcgcct cgccaaatgg gctgaagatg acacggtcac tttcttcatg    540
agcgaaagaa agtacacttt ccgggattat gagaaaatgg ccaacaaggt gttcgccaag    600
aaatactcaa gtgctagttg tctcccagct aagtacgtgg aggaggaatt ctggcgcgaa    660
attgcttttg gtaaaatgga ttttgttgaa tatgcctgtg atgttgatgg tagtgctttc    720
tcctcttctc ctcatgatca acttgggaaa agcaactgga acttgaagaa tttttcacgg    780
ctttccaatt ctgtgcttag acttctgcag acaccaattc caggagtaac agatccaatg    840
ctttatatcg ggatgctctt cagcatgttt gcttggcatg tggaagatca ttatttgtac    900
agcatcaatt accatcattg tggggcattt aagacatggt atggcatacc gggtgatgct    960
gctcctgggt ttgaaaaggt ggctagccag tttgtataca acaaggatat tttggttggt   1020
gaaggagagg atgcagcatt tgatgttctc ttggggaaga caacaatgtt ccccccaaat   1080
gtcttgttag accacaacgt tcctgtttat aaagctgtgc aaaaacctgg ggagtttgtc   1140
attactttcc ctcgttccta ccacgcgggt ttcagccacg gcttcaattg tggcgaggct   1200
gtcaactttg ctatcagtga ctggtttcct ctgggttctg tggccagcag acgctacgcg   1260
cttctgaaca gaacaccctt gcttgcacac gaggagttac tttgccgttc tgcagtgctt   1320
ctgtcccaca aactgttaaa cagcgaccca aaatccctca ataaatctga gcatccacat   1380
tcacagcgtt gtttgaagtc ttgctttgtg cagttgatgc gattccagag aaacacacgt   1440
ggcctacttg ctaaaatggg ctctcagata cattataagc caaaaacata cccgaatctc   1500
tcatgtagca tgtgtcggcg tgattgctac attacacatg tgttgtgtgg atgcaacttt   1560
gacccagtct gtcttcatca cgaacaagaa ctccggagct gcccttgtaa atctaaccag   1620
gttgtctacg ttagggagga catacaggag ctagaagctc tatcaagaaa atttgagaag   1680
gatatttgct tggataagga aataagtggt tttgactcat acaagcaggc cgaaaagaat   1740
gagccatttt ttgagataac tcggaacctc aggaacactg aagtaaattt gatagaggat   1800
gccttctcag gagcaactgc tgctgatgct gcaaagagtt ctcctgcaac gtcaacactg   1860
acatcttttg cacaacatga tgtgcctgtt cttgctgaag caattgtctg tgctaatcaa   1920
gccgaccaat tatactccac caccgagcaa accatcagct cacctttagt caaaggaact   1980
gatgctgtgg gtgcaaattc atccagcatg gctgatgcta ataacggaac tggttcttgt   2040
aatgcttcag ctgtggaata cagtggaaat tcagattctg aatctgaaat atttcgagtc   2100
aagcgcaggt ctggcgtatc agtaaagcct gcatctgatg ccaagacatc aaacttgtct   2160
gatcaacagg ttctcaggcg gttgaagaag gtgcgccctg aaatacaaca gcacaataag   2220
cgaccagaag actatggtca ctgttcagtt ccctcaggtc gtatgagtat gaagaatttg   2280
aattcatcct cctcatgtgg tgaagaacac tggaggatga agcggcggca gttggagact   2340
cagcaggatg agagcagtta ttctgcaaag cagaagtcgt actcgtatcc atccaccagc   2400
tattctttcc gaggagagtt tgtggaaatg agtagagatg ctgctgcaga agtccgacca   2460
aagcgactga aaatccggct accttcttct agcacgaaca gagtggttga gcagggcagt   2520
tcagggcaaa gatttacaag ggatgacaag tcgcttggtt gttggcctgc aatttag      2577
```

<210> 162
<211> 858
<212> PRT
<213> Oryza sativa

<400> 162

```
Met Gln Gln Val Glu Gly Arg Asn Cys Leu Pro Ala Glu Val Arg Ile
1               5                   10                  15
Gly Leu Glu Thr Leu Lys Arg Arg Arg Leu Glu Arg Met Arg Leu Thr
            20                  25                  30
Ala Gln Asn Asn Ala Gly Asp Gly Pro Pro Val Pro Ala Arg Ser Gly
        35                  40                  45
Gly Asp Ala Leu Arg Thr Pro Ala Asn Cys Gly Val Arg Leu His Ala
    50                  55                  60
Asn Asn Gly Thr Ala Leu Pro Ser Arg Thr Thr Gln Asn Lys Asp Pro
65                  70                  75                  80
Phe Ala Lys Arg Arg Val Asp Lys Phe Asp Met Ser Ser Leu Glu Trp
                85                  90                  95
Ile Asp Lys Ile Glu Glu Cys Pro Val Tyr Tyr Pro Thr Lys Glu Glu
            100                 105                 110
Phe Glu Asp Pro Ile Gly Tyr Ile Gln Lys Ile Ala Pro Val Ala Ser
        115                 120                 125
Lys Tyr Gly Ile Cys Lys Ile Val Ser Pro Val Ser Ala Ser Val Pro
    130                 135                 140
Ala Gly Val Val Leu Met Lys Glu Gln Pro Gly Phe Lys Phe Met Thr
145                 150                 155                 160
Arg Val Gln Pro Leu Arg Leu Ala Lys Trp Ala Glu Asp Asp Thr Val
                165                 170                 175
Thr Phe Phe Met Ser Glu Arg Lys Tyr Thr Phe Arg Asp Tyr Glu Lys
            180                 185                 190
Met Ala Asn Lys Val Phe Ala Lys Lys Tyr Ser Ser Ala Ser Cys Leu
        195                 200                 205
Pro Ala Lys Tyr Val Glu Glu Glu Phe Trp Arg Glu Ile Ala Phe Gly
    210                 215                 220
Lys Met Asp Phe Val Glu Tyr Ala Cys Asp Val Asp Gly Ser Ala Phe
225                 230                 235                 240
Ser Ser Ser Pro His Asp Gln Leu Gly Lys Ser Asn Trp Asn Leu Lys
                245                 250                 255
Asn Phe Ser Arg Leu Ser Asn Ser Val Leu Arg Leu Leu Gln Thr Pro
            260                 265                 270
Ile Pro Gly Val Thr Asp Pro Met Leu Tyr Ile Gly Met Leu Phe Ser
        275                 280                 285
Met Phe Ala Trp His Val Glu Asp His Tyr Leu Tyr Ser Ile Asn Tyr
    290                 295                 300
His His Cys Gly Ala Phe Lys Thr Trp Tyr Gly Ile Pro Gly Asp Ala
305                 310                 315                 320
Ala Pro Gly Phe Glu Lys Val Ala Ser Gln Phe Val Tyr Asn Lys Asp
                325                 330                 335
Ile Leu Val Gly Glu Gly Glu Asp Ala Ala Phe Asp Val Leu Leu Gly
            340                 345                 350
Lys Thr Thr Met Phe Pro Pro Asn Val Leu Leu Asp His Asn Val Pro
        355                 360                 365
Val Tyr Lys Ala Val Gln Lys Pro Gly Glu Phe Val Ile Thr Phe Pro
    370                 375                 380
Arg Ser Tyr His Ala Gly Phe Ser His Gly Phe Asn Cys Gly Glu Ala
385                 390                 395                 400
Val Asn Phe Ala Ile Ser Asp Trp Phe Pro Leu Gly Ser Val Ala Ser
                405                 410                 415
Arg Arg Tyr Ala Leu Leu Asn Arg Thr Pro Leu Leu Ala His Glu Glu
            420                 425                 430

Leu Leu Cys Arg Ser Ala Val Leu Leu Ser His Lys Leu Leu Asn Ser
        435                 440                 445
Asp Pro Lys Ser Leu Asn Lys Ser Glu His Pro His Ser Gln Arg Cys
```

```
    450                 455                 460
Leu Lys Ser Cys Phe Val Gln Leu Met Arg Phe Gln Arg Asn Thr Arg
465                 470                 475                 480
Gly Leu Leu Ala Lys Met Gly Ser Gln Ile His Tyr Lys Pro Lys Thr
                485                 490                 495
Tyr Pro Asn Leu Ser Cys Ser Met Cys Arg Arg Asp Cys Tyr Ile Thr
            500                 505                 510
His Val Leu Cys Gly Cys Asn Phe Asp Pro Val Cys Leu His His Glu
        515                 520                 525
Gln Glu Leu Arg Ser Cys Pro Cys Lys Ser Asn Gln Val Val Tyr Val
    530                 535                 540
Arg Glu Asp Ile Gln Glu Leu Glu Ala Leu Ser Arg Lys Phe Glu Lys
545                 550                 555                 560
Asp Ile Cys Leu Asp Lys Glu Ile Ser Gly Phe Asp Ser Tyr Lys Gln
                565                 570                 575
Ala Glu Lys Asn Glu Pro Phe Phe Glu Ile Thr Arg Asn Leu Arg Asn
            580                 585                 590
Thr Glu Val Asn Leu Ile Glu Asp Ala Phe Ser Gly Ala Thr Ala Ala
        595                 600                 605
Asp Ala Ala Lys Ser Ser Pro Ala Thr Ser Thr Leu Thr Ser Phe Ala
    610                 615                 620
Gln His Asp Val Pro Val Leu Ala Glu Ala Ile Val Cys Ala Asn Gln
625                 630                 635                 640
Ala Asp Gln Leu Tyr Ser Thr Thr Glu Gln Thr Ile Ser Ser Pro Leu
                645                 650                 655
Val Lys Gly Thr Asp Ala Val Gly Ala Asn Ser Ser Ser Met Ala Asp
            660                 665                 670
Ala Asn Asn Gly Thr Gly Ser Cys Asn Ala Ser Ala Val Glu Tyr Ser
        675                 680                 685
Gly Asn Ser Asp Ser Glu Ser Glu Ile Phe Arg Val Lys Arg Arg Ser
    690                 695                 700
Gly Val Ser Val Lys Pro Ala Ser Asp Ala Lys Thr Ser Asn Leu Ser
705                 710                 715                 720
Asp Gln Gln Val Leu Arg Arg Leu Lys Lys Val Arg Pro Glu Ile Gln
                725                 730                 735
Gln His Asn Lys Arg Pro Glu Asp Tyr Gly His Cys Ser Val Pro Ser
            740                 745                 750
Gly Arg Met Ser Met Lys Asn Leu Asn Ser Ser Ser Ser Cys Gly Glu
        755                 760                 765
Glu His Trp Arg Met Lys Arg Arg Gln Leu Glu Thr Gln Gln Asp Glu
    770                 775                 780
Ser Ser Tyr Ser Ala Lys Gln Lys Ser Tyr Ser Tyr Pro Ser Thr Ser
785                 790                 795                 800
Tyr Ser Phe Arg Gly Glu Phe Val Glu Met Ser Arg Asp Ala Ala Ala
                805                 810                 815
Glu Val Arg Pro Lys Arg Leu Lys Ile Arg Leu Pro Ser Ser Ser Thr
            820                 825                 830
Asn Arg Val Val Glu Gln Gly Ser Ser Gly Gln Arg Phe Thr Arg Asp
        835                 840                 845
Asp Lys Ser Leu Gly Cys Trp Pro Ala Ile
    850                 855
```

<210> 163
<211> 1566
<212> DNA
<213> Oryza sativa

<400> 163

```
atgccgagcg ccttccactc cctcctcctc cccgccattc gcaaccccaa acctagccgt      60
cgccgcggcc gcggccgcgg cggcagcaaa cgccccaaga agaccaccaa atccaagaac     120
cgcctcgccg acgccgccgc cggagacgcc accgccttcc acctgaagac ctccgcgcgc     180
```

```
gccggtccgg ggggtgccgg gagcggccgg cgaggcgatg ggggatgcct cgtgcagccg    240
ctcggcaacc tcctcctcct cggcggcggc ggcaacctcc gcgacgcggg gctcggcgcg    300
ctccgcccgc tccccgacga cgtcctcctc gacgtgctcg gcctgctcgc ggcgcgcgac    360
ctcgctaggc tctccgcggc gtcgagggcg ctctacgtcg tcgcctccca cgacccgctc    420
tggcgggcgc tcgtcctcga cgagctcggc ggggacttcg ccttctcggg ctcgtggcgc    480
gccacctaca tcgccgccgc gtcgggcggc cgcgcccacc tccccccgcg gggcctagag    540
atcagggggt tctactccga ctacctcttc cagagctggc tctgcgccaa catggagatg    600
cggccggagt ggctccaccg ggacaccatc gatcgccgcc gcggcatgtc cgtcgagcaa    660
ttcgtctccg aattcgagga gcccaatagg ccggtgcttc tggaaggctg cctcgagagc    720
tggccagcat tgcagaagtg gaccagggag cacttgctga aggtctcggc cgggaaggag    780
ttcgccgtcg ggccggtgag catgacgctg gataggtacc tccagtatgc cgacaatgtg    840
caggaggaga ggccattgta cctgttcgat gccaagttca ccgagaaggt gccggagatg    900
gggagggact atgaggtgcc ggcgtacttc cgagaggacc tattcggggt gcttggggag    960
gaaaggccgg accaccgatg ggttatcatt gggccggcag gttcagggtc gtcgtttcat   1020
gttgatccaa actcgacatc ggcgtggaat gctgtgatca agggagccaa gaagtgggtg   1080
atgttcccac cggaggtggt gccgccagga gttcatccaa gtgcggatgg agcagaagtc   1140
actagccctg tatctatcat ggaatggttc atgaattttt atggggcatg taagacctgg   1200
gaaaagaggc ctgttgagtg tatatgccgg gctggagagg tggtttttgt gcctaatgga   1260
tggtggcatt tggttatcaa tctggaggaa tctattgcga ttactcagaa ttatgtgagc   1320
aggaggaatc tgttgaatgt tcttgacttt ctcaagaggc ccaatgcaag tgaacttgta   1380
tcagggacta cagacagggt aaacctgcat gacaagttcc gcaatgccat cgatatgact   1440
tatcctggga tgattaaaca gcttgaactt gaagctcagc agaaggctgc tgctcgcaag   1500
aagaaagtct cgttctggga atctgcggta gatgccaata ctggaggatt caagttctca   1560
ttctga                                                              1566
```

<210> 164
<211> 521
<212> PRT
<213> Oryza sativa

<400> 164

```
Met Pro Ser Ala Phe His Ser Leu Leu Leu Pro Ala Ile Arg Asn Pro
1               5                   10                  15
Lys Pro Ser Arg Arg Arg Gly Arg Gly Arg Gly Gly Ser Lys Arg Pro
            20                  25                  30
Lys Lys Thr Thr Lys Ser Lys Asn Arg Leu Ala Asp Ala Ala Ala Gly
        35                  40                  45
Asp Ala Thr Ala Phe His Leu Lys Thr Ser Ala Arg Ala Gly Pro Gly
    50                  55                  60
Gly Ala Gly Ser Gly Arg Arg Gly Asp Gly Gly Cys Leu Val Gln Pro
65                  70                  75                  80
Leu Gly Asn Leu Leu Leu Leu Gly Gly Gly Gly Asn Leu Arg Asp Ala
                85                  90                  95
Gly Leu Gly Ala Leu Arg Pro Leu Pro Asp Asp Val Leu Leu Asp Val
            100                 105                 110
Leu Gly Leu Leu Ala Ala Arg Asp Leu Ala Arg Leu Ser Ala Ala Ser
        115                 120                 125
Arg Ala Leu Tyr Val Val Ala Ser His Asp Pro Leu Trp Arg Ala Leu
    130                 135                 140
Val Leu Asp Glu Leu Gly Gly Asp Phe Ala Phe Ser Gly Ser Trp Arg
145                 150                 155                 160
Ala Thr Tyr Ile Ala Ala Ala Ser Gly Gly Arg Ala His Leu Pro Pro
                165                 170                 175
Arg Gly Leu Glu Ile Arg Gly Phe Tyr Ser Asp Tyr Leu Phe Gln Ser
            180                 185                 190
Trp Leu Cys Ala Asn Met Glu Met Arg Pro Glu Trp Leu His Arg Asp
        195                 200                 205
Thr Ile Asp Arg Arg Arg Gly Met Ser Val Glu Gln Phe Val Ser Glu
    210                 215                 220
Phe Glu Glu Pro Asn Arg Pro Val Leu Leu Glu Gly Cys Leu Glu Ser
225                 230                 235                 240
```

```
Trp Pro Ala Leu Gln Lys Trp Thr Arg Glu His Leu Leu Lys Val Ser
                245                 250                 255
Ala Gly Lys Glu Phe Ala Val Gly Pro Val Ser Met Thr Leu Asp Arg
            260                 265                 270
Tyr Leu Gln Tyr Ala Asp Asn Val Gln Glu Glu Arg Pro Leu Tyr Leu
        275                 280                 285
Phe Asp Ala Lys Phe Thr Glu Lys Val Pro Glu Met Gly Arg Asp Tyr
    290                 295                 300
Glu Val Pro Ala Tyr Phe Arg Glu Asp Leu Phe Gly Val Leu Gly Glu
305                 310                 315                 320
Glu Arg Pro Asp His Arg Trp Val Ile Ile Gly Pro Ala Gly Ser Gly
                325                 330                 335
Ser Ser Phe His Val Asp Pro Asn Ser Thr Ser Ala Trp Asn Ala Val
            340                 345                 350
Ile Lys Gly Ala Lys Lys Trp Val Met Phe Pro Pro Glu Val Val Pro
        355                 360                 365
Pro Gly Val His Pro Ser Ala Asp Gly Ala Glu Val Thr Ser Pro Val
    370                 375                 380
Ser Ile Met Glu Trp Phe Met Asn Phe Tyr Gly Ala Cys Lys Thr Trp
385                 390                 395                 400
Glu Lys Arg Pro Val Glu Cys Ile Cys Arg Ala Gly Glu Val Val Phe
                405                 410                 415
Val Pro Asn Gly Trp Trp His Leu Val Ile Asn Leu Glu Glu Ser Ile
            420                 425                 430
Ala Ile Thr Gln Asn Tyr Val Ser Arg Arg Asn Leu Leu Asn Val Leu
        435                 440                 445
Asp Phe Leu Lys Arg Pro Asn Ala Ser Glu Leu Val Ser Gly Thr Thr
    450                 455                 460
Asp Arg Val Asn Leu His Asp Lys Phe Arg Asn Ala Ile Asp Met Thr
465                 470                 475                 480
Tyr Pro Gly Met Ile Lys Gln Leu Glu Leu Glu Ala Gln Gln Lys Ala
                485                 490                 495
Ala Ala Arg Lys Lys Lys Val Ser Phe Trp Glu Ser Ala Val Asp Ala
            500                 505                 510
Asn Thr Gly Gly Phe Lys Phe Ser Phe
        515                 520
```

<210> 165
<211> 432
<212> DNA
<213> Oryza sativa

<400> 165

```
atggtggcag acagcgcggt cggcgtgcac ggcagtgcgg ccgacagtgg gtcggtgcgg     60
tcggcgcgga cggcggcaga gagggacgcg gaggcggagg ctacgcggga cacaaaggca    120
gcggcggtgg agccgccaga gtggctgcag accctgcccg tggcgccgga gtaccacccg    180
acgctggtgg agttcgccga ccccatcgcc tacatcctca ggatcaagcc cgaggcgtcc    240
cgcaacggca tttgcaagat ccaggtcggc ctctccacca agaaccgccg tgccgccagc    300
cgccgagtgt gggagagcgg ggagcgctac accctggagg cgttctgcgc caaggtgccc    360
gagttcgagc cctcgaggca cgccgcactg cccaagaacc ccacccacct ccagctcaag    420
gccctcttct ag                                                        432
```

<210> 166
<211> 143
<212> PRT
<213> Oryza sativa

<400> 166

```
Met Val Ala Asp Ser Ala Val Gly Val His Gly Ser Ala Ala Asp Ser
1               5                   10                  15
Gly Ser Val Arg Ser Ala Arg Thr Ala Ala Glu Arg Asp Ala Glu Ala


            20                  25                  30
Glu Ala Thr Arg Asp Thr Lys Ala Ala Ala Val Glu Pro Pro Glu Trp
        35                  40                  45
Leu Gln Thr Leu Pro Val Ala Pro Glu Tyr His Pro Thr Leu Val Glu
    50                  55                  60
Phe Ala Asp Pro Ile Ala Tyr Ile Leu Arg Ile Lys Pro Glu Ala Ser
65                  70                  75                  80
Arg Asn Gly Ile Cys Lys Ile Gln Val Gly Leu Ser Thr Lys Asn Arg
                85                  90                  95
Arg Ala Ala Ser Arg Arg Val Trp Glu Ser Gly Glu Arg Tyr Thr Leu
            100                 105                 110
Glu Ala Phe Cys Ala Lys Val Pro Glu Phe Glu Pro Ser Arg His Ala
        115                 120                 125
Ala Leu Pro Lys Asn Pro Thr His Leu Gln Leu Lys Ala Leu Phe
    130                 135                 140
```

<210> 167
<211> 4101
<212> DNA
<213> Oryza sativa

<400> 167

```
atgtcgctgc agccgccggc ggtggagccg ccggagtggc tgcggacgct gcccgtggcg      60
ccggagtacc acccgacgct ggcggagttc gccgacccca tcgcctacat cctcaggatc     120
gagccggagg cgtcccgcta cggcatctgc aagatcgtgc cccgctccc gcggcccccc      180
gaggacgaca ccttccgccg cctccaggcc gccttcgccg ccgcggcctc ctccaatggc     240
gacccctccc cgaccttccc cacgcggctc cagcaggtcg gcctctccgc caggaaccgc     300
cgcgccgcca gccgccgggt gtgggagagc ggggagcgct acaccctgga ggcgttccgc     360
gccaaggcgg ccgagttcga gcccccgagg cacgccgcgc cgcccaggaa ccccacccac     420
ctccagctcg aggccctctt ctgggccgcc tgcgcctcca ggcccttcag cgtcgagtac     480
ggcaacgaca tgcccggctc cggcttcgcc tcccccgacg agctccccga cgccgccaat     540
gccaccgacg tcggggagac ggagtggaac atgcgggtgg cgccccgcgc ccgggggtcg     600
ctgctccgcg ccatggcccg cgacgtggcg ggcgtcacca ccccgatgct gtacgtggcc     660
atgctctaca gctggttcgc ctggcacgtg gaggaccacg agctccacag cctcaacttc     720
ctccacttcg gcaaggccaa gacctggtac ggcgtccccc gcgacgccat gctcgccttc     780
gaggagacgg tgcgcgtcca cggctacgcc gacgacctca acgccatcat ggcctttcaa     840
acgttaaatg agaagacaac tgtcttatct cctgaggtgc ttctttctgc tggtgttccc     900
tgctgcagat tggttcaaaa agcgggagaa tttgttatca cattccctgg agcttatcat     960
tcaggcttta gccatggatt taattgtggg gaagcatcaa atattgcaac tccccattgg    1020
ttacaagtgg ctaaagaagc tgcaatccgg agggcttcaa ctaattgtgg tccgatggtg    1080
tctcattatc agctgcttta tgagctagca ctctcattgc gtccaaggga gcctaagaat    1140
ttctattctg ttccaagaag ctcacgttta agggacaaga ataagaatga aggtgatata    1200
atggtcaaag aaaattttgt tggaagtgta accgaaaaca acaatttgct tagcgccctt    1260
ctggataaga attcttgtat aattgttcca aacgctgatt tcttcgttcc atcctttcct    1320
gttgcactgg aatctgaagt tactgttaaa caaaggttta cagctggtcc ctgcagtatt    1380
agccagcaag gagcagaaaa tatggctgct gatcatgtgg ctgtagacaa ggttacagag    1440
attcaggaca tgagtggatc cttatatcct tgtgaaacca gtcttgtggg ctgtagcaat    1500
agaaaacttt atgaaacaaa atatggccaa cgggatgctg ctgctttgtg cttatctact    1560
tcagagattc agagcagagg aattgataca gcaagatcac atccagcagg cgggatctta    1620
gatcaaggac ggttgccatg tgtacagtgc ggaatactaa gctttgcatg tgtagccatc    1680
attcaaccta gagaagcagc agtacagttt atcatgtcta aagagtgcat ctcatcgagt    1740
gcaaaacaag gaggaattgg tgcatctgat gatacctcaa actggattga ccagagtcat    1800
gagattagtc ctccaccagg tccagcatct ggaacagatg ataatgtaaa gcatgccgta    1860
agtttggccc atgtctctga tcggtgtaga gaactatatg ccagcaatac agatggatgc    1920
acctctgctc ttgggcttct agcttctgca tatgactcat cagactctga tgatgaaaca    1980
actgaggatg tttcaaaaca tagcaagaaa aatgattcag taaaccaaag cacggatcct    2040
caaatcttgg aaacatcagc tagctgttcc agtacagttc agtgtcaaaa gacaaattca    2100
cacttgcatg aagaggaatg tgaggcaaga gccacatcat tgatgaaacc tgtaagccat    2160
aatagtaggc ccattagtca gtctaacagg gatacagata tcgaccattt tatagaactg    2220
ggaaagtcag gaacacagtg ttcaggttat cttgatttgg ttgatgatct aactacatca    2280
gttttaaaat cctcttcaga tacttgtgta agtgcagcta aagcctcaat ggatccagat    2340
```

```
gttttgacca tgctcaggta caataaagac tcttgcagaa tgcatgtgtt ttgtcttgaa   2400
catgctttgg aaacatggac acagcttcaa caaatcggtg gtgctaatat tatgcttttg   2460
tgccatccag aataccctag agcagagtca gcagcaaaag tcatagcaga agaacttggt   2520
atcaagcatg attggaaaga tatcactttc aaagaagcaa ctgaagagga cgttaaaaag   2580
attcagttgg ctttacagga tgaagatgct gaacccactg gcagtgactg ggcagtcaaa   2640
atgggtatca acatatatta cagtgccaaa cagagcaaat caccacttta cagcaagcag   2700
ataccataca attccatcat ttataaggca tttggccaag aaaacccaga cagtttgaca   2760
gattatggat gtcaaaagtc aggctcaaca aagaaaaagg tggctgggtg gtggtgtggg   2820
aaagtttgga tgtcaaatca agttcatcca cttctagctc gtgagcgtga agaacagaac   2880
agtagcgtag tgtacggcaa agcaatgttc actactattt cccatggcaa agtacaagat   2940
gaagcgtcaa caagatgcaa tacaagcaac cgaaccccat caagaagaac atcaagaaga   3000
aaaaaggggg tatctgctga gaaatctaaa ccaaaaaaca agagatccac tgcttctgat   3060
gaagctagta tgctttgcag tggccttgga atgaactctg gagttatcca tgaccaaact   3120
gaaaactctg atgattatga caaacatggc aatggagatg aaattgagga aggaacaaat   3180
cctcagaaat atcaacaacg taaattgcaa aacgtgacca ggaaatcaag ttctaagaag   3240
cggaaggatg agaaaagaac agatagtttt catgaactat atgacgagga taatggtgtg   3300
gattattggc ttaacatggg tagtggagat gatgccacac taggtaactc tcgacaacaa   3360
agtcctgatc cagtgaaagt gaaatctgga ggcaaattgc aaggtaagag aaaatctagc   3420
aagtacaagt ccaatgatga tttgttgaat gaagaaaata agttacaaaa gatgaacaaa   3480
aaatcaagct ctaagaagca aaagaatgat aagataaata gacaacttca agaagatcaa   3540
accgaagatg accatatgga ccacttagtt gacgtagcgg ttgcagatga agtcacacta   3600
gacaatgagg ataaaattac agaagacaaa attgatgacg tgaaagtgaa atctagagga   3660
aaatcgcaaa atggtaagag aaaaggcagc aaacatcagg ctaccgatgg tttgcgcgct   3720
ggaaataaag tggccaaatt tccgtgtgat atagaaggat gtgacatgag cttcagtacc   3780
cagcaggact tgttattgca taagcgtgac atttgtcctg tcaaaggatg caagaagaag   3840
ttcttctgcc acaagtactt gcttcagcac cggaaggtgc atatagatga aaggccactt   3900
aagtgtacat ggaaggggtg taagaaggca ttcaagtggc catgggcgag gacggagcac   3960
atgagagtcc atacgggggt aagaccttac gagtgccagg aacctggctg tggtcagaca   4020
ttccgattcg tttcagattt cagccgccac aagagaaaga ctggccattc ctctgataag   4080
agaagaaaga acagtacata a                                            4101
```

<210> 168
<211> 1366
<212> PRT
<213> Oryza sativa

<400> 168

```
Met Ser Leu Gln Pro Pro Ala Val Glu Pro Pro Glu Trp Leu Arg Thr
1               5                   10                  15
Leu Pro Val Ala Pro Glu Tyr His Pro Thr Leu Ala Glu Phe Ala Asp
            20                  25                  30
Pro Ile Ala Tyr Ile Leu Arg Ile Glu Pro Glu Ala Ser Arg Tyr Gly
        35                  40                  45
Ile Cys Lys Ile Val Pro Pro Leu Pro Arg Pro Pro Glu Asp Asp Thr
    50                  55                  60
Phe Arg Arg Leu Gln Ala Ala Phe Ala Ala Ala Ala Ser Ser Asn Gly
65                  70                  75                  80
Asp Pro Ser Pro Thr Phe Pro Thr Arg Leu Gln Gln Val Gly Leu Ser
                85                  90                  95
Ala Arg Asn Arg Arg Ala Ala Ser Arg Arg Val Trp Glu Ser Gly Glu
            100                 105                 110
Arg Tyr Thr Leu Glu Ala Phe Arg Ala Lys Ala Ala Glu Phe Glu Pro
        115                 120                 125
Pro Arg His Ala Ala Pro Pro Arg Asn Pro Thr His Leu Gln Leu Glu
    130                 135                 140
Ala Leu Phe Trp Ala Ala Cys Ala Ser Arg Pro Phe Ser Val Glu Tyr
145                 150                 155                 160
Gly Asn Asp Met Pro Gly Ser Gly Phe Ala Ser Pro Asp Glu Leu Pro
                165                 170                 175
Asp Ala Ala Asn Ala Thr Asp Val Gly Glu Thr Glu Trp Asn Met Arg
            180                 185                 190
```

```
Val Ala Pro Arg Ala Arg Gly Ser Leu Leu Arg Ala Met Ala Arg Asp
        195                 200                 205
Val Ala Gly Val Thr Thr Pro Met Leu Tyr Val Ala Met Leu Tyr Ser
    210                 215                 220
Trp Phe Ala Trp His Val Glu Asp His Glu Leu His Ser Leu Asn Phe
225                 230                 235                 240
Leu His Phe Gly Lys Ala Lys Thr Trp Tyr Gly Val Pro Arg Asp Ala
                245                 250                 255
Met Leu Ala Phe Glu Glu Thr Val Arg Val His Gly Tyr Ala Asp Asp
            260                 265                 270
Leu Asn Ala Ile Met Ala Phe Gln Thr Leu Asn Glu Lys Thr Thr Val
        275                 280                 285
Leu Ser Pro Glu Val Leu Leu Ser Ala Gly Val Pro Cys Cys Arg Leu
    290                 295                 300
Val Gln Lys Ala Gly Glu Phe Val Ile Thr Phe Pro Gly Ala Tyr His
305                 310                 315                 320
Ser Gly Phe Ser His Gly Phe Asn Cys Gly Glu Ala Ser Asn Ile Ala
                325                 330                 335
Thr Pro His Trp Leu Gln Val Ala Lys Glu Ala Ala Ile Arg Arg Ala
            340                 345                 350
Ser Thr Asn Cys Gly Pro Met Val Ser His Tyr Gln Leu Leu Tyr Glu
        355                 360                 365
Leu Ala Leu Ser Leu Arg Pro Arg Glu Pro Lys Asn Phe Tyr Ser Val
    370                 375                 380
Pro Arg Ser Ser Arg Leu Arg Asp Lys Asn Lys Asn Glu Gly Asp Ile
385                 390                 395                 400
Met Val Lys Glu Asn Phe Val Gly Ser Val Thr Glu Asn Asn Asn Leu
                405                 410                 415
Leu Ser Ala Leu Leu Asp Lys Asn Ser Cys Ile Ile Val Pro Asn Ala
            420                 425                 430
Asp Phe Phe Val Pro Ser Phe Pro Val Ala Leu Glu Ser Glu Val Thr
        435                 440                 445
Val Lys Gln Arg Phe Thr Ala Gly Pro Cys Ser Ile Ser Gln Gln Gly
    450                 455                 460
Ala Glu Asn Met Ala Ala Asp His Val Ala Val Asp Lys Val Thr Glu
465                 470                 475                 480
Ile Gln Asp Met Ser Gly Ser Leu Tyr Pro Cys Glu Thr Ser Leu Val
                485                 490                 495
Gly Cys Ser Asn Arg Lys Leu Tyr Glu Thr Lys Tyr Gly Gln Arg Asp
            500                 505                 510
Ala Ala Ala Leu Cys Leu Ser Thr Ser Glu Ile Gln Ser Arg Gly Ile
        515                 520                 525
Asp Thr Ala Arg Ser His Pro Ala Gly Gly Ile Leu Asp Gln Gly Arg
    530                 535                 540
Leu Pro Cys Val Gln Cys Gly Ile Leu Ser Phe Ala Cys Val Ala Ile
545                 550                 555                 560
Ile Gln Pro Arg Glu Ala Ala Val Gln Phe Ile Met Ser Lys Glu Cys
                565                 570                 575
Ile Ser Ser Ser Ala Lys Gln Gly Gly Ile Gly Ala Ser Asp Asp Thr
            580                 585                 590
Ser Asn Trp Ile Asp Gln Ser His Glu Ile Ser Pro Pro Pro Gly Pro
        595                 600                 605
Ala Ser Gly Thr Asp Asp Asn Val Lys His Ala Val Ser Leu Ala His
    610                 615                 620
Val Ser Asp Arg Cys Arg Glu Leu Tyr Ala Ser Asn Thr Asp Gly Cys
625                 630                 635                 640
Thr Ser Ala Leu Gly Leu Leu Ala Ser Ala Tyr Asp Ser Ser Asp Ser
                645                 650                 655
Asp Asp Glu Thr Thr Glu Asp Val Ser Lys His Ser Lys Lys Asn Asp
            660                 665                 670
Ser Val Asn Gln Ser Thr Asp Pro Gln Ile Leu Glu Thr Ser Ala Ser
```

```
        675                 680                 685
Cys Ser Ser Thr Val Gln Cys Gln Lys Thr Asn Ser His Leu His Glu
    690                 695                 700
Glu Glu Cys Glu Ala Arg Ala Thr Ser Leu Met Lys Pro Val Ser His
705                 710                 715                 720
Asn Ser Arg Pro Ile Ser Gln Ser Asn Arg Asp Thr Asp Ile Asp His
                725                 730                 735
Phe Ile Glu Leu Gly Lys Ser Gly Thr Gln Cys Ser Gly Tyr Leu Asp
            740                 745                 750
Leu Val Asp Asp Leu Thr Thr Ser Val Leu Lys Ser Ser Ser Asp Thr
        755                 760                 765
Cys Val Ser Ala Ala Lys Ala Ser Met Asp Pro Asp Val Leu Thr Met
    770                 775                 780
Leu Arg Tyr Asn Lys Asp Ser Cys Arg Met His Val Phe Cys Leu Glu
785                 790                 795                 800
His Ala Leu Glu Thr Trp Thr Gln Leu Gln Gln Ile Gly Gly Ala Asn
                805                 810                 815
Ile Met Leu Leu Cys His Pro Glu Tyr Pro Arg Ala Glu Ser Ala Ala
            820                 825                 830
Lys Val Ile Ala Glu Glu Leu Gly Ile Lys His Asp Trp Lys Asp Ile
        835                 840                 845
Thr Phe Lys Glu Ala Thr Glu Glu Asp Val Lys Lys Ile Gln Leu Ala
    850                 855                 860
Leu Gln Asp Glu Asp Ala Glu Pro Thr Gly Ser Asp Trp Ala Val Lys
865                 870                 875                 880
Met Gly Ile Asn Ile Tyr Tyr Ser Ala Lys Gln Ser Lys Ser Pro Leu
                885                 890                 895
Tyr Ser Lys Gln Ile Pro Tyr Asn Ser Ile Ile Tyr Lys Ala Phe Gly
            900                 905                 910
Gln Glu Asn Pro Asp Ser Leu Thr Asp Tyr Gly Cys Gln Lys Ser Gly
        915                 920                 925
Ser Thr Lys Lys Lys Val Ala Gly Trp Trp Cys Gly Lys Val Trp Met
    930                 935                 940
Ser Asn Gln Val His Pro Leu Leu Ala Arg Glu Arg Glu Glu Gln Asn
945                 950                 955                 960
Ser Ser Val Val Tyr Gly Lys Ala Met Phe Thr Thr Ile Ser His Gly
                965                 970                 975
Lys Val Gln Asp Glu Ala Ser Thr Arg Cys Asn Thr Ser Asn Arg Thr
            980                 985                 990
Pro Ser Arg Arg Thr Ser Arg Arg  Lys Lys Gly Val Ser  Ala Glu Lys
        995                 1000                1005
Ser Lys  Pro Lys Asn Lys Arg  Ser Thr Ala Ser Asp  Glu Ala Ser
    1010                 1015                1020
Met Leu  Cys Ser Gly Leu Gly  Met Asn Ser Gly Val  Ile His Asp
    1025                 1030                1035
Gln Thr  Glu Asn Ser Asp Asp  Tyr Asp Lys His Gly  Asn Gly Asp
    1040                 1045                1050
Glu Ile  Glu Glu Gly Thr Asn  Pro Gln Lys Tyr Gln  Gln Arg Lys
    1055                 1060                1065
Leu Gln  Asn Val Thr Arg Lys  Ser Ser Ser Lys Lys  Arg Lys Asp
    1070                 1075                1080
Glu Lys  Arg Thr Asp Ser Phe  His Glu Leu Tyr Asp  Glu Asp Asn
    1085                 1090                1095
Gly Val  Asp Tyr Trp Leu Asn  Met Gly Ser Gly Asp  Asp Ala Thr
    1100                 1105                1110
Leu Gly  Asn Ser Arg Gln Gln  Ser Pro Asp Pro Val  Lys Val Lys
    1115                 1120                1125
Ser Gly  Gly Lys Leu Gln Gly  Lys Arg Lys Ser Ser  Lys Tyr Lys
    1130                 1135                1140
Ser Asn  Asp Asp Leu Leu Asn  Glu Glu Asn Lys Leu  Gln Lys Met
    1145                 1150                1155
```

```
Asn Lys  Lys Ser Ser Ser Lys  Lys Gln Lys Asn Asp  Lys Ile Asn
    1160                 1165                 1170
Arg Gln  Leu Gln Glu Asp Gln  Thr Glu Asp Asp His  Met Asp His
    1175                 1180                 1185
Leu Val  Asp Val Ala Val Ala  Asp Glu Val Thr Leu  Asp Asn Glu
    1190                 1195                 1200
Asp Lys  Ile Thr Glu Asp Lys  Ile Asp Asp Val Lys  Val Lys Ser
    1205                 1210                 1215
Arg Gly  Lys Ser Gln Asn Gly  Lys Arg Lys Gly Ser  Lys His Gln
    1220                 1225                 1230
Ala Thr  Asp Gly Leu Arg Ala  Gly Asn Lys Val Ala  Lys Phe Pro
    1235                 1240                 1245
Cys Asp  Ile Glu Gly Cys Asp  Met Ser Phe Ser Thr  Gln Gln Asp
    1250                 1255                 1260
Leu Leu  Leu His Lys Arg Asp  Ile Cys Pro Val Lys  Gly Cys Lys
    1265                 1270                 1275
Lys Lys  Phe Phe Cys His Lys  Tyr Leu Leu Gln His  Arg Lys Val
    1280                 1285                 1290
His Ile  Asp Glu Arg Pro Leu  Lys Cys Thr Trp Lys  Gly Cys Lys
    1295                 1300                 1305
Lys Ala  Phe Lys Trp Pro Trp  Ala Arg Thr Glu His  Met Arg Val
    1310                 1315                 1320
His Thr  Gly Val Arg Pro Tyr  Glu Cys Gln Glu Pro  Gly Cys Gly
    1325                 1330                 1335
Gln Thr  Phe Arg Phe Val Ser  Asp Phe Ser Arg His  Lys Arg Lys
    1340                 1345                 1350
Thr Gly  His Ser Ser Asp Lys  Arg Arg Lys Asn Ser  Thr
    1355                 1360                 1365
```

<210> 169
<211> 1242
<212> DNA
<213> Glycine max

<400> 169

```
atgttgacga caactgtatc cggcggcgac cctccttctc gcggttttga tacgccgacg     60
ctggaccggg aagcggcggc gctgctccac gcgatctccg agcatggcgg gtacgcgtac    120
gtgagcatgg cggtgctggc ttccggcggc gacattcgcg cggcggaggc ggcgtgggag    180
atggcgtggg agcagctgca ctcgggtccg tggcactcgg tgctgccggt gtggcgcgac    240
gcttactcca tggcgtgcct ccacgtggcg cgccaccact acggcaacgg tgagttcttg    300
gacgcactta gggttttgga tttgggaatc atcatgggag gcacgctcct ccgcaaggat    360
ttggactccg ccatcgagaa aatgtcggaa caaacgagga agaccgttag ggtttctgat    420
ttggggaact ccgagcaccg actcgtcgat cgcgaatttg atatggcaga ggtgctccaa    480
cttttacctg tgaagtctct ttcaacgaaa cttgtggtga agaaatcggc gctgtccttg    540
gagaaattcc tgaaggatca ttacctgtct ggctgcccgg ttattatcag tgattgtatg    600
tctcactggc cagccaagat gaaatggaat gacgaagatt acttgctgag agttgccgga    660
gaccgtacag ttccagttga ggttgggaaa aactatttat gtactgagtg gaagcaagag    720
ctaattactt tttcagagtt tcttcagcgg ataaagtctg atagctgttc tcctggtggt    780
cctacatatc ttgctcagca tccattattt gatcagataa atgagcttcg gaaagatatc    840
tttattcctg actattgttt tactggtgga ggggagctac gatctctcaa tgcttggttt    900
ggtccagcag gaacagtaac accgttacac catgatccac atcataacat actagctcag    960
gttgttggaa agaaatacat taggctatac tcttcgtctt tatctgagga actttccccc   1020
cactctggta ccatgctcca caactccagc caggttgatt tagatgatat ggatgaaaag   1080
aagtttccga aggtgcaaga cttggaattt gtagactgta ttttagagga aggcgaaatg   1140
ttatatatcc cgccaaaatg gtggcactat gtgcggtctt tgactaccag tttttcggtt   1200
agcttttggt ggagtgaggg tgaaagttcg gatgcatcct aa                      1242
```

<210> 170
<211> 413
<212> PRT

<213> Glycine max

```
<400>  170
Met Leu Thr Thr Thr Val Ser Gly Gly Asp Pro Pro Ser Arg Gly Phe
1               5                   10                  15
Asp Thr Pro Thr Leu Asp Arg Glu Ala Ala Ala Leu Leu His Ala Ile
            20                  25                  30
Ser Glu His Gly Gly Tyr Ala Tyr Val Ser Met Ala Val Leu Ala Ser
        35                  40                  45
Gly Gly Asp Ile Arg Ala Ala Glu Ala Ala Trp Glu Met Ala Trp Glu
    50                  55                  60
Gln Leu His Ser Gly Pro Trp His Ser Val Leu Pro Val Trp Arg Asp
65                  70                  75                  80
Ala Tyr Ser Met Ala Cys Leu His Val Ala Arg His His Tyr Gly Asn
                85                  90                  95
Gly Glu Phe Leu Asp Ala Leu Arg Val Leu Asp Leu Gly Ile Ile Met
            100                 105                 110
Gly Gly Thr Leu Leu Arg Lys Asp Leu Asp Ser Ala Ile Glu Lys Met
        115                 120                 125
Ser Glu Gln Thr Arg Lys Thr Val Arg Val Ser Asp Leu Gly Asn Ser
    130                 135                 140
Glu His Arg Leu Val Asp Arg Glu Phe Asp Met Ala Glu Val Leu Gln
145                 150                 155                 160
Leu Leu Pro Val Lys Ser Leu Ser Thr Lys Leu Val Val Lys Lys Ser
                165                 170                 175
Ala Leu Ser Leu Glu Lys Phe Leu Lys Asp His Tyr Leu Ser Gly Cys
            180                 185                 190
Pro Val Ile Ile Ser Asp Cys Met Ser His Trp Pro Ala Lys Met Lys
        195                 200                 205
Trp Asn Asp Glu Asp Tyr Leu Leu Arg Val Ala Gly Asp Arg Thr Val
    210                 215                 220
Pro Val Glu Val Gly Lys Asn Tyr Leu Cys Thr Glu Trp Lys Gln Glu
225                 230                 235                 240
Leu Ile Thr Phe Ser Glu Phe Leu Gln Arg Ile Lys Ser Asp Ser Cys
                245                 250                 255
Ser Pro Gly Gly Pro Thr Tyr Leu Ala Gln His Pro Leu Phe Asp Gln
            260                 265                 270
Ile Asn Glu Leu Arg Lys Asp Ile Phe Ile Pro Asp Tyr Cys Phe Thr
        275                 280                 285
Gly Gly Gly Glu Leu Arg Ser Leu Asn Ala Trp Phe Gly Pro Ala Gly
    290                 295                 300
Thr Val Thr Pro Leu His His Asp Pro His His Asn Ile Leu Ala Gln
305                 310                 315                 320
Val Val Gly Lys Lys Tyr Ile Arg Leu Tyr Ser Ser Ser Leu Ser Glu
                325                 330                 335
Glu Leu Ser Pro His Ser Gly Thr Met Leu His Asn Ser Ser Gln Val
            340                 345                 350
Asp Leu Asp Asp Met Asp Glu Lys Lys Phe Pro Lys Val Gln Asp Leu
        355                 360                 365
Glu Phe Val Asp Cys Ile Leu Glu Glu Gly Glu Met Leu Tyr Ile Pro
    370                 375                 380
Pro Lys Trp Trp His Tyr Val Arg Ser Leu Thr Thr Ser Phe Ser Val
385                 390                 395                 400
Ser Phe Trp Trp Ser Glu Gly Glu Ser Ser Asp Ala Ser
                405                 410
```

<210> 171
<211> 1464
<212> DNA
<213> Arabidopsis thaliana

<400> 171

```
cttagtcaat agcatcttct ccgatcatcg acgtccggag atttcttcgg ccctctcata     60
cttgagttgt tgtaggattt gttgctctgg ctctggtggt aggtctatga aatcaaccca    120
tatcgtgaat ggactgcaac atggtatctt cgtcccagtg ggattgggag catttgatca    180
tgtccaatcc gtcaaggact gaagatgaca gcaaacagct acctactgag tgggaaattg    240
aaaaaggtga aggaattgaa tctatagttc cacatttctc aggccttgag agagtcagta    300
gtggctctgc caccagcttc tggcacactg ctgtatcgaa aagctcacag tcgacctcta    360
tcaactcatc atctcccgaa gccaaacgat gcaagcttgc atcagaaagt tcccctggag    420
attcttgcag caacatagac tttgtccagg tgaaggctcc cacagctctc gaggtatccg    480
ttgcctcagc tgaatcagat ctttgtttaa aactaggaaa gcggacatac tctgaagaat    540
actggggtag aaacaataat gaaatttcag cggtttctat gaagttgtta actccatctg    600
ttgtcgctgg gaaatccaaa ttgtgtggtc agagcatgcc agtcccgcgt tgccaaattg    660

atggctgtga actggatctc tcatctgcta agggttatca tcgtaagcac aaagtctgcg    720
aaaagcattc aaagtgccca aaagttagcg tgagtggcct ggaacgtcgg ttctgccaac    780
agtgtagcag gttccatgct gtctctgaat ttgatgagaa gaaacgaagc tgccgaaaac    840
gtctttctca tcataatgcg aggcgtcgta agccacaagg agtattttca atgaatcccg    900
agagggtgta tgatcgaaga cagcatacaa atatgttgtg gaatggggtg tcccttaacg    960
cgagatctga agaaatgtat gaatggggta ataacactta tgatacaaag cctagacaaa   1020
cggaaaaaag ctttactctg agcttccaga gaggtaatgg ctctgaggac cagctggttg   1080
ctagtagcag ccgtatgttc tctacatctc aaacctcagg tgggttccca gcaggaaagt   1140
ccaagtttca acttcatggc gaagatgtgg gagaatactc aggagtcctc catgaatctc   1200
aagatatcca ccgtgctctc tctcttctgt caacctcttc ggatcccctg gcccaaccac   1260
atgtgcagcc attttctcta ctctgttcat atgatgttgt accaaaatag atgagtaagt   1320
aatgtgtaat ttgtaaacct gttactcagt tggtggatac ttttccaaac ctatgataaa   1380
aacctcgtcc tagatcccgt taaatgccaa actttcggct actataacta tgttatcgtt   1440
atcattatca ttgtttaaca ccct                                          1464
```

<210> 172
<211> 393
<212> PRT
<213> Arabidopsis thaliana

<400> 172

```
Met Asp Cys Asn Met Val Ser Ser Ser Gln Trp Asp Trp Glu His Leu
1               5                   10                  15
Ile Met Ser Asn Pro Ser Arg Thr Glu Asp Asp Ser Lys Gln Leu Pro
            20                  25                  30
Thr Glu Trp Glu Ile Glu Lys Gly Glu Gly Ile Glu Ser Ile Val Pro
        35                  40                  45
His Phe Ser Gly Leu Glu Arg Val Ser Ser Gly Ser Ala Thr Ser Phe
    50                  55                  60
Trp His Thr Ala Val Ser Lys Ser Ser Gln Ser Thr Ser Ile Asn Ser
65                  70                  75                  80
Ser Ser Pro Glu Ala Lys Arg Cys Lys Leu Ala Ser Glu Ser Ser Pro
                85                  90                  95
Gly Asp Ser Cys Ser Asn Ile Asp Phe Val Gln Val Lys Ala Pro Thr
            100                 105                 110
Ala Leu Glu Val Ser Val Ala Ser Ala Glu Ser Asp Leu Cys Leu Lys
        115                 120                 125
Leu Gly Lys Arg Thr Tyr Ser Glu Glu Tyr Trp Gly Arg Asn Asn Asn
    130                 135                 140
Glu Ile Ser Ala Val Ser Met Lys Leu Leu Thr Pro Ser Val Val Ala
145                 150                 155                 160
Gly Lys Ser Lys Leu Cys Gly Gln Ser Met Pro Val Pro Arg Cys Gln
                165                 170                 175
Ile Asp Gly Cys Glu Leu Asp Leu Ser Ser Ala Lys Gly Tyr His Arg
            180                 185                 190
Lys His Lys Val Cys Glu Lys His Ser Lys Cys Pro Lys Val Ser Val
        195                 200                 205
Ser Gly Leu Glu Arg Arg Phe Cys Gln Gln Cys Ser Arg Phe His Ala
    210                 215                 220
```

```
Val Ser Glu Phe Asp Glu Lys Lys Arg Ser Cys Arg Lys Arg Leu Ser
225                 230                 235                 240
His His Asn Ala Arg Arg Arg Lys Pro Gln Gly Val Phe Ser Met Asn
                245                 250                 255
Pro Glu Arg Val Tyr Asp Arg Arg Gln His Thr Asn Met Leu Trp Asn
            260                 265                 270
Gly Val Ser Leu Asn Ala Arg Ser Glu Glu Met Tyr Glu Trp Gly Asn
        275                 280                 285
Asn Thr Tyr Asp Thr Lys Pro Arg Gln Thr Glu Lys Ser Phe Thr Leu
    290                 295                 300
Ser Phe Gln Arg Gly Asn Gly Ser Glu Asp Gln Leu Val Ala Ser Ser
305                 310                 315                 320
Ser Arg Met Phe Ser Thr Ser Gln Thr Ser Gly Gly Phe Pro Ala Gly
                325                 330                 335
Lys Ser Lys Phe Gln Leu His Gly Glu Asp Val Gly Glu Tyr Ser Gly
            340                 345                 350
Val Leu His Glu Ser Gln Asp Ile His Arg Ala Leu Ser Leu Leu Ser
        355                 360                 365
Thr Ser Ser Asp Pro Leu Ala Gln Pro His Val Gln Pro Phe Ser Leu
    370                 375                 380
Leu Cys Ser Tyr Asp Val Val Pro Lys
385                 390
```

<210> 173
<211> 2124
<212> DNA
<213> Nicotiana tabacum

<400> 173

```
gtacaaaaaa gcaggctggt accggtccgg aattcccggg atatcgtcga cccacgcgtc     60
cgccgtgatg taatcttggt gatgctactt attccctttt cccttcttga gcccaaactc    120
aagaaggtca aaaacaaaaa aattacaaaa agctggaatc ttgcagtttt tttatttaat    180
ttatttatcc tatgttgaat taatttttgg ggtcaatatt tcccaatttg tagtctccaa    240
tggagcctcg tgttggtaat aagttccggc ttggccggaa aatcggtagc ggttcttttg    300
gagagatcta tctcggcgct aatgttcaaa ctaacgaaga ggttgcaatt aagctggaaa    360
atgtgaaaac aaagcatcct caactattat acgaagcaaa gttgtataaa atactacaag    420
gaggaactgg aatccccaat ttaaaatggt ttggagttga aggagattat aatgcccttg    480
tgatggattt gctggggcct agtcttgaag atctcttcaa cttctgcagt aggaagctgt    540
ctttaaagac cgttctcatg ctcgcagatc agatgattaa tcgggttgaa tttgttcatg    600
ccaaatcttt tcttcatcga gatataaaac ctgacaactt tcttatggga ttaggaagac    660
gtgcaaatca ggtctatatg attgattttg ggctggccaa gaagtataga gactcatcaa    720
ctcatcagca tattccgtat agagaaaaca aaaatttgac aggaactgct agatacgcaa    780
gcatgaatac tcatcttggc attgaacaaa gtcgaaggga tgatttggaa tcgctgggtt    840
atgttttaat gtacttctta agaggaagtc tcccttggca ggggctgaaa gcaggcacta    900
agaaacagaa gtatgagaag atcagtgaga agaaagtatc aacatcaata gagaccttgt    960
gtaggggata tcctgcagag tttgcatcat attttcatta ctgtcgatca ctaagatttg   1020
atgataaacc agattatgct tatctgaaga gaattttccg tgatcttttc attcgtgaag   1080
ggtttcaatt tgattatata tttgactgga ccattttgaa atatcagcaa tcacagcttg   1140
ccaatcctcc atctcgtgct cttggtggta ctgctgggcc aagctcaggg atgcctcatg   1200
ctcttgttaa tgttgagagg caatcaggtg gagatgaagg tcgaccaact ggttggtctt   1260
catcaaatct tacacgtaat aagagcacgg ggctgcattt caattctgga agcttattga   1320
agcaaaaagg cacagttgct aatgatttat ccatgggtaa agagttatcc agttctaatt   1380
ttttccggtc aagtggacca ttgaggcgtc cagttgtctc tagcatccga gacccagtga   1440
ttgcaggggg tgaacctgac ccctccggca ctctgacaaa agatgcaagc ccgggaccat   1500
tgcgtaaagt atccagtgct gcacggagga gttcaccagt tgtgtcctca gatcacaagc   1560
gcagctcctc tatcaaaaat gccaacataa agaatttaga gtccaccgtc aagggaatag   1620
agggtttaag ttttcgatga tgagggactg cattagtagc tgtgctttgt ctcagttctc   1680
cgttcactgt aaattttggc acaccaactt ggggagtaag agttctgata ttagttgctg   1740
tcaggaagta ccataaagct gaattataca attaaaattt gggatccaat cgcaaaagca   1800
cattaaggat atgatggggt tgcagatcca aactcacaga ttccagttta tgctcgtcca   1860
tacagttata ggcactttcc atattctttt ctttaatctc tgtctcttgc ttgttattgt   1920

tatgtcgtgg tattcttgtt gaggtcatgt ttgtgaattg cgaagatggt catgtataat   1980
tgccgagaaa tcatgtacta gtttgtttta aacatgagca aactgttatt ttgttcaagc   2040
tactttaata tcaaaaaaaa aaaaaaaagg gcggccgctc tagagtatcc ctcgaggggc   2100
ccaagcttac gcgtacccag cttt                                           2124
```

<210> 174
<211> 466
<212> PRT
<213> Nicotiana tabacum

<400> 174

```
Met Glu Pro Arg Val Gly Asn Lys Phe Arg Leu Gly Arg Lys Ile Gly
1               5                   10                  15
Ser Gly Ser Phe Gly Glu Ile Tyr Leu Gly Ala Asn Val Gln Thr Asn
            20                  25                  30
Glu Glu Val Ala Ile Lys Leu Glu Asn Val Lys Thr Lys His Pro Gln
        35                  40                  45
Leu Leu Tyr Glu Ala Lys Leu Tyr Lys Ile Leu Gln Gly Gly Thr Gly
    50                  55                  60
Ile Pro Asn Leu Lys Trp Phe Gly Val Glu Gly Asp Tyr Asn Ala Leu
65                  70                  75                  80
Val Met Asp Leu Leu Gly Pro Ser Leu Glu Asp Leu Phe Asn Phe Cys
                85                  90                  95
Ser Arg Lys Leu Ser Leu Lys Thr Val Leu Met Leu Ala Asp Gln Met
            100                 105                 110
Ile Asn Arg Val Glu Phe Val His Ala Lys Ser Phe Leu His Arg Asp
        115                 120                 125
Ile Lys Pro Asp Asn Phe Leu Met Gly Leu Gly Arg Arg Ala Asn Gln
    130                 135                 140
Val Tyr Met Ile Asp Phe Gly Leu Ala Lys Lys Tyr Arg Asp Ser Ser
145                 150                 155                 160
Thr His Gln His Ile Pro Tyr Arg Glu Asn Lys Asn Leu Thr Gly Thr
                165                 170                 175
Ala Arg Tyr Ala Ser Met Asn Thr His Leu Gly Ile Glu Gln Ser Arg
            180                 185                 190
Arg Asp Asp Leu Glu Ser Leu Gly Tyr Val Leu Met Tyr Phe Leu Arg
        195                 200                 205
Gly Ser Leu Pro Trp Gln Gly Leu Lys Ala Gly Thr Lys Lys Gln Lys
    210                 215                 220
Tyr Glu Lys Ile Ser Glu Lys Lys Val Ser Thr Ser Ile Glu Thr Leu
225                 230                 235                 240
Cys Arg Gly Tyr Pro Ala Glu Phe Ala Ser Tyr Phe His Tyr Cys Arg
                245                 250                 255
Ser Leu Arg Phe Asp Asp Lys Pro Asp Tyr Ala Tyr Leu Lys Arg Ile
            260                 265                 270
Phe Arg Asp Leu Phe Ile Arg Glu Gly Phe Gln Phe Asp Tyr Ile Phe
        275                 280                 285
Asp Trp Thr Ile Leu Lys Tyr Gln Gln Ser Gln Leu Ala Asn Pro Pro
    290                 295                 300
Ser Arg Ala Leu Gly Gly Thr Ala Gly Pro Ser Ser Gly Met Pro His
305                 310                 315                 320
Ala Leu Val Asn Val Glu Arg Gln Ser Gly Gly Asp Glu Gly Arg Pro
                325                 330                 335
Thr Gly Trp Ser Ser Ser Asn Leu Thr Arg Asn Lys Ser Thr Gly Leu
            340                 345                 350
His Phe Asn Ser Gly Ser Leu Leu Lys Gln Lys Gly Thr Val Ala Asn
        355                 360                 365
Asp Leu Ser Met Gly Lys Glu Leu Ser Ser Ser Asn Phe Phe Arg Ser
    370                 375                 380
Ser Gly Pro Leu Arg Arg Pro Val Val Ser Ser Ile Arg Asp Pro Val


385                 390                 395                 400
Ile Ala Gly Gly Glu Pro Asp Pro Ser Gly Thr Leu Thr Lys Asp Ala
                405                 410                 415
Ser Pro Gly Pro Leu Arg Lys Val Ser Ser Ala Ala Arg Arg Ser Ser
            420                 425                 430
Pro Val Val Ser Ser Asp His Lys Arg Ser Ser Ser Ile Lys Asn Ala
        435                 440                 445
Asn Ile Lys Asn Leu Glu Ser Thr Val Lys Gly Ile Glu Gly Leu Ser
    450                 455                 460
Phe Arg
465
```

<210> 175
<211> 1828
<212> DNA
<213> Oryza sativa

<400> 175

```
gcttgagtca tagggagaaa acaaatcgat catatttgac tcttttccct ccatctctct      60
taccggcaaa aaaagtagta ctggtttata tgtaaagtaa gattctttaa ttatgtgaga     120
tccggcttaa tgcttttctt ttgtcacata tactgcattg caacaattgc catatattca     180
cttctgccat cccattatat agcaactcaa gaatggattg atatatcccc tattactaat     240
ctagacatgt taaggctgag ttgggcagtc catcttccca acccaccacc ttcgtttttc     300
gcgcacatac ttttcaaact actaaatggt gtgtttttta aaaatatttt caatacaaaa     360
gttgctttaa aaaattatat tgatccattt ttttaaaaaa aatagctaat acttaattaa     420
tcacgtgtta aaagaccgct ccgttttgcg tgcaggaggg ataggttcac atcctgcatt     480
accgaacaca gcctaaatct tgttgtctag attcgtagta ctggatatat taaatcatgt     540
tctaagttac tatatactga gatgaataga ataagtaaaa ttagacccac cttaagtctt     600
gatgaagtta ctactagctg cgtttgggag gacttcccaa aaaaaaaagt attagccatt     660
agcacgtgat taattaagta ctagtttaaa aaacttaaaa aataaattaa tatgattctc     720
ttaagtaact ctcctataga aaacttttac aaaattacac cgtttaatag tttggaaaat     780
atgtcagtaa aaaataagag agtagaagtt atgaaagtta gaaaaagaat tgttttagta     840
gtatacagtt ataaactatt ccctctgttc taaaacataa gggattatgg atggattcga     900
catgtaccag taccatgaat cgaatccaga caagtttttt atgcatattt attctactat     960

aatatatcac atctgctcta aatatcttat atttcgaggt ggagactgtc gctatgtttt    1020
tctgcccgtt gctaagcaca cgccaccccc gatgcgggga cgcctctggc cttcttgcca    1080
cgataattga atggaacttc cacattcaga ttcgataggt gaccgtcgac tccaagtgct    1140
ttgcacaaaa caactccggc ctcccggcca ccagtcacac gactcacggc actaccaccc    1200
ctgactccct gaggcggacc tgccactgtt ctgcatgcga agctatctaa aattctgaag    1260
caaagaaagc acagcacatg ctccgggaca cgcgccaccc ggcggaaaag ggctcggtgt    1320
ggcgatctca cagccgcata tcgcatttca caagccgccc atctccaccg gcttcacgag    1380
gctcatcgcg gcacgaccgc gcacggaacg cacgcggccg acccgcgcgc ctcgatgcgc    1440
gagcccatcc gccgcgtcct ccctttgcct ttgccgctat cctctcggtc gtatcccgtt    1500
tctctgtctt ttgctccccg gcgcgcgcca gttcggagta ccagcgaaac ccggacacct    1560
ggtacacctc cgccggccac aacgcgtgtc ccccctacgt ggccgcgcag cacatgccca    1620
tgcgcgacac gtgcacctcc tcatccaaac tctcaagtct caacggtcct ataaatgcac    1680
ggatagcctc aagctgctcg tcacaaggca agaggcaaga ggcaagagca tccgtattaa    1740
ccagcctttt gagacttgag agtgtgtgtg actcgatcca gcgtagtttc agttcgtgtg    1800
ttggtgagtg attccagcca agtttgcg                                       1828
```

<210> 176
<211> 2194
<212> DNA
<213> Oryza sativa

<400> 176

```
aatccgaaaa gtttctgcac cgttttcacc ccctaactaa caatataggg aacgtgtgct      60
aaatataaaa tgagacctta tatatgtagc gctgataact agaactatgc aagaaaaact     120
catccaccta ctttagtggc aatcgggcta aataaaaaag agtcgctaca ctagtttcgt     180
tttccttagt aattaagtgg gaaaatgaaa tcattattgc ttagaatata cgttcacatc     240
```

```
tctgtcatga agttaaatta ttcgaggtag ccataattgt catcaaactc ttcttgaata     300
aaaaaatctt tctagctgaa ctcaatgggt aaagagagag attttttta aaaaaataga      360
atgaagatat tctgaacgta ttggcaaaga tttaaacata taattatata attttatagt     420
ttgtgcattc gtcatatcgc acatcattaa ggacatgtct tactccatcc caatttttat     480
ttagtaatta aagacaattg acttattttt attatttatc ttttttcgat tagatgcaag     540
gtacttacgc acacactttg tgctcatgtg catgtgtgag tgcacctcct caatacacgt     600
tcaactagca acacatctct aatatcactc gcctatttaa tacatttagg tagcaatatc     660
tgaattcaag cactccacca tcaccagacc acttttaata atatctaaaa tacaaaaaat     720
aattttacag aatagcatga aaagtatgaa acgaactatt taggtttttc acatacaaaa     780
aaaaaaagaa ttttgctcgt gcgcgagcgc caatctccca tattgggcac acaggcaaca     840
acagagtggc tgcccacaga acaacccaca aaaaacgatg atctaacgga ggacagcaag     900
tccgcaacaa ccttttaaca gcaggctttg cggccaggag agaggaggag aggcaaagaa     960
aaccaagcat cctccttctc ccatctataa attcctcccc ccttttcccc tctctatata    1020
ggaggcatcc aagccaagaa gagggagagc accaaggaca cgcgactagc agaagccgag    1080
cgaccgcctt ctcgatccat atcttccggt cgagttcttg gtcgatctct tccctcctcc    1140
acctcctcct cacagggtat gtgcctccct tcggttgttc ttggatttat tgttctaggt    1200
tgtgtagtac gggcgttgat gttaggaaag gggatctgta tctgtgatga ttcctgttct    1260
tggatttggg atagaggggt tcttgatgtt gcatgttatc ggttcggttt gattagtagt    1320
atggttttca atcgtctgga gagctctatg gaaatgaaat ggtttaggga tcggaatctt    1380
gcgattttgt gagtaccttt tgtttgaggt aaaatcagag caccggtgat tttgcttggt    1440
gtaataaagt acggttgttt ggtcctcgat tctggtagtg atgcttctcg atttgacgaa    1500
gctatccttt gtttattccc tattgaacaa aaataatcca actttgaaga cggtcccgtt    1560
gatgagattg aatgattgat tcttaagcct gtccaaaatt tcgcagctgg cttgtttaga    1620
tacagtagtc cccatcacga aattcatgga aacagttata atcctcagga acaggggatt    1680
ccctgttctt ccgatttgct ttagtcccag aatttttttt cccaaatatc ttaaaaagtc    1740
actttctggt tcagttcaat gaattgattg ctacaaataa tgcttttata gcgttatcct    1800
agctgtagtt cagttaatag gtaatacccc tatagtttag tcaggagaag aacttatccg    1860
atttctgatc tccattttta attatatgaa atgaactgta gcataagcag tattcatttg    1920
gattattttt tttattagct ctcacccctt cattattctg agctgaaagt ctggcatgaa    1980
ctgtcctcaa ttttgttttc aaattcacat cgattatcta tgcattatcc tcttgtatct    2040
acctgtagaa gtttcttttt ggttattcct tgactgcttg attacagaaa gaaatttatg    2100
aagctgtaat cgggatagtt atactgcttg ttcttatgat tcatttcctt tgtgcagttc    2160
ttggtgtagc ttgccacttt caccagcaaa gttc                                2194
```

<210> 177
<211> 56
<212> DNA
<213> Artificial sequence

<220>
<223> primer: prm8667

<400> 177
ggggacaagt ttgtacaaaa aagcaggctt aaacaatgga ctgcaacatg gtatct 56

<210> 178
<211> 54
<212> DNA
<213> Artificial sequence

<220>
<223> primer: prm8668

<400> 178
ggggaccact ttgtacaaga aagctgggtc acattactta ctcatctatt ttgg 54

<210> 179
<211> 2046
<212> DNA
<213> Oryza sativa

<400> 179

```
atgaataccc cagaaaagaa accactgtgc tatacttcta gaagagcact gcaacagagg      60
acagaatcct cttcggaatt gatctctgta tcaaaaagag caacccgcca gaatactcca     120
cgcaagccgg attctcctcc caaacgaaca acaagaagta gcgctaacct ggcgaaatgt     180
atagagaaca aacaccatag cagtcctctg aagcggcggc ggggttcaga tgcggctacc     240
ggaaagtctg caacagggcc aacaaggagg aagcataaac agaaaaggaa aaatgatgag     300
agtgatgagg tcagtcgcat ggaaaaaaga gcaagatatt tacttatcaa aataaaacag     360
gagcagaatt tgctagatgc atactctgga gatggttgga atgggcacag tcgagagaaa     420
ataaagccag agaaggagct acagcgtgct aagaaacaga tcatgaaata caaaatcgct     480
atccgtgacg tcattcacca acttgatttg tgtagttcca gtgggagcaa ggatgactct     540
gtgattccac cagatgggtg tcacgagtct gtcaatcctg aacatacaat atgttcaagg     600
tgcaaatccc acgagtcatt tcctgacaat aacattatat tctgtgaggg gggctgcaaa     660
ctggcgtgtc atcagaaatg tttggagcct ccttttgata aaattcttcc aactacccgc     720
catgggcggc tttgcaaaca ctgttcttcc aagatgaaaa ttttagacgc cattaatgcc     780
catctgggga caagttttac agtgaagtgt ccctccagtg atattttcaa ggaagcagct     840
gaacatttca actctgatga tggactgggc caagattggc tatctgagta ttcaggtgat     900
gaggactatg accctgaaga aaatgaggcc agcagcagtg gtgaagagaa taaatctgcc     960
gattccaatt gttcagggag tccactttat tctccaaatg atgatattcc agacttcata    1020
tcagcagatt tcaatgatgc tgaaggattc tgtcgtgaaa gttcaaatct aggaatcgat    1080
tttggtgaag atggtttggc tgagattctt acccaccaaa ggccaagaag agatgttgac    1140
tatacacaac ttaatgagca aatgtttgga gagcctattg gcaatgacga acagagtgaa    1200
gatgaagatt ggggtcttaa caagagaaag aaaagaagaa ctggttcaac tggtgttggg    1260
actaattccg tagagggtcg atcagatgtc aaatccaata agaaagccca acctcggaga    1320
aaacttttca ggattcctcc tgcggcagtt gaggtgctcc gtaaagcttt tgctgaaaat    1380
gagcttcctg cccggagtgt caaagaaaat ctctcaacag agctgggtat ttcttttgaa    1440

aagattgata agtggttcaa aaatacacgg tgtgctgctc tcagggatag gaagggtgaa    1500
agtcgttatt ctggtcccag caaaaggtca agaacaagca tagaaaaggc tgaaacttca    1560
gctaaagtgg atcaaatgga caactcatgc tttcttccct tatctgaaat aatcaacgtg    1620
cccacacgtc tgcagaaagg tcttgataag aagccaaaat caattaacag ccctccaaga    1680
cctcaggaca atgaaacttg cttgtcgcca actgataaaa ccaaggaggg tacaccgcct    1740
acgatcaagc cttccatcac agattctagt caactgatga ataacgacat cggcactgag    1800
gagactgctg tttcttgggt ggacacttgg gcctctgacg ctctgcattt cttggacgtg    1860
agcgatgatg aacatttctt cgatgtgatc gagaaggtgt gcggtctcga gaatcggctg    1920
cagcggttga aggagaacat gctatcatcg tcatcgtcaa ctgacaacaa tgtggctgct    1980
gagagtggct tgcaaaacga agttgtgctt gtaccagctg ctgagctcaa ggataaagca    2040
tcttaa                                                                2046
```

<210> 180
<211> 681
<212> PRT
<213> Oryza sativa

<400> 180

```
Met Asn Thr Pro Glu Lys Lys Pro Leu Cys Tyr Thr Ser Arg Arg Ala
1               5                   10                  15
Leu Gln Gln Arg Thr Glu Ser Ser Ser Glu Leu Ile Ser Val Ser Lys
            20                  25                  30
Arg Ala Thr Arg Gln Asn Thr Pro Arg Lys Pro Asp Ser Pro Pro Lys
        35                  40                  45
Arg Thr Thr Arg Ser Ser Ala Asn Leu Ala Lys Cys Ile Glu Asn Lys
    50                  55                  60
His His Ser Ser Pro Leu Lys Arg Arg Arg Gly Ser Asp Ala Ala Thr
65                  70                  75                  80
Gly Lys Ser Ala Thr Gly Pro Thr Arg Arg Lys His Lys Gln Lys Arg
                85                  90                  95
Lys Asn Asp Glu Ser Asp Glu Val Ser Arg Met Glu Lys Arg Ala Arg
            100                 105                 110
Tyr Leu Leu Ile Lys Ile Lys Gln Glu Gln Asn Leu Leu Asp Ala Tyr
        115                 120                 125
Ser Gly Asp Gly Trp Asn Gly His Ser Arg Glu Lys Ile Lys Pro Glu
```

```
        130                 135                 140
    Lys Glu Leu Gln Arg Ala Lys Lys Gln Ile Met Lys Tyr Lys Ile Ala
    145                 150                 155                 160
    Ile Arg Asp Val Ile His Gln Leu Asp Leu Cys Ser Ser Ser Gly Ser
                    165                 170                 175
    Lys Asp Asp Ser Val Ile Pro Pro Asp Gly Cys His Glu Ser Val Asn
                180                 185                 190
    Pro Glu His Thr Ile Cys Ser Arg Cys Lys Ser His Glu Ser Phe Pro
            195                 200                 205
    Asp Asn Asn Ile Ile Phe Cys Glu Gly Gly Cys Lys Leu Ala Cys His
        210                 215                 220
    Gln Lys Cys Leu Glu Pro Pro Phe Asp Lys Ile Leu Pro Thr Thr Arg
    225                 230                 235                 240
    His Gly Arg Leu Cys Lys His Cys Ser Ser Lys Met Lys Ile Leu Asp
                    245                 250                 255
    Ala Ile Asn Ala His Leu Gly Thr Ser Phe Thr Val Lys Cys Pro Ser
                260                 265                 270
    Ser Asp Ile Phe Lys Glu Ala Ala Glu His Phe Asn Ser Asp Asp Gly
            275                 280                 285
    Leu Gly Gln Asp Trp Leu Ser Glu Tyr Ser Gly Asp Glu Asp Tyr Asp
        290                 295                 300
    Pro Glu Glu Asn Glu Ala Ser Ser Ser Gly Glu Glu Asn Lys Ser Ala
    305                 310                 315                 320
    Asp Ser Asn Cys Ser Gly Ser Pro Leu Tyr Ser Pro Asn Asp Asp Ile
                    325                 330                 335
    Pro Asp Phe Ile Ser Ala Asp Phe Asn Asp Ala Glu Gly Phe Cys Arg
                340                 345                 350
    Glu Ser Ser Asn Leu Gly Ile Asp Phe Gly Glu Asp Gly Leu Ala Glu
            355                 360                 365
    Ile Leu Thr His Gln Arg Pro Arg Arg Asp Val Asp Tyr Thr Gln Leu
        370                 375                 380
    Asn Glu Gln Met Phe Gly Glu Pro Ile Gly Asn Asp Glu Gln Ser Glu
    385                 390                 395                 400
    Asp Glu Asp Trp Gly Leu Asn Lys Arg Lys Lys Arg Arg Thr Gly Ser
                    405                 410                 415
    Thr Gly Val Gly Thr Asn Ser Val Glu Gly Arg Ser Asp Val Lys Ser
                420                 425                 430
    Asn Lys Lys Ala Gln Pro Arg Arg Lys Leu Phe Arg Ile Pro Pro Ala
            435                 440                 445
    Ala Val Glu Val Leu Arg Lys Ala Phe Ala Glu Asn Glu Leu Pro Ala
        450                 455                 460
    Arg Ser Val Lys Glu Asn Leu Ser Thr Glu Leu Gly Ile Ser Phe Glu
    465                 470                 475                 480
    Lys Ile Asp Lys Trp Phe Lys Asn Thr Arg Cys Ala Ala Leu Arg Asp
                    485                 490                 495
    Arg Lys Gly Glu Ser Arg Tyr Ser Gly Pro Ser Lys Arg Ser Arg Thr
                500                 505                 510
    Ser Ile Glu Lys Ala Glu Thr Ser Ala Lys Val Asp Gln Met Asp Asn
            515                 520                 525
    Ser Cys Phe Leu Pro Leu Ser Glu Ile Ile Asn Val Pro Thr Arg Leu
        530                 535                 540
    Gln Lys Gly Leu Asp Lys Lys Pro Lys Ser Ile Asn Ser Pro Pro Arg
    545                 550                 555                 560
    Pro Gln Asp Asn Glu Thr Cys Leu Ser Pro Thr Asp Lys Thr Lys Glu
                    565                 570                 575
    Gly Thr Pro Pro Thr Ile Lys Pro Ser Ile Thr Asp Ser Ser Gln Leu
                580                 585                 590
    Met Asn Asn Asp Ile Gly Thr Glu Glu Thr Ala Val Ser Trp Val Asp
            595                 600                 605
    Thr Trp Ala Ser Asp Ala Leu His Phe Leu Asp Val Ser Asp Asp Glu
        610                 615                 620
```

```
His Phe Phe Asp Val Ile Glu Lys Val Cys Gly Leu Glu Asn Arg Leu
625                 630                 635                 640
Gln Arg Leu Lys Glu Asn Met Leu Ser Ser Ser Ser Ser Thr Asp Asn
                645                 650                 655
Asn Val Ala Ala Glu Ser Gly Leu Gln Asn Glu Val Val Leu Val Pro
            660                 665                 670
Ala Ala Glu Leu Lys Asp Lys Ala Ser
        675                 680
```

<210> 181
<211> 2391
<212> DNA
<213> Arabidopsis thaliana

<400> 181

```
atggaggaga gtgaaacaaa ggggagaatc agtcaagaaa ctgataaggc ttgtgtttca      60
gttgagagaa ttgggtctac tttactctca tcatttgtga agaaaggtaa agaagtatca     120
aataagagaa attctaagca gaataagcga aaagcggaag aagaactctg ttcgaaatca     180
agaaccaaga agtactctcg ggatgggtc cgttgtgagg aaatggagga ggagaaggtt     240
aagaaaacaa gaaagaggaa aagtaagaga cagcaaaagg ataataaggt tgaggttgat     300
gattctttaa ggttgcaaag gaggacaagg tatttgctca ttaagatgaa gatgcagcag     360
aatcttatcg atgcatacgc aactgaaggt tggaaaggtc agagccggga aaagataaga     420
ccagacaagg agcttgagag agcccggaaa gagatcttaa actgcaagct tggactacgc     480
gacgccattc gccaactgga tctgcttagt tctgtgggaa gcatggaaga gaaagtgata     540
gcttcagatg gatctattca tcatgatcat atattttgtg cagaatgcaa ttctcgagaa     600
gcttttccag acaatgatat aatactttgt gatggaacgt gtaatcgagc atttcaccag     660
aaatgccttg accctccttt ggaaacagaa agcatacctc ccggagatca gggctggttt     720
tgcaaatttt gtgattgcaa aattgaaatt atcgacacaa tgaatgcaca aattggaact     780
catttccctg tggacagcaa ctggcaggat atcttcaatg aagaagctag tcttcctatt     840
ggatctgaag ctacagttaa caatgaagca gattggcctt cggatgactc caaggatgat     900
gattatgacc ctgaaatgag ggaaaacggt ggaggaaaca gcagcaacgt tagtggtgat     960
ggtggtggtg ataacgatga agaaagcatt tcaactagct tgagcttatc atctgatggt    1020
gttgcccttt caacgggatc atgggaaggc catagactca gcaatatggt ggagcagtgc    1080
gaaacaagta atgaagaaac tgtatgtggg ccaagacagc gaaggactgt tgactataca    1140
cagttgtact atgaaatgtt tggaaaggat gcagttttgc aagagcaagg tagtgaagat    1200
gaagactggg gtccgaatga cagaaggaag agaaaaaggg aatctgatgc aggaagcaca    1260
cttgtaacta tgtgtgaaag cagtaagaaa gatcaagatg ttgtagaaac gctagaacag    1320
agtgagagag attctgtctc tgtggaaaat aaaggaggtc gaaggcggat gttcagactc    1380
ccaagaaatg cagttgagaa gctacgtcaa gtatttgcag agactgagct tccctcaaaa    1440
gctgtgaggg atcgtcttgc aaaagagctg agtcttgatc cagagaaggt caataaatgg    1500
tttaaaaata ctcggtatat ggcactgcgg aataggaaga ctgagagcgt gaaacaacct    1560
ggggattcta agacagtctc tggaggagat tctggaccag aagcagtcat ggagaacaac    1620
actgaaacaa atgaagttca agatactttg gatgacactg ttccacccgg atttgacgct    1680
acaaatcaaa acatactctc cccatgtaat aacaaccagg aggaatttca acaggaaaat    1740
gtctcttttc cttcacccac agatgaaagt caacagtatc tggaacaaaa cgattcttct    1800
ttcgttctag tgccacatga aaagcaaagc agcgaaatta gcttaaagac ggctgtagag    1860
gagaatgaga cagaaagcaa gatgatgaaa gagccacatg aagagctaag cagtgaaatg    1920
agcctaaaga cggctgctga ggagaaggag acagagagca agatgataga agagccacat    1980
gaagagctaa gcagagaaat gagcttaaag acggctgtag aggagaagga gacagagagc    2040
aagatgatgg aagagccaca tgatgagcta aacagcgaaa tgagcttaag tacagcggta    2100
gaggagaagg agacagggag caagatgacg gaagagtcac atgaagagct aagcaacgaa    2160
atgagcttag aagagaagga gacgggggaga aagatgacgg aagaggaaga attggaagca    2220
gtaatggaga tgctttgtag aacagaaaac aagttactgg acgtgacgca gaggcttgat    2280
agatttaaaa caccaaaagg ccgaaaaaag ttaggcaatt cttcttctcc tttacttgaa    2340
gaagactcag tagtgtatgt tccaatagca gagatcaagg agaaaaggta a             2391
```

<210> 182
<211> 796
<212> PRT
<213> Arabidopsis thaliana

<400> 182

```
Met Glu Glu Ser Glu Thr Lys Gly Arg Ile Ser Gln Glu Thr Asp Lys
1               5                   10                  15
Ala Cys Val Ser Val Glu Arg Ile Gly Ser Thr Leu Leu Ser Ser Phe
            20                  25                  30
Val Lys Lys Gly Lys Glu Val Ser Asn Lys Arg Asn Ser Lys Gln Asn
        35                  40                  45
Lys Arg Lys Ala Glu Glu Glu Leu Cys Ser Lys Ser Arg Thr Lys Lys
    50                  55                  60
Tyr Ser Arg Gly Trp Val Arg Cys Glu Glu Met Glu Glu Glu Lys Val
65                  70                  75                  80
Lys Lys Thr Arg Lys Arg Lys Ser Lys Arg Gln Gln Lys Asp Asn Lys
                85                  90                  95
Val Glu Val Asp Asp Ser Leu Arg Leu Gln Arg Arg Thr Arg Tyr Leu
            100                 105                 110
Leu Ile Lys Met Lys Met Gln Gln Asn Leu Ile Asp Ala Tyr Ala Thr
        115                 120                 125
Glu Gly Trp Lys Gly Gln Ser Arg Glu Lys Ile Arg Pro Asp Lys Glu

    130                 135                 140
Leu Glu Arg Ala Arg Lys Glu Ile Leu Asn Cys Lys Leu Gly Leu Arg
145                 150                 155                 160
Asp Ala Ile Arg Gln Leu Asp Leu Leu Ser Ser Val Gly Ser Met Glu
                165                 170                 175
Glu Lys Val Ile Ala Ser Asp Gly Ser Ile His His Asp His Ile Phe
            180                 185                 190
Cys Ala Glu Cys Asn Ser Arg Glu Ala Phe Pro Asp Asn Asp Ile Ile
        195                 200                 205
Leu Cys Asp Gly Thr Cys Asn Arg Ala Phe His Gln Lys Cys Leu Asp
    210                 215                 220
Pro Pro Leu Glu Thr Glu Ser Ile Pro Pro Gly Asp Gln Gly Trp Phe
225                 230                 235                 240
Cys Lys Phe Cys Asp Cys Lys Ile Glu Ile Ile Asp Thr Met Asn Ala
                245                 250                 255
Gln Ile Gly Thr His Phe Pro Val Asp Ser Asn Trp Gln Asp Ile Phe
            260                 265                 270
Asn Glu Glu Ala Ser Leu Pro Ile Gly Ser Glu Ala Thr Val Asn Asn
        275                 280                 285
Glu Ala Asp Trp Pro Ser Asp Asp Ser Lys Asp Asp Asp Tyr Asp Pro
    290                 295                 300
Glu Met Arg Glu Asn Gly Gly Gly Asn Ser Ser Asn Val Ser Gly Asp
305                 310                 315                 320
Gly Gly Gly Asp Asn Asp Glu Glu Ser Ile Ser Thr Ser Leu Ser Leu
                325                 330                 335
Ser Ser Asp Gly Val Ala Leu Ser Thr Gly Ser Trp Glu Gly His Arg
            340                 345                 350
Leu Ser Asn Met Val Glu Gln Cys Glu Thr Ser Asn Glu Glu Thr Val
        355                 360                 365
Cys Gly Pro Arg Gln Arg Arg Thr Val Asp Tyr Thr Gln Leu Tyr Tyr
    370                 375                 380
Glu Met Phe Gly Lys Asp Ala Val Leu Gln Glu Gln Gly Ser Glu Asp
385                 390                 395                 400
Glu Asp Trp Gly Pro Asn Asp Arg Arg Lys Arg Lys Arg Glu Ser Asp
                405                 410                 415
Ala Gly Ser Thr Leu Val Thr Met Cys Glu Ser Ser Lys Lys Asp Gln
            420                 425                 430
Asp Val Val Glu Thr Leu Glu Gln Ser Glu Arg Asp Ser Val Ser Val
        435                 440                 445
Glu Asn Lys Gly Gly Arg Arg Arg Met Phe Arg Leu Pro Arg Asn Ala
    450                 455                 460
Val Glu Lys Leu Arg Gln Val Phe Ala Glu Thr Glu Leu Pro Ser Lys
```

465 470 475 480
Ala Val Arg Asp Arg Leu Ala Lys Glu Leu Ser Leu Asp Pro Glu Lys
485 490 495
Val Asn Lys Trp Phe Lys Asn Thr Arg Tyr Met Ala Leu Arg Asn Arg
500 505 510
Lys Thr Glu Ser Val Lys Gln Pro Gly Asp Ser Lys Thr Val Ser Gly
515 520 525
Gly Asp Ser Gly Pro Glu Ala Val Met Glu Asn Asn Thr Glu Thr Asn
530 535 540
Glu Val Gln Asp Thr Leu Asp Asp Thr Val Pro Pro Gly Phe Asp Ala
545 550 555 560
Thr Asn Gln Asn Ile Leu Ser Pro Cys Asn Asn Asn Gln Glu Glu Phe
565 570 575
Gln Gln Glu Asn Val Ser Phe Pro Ser Pro Thr Asp Glu Ser Gln Gln
580 585 590
Tyr Leu Glu Gln Asn Asp Ser Ser Phe Val Leu Val Pro His Glu Lys
595 600 605
Gln Ser Ser Glu Ile Ser Leu Lys Thr Ala Val Glu Glu Asn Glu Thr
610 615 620
Glu Ser Lys Met Met Lys Glu Pro His Glu Glu Leu Ser Ser Glu Met
625 630 635 640
Ser Leu Lys Thr Ala Ala Glu Glu Lys Glu Thr Glu Ser Lys Met Ile
645 650 655
Glu Glu Pro His Glu Glu Leu Ser Arg Glu Met Ser Leu Lys Thr Ala
660 665 670
Val Glu Glu Lys Glu Thr Glu Ser Lys Met Met Glu Glu Pro His Asp
675 680 685
Glu Leu Asn Ser Glu Met Ser Leu Ser Thr Ala Val Glu Glu Lys Glu
690 695 700
Thr Gly Ser Lys Met Thr Glu Glu Ser His Glu Glu Leu Ser Asn Glu
705 710 715 720
Met Ser Leu Glu Glu Lys Glu Thr Gly Arg Lys Met Thr Glu Glu Glu
725 730 735
Glu Leu Glu Ala Val Met Glu Met Leu Cys Arg Thr Glu Asn Lys Leu
740 745 750
Leu Asp Val Thr Gln Arg Leu Asp Arg Phe Lys Thr Pro Lys Gly Arg
755 760 765
Lys Lys Leu Gly Asn Ser Ser Ser Pro Leu Leu Glu Glu Asp Ser Val
770 775 780
Val Tyr Val Pro Ile Ala Glu Ile Lys Glu Lys Arg
785 790 795

<210> 183
<211> 2151
<212> DNA
<213> Eucalyptus grandis

<400> 183

```
atgcattcca gaaagaatct tgcccaaaaa gaagactttt tgaaggcaga atctggatcg     60
gaggttcttg cctcgctgat attgagaaat aggagaagga aaaagcacaa acagaaacta    120
caatcttctg taaaagcaag aggctcaacc cttgccaaga agagggccaa taactccatc    180
agaaagagac tggtgcgtag gaacagctct gataaagaca aactcataaa tttaaaagct    240
tttcataata agaaaattga aatagttccc gagaagcttt catcccctcc atcttcagaa    300
agagagctgc ctattgcttc tgacgaaaat gtgcaaaaca ccgatgagga tgtcaaagtt    360
aacaagctga gaaggaagag gaagaagaaa atgagaaagc atttttcgaa gttagatgag    420
gcttcacgat tgcagaggag aacaagatac cttcttatta aaatgaagct ggaacagaac    480
cttattgatg cttactctgg tgaaggctgg aaaggccaga gtcgggaaaa gatcaagcca    540
acaaaggaat tacaaagggc caggaagcag atactgaatt gcaaactcgg aatacgtgat    600
gctattcgac agctagactc cattagttca gtgggcagca ttgaagactc tgtgattgct    660
cctgatggtt ctgttcacca tgaacatata ttctgtgcaa aatgcaagac aaatgaaact    720
tcctcagata atgatatcat actctgcgat gggacatgca attgtgcttt tcatcaaaaa    780

tgtcttgatc ctccactgga aactgaaagc attcccccag gagatcaggg ctggttttgc    840
agactctgcg agtgtaagat ggaaattata gagtcaacaa atgcacatct ggggacccac    900
ttttctcttg acagcaactg gcaggatata ttcgaggaag aagctgcatt gcctgatggg    960
ggaattcagg tgcttgacct agacgaaaag tggccttcag atgactctga agatgatgac   1020
tatgacccag aaaagatgac gaacaccggc atcaactgtg ccggcaatga tgattctcaa   1080
tcatacgaca caagtagctc taccagcctg agttggtcat tagatggtga aattttgtca   1140
ggaccaacaa tatcaggcaa ggaagggttg aactatcctg agagcattgc agggtctgat   1200
gtattaatgg acacggatac tgatactgta tgtggcccac gacagcgaaa tgctgttgat   1260
tataagcagc tttataatga aatgttcggt aaggatactc ctgcctgtga acaagccagt   1320
gaagatgaag actggggtcc taataaaaga agaagaagag aaagggaaac agatgcagcc   1380
agcaccctca tgactctctg tgaaagtgag aaaaacaatc cattcattga ccaagcctca   1440
gaaggtgata agaaactctc tgcagaagca agaatgagaa ggcggatttt cagaattcct   1500
ccaactgcag ttgagaagct tcgccaagta tttgcagaga atgaacttcc ctcaagatct   1560
gtcaaagaaa atcttgctaa agagtttggt cttgaacctg agaaggttag caaatggttc   1620
aagaatgcac gatacatggc actcaaaaca agaaaggctg ggggggctga tcaacctcca   1680
aaatccccgc aaaagcataa agaatctgaa ctagcaaaaa tgatgaaagg ggctgcgcct   1740
attttatcta aggatgcctc aacagacatt gaattccatt cctctctaaa tactgagaaa   1800
gtcatccagc aaaagagtac gaagccaata cacgggaagg gttcacctat ggtacctgaa   1860
catagctata tggagaattt tgagttcgat gacaatatgt ccttgaaaca aataaagcta   1920
ttgaaaggaa aaccaaagga caggaagagg gttgacttcg cagccagtga gggattacga   1980
gaaagagaag ctcagttgcg ggagctatgt aaaattagaa gtaggctaga tagtctgagg   2040
aagagaaccc ttacatttca gtcgagtgaa tccaaggctt cagacgaatt caacacttgt   2100
agatcacgtg tagtatatgt tccagtagca gaaataaagg agagagcttg a            2151
```

<210> 184
<211> 716
<212> PRT
<213> Eucalyptus grandis

<400> 184

```
Met His Ser Arg Lys Asn Leu Ala Gln Lys Glu Asp Phe Leu Lys Ala
1               5                   10                  15
Glu Ser Gly Ser Glu Val Leu Ala Ser Leu Ile Leu Arg Asn Arg Arg
            20                  25                  30
Arg Lys Lys His Lys Gln Lys Leu Gln Ser Ser Val Lys Ala Arg Gly
        35                  40                  45
Ser Thr Leu Ala Lys Lys Arg Ala Asn Asn Ser Ile Arg Lys Arg Leu
    50                  55                  60
Val Arg Arg Asn Ser Ser Asp Lys Asp Lys Leu Ile Asn Leu Lys Ala
65                  70                  75                  80
Phe His Asn Lys Lys Ile Glu Ile Val Pro Glu Lys Leu Ser Ser Pro
                85                  90                  95
Pro Ser Ser Glu Arg Glu Leu Pro Ile Ala Ser Asp Glu Asn Val Gln
            100                 105                 110
Asn Thr Asp Glu Asp Val Lys Val Asn Lys Leu Arg Arg Lys Arg Lys
        115                 120                 125
Lys Lys Met Arg Lys His Phe Ser Lys Leu Asp Glu Ala Ser Arg Leu
    130                 135                 140
Gln Arg Arg Thr Arg Tyr Leu Leu Ile Lys Met Lys Leu Glu Gln Asn
145                 150                 155                 160
Leu Ile Asp Ala Tyr Ser Gly Glu Gly Trp Lys Gly Gln Ser Arg Glu
                165                 170                 175
Lys Ile Lys Pro Thr Lys Glu Leu Gln Arg Ala Arg Lys Gln Ile Leu
            180                 185                 190
Asn Cys Lys Leu Gly Ile Arg Asp Ala Ile Arg Gln Leu Asp Ser Ile
        195                 200                 205
Ser Ser Val Gly Ser Ile Glu Asp Ser Val Ile Ala Pro Asp Gly Ser
    210                 215                 220
Val His His Glu His Ile Phe Cys Ala Lys Cys Lys Thr Asn Glu Thr
225                 230                 235                 240
```

```
Ser Ser Asp Asn Asp Ile Ile Leu Cys Asp Gly Thr Cys Asn Cys Ala
                245                 250                 255
Phe His Gln Lys Cys Leu Asp Pro Pro Leu Glu Thr Glu Ser Ile Pro
            260                 265                 270
Pro Gly Asp Gln Gly Trp Phe Cys Arg Leu Cys Glu Cys Lys Met Glu
        275                 280                 285
Ile Ile Glu Ser Thr Asn Ala His Leu Gly Thr His Phe Ser Leu Asp
    290                 295                 300
Ser Asn Trp Gln Asp Ile Phe Glu Glu Glu Ala Ala Leu Pro Asp Gly
305                 310                 315                 320
Gly Ile Gln Val Leu Asp Leu Asp Glu Lys Trp Pro Ser Asp Asp Ser
                325                 330                 335
Glu Asp Asp Asp Tyr Asp Pro Glu Lys Met Thr Asn Thr Gly Ile Asn
            340                 345                 350
Cys Ala Gly Asn Asp Asp Ser Gln Ser Tyr Asp Thr Ser Ser Ser Thr
        355                 360                 365
Ser Leu Ser Trp Ser Leu Asp Gly Glu Ile Leu Ser Gly Pro Thr Ile
    370                 375                 380
Ser Gly Lys Glu Gly Leu Asn Tyr Pro Glu Ser Ile Ala Gly Ser Asp
385                 390                 395                 400
Val Leu Met Asp Thr Asp Thr Asp Thr Val Cys Gly Pro Arg Gln Arg
                405                 410                 415
Asn Ala Val Asp Tyr Lys Gln Leu Tyr Asn Glu Met Phe Gly Lys Asp
            420                 425                 430
Thr Pro Ala Cys Glu Gln Ala Ser Glu Asp Glu Asp Trp Gly Pro Asn
        435                 440                 445
Lys Arg Arg Arg Arg Glu Arg Glu Thr Asp Ala Ala Ser Thr Leu Met
    450                 455                 460
Thr Leu Cys Glu Ser Glu Lys Asn Asn Pro Phe Ile Asp Gln Ala Ser
465                 470                 475                 480
Glu Gly Asp Lys Lys Leu Ser Ala Glu Ala Arg Met Arg Arg Arg Ile
                485                 490                 495
Phe Arg Ile Pro Pro Thr Ala Val Glu Lys Leu Arg Gln Val Phe Ala
            500                 505                 510
Glu Asn Glu Leu Pro Ser Arg Ser Val Lys Glu Asn Leu Ala Lys Glu
        515                 520                 525
Phe Gly Leu Glu Pro Glu Lys Val Ser Lys Trp Phe Lys Asn Ala Arg
    530                 535                 540
Tyr Met Ala Leu Lys Thr Arg Lys Ala Gly Gly Ala Asp Gln Pro Pro
545                 550                 555                 560
Lys Ser Pro Gln Lys His Lys Glu Ser Glu Leu Ala Lys Met Met Lys
                565                 570                 575
Gly Ala Ala Pro Ile Leu Ser Lys Asp Ala Ser Thr Asp Ile Glu Phe
            580                 585                 590
His Ser Ser Leu Asn Thr Glu Lys Val Ile Gln Gln Lys Ser Thr Lys
        595                 600                 605
Pro Ile His Gly Lys Gly Ser Pro Met Val Pro Glu His Ser Tyr Met
    610                 615                 620
Glu Asn Phe Glu Phe Asp Asp Asn Met Ser Leu Lys Gln Ile Lys Leu
625                 630                 635                 640
Leu Lys Gly Lys Pro Lys Asp Arg Lys Arg Val Asp Phe Ala Ala Ser
                645                 650                 655
Glu Gly Leu Arg Glu Arg Glu Ala Gln Leu Arg Glu Leu Cys Lys Ile
            660                 665                 670
Arg Ser Arg Leu Asp Ser Leu Arg Lys Arg Thr Leu Thr Phe Gln Ser
        675                 680                 685
Ser Glu Ser Lys Ala Ser Asp Glu Phe Asn Thr Cys Arg Ser Arg Val
    690                 695                 700
Val Tyr Val Pro Val Ala Glu Ile Lys Glu Arg Ala
705                 710                 715
```

<210> 185
<211> 2124

<212> DNA
<213> Medicago truncatula

<400> 185

```
atgcgggata ctgagaggtt aaataaccaa ggatctacaa aaccgagtaa cacaaaggaa      60
catatcaagt tgaaggtaga ttccccacag ttcaaaacaa gtagcacaaa acaccaaagg     120
aagaaacaca gactgaaatc aaaatctcat aaactcggag gttgcacaaa tgcgtcagtg     180
aggacagcta ctgattcttc caacaaggcg tcaaccaagg attcttcaaa taaaacggat     240
aggaattcaa cacaggttca gccttcaaaa aagatacaag gaggaaaaac ttctcttaat     300
aatgatagga aaggtgagaa agatgttgtt gatcaagagg gaaatattca aaaacgtaag     360
agaaggagaa agaagaaaag acaaagacat aatgtagatc ttgatgatac tgtacgcctc     420
cagagaagaa caaggaatat cttaatcaga atgaagcaag aacagaatct tatcgatgct     480
tacgctggag aaggttggaa aggtcagagt cgagagaaga ttaggccaga aatggagcta     540
caaagagcaa aaaagcagat tttaaaatgc aagcttagta tccgtgatgc cattcaccaa     600
ctggattctc ttagttcagt tggtagtatt gaaggttctg tcattgctac agatggatct     660
gtttctcatg aacatatatt ctgtgcaaat tgcaagataa acgaagtttc tccagataat     720
gatattatac tctgtgatgg cacatgcaat cgtgcgtttc accagagatg tcttgatcct     780
cctttggaga ctgaagacat tcctcctgaa gaccaaggct ggttttgcaa gtattgtgac     840
tgtaagatag aaatattgga ggcaacaaac gcgcatcttg ggactcgttt ccccttagac     900
agtacttggc aggatgtatt caaggaagaa gctgctattc ctgacggcga tgctgcatta     960
ctaaatcaag aagaagaatg gccgtcagat gatcctgaag acgatgacta taatccagag    1020
aggaaagaag aaagccacgg tggcttcaac acagaaggaa atgataaaaa cgcatctgat    1080
gattcaagca gttcttccag catgtggtct ctaaatggag aatgctctct gttagatgaa    1140
ggcatcaacc ttgaatatta ttctaacgac cacatagatt ctgatgaatc cggggaaata    1200
gcatgcggtc gtaggcagcg tagagctgtt gactacaaga aactttatga tgaaatgttt    1260
ggaaaggatg ctccaccttg tgaacaagtg agtgaagatg aagattgggg tcctcgaaaa    1320
agacggcgaa gagaaaagga gtctgaggct gttaatactc ttatgactct acatgagagt    1380
gaaaataaat atccgaataa taagaacaat gatagaataa gagggaattc ttcaggcata    1440
aaaagacctt gttttaggtt ttcacatgag gcagttgaga agcttcgcca agtttttgca    1500
gagaacgagc ttcctccaaa atccgtgaag gatgcccttt caaaagaatt gggacttgat    1560
gctgcaaagg ttaacaaatg gttcaaaaat gcacgttaca tggcacttaa aataagaaag    1620
cttcaagaag gaggacaaca gcttcagagt atcacttcta agacctcaaa ggattctaca    1680
tcacaacatg tgcaggaaga tgaggttttg aacccgaagt ccgcgaaaat tactgtgatt    1740
agttccctga agaagtgtga aaatgttact ggcaaagaaa aaacaaaagc atctagcaat    1800
cctttgaaga aaaagcgacc agaaatacct ccatgcttgg gagaaaatgg caacaaggat    1860
tccatggagg tcagtgatga tgttagctta atgaaactat tacaagacag gaaaaagaag    1920
gtaagttttg catttgaagg agactctgag gcagcagagt tggaatttga aagactcagc    1980
aaagtgaaga cgaaaataga tagattgaag caaagattaa ctggagttca aaattgcaga    2040
tcaaagggtt cagatgaagt tcatttgaat gagccatcta ttatgtatgt tcctatagct    2100
gagttaaggg aaaaggtgaa atag                                           2124
```

<210> 186
<211> 707
<212> PRT
<213> Medicago truncatula

<400> 186

```
Met Arg Asp Thr Glu Arg Leu Asn Asn Gln Gly Ser Thr Lys Pro Ser
1               5                   10                  15
Asn Thr Lys Glu His Ile Lys Leu Lys Val Asp Ser Pro Gln Phe Lys
            20                  25                  30
Thr Ser Ser Thr Lys His Gln Arg Lys Lys His Arg Leu Lys Ser Lys
        35                  40                  45
Ser His Lys Leu Gly Gly Cys Thr Asn Ala Ser Val Arg Thr Ala Thr
    50                  55                  60
Asp Ser Ser Asn Lys Ala Ser Thr Lys Asp Ser Ser Asn Lys Thr Asp
65                  70                  75                  80
Arg Asn Ser Thr Gln Val Gln Pro Ser Lys Lys Ile Gln Gly Gly Lys
                85                  90                  95
```

```
Thr Ser Leu Asn Asn Asp Arg Lys Gly Glu Lys Asp Val Val Asp Gln
            100                 105                 110
Glu Gly Asn Ile Gln Lys Arg Lys Arg Arg Arg Lys Lys Lys Arg Gln
        115                 120                 125
Arg His Asn Val Asp Leu Asp Asp Thr Val Arg Leu Gln Arg Arg Thr
    130                 135                 140
Arg Asn Ile Leu Ile Arg Met Lys Gln Glu Gln Asn Leu Ile Asp Ala
145                 150                 155                 160
Tyr Ala Gly Glu Gly Trp Lys Gly Gln Ser Arg Glu Lys Ile Arg Pro
                165                 170                 175
Glu Met Glu Leu Gln Arg Ala Lys Lys Gln Ile Leu Lys Cys Lys Leu
            180                 185                 190
Ser Ile Arg Asp Ala Ile His Gln Leu Asp Ser Leu Ser Ser Val Gly
        195                 200                 205
Ser Ile Glu Gly Ser Val Ile Ala Thr Asp Gly Ser Val Ser His Glu
    210                 215                 220
His Ile Phe Cys Ala Asn Cys Lys Ile Asn Glu Val Ser Pro Asp Asn
225                 230                 235                 240
Asp Ile Ile Leu Cys Asp Gly Thr Cys Asn Arg Ala Phe His Gln Arg
                245                 250                 255
Cys Leu Asp Pro Pro Leu Glu Thr Glu Asp Ile Pro Pro Glu Asp Gln
            260                 265                 270
Gly Trp Phe Cys Lys Tyr Cys Asp Cys Lys Ile Glu Ile Leu Glu Ala
        275                 280                 285
Thr Asn Ala His Leu Gly Thr Arg Phe Pro Leu Asp Ser Thr Trp Gln
    290                 295                 300
Asp Val Phe Lys Glu Glu Ala Ala Ile Pro Asp Gly Asp Ala Ala Leu
305                 310                 315                 320
Leu Asn Gln Glu Glu Glu Trp Pro Ser Asp Asp Pro Glu Asp Asp Asp
                325                 330                 335
Tyr Asn Pro Glu Arg Lys Glu Glu Ser His Gly Gly Phe Asn Thr Glu
            340                 345                 350
Gly Asn Asp Lys Asn Ala Ser Asp Asp Ser Ser Ser Ser Ser Ser Met
        355                 360                 365
Trp Ser Leu Asn Gly Glu Cys Ser Leu Leu Asp Glu Gly Ile Asn Leu
    370                 375                 380
Glu Tyr Tyr Ser Asn Asp His Ile Asp Ser Asp Glu Ser Gly Glu Ile
385                 390                 395                 400
Ala Cys Gly Arg Arg Gln Arg Arg Ala Val Asp Tyr Lys Lys Leu Tyr
                405                 410                 415
Asp Glu Met Phe Gly Lys Asp Ala Pro Pro Cys Glu Gln Val Ser Glu
            420                 425                 430
Asp Glu Asp Trp Gly Pro Arg Lys Arg Arg Arg Arg Glu Lys Glu Ser
        435                 440                 445
Glu Ala Val Asn Thr Leu Met Thr Leu His Glu Ser Glu Asn Lys Tyr
    450                 455                 460
Pro Asn Asn Lys Asn Asn Asp Arg Ile Arg Gly Asn Ser Ser Gly Ile
465                 470                 475                 480
Lys Arg Pro Cys Phe Arg Phe Ser His Glu Ala Val Glu Lys Leu Arg
                485                 490                 495
Gln Val Phe Ala Glu Asn Glu Leu Pro Pro Lys Ser Val Lys Asp Ala
            500                 505                 510
Leu Ser Lys Glu Leu Gly Leu Asp Ala Ala Lys Val Asn Lys Trp Phe
        515                 520                 525
Lys Asn Ala Arg Tyr Met Ala Leu Lys Ile Arg Lys Leu Gln Glu Gly
    530                 535                 540
Gly Gln Gln Leu Gln Ser Ile Thr Ser Lys Thr Ser Lys Asp Ser Thr
545                 550                 555                 560
Ser Gln His Val Gln Glu Asp Glu Val Leu Asn Pro Lys Ser Ala Lys
                565                 570                 575
Ile Thr Val Ile Ser Ser Leu Lys Lys Cys Glu Asn Val Thr Gly Lys
```

```
            580                 585                 590
Glu Lys Thr Lys Ala Ser Ser Asn Pro Leu Lys Lys Lys Arg Pro Glu
        595                 600                 605
Ile Pro Pro Cys Leu Gly Glu Asn Gly Asn Lys Asp Ser Met Glu Val
    610                 615                 620
Ser Asp Asp Val Ser Leu Met Lys Leu Leu Gln Asp Arg Lys Lys Lys
625                 630                 635                 640
Val Ser Phe Ala Phe Glu Gly Asp Ser Glu Ala Ala Glu Leu Glu Phe
                645                 650                 655
Glu Arg Leu Ser Lys Val Lys Thr Lys Ile Asp Arg Leu Lys Gln Arg
            660                 665                 670
Leu Thr Gly Val Gln Asn Cys Arg Ser Lys Gly Ser Asp Glu Val His
        675                 680                 685
Leu Asn Glu Pro Ser Ile Met Tyr Val Pro Ile Ala Glu Leu Arg Glu
    690                 695                 700
Lys Val Lys
705
```

<210> 187
<211> 2556
<212> DNA
<213> Pinus radiata

<400> 187

```
atgccgaaag caggaaaagg ctcttccggt tcactccgaa agaagcaagc gttgtcaaat      60
acagcatcag agctaaggcc taaagctgca attcagaaga ctaacaatgc cgggagtatt     120
tctggcagga tgagggaaag gaaaccacgg ttacatgtac agatgattgc ctcaattctc     180
acgaaaaaaa ggacaggtga agcatctgaa tcgagaaatg attctgagct agaagacaaa     240
caatatctga gcaatagttc aaaaacaaat gccaatttg tagcggggaa gttagttaaa     300
aagactttga aaaaagcccc aagattaaac ttgattaaga atgacagtca gaaaaaaccc     360
agtaagagtt ctgcttcgag gtcttatgct gcacagaaac ctgaacggcc acaacagaga     420
aaaattagga ggacaaagtg gaagggtgat tctaaaaata taacaaaggt gaaaactgtg     480
agaactagta atatagatgg cttgaagagg aagcagcaaa ggagaaagaa gaaaaagaag     540
gcgcagaatg ttccacagga tgaagtatca cgtgcaaaga gaaggatcaa atatcttttg     600
ctcaaaatga aacttgagca gaatcttatt gatgcatatt caggggaggg ctggaaaggc     660
cagagtcggg agaaaataaa gccagagaag gagttacaga gagctgaaaa gcagattctg     720
caatgcaaac ttggaattcg tgaagcagtg catcagcttg atcttctaag ttcggaagga     780
tctatagcag atgccgttat tgatccagaa gggcgtgtat ttcatgagca tatattttgt     840
gcaaaatgca aaatgacaga tgctcttccg gataatgaca taatattgtg tgatggagct     900
tgcaatcgtg gcttccatca gaaatgtctg gatccacctt tggctactga aaacattcct     960
cctggagatc agggttggct ctgtaaagtt tgcgaatgta agctagagag tttggaagca    1020
ataaatgctc atttgggcac ccagtttaca gttgataata gctgggaggg aatttttgct    1080
gatgcagcta gaattgcgaa tggtgaaagt acagctactg ggaatgggga agaatggcca    1140
tcagatgatt cagaagatga tgactatgat ccagagaaac aagagattag agatgaaacg    1200
ggctatgaga ataatatgag tgattctgga aagtcaagtg gatggtcaga atcatataat    1260
gaaactgaca atgaatcaga tgcttcctta acagataatc taaatgaata catctctaag    1320
ggaaagaaga ggagcagaga cactgatgaa aaaaacggac aagaagctat aatagattta    1380
gatactgatg agaatgatgg ctcagacatg ccagtttcag ggagaaggca gcgaagagat    1440
gttgattata aaaagttgca tgatgaaatg tttggaaaag atggaccttg tgaagatgaa    1500
gtcagtgaag atgaagactg gggacccaat aggaggcgta gaagagcgaa acagccggat    1560
acaaatacca ctaaggattc ttcaaatgaa gagaaagagc atgttagaaa ggaagcagat    1620
gagaaagtag cagagcattc acaaagtagc ataaaggata agaaacatat tgtcagactg    1680
ccagctaatg cagttgagaa gctgcgcaaa gtctttgctg aaaatgagct accgtcaagg    1740
tcttacaagg aaaatctgtc acagcaactg ggccttacat ttcgaaaggt ccatatgtgg    1800
ttcaaaaatg cccgttatat gtctctgaag agccgaaagg aaatgcctag aaatgagaac    1860
tgcaatgaaa ctaatctcat gggagaaggt gatatggggc caaagaaaaa aataccaaaa    1920
gagaagaaag cagaatcaac atgtctttta tcttctgtta gagcagaata taaatctaga    1980
aatacaaaaa ggaagcatag gagaaaaaac atcaaagcac ccttatctat ctcttccaga    2040
agtgagagaa aaagaactat gtgcagactg cctgccaatg cagttgagag gtttcgccaa    2100
gtctttgctg agaatgagtt gccttcaatg tcctgtaaaa tggcattatc aaagcagttg    2160
gatctacctt atcggcaggt gcatatgtgg ttcaaaaatg ctcgttatat gtctttgaag    2220
```

```
aagaaaaaga tgaaccgttt aaaagaatca gctcccgtta tttcctcaca taccagaaga   2280
cttgaagtag aaagtagcaa acccaatcca caaatcaaag agccagatga tgctggcaat   2340
tcatacttaa cagagatgga aaaattgggt aatattgagt tgaaacttga aaatatgaga   2400
aagattttag aaacagtctg tcctaaaaga ggtactgatt tttccaatga taagaatcaa   2460
gaaggaatcg ccaaccttct tagtgaggag cttcttgtgt atgtaccagt ggtggagttg   2520
agggagaaga gcttacaggt tgctcctgtc aggtga                             2556
```

<210> 188
<211> 851
<212> PRT
<213> Pinus radiata

<400> 188

```
Met Pro Lys Ala Gly Lys Gly Ser Ser Gly Ser Leu Arg Lys Lys Gln
1               5                   10                  15
Ala Leu Ser Asn Thr Ala Ser Glu Leu Arg Pro Lys Ala Ala Ile Gln
            20                  25                  30
Lys Thr Asn Asn Ala Gly Ser Ile Ser Gly Arg Met Arg Glu Arg Lys
        35                  40                  45
Pro Arg Leu His Val Gln Met Ile Ala Ser Ile Leu Thr Lys Lys Arg
    50                  55                  60
Thr Gly Glu Ala Ser Glu Ser Arg Asn Asp Ser Glu Leu Glu Asp Lys
65                  70                  75                  80
Gln Tyr Leu Ser Asn Ser Ser Lys Thr Asn Ala Asn Phe Val Ala Gly
                85                  90                  95
Lys Leu Val Lys Lys Thr Leu Lys Lys Ala Pro Arg Leu Asn Leu Ile
            100                 105                 110
Lys Asn Asp Ser Gln Lys Lys Pro Ser Lys Ser Ser Ala Ser Arg Ser
        115                 120                 125
Tyr Ala Ala Gln Lys Pro Glu Arg Pro Gln Gln Arg Lys Ile Arg Arg
    130                 135                 140
Thr Lys Trp Lys Gly Asp Ser Lys Asn Ile Thr Lys Val Lys Thr Val
145                 150                 155                 160
Arg Thr Ser Asn Ile Asp Gly Leu Lys Arg Lys Gln Gln Arg Arg Lys
                165                 170                 175
Lys Lys Lys Lys Ala Gln Asn Val Pro Gln Asp Glu Val Ser Arg Ala
            180                 185                 190
Lys Arg Arg Ile Lys Tyr Leu Leu Leu Lys Met Lys Leu Glu Gln Asn
        195                 200                 205
Leu Ile Asp Ala Tyr Ser Gly Glu Gly Trp Lys Gly Gln Ser Arg Glu
    210                 215                 220
Lys Ile Lys Pro Glu Lys Glu Leu Gln Arg Ala Glu Lys Gln Ile Leu
225                 230                 235                 240
Gln Cys Lys Leu Gly Ile Arg Glu Ala Val His Gln Leu Asp Leu Leu
                245                 250                 255
Ser Ser Glu Gly Ser Ile Ala Asp Ala Val Ile Asp Pro Glu Gly Arg
            260                 265                 270
Val Phe His Glu His Ile Phe Cys Ala Lys Cys Lys Met Thr Asp Ala
        275                 280                 285
Leu Pro Asp Asn Asp Ile Ile Leu Cys Asp Gly Ala Cys Asn Arg Gly
    290                 295                 300
Phe His Gln Lys Cys Leu Asp Pro Pro Leu Ala Thr Glu Asn Ile Pro
305                 310                 315                 320
Pro Gly Asp Gln Gly Trp Leu Cys Lys Val Cys Glu Cys Lys Leu Glu
                325                 330                 335
Ser Leu Glu Ala Ile Asn Ala His Leu Gly Thr Gln Phe Thr Val Asp
            340                 345                 350
Asn Ser Trp Glu Gly Ile Phe Ala Asp Ala Ala Arg Ile Ala Asn Gly
        355                 360                 365
Glu Ser Thr Ala Thr Gly Asn Gly Glu Glu Trp Pro Ser Asp Asp Ser
    370                 375                 380
```

Glu Asp Asp Asp Tyr Asp Pro Glu Lys Gln Glu Ile Arg Asp Glu Thr
385 390 395 400
Gly Tyr Glu Asn Asn Met Ser Asp Ser Gly Lys Ser Ser Gly Trp Ser
405 410 415
Glu Ser Tyr Asn Glu Thr Asp Asn Glu Ser Asp Ala Ser Leu Thr Asp
420 425 430
Asn Leu Asn Glu Tyr Ile Ser Lys Gly Lys Lys Arg Ser Arg Asp Thr
435 440 445
Asp Glu Lys Asn Gly Gln Glu Ala Ile Ile Asp Leu Asp Thr Asp Glu
450 455 460
Asn Asp Gly Ser Asp Met Pro Val Ser Gly Arg Arg Gln Arg Arg Asp
465 470 475 480
Val Asp Tyr Lys Lys Leu His Asp Glu Met Phe Gly Lys Asp Gly Pro
485 490 495
Cys Glu Asp Glu Val Ser Glu Asp Glu Asp Trp Gly Pro Asn Arg Arg
500 505 510
Arg Arg Arg Ala Lys Gln Pro Asp Thr Asn Thr Thr Lys Asp Ser Ser
515 520 525
Asn Glu Glu Lys Glu His Val Arg Lys Glu Ala Asp Glu Lys Val Ala
530 535 540
Glu His Ser Gln Ser Ser Ile Lys Asp Lys Lys His Ile Val Arg Leu
545 550 555 560
Pro Ala Asn Ala Val Glu Lys Leu Arg Lys Val Phe Ala Glu Asn Glu
565 570 575
Leu Pro Ser Arg Ser Tyr Lys Glu Asn Leu Ser Gln Gln Leu Gly Leu
580 585 590
Thr Phe Arg Lys Val His Met Trp Phe Lys Asn Ala Arg Tyr Met Ser
595 600 605
Leu Lys Ser Arg Lys Glu Met Pro Arg Asn Glu Asn Cys Asn Glu Thr
610 615 620
Asn Leu Met Gly Glu Gly Asp Met Gly Pro Lys Lys Lys Ile Pro Lys
625 630 635 640
Glu Lys Lys Ala Glu Ser Thr Cys Leu Leu Ser Ser Val Arg Ala Glu
645 650 655
Tyr Lys Ser Arg Asn Thr Lys Arg Lys His Arg Arg Lys Asn Ile Lys
660 665 670
Ala Pro Leu Ser Ile Ser Ser Arg Ser Glu Arg Lys Arg Thr Met Cys
675 680 685
Arg Leu Pro Ala Asn Ala Val Glu Arg Phe Arg Gln Val Phe Ala Glu
690 695 700
Asn Glu Leu Pro Ser Met Ser Cys Lys Met Ala Leu Ser Lys Gln Leu
705 710 715 720
Asp Leu Pro Tyr Arg Gln Val His Met Trp Phe Lys Asn Ala Arg Tyr
725 730 735
Met Ser Leu Lys Lys Lys Lys Met Asn Arg Leu Lys Glu Ser Ala Pro
740 745 750
Val Ile Ser Ser His Thr Arg Arg Leu Glu Val Glu Ser Ser Lys Pro
755 760 765
Asn Pro Gln Ile Lys Glu Pro Asp Asp Ala Gly Asn Ser Tyr Leu Thr
770 775 780
Glu Met Glu Lys Leu Gly Asn Ile Glu Leu Lys Leu Glu Asn Met Arg
785 790 795 800
Lys Ile Leu Glu Thr Val Cys Pro Lys Arg Gly Thr Asp Phe Ser Asn
805 810 815
Asp Lys Asn Gln Glu Gly Ile Ala Asn Leu Leu Ser Glu Glu Leu Leu
820 825 830
Val Tyr Val Pro Val Val Glu Leu Arg Glu Lys Ser Leu Gln Val Ala
835 840 845
Pro Val Arg
850

<210> 189
<211> 2178

<212> DNA
<213> Populus tremuloides

<400> 189

```
atgggtgatt ctggaaagac gtcaaagctg caagactcgc acaaatgttc accttctgac      60
acagtgactg ggtcgttgct gattaaatca ttgaagataa agaaggacag caaaatatct     120
cctagaaaag gccaaaaaac gaaaacaaaa tcaaaaccaa aaccaatacc tcatctaaaa     180
actatcatca gttcagctgt atcaaaaaga aaagtttctc ctaagggcat tgggaatggc     240
tctaccagta gaaaactgat tcataggaaa attctgcata aagcacttga taagaaggcc     300
tcaaggaagg gggcttcctc agggctacaa ttgtcaacta ttgattctaa gggaaatgga     360
aaaaatggtg atgaaggtgc aattaaaaaa ctcaagaaga ggaagcctaa gaaaaggcaa     420

agggacaagg taaagctaga tgaaccatca cgattgcaga ggagagcaag gtacctgatg     480
attaaaatga agctggacca gaaccttata gatgcttact ctggagaagg ctggaaaggt     540
caaagtcgag agaagatcag gccagaaaag gaactactgc gagcaaggaa gcagattttg     600
aagtgtaaac ttggtttacg tgatataatt cggcaggtgg attctcttag tacagtagga     660
tgtattgaag agacagttat ggctccagat ggatctgttt cacatgaaca tatattctgt     720
gcgaagtgca aattgaatga agtttcccca gataatgata ttgtactctg tgatggtaca     780
tgcaactgtg ctttccacca aaaatgcctt gaacctccct tggatactga aagtatacct     840
ccaggagatc agggatggtt ttgcaagttc tgtgaatgca ggatggatat tatagaagct     900
atgaatgctc atttgggaac ccatttctca gaggacagca gttggcagga tattttcaca     960
gaagaagcag caattcctga tgctggaaat gtgctattaa accccgaaga agaatggcct    1020
tcagatgatt ctgaagatga taattacgat ccagagagga gagacaacat catgagtgaa    1080
gcgggtactg atgatgatgc atctgatgac atcagcagtt caaccagctt gggttggtct    1140
tcagatggtg aggttttttt gggatccagg aggtgggaga tgcatggctt ggacttcaga    1200
aacaattcta tttatagcag tttagattct gatgaaacca gtgatgggga aattgtttgt    1260
ggccgtaggc agagaagagc aattgactat aagaagttat atgatgaggt gtttggaaag    1320
gatgctcctg cacacgagca agccagtgaa gacgaagatt ggggtcctgg caaaagaaag    1380
cgacgggaga aggagtcaaa tgcagccagc acccttatga cactgtgtga aagtaaaaaa    1440
aaaagtaaaa gtgatgagac cattgaaggt atgatgaacc ttccccctca aactcgaagg    1500
ccaattttca gactcccccc tgatgcagtt gagaaacttc gccaagtatt tgtagaaaat    1560
gaacttccat ctagaactgt caaggagaat ctgtcaaaag aattgggcct tgaacctggg    1620
aaggttagca aatggttcaa gaattcccgt tatttagctc tgaagtcccg gaaggtagag    1680
aaaggagaac aacttcataa ctctagttcc aaagtctctg ctgaacccac attaaatgtc    1740
atggagggca aaactgctga tctttcacag gattcttggg aagaaactga ggtatgtatc    1800
ccaaagaatt tgaaaaggat ccttcagagg aagaagctga agtcaataag cagaagttta    1860
aggaaaaatg aacagaaaag aggttcctac gaatcaccta ctaaaagcaa tgagatgaat    1920
gctgagtgca gtgatgatgt gagcttgaag aagttgttaa aagcaaaacc aaagggagta    1980
aagaaaaagg ttaatcctat ttcagtagcg gctgaatatg atatggagag actctgtaga    2040
gcgaaggcta tattagagaa gatgaagcag aaagtggtga agctacaaaa tggcaaagct    2100
aggaagtcat ccaaaacccg tccacttgaa gaatttatcg tttacattcc cattgctgag    2160
ttgaggggaa aaaattga                                                   2178
```

<210> 190
<211> 725
<212> PRT
<213> Populus tremuloides

<400> 190

```
Met Gly Asp Ser Gly Lys Thr Ser Lys Leu Gln Asp Ser His Lys Cys
1               5                   10                  15
Ser Pro Ser Asp Thr Val Thr Gly Ser Leu Leu Ile Lys Ser Leu Lys
            20                  25                  30
Ile Lys Lys Asp Ser Lys Ile Ser Pro Arg Lys Gly Gln Lys Thr Lys
        35                  40                  45
Thr Lys Ser Lys Pro Lys Pro Ile Pro His Leu Lys Thr Ile Ile Ser
    50                  55                  60
Ser Ala Val Ser Lys Arg Lys Val Ser Pro Lys Gly Ile Gly Asn Gly
65                  70                  75                  80
```

```
Ser Thr Ser Arg Lys Leu Ile His Arg Lys Ile Leu His Lys Ala Leu
                85                  90                  95
Asp Lys Lys Ala Ser Arg Lys Gly Ala Ser Ser Gly Leu Gln Leu Ser
            100                 105                 110
Thr Ile Asp Ser Lys Gly Asn Gly Lys Asn Gly Asp Glu Gly Ala Ile
        115                 120                 125
Lys Lys Leu Lys Lys Arg Lys Pro Lys Lys Arg Gln Arg Asp Lys Val
    130                 135                 140
Lys Leu Asp Glu Pro Ser Arg Leu Gln Arg Arg Ala Arg Tyr Leu Met
145                 150                 155                 160
Ile Lys Met Lys Leu Asp Gln Asn Leu Ile Asp Ala Tyr Ser Gly Glu
                165                 170                 175
Gly Trp Lys Gly Gln Ser Arg Glu Lys Ile Arg Pro Glu Lys Glu Leu
            180                 185                 190
Leu Arg Ala Arg Lys Gln Ile Leu Lys Cys Lys Leu Gly Leu Arg Asp
        195                 200                 205
Ile Ile Arg Gln Val Asp Ser Leu Ser Thr Val Gly Cys Ile Glu Glu
    210                 215                 220
Thr Val Met Ala Pro Asp Gly Ser Val Ser His Glu His Ile Phe Cys
225                 230                 235                 240
Ala Lys Cys Lys Leu Asn Glu Val Ser Pro Asp Asn Asp Ile Val Leu
                245                 250                 255
Cys Asp Gly Thr Cys Asn Cys Ala Phe His Gln Lys Cys Leu Glu Pro
            260                 265                 270
Pro Leu Asp Thr Glu Ser Ile Pro Pro Gly Asp Gln Gly Trp Phe Cys
        275                 280                 285
Lys Phe Cys Glu Cys Arg Met Asp Ile Ile Glu Ala Met Asn Ala His
    290                 295                 300
Leu Gly Thr His Phe Ser Glu Asp Ser Ser Trp Gln Asp Ile Phe Thr
305                 310                 315                 320
Glu Glu Ala Ala Ile Pro Asp Ala Gly Asn Val Leu Leu Asn Pro Glu
                325                 330                 335
Glu Glu Trp Pro Ser Asp Asp Ser Glu Asp Asp Asn Tyr Asp Pro Glu
            340                 345                 350
Arg Arg Asp Asn Ile Met Ser Glu Ala Gly Thr Asp Asp Asp Ala Ser
        355                 360                 365
Asp Asp Ile Ser Ser Ser Thr Ser Leu Gly Trp Ser Ser Asp Gly Glu
    370                 375                 380
Val Phe Leu Gly Ser Arg Arg Trp Glu Met His Gly Leu Asp Phe Arg
385                 390                 395                 400
Asn Asn Ser Ile Tyr Ser Ser Leu Asp Ser Asp Glu Thr Ser Asp Gly
                405                 410                 415
Glu Ile Val Cys Gly Arg Arg Gln Arg Arg Ala Ile Asp Tyr Lys Lys
            420                 425                 430
Leu Tyr Asp Glu Val Phe Gly Lys Asp Ala Pro Ala His Glu Gln Ala
        435                 440                 445
Ser Glu Asp Glu Asp Trp Gly Pro Gly Lys Arg Lys Arg Arg Glu Lys
    450                 455                 460
Glu Ser Asn Ala Ala Ser Thr Leu Met Thr Leu Cys Glu Ser Lys Lys
465                 470                 475                 480
Lys Ser Lys Ser Asp Glu Thr Ile Glu Gly Met Met Asn Leu Pro Pro
                485                 490                 495
Gln Thr Arg Arg Pro Ile Phe Arg Leu Pro Pro Asp Ala Val Glu Lys
            500                 505                 510
Leu Arg Gln Val Phe Val Glu Asn Glu Leu Pro Ser Arg Thr Val Lys
        515                 520                 525

Glu Asn Leu Ser Lys Glu Leu Gly Leu Glu Pro Gly Lys Val Ser Lys
    530                 535                 540
Trp Phe Lys Asn Ser Arg Tyr Leu Ala Leu Lys Ser Arg Lys Val Glu
545                 550                 555                 560
```

```
Lys Gly Glu Gln Leu His Asn Ser Ser Ser Lys Val Ser Ala Glu Pro
                565                 570                 575
Thr Leu Asn Val Met Glu Gly Lys Thr Ala Asp Leu Ser Gln Asp Ser
            580                 585                 590
Trp Glu Glu Thr Glu Val Cys Ile Pro Lys Asn Leu Lys Arg Ile Leu
        595                 600                 605
Gln Arg Lys Lys Leu Lys Ser Ile Ser Arg Ser Leu Arg Lys Asn Glu
    610                 615                 620
Gln Lys Arg Gly Ser Tyr Glu Ser Pro Thr Lys Ser Asn Glu Met Asn
625                 630                 635                 640
Ala Glu Cys Ser Asp Asp Val Ser Leu Lys Lys Leu Leu Lys Ala Lys
                645                 650                 655
Pro Lys Gly Val Lys Lys Lys Val Asn Pro Ile Ser Val Ala Ala Glu
            660                 665                 670
Tyr Asp Met Glu Arg Leu Cys Arg Ala Lys Ala Ile Leu Glu Lys Met
        675                 680                 685
Lys Gln Lys Val Val Lys Leu Gln Asn Gly Lys Ala Arg Lys Ser Ser
    690                 695                 700
Lys Thr Arg Pro Leu Glu Glu Phe Ile Val Tyr Ile Pro Ile Ala Glu
705                 710                 715                 720
Leu Arg Gly Lys Asn
                725
```

<210> 191
<211> 2256
<212> DNA
<213> Saccharum officinarum

<400> 191

```
atgcattctt cggaaaataa actggtatgc tccaactcgg gaagatcatc aaaagggaat     60
gagacctcta tggagttggt tcctgtacca aaaagaccaa ctagacatga tgcttcccgc    120
caatgcaaat ctgattctcc tttgaagcga tcaccaagga aagctagaaa tgctactctg    180
gcaaaaagca taaagaataa atatcattgt agtcccctca aacagcggag ggcttcagat    240
tctgtttctg gaaaagttgc gacaggacta actgtgagga ggaggaagaa aagaaaaatg    300
caaaatactg atgaggcaac tcgcttggaa cgaagagcga gatattttct aatcaagata    360
aaattggagc agaatttgct agatgcttac tctggagatg gatggaatgg gcaaagccgg    420
gaaaaaataa agccagataa ggatctgcgg cgtgccagga aacaaatcat aaaatgcaaa    480
attgctatac gtgatatcat tcgccaactt ggtttgtata cttctactgg gagtcttaat    540
gacccagcaa tgcctccaga tcagtctacc aatccggaac ataccatgtg ctcaacatgc    600
aagtctcatg aatcatttcc cagcaataaa tttatcttct gtgaagggcc ctgcaaaagg    660
gcatatcatg agaaatgttt ggaacctccc ttaaacaaaa gcgtacttcc aacaagtagc    720
catggatggc tttgcaaatt ctgtttgtgc aaagtgaaaa ttttagaaac tattaatgca    780
catctaggaa caagttttac agtgaagtgc cccttcgaag atatattcaa ggaagcaacg    840
gaacaaatag actccgagga tgcactagat gaagattggc tttctgagta ttcaggtgat    900
gaggactatg atcctgatga aaatgaggac agcggcaact gtatggacag cggggaggaa    960
attatgtctg atgattccga tggttcagga agccccottt attctccaaa tgacgatatt   1020
cctgacttca tatcagcaga cttaaatgac gtggaagggt tttgtcacac caatttagac   1080
ttaggcattg atgccgttga agatgatttg gcacagatcc ttacctacca aaggccaaga   1140
agagatgttg attatagaag gcttaatgag gaaatgtttg gaaaaataat aggaaatgaa   1200
ggacagagtg aggatgaaga ttggggccat gaaaggagaa agaaaaggac ttgttcaggt   1260
ggtgctgggg ataattctgt gggtttctca aatgttatat ctgaggaaaa gagccaaaag   1320
aaggggagaa aacttttcag gattcctcct gcagctgtcg aggtacttcg caaagctttt   1380
gctgaaaatg agcttccacc ccgggatgtc aaagaaaatc tctcaagaga attgggaatt   1440
tcttttgaaa agattgataa atggttcaaa aatacacggt gtgctgctct cagagatcgc   1500
aaggctgaag ggaacagtca taatacggct cccagcaaaa gctcaagaaa taaaggaaaa   1560
gctggaatct caagccaggc tgaaaggaat ggtcatgtta ctggtccctg caataactcg   1620
agaacaaatg aagaaaaatc gttgacacta gctcctaatg cccagggaga gaagaatggg   1680
attgcagcag attatgtctt acaaaaatct ggtatatcgg ggaaggttga ttcagtagac   1740
aactcttgct tggttccctt gtctgaaagc atcaatgtgc atacacgatt gcaacagaat   1800
cttgagatga ggaagatgga atcaactagc agtcctgtgt ggctgcacaa taaaggaggt   1860
tgcttgtttc caacaggcca agccaaggag agtacattac ctacaggcaa gccttgtttg   1920

cagtcagaaa taacccattc aacaattaat gaagtgagca ctttagtgca agctacttct   1980
tggatggatg ctgggtcgtg cgctgaggtg caagaagcca ctccttgggt ggacatcggg   2040
gcctcagatt accagccttt cctggacgtg atcgatgaga tgtgcggact tgagtgcagg   2100
ctgcagagac tgaaggagaa catgctctca tctggcatag acggcaaaac cacaggtgag   2160
agtgacatgg gaaaccaggc tgtggtgctc gtgccgaccg ccgagctcaa ggaaaaagcg   2220
ccgcatggca gtttttttgg gcattattgc ccatag                              2256
```

<210> 192
<211> 751
<212> PRT
<213> Saccharum officinarum

<400> 192

```
Met His Ser Ser Glu Asn Lys Leu Val Cys Ser Asn Ser Gly Arg Ser
1               5                   10                  15
Ser Lys Gly Asn Glu Thr Ser Met Glu Leu Val Pro Val Pro Lys Arg
            20                  25                  30
Pro Thr Arg His Asp Ala Ser Arg Gln Cys Lys Ser Asp Ser Pro Leu
        35                  40                  45
Lys Arg Ser Pro Arg Lys Ala Arg Asn Ala Thr Leu Ala Lys Ser Ile
    50                  55                  60
Lys Asn Lys Tyr His Cys Ser Pro Leu Lys Gln Arg Arg Ala Ser Asp
65                  70                  75                  80
Ser Val Ser Gly Lys Val Ala Thr Gly Leu Thr Val Arg Arg Arg Lys
                85                  90                  95
Lys Arg Lys Met Gln Asn Thr Asp Glu Ala Thr Arg Leu Glu Arg Arg
            100                 105                 110
Ala Arg Tyr Phe Leu Ile Lys Ile Lys Leu Glu Gln Asn Leu Leu Asp
        115                 120                 125
Ala Tyr Ser Gly Asp Gly Trp Asn Gly Gln Ser Arg Glu Lys Ile Lys
    130                 135                 140
Pro Asp Lys Asp Leu Arg Arg Ala Arg Lys Gln Ile Ile Lys Cys Lys
145                 150                 155                 160
Ile Ala Ile Arg Asp Ile Ile Arg Gln Leu Gly Leu Tyr Thr Ser Thr
                165                 170                 175
Gly Ser Leu Asn Asp Pro Ala Met Pro Pro Asp Gln Ser Thr Asn Pro
            180                 185                 190
Glu His Thr Met Cys Ser Thr Cys Lys Ser His Glu Ser Phe Pro Ser
        195                 200                 205
Asn Lys Phe Ile Phe Cys Glu Gly Pro Cys Lys Arg Ala Tyr His Glu
    210                 215                 220
Lys Cys Leu Glu Pro Pro Leu Asn Lys Ser Val Leu Pro Thr Ser Ser
225                 230                 235                 240
His Gly Trp Leu Cys Lys Phe Cys Leu Cys Lys Val Lys Ile Leu Glu
                245                 250                 255
Thr Ile Asn Ala His Leu Gly Thr Ser Phe Thr Val Lys Cys Pro Phe
            260                 265                 270
Glu Asp Ile Phe Lys Glu Ala Thr Glu Gln Ile Asp Ser Glu Asp Ala
        275                 280                 285
Leu Asp Glu Asp Trp Leu Ser Glu Tyr Ser Gly Asp Glu Asp Tyr Asp
    290                 295                 300
Pro Asp Glu Asn Glu Asp Ser Gly Asn Cys Met Asp Ser Gly Glu Glu
305                 310                 315                 320
Ile Met Ser Asp Asp Ser Asp Gly Ser Gly Ser Pro Leu Tyr Ser Pro
                325                 330                 335
Asn Asp Asp Ile Pro Asp Phe Ile Ser Ala Asp Leu Asn Asp Val Glu
            340                 345                 350
Gly Phe Cys His Thr Asn Leu Asp Leu Gly Ile Asp Ala Val Glu Asp
        355                 360                 365
Asp Leu Ala Gln Ile Leu Thr Tyr Gln Arg Pro Arg Arg Asp Val Asp
    370                 375                 380
```

```
Tyr Arg Arg Leu Asn Glu Glu Met Phe Gly Lys Ile Ile Gly Asn Glu
385                 390                 395                 400
Gly Gln Ser Glu Asp Glu Asp Trp Gly His Glu Arg Arg Lys Lys Arg
                405                 410                 415
Thr Cys Ser Gly Gly Ala Gly Asp Asn Ser Val Gly Phe Ser Asn Val
            420                 425                 430
Ile Ser Glu Glu Lys Ser Gln Lys Lys Gly Arg Lys Leu Phe Arg Ile
        435                 440                 445
Pro Pro Ala Ala Val Glu Val Leu Arg Lys Ala Phe Ala Glu Asn Glu
    450                 455                 460
Leu Pro Pro Arg Asp Val Lys Glu Asn Leu Ser Arg Glu Leu Gly Ile
465                 470                 475                 480
Ser Phe Glu Lys Ile Asp Lys Trp Phe Lys Asn Thr Arg Cys Ala Ala
                485                 490                 495
Leu Arg Asp Arg Lys Ala Glu Gly Asn Ser His Asn Thr Ala Pro Ser
            500                 505                 510
Lys Ser Ser Arg Asn Lys Gly Lys Ala Gly Ile Ser Ser Gln Ala Glu
        515                 520                 525
Arg Asn Gly His Val Thr Gly Pro Cys Asn Asn Ser Arg Thr Asn Glu
    530                 535                 540
Glu Lys Ser Leu Thr Leu Ala Pro Asn Ala Gln Gly Glu Lys Asn Gly
545                 550                 555                 560
Ile Ala Ala Asp Tyr Val Leu Gln Lys Ser Gly Ile Ser Gly Lys Val
                565                 570                 575
Asp Ser Val Asp Asn Ser Cys Leu Val Pro Leu Ser Glu Ser Ile Asn
            580                 585                 590
Val His Thr Arg Leu Gln Gln Asn Leu Glu Met Arg Lys Met Glu Ser
        595                 600                 605
Thr Ser Ser Pro Val Trp Leu His Asn Lys Gly Gly Cys Leu Phe Pro
    610                 615                 620
Thr Gly Gln Ala Lys Glu Ser Thr Leu Pro Thr Gly Lys Pro Cys Leu
625                 630                 635                 640
Gln Ser Glu Ile Thr His Ser Thr Ile Asn Glu Val Ser Thr Leu Val
                645                 650                 655
Gln Ala Thr Ser Trp Met Asp Ala Gly Ser Cys Ala Glu Val Gln Glu
            660                 665                 670
Ala Thr Pro Trp Val Asp Ile Gly Ala Ser Asp Tyr Gln Pro Phe Leu
        675                 680                 685
Asp Val Ile Asp Glu Met Cys Gly Leu Glu Cys Arg Leu Gln Arg Leu
    690                 695                 700
Lys Glu Asn Met Leu Ser Ser Gly Ile Asp Gly Lys Thr Thr Gly Glu
705                 710                 715                 720
Ser Asp Met Gly Asn Gln Ala Val Val Leu Val Pro Thr Ala Glu Leu
                725                 730                 735
Lys Glu Lys Ala Pro His Gly Ser Phe Phe Gly His Tyr Cys Pro
            740                 745                 750
```

<210> 193
<211> 2181
<212> DNA
<213> Vitis vinifera

<400> 193

```
atgcgtggaa ctggaaagaa agcaggacat caggaatctg gaaagtcttg ctttcctaaa      60
agaaatatcg ggcctaagct gaatgcagca ttgcagatta aaaatggtag taaaatatct     120
cagaccagga aatgcaagcc aaaatcaaaa tctcatgcaa agacaattgg tgcaatcctg     180
tctaagagaa caactactga ctctccaagc aagggaagca ggagtggatc cacaaccaga     240
aaactgattc acaagaaaac cctgcataaa gccattgata ctgagtcttc caagaaggag     300
tcttcatcaa agcttaaagg tgagaagccc ccacaaatta gcacaaacaa aaatggggaa     360
actgtggaca agaatgttaa acctcaaaaa ttaaagaaaa gaggaaagcg gaagcggcga     420
aaggataatt cagagctaga tgaagcttct cgcttgcaaa gaagaaccag gtacctcttg     480
```

```
atcaaaatga aactggagca gaatcttatt gatgcttact ctggggaagg ttggaaaggt     540
catagtcgag aaaagatcag gccagagaag gaactacaaa gagctacgaa gcaaatattg     600
aaatgtaaac ttgggatccg tgatgcaatt cgccagctgg agtcactgag ctccatagga     660
tgtattgaag atactgcaat tgcttcagat ggatctgttt atcatgaaca cataatctgt     720
gcaaagtgca agttgcggga ggctttccca gataatgata ttatactctg tgatgggacg     780
tgcaattgtg ctttccacca gaaatgtctt gatcctccat tggaaactga aaatattcct     840
ccgggagacc aaggttggtt ttgcaagttc tgcgagtgta agatggaaat actagaagca     900
atgaatgcac atcttggaac ccgcttctct gtggacagta cttggcagga tattttcaaa     960
gaagaagctg ctttgcctga tggtgggagt gcactgccct atccagagga agattggcct    1020
tctgatgatt cacaagatca tgattatgat cctgaaagaa atgaaaatag ctgcagcatc    1080
agcactgctg gcactgaagg aaatgcttca gatgatacaa atagttcttt aagcttgagt    1140
tggtcgtttg aagacgaaat cctttctgga tccaaaagat cagggattat cagtgcagat    1200
tctgatgaaa catctgattg tgaaatcata agtggtcgta ggcaacgaag agctgttgat    1260
tatagaaagt tatatgatga aatgtttgga aaggatgctc atgcaaatga acaagttagt    1320
gaagatgaag actggggtcc tgctaacaaa aggcggagag aaaaggagtc tgatgcagcc    1380
agcaccctca taactctata tgaaggtgag aaaaagttac ctaatgtgga aaccatggaa    1440
gccaaacaaa aaatttcttc agatccacaa actaaaaggc cattttccag aattccactt    1500
gatgcggttg agaagcttcg ccaagcattt ggggagaatg aacttccctc tagagatgtg    1560
agggagaatc ttgcaaagca gttgggtctt gattatgaga aggtgaacaa atggttcaaa    1620
aatgcacgat atatagcgct aaaaacaaga aaggcagaaa gagcaaaaca acttcagact    1680
tctcccagaa tctccaagga atccagatca gaaatcgtga aggacaaaac tgttgatcta    1740
gtggcatcaa gggataactc atcagcatca ctggtccgtg cactaaagaa tttgaaaaag    1800
gtgcggagga gaaaaaatcc aaagccaata atgaccagcc ctgtaaagaa aaagcatcat    1860
agaagggctt tacttgagtc acctacaaat gacaaggtta ctatggagtt tgacgatgat    1920
gtgagcttga agaaacaact gaagcttttg aaagaaaaaa gtaagaggga caagcaaagg    1980
gtcgacttca aggagggaac tggtgtacaa gatgcagaga aggagatgga gagactctgc    2040
caaattaagg ataagataga gaagttgaag caggtaatac tgagacttca atgtgacaaa    2100
actaatcaat ggcaagacca gtcagtgatc tatgttcctg tggcagagct aagggagaaa    2160
ggtcgatttt gcatcacatc t                                              2181
```

<210> 194
<211> 727
<212> PRT
<213> Vitis vinifera

<400> 194

```
Met Arg Gly Thr Gly Lys Lys Ala Gly His Gln Glu Ser Gly Lys Ser
1               5                   10                  15
Cys Phe Pro Lys Arg Asn Ile Gly Pro Lys Leu Asn Ala Ala Leu Gln
            20                  25                  30
Ile Lys Asn Gly Ser Lys Ile Ser Gln Thr Arg Lys Cys Lys Pro Lys
        35                  40                  45
Ser Lys Ser His Ala Lys Thr Ile Gly Ala Ile Leu Ser Lys Arg Thr
    50                  55                  60
Thr Thr Asp Ser Pro Ser Lys Gly Ser Arg Ser Gly Ser Thr Thr Arg
65                  70                  75                  80
Lys Leu Ile His Lys Lys Thr Leu His Lys Ala Ile Asp Thr Glu Ser
                85                  90                  95
Ser Lys Lys Glu Ser Ser Ser Lys Leu Lys Gly Glu Lys Pro Pro Gln
            100                 105                 110
Ile Ser Thr Asn Lys Asn Gly Glu Thr Val Asp Lys Asn Val Lys Pro
        115                 120                 125
Gln Lys Leu Lys Lys Arg Gly Lys Arg Lys Arg Arg Lys Asp Asn Ser
    130                 135                 140
Glu Leu Asp Glu Ala Ser Arg Leu Gln Arg Arg Thr Arg Tyr Leu Leu
145                 150                 155                 160
Ile Lys Met Lys Leu Glu Gln Asn Leu Ile Asp Ala Tyr Ser Gly Glu
                165                 170                 175
Gly Trp Lys Gly His Ser Arg Glu Lys Ile Arg Pro Glu Lys Glu Leu
            180                 185                 190
Gln Arg Ala Thr Lys Gln Ile Leu Lys Cys Lys Leu Gly Ile Arg Asp
```

```
        195                 200                 205
Ala Ile Arg Gln Leu Glu Ser Leu Ser Ser Ile Gly Cys Ile Glu Asp
    210                 215                 220
Thr Ala Ile Ala Ser Asp Gly Ser Val Tyr His Glu His Ile Ile Cys
225                 230                 235                 240
Ala Lys Cys Lys Leu Arg Glu Ala Phe Pro Asp Asn Asp Ile Ile Leu
                245                 250                 255
Cys Asp Gly Thr Cys Asn Cys Ala Phe His Gln Lys Cys Leu Asp Pro
            260                 265                 270
Pro Leu Glu Thr Glu Asn Ile Pro Pro Gly Asp Gln Gly Trp Phe Cys
        275                 280                 285
Lys Phe Cys Glu Cys Lys Met Glu Ile Leu Glu Ala Met Asn Ala His
    290                 295                 300
Leu Gly Thr Arg Phe Ser Val Asp Ser Thr Trp Gln Asp Ile Phe Lys
305                 310                 315                 320
Glu Glu Ala Ala Leu Pro Asp Gly Gly Ser Ala Leu Pro Tyr Pro Glu
                325                 330                 335
Glu Asp Trp Pro Ser Asp Asp Ser Gln Asp His Asp Tyr Asp Pro Glu
            340                 345                 350
Arg Asn Glu Asn Ser Cys Ser Ile Ser Thr Ala Gly Thr Glu Gly Asn
        355                 360                 365
Ala Ser Asp Asp Thr Asn Ser Ser Leu Ser Leu Ser Trp Ser Phe Glu
    370                 375                 380
Asp Glu Ile Leu Ser Gly Ser Lys Arg Ser Gly Ile Ile Ser Ala Asp
385                 390                 395                 400
Ser Asp Glu Thr Ser Asp Cys Glu Ile Ile Ser Gly Arg Arg Gln Arg
                405                 410                 415
Arg Ala Val Asp Tyr Arg Lys Leu Tyr Asp Glu Met Phe Gly Lys Asp
            420                 425                 430
Ala His Ala Asn Glu Gln Val Ser Glu Asp Glu Asp Trp Gly Pro Ala
        435                 440                 445
Asn Lys Arg Arg Arg Glu Lys Glu Ser Asp Ala Ala Ser Thr Leu Ile
    450                 455                 460
Thr Leu Tyr Glu Gly Glu Lys Lys Leu Pro Asn Val Glu Thr Met Glu
465                 470                 475                 480
Ala Lys Gln Lys Ile Ser Ser Asp Pro Gln Thr Lys Arg Pro Phe Ser
                485                 490                 495
Arg Ile Pro Leu Asp Ala Val Glu Lys Leu Arg Gln Ala Phe Gly Glu
            500                 505                 510
Asn Glu Leu Pro Ser Arg Asp Val Arg Glu Asn Leu Ala Lys Gln Leu
        515                 520                 525
Gly Leu Asp Tyr Glu Lys Val Asn Lys Trp Phe Lys Asn Ala Arg Tyr
    530                 535                 540
Ile Ala Leu Lys Thr Arg Lys Ala Glu Arg Ala Lys Gln Leu Gln Thr
545                 550                 555                 560
Ser Pro Arg Ile Ser Lys Glu Ser Arg Ser Glu Ile Val Lys Asp Lys
                565                 570                 575
Thr Val Asp Leu Val Ala Ser Arg Asp Asn Ser Ser Ala Ser Leu Val
            580                 585                 590
Arg Ala Leu Lys Asn Leu Lys Lys Val Arg Arg Arg Lys Asn Pro Lys
        595                 600                 605
Pro Ile Met Thr Ser Pro Val Lys Lys Lys His His Arg Arg Ala Leu
    610                 615                 620
Leu Glu Ser Pro Thr Asn Asp Lys Val Thr Met Glu Phe Asp Asp Asp
625                 630                 635                 640
Val Ser Leu Lys Lys Gln Leu Lys Leu Leu Lys Glu Lys Ser Lys Arg
                645                 650                 655
Asp Lys Gln Arg Val Asp Phe Lys Glu Gly Thr Gly Val Gln Asp Ala
            660                 665                 670
Glu Lys Glu Met Glu Arg Leu Cys Gln Ile Lys Asp Lys Ile Glu Lys
        675                 680                 685
```

```
Leu Lys Gln Val Ile Leu Arg Leu Gln Cys Asp Lys Thr Asn Gln Trp
    690                 695                 700
Gln Asp Gln Ser Val Ile Tyr Val Pro Val Ala Glu Leu Arg Glu Lys
705                 710                 715                 720
Gly Arg Phe Cys Ile Thr Ser
                725
```

<210> 195
<211> 2166
<212> DNA
<213> Zea mays

<400> 195

```
atgcattctt cggaaaataa actgggttgg aatgagacct ctatggggtt ggttcctgta      60
ccaaaaagac cagctagacc tgatgcttcc caccaatgca aatctgattc ctttatgaag     120
cgatcaccaa ggaaagttag aaatgctact ctggcaaaaa gcataaagag caaataccac     180
tatagtcccc tcaaacagcg gaagggttca gattctgttc ctgggaaaat cgtaacagga     240
ctaaccgcaa ggaaaaagaa gaaaaggaaa atccaaatta cagacgaggc aactcgtttg     300
gaacgaagag cgagatattt tctaatcaag ataaaactgg agcagaattt gctagatgct     360
tactctggag atggatggaa tggcaaagc cgagagaaaa taaagccaga gaaggaactg      420
caacgtgcca ggaaacaaat cataaaatgc aaaattgcta tacgtgatat catccgccaa     480
cttgatttgt atacttctac tgggagtgtt gatgacccac taatgcctac agatcagtcc     540
accaatcccg aacataccat gtgctcaaca tgcaagtctc atgaatcatt tcccagcaat     600
aaaattatct tctgcaaagg gccctgcaaa agggcatgtc atgagaaatg tttggaacct     660
cccttaaaca aaagcgtact tccaacaagt agccatgggt ggctttgcaa attctgtttg     720
tgcaaggtga ggattttaga aactattaat gcacatctag ggacgagttt tacagtgaag     780
tgccactttg aagatatatt caaggaaact actgaactaa tagactctga ggatgcacta     840
gatgaagatt ggctttctga gtattcaggt gatgaggact atgatcctga tgaaaatgag     900
gccagtggcg actgtatgga cagcggggag aagattatgt ctgatgattc caatggttca     960
ggaagccccc tttattctcc aaatgacgat attcctgact tcatatcagc agacttaaat    1020
gttgtggaag ggttttgtca taccaattta gatttaggca ttgatgccgt tgaagatgat    1080
tttgcacaga tcctcaccta ccaaaggcca agaagagatg ttgattatag aaggcttaac    1140
gaggaaatgt ttgggaaaat aaccgggaat gaagaacaga gcgaggacga agattggggc    1200
catgaaagga gaaagaaaag gacgcattca ggtgttgctg ggataattc tgttggtttc     1260
ttgaacgtta tatctgatga aaagagccag aagaagggga gaaaactttt caggattcct    1320
cctgcagctg ttgaggtact tcgcagagct tttgctgaaa atgagcttcc accccgggat    1380
gttaaagaaa atctctcaag agaattggga atttcttttg aaaagattga taaatggttc    1440
aaaaatacac ggtgtgctgc tctcagagat cggaaggctg aaggaaacag tcataataca    1500
gctcccagca aaagctcaaa aaataaagga aaagctggaa tctcaggcaa gactggaagg    1560
aatggtcatg ttactggtcc ctgcaataat ttgagaacaa atgaagaaaa aacaggtata    1620
tcggggaagt ttgattcagg agacaactct tgtttggttc ccttctctga agtcatcaat    1680
gtgcctacgc gattgccaca caactttgag atgaggaaga tggaatcaac tagaagtcct    1740
gcgaggctac acaataaagg agggttcctg tctgcaacag tccaaattaa ggagagtacg    1800
ttgttaccca caggcaagcc ttgttggcag tcagaaatga gccatccaac aactaatgaa    1860
gtgagcactt cagcgcaagc tactacttgg atcgacgcgg gggcgtgtgc cgaggagcaa    1920
gaacctactc cttgggtgga catcggggtc tcagactacc agcctttcct ggatgtgatc    1980
gacgacatgt gcggacttga gtggaggctg cagagactga aggagaacat gctctcatct    2040
agcatagacg gccaaaccgc cgccagtgag agtgacaaaa gaaaccagac cgtggtgcta    2100
gtgccaaccg ccgagctcaa ggaaaagctg ccgcatgacg gtttattcgg gcattattgc    2160
ccatag                                                               2166
```

<210> 196
<211> 721
<212> PRT
<213> Zea mays

<400> 196

```
Met His Ser Ser Glu Asn Lys Leu Gly Trp Asn Glu Thr Ser Met Gly
1               5                   10                  15
Leu Val Pro Val Pro Lys Arg Pro Ala Arg Pro Asp Ala Ser His Gln
            20                  25                  30
```

```
Cys Lys Ser Asp Ser Phe Met Lys Arg Ser Pro Arg Lys Val Arg Asn
        35                  40                  45
Ala Thr Leu Ala Lys Ser Ile Lys Ser Lys Tyr His Tyr Ser Pro Leu
    50                  55                  60
Lys Gln Arg Lys Gly Ser Asp Ser Val Pro Gly Lys Ile Val Thr Gly
65                  70                  75                  80
Leu Thr Ala Arg Lys Lys Lys Lys Arg Lys Ile Gln Ile Thr Asp Glu
                85                  90                  95
Ala Thr Arg Leu Glu Arg Arg Ala Arg Tyr Phe Leu Ile Lys Ile Lys
            100                 105                 110
Leu Glu Gln Asn Leu Leu Asp Ala Tyr Ser Gly Asp Gly Trp Asn Gly
        115                 120                 125
Gln Ser Arg Glu Lys Ile Lys Pro Glu Lys Glu Leu Gln Arg Ala Arg
    130                 135                 140
Lys Gln Ile Ile Lys Cys Lys Ile Ala Ile Arg Asp Ile Ile Arg Gln
145                 150                 155                 160
Leu Asp Leu Tyr Thr Ser Thr Gly Ser Val Asp Asp Pro Leu Met Pro
                165                 170                 175
Thr Asp Gln Ser Thr Asn Pro Glu His Thr Met Cys Ser Thr Cys Lys
            180                 185                 190
Ser His Glu Ser Phe Pro Ser Asn Lys Ile Ile Phe Cys Lys Gly Pro
        195                 200                 205
Cys Lys Arg Ala Cys His Glu Lys Cys Leu Glu Pro Pro Leu Asn Lys
    210                 215                 220
Ser Val Leu Pro Thr Ser Ser His Gly Trp Leu Cys Lys Phe Cys Leu
225                 230                 235                 240
Cys Lys Val Arg Ile Leu Glu Thr Ile Asn Ala His Leu Gly Thr Ser
                245                 250                 255
Phe Thr Val Lys Cys His Phe Glu Asp Ile Phe Lys Glu Thr Thr Glu
            260                 265                 270
Leu Ile Asp Ser Glu Asp Ala Leu Asp Glu Asp Trp Leu Ser Glu Tyr
        275                 280                 285
Ser Gly Asp Glu Asp Tyr Asp Pro Asp Glu Asn Glu Ala Ser Gly Asp
    290                 295                 300
Cys Met Asp Ser Gly Glu Lys Ile Met Ser Asp Asp Ser Asn Gly Ser
305                 310                 315                 320
Gly Ser Pro Leu Tyr Ser Pro Asn Asp Asp Ile Pro Asp Phe Ile Ser
                325                 330                 335
Ala Asp Leu Asn Val Val Glu Gly Phe Cys His Thr Asn Leu Asp Leu
            340                 345                 350
Gly Ile Asp Ala Val Glu Asp Asp Phe Ala Gln Ile Leu Thr Tyr Gln
        355                 360                 365
Arg Pro Arg Arg Asp Val Asp Tyr Arg Arg Leu Asn Glu Glu Met Phe
    370                 375                 380
Gly Lys Ile Thr Gly Asn Glu Glu Gln Ser Glu Asp Glu Asp Trp Gly
385                 390                 395                 400
His Glu Arg Arg Lys Lys Arg Thr His Ser Gly Val Ala Gly Asp Asn
                405                 410                 415
Ser Val Gly Phe Leu Asn Val Ile Ser Asp Glu Lys Ser Gln Lys Lys
            420                 425                 430
Gly Arg Lys Leu Phe Arg Ile Pro Pro Ala Ala Val Glu Val Leu Arg
        435                 440                 445
Arg Ala Phe Ala Glu Asn Glu Leu Pro Pro Arg Asp Val Lys Glu Asn
    450                 455                 460
Leu Ser Arg Glu Leu Gly Ile Ser Phe Glu Lys Ile Asp Lys Trp Phe
465                 470                 475                 480
Lys Asn Thr Arg Cys Ala Ala Leu Arg Asp Arg Lys Ala Glu Gly Asn
                485                 490                 495
Ser His Asn Thr Ala Pro Ser Lys Ser Ser Lys Asn Lys Gly Lys Ala
            500                 505                 510
Gly Ile Ser Gly Lys Thr Gly Arg Asn Gly His Val Thr Gly Pro Cys
```

```
            515                 520                 525
Asn Asn Leu Arg Thr Asn Glu Glu Lys Thr Gly Ile Ser Gly Lys Phe
        530                 535                 540
Asp Ser Gly Asp Asn Ser Cys Leu Val Pro Phe Ser Glu Val Ile Asn
545                 550                 555                 560
Val Pro Thr Arg Leu Pro His Asn Phe Glu Met Arg Lys Met Glu Ser
                565                 570                 575
Thr Arg Ser Pro Ala Arg Leu His Asn Lys Gly Gly Phe Leu Ser Ala
            580                 585                 590
Thr Val Gln Ile Lys Glu Ser Thr Leu Leu Pro Thr Gly Lys Pro Cys
        595                 600                 605
Trp Gln Ser Glu Met Ser His Pro Thr Thr Asn Glu Val Ser Thr Ser
    610                 615                 620
Ala Gln Ala Thr Thr Trp Ile Asp Ala Gly Ala Cys Ala Glu Glu Gln
625                 630                 635                 640
Glu Pro Thr Pro Trp Val Asp Ile Gly Val Ser Asp Tyr Gln Pro Phe
                645                 650                 655
Leu Asp Val Ile Asp Asp Met Cys Gly Leu Glu Trp Arg Leu Gln Arg
            660                 665                 670
Leu Lys Glu Asn Met Leu Ser Ser Ser Ile Asp Gly Gln Thr Ala Ala
        675                 680                 685
Ser Glu Ser Asp Lys Arg Asn Gln Thr Val Val Leu Val Pro Thr Ala
    690                 695                 700
Glu Leu Lys Glu Lys Leu Pro His Asp Gly Leu Phe Gly His Tyr Cys
705                 710                 715                 720
Pro
```

<210> 197
<211> 2172
<212> DNA
<213> Arabidopsis thaliana

<400> 197

```
atgtataaag cagtaagtaa gcgggtaact agaagtagtg gatctggttt gaagcagaca     60
aatgtagata atggaggaga gattagtcca actgttgaca gagtatctga acaaggcaaa    120
tcatcagagg cagggagtca catgccaact gatgcaaacg gaaatggaca tttgcatcat    180
gagattatgg atcatggaaa ggggaatgaa gaacagaaac caacccctca aaccgtgaag    240
aaggattcta ataccaatac caaattttca gggtctcatc gtgagcttgt catcggtctt    300
ccttgccgtg gacaatttga aatccacaac cggtccaggg catctacgag ttcgaagagg    360
ttaggaggag gtggagaaag gaatgtgttg tttgcaagtc acaagagagc tcagagatct    420
aaggaagatg ctggaccatc ttcggttgtt gctaattcca cacctgtagg tcgaccaaag    480
aaaaagaata agactatgaa caaaggacaa gttagagaag atgatgagta cacaagaatc    540
aaaaagaaac ttcgatactt tctaaaccgg attaactatg agcaaagcct aattgacgcc    600
tattcgttag aaggctggaa aggttcaagc ctggaaaaga taaggccaga gaaagagctt    660
gaacgagcca caaaggaaat tttgcggcgc aagcttaaaa ttagagatct tttcagcac     720
cttgacacac tctgtgctga aggaagcctt ccagagtctt tatttgatac tgatggagag    780
atttctagcg aggatatctt ctgtgcaaaa tgtggttcaa aggacttgtc tgttgataat    840
gatatcatac tatgcgatgg gttttgcgat cgaggctttc accaatactg tcttgaacca    900
cctctgcgga aagaagatat tcctccggat gatgagggtt ggctatgccc cggatgtgat    960
tgcaaagatg acagcttgga cttgcttaat gattctttag ggacaaaatt ttcggtcagc   1020
gacagttggg agaaaatatt tcctgaggca gcagcagcgc tggttggtgg aggtcaaaat   1080
cttgactgtg atcttccttc agatgattct gatgatgaag agtatgatcc agattgtttg   1140
aatgataatg aaaatgatga agatggcagt gatgacaatg aagaatctga gaacgaagat   1200
ggtagttctg atgaaactga attcacatct gcatctgacg aaatgattga gtcatttaaa   1260
gaagggaaag atataatgaa agatgtaatg gcccttccat ctgatgattc tgaggatgat   1320
gactatgatc ctgatgcccc gacttgtgat gatgataaag aaagttcaaa ttctgattgt   1380
acatcagaca ctgaagatct tgaaacttcc ttcaaaggag atgagactaa tcagcaagct   1440
gaagatacac cacttgagga tcctggcaga cagacgtctc agctccaagg tgatgcaatt   1500
ttagagtcag atgttgggct tgatgatggt cctgccggtg tatccagaag gaggaatgtt   1560
gaaaggctgg attacaaaaa gttgtatgat gaggaatatg acaatgttcc gacttcgtca   1620
```

```
agtgatgatg acgattggga taaaactgca agaatgggaa aagaagattc cgagtcggaa   1680
gatgaagggg acactgtacc tctgaaacag tcttcaaatg cagaagatca tacttctaag   1740
aaacttatac ggaagtcgaa aagggcagat aaaaaggata ccttagaaat gccacaagaa   1800
ggtcctggag aaaatggtgg ttctggtgaa attgaaaaaa gcagctcttc agcatgtaaa   1860
cagacagatc ccaaaactca gagactgtac atatcttttc aagagaatca atatccagac   1920
aaagccacga aggagagctt agccaaagag ctacaaatga cagttaagca agttaataac   1980
tggtttaagc ataggcgttg gtcgataaac agcaaacctt tagtttcaga ggaaaatgtc   2040
gagaagttaa aaacaggcaa ggaaggtgaa tgtgagacat ctgttgctgg aagtagcaaa   2100
caaactatgg aaaccgagtc agttgcagag aaaccaacca atactggatc acggaaaaga   2160
agaagaaagt ag                                                       2172
```

<210> 198
<211> 723
<212> PRT
<213> Arabidopsis thaliana

<400> 198

```
Met Tyr Lys Ala Val Ser Lys Arg Val Thr Arg Ser Ser Gly Ser Gly
1               5                   10                  15
Leu Lys Gln Thr Asn Val Asp Asn Gly Gly Glu Ile Ser Pro Thr Val
            20                  25                  30
Asp Arg Val Ser Glu Gln Gly Lys Ser Ser Glu Ala Gly Ser His Met
        35                  40                  45
Pro Thr Asp Ala Asn Gly Asn Gly His Leu His His Glu Ile Met Asp
    50                  55                  60
His Gly Lys Gly Asn Glu Glu Gln Lys Pro Thr Pro Gln Thr Val Lys
65                  70                  75                  80
Lys Asp Ser Asn Thr Asn Thr Lys Phe Ser Gly Ser His Arg Glu Leu
                85                  90                  95
Val Ile Gly Leu Pro Cys Arg Gly Gln Phe Glu Ile His Asn Arg Ser
            100                 105                 110
Arg Ala Ser Thr Ser Ser Lys Arg Leu Gly Gly Gly Gly Glu Arg Asn
        115                 120                 125
Val Leu Phe Ala Ser His Lys Arg Ala Gln Arg Ser Lys Glu Asp Ala
    130                 135                 140
Gly Pro Ser Ser Val Val Ala Asn Ser Thr Pro Val Gly Arg Pro Lys
145                 150                 155                 160
Lys Lys Asn Lys Thr Met Asn Lys Gly Gln Val Arg Glu Asp Asp Glu
                165                 170                 175
Tyr Thr Arg Ile Lys Lys Lys Leu Arg Tyr Phe Leu Asn Arg Ile Asn
            180                 185                 190
Tyr Glu Gln Ser Leu Ile Asp Ala Tyr Ser Leu Glu Gly Trp Lys Gly
        195                 200                 205
Ser Ser Leu Glu Lys Ile Arg Pro Glu Lys Glu Leu Glu Arg Ala Thr
    210                 215                 220
Lys Glu Ile Leu Arg Arg Lys Leu Lys Ile Arg Asp Leu Phe Gln His
225                 230                 235                 240
Leu Asp Thr Leu Cys Ala Glu Gly Ser Leu Pro Glu Ser Leu Phe Asp
                245                 250                 255
Thr Asp Gly Glu Ile Ser Ser Glu Asp Ile Phe Cys Ala Lys Cys Gly
            260                 265                 270
Ser Lys Asp Leu Ser Val Asp Asn Asp Ile Ile Leu Cys Asp Gly Phe
        275                 280                 285
Cys Asp Arg Gly Phe His Gln Tyr Cys Leu Glu Pro Pro Leu Arg Lys
    290                 295                 300
Glu Asp Ile Pro Pro Asp Asp Glu Gly Trp Leu Cys Pro Gly Cys Asp
305                 310                 315                 320
Cys Lys Asp Asp Ser Leu Asp Leu Leu Asn Asp Ser Leu Gly Thr Lys
                325                 330                 335
Phe Ser Val Ser Asp Ser Trp Glu Lys Ile Phe Pro Glu Ala Ala Ala
            340                 345                 350
```

```
Ala Leu Val Gly Gly Gly Gln Asn Leu Asp Cys Asp Leu Pro Ser Asp
        355                 360                 365
Asp Ser Asp Asp Glu Glu Tyr Asp Pro Asp Cys Leu Asn Asp Asn Glu
    370                 375                 380
Asn Asp Glu Asp Gly Ser Asp Asp Asn Glu Glu Ser Glu Asn Glu Asp
385                 390                 395                 400
Gly Ser Ser Asp Glu Thr Glu Phe Thr Ser Ala Ser Asp Glu Met Ile
                405                 410                 415
Glu Ser Phe Lys Glu Gly Lys Asp Ile Met Lys Asp Val Met Ala Leu
            420                 425                 430
Pro Ser Asp Asp Ser Glu Asp Asp Asp Tyr Asp Pro Asp Ala Pro Thr
        435                 440                 445
Cys Asp Asp Asp Lys Glu Ser Ser Asn Ser Asp Cys Thr Ser Asp Thr
    450                 455                 460
Glu Asp Leu Glu Thr Ser Phe Lys Gly Asp Glu Thr Asn Gln Gln Ala
465                 470                 475                 480
Glu Asp Thr Pro Leu Glu Asp Pro Gly Arg Gln Thr Ser Gln Leu Gln
                485                 490                 495
Gly Asp Ala Ile Leu Glu Ser Asp Val Gly Leu Asp Asp Gly Pro Ala
            500                 505                 510
Gly Val Ser Arg Arg Arg Asn Val Glu Arg Leu Asp Tyr Lys Lys Leu
        515                 520                 525
Tyr Asp Glu Glu Tyr Asp Asn Val Pro Thr Ser Ser Ser Asp Asp Asp
    530                 535                 540
Asp Trp Asp Lys Thr Ala Arg Met Gly Lys Glu Asp Ser Glu Ser Glu
545                 550                 555                 560
Asp Glu Gly Asp Thr Val Pro Leu Lys Gln Ser Ser Asn Ala Glu Asp
                565                 570                 575
His Thr Ser Lys Lys Leu Ile Arg Lys Ser Lys Arg Ala Asp Lys Lys
            580                 585                 590
Asp Thr Leu Glu Met Pro Gln Glu Gly Pro Gly Glu Asn Gly Gly Ser
        595                 600                 605
Gly Glu Ile Glu Lys Ser Ser Ser Ser Ala Cys Lys Gln Thr Asp Pro
    610                 615                 620
Lys Thr Gln Arg Leu Tyr Ile Ser Phe Gln Glu Asn Gln Tyr Pro Asp
625                 630                 635                 640
Lys Ala Thr Lys Glu Ser Leu Ala Lys Glu Leu Gln Met Thr Val Lys
                645                 650                 655
Gln Val Asn Asn Trp Phe Lys His Arg Arg Trp Ser Ile Asn Ser Lys
            660                 665                 670
Pro Leu Val Ser Glu Glu Asn Val Glu Lys Leu Lys Thr Gly Lys Glu
        675                 680                 685
Gly Glu Cys Glu Thr Ser Val Ala Gly Ser Ser Lys Gln Thr Met Glu
    690                 695                 700
Thr Glu Ser Val Ala Glu Lys Pro Thr Asn Thr Gly Ser Arg Lys Arg
705                 710                 715                 720
Arg Arg Lys
```

<210> 199
<211> 2901
<212> DNA
<213> Lotus japonicus

<400> 199

```
atgattagtt caactggaga attgacaagc cataacaaca gtagcattga gcacatggaa      60
actgagcaac ctgaattgtc tgaaaaaaca cctcagatag gctctgaatg tctggacggt     120
gaacaaaggg tgcttggtac tgtgtcaacc agttctgtta ataatgaaaa ttcaaatcaa     180
gtttctgcca atgtgaccga gaattctgtt tttcaactac cagcaacacc tcaacattgc     240
ttagaaaaga gtggtcagac tgtaacaagt ccacccctgg aagaaagcac cttgaaacaa     300
gttgctacta atgtatctaa tggtaaatca gaaaacaaat gtcagacatc ctctcaagat     360
```

```
gttcaaaacg aacttgtgga agcaagtgat gtaataactg gctcttttgt tgcggaccaa    420
acacaatcca ttcctgctca agtgaatacg agttctgtga atgaactatt ggatattcct    480
tccggagatg ttgcagtaga agttggttct gatagtttag aaaaaaagtc taaagggtgc    540
ttggtagaca gcgaacaaag ggtacttggt actgtgttaa ccaattctat taatgatgga    600
aattcaaatc aagtttctgc caatgtgacc gagaattctg ttattcaact gccagcacca    660
cctcaacatt gctttgaaaa gagtggacag actgtaaaaa gttcatctct ggaagaaagt    720
accttaaaac aagtctccac taatttatct aacagtaaat cagaaaacat atgtcagaca    780
tcctctcaag atgttcaaaa cgaacttgtg gaaacaagtg atgcagtaac tggctctgtt    840
gttgcagaca aaacacaatc cattcctgct caagtgaata caagttctgt gaatgaacta    900
ttggatattc cttccagaga tgttgcagaa gaagttggtt ctgatagttc agaaaggaag    960
tctaaaagca caactccttc acaattgaga catataggta aaagtaactc aaagttatcg   1020
aaaaagaagt atatcctaag gtcgttagga agcagtgaca gagctttgcg gtccaggaca   1080
aaagaaaaac ctaaagcgcc tgaaccaagt agtaactcag ttgacgttaa taatgatgga   1140
gtgaagaagg aaaaaaggag gaagaagaaa aaaacaggag gggaggaaaa gaaggatcaa   1200
ttttataaaa tcaaagctca cctaagatat ttattaaaca gagtaagcta tgagcaaaac   1260
ttaattgatg cttactctgg tgacggctgg aaaggataca gtatggacaa gttaaagcct   1320
gataaggaga ttcaacgggc taaatctgaa attcttcgac gtaaattgaa aattagggat   1380
ctatttcaaa acttggattc cttgtgtgct gaaggaaagt ttccagaatc tttgtttgat   1440
tctgagggag agattgacag tgaggatata ttctgttcaa tttgccagac caaagagttg   1500
agcactgata atgatataat tctctgtgat ggtgcttgtg accgtggatt tcaccagcac   1560
tgcttggatc ctccattgtt aactgaagac attccacctg gcgatgaggg ttggttatgc   1620
cccggatgtg attgcaaaga tgactgcgtt gaccttctta acgactcctt aggaacacgt   1680
ctctctctta gtgacacctg ggagaaggtt tttcctgaag cagcagctgt tactggaaat   1740
aatatggatc agggacttcc ttctgatgat tctgatgatg atgattataa ccctaatggt   1800
aaagaggatg tggaggttga aggaggtgaa tcaagttctg atgaatctga atatgcttct   1860
gcttctgaga aattggaaga ttcacaccat gaggatccat acttaggcct tccttctgaa   1920
gattcagagg acaatgatta tgatcctagt gctccagatc ttgacaataa agtcacagaa   1980
gaaagttcaa gctctgattt cacatctgac tctgaggatc ttgctgctgc tattgaggat   2040
aacacgtccc ctggacatgt taaacagact aaaagcagac aaaaaagtaa agttggtaag   2100
aacccatcaa tggttgatga agtgtcatct ttacgagagc cagaacttga ggaagagggt   2160
ttcactccag tttctgggaa aagaaatgtg gaaaggctgg actacaaaaa gctgtatgat   2220
gagacataca aaagtgatac tagtgaagat gaagaatgga ctgccagtgc cactccaagt   2280
agaaaaaaga aactctgtgg caaaatgact ccagtgtcat cagatggaaa ggcctcaaat   2340
aattctagac atactcctga aaggaacacc caacaagata aagttgaaaa tacaaataat   2400
tcacccacta aatcacttga aggctgtctg gaatctggct caagggataa aaagcctagg   2460
tcttcaacac gtcaaagact tggagacgtt gtagtgcaga gacttcacaa atcttttaaa   2520
gacaatcagt atccggatcg agccacaaaa gaaagcttgg cagaagaact ggggctcact   2580
tttttccagg ttgacaaatg gtttggcaat gctcgatggg gctttcggcg ttcatcgcgt   2640
atgggagcta gtccaggcga atatgcttcg ccacaggcca ctggcagcgg acctgaaaat   2700
actggggaga gggaacgcga attagcttcc caggaagtcg gtgaagaaaa attgaaaacc   2760
cccagtccta gaaaaaggaa gcatttgtct gaaccgcagg catctgaagc accgcagatt   2820
attgttcttg gcttagcagc atctcctggc tcaccacggg ctcatgcagg caataaaaag   2880
aagacagtga aaaggaaatg a                                             2901
```

<210> 200
<211> 966
<212> PRT
<213> Lotus japonicus

<400> 200

```
Met Ile Ser Ser Thr Gly Glu Leu Thr Ser His Asn Asn Ser Ser Ile
1               5                   10                  15
Glu His Met Glu Thr Glu Gln Pro Glu Leu Ser Glu Lys Thr Pro Gln
            20                  25                  30
Ile Gly Ser Glu Cys Leu Asp Gly Glu Gln Arg Val Leu Gly Thr Val
        35                  40                  45
Ser Thr Ser Ser Val Asn Asn Glu Asn Ser Asn Gln Val Ser Ala Asn
    50                  55                  60
Val Thr Glu Asn Ser Val Phe Gln Leu Pro Ala Thr Pro Gln His Cys
65                  70                  75                  80
Leu Glu Lys Ser Gly Gln Thr Val Thr Ser Pro Pro Leu Glu Glu Ser
```

```
                        85                  90                  95
Thr Leu Lys Gln Val Ala Thr Asn Val Ser Asn Gly Lys Ser Glu Asn
            100                 105                 110
Lys Cys Gln Thr Ser Ser Gln Asp Val Gln Asn Glu Leu Val Glu Ala
        115                 120                 125
Ser Asp Val Ile Thr Gly Ser Phe Val Ala Asp Gln Thr Gln Ser Ile
    130                 135                 140
Pro Ala Gln Val Asn Thr Ser Ser Val Asn Glu Leu Leu Asp Ile Pro
145                 150                 155                 160
Ser Gly Asp Val Ala Val Glu Val Gly Ser Asp Ser Leu Glu Lys Lys
                165                 170                 175
Ser Lys Gly Cys Leu Val Asp Ser Glu Gln Arg Val Leu Gly Thr Val
            180                 185                 190
Leu Thr Asn Ser Ile Asn Asp Gly Asn Ser Asn Gln Val Ser Ala Asn
        195                 200                 205
Val Thr Glu Asn Ser Val Ile Gln Leu Pro Ala Pro Pro Gln His Cys
    210                 215                 220
Phe Glu Lys Ser Gly Gln Thr Val Lys Ser Ser Ser Leu Glu Glu Ser
225                 230                 235                 240
Thr Leu Lys Gln Val Ser Thr Asn Leu Ser Asn Ser Lys Ser Glu Asn
                245                 250                 255
Ile Cys Gln Thr Ser Ser Gln Asp Val Gln Asn Glu Leu Val Glu Thr
            260                 265                 270
Ser Asp Ala Val Thr Gly Ser Val Val Ala Asp Lys Thr Gln Ser Ile
        275                 280                 285
Pro Ala Gln Val Asn Thr Ser Ser Val Asn Glu Leu Leu Asp Ile Pro
    290                 295                 300
Ser Arg Asp Val Ala Glu Glu Val Gly Ser Asp Ser Ser Glu Arg Lys
305                 310                 315                 320
Ser Lys Ser Thr Thr Pro Ser Gln Leu Arg His Ile Gly Lys Ser Asn
                325                 330                 335
Ser Lys Leu Ser Lys Lys Lys Tyr Ile Leu Arg Ser Leu Gly Ser Ser
            340                 345                 350
Asp Arg Ala Leu Arg Ser Arg Thr Lys Glu Lys Pro Lys Ala Pro Glu
        355                 360                 365
Pro Ser Ser Asn Ser Val Asp Val Asn Asn Asp Gly Val Lys Lys Glu
    370                 375                 380
Lys Arg Arg Lys Lys Lys Lys Thr Gly Gly Glu Glu Lys Lys Asp Gln
385                 390                 395                 400
Phe Tyr Lys Ile Lys Ala His Leu Arg Tyr Leu Leu Asn Arg Val Ser
                405                 410                 415
Tyr Glu Gln Asn Leu Ile Asp Ala Tyr Ser Gly Asp Gly Trp Lys Gly
            420                 425                 430
Tyr Ser Met Asp Lys Leu Lys Pro Asp Lys Glu Ile Gln Arg Ala Lys
        435                 440                 445
Ser Glu Ile Leu Arg Arg Lys Leu Lys Ile Arg Asp Leu Phe Gln Asn
    450                 455                 460
Leu Asp Ser Leu Cys Ala Glu Gly Lys Phe Pro Glu Ser Leu Phe Asp
465                 470                 475                 480
Ser Glu Gly Glu Ile Asp Ser Glu Asp Ile Phe Cys Ser Ile Cys Gln
                485                 490                 495
Thr Lys Glu Leu Ser Thr Asp Asn Asp Ile Ile Leu Cys Asp Gly Ala
            500                 505                 510
Cys Asp Arg Gly Phe His Gln His Cys Leu Asp Pro Pro Leu Leu Thr
        515                 520                 525
Glu Asp Ile Pro Pro Gly Asp Glu Gly Trp Leu Cys Pro Gly Cys Asp
    530                 535                 540
Cys Lys Asp Asp Cys Val Asp Leu Leu Asn Asp Ser Leu Gly Thr Arg
545                 550                 555                 560
Leu Ser Leu Ser Asp Thr Trp Glu Lys Val Phe Pro Glu Ala Ala Ala
                565                 570                 575
```

Val Thr Gly Asn Asn Met Asp Gln Gly Leu Pro Ser Asp Asp Ser Asp
580 585 590
Asp Asp Asp Tyr Asn Pro Asn Gly Lys Glu Asp Val Glu Val Glu Gly
595 600 605
Gly Glu Ser Ser Ser Asp Glu Ser Glu Tyr Ala Ser Ala Ser Glu Lys
610 615 620
Leu Glu Asp Ser His His Glu Asp Pro Tyr Leu Gly Leu Pro Ser Glu
625 630 635 640
Asp Ser Glu Asp Asn Asp Tyr Asp Pro Ser Ala Pro Asp Leu Asp Asn
645 650 655
Lys Val Thr Glu Glu Ser Ser Ser Ser Asp Phe Thr Ser Asp Ser Glu
660 665 670
Asp Leu Ala Ala Ala Ile Glu Asp Asn Thr Ser Pro Gly His Val Lys
675 680 685
Gln Thr Lys Ser Arg Gln Lys Ser Lys Val Gly Lys Asn Pro Ser Met
690 695 700
Val Asp Glu Val Ser Ser Leu Arg Glu Pro Glu Leu Glu Glu Glu Gly
705 710 715 720
Phe Thr Pro Val Ser Gly Lys Arg Asn Val Glu Arg Leu Asp Tyr Lys
725 730 735
Lys Leu Tyr Asp Glu Thr Tyr Lys Ser Asp Thr Ser Glu Asp Glu Glu
740 745 750
Trp Thr Ala Ser Ala Thr Pro Ser Arg Lys Lys Lys Leu Cys Gly Lys
755 760 765
Met Thr Pro Val Ser Ser Asp Gly Lys Ala Ser Asn Asn Ser Arg His
770 775 780
Thr Pro Glu Arg Asn Thr Gln Gln Asp Lys Val Glu Asn Thr Asn Asn
785 790 795 800
Ser Pro Thr Lys Ser Leu Glu Gly Cys Leu Glu Ser Gly Ser Arg Asp
805 810 815
Lys Lys Pro Arg Ser Ser Thr Arg Gln Arg Leu Gly Asp Val Val Val
820 825 830
Gln Arg Leu His Lys Ser Phe Lys Asp Asn Gln Tyr Pro Asp Arg Ala
835 840 845
Thr Lys Glu Ser Leu Ala Glu Glu Leu Gly Leu Thr Phe Phe Gln Val
850 855 860
Asp Lys Trp Phe Gly Asn Ala Arg Trp Gly Phe Arg Arg Ser Ser Arg
865 870 875 880
Met Gly Ala Ser Pro Gly Glu Tyr Ala Ser Pro Gln Ala Thr Gly Ser
885 890 895
Gly Pro Glu Asn Thr Gly Glu Arg Glu Arg Glu Leu Ala Ser Gln Glu
900 905 910
Val Gly Glu Glu Lys Leu Lys Thr Pro Ser Pro Arg Lys Arg Lys His
915 920 925
Leu Ser Glu Pro Gln Ala Ser Glu Ala Pro Gln Ile Ile Val Leu Gly
930 935 940
Leu Ala Ala Ser Pro Gly Ser Pro Arg Ala His Ala Gly Asn Lys Lys
945 950 955 960
Lys Thr Val Lys Arg Lys
965

<210> 201
<211> 2379
<212> DNA
<213> Oryza sativa

<400> 201

atggataaga caactacttc tgatcttgtt ttggacaacg acaacattgg gagtaatgct 60
ggttctgcac aggagcctct tactaccaat ggtaagacta gtggggtcag aaatagatac 120
aaacaaactg ttaaaagggg aaggaaaggc tcccaaatat caccaagtaa aacatatccc 180
ctgagatctt cacatagtaa tgtcagggtg cttcgctctg cctcaaaaaa gaagaatgag 240

```
acacctattg tgcccacaaa tgataatact gctgttcaac gagttgcgaa gaaaaggaaa     300
agaagcaaac ccttgagacc tgcacctagc agggtgcttc gctctacctc ggaaaagaag     360
aataaggcac ataatgagct tctaaatgat ggtgctggtg ttcaaccagc cgaaaagaaa     420
agaaaggtag gcagaccccc aaaaggagga actcccaagg acgattacct catgatccga     480
aagcgagtta gatatgtttt gaatcgaatg aactatgaac aaagtttaat tcaagcctat     540
gccagtgaag gttggaaagg tcaaagcttg gaaaaaataa gacctgagaa ggagcttgaa     600
cgagccaagg tggaaattct gcgatgcaag tcaagaatac gggaagcttt tcggaatttg     660
gattccctcc tatctgaggg aaagctggat gaatctatgt ttgattctgc tggagaaata     720
tctagtgaag atattttctg tgctgcatgt ggttcaaagg atgttacatt aaaaaatgac     780
ataattcttt gtgatggaat ctgcgacaga gggttccacc agtattgttt aaaccctcct     840
ttgcttgctg aagatattcc acagggggat gaaggatggc tttgccctgc atgtgattgc     900
aaaattgact gcatagatgt attaaatgaa ctccaagggg tcaaactctc tatccatgac     960
tcttgggaga aagtttttcc tgaggcagca tcctttttga atggttctaa gcaaattgat    1020
gcgtccgatc ttccatcaga tgattcagca gacaatgatt atgaccctac tttggctcaa    1080
gggcacaagg tggatgaaga aaaatcttct ggagaagatg gtggtgaagg attagattct    1140
gatgactcat cttctgagga ttctgaatct tctgagaagg aaaagtctaa aacttcacag    1200
aatggaagga cagttgatga tcttggtttg ccttctgagg attcagagga tggtgacttt    1260
gatccagcag gtccagattc cgacaaagaa caaaatgatg aatcaaactc agatcagtca    1320
gatgaatcag attttacatc tgactctgat gatttttgtg ctgagattgc taaatcctgt    1380
ggtcaggatg aaatctcagg cccttcatca tcacaaatca gaacagtcga tcgcaccgat    1440
ggaagtggtt ttgatggtga gcctaatgca gaaaattcga atcttgcatt tatggagaca    1500
gagctagagc aagacatggt gctaccaatt tctagtaaac gacaagttga acgtttggat    1560
tacaaaaaac tgtataatga ggcttatggt aaagcatcat ctgattcaag tgacgatgaa    1620
gagtggtatg gaaatagtac acccgaaaaa ggtaatttag aagacagtga aacagattcg    1680
cttgctgaat cacctcaggg tggaaaagga ttctctagaa gagcaccagt taggtaccat    1740
aataatgaac atactccaca gaatgtaagg cccggtggtt cagtaagcga ccagcaaact    1800
gaagtacttt gctccaatag caatggtagc acagccaaaa acagacattt tggtcctgca    1860
attaatcaga aactgaaggc acatttcaag gaagatccat accctagtcg tgcaacaaaa    1920
gaaaatctgg cacaagagtt aggcctcaca tttaatcagg tcactaaatg gttttccagt    1980
actcgtcatt acgcaagagt ggcagccaca aaaaaggaga acaacattga aaatcatacg    2040
gctgagaata ataacaatac caacactgtt gatagcatac aactgagagg atccaatgat    2100
attgttagtg tagatagaaa tgatatggtc tccgaggaaa ggacaggaca aagtaatctc    2160
aatgaaggta ctccattaag atcagatacc agctgtgggc agagtgtggc tgtgactcct    2220
atggtacacc cagaaaacca gggcaatgat tcctcaagta atgttagaac accaaatgct    2280
aagtctgcgg agaaattgat ccctggactg gaaaattcag atgaggctag gcgaaaggcg    2340
gtacaacggg aactgagaaa gatgaagact ggcaggtga                           2379
```

<210> 202
<211> 792
<212> PRT
<213> Oryza sativa

<400> 202

```
Met Asp Lys Thr Thr Thr Ser Asp Leu Val Leu Asp Asn Asp Asn Ile
1               5                   10                  15
Gly Ser Asn Ala Gly Ser Ala Gln Glu Pro Leu Thr Thr Asn Gly Lys
            20                  25                  30
Thr Ser Gly Val Arg Asn Arg Tyr Lys Gln Thr Val Lys Arg Gly Arg
        35                  40                  45
Lys Gly Ser Gln Ile Ser Pro Ser Lys Thr Tyr Pro Leu Arg Ser Ser
    50                  55                  60
His Ser Asn Val Arg Val Leu Arg Ser Ala Ser Lys Lys Lys Asn Glu
65                  70                  75                  80
Thr Pro Ile Val Pro Thr Asn Asp Asn Thr Ala Val Gln Arg Val Ala
                85                  90                  95
Lys Lys Arg Lys Arg Ser Lys Pro Leu Arg Pro Ala Pro Ser Arg Val
            100                 105                 110
Leu Arg Ser Thr Ser Glu Lys Lys Asn Lys Ala His Asn Glu Leu Leu
        115                 120                 125
Asn Asp Gly Ala Gly Val Gln Pro Ala Glu Lys Lys Arg Lys Val Gly
    130                 135                 140
```

Arg Pro Pro Lys Gly Gly Thr Pro Lys Asp Asp Tyr Leu Met Ile Arg
145 150 155 160
Lys Arg Val Arg Tyr Val Leu Asn Arg Met Asn Tyr Glu Gln Ser Leu
165 170 175
Ile Gln Ala Tyr Ala Ser Glu Gly Trp Lys Gly Gln Ser Leu Glu Lys
180 185 190
Ile Arg Pro Glu Lys Glu Leu Glu Arg Ala Lys Val Glu Ile Leu Arg
195 200 205
Cys Lys Ser Arg Ile Arg Glu Ala Phe Arg Asn Leu Asp Ser Leu Leu
210 215 220
Ser Glu Gly Lys Leu Asp Glu Ser Met Phe Asp Ser Ala Gly Glu Ile
225 230 235 240
Ser Ser Glu Asp Ile Phe Cys Ala Ala Cys Gly Ser Lys Asp Val Thr
245 250 255
Leu Lys Asn Asp Ile Ile Leu Cys Asp Gly Ile Cys Asp Arg Gly Phe
260 265 270
His Gln Tyr Cys Leu Asn Pro Pro Leu Leu Ala Glu Asp Ile Pro Gln
275 280 285
Gly Asp Glu Gly Trp Leu Cys Pro Ala Cys Asp Cys Lys Ile Asp Cys
290 295 300
Ile Asp Val Leu Asn Glu Leu Gln Gly Val Lys Leu Ser Ile His Asp
305 310 315 320
Ser Trp Glu Lys Val Phe Pro Glu Ala Ala Ser Phe Leu Asn Gly Ser
325 330 335
Lys Gln Ile Asp Ala Ser Asp Leu Pro Ser Asp Asp Ser Ala Asp Asn
340 345 350
Asp Tyr Asp Pro Thr Leu Ala Gln Gly His Lys Val Asp Glu Glu Lys
355 360 365
Ser Ser Gly Glu Asp Gly Gly Glu Gly Leu Asp Ser Asp Asp Ser Ser
370 375 380
Ser Glu Asp Ser Glu Ser Ser Glu Lys Glu Lys Ser Lys Thr Ser Gln
385 390 395 400
Asn Gly Arg Thr Val Asp Asp Leu Gly Leu Pro Ser Glu Asp Ser Glu
405 410 415
Asp Gly Asp Phe Asp Pro Ala Gly Pro Asp Ser Asp Lys Glu Gln Asn
420 425 430
Asp Glu Ser Asn Ser Asp Gln Ser Asp Glu Ser Asp Phe Thr Ser Asp
435 440 445
Ser Asp Asp Phe Cys Ala Glu Ile Ala Lys Ser Cys Gly Gln Asp Glu
450 455 460
Ile Ser Gly Pro Ser Ser Ser Gln Ile Arg Thr Val Asp Arg Thr Asp
465 470 475 480
Gly Ser Gly Phe Asp Gly Glu Pro Asn Ala Glu Asn Ser Asn Leu Ala
485 490 495
Phe Met Glu Thr Glu Leu Glu Gln Asp Met Val Leu Pro Ile Ser Ser
500 505 510
Lys Arg Gln Val Glu Arg Leu Asp Tyr Lys Lys Leu Tyr Asn Glu Ala
515 520 525
Tyr Gly Lys Ala Ser Ser Asp Ser Ser Asp Asp Glu Glu Trp Tyr Gly
530 535 540
Asn Ser Thr Pro Glu Lys Gly Asn Leu Glu Asp Ser Glu Thr Asp Ser
545 550 555 560
Leu Ala Glu Ser Pro Gln Gly Gly Lys Gly Phe Ser Arg Arg Ala Pro
565 570 575
Val Arg Tyr His Asn Asn Glu His Thr Pro Gln Asn Val Arg Pro Gly
580 585 590
Gly Ser Val Ser Asp Gln Gln Thr Glu Val Leu Cys Ser Asn Ser Asn
595 600 605
Gly Ser Thr Ala Lys Asn Arg His Phe Gly Pro Ala Ile Asn Gln Lys
610 615 620
Leu Lys Ala His Phe Lys Glu Asp Pro Tyr Pro Ser Arg Ala Thr Lys

```
625                 630                 635                 640
Glu Asn Leu Ala Gln Glu Leu Gly Leu Thr Phe Asn Gln Val Thr Lys
                645                 650                 655
Trp Phe Ser Ser Thr Arg His Tyr Ala Arg Val Ala Ala Thr Lys Lys
            660                 665                 670
Glu Asn Asn Ile Glu Asn His Thr Ala Glu Asn Asn Asn Asn Thr Asn

        675                 680                 685
Thr Val Asp Ser Ile Gln Leu Arg Gly Ser Asn Asp Ile Val Ser Val
    690                 695                 700
Asp Arg Asn Asp Met Val Ser Glu Glu Arg Thr Gly Gln Ser Asn Leu
705                 710                 715                 720
Asn Glu Gly Thr Pro Leu Arg Ser Asp Thr Ser Cys Gly Gln Ser Val
                725                 730                 735
Ala Val Thr Pro Met Val His Pro Glu Asn Gln Gly Asn Asp Ser Ser
            740                 745                 750
Ser Asn Val Arg Thr Pro Asn Ala Lys Ser Ala Glu Lys Leu Ile Pro
        755                 760                 765
Gly Leu Glu Asn Ser Asp Glu Ala Arg Arg Lys Ala Val Gln Arg Glu
    770                 775                 780
Leu Arg Lys Met Lys Thr Gly Arg
785                 790
```

<210> 203
<211> 3267
<212> DNA
<213> Petroselinum crispum

<400> 203

atggaagaga tctcagaccc taaaccaaat gcattagagc aagtacttcc tactgtccca 60
aatggtaaat gtactgcacc ggtgcaaatg gaaagtttgg ctgttgatgt tcaaaaagta 120
tcaggtgaag caaaggtgag gatatgttcc tgttggtgcg agatagtacg ttctccagaa 180
gatttaacta aacttgtgcc ttgtaatgat tttgcagaag atataaaatt gtttgattct 240
gatcccatgc aacaagaagc tgaaagctcc attggaatac cattgatccc taaacaagtt 300
acaatgtccc ataaccatga ccatgaatct ggttctgaaa tggtcagtaa cgaagtaatg 360
caagaaaacc atgtaattgc cacagaaaat acttaccaga aatctgattt tgaccgtata 420
aatatgggac aaaaagaaac catgccagaa gaagtgatcc ataagtcttt cttggaatcc 480
tcaacttcct cgatagatat actattaaat aatcataaca gttatcaatc aggactgcca 540
ccagagaatg cagtaacaga ttgcaagcaa gtccaattgg gacaccgaag tgatgatgct 600
ataaagaatt ctggtcttgt ggaattggta ataggtcaga aaaatgttgc caagagtcct 660
agccagttgg tagaaacggg gaaaagaggt cgtggtcggc ccaggaaagt acaaactggc 720
cttgagcaat tggtaatagg tcagaaaact gctgccaaga gctctagcca gttaggtgac 780
acagggaaaa gatctcgtgg tcggcccaga aaagtacaaa attctccaac ttctttcttg 840
gagaatataa atatggaaca aaaagaaaca ataccagaac aagtgaccca aaattctatc 900
ttggagtcct tgactatccc gacagacaat cagtcaagaa cttataacag tgatcaatca 960
gaactgccac cagagaatgc agcaaaaaat tgcaatcacg cccaatttgg acatcaaagt 1020
gatgatacta caaagatttc tggattcaag gaattggtaa taggtcagga aacagttgca 1080
aagagtccta gccagttggt agacgcagga aaaagaggtc gtggtcggcc cagaaaagta 1140
caaactggtc ttgagcaatt ggtaccagtt caggaaactg cggccaagag ctctagccag 1200
ttgggagaca cagggaaaag atctcgcggc cggcccagga aagtacaaga ttctccaact 1260
tctttagggg gtaatgttaa agtggtacca gagaaaggaa aagattctca ggaattgtcg 1320
gtaaacagca gcagatcatt gcgctcaagg tcacaagaga aatctatcga acccgatgtc 1380
aataatattg tggcagatga aggtgctgac agagaaaaac caaggaagaa gaggaaaaag 1440
cgaatggagg aaaatagggt tgatgaattc tgtagaataa ggacacacct aaggtactta 1500
ctacacagaa taaaatacga gaagaatttc cttgatgctt actctgggga gggatggaag 1560
ggacaaagcc tagacaaaat aaaacctgag aaggagctta aacgagcaaa agctgaaatt 1620
tttggacgca aattgaaaat tagagatcta tttcagcgct tagatttagc acgttccgag 1680
ggaaggcttc cagaaattct atttgattct cgaggagaaa ttgacagcga ggatatattc 1740
tgcgcgaaat gtggatccaa ggatgtgaca ctcagtaatg atatcatcct ttgcgatggt 1800
gcatgtgatc gtggatttca ccaattttgt ttggacccac cattgctgaa agaatatatt 1860
cctcctgacg acgagggttg gctttgcccg ggatgcgagt gcaaaattga ctgcatcaag 1920

ttacttaatg attcccaaga gacaaatatt ttactcggtg acagctggga gaaagttttt 1980
gctgaggagg cagcagcagc agcttccgga aaaaatctag atgataattc tggactgcct 2040
tcagatgatt ccgaggatga tgattatgat cctgggggcc ctgatttgga tgagaaggtg 2100
cagggagatg attcgagtac ggatgaatct gattatcagt cggaatcaga tgatatgcaa 2160
gttatacgtc aaaaaaattc gcgcgggctt ccttcagatg attccgaaga tgatgaatac 2220
gatcctagtg gcttagttac tgatcagatg tataaggaca gctcatgttc tgattttaca 2280
tctgattctg aggattttac aggtgtgttt gacgattata aggatactgg taaagctcaa 2340
gggccactgg catcgacccc agatcacgtc aggaacaatg aggaagggtg tgggcatcct 2400
gagcaaggtg atactgcccc cctgtatccg agaagacaag tagagagttt ggactacaaa 2460
aagctaaatg atattgagtt ctctaaaatg tgtgacatcc ttgatattct ttcaagtcaa 2520
cttgatgtaa ttatttgtac tggtaaccag gaagagtatg ggaacacatc ttctgattca 2580
agtgatgaag attatatggt tacttcttca ccagataaaa ataattctga taaagaagct 2640
acagcaatgg agcgtggaag agagagcggt gatttggagc tagaccaaaa agcgagagaa 2700
tccactcata ataggagata tataaagaag tttgctgttg aaggcacaga ttcttttctt 2760
tctagatcgt gtgaagactc tgcagcacct gttgctggta gtaaaagtac ttcaaaaaca 2820
ttgcacgggg aacatgcaac tcagagactc ctccaatcct tcaaggaaaa tcaataccct 2880
caacgagctg tgaaagagag tttggctgca gaattagcac tttcagtccg acaggttagc 2940
aactggttta ataatagacg ttggagcttt cgccactcat cacgtattgg atcggatgta 3000
gctaaatttg attcaaacga tactccccgt cagaaaagca ttgacatgtc tggacccagc 3060
ttgaaatcgg tcttggacag tgctacttac agtgaaattg aaaagaagga acaagatacg 3120
gcaagccttg gtttgacaga aggttgtgac agatacatga cgctgaatat ggttgcagat 3180
gaaggcaatg tgcacactcc ttgtattgca gaaactaggg aagaaaaaac cgaagtcggt 3240
ataaagccac aacagaaccc actataa 3267

<210> 204
<211> 1088
<212> PRT
<213> Petroselinum crispum

<400> 204

```
Met Glu Glu Ile Ser Asp Pro Lys Pro Asn Ala Leu Glu Gln Val Leu
1               5                   10                  15
Pro Thr Val Pro Asn Gly Lys Cys Thr Ala Pro Val Gln Met Glu Ser
            20                  25                  30
Leu Ala Val Asp Val Gln Lys Val Ser Gly Glu Ala Lys Val Arg Ile
        35                  40                  45
Cys Ser Cys Trp Cys Glu Ile Val Arg Ser Pro Glu Asp Leu Thr Lys
    50                  55                  60
Leu Val Pro Cys Asn Asp Phe Ala Glu Asp Ile Lys Leu Phe Asp Ser
65                  70                  75                  80
Asp Pro Met Gln Gln Glu Ala Glu Ser Ser Ile Gly Ile Pro Leu Ile
                85                  90                  95
Pro Lys Gln Val Thr Met Ser His Asn His Asp His Glu Ser Gly Ser
            100                 105                 110
Glu Met Val Ser Asn Glu Val Met Gln Glu Asn His Val Ile Ala Thr
        115                 120                 125
Glu Asn Thr Tyr Gln Lys Ser Asp Phe Asp Arg Ile Asn Met Gly Gln
    130                 135                 140
Lys Glu Thr Met Pro Glu Glu Val Ile His Lys Ser Phe Leu Glu Ser
145                 150                 155                 160
Ser Thr Ser Ser Ile Asp Ile Leu Leu Asn Asn His Asn Ser Tyr Gln
                165                 170                 175
Ser Gly Leu Pro Pro Glu Asn Ala Val Thr Asp Cys Lys Gln Val Gln
            180                 185                 190
Leu Gly His Arg Ser Asp Asp Ala Ile Lys Asn Ser Gly Leu Val Glu
        195                 200                 205
Leu Val Ile Gly Gln Lys Asn Val Ala Lys Ser Pro Ser Gln Leu Val
    210                 215                 220
Glu Thr Gly Lys Arg Gly Arg Gly Arg Pro Arg Lys Val Gln Thr Gly
225                 230                 235                 240
Leu Glu Gln Leu Val Ile Gly Gln Lys Thr Ala Ala Lys Ser Ser Ser
```

```
                        245                 250                 255
Gln Leu Gly Asp Thr Gly Lys Arg Ser Arg Gly Arg Pro Arg Lys Val
                260                 265                 270
Gln Asn Ser Pro Thr Ser Phe Leu Glu Asn Ile Asn Met Glu Gln Lys
            275                 280                 285
Glu Thr Ile Pro Glu Gln Val Thr Gln Asn Ser Ile Leu Glu Ser Leu
    290                 295                 300
Thr Ile Pro Thr Asp Asn Gln Ser Arg Thr Tyr Asn Ser Asp Gln Ser
305                 310                 315                 320
Glu Leu Pro Pro Glu Asn Ala Ala Lys Asn Cys Asn His Ala Gln Phe
                325                 330                 335
Gly His Gln Ser Asp Asp Thr Thr Lys Ile Ser Gly Phe Lys Glu Leu
            340                 345                 350
Val Ile Gly Gln Glu Thr Val Ala Lys Ser Pro Ser Gln Leu Val Asp
        355                 360                 365
Ala Gly Lys Arg Gly Arg Gly Arg Pro Arg Lys Val Gln Thr Gly Leu
    370                 375                 380
Glu Gln Leu Val Pro Val Gln Glu Thr Ala Ala Lys Ser Ser Ser Gln
385                 390                 395                 400
Leu Gly Asp Thr Gly Lys Arg Ser Arg Gly Arg Pro Arg Lys Val Gln
                405                 410                 415
Asp Ser Pro Thr Ser Leu Gly Gly Asn Val Lys Val Val Pro Glu Lys
            420                 425                 430
Gly Lys Asp Ser Gln Glu Leu Ser Val Asn Ser Ser Arg Ser Leu Arg
        435                 440                 445
Ser Arg Ser Gln Glu Lys Ser Ile Glu Pro Asp Val Asn Asn Ile Val
    450                 455                 460
Ala Asp Glu Gly Ala Asp Arg Glu Lys Pro Arg Lys Lys Arg Lys Lys
465                 470                 475                 480
Arg Met Glu Glu Asn Arg Val Asp Glu Phe Cys Arg Ile Arg Thr His
                485                 490                 495
Leu Arg Tyr Leu Leu His Arg Ile Lys Tyr Glu Lys Asn Phe Leu Asp
            500                 505                 510
Ala Tyr Ser Gly Glu Gly Trp Lys Gly Gln Ser Leu Asp Lys Ile Lys
        515                 520                 525
Pro Glu Lys Glu Leu Lys Arg Ala Lys Ala Glu Ile Phe Gly Arg Lys
    530                 535                 540
Leu Lys Ile Arg Asp Leu Phe Gln Arg Leu Asp Leu Ala Arg Ser Glu
545                 550                 555                 560
Gly Arg Leu Pro Glu Ile Leu Phe Asp Ser Arg Gly Glu Ile Asp Ser
                565                 570                 575
Glu Asp Ile Phe Cys Ala Lys Cys Gly Ser Lys Asp Val Thr Leu Ser
            580                 585                 590
Asn Asp Ile Ile Leu Cys Asp Gly Ala Cys Asp Arg Gly Phe His Gln
        595                 600                 605
Phe Cys Leu Asp Pro Pro Leu Leu Lys Glu Tyr Ile Pro Pro Asp Asp
    610                 615                 620
Glu Gly Trp Leu Cys Pro Gly Cys Glu Cys Lys Ile Asp Cys Ile Lys
625                 630                 635                 640
Leu Leu Asn Asp Ser Gln Glu Thr Asn Ile Leu Leu Gly Asp Ser Trp
                645                 650                 655
Glu Lys Val Phe Ala Glu Glu Ala Ala Ala Ala Ser Gly Lys Asn
            660                 665                 670
Leu Asp Asp Asn Ser Gly Leu Pro Ser Asp Asp Ser Glu Asp Asp Asp
        675                 680                 685
Tyr Asp Pro Gly Gly Pro Asp Leu Asp Glu Lys Val Gln Gly Asp Asp
    690                 695                 700
Ser Ser Thr Asp Glu Ser Asp Tyr Gln Ser Glu Ser Asp Asp Met Gln
705                 710                 715                 720
Val Ile Arg Gln Lys Asn Ser Arg Gly Leu Pro Ser Asp Asp Ser Glu
                725                 730                 735
```

```
Asp Asp Glu Tyr Asp Pro Ser Gly Leu Val Thr Asp Gln Met Tyr Lys
            740                 745                 750
Asp Ser Ser Cys Ser Asp Phe Thr Ser Asp Ser Glu Asp Phe Thr Gly
        755                 760                 765
Val Phe Asp Asp Tyr Lys Asp Thr Gly Lys Ala Gln Gly Pro Leu Ala
    770                 775                 780
Ser Thr Pro Asp His Val Arg Asn Asn Glu Glu Gly Cys Gly His Pro
785                 790                 795                 800
Glu Gln Gly Asp Thr Ala Pro Leu Tyr Pro Arg Arg Gln Val Glu Ser
                805                 810                 815
Leu Asp Tyr Lys Lys Leu Asn Asp Ile Glu Phe Ser Lys Met Cys Asp
            820                 825                 830
Ile Leu Asp Ile Leu Ser Ser Gln Leu Asp Val Ile Ile Cys Thr Gly
        835                 840                 845
Asn Gln Glu Glu Tyr Gly Asn Thr Ser Ser Asp Ser Ser Asp Glu Asp
    850                 855                 860

Tyr Met Val Thr Ser Ser Pro Asp Lys Asn Asn Ser Asp Lys Glu Ala
865                 870                 875                 880
Thr Ala Met Glu Arg Gly Arg Glu Ser Gly Asp Leu Glu Leu Asp Gln
                885                 890                 895
Lys Ala Arg Glu Ser Thr His Asn Arg Arg Tyr Ile Lys Lys Phe Ala
            900                 905                 910
Val Glu Gly Thr Asp Ser Phe Leu Ser Arg Ser Cys Glu Asp Ser Ala
        915                 920                 925
Ala Pro Val Ala Gly Ser Lys Ser Thr Ser Lys Thr Leu His Gly Glu
    930                 935                 940
His Ala Thr Gln Arg Leu Leu Gln Ser Phe Lys Glu Asn Gln Tyr Pro
945                 950                 955                 960
Gln Arg Ala Val Lys Glu Ser Leu Ala Ala Glu Leu Ala Leu Ser Val
                965                 970                 975
Arg Gln Val Ser Asn Trp Phe Asn Asn Arg Arg Trp Ser Phe Arg His
            980                 985                 990
Ser Ser Arg Ile Gly Ser Asp Val  Ala Lys Phe Asp Ser  Asn Asp Thr
        995                 1000                1005
Pro Arg  Gln Lys Ser Ile Asp  Met Ser Gly Pro Ser  Leu Lys Ser
    1010                1015                1020
Val Leu  Asp Ser Ala Thr Tyr  Ser Glu Ile Glu Lys  Lys Glu Gln
    1025                1030                1035
Asp Thr  Ala Ser Leu Gly Leu  Thr Glu Gly Cys Asp  Arg Tyr Met
    1040                1045                1050
Thr Leu  Asn Met Val Ala Asp  Glu Gly Asn Val His  Thr Pro Cys
    1055                1060                1065
Ile Ala  Glu Thr Arg Glu Glu  Lys Thr Glu Val Gly  Ile Lys Pro
    1070                1075                1080
Gln Gln  Asn Pro Leu
    1085
```

<210> 205
<211> 2160
<212> DNA
<213> Zea mays

<400> 205

```
atggaaaaaa atatagctca ctgtcctgtt gagggcaatg gtgagattga aaatggtgcg     60
agctctagtc agaatcctga gtctcttgag cattcagttt tgctgtctac atctcaaacg    120
atgccaaata acttggggat aaggaaaaac tataaaaggg cagcaaacag aggtaaaaaa    180
ggttcccaag gactaacagg ccaagcatat actttgatgt catctaatag tgatgtcagg    240
gtgcttcgct ccacatcaag ttcaaagaca acatctactg agcatgttca ggctccagta    300
caaccggctg ccaagagaag aaaaatgagc agagcctcaa acaaaagttc gactgatgag    360
ttttcacaaa ttcgtaaacg agttagatat attttgaacc gaatgaatta tgagcaaagc    420
```

```
ctaattgaag cttatgctag cgaaggctgg aaaaatcaaa gtttggataa gataagacct    480
gagaaggagc ttgaacgagc caaatcagaa atcttacgat gcaaattgag aatacgagaa    540
gtcttccgga atattgattc tcttctctcc aaggggaaaa ttgacgaaac tttatttgat    600
tctgaaggag aaatatcttg cgaagatatc ttttgttcca cttgtggttc gaatgacgct    660
acattgggta atgatatcat tctctgcgat ggagcttgtg acagagggtt ccaccagaac    720
tgtttaaatc ctcccctgcg tactgaagat atccccatgg gagatgaagg atggctctgc    780
ccagcatgtg attgcaagat agattgtata gatctaataa atgaactcca tgggagtaac    840
atctccattg aggactcttg ggagaaagtt tttccagatg cagctgctat ggcaaatgac    900
tctaaacaag atgacgcatt tgatcttcca tcagatgact cagacgacaa tgactttgac    960
cccaatatgc ctgaagagca tgtggttggt aaggatgaag aatcgtctga agaggatgag   1020
gatggaggat cagactctga tgactcagac tttttgacat gttctgatga ttcggaacct   1080
ttgatagaca agaaggttga tgacctcaga ttaccttctg aagattcaga ggatgatgac   1140
tatgatccag caggtcctga ttcagataaa gatgtcgaaa agaagtcaag ttctgatgaa   1200
tctgacttca catctgactc agatgatttt tgtaaggaga tttcaaaatc tggacatgat   1260
gaagtttcat cacctctatt acccgatgcc aaggtgggtg atatggaaaa gattactgct   1320
caagctaaaa caacaagttc tgctgacgac cctatggaga ctgaaataga ccagggtgtg   1380
gttttaccag attcaaggag acggcaagct gaacggttgg actacaaaaa gttgtatgat   1440
gaggcatatg gtgaagcatc atctgattct agtgatgatg aagaatggtc tgggaagaac   1500
acgccaataa taaaaagcaa tgaagagggt gaagccaatt ctcctgcggg aaaaggctcc   1560
agagttgtgc accataatga tgaactgact acacaaagca ctaaaaaaag cttgcattct   1620
attcatggtt cagtagatga gaaacctgga gaccttactt ctaatggcag caacagcaca   1680
gccaggaaag gacattttgg cccagtaatt aatcagaagc tacatgaaca ttttaagaca   1740
caaccatacc ctagtcgttc tgttaaagaa agtctggcag aagaactagg gttaacattt   1800
cgtcaggtta acaaatggtt tgaaactagg cgtcattccg caagggtagc ttcttccagg   1860
aaaggcatca gtctagataa gcatagcccc caaaatacta atagtcaagt gacagctagt   1920
atggaaccta aagaacctga ggggactgtg gtggaagaat ctaatgtatg cctaaatggg   1980
ggtactacca tctctaaaga agcagtgtct tcaaaagttg gatcaagaac ccctggaagt   2040
gatgtgggag ggtcaaaagt tgattctgct gaggatcaga atccggggcc tgatcttgca   2100
gagaaggcaa ggcaaaaggc aatacagcag gagttgagga agaaaaaaat gggacgatga   2160
```

<210> 206
<211> 719
<212> PRT
<213> Zea mays

<400> 206

```
Met Glu Lys Asn Ile Ala His Cys Pro Val Glu Gly Asn Gly Glu Ile
1               5                   10                  15
Glu Asn Gly Ala Ser Ser Ser Gln Asn Pro Glu Ser Leu Glu His Ser
            20                  25                  30
Val Leu Leu Ser Thr Ser Gln Thr Met Pro Asn Asn Leu Gly Ile Arg
        35                  40                  45
Lys Asn Tyr Lys Arg Ala Ala Asn Arg Gly Lys Lys Gly Ser Gln Gly
    50                  55                  60
Leu Thr Gly Gln Ala Tyr Thr Leu Met Ser Ser Asn Ser Asp Val Arg
65                  70                  75                  80
Val Leu Arg Ser Thr Ser Ser Ser Lys Thr Thr Ser Thr Glu His Val
                85                  90                  95
Gln Ala Pro Val Gln Pro Ala Ala Lys Arg Arg Lys Met Ser Arg Ala
            100                 105                 110
Ser Asn Lys Ser Ser Thr Asp Glu Phe Ser Gln Ile Arg Lys Arg Val
        115                 120                 125
Arg Tyr Ile Leu Asn Arg Met Asn Tyr Glu Gln Ser Leu Ile Glu Ala
    130                 135                 140
Tyr Ala Ser Glu Gly Trp Lys Asn Gln Ser Leu Asp Lys Ile Arg Pro
145                 150                 155                 160
Glu Lys Glu Leu Glu Arg Ala Lys Ser Glu Ile Leu Arg Cys Lys Leu
                165                 170                 175
Arg Ile Arg Glu Val Phe Arg Asn Ile Asp Ser Leu Leu Ser Lys Gly
            180                 185                 190
Lys Ile Asp Glu Thr Leu Phe Asp Ser Glu Gly Glu Ile Ser Cys Glu
```

```
        195                 200                 205
Asp Ile Phe Cys Ser Thr Cys Gly Ser Asn Asp Ala Thr Leu Gly Asn
    210                 215                 220
Asp Ile Ile Leu Cys Asp Gly Ala Cys Asp Arg Gly Phe His Gln Asn
225                 230                 235                 240
Cys Leu Asn Pro Pro Leu Arg Thr Glu Asp Ile Pro Met Gly Asp Glu
                245                 250                 255
Gly Trp Leu Cys Pro Ala Cys Asp Cys Lys Ile Asp Cys Ile Asp Leu
            260                 265                 270
Ile Asn Glu Leu His Gly Ser Asn Ile Ser Ile Glu Asp Ser Trp Glu
        275                 280                 285
Lys Val Phe Pro Asp Ala Ala Ala Met Ala Asn Asp Ser Lys Gln Asp
    290                 295                 300
Asp Ala Phe Asp Leu Pro Ser Asp Asp Ser Asp Asp Asn Asp Phe Asp
305                 310                 315                 320
Pro Asn Met Pro Glu Glu His Val Val Gly Lys Asp Glu Glu Ser Ser
                325                 330                 335
Glu Glu Asp Glu Asp Gly Gly Ser Asp Ser Asp Asp Ser Asp Phe Leu
            340                 345                 350
Thr Cys Ser Asp Asp Ser Glu Pro Leu Ile Asp Lys Lys Val Asp Asp
        355                 360                 365
Leu Arg Leu Pro Ser Glu Asp Ser Glu Asp Asp Asp Tyr Asp Pro Ala
    370                 375                 380
Gly Pro Asp Ser Asp Lys Asp Val Glu Lys Lys Ser Ser Ser Asp Glu
385                 390                 395                 400
Ser Asp Phe Thr Ser Asp Ser Asp Asp Phe Cys Lys Glu Ile Ser Lys
                405                 410                 415
Ser Gly His Asp Glu Val Ser Ser Pro Leu Leu Pro Asp Ala Lys Val
            420                 425                 430
Gly Asp Met Glu Lys Ile Thr Ala Gln Ala Lys Thr Thr Ser Ser Ala
        435                 440                 445
Asp Asp Pro Met Glu Thr Glu Ile Asp Gln Gly Val Val Leu Pro Asp
    450                 455                 460
Ser Arg Arg Arg Gln Ala Glu Arg Leu Asp Tyr Lys Lys Leu Tyr Asp
465                 470                 475                 480
Glu Ala Tyr Gly Glu Ala Ser Ser Asp Ser Ser Asp Asp Glu Glu Trp
                485                 490                 495
Ser Gly Lys Asn Thr Pro Ile Ile Lys Ser Asn Glu Glu Gly Glu Ala
            500                 505                 510
Asn Ser Pro Ala Gly Lys Gly Ser Arg Val Val His His Asn Asp Glu
        515                 520                 525
Leu Thr Thr Gln Ser Thr Lys Lys Ser Leu His Ser Ile His Gly Ser
    530                 535                 540
Val Asp Glu Lys Pro Gly Asp Leu Thr Ser Asn Gly Ser Asn Ser Thr
545                 550                 555                 560
Ala Arg Lys Gly His Phe Gly Pro Val Ile Asn Gln Lys Leu His Glu
                565                 570                 575
His Phe Lys Thr Gln Pro Tyr Pro Ser Arg Ser Val Lys Glu Ser Leu
            580                 585                 590
Ala Glu Glu Leu Gly Leu Thr Phe Arg Gln Val Asn Lys Trp Phe Glu
        595                 600                 605
Thr Arg Arg His Ser Ala Arg Val Ala Ser Ser Arg Lys Gly Ile Ser
    610                 615                 620
Leu Asp Lys His Ser Pro Gln Asn Thr Asn Ser Gln Val Thr Ala Ser
625                 630                 635                 640
Met Glu Pro Lys Glu Pro Glu Gly Thr Val Val Glu Glu Ser Asn Val
                645                 650                 655
Cys Leu Asn Gly Gly Thr Thr Ile Ser Lys Glu Ala Val Ser Ser Lys
            660                 665                 670
Val Gly Ser Arg Thr Pro Gly Ser Asp Val Gly Gly Ser Lys Val Asp
        675                 680                 685
```

```
Ser Ala Glu Asp Gln Asn Pro Gly Pro Asp Leu Ala Glu Lys Ala Arg
    690                 695                 700
Gln Lys Ala Ile Gln Gln Glu Leu Arg Lys Lys Lys Met Gly Arg
705                 710                 715
```

<210> 207
<211> 2079
<212> DNA
<213> Zea mays

<400> 207

```
atggaaaaaa agacagttca cgttctggct gagggtaatg gtgagattga aaatggtacg     60
agctctagtc agaatcctga gacccttgag catccagttt tgctgtctgc atctcaaact    120
gtgccaaata acttggggat aaggaaaaac tataaaaggg cagcaaacag aggtaaaaag    180
ggttcccaag gactaacagg tcaggcatat acattgaggt catctgatat tgatgtcagg    240
gtgcttcgct ctacatcagg ttcaaagaca acatctactg agcatgtgca gcctccagga    300
caaccagctg ccaagagaag aaaaaggagc agagcctcaa acaaaaattc gacagatgag    360
ttctcacaaa ttcgtaaacg ggttagatac attttgaacc gaatgaatta tgagcaaagc    420
ctaattgaag cttatgctag tgaaggctgg aaaaatcaaa gtttggataa gataagacct    480
gagaaggagc ttgaacgagc caaatcagaa atcttacgat gcaaattgag aatacgggaa    540
gtcttccaaa atattgattc tcttctgtcc aaagggaaaa ttgacgaatc tttatttgat    600
tctgaaggag aaatatcttg tgaagatatc ttttgtgcta cttgtggttc gaaaaatgct    660
acattgggta atgatatcat tctctgtgat ggagcttgtg acagagggtt ccaccagaac    720
tgtctaaatc ctcctctgcg tactgaagat attcccatgg gagatgaagg atggctctgc    780
ccagcatgtg attgcaagtt agactgtata gatctaataa atgaactcca gggcagtgac    840
atctccattg aggacccttg ggagaaagtt tttccagagg cagctgctat gaccaatggc    900
tctaagcaag atgaacgatt tgatcttcca tctgatgatt cggatgacaa tgattttgac    960
cccaatatgc ctgaagagca tgtggctagt aaggaagaag gatcttctga agaggaagag   1020
gatgatgacg gaggatcaga ctctgacgac tctgacttct taacctgttc tgatgatttg   1080
gaacctttga tagataaaaa gaaggttgat gacctgggat tatcttctga agattcagag   1140
gatgatgact atgatccagc aggtcctgat tcggataaag atgtcgaaaa gaagtcaaat   1200
tctgatgagt ctgatttcac gtctgactcg gatgattttt gtaaggagat taaaaaatct   1260
ggtggacatg atgaagtttc atcgcctcca ttacccgatg tcaaggtggg tgatatggaa   1320
aagaacactg ctcagtctaa cacaacaagt tctgctgacg accctatgga gactgaaata   1380
gaccagagtg tggttttacc agtatcaagg agacgtcaag ctgaacggtt ggactacaaa   1440
aggctgtatg atgaggcata tggtgaagca tcatctgatt ctagtgatga agaagaatgg   1500
tctgggaaga acacaccaat aaaaagcaat gaagagggtg aagttggttc tcctgcagga   1560
aaaggctcta gagttgcgca ccataatgaa ctgactacac aaaacactaa agaaagcttg   1620
cattctcttc atggttcagt agatgagaaa catggagatc ttacttctaa tggcagcaat   1680
atcaaagata ggaaaggaca ttttggtcca gtgattagtc agaagctaca tgaacatttt   1740
aagacacaac catacccctag tcgttctctt aaagaaagct tggcagaaga actagggcta   1800
acatttcatc aggtcaacag atggtttgaa aataggcgtc attttgcaag gttagcttct   1860
tccaggaaag gcatcagtcc agataagcat agcccccaaa atactaatag cccagtgaca   1920
cctagtatgc aacctaaaga accagagggg actgtgatgg aagaatctaa tgtatgcata   1980
aacagggatg ctaccatctc taaaaaagcg gtttcttcaa aagtcggatc aagaaagaac   2040
cttagcaaga gttcccctaa aagccgggtc aaaagttga                          2079
```

<210> 208
<211> 692
<212> PRT
<213> Zea mays

<400> 208

```
Met Glu Lys Lys Thr Val His Val Leu Ala Glu Gly Asn Gly Glu Ile
1               5                   10                  15
Glu Asn Gly Thr Ser Ser Ser Gln Asn Pro Glu Thr Leu Glu His Pro
            20                  25                  30
Val Leu Leu Ser Ala Ser Gln Thr Val Pro Asn Asn Leu Gly Ile Arg
        35                  40                  45
Lys Asn Tyr Lys Arg Ala Ala Asn Arg Gly Lys Lys Gly Ser Gln Gly
    50                  55                  60
```

Leu Thr Gly Gln Ala Tyr Thr Leu Arg Ser Ser Asp Ile Asp Val Arg
65 70 75 80
Val Leu Arg Ser Thr Ser Gly Ser Lys Thr Thr Ser Thr Glu His Val
85 90 95
Gln Pro Pro Gly Gln Pro Ala Ala Lys Arg Arg Lys Arg Ser Arg Ala
100 105 110
Ser Asn Lys Asn Ser Thr Asp Glu Phe Ser Gln Ile Arg Lys Arg Val
115 120 125
Arg Tyr Ile Leu Asn Arg Met Asn Tyr Glu Gln Ser Leu Ile Glu Ala
130 135 140
Tyr Ala Ser Glu Gly Trp Lys Asn Gln Ser Leu Asp Lys Ile Arg Pro
145 150 155 160
Glu Lys Glu Leu Glu Arg Ala Lys Ser Glu Ile Leu Arg Cys Lys Leu
165 170 175
Arg Ile Arg Glu Val Phe Gln Asn Ile Asp Ser Leu Leu Ser Lys Gly
180 185 190
Lys Ile Asp Glu Ser Leu Phe Asp Ser Glu Gly Glu Ile Ser Cys Glu
195 200 205
Asp Ile Phe Cys Ala Thr Cys Gly Ser Lys Asn Ala Thr Leu Gly Asn
210 215 220
Asp Ile Ile Leu Cys Asp Gly Ala Cys Asp Arg Gly Phe His Gln Asn
225 230 235 240
Cys Leu Asn Pro Pro Leu Arg Thr Glu Asp Ile Pro Met Gly Asp Glu
245 250 255
Gly Trp Leu Cys Pro Ala Cys Asp Cys Lys Leu Asp Cys Ile Asp Leu
260 265 270
Ile Asn Glu Leu Gln Gly Ser Asp Ile Ser Ile Glu Asp Pro Trp Glu
275 280 285
Lys Val Phe Pro Glu Ala Ala Ala Met Thr Asn Gly Ser Lys Gln Asp
290 295 300
Glu Arg Phe Asp Leu Pro Ser Asp Asp Ser Asp Asp Asn Asp Phe Asp
305 310 315 320
Pro Asn Met Pro Glu Glu His Val Ala Ser Lys Glu Glu Gly Ser Ser
325 330 335
Glu Glu Glu Glu Asp Asp Asp Gly Gly Ser Asp Ser Asp Asp Ser Asp
340 345 350
Phe Leu Thr Cys Ser Asp Asp Leu Glu Pro Leu Ile Asp Lys Lys Lys
355 360 365
Val Asp Asp Leu Gly Leu Ser Ser Glu Asp Ser Glu Asp Asp Asp Tyr
370 375 380
Asp Pro Ala Gly Pro Asp Ser Asp Lys Asp Val Glu Lys Lys Ser Asn
385 390 395 400
Ser Asp Glu Ser Asp Phe Thr Ser Asp Ser Asp Asp Phe Cys Lys Glu
405 410 415
Ile Lys Lys Ser Gly Gly His Asp Glu Val Ser Ser Pro Pro Leu Pro
420 425 430
Asp Val Lys Val Gly Asp Met Glu Lys Asn Thr Ala Gln Ser Asn Thr
435 440 445
Thr Ser Ser Ala Asp Asp Pro Met Glu Thr Glu Ile Asp Gln Ser Val
450 455 460
Val Leu Pro Val Ser Arg Arg Arg Gln Ala Glu Arg Leu Asp Tyr Lys
465 470 475 480
Arg Leu Tyr Asp Glu Ala Tyr Gly Glu Ala Ser Ser Asp Ser Ser Asp
485 490 495
Glu Glu Glu Trp Ser Gly Lys Asn Thr Pro Ile Lys Ser Asn Glu Glu
500 505 510
Gly Glu Val Gly Ser Pro Ala Gly Lys Gly Ser Arg Val Ala His His
515 520 525
Asn Glu Leu Thr Thr Gln Asn Thr Lys Glu Ser Leu His Ser Leu His
530 535 540
Gly Ser Val Asp Glu Lys His Gly Asp Leu Thr Ser Asn Gly Ser Asn

```
545                 550                 555                 560
Ile Lys Asp Arg Lys Gly His Phe Gly Pro Val Ile Ser Gln Lys Leu
                565                 570                 575
His Glu His Phe Lys Thr Gln Pro Tyr Pro Ser Arg Ser Leu Lys Glu
            580                 585                 590
Ser Leu Ala Glu Glu Leu Gly Leu Thr Phe His Gln Val Asn Arg Trp
        595                 600                 605
Phe Glu Asn Arg Arg His Phe Ala Arg Leu Ala Ser Ser Arg Lys Gly
    610                 615                 620
Ile Ser Pro Asp Lys His Ser Pro Gln Asn Thr Asn Ser Pro Val Thr
625                 630                 635                 640
Pro Ser Met Gln Pro Lys Glu Pro Glu Gly Thr Val Met Glu Glu Ser
                645                 650                 655
Asn Val Cys Ile Asn Arg Asp Ala Thr Ile Ser Lys Lys Ala Val Ser
            660                 665                 670
Ser Lys Val Gly Ser Arg Lys Asn Leu Ser Lys Ser Ser Pro Lys Ser
        675                 680                 685
Arg Val Lys Ser
    690
```

<210> 209
<211> 4731
<212> DNA
<213> Zea mays

<400> 209

```
atgggaagga aagggtcaga agtatcacca agcaaaaggt atcccttgag gtctgcacac     60
agtagtggta gagttcttcg ctctgcctta gccaataata ataaggacag tgagcctctg    120
aatgcggctg ctgctaccca agcagctgtg aaaaaaagaa gtggcaacca gctagatagt    180
cctaatagca ctgtcaggct acttcgttct acgtcaaaaa ataaggacga ggcacatagc    240
gggcctctta atgacaggat cattgataag ccagctgcaa ataaaagaaa acatgccaat    300
ccctcacagg ttggaagtcc cagcagcagt gtcagggtgc ttcgctctgc aataaaaaat    360
aaagatatgg cgcgcagtga gcctcaaagt gacaggaccg ctggtgaggc agctacaaat    420
aaaagaaaaa acgccaattc ctcaaaggtg gggagtctga gctctggtct cagtgtgctt    480
cggtctgctt caaaagctaa ggatgagaca tgtatcaaac ctttaaatga tagtcctgct    540
gttgaaccag cttcaagtaa aagaaaatgc accagtccgt caaaggtggg aagtcccagc    600
aacagtgtca gggtgcttcg atctacttca aaatataaga atgagacatg taccgagcct    660
ttaaatgata gtggtgctgt tgaaccagct gcaaatagaa ggaaaggtgt cagtcgttca    720
aaggagggaa gtcccaatag cagtgtcagg gtgcttcgct ctgcctcaaa taataagatt    780
gatgtatcta ttgagccact gaataatagt actactggtc aaccggctgc gaaaagaaga    840
aaaaatggca gttcctttaa gagcagtgcc agggtgcttt gctctacctc agaaaggaaa    900
aatgaggcat ctagtgagcc tttaaatgat agtactgctg ctcagccagc tgcgaggaaa    960
aagaaaggtg gtgcaatgcc aaagactgat aacccaaaga ttggtgtcag ggttcttcgc   1020
tctgcctcag gaaagaagaa tgagacatgt actgagcctg taaatcggag tacttctcgt   1080
gaaccaactg tgacaaaaaa aagaaattgc aagccttcaa aggatcgcgg tcccaagaag   1140
gactacttaa aaatttgtca aagaattaaa tatattttga atcgaatgaa gtatgaacaa   1200
gcctttattc aggcttatgc aagtgaaggt tggaaaggcc aaagtttgga aaaaataaga   1260
cccgagaagg agcttgaacg agccaaggca gaaatccttc aatgcaagtt aagaatccgg   1320
gaagcttttc gaaatatgga ctctctttta tcgaagggga agcttgaaga atctttgttt   1380
gattcagcag gacaaatttc aagcgaagat atattttgtg ccatatgtgg ctcaaaggat   1440
gttacttcac aaaatgacat cattctttgt gatggagcct gtgacagagg attccaccag   1500
aattgtttga gccctccttt gctaactgaa gaaattcctc cgggggatga aggatggctt   1560
tgccctgcat gtgtgtgcaa agcagactat atagatgcgt taaatgaact tcaaggaagc   1620
aaactctcca ttcatgactc atgggtgaaa gttttccctg aggcagcttc aactgccaat   1680
ggttctaaac aagttgatgc ttctgatctg ctgccagatc atataaagga cagtgctaac   1740
cttgcattgg ttggaacaca catggtgaat gaaatcaggt tttctgaaga ggatgatagc   1800
aaagcggatg acctcggctt gccttcggag gactcagggg atggtgactt cgatccagca   1860
ggtccagatt ctagtgaaga ccaaaatgat ggattgaact cagaagagtc agatttcaca   1920
tctgactctg attatttttg tgctgagatt gctaaatctt gcggccagga tgaagtctcg   1980
gcatctccat tatcaaatgt gataaaccgt actgatagaa tgaaactcag ggcctacagt   2040
agacgctcta atgaagaaaa ccacaatcat gcatttatgg acatggagct agagcaagac   2100
```

```
atagtgctac cagttccaag caggcggcag gttgaacggt tggattacaa aaaactatac   2160
gatgaggcat atgaggaaga atcatctaat tcaagtgatg aagaagagtg gtctggaaaa   2220
gaactcttag aaggcagtgg aacagattca cttgatgagc cttcgtcctg taaaaggctc   2280
tctagaagag caccagcagg gcagcagaat aatgaactta caccacagag ccggcctcat   2340
ggttctaaca gtgagcagaa aactgaagtt cttcgctcca atggcagtag tagcacagga   2400
cggaagtatg atcctgaagt tacccagaaa ttgaaggtgc attttgagaa agatccatat   2460
cctagccgtg aaacaaaaga aaatctatca gaagaactag gcttgacatt taatcaggtc   2520
tccaaatggt tctctagtac tcgtcactat tcaagggttg cttctgccaa aaaagaaatg   2580
catcctgaca gccatacttg tgagaataat gatgagacaa ctgtagacag catgcaagca   2640
aaacaaccca atgctggggt gatggaaaag ttgacggggg atagaaatgg cattgttcct   2700
gagaaaccga tggtgcagga caatcttaac caaggagcga tggaaaagtt aactagggat   2760
aaaaatgaca ttgttcctga gaaaccggtg atgcagagca gtcttaacca atgtaataat   2820
gaagatatac cgctttctgg aacagaaatt gagatggagt cttacgaaca agaatcatct   2880
gactcaagtg atgaagagtg gtccatactt agcacaccac gaaaaacaag attacaagac   2940
catggaaaat catcagttac tgaatcactt cgttctgcaa aacggtgttc cagaagagca   3000
ccagctaggg agcagaataa tgaacgtatt cagagtgagc agcttcatgg ttctgcaagt   3060
gagcagcaaa ctgaatttct tcgctccaat gtcagcagtg gcaaggtctc aaaatatcat   3120
tttggcccta tagttagtca gaagcttaag gaacattttg aaaaagatcc atatccttgc   3180
cgtgcaacaa aagagagtct ggcacaagag ctaggactga catttaatca gatcagtaag   3240
tggttctctg gcactcgtca ttattcaaga gatgctgttg ccaagaatca gaaacgcccg   3300
ggagaaaata ctactgtgaa taataatagc acaacctttg atgacataca agtaatagaa   3360
cccaatgttg ggttgataga taaaccagct gcagatgtaa atgacatgat ttctgaaaag   3420
ttgatggtcc aaataaatct taacgaaggc attgaagaag atatcccacc gagccaatat   3480
gccaggtgtg aagagaaaat aactatgact ccagctgcca tctcaaggga agctggtctt   3540
ccaggatatg gtcctggtcc tggagaaaac tttcttcagg tcagttcaag gaatactagc   3600
tgtgagcaga gtgtgatact ggctcctacc gctatcgcaa gagaagttgg tccaccagga   3660
tatgcgactg gagaaaacca gggcaatggg gctccatgga atacaagtta tgagcgaaga   3720
ctatttatga atcctgtgac cagctcaaga gcagtcagtc ctccaggata tgggcctgaa   3780
gaaagccgag gcagtggcat ttcctggaat acaagcagtg aacaaatatt gtttatgagt   3840
cctacaacca tctcaagaga acttggtcca ccaggatatg ggcctagaga agaccagtgc   3900
agtggcactt catggaatgc aagctgcgag ttgggagtat ttatgagtcc tgtgaccacc   3960
tcaagagaag acagtccgcc aggatatggg cctcgagaaa accaggagaa tgacaatatg   4020
agatgcgagt tgagaatgtt tacaagtcct acaaccatct ccagagaagt tggcccacca   4080
ggatatgagc ttactggaaa aaacaagggc aacggcgctt catgtactac gaattaccag   4140
cagggagcgt ttacgagtcc tgaagccttc ccaagagaag cctgtcctcc aggatttgtg   4200
tctggagaga accagggtaa cgacactcca tggtatatga gcagcaaaca gatggcgttt   4260
acgagtccta cgaccaaccc tagagtaggt ccgccagggt atggacagga aaaccagggc   4320
agtcgtgttt cttggaatac gagttatgag cagggagtgt ttatgagttc tacaaacatc   4380
tcaagagaag ccggtccgcc aggttatggg cctggggaaa gtcagggcaa tatcacttca   4440
tggatttcga caggcgggca gagaatgttt acaggtcctt caactatccc gaacgaagtc   4500
gggcagcaag gatatggccc ttcaggaatc ccgggaacgg atggctcaag gagtatggac   4560
ttgaaagcct ttccaccagg atatggacct gctggagaaa accagggcgg tggtgcttca   4620

gggagcgtca taagccccca tgctcggtct gcagagaacg tagagtttc agacgacgct    4680
agaaaaaagg ctatacagcg ggagctgaga cggcggcaga agttcaggtg a            4731
```

<210> 210
<211> 1576
<212> PRT
<213> Zea mays

<400> 210

```
Met Gly Arg Lys Gly Ser Glu Val Ser Pro Ser Lys Arg Tyr Pro Leu
1               5                   10                  15
Arg Ser Ala His Ser Ser Gly Arg Val Leu Arg Ser Ala Leu Ala Asn
            20                  25                  30
Asn Asn Lys Asp Ser Glu Pro Leu Asn Ala Ala Ala Ala Thr Gln Ala
        35                  40                  45
Ala Val Lys Lys Arg Ser Gly Asn Gln Leu Asp Ser Pro Asn Ser Thr
    50                  55                  60
Val Arg Leu Leu Arg Ser Thr Ser Lys Asn Lys Asp Glu Ala His Ser
```

```
65                  70                  75                  80
Gly Pro Leu Asn Asp Arg Ile Ile Asp Lys Pro Ala Ala Asn Lys Arg
                85                  90                  95
Lys His Ala Asn Pro Ser Gln Val Gly Ser Pro Ser Ser Ser Val Arg
            100                 105                 110
Val Leu Arg Ser Ala Ile Lys Asn Lys Asp Met Ala Arg Ser Glu Pro
        115                 120                 125
Gln Ser Asp Arg Thr Ala Gly Glu Ala Ala Thr Asn Lys Arg Lys Asn
    130                 135                 140
Ala Asn Ser Ser Lys Val Gly Ser Leu Ser Ser Gly Leu Ser Val Leu
145                 150                 155                 160
Arg Ser Ala Ser Lys Ala Lys Asp Glu Thr Cys Ile Lys Pro Leu Asn
                165                 170                 175
Asp Ser Pro Ala Val Glu Pro Ala Ser Ser Lys Arg Lys Cys Thr Ser
            180                 185                 190
Pro Ser Lys Val Gly Ser Pro Ser Asn Ser Val Arg Val Leu Arg Ser
        195                 200                 205
Thr Ser Lys Tyr Lys Asn Glu Thr Cys Thr Glu Pro Leu Asn Asp Ser
    210                 215                 220
Gly Ala Val Glu Pro Ala Ala Asn Arg Arg Lys Gly Val Ser Arg Ser
225                 230                 235                 240
Lys Glu Gly Ser Pro Asn Ser Ser Val Arg Val Leu Arg Ser Ala Ser
                245                 250                 255
Asn Asn Lys Ile Asp Val Ser Ile Glu Pro Leu Asn Asn Ser Thr Thr
            260                 265                 270
Gly Gln Pro Ala Ala Lys Arg Arg Lys Asn Gly Ser Ser Phe Lys Ser
        275                 280                 285
Ser Ala Arg Val Leu Cys Ser Thr Ser Glu Arg Lys Asn Glu Ala Ser
    290                 295                 300
Ser Glu Pro Leu Asn Asp Ser Thr Ala Ala Gln Pro Ala Ala Arg Lys
305                 310                 315                 320
Lys Lys Gly Gly Ala Met Pro Lys Thr Asp Asn Pro Lys Ile Gly Val
                325                 330                 335
Arg Val Leu Arg Ser Ala Ser Gly Lys Lys Asn Glu Thr Cys Thr Glu
            340                 345                 350
Pro Val Asn Arg Ser Thr Ser Arg Glu Pro Thr Val Thr Lys Lys Arg
        355                 360                 365
Asn Cys Lys Pro Ser Lys Asp Arg Gly Pro Lys Lys Asp Tyr Leu Lys
    370                 375                 380
Ile Cys Gln Arg Ile Lys Tyr Ile Leu Asn Arg Met Lys Tyr Glu Gln
385                 390                 395                 400
Ala Phe Ile Gln Ala Tyr Ala Ser Glu Gly Trp Lys Gly Gln Ser Leu

                405                 410                 415
Glu Lys Ile Arg Pro Glu Lys Glu Leu Glu Arg Ala Lys Ala Glu Ile
            420                 425                 430
Leu Gln Cys Lys Leu Arg Ile Arg Glu Ala Phe Arg Asn Met Asp Ser
        435                 440                 445
Leu Leu Ser Lys Gly Lys Leu Glu Glu Ser Leu Phe Asp Ser Ala Gly
    450                 455                 460
Gln Ile Ser Ser Glu Asp Ile Phe Cys Ala Ile Cys Gly Ser Lys Asp
465                 470                 475                 480
Val Thr Ser Gln Asn Asp Ile Ile Leu Cys Asp Gly Ala Cys Asp Arg
                485                 490                 495
Gly Phe His Gln Asn Cys Leu Ser Pro Pro Leu Leu Thr Glu Glu Ile
            500                 505                 510
Pro Pro Gly Asp Glu Gly Trp Leu Cys Pro Ala Cys Val Cys Lys Ala
        515                 520                 525
Asp Tyr Ile Asp Ala Leu Asn Glu Leu Gln Gly Ser Lys Leu Ser Ile
    530                 535                 540
His Asp Ser Trp Val Lys Val Phe Pro Glu Ala Ala Ser Thr Ala Asn
```

```
545                 550                 555                 560
Gly Ser Lys Gln Val Asp Ala Ser Asp Leu Leu Pro Asp His Ile Lys
                565                 570                 575
Asp Ser Ala Asn Leu Ala Leu Val Gly Thr His Met Val Asn Glu Ile
            580                 585                 590
Arg Phe Ser Glu Glu Asp Asp Ser Lys Ala Asp Asp Leu Gly Leu Pro
        595                 600                 605
Ser Glu Asp Ser Gly Asp Gly Asp Phe Asp Pro Ala Gly Pro Asp Ser
    610                 615                 620
Ser Glu Asp Gln Asn Asp Gly Leu Asn Ser Glu Glu Ser Asp Phe Thr
625                 630                 635                 640
Ser Asp Ser Asp Tyr Phe Cys Ala Glu Ile Ala Lys Ser Cys Gly Gln
                645                 650                 655
Asp Glu Val Ser Ala Ser Pro Leu Ser Asn Val Ile Asn Arg Thr Asp
            660                 665                 670
Arg Met Lys Leu Arg Ala Tyr Ser Arg Arg Ser Asn Glu Glu Asn His
        675                 680                 685
Asn His Ala Phe Met Asp Met Glu Leu Glu Gln Asp Ile Val Leu Pro
    690                 695                 700
Val Pro Ser Arg Arg Gln Val Glu Arg Leu Asp Tyr Lys Lys Leu Tyr
705                 710                 715                 720
Asp Glu Ala Tyr Glu Glu Glu Ser Ser Asn Ser Ser Asp Glu Glu Glu
                725                 730                 735
Trp Ser Gly Lys Glu Leu Leu Glu Gly Ser Gly Thr Asp Ser Leu Asp
            740                 745                 750
Glu Pro Ser Ser Cys Lys Arg Leu Ser Arg Arg Ala Pro Ala Gly Gln
        755                 760                 765
Gln Asn Asn Glu Leu Thr Pro Gln Ser Arg Pro His Gly Ser Asn Ser
    770                 775                 780
Glu Gln Lys Thr Glu Val Leu Arg Ser Asn Gly Ser Ser Ser Thr Gly
785                 790                 795                 800
Arg Lys Tyr Asp Pro Glu Val Thr Gln Lys Leu Lys Val His Phe Glu
                805                 810                 815
Lys Asp Pro Tyr Pro Ser Arg Glu Thr Lys Glu Asn Leu Ser Glu Glu
            820                 825                 830
Leu Gly Leu Thr Phe Asn Gln Val Ser Lys Trp Phe Ser Ser Thr Arg
        835                 840                 845
His Tyr Ser Arg Val Ala Ser Ala Lys Lys Glu Met His Pro Asp Ser
    850                 855                 860
His Thr Cys Glu Asn Asn Asp Glu Thr Thr Val Asp Ser Met Gln Ala
865                 870                 875                 880
Lys Gln Pro Asn Ala Gly Val Met Glu Lys Leu Thr Gly Asp Arg Asn
                885                 890                 895
Gly Ile Val Pro Glu Lys Pro Met Val Gln Asp Asn Leu Asn Gln Gly
            900                 905                 910
Ala Met Glu Lys Leu Thr Arg Asp Lys Asn Asp Ile Val Pro Glu Lys
        915                 920                 925
Pro Val Met Gln Ser Ser Leu Asn Gln Cys Asn Asn Glu Asp Ile Pro
    930                 935                 940
Leu Ser Gly Thr Glu Ile Glu Met Glu Ser Tyr Glu Gln Glu Ser Ser
945                 950                 955                 960
Asp Ser Ser Asp Glu Glu Trp Ser Ile Leu Ser Thr Pro Arg Lys Thr
                965                 970                 975
Arg Leu Gln Asp His Gly Lys Ser Ser Val Thr Glu Ser Leu Arg Ser
            980                 985                 990
Ala Lys Arg Cys Ser Arg Arg Ala  Pro Ala Arg Glu Gln  Asn Asn Glu
        995                 1000                1005
Arg Ile  Gln Ser Glu Gln Leu  His Gly Ser Ala Ser  Glu Gln Gln
    1010                1015                1020
Thr Glu  Phe Leu Arg Ser Asn  Val Ser Ser Gly Lys  Val Ser Lys
    1025                1030                1035
```

```
Tyr His  Phe Gly Pro Ile Val  Ser Gln Lys Leu Lys  Glu His Phe
    1040                 1045                 1050
Glu Lys  Asp Pro Tyr Pro Cys  Arg Ala Thr Lys Glu  Ser Leu Ala
    1055                 1060                 1065
Gln Glu  Leu Gly Leu Thr Phe  Asn Gln Ile Ser Lys  Trp Phe Ser
    1070                 1075                 1080
Gly Thr  Arg His Tyr Ser Arg  Asp Ala Val Ala Lys  Asn Gln Lys
    1085                 1090                 1095
Arg Pro  Gly Glu Asn Thr Thr  Val Asn Asn Asn Ser  Thr Thr Phe
    1100                 1105                 1110
Asp Asp  Ile Gln Val Ile Glu  Pro Asn Val Gly Leu  Ile Asp Lys
    1115                 1120                 1125
Pro Ala  Ala Asp Val Asn Asp  Met Ile Ser Glu Lys  Leu Met Val
    1130                 1135                 1140
Gln Ile  Asn Leu Asn Glu Gly  Ile Glu Glu Asp Ile  Pro Pro Ser
    1145                 1150                 1155
Gln Tyr  Ala Arg Cys Glu Glu  Lys Ile Thr Met Thr  Pro Ala Ala
    1160                 1165                 1170
Ile Ser  Arg Glu Ala Gly Leu  Pro Gly Tyr Gly Pro  Gly Pro Gly
    1175                 1180                 1185
Glu Asn  Phe Leu Gln Val Ser  Ser Arg Asn Thr Ser  Cys Glu Gln
    1190                 1195                 1200
Ser Val  Ile Leu Ala Pro Thr  Ala Ile Ala Arg Glu  Val Gly Pro
    1205                 1210                 1215
Pro Gly  Tyr Ala Thr Gly Glu  Asn Gln Gly Asn Gly  Ala Pro Trp
    1220                 1225                 1230
Asn Thr  Ser Tyr Glu Arg Arg  Leu Phe Met Asn Pro  Val Thr Ser
    1235                 1240                 1245
Ser Arg  Ala Val Ser Pro Pro  Gly Tyr Gly Pro Glu  Glu Ser Arg
    1250                 1255                 1260
Gly Ser  Gly Ile Ser Trp Asn  Thr Ser Ser Glu Gln  Ile Leu Phe
    1265                 1270                 1275
Met Ser  Pro Thr Thr Ile Ser  Arg Glu Leu Gly Pro  Pro Gly Tyr
    1280                 1285                 1290

Gly Pro  Arg Glu Asp Gln Cys  Ser Gly Thr Ser Trp  Asn Ala Ser
    1295                 1300                 1305
Cys Glu  Leu Gly Val Phe Met  Ser Pro Val Thr Thr  Ser Arg Glu
    1310                 1315                 1320
Asp Ser  Pro Pro Gly Tyr Gly  Pro Arg Glu Asn Gln  Glu Asn Asp
    1325                 1330                 1335
Asn Met  Arg Cys Glu Leu Arg  Met Phe Thr Ser Pro  Thr Thr Ile
    1340                 1345                 1350
Ser Arg  Glu Val Gly Pro Pro  Gly Tyr Glu Leu Thr  Gly Lys Asn
    1355                 1360                 1365
Lys Gly  Asn Gly Ala Ser Cys  Thr Thr Asn Tyr Gln  Gln Gly Ala
    1370                 1375                 1380
Phe Thr  Ser Pro Glu Ala Phe  Pro Arg Glu Ala Cys  Pro Pro Gly
    1385                 1390                 1395
Phe Val  Ser Gly Glu Asn Gln  Gly Asn Asp Thr Pro  Trp Tyr Met
    1400                 1405                 1410
Ser Ser  Lys Gln Met Ala Phe  Thr Ser Pro Thr Thr  Asn Pro Arg
    1415                 1420                 1425
Val Gly  Pro Pro Gly Tyr Gly  Gln Glu Asn Gln Gly  Ser Arg Val
    1430                 1435                 1440
Ser Trp  Asn Thr Ser Tyr Glu  Gln Gly Val Phe Met  Ser Ser Thr
    1445                 1450                 1455
Asn Ile  Ser Arg Glu Ala Gly  Pro Pro Gly Tyr Gly  Pro Gly Glu
    1460                 1465                 1470
Ser Gln  Gly Asn Ile Thr Ser  Trp Ile Ser Thr Gly  Gly Gln Arg
    1475                 1480                 1485
```

```
Met Phe  Thr Gly Pro Ser Thr  Ile Pro Asn Glu Val  Gly Gln Gln
    1490                 1495                 1500
Gly Tyr  Gly Pro Ser Gly Ile  Pro Gly Thr Asp Gly  Ser Arg Ser
    1505                 1510                 1515
Met Asp  Leu Lys Ala Phe Pro  Pro Gly Tyr Gly Pro  Ala Gly Glu
    1520                 1525                 1530
Asn Gln  Gly Gly Gly Ala Ser  Gly Ser Val Ile Ser  Pro His Ala
    1535                 1540                 1545
Arg Ser  Ala Glu Asn Val Glu  Phe Ser Asp Asp Ala  Arg Lys Lys
    1550                 1555                 1560
Ala Ile  Gln Arg Glu Leu Arg  Arg Arg Gln Lys Phe  Arg
    1565                 1570                 1575
```

<210> 211
<211> 4620
<212> DNA
<213> Zea mays

<400> 211

```
atgggaagga aaggctcaga aatgtcacca agtaaaaggt atcccttgag gtctccacat     60
agtagtggta gagttcttcg ctctgcctta gccaatgata ataaggacag tgagcctctg    120
aatgctgctg ctgctaccca agccgctgtg aaaaaaagaa gtggcagtca gttggatagt    180
cctaatagca gtgtcaggtt gcttcactct acatcaaaaa ataagaacga ggtatgtagc    240
gggcctctaa atgacaggat ccctgataag ccagctgcaa ataaaagaaa acgtgccagt    300
ccttcacagg tgggaagttc cagcaacagt gtcagggtcg ttcgctctgc aataaaaaat    360
aaagatgagg catgcagtaa atctctaagt gacaggaccg ctggtgagcc agctgcaaat    420
aaaagaaaaa acgtcaatcg ctcaaagacg gggagtctga gctgtggtgt cagggtgctt    480
ctatctgctt caaaagctaa ggatgagaca tctaccaagc ctttaaatga tagtgctact    540
gttgaaccag cttcaggtaa aaggaaaggc accagtccgt caaaggtggg aagtgccagc    600
aatagtgtca gggtgcttcg atctacttca aaatataaga atgagacatg tactgagcct    660
ttaaatgata gtggtgctgt tgaaccagct gcaaataaaa ggaaaggtgt cagtcgttca    720
aaggagagaa gtctcaatag cagtgtcagg gtcgttgctc tggcctcaaa taataagatt    780
gaggtatcta ttaagcctct aggtaatagt actgctgggg aaccagctgc gaaaagaaga    840
aaaagcggca gttccttcga ggcaggaagc cctgtgagca gtgccagggt gcttcgccct    900
acctcagaaa ggaagaatga ggcatctaag cctttaaatg aaagtactgc tgctcaacca    960
gctgcgagga aaaaaaaagc tggggtaatt tcaaagaccg acaatccaaa gattggtctc   1020
agggttcttc gctctgcctc aggaaagaag aatgaagcat gtattgggca tgtaaatgat   1080
agtacttctg ctgaaccaac tgtgacaaaa agaaaccgca agccttcaat ggatcgcagt   1140
cccaagaagg actacttaaa aatttgtcaa agagttagat atattttgaa tcgaatgaac   1200
tatcaacaaa cctttattca agcttatgca agtgaaggtt ggaaaggcca aagtttggaa   1260
aaaataagac ctgaaaagga gcttgaacga gccaaggcag aaatcctcca atgcaagtta   1320
agaatccgtg aagcttttcg aaatatggac tctcttttat ccaaggggaa gcttgaagaa   1380
tctttgtttg attccgcagg agaaatttca agcgaagata tattttgtgc tgtatgtggc   1440
tcaaaggatg ttactttaca aaatgacatc attctttgtg atggagcctg tgacagagga   1500
ttccaccaga actgtttgaa ccctcctttg ctaactgaag atatacctcc ggggggatcaa   1560
agatggcttt gccctgcatg tgtctgcaaa gcagactcta tagatgcact aaatgaactt   1620
caagggagca agctctccat tcatgactca tgggagaaag ttttccctga ggcagcttca   1680
attgccaacg gttctaaaca agttgatact tctgatctgc tgccagatca tataaagcac   1740
agtgacaacc ctgcattggt tgaagggctg atggtggatg aagtcaggct ttctgcagag   1800
gatgacagca aagcggatga ccttcgcttg tcttcggagg actcagggga tggtgacttc   1860
gatccgtcag gtccagattc tagtgaagac caaaaagatg gattgaactc agaagagtca   1920
gatttcacat cagactctga tgatttttgt gctgagattg ccaaatcttg tggccaggat   1980
gaagtctcgg catctccatt atcaaatgtg ataaaccata cttatagaat gaaactcagg   2040
gccagcaaca accgctctaa tgaagaaaac catgatcatg tatttatgga catggagcta   2100
gggcaagaca tggtgctacc agtttcaagc aggcggcagg tggaacggtt ggattacaaa   2160
aaactatatg atgaggcata tgggaaagaa tcatctaatt caagtgatga caaagagtgg   2220
tctggaaaag aactattaga aggcagtgaa acagattcac ttagtgagcg acctcatcct   2280
gtaaaacggt gctctagaag agcacaagca gagcagcaga ataatgaaca tacaccacag   2340
agggagcgcc ttcatggttc tgaaagtgag cagaaaactg gaattcttcg atccaatggc   2400
agcagtagca caggacggaa gtttggtcct gtagctaccc agaaattgaa ggtacatttt   2460
```

```
gagaaagatc catatcctag ccgtgaaacg aaagagaata tatcagaaga actaggcttg   2520

acatttaatc aggtctccag atggttctct agcactcgtc actattcaag ggttgcttct   2580
gccagaaaag aaatgcatcc tgacggccat actagcgaga ataatgatac cacgactgta   2640
gatagcatgc aagcaaggca acccaacact gtggtgatgg aaaagttgac tgggaataga   2700
aatgacattg tttctgagaa accgatggtg cagaacaatc ttaaccaagg ggtggcagaa   2760
aagttgacta gggatagaaa tgacattgtt cctgagaaac aggtggtgca gagcaatctt   2820
aaccaatgta ataatgaaga tatcccgctt tctggaacag aaattgagat ggagtcttac   2880
gaacaagaat catctgaaag cagtgatgaa gagtggtcca cacttagcac accacgaaaa   2940
acaaaattac aaggcaacga aaaagtatca cttactgaat cactacgttc tgcaaaacgg   3000
tgttctagaa gagcaccagc tagggagcag aataatgaac atactcagag tgagcaactt   3060
catggttctg taagtaaaca gcaaactgaa gttcttcgct ccaatgtcag cagtggcgat   3120
gcctcaaaat gtcattttgg ccctatagtt actcagaagc tgaaggaaca ttttgaaaaa   3180
gatccatatc cttgccgtgc aacaaaagag ggtctggcac aagagctagg actgacattt   3240
aatcagatca gtaagtggtt ctctgccacc catcattatt caagagatgc tgttgccaag   3300
aaccaaaaat acccaggaga aaatactact gagaataata gtagcacaat ctttgatggt   3360
atacaagtaa tagaacccaa ttttggtttg atagataaac cagatgcaga tacaaatgac   3420
atgatttctg aaaagttgat ggtacaaatt aatcttaacg aaggcattga agaggatatc   3480
ccaccgagcc aatatactac caggtgtgaa gagaaattaa ctatgactca agctgccatc   3540
tcaagggaag ctggacctcc aggttatggt cctggagaaa actttcttca ggtcagttca   3600
aggattacta gctgtgagca gagtgtgata atggctccta gcgctatcgc aagaggagtt   3660
ggtccaccag gatatacgcc tggagaacac cagggcaatg ggctccatg gaatacaagc   3720
tatgagcgaa gaccgtttac gagtcctgcg actagctcaa gagaagtcgg acctccagga   3780
tatgggcctg aagaaaacca aggcagtggc atttcctgta atacaagcag agagcaaata   3840
ttgtttatga gccctacaac catctcgaga gaacttggtc caccaggata tttgcccaga   3900
gaaaaccagc gcagtgacac ttcatggaat acaagctgcg agtcgggact atttatgagt   3960
cctgcgacca cctcaataga acatggtcca ccaggatatg ggcctggaga aaaccaggag   4020
aatgacagta caagctgcga gttgagaatg tttaccagtc ctacaatcgt ctccagagaa   4080
gttggcccac caggatacca gcagggagtg tttatgagtc ctaaaaccat ctcaagagaa   4140
gtctgtcctc caggatttgg gtctggagag aaccagggca acgacactac atggtacaaa   4200
cagagcgcgt ttacaagtcc tacaaccatc tcgagagaag tcggtccgcc agggtatgga   4260
caggaagatc agggcaatgg tggttcttgg aacacgagtt gtgagcaggg agtgtttacg   4320
agtcctacta gaggcatctc aagagaagtc ggtccgccag gatacggggcc cggggaaagt   4380
cagggcaata ccacttcgtg gatttcgacc tgccagcaga gaatgcttgc aggaatccag   4440
agaactggtg gctcaaggag tatggacttg gaagcctttc caccgggata cggacctggt   4500
ggtgcttcag ggagtgtcat aagcccccaa gctcggtctg cagagaatgt agaattttcg   4560
gacgaggcta gaaaaaaggc tatacagcgg gagctgagac ggcggcagaa gttcaggtga   4620
```

<210> 212
<211> 1539
<212> PRT
<213> Zea mays

<400> 212

```
Met Gly Arg Lys Gly Ser Glu Met Ser Pro Ser Lys Arg Tyr Pro Leu
1               5                   10                  15
Arg Ser Pro His Ser Ser Gly Arg Val Leu Arg Ser Ala Leu Ala Asn
            20                  25                  30
Asp Asn Lys Asp Ser Glu Pro Leu Asn Ala Ala Ala Ala Thr Gln Ala
        35                  40                  45
Ala Val Lys Lys Arg Ser Gly Ser Gln Leu Asp Ser Pro Asn Ser Ser
    50                  55                  60
Val Arg Leu Leu His Ser Thr Ser Lys Asn Lys Asn Glu Val Cys Ser
65                  70                  75                  80
Gly Pro Leu Asn Asp Arg Ile Pro Asp Lys Pro Ala Ala Asn Lys Arg
                85                  90                  95
Lys Arg Ala Ser Pro Ser Gln Val Gly Ser Ser Ser Asn Ser Val Arg
            100                 105                 110

Val Val Arg Ser Ala Ile Lys Asn Lys Asp Glu Ala Cys Ser Lys Ser
        115                 120                 125
```

```
Leu Ser Asp Arg Thr Ala Gly Glu Pro Ala Ala Asn Lys Arg Lys Asn
    130                 135                 140
Val Asn Arg Ser Lys Thr Gly Ser Leu Ser Cys Gly Val Arg Val Leu
145                 150                 155                 160
Leu Ser Ala Ser Lys Ala Lys Asp Glu Thr Ser Thr Lys Pro Leu Asn
                165                 170                 175
Asp Ser Ala Thr Val Glu Pro Ala Ser Gly Lys Arg Lys Gly Thr Ser
            180                 185                 190
Pro Ser Lys Val Gly Ser Ala Ser Asn Ser Val Arg Val Leu Arg Ser
        195                 200                 205
Thr Ser Lys Tyr Lys Asn Glu Thr Cys Thr Glu Pro Leu Asn Asp Ser
    210                 215                 220
Gly Ala Val Glu Pro Ala Ala Asn Lys Arg Lys Gly Val Ser Arg Ser
225                 230                 235                 240
Lys Glu Arg Ser Leu Asn Ser Ser Val Arg Val Val Ala Leu Ala Ser
                245                 250                 255
Asn Asn Lys Ile Glu Val Ser Ile Lys Pro Leu Gly Asn Ser Thr Ala
            260                 265                 270
Gly Glu Pro Ala Ala Lys Arg Arg Lys Ser Gly Ser Ser Phe Glu Ala
        275                 280                 285
Gly Ser Pro Val Ser Ser Ala Arg Val Leu Arg Pro Thr Ser Glu Arg
    290                 295                 300
Lys Asn Glu Ala Ser Lys Pro Leu Asn Glu Ser Thr Ala Ala Gln Pro
305                 310                 315                 320
Ala Ala Arg Lys Lys Lys Ala Gly Val Ile Ser Lys Thr Asp Asn Pro
                325                 330                 335
Lys Ile Gly Leu Arg Val Leu Arg Ser Ala Ser Gly Lys Lys Asn Glu
            340                 345                 350
Ala Cys Ile Gly His Val Asn Asp Ser Thr Ser Ala Glu Pro Thr Val
        355                 360                 365
Thr Lys Arg Asn Arg Lys Pro Ser Met Asp Arg Ser Pro Lys Lys Asp
    370                 375                 380
Tyr Leu Lys Ile Cys Gln Arg Val Arg Tyr Ile Leu Asn Arg Met Asn
385                 390                 395                 400
Tyr Gln Gln Thr Phe Ile Gln Ala Tyr Ala Ser Glu Gly Trp Lys Gly
                405                 410                 415
Gln Ser Leu Glu Lys Ile Arg Pro Glu Lys Glu Leu Glu Arg Ala Lys
            420                 425                 430
Ala Glu Ile Leu Gln Cys Lys Leu Arg Ile Arg Glu Ala Phe Arg Asn
        435                 440                 445
Met Asp Ser Leu Leu Ser Lys Gly Lys Leu Glu Glu Ser Leu Phe Asp
    450                 455                 460
Ser Ala Gly Glu Ile Ser Ser Glu Asp Ile Phe Cys Ala Val Cys Gly
465                 470                 475                 480
Ser Lys Asp Val Thr Leu Gln Asn Asp Ile Ile Leu Cys Asp Gly Ala
                485                 490                 495
Cys Asp Arg Gly Phe His Gln Asn Cys Leu Asn Pro Pro Leu Leu Thr
            500                 505                 510
Glu Asp Ile Pro Pro Gly Asp Gln Arg Trp Leu Cys Pro Ala Cys Val
        515                 520                 525
Cys Lys Ala Asp Ser Ile Asp Ala Leu Asn Glu Leu Gln Gly Ser Lys
    530                 535                 540
Leu Ser Ile His Asp Ser Trp Glu Lys Val Phe Pro Glu Ala Ala Ser
545                 550                 555                 560
Ile Ala Asn Gly Ser Lys Gln Val Asp Thr Ser Asp Leu Leu Pro Asp
                565                 570                 575
His Ile Lys His Ser Asp Asn Pro Ala Leu Val Glu Gly Leu Met Val
            580                 585                 590
Asp Glu Val Arg Leu Ser Ala Glu Asp Asp Ser Lys Ala Asp Asp Leu
        595                 600                 605
Arg Leu Ser Ser Glu Asp Ser Gly Asp Gly Asp Phe Asp Pro Ser Gly
```

610 615 620
Pro Asp Ser Ser Glu Asp Gln Lys Asp Gly Leu Asn Ser Glu Glu Ser
625 630 635 640
Asp Phe Thr Ser Asp Ser Asp Asp Phe Cys Ala Glu Ile Ala Lys Ser
645 650 655
Cys Gly Gln Asp Glu Val Ser Ala Ser Pro Leu Ser Asn Val Ile Asn
660 665 670
His Thr Tyr Arg Met Lys Leu Arg Ala Ser Asn Asn Arg Ser Asn Glu
675 680 685
Glu Asn His Asp His Val Phe Met Asp Met Glu Leu Gly Gln Asp Met
690 695 700
Val Leu Pro Val Ser Ser Arg Arg Gln Val Glu Arg Leu Asp Tyr Lys
705 710 715 720
Lys Leu Tyr Asp Glu Ala Tyr Gly Lys Glu Ser Ser Asn Ser Ser Asp
725 730 735
Asp Lys Glu Trp Ser Gly Lys Glu Leu Leu Glu Gly Ser Glu Thr Asp
740 745 750
Ser Leu Ser Glu Arg Pro His Pro Val Lys Arg Cys Ser Arg Arg Ala
755 760 765
Gln Ala Glu Gln Gln Asn Asn Glu His Thr Pro Gln Arg Glu Arg Leu
770 775 780
His Gly Ser Glu Ser Glu Gln Lys Thr Gly Ile Leu Arg Ser Asn Gly
785 790 795 800
Ser Ser Ser Thr Gly Arg Lys Phe Gly Pro Val Ala Thr Gln Lys Leu
805 810 815
Lys Val His Phe Glu Lys Asp Pro Tyr Pro Ser Arg Glu Thr Lys Glu
820 825 830
Asn Ile Ser Glu Glu Leu Gly Leu Thr Phe Asn Gln Val Ser Arg Trp
835 840 845
Phe Ser Ser Thr Arg His Tyr Ser Arg Val Ala Ser Ala Arg Lys Glu
850 855 860
Met His Pro Asp Gly His Thr Ser Glu Asn Asn Asp Thr Thr Thr Val
865 870 875 880
Asp Ser Met Gln Ala Arg Gln Pro Asn Thr Val Val Met Glu Lys Leu
885 890 895
Thr Gly Asn Arg Asn Asp Ile Val Ser Glu Lys Pro Met Val Gln Asn
900 905 910
Asn Leu Asn Gln Gly Val Ala Glu Lys Leu Thr Arg Asp Arg Asn Asp
915 920 925
Ile Val Pro Glu Lys Gln Val Val Gln Ser Asn Leu Asn Gln Cys Asn
930 935 940
Asn Glu Asp Ile Pro Leu Ser Gly Thr Glu Ile Glu Met Glu Ser Tyr
945 950 955 960
Glu Gln Glu Ser Ser Glu Ser Ser Asp Glu Glu Trp Ser Thr Leu Ser
965 970 975
Thr Pro Arg Lys Thr Lys Leu Gln Gly Asn Glu Lys Val Ser Leu Thr
980 985 990
Glu Ser Leu Arg Ser Ala Lys Arg Cys Ser Arg Arg Ala Pro Ala Arg
995 1000 1005
Glu Gln Asn Asn Glu His Thr Gln Ser Glu Gln Leu His Gly Ser
1010 1015 1020
Val Ser Lys Gln Gln Thr Glu Val Leu Arg Ser Asn Val Ser Ser
1025 1030 1035
Gly Asp Ala Ser Lys Cys His Phe Gly Pro Ile Val Thr Gln Lys
1040 1045 1050
Leu Lys Glu His Phe Glu Lys Asp Pro Tyr Pro Cys Arg Ala Thr
1055 1060 1065
Lys Glu Gly Leu Ala Gln Glu Leu Gly Leu Thr Phe Asn Gln Ile
1070 1075 1080
Ser Lys Trp Phe Ser Ala Thr His His Tyr Ser Arg Asp Ala Val
1085 1090 1095

```
Ala Lys  Asn Gln Lys Tyr Pro  Gly Glu Asn Thr Thr  Glu Asn Asn
    1100                 1105                 1110
Ser Ser  Thr Ile Phe Asp Gly  Ile Gln Val Ile Glu  Pro Asn Phe
    1115                 1120                 1125
Gly Leu  Ile Asp Lys Pro Asp  Ala Asp Thr Asn Asp  Met Ile Ser
    1130                 1135                 1140
Glu Lys  Leu Met Val Gln Ile  Asn Leu Asn Glu Gly  Ile Glu Glu
    1145                 1150                 1155
Asp Ile  Pro Pro Ser Gln Tyr  Thr Thr Arg Cys Glu  Glu Lys Leu
    1160                 1165                 1170
Thr Met  Thr Gln Ala Ala Ile  Ser Arg Glu Ala Gly  Pro Pro Gly
    1175                 1180                 1185
Tyr Gly  Pro Gly Glu Asn Phe  Leu Gln Val Ser Ser  Arg Ile Thr
    1190                 1195                 1200
Ser Cys  Glu Gln Ser Val Ile  Met Ala Pro Ser Ala  Ile Ala Arg
    1205                 1210                 1215

Gly Val  Gly Pro Pro Gly Tyr  Thr Pro Gly Glu His  Gln Gly Asn
    1220                 1225                 1230
Gly Ala  Pro Trp Asn Thr Ser  Tyr Glu Arg Arg Pro  Phe Thr Ser
    1235                 1240                 1245
Pro Ala  Thr Ser Ser Arg Glu  Val Gly Pro Pro Gly  Tyr Gly Pro
    1250                 1255                 1260
Glu Glu  Asn Gln Gly Ser Gly  Ile Ser Cys Asn Thr  Ser Arg Glu
    1265                 1270                 1275
Gln Ile  Leu Phe Met Ser Pro  Thr Thr Ile Ser Arg  Glu Leu Gly
    1280                 1285                 1290
Pro Pro  Gly Tyr Leu Pro Arg  Glu Asn Gln Arg Ser  Asp Thr Ser
    1295                 1300                 1305
Trp Asn  Thr Ser Cys Glu Ser  Gly Leu Phe Met Ser  Pro Ala Thr
    1310                 1315                 1320
Thr Ser  Ile Glu His Gly Pro  Pro Gly Tyr Gly Pro  Gly Glu Asn
    1325                 1330                 1335
Gln Glu  Asn Asp Ser Thr Ser  Cys Glu Leu Arg Met  Phe Thr Ser
    1340                 1345                 1350
Pro Thr  Ile Val Ser Arg Glu  Val Gly Pro Pro Gly  Tyr Gln Gln
    1355                 1360                 1365
Gly Val  Phe Met Ser Pro Lys  Thr Ile Ser Arg Glu  Val Cys Pro
    1370                 1375                 1380
Pro Gly  Phe Gly Ser Gly Glu  Asn Gln Gly Asn Asp  Thr Thr Trp
    1385                 1390                 1395
Tyr Lys  Gln Ser Ala Phe Thr  Ser Pro Thr Thr Ile  Ser Arg Glu
    1400                 1405                 1410
Val Gly  Pro Pro Gly Tyr Gly  Gln Glu Asp Gln Gly  Asn Gly Gly
    1415                 1420                 1425
Ser Trp  Asn Thr Ser Cys Glu  Gln Gly Val Phe Thr  Ser Pro Thr
    1430                 1435                 1440
Arg Gly  Ile Ser Arg Glu Val  Gly Pro Pro Gly Tyr  Gly Pro Gly
    1445                 1450                 1455
Glu Ser  Gln Gly Asn Thr Thr  Ser Trp Ile Ser Thr  Cys Gln Gln
    1460                 1465                 1470
Arg Met  Leu Ala Gly Ile Gln  Arg Thr Gly Gly Ser  Arg Ser Met
    1475                 1480                 1485
Asp Leu  Glu Ala Phe Pro Pro  Gly Tyr Gly Pro Gly  Gly Ala Ser
    1490                 1495                 1500
Gly Ser  Val Ile Ser Pro Gln  Ala Arg Ser Ala Glu  Asn Val Glu
    1505                 1510                 1515
Phe Ser  Asp Glu Ala Arg Lys  Lys Ala Ile Gln Arg  Glu Leu Arg
    1520                 1525                 1530
Arg Arg  Gln Lys Phe Arg
    1535
```

<210> 213

<211> 2907
<212> DNA
<213> Vitis vinifera

<400> 213

```
atggatgctt cccctgccca agagagtaat cggactagga aaagttctag tcctaaacag      60
aatattttag aagaggccag aaaactttct gaaagtgtgt gtagcgaatc gtcagagcag     120
aaacgtccct ctgaaaatgg acagcatgag ccagcagaaa tcagccctgt gctatccaat     180
tgtattgtta ctgaacagtc agaactgcct ccagaagatg tgggtgatac aatattagga     240
ttgcctccag cagatgtgac caagaattct cttactgaac atttgggatt gcctccagaa     300
gatgcaatca agaatgatgg aactgaacaa ttggggtttt ttcctgaagt tgtgaccaag     360
agttctatta ttgaaaaatt aggacaatca gagccacctc ctgaaaatgt ggcccggtat     420
tctggtcttg accagtcagg gtcagcaccc aaagatttgg ccaataaaag aactgcaaaa     480
ctagttaaaa gaaaatataa attgagatct tcagtaagcg gttcccgggt tttacgctca     540
aggtcacaag agaaacctaa agcttcacag ccaagtgata attttgtaaa tgccagtgct     600
agcagagaaa ggaaaggaag aaagaagaaa aggatgaata aaacaactgc agatgaattt     660
gcgagaatca ggaaacatct tagatattta ctgaacagaa tgagctatga gcaaaatctg     720
attgatgctt attctgccga aggttggaaa ggacaaagtg tggaaaaatt aaagccagag     780
aaggaacttc aacgtgcctc atctgaaatt tctcgtcgca aactgmaaat acgtgatcta     840
tttcaacatc ttgattcatt atgtgctgaa ggaaggtttc cagaatcttt atttgattct     900
gaagggcaga ttgacagtga ggatatattc tgtgctaaat gtgagtccaa agacatgtct     960
gctgataatg acataatact ctgtgacggt gcttgtgatc gtggatttca ccagttttgt    1020
ctggaaccgc cattgttaaa agaagaaatt cctcctgatg acgagggttg gctgtgccct    1080
gcatgtgatt gcaaagttga ctgcatggac ctgcttaatg actctcaagg aacaaaactt    1140
tctgtaattg atagctggga gaaggttttt cctgaggcag ctgcagctgg gaataaccag    1200
gataacaact ctggattttc atcagatgat tctgaggata atgattatga ccccgattgt    1260
ccagaggttg atgagaaggg tcagggagat aagtcaagtt ctgataaatt tgatgaatct    1320
gatgaatttg acgaatctga tgaatctgat ttcacttctg catctgatga tatggtggtc    1380
tcaccraata atgagcagtg tttggggctt ccttctgatg attcagagga tgatgatttt    1440
gatcctgatg ctccagaaat tgatgaacag gttaatcagg ggagttcaag ttctgatttt    1500
acatctgact ccgaggattt tactgccact ttagatcgca gaaacttctc tgacaacgag    1560
gatggtcttg atgagcaaag aagatttggt aggaagaaga aagatacttt aaaggatgag    1620
ctcttatccg tgttagagtc aaattctggt caagataatg cacctctgtc tgcaaagaga    1680
catgtagaaa ggctggatta caaaaagctg catgatgagg catatggaaa tgtttcttct    1740
gattcaagtg atgatgaaga ctggacagag aatgttatac caagaaagag gaagaatctt    1800
agtggtaatg ttgcctcagt gtcaccaaat ggaaacactt caatcactga gaatggggaca    1860
aataccaagg acataaagca cgacttggaa gcagctggat gcactcccaa gcgaagaact    1920
cgtcagaagc tgaactttga aagcacaaat aattcacttg ctgagtcaca taaaggttct    1980
cgaagtcctg gttctactgg tgaaaaaagt gggcaatcat catataaaaa acttggagaa    2040

gctgtaactg agagactcta caaatccttc caggaaaatc agtaccctga tcgtgctatg    2100
aaagaaaaat tggcagaaga gctgggaata acgagccggc aggttagcaa atggtttgag    2160
aatgcccgtt ggagctttcg ccatcgacca cccaaggaag ccagtgcggg taaaagtgct    2220
gtgaaaaagg atgcatccac gtctcaaaca gatcaaaagc ctgagcaaga agtggttctt    2280
agagaaagtt ctcacaatgg agtgggaaaa aaggagtcgc ccaaagcagg tgcttcgaag    2340
gtagaccgca gcaaggaagc caatgcaggt aaaagtgctg tgaaaaagga tgcatccacg    2400
tctcaaacag atcaaaagcc tgagcaagaa gtggttatta aagaaagttc tcacaatgga    2460
gtgggaaaaa aggagtcgac caaagcaggt gcttcgaagg tagaccgctg cagtggagct    2520
aagaggagaa ggaaattggc aactgatgga agccatagac agaaatcttc aactcccaac    2580
tctacaagac aaaagacaaa gtcaaatcat gaagcatctg aggcaaccaa tggaagcaga    2640
aggcagaatt cttctactcc caagtctaga aggcaaaaga ctaagttagc tggtgaagcg    2700
tctgaagcaa ctggtggaag cagtagacag aattcgtcaa ccccaaattc taaaaggcga    2760
aaaaccaagt cagatcatga agcatctaat cccgtcctta gcggtaagaa gatagcaaag    2820
acggcaaaga gctcatctgg cacaccaaaa acaaaggaaa agctaagtga cagaatccaa    2880
acaagaagta ggaaatctat tgcttga                                        2907
```

<210> 214
<211> 968
<212> PRT
<213> Vitis vinifera

<220>
<221> UNSURE
<222> (276)..(276)
<223> Xaa can be any naturally occurring amino acid

<400> 214

```
Met Asp Ala Ser Pro Ala Gln Glu Ser Asn Arg Thr Arg Lys Ser Ser
1               5                   10                  15
Ser Pro Lys Gln Asn Ile Leu Glu Glu Ala Arg Lys Leu Ser Glu Ser
            20                  25                  30
Val Cys Ser Glu Ser Ser Glu Gln Lys Arg Pro Ser Glu Asn Gly Gln
        35                  40                  45
His Glu Pro Ala Glu Ile Ser Pro Val Leu Ser Asn Cys Ile Val Thr
    50                  55                  60
Glu Gln Ser Glu Leu Pro Pro Glu Asp Val Gly Asp Thr Ile Leu Gly
65                  70                  75                  80
Leu Pro Pro Ala Asp Val Thr Lys Asn Ser Leu Thr Glu His Leu Gly
                85                  90                  95
Leu Pro Pro Glu Asp Ala Ile Lys Asn Asp Gly Thr Glu Gln Leu Gly
            100                 105                 110
Phe Phe Pro Glu Val Val Thr Lys Ser Ser Ile Ile Glu Lys Leu Gly
        115                 120                 125
Gln Ser Glu Pro Pro Pro Glu Asn Val Ala Arg Tyr Ser Gly Leu Asp
    130                 135                 140
Gln Ser Gly Ser Ala Pro Lys Asp Leu Ala Asn Lys Arg Thr Ala Lys
145                 150                 155                 160
Leu Val Lys Arg Lys Tyr Lys Leu Arg Ser Ser Val Ser Gly Ser Arg
                165                 170                 175
Val Leu Arg Ser Arg Ser Gln Glu Lys Pro Lys Ala Ser Gln Pro Ser
            180                 185                 190
Asp Asn Phe Val Asn Ala Ser Ala Ser Arg Glu Arg Lys Gly Arg Lys
        195                 200                 205
Lys Lys Arg Met Asn Lys Thr Thr Ala Asp Glu Phe Ala Arg Ile Arg
    210                 215                 220
Lys His Leu Arg Tyr Leu Leu Asn Arg Met Ser Tyr Glu Gln Asn Leu
225                 230                 235                 240
Ile Asp Ala Tyr Ser Ala Glu Gly Trp Lys Gly Gln Ser Val Glu Lys
                245                 250                 255
Leu Lys Pro Glu Lys Glu Leu Gln Arg Ala Ser Ser Glu Ile Ser Arg
            260                 265                 270
Arg Lys Leu Xaa Ile Arg Asp Leu Phe Gln His Leu Asp Ser Leu Cys
        275                 280                 285
Ala Glu Gly Arg Phe Pro Glu Ser Leu Phe Asp Ser Glu Gly Gln Ile
    290                 295                 300
Asp Ser Glu Asp Ile Phe Cys Ala Lys Cys Glu Ser Lys Asp Met Ser
305                 310                 315                 320
Ala Asp Asn Asp Ile Ile Leu Cys Asp Gly Ala Cys Asp Arg Gly Phe
                325                 330                 335
His Gln Phe Cys Leu Glu Pro Pro Leu Leu Lys Glu Glu Ile Pro Pro
            340                 345                 350
Asp Asp Glu Gly Trp Leu Cys Pro Ala Cys Asp Cys Lys Val Asp Cys
        355                 360                 365
Met Asp Leu Leu Asn Asp Ser Gln Gly Thr Lys Leu Ser Val Ile Asp
    370                 375                 380
Ser Trp Glu Lys Val Phe Pro Glu Ala Ala Ala Ala Gly Asn Asn Gln
385                 390                 395                 400
Asp Asn Asn Ser Gly Phe Ser Ser Asp Asp Ser Glu Asp Asn Asp Tyr
                405                 410                 415
Asp Pro Asp Cys Pro Glu Val Asp Glu Lys Gly Gln Gly Asp Lys Ser
```

```
        420                 425                 430
Ser Ser Asp Lys Phe Asp Glu Ser Asp Glu Phe Asp Glu Ser Asp Glu
        435                 440                 445
Ser Asp Phe Thr Ser Ala Ser Asp Asp Met Val Val Ser Pro Asn Asn
    450                 455                 460
Glu Gln Cys Leu Gly Leu Pro Ser Asp Asp Ser Glu Asp Asp Asp Phe
465                 470                 475                 480
Asp Pro Asp Ala Pro Glu Ile Asp Glu Gln Val Asn Gln Gly Ser Ser
                485                 490                 495
Ser Ser Asp Phe Thr Ser Asp Ser Glu Asp Phe Thr Ala Thr Leu Asp
            500                 505                 510
Arg Arg Asn Phe Ser Asp Asn Glu Asp Gly Leu Asp Glu Gln Arg Arg
        515                 520                 525
Phe Gly Arg Lys Lys Lys Asp Thr Leu Lys Asp Glu Leu Leu Ser Val
    530                 535                 540
Leu Glu Ser Asn Ser Gly Gln Asp Asn Ala Pro Leu Ser Ala Lys Arg
545                 550                 555                 560
His Val Glu Arg Leu Asp Tyr Lys Lys Leu His Asp Glu Ala Tyr Gly
                565                 570                 575
Asn Val Ser Ser Asp Ser Ser Asp Asp Glu Asp Trp Thr Glu Asn Val
            580                 585                 590
Ile Pro Arg Lys Arg Lys Asn Leu Ser Gly Asn Val Ala Ser Val Ser
        595                 600                 605
Pro Asn Gly Asn Thr Ser Ile Thr Glu Asn Gly Thr Asn Thr Lys Asp
    610                 615                 620
Ile Lys His Asp Leu Glu Ala Ala Gly Cys Thr Pro Lys Arg Arg Thr
625                 630                 635                 640
Arg Gln Lys Leu Asn Phe Glu Ser Thr Asn Asn Ser Leu Ala Glu Ser
                645                 650                 655
His Lys Gly Ser Arg Ser Pro Gly Ser Thr Gly Glu Lys Ser Gly Gln
            660                 665                 670
Ser Ser Tyr Lys Lys Leu Gly Glu Ala Val Thr Glu Arg Leu Tyr Lys
        675                 680                 685
Ser Phe Gln Glu Asn Gln Tyr Pro Asp Arg Ala Met Lys Glu Lys Leu
    690                 695                 700
Ala Glu Glu Leu Gly Ile Thr Ser Arg Gln Val Ser Lys Trp Phe Glu
705                 710                 715                 720
Asn Ala Arg Trp Ser Phe Arg His Arg Pro Pro Lys Glu Ala Ser Ala
                725                 730                 735
Gly Lys Ser Ala Val Lys Lys Asp Ala Ser Thr Ser Gln Thr Asp Gln
            740                 745                 750
Lys Pro Glu Gln Glu Val Val Leu Arg Glu Ser Ser His Asn Gly Val
        755                 760                 765
Gly Lys Lys Glu Ser Pro Lys Ala Gly Ala Ser Lys Val Asp Arg Ser
    770                 775                 780
Lys Glu Ala Asn Ala Gly Lys Ser Ala Val Lys Lys Asp Ala Ser Thr
785                 790                 795                 800
Ser Gln Thr Asp Gln Lys Pro Glu Gln Glu Val Val Ile Lys Glu Ser
                805                 810                 815
Ser His Asn Gly Val Gly Lys Lys Glu Ser Thr Lys Ala Gly Ala Ser
            820                 825                 830
Lys Val Asp Arg Cys Ser Gly Ala Lys Arg Arg Arg Lys Leu Ala Thr
        835                 840                 845
Asp Gly Ser His Arg Gln Lys Ser Ser Thr Pro Asn Ser Thr Arg Gln
    850                 855                 860
Lys Thr Lys Ser Asn His Glu Ala Ser Glu Ala Thr Asn Gly Ser Arg
865                 870                 875                 880
Arg Gln Asn Ser Ser Thr Pro Lys Ser Arg Arg Gln Lys Thr Lys Leu
                885                 890                 895
Ala Gly Glu Ala Ser Glu Ala Thr Gly Gly Ser Ser Arg Gln Asn Ser
```

```
            900                 905                 910
Ser Thr Pro Asn Ser Lys Arg Arg Lys Thr Lys Ser Asp His Glu Ala
        915                 920                 925
Ser Asn Pro Val Leu Ser Gly Lys Lys Ile Ala Lys Thr Ala Lys Ser
    930                 935                 940
Ser Ser Gly Thr Pro Lys Thr Lys Glu Lys Leu Ser Asp Arg Ile Gln
945                 950                 955                 960
Thr Arg Ser Arg Lys Ser Ile Ala
                965
```

<210> 215
<211> 2805
<212> DNA
<213> Populus tremuloides

<400> 215

```
atgtctgagg ctgagaacat gggtgtttct ccatcacaag ttagttctca aactaaaagt      60
tactcatgcc cttcacaaac taaactggaa aatactcatg gatttactgc tgaatacaac     120
tgtggtggat attcggaaga gaaacataag cttgagtctg aaatcataca aactgaagcc     180
ggagatagcg gtactgcagt tctccaatct ggtgctgggg aaactgtgga accatctact     240
gaagatgtaa caaacaattc ttttactgat ttggatccac ctcccgaaga tgccaggggt     300
gctacttttg atgaaagttc aagacctata ctaactgcta tagatcagaa actcgagcca     360
ggtgctagaa gtgtgaatac tgcgtgtacg catggtgaat catcaaaagc aactgactct     420
ggtattctac aggatgaacc tggaaatacc aatgctgcat catcaagctg cattgctaat     480
gaaacctcac aagcatcact tgaaaatcta gccaataatt cttgtactga agatgtgggt     540
ccgccgtatg gagatgcaag caagggtaac cagattgata aaagttcata ccctcaacaa     600
actatatctg gacatacact tgagttactt tctgacagag cttgttgtga acgatcagaa     660
gaaagacaga agcctggatc tgaactttca gaaaatgaat caacgggaat tgacactgaa     720
ttatatagtg gcattgctat cgagaattca gagccgctta ctcaactagt gaccaagagt     780
tctcctatca aacatgtagg cttgcttcct ggtgacagca tcatcattcc tgcaaatgaa     840
caaacaagac caactcatga tgatgaggac aaaggcccag atcatgaaca tttggaaaca     900
ccatctagag ttgcgattgg tattactaga cgcggtagac ctagaggtaa gagtgcttca     960
agattgtcaa ggaagatata tatgttaaga tctttgagaa gtagtgatag agttctccgg    1020
tcaagatcac aagagaaacc taaagctcct gaatcaagta ataattcagg taatgttaat    1080
tccactggtg ataaaaaagg gaaaagaagg aaaaagagaa gaggaaagaa tattgtggct    1140
gatgaatatt ccaaaatcag ggcacatctt aggtacttat tgaatcggat gagctatgag    1200
caaagcttga tcactgctta ttctggggaa ggctggaaag gacttagcct agaaaagtta    1260
aagcccgaga aggagttgca gcgagccaca tctgaaatta ccagacgcaa agtgaaaata    1320
aggatttat ttcaacatat tgattctctg tgcagcgaag gaaggttccc atcatcttta    1380
tttgattctg aaggacagat tgacagtgag gatgtattct gtgcaaaatg tgggtccaaa    1440
gatttgaatg ctgataatga tattatacta tgtgatggtg cttgtgacag aggatttcat    1500
cagttctgtt tgataccacc attgttaaga gaagacattc ctcctgatga tgagggctgg    1560
ttatgccctg gatgtgattg caaagttgac tgcattggtt tgcttaacga ttctcaagga    1620
acaaatatat ctatcagtga tagctgggag aaagttttcc ctgaggctgc cgccacagcg    1680
tctggacaaa aactggatca caactttgga ccatcttctg atgattctga tgataatgat    1740
tatgaacctg atggtccaga tattgataag aagagtcagg aggaggaatc gagctctgac    1800
gaatctgact tcacttctgc atctgatgaa ttcaaggctc ccccggatgg caaagagtac    1860
ttggggctct cttctgatga ttcagaggat gatgactatg atcctgatgc tccagttctt    1920
gaggagaagc tgaagcagga aagttcaagt tctgatttta catcagactc tgaggatctt    1980
gctgcaacta ttaatggtga tgggttgtct ttagaagatg aatgccacat gcctattgaa    2040
cctcgtggag tttctaatgg gaggaaatct aaatttgatg gaaagaagat gcagtcttta    2100
aacagcgagc ttttatccat gctagaacca gatctgtgcc aagatgagtc agcaactgtt    2160
tctggaaaaa gaaatgttga cagattggac tacaagaagc tctatgatga aacgtatgga    2220
aacatttcta cttcaagtga tgatgattac actgatactg ttgggccaag gaagaggagg    2280
aaaaatactg gagatgttgc tacagtgaca gcaaacggag atgcctctgt taccgagaat    2340
ggaatgaata gcaagaatat gaaccaggaa ttaaaggaga acaaacgtaa tcctgagaga    2400
ggaacttgcc aaaactcgag ctttcaagaa acaaatgttt ccccggctaa atcatatgtt    2460
ggtgcatctc tatctggttc tagtggtaaa agcgttaggc cctctgctta taaaaaactt    2520
ggagaagctg taactcagag actgtacagt tacttcaggg aaaatcagta tccagaccga    2580
gctgcaaaag caagcttggc agaagagcta ggaattactt ttgagcaggt taacaagtgg    2640
tttgtgaatg cccgttggag cttcaaccat tcatcatcta caggtacaag taaagctgaa    2700

agtgcttctg gaaaaggtag ctgtgatggc caagtgaggg atagtgaatc gaagaaccga    2760
aagagcaata aacaaaaaac taacaccccg aaatctagga gataa                    2805
```

<210> 216
<211> 934
<212> PRT
<213> Populus tremuloides

<400> 216

```
Met Ser Glu Ala Glu Asn Met Gly Val Ser Pro Ser Gln Val Ser Ser
1               5                   10                  15
Gln Thr Lys Ser Tyr Ser Cys Pro Ser Gln Thr Lys Leu Glu Asn Thr
            20                  25                  30
His Gly Phe Thr Ala Glu Tyr Asn Cys Gly Gly Tyr Ser Glu Glu Lys
        35                  40                  45
His Lys Leu Glu Ser Glu Ile Ile Gln Thr Glu Ala Gly Asp Ser Gly
    50                  55                  60
Thr Ala Val Leu Gln Ser Gly Ala Gly Glu Thr Val Glu Pro Ser Thr
65                  70                  75                  80
Glu Asp Val Thr Asn Asn Ser Phe Thr Asp Leu Asp Pro Pro Pro Glu
                85                  90                  95
Asp Ala Arg Gly Ala Thr Phe Asp Glu Ser Ser Arg Pro Ile Leu Thr
            100                 105                 110
Ala Ile Asp Gln Lys Leu Glu Pro Gly Ala Arg Ser Val Asn Thr Ala
        115                 120                 125
Cys Thr His Gly Glu Ser Ser Lys Ala Thr Asp Ser Gly Ile Leu Gln
    130                 135                 140
Asp Glu Pro Gly Asn Thr Asn Ala Ala Ser Ser Ser Cys Ile Ala Asn
145                 150                 155                 160
Glu Thr Ser Gln Ala Ser Leu Glu Asn Leu Ala Asn Asn Ser Cys Thr
                165                 170                 175
Glu Asp Val Gly Pro Pro Tyr Gly Asp Ala Ser Lys Gly Asn Gln Ile
            180                 185                 190
Asp Lys Ser Ser Tyr Pro Gln Gln Thr Ile Ser Gly His Thr Leu Glu
        195                 200                 205
Leu Leu Ser Asp Arg Ala Cys Cys Glu Arg Ser Glu Glu Arg Gln Lys
    210                 215                 220
Pro Gly Ser Glu Leu Ser Glu Asn Glu Ser Thr Gly Ile Asp Thr Glu
225                 230                 235                 240
Leu Tyr Ser Gly Ile Ala Ile Glu Asn Ser Glu Pro Leu Thr Gln Leu
                245                 250                 255
Val Thr Lys Ser Ser Pro Ile Lys His Val Gly Leu Leu Pro Gly Asp
            260                 265                 270
Ser Ile Ile Ile Pro Ala Asn Glu Gln Thr Arg Pro Thr His Asp Asp
        275                 280                 285
Glu Asp Lys Gly Pro Asp His Glu His Leu Glu Thr Pro Ser Arg Val
    290                 295                 300
Ala Ile Gly Ile Thr Arg Arg Gly Arg Pro Arg Gly Lys Ser Ala Ser
305                 310                 315                 320
Arg Leu Ser Arg Lys Ile Tyr Met Leu Arg Ser Leu Arg Ser Ser Asp
                325                 330                 335
Arg Val Leu Arg Ser Arg Ser Gln Glu Lys Pro Lys Ala Pro Glu Ser
            340                 345                 350
Ser Asn Asn Ser Gly Asn Val Asn Ser Thr Gly Asp Lys Lys Gly Lys
        355                 360                 365
Arg Arg Lys Lys Arg Arg Gly Lys Asn Ile Val Ala Asp Glu Tyr Ser
    370                 375                 380
Lys Ile Arg Ala His Leu Arg Tyr Leu Leu Asn Arg Met Ser Tyr Glu
385                 390                 395                 400
Gln Ser Leu Ile Thr Ala Tyr Ser Gly Glu Gly Trp Lys Gly Leu Ser
                405                 410                 415
```

```
Leu Glu Lys Leu Lys Pro Glu Lys Glu Leu Gln Arg Ala Thr Ser Glu
            420                 425                 430
Ile Thr Arg Arg Lys Val Lys Ile Arg Asp Leu Phe Gln His Ile Asp
        435                 440                 445
Ser Leu Cys Ser Glu Gly Arg Phe Pro Ser Ser Leu Phe Asp Ser Glu
    450                 455                 460
Gly Gln Ile Asp Ser Glu Asp Val Phe Cys Ala Lys Cys Gly Ser Lys
465                 470                 475                 480
Asp Leu Asn Ala Asp Asn Asp Ile Ile Leu Cys Asp Gly Ala Cys Asp
                485                 490                 495
Arg Gly Phe His Gln Phe Cys Leu Ile Pro Pro Leu Leu Arg Glu Asp
            500                 505                 510
Ile Pro Pro Asp Asp Glu Gly Trp Leu Cys Pro Gly Cys Asp Cys Lys
        515                 520                 525
Val Asp Cys Ile Gly Leu Leu Asn Asp Ser Gln Gly Thr Asn Ile Ser
    530                 535                 540
Ile Ser Asp Ser Trp Glu Lys Val Phe Pro Glu Ala Ala Ala Thr Ala
545                 550                 555                 560
Ser Gly Gln Lys Leu Asp His Asn Phe Gly Pro Ser Ser Asp Asp Ser
                565                 570                 575
Asp Asp Asn Asp Tyr Glu Pro Asp Gly Pro Asp Ile Asp Lys Lys Ser
            580                 585                 590
Gln Glu Glu Glu Ser Ser Ser Asp Glu Ser Asp Phe Thr Ser Ala Ser
        595                 600                 605
Asp Glu Phe Lys Ala Pro Pro Asp Gly Lys Glu Tyr Leu Gly Leu Ser
    610                 615                 620
Ser Asp Asp Ser Glu Asp Asp Asp Tyr Asp Pro Asp Ala Pro Val Leu
625                 630                 635                 640
Glu Glu Lys Leu Lys Gln Glu Ser Ser Ser Ser Asp Phe Thr Ser Asp
                645                 650                 655
Ser Glu Asp Leu Ala Ala Thr Ile Asn Gly Asp Gly Leu Ser Leu Glu
            660                 665                 670
Asp Glu Cys His Met Pro Ile Glu Pro Arg Gly Val Ser Asn Gly Arg
        675                 680                 685
Lys Ser Lys Phe Asp Gly Lys Lys Met Gln Ser Leu Asn Ser Glu Leu
    690                 695                 700
Leu Ser Met Leu Glu Pro Asp Leu Cys Gln Asp Glu Ser Ala Thr Val
705                 710                 715                 720
Ser Gly Lys Arg Asn Val Asp Arg Leu Asp Tyr Lys Lys Leu Tyr Asp
                725                 730                 735
Glu Thr Tyr Gly Asn Ile Ser Thr Ser Ser Asp Asp Asp Tyr Thr Asp
            740                 745                 750
Thr Val Gly Pro Arg Lys Arg Arg Lys Asn Thr Gly Asp Val Ala Thr
        755                 760                 765
Val Thr Ala Asn Gly Asp Ala Ser Val Thr Glu Asn Gly Met Asn Ser
    770                 775                 780
Lys Asn Met Asn Gln Glu Leu Lys Glu Asn Lys Arg Asn Pro Glu Arg
785                 790                 795                 800
Gly Thr Cys Gln Asn Ser Ser Phe Gln Glu Thr Asn Val Ser Pro Ala
                805                 810                 815
Lys Ser Tyr Val Gly Ala Ser Leu Ser Gly Ser Ser Gly Lys Ser Val
            820                 825                 830
Arg Pro Ser Ala Tyr Lys Lys Leu Gly Glu Ala Val Thr Gln Arg Leu
        835                 840                 845
Tyr Ser Tyr Phe Arg Glu Asn Gln Tyr Pro Asp Arg Ala Ala Lys Ala
    850                 855                 860
Ser Leu Ala Glu Glu Leu Gly Ile Thr Phe Glu Gln Val Asn Lys Trp
865                 870                 875                 880
Phe Val Asn Ala Arg Trp Ser Phe Asn His Ser Ser Ser Thr Gly Thr
                885                 890                 895
Ser Lys Ala Glu Ser Ala Ser Gly Lys Gly Ser Cys Asp Gly Gln Val
```

```
                900                 905                 910
Arg Asp Ser Glu Ser Lys Asn Arg Lys Ser Asn Lys Gln Lys Thr Asn
            915                 920                 925
Thr Pro Lys Ser Arg Arg
        930
```

<210> 217
<211> 2793
<212> DNA
<213> Populus tremuloides

<400> 217

```
atgtccgagg ctgagcacat gggtgtttct ccatcaaaag tttctcatac taaaagttat     60
tcatgccctg cacaaactac actggaaaat acgcatgaac ccagtgctga atacaagttt    120
ggtggatatc cggaagagag acataagctt gagtgtgaaa tcatacaaac tgaagctgga    180
gataacaggg ctgcagttct ccaatcttgt tctggtgaag ttgtgcaacc atctactgac    240
gatttaacaa aaagtcctct tattgatttg gacccacctc ctgacgatgc aaggagtgct    300
ctgtttgata atagtccaag acctatatca accgctatgg atcagaaact tgagccaggt    360
gctacaagtg tgaatactgc ttgtgtgcat agtgaatcat caaaagcaat tgactctagt    420
attctactgg atgaacctag gaatagcaac acagaattgt caagctgcat tgcaaatgaa    480
acctcacaag catcacttga aggtctagcc aatgattctc gtgctgaaga tgcaggactg    540
tcacttgtag aggcaagcaa tagtgacctg attgatgaaa gttcatactc tcaacaaact    600
acatctggac agacacgtga gtttcattct gacagagctt gttgtaaacc attggaagaa    660
agacagaagc ccggctctga acttgcagaa aatgaatcaa tggaaattgg cattggatta    720
cccagtggta ttgctattga gaatttggag ccgcttactg aacttgtgac caagagttgt    780
cctatcaaac atataggctt gcctcctggt gatgacatca gcattcctgc aaatgaacaa    840
ataagaccta ctcatgataa ggagtccaaa taccctgatt gtgaacattt ggaaaaacta    900
tctggaattg tgattggtat tactagtcaa ggtgtaccca gtgttaagag aacttcaaaa    960
ttgtcaggga agaaatatac aagttcttcg agaaaaagtg atagagttct ccggtcaaat   1020
tcacaagaga aacctaaagc tcccgagccg agtaataatt ctactaatgt taattccact   1080
ggtgaggaaa aagggaaaag gaggaaaaag agaagaggga aaagtatagt ggctgatgaa   1140
tattccagaa tcagggcacg tcttaggtac ttgttgaatc ggatgagcta tgagcaaagc   1200
ttgatcactg cttattccgg ggaaggctgg aaaggactta gtctagaaaa gttaaaacct   1260
gagaaggagt tgcaacgggc cacatctgaa atcatcagac gcaaagtgaa aataagggat   1320
ttatttcaac atattgattc actatgcggt gaaggaaggt tcccagcatc tttgtttgat   1380
tctgaaggac aaattgacag tgaggatata ttctgtgcaa aatgtggatc caaagatttg   1440
actgctgata atgatattat actatgtgat ggtgcttgcg accgagggtt tcatcaattc   1500
tgtttggtac caccattgtt aagagaagac attcctcctg gtgatgaggg ctggttatgc   1560
cctggatgtg attgcaaagt tgactgcatt gacttgctta atgactctca aggaacaaat   1620
atatctatca gtgacagatg ggataatgtt ttccccgagg cagctgcagt agcatctgga   1680
caaaaactcg actacaactt tggattgtct tctgatgatt ctgatgataa tgattatgat   1740
cctgatggtc cagatattga cgagaagagt caggaggaat caagctctga cgaatctgac   1800
ttcagttctg cgtctgatga atttgaggct ccacctgatg ataaacagta cttggggctc   1860
ccttctgatg attcagagga tgatgactat gatcctgatg ctccagttct cgaggagaag   1920
ctgaagcagg aaagttccag ttctgatttt acttcggact ctgaggatct tgatgccact   1980
cttaatggtg atgggttgtc tttaggagat gaataccaca tgcctattga acctcatgaa   2040
gattctaatg ggcgaagatc gagatttggt ggaaagaaga atcattcttt aaacagcaag   2100
cttttatcca tgctagaacc agattctcac caagaaaagt ctgcacctgt ttctggaaaa   2160
agaaatattg aaagattgga ctacaagaag ctctatgatg aaacatatgg aaacatttgt   2220
acttcaagtg atgatgattt cactgatact gttgcaccaa ggaagaggag gaaaaacact   2280
ggagatgttg ctatggggat agcaaatgga gatgcttctg ttactgagaa tggattgaat   2340
agcaagaata tgaatcagga attaaagaag aatgaacata cttctgggag aactcaccaa   2400
aactcgagct ttcaagatac aaatgtttcc ccagcaaaaa cacatgttgg cgaatctctc   2460
tctggttcta gcagtaaaag agttaggccc tctgcttata aaaaacttgg agaagctgta   2520

actcagaaac tgtacagttt cttcaaggaa aatcggtatc ctgaccaagc tgcaaaggca   2580
agtttggcag aagagctagg aattactttt gagcaggtta acaagtggtt catgaatgct   2640
cgttggagct tcaaccattc atcacctgaa ggtacaagta aagctgaaag tgcttcagga   2700
aagggtagct gtgatgggca tgtgagggat agtgaatcga agaaccaaaa gagcaataaa   2760
caaaaaacta gtaccccaaa atctaggaga tag                                2793
```

<210> 218
<211> 930
<212> PRT
<213> Populus tremuloides

<400> 218

```
Met Ser Glu Ala Glu His Met Gly Val Ser Pro Ser Lys Val Ser His
1               5                   10                  15
Thr Lys Ser Tyr Ser Cys Pro Ala Gln Thr Thr Leu Glu Asn Thr His
            20                  25                  30
Glu Pro Ser Ala Glu Tyr Lys Phe Gly Gly Tyr Pro Glu Glu Arg His
        35                  40                  45
Lys Leu Glu Cys Glu Ile Ile Gln Thr Glu Ala Gly Asp Asn Arg Ala
    50                  55                  60
Ala Val Leu Gln Ser Cys Ser Gly Glu Val Val Gln Pro Ser Thr Asp
65                  70                  75                  80
Asp Leu Thr Lys Ser Pro Leu Ile Asp Leu Asp Pro Pro Pro Asp Asp
                85                  90                  95
Ala Arg Ser Ala Leu Phe Asp Asn Ser Pro Arg Pro Ile Ser Thr Ala
            100                 105                 110
Met Asp Gln Lys Leu Glu Pro Gly Ala Thr Ser Val Asn Thr Ala Cys
        115                 120                 125
Val His Ser Glu Ser Ser Lys Ala Ile Asp Ser Ser Ile Leu Leu Asp
    130                 135                 140
Glu Pro Arg Asn Ser Asn Thr Glu Leu Ser Ser Cys Ile Ala Asn Glu
145                 150                 155                 160
Thr Ser Gln Ala Ser Leu Glu Gly Leu Ala Asn Asp Ser Arg Ala Glu
                165                 170                 175
Asp Ala Gly Leu Ser Leu Val Glu Ala Ser Asn Ser Asp Leu Ile Asp
            180                 185                 190
Glu Ser Ser Tyr Ser Gln Gln Thr Thr Ser Gly Gln Thr Arg Glu Phe
        195                 200                 205
His Ser Asp Arg Ala Cys Cys Lys Pro Leu Glu Glu Arg Gln Lys Pro
    210                 215                 220
Gly Ser Glu Leu Ala Glu Asn Glu Ser Met Glu Ile Gly Ile Gly Leu
225                 230                 235                 240
Pro Ser Gly Ile Ala Ile Glu Asn Leu Glu Pro Leu Thr Glu Leu Val
                245                 250                 255
Thr Lys Ser Cys Pro Ile Lys His Ile Gly Leu Pro Pro Gly Asp Asp
            260                 265                 270
Ile Ser Ile Pro Ala Asn Glu Gln Ile Arg Pro Thr His Asp Lys Glu
        275                 280                 285
Ser Lys Tyr Pro Asp Cys Glu His Leu Glu Lys Leu Ser Gly Ile Val
    290                 295                 300
Ile Gly Ile Thr Ser Gln Gly Val Pro Ser Val Lys Arg Thr Ser Lys
305                 310                 315                 320
Leu Ser Gly Lys Lys Tyr Thr Ser Ser Ser Arg Lys Ser Asp Arg Val
                325                 330                 335
Leu Arg Ser Asn Ser Gln Glu Lys Pro Lys Ala Pro Glu Pro Ser Asn
            340                 345                 350
Asn Ser Thr Asn Val Asn Ser Thr Gly Glu Glu Lys Gly Lys Arg Arg
        355                 360                 365
Lys Lys Arg Arg Gly Lys Ser Ile Val Ala Asp Glu Tyr Ser Arg Ile
    370                 375                 380
Arg Ala Arg Leu Arg Tyr Leu Leu Asn Arg Met Ser Tyr Glu Gln Ser
385                 390                 395                 400
Leu Ile Thr Ala Tyr Ser Gly Glu Gly Trp Lys Gly Leu Ser Leu Glu
                405                 410                 415
Lys Leu Lys Pro Glu Lys Glu Leu Gln Arg Ala Thr Ser Glu Ile Ile
            420                 425                 430
Arg Arg Lys Val Lys Ile Arg Asp Leu Phe Gln His Ile Asp Ser Leu
```

```
        435                     440                     445
Cys Gly Glu Gly Arg Phe Pro Ala Ser Leu Phe Asp Ser Glu Gly Gln
    450                     455                     460
Ile Asp Ser Glu Asp Ile Phe Cys Ala Lys Cys Gly Ser Lys Asp Leu
465                     470                     475                 480
Thr Ala Asp Asn Asp Ile Ile Leu Cys Asp Gly Ala Cys Asp Arg Gly
                485                     490                     495
Phe His Gln Phe Cys Leu Val Pro Pro Leu Leu Arg Glu Asp Ile Pro

            500                     505                     510
Pro Gly Asp Glu Gly Trp Leu Cys Pro Gly Cys Asp Cys Lys Val Asp
        515                     520                     525
Cys Ile Asp Leu Leu Asn Asp Ser Gln Gly Thr Asn Ile Ser Ile Ser
    530                     535                     540
Asp Arg Trp Asp Asn Val Phe Pro Glu Ala Ala Ala Val Ala Ser Gly
545                     550                     555                 560
Gln Lys Leu Asp Tyr Asn Phe Gly Leu Ser Ser Asp Asp Ser Asp Asp
                565                     570                     575
Asn Asp Tyr Asp Pro Asp Gly Pro Asp Ile Asp Glu Lys Ser Gln Glu
            580                     585                     590
Glu Ser Ser Ser Asp Glu Ser Asp Phe Ser Ser Ala Ser Asp Glu Phe
        595                     600                     605
Glu Ala Pro Pro Asp Asp Lys Gln Tyr Leu Gly Leu Pro Ser Asp Asp
    610                     615                     620
Ser Glu Asp Asp Asp Tyr Asp Pro Asp Ala Pro Val Leu Glu Glu Lys
625                     630                     635                 640
Leu Lys Gln Glu Ser Ser Ser Ser Asp Phe Thr Ser Asp Ser Glu Asp
                645                     650                     655
Leu Asp Ala Thr Leu Asn Gly Asp Gly Leu Ser Leu Gly Asp Glu Tyr
            660                     665                     670
His Met Pro Ile Glu Pro His Glu Asp Ser Asn Gly Arg Arg Ser Arg
        675                     680                     685
Phe Gly Gly Lys Lys Asn His Ser Leu Asn Ser Lys Leu Leu Ser Met
    690                     695                     700
Leu Glu Pro Asp Ser His Gln Glu Lys Ser Ala Pro Val Ser Gly Lys
705                     710                     715                 720
Arg Asn Ile Glu Arg Leu Asp Tyr Lys Lys Leu Tyr Asp Glu Thr Tyr
                725                     730                     735
Gly Asn Ile Cys Thr Ser Ser Asp Asp Asp Phe Thr Asp Thr Val Ala
            740                     745                     750
Pro Arg Lys Arg Arg Lys Asn Thr Gly Asp Val Ala Met Gly Ile Ala
        755                     760                     765
Asn Gly Asp Ala Ser Val Thr Glu Asn Gly Leu Asn Ser Lys Asn Met
    770                     775                     780
Asn Gln Glu Leu Lys Lys Asn Glu His Thr Ser Gly Arg Thr His Gln
785                     790                     795                 800
Asn Ser Ser Phe Gln Asp Thr Asn Val Ser Pro Ala Lys Thr His Val
                805                     810                     815
Gly Glu Ser Leu Ser Gly Ser Ser Ser Lys Arg Val Arg Pro Ser Ala
            820                     825                     830
Tyr Lys Lys Leu Gly Glu Ala Val Thr Gln Lys Leu Tyr Ser Phe Phe
        835                     840                     845
Lys Glu Asn Arg Tyr Pro Asp Gln Ala Ala Lys Ala Ser Leu Ala Glu
    850                     855                     860
Glu Leu Gly Ile Thr Phe Glu Gln Val Asn Lys Trp Phe Met Asn Ala
865                     870                     875                 880
Arg Trp Ser Phe Asn His Ser Ser Pro Glu Gly Thr Ser Lys Ala Glu
                885                     890                     895
Ser Ala Ser Gly Lys Gly Ser Cys Asp Gly His Val Arg Asp Ser Glu
            900                     905                     910
Ser Lys Asn Gln Lys Ser Asn Lys Gln Lys Thr Ser Thr Pro Lys Ser
```

```
                    915                 920                 925
        Arg Arg
            930
```

<210> 219
<211> 2184
<212> DNA
<213> Populus tremuloides

<400> 219

```
atgggtgatt ctggaaagaa atcaaagcag caagacttgc acgaatcttc accatctgac     60
acagtgaatg ggtcgttgct gattaaatca ttgaagataa agaagggtgg caaattatct    120
catagaaaaa gcgaaaaacc taaaactaaa cctcatctga aaactatcat taattcatct    180
gtatcaaaga aaaaggttac tcctaagaag ggcatcagga atggctctac cagtagaaga    240
ttgattcaca ggaaaattct gcataaagca cttgataaga aggcctcaag aaatggggct    300
tcctcagagc tccaaggcaa acagttgtca actattgatt ctgaggggaa tggaaaaaat    360
gccgatgaag gtgcaattaa aaaagtcaag aagaggaagc ctaagaaaag gcaaaaggac    420
aaggtaaagc tagatgaacc accacgcttg cagaggagag caaggtactt gatgattaaa    480
atgaagctgg agcagaatct tatagatgct tactctggag aaggttggaa aggtaaaagt    540
cgagagaaga ttcggccaga aaaggaacta ctgagagcca ggaaacagat tttgaaatgt    600
aaacttggat tacgtgaaat aattcggcag gtggattctc taagtacagt aggatgtatt    660
gaagatgctg ttatggctcc agatggatct gtttcccatg aacatatatt ctgtgcgaag    720
tgcaaattga atgaagtttc ccaagataat gatattgtac tctgtgatgg gacatgcaac    780
tgtgccttcc accaaaaatg ccttgatcct cctttggata ctgaaaatat acctccagga    840
gatcagggat ggttttgcaa gttctgtgac tgtaggatgg aaattataga agccatgaat    900
gctcatttgg ggacccactt ctcagaggac agcggttggc aggatatttt caaagaagaa    960
gcagcagttc cagatggtgg aaatatgcta ttaaatccag aagaagaatg gccttctgat   1020
gattctgaag atgatgatta tgatccagag aggagagaga acgtcatgag tggagcaggt   1080
actgatgatg atgcatctga tgacaccagc aattcaaccc gcttgagttg gtcttcagat   1140
ggtgaggttt tttcaggatc caggaggtgg gaggtggacg gcttggactt cagaaacaat   1200
tctatttata gcagtttaga ttctgatgaa accagtgatg gggaaattat ttgtggccgt   1260
aggcagagaa gagcggttga ttataagaag ttatacaatg agatgtttgg aaaggacgct   1320
cctgcacatg agcaacccag cgaagatgaa gattggggtc ctagcaaaag aaagcgacga   1380
gagaaggagt cagatgcagc cagcacccta atgactctat atgaaagtaa aagaagatgt   1440
aaaaatgatg caaccattga aggcatgatg aagcttccac gagatcctca aattcgaagg   1500
ccaattttca ggctcccccc tgatgctgtt gagaaacttc gccaagtatt tgcagagaat   1560
gaacttccat ctagaactgt caaggagaat ctatcaaaag aattgggcct tgaacctggg   1620
aaggttagca aatggttcaa gaattcccgt tatttagctc tgaagtctag aaaggtagag   1680
aagggagaac aagttcatta ttctagttcc aaagtctctg ctgaacccac tttaaatgtc   1740
atgaaggaca aaactgctga tctttcactg ttaaaggata gccaggcaga aactggggtg   1800
tgtaccccag agaatttgaa aaggatcttg cagaggaaga agccgaggtc aataagtaaa   1860
agtttaaaga aaaatgaaca gaaaagaggt tccttcgaat cacctactaa aagcaatgag   1920
atgaatgttg agcacaatga tgatctgagc ttgaagaagc tgttaaaagc aaaaacaaag   1980
ggagtaaaga aaaagggtaa tcgtatttca gcagctgctg aatctgatat ggagaaactt   2040
tgtagagcaa agacgagagt agagaatctg aagcagaaac tggtgaaact acaaactggc   2100
aaagctagga agtcttccaa aatccgtccg ctagacgaat ctgttgtcta cgttcctatt   2160
gctgagctaa gagaaaagaa atga                                          2184
```

<210> 220
<211> 727
<212> PRT
<213> Populus tremuloides

<400> 220

```
Met Gly Asp Ser Gly Lys Lys Ser Lys Gln Gln Asp Leu His Glu Ser
1               5                   10                  15
Ser Pro Ser Asp Thr Val Asn Gly Ser Leu Leu Ile Lys Ser Leu Lys
            20                  25                  30
Ile Lys Lys Gly Gly Lys Leu Ser His Arg Lys Ser Glu Lys Pro Lys
        35                  40                  45
Thr Lys Pro His Leu Lys Thr Ile Ile Asn Ser Ser Val Ser Lys Lys
```

50 55 60
Lys Val Thr Pro Lys Lys Gly Ile Arg Asn Gly Ser Thr Ser Arg Arg
65 70 75 80
Leu Ile His Arg Lys Ile Leu His Lys Ala Leu Asp Lys Lys Ala Ser
85 90 95
Arg Asn Gly Ala Ser Ser Glu Leu Gln Gly Lys Gln Leu Ser Thr Ile
100 105 110
Asp Ser Glu Gly Asn Gly Lys Asn Ala Asp Glu Gly Ala Ile Lys Lys
115 120 125
Val Lys Lys Arg Lys Pro Lys Lys Arg Gln Lys Asp Lys Val Lys Leu
130 135 140
Asp Glu Pro Pro Arg Leu Gln Arg Arg Ala Arg Tyr Leu Met Ile Lys
145 150 155 160
Met Lys Leu Glu Gln Asn Leu Ile Asp Ala Tyr Ser Gly Glu Gly Trp
165 170 175
Lys Gly Lys Ser Arg Glu Lys Ile Arg Pro Glu Lys Glu Leu Leu Arg
180 185 190
Ala Arg Lys Gln Ile Leu Lys Cys Lys Leu Gly Leu Arg Glu Ile Ile
195 200 205
Arg Gln Val Asp Ser Leu Ser Thr Val Gly Cys Ile Glu Asp Ala Val
210 215 220
Met Ala Pro Asp Gly Ser Val Ser His Glu His Ile Phe Cys Ala Lys
225 230 235 240
Cys Lys Leu Asn Glu Val Ser Gln Asp Asn Asp Ile Val Leu Cys Asp
245 250 255
Gly Thr Cys Asn Cys Ala Phe His Gln Lys Cys Leu Asp Pro Pro Leu
260 265 270
Asp Thr Glu Asn Ile Pro Pro Gly Asp Gln Gly Trp Phe Cys Lys Phe
275 280 285
Cys Asp Cys Arg Met Glu Ile Ile Glu Ala Met Asn Ala His Leu Gly
290 295 300
Thr His Phe Ser Glu Asp Ser Gly Trp Gln Asp Ile Phe Lys Glu Glu
305 310 315 320
Ala Ala Val Pro Asp Gly Gly Asn Met Leu Leu Asn Pro Glu Glu Glu
325 330 335
Trp Pro Ser Asp Asp Ser Glu Asp Asp Asp Tyr Asp Pro Glu Arg Arg
340 345 350
Glu Asn Val Met Ser Gly Ala Gly Thr Asp Asp Asp Ala Ser Asp Asp
355 360 365
Thr Ser Asn Ser Thr Arg Leu Ser Trp Ser Ser Asp Gly Glu Val Phe
370 375 380
Ser Gly Ser Arg Arg Trp Glu Val Asp Gly Leu Asp Phe Arg Asn Asn
385 390 395 400
Ser Ile Tyr Ser Ser Leu Asp Ser Asp Glu Thr Ser Asp Gly Glu Ile
405 410 415
Ile Cys Gly Arg Arg Gln Arg Arg Ala Val Asp Tyr Lys Lys Leu Tyr
420 425 430
Asn Glu Met Phe Gly Lys Asp Ala Pro Ala His Glu Gln Pro Ser Glu
435 440 445
Asp Glu Asp Trp Gly Pro Ser Lys Arg Lys Arg Arg Glu Lys Glu Ser
450 455 460
Asp Ala Ala Ser Thr Leu Met Thr Leu Tyr Glu Ser Lys Arg Arg Cys
465 470 475 480
Lys Asn Asp Ala Thr Ile Glu Gly Met Met Lys Leu Pro Arg Asp Pro
485 490 495
Gln Ile Arg Arg Pro Ile Phe Arg Leu Pro Pro Asp Ala Val Glu Lys
500 505 510
Leu Arg Gln Val Phe Ala Glu Asn Glu Leu Pro Ser Arg Thr Val Lys
515 520 525
Glu Asn Leu Ser Lys Glu Leu Gly Leu Glu Pro Gly Lys Val Ser Lys
530 535 540

```
Trp Phe Lys Asn Ser Arg Tyr Leu Ala Leu Lys Ser Arg Lys Val Glu
545                 550                 555                 560
Lys Gly Glu Gln Val His Tyr Ser Ser Ser Lys Val Ser Ala Glu Pro
                565                 570                 575
Thr Leu Asn Val Met Lys Asp Lys Thr Ala Asp Leu Ser Leu Leu Lys
            580                 585                 590
Asp Ser Gln Ala Glu Thr Gly Val Cys Thr Pro Glu Asn Leu Lys Arg
        595                 600                 605
Ile Leu Gln Arg Lys Lys Pro Arg Ser Ile Ser Lys Ser Leu Lys Lys
    610                 615                 620
Asn Glu Gln Lys Arg Gly Ser Phe Glu Ser Pro Thr Lys Ser Asn Glu
625                 630                 635                 640
Met Asn Val Glu His Asn Asp Asp Leu Ser Leu Lys Lys Leu Leu Lys
                645                 650                 655
Ala Lys Thr Lys Gly Val Lys Lys Lys Gly Asn Arg Ile Ser Ala Ala
            660                 665                 670
Ala Glu Ser Asp Met Glu Lys Leu Cys Arg Ala Lys Thr Arg Val Glu
        675                 680                 685
Asn Leu Lys Gln Lys Leu Val Lys Leu Gln Thr Gly Lys Ala Arg Lys
    690                 695                 700
Ser Ser Lys Ile Arg Pro Leu Asp Glu Ser Val Val Tyr Val Pro Ile
705                 710                 715                 720
Ala Glu Leu Arg Glu Lys Lys
                725
```

<210> 221
<211> 2460
<212> DNA
<213> Ostreococcus tauri

<220>
<221> misc feature
<222> (178)..(178)
<223> n is a, c, g, or t

<220>
<221> misc_feature
<222> (214)..(214)
<223> n is a, c, g, or t

<220>
<221> misc_feature
<222> (1375)..(1375)
<223> n is a, c, g, or t

<220>
<221> misc_feature
<222> (1381)..(1381)
<223> n is a, c, g, or t

<220>
<221> misc feature
<222> (1648)..(1648)
<223> n is a, c, g, or t

<220>
<221> misc feature
<222> (1684)..(1684)
<223> n is a, c, g, or t

<400> 221

```
atgaatgagg tgcgcgacgc ggaggaacga tgtaaacgtc gtgtgcgagc ggtcgcggac      60
gcggtgcgga agggaggcgt ggtgtgggcg atcgtggggt ggaaagatag tcaatgcgcg     120
cgcgcggcgt ccgacgcgcg cgttgttgga gattgggcgc gcgcgatgtg tgtgaganga     180
ctgacgatgc atcgatcggt ggttgatgat acgnagtttg cgctcgcgcg atcgcggata     240
cagtcgcagt tcgcgacgat tcgaagacat caggcgttgg tggaagcgta cgcgagcgac     300
ggctggcgcg gacaggcggc tcaaaagccg aaaccagtca gggagattga aaaggcgcgc     360
gagaagatat tcgagggtaa attgaagata cgggagtatt ttaaggtttt ggagttcgat     420
gagcgcgagc gcgagatcac gacggtggcg gacgagtttg gggagtgcga cgccgcggat     480
atttttgta gcaagtgcac cctcgcagac gacaggcatg acgacgatat tttgctctgc     540
gatggcttct gcgatcgtgc gtatcatcaa tcatgcgtgg ctccaccggt tttggcggaa     600
gacattccgc ccgaggacga aggttggctc tgtccgagat gcgacgcgcg agtggacgtc     660
atttacgtct tgaacgacga gtacgatcaa aatttgggcc agaggtgtgt ttcagcggat     720
attttcgttg cagaggcgga catgcgagat aaagggatcg ttccaggaac ggcgcagttc     780
aaacacgcgc acgaagagga ttggccgagc gatgagagcg atgacgagga cttcgatcag     840
ggcggacaca gcgacgatgg gcgagacgac gagcacgagg cgctgagcgg aagcgcacag     900
tcatcgagcg atgaatcgag cagcgaatca gagtctgatc tcatcatcga gggcccacga     960
aggcgcacga aggttgatta cgtcgccctg aacaacgcca tgttcggcga cagcgaggcg    1020
tacgagggcg aagctgagga gctcggttgg aaacgcagca acaaaaccaa ggcggagatg    1080
ctcgcggcgc tcaagggtga gagcgcgaac gggataacga acggtgccac gaagaaggat    1140
ccaaaatcaa aacgctcgaa gcgagagcgc gtggatgaga aatctcccgc accgcgcgtc    1200
aggttcactc ccgatcaaaa acttgagctc gagcgtgtct tctccgagtg cgcgtcgctc    1260
gacattgacg cgcgcgacgc cctcgcgaag cgactcggga tacccggcgg cgcaaattcg    1320
attaaaatat ggttcatgaa tcgccgtcgt agggctaaga ttttgggtga gaggnaatac    1380
ngacagcacg ataatccacg tcccgctcgc tcgttcgacg cctcgtcaca aacaacctct    1440
gaaaaacctt caagtcacct cagaagctca cgcgcgtctc aaacatgtcg atcgccccta    1500
aacgcgcccc caaactcata tacttctccg cgtggttctg cccgttcgcc catcgagcga    1560
cgctcgcgtt ggagcattac gcgcacggcg gagagatttc ctacgagtgg aaggaatcgc    1620
tcggtgaggt cacgcttcgt tgacattnga cgctcgaatg cgacgaaact tgagcgcctc    1680
gacngaccgg agacgaatcg ccgcgtagga tgggaaaaac gcaagtcaac ctcgagtgag    1740
gtgaagacga aacacgagaa ttggtaccat tacaagtccc cagagctact gagacacaac    1800
ccgctggcca tggtgccgac actcgtgacg ccggcgacgt atgaggataa agatggagat    1860
ccatcgcgga acgtggtgac ggagagcttg gtgtgcgtac agtttatcga tgaactcgtt    1920
cggggaaacg cgtacgccgg ggagacggcg gcgatcatgc cgagcgaccc gtacgcgcgc    1980
gcgaaggcga gagtggacgc cgactgggtg aacaaaaacg tatgctcgag gtattatcac    2040
gtcctggtgc gacaagaggc gagcgagcag agagaagcgt ttgaaaagct cgtggagggg    2100
ctggagacat tcgcggcgtg gtgcggcagc ggagaaggga agttctatgg tggacaaacg    2160
acgcccggat tggtggacta cgcgctcttt ccatgggcgt ggcgcctccc ggtgttcgag    2220
cactaccgcg ggatggattt caagattcct cgcaccgcgg cactgaaatc gtatcacgat    2280
tggatggaag ccatgctcgc gcgagagcaa gttcgcaaga cgcttccacc gtgggacgat    2340
tacttggaac acattggacg atacgccgac gggagcgcgc gatcgaaagt cgccaacgca    2400
gtccgcagcg gccgtgcggc gcacgattac gacgacaagg aggacaacac cgatagttga    2460
```

<210> 222
<211> 819
<212> PRT
<213> Ostreococcus tauri

<220>
<221> UNSURE
<222> (60)..(60)
<223> Xaa can be any naturally occurring amino acid

<220>
<221> UNSURE
<222> (72)..(72)
<223> Xaa can be any naturally occurring amino acid

<220>
<221> UNSURE

<222> (459)..(459)
<223> Xaa can be any naturally occurring amino acid

<220>
<221> UNSURE
<222> (461)..(461)
<223> Xaa can be any naturally occurring amino acid

<220>
<221> UNSURE
<222> (550)..(550)
<223> Xaa can be any naturally occurring amino acid

<220>
<221> UNSURE
<222> (562)..(562)
<223> Xaa can be any naturally occurring amino acid

<400> 222

```
Met Asn Glu Val Arg Asp Ala Glu Glu Arg Cys Lys Arg Arg Val Arg
1               5                   10                  15
Ala Val Ala Asp Ala Val Arg Lys Gly Gly Val Val Trp Ala Ile Val
            20                  25                  30
Gly Trp Lys Asp Ser Gln Cys Ala Arg Ala Ala Ser Asp Ala Arg Val
        35                  40                  45
Val Gly Asp Trp Ala Arg Ala Met Cys Val Arg Xaa Leu Thr Met His
    50                  55                  60
Arg Ser Val Val Asp Asp Thr Xaa Phe Ala Leu Ala Arg Ser Arg Ile
65                  70                  75                  80
Gln Ser Gln Phe Ala Thr Ile Arg Arg His Gln Ala Leu Val Glu Ala
                85                  90                  95
Tyr Ala Ser Asp Gly Trp Arg Gly Gln Ala Ala Gln Lys Pro Lys Pro
            100                 105                 110
Val Arg Glu Ile Glu Lys Ala Arg Glu Lys Ile Phe Glu Gly Lys Leu
        115                 120                 125
Lys Ile Arg Glu Tyr Phe Lys Val Leu Glu Phe Asp Glu Arg Glu Arg
    130                 135                 140
Glu Ile Thr Thr Val Ala Asp Glu Phe Gly Glu Cys Asp Ala Ala Asp
145                 150                 155                 160
Ile Phe Cys Ser Lys Cys Thr Leu Ala Asp Asp Arg His Asp Asp Asp
                165                 170                 175
Ile Leu Leu Cys Asp Gly Phe Cys Asp Arg Ala Tyr His Gln Ser Cys
            180                 185                 190
Val Ala Pro Pro Val Leu Ala Glu Asp Ile Pro Pro Glu Asp Glu Gly
        195                 200                 205
Trp Leu Cys Pro Arg Cys Asp Ala Arg Val Asp Val Ile Tyr Val Leu
    210                 215                 220
Asn Asp Glu Tyr Asp Gln Asn Leu Gly Gln Arg Cys Val Ser Ala Asp
225                 230                 235                 240
Ile Phe Val Ala Glu Ala Asp Met Arg Asp Lys Gly Ile Val Pro Gly
                245                 250                 255
Thr Ala Gln Phe Lys His Ala His Glu Glu Asp Trp Pro Ser Asp Glu
            260                 265                 270
Ser Asp Asp Glu Asp Phe Asp Gln Gly Gly His Ser Asp Asp Gly Arg
        275                 280                 285
Asp Asp Glu His Glu Ala Leu Ser Gly Ser Ala Gln Ser Ser Ser Asp
    290                 295                 300
Glu Ser Ser Ser Glu Ser Glu Ser Asp Leu Ile Ile Glu Gly Pro Arg
305                 310                 315                 320
Arg Arg Thr Lys Val Asp Tyr Val Ala Leu Asn Asn Ala Met Phe Gly
                325                 330                 335
Asp Ser Glu Ala Tyr Glu Gly Glu Ala Glu Glu Leu Gly Trp Lys Arg
            340                 345                 350
```

```
Ser Asn Lys Thr Lys Ala Glu Met Leu Ala Ala Leu Lys Gly Glu Ser
        355                 360                 365

Ala Asn Gly Ile Thr Asn Gly Ala Thr Lys Lys Asp Pro Lys Ser Lys
    370                 375                 380
Arg Ser Lys Arg Glu Arg Val Asp Glu Lys Ser Pro Ala Pro Arg Val
385                 390                 395                 400
Arg Phe Thr Pro Asp Gln Lys Leu Glu Leu Glu Arg Val Phe Ser Glu
                405                 410                 415
Cys Ala Ser Leu Asp Ile Asp Ala Arg Asp Ala Leu Ala Lys Arg Leu
            420                 425                 430
Gly Ile Pro Gly Gly Ala Asn Ser Ile Lys Ile Trp Phe Met Asn Arg
        435                 440                 445
Arg Arg Arg Ala Lys Ile Leu Gly Glu Arg Xaa Tyr Xaa Gln His Asp
    450                 455                 460
Asn Pro Arg Pro Ala Arg Ser Phe Asp Ala Ser Ser Gln Thr Thr Ser
465                 470                 475                 480
Glu Lys Pro Ser Ser His Leu Arg Ser Ser Arg Ala Ser Gln Thr Cys
                485                 490                 495
Arg Ser Pro Leu Asn Ala Pro Pro Asn Ser Tyr Thr Ser Pro Arg Gly
            500                 505                 510
Ser Ala Arg Ser Pro Ile Glu Arg Arg Ser Arg Trp Ser Ile Thr Arg
        515                 520                 525
Thr Ala Glu Arg Phe Pro Thr Ser Gly Arg Asn Arg Ser Val Arg Ser
    530                 535                 540
Arg Phe Val Asp Ile Xaa Arg Ser Asn Ala Thr Lys Leu Glu Arg Leu
545                 550                 555                 560
Asp Xaa Pro Glu Thr Asn Arg Arg Val Gly Trp Glu Lys Arg Lys Ser
                565                 570                 575
Thr Ser Ser Glu Val Lys Thr Lys His Glu Asn Trp Tyr His Tyr Lys
            580                 585                 590
Ser Pro Glu Leu Leu Arg His Asn Pro Leu Gly Met Val Pro Thr Leu
        595                 600                 605
Val Thr Pro Ala Thr Tyr Glu Asp Lys Asp Gly Asp Pro Ser Arg Asn
    610                 615                 620
Val Val Thr Glu Ser Leu Val Cys Val Gln Phe Ile Asp Glu Leu Val
625                 630                 635                 640
Arg Gly Asn Ala Tyr Ala Gly Glu Thr Ala Ala Ile Met Pro Ser Asp
                645                 650                 655
Pro Tyr Ala Arg Ala Lys Ala Arg Val Asp Ala Asp Trp Val Asn Lys
            660                 665                 670
Asn Val Cys Ser Arg Tyr Tyr His Val Leu Val Arg Gln Glu Ala Ser
        675                 680                 685
Glu Gln Arg Glu Ala Phe Glu Lys Leu Val Glu Gly Leu Glu Thr Phe
    690                 695                 700
Ala Ala Trp Cys Gly Ser Gly Glu Gly Lys Phe Tyr Gly Gly Gln Thr
705                 710                 715                 720
Thr Pro Gly Leu Val Asp Tyr Ala Leu Phe Pro Trp Ala Trp Arg Leu
                725                 730                 735
Pro Val Phe Glu His Tyr Arg Gly Met Asp Phe Lys Ile Pro Arg Thr
            740                 745                 750
Ala Ala Leu Lys Ser Tyr His Asp Trp Met Glu Ala Met Leu Ala Arg
        755                 760                 765
Glu Gln Val Arg Lys Thr Leu Pro Pro Trp Asp Asp Tyr Leu Glu His
    770                 775                 780
Ile Gly Arg Tyr Ala Asp Gly Ser Ala Arg Ser Lys Val Ala Asn Ala
785                 790                 795                 800
Val Arg Ser Gly Arg Ala Ala His Asp Tyr Asp Asp Lys Glu Asp Asn
                805                 810                 815
Thr Asp Ser
```

<210> 223
<211> 2076

<212> DNA
<213> Aquilegia formosa x Aquilegia pubescens

<220>
<221> misc_feature
<222> (692)..(713)
<223> n is a, c, g, or t

<220>
<221> misc_feature
<222> (1459)..(1462)
<223> n is a, c, g, or t

<400> 223

```
atgcaagatg gtgggaacct tggttttcca aagaaacgtg ttggatccca aataaatacc     60
tcatttcaga agaagaatag ttgcaaaaat ccgcatatta ggagatggaa accaaagcca    120
agatctcatg tcaagacaat tgcttcgttc ttgtcgaaga gaaagacatc tgagctctca    180
agcaacagag ccagaaataa atgttccaat ggaaaacctt ttagaagaag agcgtcacag    240
aaacccaatg ataccagtcc ttctgatagg ccactttcat caatgcttaa agatttcaaa    300
tcgttaagca ataaatgtca gggacgtgaa aagacaactg acaaggatct tggaagtcaa    360
aaggctcaca gaaggagaag gagcaagaag aggaagaatg aggagttaga tgaagccgct    420
cgagtccaaa gaagaacaag gtacctcctg attaaaatga agctggagca gaaccttatt    480
gaggcttact ctggggaggg ttggaaaggc cagagtcgtg agaaaattaa gccagaaaag    540
gaactgcaaa gagccaagaa gcagatattg aagtgcaaac ttggaattcg tgatgctatt    600
cgtcagctgg aatctcttag ttctgaggga agcattgaag attctgtgat aggttcagat    660
ggatctgttt tccatgaaca tattttctgt tnnnnnnnnn nnnnnnnnnn nnnagcattt    720
ccagataatg acattatact gtgtgatgga gcatgcaatt gtgctttcca ccagaaatgc    780
ctggagcctc cattagccac tgaaaatatt cctcctggtg atcaagggtg gtattgcaaa    840
ttttgtgagt gcaagatggc aatacttgat gcaatcaatg cacatcttgg gactcagttt    900
tctctagaca gtaaatggca agatattttc atggaagcag ctaatgcacg cgataatgag    960
gatatctcaa ttcatcctga tggtgactgg ccatctgatg actctgaaga tgttgattat   1020
gacccagaaa aatatgaaat tgattgcagc tatagcagaa tgggctcgga gcacaacgtg   1080
tctgacgatg caagtagttc cggcaccttg tattggactt ccgaagaaga tgatggtttg   1140
cattctgagc gattcagaac tgatggtagt ctggaggggg gattttatgg ggataaaact   1200
aaaatccaga gttctgatag catagattcc aatgacagta ctgatggtat ggcatttcat   1260
cgtaggcagc gaagagatgt tgattataag aaactgcatg atgaaatgtt tggtaaagat   1320
atgcctgaaa gtgaagaaat cagtgaagat gaagattggg gacctagaag aagaaagcgc   1380
agaagaaatg agtctaatgc agccactact ttagtatctt cgtgtggtac tgagaatgaa   1440
ttttcgaatg tggcacttnn nntaccatca agagtggtca gggagagtct ctcgaagcaa   1500
ttgggtattg caattgagaa gatcaacaag tggttcaaga atgctcggta tacagcttta   1560
aaaattagaa aggcagaaaa gactgaacaa tcccctggag gtggcaagat gcaaagtgct   1620
gacgtattaa catcaaagga aaaccattat ttggtctcaa caggaacagc agtccaaaga   1680
tccaaagttt cgagtgtcca tagaagtaaa aaccgtaaat tgacaaccac accctcaagg   1740
gaagtacaag agaaagtgtc tgctgttgca ccatccgcta ctgcaaatga ggtgagtgta   1800
cagttaagaa aagcagtgag cgtagagaag gaaatgggca gtgtaaaaag aaaatctgca   1860
caagtaaaga ggatcaagcg cacatgctcc actaaggagc agcagctata catgattgag   1920
ttggagagaa tttgccgtct tgaggagaag cttgaaaaga tgaagaaggt attgcttagt   1980
atagatgaca atcatagtaa tgtatcagat ggaccccata tgaacgaaca gtctgccgta   2040
tacattccag ttgcagagct aagagaaaag atgtga                             2076
```

<210> 224
<211> 691
<212> PRT
<213> Aquilegia formosa x Aquilegia pubescens

<220>
<221> UNSURE
<222> (231)..(238)
<223> Xaa can be any naturally occurring amino acid

<220>
<221> UNSURE
<222> (487)..(488)
<223> Xaa can be any naturally occurring amino acid

<400> 224

```
Met Gln Asp Gly Gly Asn Leu Gly Phe Pro Lys Lys Arg Val Gly Ser
1               5                   10                  15
Gln Ile Asn Thr Ser Phe Gln Lys Lys Asn Ser Cys Lys Asn Pro His
            20                  25                  30
Ile Arg Arg Trp Lys Pro Lys Pro Arg Ser His Val Lys Thr Ile Ala
        35                  40                  45
Ser Phe Leu Ser Lys Arg Lys Thr Ser Glu Leu Ser Ser Asn Arg Ala
    50                  55                  60
Arg Asn Lys Cys Ser Asn Gly Lys Pro Phe Arg Arg Arg Ala Ser Gln
65                  70                  75                  80
Lys Pro Asn Asp Thr Ser Pro Ser Asp Arg Pro Leu Ser Ser Met Leu
                85                  90                  95
Lys Asp Phe Lys Ser Leu Ser Asn Lys Cys Gln Gly Arg Glu Lys Thr
            100                 105                 110
Thr Asp Lys Asp Leu Gly Ser Gln Lys Ala His Arg Arg Arg Arg Ser
        115                 120                 125
Lys Lys Arg Lys Asn Glu Glu Leu Asp Glu Ala Ala Arg Val Gln Arg
    130                 135                 140
Arg Thr Arg Tyr Leu Leu Ile Lys Met Lys Leu Glu Gln Asn Leu Ile
145                 150                 155                 160
Glu Ala Tyr Ser Gly Glu Gly Trp Lys Gly Gln Ser Arg Glu Lys Ile
                165                 170                 175
Lys Pro Glu Lys Glu Leu Gln Arg Ala Lys Lys Gln Ile Leu Lys Cys
            180                 185                 190
Lys Leu Gly Ile Arg Asp Ala Ile Arg Gln Leu Glu Ser Leu Ser Ser
        195                 200                 205
Glu Gly Ser Ile Glu Asp Ser Val Ile Gly Ser Asp Gly Ser Val Phe
    210                 215                 220
His Glu His Ile Phe Cys Xaa Xaa Xaa Xaa Xaa Xaa Xaa Xaa Ala Phe
225                 230                 235                 240
Pro Asp Asn Asp Ile Ile Leu Cys Asp Gly Ala Cys Asn Cys Ala Phe
                245                 250                 255
His Gln Lys Cys Leu Glu Pro Pro Leu Ala Thr Glu Asn Ile Pro Pro
            260                 265                 270
Gly Asp Gln Gly Trp Tyr Cys Lys Phe Cys Glu Cys Lys Met Ala Ile
        275                 280                 285
Leu Asp Ala Ile Asn Ala His Leu Gly Thr Gln Phe Ser Leu Asp Ser
    290                 295                 300
Lys Trp Gln Asp Ile Phe Met Glu Ala Ala Asn Ala Arg Asp Asn Glu
305                 310                 315                 320
Asp Ile Ser Ile His Pro Asp Gly Asp Trp Pro Ser Asp Asp Ser Glu
                325                 330                 335
Asp Val Asp Tyr Asp Pro Glu Lys Tyr Glu Ile Asp Cys Ser Tyr Ser
            340                 345                 350
Arg Met Gly Ser Glu His Asn Val Ser Asp Asp Ala Ser Ser Ser Gly
        355                 360                 365
Thr Leu Tyr Trp Thr Ser Glu Glu Asp Asp Gly Leu His Ser Glu Arg
    370                 375                 380
Phe Arg Thr Asp Gly Ser Leu Glu Gly Gly Phe Tyr Gly Asp Lys Thr
385                 390                 395                 400
Lys Ile Gln Ser Ser Asp Ser Ile Asp Ser Asn Asp Ser Thr Asp Gly
                405                 410                 415
Met Ala Phe His Arg Arg Gln Arg Arg Asp Val Asp Tyr Lys Lys Leu
```

```
                420                 425                 430
His Asp Glu Met Phe Gly Lys Asp Met Pro Glu Ser Glu Glu Ile Ser
            435                 440                 445
Glu Asp Glu Asp Trp Gly Pro Arg Arg Arg Lys Arg Arg Arg Asn Glu
    450                 455                 460
Ser Asn Ala Ala Thr Thr Leu Val Ser Ser Cys Gly Thr Glu Asn Glu
465                 470                 475                 480
Phe Ser Asn Val Ala Leu Xaa Xaa Pro Ser Arg Val Val Arg Glu Ser
                485                 490                 495
Leu Ser Lys Gln Leu Gly Ile Ala Ile Glu Lys Ile Asn Lys Trp Phe
            500                 505                 510
Lys Asn Ala Arg Tyr Thr Ala Leu Lys Ile Arg Lys Ala Glu Lys Thr
        515                 520                 525
Glu Gln Ser Pro Gly Gly Gly Lys Met Gln Ser Ala Asp Val Leu Thr
    530                 535                 540
Ser Lys Glu Asn His Tyr Leu Val Ser Thr Gly Thr Ala Val Gln Arg
545                 550                 555                 560
Ser Lys Val Ser Ser Val His Arg Ser Lys Asn Arg Lys Leu Thr Thr
                565                 570                 575
Thr Pro Ser Arg Glu Val Gln Glu Lys Val Ser Ala Val Ala Pro Ser
            580                 585                 590
Ala Thr Ala Asn Glu Val Ser Val Gln Leu Arg Lys Ala Val Ser Val
        595                 600                 605
Glu Lys Glu Met Gly Ser Val Lys Arg Lys Ser Ala Gln Val Lys Arg
    610                 615                 620
Ile Lys Arg Thr Cys Ser Thr Lys Glu Gln Gln Leu Tyr Met Ile Glu
625                 630                 635                 640
Leu Glu Arg Ile Cys Arg Leu Glu Glu Lys Leu Glu Lys Met Lys Lys
                645                 650                 655
Val Leu Leu Ser Ile Asp Asp Asn His Ser Asn Val Ser Asp Gly Pro
            660                 665                 670
His Met Asn Glu Gln Ser Ala Val Tyr Ile Pro Val Ala Glu Leu Arg
        675                 680                 685
Glu Lys Met
    690
```

<210> 225
<211> 1743
<212> DNA
<213> Glycine max

<220>
<221> misc_feature
<222> (261)..(261)
<223> n is a, c, g, or t

<400> 225

```
aagaagaaaa gacaaaggaa taacatagat gttgatgatg cttcacgctt gcgaaggaga      60

acaaggtacc tcttaatcaa aatgaagcta gagcagaacc ttattgatgc ttactctgga     120
gaaggttgga aaggtcaaag tcgggaaaag attaggccag aaaaggagct actacgagca     180
aaaaagcaga ttttaaaatg taaacttagt attcgcgatg ccatacacca gctggattct     240
cttagttctg tgggtagcat ngaagattct gccattgctc cagatggatc tgtttatcat     300
gaaaatatat tctgtgccaa ttgcaaacta catgaagctt tcccagataa tgatattata     360
ctctgtgatg gcacttgcaa tcgtgctttt caccaaagat gtctcaatcc tcctttggat     420
actgaaaata ttcctcctgg agatcaaggc tggttttgca agttttgtga atgtaagata     480
gaaatactag aggcaacaaa tgcccatctt gggactcaat tctccctaga cagtaattgg     540
caggatgtat tcaaggaaga ggctgctatg cctgatggtg acattgcatt actaaatcct     600
gaagaagaat ggccatcaga cgatcctgaa gatgatgatt acaatccaga gaggaaagaa     660
gacaaccaca actttgacac agaaggagct gatgaaaatg catccaatga ttcaaccagt     720
tgttccagtc tgttgtcttt gaatggcgaa tgtcctccag tagatgaagg catctgtcat     780
```

```
gaatattatt ctgttaatag ctgcttagat tctgatgaat ctggggaaat agcatgtggt     840
cctaggcagc gtaaagctgt tgactacaag aaactctatg atgaaatgta tgggaaggat     900
gctccacctt gtgaacaaat gagtgaagat gaagactggg gtcctggcaa aagaaagcga     960
agagaaaagg agtctgatgc tgttgattct cttatgactt tgcatgaaag tgagaatagg    1020
catcccaata atgagcacaa catgacaagt aaagattctt caagcataaa gatcaaaagg    1080
cattgtttta ggattccacg tgatgcagtt gagaggcttc gccaagtttt tgctgagaac    1140
gaacttcctc caagatctat aagagagggt ctttcaaaag agctgggcct tgacaccgag    1200
aaggtcagca aatggttcaa aaatgcacgt tacttagcac ttaaaaacag aaagcatcaa    1260
gcagaaggag gagcagatca gcttcagagt atcacttcta caaagaacag attacaaaaa    1320
caggagaatg tcgatccttt gaaatcaaag accccaaaaa ttactaggac acattcccag    1380
aaggatgtta aaaatgtcaa tggaagaaag aaaatgaagt catctaacaa gaaaagacaa    1440
ccagaaattc ctccacctcc aggagaaaat ggcaagaagg actttatgga gatcagtgat    1500
gatgtgagct tgaagaaact gttgaagaaa agaaagaaga ggttgataaa ttttacgttt    1560
ggaggagact ctcagttggc agagttggaa tttgaaagac taagcgaatt gaagactaaa    1620
gtagatagta tgaagcaaaa attaactgca attcaaaatt acagagttaa gggttcaccc    1680
tattcgaatg aaccatccat tgtatatgta cccacagctg tgttaaggga aaaggttgaa    1740
tga                                                                  1743
```

<210> 226
<211> 580
<212> PRT
<213> Glycine max

<220>
<221> UNSURE
<222> (87)..(87)
<223> Xaa can be any naturally occurring amino acid

<400> 226

```
Lys Lys Lys Arg Gln Arg Asn Asn Ile Asp Val Asp Asp Ala Ser Arg
1               5                   10                  15
Leu Arg Arg Arg Thr Arg Tyr Leu Leu Ile Lys Met Lys Leu Glu Gln
            20                  25                  30
Asn Leu Ile Asp Ala Tyr Ser Gly Glu Gly Trp Lys Gly Gln Ser Arg
        35                  40                  45
Glu Lys Ile Arg Pro Glu Lys Glu Leu Leu Arg Ala Lys Lys Gln Ile
    50                  55                  60
Leu Lys Cys Lys Leu Ser Ile Arg Asp Ala Ile His Gln Leu Asp Ser
65                  70                  75                  80
Leu Ser Ser Val Gly Ser Xaa Glu Asp Ser Ala Ile Ala Pro Asp Gly
                85                  90                  95
Ser Val Tyr His Glu Asn Ile Phe Cys Ala Asn Cys Lys Leu His Glu
            100                 105                 110
Ala Phe Pro Asp Asn Asp Ile Ile Leu Cys Asp Gly Thr Cys Asn Arg
        115                 120                 125
Ala Phe His Gln Arg Cys Leu Asn Pro Pro Leu Asp Thr Glu Asn Ile
    130                 135                 140
Pro Pro Gly Asp Gln Gly Trp Phe Cys Lys Phe Cys Glu Cys Lys Ile
145                 150                 155                 160
Glu Ile Leu Glu Ala Thr Asn Ala His Leu Gly Thr Gln Phe Ser Leu
                165                 170                 175
Asp Ser Asn Trp Gln Asp Val Phe Lys Glu Glu Ala Ala Met Pro Asp
            180                 185                 190
Gly Asp Ile Ala Leu Leu Asn Pro Glu Glu Glu Trp Pro Ser Asp Asp
        195                 200                 205
Pro Glu Asp Asp Asp Tyr Asn Pro Glu Arg Lys Glu Asp Asn His Asn
    210                 215                 220
Phe Asp Thr Glu Gly Ala Asp Glu Asn Ala Ser Asn Asp Ser Thr Ser
225                 230                 235                 240
Cys Ser Ser Leu Leu Ser Leu Asn Gly Glu Cys Pro Pro Val Asp Glu
                245                 250                 255
```

```
Gly Ile Cys His Glu Tyr Tyr Ser Val Asn Ser Cys Leu Asp Ser Asp
            260                 265                 270
Glu Ser Gly Glu Ile Ala Cys Gly Pro Arg Gln Arg Lys Ala Val Asp
        275                 280                 285
Tyr Lys Lys Leu Tyr Asp Glu Met Tyr Gly Lys Asp Ala Pro Pro Cys
    290                 295                 300
Glu Gln Met Ser Glu Asp Glu Asp Trp Gly Pro Gly Lys Arg Lys Arg
305                 310                 315                 320
Arg Glu Lys Glu Ser Asp Ala Val Asp Ser Leu Met Thr Leu His Glu
                325                 330                 335
Ser Glu Asn Arg His Pro Asn Asn Glu His Asn Met Thr Ser Lys Asp
            340                 345                 350
Ser Ser Ser Ile Lys Ile Lys Arg His Cys Phe Arg Ile Pro Arg Asp
        355                 360                 365
Ala Val Glu Arg Leu Arg Gln Val Phe Ala Glu Asn Glu Leu Pro Pro
    370                 375                 380
Arg Ser Ile Arg Glu Gly Leu Ser Lys Glu Leu Gly Leu Asp Thr Glu
385                 390                 395                 400
Lys Val Ser Lys Trp Phe Lys Asn Ala Arg Tyr Leu Ala Leu Lys Asn
                405                 410                 415
Arg Lys His Gln Ala Glu Gly Gly Ala Asp Gln Leu Gln Ser Ile Thr
            420                 425                 430
Ser Thr Lys Asn Arg Leu Gln Lys Gln Glu Asn Val Asp Pro Leu Lys
        435                 440                 445
Ser Lys Thr Pro Lys Ile Thr Arg Thr His Ser Gln Lys Asp Val Lys
    450                 455                 460
Asn Val Asn Gly Arg Lys Lys Met Lys Ser Ser Asn Lys Lys Arg Gln
465                 470                 475                 480
Pro Glu Ile Pro Pro Pro Pro Gly Glu Asn Gly Lys Lys Asp Phe Met
                485                 490                 495
Glu Ile Ser Asp Asp Val Ser Leu Lys Lys Leu Leu Lys Lys Arg Lys
            500                 505                 510
Lys Arg Leu Ile Asn Phe Thr Phe Gly Gly Asp Ser Gln Leu Ala Glu
        515                 520                 525
Leu Glu Phe Glu Arg Leu Ser Glu Leu Lys Thr Lys Val Asp Ser Met
    530                 535                 540
Lys Gln Lys Leu Thr Ala Ile Gln Asn Tyr Arg Val Lys Gly Ser Pro
545                 550                 555                 560
Tyr Ser Asn Glu Pro Ser Ile Val Tyr Val Pro Thr Ala Val Leu Arg
                565                 570                 575
Glu Lys Val Glu
            580
```

<210> 227
<211> 1614
<212> DNA
<213> Lotus corniculatus

<400> 227

```
atgcagggta ctgagaagtt aaatggcaca ggatctacaa aatccagtaa ctcagaggaa      60
catgctgagt caaaggtaga ttcattgaga tacagaacaa atataacaaa atgccgaggg     120
aagaaacaaa aactgaaatc aaaatctcat aaactcagcg gttgcttaag tgcatcaggg     180
aggacagtta ccaattcttc catcaagaga caagtaaagg attcttctaa taaaaagatt     240
attagacaaa gtctacataa aactgatggc aaatcttcac ggatgctgtc ttcaacaatg     300
atacaaggag gaaaatcttc tcttggttct agaaaggagg gcaaagatgt tgatggggag     360
gtaatggttc aaaaagttaa aagaaagaga aagaagaaaa ggagacagga tgatgttgat     420
cttgatgatg cttcacgctt actgagaaga acaaggtacc tcttaatcaa aatgaagcta     480
gagcagaatc ttattgatgc ttactctgga gaaggttgga aaggtcaaag tcgagagaag     540
attaggccag aaaaggagct acaaagagcc aagaagcaga ttttgaattg taaacttagt     600
attcgggatg ccattcgcca gttagattct cttagctcat tgggcagcat tgaagattct     660
gtcattgatc cagatggatc tgtttatcat gaacatatat tctgtgcaaa ttgcaagttg     720

cgtgaagctt tcccagataa tgatataata ctctgtgatg gcacatgcaa tcgtgctttt     780
caccaacggt gtctcaaccc tcctttggat actgaaaata ttcctcctgg agaccaaggc     840
tggttctgca atttttgtga atgtaagata gaaatattgg aggcaacaaa tgcccatctt     900
gggactcaaa tctccttgga cagtacttgg caggatgtat tcaaggagga ggctgctata     960
cccgatggtg ataatgcatt actgaatcca gaagaagaat ggccatcaga tgatcctgaa    1020
gacgatgatt attatccaga gaggaaagaa gacaaccaca gcatcaaagc agaaggaact    1080
gatgataatg catccaatga tttaagcagt tcttccagtc tagggtcttt gaatggtgaa    1140
tgttctccag tagatgaagg cacgggtctt gaatattatt ctgttaattg ctgcatagat    1200
tctgatgatt ctggggaaat agcatgtggc cgtaggcagc gtaaatctgt tgactacaag    1260
aaactatatg atgaaatgtt tgggaaggat gctcctccat gtgaactagt gagtgaagat    1320
gaagactggg gtactggcaa aagaaagcga agagaaaaag agtctgatgc tgttaatact    1380
ctcatgacta tgcatgaaag tgagaacaaa aatcccaata atgagaatag tgatggaata    1440
agagagggtt cttcaggcaa accaataaga aggtcttgtt tcaggattcc acatgatgca    1500
cttgagaagc ttcgccaagt ttttgcggag aatgagcttc ctccaagatc tgtcaaagaa    1560
ggtctttcga aagagttggg aattgatgct gcgaaggtta tgccttacaa cctc          1614
```

<210> 228
<211> 538
<212> PRT
<213> Lotus corniculatus

<400> 228

```
Met Gln Gly Thr Glu Lys Leu Asn Gly Thr Gly Ser Thr Lys Ser Ser
1               5                   10                  15
Asn Ser Glu Glu His Ala Glu Ser Lys Val Asp Ser Leu Arg Tyr Arg
            20                  25                  30
Thr Asn Ile Thr Lys Cys Arg Gly Lys Lys Gln Lys Leu Lys Ser Lys
        35                  40                  45
Ser His Lys Leu Ser Gly Cys Leu Ser Ala Ser Gly Arg Thr Val Thr
    50                  55                  60
Asn Ser Ser Ile Lys Arg Gln Val Lys Asp Ser Ser Asn Lys Lys Ile
65                  70                  75                  80
Ile Arg Gln Ser Leu His Lys Thr Asp Gly Lys Ser Ser Arg Met Leu
                85                  90                  95
Ser Ser Thr Met Ile Gln Gly Gly Lys Ser Ser Leu Gly Ser Arg Lys
            100                 105                 110
Glu Gly Lys Asp Val Asp Gly Glu Val Met Val Gln Lys Val Lys Arg
        115                 120                 125
Lys Arg Lys Lys Lys Arg Arg Gln Asp Asp Val Asp Leu Asp Asp Ala
    130                 135                 140
Ser Arg Leu Leu Arg Arg Thr Arg Tyr Leu Leu Ile Lys Met Lys Leu
145                 150                 155                 160
Glu Gln Asn Leu Ile Asp Ala Tyr Ser Gly Glu Gly Trp Lys Gly Gln
                165                 170                 175
Ser Arg Glu Lys Ile Arg Pro Glu Lys Glu Leu Gln Arg Ala Lys Lys
            180                 185                 190
Gln Ile Leu Asn Cys Lys Leu Ser Ile Arg Asp Ala Ile Arg Gln Leu
        195                 200                 205
Asp Ser Leu Ser Ser Leu Gly Ser Ile Glu Asp Ser Val Ile Asp Pro
    210                 215                 220
Asp Gly Ser Val Tyr His Glu His Ile Phe Cys Ala Asn Cys Lys Leu
225                 230                 235                 240
Arg Glu Ala Phe Pro Asp Asn Asp Ile Ile Leu Cys Asp Gly Thr Cys
                245                 250                 255
Asn Arg Ala Phe His Gln Arg Cys Leu Asn Pro Pro Leu Asp Thr Glu
            260                 265                 270
Asn Ile Pro Pro Gly Asp Gln Gly Trp Phe Cys Asn Phe Cys Glu Cys
        275                 280                 285
Lys Ile Glu Ile Leu Glu Ala Thr Asn Ala His Leu Gly Thr Gln Ile
    290                 295                 300
Ser Leu Asp Ser Thr Trp Gln Asp Val Phe Lys Glu Glu Ala Ala Ile
```

```
305                 310                 315                 320
Pro Asp Gly Asp Asn Ala Leu Leu Asn Pro Glu Glu Glu Trp Pro Ser
                325                 330                 335

Asp Asp Pro Glu Asp Asp Asp Tyr Tyr Pro Glu Arg Lys Glu Asp Asn
            340                 345                 350
His Ser Ile Lys Ala Glu Gly Thr Asp Asp Asn Ala Ser Asn Asp Leu
        355                 360                 365
Ser Ser Ser Ser Ser Leu Gly Ser Leu Asn Gly Glu Cys Ser Pro Val
    370                 375                 380
Asp Glu Gly Thr Gly Leu Glu Tyr Tyr Ser Val Asn Cys Cys Ile Asp
385                 390                 395                 400
Ser Asp Asp Ser Gly Glu Ile Ala Cys Gly Arg Arg Gln Arg Lys Ser
                405                 410                 415
Val Asp Tyr Lys Lys Leu Tyr Asp Glu Met Phe Gly Lys Asp Ala Pro
            420                 425                 430
Pro Cys Glu Leu Val Ser Glu Asp Glu Asp Trp Gly Thr Gly Lys Arg
        435                 440                 445
Lys Arg Arg Glu Lys Glu Ser Asp Ala Val Asn Thr Leu Met Thr Met
    450                 455                 460
His Glu Ser Glu Asn Lys Asn Pro Asn Asn Glu Asn Ser Asp Gly Ile
465                 470                 475                 480
Arg Glu Gly Ser Ser Gly Lys Pro Ile Arg Arg Ser Cys Phe Arg Ile
                485                 490                 495
Pro His Asp Ala Leu Glu Lys Leu Arg Gln Val Phe Ala Glu Asn Glu
            500                 505                 510
Leu Pro Pro Arg Ser Val Lys Glu Gly Leu Ser Lys Glu Leu Gly Ile
        515                 520                 525
Asp Ala Ala Lys Val Met Pro Tyr Asn Leu
    530                 535
```

<210> 229
<211> 573
<212> DNA
<213> Sorghum propinquum

<400> 229

```
aactcgagaa caaatgaaga aaaatctggt atatcaggga agattgattc agtagacaac     60
tcttgcttgg ttcccttgtc tgaaatcatc aatgtgcata cacgagtgca ccacaatctt    120
gagatgagga agatggaatc aactagcagt cctgtgtggc tgcacaatga aggaggctgc    180
ttgtttccaa cactccaagc caaggagagt acattaccta caagcaagcc ttgtttgccg    240
tcagaaataa cccatccaac aactaatgaa gtgagcactt tagtgcaagc tacttcttgg    300
atggatgctg gggcgtgcat tgagcagcaa gaaaccactc cttgggtgga taccggggcc    360
tcaggttacc agcctttcct ggacgtgatt gatgagatgt gcggacttga gtgcaggctg    420
cagaggctga aggagaacat gttctcatct ggcatggacg gcagaaccgc cggtgtgagt    480
gacatgggaa accaggctgt ggtgcttgtg ccgactgctg agctcaagga aaaagcgccg    540
catggcggtt tatttgggca ctattgccca tag                                 573
```

<210> 230
<211> 190
<212> PRT
<213> Sorghum propinquum

<400> 230

```
Asn Ser Arg Thr Asn Glu Glu Lys Ser Gly Ile Ser Gly Lys Ile Asp
1               5                   10                  15
Ser Val Asp Asn Ser Cys Leu Val Pro Leu Ser Glu Ile Ile Asn Val
            20                  25                  30
His Thr Arg Val His His Asn Leu Glu Met Arg Lys Met Glu Ser Thr
        35                  40                  45
Ser Ser Pro Val Trp Leu His Asn Glu Gly Gly Cys Leu Phe Pro Thr


    50                  55                  60
Leu Gln Ala Lys Glu Ser Thr Leu Pro Thr Ser Lys Pro Cys Leu Pro
65                  70                  75                  80
Ser Glu Ile Thr His Pro Thr Thr Asn Glu Val Ser Thr Leu Val Gln
                85                  90                  95
Ala Thr Ser Trp Met Asp Ala Gly Ala Cys Ile Glu Gln Gln Glu Thr
            100                 105                 110
Thr Pro Trp Val Asp Thr Gly Ala Ser Gly Tyr Gln Pro Phe Leu Asp
        115                 120                 125
Val Ile Asp Glu Met Cys Gly Leu Glu Cys Arg Leu Gln Arg Leu Lys
    130                 135                 140
Glu Asn Met Phe Ser Ser Gly Met Asp Gly Arg Thr Ala Gly Val Ser
145                 150                 155                 160
Asp Met Gly Asn Gln Ala Val Val Leu Val Pro Thr Ala Glu Leu Lys
                165                 170                 175
Glu Lys Ala Pro His Gly Gly Leu Phe Gly His Tyr Cys Pro
            180                 185                 190
```

<210> 231
<211> 532
<212> DNA
<213> Sorghum propinquum

<400> 231

```
aagataaaat tggagcagaa tctactagat gcttactctg gagatggatg gaatgggcaa     60
agccgggaga aaataaagcc agagaaggaa ctgcagcgtg ccaggaaaca aatcataaaa    120
tgcaaaattg ctatacgtga tatcattcgc caactttgtt tgtatacttc tactgggagt    180
gttgatgacc cagcaatgcc tccagatcag tttaccaatc ccgaacatac catgtgctca    240
acatgcaagt ctcatgaatc atttcccagc aataaattta tcttctgtga agggccctgc    300
aaaagggcat atcatgagaa atgtttggaa cctcccttaa acaaaggtgt acttccaaca    360
agtagccatg gatggctttg caaattctgt ttgtgcaagg tgaaaatttt agaaactatt    420
aatgcacatc taggaacaag ttttacagtg atgtgctcct ttgaagatat attcaaggaa    480
gcaacagaac aaatagactc cgaggatgca ctagatgaag attggctctc tg            532
```

<210> 232
<211> 177
<212> PRT
<213> Sorghum propinquum

<400> 232

```
Lys Ile Lys Leu Glu Gln Asn Leu Leu Asp Ala Tyr Ser Gly Asp Gly
1               5                   10                  15
Trp Asn Gly Gln Ser Arg Glu Lys Ile Lys Pro Glu Lys Glu Leu Gln
            20                  25                  30
Arg Ala Arg Lys Gln Ile Ile Lys Cys Lys Ile Ala Ile Arg Asp Ile
        35                  40                  45
Ile Arg Gln Leu Cys Leu Tyr Thr Ser Thr Gly Ser Val Asp Asp Pro
    50                  55                  60
Ala Met Pro Pro Asp Gln Phe Thr Asn Pro Glu His Thr Met Cys Ser
65                  70                  75                  80
Thr Cys Lys Ser His Glu Ser Phe Pro Ser Asn Lys Phe Ile Phe Cys
                85                  90                  95
Glu Gly Pro Cys Lys Arg Ala Tyr His Glu Lys Cys Leu Glu Pro Pro
            100                 105                 110
Leu Asn Lys Gly Val Leu Pro Thr Ser Ser His Gly Trp Leu Cys Lys
        115                 120                 125
Phe Cys Leu Cys Lys Val Lys Ile Leu Glu Thr Ile Asn Ala His Leu
    130                 135                 140
Gly Thr Ser Phe Thr Val Met Cys Ser Phe Glu Asp Ile Phe Lys Glu
145                 150                 155                 160
Ala Thr Glu Gln Ile Asp Ser Glu Asp Ala Leu Asp Glu Asp Trp Leu


                  165                 170                 175
  Ser
```

<210> 233
<211> 180
<212> PRT
<213> Oryza sativa

<400> 233

```
Asp Glu Val Ser Arg Met Glu Lys Arg Ala Arg Tyr Leu Leu Ile Lys
1               5                   10                  15
Ile Lys Gln Glu Gln Asn Leu Leu Asp Ala Tyr Ser Gly Asp Gly Trp
            20                  25                  30
Asn Gly His Ser Arg Glu Lys Ile Lys Pro Glu Lys Glu Leu Gln Arg
        35                  40                  45
Ala Lys Lys Gln Ile Met Lys Tyr Lys Ile Ala Ile Arg Asp Val Ile
    50                  55                  60
His Gln Leu Asp Leu Cys Ser Ser Ser Gly Ser Lys Asp Asp Ser Val
65                  70                  75                  80
Ile Pro Pro Asp Gly Cys His Glu Ser Val Asn Pro Glu His Thr Ile
                85                  90                  95
Cys Ser Arg Cys Lys Ser His Glu Ser Phe Pro Asp Asn Asn Ile Ile
            100                 105                 110
Phe Cys Glu Gly Gly Cys Lys Leu Ala Cys His Gln Lys Cys Leu Glu
        115                 120                 125
Pro Pro Phe Asp Lys Ile Leu Pro Thr Thr Arg His Gly Arg Leu Cys
    130                 135                 140
Lys His Cys Ser Ser Lys Met Lys Ile Leu Asp Ala Ile Asn Ala His
145                 150                 155                 160
Leu Gly Thr Ser Phe Thr Val Lys Cys Pro Ser Ser Asp Ile Phe Lys
                165                 170                 175
Glu Ala Ala Glu
            180
```

<210> 234
<211> 50
<212> PRT
<213> Oryza sativa

<400> 234

```
Arg Ile Pro Pro Ala Ala Val Glu Val Leu Arg Lys Ala Phe Ala Glu
1               5                   10                  15
Asn Glu Leu Pro Ala Arg Ser Val Lys Glu Asn Leu Ser Thr Glu Leu
            20                  25                  30
Gly Ile Ser Phe Glu Lys Ile Asp Lys Trp Phe Lys Asn Thr Arg Cys
        35                  40                  45
Ala Ala
    50
```

<210> 235
<211> 2194
<212> DNA
<213> Oryza sativa

<400> 235

```
aatccgaaaa gtttctgcac cgttttcacc ccctaactaa caatataggg aacgtgtgct      60
aaatataaaa tgagacctta tatatgtagc gctgataact agaactatgc aagaaaaact     120
catccaccta ctttagtggc aatcgggcta aataaaaaag agtcgctaca ctagtttcgt     180
tttccttagt aattaagtgg gaaaatgaaa tcattattgc ttagaatata cgttcacatc     240
tctgtcatga agttaaatta ttcgaggtag ccataattgt catcaaactc ttcttgaata     300

aaaaaatctt tctagctgaa ctcaatgggt aaagagagag atttttttta aaaaaataga     360
atgaagatat tctgaacgta ttggcaaaga tttaaacata taattatata attttatagt     420
ttgtgcattc gtcatatcgc acatcattaa ggacatgtct tactccatcc caatttttat     480
ttagtaatta aagacaattg acttattttt attatttatc tttttttcgat tagatgcaag     540
gtacttacgc acacactttg tgctcatgtg catgtgtgag tgcacctcct caatacacgt     600
tcaactagca acacatctct aatatcactc gcctatttaa tacatttagg tagcaatatc     660
tgaattcaag cactccacca tcaccagacc acttttaata atatctaaaa tacaaaaaat     720
aattttacag aatagcatga aaagtatgaa acgaactatt taggtttttc acatacaaaa     780
aaaaaaagaa ttttgctcgt gcgcgagcgc caatctccca tattgggcac acaggcaaca     840
acagagtggc tgcccacaga acaacccaca aaaaacgatg atctaacgga ggacagcaag     900
tccgcaacaa ccttttaaca gcaggctttg cggccaggag agaggaggag aggcaaagaa     960
aaccaagcat cctccttctc ccatctataa attcctcccc cctttttcccc tctctatata    1020
ggaggcatcc aagccaagaa gagggagagc accaaggaca cgcgactagc agaagccgag    1080
cgaccgcctt ctcgatccat atcttccggt cgagttcttg gtcgatctct tccctcctcc    1140
acctcctcct cacagggtat gtgcctccct tcggttgttc ttggatttat tgttctaggt    1200
tgtgtagtac gggcgttgat gttaggaaag gggatctgta tctgtgatga ttcctgttct    1260
tggatttggg atagaggggt tcttgatgtt gcatgttatc ggttcggttt gattagtagt    1320
atggttttca atcgtctgga gagctctatg gaaatgaaat ggtttaggga tcggaatctt    1380
gcgattttgt gagtaccttt tgtttgaggt aaaatcagag caccggtgat tttgcttggt    1440
gtaataaagt acggttgttt ggtcctcgat tctggtagtg atgcttctcg atttgacgaa    1500
gctatccttt gtttattccc tattgaacaa aaataatcca actttgaaga cggtcccgtt    1560
gatgagattg aatgattgat tcttaagcct gtccaaaatt tcgcagctgg cttgtttaga    1620
tacagtagtc cccatcacga aattcatgga aacagttata atcctcagga acaggggatt    1680
ccctgttctt ccgatttgct ttagtcccag aatttttttt cccaaatatc ttaaaaagtc    1740
actttctggt tcagttcaat gaattgattg ctacaaataa tgcttttata gcgttatcct    1800
agctgtagtt cagttaatag gtaatacccc tatagtttag tcaggagaag aacttatccg    1860
atttctgatc tccattttta attatatgaa atgaactgta gcataagcag tattcatttg    1920
gattattttt tttattagct ctcacccctt cattattctg agctgaaagt ctggcatgaa    1980
ctgtcctcaa ttttgttttc aaattcacat cgattatcta tgcattatcc tcttgtatct    2040
acctgtagaa gtttcttttt ggttattcct tgactgcttg attacagaaa gaaatttatg    2100
aagctgtaat cgggatagtt atactgcttg ttcttatgat tcatttcctt tgtgcagttc    2160
ttggtgtagc ttgccacttt caccagcaaa gttc                                 2194
```

<210> 236
<211> 56
<212> DNA

<213> Artificial sequence

<220>
<223> primer: prm09687

<400> 236
ggggacaagt ttgtacaaaa aagcaggctt aaacaatgaa taccccagaa aagaaa 56

<210> 237
<211> 50
<212> DNA
<213> Artificial sequence

<220>
<223> primer: prm09688

<400> 237
ggggaccact ttgtacaaga aagctgggtg atgcaaggtt aagatgcttt 50

<210> 238
<211> 2649
<212> DNA
<213> Physcomitrella patens

<400> 238

```
atgatgaaga attaccgtcc tccatcacca cttccaagtg ctcgccaagc tttcttacaa      60
gacccggcgt cgtcattcca aatgacagga cgacattggg cagacccaga gggaggtgga     120
gaacggaagc ttcacacaca ctctcactca cactcactca ctcacgaagg aaacgaaggg     180
caaaggaagg ggagccccgc cgtgagccga gagcggagag caagcattcc caacggaagt     240
ctccgtcaat gcggtgccgg agccgacgac gacgacgatg atgggctgag cctgtcatcg     300
caatggatgc attgccctgt aagtgggaag gtggaggaag gattggggaa gaggttgagg     360
aagagaaaga gaaagagaaa gagtttgttg ttgttgttgg ggaaggggag ggggcgacgg     420
atgatgccca tgggcgtagt ccgcgacgat gagtcccagg ccgctgctgg ctcgccgcct     480
gcgcccaccc ctcccccaat cgccaagccg acgctgcgat cccactcgcg ctccagggcg     540
cacaaggcgc gctcccaaca cccgccgctg ggcgatggcg atgcgccctt gcccccaccg     600
caaggaacag caacaccagg aggaggagga gcagcagcag tgggcgcacc aggggccatg     660
gatgccgagg ctatggcggc ggggcagcgg cggcgtcgac gacgccgacg acagcggggc     720
aagaactcgg atgtgccgct tgacaatcgc acgcgaatta agaggcgtgt caagtacttg     780
ctcatgaaaa tgcgagtcga ccagaacctg ctggatgcgt actccgggga gggttggaag     840
gggcagagga attctctttt tggcatatcc agtcgggaaa agatcaggcc ggagcaagaa     900
ctacagcgtg ctgaagcgca gatcctgcat tcgaaattgg ccatacggga ggctattcat     960
gaattggata acttggggtt agaaggctcg ctcgacgaaa atgcatttga tgctgaaggc    1020
cgtgtttacc atgaagagat attttgtgcc aagtgccgat ctcaagatgc tctacctgac    1080
aacgacatta ttttatgtga cggcgcatgc aacagaggct tccatcagta ctgtttagac    1140
ccacctttag ctacgatcaa cattcctcct ggagacgaag ggtggttgtg tcctgtatgt    1200
gagtgcaaga tggagtgcat tgaagttatt aatgcatact tgggcacaca ctttgaggtg    1260
gagaatagtt gggaggagtt gttttctgaa gaagctctcg tggctgcggg cggtagaact    1320
caaggggtca cagatggtga ttttccttcc tcagactcag aagacgacga ttataaccct    1380
gaaggagcgg agaagcggac agacagtgaa agcgaaggcg aaagtgatga aggtggggga    1440
gatagtgatt caggaagcga ttttagggaa aatgaagcgt ctggtagtga caaagatgag    1500
caagatccaa tgcaggcaac taatttagag agacttgcga gaacagcaaa gcggaccagc    1560
aaggaaggaa gtggaggtcc agaatcttca ggcagtgatg actcagaagt ttttcttgtt    1620
gatgggttta cggatggtgg agtcaaatta ggtttaagac gtagagactc acaagcagaa    1680
gaatcagctt cagacgagga aaccatggtt atagcaggaa aaagacatcg aaaagcagta    1740
gactacaaga gattacatga tgaaatgttt gggaacatgg agattgatgt ggacgatatc    1800
atatccgaag atgaggattg gggtccgaaa cgccgtcgga gaagaaccat tcctgatgac    1860
ccaagcaggt ctagacctcc tcgagttcga cggagaaagg cgcgaacctc aactggagat    1920
gctttaaaag aagttgatgc agatggggta ccatcagatt taccctctgg gtcacaaggc    1980
gagggagcag ccgatactct tgaagctgtt agagacaaac gaatgtggag gaagttgcct    2040
gattctgctg ttgagacgtt acgactggct gcggcagtga cgacactgcc ctcaaaatcc    2100
cgtaaagagg agttggcgac acagcttggc ttgagttttt cacaagtcca tggatggttt    2160
aaaaatcagc ggtatttagc tctcagaaat gggttggcga tttcaaagcg accagcagct    2220
gggcggatgg cggccactgt aaccatacaa tcggaacacg acaacaattc aagtgcctat    2280
ttaactgaga agttggatga agtccaaatg cgactttttg aactgaagca aactttggaa    2340
gatttcgcta agaattcatc tggtttaggc ggtggagaat cagataacag ttttgaaaat    2400
ggaggaattg gacttcctgc aaatgggaaa cggattatct atgtcccagt ggctgaggtt    2460
agagaacggc cttctctttt ctctgacagc tgtccgcttt cattattagc agtgttaaat    2520

tgcgaggtga attacaagag gaatctacgt atattcatgt gttcatggcc tagctgctta    2580
gcgtacgggg ttttgcatgc caactgccct tgtcatattg gatcatgttt cgaaggaata    2640
ggtgggtga                                                             2649
```

<210> 239

<211> 882

<212> PRT

<213> Physcomitrella patens

<400> 239

```
Met Met Lys Asn Tyr Arg Pro Pro Ser Pro Leu Pro Ser Ala Arg Gln
1               5                   10                  15
Ala Phe Leu Gln Asp Pro Ala Ser Ser Phe Gln Met Thr Gly Arg His
            20                  25                  30
Trp Ala Asp Pro Glu Gly Gly Gly Glu Arg Lys Leu His Thr His Ser
        35                  40                  45
His Ser His Ser Leu Thr His Glu Gly Asn Glu Gly Gln Arg Lys Gly
    50                  55                  60
```

Ser Pro Ala Val Ser Arg Glu Arg Arg Ala Ser Ile Pro Asn Gly Ser
65 70 75 80
Leu Arg Gln Cys Gly Ala Gly Ala Asp Asp Asp Asp Asp Asp Gly Leu
85 90 95
Ser Leu Ser Ser Gln Trp Met His Cys Pro Val Ser Gly Lys Val Glu
100 105 110
Glu Gly Leu Gly Lys Arg Leu Arg Lys Arg Lys Arg Lys Arg Lys Ser
115 120 125
Leu Leu Leu Leu Leu Gly Lys Gly Arg Gly Arg Arg Met Met Pro Met
130 135 140
Gly Val Val Arg Asp Asp Glu Ser Gln Ala Ala Ala Gly Ser Pro Pro
145 150 155 160
Ala Pro Thr Pro Pro Pro Ile Ala Lys Pro Thr Leu Arg Ser His Ser
165 170 175
Arg Ser Arg Ala His Lys Ala Arg Ser Gln His Pro Pro Leu Gly Asp
180 185 190
Gly Asp Ala Pro Leu Pro Pro Pro Gln Gly Thr Ala Thr Pro Gly Gly
195 200 205
Gly Gly Ala Ala Ala Val Gly Ala Pro Gly Ala Met Asp Ala Glu Ala
210 215 220
Met Ala Ala Gly Gln Arg Arg Arg Arg Arg Arg Arg Arg Gln Arg Gly
225 230 235 240
Lys Asn Ser Asp Val Pro Leu Asp Asn Arg Thr Arg Ile Lys Arg Arg
245 250 255
Val Lys Tyr Leu Leu Met Lys Met Arg Val Asp Gln Asn Leu Leu Asp
260 265 270
Ala Tyr Ser Gly Glu Gly Trp Lys Gly Gln Arg Asn Ser Leu Phe Gly
275 280 285
Ile Ser Ser Arg Glu Lys Ile Arg Pro Glu Gln Glu Leu Gln Arg Ala
290 295 300
Glu Ala Gln Ile Leu His Ser Lys Leu Ala Ile Arg Glu Ala Ile His
305 310 315 320
Glu Leu Asp Asn Leu Gly Leu Glu Gly Ser Leu Asp Glu Asn Ala Phe
325 330 335
Asp Ala Glu Gly Arg Val Tyr His Glu Glu Ile Phe Cys Ala Lys Cys
340 345 350
Arg Ser Gln Asp Ala Leu Pro Asp Asn Asp Ile Ile Leu Cys Asp Gly
355 360 365
Ala Cys Asn Arg Gly Phe His Gln Tyr Cys Leu Asp Pro Pro Leu Ala
370 375 380
Thr Ile Asn Ile Pro Pro Gly Asp Glu Gly Trp Leu Cys Pro Val Cys
385 390 395 400
Glu Cys Lys Met Glu Cys Ile Glu Val Ile Asn Ala Tyr Leu Gly Thr
405 410 415
His Phe Glu Val Glu Asn Ser Trp Glu Glu Leu Phe Ser Glu Glu Ala
420 425 430
Leu Val Ala Ala Gly Gly Arg Thr Gln Gly Val Thr Asp Gly Asp Phe
435 440 445
Pro Ser Ser Asp Ser Glu Asp Asp Asp Tyr Asn Pro Glu Gly Ala Glu
450 455 460
Lys Arg Thr Asp Ser Glu Ser Glu Gly Glu Ser Asp Glu Gly Gly Gly
465 470 475 480
Asp Ser Asp Ser Gly Ser Asp Phe Arg Glu Asn Glu Ala Ser Gly Ser
485 490 495
Asp Lys Asp Glu Gln Asp Pro Met Gln Ala Thr Asn Leu Glu Arg Leu
500 505 510
Ala Arg Thr Ala Lys Arg Thr Ser Lys Glu Gly Ser Gly Gly Pro Glu
515 520 525
Ser Ser Gly Ser Asp Asp Ser Glu Val Phe Leu Val Asp Gly Phe Thr
530 535 540
Asp Gly Gly Val Lys Leu Gly Leu Arg Arg Arg Asp Ser Gln Ala Glu

```
545                 550                 555                 560
Glu Ser Ala Ser Asp Glu Glu Thr Met Val Ile Ala Gly Lys Arg His
                565                 570                 575
Arg Lys Ala Val Asp Tyr Lys Arg Leu His Asp Glu Met Phe Gly Asn
            580                 585                 590
Met Glu Ile Asp Val Asp Asp Ile Ile Ser Glu Asp Glu Asp Trp Gly
        595                 600                 605
Pro Lys Arg Arg Arg Arg Arg Thr Ile Pro Asp Asp Pro Ser Arg Ser
    610                 615                 620
Arg Pro Pro Arg Val Arg Arg Arg Lys Ala Arg Thr Ser Thr Gly Asp
625                 630                 635                 640
Ala Leu Lys Glu Val Asp Ala Asp Gly Val Pro Ser Asp Leu Pro Ser
                645                 650                 655
Gly Ser Gln Gly Glu Gly Ala Ala Asp Thr Leu Glu Ala Val Arg Asp
            660                 665                 670
Lys Arg Met Trp Arg Lys Leu Pro Asp Ser Ala Val Glu Thr Leu Arg
        675                 680                 685
Leu Ala Ala Ala Val Thr Thr Leu Pro Ser Lys Ser Arg Lys Glu Glu
    690                 695                 700
Leu Ala Thr Gln Leu Gly Leu Ser Phe Ser Gln Val His Gly Trp Phe
705                 710                 715                 720
Lys Asn Gln Arg Tyr Leu Ala Leu Arg Asn Gly Leu Ala Ile Ser Lys
                725                 730                 735
Arg Pro Ala Ala Gly Arg Met Ala Ala Thr Val Thr Ile Gln Ser Glu
            740                 745                 750
His Asp Asn Asn Ser Ser Ala Tyr Leu Thr Glu Lys Leu Asp Glu Val
        755                 760                 765
Gln Met Arg Leu Phe Glu Leu Lys Gln Thr Leu Glu Asp Phe Ala Lys
    770                 775                 780
Asn Ser Ser Gly Leu Gly Gly Gly Glu Ser Asp Asn Ser Phe Glu Asn
785                 790                 795                 800
Gly Gly Ile Gly Leu Pro Ala Asn Gly Lys Arg Ile Ile Tyr Val Pro
                805                 810                 815
Val Ala Glu Val Arg Glu Arg Pro Ser Leu Phe Ser Asp Ser Cys Pro
            820                 825                 830

Leu Ser Leu Leu Ala Val Leu Asn Cys Glu Val Asn Tyr Lys Arg Asn
        835                 840                 845
Leu Arg Ile Phe Met Cys Ser Trp Pro Ser Cys Leu Ala Tyr Gly Val
    850                 855                 860
Leu His Ala Asn Cys Pro Cys His Ile Gly Ser Cys Phe Glu Gly Ile
865                 870                 875                 880
Gly Gly
```

<210> 240
<211> 2379
<212> DNA
<213> Physcomitrella patens

<400> 240

```
atggtgccga cgggcgtggt ggagcgggag gaccagcctg agcctgctgc tgcccccccca     60
gtgccggtga ttacacccaa gacgcttcga tcgcactcta tcaggacgca gaccacgcca    120
ggtgatggcg atgctggtgc ggatcatgct cctggagcag tggcagcagc agcagcatca    180
gcaggaggag gaggagcagc ggatgtcatg caaggtaacg atgcggggca ggaacggcga    240
ggacgacggc gcgggcgacg gaagaagtca gacgtgccgc tcgataatcc cacgcgaatc    300
ctaaagcatg tcaagtactt gctcattaaa atgcgagtcc accagaacct gctcgatgcg    360
tacacagggg agggttggaa gggtcagagt cgcgagaaga tcaggccgga gcaagaacta    420
caacgtgcga aggcgcagat actgcgatct aagttggcca tacgggaggc tattcatgaa    480
ctcgatgacg tgggtttaga aggttctctc gacaaaaacg cttttgattc tgaaggtcgt    540
atttaccatg aagagatttt ttgtgcgaag tgcaaatccc aagaagctct tcctgacaac    600
```

```
gacattatct tgtgcgacgg ggcgtgcaac agaggcttcc atcagtattg cttagaccca    660
cctttagcta cggaagacat tcctcctgga gatgaaggat ggttatgccc tgtatgcgac    720
tgcaagatgg agtgtattga agcgataaat tcatatttg gcaccagttt tgaggtcgag    780
aatagttggg agagcttttt ttcgaacgaa gctggtatag ctgcaggcgg tggaacgcaa    840
gaaggggctg gtggtgattg gccttcctca ggcgatgaag atgatgagta tgatccagaa    900
acagcggaag ggccttcttc agcttctgaa agtggagctc aagtaatagg gcttgctgtt    960
gactggccgt cctcagacga cgaagatcac gactatgatc cggaagcagc ggaagaacct   1020
cctggagctt ctgggagtgg aactcaaaca gtaggtcttg acgttgattg gccttccaca   1080
gacgatgaag atgacgactt tgatccagaa ggtatcagaa agcaggcaaa cagtgacgaa   1140
gaaagtagta gtgatgaaag tgaggaggac ggggaggata gtggttctgt aagcggcttc   1200
agggaaaatg aggcgtcttc aggtgtaagt gacaaagatg agcaggagcc tagtgaaaaa   1260
cataatctgg agctaactgc tgtcactaac atcaagaaac ttgcaagaaa aggaaaacgt   1320
aacataatca gcaacaaagg aaaaagtgaa tatttagagt cttcagacag tgatgattct   1380
gatgtctctc tttttgacga gttcaagggt ggtaatgcca atttaggctt aagacgtggg   1440
gatccacagg cagcagtatc agcagcagac gaggaagccg tgattattgc tgggaaaaga   1500
catcggaaag ctgtggacta caaaagacta catgatgaaa tgtatggtaa agcggaggcc   1560
gataacgatg acgacgttat ctctgaggat gaagattggg gtcctgaacg tcgccgaaga   1620
cgaaccattc ctgatgatcc taactcgcca aggtcccgaa ttcgaggtag gaaagtgaga   1680
actccaagtg gagatatttc taaaggagcc gatgcagatt taccctctgg attaccagat   1740
atgccatccc actttcccac tggttcacaa ggtgaagtag gctcaccaca agacattcca   1800
gcactagatg gagcagctga cagtcctgga ggagaaaagc ggatgtggcg gcgattgcct   1860
gattccgcag tcgaggcatt acggtgtatt cttgatgtta ataggctgcc ctcaaaatct   1920
cggaaggaag aattagcaat taagcttggg ttgagctttt cacaggttca tggatggttt   1980
aaaaatcagc ggcatcaagc tctgaggaaa gggttggcat gtcctaagcg gatacatcca   2040
gtagcatcaa gctcaagaga tttcaatact gtcgaatctc catcgtctgt ggcgacttta   2100
actgaaagca cattaccgca aaaagagaat gctccaaatt ctattttaac tgagaagttg   2160
gacgaagttc aggtgcgact cctcgaattg aagcaagcgt tggacgattt taccaagaat   2220
gcagcagtgg ttgcggatgg cggaagtatt gaattggata acaacgtgga aaacggagga   2280
attggacttc ctgaaaatgg aaaacgtgtt gtctatgtgc cagttgtggc cgacgttagg   2340
gaacaatcac caatgagtac gcatactagc gcatgttga                          2379
```

<210> 241
<211> 792
<212> PRT
<213> Physcomitrella patens

<400> 241

```
Met Val Pro Thr Gly Val Val Glu Arg Glu Asp Gln Pro Glu Pro Ala
1               5                   10                  15
Ala Ala Pro Pro Val Pro Val Ile Thr Pro Lys Thr Leu Arg Ser His
            20                  25                  30
Ser Ile Arg Thr Gln Thr Thr Pro Gly Asp Gly Asp Ala Gly Ala Asp
        35                  40                  45
His Ala Pro Gly Ala Val Ala Ala Ala Ala Ala Ser Ala Gly Gly Gly
    50                  55                  60
Gly Ala Ala Asp Val Met Gln Gly Asn Asp Ala Gly Gln Glu Arg Arg
65                  70                  75                  80
Gly Arg Arg Arg Gly Arg Arg Lys Lys Ser Asp Val Pro Leu Asp Asn
                85                  90                  95
Pro Thr Arg Ile Leu Lys His Val Lys Tyr Leu Leu Ile Lys Met Arg
            100                 105                 110
Val His Gln Asn Leu Leu Asp Ala Tyr Thr Gly Glu Gly Trp Lys Gly
        115                 120                 125
Gln Ser Arg Glu Lys Ile Arg Pro Glu Gln Glu Leu Gln Arg Ala Lys
    130                 135                 140
Ala Gln Ile Leu Arg Ser Lys Leu Ala Ile Arg Glu Ala Ile His Glu
145                 150                 155                 160
Leu Asp Asp Val Gly Leu Glu Gly Ser Leu Asp Lys Asn Ala Phe Asp
                165                 170                 175
Ser Glu Gly Arg Ile Tyr His Glu Glu Ile Phe Cys Ala Lys Cys Lys
            180                 185                 190
```

Ser Gln Glu Ala Leu Pro Asp Asn Asp Ile Ile Leu Cys Asp Gly Ala
195 200 205
Cys Asn Arg Gly Phe His Gln Tyr Cys Leu Asp Pro Pro Leu Ala Thr
210 215 220
Glu Asp Ile Pro Pro Gly Asp Glu Gly Trp Leu Cys Pro Val Cys Asp
225 230 235 240
Cys Lys Met Glu Cys Ile Glu Ala Ile Asn Ser Tyr Phe Gly Thr Ser
245 250 255
Phe Glu Val Glu Asn Ser Trp Glu Ser Phe Phe Ser Asn Glu Ala Gly
260 265 270
Ile Ala Ala Gly Gly Gly Thr Gln Glu Gly Ala Gly Gly Asp Trp Pro
275 280 285
Ser Ser Gly Asp Glu Asp Asp Glu Tyr Asp Pro Glu Thr Ala Glu Gly
290 295 300
Pro Ser Ser Ala Ser Glu Ser Gly Ala Gln Val Ile Gly Leu Ala Val
305 310 315 320
Asp Trp Pro Ser Ser Asp Asp Glu Asp His Asp Tyr Asp Pro Glu Ala
325 330 335
Ala Glu Glu Pro Pro Gly Ala Ser Gly Ser Gly Thr Gln Thr Val Gly
340 345 350
Leu Asp Val Asp Trp Pro Ser Thr Asp Asp Glu Asp Asp Asp Phe Asp
355 360 365
Pro Glu Gly Ile Arg Lys Gln Ala Asn Ser Asp Glu Glu Ser Ser Ser
370 375 380
Asp Glu Ser Glu Glu Asp Gly Glu Asp Ser Gly Ser Val Ser Gly Phe
385 390 395 400
Arg Glu Asn Glu Ala Ser Ser Gly Val Ser Asp Lys Asp Glu Gln Glu
405 410 415
Pro Ser Glu Lys His Asn Leu Glu Leu Thr Ala Val Thr Asn Ile Lys
420 425 430
Lys Leu Ala Arg Lys Gly Lys Arg Asn Ile Ile Ser Asn Lys Gly Lys
435 440 445
Ser Glu Tyr Leu Glu Ser Ser Asp Ser Asp Asp Ser Asp Val Ser Leu
450 455 460
Phe Asp Glu Phe Lys Gly Gly Asn Ala Asn Leu Gly Leu Arg Arg Gly
465 470 475 480
Asp Pro Gln Ala Ala Val Ser Ala Ala Asp Glu Glu Ala Val Ile Ile
485 490 495
Ala Gly Lys Arg His Arg Lys Ala Val Asp Tyr Lys Arg Leu His Asp
500 505 510
Glu Met Tyr Gly Lys Ala Glu Ala Asp Asn Asp Asp Asp Val Ile Ser
515 520 525
Glu Asp Glu Asp Trp Gly Pro Glu Arg Arg Arg Arg Arg Thr Ile Pro
530 535 540
Asp Asp Pro Asn Ser Pro Arg Ser Arg Ile Arg Gly Arg Lys Val Arg
545 550 555 560
Thr Pro Ser Gly Asp Ile Ser Lys Gly Ala Asp Ala Asp Leu Pro Ser
565 570 575
Gly Leu Pro Asp Met Pro Ser His Phe Pro Thr Gly Ser Gln Gly Glu
580 585 590
Val Gly Ser Pro Gln Asp Ile Pro Ala Leu Asp Gly Ala Ala Asp Ser
595 600 605
Pro Gly Gly Glu Lys Arg Met Trp Arg Arg Leu Pro Asp Ser Ala Val
610 615 620
Glu Ala Leu Arg Cys Ile Leu Asp Val Asn Arg Leu Pro Ser Lys Ser
625 630 635 640
Arg Lys Glu Glu Leu Ala Ile Lys Leu Gly Leu Ser Phe Ser Gln Val
645 650 655
His Gly Trp Phe Lys Asn Gln Arg His Gln Ala Leu Arg Lys Gly Leu
660 665 670
Ala Cys Pro Lys Arg Ile His Pro Val Ala Ser Ser Ser Arg Asp Phe

```
        675                 680                 685
Asn Thr Val Glu Ser Pro Ser Ser Val Ala Thr Leu Thr Glu Ser Thr
    690                 695                 700
Leu Pro Gln Lys Glu Asn Ala Pro Asn Ser Ile Leu Thr Glu Lys Leu
705                 710                 715                 720
Asp Glu Val Gln Val Arg Leu Leu Glu Leu Lys Gln Ala Leu Asp Asp
                725                 730                 735
Phe Thr Lys Asn Ala Ala Val Val Ala Asp Gly Gly Ser Ile Glu Leu
            740                 745                 750
Asp Asn Asn Val Glu Asn Gly Gly Ile Gly Leu Pro Glu Asn Gly Lys
        755                 760                 765
Arg Val Val Tyr Val Pro Val Val Ala Asp Val Arg Glu Gln Ser Pro
    770                 775                 780
Met Ser Thr His Thr Ser Ala Cys
785                 790
```

<210> 242
<211> 2166
<212> DNA
<213> Zea mays

<400> 242

```
atgcattctt cggaaaataa actgggttgg aatgagacct ctatggggtt ggttcctgta      60
ccaaaaagac cagctagacc tgatgcttcc caccaatgca aatctgattc ctttatgaag     120
cgatcaccaa ggaaagttag aaatgctact ctggcaaaaa gcataaagag caaataccac     180
tatagtcccc tcaaacagcg gaagggttca gattctgttc ctgggaaaat cgtaacagga     240
ctaaccgcaa ggaaaaagaa gaaaaggaaa atccaaatta cagacgaggc aactcgtttg     300
gaacgaagag cgagatattt tctaatcaag ataaaactgg agcagaattt gctagatgct     360
tactctggag atggatggaa tgggcaaagc cgagagaaaa taaagccaga gaaggaactg     420
caacgtgcca ggaaacaaat cataaaatgc aaaattgcta tacgtgatat catccgccaa     480
cttgatttgt atacttctac tgggagtgtt gatgacccac taatgcctac agatcagtcc     540
accaatcccg aacataccat gtgctcaaca tgcaagtctc atgaatcatt tcccagcaat     600
aaaattatct tctgcaaagg gccctgcaaa agggcatgtc atgagaaatg tttggaacct     660
cccttaaaca aaagcgtact tccaacaagt agccatgggt ggctttgcaa attctgtttg     720
tgcaaggtga ggattttaga aactattaat gcacatctag ggacgagttt tacagtgaag     780
tgccactttg aagatatatt caaggaaact actgaactaa tagactctga ggatgcacta     840
gatgaagatt ggctttctga gtattcaggt gatgaggact atgatcctga tgaaaatgag     900
gccagtggcg actgtatgga cagcggggag aagattatgt ctgatgattc caatggttca     960
ggaagccccc tttattctcc aaatgacgat attcctgact tcatatcagc agacttaaat    1020
gttgtggaag ggttttgtca taccaattta gatttaggca ttgatgccgt tgaagatgat    1080
tttgcacaga tcctcaccta ccaaaggcca agaagagatg ttgattatag aaggcttaac    1140
gaggaaatgt ttgggaaaat aaccgggaat gaagaacaga gcgaggacga agattggggc    1200
catgaaagga gaaagaaaag gacgcattca ggtgttgctg ggataattc tgttggtttc    1260
ttgaacgtta tatctgatga aaagagccag aagaagggga gaaaactttt caggattcct    1320
cctgcagctg ttgaggtact tcgcagagct tttgctgaaa atgagcttcc accccgggat    1380
gttaaagaaa atctctcaag agaattggga atttcttttg aaaagattga taaatggttc    1440
aaaaatacac ggtgtgctgc tctcagagat cggaaggctg aaggaaacag tcataataca    1500
gctcccagca aatctcaaa aaataaagga aaagctggaa tctcaggcaa ggctggaagg    1560
aatggtcatg ttactggtcc ctgcaataat ttgagaataa atgaagaaaa aacaggtata    1620
tcggggaagt ttgattcagg agacaactct tgtttggttc ccttctctga agtcatcaat    1680
gtgcctacgc gattgccaca caactttgag atgaggaaga tggaatcaac tagaagtcct    1740
gcgaggctac acaataaagg agggtgcctg tctgcaacaa tccaaattaa ggagagtaca    1800
ttgttaccca caggcaagcc ttgttggcag tcagaaatga gccatccaac aactaatgaa    1860
gtgagcactt cagcgcaagc tactacttgg atcgacgcgg gggcgtgtgc cgaggagcaa    1920
gaacctactc cttgggtgga catcggggcc tcagactacc agcctttcct ggatgtgatc    1980
gacgacatgt gcggacttga gtggaggctg cagagactga aggagaacat gctctcatct    2040
agcatagacg gccaaaccgc cgccagtgag agtgacaaaa gaaaccagac cgtggtgctc    2100
gtgccaactg ccgagctcaa ggaaaaactg ccgcatgacg gtttattcgg gcattattgc    2160
ccatag                                                               2166
```

<210> 243

<211> 721
<212> PRT
<213> Zea mays

<400> 243

```
Met His Ser Ser Glu Asn Lys Leu Gly Trp Asn Glu Thr Ser Met Gly
1               5                   10                  15
Leu Val Pro Val Pro Lys Arg Pro Ala Arg Pro Asp Ala Ser His Gln
            20                  25                  30
Cys Lys Ser Asp Ser Phe Met Lys Arg Ser Pro Arg Lys Val Arg Asn
        35                  40                  45
Ala Thr Leu Ala Lys Ser Ile Lys Ser Lys Tyr His Tyr Ser Pro Leu
    50                  55                  60
Lys Gln Arg Lys Gly Ser Asp Ser Val Pro Gly Lys Ile Val Thr Gly
65                  70                  75                  80
Leu Thr Ala Arg Lys Lys Lys Lys Arg Lys Ile Gln Ile Thr Asp Glu
                85                  90                  95
Ala Thr Arg Leu Glu Arg Arg Ala Arg Tyr Phe Leu Ile Lys Ile Lys
            100                 105                 110
Leu Glu Gln Asn Leu Leu Asp Ala Tyr Ser Gly Asp Gly Trp Asn Gly
        115                 120                 125
Gln Ser Arg Glu Lys Ile Lys Pro Glu Lys Glu Leu Gln Arg Ala Arg
    130                 135                 140
Lys Gln Ile Ile Lys Cys Lys Ile Ala Ile Arg Asp Ile Ile Arg Gln
145                 150                 155                 160
Leu Asp Leu Tyr Thr Ser Thr Gly Ser Val Asp Asp Pro Leu Met Pro
                165                 170                 175
Thr Asp Gln Ser Thr Asn Pro Glu His Thr Met Cys Ser Thr Cys Lys
            180                 185                 190
Ser His Glu Ser Phe Pro Ser Asn Lys Ile Ile Phe Cys Lys Gly Pro
        195                 200                 205
Cys Lys Arg Ala Cys His Glu Lys Cys Leu Glu Pro Pro Leu Asn Lys
    210                 215                 220
Ser Val Leu Pro Thr Ser Ser His Gly Trp Leu Cys Lys Phe Cys Leu
225                 230                 235                 240
Cys Lys Val Arg Ile Leu Glu Thr Ile Asn Ala His Leu Gly Thr Ser
                245                 250                 255
Phe Thr Val Lys Cys His Phe Glu Asp Ile Phe Lys Glu Thr Thr Glu
            260                 265                 270
Leu Ile Asp Ser Glu Asp Ala Leu Asp Glu Asp Trp Leu Ser Glu Tyr
        275                 280                 285
Ser Gly Asp Glu Asp Tyr Asp Pro Asp Glu Asn Glu Ala Ser Gly Asp
    290                 295                 300
Cys Met Asp Ser Gly Glu Lys Ile Met Ser Asp Asp Ser Asn Gly Ser
305                 310                 315                 320
Gly Ser Pro Leu Tyr Ser Pro Asn Asp Asp Ile Pro Asp Phe Ile Ser
                325                 330                 335
Ala Asp Leu Asn Val Val Glu Gly Phe Cys His Thr Asn Leu Asp Leu
            340                 345                 350
Gly Ile Asp Ala Val Glu Asp Asp Phe Ala Gln Ile Leu Thr Tyr Gln
        355                 360                 365
Arg Pro Arg Arg Asp Val Asp Tyr Arg Arg Leu Asn Glu Glu Met Phe
    370                 375                 380
Gly Lys Ile Thr Gly Asn Glu Glu Gln Ser Glu Asp Glu Asp Trp Gly
385                 390                 395                 400
His Glu Arg Arg Lys Lys Arg Thr His Ser Gly Val Ala Gly Asp Asn
                405                 410                 415
Ser Val Gly Phe Leu Asn Val Ile Ser Asp Glu Lys Ser Gln Lys Lys
            420                 425                 430
Gly Arg Lys Leu Phe Arg Ile Pro Pro Ala Ala Val Glu Val Leu Arg
        435                 440                 445
```

```
Arg Ala Phe Ala Glu Asn Glu Leu Pro Pro Arg Asp Val Lys Glu Asn
    450                 455                 460
Leu Ser Arg Glu Leu Gly Ile Ser Phe Glu Lys Ile Asp Lys Trp Phe
465                 470                 475                 480
Lys Asn Thr Arg Cys Ala Ala Leu Arg Asp Arg Lys Ala Glu Gly Asn
                485                 490                 495
Ser His Asn Thr Ala Pro Ser Lys Ile Ser Lys Asn Lys Gly Lys Ala
            500                 505                 510
Gly Ile Ser Gly Lys Ala Gly Arg Asn Gly His Val Thr Gly Pro Cys
        515                 520                 525
Asn Asn Leu Arg Ile Asn Glu Glu Lys Thr Gly Ile Ser Gly Lys Phe
    530                 535                 540
Asp Ser Gly Asp Asn Ser Cys Leu Val Pro Phe Ser Glu Val Ile Asn
545                 550                 555                 560
Val Pro Thr Arg Leu Pro His Asn Phe Glu Met Arg Lys Met Glu Ser
                565                 570                 575
Thr Arg Ser Pro Ala Arg Leu His Asn Lys Gly Gly Cys Leu Ser Ala
            580                 585                 590
Thr Ile Gln Ile Lys Glu Ser Thr Leu Leu Pro Thr Gly Lys Pro Cys
        595                 600                 605
Trp Gln Ser Glu Met Ser His Pro Thr Thr Asn Glu Val Ser Thr Ser
    610                 615                 620
Ala Gln Ala Thr Thr Trp Ile Asp Ala Gly Ala Cys Ala Glu Glu Gln
625                 630                 635                 640
Glu Pro Thr Pro Trp Val Asp Ile Gly Ala Ser Asp Tyr Gln Pro Phe
                645                 650                 655
Leu Asp Val Ile Asp Asp Met Cys Gly Leu Glu Trp Arg Leu Gln Arg

            660                 665                 670
Leu Lys Glu Asn Met Leu Ser Ser Ser Ile Asp Gly Gln Thr Ala Ala
        675                 680                 685
Ser Glu Ser Asp Lys Arg Asn Gln Thr Val Val Leu Val Pro Thr Ala
    690                 695                 700
Glu Leu Lys Glu Lys Leu Pro His Asp Gly Leu Phe Gly His Tyr Cys
705                 710                 715                 720
Pro
```

<210> 244
<211> 846
<212> DNA
<213> Triticum aestivum

<400> 244

```
atggcggggc agccgccgca gggcccggag gacgacttcc tcgaccagtt cttctccctc      60
accaactccc tctccgccgc cggccgcccc tccggcgacc agcccttctc cctcgccctc     120
agcctcgacg ccgcctcgga cgcctccggc agcaggggcg gcatcggcga cgacgccggc     180
aacgccgcgg agcgggacgg cgtgcagctc cccggcctct tcccgccggt gttcggcggt     240
ggcctccagc cgccgcacct tcgccccagc caccctcccc cacagatgtt ccacgcgcag     300
cagccgaagc agggcgggcc ggctggaggg ccgcagccgc cggcgccgag gccgaaggtg     360
cgggcgcgtc gcggtcaggc gactgatccc cacagcatcg cggagaggct aagaagagag     420
aggatagcgg aaaggatgag ggccctacag gaattggtcc ccaatacgaa caagacagat     480
agggcagtta tgctagatga gatcctggat tatgtgaagt tccttaggct tcaagtaaag     540
gtgttaagca tgagcagatt gggtggtgct ggtgctgttg cacagctggt ttctgacatt     600
ccactttcag ttaaggggga agcaagcgat ggtgggagca aacagcagat atgggaaaag     660
tggtcaacgg atggcacgga aaaacaggtt gcgaagctga tggacgagga catcggtgcc     720
gcaatgcaat ttctccaatc aaaggctctc tgcatgatgc cagtctccct tgccatggct     780
atctatgaca cacaacattc acaggacggc caaccagtga agcctgaacc caacaacact     840
gcctag                                                                846
```

<210> 245
<211> 281

<212> PRT
<213> Triticum aestivum

<400> 245

```
Met Ala Gly Gln Pro Pro Gln Gly Pro Glu Asp Asp Phe Leu Asp Gln
1               5                   10                  15
Phe Phe Ser Leu Thr Asn Ser Leu Ser Ala Ala Gly Arg Pro Ser Gly
            20                  25                  30
Asp Gln Pro Phe Ser Leu Ala Leu Ser Leu Asp Ala Ala Ser Asp Ala
        35                  40                  45
Ser Gly Ser Arg Gly Gly Ile Gly Asp Asp Ala Gly Asn Ala Ala Glu
    50                  55                  60
Arg Asp Gly Val Gln Leu Pro Gly Leu Phe Pro Pro Val Phe Gly Gly
65                  70                  75                  80
Gly Leu Gln Pro Pro His Leu Arg Pro Ser His Pro Pro Pro Gln Met
                85                  90                  95
Phe His Ala Gln Gln Pro Lys Gln Gly Gly Pro Ala Gly Gly Pro Gln
            100                 105                 110
Pro Pro Ala Pro Arg Pro Lys Val Arg Ala Arg Arg Gly Gln Ala Thr
        115                 120                 125
Asp Pro His Ser Ile Ala Glu Arg Leu Arg Arg Glu Arg Ile Ala Glu
    130                 135                 140
Arg Met Arg Ala Leu Gln Glu Leu Val Pro Asn Thr Asn Lys Thr Asp
145                 150                 155                 160
Arg Ala Val Met Leu Asp Glu Ile Leu Asp Tyr Val Lys Phe Leu Arg
                165                 170                 175
Leu Gln Val Lys Val Leu Ser Met Ser Arg Leu Gly Gly Ala Gly Ala
            180                 185                 190
Val Ala Gln Leu Val Ser Asp Ile Pro Leu Ser Val Lys Gly Glu Ala
        195                 200                 205
Ser Asp Gly Gly Ser Lys Gln Gln Ile Trp Glu Lys Trp Ser Thr Asp
    210                 215                 220
Gly Thr Glu Lys Gln Val Ala Lys Leu Met Asp Glu Asp Ile Gly Ala
225                 230                 235                 240
Ala Met Gln Phe Leu Gln Ser Lys Ala Leu Cys Met Met Pro Val Ser
                245                 250                 255
Leu Ala Met Ala Ile Tyr Asp Thr Gln His Ser Gln Asp Gly Gln Pro
            260                 265                 270
Val Lys Pro Glu Pro Asn Asn Thr Ala
        275                 280
```

<210> 246
<211> 6
<212> PRT
<213> Artificial sequence

<220>
<223> motif 1

<220>
<221> VARIANT
<222> (1)..(1)
<223> /replace = "Asp"

<220>
<221> VARIANT
<222> (2)..(2)
<223> /replace = "Ser" /replace = "Glu"

<220>

<221> VARIANT
<222> (3)..(3)
<223> /replace = "Met"

<220>
<221> VARIANT
<222> (4)..(4)
<223> /replace = "Phe"

<220>
<221> VARIANT
<222> (5)..(5)
<223> /replace = "Glu" /replace = "Gln" /replace = "Leu"

<220>
<221> VARIANT
<222> (6)..(6)
<223> /replace = "His" /replace = "Glu"

<400> 246

```
Glu Asp Phe Leu Asp Gln
1               5
```

<210> 247
<211> 16
<212> PRT
<213> Artificial sequence

<220>
<223> motif 2

<220>
<221> VARIANT
<222> (3)..(3)
<223> /replace = "Ile" /replace = "Gln"

<220>
<221> VARIANT
<222> (6)..(6)
<223> /replace = "His"

<400> 247

```
Arg Ala Arg Arg Gly Gln Ala Thr Asp Pro His Ser Ile Ala Glu Arg
1               5                   10                  15
```

<210> 248
<211> 9
<212> PRT
<213> Artificial sequence

<220>
<223> motif 3

<220>
<221> VARIANT
<222> (1)..(1)

<223> /replace = "Ile" /replace = "Val" /replace = "Leu"

<220>
<221> VARIANT
<222> (2)..(2)
<223> /replace = "Arg"

<220>
<221> VARIANT
<222> (3)..(3)
<223> /replace = "Ser" /replace = "Gln" /replace = "Asp" /replace = "Asn"

<220>
<221> VARIANT
<222> (6)..(6)
<223> /replace = "Asp" /replace = "Val"

<400> 248

```
Met Lys Ala Leu Gln Glu Leu Val Pro
1               5
```

<210> 249
<211> 17
<212> PRT
<213> Artificial sequence

<220>
<223> motif 4

<220>
<221> VARIANT
<222> (1)..(1)
<223> /replace = "Ile"

<220>
<221> VARIANT
<222> (2)..(2)
<223> /replace = "Ile"

<220>
<221> VARIANT
<222> (6)..(6)
<223> /replace = "Val" /replace = "Leu"

<220>
<221> VARIANT
<222> (7)..(7)
<223> /replace = "Glu" /replace = "Gly"

<220>
<221> VARIANT
<222> (9)..(9)
<223> /replace = "Leu" /replace = "Ile"

<220>
<221> VARIANT
<222> (10)..(10)

<223> /replace = "Arg"

<220>
<221> VARIANT
<222> (13)..(13)
<223> /replace = "Arg"

<220>
<221> VARIANT
<222> (16)..(16)
<223> /replace = "Ile"

<400> 249

```
Met Leu Asp Glu Ile Ile Asp Tyr Val Lys Phe Leu Gln Leu Gln Val
1               5                   10                  15
Lys
```

<210> 250
<211> 8
<212> PRT
<213> Artificial sequence

<220>
<223> motif 5

<220>
<221> VARIANT
<222> (1)..(1)
<223> /replace = "Ile"

<220>
<221> VARIANT
<222> (7)..(7)
<223> /replace = "Val"

<400> 250

```
Val Leu Ser Met Ser Arg Leu Gly
1               5
```

<210> 251
<211> 16
<212> PRT
<213> Artificial sequence

<220>
<223> motif 6

<220>
<221> VARIANT
<222> (2)..(2)
<223> /replace = "Val" /replace = "Leu" /replace = "Ile"

<220>
<221> VARIANT
<222> (3)..(3)
<223> /replace = "Arg"

<220>
<221> VARIANT
<222> (4)..(4)
<223> /replace = "Met"

<220>
<221> VARIANT
<222> (5)..(5)
<223> /replace = "Leu"

<220>
<221> VARIANT
<222> (6)..(6)
<223> /replace = "Asp"

<220>
<221> VARIANT
<222> (7)..(7)
<223> /replace = "Asp" /replace = "Ser" /replace = "Lys" /replace = "Thr"

<220>
<221> VARIANT
<222> (8)..(8)
<223> /replace = "Asn" /replace = "Ser"

<220>
<221> VARIANT
<222> (9)..(9)
<223> /replace = "Val" /replace = "Ile"

<220>
<221> VARIANT
<222> (10)..(10)
<223> /replace = "Val" /replace = "Ile"

<220>
<221> UNSURE
<222> (11)..(11)
<223> Xaa is an unknown amino acid

<220>
<221> VARIANT
<222> (15)..(15)
<223> /replace = "Leu" /replace = "Phe"

<400> 251

```
Val Ala Lys Leu Met Glu Glu Asp Met Gly Xaa Ala Met Gln Tyr Leu
1               5                   10                  15
```

<210> 252
<211> 6
<212> PRT
<213> Artificial sequence

<220>
<223> motif 7

<220>
<221> VARIANT
<222> (1)..(1)
<223> /replace = "Val"

<220>
<221> VARIANT
<222> (2)..(2)
<223> /replace = "Ser"

<220>
<221> VARIANT
<222> (3)..(3)
<223> /replace = "Val"

<220>
<221> VARIANT
<222> (4)..(4)
<223> /replace = "Thr" /replace = "Ala"

<400> 252
Met Pro Ile Ser Leu Ala

1 5

<210> 253
<211> 51
<212> PRT
<213> Artificial sequence

<220>
<223> motif 8

<400> 253

```
Ser Ile Ala Glu Arg Leu Arg Arg Glu Arg Ile Ala Glu Arg Met Arg
1               5                   10                  15
Ala Leu Gln Glu Leu Val Pro Asn Thr Asn Lys Thr Asp Arg Ala Val
            20                  25                  30
Met Leu Asp Glu Ile Leu Asp Tyr Val Lys Phe Leu Arg Leu Gln Val
        35                  40                  45
Lys Val Leu
    50
```

<210> 254
<211> 50
<212> DNA
<213> Artificial sequence

<220>
<223> primer: prm009718

<220>
<221> misc feature
<222> (47)..(47)
<223> n is a, c, g, or t

<400> 254

ggggacaagt ttgtacaaaa aagcaggctt aaacaatggc ggggcanccg 50

<210> 255
<211> 50
<212> DNA
<213> Artificial sequence

<220>
<223> primer: prm009719

<400> 255
ggggaccact ttgtacaaga aagctgggta tgggtgttgc agctgctgtt 50

<210> 256
<211> 2194
<212> DNA
<213> Oryza sativa

<400> 256

```
aatccgaaaa gtttctgcac cgttttcacc ccctaactaa caatataggg aacgtgtgct     60
aaatataaaa tgagacctta tatatgtagc gctgataact agaactatgc aagaaaaact    120
catccaccta ctttagtggc aatcgggcta aataaaaaag agtcgctaca ctagtttcgt    180
tttccttagt aattaagtgg gaaaatgaaa tcattattgc ttagaatata cgttcacatc    240
tctgtcatga agttaaatta ttcgaggtag ccataattgt catcaaactc ttcttgaata    300
aaaaaatctt tctagctgaa ctcaatgggt aaagagagag attttttta aaaaaataga    360
atgaagatat tctgaacgta ttggcaaaga tttaaacata taattatata attttatagt    420
ttgtgcattc gtcatatcgc acatcattaa ggacatgtct tactccatcc caatttttat    480
ttagtaatta aagacaattg acttattttt attatttatc ttttttcgat tagatgcaag    540
gtacttacgc acacactttg tgctcatgtg catgtgtgag tgcacctcct caatacacgt    600
tcaactagca acacatctct aatatcactc gcctatttaa tacatttagg tagcaatatc    660
tgaattcaag cactccacca tcaccagacc acttttaata atatctaaaa tacaaaaaat    720
aattttacag aatagcatga aaagtatgaa acgaactatt taggtttttc acatacaaaa    780
aaaaaaagaa ttttgctcgt gcgcgagcgc caatctccca tattgggcac acaggcaaca    840
acagagtggc tgcccacaga acaacccaca aaaaacgatg atctaacgga ggacagcaag    900
tccgcaacaa ccttttaaca gcaggctttg cggccaggag agaggaggag aggcaaagaa    960
aaccaagcat cctccttctc ccatctataa attcctcccc ccttttcccc tctctatata   1020
ggaggcatcc aagccaagaa gagggagagc accaaggaca cgcgactagc agaagccgag   1080
cgaccgcctt ctcgatccat atcttccggt cgagttcttg gtcgatctct tccctcctcc   1140
acctcctcct cacagggtat gtgcctccct tcggttgttc ttggatttat tgttctaggt   1200
tgtgtagtac gggcgttgat gttaggaaag gggatctgta tctgtgatga ttcctgttct   1260
tggatttggg atagaggggt tcttgatgtt gcatgttatc ggttcggttt gattagtagt   1320
atggttttca atcgtctgga gagctctatg gaaatgaaat ggtttaggga tcggaatctt   1380
gcgattttgt gagtaccttt tgtttgaggt aaaatcagag caccggtgat tttgcttggt   1440
gtaataaagt acggttgttt ggtcctcgat tctggtagtg atgcttctcg atttgacgaa   1500
gctatccttt gtttattccc tattgaacaa aaataatcca actttgaaga cggtcccgtt   1560
gatgagattg aatgattgat tcttaagcct gtccaaaatt tcgcagctgg cttgtttaga   1620
tacagtagtc cccatcacga aattcatgga aacagttata atcctcagga acaggggatt   1680
ccctgttctt ccgatttgct ttagtcccag aatttttttt cccaaatatc ttaaaaagtc   1740
actttctggt tcagttcaat gaattgattg ctacaaataa tgcttttata gcgttatcct   1800
agctgtagtt cagttaatag gtaatacccc tatagtttag tcaggagaag aacttatccg   1860
atttctgatc tccattttta attatatgaa atgaactgta gcataagcag tattcatttg   1920
gattattttt tttattagct ctcacccctt cattattctg agctgaaagt ctggcatgaa   1980
ctgtcctcaa ttttgtttc aaattcacat cgattatcta tgcattatcc tcttgtatct   2040
acctgtagaa gtttcttttt ggttattcct tgactgcttg attacagaaa gaaatttatg   2100
aagctgtaat cgggatagtt atactgcttg ttcttatgat tcatttcctt tgtgcagttc   2160
ttggtgtagc ttgccacttt caccagcaaa gttc                                2194
```

<210> 257
<211> 3093
<212> DNA

<213> Artificial sequence

<220>
<223> expression cassette

<400> 257

```
aatccgaaaa gtttctgcac cgttttcacc ccctaactaa caatataggg aacgtgtgct      60
aaatataaaa tgagacctta tatatgtagc gctgataact agaactatgc aagaaaaact     120
catccaccta ctttagtggc aatcgggcta aataaaaaag agtcgctaca ctagtttcgt     180
tttccttagt aattaagtgg gaaaatgaaa tcattattgc ttagaatata cgttcacatc     240
tctgtcatga agttaaatta ttcgaggtag ccataattgt catcaaactc ttcttgaata     300
aaaaaatctt tctagctgaa ctcaatgggt aaagagagag attttttta aaaaaataga      360
atgaagatat tctgaacgta ttggcaaaga tttaaacata taattatata attttatagt     420
ttgtgcattc gtcatatcgc acatcattaa ggacatgtct tactccatcc caatttttat     480
ttagtaatta aagacaattg acttattttt attatttatc ttttttcgat tagatgcaag     540
gtacttacgc acacactttg tgctcatgtg catgtgtgag tgcacctcct caatacacgt     600

tcaactagca acacatctct aatatcactc gcctatttaa tacatttagg tagcaatatc     660
tgaattcaag cactccacca tcaccagacc acttttaata atatctaaaa tacaaaaaat     720
aattttacag aatagcatga aaagtatgaa acgaactatt taggtttttc acatacaaaa     780
aaaaaaagaa ttttgctcgt gcgcgagcgc caatctccca tattgggcac acaggcaaca     840
acagagtggc tgcccacaga acaacccaca aaaaacgatg atctaacgga ggacagcaag     900
tccgcaacaa ccttttaaca gcaggctttg cggccaggag agaggaggag aggcaaagaa     960
aaccaagcat cctccttctc ccatctataa attcctcccc ccttttcccc tctctatata    1020
ggaggcatcc aagccaagaa gagggagagc accaaggaca cgcgactagc agaagccgag    1080
cgaccgcctt ctcgatccat atcttccggt cgagttcttg gtcgatctct tccctcctcc    1140
acctcctcct cacagggtat gtgcctccct tcggttgttc ttggatttat tgttctaggt    1200
tgtgtagtac gggcgttgat gttaggaaag gggatctgta tctgtgatga ttcctgttct    1260
tggatttggg atagaggggt tcttgatgtt gcatgttatc ggttcggttt gattagtagt    1320
atggttttca atcgtctgga gagctctatg gaaatgaaat ggtttaggga tcggaatctt    1380
gcgattttgt gagtaccttt tgtttgaggt aaaatcagag caccggtgat tttgcttggt    1440
gtaataaagt acggttgttt ggtcctcgat tctggtagtg atgcttctcg atttgacgaa    1500
gctatccttt gtttattccc tattgaacaa aaataatcca actttgaaga cggtcccgtt    1560
gatgagattg aatgattgat tcttaagcct gtccaaaatt tcgcagctgg cttgtttaga    1620
tacagtagtc cccatcacga aattcatgga aacagttata atcctcagga acaggggatt    1680
ccctgttctt ccgatttgct ttagtcccag aattttttt cccaaatatc ttaaaaagtc     1740
actttctggt tcagttcaat gaattgattg ctacaaataa tgcttttata gcgttatcct    1800
agctgtagtt cagttaatag gtaatacccc tatagtttag tcaggagaag aacttatccg    1860
atttctgatc tccattttta attatatgaa atgaactgta gcataagcag tattcatttg    1920
gattattttt tttattagct ctcacccctt cattattctg agctgaaagt ctggcatgaa    1980
ctgtcctcaa ttttgttttc aaattcacat cgattatcta tgcattatcc tcttgtatct    2040
acctgtagaa gtttcttttt ggttattcct tgactgcttg attacagaaa gaaatttatg    2100
aagctgtaat cgggatagtt atactgcttg ttcttatgat tcatttcctt tgtgcagttc    2160
ttggtgtagc ttgccacttt caccagcaaa gttcatttaa atcaactagg gatatcacaa    2220
gtttgtacaa aaaagcaggc ttaaacaatg gcggggcagc cgccgcaggg cccggaggac    2280
gacttcctcg accagttctt ctccctcacc aactccctct ccgccgccgg ccgcccctcc    2340
ggcgaccagc ccttctccct cgccctcagc ctcgacgccg cctcggacgc ctccggcagc    2400
aggggcggca tcggcgacga cgccggcaac gccgcggagc gggacggcgt gcagctcccc    2460
ggcctcttcc cgccggtgtt cggcggtggc ctccagccgc cgcaccttcg ccccagccac    2520
cctcccccac agatgttcca cgcgcagcag ccgaagcagg gcgggccggc tggagggccg    2580
cagccgccgg cgccgaggcc gaaggtgcgg gcgcgtcgcg gtcaggcgac tgatccccac    2640
agcatcgcgg agaggctaag aagagagagg atagcggaaa ggatgagggc cctacaggaa    2700
ttggtcccca atacgaacaa gacagatagg gcagttatgc tagatgagat cctggattat    2760
gtgaagttcc ttaggcttca agtaaaggtg ttaagcatga gcagattggg tggtgctggt    2820
gctgttgcac agctggtttc tgacattcca ctttcagtta agggggaagc aagcgatggt    2880
gggagcaaac agcagatatg ggaaaagtgg tcaacggatg gcacggaaaa acaggttgcg    2940
aagctgatgg acgaggacat cggtgccgca atgcaatttc tccaatcaaa ggctctctgc    3000
atgatgccag tctcccttgc catggctatc tatgacacac aacattcaca ggacggccaa    3060
ccagtgaagc ctgaacccaa caacactgcc tag                                 3093
```

<210> 258
<211> 285
<212> PRT
<213> Allium cepa

<400> 258

```
Met Ala Ser Asn Asn Asn Pro Gln Pro Pro Ser Asp Gly Leu Asn Asp
1               5                   10                  15
Asp Phe Phe Asp Gln Ile Phe Ser Met Pro Ser Ala Phe Ala Ala Ser
            20                  25                  30
Ser Ala Ser Thr Asp Ala Thr Glu Ala Leu Asn Tyr Ala Asp Ser Ser
        35                  40                  45
Thr Thr Gly Val Thr Pro Met Phe Ser Leu Gly Leu Ser Leu Asp Gln
    50                  55                  60
Gln Pro Lys Ser Asp Ser Ser Gln Ser Glu Arg Glu His Pro Val Gln
65                  70                  75                  80
Phe Ala Cys Leu Phe Pro Gln Tyr Gly His Asn Asn Gln Val His Gln


                85                  90                  95
Thr Arg Pro Asn Leu Ser Pro Pro Gln Val Leu His Gly Gln Ala Met
            100                 105                 110
Gln Ser Ser Met Ala Ala Thr Pro Gln Pro Thr Gly Pro Arg Pro Arg
        115                 120                 125
Val Arg Ala Arg Arg Gly Gln Ala Thr Asp Pro His Ser Ile Ala Glu
    130                 135                 140
Arg Leu Arg Arg Glu Arg Ile Ala Glu Arg Ile Arg Ala Leu Gln Glu
145                 150                 155                 160
Leu Val Pro Ser Thr Asn Lys Thr Asp Arg Ala Val Met Leu Asp Glu
                165                 170                 175
Ile Val Glu Tyr Val Lys Phe Leu Arg Leu Gln Val Lys Val Leu Ser
            180                 185                 190
Met Ser Arg Leu Gly Gly Ala Gly Ala Val Ala Gln Leu Val Ala Asp
        195                 200                 205
Ile Pro Met Ser Ser Ser Val Glu Gly Asp Ser Ser Glu Ser Gly Lys
    210                 215                 220
Thr Ser Tyr Gln Gly Trp Glu Lys Trp Ser Thr Asp Gly Thr Glu Arg
225                 230                 235                 240
Gln Val Ala Lys Leu Met Glu Glu Asp Val Gly Ala Ala Met Gln Phe
                245                 250                 255
Leu Gln Ser Lys Ala Leu Cys Ile Met Pro Ile Ser Leu Ala Ala Ala
            260                 265                 270
Ile Cys Gln Thr Asn Pro Ser Thr Glu Ile Ser Phe Met
        275                 280                 285
```

<210> 259
<211> 302
<212> PRT
<213> Arabidopsis thaliana

<400> 259

```
Met Ala Asn Asn Asn Asn Ile Pro His Asp Ser Ile Ser Asp Pro Ser
1               5                   10                  15
Pro Thr Asp Asp Phe Phe Glu Gln Ile Leu Gly Leu Ser Asn Phe Ser
            20                  25                  30
Gly Ser Ser Gly Ser Gly Leu Ser Gly Ile Gly Gly Val Gly Pro Pro
        35                  40                  45
Pro Met Met Leu Gln Leu Gly Ser Gly Asn Glu Gly Asn His Asn His
    50                  55                  60
Met Gly Ala Ile Gly Gly Gly Gly Pro Val Gly Phe His Asn Gln Met
65                  70                  75                  80
Phe Pro Leu Gly Leu Ser Leu Asp Gln Gly Lys Gly His Gly Phe Leu
                85                  90                  95
Lys Pro Asp Glu Thr Gly Lys Arg Phe Gln Asp Asp Val Leu Asp Asn
            100                 105                 110
Arg Cys Ser Ser Met Lys Pro Ile Phe His Gly Gln Pro Met Ser Gln
        115                 120                 125
Pro Ala Pro Pro Met Pro His Gln Gln Ser Thr Ile Arg Pro Arg Val
    130                 135                 140
Arg Ala Arg Arg Gly Gln Ala Thr Asp Pro His Ser Ile Ala Glu Arg
145                 150                 155                 160
Leu Arg Arg Glu Arg Ile Ala Glu Arg Ile Arg Ser Leu Gln Glu Leu
                165                 170                 175
Val Pro Thr Val Asn Lys Thr Asp Arg Ala Ala Met Ile Asp Glu Ile
            180                 185                 190
Val Asp Tyr Val Lys Phe Leu Arg Leu Gln Val Lys Val Leu Ser Met
        195                 200                 205
Ser Arg Leu Gly Gly Ala Gly Ala Val Ala Pro Leu Val Thr Glu Met
    210                 215                 220
Pro Leu Ser Ser Ser Val Glu Asp Glu Thr Gln Ala Val Trp Glu Lys


225                 230                 235                 240
Trp Ser Asn Asp Gly Thr Glu Arg Gln Val Ala Lys Leu Met Glu Glu
                245                 250                 255
Asn Val Gly Ala Ala Met Gln Leu Leu Gln Ser Lys Ala Leu Cys Ile
            260                 265                 270
Met Pro Ile Ser Leu Ala Met Ala Ile Tyr His Ser Gln Pro Pro Asp
        275                 280                 285
Thr Ser Ser Ser Ile Val Lys Pro Glu Met Asn Pro Pro Pro
    290                 295                 300
```

<210> 260
<211> 310
<212> PRT
<213> Arabidopsis thaliana

<400> 260

```
Met Ala Ser Asn Asn Pro His Asp Asn Leu Ser Asp Gln Thr Pro Ser
1               5                   10                  15
Asp Asp Phe Phe Glu Gln Ile Leu Gly Leu Pro Asn Phe Ser Ala Ser
            20                  25                  30
Ser Ala Ala Gly Leu Ser Gly Val Asp Gly Gly Leu Gly Gly Gly Ala
        35                  40                  45
Pro Pro Met Met Leu Gln Leu Gly Ser Gly Glu Glu Gly Ser His Met
    50                  55                  60
Gly Gly Leu Gly Gly Ser Gly Pro Thr Gly Phe His Asn Gln Met Phe
65                  70                  75                  80
Pro Leu Gly Leu Ser Leu Asp Gln Gly Lys Gly Pro Gly Phe Leu Arg
                85                  90                  95
Pro Glu Gly Gly His Gly Ser Gly Lys Arg Phe Ser Asp Asp Val Val
            100                 105                 110
Asp Asn Arg Cys Ser Ser Met Lys Pro Val Phe His Gly Gln Pro Met
        115                 120                 125
Gln Gln Pro Pro Pro Ser Ala Pro His Gln Pro Thr Ser Ile Arg Pro
    130                 135                 140
Arg Val Arg Ala Arg Arg Gly Gln Ala Thr Asp Pro His Ser Ile Ala
145                 150                 155                 160
Glu Arg Leu Arg Arg Glu Arg Ile Ala Glu Arg Ile Arg Ala Leu Gln
                165                 170                 175
Glu Leu Val Pro Thr Val Asn Lys Thr Asp Arg Ala Ala Met Ile Asp
            180                 185                 190
Glu Ile Val Asp Tyr Val Lys Phe Leu Arg Leu Gln Val Lys Val Leu
        195                 200                 205
Ser Met Ser Arg Leu Gly Gly Ala Gly Ala Val Ala Pro Leu Val Thr
    210                 215                 220
Asp Met Pro Leu Ser Ser Ser Val Glu Asp Glu Thr Gly Glu Gly Gly
225                 230                 235                 240
Arg Thr Pro Gln Pro Ala Trp Glu Lys Trp Ser Asn Asp Gly Thr Glu
                245                 250                 255
Arg Gln Val Ala Lys Leu Met Glu Glu Asn Val Gly Ala Ala Met Gln
            260                 265                 270
Leu Leu Gln Ser Lys Ala Leu Cys Met Met Pro Ile Ser Leu Ala Met
        275                 280                 285
Ala Ile Tyr His Ser Gln Pro Pro Asp Thr Ser Ser Val Val Lys Pro
    290                 295                 300
Glu Asn Asn Pro Pro Gln
305                 310
```

<210> 261
<211> 831
<212> PRT
<213> Arabidopsis thaliana

<400> 261

```
Met Met Asn Ser Ser Leu Leu Thr Pro Ser Ser Ser Ser Ser Ser His
1               5                   10                  15
Ile Gln Thr Pro Ser Thr Thr Phe Asp His Glu Asp Phe Leu Asp Gln
            20                  25                  30
Ile Phe Ser Ser Ala Pro Trp Pro Ser Val Val Asp Asp Ala His Pro
        35                  40                  45
Leu Pro Ser Asp Gly Phe His Gly His Asp Val Asp Ser Arg Asn Gln
    50                  55                  60
Pro Ile Met Met Met Pro Leu Asn Asp Gly Ser Ser Val His Ala Leu
65                  70                  75                  80
Tyr Asn Gly Phe Ser Val Ala Gly Ser Leu Pro Asn Phe Gln Ile Pro
                85                  90                  95
Gln Gly Ser Gly Gly Gly Leu Met Asn Gln Gln Gly Gln Thr Gln Thr
            100                 105                 110
Gln Thr Gln Pro Gln Ala Ser Ala Ser Thr Ala Thr Gly Gly Thr Val
        115                 120                 125
Ala Ala Pro Pro Gln Ser Arg Thr Lys Ile Arg Ala Arg Arg Gly Gln
    130                 135                 140
Ala Thr Asp Pro His Ser Ile Ala Glu Arg Leu Arg Arg Glu Arg Ile
145                 150                 155                 160
Ala Glu Arg Met Lys Ala Leu Gln Glu Leu Val Pro Asn Gly Asn Lys
                165                 170                 175
Thr Asp Lys Ala Ser Met Leu Asp Glu Ile Ile Asp Tyr Val Lys Phe
            180                 185                 190
Leu Gln Leu Gln Val Lys Val Leu Ser Met Ser Arg Leu Gly Gly Ala
        195                 200                 205
Ala Ser Val Ser Ser Gln Ile Ser Glu Ala Gly Gly Ser His Gly Asn
    210                 215                 220
Ala Ser Ser Ala Met Val Gly Gly Ser Gln Thr Ala Gly Asn Ser Asn
225                 230                 235                 240
Asp Ser Val Thr Met Thr Glu His Gln Val Ala Lys Leu Met Glu Glu
                245                 250                 255
Asp Met Gly Ser Ala Met Gln Tyr Leu Gln Gly Lys Gly Leu Cys Leu
            260                 265                 270
Met Pro Ile Ser Leu Ala Thr Ala Ile Ser Thr Ala Thr Cys His Ser
        275                 280                 285
Arg Asn Pro Leu Ile Pro Gly Ala Val Ala Asp Val Gly Gly Pro Ser
    290                 295                 300
Pro Pro Asn Leu Ser Gly Met Thr Ile Gln Ser Thr Ser Thr Lys Met
305                 310                 315                 320
Gly Ser Gly Asn Gly Lys Leu Asn Gly Asn Gly Val Thr Glu Arg Ser
                325                 330                 335
Ser Ser Ile Ala Val Lys Glu Ala Val Ser Val Ser Lys Ala Ala Thr
            340                 345                 350
Gly Met Gln Phe Asp Glu Ser Asp Ala Arg Val Trp Ile Trp Phe Glu
        355                 360                 365
Ile Arg Arg Glu Asp Asp Ile Val Glu Leu Leu Trp Gln Ser Gly Gln
    370                 375                 380
Val Val Gly Thr Asn Gln Thr His Arg Gln Ser Tyr Asp Pro Pro Pro
385                 390                 395                 400
Ile Leu Arg Gly Ser Gly Ser Gly Arg Gly Glu Glu Asn Ala Pro Leu
                405                 410                 415
Ser Gln Pro Pro Pro His Leu His Gln Gln Asn Leu Phe Ile Gln Glu
            420                 425                 430
Gly Glu Met Tyr Ser Trp Leu His His Ser Tyr Arg Gln Asn Tyr Phe
        435                 440                 445
Cys Ser Glu Leu Leu Asn Ser Thr Pro Ala Thr His Pro Gln Ser Ser
    450                 455                 460
Ile Ser Leu Ala Pro Arg Gln Thr Ile Ala Thr Arg Arg Ala Glu Asn
```

465 470 475 480
Phe Met Asn Phe Ser Trp Leu Arg Gly Asn Ile Phe Thr Gly Gly Arg
485 490 495
Val Asp Glu Ala Gly Pro Ser Phe Ser Val Val Arg Glu Ser Met Gln
500 505 510
Val Gly Ser Asn Thr Thr Pro Pro Ser Ser Ser Ala Thr Glu Ser Cys
515 520 525
Val Ile Pro Ala Thr Glu Gly Thr Ala Ser Arg Val Ser Gly Thr Leu
530 535 540
Ala Ala His Asp Leu Gly Arg Lys Gly Lys Ala Val Ala Val Glu Ala
545 550 555 560
Ala Gly Thr Pro Ser Ser Gly Val Cys Lys Ala Glu Thr Glu Pro Val
565 570 575
Gln Ile Gln Pro Ala Thr Glu Ser Lys Leu Lys Ala Arg Glu Glu Thr
580 585 590
His Gly Thr Glu Glu Ala Arg Gly Ser Thr Ser Arg Lys Arg Ser Arg
595 600 605
Thr Ala Glu Met His Asn Leu Ala Glu Arg Arg Arg Arg Glu Lys Ile
610 615 620
Asn Glu Lys Met Lys Thr Leu Gln Gln Leu Ile Pro Arg Cys Asn Lys
625 630 635 640
Val Glu Ser Asp Ser Val Ser Thr Leu Ile Ser Leu Leu Lys Phe Gln
645 650 655
Arg Trp Met Met Leu Ser Ser Thr Ser Asn Arg Tyr Arg Ala Lys Tyr
660 665 670
Lys Tyr Ala Leu Gln Asn Arg Met Cys Phe Lys Pro Met Val Gln His
675 680 685
Gly Lys Ser Ser Tyr Val Ser Ser Phe Val Glu Met Met Ser Thr Gly
690 695 700
Gln Gly Met Met Ser Pro Met Met Asn Ala Gly Asn Thr Gln Gln Phe
705 710 715 720
Met Pro His Met Ala Met Asp Met Asn Arg Pro Pro Pro Phe Ile Pro
725 730 735
Phe Pro Gly Thr Ser Phe Pro Met Pro Ala Gln Met Ala Gly Val Gly
740 745 750
Pro Ser Tyr Pro Ala Pro Arg Tyr Pro Phe Pro Asn Ile Gln Thr Phe
755 760 765
Asp Pro Ser Arg Val Arg Leu Pro Ser Pro Gln Pro Asn Pro Val Ser
770 775 780
Asn Gln Pro Gln Phe Pro Ala Tyr Met Asn Pro Tyr Ser Gln Phe Ala
785 790 795 800
Gly Pro His Gln Leu Gln Gln Pro Pro Pro Pro Pro Phe Gln Val Thr
805 810 815
Leu Tyr His Gly Ile His Cys Val Val Ala Gly Asn Pro Tyr Val
820 825 830

<210> 262
<211> 310
<212> PRT
<213> Arabidopsis thaliana

<400> 262

```
Met Asn Ser Ser Ser Leu Leu Thr Pro Ser Ser Ser Pro Ser Pro His
1               5                   10                  15
Leu Gln Ser Pro Ala Thr Phe Asp His Asp Asp Phe Leu His His Ile
            20                  25                  30
Phe Ser Ser Thr Pro Trp Pro Ser Ser Val Leu Asp Asp Thr Pro Pro
        35                  40                  45
Pro Thr Ser Asp Cys Ala Pro Val Thr Gly Phe His His His Asp Ala
    50                  55                  60
Asp Ser Arg Asn Gln Ile Thr Met Ile Pro Leu Ser His Asn His Pro
65                  70                  75                  80
Asn Asp Ala Leu Phe Asn Gly Phe Ser Thr Gly Ser Leu Pro Phe His
                85                  90                  95
Leu Pro Gln Gly Ser Gly Gly Gln Thr Gln Thr Gln Ser Gln Ala Thr
            100                 105                 110
Ala Ser Ala Thr Thr Gly Gly Ala Thr Ala Gln Pro Gln Thr Lys Pro
        115                 120                 125
Lys Val Arg Ala Arg Arg Gly Gln Ala Thr Asp Pro His Ser Ile Ala
    130                 135                 140
Glu Arg Leu Arg Arg Glu Arg Ile Ala Glu Arg Met Lys Ser Leu Gln
145                 150                 155                 160
Glu Leu Val Pro Asn Gly Asn Lys Thr Asp Lys Ala Ser Met Leu Asp
                165                 170                 175
Glu Ile Ile Asp Tyr Val Lys Phe Leu Gln Leu Gln Val Lys Val Leu
            180                 185                 190
Ser Met Ser Arg Leu Gly Gly Ala Ala Ser Ala Ser Ser Gln Ile Ser
        195                 200                 205
Glu Asp Ala Gly Gly Ser His Glu Asn Thr Ser Ser Ser Gly Glu Ala
    210                 215                 220
Lys Met Thr Glu His Gln Val Ala Lys Leu Met Glu Glu Asp Met Gly
225                 230                 235                 240
Ser Ala Met Gln Tyr Leu Gln Gly Lys Gly Leu Cys Leu Met Pro Ile
                245                 250                 255
Ser Leu Ala Thr Thr Ile Ser Thr Ala Thr Cys Pro Ser Arg Ser Pro
            260                 265                 270
Phe Val Lys Asp Thr Gly Val Pro Leu Ser Pro Asn Leu Ser Thr Thr
        275                 280                 285
Ile Val Ala Asn Gly Asn Gly Ser Ser Leu Val Thr Val Lys Asp Ala
    290                 295                 300
Pro Ser Val Ser Lys Pro
305                 310
```

<210> 263
<211> 297
<212> PRT
<213> Arabidopsis thaliana

<400> 263

Met Glu Asn Gly Asn Gly Glu Gly Lys Gly Glu Phe Ile Asn Gln Asn
1               5                   10                  15
Asn Asp Phe Phe Leu Asp Ser Met Ser Met Leu Ser Ser Leu Pro Pro
            20                  25                  30
Cys Trp Asp Pro Ser Leu Pro Pro Pro Pro Pro Pro Pro Gln Ser Leu
        35                  40                  45
Phe His Ala Leu Ala Val Asp Ala Pro Phe Pro Asp Gln Phe His His
    50                  55                  60
Pro Gln Glu Ser Gly Gly Pro Thr Met Gly Ser Gln Glu Gly Leu Gln
65                  70                  75                  80
Pro Gln Gly Thr Val Ser Thr Thr Ser Ala Pro Val Val Arg Gln Lys
                85                  90                  95
Pro Arg Val Arg Ala Arg Arg Gly Gln Ala Thr Asp Pro His Ser Ile
            100                 105                 110
Ala Glu Arg Leu Arg Arg Glu Arg Ile Ala Glu Arg Met Lys Ser Leu
        115                 120                 125
Gln Glu Leu Val Pro Asn Thr Asn Lys Thr Asp Lys Ala Ser Met Leu
    130                 135                 140
Asp Glu Ile Ile Glu Tyr Val Arg Phe Leu Gln Leu Gln Val Lys Val
145                 150                 155                 160
Leu Ser Met Ser Arg Leu Gly Gly Ala Gly Ser Val Gly Pro Arg Leu
                165                 170                 175
Asn Gly Leu Ser Ala Glu Ala Gly Gly Arg Leu Asn Ala Leu Thr Ala
            180                 185                 190
Pro Cys Asn Gly Leu Asn Gly Asn Gly Asn Ala Thr Gly Ser Ser Asn
        195                 200                 205
Glu Ser Leu Arg Ser Thr Glu Gln Arg Val Ala Lys Leu Met Glu Glu
    210                 215                 220
Asp Met Gly Ser Ala Met Gln Tyr Leu Gln Gly Lys Gly Leu Cys Leu
225                 230                 235                 240
Met Pro Ile Ser Leu Ala Thr Ala Ile Ser Ser Ser Thr Thr His Ser
                245                 250                 255
Arg Gly Ser Leu Phe Asn Pro Ile Ser Ser Ala Val Ala Ala Glu Asp
            260                 265                 270
Ser Asn Val Thr Ala Thr Ala Val Ala Ala Pro Glu Ala Ser Ser Thr
        275                 280                 285
Met Asp Asp Val Ser Ala Ser Lys Ala
    290                 295

<210> 264
<211> 351
<212> PRT
<213> Aquilegia vulgaris

<400> 264

Met Ala Asn Asn Asn Asn Pro Thr Glu Gly Leu Thr Asp Asp Phe Leu
1               5                   10                  15
Asp Gln Ile Leu Ser Tyr Gly Ala His Glu Gly Asn Leu Ala Gly Asn
            20                  25                  30
Asp Val Asn Leu Ala Gly Thr Thr Thr Pro Gly Gly Ser Met Ala Leu
        35                  40                  45
Gln Leu Ser Ser Ser Gly Gly Asp Ile Thr Gly Ser Gly Gly Tyr His
    50                  55                  60
His His His Gln His Gln His His His Gln Leu Ser Gly Gly Gly Gly
65                  70                  75                  80
Gly Phe Pro Leu Gly Leu Ser Leu Glu Gln Ser Gly Lys Gly Gly Phe
                85                  90                  95
Phe Lys Pro Asp Glu Ala Ser Gly Ser Gly Lys Arg Phe Arg Asp Asp
            100                 105                 110
Phe Val Asp Leu Lys Ala Val Ser Ser Thr Asn Asn Asp Asn Asn Asp
        115                 120                 125
Arg Gly Asp Ser Val His Leu Asn Asn Leu Phe Pro Ala Phe Gly His
    130                 135                 140
Val Gln Gln Gln Gln Gln Ala His Ser Val Arg Pro Pro Ser His Gln
145                 150                 155                 160
Asn Gln Ile Asn Gln Pro Phe Leu Gly His Pro Thr Pro Gly Ala Val
                165                 170                 175
Ala Val Val Pro His Pro Pro Ala Ile Arg Pro Arg Val Arg Ala Arg
            180                 185                 190
Arg Gly Gln Ala Thr Asp Pro His Ser Ile Ala Glu Arg Leu Arg Arg
        195                 200                 205
Glu Arg Ile Ala Glu Arg Met Lys Ala Leu Gln Glu Leu Val Pro Ser
    210                 215                 220
Ser Asn Lys Thr Asp Arg Ala Ala Met Leu Asp Glu Ile Val Asp Tyr
225                 230                 235                 240
Val Lys Phe Leu Arg Leu Gln Val Lys Val Leu Ser Met Ser Arg Leu
                245                 250                 255
Gly Gly Ala Gly Ala Val Ala Gln Leu Val Ala Asp Ile Pro Leu Ser
            260                 265                 270
Ser Ala Glu Gly Gly Ala Ser Glu Gly Gly Ser Asn Gln Pro Ala Trp
        275                 280                 285
Glu Lys Trp Ser Thr Asp Gly Thr Glu His Gln Val Ala Lys Leu Met
    290                 295                 300
Glu Glu Asp Val Gly Ala Ala Met Gln Phe Leu Gln Ser Lys Ala Leu

305                 310                 315                 320
Cys Ile Met Pro Ile Ser Leu Ala Ser Ala Ile Tyr His Asp Gln Pro
                325                 330                 335
Pro Asp Ala Ser Thr Met Ile Lys Pro Glu Pro Asn Ala Pro Ser
            340                 345                 350

<210> 265
<211> 205
<212> PRT
<213> Aquilegia vulgaris

<400> 265

Met Lys Asn Leu Gln Glu Leu Val Pro Asn Ser Asn Lys Thr Asp Lys
1               5                   10                  15
Ala Ser Met Leu Asp Glu Ile Ile Glu Tyr Val Arg Phe Leu Gln Leu
            20                  25                  30
Gln Val Lys Val Leu Ser Met Ser Arg Leu Gly Ala Thr Gly Ala Val
        35                  40                  45
Val Pro Leu Ile Thr Glu Asn Gln Pro Glu Gly Ser Thr Ser His Phe
    50                  55                  60
Leu Ser Gln Ser Ala Gly Arg Asp Val Lys His Ile Glu Ser Pro Asn
65                  70                  75                  80
Thr Leu Thr Phe Glu Gln Glu Val Val Lys Leu Met Glu Ser Asn Met
                85                  90                  95
Thr Lys Ala Met Gln Phe Leu Gln Arg Lys Gly Leu Cys Leu Met Pro
            100                 105                 110
Ile Ala Leu Ala Ala Ala Ile Ser Gly Ser Lys Ala Pro Pro Ala Ser
        115                 120                 125
Leu Ser Thr Glu Gly Lys Lys Ser Ile Leu Thr Asn Gly Phe Val His
    130                 135                 140
His Asn Ser Gly Gln Ser Asn Ile Gly Pro Gly Gln Ile Ser Ser Asp
145                 150                 155                 160
Gly Glu Thr Arg Ser Glu Glu Asn Ile Ile Gly Ile His Ser Arg Glu
                165                 170                 175
Asp Phe Ser Ser Asn Ser Cys Ser Gly Thr Ser Ile Lys Lys Glu Gly
            180                 185                 190
Thr Asn Thr Thr Asn Phe Thr Arg Glu Met Asn Gln Glu
        195                 200                 205

<210> 266
<211> 278
<212> PRT
<213> Brassica napus

<400> 266

His Ala Ser Glu Thr Pro Ser Asp Asp Phe Phe Glu Gln Ile Leu Gly
1               5                   10                  15
Leu Pro Asn Phe Ser Ala Ser Ser Ser Asp Gly Gly Leu Gly Gly Gly
            20                  25                  30

Gly Ala Pro Pro Met Met Leu Gln Leu Gly Ser Gly Glu Glu Gly Gly
        35                  40                  45
His Met Gly Gly Leu Gly Gly Gly Ser Gly Phe His Asn Gln Met Phe
    50                  55                  60
Pro Leu Gly Leu Ser Leu Glu Gln Gly Lys Gly Gln Gly Phe Leu Arg
65                  70                  75                  80
Pro Glu Gly Gly Ser Leu Gly Thr Gly Lys Arg Phe Ser Asp Asp Val
                85                  90                  95
Met Lys Pro Val Phe His Gly Gln Pro Met Gln Gln Gln Pro Ala Pro
            100                 105                 110
Ala Ala Pro His Gln Pro Thr Ser Ile Arg Pro Arg Val Arg Ala Arg

```
        115                 120                 125
Arg Gly Gln Ala Thr Asp Pro His Ser Ile Ala Glu Arg Leu Arg Arg
    130                 135                 140
Glu Arg Ile Ala Glu Arg Ile Arg Ala Leu Gln Glu Leu Val Pro Thr
145                 150                 155                 160
Val Asn Lys Thr Asp Arg Ala Ala Met Ile Asp Glu Ile Val Asp Tyr
                165                 170                 175
Val Lys Phe Leu Arg Leu Gln Val Lys Val Leu Ser Met Ser Arg Leu
            180                 185                 190
Gly Gly Ala Gly Ala Gly Ala Pro Leu Val Thr Asp Met Pro Leu Ser
        195                 200                 205
Ser Ser Val Glu Asp Glu Ser Gly Glu Gly Gly Arg Ala Pro Gln Pro
    210                 215                 220
Ala Trp Glu Lys Trp Ser Asn Asp Gly Thr Glu Arg Gln Val Ala Lys
225                 230                 235                 240
Leu Met Glu Glu Asn Val Gly Ala Ala Met Gln Leu Leu Ser Ser Lys
                245                 250                 255
Ala Leu Cys Met Met Pro Ile Ser Leu Ala Met Ala Ile Tyr His Ser
            260                 265                 270
Gln Pro Pro Asp Thr Thr
        275
```

<210> 267
<211> 345
<212> PRT
<213> Citrus clementina

<400> 267

```
Met Phe Leu Gln Leu Gly Ser Gly Gly Pro Ser Pro Gly Ala Gly Gly
1               5                   10                  15
Gly Ala Thr Ser Ala Ala Asp Ile Arg Ser Leu Ala Met Gly Ile Ser
            20                  25                  30
Met Gly Met Met Pro Leu Gly Leu Asn Leu Glu His Ser Phe Leu Arg
        35                  40                  45
Gln His Glu Asp His Asn Ser Asn His Asn Asn Asn Asn Asn Thr Asn
    50                  55                  60
Ala Ser Ser Ser Ser Pro Asn Asn Ser Ser Ala Thr Ser Gly Ile Asn
65                  70                  75                  80
Glu Arg Asp Ser Val His Met Pro Ser Leu Phe Pro Ala Phe Gly Gln
                85                  90                  95
Leu Gln Ser Gln Ser Ala Arg Pro Pro Leu Pro Arg Pro Pro Gln Val
            100                 105                 110
Gln Gln Phe Gln Ser Gln Pro Ala Ala Ala Pro Gln Pro Pro Ala Val
        115                 120                 125
Arg Pro Arg Val Arg Ala Arg Arg Gly Gln Ala Thr Asp Pro His Ser
    130                 135                 140
Ile Ala Glu Arg Leu Arg Arg Glu Arg Ile Ala Asp Arg Met Arg Ala
145                 150                 155                 160
Leu Gln Glu Leu Val Pro Ser Cys Asn Lys Thr Asp Arg Ala Ala Met
                165                 170                 175
Leu Asp Glu Ile Val Asp Tyr Val Lys Phe Leu Arg Leu Gln Val Lys
            180                 185                 190
Val Leu Ser Met Ser Arg Leu Gly Ala Ala Gly Ala Val Ala Gln Leu
        195                 200                 205
Val Ala Asp Val Pro Leu Ser Ser Ala Leu Glu Gly Glu Ser Ile Asp
    210                 215                 220
Gly Gly Ser Ser Gln Pro Glu Trp Glu Lys Trp Ser Asn Asp Gly Thr
225                 230                 235                 240
Glu Gln Gln Val Ala Lys Leu Met Glu Glu Asp Ile Gly Ala Ala Met
                245                 250                 255
Gln Phe Leu Gln Ser Lys Ala Leu Cys Ile Met Pro Ile Ser Leu Ala


            260                 265                 270
Ser Ala Ile Tyr Arg Thr Arg Gln Pro Asp Ala Pro Ala Phe Val Lys
        275                 280                 285
Pro Glu Ser Ser Thr Pro Ser Tyr Cys Leu Asn Asn Asn Trp Ser Gln
    290                 295                 300
Gln Leu Ala His Asn Asp Ser Lys Tyr Leu Ile Thr Gly Thr Tyr Ser
305                 310                 315                 320
Tyr Arg Ile Leu Leu Val Leu Leu Ser Asn Gln Ile Ile Gln Ile Pro
                325                 330                 335
Met Gln Arg Ser His Ser Ser Phe Pro
            340                 345
```

<210> 268
<211> 410
<212> PRT
<213> Curcuma longa

<400> 268

```
Met Gln Pro Ser Ser Lys Glu Thr Glu Arg Met Ala Gln Ser His Ala
1               5                   10                  15
Ala Leu Gln Leu Glu Leu Gln Asn Gly Gly Gly Gly Ala Gln Asn Asp
            20                  25                  30
Asp Phe Phe Glu Gln Thr Met Ser Gly Phe Ser Thr Ala Ala Trp Ala
        35                  40                  45
Pro Glu Leu Gly Thr Pro Arg Ser Leu Phe Gly Ala Arg Ser Ser Glu
    50                  55                  60
Glu Ser Pro Asp Asp Glu Gly Leu Asn Tyr Ala Pro Pro Tyr Gly Gly
65                  70                  75                  80
Ser Ser Leu Leu Ala Ser Arg His Arg Met His Leu Gly Thr Ser Pro
                85                  90                  95
Ala Asp Ser Ser Met Phe Leu Gln Leu Gly Gly Gln Ile Leu Leu His
            100                 105                 110
Pro Thr Ala Gly Ala Ser Gly Gly Glu Ser Gly Gly Phe Leu Arg Leu
        115                 120                 125
Pro Leu Ser Leu Gly Ser Gly Gly Ser Gly Val Ser Thr Ser Ala Ile
    130                 135                 140
Phe Gly Asp Arg Ser Arg Gly Glu Ile Asp Pro Pro Phe Glu Ser Ser
145                 150                 155                 160
Asn Pro Thr Glu Ala Glu Val Leu Tyr Asn Asp Gly Phe Thr Gly Ser
                165                 170                 175
Leu Pro Arg Ala Val Gln Ala Ser Leu His Gln Gln Leu Leu Arg Gln
            180                 185                 190
Pro Gln Asn Tyr Gly Ala Ala Thr Leu Thr Gly Gln Ala Leu Ala Ile
        195                 200                 205
Thr Ala Thr Ala Ser Gly Thr Ala Gly Ala Gly Thr Ala Pro Pro Lys
    210                 215                 220
Pro Arg Val Arg Ala Arg Arg Gly Gln Ala Thr Asp Pro His Ser Ile
225                 230                 235                 240
Ala Glu Arg Leu Arg Arg Glu Arg Ile Ala Glu Arg Met Lys Ala Leu
                245                 250                 255
Gln Glu Leu Val Pro Asn Ala Asn Lys Thr Asp Lys Ala Ser Met Leu
            260                 265                 270
Asp Glu Ile Ile Asp Tyr Leu Lys Phe Leu Gln Leu Gln Val Lys Val
        275                 280                 285
Leu Ser Met Ser Arg Leu Gly Gly Ala Ala Ala Val Ala Pro Leu Val
    290                 295                 300
Ala Asp Met Ser Ser Glu Ser Gly Gly Gly Ala Gly Thr Pro Arg Ser
305                 310                 315                 320
Asn Asn Asp Gly Leu Thr Ala Ala Glu Gln Gln Val Ala Lys Leu Leu
                325                 330                 335
Glu Glu Asp Met Gly Ser Ala Met Gln Tyr Leu Gln Gly Lys Gly Leu

            340                 345                 350

Cys Leu Met Pro Ile Ser Leu Ala Ser Ala Ile Ser Ser Ala Thr Cys
        355                 360                 365
Arg Pro Arg Pro Pro Pro Gly Val Asn Leu Arg Tyr Pro Ala Ala Gly
    370                 375                 380
Asp Ala Pro Pro Ser Pro Ser Ile Ser Ala Leu Thr Val Gln Ser Ser
385                 390                 395                 400
Asn Ala Gly Ser Ala Gly Ala Asp Ala Ser
                405                 410
```

<210> 269
<211> 281
<212> PRT
<213> Citrus paradisi hybrid

<400> 269

```
Met Ala Asn Asn Pro Asn Glu Ala Ser Ser Thr Asp Asp Phe Leu Glu
1               5                   10                  15
Gln Ile Leu Gly Ile Pro Asn Phe Gly Ser Ala Glu Ser Gly Leu Ala
            20                  25                  30
Ala Ser Asp Gly Gly Leu Ala Ala Gly Ser Pro Met Met Leu Gln Leu
        35                  40                  45
Ser Ser Gly Asp Gly Ser Ser His Ile Ser Ala Leu Gly Gly Gly Val
    50                  55                  60
Ser Ser Gly Tyr His Gly Gln Val Phe Pro Leu Gly Leu Ser Leu Glu
65                  70                  75                  80
Gln Gly Lys Gly Gly Phe Leu Lys Pro Glu Glu Ala Ser Gly Ser Gly
                85                  90                  95
Lys Arg Phe Pro Glu Glu His Ala Ile Lys Asn Val Phe His Gly Gln
            100                 105                 110
Pro Leu Pro Ser Pro Val Pro Ala Ala Pro His Pro Pro Ala Met Arg
        115                 120                 125
Pro Arg Val Arg Ala Arg Arg Gly Gln Ala Thr Asp Pro His Ser Ile
    130                 135                 140
Ala Glu Arg Leu Arg Arg Glu Arg Ile Ala Glu Arg Ile Arg Ala Leu
145                 150                 155                 160
Gln Glu Leu Val Pro Ser Val Asn Lys Thr Asp Arg Ala Ala Met Leu
                165                 170                 175
Asp Glu Ile Val Asp Tyr Val Lys Phe Leu Arg Leu Gln Val Lys Val
            180                 185                 190
Leu Ser Met Ser Arg Val Gly Ala Pro Gly Ala Val Ala Pro Leu Val
        195                 200                 205
Thr Thr Asp Leu Pro Leu Ser Ser Val Glu Asp Glu Ser Gly Glu Gly
    210                 215                 220
Val Arg Asn Gln Pro Ala Trp Glu Lys Trp Ser Asn Asp Gly Thr Glu
225                 230                 235                 240
Arg Gln Val Ala Lys Leu Met Glu Glu Asn Val Gly Ala Ala Met Gln
                245                 250                 255
Phe Leu Gln Ser Lys Ala Leu Cys Ile Met Pro Ile Ser Leu Ala Thr
            260                 265                 270
Ala Ile Tyr His Ser Gln Pro Pro Glu
        275                 280
```

<210> 270
<211> 469
<212> PRT
<213> Citrus sinesis

<400> 270

```
Met Gln Pro Cys Ser Ser Gly Gly Glu Met Gln Gly Ile Ser Ser Leu
```

```
1               5                   10                  15
Leu Ser Asn Gly Ser His Glu Gln Gln Ser Gln Ile His Gln Ser Ala
            20                  25                  30
Thr Phe Asp Pro Thr Ser Gln Asp Asp Phe Leu Glu Gln Met Leu Ser
        35                  40                  45
Ser Leu Pro Ser Cys Ser Trp Thr Asp Leu Lys Ser Pro Trp Gly Val
    50                  55                  60
Val Asp Leu Asn Pro Asn Asn Asn Asn Asn Asn Ile Asn Asn Lys Gln
65                  70                  75                  80
Pro Arg Asp Leu Ser Asp Glu Thr Ala Pro Ser Thr Thr Gln Glu Asn
                85                  90                  95
Asn Val Pro Ala Gly Phe Gln Phe Asp Glu Ser Met Ile Leu Ala Ser
            100                 105                 110
Lys Met Arg Gln His Gln Ile Ser Gly Asn Thr Gly Ser Gly Asn Asn
        115                 120                 125
Asn Ser Ala Ala Ala Lys Phe Met Met Gln Gln Gln Gln Gln Gln Ile
    130                 135                 140
Met Met Ala Ala Ala Arg Gly Gly Ile Gly Leu Pro Leu Ser Leu Gly
145                 150                 155                 160
Asn Gly Gly Asp Asn Asp Ile Val Asp Val Ser Ser Phe Lys Ser Gln
                165                 170                 175
Gln Gly Gly Asp Gly Ser Val Gln Ala Leu Tyr Asn Gly Phe Thr Gly
            180                 185                 190
Ser Leu His Gly Ser Thr Gln Pro Gln His Phe His His Leu Gln Gly

        195                 200                 205
Gly Ser Met Pro Gly Gln Thr Phe Gly Ala Pro Gly Pro Val Met Asn
    210                 215                 220
Gln Thr Gln Ala Gln Ala Ser Gly Ser Thr Gly Gly Gly Gly Gly Gly
225                 230                 235                 240
Gly Asn Thr Pro Ala Gln Gln Pro Lys Gln Arg Val Arg Ala Arg Arg
                245                 250                 255
Gly Gln Ala Thr Asp Pro His Ser Ile Ala Glu Arg Leu Arg Arg Glu
            260                 265                 270
Arg Ile Ala Glu Arg Met Lys Ala Leu Gln Glu Leu Val Pro Asn Ala
        275                 280                 285
Asn Lys Thr Asp Lys Ala Ser Met Leu Asp Glu Ile Ile Asp Tyr Val
    290                 295                 300
Lys Phe Leu Gln Leu Gln Val Lys Val Leu Ser Met Ser Arg Leu Gly
305                 310                 315                 320
Gly Ala Ala Ala Val Ala Pro Leu Val Ala Asp Met Ser Ser Glu Gly
                325                 330                 335
Gly Gly Gly Asp Cys Ile Gln Ala Asn Gly Arg Asn Pro Asn Gly Ala
            340                 345                 350
Gln Thr Thr Ser Ala Asn Asp Ser Leu Thr Val Thr Glu His Gln Val
        355                 360                 365
Ala Lys Leu Met Glu Glu Asp Met Gly Ser Ala Met Gln Tyr Leu Gln
    370                 375                 380
Gly Lys Gly Leu Cys Leu Met Pro Ile Ser Leu Ala Thr Ala Ile Ser
385                 390                 395                 400
Thr Ala Thr Cys His Ser Arg Asn Pro Ile Ile Ser Thr Ser Asn Asn
                405                 410                 415
Asn Asn Asn Asn Gly Asn Pro His His Asn Pro Leu Leu Gln Ser Asn
            420                 425                 430
Gly Glu Gly Pro Thr Ser Pro Ser Met Ser Val Leu Thr Val Gln Ser
        435                 440                 445
Ala Thr Met Gly Asn Gly Gly Ala Asp Gly Ser Val Lys Asp Ala Ala
    450                 455                 460
Ser Val Ser Lys Pro
465
```

<210> 271
<211> 320

<212> PRT
<213> Eucalyptus grandis

<400> 271

```
Met Ala Gly Ser Asn Pro Gln Glu Gly Leu Gly Asp Glu Phe Phe Glu
1               5                   10                  15
Gln Ile Leu Ala Val Pro Pro Gly Ala Tyr Ser Gly Gly Ser Thr Pro
            20                  25                  30
Met Val Leu Gln Leu Gly Ser Gly Arg Gly Gly Val Glu Asp Gly Gly
        35                  40                  45
Gly Arg Gly Gly Gly Gly Gly Leu Arg Gly Met Gly Val Met Met Pro
    50                  55                  60
Leu Gly Leu Asn Leu Glu Gln Gly Gly Leu Phe Arg His Glu Asp Val
65                  70                  75                  80
Glu Asn Ser Thr Ser Ala Ser Ser Thr Thr Ser Ala Ile Asn Met Glu
                85                  90                  95
Arg Glu Ala Gly Val His His Gly Asn Asn Met Thr Ser Cys Leu Phe
            100                 105                 110
Pro Ala Phe Gly Gln Phe Gln Thr His Gln Ser Val Gln Pro Pro Pro
        115                 120                 125
Pro His Pro Pro Pro Pro Gln Leu His Pro Ala Phe Leu Asn Gln Pro
    130                 135                 140
Ala Val Gly Ser Pro Asn Gln Pro Ala Ile Arg Pro Arg Ala Arg Ala
145                 150                 155                 160
Arg Arg Gly Gln Ala Thr Asp Pro His Ser Ile Ala Glu Arg Leu Arg
                165                 170                 175
Arg Glu Arg Ile Asn Glu Arg Met Lys Ala Leu Gln Glu Leu Val Pro
            180                 185                 190
Ser Cys Asn Lys Thr Asp Arg Ala Ala Met Leu Asp Glu Ile Val Asp
        195                 200                 205
Tyr Val Lys Phe Leu Arg Leu Gln Val Lys Val Leu Ser Met Ser Arg
    210                 215                 220
Leu Gly Gly Ala Gly Ala Val Ala Gln Leu Val Ala Asp Val Pro Leu
225                 230                 235                 240
Ser Ser Ala Glu Gly Glu Ile Ile Glu Gly Gly Asn Asn Gln Pro Ala
                245                 250                 255
Trp Glu Lys Trp Leu Thr Asp Gly Thr Glu Gln Gln Val Ala Lys Leu
            260                 265                 270
Met Glu Glu Asp Val Gly Ala Ala Met Gln Phe Leu Gln Ser Lys Thr
        275                 280                 285
Leu Cys Ile Met Pro Ile Ser Leu Ala Ser Ala Ile Phe Arg Thr Ser
    290                 295                 300
Gln Pro Asp Met Pro Arg Ser Ile Lys Pro Glu Ser Ser Ala Pro Ser
305                 310                 315                 320
```

<210> 272
<211> 346
<212> PRT
<213> Eucalyptus grandis

<400> 272

```
Met Ala Asn Asn Pro Ser Asp Gly Ala Ser Asp Glu Phe Leu Glu His
1               5                   10                  15
Leu Leu Gly Leu Pro Asn Phe Ala Ala Ala Ala Glu Ala Gly Leu Ala
            20                  25                  30
Pro Gly Asp Gly Thr Gly Leu Ser Val Thr Ala Ala Thr Pro Met Met
        35                  40                  45
Leu Gln Leu Gly Ser Gly Asp Gly Ser Gly Gly His Gly His Gly His
    50                  55                  60
```

```
Gly His Gly His Gly Gln Gly His Leu Ser Ala Leu Gly Gly Gly Gly
65                  70                  75                  80
Ala Gly Ala Gly Gly Gly Gly Gly Gly Gly Gly Gly Gly Tyr His Gly
                85                  90                  95
Ala Val Phe Pro Leu Gly Leu Ser Leu Glu Gln Gly Lys Gly Gly Phe
            100                 105                 110
Leu Lys Pro Glu Glu Ala Ser Gly Ser Gly Lys Arg Tyr Pro Glu Glu
        115                 120                 125
Val Val Asp Gly Arg Ala Ser Thr Val Lys Asn Leu His Ser Pro Leu
    130                 135                 140
Pro Asn Phe Pro Asp Val Ser Gln Met Ala Ser Phe Arg Asn Arg Asp
145                 150                 155                 160
Val Phe His Gly Gln Pro Val Pro Asn Pro Val Pro Ala Ala Pro His
                165                 170                 175
Pro Pro Ala Met Arg Pro Arg Val Arg Ala Arg Arg Gly Gln Ala Thr
            180                 185                 190
Asp Pro His Ser Ile Ala Glu Arg Leu Arg Arg Glu Arg Ile Ala Glu
        195                 200                 205
Arg Ile Arg Gln Leu Gln Asp Leu Val Pro Ser Val Asn Lys Thr Asp
    210                 215                 220
Arg Ala Ala Met Leu Asp Glu Ile Val Asp Tyr Val Lys Phe Leu Arg
225                 230                 235                 240
Leu Gln Val Lys Val Leu Ser Met Ser Arg Leu Gly Ala Ala Gly Ala
                245                 250                 255
Val Ala Pro Leu Val Thr Asp Ile Pro Leu Ser Ser Val Glu Glu Glu
            260                 265                 270
Gly Gly Glu Gly Gly Arg Asn Gln Pro Ala Trp Glu Lys Trp Ser Asn
        275                 280                 285
Asp Gly Thr Glu Arg Gln Val Ala Lys Leu Met Glu Glu Asn Val Gly
    290                 295                 300
Ala Ala Met Gln Phe Leu Gln Ser Lys Ala Leu Cys Ile Met Pro Ile
305                 310                 315                 320
Ser Leu Ala Thr Ala Ile Tyr Gln Thr Gln Pro Pro Asp Thr Ser Ser
                325                 330                 335
Leu Val Lys Ser Glu Ser Asn Pro Pro Ser
            340                 345
```

<210> 273
<211> 265
<212> PRT
<213> Gossypium hirsutum

<400> 273

```
Asp Ser Gly Gly Gly Gly Phe Arg Gly Ile Gly Met Met Pro Leu Gly
1               5                   10                  15
Leu Asn Leu Glu His Gly Phe Leu Arg His Glu Asp Gly Val Val Val
            20                  25                  30
Asp Asn Asn Asn Asn Asn Ala Ser Cys Ser Ala Ala Ser Ala Val Ser
        35                  40                  45
Gly Ile Ser Glu Arg Asp Ser Met His Met Val Ser Leu Phe Pro Pro
    50                  55                  60
Phe Gly Gln Met Gln Thr Gln Gln Ile Arg Ala Ser Pro Pro Pro Pro
65                  70                  75                  80
Gln Pro Pro Pro Gln Leu His Gln Pro Phe His Ser Gln Pro Thr Ser
                85                  90                  95
Gly Pro Val Ala Ala Ala Pro His Pro Pro Ala Val Arg Pro Arg Val
            100                 105                 110
Arg Ala Arg Arg Gly Gln Ala Thr Asp Pro His Ser Ile Ala Glu Arg
        115                 120                 125
Leu Arg Arg Glu Arg Ile Ala Glu Arg Met Lys Ala Leu Gln Glu Leu
    130                 135                 140
```

```
Val Pro Ser Cys Asn Lys Thr Asp Arg Ala Ala Met Leu Asp Glu Ile
145                 150                 155                 160
Val Asp Tyr Val Lys Phe Leu Arg Leu Gln Val Lys Val Leu Ser Met
                165                 170                 175
Ser Arg Leu Gly Ala Ala Gly Ala Val Ala Gln Leu Val Ala Asp Val
            180                 185                 190
Pro Leu Leu Ser Val Glu Gly Asp Gly Ala Gly Gly Gly Thr Gln Pro
        195                 200                 205
Pro Trp Glu Lys Trp Ser Asn Asp Gly Thr Glu Gln Gln Val Ala Lys
    210                 215                 220
Leu Met Glu Glu Asp Ile Gly Ala Ala Met Gln Phe Leu Gln Ser Gln
225                 230                 235                 240
Ala Leu Cys Ile Val Pro Ile Ser Leu Ala Ser Ala Ile Phe Pro Ala
                245                 250                 255
His Gln Pro Asp Ala Pro Thr Ile Val
            260                 265
```

<210> 274
<211> 306
<212> PRT
<213> Gossypium hirsutum

<400> 274

```
Met Ile Ser Asn Gly Gly Glu Ser Ser Glu Phe Arg Arg Ser Phe Thr
1               5                   10                  15
Gly Leu Glu Thr Leu Ser Pro Ile Pro Gln Leu Trp His Leu Gln Pro
            20                  25                  30
Tyr Asp Ser Val Ser Ser Leu Pro Thr Leu Val Gly Gln Thr Ile Val
        35                  40                  45
Asp Asp Glu Gly Asn Ile Asn Arg Phe Ile Glu Ile Asp Glu Ile Leu
    50                  55                  60
Gln Pro Glu Asn Leu Ser Ala Ser Ile Asn Ser Lys Gly Gln Gln Asp
65                  70                  75                  80
Met Gln Asn Ser Phe Cys Ser Ser Phe Pro Ala Asp His Pro Ile Thr
                85                  90                  95
Lys Thr Met Ile Gly Leu Pro Ser Leu Val Gln Gly Ala Ser Pro Asn
            100                 105                 110
Leu Asn Asn Gly Ser Asp Arg Thr Val Lys Pro Arg Val Arg Ala Arg
        115                 120                 125
Arg Gly Gln Ala Thr Asp Pro His Ser Ile Ala Glu Arg Leu Arg Arg
    130                 135                 140
Glu Lys Ile Ala Glu Arg Met Lys Asn Leu Gln Glu Leu Val Pro Asn
145                 150                 155                 160
Ser Asn Lys Thr Asp Lys Ala Ser Ile Leu Asp Glu Ile Ile Gly Tyr
                165                 170                 175
Val Lys Phe Leu Gln Leu Gln Val Lys Val Leu Ser Met Ser Arg Leu
            180                 185                 190
Gly Ala Ala Ala Ala Val Val Pro Leu Ile Thr Asp Gly Arg Ala Glu
        195                 200                 205
Val Ser Asn Gly Leu Ser Leu Ala Pro Leu Ala Gly Gln Gly Val Asp
    210                 215                 220
Phe Ser Pro Ser Pro Asp Gln Val Val Phe Glu Gln Glu Val Val Lys
225                 230                 235                 240
Leu Met Glu Ser Asn Met Thr Met Ala Met Gln Tyr Leu Gln Ser Lys
                245                 250                 255
Gly Phe Cys Leu Met Pro Val Ala Leu Ala Ala Ala Ile Ser Asn Val
            260                 265                 270
Lys Ser Ser Thr Ser Ser Ser Ser Ser Ser Val Pro Ala Ser Asp Glu
        275                 280                 285
Ser Lys Lys Leu Gly Leu His Gln Gln Tyr Ser Asn Gln Gln His Leu
    290                 295                 300
```

Gln Gln

305

<210> 275
<211> 303
<212> PRT
<213> Gossypium hirsutum

<400> 275

```
Met Ala Asn Asn Pro Asn Glu Ser Pro Ala Asp Asp Phe Leu Glu Gln
1               5                   10                  15
Ile Leu Gly Leu Ser His Phe Ala Pro Thr Glu Thr Gly Leu Ala Gly
            20                  25                  30
Pro Asp Gly Arg Leu Ser Gly Asn Ala Thr Thr Ala Gly Ala Pro Met
        35                  40                  45
Leu Leu Gln Leu Ser Ser Gly Gly Gly Thr Gly His Ile Gly Ala Ile
    50                  55                  60
Gly Gly Gly Gly Gly Gly Ala Phe His Gly Gln Val Phe Pro Leu Gly
65                  70                  75                  80
Leu Ser Leu Glu Gln Gly Lys Gly Gly Phe Leu Lys Pro Glu Glu Ala
                85                  90                  95
Ser Gly Ser Ser Lys Arg Phe Arg Asn Glu Val Val Asp Gly Arg Ala
            100                 105                 110
Phe Ser Val Lys Asn Val Phe His Gly Gln Pro Val Pro Ala Thr Val
        115                 120                 125
Ala Ala Gly Pro His Pro Pro Ala Met His Pro Arg Val Arg Ala Arg
    130                 135                 140
Arg Gly Gln Ala Thr Asp Pro His Ser Ile Ala Glu Arg Leu Arg Arg
145                 150                 155                 160
Glu Arg Ile Ala Glu Arg Ile Arg Ala Leu Gln Glu Leu Val Pro Ser
                165                 170                 175
Val Asn Lys Thr Asp Arg Ala Ala Met Leu Asp Glu Ile Val Asp Tyr
            180                 185                 190
Val Lys Phe Leu Arg Leu Gln Val Lys Val Leu Ser Met Ser Arg Leu
        195                 200                 205
Gly Ala Ala Gly Ala Val Ala Pro Leu Val Thr Asp Leu Pro Leu Ser
    210                 215                 220
Ser Val Glu Asp Glu Ser Gly Glu Gly Gly Arg Ser Gln Pro Ala Trp
225                 230                 235                 240
Glu Lys Trp Ser Asn Asp Gly Thr Glu Arg Gln Val Ala Lys Leu Met
                245                 250                 255
Glu Glu Asp Val Gly Ala Ala Met Gln Phe Leu Gln Ser Lys Ala Leu
            260                 265                 270
Cys Val Met Pro Ile Ser Leu Ala Thr Ala Ile Tyr His Thr Gln Ser
        275                 280                 285
Pro Asp Thr Ser Ser Val Val Lys Pro Glu Thr Asn Pro Pro Ser
    290                 295                 300
```

<210> 276
<211> 304
<212> PRT
<213> Gossypium hirsutum

<400> 276

```
Met Ala Asn Asn Thr Asn Glu Ala Pro Ala Ala Asp Asp Phe Leu Glu
1               5                   10                  15
Gln Ile Leu Gly Leu Pro Asn Phe Ala Pro Ser Glu Thr Gly Leu Ala
            20                  25                  30
Gly Ser Asp Ala Gly Leu Ala Ala Thr Ala Ala Gly Ala Gly Ala Pro
        35                  40                  45
Met Phe Leu Gln Leu Ser Ser Gly Asp Gly Ala Ala His Ile Gly Gly
    50                  55                  60
Ile Gly Gly Gly Gly Gly Gly Ala Phe His Gly Gln Val Phe Pro Leu
65                  70                  75                  80
Gly Leu Ser Leu Glu Gln Gly Lys Gly Gly Phe Leu Lys Pro Glu Glu
                85                  90                  95
Ala Ser Gly Ser Gly Lys Arg Phe Arg Asn Gly Val Val Asp Asp Arg
            100                 105                 110
Ala Ser Ser Val Lys Asn Val Phe His Gly Gln Pro Met Gln Ala Thr
        115                 120                 125
Val Ser Ala Ala Pro His Pro Pro Thr Met Arg Pro Arg Val Arg Ala
    130                 135                 140
Arg Arg Gly Gln Ala Thr Asp Pro His Ser Ile Ala Glu Arg Leu Arg
145                 150                 155                 160
Arg Glu Arg Ile Thr Glu Arg Ile Arg Ala Leu Gln Glu Leu Val Pro
                165                 170                 175
Ser Val Asn Lys Thr Asp Arg Ala Val Met Leu Asp Glu Ile Val Asp
            180                 185                 190
Tyr Val Lys Phe Leu Arg Leu Gln Val Lys Val Leu Ser Met Ser Arg
        195                 200                 205
Leu Gly Gly Ala Gly Ala Val Ala Pro Leu Val Thr Asp Ile Pro Leu
    210                 215                 220
Ser Ser Val Glu Tyr Glu Ser Gly Glu Gly Gly Arg Ser Gln Pro Ala
225                 230                 235                 240
Trp Glu Lys Trp Ser Asn Asp Gly Thr Glu Gln Gln Val Ala Lys Leu
                245                 250                 255
Met Glu Glu Asn Val Gly Ala Ala Met Gln Phe Leu Gln Ser Lys Ser
            260                 265                 270
Leu Cys Ile Met Pro Ile Ser Leu Ala Thr Ala Ile Tyr His Thr Gln
        275                 280                 285
Val Pro Asp Thr Ser Ser Val Val Lys Pro Glu Thr Asn Pro Pro Ala
    290                 295                 300
```

<210> 277
<211> 305
<212> PRT
<213> Gossypium hirsutum

<400> 277

```
Met Ala Asn Asn Pro Asn Glu Ala Ser Ala Asp Asp Phe Leu Glu Gln
1               5                   10                  15
Ile Leu Gly Phe Pro Asn Phe Ala Pro Ser Glu Thr Gly Leu Ala Gly
            20                  25                  30
Pro Asp Gly Gly Leu Thr Gly Thr Thr Ala Gly Ala Gly Thr Pro Met
        35                  40                  45
Phe Leu Gln Leu Ser Ser Gly Asp Gly Ala Ser His Leu Gly Gly Ile
    50                  55                  60
Gly Gly Gly Gly Gly Gly Ala Phe Pro Gly Gln Val Phe Pro Leu Gly
65                  70                  75                  80
Leu Ser Leu Asp Gln Gly Lys Ser Gly Gly Phe Leu Lys Pro Glu Glu
                85                  90                  95
Gly Ser Gly Gly Ser Ser Arg Arg Phe Arg Asp Glu Val Val Asp Gly
            100                 105                 110
Arg Ala Ser Ser Val Lys Asn Val Phe His Gly Ser Pro Met Pro Ala
        115                 120                 125
Thr Val Ala Pro Ser Pro His Pro Pro Thr Met Arg Pro Arg Val Arg
    130                 135                 140
Ala Arg Arg Gly Gln Ala Thr Asp Pro His Ser Ile Ala Glu Arg Leu
145                 150                 155                 160
Arg Arg Glu Arg Ile Ala Glu Arg Ile Arg Ala Leu Gln Glu Leu Val
                165                 170                 175
Pro Ser Val Asn Lys Thr Asp Arg Ala Val Met Leu Asp Glu Ile Val


            180                 185                 190
Asp Tyr Val Lys Phe Leu Arg Leu Gln Val Lys Val Leu Ser Met Ser
        195                 200                 205
Arg Leu Gly Gly Ala Gly Ala Val Ala Pro Leu Val Thr Asp Ile Pro
    210                 215                 220
Leu Ser Ser Val Glu Asp Glu Ser Gly Asp Gly Gly Arg Asn Gln Pro
225                 230                 235                 240
Ala Trp Glu Lys Trp Ser Asn Asp Gly Thr Glu Arg Gln Val Ala Lys
                245                 250                 255
Leu Met Glu Glu Asn Val Gly Ala Ala Met Gln Phe Leu Lys Ser Lys
            260                 265                 270
Ala Leu Cys Ile Met Pro Ile Ser Leu Ala Thr Ala Ile Tyr His Ser
        275                 280                 285
Gln Pro Leu Asp Thr Ser Ser Ile Val Lys Pro Glu Thr Asp Pro Pro
    290                 295                 300
Pro
305
```

<210> 278
<211> 427
<212> PRT
<213> Gossypium hirsutum

<400> 278

```
Met Gln Pro Cys Ser Arg Glu Met Gln Ala Met Asn Ser Leu Leu Asn
1               5                   10                  15
Pro Thr Gln Gln Ile Pro Leu Gln Asp Leu Gln Ile Asn Gly Asn Arg
            20                  25                  30
His His His Gln Gln Ile His Val Pro Ser Ser Ser Gln Phe Gln Tyr
        35                  40                  45
Pro Thr Pro Thr Ser Thr Thr His His Asp Asp Ser Phe Leu Glu Gln
    50                  55                  60
Ile Leu Ser Ser Thr Thr Ser Phe Pro Trp Ser Asp Glu Thr Gly Pro
65                  70                  75                  80
Pro Asn Pro Glu Asp Asn Asn Asn Ala Gly Phe Asn Tyr Asp Glu Met
                85                  90                  95
Val Leu Ala Ser Lys Leu Arg Gln His Gln Ile Asn Gly Gly Ala Ala
            100                 105                 110
Gly Asn Pro Phe Ala Ala Met Lys Met Met Leu Met Met Gln Gln Gln
        115                 120                 125
Gln Gln Gln Gln Gln Met Met Leu Ala Gly Arg Pro Thr Val Ala Ser
    130                 135                 140
Ala Ala Ala Gly Gly Gly Ile Thr Thr Asn His Gln Gly Gly Glu Gly
145                 150                 155                 160
Ser Met Gln Ala Leu Tyr Asn Val Phe Gly Asp Gly Ser Leu His Gly
                165                 170                 175
Thr Met Gln Ala Lys Ser Phe Val Gly Ala Asn Ser Ala Thr Glu Met
            180                 185                 190
Thr Gln Ser Gln Gly Ser Gly Pro Thr Ala Gly Glu Ala Val Thr Ala
        195                 200                 205
Pro Glu Lys Pro Lys Gln Arg Val Arg Ala Arg Arg Gly Gln Ala Thr
    210                 215                 220
Asp Pro His Ser Ile Ala Glu Arg Leu Arg Arg Val Arg Ile Ala Glu
225                 230                 235                 240
Arg Met Lys Ala Leu Gln Glu Leu Val Pro Asn Ala Asn Lys Thr Asp
                245                 250                 255
Lys Ala Ser Met Leu Asp Glu Ile Ile Asp Tyr Val Lys Phe Leu Gln
            260                 265                 270
Leu Gln Val Lys Val Leu Ser Met Ser Arg Leu Gly Gly Ala Ala Ala
        275                 280                 285
Val Ala Pro Leu Val Ser Glu Gly Gly Gly Asp Cys Ile Gln Thr Ser
    290                 295                 300
Ala Asn Gly Gly Ser His Pro Arg Asn Ser Asn Gly Asp Gln Thr Pro
305                 310                 315                 320
Ser Ala Asn Asp Arg Leu Thr Met Thr Glu His Gln Val Ala Lys Leu
                325                 330                 335
Met Glu Glu Asp Met Gly Ser Ala Met Gln Tyr Leu Gln Glu Lys Gly
            340                 345                 350
Leu Cys Leu Met Pro Ile Ser Leu Ala Thr Ala Ile Ser Ser Ala Thr
        355                 360                 365
Ser Arg Ser Arg Asn Pro Met Ile Asn His Glu Asn Pro Ala Asn Gly
    370                 375                 380
Ser His Leu Leu Ile Gln Ser Ser Gly Gly Asp Gly Pro Ser Ser Pro
385                 390                 395                 400
Ser Met Ser Val Leu Thr Val Gln Ser Ala Thr Met Gly Asn Gly Gly
                405                 410                 415
Leu Glu Gly Gly Ala Ala Ser Val Ser Lys Pro
            420                 425
```

<210> 279
<211> 210
<212> PRT
<213> Glycine max

<400> 279

```
Met Lys Pro Asp Glu Ala Ser Ala Ser Gly Lys Arg Phe Arg Asp Asp
1               5                   10                  15
Val Val Asp Asn Arg Ala Lys His Val Phe His Gly Gln Pro Met Pro
            20                  25                  30
Thr Thr Met Pro Ala Ala Pro His Pro Pro Ala Ile Arg Pro Arg Val
        35                  40                  45
Arg Ala Ile Arg Gly Gln Ala Thr Asp Pro His Ser Ile Ala Glu Arg
    50                  55                  60
Leu Arg Arg Glu Arg Ile Ala Glu Arg Ile Arg Ala Leu Gln Glu Leu
65                  70                  75                  80
Val Pro Ser Val Asn Lys Thr Asp Arg Ala Ala Met Leu Asp Glu Ile
                85                  90                  95
Val Asp Tyr Val Lys Phe Leu Arg Leu Gln Val Lys Val Leu Ser Met
            100                 105                 110
Ser Arg Leu Gly Gly Ala Gly Ala Val Ala Pro Leu Val Thr Asp Ile
        115                 120                 125
Pro Leu Ser Ser Val Glu Glu Glu Gly Gly Glu Gly Ala Arg Asn Arg
    130                 135                 140
Pro Ala Trp Asp Lys Trp Ser Asn Asp Gly Thr Glu Arg Gln Val Ala
145                 150                 155                 160
Lys Leu Met Glu Glu Asn Val Gly Ala Ala Met Gln Phe Leu Gln Ser
                165                 170                 175
Lys Ala Leu Cys Ile Met Pro Ile Ser Leu Ala Ser Ala Ile Tyr Gln
            180                 185                 190
Ser Gln Pro Pro Asp Thr Ser Ser Ile Val Lys Pro Glu Thr Asn Pro
        195                 200                 205
Pro Ser
    210
```

<210> 280
<211> 335
<212> PRT
<213> Glycine max

<400> 280

```
Met Leu Gln His Gln Leu Leu Arg Asp Ser Gly Leu Leu Asn Met Pro
1               5                   10                  15
```

```
Leu Ser Leu Pro Gly Asn Asp Val Val Val Asp Ala Ser Thr Phe Glu
            20                  25                  30
Ser Pro Asn Pro Ser Gly Lys Ala Ser Val Gln Thr Leu Tyr Asn Gly
        35                  40                  45
Phe Ala Gly Ser Leu His Gly Val Gly Gln Ser Ser Asn Gln Thr Gln
    50                  55                  60
His Phe Gln His Pro Gln Gly Ser Ser Asn Pro Met Gln Gly Gln Asn
65                  70                  75                  80
Phe Gly Ala Val Pro Ala Gly Gly Gly Ser Ala Thr Asn Gln Ala Pro
                85                  90                  95
Ala Ser Gly Ala Ala Ala Gly Gly Ala Pro Ala Gln Pro Arg Gln Arg
            100                 105                 110
Val Arg Ala Arg Arg Gly Gln Ala Thr Asp Pro His Ser Ile Ala Glu
        115                 120                 125
Arg Leu Arg Arg Glu Arg Ile Ala Glu Arg Met Lys Ala Leu Gln Glu
    130                 135                 140
Leu Val Pro Asn Ala Asn Lys Thr Asp Lys Ala Ser Met Leu Asp Glu
145                 150                 155                 160
Ile Ile Asp Tyr Val Lys Phe Leu Gln Leu Gln Val Lys Val Leu Ser
                165                 170                 175
Met Ser Arg Leu Gly Gly Ala Ala Ala Val Ala Pro Leu Val Ala Asp
            180                 185                 190
Met Ser Ser Glu Gly Gly Gly Asp Cys Ile Gln Ala Asn Gly Lys Ser

        195                 200                 205
Asn Gly Gly Gly Ala Gln Ala Ser Thr Thr Asn Thr Asn Thr Asn Gln
    210                 215                 220
Thr Thr Ala Thr Thr Thr Ser Asn Asp Ser Leu Thr Met Thr Glu His
225                 230                 235                 240
Gln Val Ala Lys Leu Met Glu Glu Asp Met Gly Ser Ala Met Gln Tyr
                245                 250                 255
Leu Gln Gly Lys Gly Leu Cys Leu Met Pro Ile Ser Leu Ala Thr Ala
            260                 265                 270
Ile Ser Thr Ala Thr Cys Pro Thr Arg Asn Val Asn Val Asn Pro Leu
        275                 280                 285
Ile Asn Ala Ala Ala Gly Ala Thr His Phe Pro Thr Ala Ala Asn Pro
    290                 295                 300
Ala Gly Glu Gly Pro Ser Ser Pro Ser Met Ser Val Leu Thr Val Gln
305                 310                 315                 320
Ser Ala Ile Ala Gly Asn Asp Gly Ala Ala Ser Val Ser Lys Pro
                325                 330                 335
```

<210> 281
<211> 331
<212> PRT
<213> Glycine max

<400> 281

```
Met Ala Gly Asn Ser Gly Ser Glu Gly Leu Gly Asp Asp Phe Phe Glu
1               5                   10                  15
Gln Ile Leu Ala Val Pro Glu Ala Gly Thr Val Gly Met Leu Gln Leu
            20                  25                  30
Gly Ser Thr Thr Gly Ala Phe Arg Gly Ala Ser Gly Leu Met Pro Leu
        35                  40                  45
Gly Leu Asn Leu Glu Gln Ala Ala Phe Leu Arg His Gln Val Asn Val
    50                  55                  60
Asp Asp Asp Val Val His Val Asn Val Asp Asp Ala Thr Ile His Gln
65                  70                  75                  80
His His Leu Thr Leu His Asn Asn Asn Asn Ser Ser Ser Pro Ser Ser
                85                  90                  95
Thr Ala Pro Ile Thr Asp Arg Asp Ser Met His Met Arg Gly Leu Phe


            100                 105                 110
Ser Ala Phe Gly Gln Leu His Thr Pro Ile Arg Pro Thr Leu Pro Leu
        115                 120                 125
Pro Pro Pro Pro Arg Gln Pro Gln Leu His Leu His His His Asn Gln
    130                 135                 140
Phe Gln Gly Gln Ala Ala Ala Ala Pro Ala Ser Met Ala Ala Met Pro
145                 150                 155                 160
Gln Pro Pro Gly Ile Arg Pro Arg Val Arg Ala Arg Arg Gly Gln Ala
                165                 170                 175
Thr Asp Pro His Ser Ile Ala Glu Arg Leu Arg Arg Glu Arg Ile Ala
            180                 185                 190
Glu Arg Met Lys Ala Leu Gln Glu Leu Val Pro Ser Ile Asn Lys Thr
        195                 200                 205
Asp Arg Ala Ala Met Leu Asp Glu Ile Val Asp Tyr Val Lys Phe Leu
    210                 215                 220
Arg Leu Gln Val Lys Val Leu Ser Met Ser Arg Leu Gly Gly Ala Gly
225                 230                 235                 240
Ala Val Ala Gln Leu Val Ala Asp Val Pro Leu Ser Ala Val Glu Gly
                245                 250                 255
Asp Gln Asp Ile Glu Gly Gly Ala Asn Glu Gln Ala Trp Asp Lys Trp
            260                 265                 270
Ser Asn Asp Gly Thr Glu Gln Gln Val Ala Lys Leu Met Glu Glu Asp
        275                 280                 285
Val Gly Ala Ala Met Gln Phe Leu Gln Ser Lys Ala Leu Cys Ile Met
    290                 295                 300
Pro Ile Ser Leu Ala Ser Ala Ile Phe Arg Met Pro Gln Ser Glu Ala
305                 310                 315                 320
Ser Thr Gly Ile Lys Pro Glu Ser Asn Ser His
                325                 330
```

<210> 282
<211> 244
<212> PRT
<213> Glycine max

<400> 282

Met Met Leu Gln Leu Asn Ser Gly Asp Ala Ala Thr His Leu Ala Ser
1               5                   10                  15
Phe His Ala Pro Pro Tyr Gln Leu Gly Leu Ser Leu Asp Gln Gly Glu
            20                  25                  30
Gly Pro Phe Leu Thr Pro Glu Asp Ala Ser Gly Ser Gly Lys Arg Phe
        35                  40                  45
Arg Asp Asp Ala Val Asp Thr Arg Pro Asn Asn Val Phe Asp Gly Gln
    50                  55                  60
Pro Met Pro Thr Thr Val Pro Thr Ala Pro His Pro Pro Ala Val Arg
65                  70                  75                  80
Pro Arg Val Arg Ala Arg Arg Gly Gln Ala Thr Asp Pro His Ser Ile
                85                  90                  95
Ala Glu Arg Leu Arg Arg Glu Arg Ile Ala Glu Arg Ile Arg Ala Leu
            100                 105                 110
Gln Glu Leu Val Pro Ser Val Asn Lys Thr Asp Arg Ala Ala Met Leu
        115                 120                 125
Asp Glu Ile Val Asp Tyr Val Lys Phe Leu Arg Leu Gln Val Lys Val
    130                 135                 140
Leu Ser Met Ser Arg Leu Gly Gly Ala Gly Ala Val Ala Pro Leu Val
145                 150                 155                 160
Thr Asp Ile Pro Leu Ser Ser Val Glu Glu Glu Gly Gly Glu Gly Arg
                165                 170                 175
Asn Gln Pro Ala Trp Glu Lys Trp Ser Asn Asp Gly Thr Glu Arg Gln
            180                 185                 190

Val Ala Lys Leu Met Glu Glu Asn Val Gly Ala Ala Met Gln Phe Leu
        195                 200                 205
Gln Ser Lys Ala Leu Cys Ile Met Pro Val Ser Leu Ala Ser Ala Ile
    210                 215                 220
Tyr Gln Ser Gln Pro Ser Gly Thr Ser Ser Ile Val Lys Pro Glu Thr
225                 230                 235                 240
Asn Pro Pro Ser

<210> 283
<211> 303
<212> PRT
<213> Gossypium raimondii

<400> 283

```
Met Ala Asn Asn Pro Asn Glu Ser Pro Ala Asp Asp Phe Leu Glu Gln
1               5                   10                  15
Ile Leu Gly Leu Ser His Phe Ala Pro Thr Glu Thr Gly Leu Ala Gly
            20                  25                  30
Pro Asp Gly Arg Leu Ser Gly Asn Ala Thr Thr Ala Gly Ala Pro Met
        35                  40                  45
Leu Leu Gln Leu Ser Ser Gly Gly Gly Thr Gly His Ile Gly Ala Ile
    50                  55                  60
Gly Gly Gly Gly Gly Gly Ala Phe His Gly Gln Val Phe Pro Leu Gly
65                  70                  75                  80
Leu Ser Leu Glu Gln Gly Lys Gly Gly Phe Leu Lys Pro Glu Glu Ala
                85                  90                  95
Ser Gly Ser Ser Lys Arg Phe Arg Asn Glu Val Val Asp Gly Arg Ala
            100                 105                 110
Phe Ser Val Lys Asn Val Phe His Gly Gln Pro Val Pro Ala Thr Val
        115                 120                 125
Ala Ala Gly Pro His Pro Pro Ala Met Arg Pro Arg Val Arg Ala Arg
    130                 135                 140
Arg Gly Gln Ala Thr Asp Pro His Ser Ile Ala Glu Arg Leu Arg Arg
145                 150                 155                 160
Glu Arg Ile Ala Glu Arg Ile Arg Ala Leu Gln Glu Leu Val Pro Ser
                165                 170                 175
Val Asn Lys Thr Asp Arg Ala Ala Met Leu Asp Glu Ile Val Asp Tyr
            180                 185                 190
Val Lys Phe Leu Arg Leu Gln Val Lys Val Leu Ser Met Ser Arg Leu
        195                 200                 205
Gly Ala Ala Gly Ala Val Ala Pro Leu Val Thr Asp Leu Pro Leu Ser
    210                 215                 220
Ser Val Glu Asp Glu Ser Gly Glu Gly Gly Arg Ser Gln Pro Ala Trp
225                 230                 235                 240
Glu Lys Trp Ser Asn Asp Gly Thr Glu Arg Gln Val Ala Lys Leu Met
                245                 250                 255
Glu Glu Asp Val Gly Ala Ala Met Gln Phe Leu Gln Ser Lys Ala Leu
            260                 265                 270
Cys Val Met Pro Ile Ser Leu Ala Thr Ala Ile Tyr His Thr Gln Ser
        275                 280                 285
Pro Asp Thr Ser Ser Val Val Lys Pro Glu Thr Asn Pro Pro Ser
    290                 295                 300
```

<210> 284
<211> 306
<212> PRT
<213> Helianthus petiolaris

<400> 284

```
Met Ser Thr Asp Pro Pro Glu Gly Tyr Gly Asp Asp Phe Leu Glu Gln
```

```
1               5                   10                  15
Ile Leu Ala Ile Pro Ser Tyr Asn Ile Thr Gly Cys Leu Pro Val Asn
            20                  25                  30
Thr Ala Asp Ser Thr Gly Ser Glu Thr Val Ser Val His His Gln Gln
        35                  40                  45
Gln Gln Pro Gln Gln Gln Leu Gln Pro Gln Lys Phe Pro Leu Gly Leu
    50                  55                  60
Ser Leu Asp Asn Gly Arg Glu Thr Ile Gly Gly Ala Phe Ala Gly Gln
65                  70                  75                  80
Gln Gln Gln Leu Gln Gln Gln Gln Gln Arg Glu Arg Gly Gly Ser Met
                85                  90                  95
Asn Met Ser Gly Leu Phe Pro Gln Phe Glu Asn Leu Gln Ser His Ser
            100                 105                 110
Leu Leu His Thr Val Pro Gln Gly Phe Gln Gly Gln Pro Thr Ser Ser
        115                 120                 125
Thr Ala Val Thr Val Pro His Pro Pro Thr Ile Arg Pro Arg Val Arg
    130                 135                 140
Ala Arg Arg Gly Gln Ala Thr Asp Pro His Ser Ile Ala Glu Arg Leu
145                 150                 155                 160
Arg Arg Glu Arg Ile Ala Glu Arg Met Arg Ala Leu Gln Glu Leu Val
                165                 170                 175
Pro Ser Cys Asn Lys Thr Asp Lys Ala Ala Met Leu Asp Glu Ile Leu
            180                 185                 190
Asp Tyr Val Lys Phe Leu Arg Leu Gln Val Lys Val Leu Ser Met Ser
        195                 200                 205
Arg Leu Gly Gly Ala Gly Ala Val Ala Gln Leu Val Ser Asp Val Pro
    210                 215                 220
Leu Gln Ser Val Glu Gly Asp Gly Ser Glu Asn Gly Tyr Asn Asn Gln
225                 230                 235                 240
Gln Gln Gly Gln Ala Ala Trp Glu Asn Trp Ser Asn Asp Asp Thr Glu
                245                 250                 255
Arg Glu Val Ala Lys Leu Met Glu Glu Asp Val Gly Ala Ala Met Gln
            260                 265                 270
Phe Leu Gln Ser Lys Ala Leu Cys Ile Met Pro Ile Ser Leu Ala Ser
        275                 280                 285
Leu Ile Tyr Pro Asn His Gln Pro Asp Val Lys Pro Glu Pro Ser Ala
    290                 295                 300
Pro Ser
305
```

<210> 285
<211> 481
<212> PRT
<213> Horedeum vulgare

<400> 285

```
Met Asn Tyr Ser Asp Gly Ser Phe Phe Pro Ser Trp Pro Gly Asn Ser
1               5                   10                  15
Ala Ser Glu Asn Tyr Ser Phe Val Asp Gly Ser Val Glu Ser Tyr Thr
            20                  25                  30
Glu Glu Gly Ser Met Pro Thr Thr Gly Tyr Phe Arg Ala Arg Ser Asn
        35                  40                  45
Gln Asn Leu Thr Phe Asp Glu His Glu Gln Asn Pro Ala Met Leu Ala
    50                  55                  60
Asn Gly Cys Leu Pro Tyr Asn Thr Gln Thr Asp Leu Leu Ser Gly Glu
65                  70                  75                  80
Ile Leu Ser Asp Asp Lys Pro Ser Asn Ser Leu Val Glu Leu Ser Gln
                85                  90                  95
Leu Gln Asn Asn Val Cys Leu Gln Asn Asn Leu Ile Pro Pro Gly Thr
            100                 105                 110
Leu Gln Cys Asn Ser Thr Pro Gly Thr Phe Asp Leu Gln Leu Asp Thr
```

```
        115                 120                 125
Pro Gly Leu Leu Glu Leu Pro His Ala Leu Ser Ser Ser Ile Glu Ser
    130                 135                 140
Asn Gly Ser Glu Val Ser Ala Phe Leu Ala Asp Val Gln Ala Val Ser
145                 150                 155                 160
Ser Ala Ser Thr Leu Cys Ser Thr Phe Gln Asn Val Pro Ser Tyr Met
                165                 170                 175
Glu Pro Val Ser Leu Glu Ala Phe Ser Phe Gln Gly Met Gln Asn Ala
            180                 185                 190
Ala Met Phe Asn Asn Ala Ser His Ser Asn Gly Asn Leu Ser Val Phe
        195                 200                 205
Asp Glu Ala Thr Met Ala Ser Leu His Asp Ser Lys Glu Phe Leu Asn
    210                 215                 220
Gly Ser Ile Ser Ser Leu Gly Thr Gly Gln Gln Ser Gln Leu Ala Gly
225                 230                 235                 240
Gly Gly Leu Lys Ala Glu Gln Gln Glu Gln Asn Thr Met Cys Asn Ile
                245                 250                 255
Pro Leu Pro Ser Phe Val Ser Ala Ser Gln Met Ala Val Ser Glu Ala
            260                 265                 270
Gln Gly Ala Leu Ile Pro Ser Lys Thr Thr Ser Ile Thr His Asn Asn
        275                 280                 285
Lys Ser Glu Tyr Pro Ile Pro Ile Ser His Ser Ala Asp Val Gln His
    290                 295                 300
Lys Ala Asn Ser Gly Asn Gly Asn Ser Ala Ser Ala Lys Pro Arg Ala
305                 310                 315                 320
Arg Ala Arg Arg Gly Gln Ala Thr Asp Pro His Ser Ile Ala Glu Arg
                325                 330                 335
Leu Arg Arg Glu Lys Ile Ser Glu Arg Met Lys Asn Leu Gln Asp Leu
            340                 345                 350
Val Pro Asn Ser Asn Lys Ala Asp Lys Ser Ser Met Leu Asp Glu Ile
        355                 360                 365
Ile Asp Tyr Val Lys Phe Leu Gln Leu Gln Val Lys Val Leu Ser Met
    370                 375                 380
Ser Arg Leu Gly Ala Pro Gly Ala Val Leu Pro Leu Leu Ala Glu Ser
385                 390                 395                 400
Gln Thr Glu Gly Arg Ser Asn Ser Pro Leu Ser Ser Pro Thr Thr Ser
                405                 410                 415
Gln Gly Leu Leu Asp Val Ala Gly Pro Glu Asp Ser Leu Val Phe Glu
            420                 425                 430
Gln Glu Val Ile Lys Leu Met Glu Thr Ser Ile Thr Asn Ala Met Gln
        435                 440                 445
Tyr Leu Gln Asn Lys Gly Leu Cys Leu Met Pro Ile Ala Leu Ala Ser
    450                 455                 460
Ala Ile Ser Asn Gln Lys Gly Thr Ser Ala Ala Ala Ile Pro Pro Glu
465                 470                 475                 480
Arg
```

<210> 286
<211> 290
<212> PRT
<213> lactuca perennis

<400> 286

```
Met Ala Thr Asp Pro Pro Glu Gly Tyr Gly Asp Asp Phe Leu Glu Gln
1               5                   10                  15
Ile Leu Ala Ile Pro Ser Tyr Asn Ile Ala Gly Cys Leu Pro Gly Asn
            20                  25                  30
Thr Thr Asp Ala Asn Ala Ser Glu Thr Val Ser Val His Arg Gln Gln
        35                  40                  45
Gln Gln Gln Gln Pro Val Phe Pro Leu Gly Leu Ser Leu Asp Asn Gly
```

```
    50                  55                  60
Arg Glu Thr Ile Gly Ala Phe Ala Gly Gln Gln Gln Gln Arg Glu Arg
65                  70                  75                  80
Gly Gly Ser Met Asn Met Thr Gly Leu Phe Pro Ser Phe Glu Asn Leu
                85                  90                  95
Gln Ser His Ser Leu Leu His Ser Val Pro Gln Ala Phe Gln Gly Gln
            100                 105                 110
Ser Thr Thr Ser Thr Ala Val Thr Val Pro His Pro Pro Ser Ile Arg
        115                 120                 125
Pro Arg Val Arg Ala Arg Arg Gly Gln Ala Thr Asp Pro His Ser Ile
    130                 135                 140
Ala Glu Arg Leu Arg Arg Glu Arg Ile Ala Glu Arg Met Arg Ala Leu
145                 150                 155                 160
Gln Glu Leu Val Pro Ser Cys Asn Lys Thr Asp Lys Ala Ala Met Leu
                165                 170                 175
Asp Glu Ile Leu Asp Tyr Val Lys Phe Leu Arg Leu Gln Val Lys Val
            180                 185                 190
Leu Ser Met Ser Arg Leu Gly Gly Ala Gly Ala Val Ala Gln Leu Val
        195                 200                 205
Ser Asp Val Pro Leu Gln Ser Val Glu Gly Asp Thr Ser Glu Asn Gly
    210                 215                 220
Tyr Asn Gln Pro Ala Trp Glu Asn Trp Ser Asn Asp Asp Thr Glu Arg
225                 230                 235                 240
Glu Val Ala Lys Leu Met Glu Glu Asp Val Gly Ala Ala Met Gln Phe
                245                 250                 255
Leu Gln Ser Lys Ala Leu Cys Ile Met Pro Ile Ser Leu Ala Ser Leu
            260                 265                 270
Ile Tyr Pro Pro Gln Thr Pro Asp Ser Asn Ser His Val Lys Pro Glu
        275                 280                 285
Thr Val
    290
```

<210> 287
<211> 296
<212> PRT
<213> Nicotiana benthamiana

<400> 287

```
Met Ala Thr Asn Gln Pro Glu Gly Tyr Ala Asp Asp Phe Leu Glu Gln
1               5                   10                  15
Ile Leu Ala Ile Pro Ser Tyr Ala Gly Leu Pro Pro Val Ala Asp Val
            20                  25                  30
Gly Thr Ser Ser Glu Thr Thr Ser Phe Thr Ser Ala Ser Ala Ala Gly
        35                  40                  45
Leu Gln Gln Pro Leu Phe Pro Leu Gly Leu Ser Leu Glu Asn Gly Arg
    50                  55                  60
Asp Asp Val Ser Asp Ala Gly Ala Tyr Ala Val Lys His Glu Arg Glu
65                  70                  75                  80
Gly Ile Asn Ile Gly Asn Leu Tyr Ala Gly Leu Glu His Leu Gln Ser
                85                  90                  95
His Ala Val Arg His Ala Val Pro Pro Val His His Ile Gln Ser Phe
            100                 105                 110
Gln Gly Gln Pro Thr Thr Ser Thr Thr Met Thr Val Pro His Ser Pro
        115                 120                 125
Ala Ile Arg Pro Arg Val Arg Ala Arg Arg Gly Gln Ala Thr Asp Pro
    130                 135                 140
His Ser Ile Ala Glu Arg Leu Arg Arg Glu Arg Ile Ser Glu Arg Ile
145                 150                 155                 160
Lys Ala Leu Gln Glu Leu Val Pro Ser Cys Asn Lys Thr Asp Arg Ala
                165                 170                 175
Ala Met Leu Asp Glu Ile Leu Asp Tyr Val Lys Phe Leu Arg Leu Gln


            180                 185                 190
Val Lys Val Leu Ser Met Ser Arg Leu Gly Gly Ala Ser Ala Val Ala
        195                 200                 205
Gln Leu Val Ala Asp Ile Pro Leu Gln Ser Met Glu Gly Asp Ser Ala
    210                 215                 220
Glu Ser Lys Ser Asn Gln His Ile Trp Glu Lys Trp Ser Asn Val Asp
225                 230                 235                 240
Thr Glu Gln Glu Val Ala Lys Leu Met Glu Glu Asp Val Gly Ala Ala
                245                 250                 255
Met Gln Tyr Leu Gln Ser Lys Ser Leu Cys Ile Met Pro Ile Ser Leu
            260                 265                 270
Ala Ala Leu Ile Tyr Pro Thr Gln Gln Pro Asp Asp Met Ser Met Val
        275                 280                 285
Lys Pro Glu Pro Ala Ala Pro Ser
    290                 295
```

<210> 288
<211> 443
<212> PRT
<213> Nicotiana benthamiana

<400> 288

Met Gln Ala Met Asn Gln Tyr Asp Pro Thr Ser Ser His Asp Asp Phe
1               5                   10                  15
Leu Asp Gln Ile Leu Ser Ser Val Pro Ser Ser Ser Leu Cys Trp Pro
            20                  25                  30
Asp Leu Ser Lys Ser Trp Asp Pro His His His His Leu Ser Pro Pro
        35                  40                  45
Leu Pro Pro Asn Pro Ser Ser Gly Asp Asp His Gln Leu Pro Pro Ser
    50                  55                  60
Asn Pro Leu Gln His Phe Gln Tyr Asp Asp Gln Ser Ser Ser Phe Leu
65                  70                  75                  80
Ala Ala Lys Leu Arg Gln His Gln Ile Thr Gly Gly Gly Gly Gly Gly
                85                  90                  95
Gly Asp Ala Val Ala Ala Ala Lys Ala Leu Leu Leu Gln Gln Gln Leu
            100                 105                 110
Leu Leu Ser Arg Thr Leu Ala Gly Asn Gly Leu Arg Ser Pro Thr Gly
        115                 120                 125
Ala Ser Gly Asp Asn Gly Phe Leu Asn Met Leu Gly Asn Gly Asp Gln
    130                 135                 140
Asn Asp Ser Val Gly Asn Pro Ala Asn Asp Ser Ser Val Gln Ala Leu
145                 150                 155                 160
Phe Asn Gly Phe Thr Gly Ser Leu Gly Gln Asn Ser Ser Gln Pro Gln
                165                 170                 175
His Phe Leu His Pro Gln Gly Gly Arg Met Gln Ala Gln Ser Phe Gly
            180                 185                 190
Ala Pro Ala Thr Ala Pro Ala Met Asn Gln Thr Pro Ala Ala Ser Gly
        195                 200                 205
Ser Ala Gly Gly Gly Thr Thr Pro Ala Ala Gln Pro Lys Gln Gln Arg
    210                 215                 220
Val Arg Ala Arg Arg Gly Gln Ala Thr Asp Pro His Ser Ile Ala Glu
225                 230                 235                 240
Arg Leu Arg Arg Glu Arg Ile Ala Glu Arg Met Lys Ala Leu Gln Glu
                245                 250                 255
Leu Val Pro Asn Ala Asn Lys Thr Asp Lys Ala Ser Met Leu Asp Glu
            260                 265                 270
Ile Ile Asp Tyr Val Lys Phe Leu Gln Leu Gln Val Lys Val Leu Ser
        275                 280                 285
Met Ser Arg Leu Gly Gly Ala Ala Ala Val Ala Pro Leu Val Ala Asp
    290                 295                 300
Met Ser Ser Glu Gly Arg Gly Glu Gly Asn Gly Gly Arg Gly Gly Asn

305                 310                 315                 320
Gly Thr Ala Ser Ser Ser Asn Asn Asp Ser Met Thr Val Thr Glu His
                325                 330                 335
Gln Val Ala Lys Leu Met Glu Glu Asp Met Gly Ser Ala Met Gln Tyr
            340                 345                 350
Leu Gln Gly Lys Gly Leu Cys Leu Met Pro Ile Ser Leu Ala Thr Ala

        355                 360                 365
Ile Ser Thr Ala Thr Cys His Ser Arg Asn Pro Met Ile Pro Asn Gly
    370                 375                 380
Asn Gly Gly Asn Asn Pro Leu Leu Gly Gly Gly Gly Gly Leu Ala Thr
385                 390                 395                 400
Asn Gly Gly Gly Gly Glu Ala Gly Gly Pro Ser Ser Pro Lys Leu Ser
                405                 410                 415
Val Leu Thr Val Gln Ser Ala Thr Ile Gly Asn Gly Gly Ile Asp Pro
            420                 425                 430
Ser Val Lys Asp Ala Thr Ser Val Phe Glu Ala
        435                 440

<210> 289
<211> 387
<212> PRT

<213> Nicotiana benthamiana

<400> 289

```
Met Gln Ala Met Asn Ser Gln Met Ser Leu Gln Glu Glu Asn Gly Ala
1               5                   10                  15
Ser Ser Gly Gln Asn Met Ser His Ser His Phe Asp Pro Thr Ser Ser
            20                  25                  30
His Asp Asp Phe Leu Gln Gln Ile Leu Ser Ser Val Pro Ser Ser Ser
        35                  40                  45
Pro Trp Pro Asp Leu Ser Ser Gly Gly Asp Gly His Phe Asp Asp Gln
    50                  55                  60
Ser Ile Phe Val Ala Ser Lys Leu Arg Gln Asn Gln Ile Thr Gly Gly
65                  70                  75                  80
Gly Gly Gly Gly Ala Ala Ala Lys Ala Leu Met Leu Gln Gln Gln Leu
                85                  90                  95
Leu Leu Ser Arg Gly Leu Leu Ala Gly Asn Gly Asp Gln Asn Asp Asp
            100                 105                 110
Val Gly Asn Pro Glu Asn Glu Ile Ser Val Gln Ala Leu Tyr Asn Gly
        115                 120                 125
Phe Ala Gly Ser Leu Gly Gln Thr Ser Asn Gln Pro Gln His Phe His
    130                 135                 140
His Ala Gln Gly Gly Ser Met Gln Ala Gln Ser Phe Gly Ala Pro Ala
145                 150                 155                 160
Ser Ser Thr Met Asn Gln Thr Pro Ala Ala Ser Gly Gly Ser Ala Gly
                165                 170                 175
Gly Gly Gln Pro Lys Gln Gln Lys Val Arg Ala Arg Arg Gly Gln Ala
            180                 185                 190
Thr Asp Pro His Ser Ile Ala Glu Arg Leu Arg Arg Glu Arg Ile Ala
        195                 200                 205
Glu Arg Met Lys Ser Leu Gln Glu Leu Val Pro Asn Ala Asn Lys Thr
    210                 215                 220
Asp Lys Ala Ser Met Leu Asp Glu Ile Ile Asp Tyr Val Arg Phe Leu
225                 230                 235                 240
Gln Leu Gln Val Lys Val Leu Ser Met Ser Arg Leu Gly Gly Ala Ala
                245                 250                 255
Ala Val Ala Pro Leu Val Ala Asp Arg Ser Ser Glu Gly Gly Gly Gly
            260                 265                 270
Asp Cys Ala Gln Ala Asn Gly Gly Gly Arg Gly Ser Asn Gly Thr Thr


        275                 280                 285
Ser Leu Ala Asn Asn Asp Ser Met Thr Met Thr Glu His Gln Val Ala
    290                 295                 300
Lys Leu Met Glu Glu Asp Met Gly Ser Ala Met Gln Tyr Leu Gln Gly
305                 310                 315                 320
Lys Gly Leu Cys Leu Met Pro Ile Ser Leu Ala Thr Ala Ile Ser Thr
                325                 330                 335
Ala Thr Cys His Ser Met Lys Leu Asn Asn Pro Leu Leu His Gly Gly
            340                 345                 350
Gly Ser Gly Gly Ser Ala Ala Ile Asn Gly Gly Gly Gly Gly Pro Ser
        355                 360                 365
Ser Pro Ser Leu Ser Ala Ser Thr Val Gln Ser Ala Thr Met Gly Asn
    370                 375                 380
Gly Gly Ala
385
```

<210> 290
<211> 302
<212> PRT
<213> Nicotiana benthamiana

<400> 290

```
Met Ala Asn Asn Pro Ser Glu Gly Pro Asp Asp Phe Phe Asp Gln Ile
1               5                   10                  15
Leu Gly Phe Pro Ala Tyr Asn Gly Ala Glu Thr Asn Leu Ala Gly Ser
            20                  25                  30
Asp Ala Gly Ala Ile Pro Pro Ala Met Leu Leu Gln Leu Asn Ser Gly
        35                  40                  45
Asp Ala Ser Gly Gln Phe Ser Gly Met Gly Leu Gly Val Gly Leu Gly
    50                  55                  60
Gly Gly Gly Gly Phe His His Pro Ser Gln Ser Gly Ser Phe Pro Leu
65                  70                  75                  80
Gly Leu Ser Leu Glu Gly Gly Lys Ser Gly Phe Met Lys Met Asp Asp
                85                  90                  95
Val Pro Ala Val Pro Gly Arg Arg Phe Arg Asp Asp Val Val Asp Ser
            100                 105                 110
Arg Ala Ser Ser Ser Val Lys Pro Gly Phe His Gly Gln Pro Met Pro
        115                 120                 125
Ser Met Pro His Pro Pro Ala Ile Arg Pro Arg Val Arg Ala Arg Arg
    130                 135                 140
Gly Gln Ala Thr Asp Pro His Ser Ile Ala Glu Arg Leu Arg Arg Glu
145                 150                 155                 160
Arg Ile Ala Glu Arg Ile Arg Ala Leu Gln Glu Leu Val Pro Asn Val
                165                 170                 175
Asn Lys Thr Asp Arg Ala Ile Met Leu Asp Glu Ile Val Asp Tyr Val
            180                 185                 190
Lys Phe Leu Arg Leu Gln Val Lys Val Leu Ser Met Ser Arg Leu Gly
        195                 200                 205
Gly Ala Ala Ala Val Ala Pro Leu Val Thr Glu Ile Pro Ile Ser Ser
    210                 215                 220
Val Glu Glu Glu Ile Ser Glu Gly Gly Asn Asn Gln Pro Ala Trp Glu
225                 230                 235                 240
Lys Trp Ser Ser Asp Gly Thr Glu Arg Gln Val Ala Lys Leu Met Glu
                245                 250                 255
Glu Asn Val Gly Ser Ala Met Gln Phe Leu Gln Ser Lys Ala Leu Cys
            260                 265                 270
Ile Met Pro Ile Ser Leu Ala Ser Ala Ile Tyr His Ser Gln Pro Pro
        275                 280                 285
Asp Thr Ser Ser Leu Ile Lys Pro Glu Thr Asn Pro Pro Ser
    290                 295                 300
```

<210> 291
<211> 298
<212> PRT
<213> Nicotiana tabacum

<400> 291

```
Met Ala Thr Asn Gln Pro Gly Gly Tyr Ala Asp Asp Phe Leu Glu Gln
1               5                   10                  15
Ile Leu Ala Ile Pro Ser Tyr Ala Gly Leu Pro Pro Val Ala Asp Val
            20                  25                  30
Gly Thr Ser Ser Glu Thr Thr Ser Phe Thr Ser Ser Ser Val Ala Ala
        35                  40                  45
Ala Gly Leu Gln Gln Pro Leu Phe Pro Leu Gly Leu Ser Leu Asp Asn
    50                  55                  60
Gly Arg Asp Asp Val Ser Asp Ala Gly Ala Tyr Ala Val Lys His Glu
65                  70                  75                  80
Arg Glu Gly Ile Asn Ile Gly Asn Leu Tyr Ala Gly Leu Glu His Leu
                85                  90                  95
Gln Ser His Ala Val Arg His Ala Val Pro Pro Val His His Ile Gln
            100                 105                 110
Pro Phe Gln Gly Gln Pro Thr Thr Ser Thr Thr Val Thr Val Pro His
        115                 120                 125
Pro Pro Ala Ile Arg Pro Arg Val Arg Ala Arg Arg Gly Gln Ala Thr
    130                 135                 140
Asp Pro His Ser Ile Ala Glu Arg Leu Arg Arg Glu Arg Ile Ser Glu
145                 150                 155                 160

Arg Ile Lys Ala Leu Gln Glu Leu Val Pro Ser Cys Asn Lys Thr Asp
                165                 170                 175
Arg Ala Ala Met Leu Asp Glu Ile Leu Asp Tyr Val Lys Phe Leu Arg
            180                 185                 190
Leu Gln Val Lys Val Leu Ser Met Ser Arg Leu Gly Gly Ala Ser Ala
        195                 200                 205
Val Ala Gln Leu Val Ala Asp Ile Pro Leu Gln Ser Val Glu Gly Asp
    210                 215                 220
Ser Ala Glu Ser Arg Ser Asn Gln His Ile Trp Glu Lys Trp Ser Asn
225                 230                 235                 240
Val Asp Thr Glu Gln Glu Val Ala Lys Leu Met Glu Glu Asp Val Gly
                245                 250                 255
Ala Ala Met Gln Tyr Leu Gln Ser Lys Ser Leu Cys Ile Met Pro Ile
            260                 265                 270
Ser Leu Ala Ala Leu Ile Tyr Pro Thr Gln Gln Pro Asp Asp Pro Ser
        275                 280                 285
Met Val Lys Pro Glu Pro Ala Ala Pro Ser
    290                 295
```

<210> 292
<211> 524
<212> PRT
<213> Oryza sativa

<400> 292

```
Met Gly Gly Phe Ala Tyr Pro Phe Thr Pro Ser Pro Ala Trp Ser Arg
1               5                   10                  15
Asp Ala Val Phe Ala Gly Ser Pro Trp Ala Ala Gly Gly Val Ser Ser
            20                  25                  30
Leu Ala Asp Ala Leu Val Ser Tyr Gly Ala Val Asp Asp Glu Glu Ala
        35                  40                  45
Ala Phe Leu Gly Lys Thr Ala Ala Ser Ser Pro Ser Thr Ala Arg Leu
    50                  55                  60
His Glu Gln Gln Gln Leu Leu Leu Glu Ala Glu Leu Leu Arg His Gly
```

```
65                  70                  75                  80
Asp Gly Leu Gly Phe Ala Ala Met Asp Asp Asp Gly Gly Ala Ala Met
                85                  90                  95
Leu Gly Ala Leu Glu Pro Cys Ala Met Pro Leu Thr Asp Ser Gly Gly
            100                 105                 110
Pro Pro Val Ile Cys Ser Ser Ser Ser Asn Asp Ser Ser Gly Ser Glu
        115                 120                 125
His Ser Ala Ala Met Pro Ala Gly Gly Gly Phe Leu Val Gly Glu Gln
    130                 135                 140
Gln Gln His Val Pro Pro Ala Ala Tyr Ala Ala Gly Gly Val Leu Pro
145                 150                 155                 160
Ser Met Ala Ala Gly Glu Glu Thr Pro Gln Ser Phe Gly Phe Gly Ser
                165                 170                 175
Leu Phe Asn Gly Asp Leu Leu Gln Glu Ala Thr Val Ser Lys Tyr His
            180                 185                 190
His His Gln Gln Gln Gln Gln Leu Gly Val Val Pro Ser Ser Gln Pro
        195                 200                 205
His His Leu Asn Asp Asp Ile Asp Phe Asn Thr Gly Lys Leu Met Ser
    210                 215                 220
Phe Ala Ser Gly Gln Gln His Val Thr Pro Ser Ile Asp Ser Leu Gln
225                 230                 235                 240
Ile Asp Gln Lys Glu Phe Ser Ser Gly Leu His His Leu Asn Leu Ser
                245                 250                 255
Ser Leu Ile Ser Gly Pro Leu Ala Ser Phe Asn Ala Thr Gln Ser His
            260                 265                 270
Arg Gln Pro Ala Glu Ala Cys Gly Gly Lys Asn Gly Gly Ala Ala Pro
        275                 280                 285
Phe Val Asn Leu Ser Glu Val Leu Pro Lys Gly Asn Gly Ser Gly Ser
    290                 295                 300
Ala Gly Asn Gly Ala Pro Lys Pro Arg Val Arg Ala Arg Arg Gly Gln
305                 310                 315                 320
Ala Thr Asp Pro His Ser Ile Ala Glu Arg Leu Arg Arg Glu Lys Ile
                325                 330                 335
Ser Asp Arg Met Lys Asp Leu Gln Glu Leu Val Pro Asn Ser Asn Lys
            340                 345                 350
Thr Asn Lys Ala Ser Met Leu Asp Glu Ile Ile Asp Tyr Val Lys Phe
        355                 360                 365
Leu Gln Leu Gln Val Lys Val Leu Ser Met Ser Arg Leu Gly Ala Ala
    370                 375                 380
Glu Ala Val Val Pro Leu Leu Thr Glu Thr Gln Thr Glu Ser Pro Gly
385                 390                 395                 400
Phe Leu Leu Ser Pro Arg Ser Ser Ser Gly Glu Arg Gln Ala Gly Ala
                405                 410                 415
Gly Ala Val Thr Gly Gly Leu Pro Gly Asp Gln Pro Glu Leu Leu Asp
            420                 425                 430
Gly Gly Ala Met Phe Glu Gln Glu Val Val Lys Leu Met Glu Asp Asn
        435                 440                 445
Met Thr Thr Ala Met Gln Tyr Leu Gln Ser Lys Gly Leu Cys Leu Met
    450                 455                 460
Pro Val Ala Leu Ala Ser Ala Ile Ser Ala Gln Lys Gly Thr Ser Ser
465                 470                 475                 480
Ala Ala Val Arg Pro Glu Lys Lys Lys Asn Gly Asp Gly Asp Gly Gly
                485                 490                 495
Gly Asp Glu Glu Asp Val Lys Gly Glu Phe Asp Ala Pro Arg Arg Pro
            500                 505                 510
Pro Val Gly Arg Pro Lys Glu Met Arg Ser Arg Val
        515                 520
```

<210> 293
<211> 441
<212> PRT
<213> Oryza sativa

<400> 293

```
Met Gln Pro Asn Ala Arg Gln Met Arg Gly Gly Gly Gly Gly Gly Gly
1               5                   10                  15
Gly Gly Gln Asp Asp Phe Phe Asp Gln Met Leu Ser Thr Leu Pro Ala
            20                  25                  30
Val Trp Ser Glu Leu Gly Ser Gly Lys Pro Ala Trp Asp Leu Thr Ala
        35                  40                  45
Gly Ala Val Gly Gly Gly Gly Gly Ala Ser Asp Asp His Ser Ala Ala
    50                  55                  60
Ala Phe Asp Asp Ser Ala Leu Leu Ala Ser Arg Leu Arg Gln His Gln
65                  70                  75                  80
Ile Asp Gly Gly Gly Asp Lys Pro Ile Met Leu Gln Leu Ser Asp Leu
                85                  90                  95
His Arg His His Gly Leu Ala Ala Gly Asp Asp Ser Gly Gly Ala Ala
            100                 105                 110
Gly Phe Leu Pro Leu Ser Leu Phe Ala Asp Arg Ser Gln Asp Asp Ile
        115                 120                 125
Asp Ala Ala Phe Lys Ser Pro Asn Gly Ala Arg Gly Asp His Ala Leu
    130                 135                 140
Tyr Asn Gly Phe Gly Ala Ala Gly Met His Gly Ala Ala Ala Met Gln
145                 150                 155                 160
Pro Pro Pro Phe Gly Gln Gly Gly Ser Met Pro Ala Gln Ser Phe Gly
                165                 170                 175
Gly Gly Ala Ala Ala Ser Gly Gly Gly Gly Gly Gly Ser Ala Ser Ala
            180                 185                 190
Ala Ala Ala Ala Gly Ala Ser Ser Gly Gly Gly Ala Ala Ala Pro Pro
        195                 200                 205
Arg Gln Arg Gln Arg Ala Arg Arg Gly Gln Ala Thr Asp Pro His Ser
    210                 215                 220
Ile Ala Glu Arg Leu Arg Arg Glu Arg Ile Ala Glu Arg Met Lys Ala
225                 230                 235                 240
Leu Gln Glu Leu Val Pro Asn Ala Asn Lys Thr Asp Lys Ala Ser Met
                245                 250                 255
Leu Asp Glu Ile Ile Asp Tyr Val Lys Phe Leu Gln Leu Gln Val Lys
            260                 265                 270
Val Leu Ser Met Ser Arg Leu Gly Gly Ala Ser Ala Val Ala Pro Leu
        275                 280                 285
Val Ala Asn Met Ser Ser Glu Ser Asn Gly Asn Gly Asn Ala Thr Ser
    290                 295                 300
Ser Ser Gly Asn Gly Glu Ala Ala Asn Gly Ser Ser Asn Gly Asp Asn
305                 310                 315                 320
Asn Gly Gly Gly Thr Leu Arg Val Thr Glu Gln Gln Val Ala Lys Leu
                325                 330                 335
Met Glu Glu Asp Met Gly Ser Ala Met Gln Tyr Leu Gln Gly Lys Gly

            340                 345                 350
Leu Cys Leu Met Pro Ile Ser Leu Ala Thr Ala Ile Ser Ser Ala Thr
        355                 360                 365
Ser Ser Ser Leu Leu Pro Arg Thr Gly Gly Gly Ala Gly Gly Ser Leu
    370                 375                 380
His Glu Gly Gly Asn Gly Thr Ser Pro Pro Leu Val Asn Gly Thr Ala
385                 390                 395                 400
Thr Gly Cys Asp Asp Ala Gly Val Phe Phe Ser Val Lys Phe Val Val
                405                 410                 415
Glu Leu Ser Phe Leu Leu Leu Asn Glu Asp Cys Arg Gly Lys Glu Glu
            420                 425                 430
Ser Lys Leu Leu Val Gln Lys Gly Pro
        435                 440
```

<210> 294
<211> 294
<212> PRT

<213> Oryza sativa

<400> 294

```
Met Ala Gly Gln Gln Pro Gln Gln Gln Gly Pro Pro Glu Asp Asp Phe
1               5                   10                  15
Phe Asp Gln Phe Phe Ser Leu Thr Ser Ser Phe Pro Gly Ala Ala Pro
            20                  25                  30
Gly Gly Arg Ala Ala Gly Asp Gln Pro Phe Ser Leu Ala Leu Ser Leu
        35                  40                  45
Asp Ala Ala Ala Ala Ala Glu Ala Ser Gly Ser Gly Lys Arg Leu Gly
    50                  55                  60
Val Gly Asp Asp Ala Glu Gly Gly Gly Ser Lys Ala Asp Arg Glu Thr
65                  70                  75                  80
Val Gln Leu Thr Gly Leu Phe Pro Pro Val Phe Gly Gly Gly Gly Val
                85                  90                  95
Gln Pro Pro Asn Leu Arg Pro Thr Pro Pro Thr Gln Val Phe His Pro
            100                 105                 110
Gln Gln Ser Lys Gln Gly Gly Ala Ala Val Gly Pro Gln Pro Pro Ala
        115                 120                 125
Pro Arg Pro Lys Val Arg Ala Arg Arg Gly Gln Ala Thr Asp Pro His
    130                 135                 140
Ser Ile Ala Glu Arg Leu Arg Arg Glu Arg Ile Ala Glu Arg Met Arg
145                 150                 155                 160
Ala Leu Gln Glu Leu Val Pro Asn Thr Asn Lys Thr Asp Arg Ala Ala
                165                 170                 175
Met Leu Asp Glu Ile Leu Asp Tyr Val Lys Phe Leu Arg Leu Gln Val
            180                 185                 190
Lys Val Leu Ser Met Ser Arg Leu Gly Gly Ala Gly Ala Val Ala Gln
        195                 200                 205
Leu Val Ala Asp Ile Pro Leu Ser Val Lys Gly Glu Ala Ser Asp Ser
    210                 215                 220
Gly Gly Asn Gln Gln Ile Trp Glu Lys Trp Ser Thr Asp Gly Thr Glu
225                 230                 235                 240
Arg Gln Val Ala Lys Leu Met Glu Glu Asp Ile Gly Ala Ala Met Gln
                245                 250                 255
Phe Leu Gln Ser Lys Ala Leu Cys Met Met Pro Ile Ser Leu Ala Met
            260                 265                 270
Ala Ile Tyr Asp Thr Gln Gln Thr Gln Asp Gly Gln Pro Val Lys His
        275                 280                 285
Glu Pro Asn Thr Pro Ser
    290
```

<210> 295
<211> 367
<212> PRT
<213> Oryza sativa

<400> 295

```
Met Gln Pro Ser Ser Arg Asp Thr Val Ala Gly Gly Gly Gly Glu Gly
1               5                   10                  15
Thr Gln Asp Asp Phe Phe Asp Gln Met Leu Ser Thr Leu Pro Ser Ala
            20                  25                  30
Trp Ala Asp Leu Gly Gly Gly Gly Gly Gly Ala Ala Gly Lys Ser Pro
        35                  40                  45
Trp Glu Val Asp Pro Ala Ala Ala Ala Ala Ala Ser Gln Val Phe Asp
    50                  55                  60
Glu Ser Ala Leu Leu Ala Ser Arg Leu Arg His His Gln Ile Gly Gly
65                  70                  75                  80
```

```
Ala Gly Gly Gly Gly Gly Glu Lys Pro Val Met Leu Gln Leu Ser Glu
                85                  90                  95
Leu His Arg Gln Ala Gly Gly Gly Glu Glu Asp Gly Ser Gly Ala Phe
            100                 105                 110
Ser Pro Leu Pro Leu Phe Thr Asp Arg Thr Asn Val Pro Pro Arg Glu
        115                 120                 125
Glu Met Glu Gly Gly Phe Lys Ser Pro Asn Ala Ala Ala Gly Gly Glu
    130                 135                 140
His Ala Leu Phe Asn Gly Phe Gly Val His Gly Gly Ser Gly Gly Ala
145                 150                 155                 160
Gly Gln Pro Pro Phe Gly Gln Leu Arg Arg Glu Arg Ile Ala Glu Arg
                165                 170                 175
Met Lys Ser Leu Gln Glu Leu Val Pro Asn Ala Asn Lys Thr Asp Lys
            180                 185                 190
Ala Ser Met Leu Asp Glu Ile Ile Asp Tyr Val Lys Phe Leu Gln Leu
        195                 200                 205
Gln Val Lys Val Leu Ser Met Ser Arg Leu Gly Gly Ala Ala Gly Met
    210                 215                 220
Ala Pro Leu Val Ala Ser Met Ser Ser Glu Gly Asn Ser Asn Gly Ser
225                 230                 235                 240
Ser Asn Gly Gly Gly Gly Lys Ala Ser Lys Gly Gly Thr Gly Gly Glu
                245                 250                 255
Gly Gly Gly Gly Gly Gly Gly Gly Gly Gly Gly Gly Thr Gly Gly Gly
            260                 265                 270
Met Arg Val Thr Glu Gln Gln Val Ala Lys Met Met Glu Glu Asp Met
        275                 280                 285
Gly Thr Ala Met Gln Tyr Leu Gln Gly Lys Gly Leu Cys Leu Met Pro
    290                 295                 300
Ile Ser Leu Ala Ser Ala Ile Ser Ser Ala Thr Ser Ser Ala Ser Leu
305                 310                 315                 320
Leu Ser Arg Pro Ser Ile Arg His Ala Gly Ala Pro Pro Gln Thr Met
                325                 330                 335
Leu Asp Ala Ala Gly Pro Thr Ser Pro Ala Ala Met Ser Asn Gly Asp
            340                 345                 350
Asp Pro Arg His Ala Lys Ala Asp Gly Gly Ala Gly Gly Thr Gln
        355                 360                 365
```

<210> 296
<211> 478
<212> PRT
<213> Oryza sativa

<400> 296

```
Met Asp Tyr Ser Ala Gly Ser Tyr Met Trp Pro Gly Asn Ser Gly Ser
1               5                   10                  15
Glu Asn Tyr Asn Phe Val Asp Gly Ser Ser Glu Ser Tyr Ala Glu Glu
            20                  25                  30
Gly Ser Leu Pro Pro Ser Gly Tyr Phe Met Gly Ala Gly Ser Asp Arg
        35                  40                  45
Ser Leu Lys Ile Thr Glu Asn Glu Arg Asn Pro Thr Met Leu Ala Asn
    50                  55                  60
Gly Cys Leu Pro Tyr Asn Thr Gln Ala His Pro Leu Ser Gly Gln Ile
65                  70                  75                  80
Leu Pro Lys Gly Glu Leu Pro Asn Asn Leu Leu Asp Leu Gln Gln Leu
                85                  90                  95
Gln Asn Ser Ser Asn Leu Arg Ser Asn Ser Ile Pro Pro Gly Val Leu
            100                 105                 110
Gln Cys Asn Ser Thr Ser Gly Thr Phe Asp Ala Lys Leu Asp Thr Pro
        115                 120                 125
Gly Leu Ala Glu Leu Pro His Ala Leu Ser Ser Ser Ile Asp Ser Asn
    130                 135                 140
```

```
Gly Ser Asp Ile Ser Ala Phe Leu Ala Asp Val His Ala Val Ser Ser
145                 150                 155                 160
Ala Pro Thr Leu Cys Ser Ala Phe Gln Asn Val Ser Ser Phe Met Glu
                165                 170                 175
Pro Val Asn Leu Asp Ala Phe Gly Phe Gln Gly Ala Gln Asn Val Ala
            180                 185                 190
Met Leu Asn Lys Thr Ser Leu Pro Asn Gly Asn Pro Ser Leu Phe Asp
        195                 200                 205
Asn Ala Ala Ile Ala Ser Leu His Asp Ser Lys Glu Phe Leu Asn Gly
    210                 215                 220
Gly Ser Ile Pro Ser Phe Gly Thr Val Leu Gln Ala Leu Gly Ala Gly
225                 230                 235                 240
Gly Leu Lys Ala Ala Gln Gln Glu Gln Asn Ile Arg Asn Ile Pro Leu
                245                 250                 255
Pro Thr Phe Thr Ser Gly Ser His Leu Ala Val Thr Asp Ala Gln Gly
            260                 265                 270
Pro Pro Leu Pro Ser Lys Ile Pro Pro Leu Ile His Asp His Asn Ser
        275                 280                 285
Glu Tyr Pro Ile Asn His Ser Ser Asp Val Glu Pro Gln Ala Asn Ser
    290                 295                 300
Ala Pro Gly Asn Ser Ala Asn Ala Lys Pro Arg Thr Arg Ala Arg Arg
305                 310                 315                 320
Gly Gln Ala Thr Asp Pro His Ser Ile Ala Glu Arg Leu Arg Arg Glu
                325                 330                 335
Lys Ile Ser Glu Arg Met Lys Asn Leu Gln Val Leu Val Pro Asn Ser
            340                 345                 350
Asn Lys Ala Asp Lys Ala Ser Met Leu Asp Glu Ile Ile Asp Tyr Val
        355                 360                 365
Lys Phe Leu Gln Leu Gln Val Lys Val Leu Ser Met Ser Arg Leu Gly
    370                 375                 380
Ala Pro Gly Ala Val Leu Pro Leu Leu Arg Glu Ser Gln Thr Glu Cys
385                 390                 395                 400
His Ser Asn Pro Ser Leu Ser Ala Ser Thr Ile Ser Gln Gly Pro Pro
                405                 410                 415
Asp Met Pro Asp Ser Glu Asp Ser Ser Ala Phe Glu Gln Glu Val Val
            420                 425                 430
Lys Leu Met Glu Thr Ser Ile Ile Ser Ala Met Gln Tyr Leu Gln Asn
        435                 440                 445
Lys Gly Leu Cys Leu Met Pro Ile Ala Leu Ala Ser Ala Ile Ser Asn
    450                 455                 460
Gln Lys Gly Met Ala Ala Ala Ala Ala Ile Pro Pro Glu Lys
465                 470                 475
```

<210> 297
<211> 499
<212> PRT
<213> Oryza sativa

<400> 297

```
Met Ala Ala Ala Ala Ala Ala Gly Gln Ile Ser Leu Asp Asp Leu Arg
1               5                   10                  15
Asn Gly Gly Gly Val Ala Ala Asn Ala Gly Gly Gly Gly Gly Val His
            20                  25                  30
Asp Asp Phe Leu Asp Gln Met Leu Ser Ser Leu Pro Pro Ser Ala Trp
        35                  40                  45
Pro Asp Leu Ala Ala Gly Lys Ala Ala Glu Asp Asp Ala Glu Gly Met
    50                  55                  60
His His His His His Gln Gln Gln Gln Gln Phe Gly Gly Pro Tyr Asp
65                  70                  75                  80
Glu Ser Ala Met Leu Ala Ser Arg Leu Arg Gln His Gln Ile Ser Gly
                85                  90                  95
```

```
Gly Gly Gly Gly Gly Gly Gly Gly Ala Ala Ala Val Lys Gln Met Val
            100                 105                 110
Leu Gln Gln Leu Ala Asp Leu Arg Gln Gly His His Met Met Leu Gln
        115                 120                 125
Gly Leu Gly Gly Arg Ser Pro Ala Gly Gly Gly Gly Gly Gly Gly Asp
    130                 135                 140
Gly Gly Leu Leu Leu Pro Leu Thr Leu Gly Ser Gly Gly Ser Gly Gly
145                 150                 155                 160
Asp Val Gln Ala Leu Leu Lys Ala Ala Ala Ala Asn Ser Ala Gly Gly
                165                 170                 175
Gly Asp Ala Gly Gly Val Tyr Gly Gly Gly Phe Ala Gly Ser Leu His
            180                 185                 190
Gln Gln Gln Gln His Phe Gln Pro His Pro Gln Thr Ala Pro Thr Ile
        195                 200                 205
Pro Thr Gln Ser Phe Gly Gly Gly Gly Gly Gly Gly Gly Gly Gly Thr
    210                 215                 220
Ala Ser Gly Gly Gly Ala Ala Gln Pro Gln Ala Gly Ala Ala Gly Gly
225                 230                 235                 240
Gly Ala Pro Ala Pro Pro Arg Gln Arg Val Arg Ala Arg Arg Gly Gln
                245                 250                 255
Ala Thr Asp Pro His Ser Ile Ala Glu Arg Leu Arg Arg Glu Arg Ile
            260                 265                 270
Ala Glu Arg Met Lys Ala Leu Gln Glu Leu Val Pro Asn Ala Asn Lys
        275                 280                 285
Leu Met Gln Thr Asp Lys Ala Ser Met Leu Asp Glu Ile Ile Asp Tyr
    290                 295                 300
Val Lys Phe Leu Gln Leu Gln Val Lys Ala Ser Thr Tyr Thr Lys Leu
305                 310                 315                 320
Leu Ile His Val Leu Ser Met Ser Arg Leu Gly Gly Ala Ala Ala Val
                325                 330                 335
Ala Pro Leu Val Ala Asp Met Ser Ser Glu Gly Arg Gly Gly Gly Ala
            340                 345                 350
Ala Asn Gly Gly Ala Pro Ala Ala Ala Gly Ser Asp Ser Leu Thr Val
        355                 360                 365
Thr Glu Gln Gln Val Ala Lys Leu Met Glu Glu Asp Met Gly Thr Ala
    370                 375                 380
Met Gln Tyr Leu Gln Gly Lys Gly Leu Cys Leu Met Pro Ile Ser Leu
385                 390                 395                 400
Ala Ser Ala Ile Ser Ser Ala Thr Cys His Leu Arg Pro Pro Val Val
                405                 410                 415
Ala Ala Ala Gln Gln Phe Pro Ala Gly Leu Gly Ala Ala Ala Ala Ala
            420                 425                 430
Ala His His His Gln Leu Ser Ala Ala Ala Ala Ala Ala Ala Met Arg
        435                 440                 445
Gly His Leu Pro Gly Leu Asn Ala Asp Gly Ser Val Pro Ala Ser Pro
    450                 455                 460
Ser Met Ser Val Leu Thr Ala Gln Ser Ala Met Ala Asn Gly Gly Gly
465                 470                 475                 480
Gly Ala Ala Asp Gly Glu Gly Ser Gln Leu Lys Asp Ala Ala Ser Val
                485                 490                 495
Ser Lys Pro
```

<210> 298
<211> 293
<212> PRT
<213> Oryza sativa

<400> 298

```
Met Ala Gly Gln Gln Pro Pro Thr Gly Gly Ala Glu Asp Asp Phe Phe
1               5                   10                  15
```

```
Asp His Phe Phe Ser Ile Pro Ser Ala Ala Ala Ala Gly Ala Gly Gly
            20                  25                  30
Val Gly Gly Phe Gly Ser Gly Asp His His Pro Phe Pro Leu Ala Leu
        35                  40                  45
Ser Leu Asp Ala Glu Gly Ala Gly Ala Ala Arg Arg Leu Leu Asp Gly
    50                  55                  60
Gly His Asp Gly Gly Arg Thr Asp Arg Asp Pro Val Gln Leu Ala Gly
65                  70                  75                  80
Leu Phe Ala Pro Val Phe Gly Ala Ala Ala Gly Val Gln Pro Pro His
                85                  90                  95
Leu Arg Ala Pro Pro Pro Pro Gln Val Phe His Ala Gln Pro Lys Pro
            100                 105                 110
Gly Glu Gly Ala Met Ala Ala Pro Gln Pro Gln Gln Pro Pro Ala Pro
        115                 120                 125
Arg Pro Lys Val Arg Ala Arg Arg Gly Gln Ala Thr Asp Pro His Ser
    130                 135                 140
Ile Ala Glu Arg Leu Arg Arg Glu Arg Ile Ala Glu Arg Met Arg Ala
145                 150                 155                 160
Leu Gln Asp Leu Val Pro Asn Thr Asn Lys Thr Asp Arg Ala Ala Met
                165                 170                 175
Leu Asp Glu Ile Leu Asp Tyr Val Lys Phe Leu Arg Leu Gln Val Lys
            180                 185                 190
Val Leu Ser Met Ser Arg Leu Gly Gly Ala Gly Ala Val Ala Gln Leu
        195                 200                 205
Val Ala Asp Ile Pro Ile Ser Val Lys Gly Glu Ala Ser Asp Ser Gly
    210                 215                 220
Ser Lys Gln Gln Ile Trp Glu Lys Trp Ser Thr Asp Gly Thr Glu Lys
225                 230                 235                 240
Gln Val Ala Lys Leu Met Glu Glu Asp Ile Gly Ala Ala Met Gln Phe
                245                 250                 255
Leu Gln Ser Lys Ala Leu Cys Met Met Pro Ile Ser Leu Ala Met Ala
            260                 265                 270
Ile Tyr Asp Thr Gln His Ser Gln Asp Gly His Ser Val Lys Pro Glu
        275                 280                 285
Pro Asn Thr Pro Ser
    290
```

<210> 299
<211> 274
<212> PRT
<213> Picea abies

<220>
<221> misc_feature
<222> (257)..(259)
<223> Xaa can be any naturally occurring amino acid

<400> 299

```
Met Leu Pro Leu Pro Leu Ser Leu Gly Gln Ala Gly Lys Ser Gly Asp
1               5                   10                  15
Met Asn Glu Pro Gly Ser Arg Glu Glu Ile Glu Ala Ser Phe Lys Ser
            20                  25                  30
Val Asn Asn Ala Arg Asp Ser Asn Leu Gly Gly Leu Phe Gln Pro Phe
        35                  40                  45
Ala Val Ser Pro Arg Gly Val Arg Pro Thr Gly Gln Asn Phe His Ala
    50                  55                  60
Gln Pro Gly Gln Val Pro Leu Gln Gly Tyr Gly Gly Met Pro Gln Pro
65                  70                  75                  80
Gln His Gln Asn Pro Pro Pro Thr Gly Val Gly Ala Ala Pro Pro Val
                85                  90                  95
Arg Pro Arg Val Arg Ala Arg Arg Gly Gln Ala Thr Asp Pro His Ser


            100                 105                 110
Ile Ala Glu Arg Leu Arg Arg Glu Arg Ile Ala Glu Arg Met Lys Ala
        115                 120                 125
Leu Gln Glu Leu Val Pro Asn Ser Asn Lys Thr Asp Lys Ala Ser Met
    130                 135                 140
Leu Asp Glu Ile Ile Asp Tyr Val Lys Phe Leu Gln Leu Gln Val Lys
145                 150                 155                 160
Val Leu Ser Met Ser Arg Leu Gly Gly Ala Gly Ala Val Ala Pro Leu
                165                 170                 175
Val Ala Asp Ile Pro Ala Glu Gly Ala Asn Ala Pro Gln Gly Thr Arg
            180                 185                 190
Thr Asn Gly Ser Gln Asn Ser Ser Pro Asp Gly Leu Ala Leu Thr Glu
        195                 200                 205
Arg Gln Val Ala Lys Leu Met Glu Glu Asp Met Gly Thr Ala Met Gln
    210                 215                 220
Tyr Leu Gln Gly Lys Gly Leu Cys Leu Met Pro Ile Ser Leu Ala Ser
225                 230                 235                 240
Ala Ile Asn Asn Ser Gly Ser Arg Pro Gln Ala Pro Ser Thr Pro Ser
                245                 250                 255
Xaa Xaa Xaa Leu Leu Ala Ser Asn Val Asn Asp Arg Gln Val Leu Glu
            260                 265                 270
Pro Ser
```

<210> 300
<211> 300
<212> PRT
<213> Populus deltoides

<400> 300

```
Met Ala Asn Asn Pro Thr Glu Pro Pro Ala Asp Asp Phe Leu Gln Glu
1               5                   10                  15
Ile Leu Gly Met Pro Asn Phe Ala Ser Ala Glu Ala Gly Leu Val Gly
            20                  25                  30
Ala Asp Ala Gly Leu Ala Gly Ala Ala Ala Ala Gln Ala Ser Met Met
        35                  40                  45
Leu Gln Leu Ser Ser Gly Asp Gly Ser Gly His Ile Ser Asp Leu Gly
    50                  55                  60
Gly Ala Pro Gly Gly Gly Ser Ala Gly Phe His Gly Phe Pro Leu Gly
65                  70                  75                  80
Leu Ser Leu Glu Gln Gly Lys Gly Gly Phe Leu Lys Pro Glu Glu Ala
                85                  90                  95
Ser Gly Ser Gly Lys Arg Phe Arg Asp Glu Ile Val Asp Gly Arg Ala
            100                 105                 110
Lys Asn Val Phe His Gly Gln Pro Met Pro Thr Thr Val Ala Ile Ala
        115                 120                 125
Pro His Pro Pro Ala Met Arg Pro Arg Val Arg Ala Arg Arg Gly Gln
    130                 135                 140
Ala Thr Asp Pro His Ser Ile Ala Glu Arg Leu Arg Arg Glu Arg Ile
145                 150                 155                 160
Ala Glu Arg Ile Arg Ala Leu Gln Glu Leu Val Pro Ser Val Asn Lys
                165                 170                 175
Thr Asp Arg Ala Thr Met Leu Asp Glu Ile Val Asp Tyr Val Lys Phe
            180                 185                 190
Leu Arg Leu Gln Val Lys Val Leu Ser Met Ser Arg Leu Gly Gly Ala
        195                 200                 205
Gly Ala Val Ala Pro Leu Val Thr Asp Ile Pro Leu Ser Ser Val Glu
    210                 215                 220
Asp Glu Thr Gly Glu Gly Gly Arg Asn Gln Pro Ala Trp Glu Lys Trp
225                 230                 235                 240
Ser Asn Asp Gly Thr Glu Arg Gln Val Ala Lys Leu Met Glu Glu Asn

                245                 250                 255
Val Gly Ala Ala Met Gln Phe Leu Gln Ser Lys Ala Leu Cys Ile Met
            260                 265                 270
Pro Ile Ser Leu Ala Thr Ala Ile Tyr His Thr Gln Pro Pro Asp Thr
        275                 280                 285
Thr Ser Ile Val Lys Pro Glu Thr Asn Pro Gln Ser
    290                 295                 300
```

<210> 301
<211> 463
<212> PRT
<213> Pinus radiata

<400> 301

```
Met Ser Leu Leu Pro Pro Gly Ile Gly Gly Gln Gly Leu Asn Ser Gln
1               5                   10                  15
Glu Asn Asp Pro Tyr Pro Tyr Asp Asp Ser Gln Phe Leu Ala Asn Arg
            20                  25                  30
Leu Arg Gln His Gln Leu Ser Gly Asn Ser Ser Met Asn Gln Thr Pro
        35                  40                  45
Asn Gln Ala Ser Gln Gly Ile Asn Glu Thr Asn Thr Ser Pro Gly Arg
    50                  55                  60
Ser Met Val Leu Gln Leu Ser Thr Gly Ser Ala Ser Ala Ser Gln Leu
65                  70                  75                  80
Leu Ala Ser Met Gly Asn Ser Pro Arg Gly Thr Ala Val Met Thr Gln
                85                  90                  95
Ser Pro Ser Thr Gly Gly Ser Cys Asn Gly Ser Asp Gly Gly Met Leu
            100                 105                 110
Pro Leu Pro Leu Ser Leu Gly Gln Ala Gly Lys Ser Gly Asp Ile Asn
        115                 120                 125
Glu Ser Arg Ser Arg Asp Glu Ile Glu Ala Ser Phe Lys Ser Ala Asn
    130                 135                 140
His Ala Arg Asp Ser Asn Leu Gly Gly Leu Phe Pro Pro Phe Ala Ala
145                 150                 155                 160
Ser Pro Arg Gly Val Arg Pro Thr Gly Gln Asn Phe His Thr Gln Ser
                165                 170                 175
Gly Gln Val Pro Met Gln Val Tyr Gly Gly Met Pro Gln Pro Gln His
            180                 185                 190
Gln Asn Pro Ser Pro Thr Gly Val Gly Ala Ala Pro Pro Val Arg Pro
        195                 200                 205
Arg Val Arg Ala Arg Arg Gly Gln Ala Thr Asp Pro His Ser Ile Ala
    210                 215                 220
Glu Arg Leu Arg Arg Glu Arg Ile Ala Glu Arg Met Lys Ala Leu Gln
225                 230                 235                 240
Glu Leu Val Pro Asn Ser Asn Lys Thr Asp Lys Ala Ser Met Leu Asp
                245                 250                 255
Glu Ile Ile Asp Tyr Val Lys Phe Leu Gln Leu Gln Val Lys Val Leu
            260                 265                 270
Ser Met Ser Arg Leu Gly Gly Ala Gly Ala Val Ala Pro Leu Val Ala
        275                 280                 285
Asp Ile Pro Ala Glu Gly Ala Asn Ala Pro Gln Gly Thr Arg Thr Asn
    290                 295                 300
Ser Ser Gln Asn Ser Ser Pro Asp Gly Leu Ala Leu Thr Glu Arg Gln
305                 310                 315                 320
Val Ala Lys Leu Met Glu Glu Asp Met Gly Ser Ala Met Gln Tyr Leu
                325                 330                 335
Gln Gly Lys Gly Leu Cys Leu Met Pro Ile Ser Leu Ala Ser Ala Ile
            340                 345                 350
Asn Asn Ser Gly Ala Arg Pro Gln Ala Pro Ser Thr Pro Ser Leu Gln
        355                 360                 365
Gly Leu Leu Pro Pro Asn Ala Asn Asp Arg Gln Ile Leu Glu Pro Ser
    370                 375                 380
Asn Ser Ala Leu Ser Ala Leu Thr Ser Thr Ser Met Thr Ile Gln Ser
385                 390                 395                 400
Ser Ile Ser Ser Pro Gly Ser Gly Ile Thr Glu Gln Gly Asp Asn Ala
                405                 410                 415
His Lys Ala Val Asn Gly Thr Arg Asn Pro Lys Asp Ser Arg Glu Ala
            420                 425                 430
Asn Ser Val Thr Lys Ser Asn Gly Ile Gly Gly Pro Ala Leu Ser Lys
        435                 440                 445
Asn Ala Val Lys Gly Glu Asp Glu Pro Gln Arg Arg Thr Gly Gln
    450                 455                 460
```

<210> 302

<211> 325
<212> PRT
<213> Picea sitchensis

<400> 302

```
Met Asp Asp Ile Phe Asp Gln Leu Leu Ser Ser Ser Trp Glu Asp Ile
1               5                   10                  15
Asn Gly Ala Asp Arg Ser Ser Leu Asp Ser Ser Ala Gly Gly Pro Thr
            20                  25                  30
Asn Cys Leu Pro Gly Asp Ser Met Gly Thr Leu Gln Ala Ser Ser Arg
        35                  40                  45
Val Ser Thr Met Ser Val Thr Pro Ser Ser His Met Ser Pro Asn Leu
    50                  55                  60
Glu Gly Gln Ala Gln Asn Asn Asp Val Gln Asn Ser Ser Ser Ser Val
65                  70                  75                  80
Ile Ala Gly Glu Asn Thr Pro Tyr Leu Leu Lys Glu Leu Leu Val Cys
                85                  90                  95
Ser Gln Ala Thr Asp Pro His Ser Ile Ala Glu Arg Glu Arg Arg Glu
            100                 105                 110
Arg Ile Ala Lys Asn Leu Lys Ser Leu Gln Glu Leu Val Pro Asn Ala
        115                 120                 125
Asn Lys Thr Asp Lys Ala Ser Met Leu Asp Glu Ile Ile Asp Tyr Val
    130                 135                 140
Lys Phe Leu Gln Leu Gln Val Lys Val Leu Ser Met Ser Arg Leu Gly
145                 150                 155                 160
Gly Ala Gly Ala Val Ala Pro Leu Ile Ala Asp Val Pro Ala Glu Gly
                165                 170                 175
Ser Gly Ser Leu Ala Ser Ala Ala Leu Gly Gln Ala Gly Gly Ser Leu
            180                 185                 190
Ser Gln Asp Gly Leu Ala Phe Glu Gln Asn Val Val Lys Leu Met Glu
        195                 200                 205
Lys Asp Met Thr Ala Ala Met Gln Tyr Leu Gln Asn Lys Gly Leu Cys
    210                 215                 220
Leu Met Pro Ile Ala Leu Ala Thr Ala Ile Ser Ser Ser Thr Gly Lys
225                 230                 235                 240
Pro Leu Leu Gly Ser Gly Val Thr Asn Ser Val Asp Ser Thr Ser Asp
                245                 250                 255
Arg Gln Ser Ser Gly Asn Gln Pro Ala Ser Leu Thr Val Asp Ser Cys
            260                 265                 270
Ser Gly Ala Leu Gly Met Gly Ser Ala Gly Phe Gly Ser Asp Phe Leu
        275                 280                 285
Leu Lys Glu Asn Thr Val Glu Lys Arg Gly Arg Glu Leu Val Pro Thr
    290                 295                 300
Ala Glu Ser Asn Gly Ala Glu Phe Gly Ile Ile Ser Ser Leu Pro Lys
305                 310                 315                 320
Leu Arg Lys Lys Glu
                325
```

<210> 303
<211> 333
<212> PRT
<213> Populus taeda

<400> 303

Met Leu Ala Arg Glu Asn Arg Ser Gly Asp Thr Glu His Asp Asp Leu
1 5 10 15
Gln Gly Thr Leu Leu Thr Ser Tyr Thr Gly Pro Gln Gly Gly Gln Thr
20 25 30
Val Leu Pro Arg Thr Pro Gly Ala Gln Ser His Gln Gln Asn Leu Ser
35 40 45
Thr Gln Thr Ile Gln Ser Gly Glu Gly Thr Ser Leu Gln Gln Tyr Gly
50 55 60
Asn His Ser Ala Val Ser Gln Leu Gln Ser Gly Ala Gly Gly Gly Asn
65 70 75 80
Ala Ala Asn Gly Ala Ala Arg Pro Arg Val Arg Ala Arg Arg Gly Gln
85 90 95
Ala Thr Asp Pro His Ser Ile Ala Glu Arg Leu Arg Arg Glu Arg Ile
100 105 110
Ala Glu Arg Met Lys Ser Leu Gln Glu Leu Val Pro Asn Ser Asn Lys
115 120 125
Thr Asp Lys Ala Ser Met Leu Asp Glu Ile Ile Glu Tyr Val Lys Phe
130 135 140
Leu Gln Leu Gln Val Lys Val Leu Ser Met Ser Arg Leu Gly Gly Ala
145 150 155 160
Gly Ala Val Ala Pro Leu Ile Ala Asp Val Pro Ser Gln Gly Ser Gly
165 170 175
Gly Val Met Ser Thr Ala Leu Gly Gln Ala Ser Gly Pro Leu Thr Leu
180 185 190
Ser Gln Asp Gly Leu Ala Phe Glu Gln Glu Val Ala Arg Leu Met Glu
195 200 205
Ser Asn Met Thr Ser Ala Met Gln Tyr Leu Gln Asn Lys Gly Leu Cys
210 215 220
Leu Met Ser Ile Ala Leu Ala Thr Ala Ile Ser Ser Ser Ser Gly Lys
225 230 235 240
Pro Val Leu Ala Thr Val Pro Gly Thr Gly Val Asp Ser Ser Glu Lys
245 250 255
Gln Asn Ser Asp Met Gln Ser Ile Val Leu Pro Phe Ser Ser Ser Ser
260 265 270
Thr Thr Met Gly Leu Arg Ala Thr Ala Ser Gly Ser Asp Ala Pro Ile
275 280 285
Asn Glu Asn Ser Ile Asn Lys Ala Ser Ile Glu Lys Val Val Ala Glu
290 295 300
Lys Ser Asn Gly Ile Ser Pro Gly Leu Ser Ser Asp Val Pro Lys Val
305 310 315 320
Gly Ser Gln Ser Arg Glu Glu Leu Leu Lys Arg Ala Gln
325 330

<210> 304
<211> 300
<212> PRT
<213> Populus trichocarpa

<400> 304

Met Ala Asn Asn Pro Thr Glu Pro Pro Thr Asp Asp Phe Leu Gln Glu
1 5 10 15
Ile Leu Gly Met Pro Asn Phe Ala Ser Ala Glu Ala Gly Leu Val Gly
20 25 30
Ala Asp Ala Gly Leu Ala Gly Thr Ala Ser Val Gln Ala Pro Met Met

```
        35                  40                  45
Leu Gln Leu Ser Ser Gly Asp Gly Ser Gly His Ile Ser Ala Leu Gly
    50                  55                  60
Gly Ala Pro Gly Gly Gly Gly Ala Gly Phe His Gly Phe Pro Leu Gly
65                  70                  75                  80
Leu Ser Leu Glu Gln Gly Lys Gly Gly Phe Leu Lys Pro Glu Glu Ala
                85                  90                  95
Ser Gly Ser Gly Asn Arg Phe Arg Asp Asp Ile Val Asp Gly Arg Val
            100                 105                 110
Arg Asn Val Phe His Gly Gln Pro Met Pro Thr Thr Val Thr Ala Ala
        115                 120                 125
Thr His Pro Pro Ala Met Arg Pro Arg Val Arg Ala Arg Arg Gly Gln
    130                 135                 140
Ala Thr Asp Pro His Ser Ile Ala Glu Arg Leu Arg Arg Glu Arg Ile
145                 150                 155                 160
Ala Glu Arg Ile Arg Ala Leu Gln Glu Leu Val Pro Ser Val Asn Lys
                165                 170                 175
Thr Asp Arg Ala Ala Met Leu Asp Glu Ile Val Asp Tyr Val Lys Phe
            180                 185                 190
Leu Arg Leu Gln Val Lys Ile Leu Ser Met Ser Arg Leu Gly Gly Ala
        195                 200                 205
Gly Ala Val Ala Pro Leu Val Thr Asp Ile Pro Leu Ser Pro Val Glu
    210                 215                 220
Asp Glu Thr Gly Glu Gly Gly Arg Asn Gln Leu Ala Trp Glu Lys Trp
225                 230                 235                 240
Ser Asn Asp Gly Thr Glu Arg Gln Val Ala Lys Leu Met Glu Glu Asn
                245                 250                 255
Val Gly Ala Ala Met Gln Phe Leu Gln Ser Lys Ala Leu Cys Ile Met
            260                 265                 270
Pro Ile Thr Leu Ala Thr Ala Ile Tyr His Thr Gln Pro Pro Asp Thr
        275                 280                 285
Thr Thr Ile Val Lys Pro Glu Thr Asn Pro Pro Ser
    290                 295                 300
```

<210> 305
<211> 331
<212> PRT
<213> Populus trichocarpa

<400> 305

```
Met Ala Gly Asn Pro Pro Pro Glu Gly Leu Gly Asp Asp Phe Phe Glu
1               5                   10                  15
Gln Ile Leu Ala Gly Gln Pro Pro Gly Tyr Gly Gly Glu Ala Val Gly
            20                  25                  30
Ser Thr Ser Leu Pro Met Met Gly Leu Gln Leu Gly Ser Ser Ala Ala
        35                  40                  45
Asn Val Gly Leu Met Arg Ser Ser Ala Asn Asn Asn Met Gly Met Met
    50                  55                  60
Pro Leu Gly Leu Asn Leu Glu His His Gly Phe Leu Arg Gln Gln Gln
65                  70                  75                  80
Asp Asp Gly Ser Ser Ser Leu Asp Thr Asn Asn Ser Asn Asn Ile Asn
                85                  90                  95
Asn Ala Ser Pro Phe Ser Ile Thr Ser Ala Gly Phe Leu Gly Arg Asp
            100                 105                 110
Ser Val His Met Thr Ser Leu Phe Pro Thr Phe Gly Gln Leu Gln Ile
        115                 120                 125
His Ser Ser Ile Arg Ser Ala Pro Pro Pro Leu Gly Pro Pro Gln Ile
    130                 135                 140
His Gln Phe Asn Ser Gln Pro Thr Ser Gly Ala Val Ser Ala Val Pro
145                 150                 155                 160
Gln Pro Pro Gly Ile Arg Pro Arg Val Arg Ala Arg Arg Gly Gln Ala


                165                 170                 175
Thr Asp Pro His Ser Ile Ala Glu Arg Leu Arg Arg Val Arg Ile Thr
            180                 185                 190
Glu Arg Val Lys Ala Leu Gln Glu Leu Val Pro Thr Cys Asn Lys Thr
        195                 200                 205
Asp Arg Ala Ala Met Leu Asp Glu Ile Val Asp Tyr Val Lys Phe Leu
    210                 215                 220
Arg Leu Gln Val Lys Val Leu Ser Met Ser Arg Leu Gly Ala Ala Gly
225                 230                 235                 240
Ala Val Ala Gln Leu Val Ala Asp Val Pro Leu Ser Ser Val Gln Gly
                245                 250                 255
Glu Gly Ile Glu Gly Gly Ala Asn Gln Gln Ala Trp Glu Asn Trp Ser
            260                 265                 270
Asn Asp Gly Thr Glu Gln Glu Val Ala Lys Leu Met Glu Glu Asp Val
        275                 280                 285
Gly Ala Ala Met Gln Leu Leu Gln Ser Lys Ala Leu Cys Ile Met Pro
    290                 295                 300
Val Ser Leu Ala Ser Ala Ile Phe Arg Ala Arg Pro Pro Asn Ala Pro
305                 310                 315                 320
Thr Leu Val Lys Pro Glu Ser Asn Pro Pro Ser
                325                 330
```

<210> 306
<211> 334
<212> PRT
<213> Populus trichocarpa

<400> 306

```
Met Ala Gly Asn Pro Pro Pro Glu Gly Leu Gly Asp Asp Phe Leu Glu
1               5                   10                  15
Gln Ile Leu Ala Ala Gln Pro Pro Gly Tyr Gly Gly Gly Gly Glu Val
            20                  25                  30
Met Gly Ser Thr Ser Leu Pro Met Met Gly Leu Gln Leu Gly Ser Cys
        35                  40                  45
Ala Gly Asn Val Gly Leu Leu Arg Ser Asn Ala Asn Asn Asn Met Gly
    50                  55                  60
Met Met Pro Leu Gly Leu Asn Leu Glu His His Gly Phe Leu Arg Gln
65                  70                  75                  80
Gln Gln Asp Asp Ser Gly Ser Ser Leu Asp Thr Asn Asn Ser Asn Asn
                85                  90                  95
Ile Asn Asn Thr Pro Pro Ser Ile Lys Ser Ala Arg Ile Leu Gly Arg
            100                 105                 110
Asp Ser Val His Met Thr Ser Leu Phe Pro Thr Phe Gly His Leu Gln
        115                 120                 125
Ile His Ser Ser Ile Arg Pro Thr Pro Pro Pro Pro Gly Pro Pro Gln
    130                 135                 140
Ile His Gln Ile Asn Ser His Pro Asn Pro Gly Ala Val Ser Ala Val
145                 150                 155                 160
Pro Gln Pro Pro Gly Ile Arg Pro Arg Val Arg Ala Arg Arg Gly Gln
                165                 170                 175
Ala Thr Asp Pro His Ser Ile Ala Glu Arg Leu Arg Arg Val Arg Ile
            180                 185                 190
Thr Glu Arg Val Lys Ala Leu Gln Glu Leu Val Pro Thr Cys Asn Lys
        195                 200                 205
Thr Asp Arg Ala Ala Met Leu Asp Glu Ile Val Asp Tyr Val Lys Phe
    210                 215                 220
Leu Arg Leu Gln Ile Lys Val Leu Ser Met Ser Arg Leu Gly Ala Ala
225                 230                 235                 240
Gly Ala Val Ala Gln Leu Val Ala Asp Val Pro Leu Ser Ser Val Gln
                245                 250                 255
Ile Lys Gly Glu Gly Asn Glu Gly Gly Ala Asn Gln Gln Ser Trp Glu


            260                 265                 270
Asn Trp Ser Asn Asp Asp Thr Glu Gln Glu Val Ala Lys Leu Met Glu
        275                 280                 285
Glu Asp Val Gly Ala Ala Met Gln Phe Leu Gln Ser Lys Ala Leu Cys
    290                 295                 300
Ile Met Pro Ile Ser Leu Ala Ser Ala Ile Phe Arg Ala Arg Pro Pro
305                 310                 315                 320
Asn Ala Ser Thr Leu Ile Asn Thr Glu Ser Asn Thr Pro Ser
                325                 330
```

<210> 307
<211> 456
<212> PRT
<213> Populus trichocarpa

<400> 307

```
Met Ile Ser Ser Asn Gln Ser Val Glu Ser Leu Ala Thr Gln Asp Thr
1               5                   10                  15
Ser Ser Val Val Leu Gly Ser Glu Ser Asp Tyr Ala Val Asp Lys Val
            20                  25                  30
Leu Ile Ser Glu Gln Ala Arg Leu Gln Asn Asp Cys Gln Asn Cys Asn
        35                  40                  45
Gly Asn Pro Ser Pro Asp Gly Met Ala Arg Gly Asn Leu Lys Phe Gly
    50                  55                  60
Asn Thr Gly Leu Gln Cys Asn Gly Ile Leu Pro Thr Leu Ser Ser Leu
65                  70                  75                  80
Asn Tyr Pro Asn Gln Leu Pro Ile Val Gly Asp Leu Thr Ser Tyr Leu
                85                  90                  95
Ser Phe Ser Glu Ala Ser Asn Ala Gly Cys Asn Gly Arg Glu Gln Ser
            100                 105                 110
Glu Tyr Leu Arg Ser Leu Lys Asn Leu Gln Asn Leu Ser Ser Ile Pro
        115                 120                 125
Gln Leu Trp Pro Ser Gln Ser Tyr Glu Gly Val Ser Ser Leu Pro Pro
    130                 135                 140
Leu Met Gly Gln Asp Arg Ile Glu Gly Ser Gly Leu Arg Gly Gly Asn
145                 150                 155                 160
Leu Asp Asp Asp Met His Ile Met Gly Lys Gly Tyr Met Gly Met Asp
                165                 170                 175
Glu Ile Leu Arg Leu Asp Lys Leu Ser Ala Ser Pro Thr Thr Glu Gly
            180                 185                 190
Lys Glu Asp Leu Gln Ser Cys Pro Phe Ser Ser Gly Ile Ala Glu Pro
        195                 200                 205
Asn Val Asn Met Ser Met Asn Gln Leu Ser Ser Met Pro Gln Thr Thr
    210                 215                 220
Ser Ala Ala Pro Val Glu Gly Cys Asn Gly Thr Gly Lys Thr Arg Val
225                 230                 235                 240
Arg Ala Arg Arg Gly His Ala Thr Asp Pro His Ser Ile Ala Glu Arg
                245                 250                 255
Leu Arg Arg Glu Lys Ile Ala Glu Arg Met Lys Asn Leu Gln Glu Leu
            260                 265                 270
Val Pro Asn Ser Asn Lys Val Asp Lys Ala Ser Met Leu Asp Glu Ile
        275                 280                 285
Ile Glu Tyr Val Lys Phe Leu Gln Leu Gln Val Lys Val Leu Ser Met
    290                 295                 300
Ser Arg Leu Gly Ala Ala Gly Ala Val Ile Pro Leu Leu Thr Asp Gly
305                 310                 315                 320
Gln Pro Glu Gly His Asn Ser Leu Ser Leu Ser Pro Ser Ala Gly Leu
                325                 330                 335
Gly Ile Asp Ile Ser Pro Ser Ala Asp Gln Ile Ala Phe Glu Gln Glu
            340                 345                 350
Val Leu Lys Leu Leu Glu Ser Asp Val Thr Met Ala Met Gln Tyr Leu


        355                 360                 365
Gln Ser Lys Gly Leu Cys Leu Met Pro Ile Ala Leu Ala Ala Ala Ile
    370                 375                 380
Ser Ser Val Lys Ala Ser Leu Ser Gly Thr Thr Ser Glu Glu Arg Lys
385                 390                 395                 400
Asn Asn Gly Tyr Thr Ser Gly Leu Val Ser Ser Ser Ser Ile Thr
                405                 410                 415
Gly Ile Asp Thr His Pro Met Ser Asn Asp Asn Asn Ile Ala Thr Gly
            420                 425                 430
Thr Leu Ser Ser Lys Gly Met Ile Val Asn Gly Cys Asn Glu Val Val
        435                 440                 445
Lys Gln Glu Val Leu Lys Asn Thr
    450                 455
```

<210> 308
<211> 300

<212> PRT
<213> Populus trichocarpa

<400> 308

```
Met Ala Asn Asn Pro Thr Glu Pro Pro Thr Asp Asp Phe Leu Gln Glu
1               5                   10                  15
Ile Leu Gly Met Pro Asn Phe Ala Ser Ala Glu Ala Gly Leu Val Gly
            20                  25                  30
Ala Asp Ala Gly Leu Ala Gly Ala Ala Ala Ala Gln Ala Ser Met Met
        35                  40                  45
Leu Gln Leu Ser Ser Gly Asp Gly Ser Gly His Ile Ser Asp Leu Gly
    50                  55                  60
Gly Ala Pro Gly Gly Gly Ser Ala Gly Phe His Gly Phe Pro Leu Gly
65                  70                  75                  80
Leu Ser Leu Glu Gln Gly Lys Gly Gly Phe Leu Lys Pro Glu Glu Ala
                85                  90                  95
Ser Gly Ser Gly Lys Arg Phe Arg Asp Glu Ile Val Asp Gly Arg Ala
            100                 105                 110
Lys Asn Val Phe His Gly Gln Pro Met Pro Thr Thr Val Ala Ile Ala
        115                 120                 125
Pro His Pro Pro Ala Met Arg Pro Arg Val Arg Ala Arg Arg Gly Gln
    130                 135                 140
Ala Thr Asp Pro His Ser Ile Ala Glu Arg Leu Arg Arg Glu Arg Ile
145                 150                 155                 160
Ala Glu Arg Ile Arg Ala Leu Gln Glu Leu Val Pro Ser Val Thr Lys
                165                 170                 175
Thr Asp Arg Ala Thr Met Leu Asp Glu Ile Val Asp Tyr Val Lys Phe
            180                 185                 190
Leu Arg Leu Gln Val Lys Val Leu Ser Met Ser Arg Leu Gly Gly Ala
        195                 200                 205
Gly Ala Val Ala Pro Leu Val Thr Asp Ile Pro Leu Ser Ser Val Glu
    210                 215                 220
Asp Glu Thr Gly Glu Gly Gly Arg Asn Gln Pro Ala Trp Glu Lys Trp
225                 230                 235                 240
Ser Asn Asp Gly Thr Glu Arg Gln Val Ala Lys Leu Met Glu Glu Asn
                245                 250                 255
Val Gly Ala Ala Met Gln Phe Leu Gln Ser Lys Ala Leu Cys Ile Met
            260                 265                 270
Pro Ile Ser Leu Ala Thr Ala Ile Tyr His Thr Gln Pro Pro Asp Thr
        275                 280                 285
Thr Thr Ile Val Lys Pro Glu Thr Asn Pro Pro Ser
    290                 295                 300
```

<210> 309
<211> 468
<212> PRT
<213> Poncirus trifoliata

<400> 309

```
Met Gln Pro Cys Ser Ser Gly Gly Glu Met Gln Gly Ile Ser Ser Leu
1               5                   10                  15
Leu Ser Asn Gly Ser His Glu Gln Gln Ser Gln Ile His Gln Ser Ala
            20                  25                  30
Thr Phe Asp Pro Thr Ser Gln Asp Asp Phe Leu Glu Gln Met Leu Ser
        35                  40                  45
Ser Leu Pro Ser Cys Ser Trp Thr Asp Leu Lys Ser Pro Trp Gly Val
    50                  55                  60
Val Asp Leu Asn Pro Asn Asn Asn Asn Asn Asn Ile Asn Asn Lys Gln
65                  70                  75                  80
Pro Arg Asp Leu Ser Asp Glu Thr Ala Pro Ser Thr Thr Gln Glu Asn
                85                  90                  95
Asn Val Pro Ala Gly Phe Gln Phe Asp Glu Ser Met Ile Leu Ala Ser
            100                 105                 110
Lys Met Arg Gln His Gln Ile Ser Gly Asn Thr Gly Ser Gly Asn Asn
        115                 120                 125
Asn Ser Ala Ala Ala Lys Phe Met Met Gln Gln Gln Gln Gln Gln Gln
    130                 135                 140
Ile Met Met Ala Ala Ala Arg Gly Gly Ile Gly Leu Pro Leu Ser Leu
145                 150                 155                 160
Gly Asn Gly Gly Asp Asn Asp Ile Val Asp Ala Ser Ser Phe Lys Ser
                165                 170                 175
Gln Gln Gly Gly Asp Gly Ser Val Gln Ala Leu Tyr Asn Gly Phe Thr
            180                 185                 190
Gly Ser Leu His Gly Ser Thr Gln Pro Gln His Phe His His Leu Gln
        195                 200                 205
Gly Gly Ser Met Pro Gly Gln Thr Phe Gly Ala Pro Gly Pro Val Met
    210                 215                 220
Asn Gln Thr Gln Ala Gln Ala Ser Gly Ser Thr Gly Gly Gly Gly Asn
225                 230                 235                 240
Thr Pro Ala Gln Gln Pro Lys Gln Arg Val Arg Ala Arg Arg Gly Gln
                245                 250                 255
Ala Thr Asp Pro His Ser Ile Ala Glu Arg Leu Arg Arg Glu Arg Ile
            260                 265                 270
Ala Glu Arg Met Lys Ala Leu Gln Glu Leu Val Pro Asn Ala Asn Lys
        275                 280                 285
Thr Asp Lys Ala Ser Met Leu Asp Glu Ile Ile Asp Tyr Val Lys Phe
    290                 295                 300
Leu Gln Leu Gln Val Lys Val Leu Ser Met Ser Arg Leu Gly Gly Ala
305                 310                 315                 320
Ala Ala Val Ala Pro Leu Val Ala Asp Met Ser Ser Glu Gly Ala Gly
                325                 330                 335
Gly Gly Asp Cys Ile Gln Ala Asn Gly Arg Asn Pro Asn Gly Ala Gln
            340                 345                 350
Thr Thr Ser Ala Asn Asp Ser Leu Thr Val Thr Glu His Gln Val Ala
        355                 360                 365
Lys Leu Met Glu Glu Asp Met Gly Ser Ala Met Gln Tyr Leu Gln Gly
    370                 375                 380
Lys Gly Leu Cys Leu Met Pro Ile Ser Leu Ala Thr Ala Ile Ser Ser
385                 390                 395                 400
Ala Thr Cys His Ser Arg Asn Pro Ile Ile Ser Thr Ser Asn Asn Asn
                405                 410                 415
Asn Asn Asn Gly Asn Pro His His Asn Pro Leu Phe Gln Ser Asn Gly
            420                 425                 430
Glu Gly Pro Thr Ser Pro Ser Met Ser Val Leu Thr Val Gln Ser Ala
        435                 440                 445
Thr Met Gly Asn Gly Gly Ala Asp Gly Ser Val Lys Asp Ala Ala Ser

                450                 455                 460
            Val Ser Lys Pro
            465
```

<210> 310
<211> 326
<212> PRT
<213> Ricinus communis

<400> 310

```
Met Ala Gly Asn Pro Pro Pro Glu Gly Leu Gly Asp Asp Phe Phe Glu
1               5                   10                  15
Gln Ile Leu Ala Val Gln Pro Gly Tyr Gly Gly Gly Asp Gly Gly Gly
            20                  25                  30
Gly Gly Asp Val Val Gly Ser Thr Met Pro Met Met Gly Leu Gln Leu
        35                  40                  45
Gly Ser Ala Ser Ser Gly Gly Gly Gly Leu Arg Thr Asn Asn Met Gly
    50                  55                  60
Met Met Pro Leu Gly Leu Asn Leu Glu Phe Leu Arg Gln Gln Glu Asp
65                  70                  75                  80
Gly Ser Gly Ala Leu Asp Asn Asn Asn His Thr Asn Asn Asn Asn Asn
                85                  90                  95
Asn Val Ser Ser Ser Thr Thr Ser Val Ile Asn Asp Arg Asp Ser Val
            100                 105                 110
His Met Ala Ser Leu Phe Pro Thr Phe Gly Gln Leu Gln Thr Gln Thr
        115                 120                 125
Leu Arg Pro Pro Pro Pro Pro His Leu His Gln Pro Phe His Gly Gln
    130                 135                 140
Pro Thr Pro Gly Val Val Ser Ala Ala Ser Gln Pro Pro Ala Ile Arg
145                 150                 155                 160
Pro Arg Val Arg Ala Arg Arg Gly Gln Ala Thr Asp Pro His Ser Ile
                165                 170                 175
Ala Glu Arg Leu Arg Arg Glu Arg Ile Ala Glu Arg Met Lys Ala Leu
            180                 185                 190
Gln Glu Leu Val Pro Thr Ala Asn Lys Thr Asp Arg Ala Ala Met Ile
        195                 200                 205
Asp Glu Ile Val Asp Tyr Val Lys Phe Leu Arg Leu Gln Val Lys Val
    210                 215                 220
Leu Ser Met Ser Arg Leu Gly Ala Ala Gly Ala Val Ala Gln Leu Val
225                 230                 235                 240
Ala Asp Val Pro Leu Ala Ser Val Glu Gly Glu Ser Ile Asp Gly Ala
                245                 250                 255
Ala Ala Asn Gln Gln Thr Trp Glu Lys Trp Ser Asn Asp Gly Thr Glu
            260                 265                 270
Gln Gln Val Ala Lys Leu Met Glu Glu Asp Ile Gly Ala Ala Met Gln
        275                 280                 285
Phe Leu Gln Ser Lys Ala Leu Cys Ile Met Pro Ile Ser Leu Ala Ser
    290                 295                 300
Ala Ile Leu Arg Thr His Pro Pro Asp Ala Pro Ser Ile Ile Lys Pro
305                 310                 315                 320
Glu Ser Asn Thr Pro Ser
                325
```

<210> 311
<211> 299
<212> PRT
<213> Ricinus communis

<400> 311

```
Met Ala Asn Asn Pro Thr Glu Pro Pro Ala Asp Asp Phe Leu Gln Glu
1               5                   10                  15
```

```
Ile Leu Gly Leu Pro Asn Phe Ala Ser Ala Asp Ala Ala Gly Leu Val
            20                  25                  30
Gly Ala Asp Gly Ala Leu Ala Thr Pro Met Met Leu Gln Leu Ser Ser
        35                  40                  45
Gly Asp Gly Ser Asn His Ile Thr Ala Leu Gly Gly Gly Gly Gly Gly
    50                  55                  60
Gly Gly Gly Ala Gly Phe His Gly Phe Pro Leu Gly Leu Ser Leu Glu
65                  70                  75                  80
Gln Gly Lys Gly Gly Phe Leu Lys Pro Glu Glu Ala Ser Gly Ser Gly
                85                  90                  95
Lys Arg Phe Arg Asp Asp Val Val Asp Gly Arg Ala Asn Thr Val Lys
            100                 105                 110
Asn Val Phe His Gly Gln Pro Met Pro Thr Thr Met Ala Ala Ala Pro
        115                 120                 125
His Pro Pro Thr Met Arg Pro Arg Val Arg Ala Arg Arg Gly Gln Ala
    130                 135                 140
Thr Asp Pro His Ser Ile Ala Glu Arg Leu Arg Arg Glu Arg Ile Ala
145                 150                 155                 160
Glu Arg Ile Arg Ala Leu Gln Glu Leu Val Pro Ser Val Asn Lys Thr
                165                 170                 175
Asp Arg Ala Ala Met Leu Asp Glu Ile Val Asp Tyr Val Lys Phe Leu
            180                 185                 190
Arg Leu Gln Val Lys Val Leu Ser Met Ser Arg Leu Gly Gly Ala Gly
        195                 200                 205
Ala Val Ala Pro Leu Val Thr Asp Ile Pro Leu Ser Ser Val Glu Asp
    210                 215                 220
Glu Thr Gly Glu Gly Gly Arg Asn Gln Pro Ala Trp Glu Lys Trp Ser
225                 230                 235                 240
Asn Asp Gly Thr Glu Arg Gln Val Ala Lys Leu Met Glu Glu Asn Val
                245                 250                 255
Gly Ala Ala Met Gln Phe Leu Gln Ser Lys Ala Leu Cys Ile Met Pro
            260                 265                 270
Ile Ser Leu Ala Thr Ala Ile Tyr His Thr Gln Ala Pro Asp Thr Ser
        275                 280                 285
Thr Ile Val Lys Pro Glu Thr Asn Pro Pro Ser
    290                 295
```

<210> 312
<211> 277
<212> PRT
<213> Sorghum bicolor

<400> 312

```
Met Ala Gly Gln Pro Pro Pro Pro Gly Gly Ser Glu Asp Asp Phe Leu
1               5                   10                  15
Glu His Phe Phe Ala Phe Pro Ser Ala Ala Ser Ala Ala Ala Gly Gly
            20                  25                  30
His Ala Gly Ala Gly Ala Gly Gly Asp His Pro Phe Pro Leu Ala Leu
        35                  40                  45
Ser Leu Asp Ala Ala Ala Glu Pro Lys Pro Asp Arg Asp Pro Val Gln
    50                  55                  60
Leu Ala Gly Leu Phe Pro Pro Val Phe Ala Gly Ala Gly Gly Val Gln
65                  70                  75                  80
Gln Pro His Leu Arg Gly Pro Pro Pro Pro Gln Met Phe Gln Ala Gln
                85                  90                  95
Pro Lys Pro Gly Glu Gly Gly Met Ala Pro Gln Pro Pro Ala Pro Arg
            100                 105                 110
Pro Lys Val Arg Ala Arg Arg Gly Gln Ala Thr Asp Pro His Ser Ile
        115                 120                 125
Ala Glu Arg Leu Arg Arg Glu Arg Ile Ala Glu Arg Met Arg Ala Leu
    130                 135                 140
```

```
Gln Glu Leu Val Pro Asn Thr Asn Lys Thr Asp Arg Ala Ala Met Leu
145                 150                 155                 160
Asp Glu Ile Leu Asp Tyr Val Lys Phe Leu Arg Leu Gln Val Lys Val
                165                 170                 175
Leu Ser Met Ser Arg Leu Gly Gly Ala Gly Ala Val Ala Gln Leu Val
            180                 185                 190
Ala Asp Ile Pro Leu Ser Val Lys Gly Glu Ala Ser Asp Ser Gly Ser
        195                 200                 205
Thr Gln His Ile Trp Glu Lys Trp Ser Thr Asp Gly Thr Glu Lys Gln
    210                 215                 220
Val Ala Lys Leu Met Glu Glu Asp Ile Gly Ala Ala Met Gln Phe Leu
225                 230                 235                 240
Gln Ser Lys Ala Leu Cys Met Met Pro Ile Ser Leu Ala Met Ala Ile
                245                 250                 255
Tyr Asp Thr Gln His Ser Gln Asp Gly His Ser Leu Met Lys Pro Glu
            260                 265                 270
Pro Asn Thr Ser Ser
        275
```

<210> 313
<211> 302
<212> PRT
<213> Solanum lycopersicum

<400> 313

```
Met Ala Asn Asn Pro Ser Glu Gly Pro Ser Asp Asp Phe Phe Asp Gln
1               5                   10                  15
Ile Leu Gly Phe Pro Ala Tyr Asn Gly Ala Glu Pro Asn Leu Ala Gly
            20                  25                  30
Asn Asp Ala Gly Ala Ile Pro Pro Ala Met Met Leu Gln Leu Asn Ser
        35                  40                  45
Gly Asp Gly Ser Ser Gln Phe Thr Gly Val Gly Leu Gly Val Gly Leu
    50                  55                  60
Gly Gly Gly Gly Phe His Gly His Gly Gly Gly Gly Ser Phe Pro Leu
65                  70                  75                  80
Gly Leu Ser Leu Glu Gln Gly Lys Gly Gly Phe Leu Lys Met Asp Asp
                85                  90                  95
Val Ser Ala Pro Gly Arg Arg Phe Arg Asp Asp Val Val Asp Ser Arg
            100                 105                 110
Ala Ser Ser Ser Val Lys Pro Gly Phe His Gly Gln Pro Met Pro Ser
        115                 120                 125
Met Pro His Pro Pro Ala Ile Arg Pro Arg Val Arg Ala Arg Arg Gly
    130                 135                 140
Gln Ala Thr Asp Pro His Ser Ile Ala Glu Arg Leu Arg Arg Glu Arg
145                 150                 155                 160
Ile Ala Glu Arg Ile Arg Ala Leu Gln Glu Leu Val Pro Ser Val Asn
                165                 170                 175
Lys Thr Asp Arg Ala Val Met Leu Asp Glu Ile Val Asp Tyr Ile Lys
            180                 185                 190
Phe Leu Arg Leu Gln Val Lys Val Leu Ser Met Ser Arg Leu Gly Gly
        195                 200                 205
Ala Gly Ala Val Ala Pro Leu Val Thr Asp Ile Pro Ile Ser Ser Val
    210                 215                 220
Glu Glu Glu Ser Ser Glu Gly Gly Asn Asn Asn Gln Pro Ala Trp Glu
225                 230                 235                 240
Lys Trp Ser Ser Asp Gly Thr Glu Arg Gln Val Ala Lys Leu Met Glu
                245                 250                 255
Glu Asn Val Gly Ala Ala Met Gln Phe Leu Gln Ser Lys Ala Leu Cys
            260                 265                 270
Ile Met Pro Ile Ser Leu Ala Ser Ala Ile Tyr His Ser Gln Pro Pro
        275                 280                 285
```

```
Asp Thr Ser Ser Leu Val Lys Pro Glu Thr Asn Pro Pro Ser
    290                 295                 300
```

<210> 314
<211> 296
<212> PRT
<213> Solanum lycopersicum

<400> 314

```
Met Ala Asp Asn Pro Pro Glu Val Tyr Ala Ala Asp Asp Phe Leu Glu
1               5                   10                  15
Gln Ile Leu Ala Ile Pro Ser Tyr Ala Ser Leu Pro Val Thr Asp Leu
            20                  25                  30
Thr Ala Gly Ala Ser Ser Glu Asn Ser Thr Ser Gly Val Ser Gln Leu
        35                  40                  45
Gln Gln Gln Pro Leu Phe Pro Leu Gly Leu Ser Leu Asp Asn Gly Phe
    50                  55                  60
Ala Asp Ala Asn Asn Thr Gly Gly Phe Gln Val Lys Thr Glu Arg Glu
65                  70                  75                  80
Ala Met Asn Met Gly Asn Leu Tyr Pro Gly Leu Glu His Leu Gln Ser
                85                  90                  95
His Ala Val Cys Leu Ser Val Pro Gln Val His Gln Val Gln Pro Phe
            100                 105                 110
Gln Gly His Pro Thr Ser Ser Ala Ile Val Thr Ile Pro His Gln Pro
        115                 120                 125
Ala Ile Arg Pro Arg Val Arg Ala Gln Arg Gly Gln Ala Thr Asp Pro
    130                 135                 140
His Ser Ile Ala Glu Arg Leu Arg Arg Glu Arg Ile Ser Glu Arg Ile
145                 150                 155                 160
Lys Ala Leu Gln Glu Leu Val Pro Ser Cys Asn Lys Thr Asp Arg Ala
                165                 170                 175
Ala Met Leu Asp Glu Ile Leu Asp Tyr Val Lys Phe Leu Arg Leu Gln
            180                 185                 190
Val Lys Val Leu Ser Met Ser Arg Leu Gly Gly Thr Ser Ala Ala Ala
        195                 200                 205
Gln Val Val Ala Asp Ile Pro Leu Gln Ser Val Glu Gly Asp Thr Cys
    210                 215                 220
Glu Ser His Ser Asn Gln His Val Trp Glu Lys Trp Ser Asp Ser Glu
225                 230                 235                 240
Thr Glu Gln Glu Val Ala Lys Leu Met Glu Glu Asp Val Gly Thr Ala
                245                 250                 255
Met Gln Tyr Leu Gln Ser Lys Ser Leu Cys Ile Met Pro Ile Ser Leu
            260                 265                 270
Ala Ala Leu Ile Tyr Pro Thr Gln Gln Ser Asp Asn Gln Ser Met Val
        275                 280                 285
Lys Pro Glu Gln Ala Ala Pro Leu
    290                 295
```

<210> 315
<211> 304
<212> PRT
<213> Solanum lycopersicum

<400> 315

```
Met Ala Ala Asn Gln Pro Glu Gly Tyr Ala Asp Asp Phe Leu Glu Gln
1               5                   10                  15
Ile Leu Ala Ile Pro Pro Tyr Ser Gly Leu Pro Val Ala Asp Val Gly
            20                  25                  30
Thr Pro Ser Glu Thr Thr Ser Phe Thr Ser Ala Ser Ala Val Ser His
        35                  40                  45

Leu Asn Ser Ala Ala Ala Ala Gly Leu Gln Gln Pro Leu Phe Pro Leu
    50                  55                  60
Gly Leu Ser Leu Asp Asn Gly Arg Asp Asp Val Gly Asp Ala Gly Pro
65                  70                  75                  80
Tyr Ala Val Lys His Glu Arg Asp Gly Met Asn Ile Gly Asn Leu Tyr
                85                  90                  95
Ala Gly Leu Glu His Leu Gln Ser His Ala Val Arg His Ser Val Pro
            100                 105                 110
Ser Val His His Val Gln Pro Phe Gln Gly Pro Pro Thr Thr Ser Thr
        115                 120                 125
Thr Val Thr Val Pro His Pro Pro Ser Ile Arg Pro Arg Val Arg Ala
    130                 135                 140
Arg Arg Gly Gln Ala Thr Asp Pro His Ser Ile Ala Glu Arg Leu Arg
145                 150                 155                 160
Arg Glu Arg Ile Ser Glu Arg Ile Lys Ala Leu Gln Glu Leu Val Pro
                165                 170                 175
Ser Cys Asn Lys Thr Asp Arg Ala Ala Met Leu Asp Glu Ile Leu Asp
            180                 185                 190
Tyr Val Lys Phe Leu Arg Leu Gln Val Lys Val Leu Ser Met Ser Arg
        195                 200                 205
Leu Gly Gly Ala Ser Ala Val Ala Gln Leu Val Ala Asp Ile Pro Leu
    210                 215                 220
Asn Leu Trp Glu Gly Asp Ser Gly Glu Ser Arg Ser Asn Gln His Ile
225                 230                 235                 240
Trp Asp Lys Trp Ser Asn Val Asp Thr Glu Arg Glu Val Ala Lys Leu
                245                 250                 255
Met Glu Glu Asp Val Gly Ala Ala Met Gln Tyr Leu Gln Ser Lys Ser
            260                 265                 270
Leu Cys Ile Met Pro Ile Ser Leu Ala Ala Leu Ile Tyr Pro Thr Gln
        275                 280                 285
Gln Pro Asp Asp Gln Ser Leu Val Lys Pro Glu Ala Ala Ala Pro Ser
    290                 295                 300
```

<210> 316
<211> 302
<212> PRT
<213> Solanum tuberosum

<400> 316

```
Met Ala Asn Asn Pro Ser Glu Gly Pro Ser Asp Asp Phe Phe Asp Gln
1               5                   10                  15
Ile Met Gly Phe Pro Ala Tyr Asn Gly Ala Glu Thr Asn Leu Ala Gly
            20                  25                  30
Asn Asp Ala Gly Ala Ile Pro Pro Ala Met Met Leu Gln Leu Asn Ser
        35                  40                  45
Gly Asp Gly Ser Gly Gln Phe Thr Gly Val Gly Leu Gly Val Gly Leu
    50                  55                  60
Gly Gly Gly Gly Phe His Gly His Gly Gly Gly Ala Ser Phe Pro Leu
65                  70                  75                  80
Gly Leu Ser Leu Glu Gln Gly Lys Gly Gly Phe Leu Lys Met Asp Asp
                85                  90                  95
Val Ser Ala Pro Gly Arg Arg Phe Arg Asp Asp Val Val Asp Ser Arg
            100                 105                 110
Ala Ser Ser Ser Val Lys Pro Gly Phe His Gly Gln Pro Met Pro Ser

        115                 120                 125
Met Pro His Pro Pro Ala Ile Arg Pro Arg Val Arg Ala Arg Arg Gly
    130                 135                 140
Gln Ala Thr Asp Pro His Ser Ile Ala Glu Arg Leu Arg Arg Glu Arg
145                 150                 155                 160
Ile Ala Glu Arg Ile Arg Ala Leu Gln Glu Leu Val Pro Ser Val Asn


                165                 170                 175
Lys Thr Asp Arg Ala Val Met Leu Asp Glu Ile Val Asp Tyr Val Lys
            180                 185                 190
Phe Leu Arg Leu Gln Val Lys Val Leu Ser Met Ser Arg Leu Gly Gly
        195                 200                 205
Ala Gly Ala Val Ala Pro Leu Val Thr Asp Ile Pro Ile Ser Ser Val
    210                 215                 220
Glu Glu Glu Ser Ser Glu Gly Gly Asn Asn Asn Gln Pro Ala Trp Glu
225                 230                 235                 240
Lys Trp Ser Ser Asp Gly Thr Glu Arg Gln Val Ala Lys Leu Met Glu
                245                 250                 255
Glu Asn Val Gly Ala Ala Met Gln Phe Leu Gln Ser Lys Ala Leu Cys
            260                 265                 270
Ile Met Pro Ile Ser Leu Ala Ser Ala Ile Tyr His Ser Gln Pro Pro
        275                 280                 285
Asp Thr Ser Ser Leu Val Lys Pro Glu Thr Asn Pro Pro Ser
    290                 295                 300
```

<210> 317
<211> 304
<212> PRT
<213> Solanum tuberosum

<400> 317

```
Met Ala Ala Asn Gln Pro Glu Ala Tyr Ala Asp Asp Phe Leu Glu Gln
1               5                   10                  15
Ile Leu Ala Ile Pro Ser Tyr Ser Gly Leu Pro Val Ala Asp Val Gly
            20                  25                  30
Thr Pro Ser Glu Thr Thr Ser Phe Thr Ser Ala Ser Ala Val Ser His
        35                  40                  45
Leu Asn Ser Ala Ala Ala Ala Gly Leu Gln Gln Pro Leu Phe Pro Leu
    50                  55                  60
Gly Leu Ser Leu Asp Asn Gly Arg Asp Asp Val Ser Glu Ala Gly Ala
65                  70                  75                  80
Tyr Ala Val Lys His Glu Arg Asp Gly Met Asn Ile Gly Asn Leu Tyr
                85                  90                  95
Ala Gly Leu Glu His Leu Gln Ser His Ala Val Arg His Ser Val Pro
            100                 105                 110
Ser Ile His His Val Gln Pro Phe Gln Gly Pro Pro Thr Thr Ser Thr
        115                 120                 125
Thr Val Thr Val Pro His Pro Pro Ser Ile Arg Pro Arg Val Arg Ala
    130                 135                 140
Arg Arg Gly Gln Ala Thr Asp Pro His Ser Ile Ala Glu Arg Leu Arg
145                 150                 155                 160
Arg Glu Arg Ile Ser Glu Arg Ile Lys Ala Leu Gln Glu Leu Val Pro
                165                 170                 175
Ser Cys Asn Lys Thr Asp Arg Ala Ala Met Leu Asp Glu Ile Leu Asp
            180                 185                 190
Tyr Val Lys Phe Leu Arg Leu Gln Val Lys Val Leu Ser Met Ser Arg
        195                 200                 205
Leu Gly Gly Ala Ser Ala Val Ala Gln Leu Val Ala Asp Ile Pro Leu
    210                 215                 220
Gln Ser Val Glu Gly Asp Ser Gly Glu Ser Arg Ser Asn Gln His Ile
225                 230                 235                 240
Trp Asp Lys Trp Ser Asn Val Asp Thr Glu Gln Glu Val Ala Lys Leu
                245                 250                 255
Met Glu Glu Asp Val Gly Ala Ala Met Gln Tyr Leu Gln Ser Lys Ser
            260                 265                 270
Leu Cys Ile Met Pro Ile Ser Leu Ala Ala Leu Ile Tyr Pro Thr Gln
        275                 280                 285
Gln Pro Asp Asp Gln Ser Leu Val Lys Pro Glu Ala Ala Ala Pro Ser

                290                 295                 300
```

<210> 318
<211> 447
<212> PRT
<213> Solanum tuberosum

<400> 318

```
Met Gln Ala Met Asn Ser Leu Leu Ser Gln Gln Gln Gln Ser Gln Met
1               5                   10                  15
Ser Leu Gln Asp Leu Gln Asn Gly Gly Asn Gly Gly Ser Thr Gly Gly
            20                  25                  30
Val Gly Gly Leu Gly Gln His Asn Met Gly His Phe His Phe Asp Pro
        35                  40                  45
Thr Ser Ser His Asp Asp Phe Leu Glu Gln Ile Leu Ser Ser Val Pro
    50                  55                  60
Ser Ser Ser Pro Trp Pro Asp Leu Ser Lys Ser Trp Asp Pro His His
65                  70                  75                  80
His Leu Ser Ser Pro Pro His Asn Pro Ser Ser Gly Glu Asp Gln Pro
                85                  90                  95
Pro Ser Asn Pro Leu His Ser Gln Phe His Tyr Asp Asp Gln Ala Ser
            100                 105                 110
Ser Leu Leu Ala Ser Lys Leu Arg Gln His Gln Ile Thr Gly Gly Gly
        115                 120                 125
Ala Ala Ala Ala Ala Lys Ala Leu Met Leu Gln Gln Gln Leu Leu Leu
    130                 135                 140
Ser Arg Thr Leu Ala Gly Asn Gly Leu Arg Ser Pro Asn Gly Ala Ser
145                 150                 155                 160
Gly Asp Asn Gly Leu Leu Ser Leu Pro Leu Asn Leu Ser Asn Asn Gly
                165                 170                 175
Asp Gln Asn Asp Gly Val Ala Asn Pro Ala Asn Asp Asn Ser Val Gln
            180                 185                 190
Ala Leu Phe Asn Gly Phe Thr Gly Ser Leu Gly Gln Thr Ser Asn Gln
        195                 200                 205
Pro Gln His Phe His His Pro Gln Gly Gly Ser Met Gln Ser Gln Ser
    210                 215                 220
Phe Gly Ala Pro Ala Met Asn Gln Thr Pro Ala Ala Ser Gly Ser Ala
225                 230                 235                 240
Gly Gly Gly Gly Gly Ser Thr Pro Ala Ala Gln Pro Lys Gln Gln Arg
                245                 250                 255
Val Arg Ala Arg Arg Gly Gln Ala Thr Asp Pro His Ser Ile Ala Glu
            260                 265                 270
Arg Leu Arg Arg Glu Arg Ile Ala Glu Arg Met Lys Ala Leu Gln Glu
        275                 280                 285
Leu Val Pro Asn Ala Asn Lys Thr Asp Lys Ala Ser Met Leu Asp Glu
    290                 295                 300
Ile Ile Asp Tyr Val Lys Phe Leu Gln Leu Gln Val Lys Val Leu Ser
305                 310                 315                 320
Met Ser Arg Leu Gly Gly Ala Ala Ala Val Ala Pro Leu Val Ala Asp
                325                 330                 335
Met Ser Ser Glu Gly Arg Gly Glu Gly Asn Val Gly Arg Gly Gly Asn
            340                 345                 350
Gly Thr Ala Ala Ser Ser Ser Asn Lys Glu Thr Met Thr Val Thr Glu
        355                 360                 365
His Gln Val Ala Lys Leu Met Glu Glu Asp Met Gly Ser Ala Met Gln
    370                 375                 380
Tyr Leu Gln Gly Lys Gly Leu Cys Leu Met Pro Ile Ser Leu Ala Thr
385                 390                 395                 400
Ala Ile Ser Thr Ser Thr Arg Ile Pro Leu Leu Ser Pro Glu Ala Gly
                405                 410                 415
Arg Pro Ala Ser Pro Thr Leu Ser Ala Leu Thr Val Gln Ser Ala Thr

                420                 425                 430
    Ile Gly Asn Ala Ala Gly Lys Asp Ala Thr Ser Leu Phe Glu Ala
            435                 440                 445
```

<210> 319
<211> 382
<212> PRT
<213> Solanum tuberosum

<400> 319

```
Met Gln Ala Met Asn Ser Phe Gln Ser Thr Gly Glu Asn Gly Ala Ser
1               5                   10                  15
Ser Gly Glu His Ile Ser His Ser His Phe Asp Pro Ser Ser Ser His
            20                  25                  30
Asp Asp Phe Leu Gln Gln Ile Leu Ser Ser Val Pro Ser Ser Ser Pro
        35                  40                  45
Trp Pro Glu Ile Ser Gly Asp Gly His Pro Tyr Asn Phe Asp Asp His
    50                  55                  60
Gln Ser Thr Leu Leu Ala Ser Lys Leu Arg Gln His Gln Ile Asn Gly
65                  70                  75                  80
Gly Ser Ser Ala Ala Ala Ala Ala Lys Ala Leu Met Leu Gln Gln Gln
                85                  90                  95
Leu Leu Leu Ser Arg Gly Ile Ala Gly Asn Gly Gly Asp Gln Asn Asp
            100                 105                 110
Asp Gly Leu Asn Pro Gly Asn Asp Ile Ser Val Gln Ala Leu Tyr Asn
        115                 120                 125
Gly Phe Ala Gly Ser Leu Gly Gln Thr Ser Asn Gln Ser Gln His Phe
    130                 135                 140
His His Ser Gln Ala Gln Ser Phe Gly Ala Pro Ala Ala Ser Leu Thr
145                 150                 155                 160
Met Asn Gln Thr Pro Ala Ala Ser Gly Ser Ala Gly Gly Ala Gln Pro
                165                 170                 175
Lys Gln Gln Lys Val Arg Ala Arg Arg Gly Gln Ala Thr Asp Pro His
            180                 185                 190
Ser Ile Ala Glu Arg Leu Arg Arg Glu Arg Ile Ala Glu Arg Met Lys
        195                 200                 205
Ser Leu Gln Glu Leu Val Pro Asn Ala Asn Lys Thr Asp Lys Ala Ser
    210                 215                 220
Met Leu Asp Glu Ile Ile Asp Tyr Val Arg Phe Leu Gln Leu Gln Val
225                 230                 235                 240
Lys Val Leu Ser Met Ser Arg Leu Gly Gly Ala Ala Ala Val Ala Pro
                245                 250                 255
Leu Val Ala Asp Arg Ser Ser Glu Gly Gly Gly Asp Cys Val Gln Gly
            260                 265                 270
Asn Gly Gly Arg Gly Gly Ser Asn Gly Thr Thr Ser Ser Ala Asn Asn
        275                 280                 285
Asp Ser Ser Met Thr Met Thr Glu His Gln Val Ala Lys Leu Met Glu
    290                 295                 300
Glu Asp Met Gly Ser Ala Met Gln Tyr Leu Gln Gly Lys Gly Leu Ser
305                 310                 315                 320
Leu Met Pro Ile Ser Leu Ala Thr Ala Ile Ser Thr Ser Thr Cys His
                325                 330                 335
Ser Met Lys Pro Asn Asn Pro Leu Leu Leu Gly Gly Gly Ser Ala Ile
            340                 345                 350
Asn Gly Gly Gly Glu Thr Gly Gly Gly Pro Ser Ser Pro Thr Leu Ser
        355                 360                 365
Ala Ser Thr Val Gln Ser Ala Thr Met Gly Asn Gly Gly Thr
    370                 375                 380
```

<210> 320
<211> 290
<212> PRT
<213> Triticum aestivum

<400> 320

Met Leu Leu Cys Ser Thr Ile Gln Val Ile Gln Met Gly Asn Leu Ser
1               5                   10                  15
Val Phe Asp Glu Ala Thr Met Ala Ser Leu His Asp Ser Lys Glu Phe
            20                  25                  30
Leu Ser Gly Ser Ile Ser Ser Phe Gly Thr Ala Glu Gln Ser Gln Leu
        35                  40                  45
Ala Gly Ser Gly Leu Lys Ala Glu Gln Gln Glu Gln Asn Ala Met Cys
    50                  55                  60
Asn Ile Pro Leu Pro Phe Ala Ser Gly Ser Gln Met Ala Val Ser Glu
65                  70                  75                  80
Ala Gln Gly Ala Met Ile Pro Ser Lys Ile Ser Ser Thr Ile His Asn
                85                  90                  95
Asn Lys Ser Glu Tyr Pro Val Pro Ile Ser His Ser Ala Asp Ala Gln
            100                 105                 110
Asn Lys Ala Asn Ser Ala Asn Gly Asn Ser Ala Ser Ala Lys Pro Arg
        115                 120                 125
Ala Arg Ala Arg Arg Gly Gln Ala Thr Asp Pro His Ser Ile Ala Glu
    130                 135                 140
Arg Leu Arg Arg Glu Lys Ile Ser Glu Arg Met Lys Asn Leu Gln Asp
145                 150                 155                 160
Leu Val Pro Asn Ser Asn Lys Ala Asp Lys Ser Ser Met Leu Asp Glu
                165                 170                 175
Ile Ile Asp Tyr Val Lys Phe Leu Gln Leu Gln Val Lys Val Leu Ser
            180                 185                 190
Met Ser Arg Leu Gly Ala Pro Gly Ala Val Leu Pro Leu Leu Ala Glu
        195                 200                 205
Ser Gln Thr Glu Gly Arg Ser Asn Ser Pro Leu Ser Ser Pro Thr Ala
    210                 215                 220
Ser Gln Gly Leu Leu Asp Ala Ala Gly Pro Glu Asp Ser Leu Val Phe
225                 230                 235                 240
Glu Gln Glu Val Ile Lys Leu Met Glu Thr Ser Ile Thr Asn Ala Met
                245                 250                 255
Gln Tyr Leu Gln Asn Lys Gly Leu Cys Leu Met Pro Ile Ala Leu Ala
            260                 265                 270
Ser Ala Ile Ser Asn Gln Lys Gly Thr Ser Ala Ala Ala Ile Pro Pro
        275                 280                 285
Glu Arg
    290

<210> 321
<211> 485
<212> PRT
<213> Vitis vinifera

<400> 321

Met Asp Glu Tyr Leu Asp His Leu Phe Ser Ser Thr Ser Trp Ser Asp
1               5                   10                  15
Val Asn Val Lys Glu Gly Ser Ser Trp Ile Cys Gly Glu Pro Ser Gln
            20                  25                  30
Thr Asn Gly Met Leu Ser Asp Ser Ile Gly Ile Tyr Glu Gly Asp Lys
        35                  40                  45
Lys Asn Ser Pro Val Gly Met Thr Asn Ser Asn Leu Met Ile Glu Gly
    50                  55                  60
Leu Val Thr Gln Asp Thr Ser Ser Ile Val Leu Gly Gly Glu Ser Asp
65                  70                  75                  80
His Gly Leu Gly Lys Gly Leu Leu Leu Glu Glu Ala Pro Arg Gln Gln
                85                  90                  95

Asp Leu Gln Asn Thr Glu Gly Asn Ser Ser Leu Asn Gly Ala Val Asn
100 105 110
Gly Ser Ser Glu Val Gly Tyr Val Gly Leu Gln Leu Asn Thr Pro Thr
115 120 125
Ser Thr Pro Cys Ser Leu Asp Leu Gly Ser Pro Lys Gln Leu Ser Leu
130 135 140
Ile Gly Gly Met Ser Arg Ser Ser Arg Pro Phe Thr Glu Leu Asp His
145 150 155 160
Val Gly Cys Asn Gly Asn Glu Pro Ser Asp Phe Gln Arg Ser Val Gly
165 170 175
Asn Phe Gln Ala Leu Pro Pro Ile Pro Gln Leu Trp Ser Gln Pro Ser
180 185 190
Tyr Gly Gly Gly Ser Ser Leu Ser Pro Val Met Gly Glu Tyr Lys Met
195 200 205
Gln Gly Phe Gly Leu Gln Gly Glu Tyr Val Asp Asn Glu Met Asp Ile
210 215 220
Met Arg Asn Arg Tyr Val Gly Asp Glu Ile Leu Gln Leu Asp Asn Ile
225 230 235 240
Ser Ser Ala Ile Pro Ile Lys Gly Lys Glu Glu Gln His Thr His Pro
245 250 255
Phe Ser Pro Ser Ala Val Gly Pro His Met Thr Met Thr Ala Ser Gly
260 265 270
Leu Gln Ser Leu Pro Gln Thr Thr Val Gly Thr Ala Ser Gly Gly Cys
275 280 285
Asn Gly Thr Gly Lys Pro Arg Val Arg Ala Arg Arg Gly Gln Ala Thr
290 295 300
Asp Pro His Ser Ile Ala Glu Arg Leu Arg Arg Glu Lys Ile Ala Glu
305 310 315 320
Arg Met Lys Asn Leu Gln Glu Leu Val Pro Asn Ser Asn Lys Thr Asp
325 330 335
Lys Ala Ser Met Leu Asp Glu Ile Ile Glu Tyr Val Lys Phe Leu Gln
340 345 350
Leu Gln Val Lys Val Leu Ser Met Ser Arg Leu Gly Ala Ala Glu Ala
355 360 365
Val Val Pro Leu Ile Thr Asp Gly Gln Ala Glu Gly Ser Lys Gly Leu
370 375 380
Ser Leu Ser Pro Ser Ala Gly Gln Ala Glu Asp Ile Cys Gln Ser Pro
385 390 395 400
Asp Gln Ile Ala Phe Glu Gln Glu Val Val Lys Leu Met Glu Ser Asn
405 410 415
Val Thr Met Ala Met Gln Tyr Leu Gln Ser Lys Gly Leu Cys Leu Met
420 425 430
Pro Ile Ala Leu Ala Thr Ala Ile Ser Ser Gly Lys Ala Ala Ser Ser
435 440 445
Gly Thr Gly Ser Asp Glu Gly Lys Asn Ala Ala Thr Pro Val Ala Ala
450 455 460
Thr Pro Val Ala Thr Ala Tyr Leu Ala Leu Gly His Ile Ile His Leu
465 470 475 480
Leu Met Ala Met Phe
485

<210> 322
<211> 418
<212> PRT
<213> Vitis vinifera

<400> 322

Met Leu Ser Thr Leu Pro Ser Trp Ser Asp Leu Pro Ala Asn Pro Lys
1 5 10 15
Ser Pro Trp Glu Leu Asn Ala Ser Asn Pro Ile Ser Met Pro Ser Asn
20 25 30

Lys Ser Arg Asp Leu Ser Asp Asp Thr Thr Pro Ser Asn Pro Asp Asn
35 40 45
Val Gln Phe Ala Phe Asp Glu Ser Ala Met Leu Ala Ser Lys Leu Arg
50 55 60
Gln His Gln Ile Ser Gly Asn Ser Ser Ala Ala Lys Ser Ala Leu Met
65 70 75 80
Leu Gln Gln Gln Leu Leu Leu Ser Arg Gly Val Ala Met Gly Arg Ser
85 90 95
Pro Ser Asn Gly Ser Gly Ala Gly Glu Ser Gly Leu Leu Gln Leu Pro
100 105 110
Leu Ser Leu Ser Asn Gly Asp Ser Cys Leu Val Asp Arg Ser Gln Asn
115 120 125
Asp Val Val Asp Gly Ser Ser Ser Phe Lys Ser Pro Asn Gln Gly Gly
130 135 140
Asp Gly Ser Val Gln Ala Leu Tyr Asn Gly Phe Ala Gly Ala Leu His
145 150 155 160
Gly Ser Gly Gln Ala Ser Asn Gln Ala Gln Asn Phe His His Pro Gln
165 170 175
Gly Gly Ser Met Gln Ala Gln Asn Tyr Gly Ala Pro Ala Thr Arg Val
180 185 190
Arg Ala Arg Arg Gly Gln Ala Thr Asp Pro His Ser Ile Ala Glu Arg
195 200 205
Leu Arg Arg Glu Arg Ile Ala Glu Arg Met Lys Ala Leu Gln Glu Leu
210 215 220
Val Pro Asn Ala Asn Lys Thr Asp Lys Ala Ser Met Leu Asp Glu Ile
225 230 235 240
Ile Asp Tyr Val Lys Phe Leu Gln Leu Gln Val Lys Val Leu Ser Met
245 250 255
Ser Arg Leu Gly Gly Ala Ala Ala Val Ala Pro Leu Val Ala Asp Met
260 265 270
Ser Ser Glu Gly Gly Gly Asp Cys Ile Gln Ala Ser Gly Thr Ser Gly
275 280 285
Pro Thr Gly Gly Arg Ala Thr Asn Gly Thr Gln Thr Thr Thr Ser Asn
290 295 300
Asp Ser Leu Thr Val Thr Glu His Gln Val Ala Lys Leu Met Glu Glu
305 310 315 320
Asp Met Gly Ser Ala Met Gln Tyr Leu Gln Gly Lys Gly Leu Cys Leu
325 330 335
Met Pro Ile Ser Leu Ala Thr Ala Ile Ser Thr Thr Thr Cys His Ser
340 345 350
Arg Asn Pro Met Val Ala Ala Ala Ala Val Ala Ala Ser Asn Ile Asn
355 360 365
Asn Gly Ser His Thr His Pro Leu Leu Pro Asn Ser Asn Ala Asp Gly
370 375 380
Pro Ser Ser Pro Ser Met Ser Val Leu Thr Val Gln Ser Ala Thr Met
385 390 395 400
Gly Asn Gly Leu Ala Asp Ala Pro Val Lys Asp Ala Ala Ser Val Ser
405 410 415
Lys Pro

<210> 323
<211> 289
<212> PRT
<213> Vitis vinifera

<400> 323

Met Ala Ser Asn Pro Ser Glu Ala Pro Ala Asp Asp Phe Leu Glu Gln
1 5 10 15
Ile Leu Gly Ile Pro Thr Tyr Pro Ala Ala Asp Pro Asn Leu Ala Ala
20 25 30

```
Asn Asp Val Asn Leu Ala Ala Pro Met Val Leu Gln Leu Gly Ser Gly
        35                  40                  45
Glu Gly Ser Gly His Ile Ala Gly Gly Tyr Gln Gly Thr Met Phe Pro
    50                  55                  60
Leu Gly Leu Arg Leu Glu Gln Gly Lys Ser Ser Phe Leu Lys Pro Glu
65                  70                  75                  80
Asp Ala Ser Gly Ser Gly Lys Arg Phe Arg Glu Glu Val Ile Asp Gly
                85                  90                  95
Arg Ala Ser Thr Val Lys Asn Ala Phe His Gly Gln Pro Met Gln Ala
            100                 105                 110
Thr Val Ala Ala Ala Pro His Pro Pro Ala Ile Arg Pro Arg Val Arg
        115                 120                 125
Ala Arg Arg Gly Gln Ala Thr Asp Pro His Ser Ile Ala Glu Arg Leu
    130                 135                 140
Arg Arg Glu Arg Ile Ala Glu Arg Ile Arg Ala Leu Gln Glu Leu Val
145                 150                 155                 160
Pro Ser Val Asn Lys Thr Asp Arg Ala Ala Met Leu Asp Glu Ile Val
                165                 170                 175
Asp Tyr Val Lys Phe Leu Arg Leu Gln Val Lys Val Leu Ser Met Ser
            180                 185                 190
Arg Leu Gly Gly Ala Gly Ala Val Ala Pro Leu Val Thr Asp Ile Pro
        195                 200                 205
Leu Ala Ser Val Glu Glu Glu Ala Ser Glu Gly Gly Arg Asn Glu Pro
    210                 215                 220
Ala Trp Glu Lys Trp Ser Asn Asp Gly Thr Glu Arg Gln Val Ala Lys
225                 230                 235                 240
Leu Met Glu Glu Asn Val Gly Ala Ala Met Gln Phe Leu Gln Ser Lys
                245                 250                 255
Ala Leu Cys Ile Met Pro Ile Ser Leu Ala Ser Ala Ile Tyr His Ser
            260                 265                 270
Gln Gln Pro Asp Thr Thr Pro Leu Ile Lys Pro Gln Thr Asn Pro Pro
        275                 280                 285
Ser
```

<210> 324
<211> 313
<212> PRT
<213> Vitis vinifera

<400> 324

```
Met Ala Thr Asn Pro Pro Glu Gly Tyr Ala Asp Asp Phe Leu Glu Gln
1               5                   10                  15
Ile Leu Ala Ile Pro Ser Tyr Pro Gly His Asp Pro Asn Met Val Gly
            20                  25                  30
Thr Gly Ser Thr Met Val Leu Gln Leu Ser Ser Gly Asp Gly Ser Gly
        35                  40                  45
His Val Ala Gly Gly Gly Phe Gln Gly Pro Val Phe Pro Leu Gly Leu
    50                  55                  60
Ser Leu Glu Ser Gly Ile Pro Ala Thr Gln Val Ala Pro Gly Ser Gly
65                  70                  75                  80
Glu Arg Phe Arg Asp Asp Val Asp Ala Arg Gly Ser Ser Gly Arg Ser
                85                  90                  95
Glu Arg Glu Ser Val His Leu Asp Gly Leu Phe Pro Ala Tyr Gly His
            100                 105                 110
Val Gln Ser Leu Ser Val Arg Pro Ala Val Pro Gln Val His Gln Ala
        115                 120                 125
Phe His Gly Gln Pro Thr Pro Val Pro Ile Thr Ala Ala Pro His Pro
    130                 135                 140
Pro Ala Ile Arg Pro Lys Val Arg Ala Arg Arg Gly Gln Ala Thr Asp
145                 150                 155                 160
```

```
Pro His Ser Ile Ala Glu Arg Leu Arg Arg Glu Arg Ile Ala Glu Arg
                165                 170                 175
Met Lys Ala Leu Gln Glu Leu Val Pro Ser Ser Asn Lys Thr Asp Arg
            180                 185                 190
Ala Ala Met Leu Asp Glu Ile Val Asp Tyr Val Lys Phe Leu Arg Leu
        195                 200                 205
Gln Val Lys Val Leu Ser Met Ser Arg Leu Gly Gly Ala Gly Ala Val
    210                 215                 220
Ala Gln Leu Val Ala Asp Ile Pro Leu Pro Ala Val Glu Gly Glu Thr
225                 230                 235                 240
Gly Glu Gly Gly Ser Asn Gln Gln Ala Trp Asp Lys Trp Ser Asn Asp
                245                 250                 255
Gly Thr Glu Arg Glu Val Ala Lys Leu Met Glu Glu Asp Val Gly Ala
            260                 265                 270
Ala Met Gln Phe Leu Gln Ser Lys Ala Leu Cys Ile Met Pro Ile Ser
        275                 280                 285
Leu Ala Ala Ala Ile Tyr Pro Ala His Gln Thr Asp Thr Pro Thr Leu
    290                 295                 300
Ile Lys Pro Glu Pro Asn Ala Pro Ser
305                 310
```

<210> 325
<211> 285
<212> PRT
<213> Zea mays

<400> 325

```
Met Ala Gly Gln Pro Pro Pro Gln Gly Pro Glu Asp Asp Phe Phe Asp
1               5                   10                  15
Gln Phe Phe Ser Met Thr Ala Gly Gly Ser Tyr Pro Gly Ala Thr Ala
            20                  25                  30
Gly Gly Gly Arg Ala Pro Gly Asp Gln Pro Phe Ser Leu Ala Leu Ser
        35                  40                  45
Leu Asp Ala Ala Ala Ala Glu Ala Ser Gly Ser Gly Lys His Ala Asp
    50                  55                  60
Gly Gly Lys Ala Asp Arg Glu Ala Ile Gln Leu Pro Gly Leu Phe Pro
65                  70                  75                  80
Pro Ala Phe Gly Gly Gly Val Gln Pro Pro His Leu Arg Ala Thr Pro
                85                  90                  95
Pro Thr Gln Val Phe His Ala Gln Gln Pro Lys Gln Gly Gly Ala Ala
            100                 105                 110
Val Gly Pro Gln Pro Pro Ala Pro Arg Pro Lys Val Arg Ala Arg Arg
        115                 120                 125
Gly Gln Ala Thr Asp Pro His Ser Ile Ala Glu Arg Leu Arg Arg Glu
    130                 135                 140
Arg Ile Ala Glu Arg Met Arg Ala Leu Gln Glu Leu Val Pro Asn Thr
145                 150                 155                 160
Asn Lys Thr Asp Arg Ala Ala Met Leu Asp Glu Ile Leu Asp Tyr Val
                165                 170                 175
Lys Phe Leu Arg Leu Gln Val Lys Val Leu Ser Met Ser Arg Leu Gly
            180                 185                 190
Gly Ala Gly Ala Val Ala Gln Leu Val Ala Asp Ile Pro Leu Ser Val
        195                 200                 205
Lys Gly Glu Ala Ser Asp Ser Gly Ser Lys Gln Gln Ile Trp Glu Lys
    210                 215                 220
Trp Ser Thr Asp Gly Thr Glu Arg Gln Val Ala Lys Leu Met Glu Glu
225                 230                 235                 240
Asp Ile Gly Ala Ala Met Gln Phe Leu Gln Ser Lys Ala Leu Cys Met
                245                 250                 255
Met Pro Ile Ser Leu Ala Met Ala Ile Tyr Asp Thr Gln His Ser Gln


                260                 265                 270
    Asp Gly Gln Pro Val Lys Pro Glu Pro Asn Thr Pro Ser
            275                 280                 285
```

<210> 326
<211> 285
<212> PRT
<213> Zea mays

<400> 326

```
Met Ala Gly Gln Pro Pro Pro Ser Gly Ala Ser Glu Asp Asp Phe Leu
1               5                   10                  15
Glu His Phe Phe Ala Phe Pro Ser Ala Ala Ser Ala Gly Ala Ala Gly
            20                  25                  30
Gly His Ala Gly Ala Gly Val Gly Gly Asp His Pro Phe Pro Leu Ala
        35                  40                  45
Leu Ser Leu Asp Ala Ala Ala Glu Ala Lys Pro Asp Arg Asp Pro Val
    50                  55                  60
Gln Leu Ala Gly Leu Phe Pro Pro Val Phe Ala Gly Ala Gly Gly Val
65                  70                  75                  80
His Gln Pro His Leu Arg Gly Pro Pro Pro Pro Gln Met Phe Gln Ala
                85                  90                  95
Gln Pro Lys Pro Gly Glu Gly Gly Met Ala Pro Gln Pro Pro Ala Pro
            100                 105                 110
Arg Pro Lys Val Arg Ala Arg Arg Gly Gln Ala Thr Asp Pro His Ser
        115                 120                 125
Ile Ala Glu Arg Leu Arg Arg Glu Arg Ile Ala Glu Arg Met Arg Ala
    130                 135                 140
Leu Gln Glu Leu Val Pro Asn Thr Asn Lys Thr Asp Arg Ala Ala Met
145                 150                 155                 160
Leu Asp Glu Ile Leu Asp Tyr Val Lys Phe Leu Arg Leu Gln Val Lys
                165                 170                 175
Val Leu Ser Met Ser Arg Leu Gly Gly Ala Gly Ala Val Ala Gln Leu
            180                 185                 190
Val Ala Asp Ile Pro Leu Ser Val Lys Gly Glu Ala Gly Asp Gly Gly
        195                 200                 205
Gly Ala Pro Gln Gln Gln Gln Gln Gln His Val Trp Glu Lys Trp Ser
    210                 215                 220
Thr Asp Gly Thr Glu Lys Gln Val Ala Lys Leu Met Glu Glu Asp Ile
225                 230                 235                 240
Gly Ala Ala Met Gln Phe Leu Gln Ser Lys Ala Leu Cys Met Met Pro
                245                 250                 255
Val Ser Leu Ala Met Ala Ile Tyr Asp Thr Gln His Pro Leu Asp Gly
            260                 265                 270
His Gly His Ser Leu Lys Pro Glu Pro Asn Ala Ser Ser
        275                 280                 285
```

<210> 327
<211> 922
<212> DNA
<213> Allium cepa

<400> 327

```
cacttcactt cccctttctt catttcttca atacctacaa accctagctt cactttacta     60
tttaaaaatg gcgtctaata acaacccgca acctccttcc gatggactca acgacgactt    120
cttcgaccaa atttttttcca tgccttccgc cttcgccgcc tcctcagcct ctactgacgc   180
caccgaagct ctcaactatg ctgattcctc caccactggg gttaccccga tgttctcact    240
tggcctcagc ttggatcagc agcctaagtc tgattcgtcc caatcggaaa gagagcatcc    300
ggttcagttt gcttgcttgt ttccacagta tgggcacaat aatcaagttc atcaaacccg    360
gcctaatctt tctcctcctc aagttcttca tggacaagca atgcagagca gtatggctgc    420
aacaccacaa cctacaggtc cacggccaag ggttagggct aggagaggcc aagctaccga    480
```

```
tcctcatagt atagctgagc gattgaggag ggaaagaata gcagaaagga ttagagcttt     540
gcaggaattg gtgcccagca ctaataagac cgatagagct gtgatgctgg atgaaattgt     600
agagtatgtg aaattcttga ggcttcaagt aaaggtacta agcatgagta gactaggtgg     660
tgctggtgct gttgcacagc tggtagccga tatccctatg tcttcttcag ttgagggtga     720
ttcaagtgaa agtgggaaga caagctacca agggtgggaa aaatggtcaa ccgacggcac     780
cgagcgtcag gtggcaaaac taatggaaga agatgtaggg gctgcaatgc aatttctcca     840
atcaaaagcc ctctgcatca tgcccatatc tttagcagct gcaatttgtc aaacgaatcc     900
ctcaaccgaa atctctttca tg                                               922
```

<210> 328
<211> 1456
<212> DNA
<213> Arabidopsis thaliana

<400> 328

```
gtctgtgtct cttcgtcctc attagcttaa gcatcatcat tctctcacct aacaactcca      60
cacaagattc ttcgcatgga aagttgttct tcgacttctc ttctctaact cgctatcttt     120
taactcaccc agctccactg agtcgaaaat ttcaaacctt tactcgtttc cttcatggct     180
aataacaaca acatcccaca tgatagcatc tccgatccat ctcctaccga cgatttcttc     240
gagcagatcc tcgggctttc caacttctcc ggttcttcag gttctggtct ctctggaatc     300
ggcggcgtgg gtccacctcc gatgatgctt cagcttggtt caggcaacga agggaatcat     360
aatcatatgg gtgccattgg aggaggtgga cctgtagggt ttcataatca gatgtttccg     420
ttgggattaa gtctcgatca agggaaagga catggctttc ttaaacctga tgaaactggt     480
aaacgtttcc aagacgatgt tcttgataat cgatgttcct ctatgaaacc tattttccat     540
gggcagccaa tgtcacagcc agctccacca atgccgcatc aacagtctac tattcggcct     600
agagttaggg ctaggcgagg tcaagctacc gatccacata gcatcgctga gaggctccga     660

agggaaagaa tagcagaacg gatcaggtcg ttgcaggaac ttgtacctac cgttaacaag     720
acagataggg ctgctatgat cgacgagatt gtcgattatg taaagtttct caggctccaa     780
gttaaggtcc tgagcatgag ccgtcttggt ggagccggtg ctgtcgcacc actagtcact     840
gaaatgccat tatcttcatc agttgaggat gagacgcagg ccgtgtggga gaaatggtca     900
aacgatggga cagagaggca agtggctaag ctgatggaag aaaacgttgg agcagcgatg     960
caacttttgc aatcaaaggc tctttgcata atgccgatct cattggcaat ggcgatttac    1020
cattctcagc caccagacac atcttcttca atcgtcaaac cagagatgaa tcctccaccg    1080
tagatttttg ttcatccaac ggtccccagc tgatgattga catttgctc tgtttcccac    1140
tactagactt ttgtgactca tgaaaggtaa gtaaaaaggc attggagatg gaatctaagt    1200
aggatttgtg cagtaaagaa gtaaaacggg atctgtcaaa agaaggaaaa agctctcgct    1260
tgcttggcta gtatttatca ttttgatgaa agtaactctt ttttgttcaa agactttagt    1320
gtgattttca ggaccaaggg ctttgagggt agtgctagct gtagtaatag taatgaaggt    1380
gtgggatcgt gtttctgaat tatgtaaaaa aggaagaaaa aacaaatgtt ggtattatat    1440
tatggttttg cctctc                                                      1456
```

<210> 329
<211> 1588
<212> DNA
<213> Arabidopsis thaliana

<400> 329

```
gctcctttct cgtctctgtc ttcttcgtcc tcattcgttt taaagcatca aaatttcatc     60
aacccaaaat agattaaaaa aatctgtagc tttcgcatgt aaatctctct ttgaaggttc    120
ctaactcgtt aatcgtaact cacagtgact cgttcgagtc aaagtctctg tctttagctc    180
aaaccatggc tagtaacaac cctcacgaca acctttctga ccaaactcct tctgatgatt    240
tcttcgagca aatcctcggc cttcctaact tctcagcctc ttctgccgcc ggtttatctg    300
gagttgacgg aggattaggt ggtggagcac cgcctatgat gctgcagttg ggttccggag    360
aagaaggaag tcacatgggt ggcttaggag gaagtggacc aactgggttt cacaatcaga    420
tgtttccttt ggggttaagt cttgatcaag ggaaaggacc tgggtttctt agacctgaag    480
gaggacatgg aagtgggaaa agattctcag atgatgttgt tgataatcga tgttcttcta    540
tgaaacctgt tttccacggg cagcctatgc aacagccacc tccatcggcc ccacatcagc    600
ctacttcaat ccgtcccagg gttcgagcta ggcgtggtca ggctactgat ccacatagca    660
tcgctgagcg gctacgtaga gaaagaatag cagaacggat cagggcgctg caggaacttg    720
tacctactgt gaacaagacc gatagagctg ctatgatcga tgagattgtc gattatgtaa    780

agtttctcag gctccaagtc aaggttttga gcatgagccg acttggtgga gccggtgcgg    840
ttgctccact tgttactgat atgcctcttt catcatcagt tgaggatgaa acgggtgagg    900
gtggaaggac tccgcaacca gcgtgggaga aatggtctaa cgatgggact gaacgtcaag    960
tggctaaact gatggaagag aacgttggag ccgcgatgca gcttcttcaa tcaaaggctc   1020
tttgtatgat gccaatctca ttggcaatgg caatttacca ttctcaacct ccggatacat   1080
cttcagtggt caagcctgag aacaatcctc cacagtagga tttctgcaat aaagagtttg   1140
tacagctaat ccaactgtcc aacatgggtt tttcttctgc tctaatgact ctggtttctt   1200
ctctcctctc tcacccactt gaaaggtaaa aaagtgaaaa aggctttgta gatggaatca   1260
atgtaggatt tgcagtagag ggaaaaaaaa tgtcaaaaag ctcaattgat caagtattat   1320
tgtaatcatt gtacctttat tttaggtgga ctttgatgaa agcaactttt tgttttcaag   1380
actttagtgg gaggttgagg aaggagcttg aagggtgtta tttattagta gtagtagtag   1440
tgggaagttg tgggaccttg ttgagttgtg ttcaaattga agaaaaaaca agtatttgta   1500
atttgtcacc ccttgtatta ttatttattt tgtatgactt tggagtagta taattaatta   1560
tcaaataata ttattagttt gatctagt                                      1588
```

<210> 330

<211> 1407

<212> DNA

<213> Arabidopsis thaliana

<400> 330

```
tcttcccaaa aaaaagtgta gaagcagaag aaacccatca tcatcatgat gaactcttct     60
cttctaactc cttcttcttc atcttcttcc catatccaaa ctccatcaac aactttcgac    120
cacgaagact tcctcgatca aatcttttcc tcggccccgt ggccctccgt cgtcgatgat    180
gctcatcctc ttccctccga tggcttccac ggccacgatg tcgactcaag gaatcagccg    240
atcatgatga tgcctttgaa tgatggctcc tccgtccacg ctctttataa tggcttctcc    300
gtcgccggat ctcttcctaa ctttcaaatc cctcagggat cgggaggagg attgatgaac    360
caacaaggac aaacgcaaac gcaaacgcaa cctcaggcga gcgcgtctac agctactggt    420
ggtacggtgg cggctccgcc gcagagtagg actaaaatcc gagctaggag aggtcaagca    480
actgatcctc atagtatcgc cgaaaggtta cgaagagaga gaattgcgga aagaatgaaa    540
gctcttcaag aactcgttcc taacggcaat aagacagaca aggcatcgat gctcgatgag    600
atcatagatt atgtcaagtt tttacaactc caagtcaagg tactgagcat gagcagattg    660
ggaggtgctg cttccgtttc ttctcaaatc tccgaggctg gtggatccca cgggaacgca    720
tcctccgcca tggtcggcgg tagccagacg gccggaaact ccaacgacag cgttacaatg    780
acggaacatc aagtggccaa actaatggaa gaagacatgg gctcggccat gcaatatctt    840
caagggaaag gtctttgtct catgccaatc tctttagcca ccgccatctc aaccgccacg    900
tgtcactccc gtaacccttt gatccctgga gctgttgccg acgtcggagg tccttcccct    960
cccaatcttt ctggcatgac catacagtcg acgagtacaa aaatgggtag cggtaatggg   1020
aaattaaacg gtaacggcgt gaccgagagg tcgtcttcta tcgccgttaa agaggccgta   1080
tccgtttcga aagcgtgata acggccgttt acttttgggg ttaggcagag agataccaaa   1140
aacaaagaga aagtggggtg aagtgggaga taaagtcaaa gtctacgaag aatgaggttg   1200
aggttgtttg atgtgttctc tgaacgaagc catatttttc gtagagtttt ggttctttta   1260
cctacgcact acaaaaccat tcaaggacat tttctttttc tttttaaat atatggttgc   1320
aatatgtttt atttttatta aataaagttg gtatcgttga tctgaatatt aggctagaaa   1380
aagaaaaaaa tagtatattg gtttttc                                       1407
```

<210> 331
<211> 1230
<212> DNA
<213> Arabidopsis thaliana

<400> 331

```
aacattcatt ttcattctca ctccccacca aaaaaaaaa aaacagaagc catgaactcc     60
tcgtctcttc taactccttc atcatctcct tctccacatc ttcaatctcc tgcaacattc    120
gaccacgatg atttcctcca ccacatcttc tcctccactc cttggccctc atccgttctc    180
gacgacactc ctccaccaac ttccgattgt gcccccgtca ctggattcca ccaccacgac    240
gccgattcaa gaaaccagat cactatgatt cctttgtcac ataaccatcc taatgacgct    300
ctcttcaatg gcttctccac cggatctctc cctttccacc tccctcaagg atcgggaggt    360
caaacgcaaa cgcagtcgca ggcgacggcg tcagccacca ccggtggtgc aacggcgcaa    420
cctcagacaa agcctaaagt ccgagctagg agaggtcaag ccactgatcc tcacagtatc    480
gccgaacggt tacggagaga gaggatagcg gaaagaatga aatctcttca agaacttgtc    540

cctaatggta acaagacaga caaagcatca atgctcgatg agattatcga ttatgtcaag    600
ttcttacagc tccaagtcaa ggtactaagc atgagtagac tgggcggtgc tgcttctgct    660
tcttctcaaa tctctgagga tgccggtgga tcccacgaaa acacctcctc ctccggcgag    720
gcgaagatga cggagcacca agttgcaaag ctaatggaag aggacatggg atcagccatg    780
caatatctac aaggcaaagg tctttgcctc atgcccatct cgttagccac caccatctcc    840
accgccacgt gtccttctcg tagccccttc gttaaagata ccggcgttcc tttgtctcct    900
aacctatcca ctacaatagt tgctaacggt aatggctcat cgttggtcac cgttaaagac    960
gctccctccg tttccaagcc gtgataacgg ccatttgtcc atttcatttt ccctttttg    1020
ggtgggaaag agagaaaaaa gtttagaaga caaagacaag tgggataggt ggttttggtc   1080
aaagtttaga aagaataagg tcgtgttttc ggatacgaca ccgtatttgc gtacactttg   1140
gttttctgtc tttacctact acaaaccacc cataagcaca ctcatgttat catgtttttt   1200
ttttttggt ttataaagtt atatccttaa                                     1230
```

<210> 332
<211> 1019
<212> DNA
<213> Arabidopsis thaliana

<400> 332

```
atggaaaatg gaaatggaga aggaaaagga gaattcataa accaaaacaa tgacttcttc     60-
cttgattcca tgtcaatgct ctcctctctc cctccttgtt gggatccatc tcttcctcct    120
cctcctcctc cgccacaatc tcttttccac gctctagccg tcgacgcccc cttccccgac    180
cagttccatc atcctcagga gtcaggaggt ccaacaatgg gtagccaaga agggttacag    240
ccacaaggta cagtgtcgac cacgagcgca cctgttgttc gtcaaaagcc aagagttcga    300
gccaggagag gccaagccac cgatcctcac agtatcgctg agaggcttag aagggaacgc    360
atcgcagagc gtatgaagtc tctccaagaa ctggttccca acaccaacaa gacggataag    420
gcatcaatgt tggacgagat catcgagtat gttaggttcc ttcagcttca agtcaaagta    480
ctaagcatga gcagattggg aggtgcagga tcagttggtc cacgcctcaa tggtctctcc    540
gcagaggcag gaggacggct caatgccctc actgcaccgt gcaatggctt aaatgggaat    600
ggaaacgcta caggatcgtc caatgagagc ctaaggtcaa cagaacaaag agtggcgaaa    660
ctgatggagg aagacatggg atcagcaatg caataccttc aagggaaggg gctttgctta    720
atgccaatat ctctcgcaac cgcgatatca tcatcaacta ctcactctcg tggatccctc    780
tttaacccca tctccagtgc tgtagcagca gaggattcaa atgtaacagc aactgcagtt    840
gctgcacctg aagcctcgtc tactatggat gatgtatctg cttccaaggc ctgaaccata    900
gaatactcat gtgtttcgtt ttctcagttt aagtcaaacg ctaatctccc tatcttttta    960
ctattttctc tgctaagcaa caataatata aaacagttgc aacaaagaac aaaaccgac    1019
```

<210> 333
<211> 1763
<212> DNA
<213> Aquilegia vulgaris

<400> 333

```
cactttcaaa actctctctc tctttctctt cccgaaactc aaaatttctc cggtttatcg     60
ggtatcggat ctgtaacttg tttcgccgac aacctcgttt ttcgattttc tctcataaac    120
ccaaaacaaa accctttgtc tatttctatg tttaatgttc ttcaaaaacc ctaacacttc    180
aatcaaacac gcatacactc ttactctctt ctttctcaca acatggctaa taataataat    240
cctacagaag gattaacaga tgatttcctt gatcagatct tatcttatgg tgctcacgaa    300
ggtaatttgg cgggaaatga tgttaatttg gcgggaacaa ctacacctgg aggttcgatg    360
gccttgcagt tgagttcttc tggaggtgat attaccggta gtggtgggta tcatcatcat    420
catcagcatc aacatcatca tcagttaagt ggtggtggtg gtggttttcc gttagggtta    480
agtttagaac aaagtggtaa aggtggtttt tttaaaccag atgaagcttc tggaagtggt    540
aagcggtttc gtgacgattt cgttgatttg aaagctgttt cttctactaa caacgataac    600
aacgatagag gagactctgt acatttgaat aatttgtttc cagcatttgg acatgtgcaa    660
caacagcaac aggctcactc tgttcgacct ccttcacatc aaaatcaaat caaccagcct    720
ttccttgggc atccaacacc tggggctgtt gctgttgtac cacatccacc tgccattcgg    780
cctagagtgc gagcaagaag gggcaagcc actgatcctc acagtattgc agagaggtta    840
cgtagagaaa gaatagctga aagaatgaag gcactacagg aactggttcc tagttccaac    900
aagactgata gggcagctat gcttgatgaa attgttgatt atgttaaatt tctaaggctc    960
caggtcaagg ttttgagcat gagcagactg ggaggagctg gtgcagtagc acagcttgtg   1020
gctgacattc cactttcatc agctgagggc ggagccagtg aaggtggcag taatcagcca   1080

gcatgggaga aatggtctac agatggcaca gaacatcaag tagcaaagct catggaagaa   1140
gatgttggag ctgccatgca attcctccaa tctaaagcac tttgcatcat gcccatttca   1200
cttgcctctg caatatacca cgaccaacca cccgatgcct ccacaatgat caaacccgaa   1260
cctaatgcac cttcataaaa tgaaaaggat acatgcaatt ggacgtgccc atgaacaact   1320
ccaaacttac aactatccca ccttccattt gttcccccca cgtcagactt agtcaactcc   1380
cctcagcatt gcaatcaaag tcagatgcca tattattcgt tctgcctttc atttttttcg   1440
tcaaataggt tgtaatgtga tgcctagaaa aataatcata ggtactcctg atgtggtgtg   1500
ggatagtggt ggatagccag aacactttcc ggtggggacc cttcttttgg atagtggagg   1560
gtggggcatt tttaaagggg gaagatgggt acatagacat gattaatgta ttagaaggaa   1620
actgaaaagg gaattgaaat ggatattgga tagttgttgg tcgcttaaaa aagttgtgtt   1680
ggaaggttgc actactcggt tttgagtggg ttgtgttttt tgtaaagtgc ttgatttatt   1740
tttctcaagt atctctgagt cat                                           1763
```

<210> 334
<211> 931
<212> DNA
<213> Aquilegia vulgaris

<400> 334

```
tcaactgctg ttggcagttg taatggggct gtgaagctcg tgtgagagct cgcaggggtc     60
aagcaacgga tccacacagt attgctgaaa ggcttcgaag agagaaaata gcagagagga    120
tgaagaatct acaagaactt gttccaaact ctaataagac ggataaagca tctatgcttg    180
atgaaatcat tgagtatgtc aggtttcttc agcttcaagt caaggttcta agcatgagca    240
ggctgggggc aacaggggca gttgttcccc tcattacaga aaatcagcct gagggatcta    300
ctagtcattt tctgtcgcag tcagctggtc gagatgttaa acatattgaa tctcctaaca    360
cccttacatt tgagcaagaa gtagtgaagc tgatggaatc caacatgacc aaagctatgc    420
aatttctgca gagaaaaggc ctctgcctaa tgcctattgc ccttgcagct gccatttccg    480
gtagtaaagc accacccgcc tctctttcta ctgagggaaa gaagtctatt ttgaccaatg    540
gctttgttca tcataacagt gggcaatcta acattgggcc aggtcagata tcatctgatg    600
gagagactag atcagaggaa aacataattg ggattcacag cagagaagac ttttcaagca    660
acagctgcag tgggaccagc attaaaaaag agggaacaaa caccacaaac ttcaccagag    720
aaatgaatca agagtagaca tagttttgcc ggtctcaatc tttgtggctt catacctggt    780
ctccaattct caagtttcag taacctagca agtagataag tagcagagtc tgggactgtt    840
ttgttttttt tttctcgctt caaagatgag aatgtggtat agatttctaa ttatacgtta    900
taggtttcat tgtacatgca cacatatatg c                                   931
```

<210> 335
<211> 838

<212> DNA
<213> Brasssica napus

<400> 335

```
cccacgcgtc cgaaacacct tccgatgact tcttcgagca aatcctcggt ctccccaact     60
tctcagcctc ttcatccgac ggcgggttag gcggaggagg agcaccgccg atgatgctgc    120
agctgggctc cggcgaggaa ggaggtcaca tgggtgggct aggaggagga agtgggtttc    180
acaaccagat gtttcctcta ggtttgagtc ttgaacaagg caaaggacaa ggctttctta    240
gacccgaagg tggtagtctt ggaactggga aacgtttctc tgatgacgtc atgaaacccg    300
tttttcatgg gcagccaatg caacaacagc cagctccagc ggcgccgcat cagcctacat    360
cgatccgtcc cagggttcga gctaggcgtg gtcaggctac tgacccacat agcatagctg    420
aaaggcttcg tagggaaaga atagcggagc ggatcagggc gttgcaagag cttgtaccta    480
ctgttaacaa gacagataga gctgctatga ttgatgagat tgtggattat gtaaagtttc    540
tcaggctcca agttaaggtt ttgagcatga gtcgtcttgg tggagccggt gcaggtgctc    600
cacttgttac tgatatgcct ttgtcatcat cagttgagga tgaatctggt gaaggtggaa    660
gggcaccaca acctgcgtgg gagaaatggt ctaacgatgg cactgaacgc caagtcgcta    720
aactgatgga agaaaacgtt ggagcagcga tgcagcttct ttcatctaag gcgctttgta    780
tgatgccaat ctcattggct atggctattt accattctca gcctccagat acaacttc      838
```

<210> 336
<211> 1291
<212> DNA
<213> Citrus clementina

<400> 336

```
caaccgtgtt acaccgaccc atctgctgct gcttcaaact ccgctgatga catacccatg     60
ttcctacagc tgggctccgg cggcccttct cctggtgccg gaggaggcgc caccagtgct    120
gctgacataa gaagtttggc tatgggaatc agcatgggaa tgatgcctct aggggttaaat   180
ttggagcata gcttcttaag gcagcatgaa gatcataata gtaaccataa caacaacaac    240
aacactaatg cttcttcttc ttctcctaat aattcttctg ccacttctgg aatcaacgag    300
agagattccg tgcacatgcc aagtttgttt ccagcgtttg gacagttgca aagtcaatca    360
gcccggccgc cgctgccacg tcctcctcaa gtacagcagt ttcagagcca accagcagcc    420
gcaccgcagc ctccagctgt ccgtccaagg gtgcgagcaa gacgagggca agccacggat    480
cctcacagca ttgccgagcg gttgcgtagg gaaagaattg cggatagaat gagggctctg    540
caagagctgg ttcctagctg caacaagacg gatagggcag ccatgcttga tgaaatcgtg    600
gattacgtga agtttctaag gcttcaggtc aaggttttga gcatgagtag actgggtgca    660
gctggtgccg tggctcagct tgtagctgat gttcctctgt catcagctct tgagggagag    720
agcattgatg gtggaagcag tcagccagaa tgggagaaat ggtcaaatga cggaactgaa    780
caacaagtag ccaagctaat ggaagaagac attggagctg ctatgcagtt ccttcaatcc    840
aaggcacttt gcatcatgcc catatcactt gccagtgcaa tctatcgcac ccgtcaaccg    900
gatgccccag cattcgtcaa gcctgaatcg agcacccctt catattgtct aaacaacaat    960
tggagtcagc agttggctca caatgactca aaatatttaa tcacgggtac ctattcctat   1020
agaatacttt tggtcctctt atccaaccaa ataattcaaa ttccaatgca acgttcccat   1080
tcttctttcc cttaattaac ctagggcaaa aaagggaaat gaaaagaagg caatgggcaa   1140
tggcaagggg gaaaaagggg aattttttc caaagttttt tttttttata aaaactcccc    1200
ttttcaaaaa aaattataaa gggggggggg gggtttttta gggggggggg aaaaaacccc   1260
caaaaaaggt tttcgggggg tgttttatat g                                  1291
```

<210> 337
<211> 1344
<212> DNA
<213> Curcuma longa

<400> 337

gcacccgttg agacctaacc ttccccggga aactggcggc tgtgcctatg caaccgagca 60
gcaaagagac ggaaaggatg gcgcagtccc acgccgccct ccagctggag ctccagaacg 120
gcggcggagg cgcccagaac gacgatttct tcgagcagac gatgtccggc ttctccaccg 180
ccgcctgggc accggagttg gggaccccca ggtcgctttt cggggcgcgg tcttcggagg 240
agtcgccgga cgacgaggga ttgaactacg ccccaccgta cggtgggtcg tctcttctcg 300
cctcgcggca ccggatgcac ctcggcacct cgccggcgga ttcgtcgatg tttctccagc 360
tcggtgggca gattctgtta catcctacgg ccggcgcctc cgggggcgag tccgggggct 420
tcctccggtt gcccctctcc ctcggtagcg gaggctctgg ggtctcgact tctgccatct 480
tcggcgacag atcccgagga gaaatcgacc cgccgttcga atcgtccaat cccaccgagg 540
ctgaggtgtt gtataacgac ggattcaccg gatcgcttcc acgggcggtg caagcgtccc 600
ttcatcagca actcctccga caaccccaga actacggagc tgctacgctg accggacagg 660
ctcttgcgat aacagcaacg gcttcaggta ccgccggcgc aggtactgcg ccacccaagc 720
cgagggtgag ggccagaaga ggtcaagcga ccgatcccca tagcattgcc gaaagacttc 780
ggagggagag aatcgcagag cgtatgaaag ctctgcagga attggtgccc aacgctaaca 840
agacggacaa ggcgtcgatg ctggacgaga tcatcgacta ccttaaattt ctccagctcc 900
aagtcaaggt cttgagcatg agcagattgg gcggagcagc cgccgtcgcc ccgctcgtcg 960
ctgacatgtc ttcagagagc ggcggcggag cgggaacgcc gaggagtaat aacgatggcc 1020
tgacggcggc ggagcagcag gtggcgaagt tgctggagga ggacatgggt tcagcgatgc 1080
agtacctgca ggggaaaggc ctctgcctca tgcccatctc cctcgcctcc gccatctcct 1140
ccgccacctg ccgtcctcgg cctccccccg gtgtcaacct ccgctacccg gcagccggcg 1200
acgcgcctcc gtcgccgagc atatcggcgc tgacagtcca atcgtccaac gccggcagcg 1260
ccggcgccga cgccagctag gatggagctg ttcgttaaga tcgctgtcca aaaagtgctt 1320
ggaattggaa tttcaatcct tagt 1344

<210> 338
<211> 1235
<212> DNA
<213> Citrus paradisi hybrid

<400> 338

cgactttgcc gagtcaaact catggcgaac aacccgaacg aagcctcctc gactgacgat 60
ttcctcgagc aaatcctcgg aattcctaat ttcggttctg cggagtccgg cttggcggct 120
agcgatggcg gcttggctgc aggctcgccg atgatgctgc agctgagctc cggcgacggc 180
tccagccaca tctcggctct cggaggcgga gtaagtagtg ggtatcatgg gcaggtgttt 240
ccgttaggct tgagcttgga gcaaggtaaa ggaggatttt tgaagcccga ggaagcctct 300
ggaagcggaa agcggtttcc tgaagaacat gctataaaaa atgtttttca tggccaacca 360
ctgccctcac ctgttcctgc agctccacat ccaccagcaa tgcgccccag ggtgcgagct 420
agaagaggac aggccactga tccacacagc attgctgaac gtctacgaag agaaagaata 480
gcagaaagaa ttagggcact gcaggagctg gtccctagtg tcaacaagac tgatcgagca 540
gccatgcttg atgaaattgt ggattatgtg aagtttttga ggcttcaagt gaaggttttg 600
agtatgagta gagtgggcgc tcctggcgct gtggctccac tggtaacaac cgacctccca 660
ctatcatcag tcgaggatga atctggtgaa ggggtaagaa accaaccagc ctgggagaaa 720
tggtcaaatg atggcacaga gcgacaggta gctaagctca tggaagaaaa tgttggggct 780
gccatgcagt tccttcaatc aaaggctctt tgcataatgc cgatctcact ggccacggca 840
atttaccatt cgcagccacc ggaatgaaga ggtaggatgg ataatggttg gttgtttgaa 900
aatgcttagt gttttcccag tccaacataa aatgatagtg cttgctttgt cgaggaggcg 960
tgggctttgt tttcaaaaag taatttgact tctttctcat tttctcctga gtcattcatt 1020
attggagggc gacgggggctg ctgacgatgg ctgatggctg atggcattgg tattggtgga 1080
ctgcgtttgg tataaattac ttacgtattt gaacttaacc tgattttcaa ttctaaagtc 1140
atttactgcg gtacaaaatt tgaaaaatct attactgaat ttagttgata ataaccaact 1200
tagcgtgttc ctttcgggga tcatccatcc aactc 1235

<210> 339
<211> 1916
<212> DNA
<213> Citrus sinesis

<400> 339

```
ggggaaacac caaattcctt ctctccctct ctctccctct ctctctctct tcttcctgtt      60
ctcacctatt tagtatttaa catccatcag aagaatggtt tccattctct tgaagaaact     120
cttgactctt caaacttaaa tttccatatc gtaaagactt attctcaaga aattaagatg     180
cagccctgca gcagcggcgg agaaatgcaa ggaatcagct cgctcttgag caacggaagc     240
cacgagcagc agtcgcagat ccatcaaagc gcgacgtttg atccaacttc gcaagacgac     300
ttcttagagc aaatgctatc ctctctccca tcgtgttctt ggactgactt gaagtctccc     360
tgggggggttg ttgaccttaa ccctaataac aataataata atattaacaa caagcagccc     420
agagacttat ccgacgaaac ggcgccgtcc actactcaag agaataatgt ccctgcaggg     480
tttcagttcg acgagtccat gattttagct tccaagatga gacagcatca gatcagcggg     540
aatactggga gtgggaataa taattctgct gcagcaaagt ttatgatgca gcagcagcag     600
caacaaatca tgatggctgc tgctagaggt ggcatcgggt tgcctttatc acttggaaat     660
ggtggcgata acgacatcgt tgatgtctcg tcattcaagt cccaacaggg aggagatggc     720
tcagtgcaag cactctacaa tggcttcact ggatctttgc atggctcaac ccagcctcaa     780
cattttcatc atcttcaggg aggatcgatg cccgggcaga cctttggggc accagggccg     840
gttatgaacc agacgcaggc tcaggcaagt gggtcaaccg gtggtggtgg tggtggtgga     900
aacacacccg ctcagcagcc taaacaaagg gttagggcta ggagaggtca agctactgac     960
cctcacagca tagctgaaag attgcgcaga gagagaattg cagagagaat gaaagctctt    1020
caagaacttg tacctaatgc caacaagaca gataaggctt cgatgctgga tgagatcatt    1080
gactatgtca aattcctcca gctgcaagtg aaggtcctga gcatgagtag attgggcggt    1140
gctgctgctg tcgcacccct tgttgctgat atgtcctcag agggaggagg aggtgattgt    1200
atccaagcaa acgggcggaa ccctaacgga gctcagacga cgtcagctaa cgacagctta    1260
actgtgacgg aacaccaagt ggctaagctg atggaagaag atatggggtc agccatgcag    1320
tacttacaag gcaaagggct ttgtctaatg cccatctcac ttgcaactgc tatatcgact    1380
gccacgtgtc actctaggaa ccccatcatc agcaccagca acaacaacaa taacaacggc    1440
aatccccacc acaatccttt gcttcagtcc aatggtgagg gcccgacctc acctagcatg    1500
tcagtgctga ctgtccagtc agcaactatg ggtaacggtg gggctgacgg ctccgtcaaa    1560
gatgctgctt ccgtttccaa gccttgatca acggcgtcga ccgactttat gatgcggctc    1620
gtccaaagtc tacgaaaata aggcgtcact agatgttgga ccttcacgac gtcgtatttg    1680
tgtagacttt gtgactgaat ctaacaaaca acaaaaaagc aagaaagaag ccaatcccaa    1740
gaacaaaaac cttattttat gttattatta ttattttaat ttgcgggtta ggttttgtta    1800
attttactat tattaaattt ttaatttctc agatgggata tctccctctt tttcttttat    1860
ttaatattct ctgtcatgat tctctattaa ataattattt acaaaagcct ttaatc        1916
```

<210> 340
<211> 1835
<212> DNA
<213> Eucalyptus grandis

<400> 340

```
agcactttca cttatcagag gccgaaagca aaaccccctg aacacttcaa agaaaagagg     60
ttttttcaga caccctctcg ccctctctct ctctctctct ctgagctgag agacaatacc    120
agcttataaa acccgagacc cataaaacct cccatcggaa ttcccgaatc acgttaccgg    180
gtcatcgggt ttcggatctg ccacccagtt caccaacacc accaccacca ccttccttcc    240
ccccctcctt tttgggtttc ccaatgagca catctcacct ccacttcctc cctccccttc    300
ccttaaacat tctctcccac caataatttc cacattggca accaatttct tattcatcat    360
cccctccccg agtcccacca accttaggct ctctctctct ctctctctct gccactcaat    420
ctttccactt agaaagaaac caacttttg ctgggtttcg catagagcag aaaacccatc    480
gtagactttg atctccagtt gacacttcta tagagaaagg gagatagagt ggagagagag    540
agagagagtt tataatcatc ggcttatggc aggctcaaat ccgcaggaag gactggggga    600
tgagttcttt gagcagatct tggccgtgcc gcccggagct tactccggtg gctccacgcc    660
catggtcttg caactcggct ccggccgcgg cggcgtggaa gatggcggcg gcagaggagg    720
cggcgggggg ctgaggggca tgggggtgat gatgcccctg gggctgaatt tggagcaagg    780
tggattgttt aggcacgagg atgttgagaa cagtactagt gcatcttcca ccacttctgc    840
catcaatatg gagagagaag caggagtcca ccacggcaac aacatgacaa gctgcttgtt    900
tccagcattt ggacagttcc agactcatca gtcagtccag ccacctcctc cccatccacc    960
ccctcctcaa cttcacccgg ccttcttgaa tcagcccgca gtgggatcgc cgaaccagcc   1020
ggcgatacgc ccaagggcac gagctagacg agggcaagcc acggatcctc acagcatcgc   1080
tgagcggttg cgccgagaaa gaattaatga aagaatgaag gcgttgcagg agttggttcc   1140
cagttgcaac aagacggata gagcagccat gcttgatgaa atcgtggatt acgtgaagtt   1200
cttgagactc caagtcaagg tcctaagcat gagtagattg ggcggagctg gtgctgtggc   1260
tcaacttgta gctgatgtac ccctctcatc agctgagggc gagatcatcg aaggcgggaa   1320
caatcaaccg gcgtgggaga agtggttgac agacggcaca gagcagcaag tggctaagct   1380
gatggaagaa gatgttgggg ctgcaatgca gttcctccag tccaagacgc tctgcatcat   1440
gcccatttcg ctcgcctccg caatcttccg cacaagccaa ccagacatgc cccggtccat   1500
caagcccgaa tccagcgccc cttcctgaag caccctgaac aagttcctgg cgacatcata   1560
gttgcaacaa tgcctaggtt tctcaggtct gaccggttcg aggttgtccc caagtcgacg   1620
taactccttt gccaaccaag ttgcttagtg ccctctgttc atagttcact gtcgctgtct   1680
tagagtgggt gtgaacttgt ggttctcatg ccttaattac aacctaaaaa aatgcatcag   1740
tttcatgttt tttccaattc atccaaggaa aagtcaaaag caactttatt ccaaattcag   1800
cttattaaaa cctatcttct gatgaaaaaa aaaaa                              1835
```

<210> 341
<211> 1765
<212> DNA
<213> Eucalyptus grandis

<400> 341

```
cctcctcgcg tccctctgcg ttccgccagc taaccgaaat tccggccacc cccgccggag     60
ctccgccgtg aaaccctctc tccgtctccg cctccgcctc cgcctccgtc tccgtcgctt    120
ccttccgccg tcgcgaccgt agatagatgc ccttcccgcc cgctcatggc gaacaacccg    180
agtgacggcg ccagcgacga gttcctggag cacctcctcg gcctccccaa cttcgctgcc    240
gcggcggagg ccggcctcgc cccgggcgac gggaccggcc tgtccgtcac cgcggccacc    300
ccgatgatgc tccagctcgg ctccggcgac ggctccggcg gccacggcca cggccacggc    360
cacggccatg gccaaggcca cttgtcggcc ctcggcggcg ggggagcggg ggcgggggc    420
ggcggtggcg gcggtggcgg ggggtaccat ggggcggtgt tcccgctggg gctgagcttg    480
gagcagggga agggagggtt cctgaagccc gaggaggcgt ccgggagcgg caagcggtac    540
cccgaggagg tcgtcgacgg cagagcttcg actgtgaaaa acctgcactc cccgttgccc    600
aacttcccag atgtttcgca aatggcttct tttaggaacc gagatgtgtt tcatggacaa    660
ccagtgccca accctgttcc tgcagcacca catccaccag caatgcgccc gagagtaagg    720
gctagacgag gacaagctac agatccccac agcatcgctg aacgattgcg gagagagaga    780
atagcagaaa ggattcgaca attgcaagat ctggttccaa gtgttaacaa gacagataga    840
gctgccatgc ttgatgaaat tgtggactat gtgaagttcc tgaggctcca agttaaggtt    900
ttaagcatga gcagattggg cgcagctggt gcagtggccc cacttgtgac cgacatccca    960
ctatcatcag tcgaggaaga aggcggtgaa ggtggaagaa accagccagc gtgggaaaag   1020
```

```
tggtcgaatg acggcacgga gcggcaagtt gctaagctca tggaggagaa tgttggcgcc    1080
gcaatgcagt tccttcaatc caaggctctc tgcatcatgc caatctctct agccaccgcg    1140
atctaccaaa ctcaaccgcc cgacacctcc tcccttgtca agtctgaaag caatccacca    1200
tcctagacca gccacaacac aggcacctcc ccttttttcca ggtcccacca aaggctcaag    1260
acttaaatgc tccttggtcc ccgctttagt ttgtcccgtt tctacacctt caaaaaccag    1320
ttgatggccc ttcttccttc ctcaaattgc aatcaaagtc agatgccata gcccttgctt    1380
ttctgccctc tctttaagct tttcgcccgg acaggttcaa tgtcaatgcc taataaaata    1440
ctagcaggcc cttcatgtct tgccaagttc aactaaagga gagatattca aaccctcact    1500
tgatatgctc tctctctctc tctctctctc tctctctctc tctctctctc tctctctctc    1560
tctctctctc taccaataga atatgcttag tggagaatga tgtgagcgac tttatagtgg    1620
gtgggggctc ttgtttttct tggtagtggg tcgtgggacc ttgtaaaagg gattggttgt    1680
gtaaagtacc atttgtataa tgtaatgcgt aatgtgttcg atgaggggga atggattgct    1740
tggtagaaaa ttgagaaaaa aaaaa                                           1765
```

<210> 342
<211> 796
<212> DNA
<213> Gossypium hirsutum

<400> 342

```
cgatagtggt ggtggtgggt ttagaggaat aggcatgatg cctttagggt tgaatttgga      60
acatggattc ttaagacatg aagatggagt tgtagttgac aacaacaaca acaatgcttc     120
ttgttcagct gcttctgctg tttctggaat cagtgagaga gattctatgc atatggtaag     180
cttgtttcca ccttttggac aaatgcaaac tcagcaaatc cgcgcttcac cgccacctcc     240
tcagcctcca ccgcagctcc accagccatt tcacagccag ccaacatcag gtccagttgc     300
tgctgcaccg cacccacctg ccgtccgtcc aagggtacgg gctaggcgag gacaggcaac     360
ggatccccac agtattgctg agcggttgcg tagagaaaga atagctgaaa gaatgaaggc     420
tttgcaagag ttggttccta gctgcaataa gacggatagg gccgctatgc ttgatgaaat     480
tgtggattat gtgaagtttc ttagacttca agttaaggtt ctgagcatga gcagactcgg     540
tgcagcaggt gcggtggctc agcttgttgc cgatgtaccc ctactatccg ttgagggtga     600
tggtgcagga ggtggaaccc agcctccttg ggagaaatgg tcaaacgatg gcactgaaca     660
gcaagtcgct aagctgatgg aagaagacat cggagctgcc atgcagtttc ttcagtccca     720
ggcactttgc atcgtgccga tatcactggc atctgcgatc ttccctgcac atcaacctga     780
tgcaccaaca atcgtc                                                     796
```

<210> 343
<211> 918
<212> DNA
<213> Gossypium hirsutum

<400> 343

```
atgataagca atggaggtga atcttctgag tttcggagat catttacagg cttggaaact      60
ctttctccaa ttccacaatt atggcatcta caaccttatg actctgtttc ttcccttccc     120
actttggtgg gacaaaccat agtagatgat gaaggcaata ttaacagatt tattgagatt     180
gatgaaattc tgcaacctga gaacttatct gcttccatca attcaaaggg acaacaggac     240
atgcaaaatt cctttttgctc ttcttttccc gctgaccacc ctataacaaa gaccatgatt    300
gggttgccat ctctggtgca gggtgcatcg cctaacctga acaatggttc cgaccgaact     360
gtgaagcctc gagtaagggc acgtcgaggt caggctactg atccacacag cattgcagag     420
aggcttcgaa gagagaagat tgctgaaaga atgaaaaacc tgcaagaact tgtacctaat     480
tccaataaga cagacaaagc ctctatactt gatgaaatca tagggtatgt caagtttctt     540
caactccaag tcaaggtact aagcatgagc aggcttgggg cagcagctgc agttgttcct     600
ctcatcactg atggtcgagc tgaggtatct aatggtttat cacttgcacc attagcgggt     660
caagggggttg atttttcacc atctcctgat caagttgttt tcgaacaaga agtggtgaag    720
ctcatggaat ccaatatgac aatggccatg caatatctac aaagtaaagg tttctgtctt     780
atgcctgttg cacttgctgc tgccatatcc aatgttaagt catccacatc ctcatcgtca     840
tcatcagttc ctgcttctga tgagagtaag aaacttgggc ttcaccaaca gtactctaat     900
caacaacacc tgcagcag                                                   918
```

<210> 344
<211> 1530
<212> DNA
<213> Gossypium hirsutum

<400> 344

```
aaaaacccaa cactcttttt cattcctctg tttttctctc tcccactttc cgtttctctt      60
tcagaaaaag gaaagttcaa gctaagctaa aactctgaac tttccccttc tcagtctcct     120
cagggacaga gaaagattaa acgaaaggaa aagaaaaaga cttcttcttt ttttccctaa     180
aagaaaagtt gatctttttt tgtttggttc gattctcttt agccatttcc tttttgcatg     240
tagatgctct ttggtagcct taactcgcca cttcgactca cttcaatcca tggctaataa     300
ccccaacgag tcccccgctg atgatttcct cgagcaaatc ctcggactct cccactttgc     360
gcctactgag actggcttag cggggcccga cggtagattg tctggaaacg ccacgacggc     420
tggagcgcca atgcttctgc agctcagctc cggcggtggc accggacaca tcggcgctat     480
aggcggcggt ggaggcggtg cgtttcacgg acaggttttt ccattgggat tgagcttgga     540
gcaaggaaaa ggtgggtttt tgaagcccga ggaagcatcc ggtagcagca agcgcttccg     600
caacgaggtc gttgatggca gagctttttc tgttaaaaac gttttccatg gacaaccagt     660
gccagctact gttgctgctg gaccacatcc accagcaatg catccgaggg tgagggctag     720
acgaggacag gccacagatc cacatagcat tgctgagcgg ttgcgcaggg agagaattgc     780
cgaaagaatc cgggcattgc aggaacttgt tcctagtgtt aacaagactg atagagcagc     840
catgcttgat gaaattgtag attatgtgaa gttcctgagg ctccaagtta aggtcttgag     900
catgagtagg ttgggcgcag ctggtgcagt ggcaccactc gtaacggacc tcccactatc     960
gtcagttgag gatgaaagtg gtgagggagg aagaagccaa ccagcctggg agaaatggtc    1020
aaatgatggc acagagagac aagtagctaa gctgatggaa gaagatgtcg gagctgccat    1080
gcaattcctt caatcaaagg ctctttgcgt catgccaatt tcactggcca ccgcaattta    1140
ccatacgcaa tctccggata cctcctctgt tgttaagccg gaaacaaatc ctccttcata    1200
gacacctcac atgagaaaaa gaggaaaaga aaacggtgta tcatatggtg ggggagactg    1260
tggacaaaga cggaagcact tttcttgtgt agtgatgggg catggatttt tgggtttggt    1320
gggatgaggg acccgcaaaa ggagatggat gagatgagta tatttacata ttataacaat    1380
aaaatattac aatattgata gtgttagtgt aagtaacagt gtaatttcac aggaaagggc    1440
taaagtagaa gaagggataa tggttggctg attttataaa atgctgattc aataaaatag    1500
atggattggt tcacgtttca aaaaaaacct                                     1530
```

<210> 345
<211> 1561
<212> DNA
<213> Gossypium hirsutum

<400> 345

```
aaaaaaagaa gaggaaagcc aaaactccaa actttctatt ttgcttttat attatatttt     60
tttttggttc aattcagttt cgccatttcc tttgcatgta gatgctcttt ggttagtccc    120
taacgccgct acttggactc actttccgct gctatctttt ttccggcttt ctcatggcta    180
ataacaccaa cgaggccccc gccgccgatg atttcctcga gcaaatcctc ggcctcccta    240
actttgcgcc ctccgaaacg ggcttagctg gatccgacgc tggattggct gcaaccgctg    300
ctggagctgg agctccaatg tttttgcagc tcagctccgg tgatggcgcc gctcatatcg    360
gtggaatcgg cggcggtgga ggcggtgcgt ttcatgggca ggttttcccc ttgggcttga    420
gcttggagca agggaaaggc gggtttttga aacccgagga agcttccggt agtgggaagc    480
ggtttcggaa cggggtcgtt gatgaccgag cttcttctgt aaaaaatgtt ttccatggcc    540
aacccatgca agcaactgtt tctgcagcac cacatccacc gacaatgcgt ccaagggtgc    600
gcgctagacg aggacaggcc acggatcctc acagcattgc tgaacggttg cgcagggaga    660
gaattactga aagaatcagg gcattgcagg aacttgttcc tagtgttaac aagactgata    720
gggcagtcat gcttgatgaa attgtagatt atgtcaagtt cctgaggctt caagttaagg    780
ttttgagcat gagtaggttg ggcggagctg gtgcagtggc accgcttgta acagacattc    840
cactatcatc agtagagtat gaaagcggtg agggtggaag aagccagcca gcatgggaga    900
agtggtcaaa tgatggcaca gagcaacagg tagctaagct gatggaagaa aacgttggag    960
ctgccatgca attcctccaa tcaaagtctc tttgcattat gcctatctca ctagccactg   1020
caatttacca cacacaggta ccagatacct cctctgttgt taagccagaa acaaatcctc   1080
ctgcatagac ctgaggggaa aaaaagggta ggcatcccac agtccatcct tcatcccgcc   1140
ttttatttgt cctattctaa cacacccact ccctccctct ctggcttgaa aatgccatca   1200
aagtcagatg ccatagctct tgctttttgc cttcctttcc agctcttttt cgatttttca   1260
aaacagggtt tgatgtcaat gccttaaaaa aatcagagac aggcaattga tgtctcgaca   1320
agttcgagta aaggggaaga attgtcaaat ttcacttgat atgctcttct tttttgtatc   1380
aaaatgggat agtggacaat gatgggagca cttttgtagt ggggctctga ttttgtggtt   1440
agtggattgt gggacctgca aaaaaaaaaa aaaggataga gatgatccag ggtataatat   1500

tgttattgta acaatgtatt ttgaagataa aatcgaatgc aaaagtagaa gaattgatat   1560
t                                                                   1561
```

<210> 346
<211> 1411
<212> DNA
<213> Gossypium hirsutum

<400> 346

```
aaaaaatccc tttgaaaaaa aaaattccct cttttccttt ctgttacttt cacagagctg     60
aaagtacaga acagaaaaga aaccccaaac ctcaaaattt tctctttctc acgttgatag    120
aaggatttaa gctagaacgg caaagatatg aaataatatc gtgttttttg tctataaata    180
ttttgttggg ttttcaattc ttttttcgcc atttcttttg catgttgatg atctttggtg    240
gctttaactc ggtacttgga ctcacttccg ctactacttc ttcgggtttc acatggctaa    300
taaccccaac gaggcttccg ctgatgattt cctcgagcaa atcctcggat tccctaactt    360
cgcgccttct gagacgggtt tggcgggacc cgacggtgga ctgacaggaa caactgctgg    420
tgccggaacg ccaatgtttc tacagctcag ctctggtgat ggagccagtc atctcggcgg    480
aatcggcggc ggtggaggcg gcgcgtttcc cggacaggtt tttccgttgg ggttgagctt    540
agaccaaggg aaaagcggcg ggttcttgaa gccggaggaa ggttctggtg gtagcagcag    600
gcggtttcgc gatgaagtcg ttgatggcag ggcttcctca gttaaaaacg ttttccatgg    660
ttcaccaatg ccggctaccg ttgctccatc accgcatcca ccaacaatgc gtccaagggt    720
gcgggctaga cgaggacagg ccacggaccc gcatagcatt gctgaacggt tgcggaggga    780
aagaattgcc gaaagaatcc gagcattaca ggaacttgtt cctagtgtta acaagactga    840
tagagcggtc atgcttgatg aaattgtaga ttatgtcaag ttcctgaggc tccaagttaa    900
ggttttgagt atgagtaggt taggcggggc gggggcagtg gcaccgcttg ttacagacat    960
cccattatca tcggtcgagg acgaaagcgg cgacggtgga aggaaccaac cggcatggga   1020
gaaatggtca aacgacggca ccgagcgaca agtagccaag ctcatggaag aaaacgtagg   1080
agctgccatg caattcctta aatcaaaagc tctttgcatt atgccaatct cactagccac   1140
cgccatctac cactcgcaac cactggatac ctcttctatc gtcaagcccg aaacagatcc   1200
cccaccataa ctctaacaaa caaaaaataa gggtatgcat cccacacagc ccatcctata   1260
tgtccaacac tatgaggaaa aaaaaaggaa ggggggcct tcatgtccgg atttgtcaat     1320
ggtgatctct tttgtagtca atggtggggg taggggataa tgatggaaga agaggggcac   1380
tctcatagtg gggctttaa atttttttgt t                                   1411
```

<210> 347
<211> 1526
<212> DNA
<213> Gossypium hirsutum

<400> 347

```
caaaaagaaa aaaagagaaa ctcgtccctt catcttcaat ctcattcatc atcaaagcaa      60
aaaaaaaacc ctaattatgc agccttgtag tcgtgaaatg caagcaatga actctctctt     120
aaacccgacc caacaaatcc ctctccaaga ccttcaaatc aacggtaaca gacaccacca     180
tcaacagatc cacgtcccat catcatcgca gttccagtat cccacaccca cttcaacaac     240
ccaccatgac gacagcttct tagaacagat actctcctcc accacgtcct tcccgtggtc     300
cgacgaaacg ggtcctccca accccgagga taacaataac gccggcttca actacgatga     360
gatggttcta gcttccaagc ttcgacagca tcaaatcaac ggcggagctg ctggtaaccc     420
ttttgccgct atgaagatga tgttgatgat gcaacaacaa caacagcaac agcagatgat     480
gttagcggga agacccacgg ttgcctccgc cgccgccgga ggcggaatca ccaccaatca     540
tcagggtgga gagggttcca tgcaagcttt gtataatgtt tttggcgatg gatctttgca     600
tggaactatg caggcgaaaa gctttgtggg ggctaattca gcaacagaga tgacccaaag     660
tcagggaagt ggtccgacgg ccggagaagc ggtgacggcg ccggaaaagc ctaaacaaag     720
agttagggca aggaggggtc aagctactga cccccacagt atcgcggaaa gactacgtag     780
agttagaatt gcagagagaa tgaaagctct tcaagaactg gttcccaacg ccaacaagac     840
agataaagct tcaatgcttg atgagatcat cgactatgtc aaattcctcc agctccaagt     900
gaaggttctg agtatgagca gattgggtgg tgctgctgct gttgctcccc ttgtttctga     960
gggaggtggc gattgtattc aaacaagtgc caatggtggg tcccatccac gaaattccaa    1020
cggcgaccaa acgccgtcgg ccaacgacag actgacgatg acggagcacc aagtggccaa    1080
gcttatggaa gaggacatgg gctccgccat gcagtatctg caagagaagg gtctgtgcct    1140
catgccgatc tctctcgcca ccgccatctc aagcgccacg tctcgttcaa ggaaccccat    1200
gatcaaccat gaaaacccgg ccaacggcag ccaccttttg attcaatcaa gcggcggcga    1260

tggaccttcc tcgcctagca tgtccgtgtt gacagtccag tcagccacca tgggtaacgg    1320
tggccttgaa ggtggcgcag cctcggtttc caagccctga taacggcggt gactgacata    1380
cgtagagagg aattgaaggg aaacagcttt tagtctatga gaataaggcg gtcttcttcc    1440
caagtatttt cttaaaagta cgaaacgacg ccgtatttga cgtagactta taagctaacc    1500
cctaagactg acctcatcca atcaac                                         1526
```

<210> 348
<211> 1052
<212> DNA
<213> Glycine max

<400> 348

```
ggattcatga agcccgacga agcctccgct agcggaaagc gctttcgcga cgacgtcgtt      60
gataatagag ctaagcatgt ttttcatggg caacccatgc ctactactat gcctgctgct     120
cctcatcctc cagcaatacg tcctagggtg cgggccataa gaggacaggc tacagatcca     180
cacagcatag ctgaacgatt gcgcagagaa agaatagcag aaagaatcag ggcattgcaa     240
gaactggttc caagtgtcaa caagacagat agagctgcca tgttagatga aattgtggat     300
tatgtcaagt tcttaaggct tcaagtgaag gttttgagca tgagtagact gggtggagcc     360
ggtgcagtgg caccactggt aactgacatc ccattatcat cagtcgagga agaaggtggt     420
gaaggtgcgc gaaaccggcc agcatgggac aagtggtcaa atgatggcac agaaagacag     480
gtagctaagc ttatggaaga aaacgttggg gctgccatgc agtttcttca atcaaaggct     540
ctctgcatca tgccaatctc actggcatcg gcaatatacc agtcacaacc accggacact     600
tctagcatag tcaagcctga aactaatcct ccttcataga cctaattcac atgttgcaca     660
atctattatt attggtccca tcccaggctc acctaatact tgctgggacc cacacctaac     720
gcgttatttg tctgttccaa atcccccccc ataaaataag tcattggcaa gcatcaagtt     780
gcaatcaaag tcagatgcca taattcttcc ttttttttgcc tttctttcag cttttgtcaa     840
tacagggttc gatatcaatg ccttggaaaa gacaagcaaa cccctatatc atgtcttgcc     900
gtatttcaag tagagaggaa gacattgtca aaagttcaat tgatatgcgc ttcttttcta     960
gtgtcaaatg ggtgggggta gtggaccatg atgtaggcgc ttttatagtg ggcttagtt     1020
tttgtagtgg gctgtgggac cttctaaaag gt                                  1052
```

<210> 349
<211> 1490
<212> DNA
<213> Glycine max

<400> 349

```
gtccaacaac aacaacgacg ctactactaa tgtcgcatct ttttcctcct tcgatgagca     60
ctccaccttca gcctccaagt tccgcaacca ccagatcagc tccaataaca acgcgcccaa    120
aaacgccgcg gcggcggctc tcatgctcca acaccaactc ctcagagact ctggtcttct    180
caacatgcct ctttccttgc ccggaaacga cgtcgtggtt gacgcttcta ccttcgaatc    240
ccccaatccg agtggtaaag cttcagtcca aactctctac aacgggttcg ccggatctct    300
gcatggcgta ggccaatcct cgaaccagac tcaacatttt caacaccctc agggaagttc    360
gaatccgatg caagggcaaa attttggggc ggtgccagct ggtggtggta gcgcgacgaa    420
tcaggctcca gcgagtggcg ctgccgcggg tggcgcgccg gctcagccga ggcagagggt    480
tcgagcgagg agaggtcaag ccacggaccc acacagcatc gctgaaaggt tacgtaggga    540
gagaatcgcc gagagaatga aggccctaca agaactggtt cccaatgcca acaagacaga    600
caaggcatcc atgctagatg agatcatcga ttacgtgaag ttcctgcagc tccaagtcaa    660
ggttctgagc atgagcagat tgggcggtgc tgctgctgtc gctccccttg ttgctgatat    720
gtcctctgag ggaggcggag actgcattca agccaacggc aagagtaacg gcggtggggc    780
ccaggcttca accaccaaca ccaacaccaa ccaaacgaca gcgacaacaa cctcaaacga    840
tagcctaacg atgacggagc accaggtcgc gaaactaatg gaagaagaca tgggttcggc    900
tatgcagtat cttcaaggaa aaggtctttg cctcatgcca atttcacttg ccactgcaat    960
ctccactgcc acgtgtccca ccaggaacgt taacgttaac cccttgatca acgccgccgc   1020
cggagccacc cattttccga ccgcagccaa ccccgccggc gaagggccgt cgtctccgag   1080
catgtccgta ctgacggtgc agtccgccat cgcaggcaac gacggcgccg cctccgtttc   1140
gaagccgtga tgacggtgtt tgagtgactt tataggaaaa agaagaaaaa aacaaagtga   1200
acaaaggaca gaaaatgacg tgtgtttttt tttcttaaaa aaaaaggagc cgttaaagtc   1260
tacgaaaata aggcgtcagg gatacggagg actcgacgac gccgtattta cctagacttt   1320
gtttggctga tgctaacttt caaccaacca attacaaact ccaccatgcc aattacctac   1380
ttcttcgtct tctttttctt tattattatt gttaattgtt aactgtcgct ttgggtttct   1440

tctgttcatt gtttttaaaa atctggagtg gaatttctct ttttggtgtt                1490
```

<210> 350
<211> 1850
<212> DNA
<213> Glycine max

<400> 350

```
ttttcttctt ccttttcgtg ctctctctca aattcaacac gagagacaca aaacaaaaca     60
ttctcgggaa gcgtaaccgc ccagaattcc gaccattttc acagaccttg ttttctgctc    120
ttgatgtttt tcacccccca aatgtggcaa taaaaaacgg gtggcttaat ttgacacaat    180
cggtggttat tgcaaagttg agatggcagg taatagtggt tcagaggggt tgggcgatga    240
tttcttcgaa cagatcttag cagtgcctga agccggcaca gtgggaatgt tgcaattggg    300
gtccacaact ggtgctttca gaggagcttc tgggttaatg cctctgggct tgaatttgga    360
acaagctgcc ttcctaagac accaagttaa cgttgacgac gacgttgttc atgttaatgt    420
tgatgatgcc accatccacc aacatcacct cactctccat aacaacaaca actcctcatc    480
accctcttcc actgcaccaa tcaccgatag ggattctatg catatgaggg gtttattttc    540
cgcgtttgga caactgcata ctcctatccg ccccacgctg ccattgcctc caccacctcg    600
tcagccacaa ctccacctcc accatcataa ccaatttcag ggccaggcag ctgcagctcc    660
agcgtcaatg gctgctatgc cacaaccacc tgggatccgc cctcgtgtga gagcaagaag    720
agggcaagct acagatcctc acagtattgc tgagcggttg cgtcgtgaaa gaatcgctga    780
aagaatgaag gcattgcagg aattagttcc cagcatcaat aagacggaca gagcggcgat    840
gcttgatgaa attgtggact atgtgaagtt ccttaggctt caggttaagg tcctgagcat    900
gagtagactg ggtggagctg gtgctgttgc tcagcttgtg gctgatgttc ccctttctgc    960
agtggagggg gatcaggaca tagaaggtgg agccaatgag caagcctggg acaagtggtc   1020
caatgatggc acagaacaac aggtggctaa gcttatggaa gaagatgtgg gagcagctat   1080
gcaatttctt cagtccaagg cactttgtat catgcccata tctctagcct cagccatttt   1140
tcgtatgcct caatcagaag catcaacagg catcaagcct gaatctaaca gtcactgata   1200
ttagcgatag aaccgaaaca aaaagtatca tgacagacac tagataatta aatttcttca   1260
tccttaattt atgacaaggg taatattcta ctttcttgta taatttaatt ttcctgattg   1320
gatggagaag gatctagtcg gtgcagcagc tggttcactc ttctcaagtt ttgtcaaaga   1380
aggagtaatt gttattccta ttaagtaaaa tcggaggagc caagcaaatg tcctttttcat   1440
ggtgaagatt aatattttca atggccttat tcaagttttt cttgcaaata tctgtggtgg   1500
ggttggtggt ggatgaccaa aatatttgta gtggaacctc ttttcttggt ttagtatttt   1560
tagtcagtgg ggagtgggac catttaaaag gaaaggatgg ttacgaatga tgtatcggga   1620
gaaaacttta atacaagttg aattataata tggttagtta ccttgtaaag atgttttgga   1680
agcatctgac cattggaaaa gctaaagata ttttaaaata atcacacatt ttgagtcttg   1740
tttgcttcta aaacaagtga tttccagcac aaaattaggt gagagtactg aaccgactgc   1800
ttgctgagaa agtggaaaat gacagtttgt taatttactg aacaaaaaaa              1850
```

<210> 351
<211> 1392
<212> DNA
<213> Glycine max

<400> 351

```
cgcgtccgct ccgccgacgc ctctccgatg atgcttcagc tcaactccgg cgacgccgcc     60
acccacctag cctccttcca cgcgccgccc taccagctcg gcctcagcct ggaccaaggt    120
gagggaccct tcctcacgcc tgaggacgct tctggaagcg gcaagcgctt ccgcgacgac    180
gccgttgata ccagacctaa caacgttttt gatgggcaac ccatgcctac aactgttcct    240
actgctccac atccaccagc agtgcgacct agagtgcggg ctagaagagg acaggctaca    300
gatccacata gtatagctga aaggttgcgc agagagagaa tagcagaaag aatcagggct    360
ttgcaagaac tggtccctag tgtcaacaag acagatagag ctgccatgct ggacgaaatt    420
gtggattatg tcaagttctt gaggcttcaa gtgaaggttt tgagcatgag tagattgggt    480
ggagctggtg cagtggcacc actggtaact gatatcccat tatcgtcagt cgaggaagaa    540
ggcggtgagg gaagaaacca gccagcctgg gagaagtggt caaatgatgg cacagaaaga    600
caggtagcca agcttatgga agaaaatgtg ggtgctgcca tgcagtttct tcaatcaaaa    660
gcactctgca tcatgcccgt ctcactagct tcagcaatat accagtcaca accctcaggc    720
acttctagta tagttaagcc tgaaactaat cctccttcat aatcatagtc agaactctgg    780
cacagatgca ccatccagtg gtcccatcaa gggctcacct aacactgcta ggacccacgt    840
gtaatttgcc tgtttcaaac cccttaacc ttagtcattg gttcgcatca agttgcattc    900
```

```
aaggttggat gccatagtct ttccttttg tcgttctttc aacttttttg tctagacagg     960
gtttgatgtc aatgcccttc aaaacacaag caaaccccat cttgataaat ttcatgtaaa    1020
gggaaagaaa ttgtcgaagt tcacttgata tgctcttcct ttttgtatca attagtgggg    1080
gttagtggaa cgtgatggaa gcgcttttat agtggggctt ggtttttgt agtgggcagt    1140
gggaccttct aaaaggttag ggtggtgtgt atattttatt agtgatgtaa ctcttaggga    1200
atttgaattg cagagctgaa atggataatg attgctttaa caatgctggg tgctgctagt    1260
ccaccagaaa atgatattgc tttttttttt tttatttcag gtgtgctcta gttagttatt    1320
taaaagtact ttcgacccct tttttctcat gcagggagag atggaaggag agtatggtgc    1380
tattttaaac ca                                                        1392
```

<210> 352
<211> 1497
<212> DNA
<213> Gossypium raimondii

<400> 352

```
ttcctttgtt tttctttctc tcacttcccg tttctctttc agaaaaagga aagttcaagc      60
taagctaaaa ctctgaactt tccccttctc agtctcctca gggacagaga aagattaaac     120
gaaaggaaaa gaaaaagact tcttcttttt ttccctaaaa gaaaagttga tctttttttg     180
tttggttcga ttctctttag ccatttcctt tttgcatgta gatgctcttt ggtagcctta     240
actcgccact tcgactcact tcaatccatg gctaataacc ccaacgagtc ccccgctgac     300
gatttcctcg agcaaatcct cggactctcc cactttgcgc ctactgagac tggcttagcg     360
gggcccgacg gtagattgtc tggaaacgcc acgacggctg gagcgccaat gcttctgcag     420
ctcagctccg gcggtggcac cggacacatc ggcgctatag gcggcggtgg aggcggtgcg     480
tttcacggac aggtttttcc attgggattg agcttggagc aaggaaaagg tgggtttttg     540
aagcccgagg aagcatccgg tagcagcaag cgcttccgca acgaggtcgt tgatggcaga     600
gctttttctg ttaaaaacgt tttccatgga caaccagtgc cagctactgt tgctgctgga     660
ccacatccac cagcaatgcg tccgagggtg agggctagac gaggacaggc cacagatcca     720
catagcattg ctgagcggtt gcgcagggag agaattgccg aaagaatccg ggcattgcag     780
gaacttgttc ctagtgttaa caagactgat agagcagcaa tgcttgatga aattgtagat     840
tatgtgaagt tcctgaggct ccaagttaag gtcttgagca tgagtaggtt gggcgcagct     900
ggtgcagtgg caccactcgt aacggacctc ccactatcgt cagttgagga tgaaagtggc     960
gagggaggaa gaagccaacc agcctgggag aaatggtcaa atgatggcac agagagacaa    1020
gtagctaagc tgatggaaga agatgtcgga gctgccatgc aattccttca atcaaaggct    1080
ctttgcgtca tgccaatttc actggccact gcaatttacc acacgcaatc tccggatacc    1140
tcctctgttg ttaagccgga aacaaatcct ccttcataga cacctcacat gagaaaaaga    1200
ggaaaagaaa acggtgtatc atatggtggg ggagactgtg gacaaagacg gaagcacttt    1260
tcttgtgtag tgatggggca tggatttttg ggtttggtgg gatgtgggac ccgcaaaagg    1320
agatggatga gatgagtata tttacatatt ataacaataa aatattacaa tattgatagt    1380
gttagtgtaa gtaacagtgt aatttcacag ggaagggcta aagtagaaga agggataatg    1440
gttggctgat tttataaaat gctgattgaa taaaatagat ggattggttc acgtttc       1497
```

<210> 353
<211> 1301
<212> DNA
<213> Helianthus petiolaris

<400> 353

```
cggggatctc atcttttcac tcttcttccc tctctctctc tctcttcttt ctctctctag     60
gttttaggat tcgccggcga tacgcttctc cggcaggcat gtcgactgac ccgccggagg    120
gctacggtga cgactttctc gaacaaattc tcgcgattcc gtcgtacaac atcaccggat    180
gcttacctgt taataccgct gattctactg gctctgaaac agtttccgtt catcatcaac    240
aacaacaacc gcaacaacaa ctgcaaccgc aaaagtttcc gttaggtttg agtttagata    300
acggccgtga aacaatcgga ggagcgtttg caggacagca acagcaactg cagcagcagc    360
agcagcgtga gagaggaggg agcatgaata tgagtggttt gtttcctcag tttgagaatt    420
tgcagtcaca ctcgctgtta catactgttc cacagggttt tcaagggcag ccaactagca    480
gtacagcagt gactgtgcct catccgccta cgattcgccc tagggtacga gctcgacgag    540
gacaagctac agatcctcat agcatagctg agcgtctacg ccgagaaaga attgcagaaa    600
gaatgagggc tttgcaggag cttgttccca gctgcaacaa gactgataag gctgctatgc    660
ttgacgagat tctggattat gtgaagttct tacgactcca ggtcaaggtt cttagcatga    720
gtaggctggg tggagctggt gcagtggcac agctcgtatc tgatgtcccg ttgcaatctg    780

ttgagggggga cgggagtgaa aatggatata ataatcaaca gcagggccag gcagcgtggg    840
agaattggtc aaacgacgac acagaacgtg aagtagcaaa gctgatggaa gaggatgttg    900
gggcggccat gcaattcctg cagtcaaaag cattatgcat catgcccatt tcacttgctt    960
cactaatcta ccctaatcac caacctgatg ttaaacccga gccatctgct ccttcttaga   1020
gagcatctta catgcttctc tcttataaca actactacta ctactacctt tttctgtcat   1080
ttggttttgt gccccaaccc caacccgatg tcttctctaa ttgatggaca ttttgtatga   1140
ggaaagtagg aaggaaacag gggatgtgta tggccatctc attgtgtcct ggggatttta   1200
tttattatct agattaaaat gtattctaag aaaatctctt cacatggaca accctaccca   1260
acttagctgt gtttattatt gttaatatct aaatgatttg g                       1301
```

<210> 354
<211> 713
<212> DNA
<213> Hordeum vulgare

<400> 354

```
cgggcaggcg accgatcccc acagcatcgc agagaggcta agaagagaga ggatagcgga     60
aaggatgagg gccctacagg aattggtccc caatacgaac aagactgata gggcagttat    120
gctagatgag atcctggatt atgtgaaatt ccttaggctt caagtaaagg ttttaagcat    180
gagcagattg ggtggtgctg gtgctgttgc acagcttgtt tctgacattc cactttcagt    240
taagggggaa gcaagcgata gtgggggcaa acagcagata tgggaaaagt ggtcaacgga    300
tggcacggag aaacaggttg cgaagctgat ggacgaggac atcggtgctg caatgcaatt    360
tctccaatcc aaggcgctct gcatgatgcc agtctctctt gccatggcta tctatgacac    420
acaacattca caggacggcc aaccagtgaa gcctgaaccc aacaacactg cctagtatag    480
taacagcagc tgcaagccgc atccctgcag ctgctagtag gcatgcatga acctaccatc    540
aaagtgttaa gatgaaaact gctggttttg atccactaac aacaaggagg caggaaaaga    600
cctaaaagat cctctccact ggaagttgta tcgacggtat atgatgtcac ctactacccc    660
ttttccatct tttgttcaaa ggtgctttaa ttttcaagga tatggtaagc aat           713
```

<210> 355
<211> 934
<212> DNA
<213> Lactuca perennis

<400> 355

```
tttcaccctc ctcatacgcc cactacgctt cttccttcta aaccagacta cgattctccg      60
gcatggcgac agacccgccg gagggctacg gagacgactt ccttgaacaa attctcgcga     120
ttccttctta caacatcgcc ggatgcttgc ctggtaatac caccgatgct aatgcttctg     180
aaacggtgtc tgttcatcgt caacagcaac agcagcaacc ggtttttccg ttaggtttga     240
gtcttgataa tggccgtgaa acaatcggag cgtttgcagg gcagcagcag cagagggaga     300
gaggagggag tatgaacatg actggattgt ttccttcatt tgaaaatttg cagtcacatt     360
cgctgcttca ttctgttcct caggcttttc aagggcaatc aactaccagt acagctgtca     420
ctgttcccca tccaccttct attcgcccta gggtccgggc ccgcagagga caagccacag     480
atcctcatag catagctgag cgtctacgcc gagaaagaat tgcagaaaga atgcgggctt     540
tgcaggaact tgttcctagc tgcaacaaga cggataaggc tgcaatgctt gatgaaattc     600
ttgattatgt taagttctta cggcttcagg tcaaggttct tagcatgagt aggctgggtg     660
gagctggtgc agtggcacaa ctcgtatctg acgtcccctt acaatccgtg gaggggggaca     720
cgagtgaaaa cggatataat caacccgcat gggaaaactg gtcaaacgat gacacagaac     780
gtgaagtagc aaagctcatg gaagaaagat gtgggggccg ctatgcaatt tctccaatca     840
aaagctctat gcatcatgcc catatcactt gcttcactaa tttacccgcc ccaaacaccc     900
gactccaatt cccatgttaa gcccgaaact gtcg                                 934
```

<210> 356
<211> 948
<212> DNA
<213> Nicotinana benthamiana

<400> 356

```
tagccggaga taatctccgg catggcgaca aaccaaccgg agggctatgc cgacgatttc      60
ctcgagcaaa ttctcgcgat tccttcctac gctggcttgc cgccggtggc tgacgtggga     120
acttcatcag aaacgacgtc gttcacttcg gcatctgctg ctggtctaca gcaaccgttg     180

ttcccgctgg gactaagctt ggaaaacggc cgtgatgacg tcagcgatgc tggtgcttat     240
gcagtgaagc atgaaagaga aggaataaat atcgggaatt tatatgcggg tctagaacat     300
ttgcaatctc atgcggttcg tcacgctgtg cctcctgttc accatatcca gtctttccaa     360
ggccaaccaa caacaagcac aacgatgact gtaccgcact caccagcaat tcgtcctagg     420
gttcgagctc ggaggggaca agccacagat ccacatagca tagctgagag gttgagaaga     480
gagaggatat cagaaagaat aaaggctttg caagaacttg tccccagctg caataagaca     540
gatagggctg ctatgcttga tgagattctg gactatgtga agttcctaag gcttcaagtt     600
aaggtattga gcatgagtag gctgggagga gccagtgcag tggcacaact tgttgctgac     660
attccgttgc aatctatgga gggggacagt gctgaaagta aatccaacca gcatatctgg     720
gaaaagtggt ccaatgttga cacagaacaa gaggttgcta agctcatgga ggaagatgtt     780
ggtgcagcca tgcaatacct tcagtccaaa tcactctgca tcatgcccat atcacttgct     840
gcacttatct atcctactca gcaaccggat gacatgtcca tggtcaagcc ggaaccggca     900
gccccgtcat agacctccaa tttattgcac gtgcctctga gaactcct                   948
```

<210> 357
<211> 1792
<212> DNA
<213> Nicotinana benthamiana

<400> 357

```
cttctttatt tctctcttct ccacaaaatc atctatttct aaattgagtc atatacttgc      60
acagaagtta ctaaagatat aatgcaagcc atgaaccagt acgatccgac gtcgtctcac     120
gacgatttcc tcgaccaaat tctctcttcc gttccttctt cttcactttg ttggcctgac     180
ctttccaaat cctgggaccc acaccaccac cacctctctc cgccgctgcc tcctaaccct     240
agctccggcg atgaccacca gcttcctcct tctaatcctc ttcagcattt ccaatatgac     300
gaccagtctt cttctttcct tgctgccaag ctccgccagc accagatcac tggtggtggt     360
ggtggcggcg gcgatgctgt agcagctgct aaagcacttt tgctccagca gcaacttttg     420
ctttctagaa cactagccgg aaacggcctt aggtctccga ctggagcctc cggcgataac     480
ggcttcctga acatgctcgg taatggtgac caaaacgaca gcgtcggaaa tccggctaat     540
gacagttccg ttcaagctct tttcaacgga ttcactggat ctcttggtca aaactcaagt     600
caacctcaac attttctcca tcctcaggga ggaaggatgc aagcgcagag tttcggagct     660
ccggcaacgg cgccggcgat gaatcaaact ccggcagcaa gtggttcagc tggtggaggt     720
acaacgccgg cggcacagcc aaagcaacag cgagtgagag ctcgtagagg acaagcaact     780
gatcctcaca gtattgctga acgattacgt agagagagaa ttgcagagag aatgaaggct     840
ttgcaggagc tggtacccaa tgccaataag acggacaagg cttcaatgct ggatgagatc     900
atcgactatg tcaaattcct acagctccaa gtcaaagttc tgagtatgag cagattgggt     960
ggtgctgcag ctgttgctcc cctagttgct gatatgtcct ctgagggaag aggagaagga    1020
aatggaggaa ggggaggaaa cggaacggcg tcgtcttcaa acaatgacag tatgacggta    1080
acggagcacc aggtggctaa actaatggag gaggatatgg gttcagcaat gcaatatcta    1140
caagggaaag gcttatgcct aatgccaatt tccttagcta cagctatttc aactgccacg    1200
tgtcactcca ggaatcccat gatccctaac ggcaacggtg gcaacaaccc actactaggc    1260
ggaggaggag gcctcgccac caacggcggt ggtggcgagg ctggtggacc atcctctcct    1320
aagttgtcgg ttttgactgt ccagtcagcc acgattggta acggtggaat tgatccctcc    1380
gttaaagacg ctacttctgt ttttgaagct taagaagttt gtttggaagt tttttaacgg    1440
cgttaactgt ttcactgacc ccctttgcca cggagaaaaa agaaaatgtg aaaaatagtg    1500
cgtggccgtc aaagttaagt ctaccaaaat aagggtcttt tagtctttat ttttatcttt    1560
tttccagttt catatgaaaa aaaaaaaaga agaagagaga accaagaacg acgccgtatt    1620
taggtagact tggctgaagt aagcttaact taagctaaca gcaggaaatg ccaatggaaa    1680
tgaagtactt atgtttcttt taatcttttc tccataatga tgatcctttg atttccctgt    1740
tctacttaag tttttcctta atggagtatt gtttggaaag gatatatata ta            1792
```

<210> 358
<211> 1484
<212> DNA
<213> Nicotinana benthamiana

<400> 358

```
gatctctcat tcattaacgg ggaaaagaaa tgcaagctat gaactcacaa atgtcactac      60
aagaagaaaa tggagcttcg tcgggccaaa atatgagtca ctctcatttt gacccgacgt     120
cgtctcacga tgactttctt caacagattc tctcttccgt tccttcttcc tctccctggc     180
cggacctctc cagcggcggt gacggtcatt ttgacgacca gtcaattttc gtagcttcca     240
```

agctccggca gaatcagatc accggcggtg gtggcggtgg cgcagctgct aaagcgttga 300
tgcttcaaca gcagcttttg ctttctagag gattactcgc cggtaatggt gaccaaaatg 360
atgacgttgg aaatccggaa aatgagattt cagttcaagc tctttacaat ggattcgctg 420
gatctcttgg tcaaacctct aatcaacctc agcattttca ccatgctcag ggaggatcca 480
tgcaagcgca gagtttcgga gcaccggcgt cgtcgacaat gaatcaaacg ccggcggcga 540
gtggtggttc agctggtgga gggcagccaa agcaacagaa agttagggct cggagaggac 600
aagcaactga ccctcacagc attgctgaaa gattacggag agaaagaatt gctgagagaa 660
tgaagtcgtt gcaggagttg gtacccaatg ctaataagac agacaaggct tcaatgttag 720
atgagatcat cgactatgtc agattcctcc agcttcaagt caaagttctg agtatgagta 780
gattgggtgg tgctgcagct gttgctcccc tagttgctga tcggtcctct gagggggag 840
gaggtgattg tgcacaagca aatggaggag ggaggggcag taatggaacg acgtcgttag 900
ctaacaatga cagcatgacg atgacggaac accaggtggc aaagctaatg gaggaggata 960
tgggatcagc catgcaatac ttacaaggaa aaggcttatg tcttatgcca atttctttag 1020
ccacagctat ttccaccgcc acgtgtcact ccatgaaact caacaaccca ctactacacg 1080
gcggcggaag cggaggctct gccgccatca acggcggtgg tggtggacca tcctctccta 1140
gcttgtcggc ttccactgtc cagtcagcca cgatgggtaa cgggggcgct tgagtttgtt 1200
gaaatatact tcgtaattta cgacgttgac tgtttcaatt atcttttctt agaaaaatac 1260
cggaaaaatg tgaaaaaagt ggtgaaaatt tcgttatttt agagctgaaa tttcaaggcc 1320
ttttcacatg ggacaaaaaa gaaaaagaaa agaaaaaagg ggtaattgaa atgtttagct 1380
ctaaatatta ttggtaattt ctgatttag ttcgtgtgat attttattaa ttgtatttaa 1440
cattgaatta atgataatat gggtgttttt gtttatttac atat 1484

<210> 359
<211> 1500
<212> DNA
<213> Nicotinana benthamiana

<400> 359

gaaatattta aacaattgct tttatttggt aaccctattg gacaatatat ttattaatca 60
aagcttttgg tgacaccatt tgctttttat tttcttcatc gctctcactg ttctatagaa 120
agtagcagca accaaataat aaacacatag aaacactcaa aacacacact tccatttctt 180
ttcctatata tatacacaag tacactcgtg agtagtactg agtactagtt tgtacatcta 240
atattacaca tgttaaaaaa acacttacta gtaattgctt ttctgagaaa ttaaggtggt 300
cgagaaaaaa aatctgttac agctatggct aacaacccat cagaaggtcc tgatgatttc 360
tttgaccaaa tcttgggatt cccagcttat aacggagcag aaaccaattt ggcgggaagt 420
gatgccggag caattccgcc ggcgatgctg ctgcagctaa actccggcga tgcgtcgggt 480
cagttttccg gaatgggtct tggtgttgga ctaggtggtg gtggtgggtt ccatcatcca 540
tctcagtccg gttcttttcc attggggttg agtttagagg gagggaaaag tggattcatg 600
aagatggatg atgttccggc cgtacccgga aggagattta gagatgatgt tgttgatagc 660
agagcttctt cttctgttaa acctggtttt catggccaac cgatgccttc aatgccacat 720
cccccagcaa tacgtccaag ggtaagagca aggcgaggac aagctactga tccacacagc 780
attgcagaga ggttacgtag agagaggata gcagaaagaa ttagagcatt gcaagagttg 840
gttcccaatg tcaataagac cgatagagcc ataatgcttg atgaaattgt agattatgtc 900
aagttcctac gcctgcaagt gaaggtgttg agtatgagta ggttgggagg agctgctgca 960
gtggcaccac ttgttacaga aattccaata tcatcagtag aggaagagat cagtgaaggt 1020
ggaaataatc aaccagcctg ggaaaagtgg tcaagtgatg gtacagaaag gcaagtggcc 1080
aaacttatgg aagaaaatgt tggttctgca atgcaatttc ttcagtctaa ggcactctgt 1140
attatgccta tttctcttgc atcagcaatt taccactctc aaccaccaga tacatcaagt 1200
cttattaagc cagaaactaa tcctccttct tagagatcct tattagaaag ctggctcgga 1260
catcacgttt aatttttttt aaaaagaaaa agatccttat taaaaaggtg acaacatgca 1320
aatgaccctg agaattttct ttgggagtgg atggtcccac agctattttt acttattaag 1380
tagtactatt tagctttgtt gaaagacacg gttcaatgtt atgtataaag taaccttttt 1440
ttttgtgatt gatatgatat ataattcaag cagaagaaga aaaattgtca aagttctgcc 1500

<210> 360
<211> 1451
<212> DNA
<213> Nicotiana tabacum

<400> 360

aaaacagagt cattccattc catttccgtt cctcaaaacc ctttattaac ttcttctcgc 60

cgggaaaaac ccttttcctc gccggagata atctccggca tggcgacaaa ccaaccgggg 120
ggctatgccg acgatttcct cgagcaaatt ctcgcgattc cttcctacgc tggcttgccg 180
ccagtagctg acgtcggaac ttcatcagaa acgacgtcgt tcacttcgtc atctgttgct 240
gctgctggtc tacagcaacc gttgttcccg ttgggactaa gcttggataa cggccgtgat 300
gacgtcagcg atgctggtgc ttatgctgtg aagcatgaaa gagaaggaat aaatatcggg 360
aatttatatg caggtctaga acatttgcaa tctcatgcag ttcgtcacgc tgtgcctcct 420
gttcaccata tccagccttt ccaaggccaa ccaacaacaa gcacaacagt aactgtaccg 480
cacccaccag caattcgtcc tagggttcga gctcggaggg gacaagccac agatccacat 540
agcatagctg agaggttgag aagagagagg atatcagaaa gaataaaggc tttgcaagaa 600
cttgtcccca gctgcaataa gaccgatagg gccgcaatgc ttgatgagat tctggactat 660
gtgaagttct taaggcttca agttaaggta ttgagcatga gtaggctggg aggagccagt 720
gcagtggcac aactggttgc tgacattccg ttgcaatctg tggaggggga cagtgccgaa 780
agtagatcca accagcatat ctgggaaaag tggtccaatg tcgacacaga acaagaggtc 840
gctaagctca tggaggaaga tgttggagca gccatgcaat accttcagtc caaatcactc 900
tgcatcatgc ccatatcact tgctgcactt atctatccta ctcagcaacc ggatgacccg 960
tcaatggtca agccggaacc ggcagccccg tcatagacct ccaatttgtt gcacgtgcct 1020
ctgagaactc ctaatagcct tgtgtcttac cctcctttgt atccttacct actatcactt 1080
gtattttcct ctgcaattgg tggcaatgca atcaatgtca gatgccacaa tttttgcttt 1140
tagcccaacc attggcccct tttattacct ttccggagtt ttcgtcaaac aaggcatgat 1200
gtcatacctt gggcaaaggt aaacgtctga aagcacccaa tccattatca tcattttgga 1260
tgaggtgagc agcttttca aagttatcta tgattagctg ctcttctgcc attttgtttg 1320
tgggatagtg gggactcctt tatcttttgc atagtggatt ggtggggcga tgtaaaagta 1380
atcaactggt tgttgtaaag tgtaatgtaa tttaagtata ttattgcaat ggatagttga 1440
tgtggctctc c 1451

<210> 361
<211> 2056
<212> DNA
<213> Oryza sativa

<400> 361
gacgcgagtc gagagacggg agagcagtgc agtgcagtgc agtgccgcct cgtcttcctt 60

```
ccctccctcc tcatactctc tctcctcccg gtcttctcct cccccgccca aagcggagcg     120
cggcggttat atatatatat atatatacag cacttcctcc tccttcctcg ccgcacgtcg     180
ccctccgact tcgcctcgtc agccgtcgtc accgccccca cccgtggatt gcaccggaga     240
tcggaatggg cggcttcgcc taccccttca cgccgtcgcc ggcgtggtcg cgggacgccg     300
tgttcgctgg ctcgccgtgg gccgcaggcg gcgtctcctc cctcgccgac gctttggtga     360
gctacggtgc cgtcgacgac gaggaggccg cgttcctcgg caagaccgcc gcgtcgtctc     420
ccagtacggc gaggctgcac gagcagcagc agctgctgct ggaggctgag ctgctgcgcc     480
acggcgacgg cctcggcttc gccgcgatgg acgacgacgg cggcgccgcg atgctcggcg     540
cgctggagcc gtgcgccatg ccgctcaccg acagcggcgg ccccccggtc atctgcagca     600
gcagctccaa cgacagcagc gggagcgagc actccgccgc catgcctgca ggaggcggct     660
tcctcgtcgg agagcagcag cagcacgtgc cgccggcggc ctacgccgcg ggaggtgtac     720
tcccgtccat ggccgcggga gaagaaacgc cgcagagctt cggctttggc agcctcttca     780
atggcgatct cctgcaggag gcgaccgtga gcaagtatca tcatcatcag cagcagcagc     840
agctgggcgt tgtgccttcg tcgcagccgc atcatctgaa tgatgacatc gatttcaaca     900
ctggaaaatt gatgtctttt gcttctggac agcagcacgt tacccccagc attgacagcc     960
tgcagatcga ccagaaggaa ttcagcagtg gcctgcacca cctcaatctc tcctcgctga    1020
tttctggacc actcgcatca ttcaatgcaa cacagagcca tagacagcct gctgaagctt    1080
gcggtggcaa aaatggcggc gccgctccat ttgtgaacct atctgaggtg ctaccaaagg    1140
gcaatgggag tggtagtgct gggaatggag cacccaagcc tcgtgtcagg gctcgccgag    1200
gccaggccac cgatccccac agcatcgcag agaggctccg gagggagaag atctctgaca    1260
ggatgaagga tttacaggag cttgtcccaa actcaaacaa gactaacaag gcatccatgc    1320
tcgatgagat catcgactat gtgaaatttc tccaattaca agtcaaggtc cttagcatga    1380
gcaggcttgg agcggccgaa gccgttgtcc ctcttctgac agaaacacaa accgagagcc    1440
ccgggtttct cctgtcccca agatcatcat caggagaaag gcaggcagga gcaggagcag    1500
taacaggagg gctccccggc gatcagccgg agctcctgga cggcggcgcc atgttcgagc    1560
aggaggtggt gaagctgatg gaggacaaca tgacgacggc gatgcagtac ctgcagagca    1620
aggggctctg cctcatgccg gtggcgctgg cgtcggcgat atcggcacag aaggggacgt    1680
cctcggccgc cgtccgaccg gagaagaaga agaacggtga tggcgatggc ggcggcgacg    1740

aggaggacgt gaagggagag tttgacgcac cgaggaggcc tcctgtcggc cggccgaagg    1800
agatgaggtc cagagtctaa gctagaacct ctgctttgtt caggagagat ggagcttaat    1860
ttagatccta acactttgct acttatatgt ttattattat gaaattttaa ttgcaagttg    1920
caactggtga tctcgaacta cattttgccg gtgattagaa aattgttata tggaggttag    1980
ttattaaatc atatataatt atatttgta ttgttgcggt ccttgcggac aatgccattc     2040
agtaccttga tatttg                                                    2056
```

<210> 362
<211> 1326
<212> DNA
<213> Oryza sativa

<400> 362

```
atgcaaccca acgcccgcca aatgcgcggc ggcggcggcg gcggcggcgg cgggcaggac    60
gacttcttcg accagatgct gtccacgctg ccggcggtgt ggtccgagct cggctccggc   120
aagcccgcct gggacctcac ggccggcgcc gtaggaggag gaggaggagc ctccgatgac   180
cactccgccg cggcgttcga cgactccgcg ctcctcgcct cccgcctccg ccagcaccag   240
atcgacggtg gaggcgacaa gcccatcatg ctccaactca gcgacctcca ccgtcatcac   300
ggcctcgccg ccggcgatga cagcggcggc gctgccgggt tccttcccct gtcgctgttc   360
gctgaccggt cgcaggacga catcgacgcc gccttcaagt cccccaatgg cgctagaggt   420
gaccatgcgc tgtacaatgg gttcgggggcc gccggaatgc atggcgccgc cgccatgcag   480
ccgccgccgt tcgggcaggg aggatcaatg ccggcgcaga gcttcggtgg cggagcggcc   540
gcgagcggcg gcggcggcgg cgggtcggcg tcggcggcag cggcggctgg ggcatcgtcg   600
ggcggagggg cggcggcgcc gccgcggcag cggcagcggg cgaggagagg gcaagccact   660
gacccacaca gcatcgccga acgtctccgg agggagagga tagctgagag gatgaaggcg   720
ttgcaggagc tggtcccaaa tgccaacaag accgacaagg catcgatgct cgacgagatc   780
attgattatg tgaagttcct ccagctccaa gtcaaggttc tcagcatgag cagattgggg   840
ggagcttctg cagtcgcgcc tctagtggct aacatgtcat cagagagtaa tgggaatggc   900
aatgccacca gcagcagcgg gaacggcgag gcggccaatg gcagcagcaa cggcgacaac   960
aatggtggcg gcacactgcg ggtgacggag cagcaggtgg cgaagctgat ggaggaggac  1020
atgggctccg ccatgcagta cctgcagggg aaggggctat gcctgatgcc gatctctctc  1080
gcaacggcca tctcctccgc cacctcctcg tcgctcctcc cccgcactgg gggaggcgct  1140
ggagggtccc tacatgaagg tggtaatggt acatcaccac cattggtgaa tggcaccgcc  1200
accggatgtg acgacgccgg agtattcttt tctgttaaat ttgtcgtgga gctgtcgttt  1260
ctcctgctga atgaagactg tagaggtaag gaggaatcga aattattggt ccagaaggga  1320
ccctga                                                             1326
```

<210> 363
<211> 1831
<212> DNA
<213> Oryza sativa

<400> 363

```
acctcgcttc acactagccg aggagacgcg agcgcgagct gctctcctct cctcccgccc    60
cgcgtcgccg acgtcgaggc ggcggtcgac gtcgacgtcg ccggggatgg ccgggcagca   120
gccgcagcag cagggcccac cggaggacga ctttttcgac cagttcttct ccctgaccag   180
ctccttccct ggcgccgcgc cgggcggccg cgccgccggt gaccagccct tctccctcgc   240
gctcagcctc gacgccgccg cggcggccga ggcgtccggg agcgggaaga ggctcggggt   300
cggcgatgac gccgaggtg gcggcagcaa ggcggatcgg gagaccgtgc agctcaccgg   360
actcttcccg ccggtgttcg gcggcggcgg cgtgcagccg ccgaacctcc gccccacccc   420
gcctacccag gtgttccacc cgcagcagtc gaagcagggc ggagcagccg tcgggccgca   480
gccgccggcg ccgaggccga aggtgcgagc gcggcgtggg caggcgaccg acccccacag   540
catcgcggag aggctaagaa gagagagaat agcagaaagg atgagggccc tacaggaatt   600
ggtccccaat acaaacaaga cagatagggc agctatgcta gatgagatcc ttgattatgt   660
gaaattcctg aggctgcaag taaaggtttt aagcatgagc aggctgggtg gcgcgggtgc   720
tgttgcacag ctggttgctg atattccact ttcagttaag gggggaagcaa gcgatagtgg   780
gggcaaccaa cagatttggg aaaagtggtc aacggatggc acagaaagac aggtagcgaa   840
gctgatggaa gaagacatcg ggcagcgat gcaatttctc caatccaaag cactctgcat   900
gatgccaatc tcgctcgcca tggcaatcta tgacacacaa caaacacagg atggacaacc   960
agtgaagcac gaacccaaca ctccttccta gtatagtaat agcaactgta agtctgtaac  1020
```

```
atgaatcgtt acaaccccta gtagacatca acctatcaaa gtgtaagata aaacagctcg   1080
ttttgattca ctaacaagga ggcaggaaaa gacctaagag atcctctcca cttgaagttg   1140
taccaatggt atatgatgtc acctttcgtt atcatctttt gttcaaaggt gctttgattg   1200
caaggatatg gtaagcaata gcctgctcat cctcctacac ctttcaatgc ccctatgagc   1260
gcgggtgatt gagagagcta ctaaagagtc tggcatgctc tcacattgtc tcttttcgag   1320
tctgtagtgc accaaccata cactttgtgg tggggactgc ttttttggta gtgggtgttt   1380
ggactaatca aaagggattg atgtatgcct gtgtctgcag cctgcacaat ggttgacgga   1440
atttgaacac aagatgaaat ggaagatgat tttgatggtg atgttcattt atctccattt   1500
ttccaaagtg gaggcggagg ctttggtcaa tggaggctga gcgtgcgcta agctgatcgc   1560
tagtgggtct gctatctaca tctacatgca ctactgttgt atctattgtg acatactctt   1620
gataagaaac tgcggtataa agtttaatct ttctattggc gattaatcca tagaattact   1680
ttgtattttc tgccatgagt tctcaactac ccacattatt tgtgaagaac agctgttctt   1740
gtactgttca ctgccctcta ccacagggtt tcctgagttt gtggacaaat tgtgagaatc   1800
tacagcaagg actattgagt ttttttttgt c                                  1831
```

<210> 364
<211> 1104
<212> DNA
<213> Oryza sativa

<400> 364

```
atgcagccga gcagccgcga cacggtcgcc ggcggcggcg gcgaggggac gcaggatgac     60
ttcttcgacc agatgctctc cacgctgccg tcggcgtggg ccgacctggg gggcggcggc    120
ggcggcgcgg cggggaagtc gccgtgggag gtcgatccgg cagcggcggc ggcggcgtcg    180
caggtgttcg acgagtcggc gctgctcgcg tcccgcctcc ggcatcacca gatcggtggg    240
gctggcggcg gcgggggaga gaagccggtg atgctgcagc tgagcgagct gcaccggcag    300
gccggcggcg gcgaggagga cgggagcggc gcgttctcgc cgctgccgct gttcacggac    360
cggacgaacg tgccgccgcg ggaggagatg gagggcggct tcaagtcgcc caatgccgcc    420
gccggaggcg agcacgcgct gttcaacggg ttcggcgtgc acggcggcag cggcggcgcc    480
gggcagccgc cgttcggcca gcttcggagg gagaggatag cggagcggat gaaatcgctg    540
caggagctag tcccaaacgc caacaagacg gacaaggcgt cgatgctgga cgagatcatc    600
gactacgtca agttcctgca gctccaagtc aaggttctca gcatgagccg gctcggcggc    660
gccgccggca tggcgccgct ggtggcgagc atgtcgtcgg aggggaacag caacgggagc    720
agcaatggcg gcggtggcaa ggcgagcaag ggcggcaccg gcggcgaggg cggcggcggc    780
ggcggaggag gaggaggagg tggcaccggt ggtgggatgc gggtgacgga gcagcaggtg    840
gcgaagatga tggaggagga catgggcacg gcgatgcagt acctgcaggg gaaggggctc    900
tgtctgatgc cgatctccct cgcctccgcc atctcgtcgg cgacctcatc ggcgtcgctc    960
ctctcccgcc cgtccatccg ccacgccggc gcgccgccgc agacgatgct cgatgccgcc   1020
ggcccgacct cgccggcggc gatgtctaac ggcgatgacc cacggcacgc aaaggcggac   1080
ggcggcgccg gcgggacgca atga                                          1104
```

<210> 365
<211> 2221
<212> DNA
<213> Oryza sativa

<400> 365

```
agaaggggtg tactagtaaa caaaacaaaa agaataggtt gagactttcc tgctatcgtc      60
agcattttca gcccccctcc cctgggctgg ctctgcatca tcattcatca tcagcagcag     120
tcagtccatc caacgcgtct tcagtcctca cccgcagcaa gaaagccaca cttttcctcc     180
tcctcctcct cctcctcctc cgcctcttcc tcttcctccc ccttcgttct ccacacccac     240
tctcgggctg cggcttctcc ggcttccgcg cctcttcctc tgtcgggtta acaaagttac     300
tttcagcgtc agagggtatt tcgtacaaaa aggaactgct gttggtacag gattatcagc     360
tgtgaacact ttctgtttct agaggccatt ctctgtaaca ctttggagca tcaattttgt     420
agcgtgctat catggactac tctgctggtt cctacatgtg gcctggcaat tcaggttctg     480
agaactataa ttttgttgac ggttcgtcgg aatcatatgc agaagaagga agcctaccac     540
cttcaggcta tttcatggga gctggatcag atcgcagttt aaagatcacg gagaatgaaa     600
ggaaccccac tatgcttgca aatggatgct tgccatacaa cacccaggct catccattat     660
ctggccagat tctacctaag ggtgagctcc ctaacaatct tctggatctt caacagctac     720
agaacagcag caatctgcga agcaattcaa ttcccccagg ggttcttcag tgcaattcaa     780
catctggaac atttgatgcg aaattggata cacctggtct tgcagaactg cctcatgctt     840

tgtccagttc aattgatagc aatggtagtg acatttctgc ttttcttgct gatgtgcatg     900
ccgtttcttc agccccgacg ttgtgttcag cattccaaaa cgtttcctcc ttcatggaac     960
cagtaaatct agatgctttc ggtttccaag gggcacaaaa tgttgctatg ttgaacaaaa    1020

caagtcttcc aaatgggaat ccctcgctgt ttgataatgc tgccatagca tcactacatg    1080
atagcaaaga gtttctcaat ggtggttcca tcccttcgtt tggtactgtc ctgcaggcac    1140
taggagcggg tggtttgaag gctgctcaac aggagcaaaa tatccggaat atacctctcc    1200
ctacattcac atctggtagt catttggcag ttactgatgc acaagggcca ccacttcctt    1260
caaagatacc accattgatc catgaccata atagtgagta ccctattaac cattcttctg    1320
atgtggaacc ccaagcaaat tcagctcctg gaaatagtgc caatgcgaag ccacgtacaa    1380
gggctcgccg tggacaggca actgatcctc acagtattgc tgaacggctt cgccgagaga    1440
aaatttcaga aaggatgaaa aatctccaag tccttgtccc aaactccaat aaggcagata    1500
aggcatcaat gcttgatgaa ataattgatt atgtgaaatt tcttcagctt caggtcaagg    1560
tattgagcat gagcaggcta ggagctcctg gagcagttct tccccttctt agggaatctc    1620
aaaccgagtg ccatagtaat ccatctctat cagcttcaac catttcacaa gggccaccag    1680
acatgccaga ctcagaagac agctctgcct ttgagcaaga ggtagtgaag ctgatggaaa    1740
cgagcatcat cagcgcgatg cagtatcttc agaacaaggg tctctgcctg atgcccattg    1800
ctctcgcttc ggccatatcc aaccagaaag gcatggctgc tgctgctgca atccctcctg    1860
aaaaatgaaa aaaaaaatga aatcggaaaa atatgggggg aacaactgca gaagacataa    1920
taactcaacg gctgcagaaa tgtgctggac actgcaggtt ttgtgaatga agtgaaatcc    1980
aatccagggg gtcataaaac ataggaagtt atctgactaa ttcaggctta tgacacaatc    2040
tactctatag tctatagcta ttgtgtgcct gttgcacatc agttgatgtg tattcttttg    2100
tttttttgt attgcatccg gatgctaatt aggtagtagt gtttcctttg atgtacatga    2160
gtattatcta tgcggtttta tctaatgtgc atataaagta aaatctggta tgttgctgct    2220
a                                                                    2221
```

<210> 366
<211> 1500
<212> DNA
<213> Oryza sativa

<400> 366

```
atggctgcgg cggcggcggc gggccagatc tccctggacg acctccgcaa cggcggcggg     60
gtcgccgcca atgccggcgg cggcggcggc gtccacgacg acttcctcga ccagatgctc    120
agcagcctgc cgccctcggc gtggcccgac ctcgccgccg ggaaggcggc cgaggacgac    180
gccgagggga tgcatcacca ccaccaccag cagcagcagc agtttggtgg gccgtacgac    240
gagtcggcga tgctggcgtc gcggctccgg cagcaccaga tcagcggcgg cggaggagga    300
ggcggcggtg gcgcggccgc cgtgaagcag atggtgctgc agcagctcgc cgatctgagg    360
caggggcacc acatgatgct ccagggcttg gggggacggt cgccggcggg gggcggcggc    420
ggcggcggcg acggggcct tctcctcccg ctcaccctcg gcagcggcgg atcgggcggc    480
gacgtgcagg cgttgctcaa ggccgccgcg gccaattccg ccggaggagg agacgccggc    540
ggcgtctacg gcggcggatt cgccggctcg ctccatcaac agcagcaaca tttccagcca    600
catccacaga cggcgccgac gattccgacg cagagcttcg gcggcggcgg cggcggagga    660
ggaggaggaa ccgcgtcagg cggcggggcg gcgcagcctc aggccggcgc ggctggagga    720
ggcgcgccgg cgccgccgcg gcagcgggtg cgggcacggc gaggccaggc caccgacccc    780
cacagcatcg ccgagcggct tcggagggag aggatcgccg agaggatgaa ggcactgcaa    840
gaactggttc ccaacgccaa caagttgatg cagacggaca aggcttccat gctggacgag    900
atcatcgact acgtcaagtt cctacaactc caagtcaagg caagcacgta cactaaatta    960
ctaatacatg ttttgagcat gagccggtta ggtggcgccg cagccgtcgc gccgctcgtg   1020
gccgacatgt cctcggaggg gcgaggcggc ggcgcggcga acggcggagc accggcggcg   1080
gcggggagcg atagcctgac ggtgacggag cagcaggtgg ccaagctgat ggaggaggac   1140
atgggcaccg ccatgcagta cctgcagggg aagggcctct gcctcatgcc gatctccctc   1200
gcgtccgcca tctcctcggc gacgtgccat ctccggccgc cggtggtcgc cgccgcgcag   1260
cagttcccgg ccgggctcgg cgccgccgct gccgccgcgc accaccacca actgagcgcg   1320
gcggcggcgg cggcggccat gcgcggacac ctgcccggcc tgaacgccga cggctccgtg   1380
ccggcctcgc cgagcatgtc ggtgctcacg gcgcagtcgg ccatggccaa cggcggcggg   1440
ggcgccgccg acggcgaggg gtcgcagctc aaggacgccg cctccgtctc caagccgtga   1500
```

<210> 367
<211> 411
<212> DNA
<213> Oryza sativa

<400> 367

```
atgagggccc tgcaggactt ggtccccaac acaaacaaga cagacagggc agctatgcta     60
gacgaaatcc ttgattatgt gaagtttctt cggcttcaag taaaggttct gagtatgagc    120
aggctgggtg gtgctggtgc agttgcgcag ctggttgctg atattccaat ctcagttaag    180
ggggaggcaa gtgacagtgg gagcaaacaa cagatttggg aaaagtggtc aaccgatggc    240
acagaaaagc aggtagcgaa gctgatggaa gaagacatcg gagcagcaat gcaattcctc    300
caatccaaag cactatgcat gatgccaatc tctcttgcga tggcgatcta tgacacacag    360
cattcgcagg acggacactc agtgaagcct gaacccaaca ctccttcata g             411
```

<210> 368
<211> 913
<212> DNA
<213> Picea abies

<220>
<221> misc_feature
<222> (804)..(809)
<223> n is a, c, g, or t

<220>
<221> misc_feature
<222> (858)..(862)
<223> n is a, c, g, or t

<220>
<221> misc_feature
<222> (875)..(882)
<223> n is a, c, g, or t

<220>
<221> misc feature
<222> (886)..(893)
<223> n is a, c, g, or t

<400> 368

```
cacaggaggc agctgtaacg gcagcgacgg aggaatgctt cctcttcctc tcagtcttgg     60
tcaggctgga aagtccggcg acatgaacga gcccggatcg cgggaagaaa ttgaagcttc    120
tttcaagtcg gtaaataatg cccgagattc gaacctcgga gggctattcc agccgttcgc    180
cgtatcaccg cggggcgttc gaccgaccgg tcaaaacttc catgcgcagc ccggacaagt    240
gccattgcaa gggtatggtg gaatgcccca acctcagcat cagaatccac ctccgaccgg    300
agttggtgct gcaccacctg ttcgacctag agtaagggca cgccgtggac aggctacaga    360
tccccacagt attgcagaac gattgcgtag ggagaggatt gcagagagaa tgaaggctct    420
gcaagaactg gttcctaatt ccaataagac ggataaggct tctatgctgg atgaaataat    480
tgattacgtc aagtttcttc agctgcaagt gaaggttcta agcatgagtc gactgggagg    540
tgcaggagca gttgctcccc ttgtggctga tatacctgct gagggtgcca acgccccaca    600
aggcacaaga accaacggta gccagaactc ttctccggac ggtctagcat taacagaacg    660
ccaagtagca aagctaatgg aagaagacat gggaacggcc atgcagtatc ttcagggaaa    720
aggtctctgt ctgatgccta tatctctggc ctctgccatt aataactccg gttcgagacc    780
ccaggctcct tccacccctt ctcnnnnnng actattggca tctaatgtta acgacaggca    840
ggtactcgaa ccctcctnnn nntcggcact atctnnnnnn nnctcnnnnn nnnatgacat    900
acagccatcc atc                                                       913
```

<210> 369
<211> 1266
<212> DNA
<213> Populus deltoides

<400> 369

```
attácggccg gggggtactc ctcactagtc actggctacc tgtgctcgta ttagaaagca     60
aaccaaagca aagcgaaaag ctgaaacctt gaaagccgaa gtacttagct ataaagaaaa    120
agagaaaaag aaagaaagaa gaaggtaacc caacagtcct taactctctc aattaaaacg    180
attttgcatg taaattgatg ctattttgta tccccaactc tctatctcaa ctcacttcga    240
ctcctccgag tccaactcca aatggccaat aatccaaccg aacctccagc cgacgatttc    300
ctccaggaaa tcctcgggat gcctaacttt gcttcggctg aagccgggtt ggttggagcc    360
gatgctggcc tggctggcgc cgctgctgct caagcttcta tgatgcttca gcttagctct    420
ggtgatggtt ccggccacat ctccgacctt ggcggtgctc ctggaggagg aagcgccggg    480
tttcacggct ttcccttggg gcttagcttg gagcaaggga agggagggtt tctgaagccc    540
gaggaggcgt ctgggagtgg aaaaaggttc cgtgatgaga ttgttgatgg tagagcgaaa    600
aatgtttttc atggtcaacc aatgccgaca acggttgcta tagcaccgca tccaccagca    660
atgcgcccaa gggtacgggc tagacgaggt caagccacag atccacacag tattgctgaa    720
cggttgcgca gagaaagaat agccgaaaga atcagggcat tgcaggagct ggttcctagt    780
gtcaacaaga cggatcgagc caccatgctt gatgaaattg tggattatgt gaagttttta    840
aggcttcaag taaaggtatt aagcatgagt agactggcgg gagctggtgc tgtcgcacca    900
cttgtaacag acatcccact atcatcagtt gaggatgaaa ctggcgaagg aggaagaaac    960
cagccagctt gggagaagtg gtcgaatgat ggcactgaac ggcaggtagc taagctaatg   1020
gaggaaaatg ttggcgctgc catgcaattt cttcaatcga aggctctttg catcatgccc   1080
atctcactag cgacggccat ttaccataca caaccaccag ataccactag tattgtcaag   1140
ccagaaacaa atccccaatc atagaaccgt tgaaaggtgg gggagcatcc catggtcctg   1200
tctaaaagag taaagtccaa atgcttaggg tcccacgtct tatttgtcac gtttcacccc   1260
cacaat                                                              1266
```

<210> 370
<211> 1853
<212> DNA
<213> Pinus radiata

<400> 370

```
gttgcaaagc aatacccaga aattattcac aatgtcattg ctgcctcccg ggataggagg     60
ccagggcttg aactctcagg agaacgaccc gtatccgtac gacgattccc agttcctagc    120
taacaggcta aggcagcatc aactgagcgg taattcttcg atgaatcaga cgccaaacca    180
ggcctcgcaa gggattaatg aaaccaatac gtctccgggc cgatccatgg ttttgcagct    240
aagcacagga agtgcaagcg cttcacagct gctggcctcc atgggaaatt cgcctcgcgg    300
caccgcggta atgacgcagt cgcccagtac aggaggcagc tgtaatggca gcgacggagg    360
aatgcttcct cttcctctca gtctcggcca ggcgggtaaa tcaggcgaca tcaacgagtc    420
ccgctcgcgg gatgaaattg aagcttcctt caagtcggca aatcatgccc gagattcgaa    480
cctcggaggg ctatttccgc cgttcgccgc atcaccacgg ggcgttcgac cgacaggcca    540
aaacttccat acgcagtccg gacaagtgcc aatgcaagtg tatggtggaa tgccccaacc    600
tcagcatcag aatccatctc cgaccggagt tggtgctgca ccacctgttc gacctcgagt    660
aagggcacgc cgtggacagg ctacagatcc ccacagtatt gcagaaagat tgcgtcggga    720
gaggattgca gagagaatga aggctctgca agaactggtt cctaattcca ataagacgga    780
taaggcttcg atgctggatg agataattga ttacgtcaag tttcttcagc tgcaagttaa    840
ggttctaagc atgagtcgac tgggaggtgc aggagcagtt gctccccttg tggctgatat    900
acctgctgag ggtgccaacg ccccacaagg tacaagaacc aacagtagcc agaactcttc    960
tccagacggt ctagcattaa cagaacgcca agtagcaaag ctaatggagg aagacatggg   1020
atcagccatg cagtatcttc aaggcaaagg tctctgtctg atgcccatat ctctggcctc   1080
agccattaat aactccggtg cgagacccca ggcaccatcc accccttctc ttcaagggct   1140
attgccacct aatgccaacg acaggcagat actggaaccc tcgaactcgg cactctctgc   1200
cctcacgtcc acctccatga ccatacagtc ttccatcagc tcgcctggga gcggaataac   1260
ggagcaaggt gataatgcac acaaggcagt gaacggaaca aggaatccaa aggattccag   1320
agaagccaat tccgttacga agtccaacgg gataggaggc ccagctcttt ccaagaacgc   1380
cgttaaagga gaggacgaac ctcaaaggag gacgggccag taaacagcac caccttgcta   1440
acggaaactg taccatactt ctattaacgc cagattgcat gaaattgttc aagcatacga   1500
aatttacttt tcacatgctt caacaatttc taaatagtcg tagtcgcagt cgcagtcgct   1560
ctatgggaga gcattcattc aaaaatccca aaaacacact tcaaattttc ctttctcctg   1620
aagtttggtg tttattttta ttgaatgggt aaaagaatcg agcaagtcta tcaaaataag   1680
gcacgcgccc cactttgatg atgccttatt taaatagact tgcataaata tgacattatg   1740
tatggttgac ccaagcgctt tgttaatcac tacgtaagtt atatcttaag ttggataata   1800
attattgctc atgtcagcta cactacgaaa ttcttgttta tttaaaaaaa aaa          1853
```

<210> 371
<211> 1877
<212> DNA
<213> Picea sitchensis

<400> 371

```
caggtaaaaa gaaactttct ttttgattcg gaataaaatt cacacaatgt gtgaaaattc     60
caaaactctc acggtaaacg ttttagaatg taacattgac ttgcatggga aggatgtgaa    120
taattaaaaa tccttcccac actaaaaccc acttgatctt tatatgctta atactaatcc    180
cggaagctct taaaccagaa gattgtacaa ttgaatcttc atactcgaat aaaaacaact    240
atttctggtt aaacaaaaat cgagaaaaga cggacggcca tgttacaacc acggcagttt    300
ctctcctctg gttcttgtta gaaagtggtc aattattatg ctatttgcag agaactaaga    360
ttgccaacaa acagctagtt cattgtagct gggagatttc agtttaagat gacaggcaaa    420
atcaacattg caaaaaactc cataagagat ggctcctaac ccacgtcttt tcgtatgtga    480
gccaccagta tactttccat atattcgaga atccttgaag ttagaagcaa cctttttttgg    540
cctcggatat cttccctatt cctttttcct aagtttcggc aagctagata ttatcccaaa    600
ttctgctcca tttgactcag cggtgggaac taattctctt cctctctttt caactgtgtt    660
ttcctttaaa aggaaatcac tgccaaatcc agcagatccc attccaagag cacctgagca    720
actatcgaca gttaatgaag caggctgatt gcctgagctt tgcctgtcac tagtactgtc    780
aacagaattt gttaccccag aacccaacag tggctttcca gttgaactcg atatagcagt    840
tgccagtgca attggcatca aacaaagccc tttattttgc aaatactgca tggctgcagt    900
catatccttc tccatgagct tcacaacatt ttgctcgaag gctagaccat cctgcgatag    960
tgagccgcca gcttgaccca gagccgctga tgccaggctg ccagatccct cagctggaac   1020
atcagcaatt agaggcgcaa ctgcgcctgc accaccaagc ctactcatac tcaaaacctt   1080
tacttgcagt tgtagaaatt tgacatagtc aatgatttca tccagcatgg acgctttgtc   1140
tgtcttatta gcgtttggaa caagctcctg caaggatttc aaatttttttg caatcctttc   1200
cctgcgttcc cgctcagcta tactgtgtgg atcagtagct tgactacaaa ctaatagctc   1260
cttcaaaaga tacggtgtgt tttcgccagc aatgacacta gaagaactat tctgaacatc   1320
attattttga gcctggcctt ctaagttcgg actcatatga cttgacggtg ttactgacat   1380
tgttgagact cttgagctcg cttgaagtgt tcccatagaa tcaccaggca agcaatttgt   1440
tggtcctcca gcactactat ccaaagatga tctgtcggca ccatttatgt cttcccatga   1500
agatgatagt aattgatcaa aaatgtcatc cattacagaa ctcaagagct ccactcactt   1560
gcaaaacaat tgctatgaaa ttgccctttg tccagcaata aactagctga aacagcctta   1620
gctgatagat ttcatttctt gttgctctca aaaatccaaa atcaggcttt cccctcagca   1680
cctacattaa ccgctagcag cagccatgtc cgcctataaa atagcagaat ttaaccaaag   1740
aacgatttct ccgtttccaa tttagccttc tttaatgtcg cggcttggca aaactgcaac   1800
caacatgtgc cggcattgaa cgttaacagg agagcagagc agagcagcca cattagcata   1860
acgttatcag aatttca                                                  1877
```

<210> 372
<211> 1548
<212> DNA
<213> Pinus taeda

<400> 372

```
ttttgggcaa aggtctcact ctgaagacaa aatgctagcc agagaaaata gatctggaga     60
tactgagcat gatgatcttc aagggaccct tttgacatct tatacaggac cacaaggagg    120
tcagacagtg ctacctagaa ctccaggggc acaatcccat caacagaatc ttagcacaca    180
aactatacaa tcaggtgaag gaacttctct acagcagtat ggaaaccatt cagctgtttc    240
tcagctgcag tctggggctg gtggtggcaa tgctgcaaat ggagctgcaa ggccacgtgt    300
cagggcacgt cgaggtcaag ctaccgatcc acacagtatt gctgagcggc ttcgcaggga    360
gaggattgca gaaaggatga aatctttaca agaacttgtt ccaaactcta acaagacaga    420
caaagcatcc atgcttgatg agatcataga gtatgtcaag tttttgcagc tgcaagtgaa    480
ggttttgagc atgagtaggc ttgggggtgc tggtgcagtt gcacctctga ttgctgatgt    540
tccatctcag ggatcaggtg gtgtcatgtc aacagccttg ggccaagcaa gtgggccctt    600
gactctgtca caggatggtc ttgcttttga acaggaagtt gcaagactca tggaatcgaa    660
catgacctca gctatgcaat atttacaaaa taaaggactt tgtttgatgt ccattgcact    720
tgcaacagct atatcaagtt caagtggaaa gcctgtactg gctactgtgc caggaacagg    780
tgttgatagt agtgagaagc aaaattctga catgcaatcc attgtcttac cttttagtag    840
cagttcaaca accatgggac tcagagctac tgcatctggc agtgacgccc ctataaatga    900
gaattcaata aataaagcta gcatagaaaa agttgtggca gaaaaatcaa atggcatttc    960
acctgggcta tcatctgatg tgcccaaggt tggatcacag agcagggaag aactcttgaa   1020
```

```
gcgagcacaa tgatttctat ctgcaagtgt gtctgatttc ggtattcagg agtgagcctc   1080
aatgctaagg cggttggtga gattttctag tacgcaattt tttaacagat aggttatata   1140
ccactgatta tggttcacac atctgggatc gtgtgagatt tcaataggag caaaatgtgt   1200
aatgtataag tgaacccctg ttttaatgag aaatattggt ggaactctgt tggtgcaagg   1260
ttgccaacct ttttatttcc actttccttt taggcaaatt taaggttacc aagtatttat   1320
tctaacttga acatgactgg tgctgaccat actagggtcc tgctactgag ataccaagat   1380
ggctcaggca tccattttcc tgtttccctt agttttgatt tgtggataag tggatattta   1440
tccaaacttt aaacaattgg atgatcttat ttcaatttgc ttcaacaaat gttaaatgca   1500
atgttctcaa taccaaggta agtattacca ctgggttgtt ttctgttt                1548
```

<210> 373
<211> 1013
<212> DNA
<213> Populus trichocarpa

<400> 373

```
tcactttgac tcctcccaga cctactccaa atggccaata acccaaccga acctccaacc     60
gatgatttcc tgcaggaaat tcttgggatg cctaattttg cttcagctga agctggcttg    120
gttggagctg acgctggctt ggctggcact gcttctgttc aagctcctat gatgcttcag    180
cttagctccg gtgatggttc cggccacatc tccgcactcg gcggtgctcc tggaggcgga    240
ggcgcgggat ttcacgggtt tccgctgggg cttagcttgg agcaggggaa gggagggttt    300
ctgaagcccg aggaggcgtc cgggagtggg aatcggttcc gtgatgatat tgttgatggt    360
agagttagaa atgtttttca tggtcaacca atgcccacaa cagtcactgc agctacacat    420
ccaccagcaa tgcgcccaag ggtacgggct aggcgaggcc aggccacaga tcctcacagt    480
attgctgaac ggttgcgcag agaaagaata gccgaaagaa tcagggcatt gcaggagctg    540
gttcctagcg tgaacaagac ggatcgagcc gccatgcttg atgaaattgt ggattatgtg    600
aagtttttaa ggcttcaagt aaagatatta agcatgagta gactgggcgg agctggtgct    660
gttgcgccac ttgtaacgga catcccacta tcaccagttg aggatgaaac tggcgaaggc    720
ggaagaaacc aactggcgtg ggagaagtgg tcaaatgatg gcactgaaag acaggtagct    780
aagctaatgg aggaaaatgt tggtgctgcc atgcagtttc tccaatcaaa ggctctttgc    840
atcatgccca tcacactagc cactgcaatt taccacacac aaccaccaga taccactact    900
attgtgaaac cagaaactaa ccccccgtca tagaaccgtg gaaagggggg agcatcccgc    960
ggtcccgttt aaaagtcacc aaatgcttag ggtcccactc cttatttgtc ccg           1013
```

<210> 374
<211> 1106
<212> DNA
<213> Populus trichocarpa

<400> 374

```
agtagtattt tgtgtcatat atttgcatgc atggcaggaa atcccccacc tgaagggtta     60
ggagatgatt tctttgagca gatcttggca gggcagccac ctggttatgg tggtgaggct    120
gtggggtcca ccagcctgcc catgatgggg ttgcagttag ggtctagtgc tgctaatgtt    180
ggattaatga ggagtagtgc taataataat atgggaatga tgcctttagg gttaaacttg    240
gagcatcatg ggtttttaag gcaacaacaa gatgatggca gtagttcttt agataccaac    300
aacagcaata atatcaataa tgcttctcct ttttctatca cttctgctgg attcttgggg    360
agagattctg tacacatgac tagcttgttc ccaacgtttg gacaattgca gattcattca    420
tcaatccggt ctgcaccacc accacttgga ccacctcaga ttcaccagtt taatagccag    480
ccaacatcag gagcagtttc agctgtacca caaccacctg gcattcgacc aagggtgcga    540
gcaagacggg gccaagctac agatccgcac agtattgctg agaggttgcg aagagtaaga    600
atcacggaga gggtgaaggc gttgcaggaa ttggttccca cttgcaacaa gacagatagg    660
gcagccatgc tggatgaaat tgtggattat gtgaagtttc ttagactcca agtcaaggtt    720
ttgagtatga gcagactcgg tgcggcgggc gctgtggctc agcttgtagc tgatgtacca    780
ctgtcatcag ttcagggaga aggcattgaa ggagggggcca atcagcaagc ttgggagaat    840
tggtcaaacg atggcactga acaggaagta gctaagttga tggaagaaga tgttggagct    900
gccatgcagc tccttcagtc caaggcactc tgcatcatgc ctgtatcact tgcttcagca    960
atcttccgag cacgcccacc gaatgctcca acactggtca agcccgaatc gaaccccccc   1020
tcataaacct aagcaagaac gtgcaagctt tgcatacaca tcgatcattc caggggggcat  1080
ctgtttccca aattaaaaac tgctct                                        1106
```

<210> 375
<211> 1115
<212> DNA
<213> Populus trichocarpa

<400> 375

```
gtagtatttt ggtgtcatat atttgcatgc atggcaggaa atcctccacc tgaagggttg     60
ggagatgatt tcctcgagca gatcttggca gcgcagccac ctggttacgg tggtggtggg    120
gaggttatgg ggtccactag cctgcccatg atggggctgc agttagggtc ttgtgctggt    180
aatgttggat tactgaggag taatgctaat aataacatgg gaatgatgcc tttagggctg    240
aacttggagc atcatgggtt tttaaggcaa cagcaagatg atagtggtag ttccttagat    300
accaacaaca gcaataatat caataacact cctccttcta taaaatctgc tcgaatcttg    360
gggagagatt cggtgcacat gacaagcttg tttccaacat ttggacatct gcagattcat    420
tcatcaatcc ggcctacacc acctcctcct ggaccacctc aaattcacca gattaatagc    480
catccaaacc caggagcagt ttcagctgta ccacaaccgc ctggcatacg gccaagggtg    540
cgagcaagac ggggccaagc cacggatcca cacagtattg ctgagaggct gcgaagagta    600
agaatcacgg agagggtgaa ggcgttgcaa gaattggttc ccacttgcaa caagacagat    660
agggcagcca tgcttgatga aattgtggac tatgtgaagt ttctaagact ccaaatcaag    720
gttctgagca tgagcagact aggtgcagca ggcgctgtgg ctcagcttgt agccgatgta    780
ccattgtcat cagttcagat taagggagag ggcaatgaag gaggagccaa tcagcaatct    840
tgggaaaatt ggtcaaatga tgacactgaa caagaagtag ctaagttaat ggaagaagac    900
gttggagctg ccatgcaatt ccttcagtcg aaggcactct gcatcatgcc catatcactt    960
gcttcagcaa tcttccgagc acgcccacct aacgcatcaa cactcatcaa caccgaatcg   1020
aacacccctt cataaaccta agcaagaaag tgcatacttc gtgtgctcgt cgatcattcc   1080
agcaggcatc tgggtcccaa atcttgttct tgtcc                              1115
```

<210> 376
<211> 1481
<212> DNA
<213> Populus trichocarpa

<400> 376

```
caagacgaca aaaaaaattc acctgtaagc atgataagtt caaatcaaag tgtggagtcc      60
ttggccactc aagacacttc atctgtagtt cttggcagtg agtcagatta tgctgttgat     120
aaagtcttaa tttctgaaca agctcgattg caaaatgatt gccagaattg caatggaaac     180
ccctcccctg atggaatggc acgtggaaac ttaaaatttg gaaacacagg cctgcaatgc     240
aatggaattc ttcctaccct gagttctctg aactatccaa atcagcttcc aatagttggt     300
gatttgacat cgtatctctc tttttctgag gcaagcaatg ctggatgcaa tggaagggaa     360
caatctgagt acctgagatc cttaaaaaat ctgcaaaact tatcatcaat cccacaattg     420
tggccttcac aatcttatga gggtgtttct tctctccctc ctttgatggg acaagacaga     480
atagagggat ctggtcttcg aggagggaac ttggatgatg atatgcatat tatggggaag     540
ggatacatgg gcatggatga aattctccga cttgataagt tatctgcatc acctactaca     600
gaggggaaag aagatttgca aagttgtcct ttctcatctg gcatagctga gcccaatgta     660
aacatgtcaa tgaatcaatt atcatctatg cctcagacta catcagctgc tccagtagaa     720
ggctgcaatg gaactggaaa aactcgtgta agagcacgac gtggccatgc cactgaccca     780
catagtatag ctgaaaggct tcgaagagag aaaattgctg aaaggatgaa gaatctgcaa     840
gaacttgtac ctaattccaa taaggttgac aaggcatcta tgcttgatga gatcatagag     900
tatgtcaaat ttcttcagct ccaagtcaag gtgctaagca tgagcaggtt aggggcagcc     960
ggagcagtca ttcctctcct cacagatggt caacctgagg gtcataatag tttgtcgctc    1020
tctccatcag ctggtctagg aattgatatt tctccatctg ctgatcagat tgcctttgag    1080
caagaagtac ttaagctact ggaatccgat gtgaccatgg ccatgcaata tcttcaaagc    1140
aaaggtctct gcctcatgcc cattgctctt gctgctgcca tatccagtgt taaggcatcg    1200
ttatcaggta caacttctga ggagaggaag aacaatggct acaccagtgg tcttgttagc    1260
agcagcagca gcataactgg cattgacacc catccaatgt ccaatgataa caatattgca    1320
accggtacac ttagcagcaa aggcatgatt gtcaatggct gcaatgaggt tgtcaagcaa    1380
gaagtgctga agaacacttg atgcatttct agagcacgga aagtaaagat gtaatttcat    1440
tattttagat ggggtttcat ttttcaagct gaggcagata a                         1481
```

<210> 377

<211> 1711

<212> DNA

<213> Populus trichocarpa

<400> 377

```
gggtctctcc gctctcagta ctcctcacta gtcactggct acctgtgcta gtattagaaa      60
gcaaaccaaa gcaaagcgaa aagctgaaac cttgaaagcc gaagtactta gctataaaga     120
aaaagaggaa aaagaaagaa agaagaaggt aaccaaacag tccttaactc cctcaattaa     180
aacgattttg catgtaaatt gatgctattt tgtatcccca actcgctatc tcaactcact     240
tcgactcctc cgagtccaac tccaaatggc caataatcca accgaacctc caaccgacga     300
tttcctccag gaaatcctcg ggatgcctaa ctttgcttcg gctgaagccg gtttggttgg     360
agccgatgct ggcctggctg gcgccgctgc tgctcaagct tctatgatgc tgcagcttag     420
ctccggtgat ggttccggcc acatctccga ccttggcggt gctcctggag gaggaagcgc     480
cgggtttcac ggctttccct tggggcttag cttggagcaa gggaagggag ggtttctgaa     540
gcccgaggag gcgtctggga gtggaaaaag gttccgtgat gagattgttg atggcagagc     600
gaaaaatgtt tttcatggtc aaccaatgcc cacaaccgtt gctatagcac cgcatccacc     660
agcaatgcgc ccaagggtac gggctagacg aggtcaagcc acagatccac acagtattgc     720
tgaacggttg cgcagagaaa gaatagccga aagaatcagg gcattgcagg agctggttcc     780
tagtgtcacc aagacggatc gagccaccat gcttgatgaa atcgtggatt atgtgaagtt     840
tttaaggctt caagtaaagg tattaagcat gagtagactg ggcggagctg gtgctgtcgc     900
accacttgta acagacatcc cactatcatc agttgaggat gaaactggcg aaggaggaag     960
aaaccaacca gcttgggaga agtggtcgaa tgatggcact gaacggcagg tagctaagct    1020
aatggaggaa aatgttggcg ctgccatgca atttcttcaa tcgaaggctc tttgcatcat    1080
gcccatctca ctagccacgg ccatttacca tacacaacca ccagatacca ctactattgt    1140
caagccagaa acaaatcccc catcatagaa ccgttgaaag gggggggagc atcccatggt    1200
cctgtctaaa agagtaaagt caccaaatgc ttagggtccc acgtcttatt tgtcacgttt    1260
caaccccac aatcaaatgt tcccctagtt tcaaattgca atcaaagtca gatgccatag    1320
ttcttgcttt tgccttttct ttccagcctt tttttttca aacggggttc gatgtcaatg    1380
cctaaaaaaa tgcaggcagg cccttggtgt cctgccaagt ccaagtgaga ggagaatttt    1440
caaagcttac tcgatatgct cttcttttgg aatcagtggt ggggaagtgg aaaatgatgg    1500
aagcgctttt gtaatggggc tctaattttt cgtagtgggt tgtgggacct tgtaaaagag    1560
gcaagactga cgggttatat aaatatatca ttattgtaat agtgtattca gcgtaaatgg    1620
aatgcaaagg tagaattgaa taatggctgg ttgtttcaat atgctgggtg tttcaagtcc    1680
aacagaaaat gatagcagtg ctactttgtt c                                   1711
```

<210> 378
<211> 1890
<212> DNA
<213> Poncirus trifoliata

<400> 378

```
ctctctctct tcttcctgtt ctcacctatt tagtatttaa catccatcag aagaatggtt     60
tccattctct tgaagaaatt cttgactctt caaatttaaa tttccatata tcataaagac    120
ttgttctcaa gaaattaaga tgcagccttg cagcagcggc ggtgaaatgc aaggaatcag    180
ctcgctcttg agcaacggaa gccacgagca gcagtcgcag atccatcaaa gcgcgacgtt    240
tgatccaact tcgcaagacg acttcttaga gcaaatgcta tcctctctcc catcgtgttc    300
ttggactgac ttgaagtctc cctggggggt tgttgacctt aaccctaata acaataataa    360
taatattaac aacaagcagc ccagagactt atctgacgaa acggcgccgt ccactactca    420
agagaataat gtccctgcag ggtttcagtt cgatgagtcc atgatcttag cttccaagat    480
gagacagcat cagatcagcg ggaatactgg gagtgggaat aataattctg ctgcagcaaa    540
gtttatgatg cagcagcagc agcaacaaca aatcatgatg gctgctgcta gaggtggcat    600
cgggttgcct ttatcacttg gaaatggtgg cgataacgac atcgttgatg cctcatcatt    660
caagtcccaa cagggaggag atggctcagt gcaagcactc tacaatggct tcactggatc    720
tttgcatggc tcaacccagc ctcaacattt tcatcatctt cagggaggat cgatgcccgg    780
gcagaccttt ggggcaccag ggccggtgat gaaccagacg caggctcagg caagtgggtc    840
aaccggtggt ggtggaaaca cacccgctca gcagcctaaa caaagggtta gggctaggag    900
aggtcaagct actgaccctc acagcatagc tgaaagattg cgcagagaga gaattgcaga    960
gagaatgaaa gctcttcaag aacttgttcc caatgccaac aagacagata aggcttcgat   1020
gctggatgag atcattgact atgtcaaatt cctccagctg caagtgaagg tcctgagcat   1080
gagtagattg ggcggtgctg ctgctgtcgc accccttgtt gctgatatgt cctcagaggg   1140
ggcaggagga ggtgattgta tccaagcaaa cgggcggaac cctaacggag ctcagacgac   1200
gtcagctaac gacagcttaa ctgtgacgga acaccaagtg gctaagctga tggaagaaga   1260
tatggggtca gccatgcagt acttacaagg caaagggctt tgtctaatgc ccatctcact   1320

tgcaactgct atatcgtctg ccacgtgtca ctctaggaac cccatcatca gcaccagcaa   1380
caacaacaat aacaacggca atccccacca caatcctttg tttcagtcca atggggaagg   1440
cccgacctca cctagcatgt cagtgctgac tgtccagtca gcaactatgg gtaacggtgg   1500
ggctgacggc tccgtcaaag atgctgcttc cgtttccaag ccctgatcaa cggacgtcga   1560
ctgactttat gatgaggctc gtccaaagtc tacgaaaata aggcgtcact agatgttgga   1620
ccttcacgac gtcgtatttg tgtagacttt gtgactgaat ctaacaaaca acaagaaagc   1680
aagaaagaag ccaatcccaa gaacaaaaac cttattttat gttgttatta ttattttaat   1740
ttgcgggtta ggttttgtta attttactat tattaaaatt ttaatttctc tgatgggata   1800
tctccctctt tttcttttat ttaatattct ctgtcatgat tctctattaa ataattattt   1860
ataaaagcct ttaataatta gattataatt                                    1890
```

<210> 379
<211> 1870
<212> DNA
<213> Ricinus communis

<400> 379

```
cccaacgctg cattttcagg atttctctct ctacactaca agaacttgaa gccagtaccc     60
ataaaaccat ataccatctt ggattccaca acccctttcc tggttattgg gctgtggatc    120
tgccacccaa ttcaccaaca ctttcttttt ctcgggtttc gcatacttca attcccctct    180
tctctcgtct cttctatttg tctctctaaa aacatactct ctccctctct ctctctctct    240
ctctctctcc tcagctaatt gaaacatcaa aaaggaaaaa gcattaccat atttttttaca    300
ctctaaaaca tcaaacttta tcactaatac tacgactgct actgctactc taaaactact    360
tttgcaatat tattgcatgg cgggaaatcc cccacctgag ggattaggag atgatttctt    420
tgagcagatc ttggcagtgc agccagggta tggaggcggt gatggtggtg gcggtggaga    480
cgtggtgggg tccaccatgc ctatgatggg tctacagttg gggtctgcca gtagtggtgg    540
tggtggattg agaactaata atatgggcat gatgccttta gggttaaact tagagttctt    600
gaggcaacaa gaagatggta gtggtgcttt agataacaac aaccatacca ataacaacaa    660
taataatgtt tcatcttcta caacttctgt tatcaatgat agagattctg ttcacatggc    720
gagtttgttc ccaacgtttg gacagttgca aacccagacg ctccggcctc caccaccacc    780
tcacctccac cagccatttc atggtcagcc aacaccagga gtggtttctg ctgcatcaca    840
gccacctgcc attcgcccaa gggtgcgagc aagacgagga caagccactg atcctcacag    900
cattgctgaa cggctgcgta gagagaggat tgcagagaga atgaaggctt tgcaggagtt    960
ggttcctaca gccaacaaga cggacagggc agccatgatt gatgagattg tggactatgt   1020
taagtttcta aggcttcaag tgaaggtatt gagcatgagt agactgggag cagcaggtgc   1080
agtggctcag cttgtagctg atgtaccgct agcatcagtt gagggagaga gcattgacgg   1140
agcagcagca aatcagcaga cttgggagaa gtggtcaaat gatggtactg agcaacaagt   1200
agctaagttg atggaagaag acataggagc tgctatgcaa ttcctccagt ccaaggcact   1260
ttgcatcatg cctatatcac ttgcttcagc aatccttcga acacaccccc ctgatgcacc   1320
atcaattatc aagcctgaat caaacacccc atcataaacc taaccaagaa attaatctcc   1380
tttcacgtgc agatgaattg ctaccagcag acatctgggc cccaagtgct ggtatagtcc   1440
catataactc attctaactc ctcattataa atatatatat atatataata aagaaacaat   1500
atccagttgg tgcagctgtt cattttcttt aggaggagtc attttcatgt caagtaagaa   1560
gaatcacggg gcacttgatg tttataatgc tatggtcaag gcaaatccaa aatattcaaa   1620
aagctccatt gatgtgcttc tatttcttgt atctgtggtg gggttagtgg agaatgacca   1680
aaaaattaat agatagtgct aagaaactct tctgtttcat ttctcttttg gatagtggaa   1740
gtgtagggcc atcaaaaagg gggttgatag gcagtgtgca tgatgtattt gaaaaatttg   1800
tgtggaagtt gaatcggata aatgtaggtt tgtcgtgtaa atccatgttg gaaacttcat   1860
gcttgacaat                                                          1870
```

<210> 380
<211> 1008
<212> DNA
<213> Ricinus communis

<400> 380

```
ctcctctcag gccgactcct gcaaatggca aacaatccca cagaacctcc agccgacgat     60
ttcctccaag aaatactcgg cctacctaac ttcgcttcgg ctgacgcagc cggcttggtc    120
ggagccgacg gtgccttggc tactccgatg atgctgcagc ttagctctgg agacggctcc    180
aaccacatca ctgccctagg tggaggggga ggaggaggag gaggcgctgg atttcacggg    240
tttccgttgg ggctaagctt ggagcaagga aaaggagggt ttttaaagcc tgaggaagct    300

tcgggaagtg gaaagaggtt tcgtgatgac gttgtcgatg gcagagctaa tactgttaag    360
aacgtttttc atggtcaacc aatgcccaca actatggctg cagccccaca tcctcccaca    420
atgcgcccca gggtgcgggc tagacgagga caagccacag atccacacag cattgctgag    480
cggttgcgca gagaaagaat agctgaaaga attagggcat tgcaggagct ggttcctagt    540
gtcaacaaga cagatagagc tgccatgctt gatgaaattg tggattatgt gaagtttcta    600
aggctccaag tgaaggtatt aagcatgagt agattgggcg gagctggtgc agtcgcacca    660
cttgtaacgg acatcccact atcatcagtt gaggatgaaa ctggggaagg tgggagaaac    720
caaccagcat gggagaagtg gtcgaacgat ggcacagaac gacaagtggc taaactgatg    780
gaagaaaatg tgggtgctgc catgcagttt ctgcaatcaa aggctctttg catcatgccc    840
atctcactgg ccacagcaat ttaccataca caagcaccgg atacctcaac tattgtaaaa    900
ccagaaacaa atccccccatc atagaccgta caaacgggta agcatcccat ggtcccatct    960
aaaaaggtcg cctaagtgct tggggtccca cattttgttt gtcccgtt               1008
```

<210> 381

<211> 1141
<212> DNA
<213> Sorghum bicolor

<400> 381

```
tactggactt ccccttcccc agcagagcag agcagaccaa ccgcttgtcg cgagccgccc      60
gaagcttcgg gccctgacgc gtgggccccg accgcgcccg cccccgcccc cgcgtcgccg     120
ggcatggcgg ggcagccgcc gccgcccggg ggctccgagg acgacttcct cgagcacttc     180
ttcgccttcc cctccgcggc ctccgccgcc gccgggggac acgcgggcgc cggtgccggc     240
ggggaccacc ccttcccccct cgccctcagc ctcgacgccg ccgccgagcc caagccggac     300
cgtgaccccg tgcagctcgc cggcctcttc ccgccggtgt tcgctggcgc cggcggcgtg     360
cagcaaccgc acctccgcgg cccaccgcct ccgcagatgt tccaggcgca gccgaagccg     420
ggcgagggag gcatggcgcc gcagccgcca gccccacggc ccaaggtgcg cgcgcggcgg     480
gggcaggcca ccgatcccca cagtatcgcg gagaggctaa ggagagagag aattgcagaa     540
aggatgaggg cattgcagga attggtcccc aacacaaaca agacagatag ggcagctatg     600
ctagatgaga tccttgatta cgtgaagttt cttaggcttc aagtaaaggt tttgagtatg     660
agcagacttg gtggtgctgg tgcagttgca cagctggttg ctgatattcc actctcagtt     720
aagggtgagg caagcgacag tgggagcaca cagcacatat gggaaaagtg gtcaactgat     780
ggcacagaaa agcaggtagc gaagctgatg gaagaagaca tcggggcagc gatgcagttc     840
cttcaatcca aagcgctatg catgatgccg atctcgcttg caatggcaat ctacgacacc     900
caacattccc aggatggcca ctcactgatg aagcctgagc ccaacacatc ctcgtagtat     960
agtaacatca accctagcgt gcatttcaac cccctaatac tattaggcac cgatatatcc    1020
aaaaaaaaaa agtgtatgat atacggagcg ttccaatgtg ctaaccgtga gataggaaag    1080
gaccttaaat tggtatatga tgtagcctcc cctttcccta ttttatttca aatcagagca    1140
g                                                                    1141
```

<210> 382
<211> 1409
<212> DNA
<213> Solanum lycopersicum

<400> 382

```
agcaaagctt taggtgactc cctttccttt attttcttca tggctctcac tgctctttag      60
aaagtagcaa aacacacaca caaacaaccc aaaacacaca cttccatttc catttttttgc    120
ctatatatat atatatatat gtaacaagta cttgttagag tactaatttg tacaccctct     180
tatcaattcc gttgtaaaga acactttttt aactagtatt tgttcaattg aatgcttttc     240
tgaggaatta aggtggtcga aaacagctcc gatccaagaa agattgaatc tttatagttt     300
ctgctatggc taacaaccct tctgagggac cttctgatga tttctttgac caaatcttgg     360
gatttcctgc ttacaatgga gcagaaccta atttggcggg aaatgatgcc ggagctatac     420
cgccggcgat gatgctccag cttaactctg gtgatgggtc aagtcagttt actggagtgg     480
gtcttggggt tggtttaggt ggtgggggt tccatggtca tggtggggt ggttcttttc       540
ctttagggtt gagtttagaa caagggaaag gtgggttctt gaagatggat gatgtttcag     600
cacctggaag gaggtttaga gatgatgttg ttgatagcag agcttcttct tctgtcaaac     660
ctggttttca tggacaaccg atgccttcaa tgccgcatcc ccctgcaata cgtccaaggg     720
tgagagctag gcgaggacaa gctactgatc cacatagcat agcggagagg ttacgtagag     780
agaggatagc agaaagaatt agagcattgc aagagttggt tcccagtgtc aataagactg     840
atagagctgt aatgcttgat gaaattgtag attatatcaa attcctacgg ctgcaagtga     900

aggtgttgag catgagtagg ttaggaggag ctggtgcagt agcaccactt gttacagaca     960
ttccaatatc atcagtagag gaagagagca gtgaaggtgg aaataataac caaccagctt    1020
gggaaaagtg gtcaagtgat ggcacagaaa ggcaagtggc taaactcatg gaagaaaatg    1080
ttggtgctgc aatgcaattt cttcagtcta aagcactatg tataatgcct atttctcttg    1140
catcagcaat ttatcactct cagccaccag atacatcaag tcttgttaag ccagaaacaa    1200
atcctccttc atagatgaac acccttatag aaaaaggtga cataacatgc taatgacctt    1260
aaagaatttt cttgcagtgg atggttccta cttttatcta ttcaagtaat atttagcttt    1320
ttgttgaaaa aacaggttc tttttaaaa atatttatgt ataaagtaaa ccattttcta     1380
tcatttatat atttgagtag ttttgttct                                      1409
```

<210> 383
<211> 1632
<212> DNA
<213> Solanum lycopersicum

<400> 383

```
gctcattttc cgttcctctt aaagctcaat aaacctatac gccggcaaac tccggcatgg     60
cggacaaccc accggaggta tatgctgccg atgattttct cgagcaaatt ctggcgattc    120
cctcttatgc tagcctgccg gtaactgatt taaccgccgg tgcctcatcg gagaattcaa    180
catctggtgt ctctcagctc cagcagcagc cgttgttccc attggggtta agtttggata    240
atggctttgc tgacgccaac aacactggag gttttcaagt gaagactgaa agagaagcaa    300
tgaacatggg gaatttgtat ccaggtctag aacatttgca atctcatgct gtttgcctaa    360
gtgttcctca agttcaccaa gtccagcctt ttcaaggcca ccctacatca agcgcaatag    420
taacaatacc acaccaacct gcaattcgtc ctagggttcg ggcacaaaga ggacaggcca    480
ctgatccaca tagtattgct gagcggttaa ggagagaaag gatatcagaa cgaataaagg    540
ctttacagga acttgtcccc agctgcaata agaccgacag ggcagcaatg cttgatgaga    600
ttctggacta tgtgaagttc ttaaggcttc aagttaaggt actgagcatg agtaggttgg    660
gaggaactag cgcggcggca caagttgttg ctgatattcc attgcagtct gttgagggag    720
acacctgtga aagtcattcc aaccagcacg tctgggagaa gtggtctgat tctgaaacag    780
agcaagaggt agcaaagctc atggaggaag atgttggaac agccatgcag taccttcaat    840
ccaaatcact ctgcatcatg cctatttcac ttgctgcact tatctatccg actcagcaaa    900
gtgacaacca atcaatggtc aagccagaac aagcggcccc attgtagact tctaaattac    960
tgcaggtgca atcaagaact cctaatttct tttaggccca tccgtctata tatccttacc   1020
tattttctct tgaatcaccc tctgaaatcc ttggtaatgc aatcaaactc agatgacaca   1080
atttttgctt ttagctcaat cattggcccc tttatcaact ttcttgagtt cagtcacacc   1140
ttgaacaatg gcaacactta agaaagcaac caatccatct atctatatca ttgtagggag   1200
gagtgtatat tttttttttcc aaactctctg ttattagctg cttgtttccc cctcactctt   1260
gtgggttggt tgtgactcct tgtatctttt ttatagtgga tagctagcga tgtattataa   1320
aagaaaaattg tcccagcttg catttggtgg actgtgcggg gagaaagaaa ccaagtttgt   1380
ttgaagacaa agccattagt atacagaaac taaagatgga atgtttaggg cttttctttt   1440
tttggtgtaa ccatagctta atggatgatt tagaatcaac ttatgatgta ctagattcct   1500
tgtgagaaga ttaaggatta ggagcttctt catttcttta tttctgttct tttgctcatc   1560
tggatgtata tatgggggat tacacaccct ttgtgtagtt tgttattaat acacaaaaat   1620
gttacctgtt cc                                                        1632
```

<210> 384
<211> 1135
<212> DNA
<213> Solanum lycopersicum

<400> 384

```
ttccatttca gttcctcaaa acccttaact tcttctcgcc gggaaaaaaa ttaatttcgc     60
cggataaatc tccggcatgg cagcaaacca accggaaggc tatgccgacg atttcctcga    120
gcaaattctc gctattcctc cctactctgg cttgccggtt gctgatgttg gcactccatc    180
agagacgacg tcgtttacct cggcgtctgc cgtatctcat cttaactctg ccgctgctgc    240
tggcctacag caacctttgt ttccgctggg attaagcttg gataacggcc gtgatgacgt    300
cggcgatgca ggtccttatg cagtgaagca tgaaagagat ggaatgaata tcggaaatct    360
atatgcaggt ctagaacact tgcaatctca tgcagttcgt cactctgtgc cttctgttca    420
ccatgtccag ccttttcaag gcccaccaac aacgagcaca acagtaactg tgccacaccc    480
accttcaatt cgtcctaggg ttcgagctcg gaggggacaa gccacagatc cacatagcat    540
tgctgagagg ctgagaagag agaggatatc agaaagaata aaggctttgc aggaactggt    600
```

```
acccagctgc aataagacag atagggccgc aatgcttgat gagattctgg actatgtgaa     660
gttcttaagg cttcaagtta aggtactgag catgagtagg ctgggaggag ccagtgcagt     720
ggcacaactt gttgctgaca ttccattaca atctgtggga gggggacagt ggtgaaagta     780
gatccaacca gcatatatgg gataagtggt ccaatgttga cacagaacga gaggttgcta     840
agctcatgga ggaagacgtc ggtgcagcca tgcaatacct tcagtctaaa tcactctgca     900
tcatgcccat atcacttgct gcactcatct atcctactca acaaccggat gaccagtccc     960
tggtcaagcc tgaagcagca gccccatcat agaccttcaa ttcgttgcac gtgcctctaa    1020
gaactcccaa tagccttgtg tccccctcc tttgtatcct tacctaccat ccatttgtat    1080
tttcctgtgt aattggtggc aatgcaatca atgtcagatg ccacaacttt tgctt         1135
```

<210> 385
<211> 1348
<212> DNA
<213> Solanum tuberosum

<400> 385

```
ttagaaagta gcaacacaca caaacaccca aaacacacac ttccatttcc attttttttgc      60
ctatatatat atatatatgt aacaagtact tgtgagagta ctaatttgta caccatctta     120
tcattttact tgtaaagaac acttttttaa gtagtatttg ttcaattgaa tgcttttctg     180
agaaattaag gtggtcgaaa acagctccga tccaagaaag attgaatctt tatagttctg     240
ctatggctaa caacccttct gagggacctt ctgatgattt ctttgaccaa atcatgggat     300
ttcctgctta caatggagca gaaactaatt tggcgggaaa tgatgccgga gctataccgc     360
cggcgatgat gctccagctt aactccggtg atgggtcggg tcaatttact ggagtgggtc     420
ttggggttgg gttaggtggt gggggttcc atggtcatgg tgggggtgct tcttttcctt     480
tagggttgag tttagaacaa gggaaaggtg ggttcttgaa gatggatgat gtttcagcac     540
ctggaaggag gtttagagat gatgttgttg atagcagagc ttcttcttct gtcaaacctg     600
gttttcatgg acaacccatg ccttcaatgc cgcatccccc tgcaatacgt ccaagggtga     660
gagctaggcg aggacaagct actgatccac acagcattgc ggagaggtta cgtagagaga     720
ggatagcaga aagaattaga gcattgcaag agttggttcc cagtgtcaat aagactgata     780
gagctgtaat gctcgatgaa attgtagatt atgtcaaatt cctacggctg caagtgaagg     840
tgttgagcat gagtaggtta ggaggagctg gtgcagtagc accacttgtt acagacattc     900
caatatcatc tgtagaggaa gagagcagtg aaggtggaaa taataaccaa ccagcttggg     960
aaaagtggtc aagtgatggc acagaaaggc aagtggctaa acttatggaa gaaaatgttg    1020
gtgctgcaat gcaatttctt cagtctaagg cactatgtat aatgcctatt tctcttgcat    1080
cagcaattta tcactctcaa ccaccagata catcaagtct tgttaagcca gaaacaaatc    1140
ctccttcata gatgacaccc ttatagaaaa aggtgacata acatgctaat gaccttaaga    1200
attttcttga agtggatggt tcctactttt atctatttaa gtaattttta gctttttgtt    1260
gaaagaaaca gggttttttt tttttatgtt tatgtataaa ttaaaccatt ttctatcatt    1320
gatatattca agtagttttg ttttcagc                                        1348
```

<210> 386
<211> 1469
<212> DNA
<213> Solanum tuberosum

<400> 386

```
gatttcagtt cctcaaaacc cttaacttct tctcgccgga aatttttttt aatttcgccg      60
ggtaaatctc cggcatggca gcaaaccaac cggaagccta tgccgatgat ttcctcgagc     120
aaattctcgc tattccttcc tactctggct tgccggtagc tgatgttggc actccatcag     180
agacgacgtc gtttacctcg gcgtctgccg tatctcatct taactctgcc gctgctgctg     240
gcctacagca acctttgttc ccgttgggat tgagcttgga taacggccgt gatgacgtca     300
gcgaagctgg tgcttatgca gtgaagcatg aaagagatgg aatgaatatc ggaaatttat     360
atgcaggtct agaacacttg caatctcatg cagttcgtca ctctgtgcct tctattcacc     420
atgtccagcc ttttcaaggc ccaccaacaa cgagcacaac agtaactgta cctcacccac     480
cttcaattcg tcctagggtt cgagctcgga ggggacaagc cacagatcca catagcattg     540
ctgagaggct gagaagagag aggatatctg aaagaataaa ggctttgcag gaactggtac     600
ccagctgcaa taagacagat agggcagcaa tgcttgatga gattctggac tatgtgaagt     660
tcttaaggct tcaagttaag gtactgagca tgagtaggct gggaggagcc agtgcagtgg     720
cacaacttgt tgctgacatt ccattgcaat ctgtggaggg ggacagtggt gaaagtagat     780
ccaaccagca tatatgggat aaatggtcca atgtagacac agaacaagag gttgctaagc     840
tcatggagga agacgtcggt gcagccatgc aataccttca gtctaaatca ctctgcatca     900

tgcccatatc acttgctgca ctcatctatc ctactcagca accggatgac cagtcactgg     960
tcaagcctga agcggcagcc ccatcataga ccttcaattc gttgcacgtg cctctgagaa    1020
ctcctaatag ccttgtgtcc cccctccttt gtatccttac ctaccatcca tttgtatttt    1080
cctgtgtaat tggtggcaat gcaatcaatg tcagatgcca caacttttgc ttttatccca    1140
accattggcc cttttttttt aattaccttt ctggagtttc gtcaaacaag gcatgatgtc    1200
atcatacctt ggagaaaggt aaacgtatga aagcacccaa tcaattagca tcattttcgg    1260
atgaggtgag cttctttttc aaagttatct atgattagct gctcttcttc caccattttg    1320
ttgtgggatt gtggggactc cctttatcct ttgcatagtg gatggtgggg ctttgtaaaa    1380
gtaaccaact ggttgttgga aagtatattg taattttagt atattattgt aatgggtagt    1440
tgaagttgct ctccctccaa aaagttttg                                      1469
```

<210> 387
<211> 772
<212> DNA
<213> Solanum tuberosum

<400> 387

```
cttctatctt tctctcttct ccttttagaa ccaaactttc acacacaaag ctactgaaga      60
aaaaatttct ctctgcaaaa tctcagagag gaaaaaaaaa atgcaagcca tgaattcact     120
tctaagtcaa caacagcagt cacagatgtc actgcaagac cttcaaaatg gcggaaatgg     180
cggttctacc ggtggtgttg gtggtttagg tcaacacaat atgggtcact ttcactttga     240
tccgacgtcg tctcacgacg atttctcgga acagattctc tcttctgttc cttcttcttc     300
tccttggcct gacctttcca aatcctggga cccacatcac catctctcat cgccgccgca     360
taaccctagc tccggcgaag atcaacctcc ttctaatcca cttcactcac agttccatta     420
cgacgaccag gcttcttctc tactagcctc aaagctccgt cagcatcaga tcactggcgg     480
cggcgcggct gcagctgcta aagcacttat gctacaacag cagcttttgc tttctagaac     540
actcgccgga aacggactca ggtctcctaa tggagcttcc ggcgataacg gcctcctttc     600
cctacctcta aacctcagta ataatggtga ccaaaacgat ggcgtcgcta acccggctaa     660
tgacaattcc gttcaagctc ttttcaatgg attcaccgga tctcttggtc aaacctccaa     720
tcaacctcaa cattttcatc atcctcaggg aggatcgatg caatcgcaga gt             772
```

<210> 388
<211> 1290
<212> DNA
<213> Solanum tuberosum

<400> 388

```
gaaagaaaaa aacaatgcaa gctatgaact catttcaaag caccggcgaa aatggtgctt      60
cgtccggcga acacatcagt cattcccatt tcgatccgtc gtcgtcacac gacgacttcc     120
tccaacagat tctctcttcc gttccttctt cttctccctg gcctgaaatc tccggcgacg     180
gtcaccccta caatttcgac gaccatcagt caactctctt ggcttccaag ctccggcagc     240
atcagatcaa cggcggcagc tctgctgccg cagctgctaa agcgttgatg cttcagcagc     300
agcttctgct ttctagagga atcgccggta atggcggcga tcaaaacgac gacggtttaa     360
atccggggaa tgatatttca gttcaagctc tttacaatgg attcgctgga tctcttggtc     420
aaacctcaaa tcaatctcag cattttcacc attctcaggc gcagagtttc ggagctccgg     480
cggcgtcgtt gacgatgaat caaacgccgg cggcgagtgg ttcagctggt ggagcgcagc     540
caaagcaaca gaaagttagg gctcgaagag gacaagcaac tgatcctcac agcattgctg     600
aaagattacg gagagagaga attgcagaga gaatgaagtc tttgcaggag ctggtaccta     660
atgccaataa gacagacaag gcttcaatgt tagatgagat catcgactat gtcaggttcc     720
tccagctcca agtcaaagtt ctgagtatga gcagattggg tggtgctgca gctgttgctc     780
ccctagttgc tgatagatcc tctgagggag gaggtgattg tgtacaagga aatggaggtc     840
ggggtggcag taatggaacg acgtcgtctg caaacaatga cagcagcatg acgatgacgg     900
agcaccaggt agctaagcta atggaagaag atatgggatc agcaatgcag tatcttcaag     960
gaaaaggctt atcccttatg ccaatttcct tagccactgc tatttccacc tccacgtgtc    1020
actccatgaa acccaacaac ccactactac tcggcggagg ctccgctatc aacggcggtg    1080
gtgagaccgg cggtggaccg tcttctccta ccttgtcagc ttccactgtt cagtcagcta    1140
cgatgggcaa tggcgggact tgagttagtc ctcctatccc aatgtgactc actgttttta    1200
tttagaatca atttgatttt atatttctcg ttttatacta ttatggaatt atttatagtc    1260
atattaatat tgatgacttg tttttagaaa                                     1290
```

<210> 389
<211> 2210
<212> DNA
<213> Triticum aestivum

<400> 389

```
cacgagggca agccaaagcc tccgcctctc tcctcctttt ccgcccaaac aaaccgcccc      60
ctcccctccc gtcgccctca cctccgccct cgccggcagt agtatacgcc cgccaccagc     120
tccgacgacc cgcgccttcc cccccgctcg cccgccgtcg tctcccctgc ccgctccccc     180
gtccctccgc cgcgggtttg ttctagaggg tgtatctgaa gttctctcag catccgagtg     240
cgcagctttg tacaaaagga accattcagg ccatctcaga ttgtcttcat ggatgaatga     300
taggatcacc agttgctgtc actttaagta tctacaagtc actcttttca taatatttct     360
gagcagaggt tggggagtca agttgtaagg gtgtaaagat ggactactct aatggttctt     420
tctttccttc atggcctggc aattccgctt ccgagaatta tagctttgtt gatggttcag     480
tggaatcata tgcagaagaa ggaagtatgc cacctacagg ctatttcaga gctagatcaa     540
atcagaattt aacatttgat gagcatgaac agaaccctgc tatgcttgca aatgggtgct     600
tgccgtacaa cacccagact gatctattat ctggtgagat tctgtcagag gacaaacatt     660
ccaacagcct tatggagctt tccacaactt cagaacaatg gcagtctgca aagtaattta     720
atcccaccag ggactcttca gtgcacttca acacctggaa catttgaccc gcagttggat     780
acccctggcc ttctagaact tcctcatgcc ttgtccagtt caattgaaag caatggtagt     840
gaagtttcag cttttcttgc tgatgtacat gctgtttctt cagcctcaac tctctgttcg     900
acattccaaa atgttccttc ctacatggag ccagtaagcc tagaagcttt cagttttcaa     960
gggatacaaa atgctcctat gttcaacaat acaagtcatt caaatgggaa acctgtcagt    1020
atttgatgag gcaaccatgg catcactaca tgatagcaaa gaatttctca gtggtagcat    1080
ctcatctttt ggtacggccg agcagtcaca actagctggt agtggtttga aggctgaaca    1140
acaggaacaa aatgcgatgt gcaatattcc actccctttc gcttctggta gtcagatggc    1200
agtgagtgaa gcacaagggg caatgattcc ttcaaagata agctcaacga tccataacaa    1260
taaaagtgag taccctgtcc ctatcagcca ttctgctgat gcgcagaaca aggcaaattc    1320
agctaatgga aacagtgcca gtgctaagcc acgagcaagg gctcgtcgtg gacaggcaac    1380
tgaccctcat agtattgctg aacggcttcg cagagagaag atctcagaga ggatgaaaaa    1440
tctccaagac cttgtaccaa actcaaataa ggcagataaa tcatcaatgc tcgatgaaat    1500
aattgattat gtgaaatttc ttcagcttca ggtgaaggtc ttaagcatga gtaggctagg    1560
agctcccggg gcagttcttc ccctccttgc agaatctcaa actgagggcc gtagcaattc    1620
acctctatca tctccaaccg cttcacaagg gcttctggat gcagcaggcc cagaagacag    1680
cttggtcttt gagcaagaag ttataaagct gatggaaaca agcatcacaa atgcaatgca    1740
gtaccttcag aacaaggccc tctgcctgat gcctatcgct cttgcttcag ccatatccaa    1800
ccagaaaggc acttctgcag ctgcaatccc tcctgaaagg tgaaaacaga aagcacatca    1860
tgctcctccc atcggcccgc ggaaacaact gtcgaaatgt gctagagttc atagaagttg    1920
tgagaaaaca aaaatccgag gctgaaggat aggaagttat gcgactaata gtacaagctt    1980
atggtataat ctagtagctt actccaagaa acctatactt tttctcttct ctgttgcaca    2040
tcggttggtg tgtattcttt tgtttttttgg acggcattct aactgggtac aagtgtctgg    2100
caccttgggt tgtacatgga tctatgaggg ttattaccga ctgttcatga tgttatatga    2160
tgatcaaagc agacctagga aaactcggtt gctttgggtt tcacttggtt                2210
```

<210> 390
<211> 1458
<212> DNA
<213> Vitis vinifera

<400> 390

```
atggatgagt atctggatca cttgttttca tccacatcat ggtcagatgt gaatgtgaag      60
gagggatcat cttggatttg tggtgagcct agccaaacaa atgggatgct ttcagattca     120
attggaatat atgaaggtga taaaaaaaac tcacctgttg gcatgactaa ttcaaacctt     180
atgatcgagg gcttggttac tcaagatact tcatctattg ttcttggtgg agagtctgat     240
catggtcttg gcaagggctt acttttggaa gaagctccac ggcagcaaga ccttcaaaac     300
actgaaggca attcctcctt gaatggagca gtgaatggca gctcagaagt tggatacgtg     360
ggcctgcaac taaatactcc tacttcaacc ccatgctcac tggatcttgg ctctcccaag     420
cagctttcgc tgattggtgg catgtctagg tcatctcgtc ctttcactga gctggatcat     480
gttgggtgca atggtaatga gccatctgat tttcagagat cagttggaaa ttttcaagca     540
ttgcctccaa ttccacagtt gtggtctcaa ccatcttacg gggtggttc ctccttgtct     600
cctgttatgg gagaatacaa gatgcagggc tttggtctgc aaggagaata tgtggataat     660
```

gaaatggata tcatgaggaa cagatacgtt ggggatgaaa ttctgcaact tgataacatt 720
tcttcagcaa tccccataaa ggggaaagaa gagcagcaca ctcatccttt ctctccttct 780
gcggttgggc cccacatgac aatgacagca tctggattac agtctttgcc acagactacg 840
gtaggtactg cttctggtgg ctgtaatgga actggaaagc cgcgtgtaag ggctcgtcga 900
ggccaggcca ctgatcctca cagtattgct gaaaggcttc ggagagagaa aattgctgaa 960
aggatgaaga acctgcaaga acttgttccc aattccaata agacggacaa agcatctatg 1020
ctcgacgaaa tcattgagta cgtcaaattt cttcagctcc aggtcaaggt actaagcatg 1080
agtaggctgg gggcagcaga ggcagttgtt cctctcatca cagatggcca agctgagggc 1140
tctaagggtc tgtcactttc accctcggct ggtcaagctg aagatatttg tcaatctcct 1200
gatcaaattg cctttgagca agaagtagtg aagttgatgg agtccaacgt gaccatggcc 1260
atgcaatatc ttcaaagcaa aggtctctgc cttatgccaa ttgctcttgc tacagccata 1320
tccagtggaa aggcagcatc atcaggtact gggtccgatg agggaaagaa cgcagcaaca 1380
ccagtagcag caacaccagt agcaacagct tacctggcat tgggacacat catacatctt 1440
ctgatggcaa tgttctga 1458

<210> 391
<211> 1257
<212> DNA
<213> Vitis vinifera

<400> 391

atgctctcta ctctcccttc ttggtctgac ctccccgcaa accctaaatc tccctgggaa 60
ctcaatgctt ccaaccctat ctccatgcct tccaacaagt ctagggattt gtccgacgat 120
accacgccgt ctaatccgga caacgttcag ttcgctttcg acgagtccgc catgttggcc 180
tccaagctcc gccagcacca gatcagcggg aactcgtcgg cggccaagtc ggcgttgatg 240
cttcagcagc agttgctttt gtcacgaggc gtcgccatgg gtaggtcccc gtcgaatggg 300
tccggcgccg gtgagtccgg ccttcttcaa ctgcctttgt cacttagcaa cggggattct 360
tgcctcgttg accgatcaca aaacgacgtc gttgatgggt cctcctcctt caaatccccc 420
aatcagggag gagacggttc agttcaagct ctctacaatg gcttcgccgg agctcttcac 480
ggctccggtc aagcctccaa ccaagctcag aatttccacc atcctcaggg tggatcaatg 540
caggcacaga actacggagc tccggcgact agggtgaggg caagaagggg tcaggcaact 600
gacccacaca gcatcgctga gagattacgt agagagagaa ttgcagagag aatgaaggca 660
ctgcaggaac tggttcccaa tgccaacaag acggacaagg cttcaatgct ggatgagatc 720
attgactatg tcaaattcct gcagctccaa gtgaaggtac tcagcatgag caggttgggc 780
ggtgccgcag ctgttgctcc cctcgttgct gatatgtcct ctgagggagg tggtgactgt 840
atacaggcta gtgggacaag cggccctaca ggaggaaggg caacgaacgg aacacaaaca 900
accacatcaa acgacagcct cacagtgacg gagcaccagg tggccaaact catggaggag 960
gacatgggtt ccgccatgca gtatcttcaa ggcaaaggct tatgccttat gcccatctcc 1020
ctcgccactg ccatctccac cactacctgc cactccagga accccatggt ggctgctgcc 1080
gccgtcgccg cctccaacat caacaacggc agccacactc acccactcct acctaattcc 1140
aacgccgatg gcccctcctc ccccagcatg tccgtcctga ccgtgcagtc agccaccatg 1200
gggaacggcc tggccgacgc gcccgtaaaa gacgctgcct ccgtttccaa gccctga 1257

<210> 392
<211> 1010
<212> DNA
<213> Vitis vinifera

<400> 392

```
tcttccttct cacggaagct gagaggccga gagctgagac ttcactccaa tcaaggccat      60
ctcttccatc tccgaaaccc taacccacct tccgtacggt tccgactcgt ccgagtttga     120
gtgatggcga gcaatccctc ggaagctcca gcggacgatt ttctcgagca aatcctcgga     180
attcccacct accccgcagc tgaccctaat ttggctgcta atgacgtgaa tttggccgct     240
cctatggtgc ttcagctcgg ctccggcgaa ggctctggcc acatcgccgg cgggtaccag     300
ggaaccatgt ttccgttagg gttgaggttg gagcagggaa agagcagctt tctgaagccc     360
gaagacgcgt ctggcagtgg caagcgcttt cgtgaggagg ttattgatgg tagagcttct     420
accgtgaaaa atgctttcca tggtcaacca atgcaggcta ccgttgctgc agcaccacat     480
cctcctgcaa ttcgcccaag ggtgcgggcg agacgtggac aggccacaga tccacacagc     540
attgctgagc ggttacgcag agaaagaata gcagagagaa ttagagcact gcaggaacta     600
gttcctagtg tcaacaagac ggatagagct gcaatgcttg atgaaattgt ggattatgtg     660
aaattcctaa ggctccaagt aaaggtcttg agcatgagta gattgggcgg agctggtgca     720

gttgcaccac ttgtaacaga cattccatta gcatcagtcg aggaagaagc aagtgaaggg     780
ggaagaaatg aaccggcatg ggagaaatgg tcaaatgacg gtacagaacg acaagttgct     840
aagctcatgg aagagaatgt tggggctgca atgcagttcc ttcagtccaa ggcactctgc     900
atcatgccca tctctctcgc atcagccatt taccactctc agcagccgga cacaactcca     960
ctgatcaagc cccagacaaa tcccccatcg taaaacctct ccccactccc                1010
```

<210> 393
<211> 1599
<212> DNA
<213> Vitis vinifera
400 393

<400> 393

```
gggagcaagg aaaccgaagc agggcatttc ccaaacatct cttaccgatt tatcggacga      60
cctctctgtg acctctttct ccaacaattt gaccttttttg ctaaacccga aacaatcct    120
catctcttct ttgcttctcg ctggaaaatc tattttccgt tcccgtccat tctctagcca     180
tggccaccaa cccgcccgaa ggttacgccg atgatttcct ggaacaaatc ctcgcaattc     240
cctcctatcc tggacacgac cctaatatgg tgggaacggg ttctaccatg gtgctgcagc     300
tgagttcagg cgacggctca ggccacgtcg ccggaggtgg cttccagggg ccggtctttc     360
cgttaggtct gagcttggag agcgggattc cggcgacaca ggtggcgcca ggaagtggcg     420
agcggtttcg agatgacgtt gatgccagag gttcttcagg gaggagcgag agagagtcgg     480
tgcatttgga tggtttgttt ccggcgtatg gacatgtaca gtctctctcc gtccgacccg     540
ccgttcctca agttcaccag gcttttcatg gccagccaac ccctgtccca attactgctg     600
cgccacaccc gccagctatc cgcccaaagg tgcgtgctcg gcgcggacaa gccactgatc     660
ctcacagcat tgctgagcgg ctacgtagag agaggatagc agaaagaatg aaggctttgc     720
aagaacttgt ccctagctcc aacaagacgg atagggctgc aatgcttgat gaaattgtgg     780
actatgtgaa gttcctaagg ctccaagtca aggttttgag catgagtaga ctgggaggtg     840
ctggtgcagt ggcacaactc gtggctgaca tcccattgcc agcagttgag ggagaaacgg     900
gtgaaggtgg aagcaaccag caagcctggg ataaatggtc aaatgatggc acagaacgag     960
aagtagcaaa gctcatggaa gaagatgttg gagctgctat gcagttcctc caatccaaag    1020
cactgtgcat catgcccata tcacttgccg cagcaatata ccctgcacac caaactgata    1080
cccccacgct catcaagcct gagccaaatg ctccatccta gacccctcga aaatgcacgt    1140
gcctgttgac aactcccatt gacccctagt tggctaatcc caaattcctt agtatcctga    1200
atccatatcc taaagtgttc ctcactatcc cttttggcaa tgcaatcata gtcagaagcc    1260
atagtcttgc ttttgcctct ttttattctg agttttgtca aagaaagagt gaatcatgct    1320
ttgggaaaaa agaaaaggca ctttcatcac ttcttgccat gctcgagtga agggggaaaa    1380
ttttcaaagc tatattgata tgctactcct tttcatttgt gctgcttgct ggatggccag    1440
aacacttgtg gtggggactc tctttggttg atgctgtggt cttgtaaagc ggatggatg     1500
actacgattc catgtaatta aaatgaattt gaagaaagct gaaatggata atggaaagtc    1560
aatttcaatg ttatattgca ttatttccag tcttggagg                            1599
```

<210> 394
<211> 1604
<212> DNA
<213> Zea mays

<400> 394

```
gtcggcggca gctggagagc gaggccgagc gaccagttac agttgccggc gggcaaagtg      60
ggccacttgg tgccaggaca tggccgggca accgccaccg cagggccccg aagacgattt     120
cttcgaccag ttcttctcca tgacggccgg cggctcctac cccggcgcaa ccgcgggcgg     180
cggccgcgca ccgggtgacc agccgttttc ccttgcgctc agcctcgacg ccgcggccgc     240
tgaggcttcc ggaagcggga agcacgccga cggtggcaag gcggaccggg aggctataca     300
gctccctggg ctcttcccgc cggcgttcgg cggcggtgtg cagccacccc acctccgcgc     360
caccccgcct acacaggtgt tccacgcgca gcagccgaag caaggcggtg cggcagtcgg     420
gccacaaccg ccggcaccga ggccaaaggt gcgggcgcgg cgtgggcagg cgaccgaccc     480
ccacagcatc gcggagaggc taagaagaga gagaatagca gaaaggatga gggccctaca     540
ggaattggtg cccaatacaa acaagacaga tagagcagct atgctagatg agatcctaga     600
ttatgtgaag tttctgaggc ttcaagtcaa ggttctaagc atgagcaggc tgggtggtgc     660
tggtgctgtg gcacagctgg ttgctgatat cccactttca gttaaggggg aagcaagtga     720
tagtgggagc aaacagcaga tttgggagaa gtggtcaacg gacggcacag aaagacaggt     780
cgcaaagctg atggaagaag acattggggc agcgatgcaa ttcctccagt ccaaagcact     840
ctgcatgatg cccatctcgc tcgccatggc aatctatgac acgcaacatt cgcaggacgg     900

acaaccagta aaaccagaac ccaacactcc ttcctagtat agtggcagca agcgtaactt     960
gcatcctttc ggggctgtta gcaggcgtta acctaccaat caaagcgtaa gatagaaatg    1020
ccggcttcga ttcactaaca aggaggaggc agggaaaaaa gacctaaaag atcctctcca    1080
cttgaagttg taccaaatgg tatatgatat cgcctttccc tttccgatct tttgttcaaa    1140
ggtgctttaa ttgcaaggat atggtaagca ataacctgct catgctcctc catctttcaa    1200
tgccccctat gagtgtgagt gatggagaga gggagctgct aaaagagtct ggcatgctct    1260
cataatgttt ctttccgggt ctgtagtgca ctagctagcc atacactttg tggtgggggg    1320
gctgctgctt ttctggtagt gggtgtatat ggactaatca aaagggactg atgtatgagt    1380
gtgtctgcgg cctgcgcaaa tggttgacaa agtttagata caggatgaaa tggaagatga    1440
tgttgatagc gatgttcttc cttctccatt tttccaaagt ggaggtccga ggcaggcttt    1500
ggtcaatgga ggctgagcgt gcgtttcagc gttgctagtg gatctgctat ctgcgttgaa    1560
tactttattt atatatggat ccacactctt gataagaaca ttcg                     1604
```

<210> 395
<211> 1260
<212> DNA
<213> Zea mays

<400> 395

```
gcttcacact tccccagcag agcaggcaaa ccgctcccag tcccagtctc tcacctgagg      60
tgactggcgt ctcttgtcgc aagccgccag aagcttcggc cctgacgcg tgggggcccc     120
gtccgccccc gcccccgtcc ccacgttgcc ggccatggcg gggcagccac cgccgtccgg     180
ggcctccgag gacgacttcc tcgagcactt cttcgccttc ccctccgcgg cctccgccgg     240
cgctgccggg ggccacgcgg gcgccggtgt tggcggggac caccccttcc ccctcgccct     300
cagcctcgac gccgccgccg aggccaagcc ggaccgtgat cccgtgcagc tcgccggcct     360
cttcccgccg gtgttcgctg gcgccggcgg cgtgcaccag ccgcacctcc gcggcccacc     420
gcctccgcag atgttccagg cgcagccgaa gccgggcgag ggaggcatgg cgccgcagcc     480
gccagccccg cggcccaagg tgcgcgcgcg gcgggggcag gccaccgatc cccacagtat     540
tgcggagagg ctaaggagag agagaattgc agaaaggatg agggcattgc aggaattagt     600
ccccaacaca aacaagacag atagggcagc catgctagat gagatccttg attatgtgaa     660
gtttcttagg cttcaagtaa aggttctgag tatgagcaga ctgggcggtg ctggcgcggt     720
tgcgcagctg gttgctgata ttccactctc agttaagggc gaggcaggcg acggcggggg     780
ggcgccgcag cagcagcagc agcagcacgt gtgggagaag tggtcgacgg acggcacgga     840
gaagcaggtg gcgaagctga tggaggagga catcggggcg gcaatgcagt tcctccagtc     900
caaggcgctg tgcatgatgc cggtctcgct cgcgatggcg atttacgaca cccagcaccc     960
cctggacggc cacggccact cgctgaagcc cgagcccaac gcgtcatcct agtacagtaa    1020
cgtcgtcctt agcgtgcctt ccaattccaa cacccacccc ccttctccct aaaaagtact    1080
gttaggcgcc gatattatcc ggaagaaaaa aagaagtgta tgatataccg agcgtgcgtt    1140
tccagtgtgc tgacccgtgg gataggaaag gaccgaaaat tgttggtata tgatgattat    1200
gtagcctcct gtttgcctgt tctgtttcaa atcaaagcaa tgctcatgca tccaggtttc    1260
```

<210> 396
<211> 417

<212> DNA
<213> Oryza sativa

<400> 396

```
atggcggagg agaagcacca ccaccacctg ttccaccaca agaaggacga cgagccggcc      60
accggagtag actcctacgg cgagggcgtc tacacgtcgg agacggtgac caccgaggtg     120
gtcgccggcg gccaggacga gtacgagagg tacaagaagg aggagaagca gcacaagcac     180
aagcagcacc tcggcgaggc cggcgccctc gccgccggcg ccttcgccct gtatgagaag     240
cacgaggcga agaaggaccc ggagaacgcg cacaggcaca agatcacgga ggagatcgcg     300
gccacggcgg cggtcggcgc cggcggctac gccttccacg agcaccacga gaagaagaag     360
gaccacaaga gcgccgagga gtccaccggc gagaagaagc accacctctt cggctga        417
```

<210> 397
<211> 138
<212> PRT
<213> Oryza sativa

<400> 397

```
Met Ala Glu Glu Lys His His His His Leu Phe His His Lys Lys Asp
1               5                   10                  15
Asp Glu Pro Ala Thr Gly Val Asp Ser Tyr Gly Glu Gly Val Tyr Thr
            20                  25                  30
Ser Glu Thr Val Thr Thr Glu Val Val Ala Gly Gly Gln Asp Glu Tyr
        35                  40                  45
Glu Arg Tyr Lys Lys Glu Glu Lys Gln His Lys His Lys Gln His Leu
    50                  55                  60
Gly Glu Ala Gly Ala Leu Ala Ala Gly Ala Phe Ala Leu Tyr Glu Lys
65                  70                  75                  80
His Glu Ala Lys Lys Asp Pro Glu Asn Ala His Arg His Lys Ile Thr
                85                  90                  95
Glu Glu Ile Ala Ala Thr Ala Ala Val Gly Ala Gly Gly Tyr Ala Phe
            100                 105                 110
His Glu His His Glu Lys Lys Lys Asp His Lys Ser Ala Glu Glu Ser
        115                 120                 125
Thr Gly Glu Lys Lys His His Leu Phe Gly
    130                 135
```

<210> 398
<211> 3246
<212> DNA
<213> Oryza sativa

<400> 398

```
aatccgaaaa gtttctgcac cgttttcacc ccctaactaa caatataggg aacgtgtgct     60
aaatataaaa tgagacctta tatatgtagc gctgataact agaactatgc aagaaaaact    120
catccaccta ctttagtggc aatcggcta aataaaaaag agtcgctaca ctagtttcgt    180
tttccttagt aattaagtgg gaaaatgaaa tcattattgc ttagaatata cgttcacatc    240
tctgtcatga agttaaatta ttcgaggtag ccataattgt catcaaactc ttcttgaata    300
aaaaaatctt tctagctgaa ctcaatgggt aaagagagag attttttta aaaaaataga    360
atgaagatat tctgaacgta ttggcaaaga tttaaacata taattatata attttatagt    420
ttgtgcattc gtcatatcgc acatcattaa ggacatgtct tactccatcc caatttttat    480
ttagtaatta aagacaattg acttattttt attatttatc tttttcgat tagatgcaag    540
gtacttacgc acacactttg tgctcatgtg catgtgtgag tgcacctcct caatacacgt    600
tcaactagca acacatctct aatatcactc gcctatttaa tacatttagg tagcaatatc    660
tgaattcaag cactccacca tcaccagacc acttttaata atatctaaaa tacaaaaaat    720
aattttacag aatagcatga aaagtatgaa acgaactatt taggtttttc acatacaaaa    780
aaaaaaagaa ttttgctcgt gcgcgagcgc caatctccca tattgggcac acaggcaaca    840
acagagtggc tgcccacaga acaacccaca aaaaacgatg atctaacgga ggacagcaag    900
tccgcaacaa cctttaaca gcaggctttg cggccaggag agaggaggag aggcaaagaa    960
aaccaagcat cctccttctc ccatctataa attcctcccc ccttttcccc tctctatata   1020
ggaggcatcc aagccaagaa gagggagagc accaaggaca cgcgactagc agaagccgag   1080
cgaccgcctt ctcgatccat atcttccggt cgagttcttg gtcgatctct tccctcctcc   1140
acctcctcct cacagggtat gtgcctccct tcggttgttc ttggatttat tgttctaggt   1200
tgtgtagtac gggcgttgat gttaggaaag ggatctgta tctgtgatga ttcctgttct   1260
tggatttggg atagaggggt tcttgatgtt gcatgttatc ggttcggttt gattagtagt   1320
atggttttca atcgtctgga gagctctatg gaaatgaaat ggtttaggga tcggaatctt   1380
gcgattttgt gagtaccttt tgtttgaggt aaaatcagag caccggtgat tttgcttggt   1440
gtaataaagt acggttgttt ggtcctcgat tctggtagtg atgcttctcg atttgacgaa   1500
gctatccttt gtttattccc tattgaacaa aaataatcca actttgaaga cggtcccgtt   1560
gatgagattg aatgattgat tcttaagcct gtccaaaatt tcgcagctgg cttgtttaga   1620
tacagtagtc cccatcacga aattcatgga aacagttata atcctcagga acaggggatt   1680
ccctgttctt ccgatttgct ttagtcccag aatttttttt cccaaatatc ttaaaaagtc   1740
actttctggt tcagttcaat gaattgattg ctacaaataa tggtgcaaat caggtctata   1800
tgattgattt tgggctggcc aagaagtata gagactcatc aactcatcag catattccgt   1860
atagagaaaa caaaaatttg acaggaactg ctagatacgc aagcatgaat actcatcttg   1920
gcattgaaca aagtcgaagg gatgatttgg aatcgctggg ttatgtttta atgtacttct   1980
taagaggaag tctcccttgg caggggctga aagcaggcac taagaaacag aagtatgaga   2040
agatcagtga gaagaaagta tcaacatcaa tagagacctt gtgtagggga tatcctgcag   2100
agtttgcatc atattttcat tactgtcgat cactaagatt tgatgataaa ccagattatg   2160

cttatctgaa gagaattttc cgtgatcttt tcattcgtga agggtttcaa tttgattata   2220
tatttgactg gaccattttg aaatatcagc aatcacagct tgccaatcct ccatctcgtg   2280
ctcttggtgg tactgctggg ccaagctcag ggatgcctca tgctcttgtt aatgttgaga   2340
ggcaatcagg tggagatgaa ggtcgaccaa ctggttggtc ttcatcaaat cttacacgta   2400
ataagagcac ggggctgcat ttcaattctg gaagcttatt gaagcaaaaa ggcacagttg   2460
ctaatgattt atccatgggt aaagagttat ccagttctaa tttttttccgg tcaagtggac   2520
cattgaggcg tccagttgtc tctagcatcc gagacccagt gattgcaggg ggtgaacctg   2580
acccctccgg cactctgaca aaagatgcaa gcccgggacc attgcgtaaa gtatccagtg   2640
ctgcacggag gagttcacca gttgtgtcct cagatcacaa gcgcagctcc tctatcaaaa   2700
atgccaacat aaagaattta gagtccaccg tcaagggaat agagggttta agttttcgat   2760
gatgagggac tgcattagta gctgtgcttt gtctcagttc tccgttcact gtaaatttg   2820
gcacaccaac ttggggagta agagttctga tattagttgc tgtcaggaag taccataaag   2880
ctgaattata caattaaaat ttgggatcca atcgcaaaag cacattaagg atatgatggg   2940
gttgcagatc caaactcaca gattccagtt tatgctcgtc catacagtta taggcacttt   3000
ccatattctt ttctttaatc tctgtctctt gcttgttatt gttatgtcgt ggtattcttg   3060
ttgaggtcat gtttgtgaat tgcgaagatg gtcatgtata attgccgaga aatcatgtac   3120
tagtttgttt taaacatgag caaactgtta ttttgttcaa gctactttaa tatcaaaaaa   3180
aaaaaaaaaa gggcggccgc tctagagtat ccctcgaggg gcccaagctt acgcgtaccc   3240
agcttt                                                               3246
```

<210> 399
<211> 52
<212> DNA
<213> Artificial sequence

<220>
<223> primer: prm06442

<400> 399
ggggacaagt ttgtacaaaa aagcaggctt aaacaatggc ggaggagaag ca 52

<210> 400
<211> 46
<212> DNA
<213> Artificial sequence

<220>
<223> primer: prm06443

<400> 400
ggggaccact ttgtacaaga aagctgggtc ggcgacgttg tgatga 46

<210> 401
<211> 546
<212> DNA
<213> Zea mays

<400> 401

```
atggccgact actaccacgg cggcagggcc atgtactcca acactgacga gtgctatgac     60
gccggcaggc acggcggcag cgtgagggcg tactcgcgca ccgacgagta ctacgacaac    120
gtggacgacc gcatgaggag gcccgcgtac ggcgccgacg actgcggcta cggcggcagg    180
cagaccgccg tgtacgccga cgagtactcc cgcggcggcc gctacgaagg ctacggcgtc    240
gagcaggagc acttcaagag ggaggagaag gagcataagc acaaggagcg cctcggcgag    300
gtgggcgccc tcgcgggcgg cgcctttgct ctgtacgagg ggcaccgtgc gaagaaggac    360
ccggcgaacg cgcagaggca caagatcgag gccggcgtgg caacggcggc ggcgctgggc    420
gccggcggct acgcgtacca cgagcaccgc gagcagaagg aggcccacta cgagagcagg    480
cagcaggagc acagggtgcc gcacgggcac gggcacgggc acggctactc ctactactgc    540
gactga                                                               546
```

<210> 402
<211> 181
<212> PRT
<213> Zea mays

<400> 402

```
Met Ala Asp Tyr Tyr His Gly Gly Arg Ala Met Tyr Ser Asn Thr Asp
1               5                   10                  15
Glu Cys Tyr Asp Ala Gly Arg His Gly Gly Ser Val Arg Ala Tyr Ser
            20                  25                  30
Arg Thr Asp Glu Tyr Tyr Asp Asn Val Asp Asp Arg Met Arg Arg Pro
        35                  40                  45
Ala Tyr Gly Ala Asp Asp Cys Gly Tyr Gly Gly Arg Gln Thr Ala Val
    50                  55                  60
Tyr Ala Asp Glu Tyr Ser Arg Gly Gly Arg Tyr Glu Gly Tyr Gly Val
65                  70                  75                  80
Glu Gln Glu His Phe Lys Arg Glu Glu Lys Glu His Lys His Lys Glu
                85                  90                  95
Arg Leu Gly Glu Val Gly Ala Leu Ala Gly Gly Ala Phe Ala Leu Tyr
            100                 105                 110
Glu Gly His Arg Ala Lys Lys Asp Pro Ala Asn Ala Gln Arg His Lys
        115                 120                 125
Ile Glu Ala Gly Val Ala Thr Ala Ala Ala Leu Gly Ala Gly Gly Tyr
    130                 135                 140
Ala Tyr His Glu His Arg Glu Gln Lys Glu Ala His Tyr Glu Ser Arg
145                 150                 155                 160
Gln Gln Glu His Arg Val Pro His Gly His Gly His Gly His Gly Tyr
                165                 170                 175
Ser Tyr Tyr Cys Asp
            180
```

<210> 403
<211> 468
<212> DNA
<213> Zea mays

<400> 403

```
tagataaatc gacggtcggt gcatatatat gagcaagact gccaggcgcg cccggtaaca      60
aatgagaatg gaagccgtca gtaactagtg gtgatggtgt ccacggtggc ggtgcttctt     120
ggcgtctttc ttctggtgat gctcgtggaa ggcaaagccg gcgctgccca cggcggcggc     180
ggcggcgacc ccttccttga tcttgtgcga gtgcgcgtgc tccgggtcct tcttcgcctt     240
ctgcttctcg tgcatagcgt aggctcctgc agcgacggcg ccgagctggc cgagctgctc     300
catgtgcttg tggtgcttct cctccttgcg ccagtcggtc accttggccg cctctcctcc     360
atggtgcttg ttgcccagga agtggtgcgc cattgtttca gtcgttggag atgcgctagt     420
cgacggccgg ttgtgtcttg gggttcgttc gatctgcttg atgatgat                  468
```

<210> 404
<211> 102
<212> PRT
<213> Zea mays

<400> 404

```
Met Ala His His Phe Leu Gly Asn Lys His His Gly Gly Glu Ala Ala
1               5                   10                  15
Lys Val Thr Asp Trp Arg Lys Glu Glu Lys His His Lys His Met Glu
            20                  25                  30
Gln Leu Gly Gln Leu Gly Ala Val Ala Ala Gly Ala Tyr Ala Met His
        35                  40                  45
Glu Lys Gln Lys Ala Lys Lys Asp Pro Glu His Ala His Ser His Lys
    50                  55                  60
Ile Lys Glu Gly Val Ala Ala Ala Ala Ala Val Gly Ser Ala Gly Phe
65                  70                  75                  80
```

```
Ala Phe His Glu His His Gln Lys Lys Asp Ala Lys Lys His Arg His
                85                  90                  95
Arg Gly His His His His
            100
```

<210> 405
<211> 434
<212> DNA
<213> Zea mays

<400> 405

```
atggagcagc tcggccagct cggcgccatc gccgccggcg cgtacgctct ggtgagacca     60
aaaaaaccca acctccatcc ctttggtcgc ctgatcgatg catgccatgg tccagctctt    120
gatctgtgtg tgtctgtgcg tgtgcatcga cgtgtggctg cagcacgaga agcacaaggc    180
caagaaggac ccggagaacg agcacggcca ccgggtcaag gaggaggtgg ccgccgtcgc    240
cgccgtgggc tccgccgggt tcgccttcca cgagcatcac gagaagaagg acgccaagaa    300
gcacgcccac aactgatccg tcgcaggctg cctcagccga agcagcagca catcgtcgcg    360
gttgctgttt catctgttct cccagcctcg tcttcgtcta ctgtgtgccg ggcaggcctt    420
gatttgaggc tttg                                                      434
```

<210> 406
<211> 144
<212> PRT
<213> Zea mays

<400> 406

```
Met Glu Gln Leu Gly Gln Leu Gly Ala Ile Ala Ala Gly Ala Tyr Ala
1               5                   10                  15
Leu Val Arg Pro Lys Lys Pro Asn Leu His Pro Phe Gly Arg Leu Ile
            20                  25                  30
Asp Ala Cys His Gly Pro Ala Leu Asp Leu Cys Val Ser Val Arg Val
        35                  40                  45
His Arg Arg Val Ala Ala Ala Arg Glu Ala Gln Gly Gln Glu Gly Pro
    50                  55                  60
Gly Glu Arg Ala Arg Pro Pro Gly Gln Gly Gly Gly Gly Arg Arg Arg
65                  70                  75                  80
Arg Arg Gly Leu Arg Arg Val Arg Leu Pro Arg Ala Ser Arg Glu Glu
                85                  90                  95
Gly Arg Gln Glu Ala Arg Pro Gln Leu Ile Arg Arg Arg Leu Pro Gln
            100                 105                 110
Pro Lys Gln Gln His Ile Val Ala Val Ala Val Ser Ser Val Leu Pro
        115                 120                 125
Ala Ser Ser Ser Ser Thr Val Cys Arg Ala Gly Leu Asp Leu Arg Leu
    130                 135                 140
```

<210> 407
<211> 417
<212> DNA
<213> Zea mays

<400> 407

```
atggcggagg agaagcacca ccaccaccac ctgttccacc acaagaagga cgaggagcag     60
ctcgccgccg gcgggtacgg cgagtccgcc gagtacacgg aggccacggt gacggaggtg    120
gtgtccacgg gcgagaacga gtacgacgag tacaagaagg aggagaagga gcacaagcac    180
aagcagcacc tcggcgaggc cggcgccatc gccgccggcg ccttcgcact ctacgagaag    240
cacgaggcga agaaggaccc ggagcacgcg caccgccaca agatcgagga ggaggtcgcg    300
gcggcggcgg ccgtcggctc cggcggcttc gccttccacg agcaccacga gaagaagaag    360
gaccacaagg aggccgagga ggccggcggc gagaagaagc accacttctt cggctga      417
```

<210> 408
<211> 138
<212> PRT
<213> Zea mays

<400> 408

```
Met Ala Glu Glu Lys His His His His His Leu Phe His His Lys Lys
1               5                   10                  15
Asp Glu Glu Gln Leu Ala Ala Gly Gly Tyr Gly Glu Ser Ala Glu Tyr
            20                  25                  30
Thr Glu Ala Thr Val Thr Glu Val Val Ser Thr Gly Glu Asn Glu Tyr
        35                  40                  45
Asp Glu Tyr Lys Lys Glu Glu Lys Glu His Lys His Lys Gln His Leu
    50                  55                  60
Gly Glu Ala Gly Ala Ile Ala Ala Gly Ala Phe Ala Leu Tyr Glu Lys
65                  70                  75                  80
His Glu Ala Lys Lys Asp Pro Glu His Ala His Arg His Lys Ile Glu
                85                  90                  95
Glu Glu Val Ala Ala Ala Ala Ala Val Gly Ser Gly Gly Phe Ala Phe
            100                 105                 110
His Glu His His Glu Lys Lys Lys Asp His Lys Glu Ala Glu Glu Ala
        115                 120                 125
Gly Gly Glu Lys Lys His His Phe Phe Gly
    130                 135
```

<210> 409
<211> 396
<212> DNA
<213> Zea mays

<400> 409

```
atgtcggagg agaagcacca ccacttcttc aaccaccaca agaaggacga ggagcagccc     60
gccggcgagt acgggtactc cgagaccgag gtggtcaccg ccaccggcga gggcgagtac    120
gagaggtaca agaaggagga gaaggagcac aagcacaagc agcacctcgg cgaggccggc    180
gccatcgccg ccggcgcctt cgcactctac gagaagcacg aggcgaagaa ggaccccgag    240
cacgcgcaca ggcacaagat cacggaggag gtcgctgccg cggcggccgt cggcgccggc    300
ggctacgtat tccacgagca ccacgagaag aagaaggacc acaaggacgc cgaggaggcc    360
agcggcgaga agaagcacca ccacctcttc ggctga                              396
```

<210> 410
<211> 130
<212> PRT
<213> Zea mays

<400> 410

```
Met Ser Glu Glu Lys His His His Phe Phe Asn His His Lys Lys Asp
1               5                   10                  15
Glu Glu Gln Pro Ala Gly Glu Tyr Gly Tyr Ser Glu Thr Glu Val Val
            20                  25                  30
Thr Ala Thr Gly Glu Gly Glu Tyr Glu Arg Tyr Lys Lys Glu Glu Lys
        35                  40                  45
Glu His Lys His Lys Gln His Leu Gly Glu Ala Gly Ala Ile Ala Ala
    50                  55                  60
Gly Ala Phe Ala Leu Tyr Glu Lys His Glu Ala Lys Lys Asp Pro Glu
65                  70                  75                  80
His Ala His Arg His Lys Ile Thr Glu Glu Val Ala Ala Ala Ala Ala
                85                  90                  95
Val Gly Ala Gly Gly Tyr Val Phe His Glu His His Glu Lys Lys Lys
            100                 105                 110
Asp His Lys Asp Ala Glu Glu Ala Ser Gly Glu Lys Lys His His His
        115                 120                 125
Leu Phe
    130
```

<210> 411
<211> 204
<212> DNA
<213> Zea mays

<400> 411

```
atggagaagc tcggcgagct cggcgccatc gccgccggcg cgtacgccct gcacgagaag     60
cacaaggcca agaaggaccc agagaacgag cacgggcacc gggtcaagga ggaggtggcc    120
gccgtcgccg ccgtgggctc cgccggcttc gctttccacg agcaccacga gaagaaggac    180
gccaagaagc acgcccacaa ctga                                           204
```

<210> 412
<211> 67
<212> PRT
<213> Zea mays

<400> 412

```
Met Glu Lys Leu Gly Glu Leu Gly Ala Ile Ala Ala Gly Ala Tyr Ala
1               5                   10                  15
Leu His Glu Lys His Lys Ala Lys Lys Asp Pro Glu Asn Glu His Gly

            20                  25                  30
His Arg Val Lys Glu Glu Val Ala Ala Val Ala Ala Val Gly Ser Ala
        35                  40                  45
Gly Phe Ala Phe His Glu His His Glu Lys Lys Asp Ala Lys Lys His
    50                  55                  60
Ala His Asn
65
```

<210> 413
<211> 1008
<212> DNA
<213> Zea mays

<400> 413

```
ttgtcacttg ctctccctcc aacaagctaa ttaaggccgg tcatccctct tctagctcgt      60
ttcattatcc atggcggagg agaagcacca ccaccaccac ctgttccacc acaagaagga     120
cgaggagcag ctcgccgccg gcgggtacgg cgagtccgcc gagtacacgg aggccacggt     180
gacggaggtg gtgtccacgg gcgagaacga gtacgacgag tacaagaagg aggagaagga     240
gcacaagcac aagcagcacc tcggcgaggc cggcgccatc gccgccggcg ccttcgcact     300
cgtacgtagt ccgatcgatc cgatcctcct tgagtagtat atatatatac atacatgaac     360
gagaaagaat aatatatata ttaatcgaac gaactgaatg acggtcacct cgtgtgacgt     420
ggacatgcac agtacgagaa gcacgaggcg aagaaggacc cggagcacgc gcaccgccac     480
aagatcgagg aggaggtcgc ggcggcggcg gccgtcggct ccggcggctt cgccttccac     540
gagcaccacg agaagaagaa ggaccacaag gaggccgagg aggccggcgg cgagaagaag     600
caccacttct tcggctgatt gatcctcccg tatcgtcgtc cctccccgtg tgctacgcgt     660
gcgtgtgaga gtgatatcga gcgcccgccg tgttgtgcgc gcgtacgtat gtatgcgctc     720
gtgtgatgca cgaataagcg tggctacgta atctatcgta tgtatacgtg tgtgtatgca     780
tgtgcttgtg tatgatcgtg gtacgaggac cgaaaaaatg tatgcaactc tgatttactt     840
acatgtttag ttgtttacgt ttctccaaaa gtattagtac ccattatgtt atattttact     900
atattatatt ttattgatta ttaaactatg tatggatgaa attactattt caagctggta     960
gctagaccga gataaaactc gtccgtgttt tttaacgggt gtattatt                 1008
```

<210> 414
<211> 88
<212> PRT
<213> Zea mays

<400> 414

```
Met Ala Glu Glu Lys His His His His His Leu Phe His His Lys Lys
1               5                   10                  15
Asp Glu Glu Gln Leu Ala Ala Gly Gly Tyr Gly Glu Ser Ala Glu Tyr
            20                  25                  30
Thr Glu Ala Thr Val Thr Glu Val Val Ser Thr Gly Glu Asn Glu Tyr
        35                  40                  45
Asp Glu Tyr Lys Lys Glu Glu Lys Glu His Lys His Lys Gln His Leu
    50                  55                  60
Gly Glu Ala Gly Ala Ile Ala Ala Gly Ala Phe Ala Leu Val Arg Ser
65                  70                  75                  80
Pro Ile Asp Pro Ile Leu Leu Glu
                85
```

<210> 415
<211> 588
<212> DNA
<213> Zea mays

<400> 415

```
agaaggagca taagcacaag gagcgcctcg gcgaggtggg cgccctcgcg ggcggcgcct      60
ttgctctggt aatatatata atacagttac catatatata tgtgcatgca cgatctatat     120
atgtggtgat gtgttgttga tggaatataa tatgtggaaa ttagtacgag ggcaccgtg      180
cgaagaagga cccggcgaac gcgcagaggc acaagatcga ggccggcgtg gcaacggcgg     240
cggcgctggg cgccggcggc tacgcgtacc acgagcaccg cgagcagaag gaggcccact     300
acgagagcag gcagcaggag cacagggtgc cgcacgggca cgggcacggg cacggctact     360
cctactactg cgactgaaac gactgatcat cccatcgatc gagatatgca tgcagaactc     420
gatatatata cggcccctag cgccgtacac gtgcgtgcgt gtaagtaaat aagagcaaac     480
gtcgtcacgt gtgtgttgct cgatcactac tccatcgatc tacttattat cgtctgcaaa     540
taacgactaa gcgcatgctt ctgcagcgtg taacgatcaa aaaaaaaa                 588
```

<210> 416

<211> 110
<212> PRT
<213> Zea mays

<400> 416

```
Met Glu Tyr Asn Met Trp Lys Leu Val Arg Gly Ala Pro Cys Glu Glu
1               5                   10                  15
Gly Pro Gly Glu Arg Ala Glu Ala Gln Asp Arg Gly Arg Arg Gly Asn
            20                  25                  30
Gly Gly Gly Ala Gly Arg Arg Arg Leu Arg Val Pro Arg Ala Pro Arg
        35                  40                  45
Ala Glu Gly Gly Pro Leu Arg Glu Gln Ala Ala Gly Ala Gln Gly Ala
    50                  55                  60
Ala Arg Ala Arg Ala Arg Ala Arg Leu Leu Leu Leu Leu Arg Leu Lys
65                  70                  75                  80
Arg Leu Ile Ile Pro Ser Ile Glu Ile Cys Met Gln Asn Ser Ile Tyr
                85                  90                  95
Ile Arg Pro Leu Ala Pro Tyr Thr Cys Val Arg Val Ser Lys
            100                 105                 110
```

<210> 417
<211> 939
<212> DNA
<213> Zea mays

<220>
<221> misc_feature
<222> (914)..(914)
<223> n is a, c, g, or t

<400> 417

```
ggtcgacgat ttcgtcggcc caacactagt agcgtagctg agagcctgag acgatggccg     60
actactacca cggcggcagg gccatgtact ccaacactga cgagtgctat gacgccggca    120
ggcacggcgg cagcgtgagg gcgtactcgc gcaccgacga gtactacgac aacgtggacg    180
accgcatgag gaggcccgcg tacggcgccg acgactgcgg ctacggcggc aggcagaccg    240
ccgtgtacgc cgacgagtac tcccgcggcg gccgctacga aggctacggc gtcgagcagg    300
agcacttcaa gagggaggag aaggagcata agcacaagga gcgcctcggc gaggtgggcg    360
ccctcgcggg cggcgccttt gctctgtacg aggggcaccg tgcgaagaag gacccggcga    420
acgcgcagag gcacaagatc gaggccggcg tggcaacggc ggcggcgctg ggcgccggcg    480
gctacgcgta ccacgagcac cgcgagcaga aggaggccca ctacgagagc aggcagcagg    540
agcacagggt gccgcacggg cacgggcacg ggcacggcta ctcctactac tgcgactgaa    600
acgactgatc atcccatcga tcgagatatg catgcagaac tcgatatata tacggcccct    660
agcgccgtac acgtgcgtgc gtgtaagtaa ataagagcaa acgtcgtcac gtgtgtgttg    720
ctcgatcact actccatcga tctacttatt atcgtctgca aataacgact aagcgcatgc    780
ttctgcagcg tgtaacgatc catcactgtt actatatcta atgatgcatg catgcaatta    840
agctatatat gtaaaaaact atgcgtgtaa aaaaaaaaaa aaaaaaaaaa aaaaaaaaaa    900
aaaaaaaaaa aaanccaacc taaaaaaaaa aaaaaaaaa                           939
```

<210> 418
<211> 181
<212> PRT
<213> Zea mays

<400> 418

```
Met Ala Asp Tyr Tyr His Gly Gly Arg Ala Met Tyr Ser Asn Thr Asp
1               5                   10                  15
Glu Cys Tyr Asp Ala Gly Arg His Gly Gly Ser Val Arg Ala Tyr Ser
            20                  25                  30
Arg Thr Asp Glu Tyr Tyr Asp Asn Val Asp Asp Arg Met Arg Arg Pro
        35                  40                  45
Ala Tyr Gly Ala Asp Asp Cys Gly Tyr Gly Gly Arg Gln Thr Ala Val
    50                  55                  60
Tyr Ala Asp Glu Tyr Ser Arg Gly Gly Arg Tyr Glu Gly Tyr Gly Val
65                  70                  75                  80
Glu Gln Glu His Phe Lys Arg Glu Glu Lys Glu His Lys His Lys Glu
                85                  90                  95
Arg Leu Gly Glu Val Gly Ala Leu Ala Gly Gly Ala Phe Ala Leu Tyr
            100                 105                 110
Glu Gly His Arg Ala Lys Lys Asp Pro Ala Asn Ala Gln Arg His Lys
        115                 120                 125
Ile Glu Ala Gly Val Ala Thr Ala Ala Ala Leu Gly Ala Gly Gly Tyr
    130                 135                 140
Ala Tyr His Glu His Arg Glu Gln Lys Glu Ala His Tyr Glu Ser Arg
145                 150                 155                 160
Gln Gln Glu His Arg Val Pro His Gly His Gly His Gly His Gly Tyr
                165                 170                 175
Ser Tyr Tyr Cys Asp
            180
```

<210> 419
<211> 348
<212> DNA
<213> Lycopersicon esculentum

<400> 419

```
atggaggagg agaaacacca ccaccaccac ctgttccacc acaaggacaa ggcggaggag     60
ggccccgtcg actacgaaaa agaaatcaaa caccataaac atctcgagca aatcggtaaa    120
cttggcactg ttgctgccgg tgcctacgcc ttgcatgaga aacatgaggc aaagaaagat    180
ccagaacatg cacacaaaca caagatagag gaagagatag cagcagctgc tgcagttggg    240
gcaggtggat ttgcattcca tgagcatcat gagaaaaaag atgccaagaa agaagaaaaa    300
aaaaagctga gggggacac caccatctct tctaaattgt tattttag                  348
```

<210> 420
<211> 115
<212> PRT
<213> Lycopersicon esculentum

<400> 420

```
Met Glu Glu Glu Lys His His His His His Leu Phe His His Lys Asp
1               5                   10                  15
Lys Ala Glu Glu Gly Pro Val Asp Tyr Glu Lys Glu Ile Lys His His
            20                  25                  30
Lys His Leu Glu Gln Ile Gly Lys Leu Gly Thr Val Ala Ala Gly Ala
        35                  40                  45
Tyr Ala Leu His Glu Lys His Glu Ala Lys Lys Asp Pro Glu His Ala
    50                  55                  60
His Lys His Lys Ile Glu Glu Glu Ile Ala Ala Ala Ala Ala Val Gly
65                  70                  75                  80
Ala Gly Gly Phe Ala Phe His Glu His His Glu Lys Lys Asp Ala Lys
                85                  90                  95
Lys Glu Glu Lys Lys Lys Leu Arg Gly Asp Thr Thr Ile Ser Ser Lys
            100                 105                 110
Leu Leu Phe
        115
```

<210> 421
<211> 429
<212> DNA
<213> Lilium longiflorum

<400> 421

```
atggccgagg agcaccacaa gcaccacctc ttccatcaca acaaggagag caacccggag     60
gtggtcgtct ccgagaccaa ctacatcacc gacggcttaa actccaccta cagcacatcc    120
agcgatgtat acggtggcaa ccagtcacag gccaactacg aggactacga gaaagagaag    180
aagcacatca agcataagga gcatctcggc gagatggcca cggccggcgc cggtgctttt    240
gccctttacg agaagcacga ggcaaagaag gaccccgagc acgcccacag gcacaaattg    300
gaggaggaga ttgccgcagc tgcagctgcg ggggctggag ggtacacctt ccatgagcac    360
cacgagaaga aaaccttgaa gaaggagaat gaggaagtcg aggggaagaa gcaccacttc    420
ttcggttaa                                                            429
```

<210> 422
<211> 142
<212> PRT
<213> Lilium longiflorum

<400> 422

```
Met Ala Glu Glu His His Lys His His Leu Phe His His Asn Lys Glu
1               5                   10                  15
Ser Asn Pro Glu Val Val Val Ser Glu Thr Asn Tyr Ile Thr Asp Gly
            20                  25                  30
Leu Asn Ser Thr Tyr Ser Thr Ser Ser Asp Val Tyr Gly Gly Asn Gln
        35                  40                  45
Ser Gln Ala Asn Tyr Glu Asp Tyr Glu Lys Glu Lys Lys His Ile Lys
    50                  55                  60
His Lys Glu His Leu Gly Glu Met Ala Thr Ala Gly Ala Gly Ala Phe
65                  70                  75                  80
Ala Leu Tyr Glu Lys His Glu Ala Lys Lys Asp Pro Glu His Ala His
                85                  90                  95
Arg His Lys Leu Glu Glu Glu Ile Ala Ala Ala Ala Ala Ala Gly Ala
            100                 105                 110
Gly Gly Tyr Thr Phe His Glu His His Glu Lys Lys Thr Leu Lys Lys
        115                 120                 125
Glu Asn Glu Glu Val Glu Gly Lys Lys His His Phe Phe Gly

            130                 135                 140
```

<210> 423
<211> 429
<212> DNA
<213> Saccharum officinarum

<400> 423

```
atggcggagg agaagcacca ccaccacctg ttccaccaca agaaggacga ggaggagcag     60
gtggagcagc ccgccggcgg cggcgggtac ggcgaggccg ccgagtacac ggagaccacg    120
gtgacggagg tggtgtccac gggcgaggac gagtacgaca agtacaagaa ggaggagaag    180
gagcacaagc acaagcagca cctcggcgag gccggcgcca tcgccgccgg cgccttcgca    240
ctctacgaga agcacgaggc gaagaaggac ccggagcacg cgcaccgcca caagatcgag    300
gaggaggtcg cggccgcggc ggccgtggga tccggcggct tcgcgttcca cgagcaccac    360
gagaagaaga aggaccacaa ggaggccgag gaggccggcg gcgagaagaa gcaccacttc    420
ttcggctga                                                            429
```

<210> 424
<211> 142
<212> PRT
<213> Saccharum officinarum

<400> 424

```
Met Ala Glu Glu Lys His His His His Leu Phe His His Lys Lys Asp
1               5                   10                  15
Glu Glu Glu Gln Val Glu Gln Pro Ala Gly Gly Gly Gly Tyr Gly Glu
            20                  25                  30
Ala Ala Glu Tyr Thr Glu Thr Thr Val Thr Glu Val Val Ser Thr Gly
        35                  40                  45
Glu Asp Glu Tyr Asp Lys Tyr Lys Lys Glu Glu Lys Glu His Lys His
    50                  55                  60
Lys Gln His Leu Gly Glu Ala Gly Ala Ile Ala Ala Gly Ala Phe Ala
65                  70                  75                  80
Leu Tyr Glu Lys His Glu Ala Lys Lys Asp Pro Glu His Ala His Arg
                85                  90                  95
His Lys Ile Glu Glu Glu Val Ala Ala Ala Ala Ala Val Gly Ser Gly
            100                 105                 110
Gly Phe Ala Phe His Glu His His Glu Lys Lys Lys Asp His Lys Glu
        115                 120                 125
Ala Glu Glu Ala Gly Gly Glu Lys Lys His His Phe Phe Gly
    130                 135                 140
```

<210> 425
<211> 777
<212> DNA
<213> Zea mays

<400> 425

```
tgtcgatcca attgtcactt gctctccctc caacaagcta attaaggccg gtcgtcatcc      60
ctcttctagc tcgttttatt atccatggcg gaggagaagc accaccacca ccacctgttc     120
caccataaga aggacgagga gcaggaggag cagctcgccg gcggcgggta cggcgagtcc     180
gccgagtaca cggaggccac ggtgacggag gtggtgtcca cgggcgagaa cgagtacgac     240
gagtacaagg aggagaagca gcataagcac aagcagcacc tcggcgaggc cggcgccatc     300
gccgccggcg ccttcgcact ctacgagaag cacgaggcaa agaaggaccc ggagcacgcg     360
caccgccaca agatcgagga ggaggtcgcg gcggcggcgg ccgtcggctc cggcggcttc     420
gccttccacg agcaccacga gaagaagaag gaccacaagg acgccgagga ggccggcggc     480
gagaagaagc accacttctt cggctgattg atccctcccg tatcgtcgtc cctccccgtg     540
tgctacgcgt gcgtgtgtga gagtgatatc gagcgcccgc cgtgttgtgc gcgcgtacgt     600
atgtatgcgc tcgtgtgatg cacgaataag cgtggctacg taatctatcg tatgtatacg     660
tgtgtgtatg catgtgcttg tgtatgatcg tggtacgagg accgaaaaaa tgtatgcaac     720
tctgatttac ttacatgttt agttgtttac gtttctccaa aaaaaaaaaa aaaaaaa        777
```

<210> 426
<211> 140
<212> PRT
<213> Zea mays

<400> 426

```
Met Ala Glu Glu Lys His His His His His Leu Phe His His Lys Lys
1               5                   10                  15
Asp Glu Glu Gln Glu Glu Gln Leu Ala Gly Gly Gly Tyr Gly Glu Ser
            20                  25                  30
Ala Glu Tyr Thr Glu Ala Thr Val Thr Glu Val Val Ser Thr Gly Glu
        35                  40                  45
Asn Glu Tyr Asp Glu Tyr Lys Glu Glu Lys Gln His Lys His Lys Gln
    50                  55                  60
His Leu Gly Glu Ala Gly Ala Ile Ala Ala Gly Ala Phe Ala Leu Tyr
65                  70                  75                  80
Glu Lys His Glu Ala Lys Lys Asp Pro Glu His Ala His Arg His Lys
                85                  90                  95
Ile Glu Glu Glu Val Ala Ala Ala Ala Ala Val Gly Ser Gly Gly Phe
            100                 105                 110
Ala Phe His Glu His His Glu Lys Lys Lys Asp His Lys Asp Ala Glu
        115                 120                 125
Glu Ala Gly Gly Glu Lys Lys His His Phe Phe Gly
    130                 135                 140
```

<210> 427
<211> 1182
<212> DNA
<213> Arabidopsis thaliana

<400> 427

```
atggactgca acatggtatc ttcgtcccag tgggattggg agcatttgat catgtccaat      60
ccgtcaagga ctgaagatga cagcaaacag ctacctactg agtgggaaat tgaaaaaggt     120
gaaggaattg aatctatagt tccacatttc tcaggccttg agagagtcag tagtggctct     180
gccaccagct tctggcacac tgctgtatcg aaaagctcac agtcgacctc tatcaactca     240
tcatctcccg aagccaaacg atgcaagctt gcatcagaaa gttcccctgg agattcttgc     300
agcaacatag actttgtcca ggtgaaggct cccacagctc tcgaggtatc cgttgcctca     360

gctgaatcag atctttgttt aaaactagga aagcggacat actctgaaga atactggggt     420
agaaacaata atgaaatttc agcggtttct atgaagttgt taactccatc tgttgtcgct     480
gggaaatcca aattgtgtgg tcagagcatg ccagtcccgc gttgccaaat tgatggctgt     540
gaactggatc tctcatctgc taagggttat catcgtaagc acaaagtctg cgaaaagcat     600
tcaaagtgcc caaaagttag cgtgagtggc ctggaacgtc ggttctgcca acagtgtagc     660
aggttccatg ctgtctctga atttgatgag aagaaacgaa gctgccgaaa acgtctttct     720
catcataatg cgaggcgtcg taagccacaa ggagtatttt caatgaatcc cgagagggtg     780
tatgatcgaa gacagcatac aaatatgttg tggaatgggg tgtcccttaa cgcgagatct     840
gaagaaatgt atgaatgggg taataacact tatgatacaa agcctagaca aacggaaaaa     900
agctttactc tgagcttcca gagaggtaat ggctctgagg accagctggt tgctagtagc     960
agccgtatgt tctctacatc tcaaacctca ggtgggttcc cagcaggaaa gtccaagttt    1020
caacttcatg gcgaagatgt gggagaatac tcaggagtcc tccatgaatc tcaagatatc    1080
caccgtgctc tctctcttct gtcaacctct tcggatcccc tggcccaacc acatgtgcag    1140
ccattttctc tactctgttc atatgatgtt gtaccaaaat ag                      1182
```

<210> 428
<211> 393
<212> PRT
<213> Arabidopsis thaliana

<400> 428

```
Met Asp Cys Asn Met Val Ser Ser Ser Gln Trp Asp Trp Glu His Leu
1               5                   10                  15
Ile Met Ser Asn Pro Ser Arg Thr Glu Asp Asp Ser Lys Gln Leu Pro
            20                  25                  30
Thr Glu Trp Glu Ile Glu Lys Gly Glu Gly Ile Glu Ser Ile Val Pro
        35                  40                  45
His Phe Ser Gly Leu Glu Arg Val Ser Ser Gly Ser Ala Thr Ser Phe
    50                  55                  60
Trp His Thr Ala Val Ser Lys Ser Ser Gln Ser Thr Ser Ile Asn Ser
65                  70                  75                  80
Ser Ser Pro Glu Ala Lys Arg Cys Lys Leu Ala Ser Glu Ser Ser Pro
                85                  90                  95
Gly Asp Ser Cys Ser Asn Ile Asp Phe Val Gln Val Lys Ala Pro Thr
            100                 105                 110
Ala Leu Glu Val Ser Val Ala Ser Ala Glu Ser Asp Leu Cys Leu Lys
        115                 120                 125
Leu Gly Lys Arg Thr Tyr Ser Glu Glu Tyr Trp Gly Arg Asn Asn Asn
    130                 135                 140
Glu Ile Ser Ala Val Ser Met Lys Leu Leu Thr Pro Ser Val Val Ala
145                 150                 155                 160
Gly Lys Ser Lys Leu Cys Gly Gln Ser Met Pro Val Pro Arg Cys Gln
                165                 170                 175
Ile Asp Gly Cys Glu Leu Asp Leu Ser Ser Ala Lys Gly Tyr His Arg
            180                 185                 190
Lys His Lys Val Cys Glu Lys His Ser Lys Cys Pro Lys Val Ser Val
        195                 200                 205
Ser Gly Leu Glu Arg Arg Phe Cys Gln Gln Cys Ser Arg Phe His Ala
    210                 215                 220
Val Ser Glu Phe Asp Glu Lys Lys Arg Ser Cys Arg Lys Arg Leu Ser
225                 230                 235                 240
His His Asn Ala Arg Arg Arg Lys Pro Gln Gly Val Phe Ser Met Asn
                245                 250                 255
Pro Glu Arg Val Tyr Asp Arg Arg Gln His Thr Asn Met Leu Trp Asn
            260                 265                 270
Gly Val Ser Leu Asn Ala Arg Ser Glu Glu Met Tyr Glu Trp Gly Asn
        275                 280                 285
Asn Thr Tyr Asp Thr Lys Pro Arg Gln Thr Glu Lys Ser Phe Thr Leu
    290                 295                 300
Ser Phe Gln Arg Gly Asn Gly Ser Glu Asp Gln Leu Val Ala Ser Ser
305                 310                 315                 320
Ser Arg Met Phe Ser Thr Ser Gln Thr Ser Gly Gly Phe Pro Ala Gly
                325                 330                 335
Lys Ser Lys Phe Gln Leu His Gly Glu Asp Val Gly Glu Tyr Ser Gly
            340                 345                 350
Val Leu His Glu Ser Gln Asp Ile His Arg Ala Leu Ser Leu Leu Ser
        355                 360                 365
Thr Ser Ser Asp Pro Leu Ala Gln Pro His Val Gln Pro Phe Ser Leu
    370                 375                 380
Leu Cys Ser Tyr Asp Val Val Pro Lys
385                 390
```

<210> 429
<211> 1464
<212> DNA
<213> Arabidopsis thaliana

<400> 429

```
cttagtcaat agcatcttct ccgatcatcg acgtccggag atttcttcgg ccctctcata      60
cttgagttgt tgtaggattt gttgctctgg ctctggtggt aggtctatga aatcaaccca     120
tatcgtgaat ggactgcaac atggtatctt cgtcccagtg ggattgggag catttgatca     180
tgtccaatcc gtcaaggact gaagatgaca gcaaacagct acctactgag tgggaaattg     240
```

```
aaaaaggtga aggaattgaa tctatagttc cacatttctc aggccttgag agagtcagta    300
gtggctctgc caccagcttc tggcacactg ctgtatcgaa aagctcacag tcgacctcta    360
tcaactcatc atctcccgaa gccaaacgat gcaagcttgc atcagaaagt tcccctggag    420
attcttgcag caacatagac tttgtccagg tgaaggctcc cacagctctc gaggtatccg    480
ttgcctcagc tgaatcagat ctttgtttaa aactaggaaa gcggacatac tctgaagaat    540
actggggtag aaacaataat gaaatttcag cggtttctat gaagttgtta actccatctg    600
ttgtcgctgg gaaatccaaa ttgtgtggtc agagcatgcc agtcccgcgt tgccaaattg    660
atggctgtga actggatctc tcatctgcta agggttatca tcgtaagcac aaagtctgcg    720
aaaagcattc aaagtgccca aaagttagcg tgagtggcct ggaacgtcgg ttctgccaac    780
agtgtagcag gttccatgct gtctctgaat ttgatgagaa gaaacgaagc tgccgaaaac    840
gtctttctca tcataatgcg aggcgtcgta agccacaagg agtattttca atgaatcccg    900
agagggtgta tgatcgaaga cagcatacaa atatgttgtg gaatggggtg tcccttaacg    960
cgagatctga agaaatgtat gaatggggta ataacactta tgatacaaag cctagacaaa   1020
cggaaaaaag ctttactctg agcttccaga gaggtaatgg ctctgaggac cagctggttg   1080
ctagtagcag ccgtatgttc tctacatctc aaacctcagg tgggttccca gcaggaaagt   1140
ccaagtttca acttcatggc gaagatgtgg gagaatactc aggagtcctc catgaatctc   1200
aagatatcca ccgtgctctc tctcttctgt caacctcttc ggatcccctg gcccaaccac   1260
atgtgcagcc attttctcta ctctgttcat atgatgttgt accaaaatag atgagtaagt   1320
aatgtgtaat ttgtaaacct gttactcagt tggtggatac ttttccaaac ctatgataaa   1380
aacctcgtcc tagatcccgt taaatgccaa actttcggct actataacta tgttatcgtt   1440
atcattatca ttgtttaaca ccct                                           1464
```

<210> 430
<211> 1551
<212> DNA
<213> Arabidopsis thaliana

<400> 430

```
ctgggtgaaa catagaaaag tttctcttgc tcaagttaat gataaaaggg tgagagcaat     60
aaacgctgat aagccttgtc tggtccttgg aattttgaat tttcttttc tatcttactt    120
atagtattgg tagttgaggg tgtcgtcgat aagttgttgt aggatttgtt gctctggctc    180
tggtggtagg tctatgaaat caacccatat cgtgaatgga ctgcaacatg gtatcttcgt    240
cccagtggga ttgggagcat ttgatcatgt ccaatccgtc aaggactgaa gatgacagca    300
aacagctacc tactgagtgg gaaattgaaa aaggtgaagg aattgaatct atagttccac    360
atttctcagg ccttgagaga gtcagtagtg gctctgccac cagcttctgg cacactgctg    420
tatcgaaaag ctcacagtcg acctctatca actcatcatc tcccgaagcc aaacgatgca    480
agcttgcatc agaaagttcc cctggagatt cttgcagcaa catagacttt gtccaggtga    540
aggctcccac agctctcgag gtatccgttg cctcagctga atcagatctt tgtttaaaac    600
taggaaagcg gacatactct gaagaatact ggggtagaaa caataatgaa atttcagcgg    660
tttctatgaa gttgttaact ccatctgttg tcgctgggaa atccaaattg tgtggtcaga    720
gcatgccagt cccgcgttgc caaattgatg gctgtgaact ggatctctca tctgctaagg    780
gttatcatcg taagcacaaa gtctgcgaaa agcattcaaa gtgcccaaaa gttagcgtga    840
gtggcctgga acgtcggttc tgccaacagt gtagcaggtt ccatgctgtc tctgaatttg    900
atgagaagaa acgaagctgc cgaaaacgtc tttctcatca taatgcgagg cgtcgtaagc    960
cacaaggagt attttcaatg aatcccgaga gggtgtatga tcgaagacag catacaaata   1020
tgttgtggaa tggggtgtcc cttaacgcga gatctgaaga aatgtatgaa tggggtaata   1080
acacttatga tacaaagcct agacaaacgg aaaaaagctt tactctgagc ttccagagag   1140
gtaatggctc tgaggaccag ctggttgcta gtagcagccg tatgttctct acatctcaaa   1200
cctcaggtgg gttcccagca ggaaagtcca agtttcaact tcatggcgaa gatgtgggag   1260
aatactcagg agtcctccat gaatctcaag atatccaccg tgctctctct cttctgtcaa   1320
cctcttcgga tcccctggcc caaccacatg tgcagccatt ttctctactc tgttcatatg   1380
atgttgtacc aaaatagatg agtaagtaat gtgtaatttg taaacctgtt actcagttgg   1440
tggatacttt tccaaaccta tgataaaaac ctcgtcctag atcccgttaa atgccaaact   1500
ttcggctact ataactatgt tatcgttatc attatcattg tttaacaccc t            1551
```

<210> 431
<211> 1182
<212> DNA
<213> Arabidopsis thaliana

<400> 431

```
atggactgca acatggtatc ttcgtcccag tgggattggg agcatttgat catgtccaat      60
ccgtcaagga ctgaagatga cagcaaacag ctacctactg agtgggaaat tgaaaaaggt     120
gaaggaattg aatctatagt tccacatttc tcaggccttg agagagtcag tagtggctct     180
gccaccagct tctggcacac tgctgtatcg aaaagctcac agtcgacctc tatcaactca     240
tcatctcccg aagccaaacg atgcaagctt gcatcagaaa gttcccctgg agattcttgc     300
agcaacatag actttgtcca ggtgaaggct cccacagctc tcgaggtatc cgttgcctca     360
gctgaatcag atctttgttt aaaactagga aagcggacat actctgaaga atactggggt     420
agaaacaata atgaaatttc agcggtttct atgaagttgt taactccatc tgttgtcgct     480
gggaaatcca aattgtgtgg tcagagcatg ccagtcccgc gttgccaaat tgatggctgt     540
gaactggatc tctcatctgc taagggttat catcgtaagc acaaagtctg cgaaaagcat     600
tcaaagtgcc caaaagttag cgtgagtggc ctggaacgtc ggttctgcca acagtgtagc     660
aggttccatg ctgtctctga atttgatgag aagaaacgaa gctgccgaaa acgtctttct     720
catcataatg cgaggcgtcg taagccacaa ggagtatttt caatgaatcc cgagagggtg     780
tatgatcgaa gacagcatac aaatatgttg tggaatgggg tgtcccttaa cgcgagatct     840
gaagaaatgt atgaatgggg taataacact tatgatacaa agcctagaca aacggaaaaa     900
agctttactc tgagcttcca gagaggtaat ggctctgagg accagctggt tgctagtagc     960
agccgtatgt tctctacatc tcaaacctca ggtgggttcc cagcaggaaa gtccaagttt    1020
caacttcatg gcgaagatgt gggagaatac tcaggagtcc tccatgaatc tcaagatatc    1080
caccgcgccc tatcgctgct atcgacctct tcggatcccc tggcccaacc acatgtgcag    1140
ccattttctc tactctgttc atatgatgtt gtaccaaaat ag                       1182
```

<210> 432
<211> 1586
<212> DNA
<213> Arabidopsis thaliana

<400> 432

```
gcccttttg ttggtctggg tgaaacatag caaggtttct cttgctgagg ttattgataa      60
acgggtgagt taaataaaaa cgttgatcag cagtgtgtgg tgcttggaat tcataaggtc     120
tatgaaatca acccacatct tgaatggact gcaacatggt atcttcgttc ccgtgggact     180
gggagaattt gatcatgtcc aatcagtcga agactgaaaa tgaaaaaaaa cagcaatcta     240
ctgagtggga atttgaaaaa ggtgaaggaa ttgaatctat agttccagat ttcttaggct     300
ttgagaaagt cagtagtggc tctgctacta gtttctggca cactgccgta tcaaaaagct     360
cgcagtcgac ctctatcaac tcatcatctc ccgaggacaa acgatgcaat cttgcatcac     420
aaagttcccc tggagattct tccagcaaca tagattttct ccaggtgaaa ccatccacag     480
ctctcgaggt acctattgcc tcagctgaat cagatctttg tttgaaacta ggaaagcgga     540
catactctga agaattttgg ggtaggaaca ataatgacct ttcagcggtt tctatgaatt     600
tgttgactcc atctgttgtt gctcggaaga aaaccaaatc gtgtggtcag agcatgcaag     660
ttccgcgttg ccaaattgat ggctgtgagc tggatctctc atcttctaag gattatcatc     720
gcaagcatag agtctgcgaa acgcattcaa agtgcccaaa agttgttgtg agtggcctgg     780
aacgtcgttt ctgccaacag tgtagcaggt tccatgctgt ctcagaattt gatgaaaaga     840
aacgaagctg ccgcaaacgt ctttctcatc ataatgcaag gcgtcgcaag ccacaaggag     900
tatttccact gaattcagag agggtgttcg atcgaagaca gcatacaagt atgttgtgga     960
atgggttgtc ccttaacacg agatctgaag aaaagtatac atggggtacc acttatgaga    1020
caaagcctac acagatggaa agcggcttta ctctgagctt ccagagaggt aatggctctg    1080
aggaccaact gtttactggt agcaccctct ctttctctgc gtttcaaaca tctggtgggt    1140
tctcagcagg gaaatccaac attcaacttc cagacaaagg tgtgggagaa tgctcaggag    1200
gcctccatga atctcatgat ttctacagtg ctctctctct tctgtcaact acttcggatt    1260
cacaagggat caaacacact cccgtggccg aaccaccgcc aatatttggc actttcccta    1320
gtcatttcat ctgaaagagt taaaaaatgc gaagtcactc cataaaaagt cgggttgcat    1380
catccgatgg agtttgacag tttgttttca aataaaataa aaatgtgcaa catccattgc    1440
tcaagctaaa ccagtcactc ggttagctca gcttttctta tacatttcca aacatattgt    1500
caatttcctt ctggattctg ttggtatgac aactttgtta ctctgtcaaa catgtctacg    1560
actattttaa aaccatttca gagatt                                         1586
```

<210> 433
<211> 396
<212> PRT

<213> Arabidopsis thaliana

<400> 433

```
Met Asp Cys Asn Met Val Ser Ser Phe Pro Trp Asp Trp Glu Asn Leu
1               5                   10                  15
Ile Met Ser Asn Gln Ser Lys Thr Glu Asn Glu Lys Lys Gln Gln Ser
            20                  25                  30
Thr Glu Trp Glu Phe Glu Lys Gly Glu Gly Ile Glu Ser Ile Val Pro
        35                  40                  45
Asp Phe Leu Gly Phe Glu Lys Val Ser Ser Gly Ser Ala Thr Ser Phe
    50                  55                  60
Trp His Thr Ala Val Ser Lys Ser Ser Gln Ser Thr Ser Ile Asn Ser
65                  70                  75                  80
Ser Ser Pro Glu Asp Lys Arg Cys Asn Leu Ala Ser Gln Ser Ser Pro
                85                  90                  95
Gly Asp Ser Ser Ser Asn Ile Asp Phe Leu Gln Val Lys Pro Ser Thr
            100                 105                 110
Ala Leu Glu Val Pro Ile Ala Ser Ala Glu Ser Asp Leu Cys Leu Lys
        115                 120                 125
Leu Gly Lys Arg Thr Tyr Ser Glu Glu Phe Trp Gly Arg Asn Asn Asn
    130                 135                 140
Asp Leu Ser Ala Val Ser Met Asn Leu Leu Thr Pro Ser Val Val Ala
145                 150                 155                 160
Arg Lys Lys Thr Lys Ser Cys Gly Gln Ser Met Gln Val Pro Arg Cys
                165                 170                 175
Gln Ile Asp Gly Cys Glu Leu Asp Leu Ser Ser Ser Lys Asp Tyr His
            180                 185                 190
Arg Lys His Arg Val Cys Glu Thr His Ser Lys Cys Pro Lys Val Val
        195                 200                 205
Val Ser Gly Leu Glu Arg Arg Phe Cys Gln Gln Cys Ser Arg Phe His
    210                 215                 220
Ala Val Ser Glu Phe Asp Glu Lys Lys Arg Ser Cys Arg Lys Arg Leu
225                 230                 235                 240
Ser His His Asn Ala Arg Arg Arg Lys Pro Gln Gly Val Phe Pro Leu
                245                 250                 255
Asn Ser Glu Arg Val Phe Asp Arg Arg Gln His Thr Ser Met Leu Trp
            260                 265                 270
Asn Gly Leu Ser Leu Asn Thr Arg Ser Glu Glu Lys Tyr Thr Trp Gly
        275                 280                 285
Thr Thr Tyr Glu Thr Lys Pro Thr Gln Met Glu Ser Gly Phe Thr Leu
    290                 295                 300
Ser Phe Gln Arg Gly Asn Gly Ser Glu Asp Gln Leu Phe Thr Gly Ser
305                 310                 315                 320
Thr Leu Ser Phe Ser Ala Phe Gln Thr Ser Gly Gly Phe Ser Ala Gly
                325                 330                 335
Lys Ser Asn Ile Gln Leu Pro Asp Lys Gly Val Gly Glu Cys Ser Gly
            340                 345                 350
Gly Leu His Glu Ser His Asp Phe Tyr Ser Ala Leu Ser Leu Leu Ser
        355                 360                 365
Thr Thr Ser Asp Ser Gln Gly Ile Lys His Thr Pro Val Ala Glu Pro
    370                 375                 380
Pro Pro Ile Phe Gly Thr Phe Pro Ser His Phe Ile
385                 390                 395
```

<210> 434
<211> 1260
<212> DNA
<213> Arabidopsis thaliana

<400> 434

```
atggagtgta atgcaaagcc accgtttcaa tgggaattgg aaaacttgat atcttttggc     60
acttctacag ctgaagttcc tagaaagcta aaaccaatgg agtgggaaat tgatggattt    120
gattgcacct ctctgtattc ttcaagcttt gcctatgctg gtagttcagg ttctgatata    180

gctcatgctt tctctaaaag ctcaaagtca acttccatta gctcttcatc agctgaagta    240
agaacacaca attttacatc cgaaactggt gaaagtcttc ctggagaatt tgcaaagggg    300
attgatactt ctccaagtct tgaactttct tttggctctg gtgatccggt tctcggttta    360
aagcttggta aacgaacgta ctttgaagac ttttgggaag tggagaatgc taaaggtttg    420
ggacttccag tgactctggc ttcatcttct gtttctcccg tgaagaaatc gaaatccatt    480
cctcagaggt tacaaactcc tcactgtcaa gttgaaggct gtaatcttga tctttcatca    540
gctaaagact atcatcggaa acataggatt tgtgaaaatc attcaaagtt tcctaaagtc    600
gttgtgagtg gcgtagagcg tcggttctgc caacaatgta gcaggttcca ctgtctctct    660
gagtttgatg agaagaaacg tagctgtcgc cgacgtctct cagatcacaa tgcaagacgt    720
cgcaagccaa atcctggaag gacatatgat gggaaaccac aggtggattt tgtatggaac    780
agatttgcac ttatccatcc aagaagtgag gaaaagttta tatggccaag ttcgaagcac    840
gtaccatcaa gagtgttaat gccgcaacct gcaaagactg agatttccga taccgagcac    900
aatagatttg gattgttgga ccccaaaacc aaaaccgcaa gagccgagtt attcagtaaa    960
gaaaaggtca caatctcttc acacatgggt gcttctcaag atcttgatgg tgctctctct   1020
cttctgtcaa attcaacaac atgggtttct tcctctgacc aaccaaggcg ttttaccctt   1080
gatcaccatc cctcaagcaa cctccaaccc gtagctcacc ggtctgcggc tcaactcaat   1140
tcagtttccg gctattggca gccggaccca cccgcagttg aaggcccgac cgctctgcat   1200
agaaatgggg taggccagtt taatgaaaac tacttcagct tgaaccagtt ttataactga   1260
```

<210> 435
<211> 419
<212> PRT
<213> Arabidopsis thaliana

<400> 435

```
Met Glu Cys Asn Ala Lys Pro Pro Phe Gln Trp Glu Leu Glu Asn Leu
1               5                   10                  15
Ile Ser Phe Gly Thr Ser Thr Ala Glu Val Pro Arg Lys Leu Lys Pro
            20                  25                  30
Met Glu Trp Glu Ile Asp Gly Phe Asp Cys Thr Ser Leu Tyr Ser Ser
        35                  40                  45
Ser Phe Ala Tyr Ala Gly Ser Ser Gly Ser Asp Ile Ala His Ala Phe
    50                  55                  60
Ser Lys Ser Ser Lys Ser Thr Ser Ile Ser Ser Ser Ser Ala Glu Val
65                  70                  75                  80
Arg Thr His Asn Phe Thr Ser Glu Thr Gly Glu Ser Leu Pro Gly Glu
                85                  90                  95
Phe Ala Lys Gly Ile Asp Thr Ser Pro Ser Leu Glu Leu Ser Phe Gly
            100                 105                 110
Ser Gly Asp Pro Val Leu Gly Leu Lys Leu Gly Lys Arg Thr Tyr Phe
        115                 120                 125
Glu Asp Phe Trp Glu Val Glu Asn Ala Lys Gly Leu Gly Leu Pro Val
    130                 135                 140
Thr Leu Ala Ser Ser Ser Val Ser Pro Val Lys Lys Ser Lys Ser Ile
145                 150                 155                 160
Pro Gln Arg Leu Gln Thr Pro His Cys Gln Val Glu Gly Cys Asn Leu
                165                 170                 175
Asp Leu Ser Ser Ala Lys Asp Tyr His Arg Lys His Arg Ile Cys Glu
            180                 185                 190
Asn His Ser Lys Phe Pro Lys Val Val Val Ser Gly Val Glu Arg Arg
        195                 200                 205
Phe Cys Gln Gln Cys Ser Arg Phe His Cys Leu Ser Glu Phe Asp Glu
    210                 215                 220
Lys Lys Arg Ser Cys Arg Arg Arg Leu Ser Asp His Asn Ala Arg Arg
225                 230                 235                 240
Arg Lys Pro Asn Pro Gly Arg Thr Tyr Asp Gly Lys Pro Gln Val Asp
                245                 250                 255
Phe Val Trp Asn Arg Phe Ala Leu Ile His Pro Arg Ser Glu Glu Lys
            260                 265                 270
Phe Ile Trp Pro Ser Ser Lys His Val Pro Ser Arg Val Leu Met Pro
        275                 280                 285

Gln Pro Ala Lys Thr Glu Ile Ser Asp Thr Glu His Asn Arg Phe Gly
    290                 295                 300
Leu Leu Asp Pro Lys Thr Lys Thr Ala Arg Ala Glu Leu Phe Ser Lys
305                 310                 315                 320
Glu Lys Val Thr Ile Ser Ser His Met Gly Ala Ser Gln Asp Leu Asp
                325                 330                 335
Gly Ala Leu Ser Leu Leu Ser Asn Ser Thr Thr Trp Val Ser Ser Ser
            340                 345                 350
Asp Gln Pro Arg Arg Phe Thr Leu Asp His His Pro Ser Ser Asn Leu
        355                 360                 365
Gln Pro Val Ala His Arg Ser Ala Ala Gln Leu Asn Ser Val Ser Gly
    370                 375                 380
Tyr Trp Gln Pro Asp Pro Pro Ala Val Glu Gly Pro Thr Ala Leu His
385                 390                 395                 400
Arg Asn Gly Val Gly Gln Phe Asn Glu Asn Tyr Phe Ser Leu Asn Gln
                405                 410                 415
Phe Tyr Asn
```

<210> 436
<211> 1410
<212> DNA
<213> Oryza sativa

<400> 436

```
atgggttctt ttgggatgga ctggaatcag aagagctcgg tgttgtggga ttgggagaat     60
atgccgccga taggaaatag cgcgaacgag aatcccaaga atgtgatgct ggctgaatca    120
aaacttgcag gtgttggggt tgacataggc catgaatcag gccattcttc tggtggtact    180
ttctcttcta gctcagagat tgggtatggt tcatccaaga gttccatatc cgcgtcgatc    240
gattctccgt ccaaagtggg gaacaccata gagctcaatt ttgcatctgc ggaagagcat    300
gataagaaca tggacaaggg taagagtaaa gttgatgaca ccggaacttc tcgatcaccg    360
gtggtagcag ccaatcgtgt agagcccttg attggcctga agcttggcaa gagaacctat    420
tttgaagatg tctgtggagg gcagaatgtc aagagttctc catctggtgt gagtgtggct    480
accccatctc ctggtctggc caagaaggtg aaggtggctc agcagaacac tcagaaccca    540
cactgtcaag ttgaaggctg caatgttgat ctctcttctg ctaaacctta ccatcgaaag    600
caccgagtct gtgaacctca cagcaagact cttaaagtca tcgttgcggg tcttgagcga    660
cgcttttgcc aacagtgtag tcggtttcac ggtttagctg agttcgacca gaaaaaacga    720
agctgtcgca ggcgcctcca tgatcacaat gcccgcagac ggaagccaca accagaagca    780
atttccttaa gttcgtcgag gctctctaca ttactctatg gtgatgcaag gcaacaggca    840
agttttctat ttggtcaagc tccttatggt cagatgggaa gctgtgcaag ttcttgggat    900
aacccagtac caggaggctt caaatttaca gcaacaaaag ctccttggtc aaggccaaca    960
atagctgccg gtgttgatgg gacgcatgta tctaatcagc aggcatcggg caatgtactg   1020
ccacatggag cacatcatag ttttgatggt ctcatggcgt tcaaagaaac caacgcgaag   1080
gtccttaacc aaggcatgga agcttctgct gtcgcttccg gctccgctag aggcccagac   1140
tttgagcatg ctctctctct tctgtcaatc gattcagtgg gtgctgcaaa ccttcagcca   1200
ggttctcaga ttcaccctgg tgtcaccgcc attgccggca cctccaaccc tgtcatgatg   1260
ccatctccag caatctggca aggaggcttg tcccttgatc agcaggcgca gtttcaggct   1320
ttcgaccgcc ttggcaacga cgacgacgaa gatcatctcc agctcccaaa gccttcctat   1380
gacaactccc actatgatca gatgaactga                                    1410
```

<210> 437
<211> 469
<212> PRT
<213> Oryza sativa

<400> 437

```
Met Gly Ser Phe Gly Met Asp Trp Asn Gln Lys Ser Ser Val Leu Trp
1               5                   10                  15
Asp Trp Glu Asn Met Pro Pro Ile Gly Asn Ser Ala Asn Glu Asn Pro
            20                  25                  30
Lys Asn Val Met Leu Ala Glu Ser Lys Leu Ala Gly Val Gly Val Asp
```

```
        35                  40                  45
Ile Gly His Glu Ser Gly His Ser Ser Gly Gly Thr Phe Ser Ser Ser
    50                  55                  60
Ser Glu Ile Gly Tyr Gly Ser Ser Lys Ser Ser Ile Ser Ala Ser Ile
65                  70                  75                  80
Asp Ser Pro Ser Lys Val Gly Asn Thr Ile Glu Leu Asn Phe Ala Ser
                85                  90                  95
Ala Glu Glu His Asp Lys Asn Met Asp Lys Gly Lys Ser Lys Val Asp
            100                 105                 110
Asp Thr Gly Thr Ser Arg Ser Pro Val Val Ala Ala Asn Arg Val Glu
        115                 120                 125
Pro Leu Ile Gly Leu Lys Leu Gly Lys Arg Thr Tyr Phe Glu Asp Val
    130                 135                 140
Cys Gly Gly Gln Asn Val Lys Ser Ser Pro Ser Gly Val Ser Val Ala
145                 150                 155                 160
Thr Pro Ser Pro Gly Leu Ala Lys Lys Val Lys Val Ala Gln Gln Asn
                165                 170                 175
Thr Gln Asn Pro His Cys Gln Val Glu Gly Cys Asn Val Asp Leu Ser
            180                 185                 190
Ser Ala Lys Pro Tyr His Arg Lys His Arg Val Cys Glu Pro His Ser
        195                 200                 205
Lys Thr Leu Lys Val Ile Val Ala Gly Leu Glu Arg Arg Phe Cys Gln
    210                 215                 220
Gln Cys Ser Arg Phe His Gly Leu Ala Glu Phe Asp Gln Lys Lys Arg
225                 230                 235                 240
Ser Cys Arg Arg Arg Leu His Asp His Asn Ala Arg Arg Arg Lys Pro
                245                 250                 255
Gln Pro Glu Ala Ile Ser Leu Ser Ser Ser Arg Leu Ser Thr Leu Leu
            260                 265                 270
Tyr Gly Asp Ala Arg Gln Gln Ala Ser Phe Leu Phe Gly Gln Ala Pro
        275                 280                 285
Tyr Gly Gln Met Gly Ser Cys Ala Ser Ser Trp Asp Asn Pro Val Pro

    290                 295                 300
Gly Gly Phe Lys Phe Thr Ala Thr Lys Ala Pro Trp Ser Arg Pro Thr
305                 310                 315                 320
Ile Ala Ala Gly Val Asp Gly Thr His Val Ser Asn Gln Gln Ala Ser
                325                 330                 335
Gly Asn Val Leu Pro His Gly Ala His His Ser Phe Asp Gly Leu Met
            340                 345                 350
Ala Phe Lys Glu Thr Asn Ala Lys Val Leu Asn Gln Gly Met Glu Ala
        355                 360                 365
Ser Ala Val Ala Ser Gly Ser Ala Arg Gly Pro Asp Phe Glu His Ala
    370                 375                 380
Leu Ser Leu Leu Ser Ile Asp Ser Val Gly Ala Ala Asn Leu Gln Pro
385                 390                 395                 400
Gly Ser Gln Ile His Pro Gly Val Thr Ala Ile Ala Gly Thr Ser Asn
                405                 410                 415
Pro Val Met Met Pro Ser Pro Ala Ile Trp Gln Gly Gly Leu Ser Leu
            420                 425                 430
Asp Gln Gln Ala Gln Phe Gln Ala Phe Asp Arg Leu Gly Asn Asp Asp
        435                 440                 445
Asp Glu Asp His Leu Gln Leu Pro Lys Pro Ser Tyr Asp Asn Ser His
    450                 455                 460
Tyr Asp Gln Met Asn
465
```

<210> 438
<211> 1428
<212> DNA
<213> Oryza sativa

<400> 438

```
atggcttctt ttgggatgaa ctggaatcag aagagccctg tgttttggga ctgggaaaat      60
ccagcgcctt tcggtccgaa tacaatggaa aatcccaaga gcatacctca ccctgaacca     120
agaggtgtag ttgtcgcggc cgcaaatcat ggatccacca attcatccgg tggcacgttc     180
acttctagct cggagctagc caatggttca tcgaagagct ccttgtcagc gtcgttcgat     240
tcctcatcca agctggggaa cagcttagag ttcaggtttg cttctgtcaa agggcatggc     300
aagaacatgt gcaaggatgg cgaggccggt agagttgaag actcgggcac ttctccagct     360
gtggcagtta gccatggtga gccggtaata ggactcaagt tagggaagag aacttacttt     420
gaaaatgttt gtggagggca gaatgtcaag agctcctctg cagcttcagg tgtgacttgt     480
ccatctactg tggtcaagaa gatgaaggtg tctcagcaga gcacacaaag ctcatactgc     540
caagttgaag gctgcaaagt cgatctgtcc tccgcaagag aataccatcg caagcacaaa     600
gtttgtgaag ctcattctaa ggcaccaaag gttattgttt ctggtctgga gcgccgtttt     660
tgccaacagt gtagtcggtt tcatggttta gctgaatttg accagaaaaa gaaaagttgc     720
cgcaggcgtc tatctgatca taatgcacga agaaggaaac cgcaacaaga ggcaatttca     780
tttggttcat caaggctcgc cacgatgttt tacgatgcaa ggcagcagac agatatttac     840
tttggccaat ctccttttgg ccaagtgaga agcaatgcaa tttcttcatg tgacaacctg     900
ggaggcttca aatttacaga agcaaaactc ccctggatga agccaatgaa aactataggc     960
cttgaggatc tgaatttctc taccctgcag atgccaggca atgttgtgtc gcatacggtg    1020
catcatcatg attttgatgg gctcatacca ttcaagggaa acaccccgaa ggtcctcaac    1080
caaggtgtgg atccagcctg tgccgtcgtg tcctccaact cgaatggagc cccggatctt    1140
cggcgtgctc tctctcttct gtcaagcgat tcctggggcc cggccgacgt tcaggccggc    1200
tcccaggtgc atcccggtgg agtgatgccg cccctcgccg ttgccgccgc caccgtcacc    1260
gccccgacga accctgtcag tgtgatgcat gctctgcacc cgtccaccgg aggaggagga    1320
ttctggcaag acggcgacga cccgcctccg ctcgatcatg cctcgcaggc tcaggcgttc    1380
atgcatcctg gcaatggcag cagctccggc tatggccatc tgcactga                 1428
```

<210> 439
<211> 475
<212> PRT
<213> Oryza sativa

<400> 439

```
Met Ala Ser Phe Gly Met Asn Trp Asn Gln Lys Ser Pro Val Phe Trp
1               5                   10                  15
Asp Trp Glu Asn Pro Ala Pro Phe Gly Pro Asn Thr Met Glu Asn Pro
            20                  25                  30
Lys Ser Ile Pro His Pro Glu Pro Arg Gly Val Val Val Ala Ala Ala
        35                  40                  45
Asn His Gly Ser Thr Asn Ser Ser Gly Gly Thr Phe Thr Ser Ser Ser
    50                  55                  60
Glu Leu Ala Asn Gly Ser Ser Lys Ser Ser Leu Ser Ala Ser Phe Asp
65                  70                  75                  80
Ser Ser Ser Lys Leu Gly Asn Ser Leu Glu Phe Arg Phe Ala Ser Val
                85                  90                  95
Lys Gly His Gly Lys Asn Met Cys Lys Asp Gly Glu Ala Gly Arg Val
            100                 105                 110
Glu Asp Ser Gly Thr Ser Pro Ala Val Ala Val Ser His Gly Glu Pro
        115                 120                 125
Val Ile Gly Leu Lys Leu Gly Lys Arg Thr Tyr Phe Glu Asn Val Cys
    130                 135                 140
Gly Gly Gln Asn Val Lys Ser Ser Ser Ala Ala Ser Gly Val Thr Cys
145                 150                 155                 160
Pro Ser Thr Val Val Lys Lys Met Lys Val Ser Gln Gln Ser Thr Gln
                165                 170                 175
Ser Ser Tyr Cys Gln Val Glu Gly Cys Lys Val Asp Leu Ser Ser Ala
            180                 185                 190
Arg Glu Tyr His Arg Lys His Lys Val Cys Glu Ala His Ser Lys Ala
        195                 200                 205
Pro Lys Val Ile Val Ser Gly Leu Glu Arg Arg Phe Cys Gln Gln Cys
    210                 215                 220
```

```
Ser Arg Phe His Gly Leu Ala Glu Phe Asp Gln Lys Lys Lys Ser Cys
225                 230                 235                 240
Arg Arg Arg Leu Ser Asp His Asn Ala Arg Arg Arg Lys Pro Gln Gln
                245                 250                 255
Glu Ala Ile Ser Phe Gly Ser Ser Arg Leu Ala Thr Met Phe Tyr Asp
            260                 265                 270
Ala Arg Gln Gln Thr Asp Ile Tyr Phe Gly Gln Ser Pro Phe Gly Gln
        275                 280                 285
Val Arg Ser Asn Ala Ile Ser Ser Cys Asp Asn Leu Gly Gly Phe Lys
    290                 295                 300
Phe Thr Glu Ala Lys Leu Pro Trp Met Lys Pro Met Lys Thr Ile Gly
305                 310                 315                 320
Leu Glu Asp Leu Asn Phe Ser Thr Leu Gln Met Pro Gly Asn Val Val
                325                 330                 335
Ser His Thr Val His His His Asp Phe Asp Gly Leu Ile Pro Phe Lys
            340                 345                 350
Gly Asn Thr Pro Lys Val Leu Asn Gln Gly Val Asp Pro Ala Cys Ala
        355                 360                 365
Val Val Ser Ser Asn Ser Asn Gly Ala Pro Asp Leu Arg Arg Ala Leu
    370                 375                 380
Ser Leu Leu Ser Ser Asp Ser Trp Gly Pro Ala Asp Val Gln Ala Gly
385                 390                 395                 400
Ser Gln Val His Pro Gly Gly Val Met Pro Pro Leu Ala Val Ala Ala
                405                 410                 415
Ala Thr Val Thr Ala Pro Thr Asn Pro Val Ser Val Met His Ala Leu
            420                 425                 430
His Pro Ser Thr Gly Gly Gly Gly Phe Trp Gln Asp Gly Asp Asp Pro
        435                 440                 445
Pro Pro Leu Asp His Ala Ser Gln Ala Gln Ala Phe Met His Pro Gly
    450                 455                 460
Asn Gly Ser Ser Ser Gly Tyr Gly His Leu His
465                 470                 475
```

<210> 440
<211> 1155
<212> DNA
<213> Populus spp.

<400> 440

```
atgagttctg tctcactgat ggagtggaat ggcaaacccc acttgcagtg ggactgggaa      60
aacctgataa tgttcaatgc aataacaaat gaaaattcaa agcagttaag cctaacagat     120
ttggaaactg atggagaaaa aggaaatgat tctgggtttt tctattcatc tgggagtgta     180
agcagaagcc gtggatctat ctctgactta gaacttgctt ctttctcaaa gagctcgaag     240
tcagcttcca ccaattcttc atcagctggg gaagttaaaa catccaaatt cactttgggg     300
gcctctaaaa caaatccatc agattacaat aaggaagaac ttgtgaaggc taaggcaact     360
gatacttctc ccacgcttga agcttcagtt ggttcaggtg accagctgct tggtttgaag     420
cttggtaaaa gaacttactt tgaagatgct tgtgctggga gcaatgctca gtcttcatca     480
atatctacga ttcctgtgcc ttcttttact ccagcaaaga aattgaagtc cagtaatcat     540
agtcagcgtg ctccacgctg tcaagtagaa ggctgtaacc ttgacctctc atcagctaaa     600
gattaccatc gcaaacatag agtttgtgaa agccattcaa agtgcccaaa ggtcattgta     660
gctggtttgg aacgcaggtt ttgccagcag tgtagcaggt tccatggtct gtcagagttt     720
gatgaaaaga agaaaagctg ccgcaggcga ctttctgatc acaatgcaag acgccgcaaa     780
cagccaggat ctgtccattt gaatccttca agactttctt catcattata tgatgagagg     840
caacagatga gtcatgtttg ggacaaggcg ccacttgttc attccaggcc caatgcaaat     900
ttgacatggg aaagcacatc cacctccaag ttcacaataa caaaagagta tatagcaaag     960
cctgcagaaa taggtggtag tgataggagg ctccacttgc ctggcattga tctgacaaat    1020
agcattgcta ttcagcacca tcataagtct aatggcttct taccatccaa ggccaagggc    1080
actgcaggtg aggttctcaa ccaaggtttg ttttgtcttc attttgagaa acaagcctca    1140
caattattgc attga                                                     1155
```

<210> 441
<211> 384
<212> PRT
<213> Populus spp.

<400> 441

```
Met Ser Ser Val Ser Leu Met Glu Trp Asn Gly Lys Pro His Leu Gln
1               5                   10                  15
Trp Asp Trp Glu Asn Leu Ile Met Phe Asn Ala Ile Thr Asn Glu Asn
            20                  25                  30
Ser Lys Gln Leu Ser Leu Thr Asp Leu Glu Thr Asp Gly Glu Lys Gly
        35                  40                  45
Asn Asp Ser Gly Phe Phe Tyr Ser Ser Gly Ser Val Ser Arg Ser Arg
    50                  55                  60
Gly Ser Ile Ser Asp Leu Glu Leu Ala Ser Phe Ser Lys Ser Ser Lys
65                  70                  75                  80
Ser Ala Ser Thr Asn Ser Ser Ser Ala Gly Glu Val Lys Thr Ser Lys
                85                  90                  95
Phe Thr Leu Gly Ala Ser Lys Thr Asn Pro Ser Asp Tyr Asn Lys Glu
            100                 105                 110
Glu Leu Val Lys Ala Lys Ala Thr Asp Thr Ser Pro Thr Leu Glu Ala
        115                 120                 125
Ser Val Gly Ser Gly Asp Gln Leu Leu Gly Leu Lys Leu Gly Lys Arg
    130                 135                 140
Thr Tyr Phe Glu Asp Ala Cys Ala Gly Ser Asn Ala Gln Ser Ser Ser
145                 150                 155                 160
Ile Ser Thr Ile Pro Val Pro Ser Phe Thr Pro Ala Lys Lys Leu Lys
                165                 170                 175
Ser Ser Asn His Ser Gln Arg Ala Pro Arg Cys Gln Val Glu Gly Cys
            180                 185                 190
Asn Leu Asp Leu Ser Ser Ala Lys Asp Tyr His Arg Lys His Arg Val
        195                 200                 205
Cys Glu Ser His Ser Lys Cys Pro Lys Val Ile Val Ala Gly Leu Glu
    210                 215                 220
Arg Arg Phe Cys Gln Gln Cys Ser Arg Phe His Gly Leu Ser Glu Phe
225                 230                 235                 240
Asp Glu Lys Lys Lys Ser Cys Arg Arg Arg Leu Ser Asp His Asn Ala
                245                 250                 255
Arg Arg Arg Lys Gln Pro Gly Ser Val His Leu Asn Pro Ser Arg Leu
            260                 265                 270
Ser Ser Ser Leu Tyr Asp Glu Arg Gln Gln Met Ser His Val Trp Asp
        275                 280                 285
Lys Ala Pro Leu Val His Ser Arg Pro Asn Ala Asn Leu Thr Trp Glu
    290                 295                 300
Ser Thr Ser Thr Ser Lys Phe Thr Ile Thr Lys Glu Tyr Ile Ala Lys
305                 310                 315                 320
Pro Ala Glu Ile Gly Gly Ser Asp Arg Arg Leu His Leu Pro Gly Ile
                325                 330                 335
Asp Leu Thr Asn Ser Ile Ala Ile Gln His His His Lys Ser Asn Gly
            340                 345                 350
Phe Leu Pro Ser Lys Ala Lys Gly Thr Ala Gly Glu Val Leu Asn Gln
        355                 360                 365
Gly Leu Phe Cys Leu His Phe Glu Lys Gln Ala Ser Gln Leu Leu His
    370                 375                 380
```

<210> 442
<211> 1398
<212> DNA
<213> Populus spp.

<400> 442

```
atggagtgga atggcaaacc ccacttacag tgggactggg agagcctgat aatgttcaat      60

ggaataacaa ctgaaaattc taagcagtta agcccaacag atttggaaac tgatggagaa     120
aaaggaaccg actctgggtt tttctattca tctgggagtg caagcagaag cggtggttct     180
agctctgatt tggaacttgc ttctttctca aagtgctcga agtcagcttc catcaattct     240
tcatcagctg ggaagttaa aacatccaaa ttcactttgg aggcctctaa agcaaatcca     300
tctgattaca ataagaaaga aattggaaag gctaagacag ctagtatgtc ttccacaatt     360
gaggctgcag gtggttcagg tgaccagctg cttggtttga agcttggtaa acgaatatac     420
tttgaagatg cttgtgctgg caacaatgtt cagtcgtcat cattttctac agttcctgtg     480
ccctctttta cttcagcaaa gaaattgaag tccactattc agagtcagcg tgctccatgc     540
tgtcaagtgg aaggctgtaa ccttgacctc tcatcagcta aagattatca tcgcaaacat     600
agagtttgtg aaagccattc aaagtgccag aaggtcattg tagctggttt ggaacgcagg     660
ttttgccagc agtgtagcag taagattatt cctagctttt atgattgcat tgacaatttg     720
ggttttggat gtcaaaggtt ccatggcctg tcagagttcg atgaaaagaa gaaaagctgt     780
cgcaggcgac tttctgatca caatgcaaga cgccgcaaac aaccaggatc ggtccattta     840
aattcaagag tgtcttcatc attatatgat gaaaggcaac agatgagtct tgcttgggac     900
agggcaccac ttgttcatgc caggcctaat gcaaatttga cgtgggaagg cacatacatc     960
tccaagttca caataacaaa agattatata gcaaagcctg cagaaatagg tggtaatgat    1020
gggcagtttc acttgcctgg ctttgatctg acaaatggca ttgatattca gcaccatcat    1080
aagtctaata gctccttacc atctaaaggt aagggcactg cagctgagat tctcaaccaa    1140
ggtttggaag aatacatcat tccttccaaa gcggaagcag caccagaatc tcatcgtgct    1200
ctctctcttc tgtcaaacaa ttcatggggt tcacgtgagc cgcaatctat ttcatttgaa    1260
caacccgtgc atacaaatca caccactcag tctgtgctgc aagttatacc ccaaaactca    1320
ccacttgctt catcagagta ttggaggact gagcaaccgt caactgactc tcaagtgcat    1380
accttgacgt ctcactga                                                  1398
```

<210> 443
<211> 465
<212> PRT
<213> Populus spp.

<400> 443

Met Glu Trp Asn Gly Lys Pro His Leu Gln Trp Asp Trp Glu Ser Leu
1 5 10 15
Ile Met Phe Asn Gly Ile Thr Thr Glu Asn Ser Lys Gln Leu Ser Pro
20 25 30
Thr Asp Leu Glu Thr Asp Gly Glu Lys Gly Thr Asp Ser Gly Phe Phe
35 40 45
Tyr Ser Ser Gly Ser Ala Ser Arg Ser Gly Gly Ser Ser Ser Asp Leu
50 55 60
Glu Leu Ala Ser Phe Ser Lys Cys Ser Lys Ser Ala Ser Ile Asn Ser
65 70 75 80
Ser Ser Ala Gly Glu Val Lys Thr Ser Lys Phe Thr Leu Glu Ala Ser
85 90 95
Lys Ala Asn Pro Ser Asp Tyr Asn Lys Lys Glu Ile Gly Lys Ala Lys
100 105 110
Thr Ala Ser Met Ser Ser Thr Ile Glu Ala Ala Gly Gly Ser Gly Asp
115 120 125
Gln Leu Leu Gly Leu Lys Leu Gly Lys Arg Ile Tyr Phe Glu Asp Ala
130 135 140
Cys Ala Gly Asn Asn Val Gln Ser Ser Ser Phe Ser Thr Val Pro Val
145 150 155 160
Pro Ser Phe Thr Ser Ala Lys Lys Leu Lys Ser Thr Ile Gln Ser Gln
165 170 175
Arg Ala Pro Cys Cys Gln Val Glu Gly Cys Asn Leu Asp Leu Ser Ser
180 185 190
Ala Lys Asp Tyr His Arg Lys His Arg Val Cys Glu Ser His Ser Lys
195 200 205
Cys Gln Lys Val Ile Val Ala Gly Leu Glu Arg Arg Phe Cys Gln Gln
210 215 220
Cys Ser Ser Lys Ile Ile Pro Ser Phe Tyr Asp Cys Ile Asp Asn Leu
225 230 235 240
Gly Phe Gly Cys Gln Arg Phe His Gly Leu Ser Glu Phe Asp Glu Lys
245 250 255
Lys Lys Ser Cys Arg Arg Arg Leu Ser Asp His Asn Ala Arg Arg Arg
260 265 270
Lys Gln Pro Gly Ser Val His Leu Asn Ser Arg Val Ser Ser Ser Leu
275 280 285
Tyr Asp Glu Arg Gln Gln Met Ser Leu Ala Trp Asp Arg Ala Pro Leu
290 295 300
Val His Ala Arg Pro Asn Ala Asn Leu Thr Trp Glu Gly Thr Tyr Ile
305 310 315 320
Ser Lys Phe Thr Ile Thr Lys Asp Tyr Ile Ala Lys Pro Ala Glu Ile
325 330 335
Gly Gly Asn Asp Gly Gln Phe His Leu Pro Gly Phe Asp Leu Thr Asn
340 345 350
Gly Ile Asp Ile Gln His His His Lys Ser Asn Ser Ser Leu Pro Ser
355 360 365
Lys Gly Lys Gly Thr Ala Ala Glu Ile Leu Asn Gln Gly Leu Glu Glu
370 375 380
Tyr Ile Ile Pro Ser Lys Ala Glu Ala Ala Pro Glu Ser His Arg Ala
385 390 395 400
Leu Ser Leu Leu Ser Asn Asn Ser Trp Gly Ser Arg Glu Pro Gln Ser
405 410 415
Ile Ser Phe Glu Gln Pro Val His Thr Asn His Thr Thr Gln Ser Val
420 425 430
Leu Gln Val Ile Pro Gln Asn Ser Pro Leu Ala Ser Ser Glu Tyr Trp
435 440 445
Arg Thr Glu Gln Pro Ser Thr Asp Ser Gln Val His Thr Leu Thr Ser
450 455 460
His
465

<210> 444
<211> 1419
<212> DNA
<213> Populus spp.

<400> 444

```
atggactgga actccactgc atctgagggg aactgggaga acatagcagg gttaagtgcc      60
aaggctagtg acattccaaa acaagtacca ttggcatacc atgagacaga gggagatgga     120
gcaattgata aagcaataac ttattcgtct ggtggtggtt tatcaagctc tgatttgtgg     180
cattgctctt catccaagag ctcagtttca ccctctgtcg actcttcatt gaaggggggg     240
atcaagactt atagcactgc agatggtttt cctggagtta tcattagaaa ggacttgact     300
agggtagaaa gtactgagaa tattccatct ctgggtgctt ctgctagctc tggtgaacca     360
gtaattggct tgaagcttgg taaacggatg tactttgaag acatttgtac taccagcact     420
gccaaatcat cgtcttcatc tattgtttcc acttcctgta ctgctacaac aaagagatct     480
agggcatcat atccaagtac acagtctcca cgttgccaag tggaaggatg caaccttgat     540
ctcaagtcag ctaaagatta ccatcgcagg catagaatct gtgaaaaaca ttccaaaagc     600
ccaaaggtta ttgttgctgg catggaacgc cgattttgcc aacagtgcag caggttccat     660
gaattatcag agtttgatga caagaagcga agctgtcgta gacgtctctc tgatcacaat     720
gcaaggcgac ggaggccaca acctgaggca atccggttca attcagcaag gccatcatca     780
tcaccatcat cattttatgg ttgtcatttc aatttcaaag atggtagaca gcagatgaat     840
gttgtattga atagggtgcc tttcctggca ggaaattcaa cttggcaaag cgagtgcggc     900
tttaaagtca ctcacgccgg agattttttt attaggcctg caaaagcagg gggcatccat     960
aggcagctta gctatccctg caatgaagtg ccagatgcag cttcaacaat accaacagac    1020
tcggataaaa ttatctcttt tgagagaagt acaccccagg tctttagtca aggtttcgag    1080
gggtctgcat taacttctaa cattgaggta gcaccagatc ttcggcgtgc tctctctctt    1140
ctgtcaacca cttcttgggg ctcgaatgag catggatcca cctctctgga tcaactgatg    1200
cttgcaaacc aaacaagcat gacacagcca atgataaatg ctgaactcca aaattgtcca    1260
gttgcttcat cagaaaatgc acggatggaa caagcatccc ttgagtctcg ggtgcattct    1320
ttggatttac atgacaatgg aaacgtccag ttgcaagagt ttcagctgct caaagcaccc    1380
tatgctaccg gctgctttta ttccaatcaa ttcagctaa                           1419
```

<210> 445
<211> 472
<212> PRT
<213> Populus spp.

<400> 445

Met Asp Trp Asn Ser Thr Ala Ser Glu Gly Asn Trp Glu Asn Ile Ala
1 5 10 15
Gly Leu Ser Ala Lys Ala Ser Asp Ile Pro Lys Gln Val Pro Leu Ala
20 25 30
Tyr His Glu Thr Glu Gly Asp Gly Ala Ile Asp Lys Ala Ile Thr Tyr
35 40 45
Ser Ser Gly Gly Gly Leu Ser Ser Ser Asp Leu Trp His Cys Ser Ser
50 55 60
Ser Lys Ser Ser Val Ser Pro Ser Val Asp Ser Ser Leu Lys Gly Gly
65 70 75 80
Ile Lys Thr Tyr Ser Thr Ala Asp Gly Phe Pro Gly Val Ile Ile Arg

85 90 95
Lys Asp Leu Thr Arg Val Glu Ser Thr Glu Asn Ile Pro Ser Leu Gly
100 105 110
Ala Ser Ala Ser Ser Gly Glu Pro Val Ile Gly Leu Lys Leu Gly Lys
115 120 125
Arg Met Tyr Phe Glu Asp Ile Cys Thr Thr Ser Thr Ala Lys Ser Ser
130 135 140
Ser Ser Ser Ile Val Ser Thr Ser Cys Thr Ala Thr Thr Lys Arg Ser
145 150 155 160
Arg Ala Ser Tyr Pro Ser Thr Gln Ser Pro Arg Cys Gln Val Glu Gly
165 170 175
Cys Asn Leu Asp Leu Lys Ser Ala Lys Asp Tyr His Arg Arg His Arg
180 185 190
Ile Cys Glu Lys His Ser Lys Ser Pro Lys Val Ile Val Ala Gly Met
195 200 205
Glu Arg Arg Phe Cys Gln Gln Cys Ser Arg Phe His Glu Leu Ser Glu
210 215 220
Phe Asp Asp Lys Lys Arg Ser Cys Arg Arg Arg Leu Ser Asp His Asn
225 230 235 240
Ala Arg Arg Arg Arg Pro Gln Pro Glu Ala Ile Arg Phe Asn Ser Ala
245 250 255
Arg Pro Ser Ser Ser Pro Ser Ser Phe Tyr Gly Cys His Phe Asn Phe
260 265 270
Lys Asp Gly Arg Gln Gln Met Asn Val Val Leu Asn Arg Val Pro Phe
275 280 285
Leu Ala Gly Asn Ser Thr Trp Gln Ser Glu Cys Gly Phe Lys Val Thr
290 295 300
His Ala Gly Asp Phe Phe Ile Arg Pro Ala Lys Ala Gly Gly Ile His
305 310 315 320
Arg Gln Leu Ser Tyr Pro Cys Asn Glu Val Pro Asp Ala Ala Ser Thr
325 330 335
Ile Pro Thr Asp Ser Asp Lys Ile Ile Ser Phe Glu Arg Ser Thr Pro
340 345 350
Gln Val Phe Ser Gln Gly Phe Glu Gly Ser Ala Leu Thr Ser Asn Ile
355 360 365
Glu Val Ala Pro Asp Leu Arg Arg Ala Leu Ser Leu Leu Ser Thr Thr
370 375 380
Ser Trp Gly Ser Asn Glu His Gly Ser Thr Ser Leu Asp Gln Leu Met
385 390 395 400
Leu Ala Asn Gln Thr Ser Met Thr Gln Pro Met Ile Asn Ala Glu Leu
405 410 415
Gln Asn Cys Pro Val Ala Ser Ser Glu Asn Ala Arg Met Glu Gln Ala
420 425 430

Ser Leu Glu Ser Arg Val His Ser Leu Asp Leu His Asp Asn Gly Asn
435 440 445
Val Gln Leu Gln Glu Phe Gln Leu Leu Lys Ala Pro Tyr Ala Thr Gly
450 455 460
Cys Phe Tyr Ser Asn Gln Phe Ser
465 470

<210> 446
<211> 2042
<212> DNA
<213> Brassica rapa

<400> 446

```
atggagtgta atgcaaagcc atcattgcag tgggagtggg ataatctaat atcttttggt     60
acttcatcag ctgaaattcc taaaaagcaa cgaccaatgg attgggaaaa tgatgggttt    120
gattgcacca ctttttactc gtccagcttt gctacagaag cagcttatgg tggtggtagt    180
tcaggttctg atctagctca tgctttctct aaaagctcaa agtcaacttc cataagctct    240
tcatcagctg aagtgagaac atacaatttc acatcagaag ctggtgaaag tgttcctcct    300
ggagaattgg ggagcagtga agagtttgca atgggaattg atgcttctcc aagtcttgaa    360
ctctccttcg gctctggtga tccggttctt ggtttgaagc ttggtaagag gacatacttt    420
gaagactttt gggaagtgga gaatgctaaa ggttcagcac ttccggtgag cctggcgtca    480
tcttctgctt ctccggtgaa gaaatccaaa acactctctc agaaattaca aactcctcac    540
tgccaagttg aaggctgtaa cctcgatctc tcctcagcta aggactatca taggaaacac    600
aggatttgtg aaaaccattc aaagttccct aaagtcgttg tcagtggcgt agagcgccgg    660
ttctgccaac aatgtagcag gtagaaaagc tagtcttaga gatattggtt tatacaaaag    720
ccttaatgac ttttttaat tgtttttggt cttcaaaggt ttcactgtct ctctgagttt    780
gatgagaaga aacgtagctg tcgccggcgt ctctcagatc acaatgcaag acgtcgcaag    840
ccaaatcccg ggaggacata tggtaatata ctagattcac ttctctctaa tcctatatat    900
taaatctaaa tttttagtta tataattagt aggtttccct tttctaagat aaatatcatt    960
ctgtatgtgc agatgggaag caacagatgg attttgtatg gaacagattt gcacttatcc   1020
atccaagaag tgaagaaaat tttctgtggc caaatccgaa gcctgtatca tcaagagggt   1080
tattgcagga acctgcaaag accgagatgc ccaataagct tttcaccgag cattgtggat   1140
ttggattgtt ggaccccaaa acgaaaacca ccagagctga gttattcagt aaaggtttgt   1200
gtttttcttt gctgtttaat atatttctc ttgttgtatt catgatcttg atgattgctt   1260
gttttatgca gatttgttag aaaaggtcac aatctcttct tcacacatgg gtgcttctca   1320
agatcttgat ggtgctctct ctcttctgtc aaattcaaca ccatgggttt cctcgaacca   1380
gccaacacgg ttttccctta accaccattc cacaagcaac ctccaacccg tggttcacgg   1440
gtctgtgact caactcagtt cagtgtccag ctactggcag ccggacccac cagcagctga   1500
gggctcaacc gctttgacta ggaatggggt aggccagttt aatgagaact acaacttgaa   1560
tcagttttat aactgaaagt gtgttgcttt taaaatcata ataagatatt ttgtgtaagg   1620
atcaggtgag cctagtggta acgtttaaat aggagctgtg aaacttgcta aagacatcaa   1680
ctcctctcgt ctttcttttg tccattgatt ttcttgggtt agggagagtt gtgacagtta   1740
agtattataa atcatcttgt caaattattt atacattaga attttcagat attgatggtg   1800
tagcaactga aaaattgatc aaacgcatca taatatacgt atgataaggt tatttgcatc   1860
tgaacttttt ttatattcgc attagtcagc attttgtgtt atactagatt tggatccgtg   1920
cgttgcaaca ggttttattt gatattacca tccattgatt tttttttttt tttgcaaaca   1980
aataatttgg taattccatt aaccgccaca gtcgttttct gagcaaaacc atgttaaaat   2040
ct                                                                  2042
```

<210> 447
<211> 282
<212> PRT
<213> Brassica rapa

<400> 447

```
Met Glu Cys Asn Ala Lys Pro Ser Leu Gln Trp Glu Trp Asp Asn Leu
1               5                   10                  15
Ile Ser Phe Gly Thr Ser Ser Ala Glu Ile Pro Lys Lys Gln Arg Pro
            20                  25                  30
Met Asp Trp Glu Asn Asp Gly Phe Asp Cys Thr Thr Phe Tyr Ser Ser
        35                  40                  45
```

Ser Phe Ala Thr Glu Ala Ala Tyr Gly Gly Gly Ser Ser Gly Ser Asp
    50                  55                  60
Leu Ala His Ala Phe Ser Lys Ser Ser Lys Ser Thr Ser Ile Ser Ser
65                  70                  75                  80
Ser Ser Ala Glu Val Arg Thr Tyr Asn Phe Thr Ser Glu Ala Gly Glu
                85                  90                  95
Ser Val Pro Pro Gly Glu Leu Gly Ser Ser Glu Glu Phe Ala Met Gly
            100                 105                 110
Ile Asp Ala Ser Pro Ser Leu Glu Leu Ser Phe Gly Ser Gly Asp Pro
        115                 120                 125
Val Leu Gly Leu Lys Leu Gly Lys Arg Thr Tyr Phe Glu Asp Phe Trp
    130                 135                 140
Glu Val Glu Asn Ala Lys Gly Ser Ala Leu Pro Val Ser Leu Ala Ser
145                 150                 155                 160
Ser Ser Ala Ser Pro Val Lys Lys Ser Lys Thr Leu Ser Gln Lys Leu
                165                 170                 175
Gln Thr Pro His Cys Gln Val Glu Gly Cys Asn Leu Asp Leu Ser Ser
            180                 185                 190
Ala Lys Asp Tyr His Arg Lys His Arg Ile Cys Glu Asn His Ser Lys
        195                 200                 205
Phe Pro Lys Val Val Val Ser Gly Val Glu Arg Arg Phe Cys Gln Gln
    210                 215                 220
Cys Ser Arg Lys Ser Ser Arg Tyr Trp Phe Ile Gln Lys Pro Leu Phe
225                 230                 235                 240
Leu Ile Val Phe Gly Leu Gln Arg Phe His Cys Leu Ser Glu Phe Asp
                245                 250                 255
Glu Lys Lys Arg Ser Cys Arg Arg Arg Leu Ser Asp His Asn Ala Arg
            260                 265                 270
Arg Arg Lys Pro Asn Pro Gly Arg Thr Tyr
        275                 280

<210> 448
<211> 1684
<212> DNA
<213> Zea mays

<400> 448

```
agccgcttcg gcaggcaagt aggacgagga gcagcagctc agatttcgga tcctggcact     60
ggcaactggc atgggctcgt ttgggatgga catggactgg aaccagaagg cctccgtgct    120
gctgtgggac tgggagaacc tgccgcccgc agccgcaaac gggagcgaga gccacaggac    180
gaccgctgcc gcgcctcagt tcgcgggcct tgaggccaca gggcatgaac cggcgccttc    240
ttccgtatcc tcgtcaatcc attctctgcc cagtgccaag gggaacaaga acaagaacaa    300
catggagctc atcagtttcg cacctgccaa agcgcccgac aaggacactg gttcggtgct    360
cagcagcgga gagccggtgg tgctaggcct gaagcttggc aagagaacgt atttcgaaga    420
tggttgtgga ctagggcaga gcggcaagag ttcggcggcg ttaggcactg ccagtgcagc    480
gacccctccg ggtcctgcga agaaggcgaa ggcggcggcg gcggctccaa ccgcgcagca    540
gcagaaatcg tactgccagg ttgaaggctg caggaccgat ctgtcctctg ctaaagacta    600
tcatcgcaag cacagagtct gcgagcccca ttccaaggcg cccaaggtgg tcgtcgctgg    660
cctggagcgg cgcttctgcc agcagtgcag ccggttccat ggactggccg agttcgacca    720
gaagaagaag agctgccgca ggcgactcaa cgaccacaac gcgcgcaggc ggaagcccca    780
gcccgaagcg ctccctttcg gctcatcgag gctgtcggcg atgttctatg acacgaggcg    840
ccaggcgagc ctcctgtttg gtgaaggtcc ctacggtcag atgagaagct gtgcgagttc    900
ttggtgggat agcccaggag caggaggagg aggcttcaaa ttcgcggaga cgagaggagc    960
tccttggttg aagccggcga gagctgctgc tgatgggctg ctgcctttgt caggtcagca   1020
gcagcagcag gtgtggaatg actcgattcc gcatgttgcc catcaggagg atttcgatgg   1080
gttcacggtc acggcgttca aaggaatcag tccaaagacc cttgatcatc aaggcgcagg   1140
cgttgaagcc tgtgcggccg tctccagccc ggacggcgcc ccggaccttc agcgtgctct   1200
ctctcttctg tcaaacagtc cggccggtgg cggcggcacc aacaccaacc acccgccgcc   1260
gccgccggcg gctgagctgc accaccaccg cgctgccccg agcagctgcc tcgccgccgg   1320
ctccgtctcc gtgctgcagg aggcctcgtc gccggggctg tggcaggacg gcggcggcag   1380
cgcggccctg ggccaccacg cgcacgcgcg gctccaggct ctcgacgccg ccatggcgac   1440

ggcgcaggag ctcctccagc tccccaggcc gccgccatcg tacggcggct cctcgtccca   1500
ctacgacctg atgcgctgat gcttccttcc ttgtggattg tggtggagaa ctggagatgg   1560
agcgggagcg ccgccgtgcc cccggttggc gcgtggaaat cagtcagacg tggcttctgt   1620
ggcgtcgtct ctgcaacgcg ggagcgggat tttgcttgtg ttctgaacga acagaaaaaa   1680
aaaa                                                                1684
```

<210> 449
<211> 482
<212> PRT
<213> Zea mays

<400> 449

```
Met Gly Ser Phe Gly Met Asp Met Asp Trp Asn Gln Lys Ala Ser Val
1               5                   10                  15
Leu Leu Trp Asp Trp Glu Asn Leu Pro Pro Ala Ala Ala Asn Gly Ser
            20                  25                  30
Glu Ser His Arg Thr Thr Ala Ala Ala Pro Gln Phe Ala Gly Leu Glu
        35                  40                  45
Ala Thr Gly His Glu Pro Ala Pro Ser Ser Val Ser Ser Ser Ile His
    50                  55                  60
Ser Leu Pro Ser Ala Lys Gly Asn Lys Asn Lys Asn Asn Met Glu Leu
65                  70                  75                  80
Ile Ser Phe Ala Pro Ala Lys Ala Pro Asp Lys Asp Thr Gly Ser Val
                85                  90                  95
Leu Ser Ser Gly Glu Pro Val Val Leu Gly Leu Lys Leu Gly Lys Arg
            100                 105                 110
Thr Tyr Phe Glu Asp Gly Cys Gly Leu Gly Gln Ser Gly Lys Ser Ser
        115                 120                 125
Ala Ala Leu Gly Thr Ala Ser Ala Ala Thr Pro Pro Gly Pro Ala Lys
    130                 135                 140
Lys Ala Lys Ala Ala Ala Ala Ala Pro Thr Ala Gln Gln Gln Lys Ser

145                 150                 155                 160
Tyr Cys Gln Val Glu Gly Cys Arg Thr Asp Leu Ser Ser Ala Lys Asp
                165                 170                 175
Tyr His Arg Lys His Arg Val Cys Glu Pro His Ser Lys Ala Pro Lys
            180                 185                 190
Val Val Val Ala Gly Leu Glu Arg Arg Phe Cys Gln Gln Cys Ser Arg
        195                 200                 205
Phe His Gly Leu Ala Glu Phe Asp Gln Lys Lys Lys Ser Cys Arg Arg
    210                 215                 220
Arg Leu Asn Asp His Asn Ala Arg Arg Arg Lys Pro Gln Pro Glu Ala
225                 230                 235                 240
Leu Pro Phe Gly Ser Ser Arg Leu Ser Ala Met Phe Tyr Asp Thr Arg
                245                 250                 255
Arg Gln Ala Ser Leu Leu Phe Gly Glu Gly Pro Tyr Gly Gln Met Arg
            260                 265                 270
Ser Cys Ala Ser Ser Trp Trp Asp Ser Pro Gly Ala Gly Gly Gly Gly
        275                 280                 285
Phe Lys Phe Ala Glu Thr Arg Gly Ala Pro Trp Leu Lys Pro Ala Arg
    290                 295                 300
Ala Ala Ala Asp Gly Leu Leu Pro Leu Ser Gly Gln Gln Gln Gln Gln
305                 310                 315                 320
Val Trp Asn Asp Ser Ile Pro His Val Ala His Gln Glu Asp Phe Asp
                325                 330                 335
Gly Phe Thr Val Thr Ala Phe Lys Gly Ile Ser Pro Lys Thr Leu Asp
            340                 345                 350
His Gln Gly Ala Gly Val Glu Ala Cys Ala Ala Val Ser Ser Pro Asp
        355                 360                 365
Gly Ala Pro Asp Leu Gln Arg Ala Leu Ser Leu Leu Ser Asn Ser Pro
    370                 375                 380
```

```
Ala Gly Gly Gly Gly Thr Asn Thr Asn His Pro Pro Pro Pro Pro Ala
385                 390                 395                 400
Ala Glu Leu His His His Arg Ala Ala Pro Ser Ser Cys Leu Ala Ala
                405                 410                 415
Gly Ser Val Ser Val Leu Gln Glu Ala Ser Ser Pro Gly Leu Trp Gln
            420                 425                 430
Asp Gly Gly Gly Ser Ala Ala Leu Gly His His Ala His Ala Arg Leu
        435                 440                 445
Gln Ala Leu Asp Ala Ala Met Ala Thr Ala Gln Glu Leu Leu Gln Leu
    450                 455                 460
Pro Arg Pro Pro Pro Ser Tyr Gly Gly Ser Ser Ser His Tyr Asp Leu
465                 470                 475                 480
Met Arg
```

<210> 450
<211> 1320
<212> DNA
<213> Zea mays

<400> 450

```
atgggttctt ttgggatgaa ctggaatcag aaggacccca tggtgtggga ttgggaacat     60
ctagtaccgt ctgtctcaaa tgcagttaca aggcacggat ctgctaattc atctggtggt    120
actcttactt ctaactcaga gctagggcat ggttcatcca agagctctat ttcagcgtcc    180
attgattcac cctctggagt agggaacagc ttagagttca acttcgccgc tgttgagagg    240
catgttaaga acacgggcac gaacggcaga gtcgatgact cggggaattc tccatcgtca    300
atgatagctt tcaaccaagg agagccatta atcagcctga aacttgggaa gagggcttac    360
ttcgaaaacg cctgcggagg acaggatgcc aaggtttctg cagcttcaga cgttacttct    420
gcagccagcg tggtcaagaa gactaaggtg tctcagcaga atgcaaagaa ctggtactgt    480
caggttgaag ggtgcaaagt tgacctgtct tctgctaaag attacaatcg caagcacaag    540
gtctgtgtag tccattctaa agctaccaag gtggttgttg ctggtctaga gcgtcggttt    600
tgtcaacagt gtagccgttt tcatggttta gcggagtttg atcagaacaa acgaagctgt    660
cgtaggcgtc tgatgcatca taatgcacgg aggaggaaac ctcaggcaga cacaatttca    720
ttcaattcat cgacaatgtt ttatgataca aggcagcgga caaatctttt ctttagtcaa    780
ccactttatg gccaagtgag gagcaatgca gggtcttcat gggataactt gggaggctta    840
aaattcatgg agacgaaaca tccgccagtg catccaacaa aaacagcatc ccctgatgaa    900
ctgcatttct cagccctcca gataactagt gctgcggctc acaccggaca tcatcatgat    960
ctcgatgggt tcatggcgtt caagggaacc agcacaaagg tccttaacca aggcgtggag   1020
gcttgggcgg ccgcttccag ctcgaacaac ggaggcccag aaggtgggcg tgctctctct   1080
cttctgtcag acggctcgtg ggctcgagt tcagccgtca tccagcagcc cacatctcac   1140
gcggacgccg gtgcattgct gccgcccctc gccaccgttg ccgtctccaa cgccgccgcc   1200
gccgccgggc atcctctgga cccgtccccg ggaaggttct ggccgcaaga cgatcatccc   1260
ccgctcgtcg acggacccgc cacgcagatt ccggagctgg cgcacctccg gatatggtga   1320
```

<210> 451
<211> 439
<212> PRT
<213> Zea mays

<400> 451

```
Met Gly Ser Phe Gly Met Asn Trp Asn Gln Lys Asp Pro Met Val Trp
1               5                   10                  15
Asp Trp Glu His Leu Val Pro Ser Val Ser Asn Ala Val Thr Arg His
            20                  25                  30
Gly Ser Ala Asn Ser Ser Gly Gly Thr Leu Thr Ser Asn Ser Glu Leu
        35                  40                  45
Gly His Gly Ser Ser Lys Ser Ser Ile Ser Ala Ser Ile Asp Ser Pro
    50                  55                  60
Ser Gly Val Gly Asn Ser Leu Glu Phe Asn Phe Ala Ala Val Glu Arg
65                  70                  75                  80
His Val Lys Asn Thr Gly Thr Asn Gly Arg Val Asp Asp Ser Gly Asn
                85                  90                  95
Ser Pro Ser Ser Met Ile Ala Phe Asn Gln Gly Glu Pro Leu Ile Ser
            100                 105                 110
Leu Lys Leu Gly Lys Arg Ala Tyr Phe Glu Asn Ala Cys Gly Gly Gln
        115                 120                 125
Asp Ala Lys Val Ser Ala Ala Ser Asp Val Thr Ser Ala Ala Ser Val
    130                 135                 140
Val Lys Lys Thr Lys Val Ser Gln Gln Asn Ala Lys Asn Trp Tyr Cys
145                 150                 155                 160
Gln Val Glu Gly Cys Lys Val Asp Leu Ser Ser Ala Lys Asp Tyr Asn
                165                 170                 175
Arg Lys His Lys Val Cys Val Val His Ser Lys Ala Thr Lys Val Val
            180                 185                 190
Val Ala Gly Leu Glu Arg Arg Phe Cys Gln Gln Cys Ser Arg Phe His
        195                 200                 205
Gly Leu Ala Glu Phe Asp Gln Asn Lys Arg Ser Cys Arg Arg Arg Leu
    210                 215                 220
Met His His Asn Ala Arg Arg Arg Lys Pro Gln Ala Asp Thr Ile Ser
225                 230                 235                 240
Phe Asn Ser Ser Thr Met Phe Tyr Asp Thr Arg Gln Arg Thr Asn Leu
                245                 250                 255
Phe Phe Ser Gln Pro Leu Tyr Gly Gln Val Arg Ser Asn Ala Gly Ser
            260                 265                 270
Ser Trp Asp Asn Leu Gly Gly Leu Lys Phe Met Glu Thr Lys His Pro
        275                 280                 285
Pro Val His Pro Thr Lys Thr Ala Ser Pro Asp Glu Leu His Phe Ser
    290                 295                 300
Ala Leu Gln Ile Thr Ser Ala Ala Ala His Thr Gly His His His Asp
305                 310                 315                 320
Leu Asp Gly Phe Met Ala Phe Lys Gly Thr Ser Thr Lys Val Leu Asn
                325                 330                 335
Gln Gly Val Glu Ala Trp Ala Ala Ala Ser Ser Ser Asn Asn Gly Gly
            340                 345                 350
Pro Glu Gly Gly Arg Ala Leu Ser Leu Leu Ser Asp Gly Ser Trp Gly
        355                 360                 365
Ser Ser Ser Ala Val Ile Gln Gln Pro Thr Ser His Ala Asp Ala Gly
    370                 375                 380
Ala Leu Leu Pro Pro Leu Ala Thr Val Ala Val Ser Asn Ala Ala Ala
385                 390                 395                 400
Ala Ala Gly His Pro Leu Asp Pro Ser Pro Gly Arg Phe Trp Pro Gln
                405                 410                 415
Asp Asp His Pro Pro Leu Val Asp Gly Pro Ala Thr Gln Ile Pro Glu
            420                 425                 430
Leu Ala His Leu Arg Ile Trp
        435
```

<210> 452
<211> 1422
<212> DNA
<213> Triticum aestivum

<400> 452

```
atgggctcgt tcgggatgga gtggaaccag aggagctcgg tgctgtggga ctgggagaat      60
ttcccgccga taggcgagaa ccccaagaac gcgatgcagg ccgatccaag atttgccgcc     120
gttgcggcta ccatggggaa tgaaccgctc cattcttctg gcggtagcgg caccttctct     180
tccagctcag agatggggta tggctcttcc aagagctcca tgtccgcgtc gatcgattct     240
tcgaacaggg ctgggaacaa catggagttc agatttgcgc ctgtcaaaaa ccctgatagg     300
aacacgagca agaacaccga gctgggtaaa gttgataaca cgagaactgg aacatctccg     360
tcgcctgtgg tggcagtgag cagtggagag ccggtgatcg gcctgaagct tggcaagaga     420
acttacttcg aggatgtctg tggagggcag aatgtcaaga gctcgccatc gggtgctgcg     480
agcgcgccaa acaaatctcc tgctttgggc aagaaggcaa aggcggaaca acagaagcca     540

cataactcgt actgtcaggt tgaaggctgc aaagtcgacc tctcttctgt taaagattac     600
catcgaaagc acagagtctg tgaacttcac tctaaggctc cgaaagttgt tgtcgctggt     660
ctggagcgac gcttttgcca acagtgcagc cggtttcatg ctttagctga gtttgaccag     720
aaaaagcgaa gctgccgtag gcgtctcaat gatcataatt cccgcaggcg gaagccacag     780
ccagaagcaa tttctttcag ttcatcaagg atgtctacga tgttttatga tgcaaggcaa     840
cagccaaatt tcctatttgg tcaggctcct tatgttcaaa tgagaagctg tggaagttct     900
tcatgggatg acccaggagg cttcaaagtt acacacacaa aagctccttg gttaaaacca     960
acaactgctg caggtgttca tgggatacat ttatctagtc agcagatgtc ggacaatatt    1020
atgccacatg gtgcacatca tggtttcgat gggttcatgg cattcaaggg aacttgtaca    1080
aagttcccta atcaaggtgt ccaagcttct gctgttgctt ccgactccag tggagccccg    1140
gatcttcagc atgctctctc tcttctgtca agcaacccag tgggtgctgc caacctccag    1200
ccaagtcccc agatgcactc tggggtggca gccattgccg gcgcccccaa ccccgcgatg    1260
cacgcgctgg gatcatcgac ggggctctgg ctagacggca gccagcccct cgatgatcac    1320
ccgcggttcc aggtcttcga gcgcttgggg gaccatgaca gcgagctcca gctcccaaag    1380
ccttcctacg accatgcctc gcacttcgac cggatgcact ga                       1422
```

<210> 453
<211> 473
<212> PRT
<213> Triticum aestivum

<400> 453

Met Gly Ser Phe Gly Met Glu Trp Asn Gln Arg Ser Ser Val Leu Trp
1 5 10 15
Asp Trp Glu Asn Phe Pro Pro Ile Gly Glu Asn Pro Lys Asn Ala Met
20 25 30
Gln Ala Asp Pro Arg Phe Ala Ala Val Ala Ala Thr Met Gly Asn Glu
35 40 45
Pro Leu His Ser Ser Gly Gly Ser Gly Thr Phe Ser Ser Ser Ser Glu
50 55 60
Met Gly Tyr Gly Ser Ser Lys Ser Ser Met Ser Ala Ser Ile Asp Ser
65 70 75 80
Ser Asn Arg Ala Gly Asn Asn Met Glu Phe Arg Phe Ala Pro Val Lys
85 90 95
Asn Pro Asp Arg Asn Thr Ser Lys Asn Thr Glu Leu Gly Lys Val Asp
100 105 110
Asn Thr Arg Thr Gly Thr Ser Pro Ser Pro Val Val Ala Val Ser Ser
115 120 125
Gly Glu Pro Val Ile Gly Leu Lys Leu Gly Lys Arg Thr Tyr Phe Glu
130 135 140
Asp Val Cys Gly Gly Gln Asn Val Lys Ser Ser Pro Ser Gly Ala Ala
145 150 155 160
Ser Ala Pro Asn Lys Ser Pro Ala Leu Gly Lys Lys Ala Lys Ala Glu
165 170 175
Gln Gln Lys Pro His Asn Ser Tyr Cys Gln Val Glu Gly Cys Lys Val
180 185 190
Asp Leu Ser Ser Val Lys Asp Tyr His Arg Lys His Arg Val Cys Glu
195 200 205
Leu His Ser Lys Ala Pro Lys Val Val Val Ala Gly Leu Glu Arg Arg
210 215 220
Phe Cys Gln Gln Cys Ser Arg Phe His Ala Leu Ala Glu Phe Asp Gln
225 230 235 240
Lys Lys Arg Ser Cys Arg Arg Arg Leu Asn Asp His Asn Ser Arg Arg
245 250 255
Arg Lys Pro Gln Pro Glu Ala Ile Ser Phe Ser Ser Ser Arg Met Ser
260 265 270
Thr Met Phe Tyr Asp Ala Arg Gln Gln Pro Asn Phe Leu Phe Gly Gln
275 280 285
Ala Pro Tyr Val Gln Met Arg Ser Cys Gly Ser Ser Ser Trp Asp Asp
290 295 300
Pro Gly Gly Phe Lys Val Thr His Thr Lys Ala Pro Trp Leu Lys Pro

305 310 315 320
Thr Thr Ala Ala Gly Val His Gly Ile His Leu Ser Ser Gln Gln Met
325 330 335
Ser Asp Asn Ile Met Pro His Gly Ala His His Gly Phe Asp Gly Phe
340 345 350
Met Ala Phe Lys Gly Thr Cys Thr Lys Phe Pro Asn Gln Gly Val Gln
355 360 365
Ala Ser Ala Val Ala Ser Asp Ser Ser Gly Ala Pro Asp Leu Gln His
370 375 380
Ala Leu Ser Leu Leu Ser Ser Asn Pro Val Gly Ala Ala Asn Leu Gln
385 390 395 400
Pro Ser Pro Gln Met His Ser Gly Val Ala Ala Ile Ala Gly Ala Pro
405 410 415
Asn Pro Ala Met His Ala Leu Gly Ser Ser Thr Gly Leu Trp Leu Asp
420 425 430
Gly Ser Gln Pro Leu Asp Asp His Pro Arg Phe Gln Val Phe Glu Arg
435 440 445
Leu Gly Asp His Asp Ser Glu Leu Gln Leu Pro Lys Pro Ser Tyr Asp
450 455 460
His Ala Ser His Phe Asp Arg Met His
465 470

<210> 454
<211> 1491
<212> DNA
<213> Vitis vinifera

<400> 454

```
atggatgctg ttgttttgac aggtttggaa gaatctgggg taagagtaag ggtggttaat     60
tttatacagg gcgggcgaga aatggcattt ttgttggaat ctgtgcaaga agaggacttg    120
ggtagaaaca tcatcggttc tacttggtcg acttggggaa gaaaggacaa tcgatatcct    180
tctctaaaag gggataagga atttcctttc tgtggcatgt gggactggga gaaccttgac    240
attttcaatg caaaatcaac tgaaattcca aaaaagttac aatcagactg ggaaattgaa    300
ggagaaggag gaattgacac tggttctttc tattcatctg gatgtggtgc tggtagtggt    360
ggttctggct ctgatttggg acatgcatat tcatcaaaga gctcaaaatc agcttcaatt    420
gattcttcat caaaggtggg aataaagaca tccaacttca cttttgaggc tttccaagat    480
ttttctcaag attttaacaa gaagagagat ttggagaggg ctgagccaac tggaagttct    540
ccaaaccttg aagcctcgat tggctctggt gaacctttaa ttggtctaaa gcttggtaaa    600
aggacatact tcgaggatgt ttctgcaggg ggcaatgcta agggctcagt gttttctgtg    660
attcccatat catctgatac cacagcaaag agatccaggt tatcttgtca gaatgcacat    720
gtctcgcgct gccaagttga aggatgcaac cttgaccttt caacagccaa agattaccat    780
cgcaaacata gagtttgtga aagtcatacc aaatgcccaa aggtcattgt aggtggtctg    840
gagcgccgtt tttgccaaca gtgtagcagg ttccatagtt tgtcagagtt tgatgaaaag    900
aagagaagct gtcgcaggcg tctttctgat cacaatgcac gccggcgcaa accacaacca    960
gaagggatcc agtcaaattc ggcgaggctt tcatcattct atggtgggaa gcagcaaatg   1020
agtcttgtgt tgagctcggt ccccattgtt catacaaggc ctgctgtaaa tccttcatgg   1080
gaaggcacat gcagcaagtt cacacaaaca aaagggttag taaggcctgg taacacagga   1140
ggtatcgacc ggcagctgca tttgcctggc agtgagctgc agaatggcat tagtacgctt   1200
tgtcatgatt ccagtaggct tttgcactct aagggcgcaa tagctgaggt tctcaatcaa   1260
gtgtcccaag gtatgttaca ttcaacctca gaacactgga ggaccgagct tccctcaact   1320
gattcccggg tgcatatctt gccttcacaa ggcgatggta acacccactt tcaagagttc   1380
aagctgttca aagcgccata cgagtctggc ttttcttctg gatattcaga gtatagggaa   1440
ttgagatatc cgattcctag gagtaacgcc cgtgtcatga agcaaacata g             1491
```

<210> 455
<211> 496
<212> PRT
<213> Vitis vinifera

<400> 455

```
Met Asp Ala Val Val Leu Thr Gly Leu Glu Glu Ser Gly Val Arg Val
```

1 5 10 15
Arg Val Val Asn Phe Ile Gln Gly Gly Arg Glu Met Ala Phe Leu Leu
20 25 30
Glu Ser Val Gln Glu Glu Asp Leu Gly Arg Asn Ile Ile Gly Ser Thr
35 40 45
Trp Ser Thr Trp Gly Arg Lys Asp Asn Arg Tyr Pro Ser Leu Lys Gly
50 55 60
Asp Lys Glu Phe Pro Phe Cys Gly Met Trp Asp Trp Glu Asn Leu Asp
65 70 75 80
Ile Phe Asn Ala Lys Ser Thr Glu Ile Pro Lys Lys Leu Gln Ser Asp
85 90 95
Trp Glu Ile Glu Gly Glu Gly Gly Ile Asp Thr Gly Ser Phe Tyr Ser
100 105 110
Ser Gly Cys Gly Ala Gly Ser Gly Gly Ser Gly Ser Asp Leu Gly His
115 120 125
Ala Tyr Ser Ser Lys Ser Ser Lys Ser Ala Ser Ile Asp Ser Ser Ser
130 135 140
Lys Val Gly Ile Lys Thr Ser Asn Phe Thr Phe Glu Ala Phe Gln Asp
145 150 155 160
Phe Ser Gln Asp Phe Asn Lys Lys Arg Asp Leu Glu Arg Ala Glu Pro
165 170 175
Thr Gly Ser Ser Pro Asn Leu Glu Ala Ser Ile Gly Ser Gly Glu Pro
180 185 190
Leu Ile Gly Leu Lys Leu Gly Lys Arg Thr Tyr Phe Glu Asp Val Ser
195 200 205
Ala Gly Gly Asn Ala Lys Gly Ser Val Phe Ser Val Ile Pro Ile Ser
210 215 220
Ser Asp Thr Thr Ala Lys Arg Ser Arg Leu Ser Cys Gln Asn Ala His
225 230 235 240
Val Ser Arg Cys Gln Val Glu Gly Cys Asn Leu Asp Leu Ser Thr Ala
245 250 255
Lys Asp Tyr His Arg Lys His Arg Val Cys Glu Ser His Thr Lys Cys
260 265 270
Pro Lys Val Ile Val Gly Gly Leu Glu Arg Arg Phe Cys Gln Gln Cys
275 280 285
Ser Arg Phe His Ser Leu Ser Glu Phe Asp Glu Lys Lys Arg Ser Cys
290 295 300
Arg Arg Arg Leu Ser Asp His Asn Ala Arg Arg Arg Lys Pro Gln Pro
305 310 315 320
Glu Gly Ile Gln Ser Asn Ser Ala Arg Leu Ser Ser Phe Tyr Gly Gly
325 330 335
Lys Gln Gln Met Ser Leu Val Leu Ser Ser Val Pro Ile Val His Thr
340 345 350
Arg Pro Ala Val Asn Pro Ser Trp Glu Gly Thr Cys Ser Lys Phe Thr
355 360 365
Gln Thr Lys Gly Leu Val Arg Pro Gly Asn Thr Gly Gly Ile Asp Arg
370 375 380
Gln Leu His Leu Pro Gly Ser Glu Leu Gln Asn Gly Ile Ser Thr Leu
385 390 395 400
Cys His Asp Ser Ser Arg Leu Leu His Ser Lys Gly Ala Ile Ala Glu
405 410 415
Val Leu Asn Gln Val Ser Gln Gly Met Leu His Ser Thr Ser Glu His
420 425 430
Trp Arg Thr Glu Leu Pro Ser Thr Asp Ser Arg Val His Ile Leu Pro
435 440 445
Ser Gln Gly Asp Gly Asn Thr His Phe Gln Glu Phe Lys Leu Phe Lys
450 455 460
Ala Pro Tyr Glu Ser Gly Phe Ser Ser Gly Tyr Ser Glu Tyr Arg Glu
465 470 475 480
Leu Arg Tyr Pro Ile Pro Arg Ser Asn Ala Arg Val Met Lys Gln Thr
485 490 495

<210> 456

<211> 79
<212> PRT
<213> Artificial sequence

<220>
<223> sbp domain of SEQ ID NO: 2

<400> 456

```
Arg Cys Gln Ile Asp Gly Cys Glu Leu Asp Leu Ser Ser Ala Lys Gly
1               5                   10                  15
Tyr His Arg Lys His Lys Val Cys Glu Lys His Ser Lys Cys Pro Lys
            20                  25                  30
Val Ser Val Ser Gly Leu Glu Arg Arg Phe Cys Gln Gln Cys Ser Arg
        35                  40                  45
Phe His Ala Val Ser Glu Phe Asp Glu Lys Lys Arg Ser Cys Arg Lys
    50                  55                  60
Arg Leu Ser His His Asn Ala Arg Arg Arg Lys Pro Gln Gly Val
65                  70                  75
```

<210> 457
<211> 79
<212> PRT
<213> Artificial sequence

<220>
<223> sbp domain of SEQ ID NO: 7

<400> 457

```
Arg Cys Gln Ile Asp Gly Cys Glu Leu Asp Leu Ser Ser Ser Lys Asp
1               5                   10                  15
Tyr His Arg Lys His Arg Val Cys Glu Thr His Ser Lys Cys Pro Lys
            20                  25                  30
Val Val Val Ser Gly Leu Glu Arg Arg Phe Cys Gln Gln Cys Ser Arg
        35                  40                  45
Phe His Ala Val Ser Glu Phe Asp Glu Lys Lys Arg Ser Cys Arg Lys
    50                  55                  60
Arg Leu Ser His His Asn Ala Arg Arg Arg Lys Pro Gln Gly Val
65                  70                  75
```

<210> 458
<211> 79
<212> PRT
<213> Artificial sequence

<220>
<223> sbp domain of SEQ ID NO: 9

<400> 458

```
His Cys Gln Val Glu Gly Cys Asn Leu Asp Leu Ser Ser Ala Lys Asp
1               5                   10                  15
Tyr His Arg Lys His Arg Ile Cys Glu Asn His Ser Lys Phe Pro Lys
            20                  25                  30
Val Val Val Ser Gly Val Glu Arg Arg Phe Cys Gln Gln Cys Ser Arg
        35                  40                  45
Phe His Cys Leu Ser Glu Phe Asp Glu Lys Lys Arg Ser Cys Arg Arg
    50                  55                  60
Arg Leu Ser Asp His Asn Ala Arg Arg Arg Lys Pro Asn Pro Gly
65                  70                  75
```

<210> 459
<211> 79
<212> PRT
<213> Artificial sequence

<220>
<223> sbp domain of SEQ ID NO: 11

<400> 459

```
His Cys Gln Val Glu Gly Cys Asn Val Asp Leu Ser Ser Ala Lys Pro
1               5                   10                  15
Tyr His Arg Lys His Arg Val Cys Glu Pro His Ser Lys Thr Leu Lys
            20                  25                  30
Val Ile Val Ala Gly Leu Glu Arg Arg Phe Cys Gln Gln Cys Ser Arg
        35                  40                  45
Phe His Gly Leu Ala Glu Phe Asp Gln Lys Lys Arg Ser Cys Arg Arg
    50                  55                  60
Arg Leu His Asp His Asn Ala Arg Arg Arg Lys Pro Gln Pro Glu
65                  70                  75
```

<210> 460
<211> 79
<212> PRT
<213> Artificial sequence

<220>
<223> sbp domain of SEQ ID NO: 13

<400> 460

```
Tyr Cys Gln Val Glu Gly Cys Lys Val Asp Leu Ser Ser Ala Arg Glu
1               5                   10                  15
Tyr His Arg Lys His Lys Val Cys Glu Ala His Ser Lys Ala Pro Lys
            20                  25                  30
Val Ile Val Ser Gly Leu Glu Arg Arg Phe Cys Gln Gln Cys Ser Arg
        35                  40                  45
Phe His Gly Leu Ala Glu Phe Asp Gln Lys Lys Lys Ser Cys Arg Arg
    50                  55                  60
Arg Leu Ser Asp His Asn Ala Arg Arg Arg Lys Pro Gln Gln Glu
65                  70                  75
```

<210> 461
<211> 108
<212> PRT
<213> Artificial sequence

<220>
<223> sbp domain of SEQ ID NO: 15

<400> 461

```
Thr Pro Ala Lys Lys Leu Lys Ser Ser Asn His Ser Gln Arg Ala Pro
1               5                   10                  15
Arg Cys Gln Val Glu Gly Cys Asn Leu Asp Leu Ser Ser Ala Lys Asp
            20                  25                  30
Tyr His Arg Lys His Arg Val Cys Glu Ser His Ser Lys Cys Pro Lys
        35                  40                  45
Val Ile Val Ala Gly Leu Glu Arg Arg Phe Cys Gln Gln Cys Ser Arg
    50                  55                  60
Phe His Gly Leu Ser Glu Phe Asp Glu Lys Lys Lys Ser Cys Arg Arg
65                  70                  75                  80
Arg Leu Ser Asp His Asn Ala Arg Arg Arg Lys Gln Pro Gly Ser Val
                85                  90                  95

            His Leu Asn Pro Ser Arg Leu Ser Ser Ser Leu Tyr
                        100                 105
```

<210> 462
<211> 98
<212> PRT
<213> Artificial sequence

<220>
<223> sbp domain of SEQ ID NO: 17

<400> 462

```
Cys Cys Gln Val Glu Gly Cys Asn Leu Asp Leu Ser Ser Ala Lys Asp
1               5                   10                  15
Tyr His Arg Lys His Arg Val Cys Glu Ser His Ser Lys Cys Gln Lys
            20                  25                  30
Val Ile Val Ala Gly Leu Glu Arg Arg Phe Cys Gln Gln Cys Ser Ser
        35                  40                  45
Lys Ile Ile Pro Ser Phe Tyr Asp Cys Ile Asp Asn Leu Gly Phe Gly
    50                  55                  60
Cys Gln Arg Phe His Gly Leu Ser Glu Phe Asp Glu Lys Lys Lys Ser
65                  70                  75                  80
Cys Arg Arg Arg Leu Ser Asp His Asn Ala Arg Arg Arg Lys Gln Pro
                85                  90                  95
Gly Ser
```

<210> 463
<211> 98
<212> PRT
<213> Artificial sequence

<220>
<223> sbp domain of SEQ ID NO: 19

<400> 463

```
Asp Leu Lys Ser Ala Lys Asp Tyr His Arg Arg His Arg Ile Cys Glu
1               5                   10                  15
Lys His Ser Lys Ser Pro Lys Val Ile Val Ala Gly Met Glu Arg Arg
            20                  25                  30
Phe Cys Gln Gln Cys Ser Arg Phe His Glu Leu Ser Glu Phe Asp Asp
        35                  40                  45
Lys Lys Arg Ser Cys Arg Arg Arg Leu Ser Asp His Asn Ala Arg Arg
    50                  55                  60
Arg Arg Pro Gln Pro Glu Ala Ile Arg Phe Asn Ser Ala Arg Pro Ser
65                  70                  75                  80
Ser Ser Pro Ser Ser Phe Tyr Gly Cys His Phe Asn Phe Lys Asp Gly
                85                  90                  95
Arg Gln
```

<210> 464
<211> 79
<212> PRT
<213> Artificial sequence

<220>
<223> sbp domain of SEQ ID NO: 21

<400> 464

```
Thr Leu Ser Gln Lys Leu Gln Thr Pro His Cys Gln Val Glu Gly Cys
1               5                   10                  15
Asn Leu Asp Leu Ser Ser Ala Lys Asp Tyr His Arg Lys His Arg Ile
            20                  25                  30
Cys Glu Asn His Ser Lys Phe Pro Lys Val Val Val Ser Gly Val Glu
        35                  40                  45
Arg Arg Phe Cys Gln Gln Cys Ser Arg Lys Ser Ser Arg Tyr Trp Phe
    50                  55                  60
Ile Gln Lys Pro Leu Phe Leu Ile Val Phe Gly Leu Gln Arg Phe
65                  70                  75
```

<210> 465
<211> 82
<212> PRT
<213> Artificial sequence

<220>
<223> sbp domain of SEQ ID NO: 23

<400> 465

```
Ser Tyr Cys Gln Val Glu Gly Cys Arg Thr Asp Leu Ser Ser Ala Lys
1               5                   10                  15
Asp Tyr His Arg Lys His Arg Val Cys Glu Pro His Ser Lys Ala Pro
            20                  25                  30
Lys Val Val Val Ala Gly Leu Glu Arg Arg Phe Cys Gln Gln Cys Ser
        35                  40                  45
Arg Phe His Gly Leu Ala Glu Phe Asp Gln Lys Lys Lys Ser Cys Arg
    50                  55                  60
Arg Arg Leu Asn Asp His Asn Ala Arg Arg Arg Lys Pro Gln Pro Glu
65                  70                  75                  80
Ala Leu
```

<210> 466

<211> 75
<212> PRT
<213> Artificial sequence

<220>
<223> sbp domain of SEQ ID NO: 25

<400> 466

```
Cys Gln Val Glu Gly Cys Lys Val Asp Leu Ser Ser Ala Lys Asp Tyr
1               5                   10                  15
Asn Arg Lys His Lys Val Cys Val Val His Ser Lys Ala Thr Lys Val
            20                  25                  30
Val Val Ala Gly Leu Glu Arg Arg Phe Cys Gln Gln Cys Ser Arg Phe
        35                  40                  45
His Gly Leu Ala Glu Phe Asp Gln Asn Lys Arg Ser Cys Arg Arg Arg
    50                  55                  60
Leu Met His His Asn Ala Arg Arg Arg Lys Pro
65                  70                  75
```

<210> 467
<211> 85
<212> PRT
<213> Artificial sequence

<220>
<223> sbp domain of SEQ ID NO: 27

```
<400>  467
Tyr Cys Gln Val Glu Gly Cys Lys Val Asp Leu Ser Ser Val Lys Asp
1               5                   10                  15
Tyr His Arg Lys His Arg Val Cys Glu Leu His Ser Lys Ala Pro Lys
            20                  25                  30
Val Val Val Ala Gly Leu Glu Arg Arg Phe Cys Gln Gln Cys Ser Arg
        35                  40                  45
Phe His Ala Leu Ala Glu Phe Asp Gln Lys Lys Arg Ser Cys Arg Arg
    50                  55                  60
Arg Leu Asn Asp His Asn Ser Arg Arg Arg Lys Pro Gln Pro Glu Ala
65                  70                  75                  80
Ile Ser Phe Ser Ser
                85
```

<210> 468
<211> 81
<212> PRT
<213> Artificial sequence

<220>
<223> sbp domain of SEQ ID NO: 29

<400> 468

```
Arg Cys Gln Val Glu Gly Cys Asn Leu Asp Leu Ser Thr Ala Lys Asp
1               5                   10                  15
Tyr His Arg Lys His Arg Val Cys Glu Ser His Thr Lys Cys Pro Lys
            20                  25                  30
Val Ile Val Gly Gly Leu Glu Arg Arg Phe Cys Gln Gln Cys Ser Arg
        35                  40                  45
Phe His Ser Leu Ser Glu Phe Asp Glu Lys Lys Arg Ser Cys Arg Arg
    50                  55                  60
Arg Leu Ser Asp His Asn Ala Arg Arg Arg Lys Pro Gln Pro Glu Gly
65                  70                  75                  80
Ile
```

<210> 469
<211> 6
<212> PRT
<213> Artificial sequence

<220>
<223> motif 1

<400> 469

```
Trp Asp Trp Glu Asn Leu
1               5
```

<210> 470
<211> 12
<212> PRT
<213> Artificial sequence

<220>
<223> motif 2

<400> 470

```
Ser Lys Ser Ser Gln Ser Thr Ser Ile Asn Ser Ser
1               5                   10
```

<210> 471
<211> 7
<212> PRT
<213> Artificial sequence

<220>
<223> motif 3

<400> 471
Ala Leu Ser Leu Leu Ser Thr

1 5

<210> 472
<211> 6
<212> PRT
<213> Artificial sequence

<220>
<223> motif 4

<400> 472

Leu Lys Leu Gly Lys Arg
1               5

<210> 473
<211> 56
<212> DNA
<213> Artificial sequence

<220>
<223> primer 1

<400> 473
ggggacaagt ttgtacaaaa aagcaggctt aaacaatgga ctgcaacatg gtatct 56

<210> 474
<211> 54
<212> DNA
<213> Artificial sequence

<220>
<223> primer 2

<400> 474
ggggaccact ttgtacaaga aagctgggtc acattactta ctcatctatt ttgg 54

<210> 475
<211> 9
<212> DNA
<213> Artificial sequence

<220>
<223> promoter box

<220>
<221> misc_feature
<222> (2)..(2)
<223> n is a, c, g, or t

<400> 475
tncgtacaa 9

<210> 476
<211> 2194
<212> DNA
<213> Oryza sativa

<400> 476

```
aatccgaaaa gtttctgcac cgttttcacc ccctaactaa caatataggg aacgtgtgct      60
aaatataaaa tgagacctta tatatgtagc gctgataact agaactatgc aagaaaaact     120
catccaccta ctttagtggc aatcgggcta aataaaaaag agtcgctaca ctagtttcgt     180
tttccttagt aattaagtgg gaaaatgaaa tcattattgc ttagaatata cgttcacatc     240
tctgtcatga agttaaatta ttcgaggtag ccataattgt catcaaactc ttcttgaata     300
aaaaaatctt tctagctgaa ctcaatgggt aaagagagag atttttttta aaaaaataga     360
atgaagatat tctgaacgta ttggcaaaga tttaaacata taattatata atttatagt      420
ttgtgcattc gtcatatcgc acatcattaa ggacatgtct tactccatcc caatttttat     480
ttagtaatta aagacaattg acttattttt attatttatc tttttcgat tagatgcaag      540
gtacttacgc acacactttg tgctcatgtg catgtgtgag tgcacctcct caatacacgt     600
tcaactagca acacatctct aatatcactc gcctatttaa tacatttagg tagcaatatc     660
tgaattcaag cactccacca tcaccagacc acttttaata atatctaaaa tacaaaaaat     720
aattttacag aatagcatga aaagtatgaa acgaactatt taggtttttc acatacaaaa     780
aaaaaaagaa ttttgctcgt gcgcgagcgc caatctccca tattgggcac acaggcaaca     840
acagagtggc tgcccacaga acaacccaca aaaaacgatg atctaacgga ggacagcaag     900
tccgcaacaa ccttttaaca gcaggctttg cggccaggag agaggaggag aggcaaagaa     960
aaccaagcat cctccttctc ccatctataa attcctcccc ccttttcccc tctctatata    1020
ggaggcatcc aagccaagaa gagggagagc accaaggaca cgcgactagc agaagccgag    1080
cgaccgcctt ctcgatccat atcttccggt cgagttcttg gtcgatctct tccctcctcc    1140
acctcctcct cacagggtat gtgcctccct tcggttgttc ttggatttat tgttctaggt    1200
tgtgtagtac gggcgttgat gttaggaaag gggatctgta tctgtgatga ttcctgttct    1260
tggatttggg atagaggggt tcttgatgtt gcatgttatc ggttcggttt gattagtagt    1320
atggttttca atcgtctgga gagctctatg gaaatgaaat ggtttaggga tcggaatctt    1380
gcgattttgt gagtaccttt tgtttgaggt aaaatcagag caccggtgat tttgcttggt    1440
gtaataaagt acggttgttt ggtcctcgat tctggtagtg atgcttctcg atttgacgaa    1500
gctatccttt gtttattccc tattgaacaa aaataatcca actttgaaga cggtcccgtt    1560
gatgagattg aatgattgat tcttaagcct gtccaaaatt tcgcagctgg cttgtttaga    1620
tacagtagtc cccatcacga aattcatgga aacagttata atcctcagga acaggggatt    1680
ccctgttctt ccgatttgct ttagtcccag aatttttttt cccaaatatc ttaaaaagtc    1740
actttctggt tcagttcaat gaattgattg ctacaaataa tgcttttata gcgttatcct    1800
agctgtagtt cagttaatag gtaatacccc tatagtttag tcaggagaag aacttatccg    1860
atttctgatc tccattttta attatatgaa atgaactgta gcataagcag tattcatttg    1920
gattattttt tttattagct ctcacccctt cattattctg agctgaaagt ctggcatgaa    1980
ctgtcctcaa ttttgtttc aaattcacat cgattatcta tgcattatcc tcttgtatct      2040
acctgtagaa gtttcttttt ggttattcct tgactgcttg attacagaaa gaaatttatg    2100
aagctgtaat cgggatagtt atactgcttg ttcttatgat tcatttcctt tgtgcagttc    2160
ttggtgtagc ttgccacttt caccagcaaa gttc                                2194
```

<210> 477
<211> 1827
<212> DNA
<213> Oryza sativa

<400> 477

```
gcttgagtca tagggagaaa acaaatcgat catatttgac tcttttccct ccatctctct      60
taccggcaaa aaaagtagta ctggtttata tgtaaagtaa gattctttaa ttatgtgaga     120
tccggcttaa tgcttttctt ttgtcacata tactgcattg caacaattgc catatattca     180
cttctgccat cccattatat agcaactcaa gaatggattg atatatcccc tattactaat     240
ctagacatgt taaggctgag ttgggcagtc catcttccca acccaccacc ttcgtttttc     300
gcgcacatac ttttcaaact actaaatggt gtgtttttta aaaatatttt caatacaaaa     360
gttgctttaa aaaattatat tgatccattt ttttaaaaaa aatagctaat acttaattaa     420
tcacgtgtta aaagaccgct ccgttttgcg tgcaggaggg ataggttcac atcctgcatt     480
accgaacaca gcctaaatct tgttgtctag attcgtagta ctggatatat taaatcatgt     540
tctaagttac tatatactga gatgaataga ataagtaaaa ttagacccac cttaagtctt     600
gatgaagtta ctactagctg cgtttgggag gacttcccaa aaaaaaaagt attagccatt     660
```

```
agcacgtgat taattaagta ctagtttaaa aaacttaaaa aataaattaa tatgattctc    720
ttaagtaact ctcctataga aaacttttac aaaattacac cgtttaatag tttggaaaat    780
atgtcagtaa aaaataagag agtagaagtt atgaaagtta gaaaaagaat tgttttagta    840
gtatacagtt ataaactatt ccctctgttc taaaacataa gggattatgg atggattcga    900
catgtaccag taccatgaat cgaatccaga caagtttttt atgcatattt attctactat    960
aatatatcac atctgctcta aatatcttat atttcgaggt ggagactgtc gctatgtttt   1020
tctgcccgtt gctaagcaca cgccaccccc gatgcgggga cgcctctggc cttcttgcca   1080
cgataattga atggaacttc cacattcaga ttcgataggt gaccgtcgac tccaagtgct   1140
ttgcacaaaa caactccggc ctcccggcca ccagtcacac gactcacggc actaccaccc   1200
ctgactccct gaggcggacc tgccactgtt ctgcatgcga agctatctaa aattctgaag   1260
caaagaaagc acagcacatg ctccgggaca cgcgccaccc ggcggaaaag ggctcggtgt   1320
ggcgatctca cagccgcata tcgcatttca caagccgccc atctccaccg gcttcacgag   1380
gctcatcgcg gcacgaccgc gcacggaacg cacgcggccg acccgcgcgc ctcgatgcgc   1440
gagcccatcc gccgcgtcct ccctttgcct ttgccgctat cctctcggtc gtatcccgtt   1500
tctctgtctt ttgctccccg gcgcgcgcca gttcggagta ccagcgaaac ccggacacct   1560
ggtacacctc cgccggccac aacgcgtgtc cccctacgtg gccgcgcagc acatgcccat   1620
gcgcgacacg tgcacctcct catccaaact ctcaagtctc aacggtccta taaatgcacg   1680
gatagcctca agctgctcgt cacaaggcaa gaggcaagag gcaagagcat ccgtattaac   1740
cagccttttg agacttgaga gtgtgtgtga ctcgatccag cgtagtttca gttcgtgtgt   1800
tggtgagtga ttccagccaa gtttgcg                                       1827
```

<210> 478
<211> 100
<212> PRT
<213> Artificial sequence

<220>
<223> consensu sbp domain on SPL11 polypeptides

<220>
<221> UNSURE
<222> (26)..(26)
<223> Xaa can be any naturally occurring amino acid

<220>
<221> UNSURE
<222> (30)..(30)
<223> Xaa can be any naturally occurring amino acid

<220>
<221> UNSURE
<222> (49)..(69)
<223> Xaa can be any naturally occurring amino acid or a gap

<400> 478

```
His Cys Gln Val Glu Gly Cys Asn Leu Asp Leu Ser Ser Ala Lys Asp
1               5                   10                  15
Tyr His Arg Lys His Arg Val Cys Glu Xaa His Ser Lys Xaa Pro Lys
            20                  25                  30
Val Ile Val Ala Gly Leu Glu Arg Arg Phe Cys Gln Gln Cys Ser Arg
        35                  40                  45
Xaa Xaa Xaa Xaa Xaa Xaa Xaa Xaa Xaa Xaa Xaa Xaa Xaa Xaa Xaa Xaa
    50                  55                  60
Xaa Xaa Xaa Xaa Xaa Phe His Gly Leu Ser Glu Phe Asp Glu Lys Lys
65                  70                  75                  80
Arg Ser Cys Arg Arg Arg Leu Ser Asp His Asn Ala Arg Arg Arg Lys
                85                  90                  95
Pro Gln Pro Glu
            100
```